# EUROPEAN PATENT SPECIFICATION

(11) **EP 2 970 215 B1**
(45) Date of publication and mention of the grant of the patent: **17.04.2019**
(21) Application number: 14710280.0
(22) Date of filing: 14.03.2014
(51) Int. Cl.: C07D 403/12, C07D 401/14, C07D 401/12, C07D 403/14, C07D 405/12, C07D 409/12, C07D 413/12, C07D 417/12, C07D 207/48, A61K 31/4155, A61K 31/4178, A61K 31/4439, A61P 25/00

(54) **PYRROLIDINE DERIVATIVES, PHARMACEUTICAL COMPOSITIONS CONTAINING THEM, AND THEIR USE IN THERAPY**
PYRROLIDINDERIVATE, PHARMAZEUTISCHE ZUSAMMENSETZUNGEN DAMIT UND DEREN VERWENDUNG IN DER THERAPIE
DÉRIVÉS DE PYRROLIDINE, COMPOSITIONS PHARMACEUTIQUES LES CONTENANT, ET LEUR UTILISATION EN THÉRAPIE

(30) Priority: 15.03.2013 US 201361788538 P; 15.03.2013 US 201361789382 P
(43) Date of publication of application: 20.01.2016
(73) Proprietor: AbbVie Deutschland GmbH & Co. KG, 65189 Wiesbaden (DE); AbbVie Inc., North Chicago, IL 60064 (US)
(72) Inventor: AMBERG, Wilhelm, 67061 Ludwigshafen (DE); POHLKI, Frauke, 67061 Ludwigshafen (DE); LANGE, Udo, 67061 Ludwigshafen (DE); WANG, Ying X., Libertyville, Illinois 60048 (US); ZHAO, Hongyu H., Libertyville, Illinois 60048 (US); LI, Huan-Qiu, Wilmette, Illinois 60091 (US); BREWER, Jason T., Zion, Illinois 60099 (US); ZANZE, Irini, Libertyville, Illinois 60048 (US); DIETRICH, Justin, Lindenhurst, Illinois 60046-4985 (US); VASUDEVAN, Anil, Union Grove, Wisconsin 53182 (US); DJURIC, Stevan, W., Libertyville, Illinois 60048 (US); LAO, Yanbin, Abbott Park, Illinois 60064-6050 (US); HUTCHINS, Charles W., Green Oaks, Illinois 60048 (US)
(74) Representative: Reitstötter Kinzebach
(86) International application number: PCT/EP2014/055159
(87) International publication number: WO 2014/140310

(56) References cited:
- WO-A1-03/068220
- WO-A1-2005/009996
- WO-A1-2012/020130
- US-A- 3 867 391
- US-A1- 2008 045 540
- SHIGEKI MATSUNAGA ET AL: "Linked-BINOL: An Approach towards Practical Asymmetric Multifunctional Catalysis", ADVANCED SYNTHESIS & CATALYSIS, vol. 344, no. 1, 1 January 2002 (2002-01-01), page 3, XP55117294, ISSN: 1615-4150, DOI: 10.1002/1615-4169(200201)344:1<3::AID-ADSC 3>3.0.CO;2-2
- KARINE ESTIEU ET AL: "New Alkylidenecyclopropane Amino Acid Derivatives for an Efficient Construction of the 6 H -Pyrrolo[3,4- b ]pyridine Skeleton", THE JOURNAL OF ORGANIC CHEMISTRY, vol. 62, no. 24, 1 November 1997 (1997-11-01), pages 8276-8277, XP55117296, ISSN: 0022-3263, DOI: 10.1021/jo9709615
- POORNACHANDRAN M ET AL: "Synthesis of pyrrolo[3,4-b]pyrroles and perhydrothiazolo[3',4'-2,3]pyrrolo[4,5-c]p yrroles", TETRAHEDRON, ELSEVIER SCIENCE PUBLISHERS, AMSTERDAM, NL, vol. 64, no. 27, 30 June 2008 (2008-06-30) , pages 6461-6474, XP022695627, ISSN: 0040-4020, DOI: 10.1016/J.TET.2008.04.063 [retrieved on 2008-04-20]
- PISANESCHI F ET AL: "Diastereoselective cycloaddition of alkylidenecyclopropane nitrones from palladium(0)-catalyzed nucleophilic substitution of asymmetric 1-alkenylcyclopropyl esters by amino acids", TETRAHEDRON ASYMMETRY, PERGAMON PRESS LTD, OXFORD, GB, vol. 11, no. 4, 1 March 2000 (2000-03-01), pages 897-909, XP004192902, ISSN: 0957-4166, DOI: 10.1016/S0957-4166(00)00006-9
- UNGUREANU I ET AL: "The reactivity of N-tosylphenylaziridine versus N-tosylphenylazetidine in heterocyclization reactions", TETRAHEDRON LETTERS, PERGAMON, GB, vol. 42, no. 35, 27 August 2001 (2001-08-27), pages 6087-6091, XP004298188, ISSN: 0040-4039, DOI: 10.1016/S0040-4039(01)01117-0
- MARCUS BAUMANN ET AL: "Synthesis of a Drug-Like Focused Library of Trisubstituted Pyrrolidines Using Integrated Flow Chemistry and Batch Methods", ACS COMBINATORIAL SCIENCE, vol. 13, no. 4, 11 July 2011 (2011-07-11), pages 405-413, XP55117263, ISSN: 2156-8952, DOI: 10.1021/co2000357 cited in the application
- DATABASE REGISTRY [Online] CHEMICAL ABSTRACTS SERVICE, COLUMBUS, OHIO, US; 3 December 2012 (2012-12-03), XP002724197, Database accession no. 1410185-83-9
- DATABASE REGISTRY [Online] CHEMICAL ABSTRACTS SERVICE, COLUMBUS, OHIO, US; 18 September 2012 (2012-09-18), XP002724198, Database accession no. 1394552-70-5
- DATABASE REGISTRY [Online] CHEMICAL ABSTRACTS SERVICE, COLUMBUS, OHIO, US; 4 July 2012 (2012-07-04), XP002724199, Database accession no. 1381432-38-7
- DATABASE REGISTRY [Online] CHEMICAL ABSTRACTS SERVICE, COLUMBUS, OHIO, US; 3 February 2014 (2014-02-03), XP002724190, Database accession no. 1535994-72-9
- DATABASE REGISTRY [Online] CHEMICAL ABSTRACTS SERVICE, COLUMBUS, OHIO, US; 27 January 2014 (2014-01-27), XP002724191, Database accession no. 1530956-16-1
- DATABASE REGISTRY [Online] CHEMICAL ABSTRACTS SERVICE, COLUMBUS, OHIO, US; 16 January 2014 (2014-01-16), XP002724192, Database accession no. 1521424-46-3
- DATABASE REGISTRY [Online] CHEMICAL ABSTRACTS SERVICE, COLUMBUS, OHIO, US; 9 January 2014 (2014-01-09), XP002724193, Database accession no. 1515211-93-4
- DATABASE REGISTRY [Online] CHEMICAL ABSTRACTS SERVICE, COLUMBUS, OHIO, US; 29 December 2013 (2013-12-29), XP002724194, Database accession no. 1506112-12-4
- DATABASE REGISTRY [Online] CHEMICAL ABSTRACTS SERVICE, COLUMBUS, OHIO, US; 5 June 2013 (2013-06-05), XP002724195, Database accession no. 1434399-98-0
- DATABASE REGISTRY [Online] CHEMICAL ABSTRACTS SERVICE, COLUMBUS, OHIO, US; 4 June 2013 (2013-06-04), XP002724196, Database accession no. 1434168-57-6

## Description

The present invention relates to pyrrolidine derivatives, pharmaceutical compositions comprising such pyrrolidine derivatives, and to such pyrrolidine derivatives for use in therapeutic methods.

### Background of the Invention

The pyrrolidine motif is an important pharmacophore possessing biological activity against a number of different targets and thus has found use in various advanced pharmaceutical research compounds and clinical candidates (such as Factor Xa inhibitors, NK3 receptor antagonists, DPP-IV inhibitors, PDE-IV inhibitors, or MC4 receptorselective agonists).

A pyrrolidine compound already known in the art as type IV phosphodiesterase inhibitor (PDE-IV) is for example:

| | |
|---|---|
| 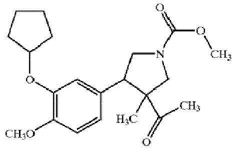 | US 5,665,754 |

WO 9508534, US 2006074123, WO 2001047915, US 20020169196, WO 2001047879 and WO 2001047914 describe further PDE-IV inhibitors having related structures.

Further, the synthesis of certain trisubstituted pyrrolidine derivatives has been reported in Baumann Marcus, et al., ACS Comb. Sci. 2011, 13, 405-413.

Dysfunction of glutamatergic pathways has been implicated in a number of disease states in the human central nervous system (CNS) including but not limited to schizophrenia, cognitive deficits, dementia, Parkinson disease, Alzheimer disease and bipolar disorder. A large number of studies in animal models lend support to the NMDA hypofunction hypothesis of schizophrenia.

NMDA receptor function can be modulated by altering the availability of the co-agonist glycine. This approach has the critical advantage of maintaining activity-dependent activation of the NMDA receptor because an increase in the synaptic concentration of glycine will not produce an activation of NMDA receptors in the absence of glutamate. Since synaptic glutamate levels are tightly maintained by high affinity transport mechanisms, an increased activation of the glycine site will only enhance the NMDA component of activated synapses.

Two specific glycine transporters, GlyT1 and GlyT2 have been identified and shown to belong to the Na/Cl-dependent family of neurotransmitter transporters which includes taurine, gamma-aminobutyric acid (GABA), proline, monoamines and orphan transporters. GlyT1 and GlyT2 have been isolated from different species and shown to have only 50% identity at the amino acid level. They also have a different pattern of expression in mammalian central nervous system, with GlyT2 being expressed in spinal cord, brainstem and cerebellum and GlyT1 present in these regions as well as forebrain areas such as cortex, hippocampus, septum and thalamus. At the cellular level, GlyT2 has been reported to be expressed by glycinergic nerve endings in rat spinal cord whereas GlyT1 appears to be preferentially expressed by glial cells. These expression studies have led to the suggestion that GlyT2 is predominantly responsible for glycine uptake at glycinergic synapses whereas GlyT1 is involved in monitoring glycine concentration in the vicinity of NMDA receptor expressing synapses. Recent functional studies in rat have shown that blockade of GlyT1 with the potent inhibitor (N-[3-(4'-fluorophenyl)-3-(4'-phenylphenoxy)propyl])-sarcosine (NFPS) potentiates NMDA receptor activity and NMDA receptor-dependent long-term potentiation in rat.

Molecular cloning has further revealed the existence of three variants of GlyT1, termed GlyT-1a, GlyT-1b and GlyT-1c, each of which displays a unique distribution in the brain and peripheral tissues. The variants arise by differential splicing and exon usage, and differ in their N-terminal regions.

The physiological effects of GlyT1 in forebrain regions together with clinical reports showing the beneficial effects of GlyT1 inhibitor sarcosine in improving symptoms in schizophrenia patients suggest that selective GlyT1 inhibitors represent a new class of antipsychotic drugs.

Glycine transporter inhibitors are already known in the art, for example:

| | |
|---|---|
| 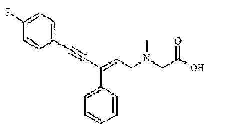 | US 200626364 |
| 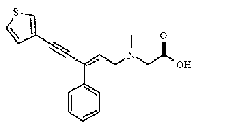 | US2002169197 |
| 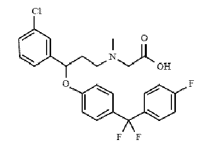 | EP 1 284 257 |
| 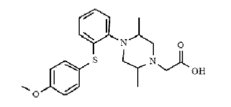 | WO 2003053942 |
| 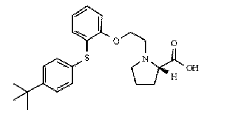 | WO 2004096761 |
| 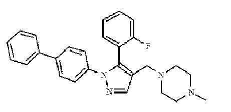 | WO 2003031435 |
| 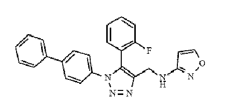 | DE 10315570 |
| 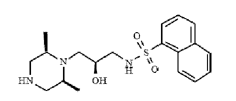 | WO 2003055478 |
| 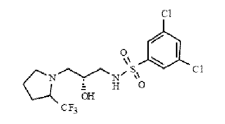 | WO 2004113280 |
| 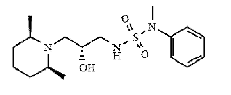 | WO 2004112787 |
| 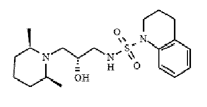 | WO 2004113301 |
| 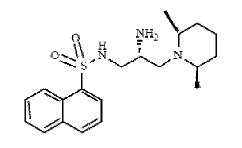 | WO 2005049023 |
| 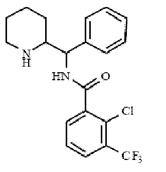 | WO 2003089411 |
| 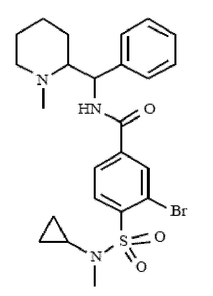 | WO 2004013100 |
| 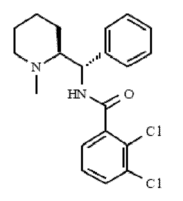 | WO 2004013101 |
| 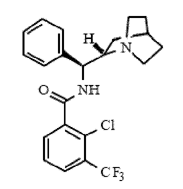 | WO 2005037783 |
| 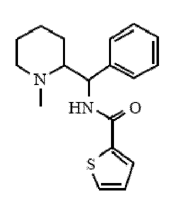 | WO 2005037792 |
| 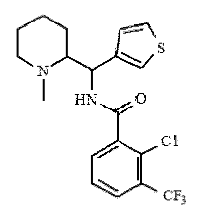 | WO 2005037781 |
| 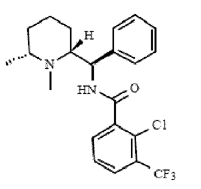 | WO 2005037782 |
| 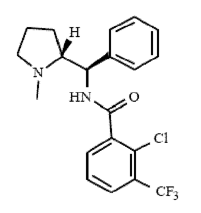 | WO 2005037785 |
| 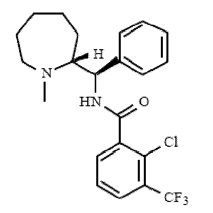 | WO 2005037785 |
| 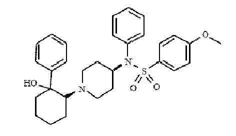 | WO 2004072034 |
| 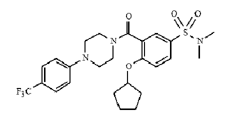 | WO 2005014563 |
| 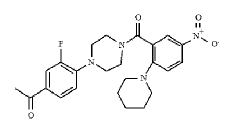 | WO 2005023260 |
| 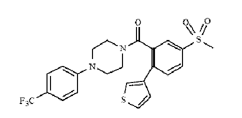 | WO 2005023261 |
| 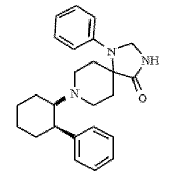 | WO 2005040166 |
| 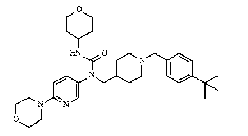 | WO 2005058882 |
| 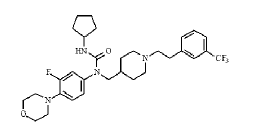 | WO 2005058885 |
| 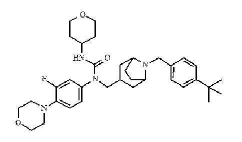 | WO 2005058317 |
| 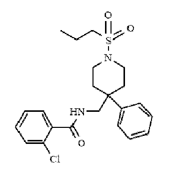 | WO 2005046601 |
| 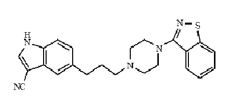 | WO 2003087086 |
| 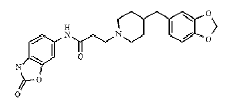 | WO2003076420 |
| 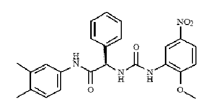 | WO2004022528 |

(see also Hashimoto K., Recent Patents on CNS Drug Discovery, 2006, 1, 43-53; Harsing L.G. et al., Current Medicinal Chemistry, 2006, 13, 1017-1044; Javitt D.C., Molecular Psychiatry (2004) 9, 984-997; Lindsley, C.W. et al., Current Topics in Medicinal Chemistry, 2006, 6, 771-785; Lindsley C.W. et al., Current Topics in Medicinal Chemistry, 2006, 6, 1883-1896).

Further glycine transporter inhibitors are known from the following documents.

WO 2009024611 describes 4-benzylaminoquinolines of formula:

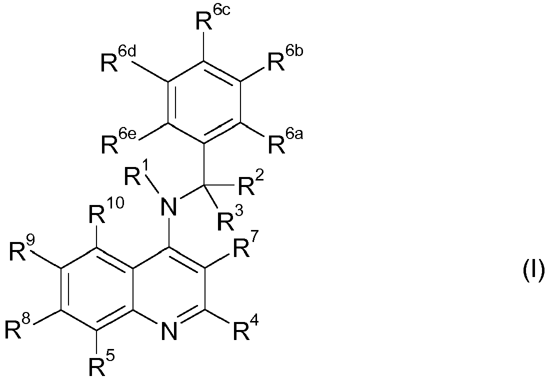

WO 2009121872 describes tetrahydroisoquinolines of formula:

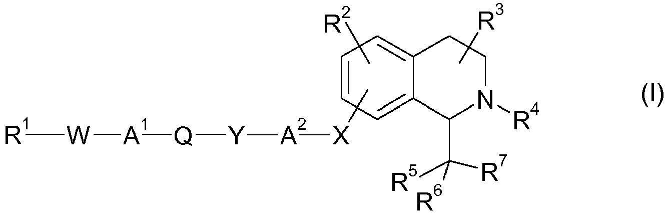

WO 2010092180 describes aminotetraline derivatives of formula:

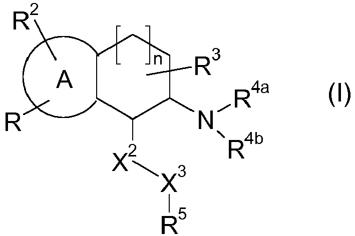

WO 2010092181 describes heterocyclic compounds of formula:

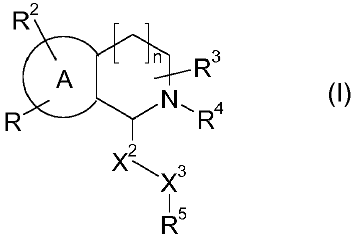

WO 2012020131 describes aminoindane derivatives of formula:

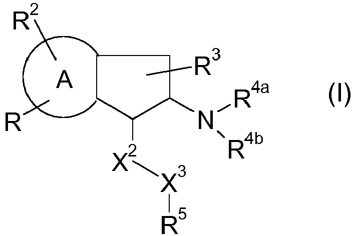

WO 2012020130 describes phenalkylamine derivatives of formula:

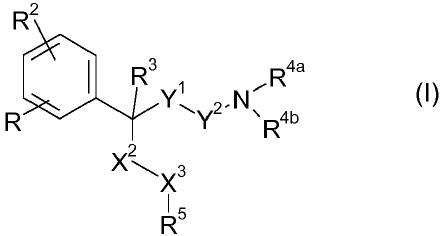

WO 2012020133 describes tetraline and indane derivatives of formula:

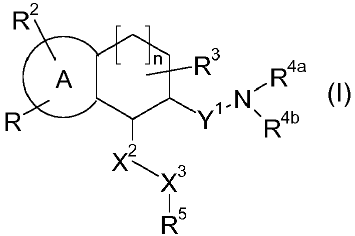

WO 2012152915 describes benzazepine derivatives of formula:

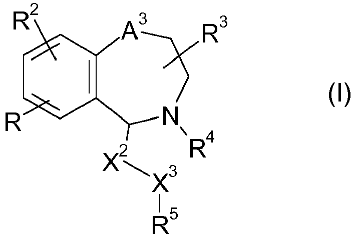

WO 2012020134 describes phenalkylamine derivatives of formulae:

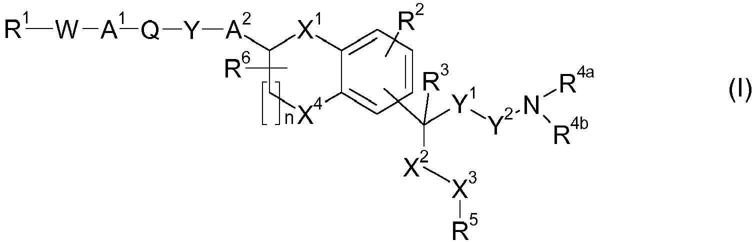

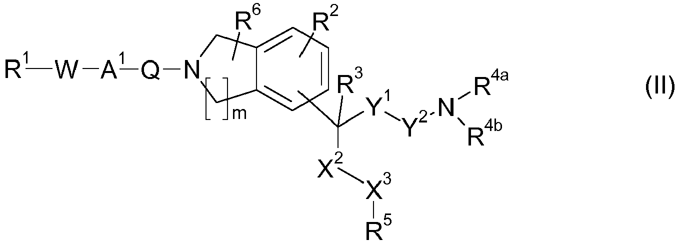

WO 2013020930 describes aminochromane, aminothiochromane and amino-1,2,3,4-tetrahydroquinoline derivatives of formula:

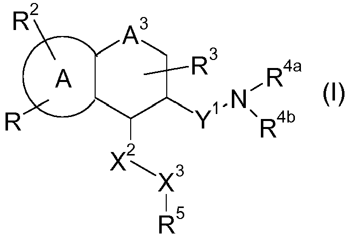

WO 2013072520 describes N-substituted aminobenzocycloheptene, aminotetraline, aminoindane and phenalkylamine derivatives of formula:

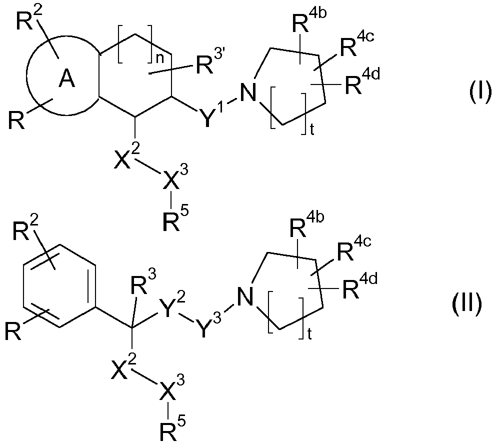

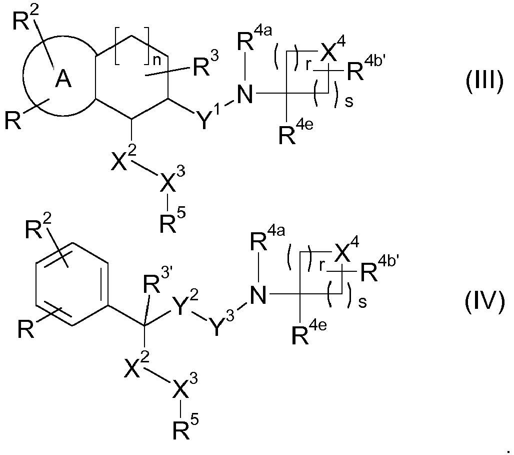

WO 2013120835 describes isoindoline derivatives of formula

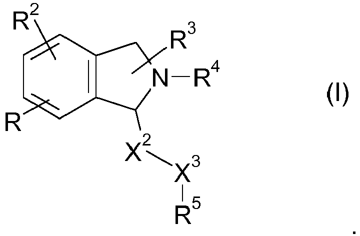

It was one object of the present invention to provide further glycine transporter inhibitors. It was a further object of the present invention to provide glycine transporter inhibitors which combine high stability with high affinity. It was a further object of the present invention to provide glycine transporter inhibitors which show favorable efflux properties. It was a further object of the present invention to provide glycine transporter inhibitors which combine high stability and high affinity with favorable efflux properties. It was a further object of the present invention to provide glycine transporter inhibitors which show good oral bioavailability.

### Summary of the Invention

The present invention relates to pyrrolidine derivatives of the formula (I) 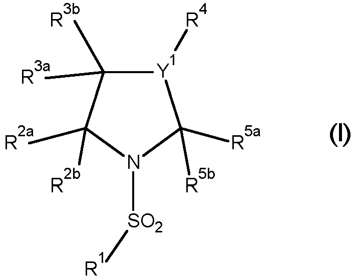 wherein
- R¹: is a 5-membered heterocyclic ring selected from pyrazolyl, imidazolyl and triazolyl, which is optionally substituted with 1, 2 or 3 substituents independently selected from the group consisting of halogen, C₁-C₆-alkyl, halogenated C₁-C₆-alkyl, C₃-C₆-cycloalkyl, C₁-C₄-alkoxycarbonyl and C₁-C₆-alkylcarbonylamino;
- R^{2a}, R^{2b}: are independently hydrogen, halogen, or C₁-C₃-alkyl, or
- R^{2a}, R^{2b}: together with the carbon atom to which they are bound may form a C=O;
- R^{3a}: is C₃-C₁₂-cycloalkyl, optionally substituted C₆-C₁₂-aryl, hydroxy, C₁-C₆-alkoxy, halogenated C₁-C₆-alkoxy, C₃-C₁₂-cycloalkyl-C₁-C₄-aₗkoxy, C₂-C₆-alkenyloxy, C₆-C₁₂-aryl-C₁-C₄-alkoxy, C₃-C₁₂-heterocyclyl-C₁-C₄-alkoxy, optionally substituted C₆-C₁₂-aryloxy, C₃-C₁₂-heterocyclyloxy, or optionally substituted C₃-C₁₂-heterocyclyl; or
- R^{3b}: is hydrogen, C₁-C₆-alkyl, or hydroxyl; or
- R^{3a} and R^{3b}: together are optionally substituted C₂-C₅-alkylene;
- Y¹: is >CR⁶- or >N-;
- R⁶: is hydrogen, C₁-C₆-alkyl, halogenated C₁-C₆-alkyl, (optionally substituted C₆-C₁₂-aryl)-C₁-C₄-alkyl, hydroxy-C₁-C₆-alkyl, C₁-C₄-alkoxy-C₁-C₆-alkyl, or hydroxyl; or
- R⁶ and R^{3a} or R^{3b}: together are optionally substituted C₁-C₅-alkylene; or
- R⁶: is C₁-C₄-alkylene that is bound to a carbon atom in R^{3a}, and R^{3a} is an optionally substituted C₆-C₁₂-aryl or an optionally substituted C₃-C₁₂-heterocyclyl;
- R⁴: is -NR^{8a}(CR^{9a}R^{9b})ₙ₄R¹³, -(CR^{7a}R^{7b})ₙ₁OR¹⁰, -(CR^{7c}R^{7d})ₙ₂NR^{11a}R^{11b}, -(CR^{7e}R^{7f})ₙ₃R¹², optionally substituted C₆-C₁₂-aryl, -NR^{8b}COR¹⁴, -NR^{8c}COOR¹⁵, -NR^{8d}CONR^{16a}R^{16b}, -NR^{8e}SO₂R¹⁷,-O(CR^{9c}R^{9d})ₙ₅R¹⁸, -COR¹⁹, -CONR^{20a}R^{20b}, -SO₂R²¹, or optionally substituted C₃-C₁₂-heterocyclyl;
- R^{7a}, R^{7b}: are independently hydrogen or C₁-C₆-alkyl,
- n1: is 1, 2, 3, or 4;
- R¹⁰: is hydrogen, optionally substituted C₆-C₁₂-aryl, or optionally substituted C₃-C₁₂-heterocyclyl;
- R^{7c}, R^{7d}: are independently hydrogen or C₁-C₆-alkyl,
- n2: is 1, 2, 3, or 4;
- R^{11a}: is C₁-C₈-alkyl, (optionally substituted C₃-C₁₂-cycloalkyl)-C₁-C₄-alkyl, C₁-C₆-alkoxy-C₁-C₆-alkyl, (optionally substituted C₆-C₁₂-aryl)-C₁-C₄-alkyl, (optionally substituted C₃-C₁₂-heterocyclyl)-C₁-C₄-alkyl, optionally substituted C₆-C₁₂-aryl, or optionally substituted C₃-C₁₂-heterocyclyl;
- R^{11b}: is hydrogen or C₁-C₆-alkyl;
- R^{7e}, R^{7f}: are independently hydrogen or C₁-C₆-alkyl,
- n3: is 1, 2, 3, or 4;
- R¹²: is optionally substituted C₆-C₁₂-aryl or optionally substituted C₃-C₁₂-heterocyclyl;
- R^{8a}, R^{8b}, R^{8c}, R^{8d}, R^{8e}: are independently hydrogen, C₁-C₆-alkyl, or C₁-C₆-alkylcarbonyl, or
- R⁶ and one of R^{8a}, R^{8b}, R^{8c}, R^{8d}, or R^{8e}: together are optionally substituted C₁-C₅-alkylene, wherein one or more -CH₂- of C₁-C₅-alkylene may be independently replaced by a an oxygen atom or C=O; or
- R^{3a} and one of R^{8a} or R^{8b}: together are optionally substituted C₁-C₅-alkylene;
- R^{9a}, R^{9b}: are independently hydrogen, halogen, C₁-C₆-alkyl, halogenated C₁-C₆-alkyl, hydroxyl, or C₁-C₆-alkoxy;
- n4: is 0, 1, 2, 3, or 4;
- R¹³: is hydrogen, C₁-C₈-alkyl, halogenated C₁-C₆-alkyl, (optionally substituted C₃-C₁₂-cycloalkyl)-C₁-C₄-alkyl, C₁-C₆-alkoxy-C₁-C₆-alkyl, optionally substituted C₃-C₁₂-cycloalkyl, C₂-C₆-alkenyl, optionally substituted C₃-C₆-cycloalkenyl, optionally substituted C₆-C₁₂-aryl, hydroxy, C₁-C₆-alkoxy, C₁-C₆-alkoxy-C₁-C₄-alkoxy, optionally substituted C₆-C₁₂-aryloxy, optionally substituted C₃-C₁₂-heterocyclyloxy, optionally substituted C₃-C₁₂-heterocyclyl, or tri-(C₁-C₄-alkyl)-silyloxy;
- R¹⁴: is C₁-C₈-alkyl, halogenated C₁-C₆-alkyl, (optionally substituted C₃-C₁₂-cycloalkyl)-C₁-C₄-alkyl, (optionally substituted C₆-C₁₂-aryl)-C₁-C₄-alkyl, hydroxy-C₁-C₆-alkyl, C₁-C₆-alkoxy-C₁-C₆-alkyl, (optionally substituted C₆-C₁₂-aryloxy)-C₁-C₄-alkyl, C₁-C₆-alkylcarbonyl-C₁-C₄-alkyl, C₁-C₆-alkoxycarbonyl-C₁-C₄-alkyl, C₁-C₆-alkylaminocarbonyl-C₁-C₄-alkyl, optionally substituted (C₃-C₁₂-heterocyclyl)-C₁-C₄-alkyl, optionally substituted C₃-C₁₂-cycloalkyl, optionally substituted C₆-C₁₂-aryl, or optionally substituted C₃-C₁₂-heterocyclyl;
- R¹⁵: is C₁-C₈-alkyl, optionally substituted C₆-C₁₂-aryl, or optionally substituted C₃-C₁₂-heterocyclyl;
- R^{16a}: is (optionally substituted C₆-C₁₂-aryl)-C₁-C₄-alkyl, optionally substituted (C₃-C₁₂-heterocyclyl)-C₁-C₄-alkyl, optionally substituted C₆-C₁₂-aryl, or optionally substituted C₃-C₁₂-heterocyclyl;
- R^{16b}: is hydrogen or C₁-C₆-alkyl;
- R¹⁷: is (optionally substituted C₆-C₁₂-aryl)-C₁-C₄-alkyl, (optionally substituted C₃-C₁₂-heterocyclyl)-C₁-C₄-alkyl, optionally substituted C₆-C₁₂-aryl, or optionally substituted C₃-C₁₂-heterocyclyl;
- R^{9c}, R^{9d}: are independently hydrogen, halogen, or C1-C6-alkyl;
- n5: is 0, 1, 2, 3, or 4;
- R¹⁸: is hydrogen, C₁-C₈-alkyl, optionally substituted C₃-C₁₂-cycloalkyl, C₁-C₆₋alkylcarbonyl, C₁-C₆-alkoxycarbonyl, halogenated C₁-C₆₋alkoxycarbonyl, C₆-C₁₂₋aryloxycarbonyl, C₁-C₆-alkylaminocarbonyl, (halogenated C₁-C₄-alkyl)aminocarbonyl, C₆-C₁₂₋arylaminocarbonyl, optionally substituted C₆-C₁₂-aryl, C₁-C₆-alkylamine, (C₃-C₁₂-cycloalkyl-C₁-C₄-alkyl)amino, (halogenated C₁-C₆-alkyl)amino, (C₁-C₆-alkoxy-C₁-C₆-alkyl)amino, (C₆-C₁₂-aryl-C₁-C₄-alkyl)amino, C₁-C₆-dialkylamine, optionally substituted C₆-C₁₂-arylamine, or optionally substituted C₃-C₁₂-heterocyclyl;
- R¹⁹: is optionally substituted C₆-C₁₂-aryl or optionally substituted C₃-C₁₂-heterocyclyl;
- R^{20a}: is C₁-C₈-alkyl, (optionally substituted C₃-C₁₂-cycloalkyl)-C₁-C₄-alkyl, C₁-C₆-alkoxy-C₁-C₆-alkyl, (optionally substituted C₆-C₁₂-aryl)-C₁-C₄-alkyl, (optionally substituted C₃-C₁₂-heterocyclyl)-C₁-C₄-alkyl, optionally substituted C₆-C₁₂-aryl, or optionally substituted C₃-C₁₂-heterocyclyl;
- R^{20b}: is hydrogen or C₁-C₈-alkyl;
- R²¹: is optionally substituted C₆-C₁₂-aryl, or optionally substituted C₃-C₁₂-heterocyclyl; and
- R^{5a}, R^{5b}: are independently hydrogen, halogen, or C1-C3-alkyl, or
- R^{5a}, R^{5b}: together with the carbon atom to which they are bound may form a C=O; or
- one of R^{2a} or R^{2b} and one of R^{5a} or R^{5b}: together are optionally substituted C1-C5-alkylene,
or a physiologically tolerated salt thereof.

According to claim 1 which is directed to the compounds per se, the following compounds and the physiologically tolerated salts thereof are excluded:
compounds of formula (II) and the physiologically tolerated salts thereof: 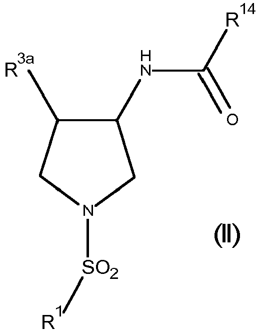 wherein R¹, R^{3a} and R¹⁴ are as defined in the following table

| | | |
|---|---|---|
| R¹ | R^{3a} | R¹⁴ |
| 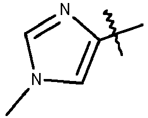 | 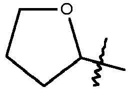 | 4-F-Ph |
| 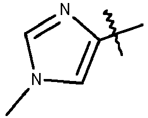 | 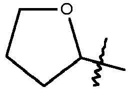 | 4-CF₃-Ph |
| 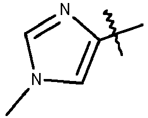 | 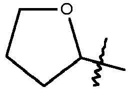 | 2-OMe-Ph |
| 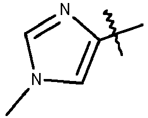 | 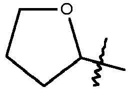 | 2-OMe-4-OCF₃-Ph |
| 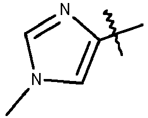 | 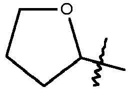 | 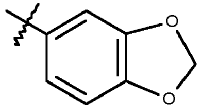 |
| 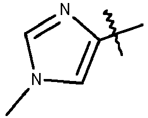 | 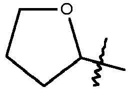 | 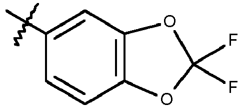 |
| 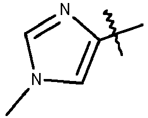 | 4-F-phenyl | 4-CF₃-phenyl |
| 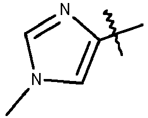 | 4-F-phenyl | 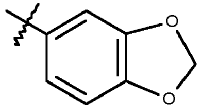 |

compounds of formula (II') 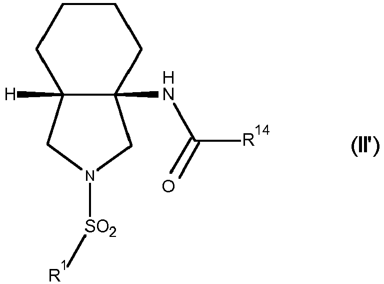 wherein R¹ and R¹⁴ are as defined in the following table

| R¹ | R¹⁴ |
|---|---|
| 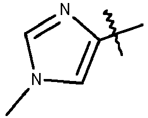 | 4-F-Ph |
| 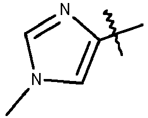 | 2-OMe-Ph |
| 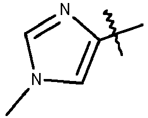 | 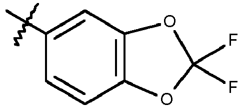 |

and the compounds of the formulae:

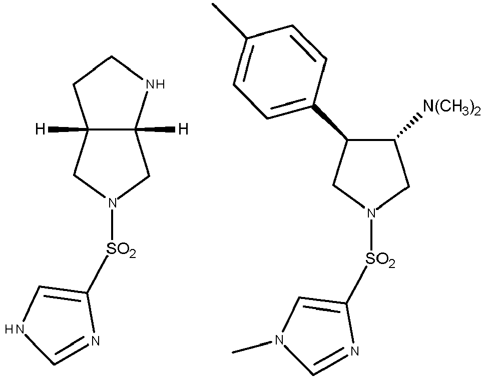

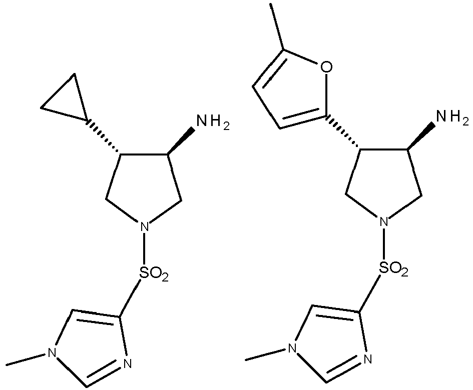

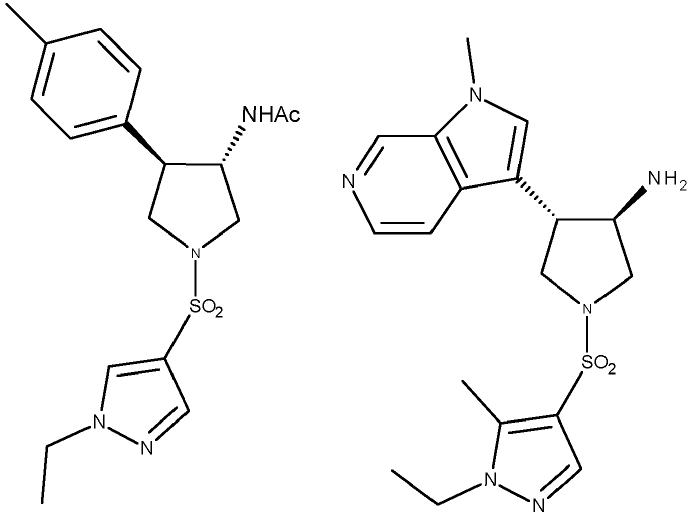

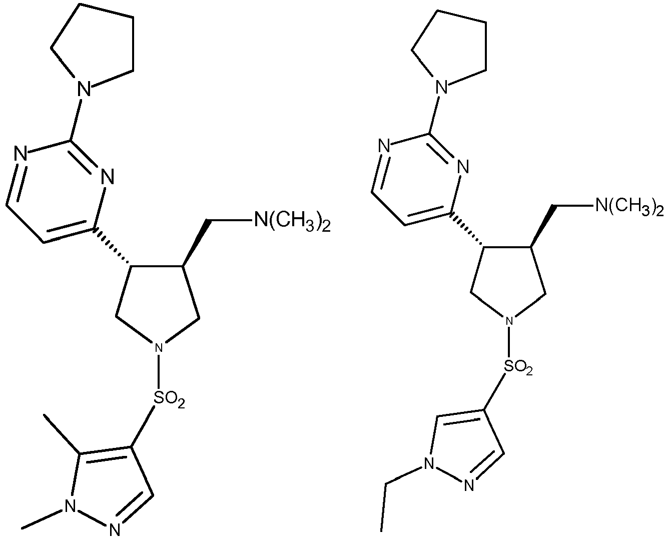

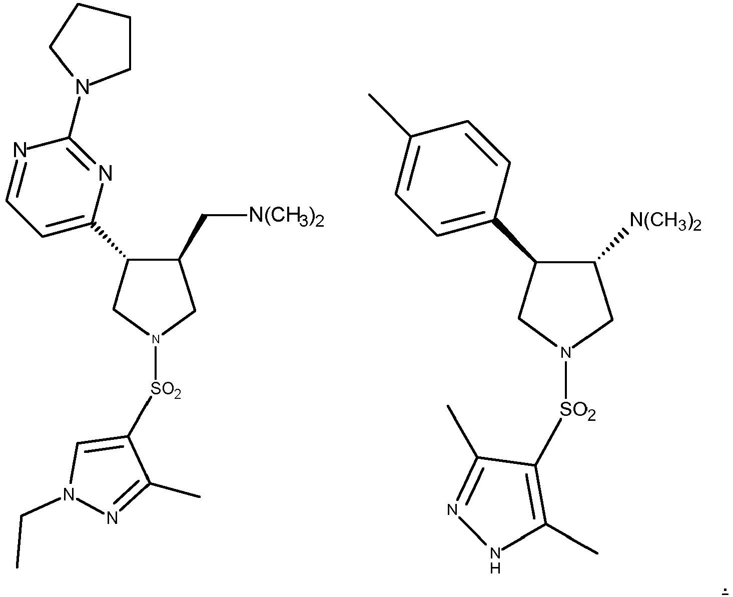

These compounds are, however, not excluded from the pharmaceutical compositions of the invention and from the medical use of the compounds of the invention.

Said compounds of formula (I), i.e., the pyrrolidine derivatives of formula (I) and their physiologically tolerated salts, are glycine transporter inhibitors and thus useful as pharmaceuticals. Compounds of formula (I) combine high metabolic stability with high affinity. Compounds of formula (I) show favorable efflux properties which may lead to enhanced oral bioavailability and/or increased brain availability. Compounds of formula (I) combine high metabolic stability and high affinity with favorable efflux properties.

The present invention thus further relates to the compounds of formula (I) for use in therapy.

The present invention also relates to pharmaceutical compositions which comprise a carrier and a compound of formula (I).

In particular, said compounds, i.e., the pyrrolidine derivatives and their physiologically tolerated salts, are inhibitors of the glycine transporter GlyT1.

The present invention thus further relates to the compounds of formula (I) for use in inhibiting the glycine transporter.

The present invention also relates to the use of the compounds of formula (I) in the manufacture of a medicament for inhibiting the glycine transporter GlyT1 and corresponding methods of inhibiting the glycine transporter GlyT1.

Glycine transport inhibitors and in particular inhibitors of the glycine transporter GlyT1 are known to be useful in treating a variety of neurologic and psychiatric disorders.

The present invention thus further relates to the compounds of formula (I) for use in treating a neurologic or psychiatric disorder.

The present invention further relates to the compounds of formula (I) for use in treating pain.

The present invention also relates to the use of the compounds of formula (I) in the manufacture of a medicament for treating a neurologic or psychiatric disorder and corresponding methods of treating said disorders. The present invention also relates to the use of the compounds of formula (I) in the manufacture of a medicament for treating pain and corresponding methods of treating pain.

### Detailed description of the Invention

Provided that the pyrrolidine derivatives of the formula (I) of a given constitution may exist in different spatial arrangements, for example if they possess one or more centers of asymmetry, polysubstituted rings or double bonds, or as different tautomers, the invention relates to the corresponding enantiomeric mixtures, in particular racemates, diastereomeric mixtures and tautomeric mixtures, as well as to the respective essentially pure enantiomers, diastereomers and tautomers of the compounds of formula (I) and/or of their salts.

According to one embodiment, an enantiomer of the pyrrolidine derivatives of the present invention has the following formula: 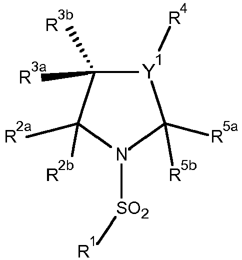 wherein R¹, R^{2a}, R^{2b}, R^{3a}, R^{3b}, R⁴, Y¹, R^{5a}, R^{5b} are as defined herein.

According to another embodiment, an enantiomer of the pyrrolidine derivatives of the present invention has the following formula: 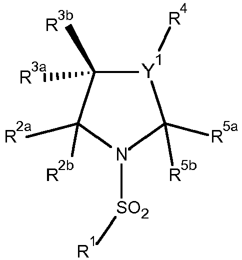 wherein R¹, R^{2a}, R^{2b}, R^{3a}, R^{3b}, R⁴, Y¹, R^{5a}, R^{5b} are as defined herein.

If Y¹ is >CR⁶- it is preferred that R^{3a} and R⁴ are in *trans* position.

Accordingly, the invention relates in particular to an enantiomer of the pyrrolidine derivatives having the following formula: 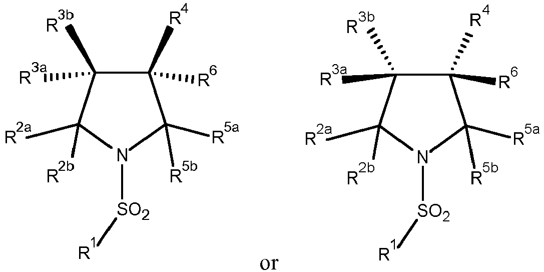 wherein R¹, R^{2a}, R^{2b}, R^{3a}, R^{3b}, R⁴, R⁶, R^{5a}, R^{5b} are as defined herein.

Preferably, the invention relates to an enantiomer of the pyrrolidine derivatives having the following formula: 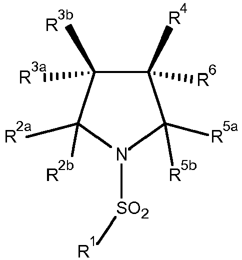 wherein R¹, R^{2a}, R^{2b}, R^{3a}, R^{3b}, R⁴, R⁶, R^{5a}, R^{5b} are as defined herein.

The physiologically tolerated salts of the pyrrolidine derivatives of the formula (I) are especially acid addition salts with physiologically tolerated acids. Examples of suitable physiologically tolerated organic and inorganic acids are hydrochloric acid, hydrobromic acid, phosphoric acid, sulfuric acid, C₁-C₄-alkylsulfonic acids, such as methanesulfonic acid, cycloaliphatic sulfonic acids, such as S-(+)-10-camphor sulfonic acid, aromatic sulfonic acids, such as benzenesulfonic acid and toluenesulfonic acid, di- and tricarboxylic acids and hydroxycarboxylic acids having 2 to 10 carbon atoms, such as oxalic acid, malonic acid, maleic acid, fumaric acid, lactic acid, tartaric acid, citric acid, glycolic acid, adipic acid and benzoic acid. Other utilizable acids are described, e.g., in Fortschritte der Arzneimittelforschung [Advances in drug research], Volume 10, pages 224 ff., Birkhäuser Verlag, Basel and Stuttgart, 1966. The physiologically tolerated salts of the isoindoline derivatives also include salts of a physiologically tolerated anion with an isoindoline derivatives wherein one or more than one nitrogen atom is quaternized, e.g. with an alkyl residue (e.g. methyl or ethyl).

The present invention moreover relates to compounds of formula (I) as defined herein, wherein at least one of the atoms has been replaced by its stable, non-radioactive isotope (e.g., hydrogen by deuterium, ¹²C by ¹³C, ¹⁴N by ¹⁵N, ¹⁶O by ¹⁸O) and preferably wherein at least one hydrogen atom has been replaced by a deuterium atom.

Of course, such compounds contain more of the respective isotope than this naturally occurs and thus is anyway present in the compounds (I).

Stable isotopes (e.g., deuterium, ¹³C, ¹⁵N, ¹⁸O) are nonradioactive isotopes which contain one or more additional neutron than the normally abundant isotope of the respective atom. Deuterated compounds have been used in pharmaceutical research to investigate the in vivo metabolic fate of the compounds by evaluation of the mechanism of action and metabolic pathway of the non-deuterated parent compound (Blake et al. J. Pharm. Sci. 64, 3, 367-391 (1975)). Such metabolic studies are important in the design of safe, effective therapeutic drugs, either because the in vivo active compound administered to the patient or because the metabolites produced from the parent compound prove to be toxic or carcinogenic (Foster et al., Advances in Drug Research Vol. 14, pp. 2-36, Academic press, London, 1985; Kato et al., J. Labelled Comp. Radiopharmaceut., 36(10):927-932 (1995); Kushner et al., Can. J. Physiol. Pharmacol., 77, 79-88 (1999).

Incorporation of a heavy atom particularly substitution of deuterium for hydrogen, can give rise to an isotope effect that could alter the pharmacokinetics of the drug. This effect is usually insignificant if the label is placed at a metabolically inert position of the molecule.

Stable isotope labeling of a drug can alter its physico-chemical properties such as pKa and lipid solubility. These changes may influence the fate of the drug at different steps along its passage through the body. Absorption, distribution, metabolism or excretion can be changed. Absorption and distribution are processes that depend primarily on the molecular size and the lipophilicity of the substance. These effects and alterations can affect the pharmacodynamic response of the drug molecule if the isotopic substitution affects a region involved in a ligand-receptor interaction.

Drug metabolism can give rise to large isotopic effect if the breaking of a chemical bond to a deuterium atom is the rate limiting step in the process. While some of the physical properties of a stable isotope-labeled molecule are different from those of the unlabeled one, the chemical and biological properties are the same, with one important exception: because of the increased mass of the heavy isotope, any bond involving the heavy isotope and another atom will be stronger than the same bond between the light isotope and that atom. In any reaction in which the breaking of this bond is the rate limiting step, the reaction will proceed slower for the molecule with the heavy isotope due to "kinetic isotope effect". A reaction involving breaking a C--D bond can be up to 700 percent slower than a similar reaction involving breaking a C--H bond. If the C--D bond is not involved in any of the steps leading to the metabolite, there may not be any effect to alter the behavior of the drug. If a deuterium is placed at a site involved in the metabolism of a drug, an isotope effect will be observed only if breaking of the C--D bond is the rate limiting step. There is evidence to suggest that whenever cleavage of an aliphatic C--H bond occurs, usually by oxidation catalyzed by a mixed-function oxidase, replacement of the hydrogen by deuterium will lead to observable isotope effect. It is also important to understand that the incorporation of deuterium at the site of metabolism slows its rate to the point where another metabolite produced by attack at a carbon atom not substituted by deuterium becomes the major pathway a process called "metabolic switching".

Deuterium tracers, such as deuterium-labeled drugs and doses, in some cases repeatedly, of thousands of milligrams of deuterated water, are also used in healthy humans of all ages, including neonates and pregnant women, without reported incident (e.g. Pons G and Rey E, Pediatrics 1999 104: 633; Coward W A et al., Lancet 1979 7: 13; Schwarcz H P, Control. Clin. Trials 1984 5(4 Suppl): 573; Rodewald L E et al., J. Pediatr. 1989 114: 885; Butte N F et al. Br. J. Nutr. 1991 65: 3; MacLennan A H et al. Am. J. Obstet Gynecol. 1981 139: 948). Thus, it is clear that any deuterium released, for instance, during the metabolism of compounds of this invention poses no health risk.

The weight percentage of hydrogen in a mammal (approximately 9%) and natural abundance of deuterium (approximately 0.015%) indicates that a 70 kg human normally contains nearly a gram of deuterium. Furthermore, replacement of up to about 15% of normal hydrogen with deuterium has been effected and maintained for a period of days to weeks in mammals, including rodents and dogs, with minimal observed adverse effects (Czajka D M and Finkel A J, Ann. N.Y. Acad. Sci. 1960 84: 770; Thomson J F, Ann. New York Acad. Sci 1960 84: 736; Czakja D M et al., Am. J. Physiol. 1961 201: 357). Higher deuterium concentrations, usually in excess of 20%, can be toxic in animals. However, acute replacement of as high as 15%-23% of the hydrogen in humans' fluids with deuterium was found not to cause toxicity (Blagojevic N et al. in "Dosimetry & Treatment Planning for Neutron Capture Therapy", Zamenhof R, Solares G and Harling O Eds. 1994. Advanced Medical Publishing, Madison Wis. pp.125-134; Diabetes Metab. 23: 251 (1997)).

Increasing the amount of deuterium present in a compound above its natural abundance is called enrichment or deuterium-enrichment. Examples of the amount of enrichment include from about 0.5, 1, 2, 3, 4, 5, 6, 7, 8, 9, 10, 12, 16, 21, 25, 29, 33, 37, 42, 46, 50, 54, 58, 63, 67, 71, 75, 79, 84, 88, 92, 96, to about 100 mol %.

The hydrogens present on a particular organic compound have different capacities for exchange with deuterium. Certain hydrogen atoms are easily exchangeable under physiological conditions and, if replaced by deuterium atoms, it is expected that they will readily exchange for protons after administration to a patient. Certain hydrogen atoms may be exchanged for deuterium atoms by the action of a deuteric acid such as D₂SO₄/D₂O. Alternatively, deuterium atoms may be incorporated in various combinations during the synthesis of compounds of the invention. Certain hydrogen atoms are not easily exchangeable for deuterium atoms. However, deuterium atoms at the remaining positions may be incorporated by the use of deuterated starting materials or intermediates during the construction of compounds of the invention.

Deuterated and deuterium-enriched compounds of the invention can be prepared by using known methods described in the literature. Such methods can be carried out utilizing corresponding deuterated and optionally, other isotope-containing reagents and/or intermediates to synthesize the compounds delineated herein, or invoking standard synthetic protocols known in the art for introducing isotopic atoms to a chemical structure. Relevant procedures and intermediates are disclosed, for instance in Lizondo, J et al., Drugs Fut, 21(11), 1116 (1996); Brickner, S J et al., J Med Chem, 39(3), 673 (1996); Mallesham, B et al., Org Lett, 5(7), 963 (2003); PCT publications WO1997010223, WO2005099353, WO1995007271, WO2006008754; US Patent Nos. 7538189; 7534814; 7531685; 7528131; 7521421; 7514068; 7511013; and US Patent Application Publication Nos. 20090137457; 20090131485; 20090131363; 20090118238;20090111840;20090105338; 20090105307; 20090105147; 20090093422; 20090088416; 20090082471, the methods are hereby incorporated by reference.

The organic moieties mentioned in the above definitions of the variables are - like the term halogen - collective terms for individual listings of the individual group members. The prefix Cₙ-Cₘ indicates in each case the possible number of carbon atoms in the group.

Unless indicated otherwise, the term "substituted" means that a radical is substituted with 1, 2 or 3, especially 1, substituent which, according to a particular embodiment of the invention, are independently selected from the group consisting of halogen, C₁-C₄-alkyl, C₃-C₆-aryl-C₁-C₄-alkyl, halogenated-C₁-C₄-alkyl, hydroxy-C₁-C₄-alkyl, hydroxy-(halogenated C₁-C₄-alkyl), C₁-C₄-alkoxy-C₁-C₄-alkyl, amino-C₁-C₄-alkyl, C₃-C₁₂-heterocyclyl-C₁-C₄-alkyl, C₃-C₇-cycloalkyl, C₂-C₄-alkenyl, -CN, -CO₂H, C₁-C₄-alkoxycarbonyl, aminocarbonyl, C₁-C₄-alkylaminocarbonyl, (di-C₁-C₄-alkylamino)carbonyl, C₆-C₁₂-arylaminocarbonyl, C₃-C₁₂-heterocyclylaminocarbonyl, C₆-C₁₂-aryl, oxo (=O), OH, C₁-C₄-alkoxy, halogenated-C₁-C₄-alkoxy, C₃-C₇-cycloalkoxy, carboxy-C₁-C₄-alkoxy, C₆-C₁₂-aryl-C₁-C₄-alkoxy, C₆-C₁₂-aryloxy, C₃-C₁₂-heterocyclyl-C₁-C₄-alkoxy, SH, C₁-C₄-alkylthio, C₁-C₄-alkylsulfonyl, C₁-C₄-alkylaminosulfonyl, di-C₁-C₄-alkylaminosulfonyl, C₃-C₆-arylsulfonyl, aminosulfonyl, C₃-C₆-arylaminosulfonyl, C₃-C₁₂-heterocyclylaminosulfonyl, NH₂, C₁-C₄-alkylamino, di-C₁-C₄-alkylamino, C₆-C₁₂-aryl-C₁-C₄-alkylamino,C₁-C₄-alkylcarbonylamino, C₃-C₆-arylcarbonylamino, C₃-C₁₂-heterocyclylcarbonylamino, C₁-C₆-alkylsulfonylamino, C₃-C₆-arylsulfonylamino, C₃-C₁₂-heterocyclylsulfonylamino and C₃-C₁₂-heterocyclyl, wherein aryl and heterocyclyl may be unsubstituted or substituted with 1, 2 or 3 substituents selected from the group consisting of halogen, C₁-C₄-alkyl, C₁-C₄-haloalkyl, C₁-C₄-alkoxy and C₁-C₄-haloalkoxy. Additional substituents may be independently selected from the group consisting of C₁-C₄-alkylamino-C₁-C₄-alkyl, di-C₁-C₄-alkylamino-C₁-C₄-alkyl, C₁-C₄-alkyl-carbonyl, halogenated C₁-C₄-alkyl-carbonyl, C₃-C₁₂-cycloalykyl-C₁-C₄-alkoxy, C₁-C₄-alkoxy-C₁-C₄-alkoxy, C₁-C₄-alkylamino-C₁-C₄-alkoxy, di-C₁-C₄-alkylamino-C₁-C₄-alkoxy, and C₃-C₁₂-heterocycloxy.

The term halogen denotes in each case fluorine, bromine, chlorine or iodine, in particular fluorine or chlorine.

C₁-C₄-Alkyl is a straight-chain or branched alkyl group having from 1 to 4 carbon atoms. Examples of an alkyl group are methyl, C₂-C₄-alkyl such as ethyl, n-propyl, iso-propyl, n-butyl, 2-butyl, iso-butyl or tert-butyl. C₁-C₂-Alkyl is methyl or ethyl, C₁-C₃-alkyl is additionally n-propyl or iso-propyl.

C₁-C₆-Alkyl is a straight-chain or branched alkyl group having from 1 to 6 carbon atoms. Examples include methyl, C₂-C₄-alkyl as mentioned herein and also pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl.

C₁-C₈-Alkyl is a straight-chain or branched alkyl group having from 1 to 8 carbon atoms. Examples include methyl, C₂-C₄-alkyl as mentioned herein and also pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, heptyl, 1-methylhexyl, 2-methylhexyl, 3-methylhexyl, 4-methylhexyl, 5-methylhexyl, 1,2-dimethylpentyl, 1,2,3-trimethylbutyl 1-ethyl-2-methylbutyl, 1-methyl-2-ethylbutyl, octyl, 1-methyl-heptyl, 2-methylheptyl, 3-methyl-hepthyl, 4-methyl-heptyl, 5-methylheptyl,6-methylheptyl,1 -methyl-2-ethylpentyl, 1,1 -dimethylhexyl, 1,2-dimethylhexyl, 1,3-dimethylhexyl, 1,4-dimethylhexyl, 2,2-dimethylhexyl, 2,3-dimethylhexyl, 3,3-dimethylhexyl, 4,5-dimethylhexyl, 1,2,3-trimethylpentyl, 1,2-dimethyl-3-ethylbutyl, 1-ethyl-2-ethylbutyl and 1,3-dimethyl-2-ethylbutyl.

Halogenated C₁-C₆-alkyl is a straight-chain or branched alkyl group having 1 to 6 carbon atoms, preferably 1 to 3 carbon atoms, more preferably 1 or 2 carbon atoms, wherein at least one, e.g. 1, 2, 3, 4 or all of the hydrogen atoms are replaced by 1, 2, 3, 4 or a corresponding number of identical or different halogen atoms, such as in halogenomethyl, dihalogenomethyl, trihalogenomethyl, (R)-1-halogenoethyl, (S)-1-halogenoethyl, 2-halogenoethyl, 1,1-dihalogenoethyl, 2,2-dihalogenoethyl, 2,2,2-trihalogenoethyl, (R)-1-halogenopropyl, (S)-1-halogenopropyl, 2-halogenopropyl, 3-halogenopropyl, 1,1-dihalogenopropyl, 2,2-dihalogenopropyl, 3,3-dihalogenopropyl, 3,3,3-trihalogenopropyl, (R)-2-halogeno-1-methylethyl, (S)-2-halogeno-1-methylethyl, (R)-2,2-dihalogeno-1-methylethyl, (S)-2,2-dihalogeno-1-methylethyl, (R)-1,2-dihalogeno-1-methylethyl, (S)-1,2-dihalogeno-1-methylethyl, (R)-2,2,2-trihalogeno-1-methylethyl, (S)-2,2,2-trihalogeno-1-methylethyl, 2-halogeno-1-(halogenomethyl)ethyl, 1-(dihalogenomethyl)-2,2-dihalogenoethyl, (R)-1-halogenobutyl, (S)-1-halogenobutyl, 2-halogenobutyl, 3-halogenobutyl, 4-halogenobutyl, 1,1-dihalogenobutyl, 2,2-dihalogenobutyl, 3,3-dihalogenobutyl, 4,4-dihalogenobutyl, 4,4,4-trihalogenobutyl, 1,1-dihalogenopentyl, 4,4-dihalogenopentyl etc. Particular examples include the fluorinated C₁-C₄ alkyl groups as defined, such as trifluoromethyl.

C₃-C₁₂-Cycloalkyl- C₁-C₄-alkyl is a straight-chain or branched alkyl group having 1 to 4 carbon atoms, preferably 1 to 3 carbon atoms, more preferably 1 or 2 carbon atoms, in particular 1 or two carbon atoms, wherein one hydrogen atom is replaced by a cycloaliphatic radical having from 3 to 12 carbon atoms such as in cyclopropylmethyl, cyclobutylmethyl, cyclopentylmethyl and cyclohexylmethyl.

C₁-C₆-Alkylcarbonyl-C₁-C₄-alkyl is a straight-chain or branched alkyl group having 1 to 4 carbon atoms, preferably 1 to 3 carbon atoms, more preferably 1 or 2 carbon atoms, in particular 1 or 2 carbon atoms, wherein one hydrogen atom is replaced by a C₁-C₆-alkylcarbonyl group, in particular by a C₁-C₄-alkylcarbonyl group, such as in methylcarbonylmethyl, methylcarbonylethyl, methylcarbonylpropyl,ethylcarbonylmethyl, n-propylcarbonylmethyl, iso-propylcarbonylmethyl, n-butylcarbonylmethyl, 2-butylcarbonylmethyl or iso-butylcarbonylmethyl.

C₁-C₆-Alkoxycarbonyl-C₁-C₄-alkyl is a straight-chain or branched alkyl group having 1 to 4 carbon atoms, preferably 1 to 3 carbon atoms, more preferably 1 or 2 carbon atoms, in particular 1 or two carbon atoms, wherein one hydrogen atom is replaced by a C₁-C₆-alkoxycarbonyl group, in particular by a C₁-C₄-alkoxycarbonyl group, such as in methoxycarbonylmethyl, methoxycarbonylpropyl, ethoxycarbonylmethyl, n-propoxycarbonylmethyl, n-butoxycarbonylmethyl, 2-butoxycarbonylmethyl or iso-butoxycarbonylmethyl.

C₆-C₁₂-Aryl-C₁-C₄-alkyl is a straight-chain or branched alkyl group having 1 to 4 carbon atoms, preferably 1 to 3 carbon atoms, more preferably 1 or 2 carbon atoms, in particular 1 or two carbon atoms, wherein one hydrogen atom is replaced by C₆-C₁₂-aryl, such as in benzyl.

Hydroxy-C₁-C₆-alkyl is a straight-chain or branched alkyl group having 1 to 6 carbon atoms, preferably 1 to 3 carbon atoms, more preferably 1 or 2 carbon atoms, wherein one or two hydrogen atoms are replaced by one or two hydroxyl groups, such as in hydroxymethyl, (R)-1-hydroxyethyl, (S)-1-hydroxyethyl, 2-hydroxyethyl, (R)-1-hydroxypropyl, (S)-1-hydroxypropyl, 2-hydroxypropyl, 3-hydroxypropyl, (R)-2-hydroxy-1-methylethyl, (S)-2-hydroxy-1-methylethyl, 2-hydroxy-1-(hydroxymethyl)ethyl, (R)-1-hydroxybutyl, (S)-1-hydroxybutyl, 2-hydroxybutyl, 3-hydroxybutyl, 4-hydroxybutyl, (R)-1-hydroxypentyl, (S)-1-hydroxypentyl, 2-hydroxypentyl and 4-hydroxypentyl.

Hydroxy-(halogenated C₁-C₄-alkyl) is a straight-chain or branched alkyl group having 1 to 4 carbon atoms, preferably 1 to 3 carbon atoms, more preferably 1 or 2 carbon atoms, wherein at least two, e.g. 2, 3, 4 or all of the hydrogen atoms are replaced by a number of identical or different halogen atoms and by one or two hydroxyl groups, such as in hydroxyhalogenomethyl, hydroxydihalogenomethyl, (R)-1-hydroxy-1-halogenoethyl, (S)-1-hydroxy-1-halogenoethyl, (R) 2,2-dihalogeno-1-hydroxyethyl, (S) 2,2-dihalogeno-1-hydroxyethyl, (R) 2,2,2-trihalogeno-1-hydroxyethyl, (S) 2,2,2-trihalogeno-1-hydroxyethyl (R)-1-hydroxy-1-halogenopropyl, (S)-1-hydroxy-1-halogenopropyl, (R)-2-halogeno-2-hydroxypropyl, (S)-2-halogeno-2-hydroxypropyl, 3-halogeno-2-hydroxypropyl, 1,1-dihalogeno-1-hydroxypropyl, 2,2-dihalogeno-1-hydroxypropyl, 3,3,3-trihalogeno-1-hydroxypropyl, (R)-2-halogeno-1-methyl-1-hydroxyethyl, (S)-2-halogeno-1-methyl-1-hydroxyethyl, (R)-2,2-dihalogeno-1-methyl-1-hydroxyethyl, (S)-2,2-dihalogeno-1-methyl-1-hydroxyethyl, (R)-2,2,2-trihalogeno-1-methyl-1-hydroxyethyl, (S)-2,2,2-trihalogeno-1-methyl-1-hydroxyethyl, (R)-1-(halogenomethyl)-1-hydroxyethyl, (S)-1-(halogenomethyl)-1-hydroxyethyl, (R)-1-(dihalogenomethyl)-1-hydroxyethyl, (S)-1-(dihalogenomethyl)-1-hydroxyethyl, (R)-1-(trihalogenomethyl)-1-hydroxyethyl, (S)-1-(trihalogenomethyl)-1-hydroxyethyl, etc. Particular examples include the hydroxyfluorinated C₁-C₄ alkyl groups as defined, such as 1-(trifluoromethyl)-1-hydroxyethyl.

C₁-C₆-Alkoxy-C₁-C₆-alkyl is a straight-chain or branched alkyl group having 1 to 6 carbon atoms, preferably 1 to 4 carbon atoms, more preferably 1 or 2 carbon atoms, wherein one or two hydrogen atoms are replaced by one or two alkoxy groups having 1 to 6, preferably 1 to 4, in particular 1 or 2 carbon atoms, such as in methoxymethyl, (R)-1-methoxyethyl, (S)-1-methoxyethyl, 2-methoxyethyl, (R)-1-methoxypropyl, (S)-1-methoxypropyl, 2-methoxypropyl, 3-methoxypropyl, (R)-2-methoxy-1-methylethyl, (S)-2-methoxy-1-methylethyl, 2-methoxy-1 -(methoxymethyl)ethyl, (R)-1-methoxybutyl, (S)-1-methoxybutyl, 2-methoxybutyl, 3-methoxybutyl, 4-methoxybutyl, (R)-1-methoxypentyl, (S)-1-methoxypentyl, 2-methoxypentyl, 3-methoxypentyl, 4-methoxypentyl, (R)-1-methoxyhexyl, (S)-1-methoxyhexyl, 2-methoxyhexyl, 3-methoxyhexyl, 4-methoxyhexyl,ethoxymethyl, (R)-1-ethoxyethyl, (S)-1-ethoxyethyl, 2-ethoxyethyl, (R)-1-ethoxypropyl, (S)-1-ethoxypropyl, 2-ethoxypropyl, 3-ethoxypropyl, (R)-2-ethoxy-1-methylethyl, (S)-2-ethoxy-1-methylethyl, 2-ethoxy-1-(ethoxymethyl)ethyl, (R)-1-ethoxybutyl, (S)-1-ethoxybutyl, 2-ethoxybutyl, 3-ethoxybutyl, 4-ethoxybutyl, (R)-1-ethoxypentyl, (S)-1-ethoxypentyl, 2-ethoxypentyl, 3-ethoxypentyl, 4-ethoxypentyl, 5-ethoxypentyl, (R)-1-ethoxyhexyl, (S)-1-ethoxyhexyl, 2-ethoxyhexyl, 3-ethoxyhexyl and 6-ethoxybutyl.

C₆-C₁₂-Aryloxy-C₁-C₄-alkyl is a straight-chain or branched alkyl group having 1 to 4 carbon atoms, preferably 1 to 3 carbon atoms, more preferably 1 or 2 carbon atoms, in particular 1 or two carbon atoms, wherein one hydrogen atom is replaced by a radical of the formula R-O-, wherein R is an aryl group having from 6 to 12, in particular 6 carbon atoms as defined herein. Examples include phenoxymethyl, (4-F-phenoxy)methyl.
Amino-C₁-C₄-alkyl is a straight-chain or branched alkyl group having 1 to 4 carbon atoms, preferably 1 to 3 carbon atoms, more preferably 1 or 2 carbon atoms, in particular 1 or two carbon atoms, wherein one hydrogen atom is replaced by an amino group, such as in aminomethyl, 2-aminoethyl.

C₁-C₆-Alkylamino-C₁-C₄-alkyl is a straight-chain or branched alkyl group having 1 to 4 carbon atoms, preferably 1 to 3 carbon atoms, more preferably 1 or 2 carbon atoms, in particular 1 or two carbon atoms, wherein one hydrogen atom is replaced by a C₁-C₆-alkylamino group, in particular by a C₁-C₄-alkylamino group, such as in methylaminomethyl, ethylaminomethyl, n-propylaminomethyl, iso-propylaminomethyl, n-butylaminomethyl, 2-butylaminomethyl, iso-butylaminomethyl or tert-butylaminomethyl.

Di-C₁-C₆-Alkylamino-C₁-C₄-alkyl is a straight-chain or branched alkyl group having 1 to 4 carbon atoms, preferably 1 to 3 carbon atoms, more preferably 1 or 2 carbon atoms, in particular 1 or two carbon atoms, wherein one hydrogen atom is replaced by a di-C₁-C₆-Alkylamino group, in particular by a di-C₁-C₄-alkylamino group, such as in dimethylaminomethyl.

C₁-C₆-Alkylcarbonylamino-C₁-C₄-alkyl is a straight-chain or branched alkyl group having 1 to 4 carbon atoms, preferably 1 to 3 carbon atoms, more preferably 1 or 2 carbon atoms, in particular 1 or two carbon atoms, wherein one hydrogen atom is replaced by a C₁-C₆-alkylcarbonylamino group, in particular by a C₁-C₄-alkylcarbonylamino group, such as in methylcarbonylaminomethyl, ethylcarbonylaminomethyl, n-propylcarbonylaminomethyl, iso-propylcarbonylaminomethyl, n-butylcarbonylaminomethyl, 2-butylcarbonylaminomethyl, iso-butylcarbonylaminomethyl or tert-butylcarbonylaminomethyl.

C₁-C₆-Alkylaminocarbonylamino-C₁-C₄-alkyl is a straight-chain or branched alkyl group having 1 to 4 carbon atoms, preferably 1 to 3 carbon atoms, more preferably 1 or 2 carbon atoms, in particular 1 or two carbon atoms, wherein one hydrogen atom is replaced by a C₁-C₆-alkylaminocarbonylamino group, in particular by a C₁-C₄-alkylaminocarbonylamino group, such as in methylaminocarbonylaminomethyl, ethylaminocarbonylaminomethyl, n-propylaminocarbonylaminomethyl, iso-propylaminocarbonylaminomethyl, n-butylaminocarbonylaminomethyl, 2-butylaminocarbonylaminomethyl, iso-butylaminocarbonylaminomethyl or tert-butylaminocarbonylaminomethyl.

Di-C₁-C₆-alkylaminocarbonylamino-C₁-C₄-alkyl is a straight-chain or branched alkyl group having 1 to 4 carbon atoms, preferably 1 to 3 carbon atoms, more preferably 1 or 2 carbon atoms, in particular 1 or two carbon atoms, wherein one hydrogen atom is replaced by a di-C₁-C₆-alkylaminocarbonylamino group, in particular by a di-C₁-C₄-alkylaminocarbonylamino group, such as in dimethylaminocarbonylaminomethyl, dimethylaminocarbonylaminoethyl, dimethylaminocarbonylamino-propyl.

C₁-C₆-Alkylsulfonylamino-C₁-C₄-alkyl is a straight-chain or branched alkyl group having 1 to 4 carbon atoms, preferably 1 to 3 carbon atoms, more preferably 1 or 2 carbon atoms, in particular 1 or two carbon atoms, wherein one hydrogen atom is replaced by a C₁-C₆-alkylsulfonylamino group, in particular by a C₁-C₄-alkylsulfonylamino group, such as in methylsulfonylaminomethyl, ethylsulfonylaminomethyl, n-propylsulfonylaminomethyl, iso-propylsulfonylaminomethyl, n-butylsulfonylaminomethyl, 2-butylsulfonylaminomethyl, iso-butylsulfonylaminomethyl or tert-butylsulfonylaminomethyl.

(C₆-C₁₂-Aryl-C₁-C₆-alkyl)amino-C₁-C₄ alkyl is a straight-chain or branched alkyl group having 1 to 4 carbon atoms, preferably 1 to 3 carbon atoms, more preferably 1 or 2 carbon atoms, in particular 1 or two carbon atoms, wherein one hydrogen atom is replaced by a (C₆-C₁₂-aryl-C₁-C₆-alkyl)amino group, in particular a (C₆-C₁₂-aryl-C₁-C₂-alkyl)amino group, such as in benzylaminomethyl.

C₃-C₁₂-Heterocyclyl-C₁-C₄-alkyl is a straight-chain or branched alkyl group having 1 to 4 carbon atoms, preferably 1 to 3 carbon atoms, more preferably 1 or 2 carbon atoms, in particular 1 or two carbon atoms, wherein one hydrogen atom is replaced by C₃-C₁₂-heterocyclyl, such as in N-pyrrolidinylmethyl, N-piperidinylmethyl, N-morpholinylmethyl, tetrahydropyran-2-yl-methyl.

C₃-C₁₂-Cycloalkyl is a cycloaliphatic radical having from 3 to 12 carbon atoms. In particular, 3 to 6 carbon atoms form the cyclic structure, such as cyclopropyl, cyclobutyl, cyclopentyl and cyclohexyl. The cyclic structure may be unsubstituted or may carry 1, 2, 3 or 4 C₁-C₄ alkyl radicals, preferably one or more methyl radicals.

Carbonyl is >C=O.

C₁-C₆-Alkylcarbonyl is a radical of the formula R-C(O)-, wherein R is an alkyl radical having from 1 to 6, preferably from 1 to 4, in particular 1 or 2 carbon atoms as defined herein. Examples include acetyl, propionyl, n-butyryl, 2-methylpropionyl, pivaloyl.

Halogenated C₁-C₆-alkylcarbonyl is C₁-C₆-alkylcarbonyl as defined herein, wherein at least one, e.g. 1, 2, 3, 4 or all of the hydrogen atoms are replaced by 1, 2, 3, 4 or a corresponding number of identical or different halogen atoms. Examples include fluoromethylcarbonyl, difluoromethylcarbonyl, trifluoromethylcarbonyl. Further examples are 1,1,1-trifluoroeth-2-ylcarbonyl, 1,1,1-trifluoroprop-3-ylcarbonyl.

C₆-C₁₂-Arylcarbonyl is a radical of the formula R-C(O)-, wherein R is an aryl radical having from 6 to 12 carbon atoms as defined herein. Examples include benzoyl.

C₁-C₆-Alkoxycarbonyl is a radical of the formula R-O-C(O)-, wherein R is an alkyl radical having from 1 to 6, preferably from 1 to 4, in particular 1 or 2 carbon atoms as defined herein. Examples include methoxycarbonyl and tert-butoxycarbonyl.

Halogenated C₁-C₆-alkoxycarbonyl is a C₁-C₆-alkoxycarbonyl as defined herein, wherein at least one, e.g. 1, 2, 3, 4 or all of the hydrogen atoms are replaced by 1, 2, 3, 4 or a corresponding number of identical or different halogen atoms.

C₆-C₁₂-Aryloxycarbonyl is a radical of the formula R-O-C(O)-, wherein R is an aryl radical having from 6 to 12 carbon atoms as defined herein. Examples include phenoxycarbonyl.

Cyano is -C=N.

Aminocarbonyl is NH₂C(O)-.

C₁-C₆-Alkylaminocarbonyl is a radical of the formula R-NH-C(O)-, wherein R is an alkyl radical having from 1 to 6, preferably from 1 to 4, in particular 1 or 2 carbon atoms as defined herein. Examples include methylaminocarbonyl.

(Halogenated C₁-C₄-alkyl)aminocarbonyl is a C₁-C₄-alkylaminocarbonyl as defined herein, wherein at least one, e.g. 1, 2, 3, 4 or all of the hydrogen atoms are replaced by 1, 2, 3, 4 or a corresponding number of identical or different hydrogen atoms.

C₆-C₁₂-Arylaminocarbonyl is a radical of the formula R-NH-C(O)-, wherein R is an aryl radical having from 6 to 12 carbon atoms as defined herein. Examples include phenylaminocarbonyl.

C₂-C₆-Alkenyl is a singly unsaturated hydrocarbon radical having 2, 3, 4, 5 or 6 carbon atoms, e.g. vinyl, allyl (2-propen-1-yl), 1-propen-1-yl, 2-propen-2-yl, methallyl(2-methylprop-2-en-1-yl) and the like. C₃-C₅-Alkenyl is, in particular, allyl, 1-methylprop-2-en-1-yl, 2-buten-1-yl, 3-buten-1-yl, methallyl, 2-penten-1-yl, 3-penten-1-yl, 4-penten-1-yl, 1-methylbut-2-en-1-yl or 2-ethylprop-2-en-1-yl, 2-hexen-1-yl.

C₃-C₆-Cycloalkenyl is a carbocyclic radical having at least one carbon-carbon double bond and from 3 to 6 carbon atoms. In particular, 3 to 6 carbon atoms form the cyclic structure, such as 2-cyclopenten-1-yl, 2-cyclohexen-1-yl. The cyclic structure may be unsubstituted or may carry 1, 2, 3 or 4 C₁-C₄ alkyl radicals, preferably one or more methyl radicals.

C₂-C₆-Alkynyl is a singly unsaturated hydrocarbon radical having 2, 3, 4, 5 or 6 carbon atoms, e.g. ethynyl, 2-propyn-1-yl, 1-propyn-1-yl, 2-propyn-2-yl and the like. C₃-C₅-Alkynyl is, in particular, 2-propyn-1-yl, 2-butyn-1-yl, 3-butyn-1-yl, 2-pentyn-1-yl, 3-pentyn-1-yl, 4-pentyn-1-yl.

C₁-C₅-Alkylene is straight-chain or branched alkylene group having from 1 to 5 carbon atoms. Examples include methylene and ethylene. A further example is propylene. Another further example is butylene.

C₂-C₄-Alkenylene is straight-chain or branched alkenylene group having from 2 to 4 carbon atoms.

C₂-C₄-Alkynylene is straight-chain or branched alkynylene group having from 2 to 4 carbon atoms. Examples include propynylene.

C₆-C₁₂-Aryl is a 6- to 12-membered, in particular 6- to 10-membered, aromatic cyclic radical which can be a monocyclic aromatic ring, for example, phenyl etc., or a fused polycyclic aromatic ring comprising a first monocyclic aromatic ring and one or more carbocycles which are saturated, partially unsaturated or aromatic, for example, naphthyl, indenyl, tetrahydronaphthyl, indanyl.

C₃-C₁₂-Arylene is an aryl diradical. Examples include phen-1,4-ylene and phen-1,3-ylene.

Hydroxy is -OH.

Oxo is =O.

C₁-C₆-Alkoxy is a radical of the formula R-O-, wherein R is a straight-chain or branched alkyl group having from 1 to 6, in particular 1 to 4 carbon atoms. Examples include methoxy, ethoxy, n-propoxy, iso-propoxy, n-butoxy, 2-butoxy, iso-butoxy (2-methylpropoxy), tert.-butoxy pentyloxy, 1-methylbutoxy, 2-methylbutoxy, 3-methylbutoxy, 2,2-dimethylpropoxy, 1-ethylpropoxy, hexyloxy, 1,1-dimethylpropoxy, 1,2-dimethylpropoxy, 1-methylpentyloxy, 2-methylpentyloxy, 3-methylpentyloxy, 4-methylpentyloxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy and 1-ethyl-2-methylpropoxy.

Halogenated C₁-C₆-alkoxy is a straight-chain or branched alkoxy group having from 1 to 6, preferably from 1 to 4, in particular 1 or 2 carbon atoms, wherein at least one, e.g. 1, 2, 3, 4 or all of the hydrogen atoms are replaced by 1, 2, 3, 4 or a corresponding number of identical or different halogen atoms, such as in halogenomethoxy, dihalogenomethoxy, trihalogenomethoxy, (R)-1-halogenoethoxy, (S)-1-halogenoethoxy, 2-halogenoethoxy, 1,1-dihalogenoethoxy, 2,2-dihalogenoethoxy, 2,2,2-trihalogenoethoxy, (R)-1-halogenopropoxy, (S)-1-halogenopropoxy, 2-halogenopropoxy, 3-halogenopropoxy, 1,1-dihalogenopropoxy, 2,2-dihalogenopropoxy, 3,3-dihalogenopropoxy, 3,3,3-trihalogenopropoxy, (R)-2-halogeno-1-methylethoxy, (S)-2-halogeno-1-methylethoxy, (R)-2,2-dihalogeno-1-methylethoxy, (S)-2,2-dihalogeno-1-methylethoxy, (R)-1,2-dihalogeno-1-methylethoxy, (S)-1,2-dihalogeno-1-methylethoxy, (R)-2,2,2-trihalogeno-1-methylethoxy, (S)-2,2,2-trihalogeno-1-methylethoxy, 2-halogeno-1-(halogenomethyl)ethoxy, 1-(dihalogenomethyl)-2,2-dihalogenoethoxy, (R)-1-halogenobutoxy, (S)-1-halogenobutoxy, 2-halogenobutoxy, 3-halogenobutoxy, 4-halogenobutoxy, 1,1-dihalogenobutoxy, 2,2-dihalogenobutoxy, 3,3-dihalogenobutoxy, 4,4-dihalogenobutoxy, 4,4,4-trihalogenobutoxy, etc. Particular examples include the fluorinated C₁-C₄ alkoxy groups as defined, such as trifluoromethoxy.

C₁-C₆-Hydroxyalkoxy is an alkoxy radical having from 1 to 6, preferably from 1 to 4 carbon atoms as defined herein, wherein one or two hydrogen atoms are replaced by hydroxy. Examples include 2-hydroxyethoxy, 3-hydroxypropoxy, 2-hydroxypropoxy, 1-methyl-2-hydroxyethoxy and the like.

C₁-C₆-Alkoxy-C₁-C₄-alkoxy is an alkoxy radical having from 1 to 4 carbon atoms, preferably 1 or 2 carbon atoms as defined herein, wherein one or two hydrogen atoms are replaced by one or two alkoxy radicals having from 1 to 6, preferably from 1 to 4 carbon atoms as defined herein. Examples include methoxymethoxy, 2-methoxyethoxy, 1-methoxyethoxy, 3-methoxypropoxy, 2-methoxypropoxy, 1-methyl-1-methoxyethoxy, ethoxymethoxy, 2-ethoxyethoxy, 1-ethoxyethoxy, 3-ethoxypropoxy, 2-ethoxypropoxy, 1-methyl-1-ethoxyethoxy and the like.

Amino-C₁-C₄-alkoxy is an alkoxy radical having from 1 to 4, preferably 1 or 2 carbon atoms as defined herein, wherein one hydrogen atom is replaced by an amino group. Examples include 2-aminoethoxy.

C₁-C₆-Alkylamino-C₁-C₄-alkoxy is an alkoxy radical having from 1 to 4, preferably 1 or 2 carbon atoms as defined herein, wherein one hydrogen atom is replaced by an alkylamino group having from 1 to 6, preferably from 1 to 4 carbon atoms as defined herein. Examples include methylaminomethoxy, ethylaminomethoxy, n-propylaminomethoxy, iso-propylaminomethoxy, n-butylaminomethoxy, 2-butylaminomethoxy, iso-butylaminomethoxy, tert-butylaminomethoxy, 2-(methylamino)ethoxy, 2-(ethylamino)ethoxy, 2-(n-propylamino)ethoxy, 2-(iso-propylamino)-ethoxy, 2-(n-butylamino)ethoxy, 2-(2-butylamino)ethoxy, 2-(iso-butylamino)ethoxy, 2-(tert-butylamino) ethoxy.

Di-C₁-C₆-alkylamino-C₁-C₄-alkoxy is an alkoxy radical having from 1 to 4, preferably 1 or 2 carbon atoms as defined herein, wherein one hydrogen atom is replaced by a di-alkylamino group having from 1 to 6, preferably from 1 to 4 carbon atoms as defined herein. Examples include di-methylaminomethoxy, diethylaminomethoxy, N-methyl-N-ethylamino)ethoxy, 2-(dimethylamino)ethoxy, 2-(diethylamino)ethoxy, 2-(N-methyl-N-ethylamino)ethoxy.

C₁-C₆-Alkylcarbonylamino-C₁-C₄-alkoxy is an alkoxy radical having from 1 to 4, preferably 1 or 2 carbon atoms as defined herein, wherein one hydrogen atom is replaced by an alkylcarbonylamino group wherein the alkyl group has from 1 to 6, preferably from 1 to 4 carbon atoms as defined herein. Examples include methylcarbonylaminomethoxy, ethylcarbonylaminomethoxy, n-propylcarbonylaminomethoxy, iso-propylcarbonylaminomethoxy, n-butylcarbonylaminomethoxy, 2-butylcarbonylaminomethoxy, iso-butylcarbonylaminomethoxy, tert-butylcarbonylaminomethoxy, 2-(methylcarbonylamino)ethoxy, 2-(ethylcarbonylamino)ethoxy, 2-(n-propylcarbonylamino)ethoxy, 2-(iso-propylcarbonylamino)ethoxy, 2-(n-butylcarbonylamino)ethoxy, 2-(2-butylcarbonylamino)ethoxy, 2-(iso-butylcarbonylamino)ethoxy, 2-(tert-butylcarbonylamino)ethoxy.

C₆-C₁₂-Arylcarbonylamino-C₁-C₄-alkoxy is an alkoxy radical having from 1 to 4, preferably 1 or 2 carbon atoms as defined herein, wherein one hydrogen atom is replaced by a C₆-C₁₂-arylcarbonylamino group as defined herein. Examples include 2-(benzoylamino)ethoxy.

C₁-C₆-Alkoxycarbonylamino-C₁-C₄-alkoxy is an alkoxy radical having from 1 to 4, preferably 1 or 2 carbon atoms as defined herein, wherein one hydrogen atom is replaced by an alkoxycarbonylamino group wherein the alkoxy group has from 1 to 6, preferably from 1 to 4 carbon atoms as defined herein. Examples include methoxycarbonylaminomethoxy, ethoxycarbonylaminomethoxy, n-propoxycarbonylaminomethoxy, iso-propoxycarbonylaminomethoxy, n-butoxycarbonylaminomethoxy, 2-butoxycarbonylaminomethoxy, iso-butoxycarbonylaminomethoxy, tert-butoxycarbonylaminomethoxy, 2-(methoxycarbonylamino)ethoxy, 2-(ethoxycarbonylamino)ethoxy, 2-(n-propoxycarbonylamino)ethoxy, 2-(iso-propoxycarbonylamino)ethoxy, 2-(n-butoxycarbonylamino)ethoxy, 2-(2-butoxycarbonylamino)ethoxy, 2-(iso-butoxycarbonylamino)ethoxy, 2-(tert-butoxycarbonylamino)ethoxy.

C₂-C₆-Alkenyloxy is a radical of the formula R-O-, wherein R is a straight-chain or branched alkenyl group having from 2 to 6, in particular 2 to 4 carbon atoms. Examples include vinyloxy, allyloxy (2-propen-1-yloxy), 1-propen-1-yloxy, 2-propen-2-yloxy, methallyloxy (2-methylprop-2-en-1-yloxy) and the like. C₃-C₅-Alkenyloxy is, in particular, allyloxy, 1-methylprop-2-en-1-yloxy, 2-buten-1-yloxy, 3-buten-1-yloxy, methallyloxy, 2-penten-1-yloxy, 3-penten-1-yloxy, 4-penten-1-yloxy, 1-methylbut-2-en-1-yloxy or 2-ethylprop-2-en-1-yloxy.

C₆-C₁₂-Aryl-C₁-C₄-alkoxy is an alkoxy radical having from 1 to 4, preferably 1 or 2 carbon atoms as defined herein, wherein one hydrogen atom is replaced by a C₆-C₁₂-aryl group as defined herein. Examples include benzyloxy.

C₁-C₆-Alkylsulfonylamino-C₁-C₄-alkoxy is an alkoxy radical having from 1 to 4, preferably 1 or 2 carbon atoms as defined herein, wherein one hydrogen atom is replaced by an alkylsulfonylamino group having from 1 to 6, preferably from 1 to 4 carbon atoms as defined herein. Examples include 2-(methylsulfonylamino)ethoxy, 2-(ethylsulfonylamino)ethoxy, 2-[(2-methylpropyl)sulfonylamino] ethoxy.

(Halogenated C₁-C₆-alkyl)sulfonylamino-C₁-C₄-alkoxy is an alkoxy radical having from 1 to 4, preferably 1 or 2 carbon atoms as defined herein, wherein one hydrogen atom is replaced by an alkylsulfonylamino group having from 1 to 6, preferably from 1 to 4 carbon atoms as defined herein, wherein the alkyl group is halogenated. Examples include 2-(trifluoromethylsulfonylamino)ethoxy.

C₆-C₁₂-Arylsulfonylamino-C₁-C₄-alkoxy is an alkoxy radical having from 1 to 4, preferably 1 or 2 carbon atoms as defined herein, wherein one hydrogen atom is replaced by a C₆-C₁₂-arylsulfonylamino group as defined herein. Examples include 2-(phenylsulfonylamino)ethoxy, 2-(naphthylsulfonylamino)ethoxy.

(C₆-C₁₂-Aryl-C₁-C₆-alkyl)sulfonylamino-C₁-C₄-alkoxy is an alkoxy radical having from 1 to 4, preferably 1 or 2 carbon atoms as defined herein, wherein one hydrogen atom is replaced by a (C₆-C₁₂-aryl-C₁-C₆-alkyl)sulfonylamino group, preferably by a (C₆-C₁₂-aryl-C₁-C₂-alkyl)sulfonylamino group. Examples include 2-(benzylsulfonylamino)ethoxy.

C₃-C₁₂-Heterocyclylsulfonylamino-C₁-C₄-alkoxy is an alkoxy radical having from 1 to 4, preferably 1 or 2 carbon atoms as defined herein, wherein one hydrogen atom is replaced by a C₃-C₁₂-heterocyclylsulfonylamino group as defined herein. Examples include 2-(pyridin-3-yl-sulfonylamino) ethoxy.

C₃-C₁₂-Heterocyclyl-C₁-C₄-alkoxy is an alkoxy radical having from 1 to 4, preferably 1 or 2 carbon atoms as defined herein, wherein one hydrogen atom is replaced by a C₃-C₁₂-heterocyclyl group as defined herein. Examples include 2-(N-pyrrolidinyl)ethoxy, 2-(N-morpholinyl)ethoxy and 2-(N-imidazolyl)ethoxy.

C₁-C₂-Alkylenedioxo is a radical of the formula -O-R-O-, wherein R is a straight-chain or branched alkylene group having from 1 or 2 carbon atoms as defined herein. Examples include methylenedioxo.

C₆-C₁₂-Aryloxy is a radical of the formula R-O-, wherein R is an aryl group having from 6 to 12, in particular 6 carbon atoms as defined herein. Examples include phenoxy. C₃-C₁₂-Heterocyclyloxy is a radical of the formula R-O-, wherein R is a C₃-C₁₂-heterocyclyl group having from 3 to 12, in particular from 3 to 7 carbon atoms as defined herein. Examples include pyridin-2-yloxy.

C₁-C₆-Alkylthio is a radical of the formula R-S-, wherein R is an alkyl radical having from 1 to 6, preferably from 1 to 4 carbon atoms as defined herein. Examples include methylthio, ethylthio, propylthio, butylthio, pentylthio, 1-methylbutylthio, 2-methylbutylthio, 3-methylbutylthio, 2,2-dimethylpropylthio, 1-ethylpropylthio, hexylthio, 1,1-dimethylpropylthio, 1,2-dimethylpropylthio, 1-methylpentylthio, 2-methylpentylthio, 3-methylpentylthio, 4-methylpentylthio, 1,1-dimethylbutylthio, 1,2-dimethylbutylthio, 1,3-dimethylbutylthio, 2,2-dimethylbutylthio, 2,3-dimethylbutylthio, 3,3-dimethylbutylthio, 1-ethylbutylthio, 2-ethylbutylthio, 1,1,2-trimethylpropylthio, 1,2,2-trimethylpropylthio, 1-ethyl-1-methylpropyl and 1-ethyl-2-methylpropyl.

Halogenated C₁-C₆-alkylthio is a radical of the formula R-S-, wherein R is a halogenated alkyl radical having from 1 to 6, preferably from 1 to 4 carbon atoms as defined herein. Examples include halogenomethylthio, dihalogenomethylthio, trihalogenomethylthio, (R)-1-halogenoethylthio, (S)-1-halogenoethylthio, 2-halogenoethylthio, 1,1-dihalogenoethylthio, 2,2-dihalogenoethylthio, 2,2,2-trihalogenoethylthio, (R)-1-halogenopropylthio, (S)-1-halogenopropylthio, 2-halogenopropylthio, 3-halogenopropylthio, 1,1-dihalogenopropylthio, 2,2-dihalogenopropylthio, 3,3-dihalogenopropylthio, 3,3,3-trihalogenopropylthio, (R)-2-halogeno-1-methylethylthio, (S)-2-halogeno-1-methylethylthio, (R)-2,2-dihalogeno-1-methylethylthio, (S)-2,2-dihalogeno-1-methylethylthio, (R)-1,2-dihalogeno-1-methylethylthio, (S)-1,2-dihalogeno-1-methylethylthio, (R)-2,2,2-trihalogeno-1-methylethylthio, (S)-2,2,2-trihalogeno-1 -methylethylthio, 2-halogeno-1-(halogenomethyl)ethylthio, 1-(dihalogenomethyl)-2,2-dihalogenoethylthio, (R)-1-halogenobutylthio, (S)-1-halogenobutylthio, 2-halogenobutylthio, 3-halogenobutylthio, 4-halogenobutylthio, 1,1-dihalogenobutylthio, 2,2-dihalogenobutylthio, 3,3-dihalogenobutylthio, 4,4-dihalogenobutylthio, 4,4,4-trihalogenobutylthio, etc. Particular examples include the fluorinated C₁-C₄ alkylthio groups as defined, such as trifluoromethylthio.

C₁-C₆-Alkylsulfinyl is a radical of the formula R-S(O)-, wherein R is an alkyl radical having from 1 to 6, preferably from 1 to 4 carbon atoms as defined herein. Examples include methylsulfinyl, ethylsulfinyl, propylsulfinyl, butylsulfinyl, pentylsulfinyl, 1-methylbutylsulfinyl, 2-methylbutylsulfinyl, 3-methylbutylsulfinyl, 2,2-dimethylpropylsulfinyl, 1-ethylpropylsulfinyl, hexylsulfinyl, 1,1-dimethylpropylsulfinyl, 1,2-dimethylpropylsulfinyl, 1-methylpentylsulfinyl, 2-methylpentylsulfinyl, 3-methylpentylsulfinyl, 4-methylpentylsulfinyl, 1,1-dimethylbutylsulfinyl, 1,2-dimethylbutylsulfinyl, 1,3-dimethylbutylsulfinyl, 2,2-dimethylbutylsulfinyl, 2,3-dimethylbutylsulfinyl, 3,3-dimethylbutylsulfinyl, 1-ethylbutylsulfinyl, 2-ethylbutylsulfinyl, 1,1,2-trimethylpropylsulfinyl, 1,2,2-trimethylpropylsulfinyl, 1-ethyl-1-methylpropyl and 1-ethyl-2-methylpropyl.

C₁-C₆-Alkylsulfonyl is a radical of the formula R-S(O)₂-, wherein R is an alkyl radical having from 1 to 6, preferably from 1 to 4 carbon atoms as defined herein. Examples include methylsulfonyl, ethylsulfonyl, propylsulfonyl, butylsulfonyl, pentylsulfonyl, 1-methylbutylsulfonyl, 2-methylbutylsulfonyl, 3-methylbutylsulfonyl, 2,2-dimethylpropylsulfonyl, 1-ethylpropylsulfonyl, hexylsulfonyl, 1,1-dimethylpropylsulfonyl, 1,2-dimethylpropylsulfonyl, 1-methylpentylsulfonyl, 2-methylpentylsulfonyl, 3-methylpentylsulfonyl, 4-methylpentylsulfonyl, 1,1-dimethylbutylsulfonyl, 1,2-dimethylbutylsulfonyl, 1,3-dimethylbutylsulfonyl, 2,2-dimethylbutylsulfonyl, 2,3-dimethylbutylsulfonyl, 3,3-dimethylbutylsulfonyl, 1-ethylbutylsulfonyl, 2-ethylbutylsulfonyl, 1,1,2-trimethylpropylsulfonyl, 1,2,2-trimethylpropylsulfonyl, 1-ethyl-1-methylpropyl and 1-ethyl-2-methylpropyl.

(Halogenated C₁-C₆-alkyl)sulfonyl is a C₁-C₆-alkylsulfonyl as defined herein, wherein at least one, e.g. 1, 2, 3, 4 or all of the hydrogen atoms are replaced by 1, 2, 3, 4 or a corresponding number of identical or different halogen atoms.

C₆-C₁₂-Arylsulfonyl is a radical of the formula R-S(O)₂-, wherein R is an aryl radical having from 6 to 12 carbon atoms as defined herein. Examples include phenylsulfonyl.

(C₆-C₁₂-Aryl-C₁-C₄-alkyl)sulfonyl is a radical of the formula R-S(O)₂-, wherein R is a C₆-C₁₂-aryl-C₁-C₄-alkyl radical, in particular a C₆-C₁₂-aryl-C₁-C₂-alkyl radical as defined herein. Examples include benzylsulfonyl.

C₃-C₁₂-Heterocyclylsulfonyl is a radical of the formula R-S(O)₂-, wherein R is C₃-C₁₂-heterocyclyl as defined herein.

Aminosulfonyl is NH₂-S(O)₂-.

C₁-C₆-Alkylaminosulfonyl is a radical of the formula R-NH-S(O)₂- wherein R is an alkyl radical having from 1 to 6, preferably from 1 to 4 carbon atoms as defined herein. Examples include methylaminosulfonyl, ethylaminosulfonyl, n-propylaminosulfonyl, iso-propylaminosulfonyl, n-butylaminosulfonyl, 2-butylaminosulfonyl, iso-butylaminosulfonyl, tert-butylaminosulfonyl. Di-C₁-C₆-alkylaminosulfonyl is a radical of the formula RR'N-S(O)₂- wherein R and R' are independently of each other an alkyl radical having from 1 to 6, preferably from 1 to 4 carbon atoms as defined herein. Examples include dimethylaminosulfonyl, diethylaminosulfonyl, N-methyl-N-ethylaminosulfonyl.

C₆-C₁₂-Arylaminosulfonyl is a radical of the formula R-NH-S(O)₂- wherein R is an aryl radical having from 6 to 12, preferably 6 carbon atoms as defined herein.

Amino is NH₂.

C₁-C₆-Alkylamino is a radical of the formula R-NH- wherein R is an alkyl radical having from 1 to 6, in particular from 1 to 4 carbon atoms as defined herein. Examples include methylamino, ethylamino, n-propylamino, iso-propylamino, n-butylamino, 2-butylamino, iso-butylamino, tert-butylamino.

(Halogenated C₁-C₆-alkyl)amino is a C₁-C₆-alkylamino as defined herein, wherein at least one, e.g. 1, 2, 3, 4 or all of the hydrogen atoms are replaced by 1, 2, 3, 4 or a corresponding number of identical or different halogen atoms.

Di-C₁-C₆-alkylamino is a radical of the formula RR'N- wherein R and R' are independently of each other an alkyl radical having from 1 to 6, in particular from 1 to 4 carbon atoms as defined herein. Examples include dimethylamino, diethylamino, N-methyl-N-ethylamino.

Di-(halogenated C₁-C₆-alkyl)amino is a di-C₁-C₆-alkylamino as defined herein, wherein at least one, e.g. 1, 2, 3, 4 or all of the hydrogen atoms are replaced by 1, 2, 3, 4 or a corresponding number of identical or different halogen atoms.

C₁-C₆-Alkylcarbonylamino is a radical of the formula R-C(O)-NH-, wherein R is an alkyl radical having from 1 to 6, in particular from 1 to 4 carbon atoms as defined herein. Examples include acetamido (methylcarbonylamino), propionamido, n-butyramido, 2-methylpropionamido (iso-propylcarbonylamino), 2,2-dimethylpropionamido and the like.

(Halogenated C₁-C₆-alkyl)carbonylamino is a C₁-C₆-alkylcarbonylamino as defined herein, wherein at least one, e.g. 1, 2, 3, 4 or all of the hydrogen atoms are replaced by 1, 2, 3, 4 or a corresponding number of identical or different halogen atoms.

C₆-C₁₂-Arylcarbonylamino is a radical of the formula R-C(O)-NH-, wherein R is an aryl radical having from 6 to 12 carbon atoms as defined herein. Examples include phenylcarbonylamino.

C₂-C₆-Alkenylamino is a radical of the formula R-NH-, wherein R is a straight-chain or branched alkenyl group having from 2 to 6, in particular 2 to 4 carbon atoms. Examples include vinylamino, allylamino (2-propen-1-ylamino), 1-propen-1-ylamino, 2-propen-2-ylamino, methallylamino (2-methylprop-2-en-1-ylamino) and the like. C₃-C₅-Alkenylamino is, in particular, allylamino, 1-methylprop-2-en-1-ylamino, 2-buten-1-ylamino, 3-buten-1-ylamino, methallylamino, 2-penten-1-ylamino, 3-penten-1-ylamino, 4-penten-1-ylamino, 1-methylbut-2-en-1-ylamino or 2-ethylprop-2-en-1-ylamino.

C₆-C₁₂-Arylamino is a radical of the formula R-NH-, wherein R is an aryl group having from 6 to 12, in particular 6 carbon atoms as defined herein. Examples include phenylamine.

C₁-C₆-Alkylsulfonylamino is a radical of the formula R-S(O)₂-NH-, wherein R is an alkyl radical having from 1 to 6, in particular from 1 to 4 carbon atoms as defined herein. Examples include methylsulfonylamino, ethylsulfonylamino, n-propylsulfonylamino, iso-propylsulfonylamino, n-butylsulfonylamino, 2-butylsulfonylamino, iso-butylsulfonylamino, tert-butylsulfonylamino.

(Halogenated C₁-C₆ alkyl)sulfonylamino is a C₁-C₆-alkylsulfonylamino as defined herein, wherein at least one, e.g. 1, 2, 3, 4 or all of the hydrogen atoms are replaced by 1, 2, 3, 4 or a corresponding number of identical or different halogen atoms.

C₆-C₁₂-Arylsulfonylamino is a radical of the formula R-S(O)₂-NH-, wherein R is an aryl radical having from 6 to 12 carbon atoms as defined herein. Examples include phenylsulfonylamino.

Nitro is -NO₂.

C₃-C₁₂-Heterocyclyl is a 3- to 12-membered heterocyclic radical including a saturated heterocyclic radical, which generally has 3, 4, 5, 6,or 7 ring forming atoms (ring members), an unsaturated nonaromatic heterocyclic radical, which generally has 5, 6 or 7 ring forming atoms, and a heteroaromatic radical (heteroaryl), which generally has 5, 6 or 7 ring forming atoms. Thus, the term C₃-C₁₂-heterocyclyl is meant to denote 3- to 12-membered heterocyclic radicals M₃-M₁₂-heterocyclyl, wherein the prefix Mₙ-Mₘ indicates in each case the possible number of ring forming atoms (ring members) in the group. The heterocyclic radicals may be bound via a carbon atom (C-bound) or a nitrogen atom (N-bound). Preferred heterocyclic radicals comprise 1 nitrogen atom as ring member atom and optionally 1, 2 or 3 further heteroatoms as ring members, which are selected, independently of each other from O, S and N. Likewise preferred heterocyclic radicals comprise 1 heteroatom as ring member, which is selected from O, S and N, and optionally 1, 2 or 3 further nitrogen atoms as ring members.

Examples of C₃-C₁₂-heterocyclyl include:
C- or N-bound 3-4-membered, saturated rings, such as 2-oxiranyl, 2-oxetanyl, 3-oxetanyl, 2-aziridinyl, 3-thiethanyl, 1-azetidinyl, 2-azetidinyl, 3-azetidinyl;
C-bound, 5-membered, saturated rings, such as tetrahydrofuran-2-yl, tetrahydrofuran-3-yl, tetrahydrothien-2-yl, tetrahydrothien-3-yl, tetrahydro-pyrrol-2-yl, tetrahydropyrrol-3-yl, tetrahydropyrazol-3-yl, tetrahydro-pyrazol-4-yl, tetrahydroisoxazol-3-yl, tetrahydroisoxazol-4-yl, tetrahydroisoxazol-5-yl, 1,2-oxathiolan-3-yl, 1,2-oxathiolan-4-yl, 1,2-oxathiolan-5-yl, tetrahydroisothiazol-3-yl, tetrahydroisothiazol-4-yl, tetrahydroisothiazol-5-yl, 1,2-dithiolan-3-yl, 1,2-dithiolan-4-yl, tetrahydroimidazol-2-yl, tetrahydroimidazol-4-yl, tetrahydrooxazol-2-yl, tetrahydrooxazol-4-yl, tetrahydrooxazol-5-yl, tetrahydrothiazol-2-yl, tetrahydrothiazol-4-yl, tetrahydrothiazol-5-yl, 1,3-dioxolan-2-yl, 1,3-dioxolan-4-yl, 1,3-oxathiolan-2-yl, 1,3-oxathiolan-4-yl, 1,3-oxathiolan-5-yl, 1,3-dithiolan-2-yl, 1,3-dithiolan-4-yl, 1,3,2-dioxathiolan-4-yl;
C-bound, 6-membered, saturated rings, such as tetrahydropyran-2-yl, tetrahydropyran-3-yl, tetrahydropyran-4-yl, piperidin-2-yl, piperidin-3-yl, piperidin-4-yl, tetrahydrothiopyran-2-yl, tetrahydrothiopyran-3-yl, tetrahydrothiopyran-4-yl, 1,3-dioxan-2-yl, 1,3-dioxan-4-yl, 1,3-dioxan-5-yl, 1,4-dioxan-2-yl, 1,3-dithian-2-yl, 1,3-dithian-4-yl, 1,3-dithian-5-yl, 1,4-dithian-2-yl, 1,3-oxathian-2-yl, 1,3-oxathian-4-yl, 1,3-oxathian-5-yl, 1,3-oxathian-6-yl, 1,4-oxathian-2-yl, 1,4-oxathian-3-yl, 1,2-dithian-3-yl, 1,2-dithian-4-yl, hexahydropyrimidin-2-yl, hexahydropyrimidin-4-yl, hexahydropyrimidin-5-yl, hexahydropyrazin-2-yl, hexahydropyridazin-3-yl, hexahydropyridazin-4-yl, tetrahydro-1,3-oxazin-2-yl, tetrahydro-1,3-oxazin-4-yl, tetrahydro-1,3-oxazin-5-yl, tetrahydro-1,3-oxazin-6-yl, tetrahydro-1,3-thiazin-2-yl, tetrahydro-1,3-thiazin-4-yl, tetrahydro-1,3-thiazin-5-yl, tetrahydro-1,3-thiazin-6-yl, tetrahydro-1,4-thiazin-2-yl, tetrahydro-1,4-thiazin-3-yl, tetrahydro-1,4-oxazin-2-yl, tetrahydro-1,4-oxazin-3-yl, tetrahydro-1,2-oxazin-3-yl, tetrahydro-1,2-oxazin-4-yl, tetrahydro-1,2-oxazin-5-yl, tetrahydro-1,2-oxazin-6-yl;
N-bound, 5-membered, saturated rings, such as tetrahydropyrrol-1-yl (pyrrolidin-1-yl), tetrahydropyrazol-1-yl, tetrahydroisoxazol-2-yl, tetrahydroisothiazol-2-yl, tetrahydroimidazol-1-yl, tetrahydrooxazol-3-yl, tetrahydrothiazol-3-yl; N-bound, 6-membered, saturated rings, such as piperidin-1-yl, hexahydropyrimidin-1-yl, hexahydropyrazin-1-yl (piperazin-1-yl), hexahydropyridazin-1-yl, tetrahydro-1,3-oxazin-3-yl, tetrahydro-1,3-thiazin-3-yl, tetrahydro-1,4-thiazin-4-yl, tetrahydro-1,4-oxazin-4-yl (morpholin-1-yl), tetrahydro-1,2-oxazin-2-yl;
C-bound, 5-membered, partially unsaturated rings, such as 2,3-dihydrofuran-2-yl, 2,3-dihydrofuran-3-yl, 2,5-dihydrofuran-2-yl, 2,5-di-hydrofuran-3-yl, 4,5-dihydrofuran-2-yl, 4,5-dihydrofuran-3-yl, 2,3-dihydro-thien-2-yl, 2,3-dihydrothien-3-yl, 2,5-dihydrothien-2-yl, 2,5-dihydrothien-3-yl, 4,5-dihydrothien-2-yl, 4,5-dihydrothien-3-yl, 2,3-dihydro-1H-pyrrol-2-yl, 2,3-dihydro-1H-pyrrol-3-yl, 2,5-dihydro-1H-pyrrol-2-yl, 2,5-dihydro-1H-pyrrol-3-yl, 4,5-dihydro-1H-pyrrol-2-yl, 4,5-dihydro-1H-pyrrol-3-yl, 3,4-dihydro-2H-pyrrol-2-yl, 3,4-dihydro-2H-pyrrol-3-yl, 3,4-dihydro-5H-pyrrol-2-yl, 3,4-dihydro-5H-pyrrol-3-yl, 4,5-dihydro-1H-pyrazol-3-yl, 4,5-dihydro-1H-pyrazol-4-yl, 4,5-dihydro-1H-pyrazol-5-yl, 2,5-dihydro-1H-pyrazol-3-yl, 2,5-dihydro-1H-pyrazol-4-yl, 2,5-dihydro-1H-pyrazol-5-yl, 4,5-dihydroisoxazol-3-yl, 4,5-dihydroisoxazol-4-yl, 4,5-dihydroisoxazol-5-yl, 2,5-dihydroisoxazol-3-yl, 2,5-dihydroisoxazol-4-yl, 2,5-dihydroisoxazol-5-yl, 2,3-dihydroisoxazol-3-yl, 2,3-dihydroisoxazol-4-yl, 2,3-dihydroisoxazol-5-yl, 4,5-dihydroisothiazol-3-yl, 4,5-dihydroisothiazol-4-yl, 4,5-dihydroisothiazol-5-yl, 2,5-dihydroisothiazol-3-yl, 2,5-dihydroisothiazol-4-yl, 2,5-dihydroisothiazol-5-yl, 2,3-dihydroisothiazol-3-yl, 2,3-dihydroisothiazol-4-yl, 2,3-dihydroisothiazol-5-yl, 4,5-dihydro-1H-imidazol-2-yl, 4,5-dihydro-1H-imidazol-4-yl, 4,5-dihydro-1H-imidazol-5-yl, 2,5-dihydro-1H-imidazol-2-yl, 2,5-dihydro-1H-imidazol-4-yl, 2,5-dihydro-1H-imidazol-5-yl, 2,3-dihydro-1H-imidazol-2-yl, 2,3-dihydro-1H-imidazol-4-yl, 4,5-dihydro-oxazol-2-yl, 4,5-dihydrooxazol-4-yl, 4,5-dihydrooxazol-5-yl, 2,5-dihydrooxazol-2-yl, 2,5-dihydrooxazol-4-yl, 2,5-dihydrooxazol-5-yl, 2,3-dihydrooxazol-2-yl, 2,3-dihydrooxazol-4-yl, 2,3-dihydrooxazol-5-yl, 4,5-dihydrothiazol-2-yl, 4,5-dihydrothiazol-4-yl, 4,5-dihydrothiazol-5-yl, 2,5-dihydrothiazol-2-yl, 2,5-dihydrothiazol-4-yl, 2,5-dihydrothiazol-5-yl, 2,3-dihydrothiazol-2-yl, 2,3-dihydrothiazol-4-yl, 2,3-dihydrothiazol-5-yl, 1,3-dioxol-2-yl, 1,3-dioxol-4-yl, 1,3-dithiol-2-yl, 1,3-dithiol-4-yl, 1,3-oxathiol-2-yl, 1,3-oxathiol-4-yl, 1,3-oxathiol-5-yl;
C-bound, 6-membered, partially unsaturated rings, such as 2H-3,4-dihydropyran-6-yl, 2H-3,4-dihydropyran-5-yl, 2H-3,4-dihydropyran-4-yl, 2H-3,4-dihydropyran-3-yl, 2H-3,4-dihydropyran-2-yl, 2H-3,4-dihydrothiopyran-6-yl, 2H-3,4-dihydrothiopyran-5-yl, 2H-3,4-dihydrothiopyran-4-yl, 2H-3,4-dihydrothiopyran-3-yl, 2H-3,4-dihydrothiopyran-2-yl, 1,2,3,4-tetrahydropyridin-6-yl, 1,2,3,4-tetrahydropyridin-5-yl, 1,2,3,4-tetrahydropyridin-4-yl, 1,2,3,4-tetra-hydropyridin-3-yl, 1,2,3,4-tetrahydropyridin-2-yl, 2H-5,6-dihydropyran-2-yl, 2H-5,6-dihydropyran-3-yl, 2H-5,6-dihydropyran-4-yl, 2H-5,6-dihydropyran-5-yl, 2H-5,6-dihydropyran-6-yl, 2H-5,6-dihydrothiopyran-2-yl, 2H-5,6-dihydrothiopyran-3-yl, 2H-5,6-dihydrothiopyran-4-yl, 2H-5,6-dihydrothiopyran-5-yl, 2H-5,6-dihydrothiopyran-6-yl, 1,2,5,6-tetrahydropyridin-2-yl, 1,2,5,6-tetrahydropyridin-3-yl, 1,2,5,6-tetrahydropyridin-4-yl, 1,2,5,6-tetrahydropyridin-5-yl, 1,2,5,6-tetrahydropyridin-6-yl, 2,3,4,5-tetrahydropyridin-2-yl, 2,3,4,5-tetrahydropyridin-3-yl, 2,3,4,5-tetrahydropyridin-4-yl, 2,3,4,5-tetrahydropyridin-5-yl, 2,3,4,5-tetrahydropyridin-6-yl, 4H-pyran-2-yl, 4H-pyran-3-yl-, 4H-pyran-4-yl, 4H-thiopyran-2-yl, 4H-thiopyran-3-yl, 4H-thiopyran-4-yl, 1,4-dihydropyridin-2-yl, 1,4-dihydropyridin-3-yl, 1,4-dihydropyridin-4-yl, 2H-pyran-2-yl, 2H-pyran-3-yl, 2H-pyran-4-yl, 2H-pyran-5-yl, 2H-pyran-6-yl, 2H-thiopyran-2-yl, 2H-thiopyran-3-yl, 2H-thiopyran-4-yl, 2H-thiopyran-5-yl, 2H-thiopyran-6-yl, 1,2-dihydropyridin-2-yl, 1,2-dihydro-pyridin-3-yl, 1,2-dihydropyridin-4-yl, 1,2-dihydropyridin-5-yl, 1,2-dihydro-pyridin-6-yl, 3,4-dihydropyridin-2-yl, 3,4-dihydropyridin-3-yl, 3,4-dihydropyridin-4-yl, 3,4-dihydropyridin-5-yl, 3,4-dihydropyridin-6-yl, 2,5-dihydropyridin-2-yl, 2,5-dihydropyridin-3-yl, 2,5-dihydropyridin-4-yl, 2,5-dihydropyridin-5-yl, 2,5-dihydropyridin-6-yl, 2,3-dihydropyridin-2-yl, 2,3-dihydropyridin-3-yl, 2,3-dihydropyridin-4-yl, 2,3-dihydropyridin-5-yl, 2,3-dihydropyridin-6-yl, 2H-5,6-dihydro-1,2-oxazin-3-yl, 2H-5,6-dihydro-1,2-oxazin-4-yl, 2H-5,6-dihydro-1,2-oxazin-5-yl, 2H-5,6-dihydro-1,2-oxazin-6-yl, 2H-5,6-dihydro-1,2-thiazin-3-yl, 2H-5,6-dihydro-1,2-thiazin-4-yl, 2H-5,6-dihydro-1,2-thiazin-5-yl, 2H-5,6-dihydro-1,2-thiazin-6-yl, 4H-5,6-dihydro-1,2-oxazin-3-yl, 4H-5,6-dihydro-1,2-oxazin-4-yl, 4H-5,6-dihydro-1,2-oxazin-5-yl, 4H-5,6-dihydro-1,2-oxazin-6-yl, 4H-5,6-dihydro-1,2-thiazin-3-yl, 4H-5,6-dihydro-1,2-thiazin-4-yl, 4H-5,6-dihydro-1,2-thiazin-5-yl, 4H-5,6-dihydro-1,2-thiazin-6-yl, 2H-3,6-dihydro-1,2-oxazin-3-yl, 2H-3,6-dihydro-1,2-oxazin-4-yl, 2H-3,6-dihydro-1,2-oxazin-5-yl, 2H-3,6-dihydro-1,2-oxazin-6-yl, 2H-3,6-dihydro-1,2-thiazin-3-yl, 2H-3,6-dihydro-1,2-thiazin-4-yl, 2H-3,6-dihydro-1,2-thiazin-5-yl, 2H-3,6-dihydro-1,2-thiazin-6-yl, 2H-3,4-dihydro-1,2-oxazin-3-yl, 2H-3,4-dihydro-1,2-oxazin-4-yl, 2H-3,4-dihydro-1,2-oxazin-5-yl, 2H-3,4-dihydro-1,2-oxazin-6-yl, 2H-3,4-dihydro-1,2-thiazin-3-yl, 2H-3,4-dihydro-1,2-thiazin-4-yl, 2H-3,4-dihydro-1,2-thiazin-5-yl, 2H-3,4-dihydro-1,2-thiazin-6-yl, 2,3,4,5-tetrahydropyridazin-3-yl, 2,3,4,5-tetrahydropyridazin-4-yl, 2,3,4,5-tetrahydropyridazin-5-yl, 2,3,4,5-tetrahydropyridazin-6-yl, 3,4,5,6-tetrahydropyridazin-3-yl, 3,4,5,6-tetrahydropyridazin-4-yl, 1,2,5,6-tetrahydropyridazin-3-yl, 1,2,5,6-tetrahydropyridazin-4-yl, 1,2,5,6-tetra-hydropyridazin-5-yl, 1,2,5,6-tetrahydropyridazin-6-yl, 1,2,3,6-tetrahydro-pyridazin-3-yl, 1,2,3,6-tetrahydropyridazin-4-yl, 4H-5,6-dihydro-1,3-oxazin-2-yl, 4H-5,6-dihydro-1,3-oxazin-4-yl, 4H-5,6-dihydro-1,3-oxazin-5-yl, 4H-5,6-dihydro-1,3-oxazin-6-yl, 4H-5,6-dihydro-1,3-thiazin-2-yl, 4H-5,6-dihydro-1,3-thiazin-4-yl, 4H-5,6-dihydro-1,3-thiazin-5-yl, 4H-5,6-dihydro-1,3-thiazin-6-yl, 3,4,5-6-tetrahydropyrimidin-2-yl, 3,4,5,6-tetrahydropyrimidin-4-yl, 3,4,5,6-tetrahydropyrimidin-5-yl, 3,4,5,6-tetrahydropyrimidin-6-yl, 1,2,3,4-tetrahydropyrazin-2-yl, 1,2,3,4-tetrahydropyrazin-5-yl, 1,2,3,4-tetrahydro-pyrimidin-2-yl, 1,2,3,4-tetrahydropyrimidin-4-yl, 1,2,3,4-tetrahydropyrimidin-5-yl, 1,2,3,4-tetrahydropyrimidin-6-yl, 2,3-dihydro-1,4-thiazin-2-yl, 2,3-dihydro-1,4-thiazin-3-yl, 2,3-dihydro-1,4-thiazin-5-yl, 2,3-dihydro-1,4-thiazin-6-yl, 2H-1,3-oxazin-2-yl, 2H-1,3-oxazin-4-yl, 2H-1,3-oxazin-5-yl, 2H-1,3-oxazin-6-yl, 2H-1,3-thiazin-2-yl, 2H-1,3-thiazin-4-yl, 2H-1,3-thiazin-5-yl, 2H-1,3-thiazin-6-yl, 4H-1,3-oxazin-2-yl, 4H-1,3-oxazin-4-yl, 4H-1,3-oxazin-5-yl, 4H-1,3-oxazin-6-yl, 4H-1,3-thiazin-2-yl, 4H-1,3-thiazin-4-yl, 4H-1,3-thiazin-5-yl, 4H-1,3-thiazin-6-yl, 6H-1,3-oxazin-2-yl, 6H-1,3-oxazin-4-yl, 6H-1,3-oxazin-5-yl, 6H-1,3-oxazin-6-yl, 6H-1,3-thiazin-2-yl, 6H-1,3-oxazin-4-yl, 6H-1,3-oxazin-5-yl, 6H-1,3-thiazin-6-yl, 2H-1,4-oxazin-2-yl, 2H-1,4-oxazin-3-yl, 2H-1,4-oxazin-5-yl, 2H-1,4-oxazin-6-yl, 2H-1,4-thiazin-2-yl, 2H-1,4-thiazin-3-yl, 2H-1,4-thiazin-5-yl, 2H-1,4-thiazin-6-yl, 4H-1,4-oxazin-2-yl, 4H-1,4-oxazin-3-yl, 4H-1,4-thiazin-2-yl, 4H-1,4-thiazin-3-yl, 1,4-dihydropyridazin-3-yl, 1,4-dihydropyridazin-4-yl, 1,4-dihydropyridazin-5-yl, 1,4-dihydropyridazin-6-yl, 1,4-dihydropyrazin-2-yl, 1,2-dihydropyrazin-2-yl, 1,2-dihydropyrazin-3-yl, 1,2-dihydropyrazin-5-yl, 1,2-dihydropyrazin-6-yl, 1,4-dihydropyrimidin-2-yl, 1,4-dihydropyrimidin-4-yl, 1,4-dihydropyrimidin-5-yl, 1,4-dihydropyrimidm-6-yl, 3,4-dihydropyrimidm-2-yl, 3,4-dihydropyrimidin-4-yl, 3,4-dihydropyrimidin-5-yl or 3,4-dihydropyrimidin-6- yl;
N-bound, 5-membered, partially unsaturated rings, such as 2,3-dihydro-1H-pyrrol-1-yl, 2,5-dihydro-1H-pyrrol-1-yl, 4,5-dihydro-1H-pyrazol-1-yl, 2,5-dihydro-1H-pyrazol-1-yl, 2,3-dihydro-1H-pyrazol-1-yl, 2,5-dihydroisoxazol-2-yl, 2,3-dihydroisoxazol-2-yl, 2,5-dihydroisothiazol-2-yl, 2,3-dihydroisoxazol-2-yl, 4,5-dihydro-1H-imidazol-1-yl, 2,5-dihydro-1H-imidazol-1-yl, 2,3-dihydro-1H-imidazol-1-yl, 2,3-dihydrooxazol-3-yl, 2,3-dihydrothiazol-3-yl;
N-bound, 6-membered, partially unsaturated rings, such as 1,2,3,4-tetrahydropyridin-1-yl, 1,2,5,6-tetrahydropyridin-1-yl, 1,4-dihydro-pyridin-1-yl, 1,2-dihydropyridin-1-yl, 2H-5,6-dihydro-1,2-oxazin-2-yl, 2H-5,6-dihydro-1,2-thiazin-2-yl, 2H-3,6-dihydro-1,2-oxazin-2-yl, 2H-3,6-dihydro-1,2-thiazin-2-yl, 2H-3,4-dihydro-1,2-oxazin-2-yl, 2H-3,4-dihydro-1,2-thiazin-2-yl, 2,3,4,5-tetrahydropyridazin-2-yl, 1,2,5,6-tetrahydropyridazin-1-yl, 1,2,5,6-tetrahydropyridazin-2-yl, 1,2,3,6-tetrahydropyridazin-1-yl, 3,4,5,6-tetrahydropyrimidin-3-yl, 1,2,3,4-tetrahydropyrazin-1-yl, 1,2,3,4-tetrahydropyrimidin-1-yl, 1,2,3,4-tetrahydropyrimidin-3-yl, 2,3-dihdro-1,4-thiazin-4-yl, 2H-1,2-oxazin-2-yl, 2H-1,2-thiazin-2-yl, 4H-1,4-oxazin-4-yl, 4H-1,4-thiazin-4-yl, 1,4-dihydropyridazin-1-yl, 1,4-dihydropyrazin-1-yl, 1,2-dihydropyrazin-1-yl, 1,4-dihydropyrimidin-1-yl or 3,4-dihydropyrimidin-3-yl;
C-bound, 5-membered, heteroaromatic rings, such as 2-furyl, 3-furyl, 5-furyl, 2-thienyl, 3-thienyl, 5-thienyl, pyrrol-2-yl, pyrrol-3-yl, pyrrol-5-yl, pyrazol-3-yl, pyrazol-4-yl, pyrazol-5-yl, isoxazol-3-yl, isoxazol-4-yl, isoxazol-5-yl, isothiazol-3-yl, isothiazol-4-yl, isothiazol-5-yl, imidazol-2-yl, imidazol-4-yl, imidazol-5-yl, oxazol-2-yl, oxazol-4-yl, oxazol-5-yl, thiazol-2-yl, thiazol-4-yl, thiazol-5-yl, 1,2,3-oxadiazol-imidazol-4-yl,4-yl, 1,2,3-oxadiazol-5-yl, 1,2,4-oxadiazol-3-yl, 1,2,4,-oxadiazol-5-yl, 1,3,4-oxadiazol-2-yl, 1,2,3-thiadiazol-4-yl, 1,2,3-thiadiazol-5-yl, 1,2,4-thiadiazol-3-yl, 1,2,4-thiadiazol-5-yl, 1,3,4-thiadiazolyl-2-yl, 1,2,3-triazol-4-yl, 1,2,4-triazol-3-yl, tetrazol-5-yl;
C-bound, 6-membered, heteroaromatic rings, such as pyridin-2-yl, pyridin-3-yl (3-pyridyl), pyridin-4-yl (4-pyridyl), pyridin-5-yl, pyridazin-3-yl, pyridazin-4-yl, pyridazin-6-yl, pyrimidin-2-yl, pyrimidin-4-yl, pyrimidin-5-yl, pyrazin-2-yl, pyrazin-5-yl, 1,3,5-triazin-2-yl, 1,2,4-triazin-3-yl, 1,2,4-triazin-5-yl, 1,2,4-triazin-6-yl, 1,2,4,5-tetrazin-3-yl;
N-bound, 5-membered, heteroaromatic rings, such as pyrrol-1-yl, pyrazol-1-yl, imidazol-1-yl, 1,2,3-triazol-1-yl, 1,2,4-triazol-1-yl, tetrazol-1-yl.

Heterocyclyl also includes bicyclic heterocycles, which comprise one of the described 5- or 6-membered heterocyclic rings and a further anellated, saturated or unsaturated or aromatic carbocycle, such as a benzene, cyclohexane, cyclohexene or cyclohexadiene ring, or a futher anellated 5- or 6-membered heterocyclic ring, this heterocyclic ring being saturated or unsaturated or aromatic. These include quinolinyl, isoquinolinyl,indolyl, indolizinyl, isoindolyl, indazolyl, benzofuryl, benzthienyl, benzo[b]thiazolyl, benzoxazolyl, benzthiazolyl, benzimidazolyl, imidazo[b]thiazolyl, thieno[b]pyridyl, imidazo[a]pyridyl, pyrazo[a]pyridyl and pyrrol[d]pyrimidyl. Examples of 5- or 6-membered heteroaromatic compounds comprising an anellated cycloalkenyl ring include dihydroindolyl, dihydroindolizinyl, dihydroisoindolyl, dihydroquinolinyl, dihydroisoquinolinyl, dihydrobenzofuryl, chromenyl, chromanyl, dihydropyrrol[a]imidazolyl and tetrahydro benzothiazolyl.

C₃-C₁₂-Heteroarylene is a heteroaryl diradical. Examples include pyrid-2,5-ylene and pyrid-2,4-ylene.

With respect to the compounds' capability of inhibiting glycine transporter 1, the variables R¹, R^{2a}, R^{2b}, R^{3a}, R^{3b}, R⁴, Y¹, R^{5a}, R^{5b}, R⁶, R^{7a}, R^{7b}, R^{7c}, R^{7d}, R^{7e}, R^{7f}, R^{8a}, R^{8b}, R^{8c}, R^{8a}, R^{8d}, R^{8e}, R^{9a}, R^{9b}, R^{9c}, R^{9d}, R¹⁰, R^{11a}, R^{11b}, R¹², R¹³, R¹⁴, R¹⁵, R^{16a}, R^{16b}, R¹⁷, R¹⁸, R¹⁹, R^{20a}, R^{20b}, R²¹, n1, n2, n3, n4 and n5 preferably have the following meanings which, when taken alone or in combination, represent particular embodiments of the pyrrolidine derivatives of the formula (I) or any other formula disclosed herein.

Such embodiments of the invention include the following embodiments E1 to E313 ((*) means not according to the invention):
E1. The compounds of formula (I) as defined in claim 1.
E2. The compounds of embodiment 1, wherein the term "substituted" means that a radical is substituted with 1, 2 or 3 substituents which are selected from the group consisting of halogen, C₁-C₄-alkyl, halogenated-C₁-C₄-alkyl, C₃-C₆-aryl-C₁-C₄-alkyl, hydroxy-C₁-C₄-alkyl, hydroxy-(halogenated C₁-C₄-alkyl), C₁-C₄-alkoxy-C₁-C₄-alkyl, amino-C₁-C₄-alkyl, C₃-C₁₂-heterocyclyl-C₁-C₄-alkyl, C₃-C₇-cycloalkyl, C₂-C₄-alkenyl, -CN, -CO₂H, C₁-C₄-alkoxycarbonyl, aminocarbonyl, C₁-C₄-alkylaminocarbonyl, (di-C₁-C₄-alkylamino)carbonyl, C₆-C₁₂-arylaminocarbonyl, C₃-C₁₂-heterocyclylaminocarbonyl, C₆-C₁₂-aryl, oxo (=O), OH, C₁-C₄-alkoxy, halogenated-C₁-C₄-alkoxy, C₃-C₇-cycloalkoxy, carboxy-C₁-C₄-alkoxy, C₆-C₁₂-aryl-C₁-C₄-alkoxy, C₆-C₁₂-aryloxy, C₃-C₁₂-heterocyclyl-C₁-C₄-alkoxy, SH, C₁-C₄-alkylthio, C₁-C₄-alkylsulfonyl, C₁-C₄-alkylaminosulfonyl, di-C₁-C₄-alkylaminosulfonyl, C₃-C₆-arylsulfonyl, aminosulfonyl, C₃-C₆-arylaminosulfonyl, C₃-C₁₂-heterocyclylaminosulfonyl, NH₂, C₁-C₄-alkylamino, di-C₁-C₄-alkylamino, C₆-C₁₂-aryl-C₁-C₄-alkylamino,C₁-C₄-alkylcarbonylamino, C₃-C₆-arylcarbonylamino, C₃-C₁₂-heterocyclylcarbonylamino, C₁-C₆-alkylsulfonylamino, C₃-C₆-arylsulfonylamino, C₃-C₁₂-heterocyclylsulfonylamino and C₃-C₁₂-heterocyclyl, wherein aryl and heterocyclyl may be unsubstituted or substituted with 1, 2 or 3 substituents selected from the group consisting of halogen, C₁-C₄-alkyl, C₁-C₄-haloalkyl, C₁-C₄-alkoxy and C₁-C₄-haloalkoxy.
E3. (*)The compounds of embodiment 1 or 2, wherein R¹ is an optionally substituted 5-membered heterocyclic ring containing at least 1 N atom and optionally 1 or 2 further heteroatoms selected from N, O and S.
E4. (*)The compounds of any one of embodiments 1-3, wherein R¹ is 5-membered heterocyclic ring containing at least 1 N atom and optionally 1 or 2 further heteroatoms selected from N, O and S, wherein the ring is optionally substituted with 1, 2, or 3 substituents independently selected from the group consisting of halogen, C₁-C₄-alkyl, halogenated C₁-C₄-alkyl, C₃-C₆-cycloalkyl, C₁-C₄-alkoxycarbonyl and C₁-C₄-alkylcarbonylamino.
E5. (*)The compounds of any one of embodiments 1-4, wherein R¹ is an optionally substituted 5-membered heterocyclic ring containing 1 or 2 N and 1 O.
E6. (*)The compounds of any one of embodiments 1-4, wherein R¹ is an optionally substituted 5-membered heterocyclic ring containing 1 or 2 N and 1 S.
E7. The compounds of any one of embodiments 1-4, wherein R¹ is an optionally substituted 5-membered heterocyclic ring containing 2 or 3 N.
E8. The compounds of embodiment 7, wherein R¹ is an optionally substituted 5-membered heterocyclic ring containing 2 N.
E9. The compounds of embodiment 8, wherein R¹ is optionally substituted 1,3-diazolyl.
E10. The compounds of embodiment 9, wherein the optionally substituted 1,3-diazolyl is 1,3-diazolyl optionally substituted with halogen or C₁-C₄-alkyl.
E11. The compounds of embodiment 9, wherein the optionally substituted 1,3-diazolyl is 1-methyl-1,3-diazol-4-yl.
E12. The compounds of embodiment 7, wherein R¹ is an optionally substituted 5-membered heterocyclic ring containing 3 N.
E13. The compounds of embodiment 12, wherein R¹ is the optionally substituted 1,2,3-triazolyl.
E14. The compounds of embodiment 13, wherein the optionally substituted 1,2,3-triazolyl is 1,2,3-triazolyl optionally substituted with C₁-C₄-alkyl.
E15. The compounds of embodiment 13, wherein the optionally substituted 1,2,3-triazolyl is 1-methyl-1,2,3-triazol-4-yl.
E16. The compounds of any one of embodiments 1-15, wherein R^{2a} is hydrogen, halogen or C₁-C₃-alkyl, and R^{2b} is hydrogen.
E17. The compounds of any one of embodiments 1-15, wherein R^{2a}, R^{2b} are hydrogen.
E18. The compounds of any one of embodiments 1-17, wherein R^{3a} is C₃-C₁₂-cycloalkyl, hydroxy, C₁-C₆-alkoxy, C₃-C₁₂-cycloalkyl-C₁-C₄-alkoxy, C₂-C₆-alkenyloxy, C₆-C₁₂-aryl-C₁-C₄-alkoxy, optionally substituted C₆-C₁₂-aryloxy, or optionally substituted C₃-C₁₂-heterocyclyl. E19. The compounds of any one of embodiments 1-18, wherein R^{3a} is optionally substituted C₆-C₁₂-aryl, provided that R^{3a} is not 3,4-di-O-substituted phenyl.
E20. The compounds of any one of embodiments 1-18, wherein R^{3a} is optionally substituted C₆-C₁₂-aryl, provided that there is not more than one O-bound substituent.
E21. The compounds of any one of embodiments 1-18, wherein R^{3a} is C₆-C₁₂-aryl optionally substituted with 1, 2, or 3 substituents independently selected from the group consisting of halogen, C₁-C₄-alkyl and C₁-C₄-alkoxy.
E22. The compounds of any one of embodiments 1-18, wherein R^{3a} is C₆-C₁₂-aryl optionally substituted with 1, 2, or 3 substituents independently selected from the group consisting of halogen and C₁-C₄-alkyl.
E23. The compounds of any one of embodiments 1-18, wherein R^{3a} is C₆-C₁₂-aryloxy optionally substituted with 1, 2, or 3 substituents independently selected from the group consisting of halogen and C₁-C₄-alkyl.
E24. The compounds of any one of embodiments 1-18, wherein R^{3a} is C₃-C₁₂-heterocyclyl optionally substituted with 1, 2, or 3 substituents independently selected from the group consisting of halogen, C₁-C₄-alkyl and C₃-C₆-cycloalkyl.
E25. The compounds of any one of embodiments 1-24, wherein R^{3b} is hydrogen.
E26. The compounds of any one of embodiments 1-25, wherein Y¹ is >CR⁶-.
E27. The compounds of any one of embodiments 1-26, wherein R⁶ is hydrogen, C₁-C₆-alkyl, C₆-C₁₂-aryl-C₁-C₄-alkyl, hydroxy-C₁-C₆-alkyl, or hydroxyl.
E28. The compounds of any one of embodiments 1-26, wherein R⁶ is hydrogen, methyl, benzyl, hydroxy-methyl, or hydroxy.
E29. The compounds of any one of embodiments 1-28, wherein Y¹ is >N-.
E30. The compounds of any one of embodiments 1-28, wherein Y¹ is >N- and R⁴ is - (CR^{7e}R^{7f})ₙ₃R¹².
E31. The compounds of any one of embodiments 1-30, wherein R^{5a} is hydrogen, halogen or C₁-C₃-alkyl, and R^{5b} is hydrogen.
E32. The compounds of any one of embodiments 1-30, wherein R^{5a}, R^{5b} are hydrogen.
E33. The compounds of any one of embodiments 1-30, wherein R^{5a}, R^{5b} together with the carbon atom to which they are bound form a C=O.
E34. The compounds of any one of embodiments 1-33, wherein R⁴ is -(CR^{7a}R^{7b})ₙ₁OR¹⁰,-(CR^{7c}R^{7d})ₙ₂NR^{11a}R^{11b}, -(CR^{7e}R^{7f})ₙ₃R¹², optionally substituted C₆-C₁₂-aryl, - NR^{8a}(CR^{9a}R^{9b})ₙ₄R¹³, -NR^{8b}COR¹⁴, -NR^{8c}COOR¹⁵, -NR^{8d}CONR^{16a}R^{16b}, -O(CR^{9c}R^{9d})ₙ₅R¹⁸,-COR¹⁹, -CONR^{20a}R^{20b}, -SO₂R²¹, or optionally substituted C₃-C₁₂-heterocyclyl.
E35. The compounds of any one of embodiments 1-34, having formula 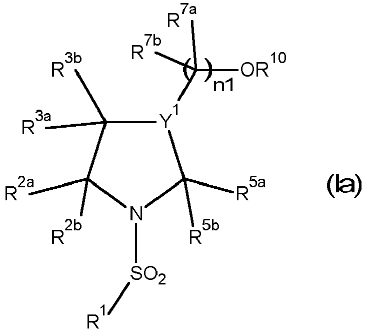
   wherein R¹, R^{2a}, R^{2b}, R^{3a}, R^{3b}, Y¹, R^{5a} and R^{5b} are as defined in any one of embodiments 1-34, and
   R^{7a}, R^{7b}
      are independently hydrogen or C₁-C₆-alkyl;
   n1 is 1, 2, 3, or 4; and
   R¹⁰ is hydrogen, optionally substituted C₆-C₁₂-aryl, or optionally substituted C₃-C₁₂-heterocyclyl.
E36. The compounds of embodiment 35, wherein R^{2a}, R^{2b} are hydrogen.
E37. The compounds of embodiment 35 or 36, wherein R^{3a} is optionally substituted C₆-C₁₂-aryl. E38. The compounds of any one of embodiments 35-37, wherein R^{3b} is hydrogen.
E39. The compounds of any one of embodiments 35-38, wherein Y¹ is >CR⁶.
E40. The compounds of any one of embodiments 35-39, wherein R⁶ is hydrogen.
E41. The compounds of any one of embodiments 35-40, wherein R^{7a}, R^{7b} are hydrogen.
E42. The compounds of any one of embodiments 35-41, wherein n1 is 1.
E43. The compounds of any one of embodiments 35-42, wherein R^{5a}, R^{5b} are hydrogen.
E44. The compounds of embodiment 35, wherein
   - R¹: is an optionally substituted 5-membered heterocyclic ring containing 2 or 3 N heteroatoms;
   - R^{2a}, R^{2b}: are hydrogen;
   - R^{3a}: is optionally substituted C₆-C₁₂-aryl;
   - R^{3b}: is hydrogen;
   - Y¹: is >CR⁶;
   - R⁶: is hydrogen;
   - R^{7A} R^{7b}: are hydrogen;
   - n1: is 1;
   - R¹⁰: is hydrogen, optionally substituted C₆-C₁₂-aryl, or optionally substituted C₃-C₁₂-heterocyclyl; and
   - R^{5a}, R^{5b}: are hydrogen.
E45. The compounds of any one of embodiments 35-44, wherein R¹ is an optionally substituted 5-membered heterocyclic ring containing 2 or 3 N.
E46. The compounds of any one of embodiments 35-45, wherein R¹ is an optionally substituted 5-membered heterocyclic ring containing 2 N.
E47. The compounds of any one of embodiments 35-46, wherein R¹ is optionally substituted 1,3-diazolyl.
E48. The compounds of any one of embodiments 35-47, wherein R¹ is 1,3-diazolyl optionally substituted with halogen or C₁-C₄-alkyl.
E49. The compounds of any one of embodiments 35-48, wherein R^{3a} is C₆-C₁₂-aryl optionally substituted with 1, 2, or 3 substituents independently selected from the group consisting of halogen and C₁-C₄-alkyl.
E50. The compounds of any one of embodiments 35-49, wherein R¹⁰ is C₆-C₁₂-aryl optionally substituted with 1, 2, or 3 substituents independently selected from the group consisting of halogen, C₁-C₄-alkyl, halogenated C₁-C₄-alkyl and halogenated C₁-C₄-alkoxy.
E51. The compounds of any one of embodiments 35-49, wherein R¹⁰ is C₃-C₁₂-heterocyclyl optionally substituted with 1, 2, or 3 substituents independently selected from the group consisting of halogenated C₁-C₄-alkyl.
E52. The compounds of embodiment 35, wherein
   - R¹: is 1,3-diazolyl optionally substituted with halogen or C₁-C₄-alkyl;
   - R^{2a},R^{2b}: are hydrogen;
   - R^{3a}: is C₆-C₁₂-aryl optionally substituted with 1, 2, or 3 substituents independently selected from the group consisting of halogen and C₁-C₄-alkyl;
   - R^{3b}: is hydrogen;
   - Y¹: is >CR⁶;
   - R⁶: is hydrogen;
   - R^{7a}, R^{7b}: are hydrogen;
   - n1: is 1;
   - R¹⁰: is hydrogen, C₆-C₁₂-aryl optionally substituted with 1, 2, or 3 substituents independently selected from the group consisting of halogen, C₁-C₄-alkyl, halogenated C₁-C₄-alkyl and halogenated C₁-C₄-alkoxy, or C₃-C₁₂-heterocyclyl optionally substituted with 1, 2, or 3 substituents independently selected from the group consisting of halogenated C₁-C₄-alkyl; and
   - R^{5a}, R^{5b}: are hydrogen.
E53. The compounds of any one of embodiments 35-52, wherein R¹ is 1-methyl-1,3-diazol-4-yl.
E54. The compounds of any one of embodiments 35-53, wherein R^{3a} is 4-F-phenyl.
E55. The compounds of any one of embodiments 1-34, having formula 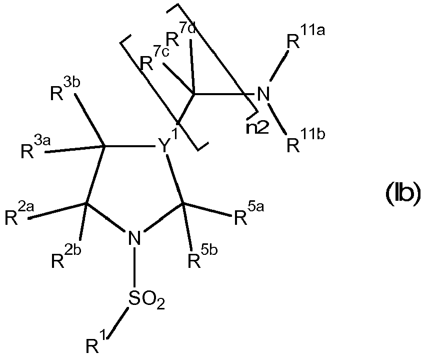
   wherein R¹, R^{2a}, R^{2b}, R^{3a}, R^{3b}, Y¹, R^{5a} and R^{5b} are as defined in any one of embodiments 1-34, and
   R^{7c}, R^{7d}
      are independently hydrogen or C₁-C₆-alkyl;
   n2 is 1, 2, 3 or 4;
   R^{11a} is C₁-C₈-alkyl, (optionally substituted C₃-C₁₂-cycloalkyl)-C₁-C₄-alkyl, C₁-C₆-alkoxy-C₁-C₆-alkyl, (optionally substituted C₆-C₁₂-aryl)-C₁-C₄-alkyl, (optionally substituted C₃-C₁₂-heterocyclyl)-C₁-C₄-alkyl, optionally substituted C₆-C₁₂-aryl, or optionally substituted C₃-C₁₂-heterocyclyl; and
   R^{11b} is hydrogen or C₁-C₆-alkyl.
E56. The compounds of embodiment 55, wherein R^{2a}, R^{2b} are hydrogen.
E57. The compounds of embodiment 55 or 56, wherein R^{3a} is optionally substituted C₆-C₁₂-aryl.
E58. The compounds of any one of embodiments 55-57, wherein R^{3b} is hydrogen.
E59. The compounds of any one of embodiments 55-58, wherein Y¹ is >CR⁶.
E60. The compounds of any one of embodiments 55-59, wherein R⁶ is hydrogen.
E61. The compounds of any one of embodiments 55-60, wherein R^{7c}, R^{7d} are hydrogen.
E62. The compounds of any one of embodiments 55-61, wherein n2 is 1.
E63. The compounds of any one of embodiments 55-62, wherein R^{11a} is C₁-C₈-alkyl, C₃-C₁₂-cycloalkyl-C₁-C₄-alkyl, C₁-C₆-alkoxy-C₁-C₆-alkyl, C₆-C₁₂-aryl-C₁-C₄-alkyl, or optionally substituted C₆-C₁₂-aryl.
E64. The compounds of any one of embodiments 55-63, wherein R^{11b} is hydrogen.
E65. The compounds of any one of embodiments 55-64, wherein R^{5a}, R^{5b} are hydrogen.
E66. The compounds of embodiment 55, wherein
   - R¹: is an optionally substituted 5-membered heterocyclic ring containing 2 or 3 N heteroatoms;
   - R^{2a}, R^{2b}: are hydrogen;
   - R^{3a}: is optionally substituted C₆-C₁₂-aryl;
   - R^{3b}: is hydrogen;
   - Y¹: is >CR⁶;
   - R⁶: is hydrogen;
   - R^{7c}, R^{7d}: are hydrogen,
   - n2: is 1;
   - R^{11a}: is C₁-C₈-alkyl, C₃-C₁₂-cycloalkyl-C₁-C₄-alkyl, C₁-C₆-alkoxy-C₁-C₆-alkyl, C₆-C₁₂-aryl-C₁-C₄-alkyl, or optionally substituted C₆-C₁₂-aryl;
   - R^{11b}: is hydrogen; and
   - R^{5a}, R^{5b}: are hydrogen.
E67. The compounds of any one of embodiments 55-66, wherein R¹ is an optionally substituted 5-membered heterocyclic ring containing 2 or 3 N.
E68. The compounds of any one of embodiments 55-67, wherein R¹ is an optionally substituted 5-membered heterocyclic ring containing 2 N.
E69. The compounds of any one of embodiments 55-68, wherein R¹ is optionally substituted 1,3-diazolyl.
E70. The compounds of any one of embodiments 55-69, wherein R¹ is 1,3-diazolyl optionally substituted with halogen or C₁-C₄-alkyl.
E71. The compounds of any one of embodiments 55-70, wherein R^{3a} is C₆-C₁₂-aryl optionally substituted with 1, 2, or 3 substituents independently selected from the group consisting of halogen and C₁-C₄-alkyl.
E72. The compounds of any one of embodiments 55-71, wherein R^{11a} is C₆-C₁₂-aryl optionally substituted with 1, 2, or 3 substituents independently selected from the group consisting of halogen, C₁-C₄-alkyl, halogenated C₁-C₄-alkyl, amino-C₁-C₄-alkyl, amino-carbonyl and halogenated C₁-C₄-alkoxy.
E73. The compounds of embodiment 55, wherein
   - R¹: is 1,3-diazolyl optionally substituted with halogen or C₁-C₄-alkyl;
   - R^{2a}, R^{2b}: are hydrogen;
   - R^{3a}: is C₆-C₁₂-aryl optionally substituted with 1, 2, or 3 substituents independently selected from the group consisting of halogen and C₁-C₄-alkyl;
   - R^{3b}: is hydrogen;
   - Y¹: is >CR⁶;
   - R⁶: is hydrogen;
   - R^{7c}, R^{7d}: are hydrogen,
   - n2: is 1;
   - R^{11a}: is C₁-C₈-alkyl, C₃-C₁₂-cycloalkyl-C₁-C₄-alkyl, C₁-C₆-alkoxy-C₁-C₆-alkyl, C₆-C₁₂-aryl-C₁-C₄-alkyl, or C₆-C₁₂-aryl optionally substituted with 1, 2, or 3 substituents independently selected from the group consisting of halogen, C₁-C₄-alkyl, halogenated C₁-C₄-alkyl, amino-C₁-C₄-alkyl, amino-carbonyl and halogenated C₁-C₄-alkoxy;
   - R^{11b}: is hydrogen; and
   - R^{5a}, R^{5b}: are hydrogen.
E74. The compounds of any one of embodiments 55-73, wherein R¹ is 1-methyl-1,3-diazol-4-yl.
E75. The compounds of any one of embodiments 55-74, wherein R^{3a} is 4-F-phenyl.
E76. The compounds of any one of embodiments 1-34, having formula 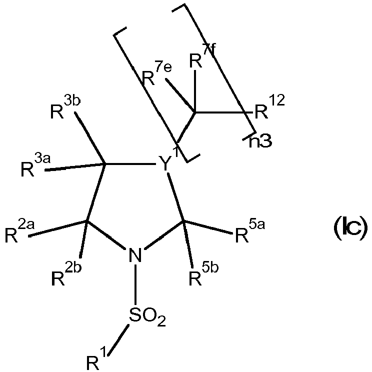
   wherein R¹, R^{2a}, R^{2b}, R^{3a}, R^{3b}, Y¹, R^{5a} and R^{5b} are as defined in any one of embodiments 1-34, and
   R^{7e}, R^{7f}
      are independently hydrogen or C₁-C₆-alkyl;
   n3 is 1, 2, 3, or 4; and
   R¹² is optionally substituted C₆-C₁₂-aryl or optionally substituted C₃-C₁₂-heterocyclyl.
E77. The compounds of embodiment 76, wherein R^{2a}, R^{2b} are hydrogen.
E78. The compounds of embodiment 76 or 77, wherein R^{3a} is C₃-C₁₂-cycloalkyl or optionally substituted C₆-C₁₂-aryl.
E79. The compounds of any one of embodiments 76-78, wherein R^{3b} is hydrogen.
E80. The compounds of any one of embodiments 76-79, wherein Y¹ is >CR⁶.
E81. The compounds of any one of embodiments 76-80, wherein R⁶ is hydrogen or hydroxy.
E82. The compounds of any one of embodiments 76-79, wherein Y¹ is >N-.
E83. The compounds of any one of embodiments 76-79, wherein Y¹ is >N- and R^{5a}, R^{5b} together with the carbon atom to which they are bound form a C=O.
E84. The compounds of any one of embodiments 76-83, wherein R^{7e}, R^{7f} are hydrogen.
E85. The compounds of any one of embodiments 76-84, wherein n3 is 1.
E86. The compounds of any one of embodiments 76-85, wherein R^{5a}, R^{5b} are hydrogen or together with the carbon atom to which they are bound may form a C=O.
E87. The compounds of embodiment 76, wherein
   - R¹: is an optionally substituted 5-membered heterocyclic ring containing 2 or 3 N heteroatoms ;
   - R^{2a}, R^{2b}: are hydrogen;
   - R^{3a}: is C₃-C₁₂-cycloalkyl or optionally substituted C₆-C₁₂-aryl;
   - R^{3b}: is hydrogen;
   - Y¹: is >CR⁶ or >N-;
   - R⁶: is hydrogen or hydroxy;
   - R^{7e}, R^{7f}: are hydrogen,
   - n3: is 1;
   - R¹²: is optionally substituted C₆-C₁₂-aryl or optionally substituted C₃-C₁₂-heterocyclyl; and
   - R^{5a}, R^{5b}: are hydrogen or together with the carbon atom to which they are bound may form a C=O.
E88. The compounds of any one of embodiments 76-87, wherein R¹ is an optionally substituted 5-membered heterocyclic ring containing 2 or 3 N.
E89. The compounds of any one of embodiments 76-88, wherein R¹ is an optionally substituted 5-membered heterocyclic ring containing 2 N.
E90. The compounds of any one of embodiments 76-89, wherein R¹ is optionally substituted 1,3-diazolyl.
E91. The compounds of any one of embodiments 76-90, wherein R¹ is 1,3-diazolyl optionally substituted with halogen or C₁-C₄-alkyl.
E92. The compounds of any one of embodiments 76-91, wherein R^{3a} is C₃-C₁₂-cycloalkyl or C₆-C₁₂-aryl optionally substituted with halogen.
E93. The compounds of any one of embodiments 76-92, wherein R¹² is C₆-C₁₂-aryl optionally substituted with 1, 2, or 3 substituents independently selected from the group consisting of halogen and C₁-C₄-alkyl.
E94. The compounds of any one of embodiments 76-92, wherein R¹² is C₃-C₁₂-heterocyclyl optionally substituted with 1, 2, or 3 substituents independently selected from the group consisting of C₁-C₄-alkyl and halogenated C₁-C₄-alkyl.
E95. The compounds of embodiment 76, wherein
   - R¹: is 1,3-diazolyl optionally substituted with halogen or C₁-C₄-alkyl;
   - R^{2a}, R^{2b}: are hydrogen;
   - R^{3a}: is C₃-C₁₂-cycloalkyl or C₆-C₁₂-aryl optionally substituted with halogen and C₁-C₄-alkyl;
   - R^{3b}: is hydrogen;
   - Y¹: is >CR⁶ or >N-;
   - R⁶: is hydrogen or hydroxy;
   - R^{7e}, R^{7f}: are hydrogen,
   - n3: is 1;
   - R¹²: is C₆-C₁₂-aryl optionally substituted with 1, 2, or 3 substituents independently selected from the group consisting of halogen, or C₃-C₁₂-heterocyclyl optionally substituted with 1, 2, or 3 substituents independently selected from the group consisting of C₁-C₄-alkyl and halogenated C₁-C₄-alkyl; and
   - R^{5a}, R^{5b}: are hydrogen or together with the carbon atom to which they are bound may form a C=O.
E96. The compounds of any one of embodiments 76-95, wherein R¹ is 1-methyl-1,3-diazol-4-yl.
E97. The compounds of any one of embodiments 1-34, wherein R⁴ is optionally substituted C₆-C₁₂-aryl.
E98. The compounds of embodiment 97, wherein R^{2a}, R^{2b} are hydrogen.
E99. The compounds of embodiment 97 or 98, wherein R^{3a} is optionally substituted C₆-C₁₂-aryl.
E100. The compounds of any one of embodiments 97-99, wherein R^{3b} is hydrogen.
E101. The compounds of any one of embodiments 97-100, wherein Y¹ is >CR⁶.
E102. The compounds of any one of embodiments 97-101, wherein R⁶ is hydrogen.
E103. The compounds of any one of embodiments 97-102, wherein R^{5a}, R^{5b} are hydrogen.
E104. The compounds of embodiment 97, wherein
   - R¹: is an optionally substituted 5-membered heterocyclic ring containing 2 or 3 N heteroatoms;
   - R^{2a}, R^{2b}: are hydrogen;
   - R^{3a}: is optionally substituted C₆-C₁₂-aryl;
   - R^{3b}: is hydrogen;
   - R⁴: is optionally substituted C₆-C₁₂-aryl;
   - Y¹: is >CR⁶;
   - R⁶: is hydrogen; and
   - R^{5a}, R^{5b}: are hydrogen.
E105. The compounds of any one of embodiments 97-104, wherein R¹ is an optionally substituted 5-membered heterocyclic ring containing 2 or 3 N.
E106. The compounds of any one of embodiments 97-105, wherein R¹ is an optionally substituted 5-membered heterocyclic ring containing 2 N.
E107. The compounds of any one of embodiments 97-106, wherein R¹ is optionally substituted 1,3-diazolyl.
E108. The compounds of any one of embodiments 97-107, wherein R¹ is 1,3-diazolyl optionally substituted with halogen or C₁-C₄-alkyl.
E109. The compounds of any one of embodiments 97-108, wherein R^{3a} is C₆-C₁₂-aryl optionally substituted with 1, 2, or 3 substituents independently selected from the group consisting of halogen and C₁-C₄-alkyl.
E110. The compounds of any one of embodiments 97-109, wherein R⁴ is C₆-C₁₂-aryl optionally substituted with 1, 2, or 3 substituents independently selected from the group consisting of halogen.
E111. The compounds of embodiment 97, wherein
   - R¹: is 1,3-diazolyl optionally substituted with halogen or C₁-C₄-alkyl;
   - R^{2a}, R^{2b}: are hydrogen;
   - R^{3a}: is C₆-C₁₂-aryl optionally substituted with halogen and C₁-C₄-alkyl;
   - R^{3b}: is hydrogen;
   - R⁴: is C₆-C₁₂-aryl optionally substituted with 1, 2, or 3 substituents independently selected from the group consisting of halogen;
   - Y¹: is >CR⁶;
   - R⁶: is hydrogen; and
   - R^{5a}, R^{5b}: are hydrogen.
E112. The compounds of any one of embodiments 97-111, wherein R¹ is 1-methyl-1,3-diazol-4-yl.
E113. The compounds of any one of embodiments 97-112, wherein R^{3a} is 4-F-phenyl.
E114. The compounds of any one of embodiments 1-34, having formula 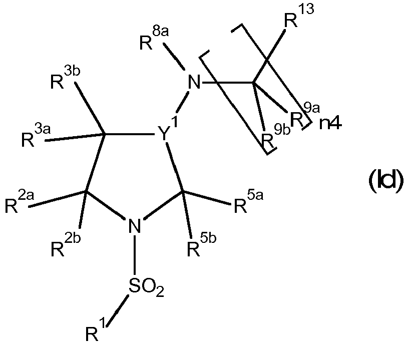
   wherein R¹, R^{2a}, R^{2b}, R^{3a}, R^{3b}, Y¹, R^{5a} and R^{5b} are as defined in any one of embodiments 1-34, and
   R^{8a} is hydrogen, C₁-C₆-alkyl, or C₁-C₆₋alkylcarbonyl, or
   R⁶, R^{8a}
      together are optionally substituted C₁-C₅-alkylene, wherein one or more -CH₂- of C₁-C₅-alkylene may be independently replaced by a an oxygen atom or C=O;
   R^{9a}, R^{9b}
      are independently hydrogen, halogen, C₁-C₆-alkyl, hydroxy, or C₁-C₆-alkoxy;
   n4 is 0, 1, 2, 3, or 4;
   R¹³ is hydrogen, C₁-C₈-alkyl, halogenated C₁-C₆-alkyl, (optionally substituted C₃-C₁₂-cycloalkyl)-C₁-C₄-alkyl, C₁-C₆-alkoxy-C₁-C₆-alkyl, optionally substituted C₃-C₁₂-cycloalkyl, C₂-C₆-alkenyl, optionally substituted C₃-C₆-cycloalkenyl, optionally substituted C₆-C₁₂-aryl, hydroxy, C₁-C₆-alkoxy, C₁-C₆-alkoxy-C₁-C₄-alkoxy, optionally substituted C₆-C₁₂-aryloxy, optionally substituted C₃-C₁₂-heterocyclyloxy, optionally substituted C₃-C₁₂-heterocyclyl, or tri-(C₁-C₄-alkyl)-silyloxy.
E115. The compounds of embodiment 114, wherein R^{2a}, R^{2b} are hydrogen.
E116. The compounds of embodiment 114 or 115, wherein R^{3a} is C₃-C₁₂-cycloalkyl, optionally substituted C₆-C₁₂-aryl, hydroxy, C₁-C₆-alkoxy, C₃-C₁₂-cycloalkyl-C₁-C₄-alkoxy, C₂-C₆-alkenyloxy, C₆-C₁₂-aryl-C₁-C₄-alkoxy, optionally substituted C₆-C₁₂-aryloxy, or optionally substituted C₃-C₁₂-heterocyclyl.
E117. The compounds of any one of embodiments 114-116, wherein R^{3b} is hydrogen or hydroxy.
E118. The compounds of any one of embodiments 114-117, wherein Y¹ is >CR⁶.
E119. The compounds of any one of embodiments 114-118, wherein R⁶ is hydrogen, C₁-C₆-alkyl, or hydroxy-C₁-C₆-alkyl.
E120. The compounds of any one of embodiments 114-119, wherein R^{9a}, R^{9b} are independently hydrogen, halogen, C₁-C₆-alkyl, or C₁-C₆-alkoxy.
E121. The compounds of any one of embodiments 114-120, wherein R^{9a} is hydrogen, halogen, C₁-C₆-alkyl, or C₁-C₆-alkoxy and R^{9b} is hydrogen.
E122. The compounds of any one of embodiments 114-121, wherein R¹³ is hydrogen, C₁-C₈-alkyl, halogenated C₁-C₆-alkyl, C₁-C₆-alkoxy-C₁-C₆-alkyl, optionally substituted C₃-C₁₂-cycloalkyl, C₂-C₆-alkenyl, optionally substituted C₃-C₆-cycloalkenyl, optionally substituted C₆-C₁₂-aryl, C₁-C₆-alkoxy, C₁-C₆-alkoxy-C₁-C₄-alkoxy, optionally substituted C₆-C₁₂-aryloxy, optionally substituted C₃-C₁₂-heterocyclyl, or tri-(C₁-C₄-alkyl)-silyloxy.
E123. The compounds of any one of embodiments 114-122, wherein R^{5a}, R^{5b} are hydrogen;
E124. The compounds of embodiment 114, wherein
   - R¹: is an optionally substituted 5-membered heterocyclic ring containing 2 or 3 N heteroatoms;
   - R^{2a}, R^{2b}: are hydrogen;
   - R^{3a}: is C₃-C₁₂-cycloalkyl, optionally substituted C₆-C₁₂-aryl, hydroxy, C₁-C₆-alkoxy, C₃-C₁₂-cycloalkyl-C₁-C₄-alkoxy, C₂-C₆-alkenyloxy, C₆-C₁₂-aryl-C₁-C₄-alkoxy, optionally substituted C₆-C₁₂-aryloxy, or optionally substituted C₃-C₁₂-heterocyclyl;
   - R^{3b}: is hydrogen or hydroxy;
   - Y¹: is >CR⁶;
   - R⁶: is hydrogen, C₁-C₆-alkyl, or hydroxy-C₁-C₆-alkyl
   - R^{8a}: is hydrogen, C₁-C₆-alkyl, or C₁-C₆₋alkylcarbonyl, or
   - R⁶,R^{8a}: are together optionally substituted C₁-C₅-alkylene, wherein one or more -CH₂- of C₁-C₅-alkylene may be independently replaced by a an oxygen atom or C=O;
   - R^{9a}: is hydrogen, halogen, C₁-C₆-alkyl, or C₁-C₆-alkoxy;
   - R^{9b}: is hydrogen;
   - n4: is 0, 1, 2, 3, or 4;
   - R¹³: is hydrogen, C₁-C₈-alkyl, halogenated C₁-C₆-alkyl, C₁-C₆-alkoxy-C₁-C₆-alkyl, optionally substituted C₃-C₁₂-cycloalkyl, C₂-C₆-alkenyl, optionally substituted C₃-C₆-cycloalkenyl, optionally substituted C₆-C₁₂-aryl, C₁-C₆-alkoxy, C₁-C₆-alkoxy-C₁-C₄-alkoxy, optionally substituted C₆-C₁₂-aryloxy optionally substituted C₃-C₁₂-heterocyclyl, or tri-(C₁-C₄-alkyl)-silyloxy; and
   - R^{5a}, R^{5b}: are hydrogen.
E125. The compounds of any one of embodiments 114-124, wherein R¹ is an optionally substituted 5-membered heterocyclic ring containing 2 or 3 N.
E126. The compounds of any one of embodiments 114-125, wherein R¹ is an optionally substituted 5-membered heterocyclic ring containing 2 N.
E127. The compounds of any one of embodiments 114-126, wherein R¹ is optionally substituted 1,3-diazolyl.
E128. The compounds of any one of embodiments 114-127, wherein R¹ is 1,3-diazolyl optionally substituted with halogen or C₁-C₄-alkyl.
E129. The compounds of any one of embodiments 114-125, wherein R¹ is an optionally substituted 5-membered heterocyclic ring containing 3 N.
E130. The compounds of any one of embodiments 114-125 or 129, wherein R¹ is optionally substituted 1,2,3-triazolyl.
E131. The compounds of in any one of embodiments 114-125, 129 or 130 wherein R¹ is 1,2,3-triazolyl optionally substituted with C₁-C₄-alkyl.
E132. The compounds of any one of embodiments 114-131, wherein R^{3a} is C₆-C₁₂-aryl optionally substituted with 1, 2, or 3 substituents independently selected from the group consisting of halogen and C₁-C₄-alkyl.
E133. The compounds of any one of embodiments 114-131, wherein R^{3a} is C₆-C₁₂-aryloxy optionally substituted with 1, 2, or 3 substituents independently selected from the group consisting of halogen and C₁-C₄-alkyl.
E134. The compounds of any one of embodiments 114-131, wherein R^{3a} is C₃-C₁₂-heterocyclyl optionally substituted with 1, 2, or 3 substituents independently selected from the group consisting of halogen, C₁-C₄-alkyl and C₃-C₆-cycloalkyl.
E135. The compounds of any one of embodiments 114-134, wherein R¹³ is C₃-C₁₂-cycloalkyl optionally substituted with 1, 2, or 3 substituents independently selected from the group consisting of halogen, C₁-C₄-alkyl, halogenated C₁-C₄-alkyl, C₁-C₄-alkoxy-C₁-C₄-alkyl and C₆-C₁₂-aryl.
E136. The compounds of any one of embodiments 114-134, wherein R¹³ is C₃-C₆-cycloalkenyl optionally substituted with 1, 2, or 3 substituents independently selected from the group consisting of C₁-C₄-alkyl.
E137. The compounds of any one of embodiments 114-134, wherein R¹³ is C₆-C₁₂-aryl optionally substituted with 1, 2, or 3 substituents independently selected from the group consisting of halogen, C₁-C₄-alkyl, halogenated C₁-C₄-alkyl, hydroxy-(halogenated C₁-C₄-alkyl), CN, C₆-C₁₂-aryl, C₁-C₄-alkoxy, halogenated C₁-C₄-alkoxy, C₆-C₁₂ aryl-C₁-C₄-alkoxy, C₆-C₁₂-aryloxy, C₁-C₄-alkyl-sulfonyl, C₁-C₄-alkyl-carbonylamino and C₃-C₁₂-heterocyclyl.
E138. The compounds of any one of embodiments 114-134, wherein R¹³ is C₆-C₁₂-aryloxy optionally substituted with 1, 2, or 3 substituents independently selected from the group consisting of halogen and C₁-C₄-alkyl.
E139. The compounds of any one of embodiments 114-134, wherein R¹³ is C₃-C₁₂-heterocyclyl optionally substituted with 1, 2, or 3 substituents independently selected from the group consisting of halogen, C₁-C₄-alkyl, halogenated C₁-C₄-alkyl, C₆-C₁₂-aryl-C₁-C₄-alkyl, C₃-C₆-cycloalkyl, hydroxy, CN, C₆-C₁₂-aryl optionally substituted with halogen or C₁-C₄-alkyl, C₁-C₄-alkoxy, halogenated C₁-C₄-alkoxy, C₃-C₆-cycloalkoxy, C₆-C₁₂ aryl-C₁-C₄-alkoxy, C₃-C₁₂-heterocyclyl-C₁-C₄-alkoxy and C₃-C₁₂-heterocyclyl.
E140. The compounds of embodiment 114, wherein
   - R¹: is 1,3-diazolyl optionally substituted with halogen or C₁-C₄-alkyl, or 1,2,3-triazolyl optionally substituted with C₁-C₄-alkyl;
   - R^{2a}, R^{2b}: are hydrogen;
   - R^{3a}: is C₃-C₁₂-cycloalkyl, C₆-C₁₂-aryl optionally substituted with 1, 2, or 3 substituents independently selected from the group consisting of halogen and C₁-C₄-alkyl, or hydroxy, C₁-C₆-alkoxy, C₃-C₁₂-cycloalkyl-C₁-C₄-alkoxy, C₂-C₆-alkenyloxy, C₆-C₁₂-aryl-C₁-C₄-alkoxy, C₆-C₁₂-aryloxy optionally substituted with 1, 2, or 3 substituents independently selected from the group consisting of halogen and C₁-C₄-alkyl, or C₃-C₁₂-heterocyclyl optionally substituted with 1, 2, or 3 substituents independently selected from the group consisting of halogen, C₁-C₄-alkyl and C₃-C₆-cycloalkyl;
   - R^{3b}: is hydrogen or hydroxy;
   - Y¹: is >CR⁶;
   - R⁶: is hydrogen, C₁-C₆-alkyl, or hydroxy-C₁-C₆-alkyl, or
   - R^{8a}: is hydrogen, C₁-C₆-alkyl, or C₁-C₆₋alkylcarbonyl, or
   - R⁶,R^{8a}: are together optionally substituted C₁-C₅-alkylene, wherein one or more -CH₂- of C₁-C₅-alkylene may be independently replaced by a an oxygen atom or C=O;
   - R^{9a}: is hydrogen, halogen, C₁-C₆-alkyl, or C₁-C₆-alkoxy;
   - R^{9b}: is hydrogen;
   - n4: is 0, 1, 2, 3, or 4;
   - R¹³: is hydrogen, C₁-C₈-alkyl, halogenated C₁-C₆-alkyl, C₁-C₆-alkoxy-C₁-C₆-alkyl, C₃-C₁₂-cycloalkyl optionally substituted with 1, 2, or 3 substituents independently selected from the group consisting of halogen, C₁-C₄-alkyl, halogenated C₁-C₄-alkyl, C₁-C₄-alkoxy-C₁-C₄-alkyl and C₆-C₁₂-aryl, or C₂-C₆-alkenyl, C₃-C₆-cycloalkenyl optionally substituted with 1, 2, or 3 substituents independently selected from the group consisting of C₁-C₄-alkyl, or C₆-C₁₂-aryl optionally substituted with 1, 2, or 3 substituents independently selected from the group consisting of halogen, C₁-C₄-alkyl, halogenated C₁-C₄-alkyl, hydroxy-(halogenated C₁-C₄-alkyl), CN, C₆-C₁₂-aryl, C₁-C₄-alkoxy, halogenated C₁-C₄-alkoxy, C₆-C₁₂ aryl-C₁-C₄-alkoxy, C₆-C₁₂-aryloxy, C₁-C₄-alkylsulfonyl, C₁-C₄-alkyl-carbonylamino and C₃-C₁₂-heterocyclyl, or C₁-C₆-alkoxy, C₁-C₆-alkoxy-C₁-C₄-alkoxy, C₆-C₁₂-aryloxy optionally substituted with 1, 2, or 3 substituents independently selected from the group consisting of halogen and C₁-C₄-alkyl, or C₃-C₁₂-heterocyclyl optionally substituted with 1, 2, or 3 substituents independently selected from the group consisting of halogen, C₁-C₄-alkyl, halogenated C₁-C₄-alkyl, C₆-C₁₂-aryl-C₁-C₆-alkyl, C₃-C₆-cycloalkyl, hydroxy, CN, C₆-C₁₂-aryl optionally substituted with halogen and C₁-C₄-alkyl, C₁-C₄-alkoxy, halogenated C₁-C₄-alkoxy, C₃-C₆-cycloalkoxy, C₆-C₁₂ aryl-C₁-C₄-alkoxy, C₃-C₁₂-heterocyclyl-C₁-C₄-alkoxy and C₃-C₁₂-heterocyclyl, or tri-(C₁-C₄-alkyl)-silyloxy; and
   - R^{5a}, R^{5b}: are hydrogen.
E141. The compounds of any one of embodiments 114-140, wherein R¹ is 1-methyl-1,3-diazol-4-yl.
E142. The compounds of any one of embodiments 114-140, wherein R¹ is 1-methyl-1,2,3-triazol-4-yl.
E143. The compounds of any one of embodiments 114-142, wherein R^{3a} is phenyl or 4-F-phenyl.
E144. The compounds of any one of embodiments 114-142, wherein R^{3a} is tetrahydrofuran-2-yl or tetrahydropyran-2-yl.
E145. The compounds of any one of embodiments 114-144, wherein R⁶ is hydrogen, methyl, or hydroxymethyl.
E146. The compounds of any one of embodiments 114-145, wherein R¹³ is a group of the formula (Id1): 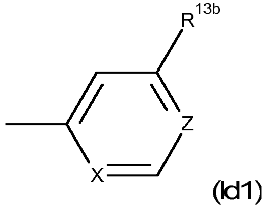 wherein
   - X: is >CH- or >N-;
   - Z: is >C-R^{13c} or >N-;
   - R^{13b}: is halogen, C₁-C₄-alkyl, halogenated C₁-C₄-alkyl, hydroxy-(halogenated C₁-C₄-alkyl), C₆-C₁₂-aryl-C₁-C₄-alkyl, C₃-C₆-cycloalkyl, CN, C₆-C₁₂-aryl optionally substituted with halogen or C₁-C₄-alkyl, C₁-C₄-alkoxy, halogenated C₁-C₄-alkoxy, C₃-C₆-cycloalkoxy, C₆-C₁₂ aryl-C₁-C₄-alkoxy, C₃-C₁₂-heterocyclyl-C₁-C₄-alkoxy, C₆-C₁₂-aryloxy, C₁-C₄-alkyl-sulfonyl, C₁-C₄-alkyl-carbonylamino or C₃-C₁₂-heterocyclyl; and
   - R^{13c}: is hydrogen or halogen.
E147. The compounds of embodiment 146, wherein R^{8a} is hydrogen.
E148. The compounds of embodiment 146 or 147, wherein n4 is 0.
E149. The compounds of embodiment 146 or 147, wherein n4 is 1.
E150. The compounds of embodiment 149, wherein R^{9a} and R^{9b} are both hydrogen.
E151. The compounds of any one of embodiments 114-150, wherein R⁶ and R^{8a} together are-C(O)OCH₂-.
E152. The compounds of any one of embodiments 1-34, having formula 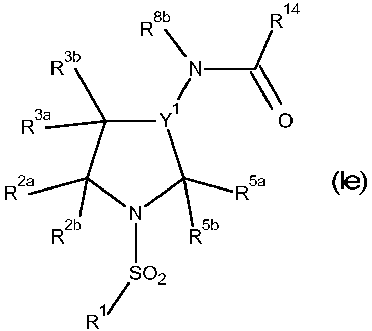 wh
   erein R¹, R^{2a}, R^{2b}, R^{3a}, R^{3b}, Y¹, R^{5a} and R^{5b} are as defined in any one of embodiments 1-34, and
   R^{8b} is hydrogen, C₁-C₆-alkyl, or C₁-C₆₋alkylcarbonyl, or
   R⁶, R^{8b}
      together are optionally substituted C₁-C₅-alkylene, wherein one or more -CH₂- of C₁-C₅-alkylene may be independently replaced by a an oxygen atom or C=O; and
   R¹⁴ is C₁-C₈-alkyl, halogenated C₁-C₆-alkyl, (optionally substituted C₃-C₁₂-cycloalkyl)-C₁-C₄-alkyl, (optionally substituted C₆-C₁₂-aryl)-C₁-C₄-alkyl, hydroxy-C₁-C₆-alkyl, C₁-C₆-alkoxy-C₁-C₆-alkyl, (optionally substituted C₆-C₁₂-aryloxy)-C₁-C₄-alkyl, C₁-C₆-alkylcarbonyl-C₁-C₄-alkyl, C₁-C₆-alkoxycarbonyl-C₁-C₄-alkyl, C₁-C₆-alkylaminocarbonyl-C₁-C₄-alkyl, optionally substituted (C₃-C₁₂-heterocyclyl)-C₁-C₄-alkyl, optionally substituted C₃-C₁₂-cycloalkyl, optionally substituted C₆-C₁₂-aryl, or optionally substituted C₃-C₁₂-heterocyclyl.
E153. The compounds of embodiment 152, wherein R^{2a}, R^{2b} are hydrogen.
E154. The compounds of embodiment 152 or 153, wherein R^{3a} is optionally substituted C₆-C₁₂-aryl or C₃-C₁₂-heterocyclyl.
E155. The compounds of any one of embodiments 152-154, wherein R^{3b} is hydrogen or hydroxy.
E156. The compounds of any one of embodiments 152-155, wherein R^{3b} is hydrogen.
E157. The compounds of any one of embodiments 152-156, wherein Y¹ is >CR⁶.
E158. The compounds of any one of embodiments 152-157, wherein R⁶ is hydrogen.
E159. The compounds of any one of embodiments 152-158, wherein R^{8b} is hydrogen.
E160. The compounds of any one of embodiments 152-159, wherein R¹⁴ is C₁-C₈-alkyl, halogenated C₁-C₆-alkyl, C₃-C₁₂-cycloalkyl-C₁-C₄-alkyl, hydroxy-C₁-C₆-alkyl, (optionally substituted C₆-C₁₂-aryloxy)-C₁-C₄-alkyl, C₁-C₆-alkylcarbonyl-C₁-C₄-alkyl, C₁-C₆-alkoxycarbonyl-C₁-C₄-alkyl, optionally substituted C₃-C₁₂-cycloalkyl, optionally substituted C₆-C₁₂-aryl, or optionally substituted C₃-C₁₂-heterocyclyl.
E161. The compounds of any one of embodiments 152-160, wherein R^{5a}, R^{5b} are hydrogen.
E162. The compounds of embodiment 152, wherein
   - R¹: is an optionally substituted 5-membered heterocyclic ring containing 2 or 3 N heteroatoms;
   - R^{2a},R^{2b}: are hydrogen;
   - R^{3a}: is optionally substituted C₆-C₁₂-aryl or C₃-C₁₂-heterocyclyl;
   - R^{3b}: is hydrogen;
   - Y¹: is >CR⁶;
   - R⁶: is hydrogen;
   - R^{8b}: is hydrogen;
   - R¹⁴: is C₁-C₈-alkyl, halogenated C₁-C₆-alkyl, C₃-C₁₂-cycloalkyl-C₁-C₄-alkyl, hydroxy-C₁-C₆-alkyl, (optionally substituted C₆-C₁₂-aryloxy)-C₁-C₄-alkyl, C₁-C₆-alkylcarbonyl-C₁-C₄-alkyl, C₁-C₆-alkoxycarbonyl-C₁-C₄-alkyl, optionally substituted C₃-C₁₂-cycloalkyl, optionally substituted C₆-C₁₂-aryl, or optionally substituted C₃-C₁₂-heterocyclyl; and
   - R^{5a}, R^{5b}: are hydrogen.
E163. The compounds of any one of embodiments 152-162, wherein R¹ is an optionally substituted 5-membered heterocyclic ring containing 2 or 3 N.
E164. The compounds of any one of embodiments 152-163, wherein R¹ is an optionally substituted 5-membered heterocyclic ring containing 2 N.
E165. The compounds of any one of embodiments 152-164, wherein R¹ is optionally substituted 1,3-diazolyl.
E166. The compounds of any one of embodiments 152-165, wherein R¹ is 1,3-diazolyl optionally substituted with halogen or C₁-C₄-alkyl.
E167. The compounds of any one of embodiments 152-166, wherein R^{3a} is C₆-C₁₂-aryl optionally substituted with 1, 2, or 3 substituents independently selected from the group consisting of halogen, C₁-C₄-alkyl and C₁-C₄-alkoxy.
E168. The compounds of any one of embodiments 152-167, wherein R¹⁴ is C₆-C₁₂-aryloxy-C₁-C₄-alkyl, with C₆-C₁₂-aryloxy being optionally substituted with 1, 2, or 3 substituents independently selected from the group consisting of halogen.
E169. The compounds of any one of embodiments 152-167, wherein R¹⁴ is C₃-C₁₂-cycloalkyl optionally substituted with 1, 2, or 3 substituents independently selected from the group consisting of halogenated C₁-C₄-alkyl.
E170. The compounds of any one of embodiments 152-167, wherein R¹⁴ is C₆-C₁₂-aryl optionally substituted with 1, 2, or 3 substituents independently selected from the group consisting of halogen, C₁-C₄-alkyl, halogenated C₁-C₄-alkyl, CN and C₁-C₄-alkoxy.
E171. The compounds of any one of embodiments 152-167, wherein R¹⁴ is C₆-C₁₂-aryl optionally substituted with 1, 2, or 3 substituents independently selected from the group consisting of C₁-C₄-alkyl and CN.
E172. The compounds of any one of embodiments 152-167, wherein R¹⁴ is C₃-C₁₂-heterocyclyl optionally substituted with 1, 2, or 3 substituents independently selected from the group consisting of halogen and halogenated C₁-C₄-alkyl.
E173. The compounds of embodiment 152, wherein
   - R¹: is 1,3-diazolyl optionally substituted with halogen or C₁-C₄-alkyl;
   - R^{2a}, R^{2b}: are hydrogen;
   - R^{3a}: is C₆-C₁₂-aryl optionally substituted with 1, 2, or 3 substituents independently selected from the group consisting of halogen, C₁-C₄-alkyl and C₁-C₄-alkoxy, or C₃-C₁₂-heterocyclyl;
   - R^{3b}: is hydrogen;
   - Y¹: is >CR⁶;
   - R⁶: is hydrogen;
   - R^{8b}: is hydrogen;
   - R¹⁴: is C₁-C₈-alkyl, halogenated C₁-C₆-alkyl, C₃-C₁₂-cycloalkyl-C₁-C₄-alkyl, hydroxy-C₁-C₆-alkyl, (halogenated C₆-C₁₂-aryloxy)-C₁-C₄-alkyl, C₁-C₆-alkylcarbonyl-C₁-C₄-alkyl, C₁-C₆-alkoxycarbonyl-C₁-C₄-alkyl, C₃-C₁₂-cycloalkyl optionally substituted with 1, 2, or 3 substituents independently selected from the group consisting of halogenated C₁-C₄-alkyl, or C₆-C₁₂-aryl optionally substituted with 1, 2, or 3 substituents independently selected from the group consisting of halogen, C₁-C₄-alkyl, halogenated C₁-C₄-alkyl, CN and C₁-C₄-alkoxy, or C₃-C₁₂-heterocyclyl optionally substituted with 1, 2, or 3 substituents independently selected from the group consisting of halogen and halogenated C₁-C₄-alkyl; and
   - R^{5a}, R^{5b}: are hydrogen.
E174. The compounds of any one of embodiments 152-173, wherein R¹ is 1-methyl-1,3-diazol-4-yl.
E175. The compounds of any one of embodiments 152-174, wherein R^{3a} is phenyl, halogenated phenyl, 4-OMe-phenyl, or pyrid-2-yl.
E176. The compounds of any one of embodiments 1-34, having formula 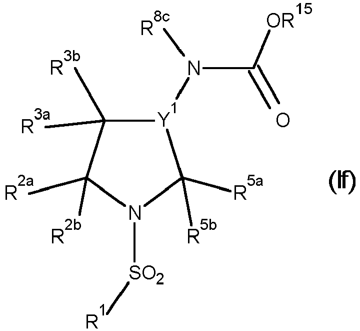
   wherein R¹, R^{2a}, R^{2b}, R^{3a}, R^{3b}, Y¹, R^{5a} and R^{5b} are as defined in any one of embodiments 1-34, and
   R^{8c} is hydrogen, C₁-C₆-alkyl, or C₁-C₆₋alkylcarbonyl, or
   R⁶, R^{8c}
      together are optionally substituted C₁-C₅-alkylene, wherein one or more -CH₂- of C₁-C₅-alkylene may be independently replaced by a an oxygen atom or C=O;
   R¹⁵ is C₁-C₈-alkyl, optionally substituted C₆-C₁₂-aryl, or optionally substituted C₃-C₁₂-heterocyclyl.
E177. The compounds of embodiment 176, wherein R^{2a}, R^{2b} are hydrogen.
E178. The compounds of embodiment 176 or 177, wherein R^{3a} is optionally substituted C₆-C₁₂-aryl.
E179. The compounds of any one of embodiments 176-178, wherein R^{3b} is hydrogen.
E180. The compounds of any one of embodiments 176-179, wherein Y¹ is >CR⁶.
E181. The compounds of any one of embodiments 176-180, wherein R⁶ is hydrogen.
E182. The compounds of any one of embodiments 176-181, wherein R^{8c} is hydrogen.
E183. The compounds of any one of embodiments 176-182, wherein R¹⁵ is C₁-C₆-alkyl or C₆-C₁₂-aryl.
E184. The compounds of any one of embodiments 176-183, wherein R^{5a}, R^{5b} are hydrogen.
E185. The compounds of embodiment 176, wherein
   - R¹: is an optionally substituted 5-membered heterocyclic ring containing 2 or 3 N heteroatoms;
   - R^{2a}, R^{2b}: are hydrogen;
   - R^{3a}: is optionally substituted C₆-C₁₂-aryl;
   - R^{3b}: is hydrogen;
   - Y¹: is >CR⁶;
   - R⁶: is hydrogen;
   - R^{8c}: is hydrogen;
   - R¹⁵: is C₁-C₆-alkyl or C₆-C₁₂-aryl; and
   - R^{5a}, R^{5b}: are hydrogen.
E186. The compounds of any one of embodiments 176-185, wherein R¹ is an optionally substituted 5-membered heterocyclic ring containing 2 or 3 N.
E187. The compounds of any one of embodiments 176-186, wherein R¹ is an optionally substituted 5-membered heterocyclic ring containing 2 N.
E188. The compounds of any one of embodiments 176-187, wherein R¹ is optionally substituted 1,3-diazolyl.
E189. The compounds of any one of embodiments 176-188, wherein R¹ is 1,3-diazolyl optionally substituted with halogen or C₁-C₄-alkyl.
E190. The compounds of any one of embodiments 176-189, wherein R^{3a} is C₆-C₁₂-aryl optionally substituted with 1, 2, or 3 substituents independently selected from the group consisting of halogen and C₁-C₄-alkyl.
E191. The compounds of embodiment 176, wherein
   - R¹: is 1-methyl-1,3-diazol-4-yl;
   - R^{2a}, R^{2b}: are hydrogen;
   - R^{3a}: is C₆-C₁₂-aryl optionally substituted with 1, 2, or 3 substituents independently selected from the group consisting of halogen and C₁-C₄-alkyl;
   - R^{3b}: is hydrogen;
   - Y¹: is >CR⁶;
   - R⁶: is hydrogen;
   - R^{8c}: is hydrogen;
   - R¹⁵: is C₁-C₈-alkyl or C₆-C₁₂-aryl; and
   - R^{5a}, R^{5b}: are hydrogen;
E192. The compounds of any one of embodiments 176-191, wherein R¹ is 1-methyl-1,3-diazol-4-yl.
E193. The compounds of any one of embodiments 176-192, wherein R^{3a} is phenyl or 4-F-phenyl.
E194. The compounds of any one of embodiments 1-34, having formula 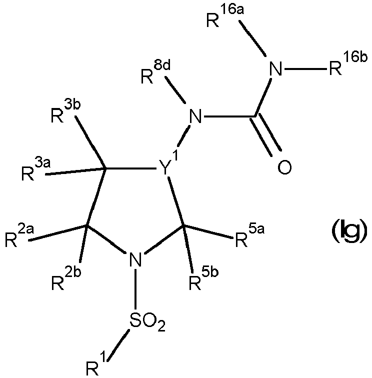
   wherein R¹, R^{2a}, R^{2b}, R^{3a}, R^{3b}, Y¹, R^{5a} and R^{5b} are as defined in any one of embodiments 1-34, and
   R^{8d} is hydrogen, C₁-C₆-alkyl, or C₁-C₆₋alkylcarbonyl, or
   R⁶, R^{8d}
      together are optionally substituted C₁-C₅-alkylene, wherein one or more -CH₂- of C₁-C₅-alkylene may be independently replaced by a an oxygen atom or C=O;
   R^{16a} is (optionally substituted C₆-C₁₂-aryl)-C₁-C₄-alkyl, optionally substituted (C₃-C₁₂-heterocyclyl)-C₁-C₄-alkyl, optionally substituted C₆-C₁₂-aryl, or optionally substituted C₃-C₁₂-heterocyclyl; and
   R^{16b} is hydrogen or C₁-C₆-alkyl.
E195. The compounds of embodiment 194, wherein R^{2a}, R^{2b} are hydrogen.
E196. The compounds of embodiment 194 or 195, wherein R^{3a} is optionally substituted C₆-C₁₂-aryl.
E197. The compounds of any one of embodiments 194-196, wherein R^{3b} is hydrogen.
E198. The compounds of any one of embodiments 194-197, wherein Y¹ is >CR⁶.
E199. The compounds of any one of embodiments 194-198, wherein R⁶ is hydrogen.
E200. The compounds of any one of embodiments 194-199, wherein R^{8d} is hydrogen.
E201. The compounds of any one of embodiments 194-200, wherein R^{16a} is (optionally substituted C₆-C₁₂-aryl)-C₁-C₄-alkyl or optionally substituted C₆-C₁₂-aryl.
E202. The compounds of any one of embodiments 194-201, wherein R^{16b} is hydrogen.
E203. The compounds of any one of embodiments 194-202, wherein R^{5a}, R^{5b} are hydrogen.
E204. The compounds of embodiment 194, wherein
   - R¹: is an optionally substituted 5-membered heterocyclic ring containing 2 or 3 N heteroatoms;
   - R^{2a}, R^{2b}: are hydrogen;
   - R^{3a}: is optionally substituted C₆-C₁₂-aryl;
   - R^{3b}: is hydrogen;
   - Y¹: is >CR⁶;
   - R⁶: is hydrogen;
   - R^{8d}: is hydrogen;
   - R^{16a}: is (optionally substituted C₆-C₁₂-aryl)-C₁-C₄-alkyl or optionally substituted C₆-C₁₂-aryl;
   - R^{16b}: is hydrogen; and
   - R^{5a}, R^{5b}: are hydrogen.
E205. The compounds of any one of embodiments 194-204, wherein R¹ is an optionally substituted 5-membered heterocyclic ring containing 2 or 3 N.
E206. The compounds of any one of embodiments 194-205, wherein R¹ is an optionally substituted 5-membered heterocyclic ring containing 2 N.
E207. The compounds of any one of embodiments 194-206, wherein R¹ is optionally substituted 1,3-diazolyl.
E208. The compounds of any one of embodiments 194-207, wherein R¹ is 1,3-diazolyl optionally substituted with halogen or C₁-C₄-alkyl.
E209. The compounds of any one of embodiments 194-208, wherein R^{3a} is C₆-C₁₂-aryl optionally substituted with 1, 2, or 3 substituents independently selected from the group consisting of halogen and C₁-C₄-alkyl.
E210. The compounds of any one of embodiments 194-209, wherein R^{16a} is C₆-C₁₂-aryl-C₁-C₄-alkyl, with C₆-C₁₂-aryl being optionally substituted with 1, 2, or 3 substituents independently selected from the group consisting of halogen.
E211. The compounds of any one of embodiments 194-209, wherein R^{16a} is C₆-C₁₂-aryl optionally substituted with 1, 2, or 3 substituents independently selected from the group consisting of halogen.
E212. The compounds of embodiment 194, wherein
   - R¹: is 1,3-diazolyl optionally substituted with halogen or C₁-C₄-alkyl;
   - R^{2a}, R^{2b}: are hydrogen;
   - R^{3a}: is C₆-C₁₂-aryl optionally substituted with 1, 2, or 3 substituents independently selected from the group consisting of halogen and C₁-C₄-alkyl;
   - R^{3b}: is hydrogen;
   - Y¹: is >CR⁶;
   - R⁶: is hydrogen;
   - R^{8d}: is hydrogen;
   - R^{16a}: is C₆-C₁₂-aryl-C₁-C₄-alkyl or C₆-C₁₂-aryl, with C₆-C₁₂-aryl being optionally substituted with 1, 2, or 3 substituents independently selected from the group consisting of halogen;
   - R^{16b}: is hydrogen; and
   - R^{5a}, R^{5b}: are hydrogen.
E213. The compounds of any one of embodiments 194-212, wherein R¹ is 1-methyl-1,3-diazol-4-yl.
E214. The compounds of any one of embodiments 194-213, wherein R^{3a} is 4-F-phenyl.
E215. The compounds of any one of embodiments 1-34, having formula 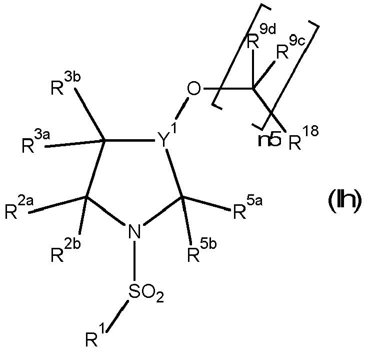
   wherein R¹, R^{2a}, R^{2b}, R^{3a}, R^{3b}, Y¹, R^{5a} and R^{5b} are as defined in any one of embodiments 1-34, and
   R^{9c}, R^{9d}
      are independently hydrogen, halogen, or C₁-C₆-alkyl;
   n5 is 0, 1, 2, 3, or 4; and
   R¹⁸ is hydrogen, optionally substituted C₁-C₈-alkyl, optionally substituted C₃-C₁₂-cycloalkyl, C₁-C₆₋alkylcarbonyl, C₁-C₆-alkoxycarbonyl, halogenated C₁-C₆-alkoxycarbonyl, C₆-C₁₂₋aryloxycarbonyl, C₁-C₆-alkylaminocarbonyl, (halogenated C₁-C₄-alkyl)aminocarbonyl, C₆-C₁₂₋arylaminocarbonyl, optionally substituted C₆-C₁₂-aryl, C₁-C₆-alkylamine, (C₃-C₁₂-cycloalkyl-C₁-C₄-alkyl)amino, (halogenated C₁-C₆-alkyl)amino, (C₁-C₆-alkoxy- C₁-C₆-alkyl)amino, (C₆-C₁₂-aryl-C₁-C₄-alkyl)amino, C₁-C₆-dialkylamine, optionally substituted C₆-C₁₂-arylamine, or optionally substituted C₃-C₁₂-heterocyclyl.
E216. The compounds of embodiment 215, wherein R^{2a}, R^{2b} are hydrogen.
E217. The compounds of embodiment 215 or 216, wherein R^{3a} is optionally substituted C₆-C₁₂-aryl or optionally substituted C₃-C₁₂-heterocyclyl.
E218. The compounds of any one of embodiments 215-217, wherein R^{3b} is hydrogen.
E219. The compounds of any one of embodiments 215-218, wherein Y¹ is >CR⁶.
E220. The compounds of any one of embodiments 215-219, wherein R⁶ is hydrogen or C₆-C₁₂-aryl-C₁-C₄-alkyl.
E221. The compounds of any one of embodiments 215-220, wherein R⁶ is hydrogen or benzyl.
E222. The compounds of any one of embodiments 215-221, wherein R⁶ is hydrogen.
E223. The compounds of any one of embodiments 215-222, wherein n5 is 0, 1, or 2.
E224. The compounds of any one of embodiments 215-223, wherein R^{9c} and R^{9d} are hydrogen.
E225. The compounds of any one of embodiments 215-224, wherein R¹⁸ is hydrogen, C₁-C₈-alkyl, C₁-C₆-alkoxycarbonyl, C₁-C₆-alkylaminocarbonyl, (halogenated C₁-C₄-alkyl)aminocarbonyl, optionally substituted C₆-C₁₂-aryl, C₁-C₆-alkylamine, (halogenated C₁-C₆-alkyl)amino, optionally substituted C₆-C₁₂-arylamine, or optionally substituted C₃-C₁₂-heterocyclyl.
E226. The compounds of any one of embodiments 215-225, wherein R^{5a}, R^{5b} are hydrogen.
E227. The compounds of embodiment 215, wherein
   - R¹: is an optionally substituted 5-membered heterocyclic ring containing 2 or 3 N heteroatoms;
   - R^{2a}, R^{2b}: are hydrogen;
   - R^{3a}: is optionally substituted C₆-C₁₂-aryl or optionally substituted C₃-C₁₂-heterocyclyl;
   - R^{3b}: is hydrogen;
   - Y¹: is >CR⁶;
   - R⁶: is hydrogen;
   - R^{9c}, R^{9d}: are hydrogen;
   - n5: is 0, 1, or 2;
   - R¹⁸: is hydrogen, C₁-C₈-alkyl, C₁-C₆-alkoxycarbonyl, C₁-C₆-alkylaminocarbonyl, (halogenated C₁-C₄-alkyl)aminocarbonyl, optionally substituted C₆-C₁₂-aryl, C₁-C₆-alkylamine, (halogenated C₁-C₆-alkyl)amino, optionally substituted C₆-C₁₂-arylamine, or optionally substituted C₃-C₁₂-heterocyclyl; and
   - R^{5a}, R^{5b}: are hydrogen.
E228. The compounds of any one of embodiments 215-227, wherein R¹ is an optionally substituted 5-membered heterocyclic ring containing 2 or 3 N.
E229. The compounds of any one of embodiments 215-228, wherein R¹ is an optionally substituted 5-membered heterocyclic ring containing 2 N.
E230. The compounds of any one of embodiments 215-229, wherein R¹ is optionally substituted 1,3-diazolyl.
E231. The compounds of any one of embodiments 215-230, wherein R¹ is 1,3-diazolyl optionally substituted with halogen or C₁-C₄-alkyl.
E232. The compounds of any one of embodiments 215-231, wherein R^{3a} is C₆-C₁₂-aryl optionally substituted with 1, 2, or 3 substituents independently selected from the group consisting of halogen and C₁-C₄-alkyl, or C₃-C₁₂-heterocyclyl optionally substituted with 1, 2, or 3 substituents independently selected from the group consisting of halogen and C₁-C₄-alkyl.
E233. The compounds of any one of embodiments 215-232, wherein R¹⁸ is C₆-C₁₂-aryl optionally substituted with 1, 2, or 3 substituents independently selected from the group consisting of halogen, C₁-C₄-alkyl and C₁-C₄-alkyl-sulfonyl.
E234. The compounds of any one of embodiments 215-232, wherein R¹⁸ is C₆-C₁₂-arylamine optionally substituted with 1, 2, or 3 substituents independently selected from the group consisting of halogen.
E235. The compounds of any one of embodiments 215-232, wherein R¹⁸ is C₃-C₁₂-heterocyclyl optionally substituted with 1, 2, or 3 substituents independently selected from the group consisting of halogenated C₁-C₄-alkyl.
E236. The compounds of embodiment 215, wherein
   - R¹: is 1,3-diazolyl optionally substituted with halogen or C₁-C₄-alkyl;
   - R^{2a}, R^{2b}: are hydrogen;
   - R^{3a}: is C₆-C₁₂-aryl optionally substituted with 1, 2, or 3 substituents independently selected from the group consisting of halogen and C₁-C₄-alkyl, or C₃-C₁₂-heterocyclyl optionally substituted with 1, 2, or 3 substituents independently selected from the group consisting of halogen and C₁-C₄-alkyl;
   - R^{3b}: is hydrogen;
   - Y¹: is >CR⁶;
   - R⁶: is hydrogen;
   - R^{9c}, R^{9d}: are hydrogen;
   - n5: is 0, 1, or 2;
   - R¹⁸: is hydrogen, C₁-C₈-alkyl, C₁-C₆-alkoxycarbonyl, C₁-C₆-alkylaminocarbonyl, (halogenated C₁-C₄-alkyl)aminocarbonyl, C₆-C₁₂-aryl optionally substituted with 1, 2, or 3 substituents independently selected from the group consisting of halogen, C₁-C₄-alkyl and C₁-C₄-alkyl-sulfonyl, or C₁-C₆-alkylamine, (halogenated C₁-C₆-alkyl)amino, C₆-C₁₂-arylamine optionally substituted with 1, 2, or 3 substituents independently selected from the group consisting of halogen, or C₃-C₁₂-heterocyclyl optionally substituted with 1, 2, or 3 substituents independently selected from the group consisting of halogenated C₁-C₄-alkyl; and
   - R^{5a}, R^{5b}: are hydrogen.
E237. The compounds of any one of embodiments 215-236, wherein R¹ is 1-methyl-1,3-diazol-4-yl.
E238. The compounds of any one of embodiments 215-237, wherein R^{3a} is phenyl or 4-F-phenyl.
E239. The compounds of any one of embodiments 1-34, having formula 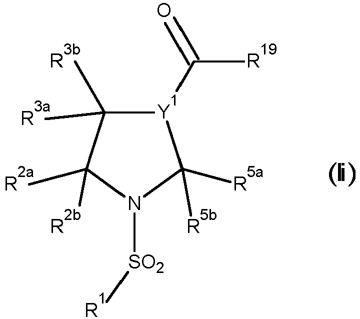
   wherein R¹, R^{2a}, R^{2b}, R^{3a}, R^{3b}, Y¹, R^{5a} and R^{5b} are as defined in any one of embodiments 1-34, and
   R¹⁹ is optionally substituted C₆-C₁₂-aryl or optionally substituted C₃-C₁₂-heterocyclyl.
E240. The compounds of embodiment 239, wherein R^{2a}, R^{2b} are hydrogen.
E241. The compounds of embodiment 239 or 240, wherein R^{3a} is optionally substituted C₆-C₁₂-aryl.
E242. The compounds of any one of embodiments 239-241, wherein R^{3b} is hydrogen.
E243. The compounds of any one of embodiments 239-242, wherein Y¹ is >CR⁶.
E244. The compounds of any one of embodiments 239-243, wherein R⁶ is hydrogen.
E245. The compounds of any one of embodiments 239-244, wherein R¹⁹ is optionally substituted C₆-C₁₂-aryl.
E246. The compounds of any one of embodiments 239-245, wherein R^{5a}, R^{5b} are hydrogen.
E247. The compounds of embodiment 239, wherein
   - R¹: is an optionally substituted 5-membered heterocyclic ring containing 2 or 3 N heteroatoms;
   - R^{2a}, R^{2b}: are hydrogen;
   - R^{3a}: is optionally substituted C₆-C₁₂-aryl;
   - R^{3b}: is hydrogen;
   - Y¹: is >CR⁶;
   - R⁶: is hydrogen;
   - R¹⁹: is optionally substituted C₆-C₁₂-aryl; and
   - R^{5a}, R^{5b}: are hydrogen.
E248. The compounds of any one of embodiments 239-247, wherein R¹ is an optionally substituted 5-membered heterocyclic ring containing 2 or 3 N.
E249. The compounds of any one of embodiments 239-248, wherein R¹ is an optionally substituted 5-membered heterocyclic ring containing 2 N.
E250. The compounds of any one of embodiments 239-249, wherein R¹ is optionally substituted 1,3-diazolyl.
E251. The compounds of any one of embodiments 239-250, wherein R¹ is 1,3-diazolyl optionally substituted with halogen or C₁-C₄-alkyl.
E252. The compounds of any one of embodiments 239-251, wherein R^{3a} is C₆-C₁₂-aryl optionally substituted with 1, 2, or 3 substituents independently selected from the group consisting of halogen and C₁-C₄-alkyl.
E253. The compounds of any one of embodiments 239-252, wherein R¹⁹ is C₆-C₁₂-aryl optionally substituted with 1, 2, or 3 substituents independently selected from the group consisting of halogen, C₁-C₄-alkyl, halogenated C₁-C₄-alkyl and halogenated C₁-C₄-alkoxy.
E254. The compounds of embodiment 239, wherein
   - R¹: is 1,3-diazolyl optionally substituted with halogen or C₁-C₄-alkyl;
   - R^{2a}, R^{2b}: are hydrogen;
   - R^{3a}: is C₆-C₁₂-aryl optionally substituted with 1, 2, or 3 substituents independently selected from the group consisting of halogen and C₁-C₄-alkyl;
   - R^{3b}: is hydrogen;
   - Y¹: is >CR⁶;
   - R⁶: is hydrogen;
   - R¹⁹: is C₆-C₁₂-aryl optionally substituted with 1, 2, or 3 substituents independently selected from the group consisting of halogen, C₁-C₄-alkyl, halogenated C₁-C₄-alkyl and halogenated C₁-C₄-alkoxy; and
   - R^{5a}, R^{5b}: are hydrogen.
E255. The compounds of any one of embodiments 239-254, wherein R¹ is 1-methyl-1,3-diazol-4-yl.
E256. The compounds of any one of embodiments 239-255, wherein R^{3a} is 4-F-phenyl.
E257. The compounds of any one of embodiments 1-34, having formula 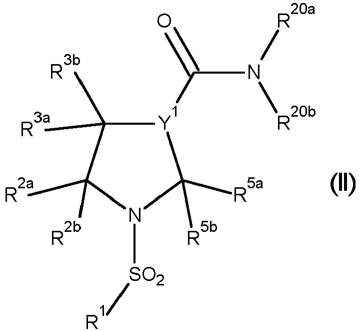
   wherein R¹, R^{2a}, R^{2b}, R^{3a}, R^{3b}, Y¹, R^{5a} and R^{5b} are as defined in any one of embodiments 1-34, and
   R^{20a} is C₁-C₈-alkyl, (optionally substituted C₃-C₁₂-cycloalkyl)-C₁-C₄-alkyl, C₁-C₆-alkoxy-C₁-C₆-alkyl, (optionally substituted C₆-C₁₂-aryl)-C₁-C₄-alkyl, (optionally substituted C₃-C₁₂-heterocyclyl)-C₁-C₄-alkyl, optionally substituted C₆-C₁₂-aryl, or optionally substituted C₃-C₁₂-heterocyclyl; and
   R^{20b} is hydrogen or C₁-C₈-alkyl.
E258. The compounds of embodiment 257, wherein R^{2a}, R^{2b} are hydrogen.
E259. The compounds of embodiment 257 or 258, wherein R^{3a} is optionally substituted C₆-C₁₂-aryl.
E260. The compounds of any one of embodiments 257-259, wherein R^{3b} is hydrogen.
E261. The compounds of any one of embodiments 257-260, wherein Y¹ is >CR⁶.
E262. The compounds of any one of embodiments 257-261, wherein R⁶ is hydrogen.
E263. The compounds of any one of embodiments 257-262, wherein R^{20a} is C₁-C₈-alkyl, C₃-C₁₂-cycloalkyl-C₁-C₄-alkyl, C₁-C₆-alkoxy-C₁-C₆-alkyl, (optionally substituted C₆-C₁₂-aryl)-C₁-C₄-alkyl, (optionally substituted C₃-C₁₂-heterocyclyl)-C₁-C₄-alkyl, optionally substituted C₆-C₁₂-aryl, or optionally substituted C₃-C₁₂-heterocyclyl.
E264. The compounds of any one of embodiments 257-263, wherein R^{5a}, R^{5b} are hydrogen.
E265. The compounds of embodiment 257, wherein
   - R¹: is an optionally substituted 5-membered heterocyclic ring containing 2 or 3 N heteroatoms;
   - R^{2a}, R^{2b}: are hydrogen;
   - R^{3a}: is optionally substituted C₆-C₁₂-aryl;
   - R^{3b}: is hydrogen;
   - Y¹: is >CR⁶;
   - R⁶: is hydrogen;
   - R^{20a}: is C₁-C₈-alkyl, C₃-C₁₂-cycloalkyl-C₁-C₄-alkyl, C₁-C₆-alkoxy-C₁-C₆-alkyl, (optionally substituted C₆-C₁₂-aryl)-C₁-C₄-alkyl, (optionally substituted C₃-C₁₂-heterocyclyl)-C₁-C₄-alkyl, optionally substituted C₆-C₁₂-aryl, or optionally substituted C₃-C₁₂-heterocyclyl;
   - R^{20b}: is hydrogen or C₁-C₈-alkyl; and
   - R^{5a}, R^{5b}: are hydrogen.
E266. The compounds of any one of embodiments 257-265, wherein R¹ is an optionally substituted 5-membered heterocyclic ring containing 2 or 3 N.
E267. The compounds of any one of embodiments 257-266, wherein R¹ is an optionally substituted 5-membered heterocyclic ring containing 2 N.
E268. The compounds of any one of embodiments 257-267, wherein R¹ is optionally substituted 1,3-diazolyl.
E269. The compounds of any one of embodiments 257-268, wherein R¹ is 1,3-diazolyl optionally substituted with halogen or C₁-C₄-alkyl.
E270. The compounds of any one of embodiments 257-269, wherein R^{3a} is C₆-C₁₂-aryl optionally substituted with 1, 2, or 3 substituents independently selected from the group consisting of halogen and C₁-C₄-alkyl.
E271. The compounds of any one of embodiments 257-270, wherein R^{20a} is C₆-C₁₂-aryl-C₁-C₄-alkyl, with C₆-C₁₂-aryl being optionally substituted with 1, 2, or 3 substituents independently selected from the group consisting of halogen, halogenated C₁-C₄-alkyl and halogenated C₁-C₄-alkoxy.
E272. The compounds of any one of embodiments 257-270, wherein R^{20a} is C₃-C₁₂-heterocyclyl-C₁-C₄-alkyl, with C₃-C₁₂-heterocyclyl being optionally substituted with 1, 2, or 3 substituents independently selected from the group consisting of halogenated C₁-C₄-alkyl.
E273. The compounds of any one of embodiments 257-270, wherein R^{20a} is C₆-C₁₂-aryl optionally substituted with 1, 2, or 3 substituents independently selected from the group consisting of halogen, C₁-C₄-alkyl, halogenated C₁-C₄-alkyl, CN and halogenated C₁-C₄-alkoxy.
E274. The compounds of any one of embodiments 257-270, wherein R^{20a} is C₃-C₁₂-heterocyclyl optionally substituted with 1, 2, or 3 substituents independently selected from the group consisting of halogenated C₁-C₄-alkyl.
E275. The compounds of embodiment 257, wherein
   - R¹: is 1,3-diazolyl optionally substituted with halogen or C₁-C₄-alkyl;
   - R^{2a}, R^{2b}: are hydrogen;
   - R^{3a}: is C₆-C₁₂-aryl optionally substituted with 1, 2, or 3 substituents independently selected from the group consisting of halogen and C₁-C₄-alkyl;
   - R^{3b}: is hydrogen;
   - Y¹: is >CR⁶;
   - R⁶: is hydrogen;
   - R^{20a}: is C₁-C₈-alkyl, C₃-C₁₂-cycloalkyl-C₁-C₄-alkyl, C₁-C₆-alkoxy-C₁-C₆-alkyl, C₆-C₁₂-aryl-C₁-C₄-alkyl and C₆-C₁₂-aryl is optionally substituted with 1, 2, or 3 substituents independently selected from the group consisting of halogen, halogenated C₁-C₄-alkyl and halogenated C₁-C₄-alkoxy, or C₃-C₁₂-heterocyclyl-C₁-C₄-alkyl and C₃-C₁₂-heterocyclyl is optionally substituted with 1, 2, or 3 substituents independently selected from the group consisting of halogenated C₁-C₄-alkyl, or C₆-C₁₂-aryl optionally substituted with 1, 2, or 3 substituents independently selected from the group consisting of halogen, C₁-C₄-alkyl, halogenated C₁-C₄-alkyl, CN and halogenated C₁-C₄-alkoxy, or C₃-C₁₂-heterocyclyl optionally substituted with 1, 2, or 3 substituents independently selected from the group consisting of halogenated C₁-C₄-alkyl;
   - R^{20b}: is hydrogen or C₁-C₈-alkyl; and
   - R^{5a}, R^{5b}: are hydrogen.
E276. The compounds of any one of embodiments 257-275, wherein R¹ is 1-methyl-1,3-diazol-4-yl.
E277. The compounds of any one of embodiments 257-276, wherein R^{3a} is 4-F-phenyl.
E278. The compounds of any one of embodiments 257-277, wherein R^{20b} is hydrogen or butyl.
E279. The compounds of any one of embodiments 1-34, having formula 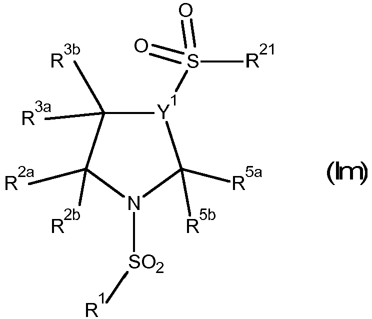 wh
   erein R¹, R^{2a}, R^{2b}, R^{3a}, R^{3b}, Y¹, R^{5a} and R^{5b} are as defined in any one of embodiments 1-34, and
   R²¹ is optionally substituted C₆-C₁₂-aryl, or optionally substituted C₃-C₁₂-heterocyclyl. E280. The compounds of embodiment 279, wherein R^{2a}, R^{2b} are hydrogen. E281. The compounds of embodiment 279 or 280, wherein R^{3a} is optionally substituted C₆-C₁₂-aryl.
E282. The compounds of any one of embodiments 279-281, wherein R^{3b} is hydrogen.
E283. The compounds of any one of embodiments 279-282, wherein Y¹ is >CR⁶.
E284. The compounds of any one of embodiments 279-283, wherein R⁶ is hydrogen.
E285. The compounds of any one of embodiments 279-284, wherein R²¹ is C₆-C₁₂-aryl.
E286. The compounds of any one of embodiments 279-285, wherein R^{5a}, R^{5b} are hydrogen.
E287. The compounds of embodiment 279, wherein
   - R¹: is an optionally substituted 5-membered heterocyclic ring containing 2 or 3 N heteroatoms;
   - R^{2a}, R^{2b}: are hydrogen;
   - R^{3a}: is optionally substituted C₆-C₁₂-aryl;
   - R^{3b}: is hydrogen;
   - Y¹: is >CR⁶;
   - R⁶: is hydrogen;
   - R²¹: is C₆-C₁₂-aryl; and
   - R^{5a}, R^{5b}: are hydrogen.
E288. The compounds of any one of embodiments 279-287, wherein R¹ is an optionally substituted 5-membered heterocyclic ring containing 2 or 3 N.
E289. The compounds of any one of embodiments 279-288, wherein R¹ is an optionally substituted 5-membered heterocyclic ring containing 2 N.
E290. The compounds of any one of embodiments 279-289, wherein R¹ is optionally substituted 1,3-diazolyl.
E291. The compounds of any one of embodiments 279-290, wherein R¹ is 1,3-diazolyl optionally substituted with halogen or C₁-C₄-alkyl.
E292. The compounds of any one of embodiments 279-291, wherein R^{3a} is C₆-C₁₂-aryl.
E293. The compounds of embodiment 279, wherein
   - R¹: is 1,3-diazolyl optionally substituted with halogen or C₁-C₄-alkyl;
   - R^{2a}, R^{2b}: are hydrogen;
   - R^{3a}: is C₆-C₁₂-aryl;
   - R^{3b}: is hydrogen;
   - Y¹: is >CR⁶;
   - R⁶: is hydrogen;
   - R²¹: is C₆-C₁₂-aryl; and
   - R^{5a}, R^{5b}: are hydrogen.
E294. The compounds of any one of embodiments 279-293, wherein R¹ is 1-methyl-1,3-diazol-4-yl.
E295. The compounds of any one of embodiments 279-294, wherein R^{3a} is phenyl.
E296. The compounds of any one of embodiments 279-295, wherein R²¹ is phenyl.
E297. The compounds of any one of embodiments 1-34, wherein R⁴ is optionally substituted C₃-C₁₂-heterocyclyl.
E298. The compounds of embodiment 297, wherein R^{2a}, R^{2b} are hydrogen.
E299. The compounds of embodiment 297 or 298, wherein R^{3a} is optionally substituted C₆-C₁₂-aryl or C₃-C₁₂-heterocyclyl.
E300. The compounds of any one of embodiments 297-299, wherein R^{3b} is hydrogen.
E301. The compounds of any one of embodiments 297-300, wherein Y¹ is >CR⁶.
E302. The compounds of any one of embodiments 297-301, wherein R⁶ is hydrogen.
E303. The compounds of any one of embodiments 297-302, wherein R^{5a}, R^{5b} are hydrogen.
E304. The compounds of embodiment 297, wherein
   - R¹: is an optionally substituted 5-membered heterocyclic ring containing 2 or 3 N heteroatoms;
   - R^{2a}, R^{2b}: are hydrogen;
   - R^{3a}: is optionally substituted C₆-C₁₂-aryl or C₃-C₁₂-heterocyclyl;
   - R^{3b}: is hydrogen;
   - R⁴: is optionally substituted C₃-C₁₂-heterocyclyl;
   - Y¹: is >CR⁶;
   - R⁶: is hydrogen; and
   - R^{5a}, R^{5b}: are hydrogen.
E305. The compounds of any one of embodiments 297-304, wherein R¹ is an optionally substituted 5-membered heterocyclic ring containing 2 or 3 N.
E306. The compounds of any one of embodiments 297-305, wherein R¹ is an optionally substituted 5-membered heterocyclic ring containing 2 N.
E307. The compounds of any one of embodiments 297-306, wherein R¹ is optionally substituted 1,3-diazolyl.
E308. The compounds of any one of embodiments 297-307, wherein R¹ is 1,3-diazolyl optionally substituted with halogen or C₁-C₄-alkyl.
E309. The compounds of any one of embodiments 297-308, wherein R^{3a} is C₆-C₁₂-aryl optionally substituted with 1, 2, or 3 substituents independently selected from the group consisting of halogen and C₁-C₄-alkyl.
E310. The compounds of any one of embodiments 297-309, wherein R⁴ is a C₃-C₁₂-heterocyclyl substituted with 1, 2, or 3 substituents independently selected from the group consisting of halogen, C₁-C₄-alkyl and halogenated C₁-C₄-alkyl.
E311. The compounds of embodiment 297, wherein
   - R¹: is 1,3-diazolyl optionally substituted with halogen or C₁-C₄-alkyl;
   - R^{2a}, R^{2b}: are hydrogen;
   - R^{3a}: is C₆-C₁₂-aryl optionally substituted with 1, 2, or 3 substituents independently selected from the group consisting of halogen and C₁-C₄-alkyl, or C₃-C₁₂-heterocyclyl;
   - R^{3b}: is hydrogen;
   - R⁴: is C₃-C₁₂-heterocyclyl substituted with 1, 2, or 3 substituents independently selected from the group consisting of halogen, C₁-C₆-alkyl and halogenated C₁-C₄-alkyl;
   - Y¹: is >CR⁶;
   - R⁶: is hydrogen; and
   - R^{5a}, R^{5b}: are hydrogen.
E312. The compounds of any one of embodiments 297-311, wherein R¹ is 1-methyl-1,3-diazol-4-yl.
E313. The compounds of any one of embodiments 297-311, wherein R^{3a} is phenyl, 4-F-phenyl, or pyrid-2-yl.

According to one embodiment, the present invention relates to pyrrolidine derivatives of the formula (I), wherein
- R¹: is a 5-membered heterocyclic ring selected from pyrazolyl, imidazolyl and triazolyl, which is optionally substituted with 1, 2 or 3 substituents independently selected from the group consisting of halogen, C₁-C₆-alkyl, halogenated C₁-C₆-alkyl, C₃-C₆-cycloalkyl, C₁-C₄-alkoxycarbonyl and C₁-C₆-alkylcarbonylamino;
- R^{2a}, R^{2b}: are independently hydrogen, halogen, or C₁-C₃-alkyl; or
- R^{2a}, R^{2b}: together with the carbon atom to which they are bound may form a C=O;
- R^{3a}: is C₃-C₁₂-cycloalkyl, optionally substituted C₆-C₁₂-aryl, hydroxy, C₁-C₆-alkoxy, halogenated C₁-C₆-alkoxy, C₃-C₁₂-cycloalkyl-C₁-C₄-alkoxy, C₂-C₆-alkenyloxy, C₆-C₁₂-aryl-C₁-C₄-alkoxy, C₃-C₁₂-heterocyclyl-C₁-C₄-alkoxy, optionally substituted C₆-C₁₂-aryloxy, C₃-C₁₂-heterocyclyloxy, or optionally substituted C₃-C₁₂-heterocyclyl;
- R^{3b}: is hydrogen, C₁-C₆-alkyl, or hydroxy;
- Y¹: is >CR⁶- or >N-;
- R⁶: is hydrogen, C₁-C₆-alkyl, halogenated C₁-C₆-alkyl, (optionally substituted C₆-C₁₂-aryl)-C₁-C₄-alkyl, hydroxy-C₁-C₆-alkyl, C₁-C₄-alkoxy-C₁-C₆-alkyl, or hydroxy;
- R⁴: is -(CR^{7a}R^{7b})ₙₗOR¹⁰, -(CR^{7c}R^{7d})ₙ₂NR^{11a}R^{11b}, -(CR^{7e}R^{7f})ₙ₃R¹², optionally substituted C₆-C₁₂-aryl, -NR^{8a}(CR^{9a}R^{9b})ₙ₄R¹³, -NR^{8b}COR¹⁴, -NR^{8c}COOR¹⁵, -NR^{8d}CONR^{16a}R^{16b}, -NR^{8e}SO₂R¹⁷,-O(CR^{9c}R^{9d})ₙ₅R¹⁸, -COR¹⁹, -CONR^{20a}R^{20b}, -SO₂R²¹, or optionally substituted C₃-C₁₂-heterocyclyl;
- R^{7a}, R^{7b}: are independently hydrogen or C₁-C₆-alkyl;
- n1: is 1, 2, 3, or 4;
- R¹⁰: is hydrogen, optionally substituted C₆-C₁₂-aryl, or optionally substituted C₃-C₁₂-heterocyclyl;
- R^{7c}, R^{7d}: are independently hydrogen or C₁-C₆-alkyl;
- n2: is 1, 2, 3, or 4;
- R^{11a}: is C₁-C₈-alkyl, (optionally substituted C₃-C₁₂-cycloalkyl)-C₁-C₄-alkyl, C₁-C₆-alkoxy-C₁-C₆-alkyl, (optionally substituted C₆-C₁₂-aryl)-C₁-C₄-alkyl, (optionally substituted C₃-C₁₂-heterocyclyl)-C₁-C₄-alkyl, optionally substituted C₆-C₁₂-aryl, or optionally substituted C₃-C₁₂-heterocyclyl;
- R^{11b}: is hydrogen or C₁-C₆-alkyl;
- R^{7e}, R^{7f}: are independently hydrogen or C₁-C₆-alkyl;
- n3: is 1, 2, 3, or 4;
- R¹²: is optionally substituted C₆-C₁₂-aryl or optionally substituted C₃-C₁₂-heterocyclyl;
- R^{8a}, R^{8b}, R^{8c}, R^{8d}, R^{8e}: are independently hydrogen, C₁-C₆-alkyl, or C₁-C₆₋alkylcarbonyl, or
- R⁶: and one of R^{8a}, R^{8b}, R^{8c}, R^{8d}, or R^{8e}
together are optionally substituted C₁-C₅-alkylene, wherein one or more -CH₂- of C₁-C₅-alkylene may be independently replaced by a an oxygen atom or C=O;
- R^{9a}, R^{9b}: are independently hydrogen, halogen, C₁-C₆-alkyl, hydroxy, or C₁-C₆-alkoxy;
- n4: is 0, 1, 2, 3, or 4;
- R¹³: is hydrogen, C₁-C₈-alkyl, halogenated C₁-C₆-alkyl, (optionally substituted C₃-C₁₂-cycloalkyl)-C₁-C₄-alkyl, C₁-C₆-alkoxy-C₁-C₆-alkyl, optionally substituted C₃-C₁₂-cycloalkyl, C₂-C₆-alkenyl, optionally substituted C₃-C₆-cycloalkenyl, optionally substituted C₆-C₁₂-aryl, hydroxy, C₁-C₆-alkoxy, C₁-C₆-alkoxy-C₁-C₄-alkoxy, optionally substituted C₆-C₁₂-aryloxy, optionally substituted C₃-C₁₂-heterocyclyloxy, optionally substituted C₃-C₁₂-heterocyclyl, or tri-(C₁-C₄-alkyl)-silyloxy;
- R¹⁴: is C₁-C₈-alkyl, halogenated C₁-C₆-alkyl, (optionally substituted C₃-C₁₂-cycloalkyl)-C₁-C₄-alkyl, (optionally substituted C₆-C₁₂-aryl)-C₁-C₄-alkyl, hydroxy-C₁-C₆-alkyl, C₁-C₆-alkoxy-C₁-C₆-alkyl, (optionally substituted C₆-C₁₂-aryloxy)-C₁-C₄-alkyl, C₁-C₆-alkylcarbonyl-C₁-C₄-alkyl, C₁-C₆-alkoxycarbonyl-C₁-C₄-alkyl, C₁-C₆-alkylaminocarbonyl-C₁-C₄-alkyl, optionally substituted (C₃-C₁₂-heterocyclyl)-C₁-C₄-alkyl, optionally substituted C₃-C₁₂-cycloalkyl, optionally substituted C₆-C₁₂-aryl, or optionally substituted C₃-C₁₂-heterocyclyl;
- R¹⁵: is C₁-C₈-alkyl, optionally substituted C₆-C₁₂-aryl, or optionally substituted C₃-C₁₂-heterocyclyl;
- R^{16a}: is (optionally substituted C₆-C₁₂-aryl)-C₁-C₄-alkyl, optionally substituted (C₃-C₁₂-heterocyclyl)-C₁-C₄-alkyl, optionally substituted C₆-C₁₂-aryl, or optionally substituted C₃-C₁₂-heterocyclyl;
- R^{16b}: is hydrogen or C₁-C₆-alkyl;
- R¹⁷: is (optionally substituted C₆-C₁₂-aryl)-C₁-C₄-alkyl, (optionally substituted C₃-C₁₂-heterocyclyl)-C₁-C₄-alkyl, optionally substituted C₆-C₁₂-aryl, or optionally substituted C₃-C₁₂-heterocyclyl;
- R^{9c}, R^{9d}: are independently hydrogen, halogen, or C₁-C₆-alkyl;
- n5: is 0, 1, 2, 3, or 4;
- R¹⁸: is hydrogen, C₁-C₈-alkyl, optionally substituted C₃-C₁₂-cycloalkyl, C₁-C₆₋alkylcarbonyl, C₁-C₆-alkoxycarbonyl, halogenated C₁-C₆₋alkoxycarbonyl, C₆-C₁₂₋aryloxycarbonyl, C₁-C₆-alkylaminocarbonyl, (halogenated C₁-C₄-alkyl)aminocarbonyl, C₆-C₁₂₋arylaminocarbonyl, optionally substituted C₆-C₁₂-aryl, C₁-C₆-alkylamine, (C₃-C₁₂-cycloalkyl-C₁-C₄-alkyl)amino, (halogenated C₁-C₆-alkyl)amino, (C₁-C₆-alkoxy- C₁-C₆-alkyl)amino, (C₆-C₁₂-aryl-C₁-C₄-alkyl)amino, C₁-C₆-dialkylamine, optionally substituted C₆-C₁₂-arylamine, or optionally substituted C₃-C₁₂-heterocyclyl;
- R¹⁹: is optionally substituted C₆-C₁₂-aryl or optionally substituted C₃-C₁₂-heterocyclyl;
- R^{20a}: is C₁-C₈-alkyl, (optionally substituted C₃-C₁₂-cycloalkyl)-C₁-C₄-alkyl, C₁-C₆-alkoxy-C₁-C₆-alkyl, (optionally substituted C₆-C₁₂-aryl)-C₁-C₄-alkyl, (optionally substituted C₃-C₁₂-heterocyclyl)-C₁-C₄-alkyl, optionally substituted C₆-C₁₂-aryl, or optionally substituted C₃-C₁₂-heterocyclyl;
- R^{20b}: is hydrogen or C₁-C₈-alkyl;
- R²¹: is optionally substituted C₆-C₁₂-aryl, or optionally substituted C₃-C₁₂-heterocyclyl; and
- R^{5a}, R^{5b}: are independently hydrogen, halogen, or C₁-C₃-alkyl, or
- R^{5a}, R^{5b}: together with the carbon atom to which they are bound may form a C=O,
or a physiologically tolerated salt thereof.

According to the invention, R¹ is, for example, 1,2-diazol-4-yl, 1-methyl-1,2-diazol-4-yl, 1-methyl-1,2-diazol-3-yl, 1-methyl-1,3-diazol-4-yl, 1-methyl-1,2-diazol-5-yl, 1,5-dimethyl-1,2-diazol-4-yl, 1,3-dimethyl-1,2-diazol-4-yl, 1,5-dimethyl-1,2-diazol-4-yl, 1,3-dimethyl-1,2-diazol-5-yl, 1,3-dimethyl-5-Cl-1,2-diazol-4-yl, 1-isopropyl-3-methyl-1,2-diazol-4-yl, 1-Me-3-CF₃-1,2-diazol-4-yl, 1-cyclopentyl-3-Me-1,2-diazol-4-yl, 1,3,5-trimethyl-1,2-diazol-4-yl, 1-CHF₂-3,5-dimethyl-1,2-diazol-4-yl, 1,2-dimethyl-1,3-diazol-4-yl, 1,2-dimethyl-1,3-diazol-5-yl, 1-methyl-5-Cl-1,3-diazol-4-yl, 1-Me-1,2,3-triazol-4-yl, a further example being 1-ethyl-1,3-diazol-4-yl or 1-methyl-1,2,4-triazol-3-yl).

According to the invention, R¹ is preferably 1,2-diazol-4-yl, 1-methyl-1,2-diazol-4-yl, 1-methyl-1,2-diazol-3-yl, 1-methyl-1,3-diazol-4-yl, 1-methyl-1,2-diazol-5-yl, 1,5-dimethyl-1,2-diazol-4-yl, 1,3-dimethyl-1,2-diazol-4-yl, 1,5-dimethyl-1,2-diazol-4-yl, 1,3-dimethyl-1,2-diazol-5-yl, 1,3-dimethyl-5-Cl-1,2-diazol-4-yl, 1-iso-propyl-3-methyl-1,2-diazol-4-yl, 1-Me-3-CF₃-1,2-diazol-4-yl, 1-cyclopentyl-3-Me-1,2-diazol-4-yl, 1,3,5-trimethyl-1,2-diazol-4-yl, 1-CHF₂-3,5-dimethyl-1,2-diazol-4-yl, 1,2-dimethyl-1,3-diazol-4-yl, 1,2-dimethyl-1,3-diazol-5-yl, 1-methyl-5-Cl-1,3-diazol-4-yl, 1-Me-1,2,3-triazol-4-yl, a further example being 1-ethyl-1,3-diazol-4-yl or 1-methyl-1,2,4-triazol-3-yl).

According to one embodiment, R¹ is an optionally substituted 5-membered heterocyclic ring containing 2 or 3 N (e.g. 1,2-diazol-4-yl, 1-methyl-1,2-diazol-4-yl, 1-methyl-1,2-diazol-3-yl, 1-methyl-1,3-diazol-4-yl, 1-methyl-1,2-diazol-5-yl, 1,5-dimethyl-1,2-diazol-4-yl, 1,3-dimethyl-1,2-diazol-4-yl, 1,5-dimethyl-1,2-diazol-4-yl, 1,3-dimethyl-1,2-diazol-5-yl, 1,3-dimethyl-5-Cl-1,2-diazol-4-yl, 1-iso-propyl-3-methyl-1,2-diazol-4-yl, 1-Me-3-CF₃-1,2-diazol-4-yl, 1-cyclopentyl-3-Me-1,2-diazol-4-yl, 1,3,5-trimethyl-1,2-diazol-4-yl, 1-CHF₂-3,5-dimethyl-1,2-diazol-4-yl, 1,2-dimethyl-1,3-diazol-4-yl, 1,2-dimethyl-1,3-diazol-5-yl, 1-methyl-5-Cl-1,3-diazol-4-yl or 1-Me-1,2,3-triazol-4-yl, a further example being 1-ethyl-1,3-diazol-4-yl or 1-methyl-1,2,4-triazol-3-yl).

Preferably, R¹ is optionally substituted 1,3-diazolyl.

According to a further preferred embodiment, R¹ is optionally substituted 1,2,3-triazolyl.

In connection with R¹, substituted 5-membered heterocyclic rings containing 2 or 3 N heteroatoms in particular include pyrazolyl, imidazolyl, and triazolyl, which are substituted with 1, 2 or 3 substituents independently selected from the group consisting of halogen, C₁-C₆-alkyl, halogenated C₁-C₆-alkyl, C₃-C₆-cycloalkyl, C₁-C₄-alkoxycarbonyl and C₁-C₆-alkylcarbonylamino.

Preferably, the substituent(s) on the heterocyclic ring are independently selected from the group consisting of halogen and C₁-C₆-alkyl.

In particular, the substituent(s) on the heterocyclic ring are independently selected from the group consisting of C₁-C₆-alkyl, especially C₁-C₄-alkyl (e.g. methyl).

According to a particular embodiment, 1,3-diazolyl is substituted with halogen or C₁-C₆-alkyl as described herein. According to a specific embodiment, R¹ is 1-methyl-1,3-diazol-4-yl.

According to a further particular embodiment, 1,2,3-triazolyl is substituted with C₁-C₆-alkyl as described herein. According to a further specific embodiment, R¹ is 1-methyl-1,2,3-triazol-4-yl.

R^{2a}, R^{2b} are independently hydrogen, halogen (e.g. F), or C₁-C₃-alkyl (e.g. methyl, ethyl, n-propyl, or iso-propyl), or R^{2a}, R^{2b} together with the carbon atom to which they are bound may form a C=O.

According to one particular embodiment R^{2a} is hydrogen, halogen (e.g. F) or C₁-C₃-alkyl (e.g. methyl or ethyl), and R^{2b} is hydrogen. Preferably, R^{2a}, R^{2b} are both hydrogen.

R^{3a} is C₃-C₁₂-cycloalkyl (cyclopropyl, cyclobutyl, cyclopentyl, or cyclohexyl), optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl, a further example being 2-Me-phenyl, 3-Me-phenyl, or 4-Me-phenyl), hydroxy, C₁-C₆-alkoxy (e.g.methoxy, ethoxy, n-propoxy, iso-propoxy, or iso-butoxy) halogenated C₁-C₆-alkoxy (e.g OCF₃), C₃-C₁₂-cycloalkyl-C₁-C₄-alkoxy (e.g. cyclopropylmethoxy or cyclohexylmethoxy), C₂-C₆-alkenyloxy (e.g. allyloxy or 2-methylprop-2-en-1-oxy), C₆-C₁₂-aryl-C₁-C₄-alkoxy (e.g. benzyloxy), C₃-C₁₂-heterocyclyl-C₁-C₄-alkoxy (e.g. 2-(N-pyrrolidinyl)ethoxy, 2-(N-morpholinyl)ethoxy, or2-(N-imidazolyl)ethoxy), optionally substituted C₆-C₁₂-aryloxy (e.g. phenoxy or 4-F-phenoxy), C₃-C₁₂-heterocyclyloxy (e.g. pyridin-2-yloxy), or optionally substituted C₃-C₁₂-heterocyclyl (tetrahydrofuran-2-yl, tetrahydrofuran-3-yl, tetrahydropyran-2-yl, tetrahydropyran-3-yl, 1-cyclopropyl-piperidin-4-yl, 1-cyclopropyl-piperidin-3-yl, 2-pyridyl, 3-pyridyl, 3-F-pyrid-2-yl, 1,3-oxazol-4-yl, 1,3-oxazol-2-yl, 3-F-azetidin-1-yl, morpholin-1-yl, pyrrolidin-1-yl, piperidin-1-yl, 2-Me-piperidin-1yl, 3-Me-piperidin-1yl, 4-Me-piperidin-1yl, 4-F-piperidin-1-yl, 4,4-diF-piperidin-1yl, or azepan-1-yl, a further example being 1,3-dioxan-2-yl, 5,5-dimethyl-1,3-dioxan-2-yl, 4,6-dimethyl-1,3-dioxan-2-yl, 1,3-dioxepan-2-yl, 1,3-dioxolan-2-yl, 5,7-dioxaspiro[2.5]octan-6-yl, morpholin-3-yl, 1-methyl-piperidin-2-yl, or 5-F-pyrid-2-yl).

In particular, R^{3a} is C₃-C₁₂-cycloalkyl (cyclopropyl, cyclobutyl, cyclopentyl, or cyclohexyl), optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl, a further example being 2-Me-phenyl, 3-Me-phenyl, or 4-Me-phenyl), C₃-C₁₂-heterocyclyloxy (e.g. pyridin-2-yloxy), or optionally substituted C₃-C₁₂-heterocyclyl (tetrahydrofuran-2-yl, tetrahydrofuran-3-yl, tetrahydropyran-2-yl, tetrahydropyran-3-yl, 1-cyclopropyl-piperidin-4-yl, 1-cyclopropyl-piperidin-3-yl, 2-pyridyl, 3-pyridyl, 3-F-pyrid-2-yl, 1,3-oxazol-4-yl, 1,3-oxazol-2-yl, 3-F-azetidin-1-yl, morpholin-1-yl, pyrrolidin-1-yl, piperidin-1-yl, 2-Me-piperidin-1yl, 3-Me-piperidin-1yl, 4-Me-piperidin-1yl, 4-F-piperidin-1-yl, 4,4-diF-piperidin-1yl, or azepan-1-yl, a further example being 1,3-dioxan-2-yl, 5,5-dimethyl-1,3-dioxan-2-yl, 4,6-dimethyl-1,3-dioxan-2-yl, 1,3-dioxepan-2-yl, 1,3-dioxolan-2-yl, 5,7-dioxaspiro[2.5]octan-6-yl, morpholin-3-yl, 1-Me-piperidin-2-yl, or 5-F-pyrid-2-yl). Preferably, R^{3a} is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl, a further example being 2-Me-phenyl, 3-Me-phenyl, or 4-Me-phenyl), or optionally substituted C₃-C₁₂-heterocyclyl (tetrahydrofuran-2-yl, tetrahydrofuran-3-yl, tetrahydropyran-2-yl, tetrahydropyran-3-yl, 1-cyclopropyl-piperidin-4-yl, 1-cyclopropyl-piperidin-3-yl, 2-pyridyl, 3-pyridyl, 3-F-pyrid-2-yl, 1,3-oxazol-4-yl, 1,3-oxazol-2-yl, 3-F-azetidin-1-yl, morpholin-1-yl, pyrrolidin-1-yl, piperidin-1-yl, 2-Me-piperidin-1yl, 3-Me-piperidin-1yl, 4-Me-piperidin-1yl, 4-F-piperidin-1-yl, 4,4-diF-piperidin-1yl, or azepan-1-yl, a further example being 1,3-dioxan-2-yl, 5,5-dimethyl-1,3-dioxan-2-yl, 4,6-dimethyl-1,3-dioxan-2-yl, 1,3-dioxepan-2-yl, 1,3-dioxolan-2-yl, 5,7-dioxaspiro[2.5]octan-6-yl, morpholin-3-yl, 1-Me-piperidin-2-yl, or 5-F-pyrid-2-yl).

According to one preferred embodiment, R^{3a} is optionally substituted C₆-C₁₂-aryl wherein C₆-C₁₂-aryl is phenyl, in particular phenyl optionally substituted with 1, 2, or 3 substituents independently selected from the group consisting of halogen (e.g. phenyl, 4-F-phenyl, 2-F-phenyl, 3-F-phenyl, 2-Cl-phenyl, 2-Br-phenyl, 3-Cl-phenyl, or 2,4,5-trifluorophenyl).

According to a further preferred embodiment, R^{3a} is optionally substituted C₃-C₁₂-heterocyclyl wherein C₃-C₁₂-heterocyclyl is in particular tetrahydrofuranyl or tetrahydropyranyl, preferably unsubstituted tetrahydrofuranyl (e.g. tetrahydrofuran-2-yl) or unsubstituted tetrahydropyranyl (e.g. tetrahydropyran-2-yl), or wherein C₃-C₁₂-heterocyclyl is pyridyl (e.g. 5-F-pyrid-2-yl) or piperidinyl.

According to an additional aspect, R^{3a} is not 3,4-di-O-substituted phenyl if R^{3a} is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl). According to a further additional aspect, there is not more than one O-bound substituent on the aryl group if R^{3a} is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl).

In connection with R^{3a}, substituted C₆-C₁₂-aryl in particular includes C₆-C₁₂-aryl, such as phenyl or naphthyl, which is substituted with 1, 2, or 3 substituents independently selected from the group consisting of halogen, C₁-C₄-alkyl and C₁-C₄-alkoxy.

Preferably, the substituent(s) on C₆-C₁₂-aryl are independently selected from the group consisting of halogen.

In connection with R^{3a}, substituted C₆-C₁₂-aryloxy in particular includes C₆-C₁₂-aryloxy, such as phenoxy, which is substituted with 1, 2, or 3 substituents independently selected from the group consisting of halogen and C₁-C₄-alkyl.

Preferably, the substituent(s) on C₆-C₁₂-aryloxy are independently selected from the group consisting of halogen.

In connection with R^{3a}, substituted C₃-C₁₂-heterocyclyl in particular includes C₃-C₁₂-heterocyclyl, such as pyridyl, piperidinyl, isoxazolyl, diazolyl, tetrahydrofuranyl, tetrahydropyranyl, or morpholinyl, a further example being dioxolanyl, dioxanyl, dioxepanyl, or dioxaspiro[2.5]octanyl, which is substituted with 1, 2, or 3 substituents independently selected from the group consisting of halogen, C₁-C₄-alkyl and C₃-C₆-cycloalkyl.

Preferably, the substituent(s) on C₃-C₁₂-heterocyclyl are independently selected from the group consisting of halogen.

R^{3b} is hydrogen, C₁-C₆-alkyl, or hydroxy. Additionally, R^{3a} and R^{3b} together may be optionally substituted C₂-C₅-alkylene (e.g. 1,2-ethylene, 1,3-propylene, 1,4-butylene, or 1,5-pentylene), preferably unsubstituted C₂-C₅-alkylene (e.g. 1,5-pentylene). Preferably, R^{3b} is hydrogen.

In connection with R^{3a} and R^{3b}, substituted C₂-C₅-alkylene in particular includes C₂-C₅-alkylene, such as 1,2-ethylene, 1,3-propylene, 1,4-butylene, or 1,5-pentylene, which is substituted with 1, 2 or 3 substituents selected from the group consisting of halogen, C₁-C₄-alkyl, C₁-C₄-haloalkyl, C₁-C₄-alkoxy and C₁-C₄-haloalkoxy.

Y¹ is >CR⁶- or >N-.

Preferably, Y¹ is >CR⁶-.

R⁶ is hydrogen, C₁-C₆-alkyl (e.g. methyl, ethyl, n-propyl, iso-propyl, or iso-butyl), halogenated C₁-C₆-alkyl (e.g. CF₃ or CF₂H), (optionally substituted C₆-C₁₂-aryl)-C₁-C₄-alkyl (e.g. benzyl), hydroxy-C₁-C₆-alkyl (e.g.-CH₂OH or -(CH₂)₂OH), C₁-C₄-alkoxy-C₁-C₆-alkyl (e.g. methoxymethyl, 2-ethoxypropyl, or 3-ethoxypropyl), or hydroxy. Additionally, R⁶ and R^{3a} or R^{3b} together may be optionally substituted C₁-C₅-alkylene (e.g. 1,3-propylene or 1,4-butylene); or R⁶ may be C₁-C₄-alkylene (e.g. methylene or 1,2-ethylene) that is bound to a carbon atom in R^{3a}, and R^{3a} is an optionally substituted C₆-C₁₂-aryl (e.g. phenyl) or an optionally substituted C₃-C₂-heterocyclyl (e.g. pyridyl or piperidinyl).

In particular, R⁶ is hydrogen, C₁-C₆-alkyl (e.g. methyl or ethyl), C₆-C₁₂-aryl-C₁-C₄-alkyl (e.g. benzyl or phenethyl), hydroxy-C₁-C₆-alkyl (e.g. -CH₂OH, or -(CH₂)₂OH), or hydroxy, i.e., R⁶ is, e.g., hydrogen, methyl, benzyl, hydroxymethyl, or hydroxy. In particular, R⁶ and R^{3a} or R^{3b} together may be optionally substituted C₁-C₅-alkylene (e.g. 1,3-propylene or 1,4-butylene) such as a group of the formula: 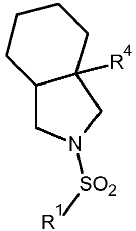 (which is shown herein for illustration purpose without being intended to limit the scope of the invention).

Alternatively, R⁶ may be C₁-C₄-alkylene (e.g. methylene or 1,2-ethylene) that is bound to a carbon atom in R^{3a}, and R^{3a} is an optionally substituted C₆-C₁₂-aryl (e.g. phenyl) such as a group of the formula: 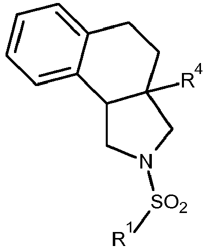 (which is shown herein for illustration purpose without being intended to limit the scope of the invention).

In connection with R⁶ and R^{3a} or R^{3b}, substituted C₁-C₅-alkylene in particular includes C₁-C₅-alkylene, such as methylene or 1,2-ethylene, which is substituted with 1, 2 or 3 substituents selected from the group consisting of halogen, C₁-C₄-alkyl, C₁-C₄-haloalkyl, C₁-C₄-alkoxy and C₁-C₄-haloalkoxy.

Preferably, R⁶ is hydrogen. It is further preferred if R⁶ and R^{3a} or R^{3b} together are C₁-C₅-alkylene (e.g. 1,3-propylene or 1,4-butylene); or if R⁶ is C₁-C₄-alkylene (e.g. methylene or 1,2-ethylene) that is bound to a carbon atom in R^{3a}, and R^{3a} is an optionally substituted C₆-C₁₂-aryl (e.g. phenyl). According to some embodiments, Y¹ is >N-. In particular, Y¹ is >N- if R⁴ is -(CR^{7e}R^{7f})ₙ₃R¹².

R⁴ is -(CR^{7a}R^{7b})ₙ₁OR¹⁰, -(CR^{7c}R^{7d})ₙ₂NR^{11a}R^{11b}, -(CR^{7e}R^{7f})ₙ₃R¹², optionally substituted C₆-C₁₂-aryl, - NR^{8a}(CR^{9a}R^{9b})ₙ₄R¹³, -NR^{8b}COR¹⁴, -NR^{8c}COOR¹⁵, -NR^{8d}CONR^{16a}R^{16b}, -NR^{8e}SO₂R¹⁷, - O(CR^{9c}R^{9d})ₙ₅R¹⁸, -COR¹⁹, -CONR^{20a}R^{20b}, -SO₂R²¹, or optionally substituted C₃-C₂-heterocyclyl (e.g. 1-propyl-1,2,3-triazol-4-yl, 4-butyl-1,2,3-triazolyl-1-yl, 4-chloroisoindoln-1-one, 7-(trifluoromethyl)-3,4-dihydro-1H-quinazolin-2-on-1-yl, a further example being 5-butyl-oxazolidin-2-on-3-yl, 1,4-thiazinan-1,1-dioxide-4-yl, indolinyl, indolin-2-on-1-yl, 6-CF₃-indolin-2-on-1-yl, isoindolinyl, or isoindolin-1-on-2-yl).

In particular, R⁴ is -(CR^{7a}R^{7b})ₙ₁OR¹⁰, -(CR^{7c}R^{7d})ₙ₂NR^{11a}R^{11b}, -(CR^{7e}R^{7f})ₙ₃R¹², optionally substituted C₆-C₁₂-aryl, -NR^{8a}(CR^{9a}R^{9b})ₙ₄R¹³, -NR^{8b}COR¹⁴, -NR^{8c}COOR¹⁵, -NR^{8d}CONR^{16a}R^{16b}, - O(CR^{9c}R^{9d})ₙ₅R¹⁸, -COR¹⁹, -CONR^{20a}R^{20b}, -SO₂R²¹, or optionally substituted C₃-C₂-heterocyclyl (e.g. 1-propyl-1,2,3-triazol-4-yl, 4-butyl-1,2,3-triazolyl-1-yl, 4-chloroisoindolin-1-one, or 7-(trifluoromethyl)-3,4-dihydro-1H-quinazolin-2-on-1-yl, a further example being 5-butyl-oxazolidin-2-on-3-yl, 1,4-thiazinan-1,1-dioxide-4-yl, indolinyl, indolin-2-on-1-yl, 6-CF₃-indolin-2-on-1-yl, isoindolinyl, or isoindolin-1-on-2-yl).

Preferably, R⁴ is -NR^{8a}(CR^{9a}R^{9b})ₙ₄R¹³ or -O(CR^{9c}R^{9d})ₙ₅R¹⁸. More preferably, R⁴ is - NR^{8a}(CR^{9a}R^{9b})ₙ₄R¹³.

In connection with R⁴, substituted C₆-C₁₂-aryl in particular includes C₆-C₁₂-aryl, such as phenyl, which is substituted with 1, 2, or 3 substituents independently selected from the group consisting of halogen. Additional substituents may be selected from the group consisting of halogenated C₁-C₄-alkyl.

In connection with R⁴, substituted C₃-C₂-heterocyclyl in particular includes C₃-C₂-heterocyclyl, such as pyridyl, isoxazolyl, diazolyl, 1,2,3-triazolyl, dihydroquinazolyn, or isoindolinyl, a further example being oxazolidinyl, thiazinanyl, or indolinyl, which is substituted with 1, 2, or 3 substituents independently selected from the group consisting of halogen, C₁-C₆-alkyl, halogenated C₁-C₆-alkyl and hydroxy.

In particular, R^{5a}, R^{5b} are independently hydrogen, halogen, or C₁-C₃-alkyl (e.g. methyl, ethyl, n-propyl, or iso-propyl) or R^{5a}, R^{5b} together with the carbon atom to which they are bound may form a C=O. In particular, one of R^{5a} or R^{5b} and one of R^{2a} or R^{2b} together may be optionally substituted C₁-C₅-alkylene, preferably unsubstituted C₁-C₅-alkylene (e.g. 1,2-ethylene).

In connection with R^{5a}, R^{5b}, R^{2a} and R^{2b}, substituted C₁-C₅-alkylene in particular includes C₁-C₅-alkylene, such as 1,2-ethylene, which is substituted with 1, 2 or 3 substituents selected from the group consisting of halogen, C₁-C₄-alkyl, C₁-C₄-haloalkyl, C₁-C₄-alkoxy and C₁-C₄-haloalkoxy.

Preferably, R^{5a} is hydrogen, halogen or C₁-C₃-alkyl (e.g. methyl or ethyl), and R^{5b} is hydrogen. More preferably, R^{5a}, R^{5b} are both hydrogen.

According to a one embodiment, R⁴ is -(CR^{7a}R^{7b})ₙ₁OR¹⁰. Thus, the present invention relates to the pyrrolidine derivatives of the formula (Ia): 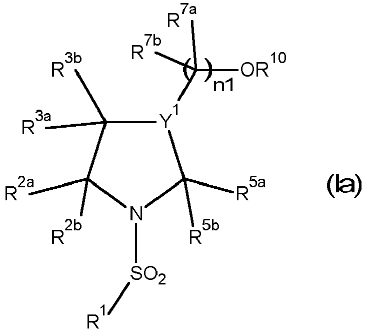
wherein R¹, R^{2a}, R^{2b}, R^{3a}, R^{3b}, Y¹, R^{5a} and R^{5b} are as defined herein, and
R^{7a}, R^{7b} are independently hydrogen or C₁-C₆-alkyl (e.g. methyl or ethyl), in particular, hydrogen;
n1 is 1, 2, 3 or 4, in particular 1; and
R¹⁰ is hydrogen, optionally substituted C₆-C₁₂-aryl (e. g. phenyl, 3-F-phenyl, 3-Me-phenyl, 3-CF₃-phenyl, 2-Cl-3-CF₃-phenyl, or 3-OCF₃-phenyl, a further example being 2-F-phenyl, 4-F-phenyl, 2-Cl-phenyl, 2,3-diF-phenyl, 2,4-diF-phenyl, 2,5-diF-phenyl, 4-Cl-phenyl, 2-Cl-4-F-phenyl, 4-Cl-3-F-phenyl, 2-Me-phenyl, 4-Me-phenyl, 2-CF₃-phenyl, 4-CF₃-phenyl, 2-Cl-4-CF₃-phenyl, 3-OCHF₂-phenyl, 2-OCF₃-phenyl, 4-OCF₃-phenyl, 3-OMe-phenyl, 2-OEt-phenyl, 2-cyclopentyl-phenyl, 2-cyclohexyl-phenyl, 3-ethynyl-phenyl, 2-CN-phenyl, 4-CN-phenyl, 2-F-4-CN-phenyl, 3-dimethylamino-phenyl, 4-(morpholin-4-yl)-phenyl, 2-pirrolidinyl-phenyl, 2-piperidin-phenyl, indan-5-yl, naphthyl, or tetralin-5-yl), or optionally substituted C₃-C₁₂-heterocyclyl (e.g. 6-CF₃-pyrimid-4-yl, 4-CF₃-pyrid-2-yl, or 2-CF₃-pyrid-4-yl, a further example being pyrid-2-yl, pyrid-3-yl, pyrid-4-yl, pyrazin-2-yl, pyridazin-3-yl, quinolin-6-yl, isoquinolin-5-yl, 1,2-bezoxazol-6-yl, or 2-Me-1,3-benzoxazol-5-yl).

In connection with R¹⁰, substituted C₆-C₁₂-aryl in particular includes C₆-C₁₂-aryl, such as phenyl or naphthyl, a further example being indanyl or tetralinyl, which is substituted with 1, 2, or 3 substituents independently selected from the group consisting of halogen, C₁-C₄-alkyl, halogenated C₁-C₄-alkyl and halogenated C₁-C₄-alkoxy. Additional substituents may be selected from the group consisting of C₃-C₆-cycloalkyl, C₁-C₄-alkynyl, CN, C₁-C₄-alkoxy, di-C₁-C₄-alkyl-amino and C₃-C₁₂-heterocyclyl.

In connection with R¹⁰, substituted C₃-C₁₂-heterocyclyl in particular includes C₃-C₁₂-heterocyclyl, such as pyridyl, pyrimidyl, or pyridazyl, a further example being pyrazyl, quinolinyl, isoquinolinyl or benzoxazolyl, which is substituted with 1, 2, or 3 substituents independently selected from the group consisting of halogenated C₁-C₄-alkyl. Additional substituents may be selected from the group consisting of C₁-C₄-alkyl.

R^{2a}, R^{2b} - in the pyrrolidine derivatives of formula (Ia) - are, in particular, hydrogen.

R^{3a} - in the pyrrolidine derivatives of formula (Ia) - is, in particular, C₃-C₁₂-cycloalkyl (e.g. cyclopropyl, cyclobutyl, cyclopentyl, or cyclohexyl), optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl, a further example being 2-Me-phenyl, 3-Me-phenyl, or 4-Me-phenyl), C₃-C₁₂-cycloalkyl-C₁-C₄-alkoxy (e.g. cyclopropylmethoxy or cyclohexylmethoxy), C₂-C₆-alkenyloxy (e.g. allyloxy or 2-methylprop-2-en-1-oxy), C₆-C₁₂-aryl-C₁-C₄-alkoxy (e.g. benzyloxy), optionally substituted C₆-C₁₂-aryloxy (e.g. phenoxy or 4-F-phenoxy). Additionally, R^{3a} - in the pyrrolidine derivatives of formula (Ia) - may be, in particular, optionally substituted C₃-C₁₂-heterocyclyl (e.g tetrahydrofuran-2-yl, tetrahydrofuran-3-yl, tetrahydropyran-2-yl, tetrahydropyran-3-yl, 1-cyclopropyl-piperidin-4-yl, 1-cyclopropyl-piperidin-3-yl, 2-pyridyl, 3-pyridyl, 3-F-pyrid-2-yl, 1,3-oxazol-4-yl, 1,3-oxazol-2-yl, 3-F-azetidin-1-yl, morpholin-1-yl, pyrrolidin-1-yl, piperidin-1-yl, 2-Me-piperidin-1yl, 3-Me-piperidin-1yl, 4-Me-piperidin-1yl, 4-F-piperidin-1-yl, 4,4-diF-piperidin-1yl, or azepan-1-yl, a further example being 1,3-dioxan-2-yl, 5,5-dimethyl-1,3-dioxan-2-yl, 4,6-dimethyl-1,3-dioxan-2-yl, 1,3-dioxepan-2-yl, 1,3-dioxolan-2-yl, 5,7-dioxaspiro[2.5]octan-6-yl, morpholin-3-yl, 1-Me-piperidin-2-yl, or 5-F-pyrid-2-yl). Preferably, R^{3a} is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl). It is further preferred if R^{3a} is C₃-C₁₂-heterocyclyl (e.g. pyrid-2yl).

R^{3b} - in the pyrrolidine derivatives of formula (Ia) - is, in particular, hydrogen.

Y¹ - in the pyrrolidine derivatives of formula (Ia) - is, in particular, >CR⁶.

R⁶ - in the pyrrolidine derivatives of formula (Ia) - is, in particular, hydrogen.

R^{5a}, R^{5b} - in the pyrrolidine derivatives of formula - (Ia) are, in particular, hydrogen.

Particular embodiments of the pyrrolidine derivatives of formula (Ia) result if:
- R¹: is an optionally substituted 5-membered heterocyclic ring as defined herein (e.g. 1,2-diazol-4-yl, 1-methyl-1,2-diazol-4-yl, 1-methyl-1,2-diazol-3-yl, 1-methyl-1,3-diazol-4-yl, 1-methyl-1,2-diazol-5-yl, 1,5-dimethyl-1,2-diazol-4-yl, 1,3-dimethyl-1,2-diazol-4-yl, 1,5-dimethyl-1,2-diazol-4-yl, 1,3-dimethyl-1,2-diazol-5-yl, 1,3-dimethyl-5-Cl-1,2-diazol-4-yl, 1-iso-propyl-3-methyl-1,2-diazol-4-yl, 1 -Me-3 -CF₃-1,2-diazol-4-yl, 1-cyclopentyl-3-Me-1,2-diazol-4-yl, 1,3,5-trimethyl-1,2-diazol-4-yl, 1-CHF₂-3,5-dimethyl-1,2-diazol-4-yl, 1,2-dimethyl-1,3-diazol-4-yl, 1,2-dimethyl-1,3-diazol-5-yl, 1-methyl-5-Cl-1,3-diazol-4-yl, 1-Me-1,2,3-triazol-4-yl, a further example being 1-ethyl-1,3-diazol-4-yl or 1-methyl-1,2,4-triazol-3-yl);
- R^{2a}, R^{2b}: are hydrogen;
- R^{3a}: is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl);
- R^{3b}: is hydrogen;
- Y¹: is >CR⁶;
- R⁶: is hydrogen;
- R^{7a}, R^{7b}: are hydrogen;
- n1: is 1;
- R¹⁰: is hydrogen, optionally substituted C₆-C₁₂-aryl (e. g. phenyl, 3-F-phenyl, 3-Me-phenyl, 3-CF₃-phenyl, 2-Cl-3-CF₃-phenyl, or 3-OCF₃-phenyl, a further example being 2-F-phenyl, 4-F-phenyl, 2-Cl-phenyl, 2,3-diF-phenyl, 2,4-diF-phenyl, 2,5-diF-phenyl, 4-Cl-phenyl, 2-Cl-4-F-phenyl, 4-Cl-3-F-phenyl, 2-Me-phenyl, 4-Me-phenyl, 2-CF₃-phenyl, 4-CF₃-phenyl, 2-Cl-4-CF₃-phenyl, 3-OCHF₂-phenyl, 2-OCF₃-phenyl, 4-OCF₃-phenyl, 3-OMe-phenyl, 2-OEt-phenyl, 2-cyclopentyl-phenyl, 2-cyclohexyl-phenyl, 3-ethynyl-phenyl, 2-CN-phenyl, 4-CN-phenyl, 2-F-4-CN-phenyl, 3-dimethylamino-phenyl, 4-(morpholin-4-yl)-phenyl, 2-pirrolidinyl-phenyl, 2-piperidin-phenyl, indan-5-yl, naphthyl, or tetralin-5-yl), or optionally substituted C₃-C₁₂-heterocyclyl (e.g. 6-CF₃-pyrimid-4-yl, 4-CF₃-pyrid-2-yl, or 2-CF₃-pyrid-4-yl, a further example being pyrid-2-yl, pyrid-3-yl, pyrid-4-yl, pyrazin-2-yl, pyridazin-3-yl, quinolin-6-yl, isoquinolin-5-yl, 1,2-bezoxazol-6-yl, or 2-Me-1,3-benzoxazol-5-yl); and
- R^{5a}, R^{5b}: are hydrogen.

Additional particular embodiments of the pyrrolidine derivatives of formula (Ia) result if:
R^{3a} is optionally substituted C₃-C₁₂-heterocyclyl (e.g. pyrid-2-yl); and
R¹, R^{2a}, R^{2b}, R^{3b}, Y¹, R⁶, R^{7a}, R^{7b}, n1, R¹⁰, R^{5a} and R^{5b} are as defined above.

According to a further particular alternative embodiment, R¹ is an optionally substituted 5-membered heterocyclic ring containing 2 or 3 N as defined herein (e.g. 1,2-diazol-4-yl, 1-methyl-1,2-diazol-4-yl, 1-methyl-1,2-diazol-3-yl, 1-methyl-1,3-diazol-4-yl, 1-methyl-1,2-diazol-5-yl, 1,5-dimethyl-1,2-diazol-4-yl, 1,3-dimethyl-1,2-diazol-4-yl, 1,5-dimethyl-1,2-diazol-4-yl, 1,3-dimethyl-1,2-diazol-5-yl, 1,3-dimethyl-5-Cl-1,2-diazol-4-yl, 1-iso-propyl-3-methyl-1,2-diazol-4-yl, 1-Me-3-CF₃-1,2-diazol-4-yl, 1-cyclopentyl-3-Me-1,2-diazol-4-yl, 1,3,5-trimethyl-1,2-diazol-4-yl, 1-CHF₂-3,5-dimethyl-1,2-diazol-4-yl, 1,2-dimethyl-1,3-diazol-4-yl, 1,2-dimethyl-1,3-diazol-5-yl, 1-methyl-5-Cl-1,3-diazol-4-yl or 1-Me-1,2,3-triazol-4-yl, a further example being 1-ethyl-1,3-diazol-4-yl or 1-methyl-1,2,4-triazol-3-yl).

Preferably, R¹ - in the pyrrolidine derivatives of formula (Ia) - is an optionally substituted 5-membered heterocyclic ring as defined herein containing 2 N which, in particular, is optionally substituted 1,3-diazolyl.

According to a further preferred embodiment, R¹ - in the pyrrolidine derivatives of formula (Ia) - is an optionally substituted 5-membered heterocyclic ring as defined herein containing 3 N which, in particular, is optionally substituted 1,2,3-triazolyl.

In connection with R¹ and the pyrrolidine derivatives of formula (la), substituted 5-membered heterocyclic ring in particular includes pyrazolyl, imidazolyl, and triazolyl, which are substituted with 1, 2 or 3 substituents independently selected from the group consisting of halogen, C₁-C₆-alkyl, halogenated C₁-C₆-alkyl, C₃-C₆-cycloalkyl, C₁-C₄-alkoxy-carbonyl and C₁-C₆-alkylcarbonylamino. Preferably, the substituent(s) on the heterocyclic ring are independently selected from the group consisting of halogen and C₁-C₆-alkyl. In particular, the substituent(s) on the heterocyclic ring are independently selected from the group consisting of C₁-C₆-alkyl, especially C₁-C₄-alkyl (e.g. methyl). According to a particular embodiment, 1,3-diazolyl is substituted with halogen or C₁-C₆-alkyl as described herein. According to a specific embodiment, R¹ is 1-methyl-1,3-diazol-4-yl.

In connection with R^{3a} and the pyrrolidine derivatives of formula (la), substituted C₆-C₁₂-aryl in particular includes C₆-C₁₂-aryl, such as phenyl or naphthyl, which is substituted with 1, 2, or 3 substituents independently selected from the group consisting of halogen, C₁-C₄-alkyl and C₁-C₄-alkoxy. Preferably, the substituents on C₆-C₁₂-aryl are independently selected from the group consisting of halogen.

According to a preferred embodiment, R^{3a} - in the pyrrolidine derivatives of formula (Ia) - is C₆-C₁₂-aryl, in particular phenyl, optionally substituted with 1, 2, or 3 substituents independently selected from the group consisting of halogen (e.g. phenyl, 2-Cl-phenyl, 2-Br-phenyl, 3-Cl-phenyl, 4-Cl-phenyl, 2-F-phenyl, 3-F-phenyl, 4-F-phenyl, or 2,4,5-trifluoro-phenyl).

Further preferred embodiments of pyrrolidine derivatives of formula (Ia) result if:
- R¹: is 1,3-diazolyl optionally substituted with halogen or C₁-C₄-alkyl (e.g. 1-methyl-1,3-diazol-4-yl);
- R^{2a}, R^{2b}: are hydrogen;
- R^{3a}: is C₆-C₁₂-aryl, in particular phenyl, optionally substituted with 1, 2, or 3 substituents independently selected from the group consisting of halogen (e.g. phenyl, 2-Cl-phenyl, 2-Br-phenyl, 3-Cl-phenyl, 4-Cl-phenyl, 2-F-phenyl, 3-F-phenyl, 4-F-phenyl, or 2,4,5-trifluoro-phenyl);
- R^{3b}: is hydrogen;
- Y¹: is >CR⁶; R⁶, is hydrogen;
- R^{7a}, R^{7b}: are hydrogen;
- n1: is 1;
- R¹⁰: is hydrogen, C₆-C₁₂-aryl (e.g. phenyl) optionally substituted with 1, 2, or 3 substituents independently selected from the group consisting of halogen, C₁-C₄-alkyl, halogenated C₁-C₄-alkyl and halogenated C₁-C₄-alkoxy, or C₃-C₂-heterocyclyl optionally substituted with 1, 2, or 3 substituents independently selected from the group consisting of halogenated C₁-C₄-alkyl; and
- R^{5a}, R^{5b}: are hydrogen.

Additional preferred embodiments of pyrrolidine derivatives of formula (Ia) result if:
- R¹⁰: is C₆-C₁₂-aryl (e.g. phenyl) optionally substituted with 1, 2, or 3 substituents independently selected from the group consisting of C₃-C₆-cycloalkyl, C₁-C₄-alkynyl, CN, C₁-C₄-alkoxy, di-C₁-C₄-alkyl-amino and C₃-C₂-heterocyclyl, or C₃-C₂-heterocyclyl optionally substituted with 1, 2, or 3 substituents independently selected from the group consisting of C₁-C₄-alkyl; and R¹, R^{2a}, R^{2b}, R^{3a}, R^{3b}, Y¹, R⁶, R^{7a}, R^{7b}, n1, R^{5a} and R^{5b} are as defined above.

According to a further embodiment, R⁴ is -(CR^{7c}R^{7d})ₙ₂NR^{11a}R^{11b}. Thus, the present invention relates to the pyrrolidine derivatives of the formula (Ib): 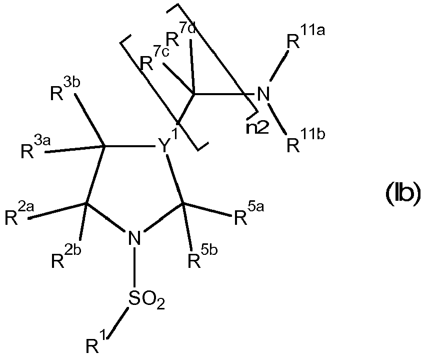
wherein R¹, R^{2a}, R^{2b}, R^{3a}, R^{3b}, Y¹, R^{5a} and R^{5b} are as defined herein, and
R^{7c}, R^{7d}
   are independently hydrogen or C₁-C₆-alkyl (e.g. methyl or ethyl), in particular, hydrogen;
n2 is 1, 2, 3, or 4, in particular, 1;
R^{11a} is C₁-C₈-alkyl (e.g. methyl, ethyl, n-propyl, or n-butyl), (optionally substituted C₃-C₁₂-cycloalkyl)-C₁-C₄-alkyl (e.g. cyclopropyl-methyl), C₁-C₆-alkoxy-C₁-C₆-alkyl (e.g. 2-methoxy-ethyl, 2-ethoxy-ethyl, 2-isopropoxy-ethyl, 1-methyl-2-methoxy-ethyl, 3-methoxy-propyl, 3-ethoxy-propyl, or 3-isopropoxy-propyl), (optionally substituted C₆-C₁₂-aryl)-C₁-C₄-alkyl (e.g. benzyl), (optionally substituted C₃-C₁₂-heterocyclyl)-C₁-C₄-alkyl, optionally substituted C₆-C₁₂-aryl (e.g. phenyl, 4-F-phenyl, 3-Cl-phenyl, 3,5-diCl-phenyl, 3-Cl-4-F-phenyl, 3-Me-phenyl, 3-(aminomethyl)-phenyl, 3-CF₃-phenyl, 4-CF₃-phenyl, 2-Cl-4-CF₃-phenyl, 3-Cl-4-CF₃-phenyl, 3-CF₃-4-F-phenyl, 3-OCF₃-phenyl, 3-OCF₃-4-F-phenyl, 3-OCF₃-4-Cl-phenyl, or 3-(aminocarbonyl)-phenyl), or optionally substituted C₃-C₁₂-heterocyclyl; and
R^{11b} is hydrogen or (e.g. methyl, ethyl, n-propyl or n-butyl).

In particular, R^{11a} is C₁-C₈-alkyl (e.g. n-propyl or n-butyl), C₃-C₁₂-cycloalkyl-C₁-C₄-alkyl (e.g. cyclopropyl-methyl), C₁-C₆-alkoxy-C₁-C₆-alkyl (e.g. 2-methoxy-ethyl, 2-ethoxy-ethyl, 2-isopropyloxy-ethyl, 1-methyl-2-methoxy-ethyl, 3-methoxy-propyl, 3-ethoxy-propyl, or 3-isopropyloxy-propyl), C₆-C₁₂-aryl-C₁-C₄-alkyl (e.g. benzyl), or optionally substituted C₆-C₁₂-aryl (e.g. phenyl, 4-F-phenyl, 3-Cl-phenyl, 3,5-diCl-phenyl, 3-Cl-4-F-phenyl, 3-Me-phenyl, 3-(aminomethyl)-phenyl, 3-CF₃-phenyl, 4-CF₃-phenyl, 2-Cl-4-CF₃-phenyl, 3-Cl-4-CF₃-phenyl, 3-CF₃-4-F-phenyl, 3-OCF₃-phenyl, 3-OCF₃-4-F-phenyl, 3-OCF₃-4-Cl-phenyl, or 3-(aminocarbonyl)-phenyl).

In connection with R^{11a}, substituted C₆-C₁₂-aryl in particular includes C₆-C₁₂-aryl, such as phenyl, substituted with 1, 2, or 3 substituents independently selected from the group consisting of halogen, C₁-C₄-alkyl, halogenated C₁-C₄-alkyl, amino-C₁-C₄-alkyl, amino-carbonyl and halogenated C₁-C₄-alkoxy.

In particular, R^{11b} is hydrogen.

R^{2a}, R^{2b} - in the pyrrolidine derivatives of formula (Ib) - are, in particular, both hydrogen.

R^{3a} - in the pyrrolidine derivatives of formula (Ib) - is, in particular, C₃-C₁₂-cycloalkyl (e.g. cyclopropyl, cyclobutyl, cyclopentyl, or cyclohexyl), optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl, a further example being 2-Me-phenyl, 3-Me-phenyl, or 4-Me-phenyl), C₃-C₁₂-cycloalkyl-C₁-C₄-alkoxy (e.g. cyclopropylmethoxy or cyclohexylmethoxy), C₂-C₆-alkenyloxy (e.g. allyloxy or 2-methylprop-2-en-1-oxy), C₆-C₁₂-aryl-C₁-C₄-alkoxy (e.g. benzyloxy), or optionally substituted C₆-C₁₂-aryloxy (e.g. phenoxy or 4-F-phenoxy). Preferably, R^{3a} is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl, a further example being 2-Me-phenyl, 3-Me-phenyl, or 4-Me-phenyl).

R^{3b} - in the pyrrolidine derivatives of formula (Ib) - is, in particular, hydrogen.

Y¹ - in the pyrrolidine derivatives of formula (Ib) - is, in particular, >CR⁶.

R⁶ - in the pyrrolidine derivatives of formula (Ib) - is, in particular, hydrogen.

R^{5a}, R^{5b} in the pyrrolidine derivatives of formula (Ib) - are, in particular, hydrogen.

Particular embodiments of the pyrrolidine derivatives of formula (Ib) result if:
- R¹: is an optionally substituted 5-membered heterocyclic ring as defined herein (e.g. 1,2-diazol-4-yl, 1-methyl-1,2-diazol-4-yl, 1-methyl-1,2-diazol-3-yl, 1-methyl-1,3-diazol-4-yl, 1-methyl-1,2-diazol-5-yl, 1,5-dimethyl-1,2-diazol-4-yl, 1,3-dimethyl-1,2-diazol-4-yl, 1,5-dimethyl-1,2-diazol-4-yl, 1,3-dimethyl-1,2-diazol-5-yl, 1,3-dimethyl-5-Cl-1,2-diazol-4-yl, 1-isopropyl-3-methyl-1,2-diazol-4-yl, 1-Me-3-CF₃-1,2-diazol-4-yl, 1-cyclopentyl-3-Me-1,2-diazol-4-yl, 1,3,5-trimethyl-1,2-diazol-4-yl, 1-CHF₂-3,5-dimethyl-1,2-diazol-4-yl, 1,2-dimethyl-1,3-diazol-4-yl, 1,2-dimethyl-1,3-diazol-5-yl, 1-methyl-5-Cl-1,3-diazol-4-yl, 1-Me-1,2,3-triazol-4-yl, a further example being 1-ethyl-1,3-diazol-4-yl or 1-methyl-1,2,4-triazol-3-yl);
- R^{2a}, R^{2b}: are hydrogen;
- R^{3a}: is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl or 2,4,5-trifluoro-phenyl, a further example being 2-Me-phenyl, 3-Me-phenyl, or 4-Me-phenyl);
- R^{3b}: is hydrogen;
- Y¹: is >CR⁶;
- R⁶: is hydrogen;
- R^{7c}, R^{7d}: are hydrogen;
- n2: is 1;
- R^{11a}: is C₁-C₈-alkyl (e.g. n-propyl or n-butyl), C₃-C₁₂-cycloalkyl-C₁-C₄-alkyl (e.g. cyclopropyl-methyl) C₁-C₆-alkoxy-C₁-C₆-alkyl (e.g. 2-methoxy-ethyl, 2-ethoxy-ethyl, 2-isopropyloxy-ethyl, 1-methyl-2-methoxy-ethyl, 3-methoxy-propyl, 3-ethoxy-propyl, or 3-isopropyloxy-propyl), C₆-C₁₂-aryl-C₁-C₄-alkyl (e.g. benzyl), optionally substituted C₆-C₁₂-aryl (e.g. phenyl, 4-F-phenyl, 3-Cl-phenyl, 3,5-diCl-phenyl, 3-Cl-4-F-phenyl, 3-Me-phenyl, 3-(aminomethyl)-phenyl, 3-CF₃-phenyl, 4-CF₃-phenyl, 2-Cl-4-CF₃-phenyl, 3-Cl-4-CF₃-phenyl, 3-CF₃-4-F-phenyl, 3-OCF₃-phenyl, 3-OCF₃-4-F-phenyl, 3-OCF₃-4-Cl-phenyl, or 3-(aminocarbonyl)-phenyl);
- R^{11b}: is hydrogen; and
- R^{5a}, R^{5b}: are hydrogen.

Preferably, R¹ - in the pyrrolidine derivatives of formula (Ib) - is an optionally substituted 5-membered heterocyclic ring as defined herein containing 2 N which, in particular, is optionally substituted 1,3-diazolyl.

According to a further preferred embodiment, R¹ - in the pyrrolidine derivatives of formula (Ib) - is an optionally substituted 5-membered heterocyclic ring as defined herein containing 3 N which, in particular, is optionally substituted 1,2,3-triazolyl.

In connection with R¹ and the pyrrolidine derivatives of formula (Ib), substituted 5-membered heterocyclic ring in particular includes pyrazolyl, imidazolyl, and triazolyl, which are substituted with 1, 2 or 3 substituents independently selected from the group consisting of halogen, C₁-C₆-alkyl, halogenated C₁-C₆-alkyl, C₃-C₆-cycloalkyl, C₁-C₄-alkoxy-carbonyl and C₁-C₆-alkyl-carbonylamino. Preferably, the substituent(s) on the heterocyclic ring are independently selected from the group consisting of halogen and C₁-C₆-alkyl. In particular, the substituent(s) on the heterocyclic ring are independently selected from the group consisting of C₁-C₆-alkyl, especially C₁-C₄-alkyl (e.g. methyl). According to a particular embodiment, 1,3-diazolyl is substituted with halogen or C₁-C₆-alkyl as described herein. According to a specific embodiment, R¹ is 1-methyl-1,3-diazol-4-yl.

In connection with R^{3a} and the pyrrolidine derivatives of formula (Ib), substituted C₆-C₁₂-aryl in particular includes C₆-C₁₂-aryl, such as phenyl or naphthyl, which is substituted with 1, 2, or 3 substituents independently selected from the group consisting of halogen, C₁-C₄-alkyl and C₁-C₄-alkoxy. Preferably, the substituents on C₆-C₁₂-aryl are independently selected from the group consisting of halogen.

According to a preferred embodiment, R^{3a} - in the pyrrolidine derivatives of formula (Ib) - is C₆-C₁₂-aryl, in particular phenyl, optionally substituted with 1, 2, or 3 substituents independently selected from the group consisting of halogen (e.g. phenyl, 2-Cl-phenyl, 2-Br-phenyl, 3-Cl-phenyl, 4-Cl-phenyl, 2-F-phenyl, 3-F-phenyl, 4-F-phenyl, or 2,4,5-trifluoro-phenyl).

Further preferred embodiments of pyrrolidine derivatives of formula (Ib) result if:
- R¹: is 1,3-diazolyl optionally substituted with halogen or C₁-C₄-alkyl (e.g.1-methyl-1,3-diazol-4-yl);
- R^{2a}, R^{2b}: are hydrogen;
- R^{3a}: is C₆-C₁₂-aryl optionally substituted with 1, 2, or 3 substituents independently selected from the group consisting of halogen (e.g. phenyl, 2-Cl-phenyl, 2-Br-phenyl, 3-Cl-phenyl, 4-Cl-phenyl, 2-F-phenyl, 3-F-phenyl, 4-F-phenyl, or 2,4,5-trifluoro-phenyl);
- R^{3b}: is hydrogen;
- Y¹: is >CR⁶;
- R⁶: is hydrogen;
- R^{7c}, R^{7d}: are hydrogen;
- n2: is 1;
- R^{11a}: is C₁-C₈-alkyl (e.g. n-propyl or n-butyl), C₃-C₁₂-cycloalkyl-C₁-C₄-alkyl (e.g. cyclopropyl-methyl), C₁-C₆-alkoxy-C₁-C₆-alkyl (e.g. 2-methoxy-ethyl, 2-ethoxy-ethyl, 2-isopropyloxy-ethyl, 1-methyl-2-methoxy-ethyl, 3-methoxy-propyl, 3-ethoxy-propyl, or 3-isopropyloxy-propyl), C₆-C₁₂-aryl-C₁-C₄-alkyl (e.g. benzyl), or C₆-C₁₂-aryl optionally substituted with 1, 2, or 3 substituents independently selected from the group consisting of halogen, C₁-C₄-alkyl, halogenated C₁-C₄-alkyl, amino-C₁-C₄-alkyl, aminocarbonyl and halogenated C₁-C₄-alkoxy (e.g. phenyl, 4-F-phenyl, 3-Cl-phenyl, 3,5-diCl-phenyl, 3-Cl-4-F-phenyl, 3-Me-phenyl, 3-(aminomethyl)-phenyl, 3-CF₃-phenyl, 4-CF₃-phenyl, 2-Cl-4-CF₃-phenyl, 3-Cl-4-CF₃-phenyl, 3-CF₃-4-F-phenyl, 3-OCF₃-phenyl, 3-OCF₃-4-F-phenyl, 3-OCF₃-4-Cl-phenyl, or 3-(aminocarbonyl)-phenyl);
- R^{11b}: is hydrogen; and
- R^{5a}, R^{5b}: are hydrogen.

According to a further embodiment, R⁴ is -(CR^{7e}R^{7f})ₙ₃R¹². Thus, the present invention relates to the pyrrolidine derivatives of the formula (Ic): 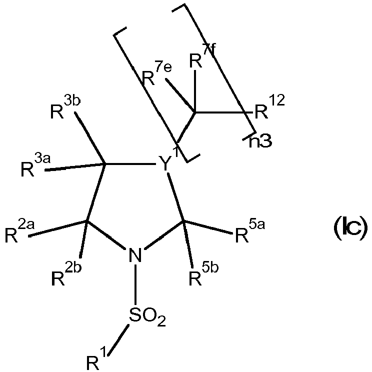
wherein R¹, R^{2a}, R^{2b}, R^{3a}, R^{3b}, Y¹, R^{5a} and R^{5b} are as defined herein, and
R^{7e}, R^{7f}
   are independently hydrogen or C₁-C₆-alkyl (e.g. methyl or ethyl), in particular, hydrogen;
n3 is 1, 2, 3, or 4, in particular, 1; and
R¹² is optionally substituted C₆-C₁₂-aryl (e.g. phenyl, 3-Cl-phenyl, or 3-Br-phenyl, a further example being 3-CF₃-phenyl) or optionally substituted C₃-C₁₂-heterocyclyl (e.g. 4-CF₃-pyrid-2-yl or 1-propyl-1,2,3-triazol-4-yl).

In connection with R¹², substituted C₆-C₁₂-aryl in particular includes C₆-C₁₂-aryl, such as phenyl, which is substituted with 1, 2, or 3 substituents independently selected from the group consisting of halogen. Additional substituents may be selected from the group consisting of halogenated C₁-C₄-alkyl.

In connection with R¹², substituted C₃-C₁₂-heterocyclyl in particular includes C₃-C₁₂-heterocyclyl, such as pyridyl or triazolyl, which is substituted with 1, 2, or 3 substituents independently selected from the group consisting of C₁-C₄-alkyl and halogenated C₁-C₄-alkyl.

R^{2a}, R^{2b} - in pyrrolidine derivatives of formula (Ic) - are, in particular, hydrogen.

R^{3a} - in pyrrolidine derivatives of formula (Ic) - is, in particular, C₃-C₂-cycloalkyl (e.g. cyclopropyl, cyclobutyl, cyclopentyl, or cyclohexyl), optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl, a further example being 2-Me-phenyl, 3-Me-phenyl, or 4-Me-phenyl), C₃-C₁₂-cycloalkyl-C₁-C₄-alkoxy (e.g. cyclopropylmethoxy or cyclohexylmethoxy), C₂-C₆-alkenyloxy (e.g. allyloxy or 2-methylprop-2-en-1-oxy), C₆-C₁₂-aryl-C₁-C₄-alkoxy (e.g. benzyloxy), or optionally substituted C₆-C₁₂-aryloxy (e.g. phenoxy or 4-F-phenoxy). Additionally, R^{3a} - in pyrrolidine derivatives of formula (Ic) - may be, in particular, optionally substituted C₃-C₁₂-heterocyclyl (e.g. tetrahydrofuran-2-yl, tetrahydrofuran-3-yl, tetrahydropyran-2-yl, tetrahydropyran-3-yl, 1-cyclopropyl-piperidin-4-yl, 1-cyclopropyl-piperidin-3-yl, 2-pyridyl, 3-pyridyl, 3-F-pyrid-2-yl, 1,3-oxazol-4-yl, 1,3-oxazol-2-yl, 3-F-azetidin-1-yl, morpholin-1-yl, pyrrolidin-1-yl, piperidin-1-yl, 2-Me-piperidin-1yl, 3-Me-piperidin-1yl, 4-Me-piperidin-1yl, 4-F-piperidin-1-yl, 4,4-diF-piperidin-1yl, or azepan-1-yl, a further example being 1,3-dioxan-2-yl, 5,5-dimethyl-1,3-dioxan-2-yl, 4,6-dimethyl-1,3-dioxan-2-yl, 1,3-dioxepan-2-yl, 1,3-dioxolan-2-yl, 5,7-dioxaspiro[2.5]octan-6-yl, morpholin-3-yl, 1-Me-piperidin-2-yl, or 5-F-pyrid-2-yl). Preferably, R^{3a} is C₃-C₁₂-cycloalkyl (e.g. cyclopropyl, cyclobutyl, cyclopentyl, or cyclohexyl) or optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl, a further example being 2-Me-phenyl, 3-Me-phenyl, or 4-Me-phenyl). It is further preferred if R^{3a} is optionally substituted C₃-C₁₂-heterocyclyl (e.g. 1,3-dioxan-2-yl, 5,5-dimethyl-1,3-dioxan-2-yl, 4,6-dimethyl-1,3-dioxan-2-yl, 1,3-dioxepan-2-yl, 1,3-dioxolan-2-yl, or 5,7-dioxaspiro[2.5]octan-6-yl).

R^{3b} - in the pyrrolidine derivatives of formula (Ic) - is, in particular, hydrogen.

Y¹ - in the pyrrolidine derivatives of formula (Ic) - is, in particular, >CR⁶.

R⁶ - in the pyrrolidine derivatives of formula (Ic) - is, in particular, hydrogen or hydroxy.

R^{5a}, R^{5b} - in the pyrrolidine derivatives of formula (Ic) - are, in particular, hydrogen or together with the carbon atom to which they are bound may form a C=O.

If Y¹ - in the pyrrolidine derivatives of formula (Ic) - is >N- R^{5a}, R^{5b} together with the carbon atom to which they are bound form, in particular, a C=O.

Particular embodiments of pyrrolidine derivatives of formula (Ic) result if:
- R¹: is an optionally substituted 5-membered heterocyclic ring as defined herein (e.g. 1,2-diazol-4-yl, 1-methyl-1,2-diazol-4-yl, 1-methyl-1,2-diazol-3-yl, 1-methyl-1,3-diazol-4-yl, 1-methyl-1,2-diazol-5-yl, 1,5-dimethyl-1,2-diazol-4-yl, 1,3-dimethyl-1,2-diazol-4-yl, 1,5-dimethyl-1,2-diazol-4-yl, 1,3-dimethyl-1,2-diazol-5-yl, 1,3-dimethyl-5-Cl-1,2-diazol-4-yl, 1-isopropyl-3-methyl-1,2-diazol-4-yl, 1-Me-3-CF₃-1,2-diazol-4-yl, 1-cyclopentyl-3-Me-1,2-diazol-4-yl, 1,3,5-trimethyl-1,2-diazol-4-yl, 1-CHF₂-3,5-dimethyl-1,2-diazol-4-yl, 1,2-dimethyl-1,3-diazol-4-yl, 1,2-dimethyl-1,3-diazol-5-yl, 1-methyl-5-Cl-1,3-diazol-4-yl, 1-Me-1,2,3-triazol-4-yl, a further example being 1-ethyl-1,3-diazol-4-yl or 1-methyl-1,2,4-triazol-3-yl);
- R^{2a}, R^{2b}: are hydrogen;
- R^{3a}: is C₃-C₁₂-cycloalkyl (e.g. cyclopropyl, cyclobutyl, cyclopentyl, or cyclohexyl) or optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl, a further example being 2-Me-phenyl, 3-Me-phenyl, or 4-Me-phenyl);
- R^{3b}: is hydrogen;
- Y¹: is >CR⁶ or >N-;
- R⁶: is hydrogen or hydroxy;
- R^{7e}, R^{7f}: are hydrogen;
- n3: is 1;
- R¹²: is optionally substituted C₆-C₁₂-aryl (e.g. phenyl, 3-Cl-phenyl, or 3-Br-phenyl, a further example being 3-CF₃-phenyl) or optionally substituted C₃-C₂-heterocyclyl (e.g. 4-CF₃-pyrid-2-yl or 1-propyl-1,2,3-triazol-4-yl); and
- R^{5a}, R^{5b}: are hydrogen or together with the carbon atom to which they are bound may form a C=O.

Further particular embodiments of pyrrolidine derivatives of formula (Ic) result if:
R^{3a} is optionally substituted C₃-C₂-heterocyclyl (e.g. 1,3-dioxan-2-yl, 5,5-dimethyl-1,3-dioxan-2-yl, 4,6-dimethyl-1,3-dioxan-2-yl, 1,3-dioxepan-2-yl, 1,3-dioxolan-2-yl, or 5,7-dioxaspiro[2.5]octan-6-yl); and
R¹, R^{2a}, R^{2b}, R^{3b}, Y¹, R⁶, R^{7e}, R^{7f}, n3, R¹², R^{5a} and R^{5b} are as defined above.

Preferably, R¹ - in the pyrrolidine derivatives of formula (Ic) - is an optionally substituted 5-membered heterocyclic ring as defined herein containing 2 N which, in particular, is optionally substituted 1,3-diazolyl.

According to a further preferred embodiment, R¹ - in the pyrrolidine derivatives of formula (Ic) - is an optionally substituted 5-membered heterocyclic ring as defined herein containing 3 N which, in particular, is optionally substituted 1,2,3-triazolyl.

In connection with R¹ and the pyrrolidine derivatives of formula (Ic), substituted 5-membered heterocyclic ring in particular includes pyrazolyl, imidazolyl, and triazolyl, which are substituted with 1, 2 or 3 substituents independently selected from the group consisting of halogen, C₁-C₆-alkyl, halogenated C₁-C₆-alkyl, C₃-C₆-cycloalkyl, C₁-C₄-alkoxy-carbonyl and C₁-C₆-alkyl-carbonylamino. Preferably, the substituent(s) on the heterocyclic ring are independently selected from the group consisting of halogen and C₁-C₆-alkyl. In particular, the substituent(s) on the heterocyclic ring are independently selected from the group consisting of C₁-C₆-alkyl, especially C₁-C₄-alkyl (e.g. methyl). According to a particular embodiment, 1,3-diazolyl is substituted with halogen or C₁-C₆-alkyl as described herein. According to a specific embodiment, R¹ is 1-methyl-1,3-diazol-4-yl.

In connection with R^{3a} and the pyrrolidine derivatives of formula (Ic), substituted C₆-C₁₂-aryl in particular includes C₆-C₁₂-aryl, such as phenyl or naphthyl, which is substituted with 1, 2, or 3 substituents independently selected from the group consisting of halogen, C₁-C₄-alkyl and C₁-C₄-alkoxy. Preferably, the substituents on C₆-C₁₂-aryl are independently selected from the group consisting of halogen.

According to a preferred embodiment, R^{3a} - in the pyrrolidine derivatives of formula (Ic) - is C₃-C₁₂-cycloalkyl (e.g. cyclopropyl, cyclobutyl, cyclopentyl, or cyclohexyl) or C₆-C₁₂-aryl, in particular phenyl, optionally substituted with 1, 2, or 3 substituents independently selected from the group consisting of halogen (e.g. phenyl, 2-Cl-phenyl, 2-Br-phenyl, 3-Cl-phenyl, 4-Cl-phenyl, 2-F-phenyl, 3-F-phenyl, 4-F-phenyl, or 2,4,5-trifluoro-phenyl).

According to an additional preferred embodiment, R^{3a} - in the pyrrolidine derivatives of formula (Ic) - may be C₃-C₁₂-heterocyclyl (e.g. 1,3-dioxan-2-yl).

Further preferred embodiments of pyrrolidine derivatives of formula (Ic) result if:
- R¹: is 1,3-diazolyl optionally substituted with halogen or C₁-C₄-alkyl and preferably 1-methyl-1,3-diazol-4-yl;
- R^{2a}, R^{2b}: are hydrogen;
- R^{3a}: is C₃-C₁₂-cycloalkyl (e. g. cyclopropyl) or C₆-C₁₂-aryl optionally substituted with halogen (e.g. phenyl, 2-Br-phenyl, or 4-F-phenyl);
- R^{3b}: is hydrogen;
- Y¹: is >CR⁶ or >N-;
- R⁶: is hydrogen or hydroxy;
- R^{7e}, R^{7f}: are hydrogen;
- n3: is 1;
- R¹²: is C₆-C₁₂-aryl optionally substituted with 1, 2, or 3 substituents independently selected from the group consisting of halogen (e.g. phenyl, 3-Cl-phenyl, or 3-Br-phenyl), or C₃-C₁₂-heterocyclyl optionally substituted with 1, 2, or 3 substituents independently selected from the group consisting of C₁-C₄-alkyl and halogenated C₁-C₄-alkyl (e.g. 4-CF₃-pyrid-2-yl or 1-propyl-1,2,3-triazol-4-yl); and
- R^{5a}, R^{5b}: are hydrogen or together with the carbon atom to which they are bound may form a C=O.

Further preferred embodiments of pyrrolidine derivatives of formula (Ic) result if:
R^{3a} is C₃-C₁₂-heterocyclyl (e.g. 1,3-dioxan-2-yl); and
R¹, R^{2a}, R^{2b}, R^{3b}, Y¹, R⁶, R^{7e}, R^{7f}, n3, R¹², R^{5a} and R^{5b} are as defined above.

According to a further embodiment, R⁴ is optionally substituted C₆-C₁₂-aryl (e.g. phenyl, 4-Cl-phenyl, or 2-Cl-phenyl, a further example being 3-CF₃-phenyl).

In connection with R⁴ being optionally substituted C₆-C₁₂-aryl, substituted C₆-C₁₂-aryl in particular includes C₆-C₁₂-aryl, such as phenyl, which is substituted with 1, 2, or 3 substituents independently selected from the group consisting of halogen. Additional substituents may be selected from the group consisting of halogenated C₁-C₄-alkyl.

R^{2a}, R^{2b} - in the pyrrolidine derivatives wherein R⁴ is optionally substituted C₆-C₁₂-aryl - are, in particular, hydrogen.

R^{3a} - in the pyrrolidine derivatives wherein R⁴ is optionally substituted C₆-C₁₂-aryl - is, in particular, C₃-C₁₂-cycloalkyl (e.g.cyclopropyl, cyclobutyl, cyclopentyl, or cyclohexyl), optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl, a further example being 2-Me-phenyl, 3-Me-phenyl, or 4-Me-phenyl), C₃-C₁₂-cycloalkyl-C₁-C₄-alkoxy (e.g. cyclopropylmethoxy or cyclohexylmethoxy), C₂-C₆-alkenyloxy (e.g. allyloxy or 2-methylprop-2-en-1-oxy), C₆-C₁₂-aryl-C₁-C₄-alkoxy (e.g. benzyloxy), or optionally substituted C₆-C₁₂-aryloxy (e.g. phenoxy or 4-F-phenoxy). Preferably, R^{3a} is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl).

R^{3b} - in the pyrrolidine derivatives wherein R⁴ is optionally substituted C₆-C₁₂-aryl - is, in particular, hydrogen.

Y¹ - in the pyrrolidine derivatives wherein R⁴ is optionally substituted C₆-C₁₂-aryl - is, in particular, >CR⁶.

R⁶ - in the pyrrolidine derivatives wherein R⁴ is optionally substituted C₆-C₁₂-aryl - is, in particular, hydrogen.

R^{5a}, R^{5b} - in the pyrrolidine derivatives wherein R⁴ is optionally substituted C₆-C₁₂-aryl - are, in particular, hydrogen.

Particular embodiments of the pyrrolidine derivatives wherein R⁴ is optionally substituted C₆-C₁₂-aryl result if:
- R¹: is an optionally substituted 5-membered heterocyclic ring as defined herein (e.g. 1,2-diazol-4-yl, 1-methyl-1,2-diazol-4-yl, 1-methyl-1,2-diazol-3-yl, 1-methyl-1,3-diazol-4-yl, 1-methyl-1,2-diazol-5-yl, 1,5-dimethyl-1,2-diazol-4-yl, 1,3-dimethyl-1,2-diazol-4-yl, 1,5-dimethyl-1,2-diazol-4-yl, 1,3-dimethyl-1,2-diazol-5-yl, 1,3-dimethyl-5-Cl-1,2-diazol-4-yl, 1-isopropyl-3-methyl-1,2-diazol-4-yl, 1-Me-3-CF₃-1,2-diazol-4-yl, 1-cyclopentyl-3-Me-1,2-diazol-4-yl, 1,3,5-trimethyl-1,2-diazol-4-yl, 1-CHF₂-3,5-dimethyl-1,2-diazol-4-yl, 1,2-dimethyl-1,3-diazol-4-yl, 1,2-dimethyl-1,3-diazol-5-yl, 1-methyl-5-Cl-1,3-diazol-4-yl, 1-Me-1,2,3-triazol-4-yl, a further example being 1-ethyl-1,3-diazol-4-yl or 1-methyl-1,2,4-triazol-3-yl);
- R^{2a}, R^{2b}: are hydrogen;
- R^{3a}: is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl);
- R^{3b}: is hydrogen;
- R⁴: is optionally substituted C₆-C₁₂-aryl (e.g. phenyl, 4-Cl-phenyl, or 2-Cl-phenyl, a further example being 3-CF₃-phenyl);
- Y¹: is >CR⁶;
- R⁶: is hydrogen; and
- R^{5a}, R^{5b}: are hydrogen.

Preferably, R¹ - in the pyrrolidine derivatives wherein R⁴ is optionally substituted C₆-C₁₂-aryl - is an optionally substituted 5-membered heterocyclic ring containing 2 N which, in particular, is optionally substituted 1,3-diazolyl.

According to a further preferred embodiment, R¹ - in the pyrrolidine derivatives wherein R⁴ is optionally substituted C₆-C₁₂-aryl - is an optionally substituted 5-membered heterocyclic ring containing 3 N which, in particular, is optionally substituted 1,2,3-triazolyl.

In connection with R¹ and the pyrrolidine derivatives wherein R⁴ is optionally substituted C₆-C₁₂-aryl, substituted 5-membered heterocyclic ring in particular includes pyrazolyl, imidazolyl, and triazolyl, which are substituted with 1, 2 or 3 substituents independently selected from the group consisting of halogen, C₁-C₆-alkyl, halogenated C₁-C₆-alkyl, C₃-C₆-cycloalkyl, C₁-C₄-alkoxy-carbonyl and C₁-C₆-alkyl-carbonylamino. Preferably, the substituent(s) on the heterocyclic ring are independently selected from the group consisting of halogen and C₁-C₆-alkyl. In particular, the substituent(s) on the heterocyclic ring are independently selected from the group consisting of C₁-C₆-alkyl, especially C₁-C₄-alkyl (e.g. methyl). According to a particular embodiment, 1,3-diazolyl is substituted with halogen or C₁-C₆-alkyl as described herein. According to a specific embodiment, R¹ is 1-methyl-1,3-diazol-4-yl.

In connection with R^{3a} and the pyrrolidine derivatives wherein R⁴ is optionally substituted C₆-C₁₂-aryl, substituted C₆-C₁₂-aryl in particular includes C₆-C₁₂-aryl, such as phenyl or naphthyl, which is substituted with 1, 2, or 3 substituents independently selected from the group consisting of halogen, C₁-C₄-alkyl and C₁-C₄-alkoxy. Preferably, the substituents on C₆-C₁₂-aryl are independently selected from the group consisting of halogen.

According to a preferred embodiment, R^{3a} - in the pyrrolidine derivatives wherein R⁴ is optionally substituted C₆-C₁₂-aryl - is C₆-C₁₂-aryl, in particular phenyl, optionally substituted with 1, 2, or 3 substituents independently selected from the group consisting of halogen (e.g. phenyl, 2-Cl-phenyl, 2-Br-phenyl, 3-Cl-phenyl, 4-Cl-phenyl, 2-F-phenyl, 3-F-phenyl, 4-F-phenyl, or 2,4,5-trifluoro-phenyl).

Further preferred embodiments of the pyrrolidine derivatives wherein R⁴ is optionally substituted C₆-C₁₂-aryl result if:
- R¹: is 1,3-diazolyl optionally substituted with halogen or C₁-C₄-alkyl and preferably 1-methyl-1,3-diazol-4-yl;
- R^{2a}, R^{2b}: are hydrogen;
- R^{3a}: is C₆-C₁₂-aryl optionally substituted with halogen (e.g. phenyl, 2-Cl-phenyl, 2-Br-phenyl, 3-Cl-phenyl, 4-Cl-phenyl, 2-F-phenyl, 3-F-phenyl, 4-F-phenyl, or 2,4,5-trifluoro-phenyl);
- R^{3b}: is hydrogen;
- R⁴: is C₆-C₁₂-aryl optionally substituted with 1, 2, or 3 substituents independently selected from the group consisting of halogen (e.g. phenyl, 4-Cl-phenyl, or 2-Cl-phenyl);
- Y¹: is >CR⁶;
- R⁶: is hydrogen; and
- R^{5a}, R^{5b}: are hydrogen.

Additional preferred embodiments of the pyrrolidine derivatives wherein R⁴ is optionally substituted C₆-C₁₂-aryl result if:
R⁴ is C₆-C₁₂-aryl optionally substituted with 1, 2, or 3 substituents independently selected from the group consisting of halogenated C₁-C₄-alkyl (e.g. 3-CF₃-phenyl), and
R¹, R^{2a}, R^{2b}, R^{3a}, R^{3b}, Y¹, R⁶, R^{5a} and R^{5b} are as defined above.

According to a further embodiment, R⁴ is -NR^{8a}(CR^{9a}R^{9b})ₙ₄R¹³. Thus, the present invention relates to the pyrrolidine derivatives of the formula (Id): 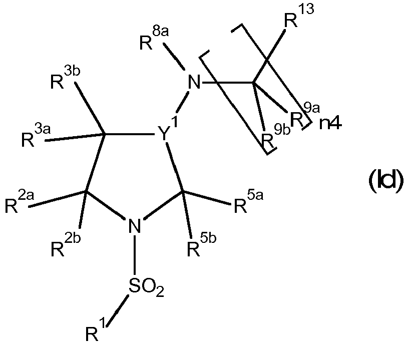 wherein R¹, R^{2a}, R^{2b}, R^{3a}, R^{3b}, Y¹, R^{5a} and R^{5b} are as defined herein, and
- R^{8a}: is hydrogen, C₁-C₆-alkyl (e.g. methyl, ethyl, n-propyl, iso-propyl, pentyl, or hexyl), or C₁-C₆-alkylcarbonyl (e.g. methylcarbonyl), or
- R⁶, R^{8a}: together are optionally substituted C₁-C₅-alkylene, wherein one or more -CH₂- of C₁-C₅-alkylene may be independently replaced by a an oxygen atom or C=O (e. g. -C(O)OCH₂-), or
- R^{3a} and R^{8a}: together are optionally substituted C₁-C₅-alkylene (e.g. 1,2-ethylene or 1,3-propylene);
- R^{9a}, R^{9b}: are independently hydrogen, halogen (e.g. F, Cl, or Br), C₁-C₆-alkyl (e.g. methyl, ethyl, tert-butyl, or 2,3-dimethyl-propyl), halogenated C₁-C₆-alkyl, (e.g. trifluoromethyl), hydroxy, or C₁-C₆-alkoxy (e.g. methoxy or ethoxy);
- n4: is 0, 1, 2, 3, or 4; and
- R¹³: is hydrogen, C₁-C₈-alkyl (e.g. methyl, ethyl, n-butyl, tert-butyl, pentyl, or hexyl), halogenated C₁-C₆-alkyl (e.g. -CF₃ or -CF₂Me), (optionally substituted C₃-C₁₂-cycloalkyl)-C₁-C₄-alkyl (e.g. cyclopropyl-methyl or cyclohexyl-methyl), C₁-C₆-alkoxy-C₁-C₆-alkyl (e.g. methoxy-methyl), optionally substituted C₃-C₁₂-cycloalkyl (e.g. cyclopropyl, 2-propyl-cyclopropyl, 1-(methoxy-methyl)-cyclopropyl, 2-phenyl-cyclopropyl, cyclopentyl, cyclopentyl, 3,3-dimethylcyclopentyl, cyclohexyl, 3,3-dimethyl-cyclohexyl, 4,4-dimethyl-cyclohexyl, 1-methyl-cyclohexyl, 1-CF₃-cyclopropyl, 4-CF₃-cyclohexyl, 3-CF₃-cyclohexyl, or 4,4-diF-cyclohexyl, a further example being 1-phenyl-cyclopropyl, 1-(4-F-phenyl)-cyclopropyl, (3-Cl-phenyl)-cyclopropyl, 2-phenyl-cyclobutyl, 3-phenyl-cyclobutyl, 3-(4-F-phenyl)-cyclobutyl, 3-(2-F-phenyl)-cyclobutyl, 2-Cl-cyclopentyl, 2-Me-cyclopentyl, 3-Me-cyclopentyl, 2-phenyl-cyclopentyl, 3-(2-pyridyl)-cyclopentyl, 3-Me-cyclohexyl, 3,3,5,5-tetraMe-cyclohexyl, 4-Me-cyclohexyl, 4-Et-cyclohexyl, 2-CF₃-cyclohexyl, 2-Me-5-isopropenyl-cyclohexyl, 2-phenyl-cyclohexyl, 3-phenyl-cyclohexyl, 4-phenyl-cyclohexyl, 2-EtO-cyclohexyl, decalin-1-yl, decalin-2-yl, norbornan-2-yl, 1,7,7-trimethyl-norbornan-2-yl, 5-iPr-2-Me-3-bicyclo[3.1.0]hexanyl, bicycle [3.2.1]octanyl, or 3-bicyclo[1.1.1]pentanyl), C₂-C₆-alkenyl (e.g. hex-2-enyl), optionally substituted C₃-C₆-cycloalkenyl (e.g. 1,3,3-trimethylcyclohexen-2-yl), optionally substituted C₆-C₁₂-aryl (e.g. phenyl, 2-F-phenyl, 4-F-phenyl, 3-F-phenyl, 2-Cl-phenyl, 3-Cl-phenyl, 4-Cl-phenyl, 2,3-diF-phenyl, 2,4-diF-phenyl, 2,5-diF-phenyl, 3,4-diF-phenyl, 2,3,4-triF-phenyl, 2,4-diCl-phenyl, 3,4-diCl-phenyl, 3-F-4-Cl-phenyl, 3-Cl-4-F-phenyl, 3-Cl-5-F-phenyl, 3,4-diF-5-Cl-phenyl, 2-Me-phenyl, 3-Me-phenyl, 4-Me-phenyl, 3-iPr-phenyl, 3-tBu-phenyl, 3-Me-4-F-phenyl, 3-Me-4-Cl-phenyl, 3-iPr-4-Cl-phenyl, 3-(1-OH-1-CF₃-Et)-phenyl, 3-CHF₂-4-F-phenyl, 2-CF₃-phenyl, 3-CF₃-phenyl, 4-CF₃-phenyl, 3-Cl-4-CF₃-phenyl, 2-F-3-CF₃-phenyl, 2-F-5-CF₃-phenyl, 3-F-5-CF₃-phenyl, 2-Cl-4-CF₃-phenyl, 2-Cl-3-CF₃-phenyl, 3-CF₃-4-F-phenyl, 3-CF₃-4-Cl-phenyl, 3-Me-4-CF₃-phenyl, 3-CF₃-4-Me-phenyl, 3,4-diCF₃-phenyl, 4-OMe-phenyl, 3-OiPr-phenyl, 3-CF₃-4-OMe-phenyl, 3-OCH₂CF₃-phenyl, 3-OCF₃-phenyl, 2-OCHF₂-5-Cl-phenyl, 3-OCF₃-4-F-phenyl, 3-OCF₃-4-Cl-phenyl, 3-OBn-phenyl, 3-OPh-phenyl, 3-CN-phenyl, 4-CN-phenyl, 2-F-3-CN-phenyl, 2-F-4-CN-phenyl, 3-F-4-CN-phenyl, 2-F-5-CN-phenyl, 3-F-5-CN-phenyl, 3-Cl-4-CN-phenyl, 2-Cl-5-CN-phenyl, 3-Cl-5-CN-phenyl, 3-CN-4-Cl-phenyl, 2-Me-3-CN-phenyl, 2-Me-5-CN-phenyl, 3-Me-5-CN-phenyl, 3-CF₃-4-CN-phenyl, 3-CN-4-OMe-phenyl, 3-CN-4-OCF₃-phenyl, 3-phenyl-phenyl, 3-MeSO₂-phenyl, 3-(piperidin-4-yl)-phenyl, 2-methylcarbonylamino-5-Cl-phenyl, 3-(pyrid-2-yl)-phenyl, 3-(pyrid-3-yl)-4 F-phenyl, 3-(pyrid-4-yl)-4 F-phenyl, 3-(pyrimid-5-yl)-4 F-phenyl, indan-5-yl, 2-chlor-indan-5-yl, or tetralin-6-yl, a further example being 2,4-diF-3-Cl-phenyl, 3-Et-4-Cl-phenyl, 3-(2-methoxyethyl)-phenyl, 3-(2-OH-ethyl)-phenyl, 3-(1-OH-1-Me-ethyl)-phenyl, 3-CHF₂-phenyl, 3-CF₃-2,4-diF-phenyl, 3-(dimethylamino-methyl)-phenyl, 3-(morpholin-4-yl-methyl)-phenyl, 3-cyclopropyl-phenyl, 3-OEt-phenyl, 3-OPr-phenyl, 3-OtBu-phenyl, 3-(cyclopropylmethoxy)-phenyl, 3-(OMe-methoxy)-phenyl, 3-(2-dimethylamino-ethoxy)-phenyl, 3-CF₃-4-OH-phenyl, 3-OCH₂CHF₂-phenyl, 3-OCHF₂-phenyl, 3-OCHF₂-4-F-phenyl, 3-OCF₃-4-OMe-phenyl, 3-cyclopropoxy-phenyl, 3-methylcarbonyl-phenyl, 3-dimethylamino-phenyl, 3-(2-pyridyloxy)-phenyl, 3-(pyrimidin-2-yloxy)-phenyl, indanyl, in-dan-2-yl, 2-F-indanyl, 4-F-indanyl, 5-F-indanyl, 6-F-indanyl, 7-F-indanyl, 3-Me-indanyl, 4-Me-indanyl, 5-Me-indanyl, 6-Me-indanyl, 4-CF₃-indanyl, 5-CF₃-indanyl, 6-CF₃-indanyl, 3,3-dimethyl-indanyl, tetralin-2-yl, 7-F-tetralin-2-yl, 6-F-tetralin-2-yl, tetralinyl, 6-F-tetralinyl, 5-F-tetralinyl, or 7-F-tetralinyl), hydroxy, C₁-C₆-alkoxy (e.g. methoxy, ethoxy, or n-propyloxy), C₁-C₆-alkoxy-C₁-C₄-alkoxy (e.g. 2-methoxy-ethoxy), optionally substituted C₆-C₁₂-aryloxy (e.g. 4-F-phenoxy or 4-tertbutyl-phenoxy), optionally substituted C₃-C₁₂-heterocyclyloxy (e.g. pyridin-2-yloxy), optionally substituted C₃-C₁₂-heterocyclyl (e.g. propyl-furan-2-yl, 2-CF₃-furan-5-yl, 1,2-dimethyl-5-CN-pyrrol-3-yl, 2,3-diMe-thiophen-5-yl, 2-Me-thiophen-5-yl, 2-Cl-thiophen-5-yl, 3-Cl-thiophen-2-yl, 2,5-diCl-thiophen-3-yl, 2-tetrahydropyranyl-thiophen-5-yl, 1,3-thiazol-5-yl, 4-Me-1,3-thiazol-2-yl, 2-Me-1,3-thiazol-5-yl, 5-Me-1,3-thiazol-2-yl, 4-Me-1,3-thiazol-5-yl, 2-Me-1,3-thiazol-4-yl, 4-iso-propyl-1,3-thiazol-2-yl, 2,4-diMe-1,3-thiazol-5-yl, 2-phenyl-4-Me-1,3-thiazol-5-yl, 4-phenyl-1,3-thiazol-5-yl, 2-(4-Me-phenyl)-1,3-thiazol-5-yl, 4-(4-F-phenyl)-1,3-thiazol-2-yl, 2-Cl-1,3-thiazol-4-yl, 4-Cl-1,3-thiazol-5-yl, 2-Br-1,3-thiazol-5-yl, 4-Br-1,3-thiazol-2-yl, 4-Me-5-Br-1,3-thiazol-2-yl, 2,4-dichloro-1,3-thiazol-5-yl, 1,5-dimethyl-1,2,4-triazol-3-yl, pyrid-2-yl, pyrid-3-yl, pyrid-4-yl, 4-F-pyrid-2-yl, 5-F-pyrid-2-yl, 6-F-pyrid-2-yl, 4-Cl-pyrid-2-yl, 5-F-pyrid-3-yl, 2-F-pyrid-4-yl, 3,5-diCl-pyrid-4-yl, 4,5-diCl-pyrid-2-yl, 2-Cl-3F-pyrid-4-yl, 5-Me-pyrid-2-yl, 4-iPr-pyrid-2-yl, 4-Me-5-F-pyrid-2-yl, 4-CF₃-pyrid-2-yl, 5-CF₃-pyrid-2-yl, 6-CF₃-pyrid-2-yl, 5-CF₃-pyrid-3-yl, 2-CF₃-pyrid-4-yl, 3-F-4-CF₃-pyrid-2-yl, 4-CF₃-5-F-pyrid-2-yl, 4-CF₃-5-Cl-pyrid-2-yl, 3,5-diF-4-CF₃-pyrid-2-yl, 4-OCH₂CF₃-pyrid-2-yl, 4-OCH₂CF₃-5-F-pyrid-2-yl, 4-OCH₂CF₃-5-Cl-pyrid-2-yl, 4-OBn-5-F-pyrid-2-yl, 3-(4-F-phenyl)-pyrid-5-yl, 2-(4-F-phenyl)-pyrid-3-yl, 4-(pyrid-4-yl)-pyrid-2-yl, 4-(pyrid-3-yl)-pyrid-2-yl, 5-cyclopropyl-pyraz-2-yl, 5-cyclobutyl-pyraz-2-yl, 5-pyrrolidin-pyraz-2-yl, pyridaz-3-yl, 4-CF₃-pyridaz-3-yl, 5-CF₃-pyridaz-3-yl, 5-F-pyrimid-2-yl, 6-Cl-pyrimid-4-yl, 6-Me-pyrimid-4-yl, 6-Et-pyrimid-4-yl, 6-Pr-pyrimid-4-yl, 6-iPr-pyrimid-4-yl, 4-CF₃-pyrimid-2-yl, 6-CF₃-pyrimid-4-yl, 2-Me-6-Cl-pyrimid-4-yl, 2-Me-6-CF₃-pyrimid-4-yl, 2-OMe-6-CF₃-pyrimid-4-yl, 2-OMe-pyrimid-4-yl, 6-OMe-pyrimid-4-yl, 6-OEt-pyrimid-4-yl, 6-OPr-pyrimid-4-yl, 6-OiPr-pyrimid-4-yl, 6-OiBu-pyrimid-4-yl, 6-OBu-pyrimid-4-yl, 6-OBn-pyrimid-4-yl, 6-cyclobutyloxy-pyrimid-4-yl, 6-cyclopentyloxy-pyrimid-4-yl, 6-cyclohexyloxy-pyrimid-4-yl, 6-cyclopropyl-pyrimid-4-yl, 6-phenyl-pyrimid-4-yl, 6-(2-F-phenyl)-pyrimid-4-yl, 6-(3-F- phenyl)-pyrimid-4-yl, 6-(4-F- phenyl)-pyrimid-4-yl, 6-Cl-pyrimid-4-yl, 6-(2-Me- phenyl)-pyrimid-4-yl, 6-(3-Me- phenyl)-pyrimid-4-yl, 6-(4-Me-phenyl)-pyrimid-4-yl, 6-(3-Cl- phenyl)-pyrimid-4-yl, 6-(4-Cl- phenyl)-pyrimid-4-yl, 2-(morpholin-1-yl)-pyrimid-4-yl, 6-(azetidin-3-yl-methoxy)-pyrimid-4-yl, 6-(pyrrolidin-3-yl-methoxy)-pyrimid-4-yl, 6-(pyrrolidin-2-yl-methoxy)-pyrimid-4-yl, benzofuran-5-yl, 2-Me-benzofuran-3-yl, 2-Et-benzofuran-3-yl, benzothiophen-5-yl, benzothiophen-6-yl, 5-Me-benzothiophen-2-yl, 5-F-benzothiophen-2-yl, 3-Cl-benzothiophen-2-yl, benzothiazol-2-yl, isoquinolin-6-yl, isoquinolin-7-yl, quinolin-6-yl, quinolin-7-yl, 4,5,6,7-tetrahydro-1,3-benzothiazol-2-yl, 2,3-dihydrobenzofuran-5-yl, 7H-pyrrolo[2,3-d]pyrimidin-4-yl, pyrazolo[1,5-a]pyridine-3-yl, pyrazolo[1,5-a]pyridine-7-yl, imidazo[1,2-a]pyridin-8-yl, 5-methyl-imidazo[1,2-a]pyridin-3-yl, 6-chloro-2-methyl-imidazo[1,2-a]pyridin-3-yl, 6-bromo-2-methyl-imidazo[1,2-a]pyridin-3-yl, thieno[2,3-b]pyridin-2-yl, N-benzyl-indolin-6-yl, indolinon-4-yl, chroman-6-yl, 4,4-dimethylchroman-6-yl, 4,4-dimethyl-1,3-dioxan-2-yl, 2-Metetrahydrofuran-3-yl, tetrahydrofuran-3-yl, tetrahydropyran-4-yl, 4-Me-tetrahydropyran-4-yl, 2,2-diMe-tetrahydropyran-4-yl, 1-iso-propyl-piperidin-4-yl, 1-ethyl-piperidin-3-yl, or 2-CF₃-piperazin-5-yl, a further example being iBu-pyrazol-4-yl, 1-CHF₂-5-Me-1,2-diazol-4-yl, 1-benzyl-5-F-1,2,4-triazol-3-yl, 2-Me-pyrid-4-yl, 2-iPr-pyrid-4-yl, 4-phenyl-pyrid-2yl, 2-cyclopropyl-pyrid-4-yl, 2-OMe-pyrid-4-yl, 2-OEt-pyrid-4-yl, 2-cyclopropylmethoxy-pyrid-4yl, 2-OiPr-pyrid-4-yl, 2-OCH₂CF₃-pyrid-4-yl, 2-cyclopropoxy-pyrid-4-yl, 2-cyclobutoxypyrid-4-yl, 4,5-diCl-pyrimidin-2yl, benzothiophen-3-yl, 2,2-diF-1,3-benzodioxol-5-yl, 5,6,7,8-tetrahydro-isoquinolin-5-yl, 5,6,7,8-tetrahydro-isoquinolin-8-yl, quinolin-8-yl, 1-Me-3,4-dihydro-2H-quinolin-4-yl, 2-Me-3,4-dihydro-1H-isoquinolin-6-yl, 5,6,7,8-tetrahydro-quinolin-5-yl, 5,6,7,8-tetrahydro-quinolin-8-yl, 5,6,7,8-tetrahydro-quinolin-6-yl, 2,3-dihydrobenzofuran-3-yl, pyrazolo[1,5-a]pyrimidin-6-yl, imidazo[1,5-a]pyrazinyl, 3-methyl-imidazo[1,5-a]pyrazinyl, 3-Me-[1,2,4]triazolo[4,3-a]pyridin-6-yl, imidazo[1,2-a]pyrazin-6-yl, 2-Me-isoindolin-1-on-6-yl, 2,2-dimethyl-7-CF₃-chroman-4-yl, 7-CF₃-chroman-4-yl, 7-OCF₃-chroman-4-yl, isochroman-4-yl, 6,6-dimethyl-1,3-dioxan-2-yl, tetrahydropyran-3-yl, 1-phenyl-pyrrolidin-3-yl, piperidin-3yl, 1,3-dimethyl-piperidin-4yl, 1-cyclopropyl-piperidin-4-yl, 1-(tri-F-methyl-carbonyl)-piperidin-3-yl, quinuclidin-2-yl, 6,7-dihydro-5H-cyclopenta[b]pyridine-7-yl, 6,7-dihydro-5H-cyclopenta[b]pyridine-5-yl, 6,7-dihydro-5H-cyclopenta[c]pyridine-7-yl, 6,7-dihydro-5H-cyclopenta[c]pyridine-5-yl, 5,6-dihydro-4H-cyclopenta[b]thiophen-4-yl, or 1,2,3,5,6,7,8,8a-octahydroindolizin), or tri-(C₁-C₄-alkyl)-silyloxy.

Further embodiments of pyrrolidine derivatives of formula (Id) result if
- R^{8a}: is hydrogen, C₁-C₆-alkyl (e.g. methyl, ethyl, n-propyl, iso-propyl, pentyl, or hexyl), or C₁-C₆-alkylcarbonyl (e.g. methylcarbonyl), or
- R⁶, R^{8a}: together are optionally substituted C₁-C₅-alkylene, wherein one or more -CH₂- of C₁-C₅-alkylene may be independently replaced by a an oxygen atom or C=O (e. g. -C(O)OCH₂-);
- R^{9a}, R^{9b}: are independently hydrogen, halogen (e.g. F, Cl, or Br), C₁-C₆-alkyl (e.g. methyl, ethyl, tert-butyl, or 2,3-dimethyl-propyl), hydroxy, or C₁-C₆-alkoxy (e.g. methoxy or ethoxy);
and R¹, R^{2a}, R^{2b}, R^{3a}, R^{3b}, Y¹, R^{5a}, R^{5b}, n4, R¹³ are as defined above.

In particular, R^{9a}, R^{9b} are independently hydrogen, halogen, C₁-C₆-alkyl (e.g. methyl, ethyl, tert-butyl, or 2,3-dimethyl-propyl), or C₁-C₆-alkoxy (e.g. methoxy). In particular, R^{9a}, R^{9b} may be independently halogenated C₁-C₆-alkyl (e.g. trifluoromethyl).

In particular, R¹³ is hydrogen, C₁-C₈-alkyl (e.g. methyl, ethyl, n-butyl, tert-butyl, pentyl, or hexyl), halogenated C₁-C₆-alkyl (e.g. -CF₃ or -CF₂Me), C₁-C₆-alkoxy-C₁-C₆-alkyl (e.g. methoxy-methyl), optionally substituted C₃-C₂-cycloalkyl (e.g. cyclopropyl, 2-propyl-cyclopropyl, 1-(methoxy-methyl)-cyclopropyl, 2-phenyl-cyclopropyl, cyclopentyl, cyclopentyl, 3,3-dimethylcyclopentyl, cyclohexyl, 3,3-dimethyl-cyclohexyl, 4,4-dimethyl-cyclohexyl, 1-methyl-cyclohexyl, 1-CF₃-cyclopropyl, 4-CF₃-cyclohexyl, 3-CF₃-cyclohexyl, or 4,4-diF-cyclohexyl, a further example being 1-phenyl-cyclopropyl, 1-(4-F-phenyl)-cyclopropyl, (3-Cl-phenyl)-cyclopropyl, 2-phenyl-cyclobutyl, 3-phenyl-cyclobutyl, 3-(4-F-phenyl)-cyclobutyl, 3-(2-F-phenyl)-cyclobutyl, 2-Cl-cyclopentyl, 2-Me-cyclopentyl, 3-Me-cyclopentyl, 2-phenyl-cyclopentyl, 3-(2-pyridyl)-cyclopentyl, 3-Me-cyclohexyl, 3,3,5,5-tetraMe-cyclohexyl, 4-Me-cyclohexyl, 4-Et-cyclohexyl, 2-CF₃-cyclohexyl, 2-Me-5-isopropenyl-cyclohexyl, 2-phenyl-cyclohexyl, 3-phenyl-cyclohexyl, 4-phenyl-cyclohexyl, 2-EtO-cyclohexyl, decalin-1-yl, decalin-2-yl, norbornan-2-yl, 1,7,7-trimethyl-norbornan-2-yl, 5-iPr-2-Me-3-bicyclo[3.1.0]hexanyl, bicycle [3.2.1]octanyl, or 3-bicyclo[1.1.1]pentanyl), C₂-C₆-alkenyl (e.g. hex-2-enyl), optionally substituted C₃-C₆-cycloalkenyl (e.g. 1,3,3-trimethylcyclohexen-2-yl), optionally substituted C₆-C₁₂-aryl (e.g. phenyl, 2-F-phenyl, 4-F-phenyl, 3-F-phenyl, 2-Cl-phenyl, 3-Cl-phenyl, 4-Cl-phenyl, 2,3-diF-phenyl, 2,4-diF-phenyl, 2,5-diF-phenyl, 3,4-diF-phenyl, 2,3,4-triF-phenyl, 2,4-diCl-phenyl, 3,4-diCl-phenyl, 3-F-4-Cl-phenyl, 3-Cl-4-F-phenyl, 3-Cl-5-F-phenyl, 3,4-diF-5-Cl-phenyl, 2-Me-phenyl, 3-Me-phenyl, 4-Me-phenyl, 3-iPr-phenyl, 3-tBu-phenyl, 3-Me-4-F-phenyl, 3-Me-4-Cl-phenyl, 3-iPr-4 Cl-phenyl, 3-(1-OH-1-CF₃-Et)-phenyl, 3-CHF₂-4-F-phenyl, 2-CF₃-phenyl, 3-CF₃-phenyl, 4-CF₃-phenyl, 3-Cl-4-CF₃-phenyl, 2-F-3-CF₃-phenyl, 2-F-5-CF₃-phenyl, 3-F-5-CF₃-phenyl, 2-Cl-4-CF₃-phenyl, 2-Cl-3-CF₃-phenyl, 3-CF₃-4-F-phenyl, 3-CF₃-4-Cl-phenyl, 3-Me-4-CF₃-phenyl, 3-CF₃-4-Me-phenyl, 3,4-diCF₃-phenyl, 4-OMe-phenyl, 3-OiPr-phenyl, 3-CF₃-4-OMe-phenyl, 3-OCH₂CF₃-phenyl, 3-OCF₃-phenyl, 2-OCHF₂-5-Cl-phenyl, 3-OCF₃-4-F-phenyl, 3-OCF₃-4-Cl-phenyl, 3-OBn-phenyl, 3-OPh-phenyl, 3-CN-phenyl, 4-CN-phenyl, 2-F-3-CN-phenyl, 2-F-4-CN-phenyl, 3-F-4-CN-phenyl, 2-F-5-CN-phenyl, 3-F-5-CN-phenyl, 3-Cl-4-CN-phenyl, 2-Cl-5-CN-phenyl, 3-Cl-5-CN-phenyl, 3-CN-4-Cl-phenyl, 2-Me-3-CN-phenyl, 2-Me-5-CN-phenyl, 3-Me-5-CN-phenyl, 3-CF₃-4-CN-phenyl, 3-CN-4-OMe-phenyl, 3-CN-4-OCF₃-phenyl, 3-phenyl-phenyl, 3-MeSO₂-phenyl, 3-(piperidin-4-yl)-phenyl, 2-methylcarbonylamino-5-Cl-phenyl, 3-(pyrid-2-yl)-phenyl, 3-(pyrid-3-yl)-4 F-phenyl, 3-(pyrid-4-yl)-4 F-phenyl, 3-(pyrimid-5-yl)-4 F-phenyl, indan-5-yl, 2-chlor-indan-5-yl, or tetralin-6-yl, a further example being 2,4-diF-3-Cl-phenyl, 3-Et-4-Cl-phenyl, 3-(2-methoxyethyl)-phenyl, 3-(2-OH-ethyl)-phenyl, 3-(1-OH-1-Me-ethyl)-phenyl, 3-CHF₂-phenyl, 3-CF₃-2,4-diF-phenyl, 3-(dimethylamino-methyl)-phenyl, 3-(morpholin-4-yl-methyl)-phenyl, 3-cyclopropyl-phenyl, 3-OEt-phenyl, 3-OPr-phenyl, 3-OtBu-phenyl, 3-(cyclopropylmethoxy)-phenyl, 3-(OMe-methoxy)-phenyl, 3-(2-dimethylamino-ethoxy)-phenyl, 3-CF₃-4-OH-phenyl, 3-OCH₂CHF₂-phenyl, 3-OCHF₂-phenyl, 3-OCHF₂-4-F-phenyl, 3-OCF₃-4-OMe-phenyl, 3-cyclopropoxy-phenyl, 3-methylcarbonyl-phenyl, 3-dimethylamino-phenyl, 3-(2-pyridyloxy)-phenyl, 3-(pyrimidin-2-yloxy)-phenyl, indanyl, indan-2-yl, 2-F-indanyl, 4-F-indanyl, 5-F-indanyl, 6-F-indanyl, 7-F-indanyl, 3-Me-indanyl, 4-Me-indanyl, 5-Me-indanyl, 6-Me-indanyl, 4-CF₃-indanyl, 5-CF₃-indanyl, 6-CF₃-indanyl, 3,3-dimethyl-indanyl, tetralin-2-yl, 7-F-tetralin-2-yl, 6-F-tetralin-2-yl, tetralinyl, 6-F-tetralinyl, 5-F-tetralinyl, or 7-F-tetralinyl), C₁-C₆-alkoxy (e.g. methoxy, ethoxy, or n-propyloxy), C₁-C₆-alkoxy-C₁-C₄-alkoxy (e.g. 2-methoxy-ethoxy), optionally substituted C₆-C₁₂-aryloxy (e.g. 4-F-phenoxy or 4-tert-butyl-phenoxy), optionally substituted C₃-C₁₂-heterocyclyl (e.g. e.g. n-propyl-furan-2-yl, 2-CF₃-furan-5-yl, 1,2-dimethyl-5-CN-pyrrol-3-yl, 2,3-diMe-thiophen-5-yl, 2-Me-thiophen-5-yl, 2-Cl-thiophen-5-yl, 3-Cl-thiophen-2-yl, 2,5-diCl-thiophen-3-yl, 2-tetrahydropyranyl-thiophen-5-yl, 1,3-thiazol-5-yl, 4-Me-1,3-thiazol-2-yl, 2-Me-1,3-thiazol-5-yl, 5-Me-1,3-thiazol-2-yl, 4-Me-1,3-thiazol-5-yl, 2-Me-1,3-thiazol-4-yl, 4-iso-propyl-1,3-thiazol-2-yl, 2,4-diMe-1,3-thiazol-5-yl, 2-phenyl-4-Me-1,3-thiazol-5-yl, 4-phenyl-1,3-thiazol-5-yl, 2-(4-Me-phenyl)-1,3-thiazol-5-yl, 4-(4-F-phenyl)-1,3-thiazol-2-yl, 2-Cl-1,3-thiazol-4-yl, 4-Cl-1,3-thiazol-5-yl, 2-Br-1,3-thiazol-5-yl, 4-Br-1,3-thiazol-2-yl, 4-Me-5-Br-1,3-thiazol-2-yl, 2,4-dichloro-1,3-thiazol-5-yl, 1,5-dimethyl-1,2,4-triazol-3-yl, pyrid-2-yl, pyrid-3-yl, pyrid-4-yl, 4-F-pyrid-2-yl, 5-F-pyrid-2-yl, 6-F-pyrid-2-yl, 4-Cl-pyrid-2-yl, 5-F-pyrid-3-yl, 2-F-pyrid-4-yl, 3,5-diCl-pyrid-4-yl, 4,5-diCl-pyrid-2-yl, 2-Cl-3F-pyrid-4-yl, 5-Me-pyrid-2-yl, 4-iPr-pyrid-2-yl, 4-Me-5-F-pyrid-2-yl, 4-CF₃-pyrid-2-yl, 5-CF₃-pyrid-2-yl, 6-CF₃-pyrid-2-yl, 5-CF₃-pyrid-3-yl, 2-CF₃-pyrid-4-yl, 3-F-4-CF₃-pyrid-2-yl, 4-CF₃-5-F-pyrid-2-yl, 4-CF₃-5-Cl-pyrid-2-yl, 3,5-diF-4-CF₃-pyrid-2-yl, 4-OCH₂CF₃-pyrid-2-yl, 4-OCH₂CF₃-5-F-pyrid-2-yl, 4-OCH₂CF₃-5-Cl-pyrid-2-yl, 4-OBn-5-F-pyrid-2-yl, 3-(4-F-phenyl)-pyrid-5-yl, 2-(4-F-phenyl)-pyrid-3-yl, 4-(pyrid-4-yl)-pyrid-2-yl, 4-(pyrid-3-yl)-pyrid-2-yl, 5-cyclopropyl-pyraz-2-yl, 5-cyclobutyl-pyraz-2-yl, 5-pyrrolidin-pyraz-2-yl, pyridaz-3-yl, 4-CF₃-pyridaz-3-yl, 5-CF₃-pyridaz-3-yl, 5-F-pyrimid-2-yl, 6-Cl-pyrimid-4-yl, 6-Me-pyrimid-4-yl, 6-Et-pyrimid-4-yl, 6-Pr-pyrimid-4-yl, 6-iPr-pyrimid-4-yl, 4-CF₃-pyrimid-2-yl, 6-CF₃-pyrimid-4-yl, 2-Me-6-Cl-pyrimid-4-yl, 2-Me-6-CF₃-pyrimid-4-yl, 2-OMe-6-CF₃-pyrimid-4-yl, 2-OMe-pyrimid-4-yl, 6-OMe-pyrimid-4-yl, 6-OEt-pyrimid-4-yl, 6-OPr-pyrimid-4-yl, 6-OiPr-pyrimid-4-yl, 6-OiBu-pyrimid-4-yl, 6-OBu-pyrimid-4-yl, 6-OBn-pyrimid-4-yl, 6-cyclobutylloxy-pyrimid-4-yl, 6-cyclopentyloxy-pyrimid-4-yl, 6-cyclohexyloxy-pyrimid-4-yl, 6-cyclopropyl-pyrimid-4-yl, 6-phenyl-pyrimid-4-yl, 6-(2-F- phenyl)-pyrimid-4-yl, 6-(3-F- phenyl)-pyrimid-4-yl, 6-(4-F- phenyl)-pyrimid-4-yl, 6-Cl-pyrimid-4-yl, 6-(2-Me- phenyl)-pyrimid-4-yl, 6-(3-Me- phenyl)-pyrimid-4-yl, 6-(4-Me- phenyl)-pyrimid-4-yl, 6-(3-Cl- phenyl)-pyrimid-4-yl, 6-(4-Cl- phenyl)-pyrimid-4-yl, 2-(morpholin-1-yl)-pyrimid-4-yl, 6-(azetidin-3-yl-methoxy)-pyrimid-4-yl, 6-(pyrrolidin-3-yl-methoxy)-pyrimid-4-yl, 6-(pyrrolidin-2-yl-methoxy)-pyrimid-4-yl, benzofuran-5-yl, 2-Me-benzofuran-3-yl, 2-Et-benzofuran-3-yl, benzothiophen-5-yl, benzothiophen-6-yl, 5-Me-benzothiophen-2-yl, 5-F-benzothiophen-2-yl, 3-Cl-benzothiophen-2-yl, benzothiazol-2-yl, isoquinolin-6-yl, isoquinolin-7-yl, quinolin-6-yl, quinolin-7-yl, 4,5,6,7-tetrahydro-1,3-benzothiazol-2-yl, 2,3-dihydrobenzofuran-5-yl, 7H-pyrrolo[2,3-d]pyrimidin-4-yl, pyrazolo[1,5-a]pyridine-3-yl, pyrazolo[1,5-a]pyridine-7-yl, imidazo[1,2-a]pyridin-8-yl, 5-methyl-imidazo[1,2-a]pyridin-3-yl, 6-chloro-2-methyl-imidazo[1,2-a]pyridin-3-yl, 6-bromo-2-methyl-imidazo[1,2-a]pyridin-3-yl, thieno[2,3-b]pyridin-2-yl, N-benzyl-indolin-6-yl, indolinon-4-yl, chroman-6-yl, 4,4-dimethylchroman-6-yl, 4,4-dimethyl-1,3-dioxan-2-yl, 2-Me-tetrahydrofuran-3-yl, tetrahydrofuran-3-yl, tetrahydropyran-4-yl, 4-Me-tetrahydropyran-4-yl, 2,2-diMe-tetrahydropyran-4-yl, 1-isopropyl-piperidin-4-yl, 1-ethyl-piperidin-3-yl, or 2-CF₃-piperazin-5-yl, a further example being iBu-pyrazol-4-yl, 1-CHF₂-5-Me-1,2-diazol-4-yl, 1-benzyl-5-F-1,2,4-triazol-3-yl, 2-Me-pyrid-4-yl, 2-iPr-pyrid-4-yl, 4-phenyl-pyrid-2yl, 2-cyclopropyl-pyrid-4-yl, 2-OMe-pyrid-4-yl, 2-OEt-pyrid-4-yl, 2-cyclopropylmethoxy-pyrid-4yl, 2-OiPr-pyrid-4-yl, 2-OCH₂CF₃-pyrid-4-yl, 2-cyclopropoxy-pyrid-4-yl, 2-cyclobutoxy-pyrid-4-yl, 4,5-diCl-pyrimidin-2yl, benzothiophen-3-yl, 2,2-diF-1,3-benzodioxol-5-yl, 5,6,7,8-tetrahydro-isoquinolin-5-yl, 5,6,7,8-tetrahydro-isoquinolin-8-yl, quinolin-8-yl, 1-Me-3,4-dihydro-2H-quinolin-4-yl, 2-Me-3,4-dihydro-1H-isoquinolin-6-yl, 5,6,7,8-tetrahydro-quinolin-5-yl, 5,6,7,8-tetrahydro-quinolin-8-yl, 5,6,7,8-tetrahydro-quinolin-6-yl, 2,3-dihydrobenzofuran-3-yl, pyrazolo[1,5-a]pyrimidin-6-yl, imidazo[1,5-a]pyrazinyl, 3-methyl-imidazo[1,5-a]pyrazinyl, 3-Me-[1,2,4]triazolo[4,3-a]pyridin-6-yl, imidazo[1,2-a]pyrazin-6-yl, 2-Me-isoindolin-1-on-6-yl, 2,2-dimethyl-7-CF₃-chroman-4-yl, 7-CF₃-chroman-4-yl, 7-OCF₃-chroman-4-yl, isochroman-4-yl, 6,6-dimethyl-1,3-dioxan-2-yl, tetrahydropyran-3-yl, 1-phenyl-pyrrolidin-3-yl, piperidin-3-yl, 1,3-dimethyl-piperidin-4-yl, 1-cyclopropyl-piperidin-4-yl, 1-(tri-F-methyl-carbonyl)-piperidin-3-yl, quinuclidin-2-yl, 6,7-dihydro-5H-cyclopenta[b]pyridine-7-yl, 6,7-dihydro-5H-cyclopenta[b]pyridine-5-yl, 6,7-dihydro-5H-cyclopenta[c]pyridine-7-yl, 6,7-dihydro-5H-cyclopenta[c]pyridine-5-yl, 5,6-dihydro-4H-cyclopenta[b]thiophen-4-yl, or 1,2,3,5,6,7,8,8a-octahydroindolizin), or tri-(C₁-C₄-alkyl)-silyloxy.

R^{2a}, R^{2b} - in the pyrrolidine derivatives of formula (Id) - are, in particular, hydrogen.

R^{3a} - in the pyrrolidine derivatives of formula (Id) - is, in particular, C₃-C₂-cycloalkyl (e.g. cyclopropyl, cyclobutyl, cyclopentyl, or cyclohexyl), optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl, a further example being 2-Me-phenyl, 3-Me-phenyl, or 4-Me-phenyl), hydroxy, C₁-C₆-alkoxy (e.g.methoxy, ethoxy, n-propoxy, iso-propoxy, or iso-butoxy), halogenated C₁-C₆-alkoxy (e.g OCF₃), C₃-C₁₂-cycloalkyl-C₁-C₄-alkoxy (e.g. cyclopropylmethoxy or cyclohexylmethoxy), C₂-C₆-alkenyloxy (e.g. allyloxy or 2-methylprop-2-en-1-oxy), C₆-C₁₂-aryl-C₁-C₄-alkoxy (e.g. benzyloxy), C₃-C₁₂-heterocyclyl-C₁-C₄-alkoxy (e.g. 2-(N-pyrrolidinyl)ethoxy, 2-(N-morpholinyl)ethoxy and 2-(N-imidazolyl)ethoxy), optionally substituted C₆-C₁₂-aryloxy (e.g. phenoxy or 4-F-phenoxy), C₃-C₁₂-heterocyclyloxy (e.g. pyridin-2-yloxy), or optionally substituted C₃-C₁₂-heterocyclyl (tetrahydrofuran-2-yl, tetrahydrofuran-3-yl, tetrahydropyran-2-yl, tetrahydropyran-3-yl, 1-cyclopropyl-piperidin-4-yl, 1-cyclopropyl-piperidin-3-yl, 2-pyridyl, 3-pyridyl, 3-F-pyrid-2-yl, 1,3-oxazol-4-yl, or 1,3-oxazol-2-yl, a further example being 1,3-dioxan-2-yl, 5,5-dimethyl-1,3-dioxan-2-yl, 4,6-dimethyl-1,3-dioxan-2-yl, 1,3-dioxepan-2-yl, 1,3-dioxolan-2-yl, 5,7-dioxaspiro[2.5]octan-6-yl, morpholin-3-yl, morpholin-4-yl, pyrrolidinyl, piperidyl, 4-F-piperidyl, 4,4-diF-piperidyl, 1-Me-piperid-2-yl, or 5-F-pyrid-2-yl).

Preferably, R^{3a} is C₃-C₁₂-cycloalkyl (e.g. cyclopropyl, cyclobutyl, cyclopentyl, or cyclohexyl), optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl, a further example being 2-Me-phenyl, 3-Me-phenyl, or 4-Me-phenyl), hydroxy, C₁-C₆-alkoxy (e.g.methoxy, ethoxy, n-propoxy, iso-propoxy, or iso-butoxy), C₃-C₁₂-cycloalkyl-C₁-C₄-alkoxy (e.g. cyclopropylmethoxy or cyclohexylmethoxy), C₂-C₆-alkenyloxy (e.g. allyloxy or 2-methylprop-2-en-1-oxy), C₆-C₁₂-aryl-C₁-C₄-alkoxy (e.g. benzyloxy), optionally substituted C₆-C₁₂-aryloxy (e.g. phenoxy or 4-F-phenoxy) or optionally substituted C₃-C₂-heterocyclyl (tetrahydrofuran-2-yl, tetrahydrofuran-3-yl, tetrahydropyran-2-yl, tetrahydropyran-3-yl, 1-cyclopropyl-piperidin-4-yl, 1-cyclopropyl-piperidin-3-yl, 2-pyridyl, 3-pyridyl, 3-F-pyrid-2-yl, 1,3-oxazol-4-yl, or 1,3-oxazol-2-yl, a further example being 1,3-dioxan-2-yl, 5,5-dimethyl-1,3-dioxan-2-yl, 4,6-dimethyl-1,3-dioxan-2-yl, 1,3-dioxepan-2-yl, 1,3-dioxolan-2-yl, 5,7-dioxaspiro[2.5]octan-6-yl, morpholin-3-yl, morpholin-4-yl, pyrrolidinyl, piperidyl, 4-F-piperidyl, 4,4-diF-piperidyl, 1-Me-piperid-2-yl, or 5-F-pyrid-2-yl).

R^{3b} - in the pyrrolidine derivatives of formula (Id) - is, in particular, hydrogen or hydroxy.

Y¹ - in the pyrrolidine derivatives of formula (Id) - is, in particular, >CR⁶.

R⁶ - in the pyrrolidine derivatives of formula (Id) - is, in particular, hydrogen, C₁-C₆-alkyl (e.g. methyl), or hydroxy-C₁-C₆-alkyl (e.g. -CH2OH).

In particular, R⁶ and R^{3a} or R^{3b} together may be optionally substituted C₁-C₅-alkylene, preferably unsubstituted C₁-C₅-alkylene (e.g. 1,3-propylene or 1,4-butylene); or R⁶ may be C₁-C₄-alkylene (e.g. methylene or 1,2-ethylene) that is bound to a carbon atom in R^{3a}, and R^{3a} is an optionally substituted C₆-C₁₂-aryl (e.g. phenyl).

R^{5a}, R^{5b} - in the pyrrolidine derivatives of formula (Id) - are, in particular, hydrogen.

Particular embodiments of pyrrolidine derivatives of formula (Id) result if:
- R¹: is an optionally substituted 5-membered heterocyclic ring as defined herein (e.g. 1,2-diazol-4-yl, 1-methyl-1,2-diazol-4-yl, 1-methyl-1,2-diazol-3-yl, 1-methyl-1,3-diazol-4-yl, 1-methyl-1,2-diazol-5-yl, 1,5-dimethyl-1,2-diazol-4-yl, 1,3-dimethyl-1,2-diazol-4-yl, 1,5-dimethyl-1,2-diazol-4-yl, 1,3-dimethyl-1,2-diazol-5-yl, 1,3-dimethyl-5-Cl-1,2-diazol-4-yl, 1-isopropyl-3-methyl-1,2-diazol-4-yl, 1-Me-3-CF₃-1,2-diazol-4-yl, 1-cyclopentyl-3-Me-1,2-diazol-4-yl, 1,3,5-trimethyl-1,2-diazol-4-yl, 1-CHF₂-3,5-dimethyl-1,2-diazol-4-yl, 1,2-dimethyl-1,3-diazol-4-yl, 1,2-dimethyl-1,3-diazol-5-yl, 1-methyl-5-Cl-1,3-diazol-4-yl, 1-Me-1,2,3-triazol-4-yl, a further example being 1-ethyl-1,3-diazol-4-yl or 1-methyl-1,2,4-triazol-3-yl);
- R^{2a}, R^{2b}: are hydrogen;
- R^{3a}: is C₃-C₁₂-cycloalkyl (e.g. cyclopropyl, cyclobutyl, cyclopentyl, or cyclohexyl), optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl, a further example being 2-Me-phenyl, 3-Me-phenyl, or 4-Me-phenyl), hydroxy, C₁-C₆-alkoxy (e.g.methoxy, ethoxy, n-propoxy, isopropoxy, or iso-butoxy), C₃-C₁₂-cycloalkyl-C₁-C₄-alkoxy (e.g. cyclopropylmethoxy or cyclohexylmethoxy), C₂-C₆-alkenyloxy (e.g. allyloxy or 2-methylprop-2-en-1-oxy), C₆-C₁₂-aryl-C₁-C₄-alkoxy (e.g. benzyloxy), optionally substituted C₆-C₁₂-aryloxy (e.g. phenoxy or 4-F-phenoxy) or optionally substituted C₃-C₁₂-heterocyclyl (tetrahydrofuran-2-yl, tetrahydrofuran-3-yl, tetrahydropyran-2-yl, tetrahydropyran-3-yl, 1-cyclopropyl-piperidin-4-yl, 1-cyclopropyl-piperidin-3-yl, 2-pyridyl, 3-pyridyl, 3-F-pyrid-2-yl, 1,3-oxazol-4-yl, or 1,3-oxazol-2-yl, a further example being 1,3-dioxan-2-yl, 5,5-dimethyl-1,3-dioxan-2-yl, 4,6-dimethyl-1,3-dioxan-2-yl, 1,3-dioxepan-2-yl, 1,3-dioxolan-2-yl, 5,7-dioxaspiro[2.5]octan-6-yl, morpholin-3-yl, morpholin-4-yl, pyrrolidinyl, piperidyl, 4-F-piperidyl, 4,4-diF-piperidyl, 1-Me-piperid-2-yl, or 5-F-pyrid-2-yl),
- R^{3b}: is hydrogen or hydroxy;
- Y¹: is >CR⁶;
- R⁶: is hydrogen, C₁-C₆-alkyl, or hydroxy-C₁-C₆-alkyl (e.g.-CH₂OH), or
- R⁶ and R^{3a} or R^{3b}: together are C₁-C₅-alkylene, or
- R⁶: is C₁-C₄-alkylene (e.g. methylene or 1,2-ethylene) that is bound to a carbon atom in R^{3a}, and R^{3a} is an optionally substituted C₆-C₁₂-aryl (e.g. phenyl);
- R^{8a}: is hydrogen, C₁-C₆-alkyl (e.g. methyl, ethyl, n-propyl, iso-propyl, pentyl, or hexyl), or C₁-C₆-alkylcarbonyl (e.g. methylcarbonyl), or
- R⁶,R^{8a}: are together optionally substituted C₁-C₅-alkylene, wherein one or more -CH₂- of C₁-C₅-alkylene may be independently replaced by a an oxygen atom or C=O e. g. -C(O)OCH₂-); or
- R^{3a} and R^{8a}: together are optionally substituted C₁-C₅-alkylene (e.g. 1,2-ethylene or 1,3-propylene);
- R^{9a}: is hydrogen, halogen (e.g. F), C₁-C₆-alkyl (e.g. methyl, ethyl, tert-butyl, or 2,3-dimethyl-propyl), halogenated C₁-C₆-alkyl (e.g. trifluoromethyl), or C₁-C₆-alkoxy (e.g. methoxy or ethoxy);
- R^{9b}: is hydrogen or halogen (e.g. F);
- n4: is 0, 1, 2, 3, or 4;
- R¹³: is hydrogen, C₁-C₈-alkyl (e.g. methyl, ethyl, n-butyl, tert-butyl, pentyl, or hexyl), halogenated C₁-C₆-alkyl (e.g. -CF₃ or -CF₂Me), C₁-C₆-alkoxy-C₁-C₆-alkyl (e.g. methoxy-methyl), optionally substituted C₃-C₁₂-cycloalkyl (e.g. cyclopropyl, 2-propyl-cyclopropyl, 1-(methoxy-methyl)-cyclopropyl, 2-phenyl-cyclopropyl, cyclopentyl, cyclopentyl, 3,3-dimethylcyclopentyl, cyclohexyl, 3,3-dimethyl-cyclohexyl, 4,4-dimethyl-cyclohexyl, 1-methyl-cyclohexyl, 1-CF₃-cyclopropyl, 4-CF₃-cyclohexyl, 3-CF₃-cyclohexyl, or 4,4-diF-cyclohexyl, a further example being 1-phenyl-cyclopropyl, 1-(4-F-phenyl)-cyclopropyl, (3-Cl-phenyl)-cyclopropyl, 2-phenyl-cyclobutyl, 3-phenyl-cyclobutyl, 3-(4-F-phenyl)-cyclobutyl, 3-(2-F-phenyl)-cyclobutyl, 2-Cl-cyclopentyl, 2-Me-cyclopentyl, 3-Me-cyclopentyl, 2-phenyl-cyclopentyl, 3-(2-pyridyl)-cyclopentyl, 3-Me-cyclohexyl, 3,3,5,5-tetraMe-cyclohexyl, 4-Me-cyclohexyl, 4-Et-cyclohexyl, 2-CF₃-cyclohexyl, 2-Me-5-isopropenyl-cyclohexyl, 2-phenyl-cyclohexyl, 3-phenyl-cyclohexyl, 4-phenyl-cyclohexyl, 2-EtO-cyclohexyl, decalin-1-yl, decalin-2-yl, norbornan-2-yl, 1,7,7-trimethyl-norbornan-2-yl, 5-iPr-2-Me-3-bicyclo[3.1.0]hexanyl, bicycle [3.2.1]octanyl, or 3-bicyclo[1.1.1]pentanyl), C₂-C₆-alkenyl (e.g. hex-2-enyl), optionally substituted C₃-C₆-cycloalkenyl (e.g. 1,3,3-trimethylcyclohexen-2-yl), optionally substituted C₆-C₁₂-aryl (e.g. phenyl, 2-F-phenyl, 4-F-phenyl, 3-F-phenyl, 2-Cl-phenyl, 3-Cl-phenyl, 4-Cl-phenyl, 2,3-diF-phenyl, 2,4-diF-phenyl, 2,5-diF-phenyl, 3,4-diF-phenyl, 2,3,4-triF-phenyl, 2,4-diCl-phenyl, 3,4-diCl-phenyl, 3-F-4-Cl-phenyl, 3-Cl-4-F-phenyl, 3-Cl-5-F-phenyl, 3,4-diF-5-Cl-phenyl, 2-Me-phenyl, 3-Me-phenyl, 4-Me-phenyl, 3-iPr-phenyl, 3-tBu-phenyl, 3-Me-4-F-phenyl, 3-Me-4-Cl-phenyl, 3-iPr-4 Cl-phenyl, 3-(1-OH-1-CF₃-Et)-phenyl, 3-CHF₂-4-F-phenyl, 2-CF₃-phenyl, 3-CF₃-phenyl, 4-CF₃-phenyl, 3-Cl-4-CF₃-phenyl, 2-F-3-CF₃-phenyl, 2-F-5-CF₃-phenyl, 3-F-5-CF₃-phenyl, 2-Cl-4-CF₃-phenyl, 2-Cl-3-CF₃-phenyl, 3-CF₃-4-F-phenyl, 3-CF₃-4-Cl-phenyl, 3-Me-4-CF₃-phenyl, 3-CF₃-4-Me-phenyl, 3,4-diCF₃-phenyl, 4-OMe-phenyl, 3-OiPr-phenyl, 3-CF₃-4-OMe-phenyl, 3-OCH₂CF₃-phenyl, 3-OCF₃-phenyl, 2-OCHF₂-5-Cl-phenyl, 3-OCF₃-4-F-phenyl, 3-OCF₃-4-Cl-phenyl, 3-OBn-phenyl, 3-OPh-phenyl, 3-CN-phenyl, 4-CN-phenyl, 2-F-3-CN-phenyl, 2-F-4-CN-phenyl, 3-F-4-CN-phenyl, 2-F-5-CN-phenyl, 3-F-5-CN-phenyl, 3-Cl-4-CN-phenyl, 2-Cl-5-CN-phenyl, 3-Cl-5-CN-phenyl, 3-CN-4-Cl-phenyl, 2-Me-3-CN-phenyl, 2-Me-5-CN-phenyl, 3-Me-5-CN-phenyl, 3-CF₃-4-CN-phenyl, 3-CN-4-OMe-phenyl, 3-CN-4-OCF₃-phenyl, 3-phenyl-phenyl, 3-MeSO₂-phenyl, 3-(piperidin-4-yl)-phenyl, 2-methylcarbonylamino-5-Cl-phenyl, 3-(pyrid-2-yl)-phenyl, 3-(pyrid-3-yl)-4 F-phenyl, 3-(pyrid-4-yl)-4 F-phenyl, 3-(pyrimid-5-yl)-4 F-phenyl, indan-5-yl, 2-chlor-indan-5-yl, or tetralin-6-yl, a further example being 2,4-diF-3-Cl-phenyl, 3-Et-4-Cl-phenyl, 3-(2-methoxyethyl)-phenyl, 3-(2-OH-ethyl)-phenyl, 3-(1-OH-1-Me-ethyl)-phenyl, 3-CHF₂-phenyl, 3-CF₃-2,4-diF-phenyl, 3-(dimethylamino-methyl)-phenyl, 3-(morpholin-4-yl-methyl)-phenyl, 3-cyclopropyl-phenyl, 3-OEt-phenyl, 3-OPr-phenyl, 3-OtBu-phenyl, 3-(cyclopropylmethoxy)-phenyl, 3-(OMe-methoxy)-phenyl, 3-(2-dimethylamino-ethoxy)-phenyl, 3-CF₃-4-OH-phenyl, 3-OCH₂CHF₂-phenyl, 3-OCHF₂-phenyl, 3-OCHF₂-4-F-phenyl, 3-OCF₃-4-OMe-phenyl, 3-cyclopropoxy-phenyl, 3-methylcarbonyl-phenyl, 3-dimethylamino-phenyl, 3-(2-pyridyloxy)-phenyl, 3-(pyrimidin-2-yloxy)-phenyl, indanyl, indan-2-yl, 2-F-indanyl, 4-F-indanyl, 5-F-indanyl, 6-F-indanyl, 7-F-indanyl, 3-Me-indanyl, 4-Me-indanyl, 5-Me-indanyl, 6-Me-indanyl, 4-CF₃-indanyl, 5-CF₃-indanyl, 6-CF₃-indanyl, 3,3-dimethyl-indanyl, tetralin-2-yl, 7-F-tetralin-2-yl, 6-F-tetralin-2-yl, tetralinyl, 6-F-tetralinyl, 5-F-tetralinyl, or 7-F-tetralinyl), C₁-C₆-alkoxy (e.g.methoxy, ethoxy, or n-propoxy), C₁-C₆-alkoxy-C₁-C₄-alkoxy (e.g. 2-methoxy-ethoxy), optionally substituted C₆-C₁₂-aryloxy (e.g. 4-F-phenoxy or 4-tertbutyl-phenoxy), optionally substituted C₃-C₁₂-heterocyclyl (e.g. e.g. n-propyl-furan-2-yl, 2-CF₃-furan-5-yl, 1,2-dimethyl-5-CN-pyrrol-3-yl, 2,3-diMe-thiophen-5-yl, 2-Me-thiophen-5-yl, 2-Cl-thiophen-5-yl, 3-Cl-thiophen-2-yl, 2,5-diCl-thiophen-3-yl, 2-tetrahydropyranyl-thiophen-5-yl, 1,3-thiazol-5-yl, 4-Me-1,3-thiazol-2-yl, 2-Me-1,3-thiazol-5-yl, 5-Me-1,3-thiazol-2-yl, 4-Me-1,3-thiazol-5-yl, 2-Me-1,3-thiazol-4-yl, 4-isopropyl-1,3-thiazol-2-yl, 2,4-diMe-1,3-thiazol-5-yl, 2-phenyl-4-Me-1,3-thiazol-5-yl, 4-phenyl-1,3-thiazol-5-yl, 2-(4-Me-phenyl)-1,3-thiazol-5-yl, 4-(4-F-phenyl)-1,3-thiazol-2-yl, 2-Cl-1,3-thiazol-4-yl, 4-Cl-1,3-thiazol-5-yl, 2-Br-1,3-thiazol-5-yl, 4-Br-1,3-thiazol-2-yl, 4-Me-5-Br-1,3-thiazol-2-yl, 2,4-dichloro-1,3-thiazol-5-yl, 1,5-dimethyl-1,2,4-triazol-3-yl, pyrid-2-yl, pyrid-3-yl, pyrid-4-yl, 4-F-pyrid-2-yl, 5-F-pyrid-2-yl, 6-F-pyrid-2-yl, 4-Cl-pyrid-2-yl, 5-F-pyrid-3-yl, 2-F-pyrid-4-yl, 3,5-diCl-pyrid-4-yl, 4,5-diCl-pyrid-2-yl, 2-Cl-3F-pyrid-4-yl, 5-Me-pyrid-2-yl, 4-iPr-pyrid-2-yl, 4-Me-5-F-pyrid-2-yl, 4-CF₃-pyrid-2-yl, 5-CF₃-pyrid-2-yl, 6-CF₃-pyrid-2-yl, 5-CF₃-pyrid-3-yl, 2-CF₃-pyrid-4-yl, 3-F-4-CF₃-pyrid-2-yl, 4-CF₃-5-F-pyrid-2-yl, 4-CF₃-5-Cl-pyrid-2-yl, 3,5-diF-4-CF₃-pyrid-2-yl, 4-OCH₂CF₃-pyrid-2-yl, 4-OCH₂CF₃-5-F-pyrid-2-yl, 4-OCH₂CF₃-5-Cl-pyrid-2-yl, 4-OBn-5-F-pyrid-2-yl, 3-(4-F-phenyl)-pyrid-5-yl, 2-(4-F-phenyl)-pyrid-3-yl, 4-(pyrid-4-yl)-pyrid-2-yl, 4-(pyrid-3-yl)-pyrid-2-yl, 5-cyclopropyl-pyraz-2-yl, 5-cyclobutyl-pyraz-2-yl, 5-pyrrolidin-pyraz-2-yl, pyridaz-3-yl, 4-CF₃-pyridaz-3-yl, 5-CF₃-pyridaz-3-yl, 5-F-pyrimid-2-yl, 6-Cl-pyrimid-4-yl, 6-Me-pyrimid-4-yl, 6-Et-pyrimid-4-yl, 6-Pr-pyrimid-4-yl, 6-iPr-pyrimid-4-yl, 4-CF₃-pyrimid-2-yl, 6-CF₃-pyrimid-4-yl, 2-Me-6-Cl-pyrimid-4-yl, 2-Me-6-CF₃-pyrimid-4-yl, 2-OMe-6-CF₃-pyrimid-4-yl, 2-OMe-pyrimid-4-yl, 6-OMe-pyrimid-4-yl, 6-OEt-pyrimid-4-yl, 6-OPr-pyrimid-4-yl, 6-OiPr-pyrimid-4-yl, 6-OiBu-pyrimid-4-yl, 6-OBu-pyrimid-4-yl, 6-OBn-pyrimid-4-yl, 6-cyclobutoxy-pyrimid-4-yl, 6-cyclopentoxy-pyrimid-4-yl, 6-cyclohexyloxy-pyrimid-4-yl, 6-cyclopropyl-pyrimid-4-yl, 6-phenyl-pyrimid-4-yl, 6-(2-F- phenyl)-pyrimid-4-yl, 6-(3-F- phenyl)-pyrimid-4-yl, 6-(4-F- phenyl)-pyrimid-4-yl, 6-Cl-pyrimid-4-yl, 6-(2-Me- phenyl)-pyrimid-4-yl, 6-(3-Me- phenyl)-pyrimid-4-yl, 6-(4-Me- phenyl)-pyrimid-4-yl, 6-(3-Cl- phenyl)-pyrimid-4-yl, 6-(4-Cl- phenyl)-pyrimid-4-yl, 2-(morpholin-1-yl)-pyrimid-4-yl, 6-(azetidin-3-yl-methoxy)-pyrimid-4-yl, 6-(pyrrolidin-3-yl-methoxy)-pyrimid-4-yl, 6-(pyrrolidin-2-yl-methoxy)-pyrimid-4-yl, benzofuran-5-yl, 2-Me-benzofuran-3-yl, 2-Et-benzofuran-3-yl, benzothiophen-5-yl, benzothiophen-6-yl, 5-Me-benzothiophen-2-yl, 5-F-benzothiophen-2-yl, 3-Cl-benzothiophen-2-yl, benzothiazol-2-yl, isoquinolin-6-yl, isoquinolin-7-yl, quinolin-6-yl, quinolin-7-yl, 4,5,6,7-tetrahydro-1,3-benzothiazol-2-yl, 2,3-dihydrobenzofuran-5-yl, 7H-pyrrolo[2,3-d]pyrimidin-4-yl, pyrazolo[1,5-a]pyridine-3-yl, pyrazolo[1,5-a]pyridine-7-yl, imidazo[1,2-a]pyridin-8-yl, 5-methyl-imidazo[1,2-a]pyridin-3-yl, 6-chloro-2-methyl-imidazo[1,2-a]pyridin-3-yl, 6-bromo-2-methyl-imidazo[1,2-a]pyridin-3-yl, thieno[2,3-b]pyridin-2-yl, N-benzyl-indolin-6-yl, indolinon-4-yl, chroman-6-yl, 4,4-dimethylchroman-6-yl, 4,4-dimethyl-1,3-dioxan-2-yl, 2-Me-tetrahydrofuran-3-yl, tetrahydrofuran-3-yl, tetrahydropyran-4-yl, 4-Metetrahydropyran-4-yl, 2,2-diMe-tetrahydropyran-4-yl, 1-isopropyl-piperidin-4-yl, 1-ethyl-piperidin-3-yl, or 2-CF₃-piperazin-5-yl, a further example being iBu-pyrazol-4-yl, 1-CHF₂-5-Me-1,2-diazol-4-yl, 1-benzyl-5-F-1,2,4-triazol-3-yl, 2-Me-pyrid-4-yl, 2-iPr-pyrid-4-yl, 4-phenyl-pyrid-2yl, 2-cyclopropyl-pyrid-4-yl, 2-OMe-pyrid-4-yl, 2-OEt-pyrid-4-yl, 2-cyclopropylmethoxy-pyrid-4yl, 2-OiPr-pyrid-4-yl, 2-OCH₂CF₃-pyrid-4-yl, 2-cyclopropoxypyrid-4-yl, 2-cyclobutoxy-pyrid-4-yl, 4,5-diCl-pyrimidin-2yl, benzothiophen-3-yl, 2,2-diF-1,3-benzodioxol-5-yl, 5,6,7,8-tetrahydro-isoquinolin-5-yl, 5,6,7,8-tetrahydro-isoquinolin-8-yl, quinolin-8-yl, 1-Me-3,4-dihydro-2H-quinolin-4-yl, 2-Me-3,4-dihydro-1H-isoquinolin-6-yl, 5,6,7,8-tetrahydro-quinolin-5-yl, 5,6,7,8-tetrahydro-quinolin-8-yl, 5,6,7,8-tetrahydroquinolin-6-yl, 2,3-dihydrobenzofuran-3-yl, pyrazolo[1,5-a]pyrimidin-6-yl, imidazo[1,5-a]pyrazinyl, 3-methyl-imidazo[1,5-a]pyrazinyl, 3-Me-[1,2,4]triazolo[4,3-a]pyridin-6-yl, imidazo[1,2-a]pyrazin-6-yl, 2-Me-isoindolin-1-on-6-yl, 2,2-dimethyl-7-CF₃-chroman-4-yl, 7-CF₃-chroman-4-yl, 7-OCF₃-chroman-4-yl, isochroman-4-yl, 6,6-dimethyl-1,3-dioxan-2-yl, tetrahydropyran-3-yl, 1-phenyl-pyrrolidin-3-yl, piperidin-3-yl, 1,3-dimethyl-piperidin-4-yl, 1-cyclopropyl-piperidin-4-yl, 1-(tri-F-methyl-carbonyl)-piperidin-3-yl, quinuclidin-2-yl, 6,7-dihydro-5H-cyclopenta[b]pyridine-7-yl, 6,7-dihydro-5H-cyclopenta[b]pyridine-5-yl, 6,7-dihydro-5H-cyclopenta[c]pyridine-7-yl, 6,7-dihydro-5H-cyclopenta[c]pyridine-5-yl, 5,6-dihydro-4H-cyclopenta[b]thiophen-4-yl, or 1,2,3,5,6,7,8,8a-octahydroindolizin), or tri-(C₁-C₄-alkyl)-silyloxy; and
- R^{5a}, R^{5b}: are hydrogen.

Further, particular embodiments of pyrrolidine derivatives of formula (Id) result if:
- R^{3a}: is C₃-C₁₂-cycloalkyl (e.g. cyclopropyl, cyclobutyl, cyclopentyl, or cyclohexyl), optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl, a further example being 2-Me-phenyl, 3-Me-phenyl, or 4-Me-phenyl), hydroxy, C₁-C₆-alkoxy (e.g.methoxy, ethoxy, n-propoxy, isopropoxy, or iso-butoxy), C₃-C₁₂-cycloalkyl-C₁-C₄-alkoxy (e.g. cyclopropylmethoxy or cyclohexylmethoxy), C₂-C₆-alkenyloxy (e.g. allyloxy or 2-methylprop-2-en-1-oxy), C₆-C₁₂-aryl-C₁-C₄-alkoxy (e.g. benzyloxy), optionally substituted C₆-C₁₂-aryloxy (e.g. phenoxy or 4-F-phenoxy) or optionally substituted C₃-C₁₂-heterocyclyl (tetrahydrofuran-2-yl, tetrahydrofuran-3-yl, tetrahydropyran-2-yl, tetrahydropyran-3-yl, 1-cyclopropyl-piperidin-4-yl, 1-cyclopropyl-piperidin-3-yl, 2-pyridyl, 3-pyridyl, 3-F-pyrid-2-yl, 1,3-oxazol-4-yl, or 1,3-oxazol-2-yl, a further example being 1,3-dioxan-2-yl, 5,5-dimethyl-1,3-dioxan-2-yl, 4,6-dimethyl-1,3-dioxan-2-yl, 1,3-dioxepan-2-yl, 1,3-dioxolan-2-yl, 5,7-dioxaspiro[2.5]octan-6-yl, morpholin-3-yl, morpholin-4-yl, pyrrolidinyl, piperidyl, 4-F-piperidyl, 4,4-diF-piperidyl, 1-Me-piperid-2-yl, or 5-F-pyrid-2-yl);
- R⁶: is hydrogen, C₁-C₆-alkyl, or hydroxy-C₁-C₆-alkyl (e.g.-CH₂OH);
- R^{8a}: is hydrogen, C₁-C₆-alkyl (e.g. methyl, ethyl, n-propyl, iso-propyl, pentyl, or hexyl), or C₁-C₆-alkylcarbonyl (e.g. methylcarbonyl), or
- R⁶, R^{8a}: are together optionally substituted C₁-C₅-alkylene, wherein one or more -CH₂- of C₁-C₅-alkylene may be independently replaced by a an oxygen atom or C=O e. g. -C(O)OCH₂-);
- R^{9a}: is hydrogen, halogen (e.g. F), C₁-C₆-alkyl (e.g. methyl, ethyl, tert-butyl, or 2,3-dimethyl-propyl), or C₁-C₆-alkoxy (e.g. methoxy or ethoxy);
- R^{9b}: is hydrogen;
and R¹, R^{2a}, R^{2b}, R^{3b}, Y¹, n4, R¹³, R^{5a} and R^{5b} are as defined above.

According to a particular embodiment, R¹ - in the pyrrolidine derivatives of formula (Id) - is an optionally substituted 5-membered heterocyclic ring as defined herein containing 2 or 3 N (e.g. 1,2-diazol-4-yl, 1-methyl-1,2-diazol-4-yl, 1-methyl-1,2-diazol-3-yl, 1-methyl-1,3-diazol-4-yl, 1-methyl-1,2-diazol-5-yl, 1,5-dimethyl-1,2-diazol-4-yl, 1,3-dimethyl-1,2-diazol-4-yl, 1,5-dimethyl-1,2-diazol-4-yl, 1,3-dimethyl-1,2-diazol-5-yl, 1,3-dimethyl-5-Cl-1,2-diazol-4-yl, 1-isopropyl-3-methyl-1,2-diazol-4-yl, 1-Me-3-CF₃-1,2-diazol-4-yl, 1-cyclopentyl-3-Me-1,2-diazol-4-yl, 1,3,5-trimethyl-1,2-diazol-4-yl, 1-CHF₂-3,5-dimethyl-1,2-diazol-4-yl, 1,2-dimethyl-1,3-diazol-4-yl, 1,2-dimethyl-1,3-diazol-5-yl, 1-methyl-5-Cl-1,3-diazol-4-yl, or 1-Me-1,2,3-triazol-4-yl, a further example being 1-ethyl-1,3-diazol-4-yl or 1-methyl-1,2,4-triazol-3-yl).

Preferably, R¹ - in the pyrrolidine derivatives of formula (Id) - is an optionally substituted 5-membered heterocyclic ring as defined herein containing 2 N and, in particular, R¹ is optionally substituted 1,3-diazolyl.

According to a further preferred embodiment, R¹ - in the pyrrolidine derivatives of formula (Id) - is an optionally substituted 5-membered heterocyclic ring as defined herein containing 3 N and, in particular, R¹ is optionally substituted 1,2,3-triazolyl.

In connection with R¹ and the pyrrolidine derivatives of formula (Id), substituted 5-membered heterocyclic ring heteroatomsin particular includes pyrazolyl, imidazolyl, and triazolyl, which are substituted with 1, 2 or 3 substituents independently selected from the group consisting of halogen, C₁-C₆-alkyl, halogenated C₁-C₆-alkyl, C₃-C₆-cycloalkyl, C₁-C₄-alkoxycarbonyl and C₁-C₆-alkylcarbonylamino. Preferably, the substituent(s) on the heterocyclic ring are independently selected from the group consisting of halogen and C₁-C₆-alkyl. In particular, the substituent(s) on the heterocyclic ring are independently selected from the group consisting of C₁-C₆-alkyl, especially C₁-C₄-alkyl (e.g. methyl). According to a particular embodiment, 1,3-diazolyl is substituted with halogen or C₁-C₆-alkyl as described herein. According to a specific embodiment, R¹ is 1-methyl-1,3-diazol-4-yl. According to a further particular embodiment, 1,2,3-triazolyl is substituted with C₁-C₆-alkyl as described herein. According to a further specific embodiment, R¹ is 1-methyl-1,2,3-triazol-4-yl.

In connection with R^{3a} and the pyrrolidine derivatives of formula (Id), substituted C₆-C₁₂-aryl in particular includes C₆-C₁₂-aryl, such as phenyl or naphthyl, which is substituted with 1, 2, or 3 substituents independently selected from the group consisting of halogen, C₁-C₄-alkyl and C₁-C₄-alkoxy. Preferably, the substituent(s) on C₆-C₁₂-aryl are independently selected from the group consisting of halogen.

In connection with R^{3a} and the pyrrolidine derivatives of formula (Id), substituted C₆-C₁₂-aryloxy in particular includes is C₆-C₁₂-aryloxy, such as phenoxy, which is substituted with 1, 2, or 3 substituents independently selected from the group consisting of halogen and C₁-C₄-alkyl.

In connection with R^{3a} and the pyrrolidine derivatives of formula (Id), substituted C₃-C₁₂-heterocyclyl in particular includes C₃-C₁₂-heterocyclyl, such as pyridyl, piperidinyl, isoxazolyl, diazolyl, tetrahydrofuranyl, tetrahydropyranyl, or morpholinyl, which is substituted with 1, 2, or 3 substituents independently selected from the group consisting of halogen, C₁-C₄-alkyl or C₃-C₆-cycloalkyl. Additionally, in connection with R^{3a} and the pyrrolidine derivatives of formula (Id), substituted C₃-C₁₂-heterocyclyl in particular may include C₃-C₁₂-heterocyclyl, such as pyrrolidinyl, dioxolanyl, dioxanyl, or dioxepanyl, which is substituted with 1, 2, or 3 substituents independently selected from the group consisting of halogen, C₁-C₄-alkyl and C₃-C₆-cycloalkyl.

According to a preferred embodiment, R^{3a} - in the pyrrolidine derivatives of formula (Id) - is C₃-C₁₂-cycloalkyl (e.g. cyclopropyl, or cyclohexyl), C₆-C₁₂-aryl, in particular phenyl, optionally substituted with 1, 2, or 3 substituents independently selected from the group consisting of halogen (e.g. phenyl, 2-Cl-phenyl, 2-Br-phenyl, 3-Cl-phenyl, 4-Cl-phenyl, 2-F-phenyl, 3-F-phenyl, 4-F-phenyl, or 2,4,5-trifluoro-phenyl), or hydroxy, C₁-C₆-alkoxy (e.g.methoxy, or iso-butoxy), C₃-C₁₂-cycloalkyl-C₁-C₄-alkoxy (e.g. cyclopropylmethoxy), C₂-C₆-alkenyloxy (e.g. allyloxy or 2-methylprop-2-en-1-oxy), C₆-C₁₂-aryl-C₁-C₄-alkoxy (e.g. benzyloxy), C₆-C₁₂-aryloxy (e.g. 4-F-phenoxy) optionally substituted with 1, 2, or 3 substituents independently selected from the group consisting of halogen, or C₃-C₁₂-heterocyclyl, in particular tetrahydrofuranyl or tetrahydropyranyl,optionally substituted with 1, 2, or 3 substituents independently selected from the group consisting of halogen and C₃-C₆-cycloalkyl (e.g. tetrahydrofuran-2-yl, tetrahydrofuran-3-yl, tetrahydropyran-2-yl, tetrahydropyran-3-yl, 1-cyclopropyl-piperidin-4-yl, 1-cyclopropyl-piperidin-3-yl, 2-pyridyl, 3-pyridyl, 3-F-pyrid-2-yl, 1,3-oxazol-4-yl, or 1,3-oxazol-2-yl), or in particular piperidinyl, pyridyl, dioxolanyl, dioxanyl, or dioxepanyl, optionally substituted with 1, 2, or 3 substituents independently selected from the group consisting of halogen, C₁-C₄-alkyl and C₃-C₆-cycloalkyl (e.g. 1,3-dioxan-2-yl, 5,5-dimethyl-1,3-dioxan-2-yl, 4,6-dimethyl-1,3-dioxan-2-yl, 1,3-dioxepan-2-yl, 1,3-dioxolan-2-yl, 5,7-dioxaspiro[2.5]octan-6-yl, piperidyl, 1-Me-piperid-2-yl, or 5-F-pyrid-2-yl).

According to a preferred embodiment, R^{9a}, R^{9b} - in the pyrrolidine derivatives of formula (Id) - are independently hydrogen, halogen (e.g. F), C₁-C₆-alkyl (e.g. methyl, ethyl, tert-butyl, or 2,3-dimethyl-propyl), or C₁-C₆-alkoxy (e.g. methoxy). It is further preferred if R^{9a}, R^{9b} - in the pyrrolidine derivatives of formula (Id) - are independently halogenated C₁-C₆-alkyl (e.g. trifluoromethyl).

More preferably, R^{9a} is hydrogen, halogen (e.g. F), C₁-C₆-alkyl (e.g. methyl, ethyl, tert-butyl, or 2,3-dimethyl-propyl), or C₁-C₆-alkoxy (e.g. methoxy) and R^{9b} is hydrogen. Or, more preferably, R^{9a} is halogenated C₁-C₆-alkyl (e.g. trifluoromethyl). Or, more preferably, R^{9a} and R^{9b} are both halogen.

In connection with R¹³, substituted C₃-C₂-cycloalkyl in particular includes C₃-C₂-cycloalkyl, such as cyclopropyl, cyclopentyl, or cyclohexyl, a further example being cyclobutyl, decalinyl, norbornanyl, bicyclo[3.1.0]hexanyl, bicyclo [3.2.1]octanyl, or 3-bicyclo[1.1.1]pentanyl, which is substituted with 1, 2, or 3 substituents independently selected from the group consisting of halogen, C₁-C₄-alkyl, halogenated C₁-C₄-alkyl, C₁-C₄-alkoxy-C₁-C₄-alkyl and C₆-C₁₂-aryl. Additionally, the substituents may be independently selected from the group consisting of C₂-C₄-alkenyl, C₁-C₄-alkoxy, C₆-C₁₂-aryl which may be substituted with halogen, and C₃-C₁₂-heterocyclyl.

In connection with R¹³, substituted C₃-C₆-cycloalkenyl in particular includes C₃-C₆-cycloalkenyl, such as cyclohexenyl, which is substituted with 1, 2, or 3 substituents independently selected from the group consisting of C₁-C₄-alkyl.

In connection with R¹³, substituted C₆-C₁₂-aryl in particular includes C₆-C₁₂-aryl, such as phenyl, a further example being indanyl or tetralinyl, which is substituted with 1, 2, or 3 substituents independently selected from the group consisting of halogen, C₁-C₄-alkyl, halogenated C₁-C₄-alkyl, hydroxy-(halogenated C₁-C₄-alkyl), CN, C₆-C₁₂-aryl, C₁-C₄-alkoxy, halogenated C₁-C₄-alkoxy, C₆-C₁₂ aryl-C₁-C₄-alkoxy, C₆-C₁₂-aryloxy, C₁-C₄-alkyl-sulfonyl, C₁-C₄-alkyl-carbonylamino and C₃-C₁₂-heterocyclyl. Additionally, the substituents may be independently selected from the group consisting of hydroxy-C₁-C₄-alkyl, C₁-C₄-alkoxy-C₁-C₄-alkyl, di-C₁-C₄-alkyl amino-C₁-C₄-alkyl, C₃-C₁₂-heterocyclyl-C₁-C₄-alkyl, C₃-C₆-cycloalkyl, C₁-C₄-alkyl-carbonyl, hydroxy, C₃-C₁₂-cycloalkyl-C₁-C₄-alkoxy, C₃-C₆-cycloalkoxy, C₁-C₄-alkoxy-C₁-C₄-alkoxy, di-C₁-C₄-alkyl amino-C₁-C₄-alkoxy, C₃-C₁₂-heterocyclyloxy and di-C₁-C₄-alkyl amino.

In connection with R¹³, substituted C₆-C₁₂-aryloxy in particular includes C₆-C₁₂-aryloxy, such as phenoxy, which is substituted with 1, 2, or 3 substituents independently selected from the group consisting of halogen and C₁-C₄-alkyl.

In connection with R¹³, substituted C₃-C₁₂-heterocyclyl in particular includes C₃-C₁₂-heterocyclyl, such as furanyl, pyrrolyl, thiophenyl, oxazolyl, diazolyl, thiazolyl, triazolyl, pyridyl, pyrimidyl, pyrazyl, pyridazyl, benzofuranyl, benzothiophenyl, benzothiazolyl, quinolinyl or isoquinolinyl, a further example being benzodioxolyl, dihydro-benzofuranyl, dihydro-quinolinyl, dihydro-isoquinolinyl, tetrahydro-quinolinyl, tetrahydro-isoquinolinyl, pyrazolo[1,5-a]pyrimidinyl, imidazo[1,5-a]pyrazinyl, triazolo[4,3-a]pyridinyl, imidazo[1,2-a]pyrazinyl, isoindolinonyl, chromanyl, chromanyl, isochromanyl, dioxanyl, tetrahydropyranyl, pyrrolidinyl, piperidinyl, quinuclidinyl, dihydrocyclopenta[b]pyridinyl, dihydrocyclopenta[c]pyridinyl, dihydrocyclopenta[b]thiophenyl, or octahydroindolizinyl, which is substituted with 1, 2, or 3 substituents independently selected from the group consisting of halogen, C₁-C₄-alkyl, halogenated C₁-C₄-alkyl, C₆-C₁₂-aryl-C₁-C₄-alkyl, C₃-C₆-cycloalkyl, hydroxy, CN, C₆-C₁₂-aryl optionally substituted with halogen and C₁-C₄-alkyl, C₁-C₄-alkoxy, halogenated C₁-C₄-alkoxy, C₃-C₆-cycloalkoxy, C₆-C₁₂ aryl-C₁-C₄-alkoxy, C₃-C₁₂-heterocyclyl-C₁-C₄-alkoxy and C₃-C₁₂-heterocyclyl. Additionally, the substituents may be independently selected from the group consisting of C₃-C₆-cycloalkyl-C₁-C₄-alkoxy and halogenated C₁-C₄-alkyl-carbonyl.

According to a preferred embodiment, R¹³ is hydrogen, C₁-C₈-alkyl (e.g. methyl, ethyl, n-butyl, tert-butyl, pentyl, or hexyl), halogenated C₁-C₆-alkyl (e.g. -CF₃ or -CF₂Me), C₁-C₆-alkoxy-C₁-C₆-alkyl (e.g. methoxy-methyl), C₃-C₁₂-cycloalkyl optionally substituted with 1, 2, or 3 substituents independently selected from the group consisting of halogen, C₁-C₄-alkyl, halogenated C₁-C₄-alkyl, C₁-C₄-alkoxy-C₁-C₄-alkyl and C₆-C₁₂-aryl which may be additionally substituted with halogen, and additionally from the group consisting of C₁-C₄-alkenyl, C₁-C₄-alkoxy and C₃-C₁₂-heterocyclyl (e.g. cyclopropyl, 2-propyl-cyclopropyl, 1-(methoxy-methyl)-cyclopropyl, 2-phenyl-cyclopropyl, cyclopentyl, 1-methyl-cyclohexyl, 1-CF₃-cyclopropyl, 4-CF₃-cyclohexyl,or 4,4-diF-cyclohexyl, a further example being 1-phenyl-cyclopropyl, 1-(4-F-phenyl)-cyclopropyl, (3-Cl-phenyl)-cyclopropyl, 2-phenyl-cyclobutyl, 3-phenyl-cyclobutyl, 3-(4-F-phenyl)-cyclobutyl, 3-(2-F-phenyl)-cyclobutyl, 2-Cl-cyclopentyl, 2-Me-cyclopentyl, 3-Me-cyclopentyl, 2-phenyl-cyclopentyl, 3-(2-pyridyl)-cyclopentyl, 3-Me-cyclohexyl, 3,3,5,5-tetraMe-cyclohexyl, 4-Me-cyclohexyl, 4-Et-cyclohexyl, 2-CF₃-cyclohexyl, 2-Me-5-isopropenyl-cyclohexyl, 2-phenyl-cyclohexyl, 3-phenyl-cyclohexyl, 4-phenyl-cyclohexyl, 2-EtO-cyclohexyl, decalin-1-yl, decalin-2-yl, norbornan-2-yl, 1,7,7-trimethyl-norbornan-2-yl, 5-iPr-2-Me-3-bicyclo[3.1.0]hexanyl, bicycle [3.2.1]octanyl, or 3-bicyclo[1.1.1]pentanyl), or C₂-C₆-alkenyl (e.g. hex-2-enyl), C₃-C₆-cycloalkenyl optionally substituted with 1, 2, or 3 substituents independently selected from the group consisting of C₁-C₄-alkyl (e.g. 1,3,3-trimethylcyclohexen-2-yl), or C₆-C₁₂-aryl optionally substituted with 1, 2, or 3 substituents independently selected from the group consisting of halogen, C₁-C₄-alkyl, halogenated C₁-C₄-alkyl, hydroxy-(halogenated C₁-C₄-alkyl), CN, C₆-C₁₂-aryl, C₁-C₄-alkoxy, halogenated C₁-C₄-alkoxy, C₆-C₁₂ aryl-C₁-C₄-alkoxy, C₆-C₁₂-aryloxy, C₁-C₄-alkylsulfonyl, C₁-C₄-alkyl-carbonylamino and C₃-C₁₂-heterocyclyl, and additionally from the group consisting of hydroxy-C₁-C₄-alkyl, C₁-C₄-alkoxy-C₁-C₄-alkyl, di-C₁-C₄-alkyl amino-C₁-C₄-alkyl, C₃-C₁₂-heterocyclyl-C₁-C₄-alkyl, C₃-C₆-cycloalkyl, C₁-C₄-alkyl-carbonyl, hydroxy, C₃-C₁₂-cycloalkyl-C₁-C₄-alkoxy, C₃-C₆-cycloalkoxy, C₁-C₄-alkoxy-C₁-C₄-alkoxy, di-C₁-C₄-alkyl amino-C₁-C₄-alkoxy, C₃-C₁₂-heterocyclyloxy and di-C₁-C₄-alkyl amine (e.g. phenyl, 2-F-phenyl, 4-F-phenyl, 3-F-phenyl, 2-Cl-phenyl, 3-Cl-phenyl, 4-Cl-phenyl, 2,3-diF-phenyl, 2,4-diF-phenyl, 2,5-diF-phenyl, 3,4-diF-phenyl, 2,3,4-triF-phenyl, 2,4-diCl-phenyl, 3,4-diCl-phenyl, 3-F-4-Cl-phenyl, 3-Cl-4-F-phenyl, 3-Cl-5-F-phenyl, 3,4-diF-5-Cl-phenyl, 2-Me-phenyl, 3-Me-phenyl, 4-Me-phenyl, 3-iPr-phenyl, 3-tBu-phenyl, 3-Me-4-F-phenyl, 3-Me-4-Cl-phenyl, 3-iPr-4 Cl-phenyl, 3-(1-OH-1-CF₃-Et)-phenyl, 3-CHF₂-4-F-phenyl, 2-CF₃-phenyl, 3-CF₃-phenyl, 4-CF₃-phenyl, 3-Cl-4-CF₃-phenyl, 2-F-3-CF₃-phenyl, 2-F-5-CF₃-phenyl, 3-F-5-CF₃-phenyl, 2-Cl-4-CF₃-phenyl, 2-Cl-3-CF₃-phenyl, 3-CF₃-4-F-phenyl, 3-CF₃-4-Cl-phenyl, 3-Me-4-CF₃-phenyl, 3-CF₃-4-Me-phenyl, 3,4-diCF₃-phenyl, 4-OMe-phenyl, 3-OiPr-phenyl, 3-CF₃-4-OMe-phenyl, 3-OCH₂CF₃-phenyl, 3-OCF₃-phenyl, 2-OCHF₂-5-Cl-phenyl, 3-OCF₃-4-F-phenyl, 3-OCF₃-4-Cl-phenyl, 3-OBn-phenyl, 3-OPh-phenyl, 3-CN-phenyl, 4-CN-phenyl, 2-F-3-CN-phenyl, 2-F-4-CN-phenyl, 3-F-4-CN-phenyl, 2-F-5-CN-phenyl, 3-F-5-CN-phenyl, 3-Cl-4-CN-phenyl, 2-Cl-5-CN-phenyl, 3-Cl-5-CN-phenyl, 3-CN-4-Cl-phenyl, 2-Me-3-CN-phenyl, 2-Me-5-CN-phenyl, 3-Me-5-CN-phenyl, 3-CF₃-4-CN-phenyl, 3-CN-4-OMe-phenyl, 3-CN-4-OCF₃-phenyl, 3-phenyl-phenyl, 3-MeSO₂-phenyl, 3-(piperidin-4-yl)-phenyl, 2-methylcarbonylamino-5-Cl-phenyl, 3-(pyrid-2-yl)-phenyl, 3-(pyrid-3-yl)-4 F-phenyl, 3-(pyrid-4-yl)-4 F-phenyl, 3-(pyrimid-5-yl)-4 F-phenyl, indan-5-yl, 2-chlor-indan-5-yl, or tetralin-6-yl, a further example being 2,4-diF-3-Cl-phenyl, 3-Et-4-Cl-phenyl, 3-(2-methoxyethyl)-phenyl, 3-(2-OH-ethyl)-phenyl, 3-(1-OH-1-Me-ethyl)-phenyl, 3-CHF₂-phenyl, 3-CF₃-2,4-diF-phenyl, 3-(dimethylamino-methyl)-phenyl, 3-(morpholin-4-yl-methyl)-phenyl, 3-cyclopropyl-phenyl, 3-OEt-phenyl, 3-OPr-phenyl, 3-OtBu-phenyl, 3-(cyclopropylmethoxy)-phenyl, 3-(OMe-methoxy)-phenyl, 3-(2-dimethylamino-ethoxy)-phenyl, 3-CF₃-4-OH-phenyl, 3-OCH₂CHF₂-phenyl, 3-OCHF₂-phenyl, 3-OCHF₂-4-F-phenyl, 3-OCF₃-4-OMe-phenyl, 3-cyclopropoxy-phenyl, 3-methylcarbonyl-phenyl, 3-dimethylamino-phenyl, 3-(2-pyridyloxy)-phenyl, 3-(pyrimidin-2-yloxy)-phenyl, indanyl, indan-2-yl, 2-F-indanyl, 4-F-indanyl, 5-F-indanyl, 6-F-indanyl, 7-F-indanyl, 3-Me-indanyl, 4-Me-indanyl, 5-Me-indanyl, 6-Me-indanyl, 4-CF₃-indanyl, 5-CF₃-indanyl, 6-CF₃-indanyl, 3,3-dimethyl-indanyl, tetralin-2-yl, 7-F-tetralin-2-yl, 6-F-tetralin-2-yl, tetralinyl, 6-F-tetralinyl, 5-F-tetralinyl, or 7-F-tetralinyl), or C₁-C₆-alkoxy (e.g. methoxy, ethoxy, or n-propoxy), C₁-C₆-alkoxy-C₁-C₄-alkoxy (e.g. 2-methoxy-ethoxy), C₆-C₁₂-aryloxy optionally substituted with 1, 2, or 3 substituents independently selected from the group consisting of halogen and C₁-C₄-alkyl (e.g. 4-F-phenoxy or 4-tertbutyl-phenoxy), or C₃-C₁₂-heterocyclyl optionally substituted with 1, 2, or 3 substituents independently selected from the group consisting of halogen, C₁-C₄-alkyl, halogenated C₁-C₄-alkyl, C₆-C₁₂-aryl-C₁-C₆-alkyl, C₃-C₆-cycloalkyl, hydroxy, CN, C₆-C₁₂-aryl optionally substituted with halogen and C₁-C₄-alkyl, C₁-C₄-alkoxy, halogenated C₁-C₄-alkoxy, C₃-C₆-cycloalkoxy, C₆-C₁₂ aryl-C₁-C₄-alkoxy, C₃-C₁₂-heterocyclyl-C₁-C₄-alkoxy and C₃-C₁₂-heterocyclyl, and additionally from the group consisting of C₃-C₆-cycloalkyl-C₁-C₄-alkoxy and halogenated C₁-C₄-alkyl-carbonyl(e.g. propyl-furan-2-yl, 2-CF₃-furan-5-yl, 1,2-dimethyl-5-CN-pyrrol-3-yl, 2,3-diMe-thiophen-5-yl, 2-Me-thiophen-5-yl, 2-Cl-thiophen-5-yl, 3-Cl-thiophen-2-yl, 2,5-diCl-thiophen-3-yl, 2-tetrahydropyranyl-thiophen-5-yl, 1,3-thiazol-5-yl, 4-Me-1,3-thiazol-2-yl, 2-Me-1,3-thiazol-5-yl, 5-Me-1,3-thiazol-2-yl, 4-Me-1,3-thiazol-5-yl, 2-Me-1,3-thiazol-4-yl, 4-isopropyl-1,3-thiazol-2-yl, 2,4-diMe-1,3-thiazol-5-yl, 2-phenyl-4-Me-1,3-thiazol-5-yl, 4-phenyl-1,3-thiazol-5-yl, 2-(4-Me-phenyl)-1,3-thiazol-5-yl, 4-(4-F-phenyl)-1,3-thiazol-2-yl, 2-Cl-1,3-thiazol-4-yl, 4-Cl-1,3-thiazol-5-yl, 2-Br-1,3-thiazol-5-yl, 4-Br-1,3-thiazol-2-yl, 4-Me-5-Br-1,3-thiazol-2-yl, 2,4-dichloro-1,3-thiazol-5-yl, 1,5-dimethyl-1,2,4-triazol-3-yl, pyrid-2-yl, pyrid-3-yl, pyrid-4-yl, 4-F-pyrid-2-yl, 5-F-pyrid-2-yl, 6-F-pyrid-2-yl, 4-Cl-pyrid-2-yl, 5-F-pyrid-3-yl, 2-F-pyrid-4-yl, 3,5-diCl-pyrid-4-yl, 4,5-diCl-pyrid-2-yl, 2-Cl-3F-pyrid-4-yl, 5-Me-pyrid-2-yl, 4-iPr-pyrid-2-yl, 4-Me-5-F-pyrid-2-yl, 4-CF₃-pyrid-2-yl, 5-CF₃-pyrid-2-yl, 6-CF₃-pyrid-2-yl, 5-CF₃-pyrid-3-yl, 2-CF₃-pyrid-4-yl, 3-F-4-CF₃-pyrid-2-yl, 4-CF₃-5-F-pyrid-2-yl, 4-CF₃-5-Cl-pyrid-2-yl, 3,5-diF-4-CF₃-pyrid-2-yl, 4-OCH₂CF₃-pyrid-2-yl, 4-OCH₂CF₃-5-F-pyrid-2-yl, 4-OCH₂CF₃-5-Cl-pyrid-2-yl, 4-OBn-5-F-pyrid-2-yl, 3-(4-F-phenyl)-pyrid-5-yl, 2-(4-F-phenyl)-pyrid-3-yl, 4-(pyrid-4-yl)-pyrid-2-yl, 4-(pyrid-3-yl)-pyrid-2-yl, 5-cyclopropyl-pyraz-2-yl, 5-cyclobutyl-pyraz-2-yl, 5-pyrrolidin-pyraz-2-yl, pyridaz-3-yl, 4-CF₃-pyridaz-3-yl, 5-CF₃-pyridaz-3-yl, 5-F-pyrimid-2-yl, 6-Cl-pyrimid-4-yl, 6-Me-pyrimid-4-yl, 6-Et-pyrimid-4-yl, 6-Pr-pyrimid-4-yl, 6-iPr-pyrimid-4-yl, 4-CF₃-pyrimid-2-yl, 6-CF₃-pyrimid-4-yl, 2-Me-6-Cl-pyrimid-4-yl, 2-Me-6-CF₃-pyrimid-4-yl, 2-OMe-6-CF₃-pyrimid-4-yl, 2-OMe-pyrimid-4-yl, 6-OMe-pyrimid-4-yl, 6-OEt-pyrimid-4-yl, 6-OPr-pyrimid-4-yl, 6-OiPr-pyrimid-4-yl, 6-OiBu-pyrimid-4-yl, 6-OBu-pyrimid-4-yl, 6-OBn-pyrimid-4-yl, 6-cyclobutylloxy-pyrimid-4-yl, 6-cyclopentyloxy-pyrimid-4-yl, 6-cyclohexyloxy-pyrimid-4-yl, 6-cyclopropyl-pyrimid-4-yl, 6-phenyl-pyrimid-4-yl, 6-(2-F- phenyl)-pyrimid-4-yl, 6-(3-F- phenyl)-pyrimid-4-yl, 6-(4-F- phenyl)-pyrimid-4-yl, 6-Cl-pyrimid-4-yl, 6-(2-Me- phenyl)-pyrimid-4-yl, 6-(3-Me- phenyl)-pyrimid-4-yl, 6-(4-Me- phenyl)-pyrimid-4-yl, 6-(3-Cl- phenyl)-pyrimid-4-yl, 6-(4-Cl- phenyl)-pyrimid-4-yl, 2-(morpholin-1-yl)-pyrimid-4-yl, 6-(azetidin-3-yl-methoxy)-pyrimid-4-yl, 6-(pyrrolidin-3-yl-methoxy)-pyrimid-4-yl, 6-(pyrrolidin-2-yl-methoxy)-pyrimid-4-yl, benzofuran-5-yl, 2-Me-benzofuran-3-yl, 2-Et-benzofuran-3-yl, benzothiophen-5-yl, benzothiophen-6-yl, 5-Me-benzothiophen-2-yl, 5-F-benzothiophen-2-yl, 3-Cl-benzothiophen-2-yl, benzothiazol-2-yl, isoquinolin-6-yl, isoquinolin-7-yl, quinolin-6-yl, quinolin-7-yl, 4,5,6,7-tetrahydro-1,3-benzothiazol-2-yl, 2,3-dihydrobenzofuran-5-yl, 7H-pyrrolo[2,3-d]pyrimidin-4-yl, pyrazolo[1,5-a]pyridine-3-yl, pyrazolo[1,5-a]pyridine-7-yl, imidazo[1,2-a]pyridin-8-yl, 5-methyl-imidazo[1,2-a]pyridin-3-yl, 6-chloro-2-methyl-imidazo[1,2-a]pyridin-3-yl, 6-bromo-2-methyl-imidazo[1,2-a]pyridin-3-yl, thieno[2,3-b]pyridin-2-yl, N-benzyl-indolin-6-yl, indolinon-4-yl, chroman-6-yl, 4,4-dimethylchroman-6-yl, 4,4-dimethyl-1,3-dioxan-2-yl, 2-Me-tetrahydrofuran-3-yl, tetrahydrofuran-3-yl, tetrahydropyran-4-yl, 4-Me-tetrahydropyran-4-yl, 2,2-diMe-tetrahydropyran-4-yl, 1-isopropyl-piperidin-4-yl, 1-ethyl-piperidin-3-yl, or 2-CF₃-piperazin-5-yl, a further example being iBu-pyrazol-4-yl, 1-CHF₂-5-Me-1,2-diazol-4-yl, 1-benzyl-5-F-1,2,4-triazol-3-yl, 2-Me-pyrid-4-yl, 2-iPr-pyrid-4-yl, 4-phenyl-pyrid-2yl, 2-cyclopropyl-pyrid-4-yl, 2-OMe-pyrid-4-yl, 2-OEt-pyrid-4-yl, 2-cyclopropylmethoxy-pyrid-4yl, 2-OiPr-pyrid-4-yl, 2-OCH₂CF₃-pyrid-4-yl, 2-cyclopropoxypyrid-4-yl, 2-cyclobutoxy-pyrid-4-yl, 4,5-diCl-pyrimidin-2yl, benzothiophen-3-yl, 2,2-diF-1,3-benzodioxol-5-yl, 5,6,7,8-tetrahydro-isoquinolin-5-yl, 5,6,7,8-tetrahydro-isoquinolin-8-yl, quinolin-8-yl, 1-Me-3,4-dihydro-2H-quinolin-4-yl, 2-Me-3,4-dihydro-1H-isoquinolin-6-yl, 5,6,7,8-tetrahydro-quinolin-5-yl, 5,6,7,8-tetrahydro-quinolin-8-yl, 5,6,7,8-tetrahydro-quinolin-6-yl, 2,3-dihydrobenzofuran-3-yl, pyrazolo[1,5-a]pyrimidin-6-yl, imidazo[1,5-a]pyrazinyl, 3-methylimidazo[1,5-a]pyrazinyl, 3-Me-[1,2,4]triazolo[4,3-a]pyridin-6-yl, imidazo[1,2-a]pyrazin-6-yl, 2-Me-isoindolin-1-on-6-yl, 2,2-dimethyl-7-CF₃-chroman-4-yl, 7-CF₃-chroman-4-yl, 7-OCF₃-chroman-4-yl, isochroman-4-yl, 6,6-dimethyl-1,3-dioxan-2-yl, tetrahydropyran-3-yl, 1-phenylpyrrolidin-3-yl, piperidin-3-yl, 1,3-dimethyl-piperidin-4-yl, 1-cyclopropyl-piperidin-4-yl, 1-(tri-F-methyl-carbonyl)-piperidin-3-yl, quinuclidin-2-yl, 6,7-dihydro-5H-cyclopenta[b]pyridine-7-yl, 6,7-dihydro-5H-cyclopenta[b]pyridine-5-yl, 6,7-dihydro-5H-cyclopenta[c]pyridine-7-yl, 6,7-dihydro-5H-cyclopenta[c]pyridine-5-yl, 5,6-dihydro-4H-cyclopenta[b]thiophen-4-yl, or 1,2,3,5,6,7,8,8a-octahydroindolizin), or tri-(C₁-C₄-alkyl)-silyloxy.

Further preferred embodiments of the pyrrolidine derivatives of formula (Id) result if:
- R¹: is 1,3-diazolyl optionally substituted with halogen or C₁-C₄-alkyl (e.g. 1-methyl-1,3-diazol-4-yl), or 1,2,3-triazolyl optionally substituted with C₁-C₄-alkyl (e.g.1-methyl-1,2,3-triazol-4-yl);
- R^{2a}, R^{2b}: are hydrogen;
- R^{3a}: is C₃-C₁₂-cycloalkyl (e.g. cyclopropyl, or cyclohexyl) C₆-C₁₂-aryl optionally substituted with 1, 2, or 3 substituents independently selected from the group consisting of halogen (e.g. phenyl, 2-Cl-phenyl, 2-Br-phenyl, 3-Cl-phenyl, 4-Cl-phenyl, 2-F-phenyl, 3-F-phenyl, 4-F-phenyl, or 2,4,5-trifluoro-phenyl), or hydroxy, C₁-C₆-alkoxy (e.g.methoxy, or iso-butoxy), C₃-C₁₂-cycloalkyl-C₁-C₄-alkoxy (e.g. cyclopropylmethoxy), C₂-C₆-alkenyloxy (e.g. allyloxy or 2-methylprop-2-en-1-oxy), C₆-C₁₂-aryl-C₁-C₄-alkoxy (e.g. benzyloxy), C₆-C₁₂-aryloxy (e.g. 4-F-phenoxy) optionally substituted with 1, 2, or 3 substituents independently selected from the group consisting of halogen, or C₃-C₂-heterocyclyl optionally substituted with 1, 2, or 3 substituents independently selected from the group consisting of halogen or C₃-C₆-cycloalkyl (e.g. tetrahydrofuran-2-yl, tetrahydrofuran-3-yl, tetrahydropyran-2-yl, tetrahydropyran-3-yl, 1-cyclopropyl-piperidin-4-yl, 1-cyclopropyl-piperidin-3-yl, 2-pyridyl, 3-pyridyl, 3-F-pyrid-2-yl, 1,3-oxazol-4-yl, or 1,3-oxazol-2-yl, a further example being 1,3-dioxan-2-yl, 5,5-dimethyl-1,3-dioxan-2-yl, 4,6-dimethyl-1,3-dioxan-2-yl, 1,3-dioxepan-2-yl, 1,3-dioxolan-2-yl, 5,7-dioxaspiro[2.5]octan-6-yl, morpholin-3-yl, pyrrolidinyl, piperidinyl,1-Me-piperidin-2-yl, or 5-F-pyrid-2-yl); or
- R^{3a} and one of R^{2a} or R^{2b}: together with the carbon atoms to which they are bound may form an anellated C₆-C₁₂-aryl;
- R^{3b}: is hydrogen or hydroxy;
- Y¹: is >CR⁶;
- R⁶: is hydrogen, C₁-C₆-alkyl (e.g. methyl), or hydroxy-C₁-C₆-alkyl (e.g.-CH₂OH); or
- R⁶ and R^{3a} or R^{3b}: together are C₁-C₅-alkylene; or
- R⁶: is C₁-C₄-alkylene (e.g. methylene or 1,2-ethylene) that is bound to a carbon atom in R^{3a}, and
R^{3a} is an optionally substituted C₆-C₁₂-aryl (e.g. phenyl);
- R^{8a}: is hydrogen, C₁-C₆-alkyl (e.g. methyl, ethyl, n-propyl, iso-propyl, pentyl, or hexyl), or C₁-C₆-alkylcarbonyl (e.g. methylcarbonyl), or
- R⁶, R^{8a}: are together optionally substituted C₁-C₅-alkylene, wherein one or more -CH₂- of C₁-C₅-alkylene may be independently replaced by a an oxygen atom or C=O e. g. -C(O)OCH₂-); or R
- ^{3a} and R^{8a}: together are optionally substituted C₁-C₅-alkylene (e.g. 1,2-ethylene or 1,3-propylene);
- R^{9a}: is hydrogen, halogen, C₁-C₆-alkyl (e.g. methyl, ethyl, tert-butyl, or 2,3-dimethyl-propyl), halogenated C₁-C₆-alkyl (e.g. trifluoromethyl) or C₁-C₆-alkoxy (e.g. methoxy or ethoxy);
- R^{9b}: is hydrogen or halogen;
- n4: is 0, 1, 2, 3, or 4;
- R¹³: is hydrogen, C₁-C₈-alkyl (e.g. methyl, ethyl, n-butyl, tert-butyl, pentyl, or hexyl), halogenated C₁-C₆-alkyl (e.g. -CF₃ or -CF₂Me), C₁-C₆-alkoxy-C₁-C₆-alkyl (e.g. methoxy-methyl), C₃-C₁₂-cycloalkyl optionally substituted with 1, 2, or 3 substituents independently selected from the group consisting of halogen, C₁-C₄-alkyl, halogenated C₁-C₄-alkyl, C₁-C₄-alkoxy-C₁-C₄-alkyl and C₆-C₁₂-aryl, which may be additionally substituted with halogen, and additionally from the group consisting of C₁-C₄-alkenyl, C₁-C₄-alkoxy and C₃-C₁₂-heterocyclyl (e.g. cyclopropyl, 2-propyl-cyclopropyl, 1-(methoxy-methyl)-cyclopropyl, 2-phenyl-cyclopropyl, cyclopentyl, cyclopentyl, 3,3-dimethylcyclopentyl, cyclohexyl, 3,3-dimethyl-cyclohexyl, 4,4-dimethyl-cyclohexyl, 1-methyl-cyclohexyl, 1-CF₃-cyclopropyl, 4-CF₃-cyclohexyl, 3-CF₃-cyclohexyl, or 4,4-diF-cyclohexyl, a further example being 1-phenyl-cyclopropyl, 1-(4-F-phenyl)-cyclopropyl, (3-Cl-phenyl)-cyclopropyl, 2-phenyl-cyclobutyl, 3-phenyl-cyclobutyl, 3-(4-F-phenyl)-cyclobutyl, 3-(2-F-phenyl)-cyclobutyl, 2-Cl-cyclopentyl, 2-Me-cyclopentyl, 3-Me-cyclopentyl, 2-phenyl-cyclopentyl, 3-(2-pyridyl)-cyclopentyl, 3-Me-cyclohexyl, 3,3,5,5-tetraMe-cyclohexyl, 4-Me-cyclohexyl, 4-Et-cyclohexyl, 2-CF₃-cyclohexyl, 2-Me-5-isopropenyl-cyclohexyl, 2-phenyl-cyclohexyl, 3-phenyl-cyclohexyl, 4-phenyl-cyclohexyl, 2-EtO-cyclohexyl, decalin-1-yl, decalin-2-yl, norbornan-2-yl, 1,7,7-trimethyl-norbornan-2-yl, 5-iPr-2-Me-3-bicyclo[3.1.0]hexanyl, bicycle [3.2.1]octanyl, or 3-bicyclo[1.1.1]pentanyl), or C₂-C₆-alkenyl (e.g. hex-2-enyl), C₃-C₆-cycloalkenyl optionally substituted with 1, 2, or 3 substituents independently selected from the group consisting of C₁-C₄-alkyl (e.g. 1,3,3-trimethylcyclohexen-2-yl), or C₆-C₁₂-aryl optionally substituted with 1, 2, or 3 substituents independently selected from the group consisting of halogen, C₁-C₄-alkyl, halogenated C₁-C₄-alkyl, hydroxy-(halogenated C₁-C₄-alkyl), CN, C₆-C₁₂-aryl, C₁-C₄-alkoxy, halogenated C₁-C₄-alkoxy, C₆-C₁₂ aryl-C₁-C₄-alkoxy, C₆-C₁₂-aryloxy, C₁-C₄-alkylsulfonyl, C₁-C₄-alkyl-carbonylamino and C₃-C₁₂-heterocyclyl, and additionally from the group consisting of hydroxy-C₁-C₄-alkyl, C₁-C₄-alkoxy-C₁-C₄-alkyl, di-C₁-C₄-alkyl amino-C₁-C₄-alkyl, C₃-C₁₂-heterocyclyl-C₁-C₄-alkyl, C₃-C₆-cycloalkyl, C₁-C₄-alkyl-carbonyl, hydroxy, C₃-C₁₂-cycloalkyl-C₁-C₄-alkoxy, C₃-C₆-cycloalkoxy, C₁-C₄-alkoxy-C₁-C₄-alkoxy, di-C₁-C₄-alkyl amino-C₁-C₄-alkoxy, C₃-C₁₂-heterocyclyloxy and di-C₁-C₄-alkyl amino (e.g. phenyl, 2-F-phenyl, 4-F-phenyl, 3-F-phenyl, 2-Cl-phenyl, 3-Cl-phenyl, 4-Cl-phenyl, 2,3-diF-phenyl, 2,4-diF-phenyl, 2,5-diF-phenyl, 3,4-diF-phenyl, 2,3,4-triF-phenyl, 2,4-diCl-phenyl, 3,4-diCl-phenyl, 3-F-4-Cl-phenyl, 3-Cl-4-F-phenyl, 3-Cl-5-F-phenyl, 3,4-diF-5-Cl-phenyl, 2-Me-phenyl, 3-Me-phenyl, 4-Me-phenyl, 3-iPr-phenyl, 3-tBu-phenyl, 3-Me-4-F-phenyl, 3-Me-4-Cl-phenyl, 3-iPr-4 Cl-phenyl, 3-(1-OH-1-CF₃-Et)-phenyl, 3-CHF₂-4-F-phenyl, 2-CF₃-phenyl, 3-CF₃-phenyl, 4-CF₃-phenyl, 3-Cl-4-CF₃-phenyl, 2-F-3-CF₃-phenyl, 2-F-5-CF₃-phenyl, 3-F-5-CF₃-phenyl, 2-Cl-4-CF₃-phenyl, 2-Cl-3-CF₃-phenyl, 3-CF₃-4-F-phenyl, 3-CF₃-4-Cl-phenyl, 3-Me-4-CF₃-phenyl, 3-CF₃-4-Me-phenyl, 3,4-diCF₃-phenyl, 4-OMe-phenyl, 3-OiPr-phenyl, 3-CF₃-4-OMe-phenyl, 3-OCH₂CF₃-phenyl, 3-OCF₃-phenyl, 2-OCHF₂-5-Cl-phenyl, 3-OCF₃-4-F-phenyl, 3-OCF₃-4-Cl-phenyl, 3-OBn-phenyl, 3-OPh-phenyl, 3-CN-phenyl, 4-CN-phenyl, 2-F-3-CN-phenyl, 2-F-4-CN-phenyl, 3-F-4-CN-phenyl, 2-F-5-CN-phenyl, 3-F-5-CN-phenyl, 3-Cl-4-CN-phenyl, 2-Cl-5-CN-phenyl, 3-Cl-5-CN-phenyl, 3-CN-4-Cl-phenyl, 2-Me-3-CN-phenyl, 2-Me-5-CN-phenyl, 3-Me-5-CN-phenyl, 3-CF₃-4-CN-phenyl, 3-CN-4-OMe-phenyl, 3-CN-4-OCF₃-phenyl, 3-phenyl-phenyl, 3-MeSO₂-phenyl, 3-(piperidin-4-yl)-phenyl, 2-methylcarbonylamino-5-Cl-phenyl, 3-(pyrid-2-yl)-phenyl, 3-(pyrid-3-yl)-4 F-phenyl, 3-(pyrid-4-yl)-4 F-phenyl, 3-(pyrimid-5-yl)-4 F-phenyl, indan-5-yl, 2-chlor-indan-5-yl, or tetralin-6-yl, a further example being 2,4-diF-3-Cl-phenyl, 3-Et-4-Cl-phenyl, 3-(2-methoxyethyl)-phenyl, 3-(2-OH-ethyl)-phenyl, 3-(1-OH-1-Me-ethyl)-phenyl, 3-CHF₂-phenyl, 3-CF₃-2,4-diF-phenyl, 3-(dimethylamino-methyl)-phenyl, 3-( morpholin-4-yl-methyl)-phenyl, 3-cyclopropyl-phenyl, 3-OEt-phenyl, 3-OPr-phenyl, 3-OtBu-phenyl, 3-(cyclopropylmethoxy)-phenyl, 3-(OMe-methoxy)-phenyl, 3-(2-dimethylamino-ethoxy)-phenyl, 3-CF₃-4-OH-phenyl, 3-OCH₂CHF₂-phenyl, 3-OCHF₂-phenyl, 3-OCHF₂-4-F-phenyl, 3-OCF₃-4-OMe-phenyl, 3-cyclopropoxy-phenyl, 3-methylcarbonyl-phenyl, 3-dimethylamino-phenyl, 3-(2-pyridyloxy)-phenyl, 3-(pyrimidin-2-yloxy)-phenyl, indanyl, indan-2-yl, 2-F-indanyl, 4-F-indanyl, 5-F-indanyl, 6-F-indanyl, 7-F-indanyl, 3-Me-indanyl, 4-Me-indanyl, 5-Me-indanyl, 6-Me-indanyl, 4-CF₃-indanyl, 5-CF₃-indanyl, 6-CF₃-indanyl, 3,3-dimethyl-indanyl, tetralin-2-yl, 7-F-tetralin-2-yl, 6-F-tetralin-2-yl, tetralinyl, 6-F-tetralinyl, 5-F-tetralinyl, or 7-F-tetralinyl), or C₁-C₆-alkoxy (e.g.methoxy, ethoxy, or n-propoxy), C₁-C₆-alkoxy-C₁-C₄-alkoxy (e.g. 2-methoxy-ethoxy), C₆-C₁₂-aryloxy optionally substituted with 1, 2, or 3 substituents independently selected from the group consisting of halogen and C₁-C₄-alkyl (e.g. 4-F-phenoxy or 4-tertbutyl-phenoxy), or C₃-C₁₂-heterocyclyl optionally substituted with 1, 2, or 3 substituents independently selected from the group consisting of halogen, C₁-C₄-alkyl, halogenated C₁-C₄-alkyl, C₆-C₁₂-aryl-C₁-C₆-alkyl, C₃-C₆-cycloalkyl, hydroxy, CN, C₆-C₁₂-aryl optionally substituted with halogen and C₁-C₄-alkyl, C₁-C₄-alkoxy, halogenated C₁-C₄-alkoxy, C₃-C₆-cycloalkoxy, C₆-C₁₂ aryl-C₁-C₄-alkoxy, C₃-C₁₂-heterocyclyl-C₁-C₄-alkoxy and C₃-C₁₂-heterocyclyl and additionally from the group consisting of C₃-C₆-cycloalkyl-C₁-C₄-alkoxy and halogenated C₁-C₄-alkylcarbonyl (e.g. n-propyl-furan-2-yl, 2-CF₃-furan-5-yl, 1,2-dimethyl-5-CN-pyrrol-3-yl, 2,3-diMe-thiophen-5-yl, 2-Me-thiophen-5-yl, 2-Cl-thiophen-5-yl, 3-Cl-thiophen-2-yl, 2,5-diCl-thiophen-3-yl, 2-tetrahydropyranyl-thiophen-5-yl, 1,3-thiazol-5-yl, 4-Me-1,3-thiazol-2-yl, 2-Me-1,3-thiazol-5-yl, 5-Me-1,3-thiazol-2-yl, 4-Me-1,3-thiazol-5-yl, 2-Me-1,3-thiazol-4-yl, 4-isopropyl-1,3-thiazol-2-yl, 2,4-diMe-1,3-thiazol-5-yl, 2-phenyl-4-Me-1,3-thiazol-5-yl, 4-phenyl-1,3-thiazol-5-yl, 2-(4-Me-phenyl)-1,3-thiazol-5-yl, 4-(4-F-phenyl)-1,3-thiazol-2-yl, 2-Cl-1,3-thiazol-4-yl, 4-Cl-1,3-thiazol-5-yl, 2-Br-1,3-thiazol-5-yl, 4-Br-1,3-thiazol-2-yl, 4-Me-5-Br-1,3-thiazol-2-yl, 2,4-dichloro-1,3-thiazol-5-yl, 1,5-dimethyl-1,2,4-triazol-3-yl, pyrid-2-yl, pyrid-3-yl, pyrid-4-yl, 4-F-pyrid-2-yl, 5-F-pyrid-2-yl, 6-F-pyrid-2-yl, 4-Cl-pyrid-2-yl, 5-F-pyrid-3-yl, 2-F-pyrid-4-yl, 3,5-diCl-pyrid-4-yl, 4,5-diCl-pyrid-2-yl, 2-Cl-3F-pyrid-4-yl, 5-Me-pyrid-2-yl, 4-iPr-pyrid-2-yl, 4-Me-5-F-pyrid-2-yl, 4-CF₃-pyrid-2-yl, 5-CF₃-pyrid-2-yl, 6-CF₃-pyrid-2-yl, 5-CF₃-pyrid-3-yl, 2-CF₃-pyrid-4-yl, 3-F-4-CF₃-pyrid-2-yl, 4-CF₃-5-F-pyrid-2-yl, 4-CF₃-5-Cl-pyrid-2-yl, 3,5-diF-4-CF₃-pyrid-2-yl, 4-OCH₂CF₃-pyrid-2-yl, 4-OCH₂CF₃-5-F-pyrid-2-yl, 4-OCH₂CF₃-5-Cl-pyrid-2-yl, 4-OBn-5-F-pyrid-2-yl, 3-(4-F-phenyl)-pyrid-5-yl, 2-(4-F-phenyl)-pyrid-3-yl, 4-(pyrid-4-yl)-pyrid-2-yl, 4-(pyrid-3-yl)-pyrid-2-yl, 5-cyclopropyl-pyraz-2-yl, 5-cyclobutyl-pyraz-2-yl, 5-pyrrolidin-pyraz-2-yl, pyridaz-3-yl, 4-CF₃-pyridaz-3-yl, 5-CF₃-pyridaz-3-yl, 5-F-pyrimid-2-yl, 6-Cl-pyrimid-4-yl, 6-Me-pyrimid-4-yl, 6-Et-pyrimid-4-yl, 6-Pr-pyrimid-4-yl, 6-iPr-pyrimid-4-yl, 4-CF₃-pyrimid-2-yl, 6-CF₃-pyrimid-4-yl, 2-Me-6-Cl-pyrimid-4-yl, 2-Me-6-CF₃-pyrimid-4-yl, 2-OMe-6-CF₃-pyrimid-4-yl, 2-OMe-pyrimid-4-yl, 6-OMe-pyrimid-4-yl, 6-OEt-pyrimid-4-yl, 6-OPr-pyrimid-4-yl, 6-OiPr-pyrimid-4-yl, 6-OiBu-pyrimid-4-yl, 6-OBu-pyrimid-4-yl, 6-OBn-pyrimid-4-yl, 6-cyclobutylloxy-pyrimid-4-yl, 6-cyclopentyloxy-pyrimid-4-yl, 6-cyclohexyloxy-pyrimid-4-yl, 6-cyclopropyl-pyrimid-4-yl, 6-phenyl-pyrimid-4-yl, 6-(2-F-phenyl)-pyrimid-4-yl, 6-(3-F- phenyl)-pyrimid-4-yl, 6-(4-F- phenyl)-pyrimid-4-yl, 6-Cl-pyrimid-4-yl, 6-(2-Me- phenyl)-pyrimid-4-yl, 6-(3-Me- phenyl)-pyrimid-4-yl, 6-(4-Me-phenyl)-pyrimid-4-yl, 6-(3-Cl- phenyl)-pyrimid-4-yl, 6-(4-Cl- phenyl)-pyrimid-4-yl, 2-(morpholin-1-yl)-pyrimid-4-yl, 6-(azetidin-3-yl-methoxy)-pyrimid-4-yl, 6-(pyrrolidin-3-ylmethoxy)-pyrimid-4-yl, 6-(pyrrolidin-2-yl-methoxy)-pyrimid-4-yl, benzofuran-5-yl, 2-Mebenzofuran-3-yl, 2-Et-benzofuran-3-yl, benzothiophen-5-yl, benzothiophen-6-yl, 5-Mebenzothiophen-2-yl, 5-F-benzothiophen-2-yl, 3-Cl-benzothiophen-2-yl, benzothiazol-2-yl, isoquinolin-6-yl, isoquinolin-7-yl, quinolin-6-yl, quinolin-7-yl, 4,5,6,7-tetrahydro-1,3-benzothiazol-2-yl, 2,3-dihydrobenzofuran-5-yl, 7H-pyrrolo[2,3-d]pyrimidin-4-yl, pyrazolo[1,5-a]pyridine-3-yl, pyrazolo[1,5-a]pyridine-7-yl, imidazo[1,2-a]pyridin-8-yl, 5-methylimidazo[1,2-a]pyridin-3-yl, 6-chloro-2-methyl-imidazo[1,2-a]pyridin-3-yl, 6-bromo-2-methyl-imidazo[1,2-a]pyridin-3-yl, thieno[2,3-b]pyridin-2-yl, N-benzyl-indolin-6-yl, indolinon-4-yl, chroman-6-yl, 4,4-dimethylchroman-6-yl, 4,4-dimethyl-1,3-dioxan-2-yl, 2-Metetrahydrofuran-3-yl, tetrahydrofuran-3-yl, tetrahydropyran-4-yl, 4-Me-tetrahydropyran-4-yl, 2,2-diMe-tetrahydropyran-4-yl, 1-isopropyl-piperidin-4-yl, 1-ethyl-piperidin-3-yl, or 2-CF₃-piperazin-5-yl, a further example being iBu-pyrazol-4-yl, 1-CHF₂-5-Me-1,2-diazol-4-yl, 1-benzyl-5-F-1,2,4-triazol-3-yl, 2-Me-pyrid-4-yl, 2-iPr-pyrid-4-yl, 4-phenyl-pyrid-2yl, 2-cyclopropyl-pyrid-4-yl, 2-OMe-pyrid-4-yl, 2-OEt-pyrid-4-yl, 2-cyclopropylmethoxy-pyrid-4yl, 2-OiPr-pyrid-4-yl, 2-OCH₂CF₃-pyrid-4-yl, 2-cyclopropoxy-pyrid-4-yl, 2-cyclobutoxypyrid-4-yl, 4,5-diCl-pyrimidin-2yl, benzothiophen-3-yl, 2,2-diF-1,3-benzodioxol-5-yl, 5,6,7,8-tetrahydro-isoquinolin-5-yl, 5,6,7,8-tetrahydro-isoquinolin-8-yl, quinolin-8-yl, 1-Me-3,4-dihydro-2H-quinolin-4-yl, 2-Me-3,4-dihydro-1H-isoquinolin-6-yl, 5,6,7,8-tetrahydro-quinolin-5-yl, 5,6,7,8-tetrahydro-quinolin-8-yl, 5,6,7,8-tetrahydro-quinolin-6-yl, 2,3-dihydrobenzofuran-3-yl, pyrazolo[1,5-a]pyrimidin-6-yl, imidazo[1,5-a]pyrazinyl, 3-methyl-imidazo[1,5-a]pyrazinyl, 3-Me-[1,2,4]triazolo[4,3-a]pyridin-6-yl, imidazo[1,2-a]pyrazin-6-yl, 2-Me-isoindolin-1-on-6-yl, 2,2-dimethyl-7-CF₃-chroman-4-yl, 7-CF₃-chroman-4-yl, 7-OCF₃-chroman-4-yl, isochroman-4-yl, 6,6-dimethyl-1,3-dioxan-2-yl, tetrahydropyran-3-yl, 1-phenyl-pyrrolidin-3-yl, piperidin-3yl, 1,3-dimethyl-piperidin-4yl, 1-cyclopropyl-piperidin-4-yl, 1-(tri-F-methyl-carbonyl)-piperidin-3-yl, quinuclidin-2-yl, 6,7-dihydro-5H-cyclopenta[b]pyridine-7-yl, 6,7-dihydro-5H-cyclopenta[b]pyridine-5-yl, 6,7-dihydro-5H-cyclopenta[c]pyridine-7-yl, 6,7-dihydro-5H-cyclopenta[c]pyridine-5-yl, 5,6-dihydro-4H-cyclopenta[b]thiophen-4-yl, or 1,2,3,5,6,7,8,8a-octahydroindolizin), or tri-(C₁-C₄-alkyl)-silyloxy; and
- R^{5a}, R^{5b}: are hydrogen.

Further preferred embodiments of the pyrrolidine derivatives of formula (Id) result if:
- R^{3a}: is C₃-C₁₂-cycloalkyl (e.g. cyclopropyl, or cyclohexyl) C₆-C₁₂-aryl optionally substituted with 1, 2, or 3 substituents independently selected from the group consisting of halogen (e.g. phenyl, 2-Cl-phenyl, 2-Br-phenyl, 3-Cl-phenyl, 4-Cl-phenyl, 2-F-phenyl, 3-F-phenyl, 4-F-phenyl, or 2,4,5-trifluoro-phenyl), or hydroxy, C₁-C₆-alkoxy (e.g.methoxy, or iso-butoxy), C₃-C₁₂-cycloalkyl-C₁-C₄-alkoxy (e.g. cyclopropylmethoxy), C₂-C₆-alkenyloxy (e.g. allyloxy or 2-methylprop-2-en-1-oxy), C₆-C₁₂-aryl-C₁-C₄-alkoxy (e.g. benzyloxy), C₆-C₁₂-aryloxy (e.g. 4-F-phenoxy) optionally substituted with 1, 2, or 3 substituents independently selected from the group consisting of halogen, or C₃-C₂-heterocyclyl optionally substituted with 1, 2, or 3 substituents independently selected from the group consisting of halogen or C₃-C₆-cycloalkyl (e.g. tetrahydrofuran-2-yl, tetrahydrofuran-3-yl, tetrahydropyran-2-yl, tetrahydropyran-3-yl, 1-cyclopropyl-piperidin-4-yl, 1-cyclopropyl-piperidin-3-yl, 2-pyridyl, 3-pyridyl, 3-F-pyrid-2-yl, 1,3-oxazol-4-yl, or 1,3-oxazol-2-yl, a further example being 1,3-dioxan-2-yl, 5,5-dimethyl-1,3-dioxan-2-yl, 4,6-dimethyl-1,3-dioxan-2-yl, 1,3-dioxepan-2-yl, 1,3-dioxolan-2-yl, 5,7-dioxaspiro[2.5]octan-6-yl, morpholin-3-yl, pyrrolidinyl, piperidinyl,1-Me-piperidin-2-yl, or 5-F-pyrid-2-yl);
- R⁶: is hydrogen, C₁-C₆-alkyl (e.g. methyl), or hydroxy-C₁-C₆-alkyl (e.g.-CH₂OH);
- R^{8a}: is hydrogen, C₁-C₆-alkyl (e.g. methyl, ethyl, n-propyl, iso-propyl, pentyl, or hexyl), or C₁-C₆-alkylcarbonyl (e.g. methylcarbonyl), or
- R⁶, R^{8a}: are together optionally substituted C₁-C₅-alkylene, wherein one or more -CH₂- of C₁-C₅-alkylene may be independently replaced by a an oxygen atom or C=O e. g. -C(O)OCH₂-);
- R^{9a}: is hydrogen, halogen, C₁-C₆-alkyl (e.g. methyl, ethyl, tert-butyl, or 2,3-dimethyl-propyl), or C₁-C₆-alkoxy (e.g. methoxy or ethoxy);
- R^{9b}: is hydrogen, and
- R¹³: is hydrogen, C₁-C₈-alkyl (e.g. methyl, ethyl, n-butyl, tert-butyl, pentyl, or hexyl), halogenated C₁-C₆-alkyl (e.g. -CF₃ or -CF₂Me), C₁-C₆-alkoxy-C₁-C₆-alkyl (e.g. methoxy-methyl), C₃-C₁₂-cycloalkyl optionally substituted with 1, 2, or 3 substituents independently selected from the group consisting of halogen, C₁-C₄-alkyl, halogenated C₁-C₄-alkyl, C₁-C₄-alkoxy-C₁-C₄-alkyl and C₆-C₁₂-aryl (e.g. cyclopropyl, 2-propyl-cyclopropyl, 1-(methoxy-methyl)-cyclopropyl, 2-phenyl-cyclopropyl, cyclopentyl, cyclopentyl, 3,3-dimethylcyclopentyl, cyclohexyl, 3,3-dimethyl-cyclohexyl, 4,4-dimethyl-cyclohexyl, 1-methyl-cyclohexyl, 1-CF₃-cyclopropyl, 4-CF₃-cyclohexyl, 3-CF₃-cyclohexyl, or 4,4-diF-cyclohexyl, a further example being 1-phenyl-cyclopropyl, 2-phenyl-cyclobutyl, 3-phenyl-cyclobutyl, 2-Cl-cyclopentyl, 2-Me-cyclopentyl, 3-Me-cyclopentyl, 2-phenyl-cyclopentyl, 3-Me-cyclohexyl, 3,3,5,5-tetraMe-cyclohexyl, 4-Me-cyclohexyl, 4-Et-cyclohexyl, 2-CF₃-cyclohexyl, 2-phenyl-cyclohexyl, 3-phenyl-cyclohexyl, 4-phenyl-cyclohexyl, decalin-1-yl, decalin-2-yl, norbornan-2-yl, 1,7,7-trimethyl-norbornan-2-yl, 5-iPr-2-Me-3-bicyclo[3.1.0]hexanyl, bicycle [3.2.1]octanyl, or 3-bicyclo[1.1.1]pentanyl), or C₂-C₆-alkenyl (e.g. hex-2-enyl), C₃-C₆-cycloalkenyl optionally substituted with 1, 2, or 3 substituents independently selected from the group consisting of C₁-C₄-alkyl (e.g. 1,3,3-trimethylcyclohexen-2-yl), or C₆-C₁₂-aryl optionally substituted with 1, 2, or 3 substituents independently selected from the group consisting of halogen, C₁-C₄-alkyl, halogenated C₁-C₄-alkyl, hydroxy-(halogenated C₁-C₄-alkyl), CN, C₆-C₁₂-aryl, C₁-C₄-alkoxy, halogenated C₁-C₄-alkoxy, C₆-C₁₂ aryl-C₁-C₄-alkoxy, C₆-C₁₂-aryloxy, C₁-C₄-alkyl-sulfonyl, C₁-C₄-alkyl-carbonylamino and C₃-C₁₂-heterocyclyl, (e.g. phenyl, 2-F-phenyl, 4-F-phenyl, 3-F-phenyl, 2-Cl-phenyl, 3-Cl-phenyl, 4-Cl-phenyl, 2,3-diF-phenyl, 2,4-diF-phenyl, 2,5-diF-phenyl, 3,4-diF-phenyl, 2,3,4-triF-phenyl, 2,4-diCl-phenyl, 3,4-diCl-phenyl, 3-F-4-Cl-phenyl, 3-Cl-4-F-phenyl, 3-Cl-5-F-phenyl, 3,4-diF-5-Cl-phenyl, 2-Me-phenyl, 3-Me-phenyl, 4-Me-phenyl, 3-iPr-phenyl, 3-tBu-phenyl, 3-Me-4-F-phenyl, 3-Me-4-Cl-phenyl, 3-iPr-4 Cl-phenyl, 3-(1-OH-1-CF₃-Et)-phenyl, 3-CHF₂-4-F-phenyl, 2-CF₃-phenyl, 3-CF₃-phenyl, 4-CF₃-phenyl, 3-Cl-4-CF₃-phenyl, 2-F-3-CF₃-phenyl, 2-F-5-CF₃-phenyl, 3-F-5-CF₃-phenyl, 2-Cl-4-CF₃-phenyl, 2-Cl-3-CF₃-phenyl, 3-CF₃-4-F-phenyl, 3-CF₃-4-Cl-phenyl, 3-Me-4-CF₃-phenyl, 3-CF₃-4-Me-phenyl, 3,4-diCF₃-phenyl, 4-OMe-phenyl, 3-OiPr-phenyl, 3-CF₃-4-OMe-phenyl, 3-OCH₂CF₃-phenyl, 3-OCF₃-phenyl, 2-OCHF₂-5-Cl-phenyl, 3-OCF₃-4-F-phenyl, 3-OCF₃-4-Cl-phenyl, 3-OBn-phenyl, 3-OPh-phenyl, 3-CN-phenyl, 4-CN-phenyl, 2-F-3-CN-phenyl, 2-F-4-CN-phenyl, 3-F-4-CN-phenyl, 2-F-5-CN-phenyl, 3-F-5-CN-phenyl, 3-Cl-4-CN-phenyl, 2-Cl-5-CN-phenyl, 3-Cl-5-CN-phenyl, 3-CN-4-Cl-phenyl, 2-Me-3-CN-phenyl, 2-Me-5-CN-phenyl, 3-Me-5-CN-phenyl, 3-CF₃-4-CN-phenyl, 3-CN-4-OMe-phenyl, 3-CN-4-OCF₃-phenyl, 3-phenyl-phenyl, 3-MeSO₂-phenyl, 3-(piperidin-4-yl)-phenyl, 2-methylcarbonylamino-5-Cl-phenyl, 3-(pyrid-2-yl)-phenyl, 3-(pyrid-3-yl)-4 F-phenyl, 3-(pyrid-4-yl)-4 F-phenyl, 3-(pyrimid-5-yl)-4 F-phenyl, indan-5-yl, 2-chlor-indan-5-yl, or tetralin-6-yl, a further example being 2,4-diF-3-Cl-phenyl, 3-Et-4-Cl-phenyl, 3-CHF₂-phenyl, 3-CF₃-2,4-diF-phenyl, 3-OEt-phenyl, 3-OPr-phenyl, 3-OtBu-phenyl, 3-OCH₂CHF₂-phenyl, 3-OCHF₂-phenyl, 3-OCHF₂-4-F-phenyl, 3-OCF₃-4-OMe-phenyl, 3-(2-pyridyloxy)-phenyl, 3-(pyrimidin-2-yloxy)-phenyl, indanyl, indan-2-yl, 2-F-indanyl, 4-F-indanyl, 5-F-indanyl, 6-F-indanyl, 7-F-indanyl, 3-Me-indanyl, 4-Me-indanyl, 5-Me-indanyl, 6-Me-indanyl, 4-CF₃-indanyl, 5-CF₃-indanyl, 6-CF₃-indanyl, 3,3-dimethyl-indanyl, tetralin-2-yl, 7-F-tetralin-2-yl, 6-F-tetralin-2-yl, tetralinyl, 6-F-tetralinyl, 5-F-tetralinyl, or 7-F-tetralinyl), or C₁-C₆-alkoxy (e.g.methoxy, ethoxy, or n-propoxy), C₁-C₆-alkoxy-C₁-C₄-alkoxy (e.g. 2-methoxy-ethoxy), C₆-C₁₂-aryloxy optionally substituted with 1, 2, or 3 substituents independently selected from the group consisting of halogen and C₁-C₄-alkyl (e.g. 4-F-phenoxy or 4-tertbutyl-phenoxy), or C₃-C₂-heterocyclyl optionally substituted with 1, 2, or 3 substituents independently selected from the group consisting of halogen, C₁-C₄-alkyl, halogenated C₁-C₄-alkyl, C₆-C₁₂-aryl-C₁-C₆-alkyl, C₃-C₆-cycloalkyl, hydroxy, CN, C₆-C₁₂-aryl optionally substituted with halogen and C₁-C₄-alkyl, C₁-C₄-alkoxy, halogenated C₁-C₄-alkoxy, C₃-C₆-cycloalkoxy, C₆-C₁₂ aryl-C₁-C₄-alkoxy, C₃-C₁₂-heterocyclyl-C₁-C₄-alkoxy and C₃-C₁₂-heterocyclyl (e.g. n-propyl-furan-2-yl, 2-CF₃-furan-5-yl, 1,2-dimethyl-5-CN-pyrrol-3-yl, 2,3-diMe-thiophen-5-yl, 2-Me-thiophen-5-yl, 2-Cl-thiophen-5-yl, 3-Cl-thiophen-2-yl, 2,5-diCl-thiophen-3-yl, 2-tetrahydropyranyl-thiophen-5-yl, 1,3-thiazol-5-yl, 4-Me-1,3-thiazol-2-yl, 2-Me-1,3-thiazol-5-yl, 5-Me-1,3-thiazol-2-yl, 4-Me-1,3-thiazol-5-yl, 2-Me-1,3-thiazol-4-yl, 4-isopropyl-1,3-thiazol-2-yl, 2,4-diMe-1,3-thiazol-5-yl, 2-phenyl-4-Me-1,3-thiazol-5-yl, 4-phenyl-1,3-thiazol-5-yl, 2-(4-Me-phenyl)-1,3-thiazol-5-yl, 4-(4-F-phenyl)-1,3-thiazol-2-yl, 2-Cl-1,3-thiazol-4-yl, 4-Cl-1,3-thiazol-5-yl, 2-Br-1,3-thiazol-5-yl, 4-Br-1,3-thiazol-2-yl, 4-Me-5-Br-1,3-thiazol-2-yl, 2,4-dichloro-1,3-thiazol-5-yl, 1,5-dimethyl-1,2,4-triazol-3-yl, pyrid-2-yl, pyrid-3-yl, pyrid-4-yl, 4-F-pyrid-2-yl, 5-F-pyrid-2-yl, 6-F-pyrid-2-yl, 4-Cl-pyrid-2-yl, 5-F-pyrid-3-yl, 2-F-pyrid-4-yl, 3,5-diCl-pyrid-4-yl, 4,5-diCl-pyrid-2-yl, 2-Cl-3F-pyrid-4-yl, 5-Me-pyrid-2-yl, 4-iPr-pyrid-2-yl, 4-Me-5-F-pyrid-2-yl, 4-CF₃-pyrid-2-yl, 5-CF₃-pyrid-2-yl, 6-CF₃-pyrid-2-yl, 5-CF₃-pyrid-3-yl, 2-CF₃-pyrid-4-yl, 3-F-4-CF₃-pyrid-2-yl, 4-CF₃-5-F-pyrid-2-yl, 4-CF₃-5-Cl-pyrid-2-yl, 3,5-diF-4-CF₃-pyrid-2-yl, 4-OCH₂CF₃-pyrid-2-yl, 4-OCH₂CF₃-5-F-pyrid-2-yl, 4-OCH₂CF₃-5-Cl-pyrid-2-yl, 4-OBn-5-F-pyrid-2-yl, 3-(4-F-phenyl)-pyrid-5-yl, 2-(4-F-phenyl)-pyrid-3-yl, 4-(pyrid-4-yl)-pyrid-2-yl, 4-(pyrid-3-yl)-pyrid-2-yl, 5-cyclopropyl-pyraz-2-yl, 5-cyclobutyl-pyraz-2-yl, 5-pyrrolidin-pyraz-2-yl, pyridaz-3-yl, 4-CF₃-pyridaz-3-yl, 5-CF₃-pyridaz-3-yl, 5-F-pyrimid-2-yl, 6-Cl-pyrimid-4-yl, 6-Me-pyrimid-4-yl, 6-Et-pyrimid-4-yl, 6-Pr-pyrimid-4-yl, 6-iPr-pyrimid-4-yl, 4-CF₃-pyrimid-2-yl, 6-CF₃-pyrimid-4-yl, 2-Me-6-Cl-pyrimid-4-yl, 2-Me-6-CF₃-pyrimid-4-yl, 2-OMe-6-CF₃-pyrimid-4-yl, 2-OMe-pyrimid-4-yl, 6-OMe-pyrimid-4-yl, 6-OEt-pyrimid-4-yl, 6-OPr-pyrimid-4-yl, 6-OiPr-pyrimid-4-yl, 6-OiBu-pyrimid-4-yl, 6-OBu-pyrimid-4-yl, 6-OBn-pyrimid-4-yl, 6-cyclobutylloxy-pyrimid-4-yl, 6-cyclopentyloxy-pyrimid-4-yl, 6-cyclohexyloxy-pyrimid-4-yl, 6-cyclopropyl-pyrimid-4-yl, 6-phenyl-pyrimid-4-yl, 6-(2-F-phenyl)-pyrimid-4-yl, 6-(3-F-phenyl)-pyrimid-4-yl, 6-(4-F- phenyl)-pyrimid-4-yl, 6-Cl-pyrimid-4-yl, 6-(2-Me- phenyl)-pyrimid-4-yl, 6-(3-Me- phenyl)-pyrimid-4-yl, 6-(4-Me-phenyl)-pyrimid-4-yl, 6-(3-Cl- phenyl)-pyrimid-4-yl, 6-(4-Cl- phenyl)-pyrimid-4-yl, 2-(morpholin-1-yl)-pyrimid-4-yl, 6-(azetidin-3-yl-methoxy)-pyrimid-4-yl, 6-(pyrrolidin-3-yl-methoxy)-pyrimid-4-yl, 6-(pyrrolidin-2-yl-methoxy)-pyrimid-4-yl, benzofuran-5-yl, 2-Me-benzofuran-3-yl, 2-Et-benzofuran-3-yl, benzothiophen-5-yl, benzothiophen-6-yl, 5-Me-benzothiophen-2-yl, 5-F-benzothiophen-2-yl, 3-Cl-benzothiophen-2-yl, benzothiazol-2-yl, isoquinolin-6-yl, isoquinolin-7-yl, quinolin-6-yl, quinolin-7-yl, 4,5,6,7-tetrahydro-1,3-benzothiazol-2-yl, 2,3-dihydrobenzofuran-5-yl, 7H-pyrrolo[2,3-d]pyrimidin-4-yl, pyrazolo[1,5-a]pyridine-3-yl, pyrazolo[1,5-a]pyridine-7-yl, imidazo[1,2-a]pyridin-8-yl, 5-methyl-imidazo[1,2-a]pyridin-3-yl, 6-chloro-2-methyl-imidazo[1,2-a]pyridin-3-yl, 6-bromo-2-methyl-imidazo[1,2-a]pyridin-3-yl, thieno[2,3-b]pyridin-2-yl, N-benzyl-indolin-6-yl, indolinon-4-yl, chroman-6-yl, 4,4-dimethylchroman-6-yl, 4,4-dimethyl-1,3-dioxan-2-yl, 2-Me-tetrahydrofuran-3-yl, tetrahydrofuran-3-yl, tetrahydropyran-4-yl, 4-Me-tetrahydropyran-4-yl, 2,2-diMe-tetrahydropyran-4-yl, 1-isopropyl-piperidin-4-yl, 1-ethyl-piperidin-3-yl, or 2-CF₃-piperazin-5-yl, a further example being iBu-pyrazol-4-yl, 1-CHF₂-5-Me-1,2-diazol-4-yl, 1-benzyl-5-F-1,2,4-triazol-3-yl, 2-Me-pyrid-4-yl, 2-iPr-pyrid-4-yl, 4-phenyl-pyrid-2yl, 2-cyclopropyl-pyrid-4-yl, 2-OMe-pyrid-4-yl, 2-OEt-pyrid-4-yl, 2-OiPr-pyrid-4-yl, 2-OCH₂CF₃-pyrid-4-yl, 2-cyclopropoxy-pyrid-4-yl, 2-cyclobutoxy-pyrid-4-yl, 4,5-diCl-pyrimidin-2yl, benzothiophen-3-yl, 2,2-diF-1,3-benzodioxol-5-yl, 5,6,7,8-tetrahydro-isoquinolin-5-yl, 5,6,7,8-tetrahydro-isoquinolin-8-yl, quinolin-8-yl, 1-Me-3,4-dihydro-2H-quinolin-4-yl, 2-Me-3,4-dihydro-1H-isoquinolin-6-yl, 5,6,7,8-tetrahydro-quinolin-5-yl, 5,6,7,8-tetrahydro-quinolin-8-yl, 5,6,7,8-tetrahydro-quinolin-6-yl, 2,3-dihydrobenzofuran-3-yl, pyrazolo[1,5-a]pyrimidin-6-yl, imidazo[1,5-a]pyrazinyl, 3-methyl-imidazo[1,5-a]pyrazinyl, 3-Me-[1,2,4]triazolo[4,3-a]pyridin-6-yl, imidazo[1,2-a]pyrazin-6-yl, 2-Me-isoindolin-1-on-6-yl, 2,2-dimethyl-7-CF₃-chroman-4-yl, 7-CF₃-chroman-4-yl, 7-OCF₃-chroman-4-yl, isochroman-4-yl, 6,6-dimethyl-1,3-dioxan-2-yl, tetrahydropyran-3-yl, 1-phenyl-pyrrolidin-3-yl, piperidin-3yl, 1,3-dimethyl-piperidin-4yl, 1-cyclopropyl-piperidin-4-yl, quinuclidin-2-yl, 6,7-dihydro-5H-cyclopenta[b]pyridine-7-yl, 6,7-dihydro-5H-cyclopenta[b]pyridine-5-yl, 6,7-dihydro-5H-cyclopenta[c]pyridine-7-yl, 6,7-dihydro-5H-cyclopenta[c]pyridine-5-yl, 5,6-dihydro-4H-cyclopenta[b]thiophen-4-yl, or 1,2,3,5,6,7,8,8a-octahydroindolizin), or tri-(C₁-C₄-alkyl)-silyloxy, and
- R¹, R^{2a}, R^{2b}, R^{3b}, Y¹, n4, R^{5a} and R^{5b}: are as defined above.

According to a particularly preferred embodiment of the pyrrolidine derivatives of the formula (Id), R^{3a} is phenyl, 4-F-phenyl, tetrahydrofuran-2-yl or tetrahydropyran-2-yl, or additionally 4-F-pyridyl or piperidinyl.

According to a further particularly preferred embodiment of the pyrrolidine derivatives of the formula (Id), R¹³ is a group of the formula (Id1): 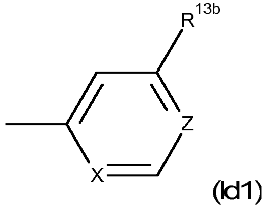 wherein
- X: is >CH- or >N-;
- Z: is >C-R^{13c} or >N-;
- R^{13b}: is halogen (e.g. fluoro or chloro), C₁-C₄-alkyl (e.g. methyl, ethyl, n-propyl, iso-propyl, tert-butyl), halogenated C₁-C₄-alkyl (e.g. CHF₂, CF₃), hydroxy-(halogenated C₁-C₄-alkyl) (e.g. 1-OH-1-CF₃-Et), C₆-C₁₂-aryl-C₁-C₄-alkyl (e.g. benzyl), C₃-C₆-cycloalkyl (e.g. cyclopropyl), CN, C₆-C₁₂-aryl optionally substituted with halogen or C₁-C₄-alkyl (e.g. phenyl, 2-F-phenyl, 4-F-phenyl, 3-F-phenyl, 3-Cl-phenyl, 4-Cl-phenyl, 2-Me-phenyl, 3-Me-phenyl, 4-Me-phenyl), C₁-C₄-alkoxy (e.g. methoxy, ethoxy, n-propoxy, iso-propoxy, a further example being n-butoxy or iso-butoxy), halogenated C₁-C₄-alkoxy (e.g. -OCHF₂, -OCH₂CF₃, -OCF₃), C₃-C₆-cycloalkoxy (e.g. cyclobutoxy, cyclopentyloxy, cyclohexyloxy, a further example being cyclopropoxy), C₆-C₁₂ aryl-C₁-C₄-alkoxy (e.g. benzyloxy), C₃-C₁₂-heterocyclyl-C₁-C₄-alkoxy (e.g. pyrrolid-3-yl-methoxy, pyrrolid-2-yl-methoxy, azetid-3-yl-methoxy), C₆-C₁₂-aryloxy (e.g. phenoxy), C₁-C₄-alkyl-sulfonyl (e.g. methylsulfonyl), C₁-C₄-alkyl-carbonylamino (e.g. 2-methylcarbonylamino) or C₃-C₁₂-heterocyclyl (e.g. piperidin-4-yl, pyrid-2-yl, pyrid-3-yl, pyrid-4-yl, pyrimid-5-yl); and
- R^{13c}: is hydrogen or halogen (e.g. fluoro, chloro).

Additional pyrrolidine derivatives of the formula (Id) result if R¹³ is a group of formula (Id1) wherein

R^{13b} is C₃-C₆-cycloalkyl-C₁-C₄-alkoxy (e.g. cyclopropyl-methoxy) and X, Z and R^{13c} are as defined above.

Especially preferred among the pyrrolidine derivatives of the formula (Id) wherein R¹³ is a group of the formula (Id1) are those wherein X is >CH- or >N-, and Z is >C-R^{13c}. According this embodiment it is particularly preferred if X is >CH-, Z is >C-R^{13c} and R^{13c} is hydrogen or fluoro, especially fluoro, or X is >N-, Z is >C-R^{13c} and R^{13c} is hydrogen or fluoro, especially fluoro.

In the pyrrolidine derivatives of the formula (Id) wherein R¹³ is a group of the formula (Id1), R^{8a}, n4, R^{9a} and R^{9b} are as defined herein. Preferably, R^{8a} is hydrogen, n4 is, in particular, 0 or 1, with n4 = 0 being particularly preferred. R^{9a} and R^{9b} are preferably both hydrogen if n4 is 1.

According to a further embodiment, R⁴ is -NR^{8b}COR¹⁴. Thus, the present invention relates to the pyrrolidine derivatives of the formula (Ie): 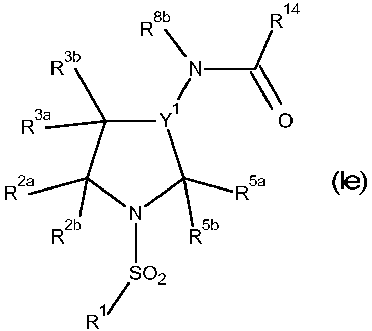 wherein R¹, R^{2a}, R^{2b}, R^{3a}, R^{3b}, Y¹, R^{5a} and R^{5b} are as defined herein, and
- R^{8b}: is hydrogen, C₁-C₆-alkyl (e.g. methyl, ethyl, iso-propyl, or n-butyl), or C₁-C₆₋alkylcarbonyl (e.g. methylcarbonyl), or
- R⁶, R^{8b}: together are optionally substituted C₁-C₅-alkylene, wherein one or more -CH₂- of C₁-C₅-alkylene may be independently replaced by a an oxygen atom or C=O; and
- R¹⁴: is C₁-C₈-alkyl (e.g. pentyl, n-butyl, or hexyl), halogenated C₁-C₆-alkyl (e.g. 1,1-diF-butyl, 3,3-diF-butyl, 4,4,4-triF-butyl, 1,1-diF-pentyl, or 4,4-diF-pentyl), (optionally substituted C₃-C₁₂-cycloalkyl)-C₁-C₄-alkyl (e.g. cyclopropyl-methyl), (optionally substituted C₆-C₁₂-aryl)-C₁-C₄-alkyl (e.g. benzyl, a further example being 4-F-benzyl, 3-F-benzyl, 3-CF₃-benzyl, 4-F-3-CF₃-benzyl, or 3-OCF₃-benzyl), hydroxy-C₁-C₆-alkyl (e.g. hydroxyl-methyl or 1-hydroxy-pentyl), C₁-C₆-alkoxy-C₁-C₆-alkyl (e.g. methoxy-methyl, ethoxy-methyl, or ethoxy-ethyl), (optionally substituted C₆-C₁₂-aryloxy)-C₁-C₄-alkyl (e.g. phenoxy-methyl or (4-F-phenoxy)-methyl), C₁-C₆-alkylcarbonyl-C₁-C₄-alkyl (e.g. 2-methylcarbonyl-ethyl or 3-methylcarbonyl-propyl), C₁-C₆-alkoxycarbonyl-C₁-C₄-alkyl (e.g. 3-methoxycarbonyl-propyl), C₁-C₆-alkylaminocarbonyl-C₁-C₄-alkyl (e.g. 3-methylaminocarbonyl-propyl), optionally substituted (C₃-C₁₂-heterocyclyl)-C₁-C₄-alkyl (e.g. 2-pyridyl-methyl), optionally substituted C₃-C₁₂-cycloalkyl (e.g.1-CF₃-cyclopropyl or 4-CF₃-cyclohexyl, a further example being 1-phenyl-cyclopropyl, 1-(4-F-phenyl)cyclopropyl, 1-(3-F-phenyl)cyclopropyl, 1-(3-Cl-phenyl)cyclopropyl, 1-(3-CF₃-phenyl)cyclopropyl, 3-hydroxymethyl-bycyclo[1.1.1]pentyl, 3-methoxymethyl-bycyclo [1.1.1]pentyl, 3-ethoxymethyl-bycyclo[1.1.1]pentyl, or 3-methoxycarbonyl-bycyclo[1.1.1]pentyl), optionally substituted C₆-C₁₂-aryl (e.g. phenyl, 2-F-phenyl, 3-F-phenyl, 4-F-phenyl, 2-Cl-phenyl, 3-Cl-phenyl, 4-Cl-phenyl, 2,3-diCl-phenyl, 2,4-diCl-phenyl, 3,5-diCl-phenyl, 2-Cl-4-F-phenyl, 2-Me-phenyl, 3-Me-phenyl, 4-Me-phenyl, 2-CF₃-phenyl, 3-CF₃-phenyl, 4-CF₃-phenyl, 3-Cl-4-CF₃-phenyl, 2-Cl-3-CF₃-phenyl, 2-Cl-4-CF₃-phenyl, 2-OMe-phenyl, 3-OMe-phenyl, 2-CN-phenyl, or 3-CN-phenyl, 4-CN-phenyl), or optionally substituted C₃-C₁₂-heterocyclyl (e.g. 3-Cl-pyridazin-6-yl, 2-Cl-pyrazin-5-yl, or 2-CF₃-pyrazin-5-yl).

Additional embodiments of pyrrolidine derivatives of formula (Ie) result if
R^{3a} and R^{8b} together are optionally substituted C₁-C₅-alkylene (e.g. 1,2-ethylene or 1,3-propylene); and
R¹, R^{2a}, R^{2b}, R^{3b}, Y¹, R⁶, R¹⁴, R^{5a} and R^{5b} are as defined above.

In particular, R^{8b} is hydrogen. In particular, R^{8b} and R^{3a} together may be optionally substituted C₁-C₅-alkylene, preferably unsubstituted C₁-C₅-alkylene (e.g. 1,2-ethylene or 1,3-propylene).

In particular, R¹⁴ is C₁-C₈-alkyl (e.g. pentyl, n-butyl, or hexyl), halogenated C₁-C₆-alkyl (e.g. 1,1-diF-butyl, 3,3-diF-butyl, 4,4,4-triF-butyl, 1,1-diF-pentyl, or 4,4-diF-pentyl), (optionally substituted C₃-C₁₂-cycloalkyl)-C₁-C₄-alkyl (e.g. cyclopropyl-methyl), hydroxy-C₁-C₆-alkyl (e.g. hydroxyl-methyl or 1-hydroxy-pentyl), (optionally substituted C₆-C₁₂-aryloxy)-C₁-C₄-alkyl (e.g. phenoxy-methyl or (4-F-phenoxy)-methyl), C₁-C₆-alkylcarbonyl-C₁-C₄-alkyl (e.g. 2-methylcarbonyl-ethyl or 3-methylcarbonyl-propyl), C₁-C₆-alkoxycarbonyl-C₁-C₄-alkyl (e.g. 3-methoxycarbonyl-propyl), optionally substituted C₃-C₁₂-cycloalkyl (e.g.1-CF₃-cyclopropyl or 4-CF₃-cyclohexyl, a further example being 1-phenyl-cyclopropyl, 1-(4-F-phenyl)cyclopropyl, 1-(3-F-phenyl)cyclopropyl, 1-(3-Cl-phenyl)cyclopropyl, 1-(3-CF₃-phenyl)cyclopropyl, 3-hydroxymethyl-bycyclo[1.1.1]pentyl, 3-methoxymethyl-bycyclo[1.1.1]pentyl, 3-ethoxymethyl-bycyclo [1.1.1]pentyl, or 3-methoxycarbonyl-bycyclo[1.1.1]pentyl), optionally substituted C₆-C₁₂-aryl (e.g. phenyl, 2-F-phenyl, 3-F-phenyl, 4-F-phenyl, 2-Cl-phenyl, 3-Cl-phenyl, 4-Cl-phenyl, 2,3-diCl-phenyl, 2,4-diCl-phenyl, 3,5-diCl-phenyl, 2-Cl-4-F-phenyl, 2-Me-phenyl, 3-Me-phenyl, 4-Me-phenyl, 2-CF₃-phenyl, 3-CF₃-phenyl, 4-CF₃-phenyl, 3-Cl-4-CF₃-phenyl, 2-Cl-3-CF₃-phenyl, 2-Cl-4-CF₃-phenyl, 2-OMe-phenyl, 3-OMe-phenyl, 2-CN-phenyl, or 3-CN-phenyl, 4-CN-phenyl), or optionally substituted C₃-C₁₂-heterocyclyl (e.g. 3-Cl-pyridazin-6-yl, 2-Cl-pyrazin-5-yl, or 2-CF₃-pyrazin-5-yl). In particular, R¹⁴ may be (optionally substituted C₆-C₁₂-aryl)-C₁-C₄-alkyl (e.g. benzyl, a further example being 4-F-benzyl, 3-F-benzyl, 3-CF₃-benzyl, 4-F-3-CF₃-benzyl, or 3-OCF₃-benzyl).

R^{2a}, R^{2b} - in pyrrolidine derivatives of formula (Ie) - are hydrogen.

R^{3a} - in pyrrolidine derivatives of formula (Ie) - is C₃-C₁₂-cycloalkyl (cyclopropyl, cyclobutyl, cyclopentyl, or cyclohexyl), optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl), C₃-C₁₂-cycloalkyl-C₁-C₄-alkoxy (cyclopropylmethoxy or cyclohexylmethoxy), C₂-C₆-alkenyloxy (e.g. allyloxy or 2-methylprop-2-en-1-oxy), C₆-C₁₂-aryl-C₁-C₄-alkoxy (e.g. benzyloxy), optionally substituted C₆-C₁₂-aryloxy (e.g. phenyloxy or 4-F-phenyloxy), or C₃-C₁₂-heterocyclyl (e.g.pyrid-2-yl). Preferably R^{3a} is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl) or C₃-C₂-heterocyclyl (e.g.pyrid-2-yl).

R^{3b} - in pyrrolidine derivatives of formula (Ie) - is hydrogen or hydroxy. Preferably, R^{3b} is hydrogen.

Y¹ - in pyrrolidine derivatives of formula (Ie) - is >CR⁶.

R⁶ - in pyrrolidine derivatives of formula (Ie) - is hydrogen. Additionally, R⁶ and R^{3a} or R^{3b} together may be optionally substituted C₁-C₅-alkylene and preferably unsubstituted C₁-C₅-alkylene (e.g. 1,3-propylene or 1,4-butylene).

R^{5a}, R^{5b} - in pyrrolidine derivatives of formula (Ie) - are hydrogen.

Particular embodiments of pyrrolidine derivatives of formula (Ie) result if:
- R¹: is an optionally substituted 5-membered heterocyclic ring as defined herein (e.g. 1,2-diazol-4-yl, 1-methyl-1,2-diazol-4-yl, 1-methyl-1,2-diazol-3-yl, 1-methyl-1,3-diazol-4-yl, 1-methyl-1,2-diazol-5-yl, 1,5-dimethyl-1,2-diazol-4-yl, 1,3-dimethyl-1,2-diazol-4-yl, 1,5-dimethyl-1,2-diazol-4-yl, 1,3-dimethyl-1,2-diazol-5-yl, 1,3-dimethyl-5-Cl-1,2-diazol-4-yl, 1-isopropyl-3-methyl-1,2-diazol-4-yl, 1-Me-3-CF₃-1,2-diazol-4-yl, 1-cyclopentyl-3-Me-1,2-diazol-4-yl, 1,3,5-trimethyl-1,2-diazol-4-yl, 1-CHF₂-3,5-dimethyl-1,2-diazol-4-yl, 1,2-dimethyl-1,3-diazol-4-yl, 1,2-dimethyl-1,3-diazol-5-yl, 1-methyl-5-Cl-1,3-diazol-4-yl, 1-Me-1,2,3-triazol-4-yl, a further example being 1-ethyl-1,3-diazol-4-yl or 1-methyl-1,2,4-triazol-3-yl);
- R^{2a}, R^{2b}: are hydrogen;
- R^{3a}: is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl) or C₃-C₁₂-heterocyclyl (e.g.pyrid-2-yl);
- R^{3b}: is hydrogen;
- Y¹: is >CR⁶;
- R⁶: is hydrogen, or
- R⁶ and R^{3a} or R^{3b}: together are C₁-C₅-alkylene (e.g. 1,3-propylene or 1,4-butylene);
- R^{8b}: is hydrogen, or
- R^{3a} and R^{8b}: together are C₁-C₅-alkylene (e.g. 1,2-ethylene or 1,3-propylene);
- R¹⁴: is C₁-C₈-alkyl (e.g. pentyl, n-butyl, or hexyl), halogenated C₁-C₆-alkyl (e.g. 1,1-diF-butyl, 3,3-diF-butyl, 4,4,4-triF-butyl, 1,1-diF-pentyl, or 4,4-diF-pentyl), (optionally substituted C₃-C₁₂-cycloalkyl)-C₁-C₄-alkyl (e.g. cyclopropyl-methyl), (optionally substituted C₆-C₁₂-aryl)-C₁-C₄-alkyl (e.g. benzyl, a further example being 4-F-benzyl, 3-F-benzyl, 3-CF₃-benzyl, 4-F-3-CF₃-benzyl, or 3-OCF₃-benzyl), hydroxy-C₁-C₆-alkyl (e.g. hydroxyl-methyl or 1-hydroxy-pentyl), (optionally substituted C₆-C₁₂-aryloxy)-C₁-C₄-alkyl (e.g. phenoxy-methyl or (4-F-phenoxy)-methyl), C₁-C₆-alkylcarbonyl-C₁-C₄-alkyl (e.g. 2-methylcarbonyl-ethyl or 3-methylcarbonyl-propyl), C₁-C₆-alkoxycarbonyl-C₁-C₄-alkyl (e.g. 3-methoxycarbonyl-propyl), optionally substituted C₃-C₁₂-cycloalkyl (e.g.1-CF₃-cyclopropyl or 4-CF₃-cyclohexyl, a further example being 1-phenyl-cyclopropyl, 1-(4-F-phenyl)cyclopropyl, 1-(3-F-phenyl)cyclopropyl, 1-(3-Cl-phenyl)cyclopropyl, 1-(3-CF₃-phenyl)cyclopropyl, 3-hydroxymethyl-bycyclo [1.1.1]pentyl, 3-methoxymethyl-bycyclo [1.1.1]pentyl, 3-ethoxymethyl-bycyclo[1.1.1]pentyl, or 3-methoxycarbonyl-bycyclo[1.1.1]pentyl), optionally substituted C₆-C₁₂-aryl (e.g. phenyl, 2-F-phenyl, 3-F-phenyl, 4-F-phenyl, 2-Cl-phenyl, 3-Cl-phenyl, 4-Cl-phenyl, 2,3-diCl-phenyl, 2,4-diCl-phenyl, 3,5-diCl-phenyl, 2-Cl-4-F-phenyl, 2-Me-phenyl, 3-Me-phenyl, 4-Me-phenyl, 2-CF₃-phenyl, 3-CF₃-phenyl, 4-CF₃-phenyl, 3-Cl-4-CF₃-phenyl, 2-Cl-3-CF₃-phenyl, 2-Cl-4-CF₃-phenyl, 2-OMe-phenyl, 3-OMe-phenyl, 2-CN-phenyl, or 3-CN-phenyl, 4-CN-phenyl), or optionally substituted C₃-C₁₂-heterocyclyl (e.g. 3-Cl-pyridazin-6-yl, 2-Cl-pyrazin-5-yl, or 2-CF₃-pyrazin-5-yl); and
- R^{5a}, R^{5b}: are hydrogen.

Further particular embodiments of pyrrolidine derivatives of formula (Ie) result if:
- R⁶: is hydrogen;
- R^{8b}: is hydrogen;
- R¹⁴: is C₁-C₈-alkyl (e.g. pentyl, n-butyl, orhexyl), halogenated C₁-C₆-alkyl (e.g. 1,1-diF-butyl, 3,3-diF-butyl, 4,4,4-triF-butyl, 1,1-diF-pentyl, or 4,4-diF-pentyl), (optionally substituted C₃-C₁₂-cycloalkyl)-C₁-C₄-alkyl (e.g. cyclopropyl-methyl), hydroxy-C₁-C₆-alkyl (e.g. hydroxyl-methyl or 1-hydroxy-pentyl), (optionally substituted C₆-C₁₂-aryloxy)-C₁-C₄-alkyl (e.g. phenoxy-methyl or (4-F-phenoxy)-methyl), C₁-C₆-alkylcarbonyl-C₁-C₄-alkyl (e.g. 2-methylcarbonyl-ethyl or 3-methylcarbonyl-propyl), C₁-C₆-alkoxycarbonyl-C₁-C₄-alkyl (e.g. 3-methoxycarbonyl-propyl), optionally substituted C₃-C₂-cycloalkyl (e.g.1-CF₃-cyclopropyl or 4-CF₃-cyclohexyl, a further example being 1-phenyl-cyclopropyl, 1-(4-F-phenyl)cyclopropyl, 1-(3-F-phenyl)cyclopropyl, 1-(3-Cl-phenyl)cyclopropyl, 1-(3-CF₃-phenyl)cyclopropyl, 3 -hydroxymethyl-bycyclo [1.1.1]pentyl, 3-methoxymethyl-bycyclo[1.1.1]pentyl, 3-ethoxymethyl-bycyclo[1.1.1]pentyl, or 3-methoxycarbonyl-bycyclo[1.1.1]pentyl), optionally substituted C₆-C₁₂-aryl (e.g. phenyl, 2-F-phenyl, 3-F-phenyl, 4-F-phenyl, 2-Cl-phenyl, 3-Cl-phenyl, 4-Cl-phenyl, 2,3-diCl-phenyl, 2,4-diCl-phenyl, 3,5-diCl-phenyl, 2-Cl-4-F-phenyl, 2-Me-phenyl, 3-Me-phenyl, 4-Me-phenyl, 2-CF₃-phenyl, 3-CF₃-phenyl, 4-CF₃-phenyl, 3-Cl-4-CF₃-phenyl, 2-Cl-3-CF₃-phenyl, 2-Cl-4-CF₃-phenyl, 2-OMe-phenyl, 3-OMe-phenyl, 2-CN-phenyl, or 3-CN-phenyl, 4-CN-phenyl), or optionally substituted C₃-C₁₂-heterocyclyl (e.g. 3-Cl-pyridazin-6-yl, 2-Cl-pyrazin-5-yl, or 2-CF₃-pyrazin-5-yl); and
- R¹, R^{2a}, R^{2b}, R^{3a}, R^{3b}, Y¹, R^{5a} and R^{5b}: are as defined above.

Preferably, R¹ - in the pyrrolidine derivatives of formula (Ie) - is an optionally substituted 5-membered heterocyclic ring containing 2 N which, in particular, is optionally substituted 1,3-diazolyl.

According to a further preferred embodiment, R¹ - in the pyrrolidine derivatives of formula (Ie) - is an optionally substituted 5-membered heterocyclic ring containing 3 N which, in particular, is optionally substituted 1,2,3-triazolyl.

In connection with R¹ and the pyrrolidine derivatives of formula (Ie), substituted 5-membered heterocyclic ring in particular includes pyrazolyl, imidazolyl, and triazolyl, which are substituted with 1, 2 or 3 substituents independently selected from the group consisting of halogen, C₁-C₆-alkyl, halogenated C₁-C₆-alkyl, C₃-C₆-cycloalkyl, C₁-C₄-alkoxy-carbonyl and C₁-C₆-alkylcarbonylamino. Preferably, the substituent(s) on the heterocyclic ring are independently selected from the group consisting of halogen and C₁-C₆-alkyl. In particular, the substituent(s) on the heterocyclic ring are independently selected from the group consisting of C₁-C₆-alkyl, especially C₁-C₄-alkyl (e.g. methyl). According to a particular embodiment, 1,3-diazolyl is substituted with halogen or C₁-C₆-alkyl as described herein. According to a specific embodiment, R¹ is 1-methyl-1,3-diazol-4-yl.

In connection with R^{3a} and the pyrrolidine derivatives of formula (Ie), substituted C₆-C₁₂-aryl in particular includes C₆-C₁₂-aryl, such as phenyl or naphthyl, which is substituted with 1, 2, or 3 substituents independently selected from the group consisting of halogen, C₁-C₄-alkyl and C₁-C₄-alkoxy.

According to a preferred embodiment, R^{3a} - in the pyrrolidine derivatives of formula (Ie) - is C₆-C₁₂-aryl, in particular phenyl, optionally substituted with 1, 2, or 3 substituents independently selected from the group consisting of halogen and C₁-C₄-alkoxy (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl) or C₃-C₁₂-heterocyclyl (e.g. 2-pyridyl).

In connection with R¹⁴, substituted C₆-C₁₂-aryl-C₁-C₄-alkyl in particular includes C₆-C₁₂-aryl-C₁-C₄-alkyl, such as benzyl, which is substituted with 1, 2, or 3 substituents independently selected from the group consisting of halogen, halogenated C₁-C₄-alkyl and halogenated C₁-C₄-alkoxy.

In connection with R¹⁴, substituted C₆-C₁₂-aryloxy-C₁-C₄-alkyl in particular includes C₆-C₁₂-aryloxy-C₁-C₄-alkyl, such as benzyloxy, which is substituted with 1, 2, or 3 substituents independently selected from the group consisting of halogen.

In connection with R¹⁴, substituted C₃-C₁₂-cycloalkyl in particular includes C₃-C₁₂-cycloalkyl, such as cyclopropyl or cyclohexyl, which is substituted with 1, 2, or 3 substituents independently selected from the group consisting of halogenated C₁-C₄-alkyl.

In connection with R¹⁴, substituted C₆-C₁₂-aryl in particular includes C₆-C₁₂-aryl, such as phenyl, which is substituted with 1, 2, or 3 substituents independently selected from the group consisting of halogen, C₁-C₄-alkyl, halogenated C₁-C₄-alkyl, CN and C₁-C₄-alkoxy. Preferably the substituents on C₆-C₁₂-aryl are independently selected from the group consisting of C₁-C₄-alkyl and CN.

In connection with R¹⁴, substituted C₃-C₁₂-heterocyclyl in particular includes C₃-C₁₂-heterocyclyl, such as pyridazyl or pyrazyl, which is substituted with 1, 2, or 3 substituents independently selected from the group consisting of halogen and halogenated C₁-C₄-alkyl.

According to a preferred embodiment, R¹⁴ is C₁-C₈-alkyl (e.g. pentyl, n-butyl, or hexyl), halogenated C₁-C₆-alkyl (e.g. 1,1-diF-butyl, 3,3-diF-butyl, 4,4,4-triF-butyl, 1,1-diF-pentyl, or 4,4-diF-pentyl), (optionally substituted C₃-C₁₂-cycloalkyl)-C₁-C₄-alkyl (e.g. cyclopropyl-methyl), hydroxy-C₁-C₆-alkyl (e.g. hydroxyl-methyl or 1-hydroxy-pentyl), (halogenated C₆-C₁₂-aryloxy)-C₁-C₄-alkyl (e.g. 4-F-phenoxy)-methyl), C₁-C₆-alkylcarbonyl-C₁-C₄-alkyl (e.g. 2-methylcarbonyl-ethyl or 3-methylcarbonyl-propyl), C₁-C₆-alkoxycarbonyl-C₁-C₄-alkyl (e.g. 3-methoxycarbonyl-propyl), C₃-C₁₂-cycloalkyl optionally substituted with 1, 2, or 3 substituents independently selected from the group consisting of halogenated C₁-C₄-alkyl (e.g.1-CF₃-cyclopropyl or 4-CF₃-cyclohexyl), C₆-C₁₂-aryl optionally substituted with 1, 2, or 3 substituents independently selected from the group consisting of halogen, C₁-C₄-alkyl, halogenated C₁-C₄-alkyl, CN and C₁-C₄-alkoxy (e.g. phenyl, 2-F-phenyl, 3-F-phenyl, 4-F-phenyl, 2-Cl-phenyl, 3-Cl-phenyl, 4-Cl-phenyl, 2,3-diCl-phenyl, 2,4-diCl-phenyl, 3,5-diCl-phenyl, 2-Cl-4-F-phenyl, 2-Me-phenyl, 3-Me-phenyl, 4-Me-phenyl, 2-CF₃-phenyl, 3-CF₃-phenyl, 4-CF₃-phenyl, 3-Cl-4-CF₃-phenyl, 2-Cl-3-CF₃-phenyl, 2-Cl-4-CF₃-phenyl, 2-OMe-phenyl, 3-OMe-phenyl, 2-CN-phenyl, 3-CN-phenyl, or 4-CN-phenyl), or C₃-C₁₂-heterocyclyl optionally substituted with 1, 2, or 3 substituents independently selected from the group consisting of halogen and halogenated C₁-C₄-alkyl (e.g. 3-Cl-pyridazin-6-yl, 2-Cl-pyrazin-5-yl, or 2-CF₃-pyrazin-5-yl). It is further preferred if R¹⁴ is (optionally substituted C₆-C₁₂-aryl)-C₁-C₄-alkyl (e.g. benzyl, a further example being 4-F-benzyl, 3-F-benzyl, 3-CF₃-benzyl, 4-F-3-CF₃-benzyl, or 3-OCF₃-benzyl).

Further preferred embodiments of the pyrrolidine derivatives of formula (Ie) result if:
- R¹: is 1,3-diazolyl optionally substituted with halogen or C₁-C₄-alkyl (e.g 1-methyl-1,3-diazol-4-yl);
- R^{2a}, R^{2b}: are hydrogen;
- R^{3a}: is C₆-C₁₂-aryl optionally substituted with 1, 2, or 3 substituents independently selected from the group consisting of halogen and C₁-C₄-alkoxy (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl) or C₃-C₁₂-heterocyclyl (e.g. 2-pyridyl)yl;
- R^{3b}: is hydrogen;
- Y¹: is >CR⁶;
- R⁶: is hydrogen, or
- R⁶ and R^{3a} or R^{3b}: together are C₁-C₅-alkylene (e.g. 1,4-butylene);
- R^{8b}: is hydrogen, or
- R^{3a} and R^{8b}: together are C₁-C₅-alkylene (e.g. 1,2-ethylene or 1,3-propylene);
- R¹⁴: is C₁-C₈-alkyl (e.g. pentyl or n-butyl), halogenated C₁-C₆-alkyl (e.g. 1,1-diF-butyl, 3,3-diF-butyl, 4,4,4-triF-butyl, 1,1-diF-pentyl, or 4,4-diF-pentyl), (optionally substituted C₃-C₁₂-cycloalkyl)-C₁-C₄-alkyl (e.g. cyclopropyl-methyl), (optionally substituted C₆-C₁₂-aryl)-C₁-C₄-alkyl (e.g. benzyl, a further example being 4-F-benzyl, 3-F-benzyl, 3-CF₃-benzyl, 4-F-3-CF₃-benzyl, or 3-OCF₃-benzyl), hydroxy-C₁-C₆-alkyl (e.g. hydroxyl-methyl or 1-hydroxy-pentyl), (halogenated C₆-C₁₂-aryloxy)-C₁-C₄-alkyl (e.g. 4-F-phenoxy)-methyl), C₁-C₆-alkylcarbonyl-C₁-C₄-alkyl (e.g. 2-methylcarbonyl-ethyl or 3-methylcarbonyl-propyl), C₁-C₆-alkoxycarbonyl-C₁-C₄-alkyl (e.g. 3-methoxycarbonyl-propyl), C₃-C₁₂-cycloalkyl optionally substituted with 1, 2, or 3 substituents independently selected from the group consisting of halogenated C₁-C₄-alkyl (e.g.1-CF₃-cyclopropyl or 4-CF₃-cyclohexyl), C₆-C₁₂-aryl optionally substituted with 1, 2, or 3 substituents independently selected from the group consisting of halogen, C₁-C₄-alkyl, halogenated C₁-C₄-alkyl, CN and C₁-C₄-alkoxy (e.g. phenyl, 2-F-phenyl, 3-F-phenyl, 4-F-phenyl, 2-Cl-phenyl, 3-Cl-phenyl, 4-Cl-phenyl, 2,3-diCl-phenyl, 2,4-diCl-phenyl, 3,5-diCl-phenyl, 2-Cl-4-F-phenyl, 2-Me-phenyl, 3-Me-phenyl, 4-Me-phenyl, 2-CF₃-phenyl, 3-CF₃-phenyl, 4-CF₃-phenyl, 3-Cl-4-CF₃-phenyl, 2-Cl-3-CF₃-phenyl, 2-Cl-4-CF₃-phenyl, 2-OMe-phenyl, 3-OMe-phenyl, 2-CN-phenyl, or 3-CN-phenyl, 4-CN-phenyl), or C₃-C₁₂-heterocyclyl optionally substituted with 1, 2, or 3 substituents independently selected from the group consisting of halogen and halogenated C₁-C₄-alkyl(e.g. 3-Cl-pyridazin-6-yl, 2-Cl-pyrazin-5-yl, or 2-CF₃-pyrazin-5-yl); and
- R^{5a}, R^{5b}: are hydrogen.

Further particular embodiments of pyrrolidine derivatives of formula (Ie) result if:
- R⁶: is hydrogen;
- R^{8b}: is hydrogen;
- R¹⁴: is C₁-C₈-alkyl (e.g. pentyl, n-butyl, or hexyl), halogenated C₁-C₆-alkyl (e.g. 1,1-diF-butyl, 3,3-diF-butyl, 4,4,4-triF-butyl, 1,1-diF-pentyl, or 4,4-diF-pentyl), (optionally substituted C₃-C₁₂-cycloalkyl)-C₁-C₄-alkyl (e.g. cyclopropyl-methyl), hydroxy-C₁-C₆-alkyl (e.g. hydroxyl-methyl or 1-hydroxy-pentyl), (optionally substituted C₆-C₁₂-aryloxy)-C₁-C₄-alkyl (e.g. phenoxy-methyl or (4-F-phenoxy)-methyl), C₁-C₆-alkylcarbonyl-C₁-C₄-alkyl (e.g. 2-methylcarbonyl-ethyl or 3-methylcarbonyl-propyl), C₁-C₆-alkoxycarbonyl-C₁-C₄-alkyl (e.g. 3-methoxycarbonyl-propyl), optionally substituted C₃-C₁₂-cycloalkyl (e.g.1-CF₃-cyclopropyl or 4-CF₃-cyclohexyl, a further example being 1-phenyl-cyclopropyl, 1-(4-F-phenyl)cyclopropyl, 1-(3-F-phenyl)cyclopropyl, 1-(3-Cl-phenyl)cyclopropyl, 1-(3-CF₃-phenyl)cyclopropyl, 3-hydroxymethyl-bycyclo[1.1.1]pentyl, 3-methoxymethylbycyclo[1.1.1]pentyl, 3-ethoxymethyl-bycyclo[1.1.1]pentyl, or 3-methoxycarbonylbycyclo[1.1.1]pentyl), optionally substituted C₆-C₁₂-aryl (e.g. phenyl, 2-F-phenyl, 3-F-phenyl, 4-F-phenyl, 2-Cl-phenyl, 3-Cl-phenyl, 4-Cl-phenyl, 2,3-diCl-phenyl, 2,4-diCl-phenyl, 3,5-diCl-phenyl, 2-Cl-4-F-phenyl, 2-Me-phenyl, 3-Me-phenyl, 4-Me-phenyl, 2-CF₃-phenyl, 3-CF₃-phenyl, 4-CF₃-phenyl, 3-Cl-4-CF₃-phenyl, 2-Cl-3-CF₃-phenyl, 2-Cl-4-CF₃-phenyl, 2-OMe-phenyl, 3-OMe-phenyl, 2-CN-phenyl, or 3-CN-phenyl, 4-CN-phenyl), or optionally substituted C₃-C₁₂-heterocyclyl (e.g. 3-Cl-pyridazin-6-yl, 2-Cl-pyrazin-5-yl, or 2-CF₃-pyrazin-5-yl); and
- R¹, R^{2a}, R^{2b}, R^{3a}, R^{3b}, Y¹, R^{5a} and R^{5b}: are as defined above.

According to a particularly preferred embodiment, R^{3a} is phenyl, or halogenated phenyl (e.g. 2-Cl-phenyl, 3-Cl-phenyl, 4-Cl-phenyl, 2-F-phenyl, 3-F-phenyl, 4-F-phenyl), 4-OMe-phenyl, or pyrid-2-yl.

According to a further embodiment, R⁴ is -NR^{8c}COOR¹⁵. Thus, the present invention relates to the pyrrolidine derivatives of the formula (If): 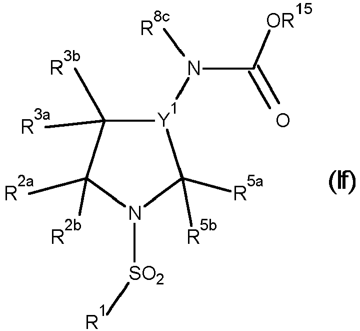 wherein R¹, R^{2a}, R^{2b}, R^{3a}, R^{3b}, Y¹, R^{5a} and R^{5b} are as defined herein, and
- R^{8c}: is hydrogen, C₁-C₆-alkyl (e.g. methyl, ethyl, iso-propyl, or n-butyl), or C₁-C₆₋alkylcarbonyl (e.g. emthylcarbonyl), or
- R⁶, R^{8c}: together are optionally substituted C₁-C₅-alkylene, wherein one or more -CH₂- of C₁-C₅-alkylene may be independently replaced by a an oxygen atom or C=O. Preferably R^{8c} is hydrogen; and
- R¹⁵: is C₁-C₈-alkyl (e.g. ethyl, n-propyl, n-butyl, iso-butyl, or tert-butyl), optionally substituted C₆-C₁₂-aryl (e.g. phenyl, 4-F-phenyl, 2-Cl-4-F-phenyl or 2-Me-phenyl), or optionally substituted C₃-C₁₂-heterocyclyl (e.g. 6-pyridazyl or 5-pyrazyl).

In particular, R¹⁵ is C₁-C₈-alkyl (e.g. ethyl, n-propyl, n-butyl, iso-butyl, or tert-butyl), or optionally substituted C₆-C₁₂-aryl (e.g. phenyl, 4-F-phenyl, 2-Cl-4-F-phenyl or 2-Me-phenyl). Preferably, R¹⁵ is C₁-C₆-alkyl (e.g. ethyl, n-propyl, n-butyl, iso-butyl, or tert-butyl) or C₆-C₁₂-aryl (e.g. phenyl).

R^{2a}, R^{2b} - in the pyrrolidine derivatives of formula (If) - are, in particular, hydrogen.

R^{3a} - in the pyrrolidine derivatives of formula (If) - is, in particular, C₃-C₁₂-cycloalkyl (e.g. cyclopropyl, cyclobutyl, cyclopentyl, or cyclohexyl), optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl), C₃-C₁₂-cycloalkyl-C₁-C₄-alkoxy (e.g. cyclopropylmethoxy or cyclohexylmethoxy), C₂-C₆-alkenyloxy (e.g. allyloxy or 2-methylprop-2-en-1-oxy), C₆-C₁₂-aryl-C₁-C₄-alkoxy (e.g. benzyloxy), optionally substituted C₆-C₁₂-aryloxy (e.g. phenyloxy or 4-F-phenyloxy), or C₃-C₁₂-heterocyclyl (e.g.pyrid-2-yl, a further example being tetrahydrofuran-2-yl or tetrahydropyran-2-yl). Preferably R^{3a} is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl).

R^{3b} - in the pyrrolidine derivatives of formula (If) - is, in particular, hydrogen.

Y¹ - in the pyrrolidine derivatives of formula (If) - is, in particular, >CR⁶.

R⁶ - in the pyrrolidine derivatives of formula (If) - is, in particular, hydrogen.

R^{5a}, R^{5b} - in the pyrrolidine derivatives of formula (If) - are, in particular, hydrogen.

Particular embodiments of the pyrrolidine derivatives of formula (If) result if:
- R¹: is an optionally substituted 5-membered heterocyclic ring as defined herein (e.g. 1,2-diazol-4-yl, 1-methyl-1,2-diazol-4-yl, 1-methyl-1,2-diazol-3-yl, 1-methyl-1,3-diazol-4-yl, 1-methyl-1,2-diazol-5-yl, 1,5-dimethyl-1,2-diazol-4-yl, 1,3-dimethyl-1,2-diazol-4-yl, 1,5-dimethyl-1,2-diazol-4-yl, 1,3-dimethyl-1,2-diazol-5-yl, 1,3-dimethyl-5-Cl-1,2-diazol-4-yl, 1-isopropyl-3-methyl-1,2-diazol-4-yl, 1-Me-3-CF₃-1,2-diazol-4-yl, 1-cyclopentyl-3-Me-1,2-diazol-4-yl, 1,3,5-trimethyl-1,2-diazol-4-yl, 1-CHF₂-3,5-dimethyl-1,2-diazol-4-yl, 1,2-dimethyl-1,3-diazol-4-yl, 1,2-dimethyl-1,3-diazol-5-yl, 1-methyl-5-Cl-1,3-diazol-4-yl, 1-Me-1,2,3-triazol-4-yl, a further example being 1-ethyl-1,3-diazol-4-yl or 1-methyl-1,2,4-triazol-3-yl);
- R^{2a}, R^{2b}: are hydrogen;
- R^{3a}: is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl);
- R^{3b}: is hydrogen;
- Y¹: is >CR⁶;
- R⁶: is hydrogen;
- R^{8c}: is hydrogen;
- R¹⁵: is C₁-C₈-alkyl (e.g. ethyl, n-propyl, n-butyl, iso-butyl, or tert-bu) or C₆-C₁₂-aryl (e.g. phenyl); and
- R^{5a}, R^{5b}: are hydrogen.

Preferably, R¹ - in the pyrrolidine derivatives of formula (If) - is an optionally substituted 5-membered heterocyclic ring as defined herein containing 2 N which, in particular, is optionally substituted 1,3-diazolyl.

According to a further preferred embodiment, R¹ - in the pyrrolidine derivatives of formula (If) - is an optionally substituted 5-membered heterocyclic ring as defined herein containing 3 N which, in particular, is optionally substituted 1,2,3-triazolyl.

In connection with R¹ and the pyrrolidine derivatives of formula (If), substituted 5-membered heterocyclic ring in particular includes pyrazolyl, imidazolyl, and triazolyl, which are substituted with 1, 2 or 3 substituents independently selected from the group consisting of halogen, C₁-C₆-alkyl, halogenated C₁-C₆-alkyl, C₃-C₆-cycloalkyl, C₁-C₄-alkoxy-carbonyl and C₁-C₆-alkylcarbonylamino. Preferably, the substituent(s) on the heterocyclic ring are independently selected from the group consisting of halogen and C₁-C₆-alkyl. In particular, the substituent(s) on the heterocyclic ring are independently selected from the group consisting of C₁-C₆-alkyl, especially C₁-C₄-alkyl (e.g. methyl). According to a particular embodiment, 1,3-diazolyl is substituted with halogen or C₁-C₆-alkyl as described herein. According to a specific embodiment, R¹ is 1-methyl-1,3-diazol-4-yl.

In connection with R^{3a} and the pyrrolidine derivatives of formula (If), substituted C₆-C₁₂-aryl in particular includes C₆-C₁₂-aryl, such as phenyl or naphthyl, which is substituted with 1, 2, or 3 substituents independently selected from the group consisting of halogen, C₁-C₄-alkyl and C₁-C₄-alkoxy. Preferably, the substituent(s) on C₆-C₁₂-aryl are independently selected from the group consisting of halogen.

According to a particular embodiment, R^{3a} - in the pyrrolidine derivatives of formula (If) - is C₆-C₁₂-aryl, in particular phenyl, optionally substituted with 1, 2, or 3 substituents independently selected from the group consisting of halogen.

Further preferred embodiments of pyrrolidine derivatives of formula (If) result if:
- R¹: is 1,3-diazolyl is substituted with halogen or C₁-C₆-alkyl (e.g. 1-methyl-1,3-diazol-4-yl);
- R^{2a}, R^{2b}: are hydrogen;
- R^{3a}: is C₆-C₁₂-aryl optionally substituted with 1, 2, or 3 substituents independently selected from the group consisting of halogen (e.g. phenyl, or 4-F-phenyl);
- R^{3b}: is hydrogen;
- Y¹: is >CR⁶;
- R⁶: is hydrogen;
- R^{8c}: is hydrogen;
- R¹⁵: is C₁-C₈-alkyl (e.g. ethyl, n-propyl, n-butyl, iso-butyl, or tert-butyl) or C₆-C₁₂-aryl (e.g. phenyl); and
- R^{5a}, R^{5b}: are hydrogen;

According to a further embodiment, R⁴ is -NR^{8d}CONR^{16a}R^{16b}. Thus, the present invention relates to the pyrrolidine derivatives of the formula (Ig): 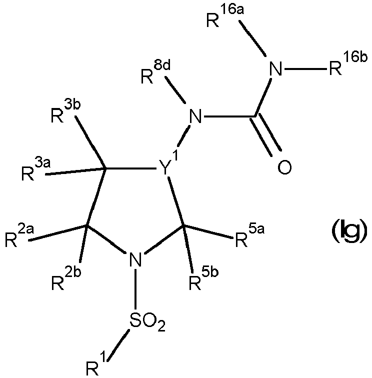 wherein R¹, R^{2a}, R^{2b}, R^{3a}, R^{3b}, Y¹, R^{5a} and R^{5b} are as defined herein, and
- R^{8d}: is hydrogen, C₁-C₆-alkyl (e.g. methyl, ethyl, iso-propyl, or n-butyl), or C₁-C₆₋alkylcarbonyl (e.g. methylcarbonyl), or
- R⁶, R^{8d}: together are optionally substituted C₁-C₅-alkylene, wherein one or more -CH₂- of C₁-C₅-alkylene may be independently replaced by a an oxygen atom or C=O;
- R^{16a}: is (optionally substituted C₆-C₁₂-aryl)-C₁-C₄-alkyl (e.g. benzyl or (2-Cl-phenyl)-methyl), optionally substituted (C₃-C₁₂-heterocyclyl)-C₁-C₄-alkyl (e.g. 2-pyridyl-methyl), optionally substituted C₆-C₁₂-aryl (e.g. phenyl, 4-F-phenyl, 3-F-phenyl, 2-F-phenyl, 4-Cl-phenyl, or 2-Cl-phenyl, a further example being 3-Cl-phenyl), or optionally substituted C₃-C₁₂-heterocyclyl (2-pyridyl, 5-pyrazyl, or 6-pyridazyl); and
- R^{16b}: is hydrogen or C₁-C₆-alkyl (e.g. methyl, ethyl, iso-propyl, or n-butyl).

In particular, R^{8d} is hydrogen.

In particular, R^{16a} is (optionally substituted C₆-C₁₂-aryl)-C₁-C₄-alkyl (e.g. benzyl or (2-Cl-phenyl)-methyl) or optionally substituted C₆-C₁₂-aryl (e.g. phenyl, 4-F-phenyl, 3-F-phenyl, 2-F-phenyl, 4-Cl-phenyl, or 2-Cl-phenyl, a further example being 3-Cl-phenyl).

In particular, R^{16b} is hydrogen.

R^{2a}, R^{2b} - in the pyrrolidine derivatives of formula (Ig) - are, in particular, hydrogen.

R^{3a} - in the pyrrolidine derivatives of formula (Ig) - is, in particular, C₃-C₁₂-cycloalkyl (e.g. cyclopropyl, cyclobutyl, cyclopentyl, or cyclohexyl), optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl), C₃-C₁₂-cycloalkyl-C₁-C₄-alkoxy (e.g. cyclopropylmethoxy or cyclohexylmethoxy), C₂-C₆-alkenyloxy (e.g. allyloxy or 2-methylprop-2-en-1-oxy), C₆-C₁₂-aryl-C₁-C₄-alkoxy (e.g. benzyloxy), optionally substituted C₆-C₁₂-aryloxy (e.g. phenyloxy or 4-F-phenyloxy), or C₃-C₁₂-heterocyclyl (e.g.pyrid-2-yl). Preferably R^{3a} is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl).

R^{3b} - in the pyrrolidine derivatives of formula (Ig) - is, in particular, hydrogen.

Y¹ - in the pyrrolidine derivatives of formula (Ig) - is, in particular, >CR⁶.

R⁶ - in the pyrrolidine derivatives of formula (Ig) - is, in particular, hydrogen.

R^{5a}, R^{5b} - in the pyrrolidine derivatives of formula (Ig) - are, in particular, hydrogen.

Particular embodiments of the pyrrolidine derivatives of formula (If) result if:
- R¹: is an optionally substituted 5-membered heterocyclic ring as defined herein (e.g. 1,2-diazol-4-yl, 1-methyl-1,2-diazol-4-yl, 1-methyl-1,2-diazol-3-yl, 1-methyl-1,3-diazol-4-yl, 1-methyl-1,2-diazol-5-yl, 1,5-dimethyl-1,2-diazol-4-yl, 1,3-dimethyl-1,2-diazol-4-yl, 1,5-dimethyl-1,2-diazol-4-yl, 1,3-dimethyl-1,2-diazol-5-yl, 1,3-dimethyl-5-Cl-1,2-diazol-4-yl, 1-isopropyl-3-methyl-1,2-diazol-4-yl, 1-Me-3-CF₃-1,2-diazol-4-yl, 1-cyclopentyl-3-Me-1,2-diazol-4-yl, 1,3,5-trimethyl-1,2-diazol-4-yl, 1-CHF₂-3,5-dimethyl-1,2-diazol-4-yl, 1,2-dimethyl-1,3-diazol-4-yl, 1,2-dimethyl-1,3-diazol-5-yl, 1-methyl-5-Cl-1,3-diazol-4-yl, 1-Me-1,2,3-triazol-4-yl, a further example being 1-ethyl-1,3-diazol-4-yl or 1-methyl-1,2,4-triazol-3-yl);
- R^{2a}, R^{2b}: are hydrogen;
- R^{3a}: is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl);
- R^{3b}: is hydrogen;
- Y¹: is >CR⁶;
- R⁶: is hydrogen;
- R^{8d}: is hydrogen;
- R^{16a}: is (optionally substituted C₆-C₁₂-aryl)-C₁-C₄-alkyl (e.g. benzyl or (2-Cl-phenyl)-methyl) or optionally substituted C₆-C₁₂-aryl (e.g. phenyl, 4-F-phenyl, 3-F-phenyl, 2F-phenyl, 4-Cl-phenyl, or 2-Cl-phenyl, a further example being 3-Cl-phenyl);
- R^{16b}: is hydrogen; and
- R^{5a}, R^{5b}: are hydrogen.

Preferably, R¹ - in the pyrrolidine derivatives of formula (Ig) - is an optionally substituted 5-membered heterocyclic ring as defined herein containing 2 N which, in particular, is optionally substituted 1,3-diazolyl.

According to a further preferred embodiment, R¹ - in the pyrrolidine derivatives of formula (Ig) - is an optionally substituted 5-membered heterocyclic ring as defined herein containing 3 N which, in particular, is optionally substituted 1,2,3-triazolyl.

In connection with R¹ and the pyrrolidine derivatives of formula (Ig), substituted 5-membered heterocyclic ring in particular includes pyrazolyl, imidazolyl, and triazolyl, which are substituted with 1, 2 or 3 substituents independently selected from the group consisting of halogen, C₁-C₆-alkyl, halogenated C₁-C₆-alkyl, C₃-C₆-cycloalkyl, C₁-C₄-alkoxy-carbonyl and C₁-C₆-alkylcarbonylamino. Preferably, the substituent(s) on the heterocyclic ring are independently selected from the group consisting of halogen and C₁-C₆-alkyl. In particular, the substituent(s) on the heterocyclic ring are independently selected from the group consisting of C₁-C₆-alkyl, especially C₁-C₄-alkyl (e.g. methyl). According to a particular embodiment, 1,3-diazolyl is substituted with halogen or C₁-C₆-alkyl as described herein. According to a specific embodiment, R¹ is 1-methyl-1,3-diazol-4-yl.

In connection with R^{3a} and the pyrrolidine derivatives of formula (Ig), substituted C₆-C₁₂-aryl in particular includes C₆-C₁₂-aryl, such as phenyl or naphthyl, which is substituted with 1, 2, or 3 substituents independently selected from the group consisting of halogen, C₁-C₄-alkyl and C₁-C₄-alkoxy. Preferably, the substituent(s) on C₆-C₁₂-aryl are independently selected from the group consisting of halogen.

According to a preferred embodiment, R^{3a} - in the pyrrolidine derivatives of formula (Ig) - is C₆-C₁₂-aryl, in particular phenyl, optionally substituted with 1, 2, or 3 substituents independently selected from the group consisting of halogen (e.g. 4-F-phenyl).

In connection with R^{16a}, substituted C₆-C₁₂-aryl-C₁-C₄-alkyl in particular includes C₆-C₁₂-aryl-C₁-C₄-alkyl, such as benzyl, wherein C₆-C₁₂-aryl, such as phenyl, is substituted with 1, 2, or 3 substituents independently selected from the group consisting of halogen.

In connection with R^{16a}, substituted C₆-C₁₂-aryl in particular includes C₆-C₁₂-aryl, such as phenyl, which is substituted with 1, 2, or 3 substituents independently selected from the group consisting of halogen.

According to a preferred embodiment, R^{16a} is C₆-C₁₂-aryl-C₁-C₄-alkyl or C₆-C₁₂-aryl, with C₆-C₁₂-aryl being optionally substituted with 1, 2, or 3 substituents independently selected from the group consisting of halogen (e.g. benzyl or (2-Cl-phenyl)-methyl and phenyl, 4-F-phenyl, 3-F-phenyl, 2F-phenyl, 4-Cl-phenyl, or 2-Cl-phenyl).

Preferred embodiments of the pyrrolidine derivatives of formula (Ig) result if:
- R¹: is 1,3-diazolyl optionally substituted with halogen or C₁-C₄-alkyl (e.g. 1-methyl-1,3-diazol-4-yl);
- R^{2a}, R^{2b}: are hydrogen;
- R^{3a}: is C₆-C₁₂-aryl optionally substituted with 1, 2, or 3 substituents independently selected from the group consisting of halogen (e.g. 4-F-phenyl);
- R^{3b}: is hydrogen;
- Y¹: is >CR⁶;
- R⁶: is hydrogen;
- R^{8d}: is hydrogen;
- R^{16a}: is C₆-C₁₂-aryl-C₁-C₄-alkyl or C₆-C₁₂-aryl, with C₆-C₁₂-aryl being optionally substituted with 1, 2, or 3 substituents independently selected from the group consisting of halogen (e.g. benzyl or (2-Cl-phenyl)-methyl and phenyl, 4-F-phenyl, 3-F-phenyl, 2F-phenyl, 4-Cl-phenyl, or 2-Cl-phenyl, a further example being 3-Cl-phenyl);
- R^{16b}: is hydrogen; and
- R^{5a}, R^{5b}: are hydrogen.

According to a further embodiment, R⁴ is -O(CR^{9c}R^{9d})ₙ₅R¹⁸. Thus, the present invention relates to the pyrrolidine derivatives of the formula (Ih): 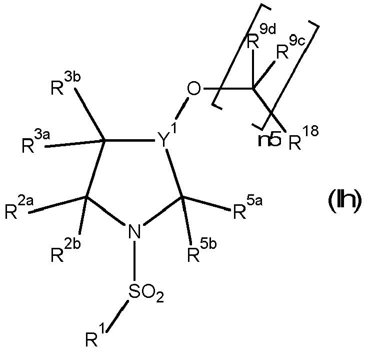 wherein R¹, R^{2a}, R^{2b}, R^{3a}, R^{3b}, Y¹, R^{5a} and R^{5b} are as defined herein, and
- R^{9c}, R^{9d}: are independently hydrogen, halogen (e.g. F), or C₁-C₆-alkyl (e.g. methyl or ethyl);
- n5: is 0, 1, 2, 3, or 4; and
- R¹⁸: is hydrogen, optionally substituted C₁-C₈-alkyl (e.g. methyl, ethyl, iso-propyl or pentyl), optionally substituted C₃-C₁₂-cycloalkyl (e.g. cyclopropyl or cyclohexyl), C₁-C₆-alkylcarbonyl (e.g. methylcarbonyl), C₁-C₆-alkoxycarbonyl (e.g. methoxycabonyl or n-butoxycarbonyl), halogenated C₁-C₆-alkoxycarbonyl (e.g. -C(O)OCF₃), C₆-C₁₂-aryloxycarbonyl (e.g. phenoxycarbonyl), C₁-C₆-alkylaminocarbonyl (e.g. n-propylaminocarbonyl or n-butylaminocarbonyl), (halogenated C₁-C₄-alkyl)aminocarbonyl (e.g. 2,2-di F-ethylaminocarbonyl, a further example being 2,2,2-tri F-ethylaminocarbonyl), C₆-C₁₂-arylaminocarbonyl (e.g.phenylaminocarbonyl), optionally substituted C₆-C₁₂-aryl (e.g. phenyl, 3-F-phenyl, 4-F-phenyl, 4-Me-phenyl, 3-MeSO₂-phenyl, or 4-MeSO₂-phenyl, a further example being 4-F-2-CF₃-phenyl, 4-F-3-CF₃-phenyl, or 4-F-3-OCF₃-phenyl), C₁-C₆-alkylamine (e.g. n-propylamine), (C₃-C₁₂-cycloalkyl-C₁-C₄-alkyl)amino (e.g. cyclopropyl-methyl-amino), (halogenated C₁-C₆-alkyl)amino (e.g. 2,2,2-triF-ethylamine, a further example being 2,2-diF-ethylamine), (C₁-C₆-alkoxy- C₁-C₆-alkyl)amino (e.g. 2-methoxyethylamine), (C₆-C₁₂-aryl-C₁-C₄-alkyl)amino (e.g. benzylamine), C₁-C₆-dialkylamine (e.g. dimethylamine), optionally substituted C₆-C₁₂-arylamine (e.g. 4-Cl-phenylamine), or optionally substituted C₃-C₁₂-heterocyclyl (e.g. 4-CF₃-pyrid-2-yl or 6-CF₃-pyrimid-4-yl, a further example being 4-Me-pyrid-2-yl).

In particular, R^{9c}, R^{9d} are hydrogen.

In particular, n5 is 0, 1, or 2.

In particular, R¹⁸ is hydrogen, optionally substituted C₁-C₈-alkyl (e.g. methyl, ethyl, isopropyl, or pentyl), C₁-C₆-alkoxycarbonyl (e.g. methoxycabonyl or n-butoxycarbonyl), C₁-C₆-alkylaminocarbonyl (e.g. n-propylaminocarbonyl or n-butylaminocarbonyl), (halogenated C₁-C₄-alkyl)aminocarbonyl (e.g. 2,2-di F-ethylaminocarbonyl, a further example being 2,2,2-tri F-ethylaminocarbonyl), optionally substituted C₆-C₁₂-aryl (e.g. phenyl, 3-F-phenyl, 4-F-phenyl, 4-Me-phenyl, 3-MeSO₂-phenyl, or 4-MeSO₂-phenyl, a further example being 4-F-2-CF₃-phenyl, 4-F-3-CF₃-phenyl, or 4-F-3-OCF₃-phenyl), C₁-C₆-alkylamine (e.g. n-propylamine), (halogenated C₁-C₆-alkyl)amino (e.g. 2,2,2-triF-ethylamine, a further example being 2,2-diF-ethylamine), optionally substituted C₆-C₁₂-arylamine (e.g. 4-Cl-phenylamine), or optionally substituted C₃-C₁₂-heterocyclyl (e.g. 4-CF₃-pyrid-2-yl or 6-CF₃-pyrimid-4-yl, a further example being 4-Me-pyrid-2-yl).

R^{2a}, R^{2b} in pyrrolidine derivatives of formula (Ih) are, in particular, hydrogen.

R^{3a} - in the pyrrolidine derivatives of formula (Ih) - is, in particular, C₃-C₁₂-cycloalkyl (e.g. cyclopropyl, cyclobutyl, cyclopentyl, or cyclohexyl), optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl), C₃-C₁₂-cycloalkyl-C₁-C₄-alkoxy (e.g. cyclopropylmethoxy or cyclohexylmethoxy), C₂-C₆-alkenyloxy (e.g. allyloxy or 2-methylprop-2-en-1-oxy), C₆-C₁₂-aryl-C₁-C₄-alkoxy (e.g. benzyloxy), optionally substituted C₆-C₁₂-aryloxy (e.g. phenoxy or 4-F-phenoxy), or C₃-C₁₂-heterocyclyl (e.g.pyrid-2-yl). Preferably, R^{3a} is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl, a further example being 2-Me-phenyl, 3-Me-phenyl, or 4-Me-phenyl), or optionally substituted C₃-C₁₂-heterocyclyl (e.g. 3-F-azetidin-1-yl, morpholin-1-yl, pyrrolidin-1-yl, piperidin-1-yl, 2-Me-piperidin-1yl, 3-Me-piperidin-1yl, 4-Me-piperidin-lyl, 4-Fpiperidin-1-yl, 4,4-diF-piperidin-1yl, or azepan-1-yl).

R^{3b} - in the pyrrolidine derivatives of formula (Ih) - is, in particular, hydrogen.

Y¹ - in the pyrrolidine derivatives of formula (Ih) - is, in particular, >CR⁶.

R⁶ - in the pyrrolidine derivatives of formula (Ih) - is, in particular, hydrogen or C₆-C₁₂-aryl-C₁-C₄-alkyl (e.g. benzyl). Additionally, R⁶ may be, in particular, C₁-C₄-alkylene (e.g. methylene or 1,2-ethylene) that is bound to a carbon atom in R^{3a}, and R^{3a} is an optionally substituted C₆-C₁₂-aryl (e.g. phenyl) or an optionally substituted C₃-C₁₂-heterocyclyl. According to a particular embodiment, R⁶ is hydrogen or benzyl. According to a further particular embodiment, R⁶ may be C₁-C₄-alkylene (e.g. 1,2-ethylene) that is bound to a carbon atom in R^{3a}, and R^{3a} is an optionally substituted C₆-C₁₂-aryl (e.g. phenyl). Preferably, R⁶ is hydrogen. It is further preferred if R⁶ is C₁-C₄-alkylene (e.g. 1,2-ethylene) that is bound to a carbon atom in R^{3a}, and R^{3a} is an optionally substituted C₆-C₁₂-aryl (e.g. phenyl).

R^{5a}, R^{5b} - in the pyrrolidine derivatives of formula (Ih) - are, in particular, hydrogen.

Particular embodiments of pyrrolidine derivatives of formula (Ih) result if:
- R¹: is an optionally substituted 5-membered heterocyclic ring as defined herein (e.g. 1,2-diazol-4-yl, 1-methyl-1,2-diazol-4-yl, 1-methyl-1,2-diazol-3-yl, 1-methyl-1,3-diazol-4-yl, 1-methyl-1,2-diazol-5-yl, 1,5-dimethyl-1,2-diazol-4-yl, 1,3-dimethyl-1,2-diazol-4-yl, 1,5-dimethyl-1,2-diazol-4-yl, 1,3-dimethyl-1,2-diazol-5-yl, 1,3-dimethyl-5-Cl-1,2-diazol-4-yl, 1-isopropyl-3-methyl-1,2-diazol-4-yl, 1-Me-3-CF₃-1,2-diazol-4-yl, 1-cyclopentyl-3-Me-1,2-diazol-4-yl, 1,3,5-trimethyl-1,2-diazol-4-yl, 1-CHF₂-3,5-dimethyl-1,2-diazol-4-yl, 1,2-dimethyl-1,3-diazol-4-yl, 1,2-dimethyl-1,3-diazol-5-yl, 1-methyl-5-Cl-1,3-diazol-4-yl, 1-Me-1,2,3-triazol-4-yl, a further example being 1-ethyl-1,3-diazol-4-yl or 1-methyl-1,2,4-triazol-3-yl);
- R^{2a}, R^{2b}: are hydrogen;
- R^{3a}: is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl, a further example being 2-Me-phenyl, 3-Me-phenyl, or 4-Me-phenyl), or optionally substituted C₃-C₁₂-heterocyclyl (e.g. 3-F-azetidin-1-yl, morpholin-4-yl, pyrrolidin-1-yl, piperidin-1-yl, 2-Me-piperidin-1yl, 3-Me-piperidin-1yl, 4-Me-piperidin-1yl, 4-F-piperidin-1-yl, 4,4-diF-piperidin-lyl, or azepan-1-yl);
- R^{3b}: is hydrogen;
- Y¹: is >CR⁶;
- R⁶: is hydrogen;
- R^{9c}, R^{9d}: are hydrogen;
- n5: is 0, 1, or 2;
- R¹⁸: is hydrogen, optionally substituted C₁-C₈-alkyl (e.g. methyl, ethyl, iso-propyl, or pentyl), C₁-C₆-alkoxycarbonyl (e.g. methoxycabonyl orbutoxycarbonyl), C₁-C₆-alkylaminocarbonyl (e.g. n-propylaminocarbonyl or n-butylaminocarbonyl), (halogenated C₁-C₄-alkyl)aminocarbonyl (e.g. 2,2-di F-ethylaminocarbonyl, a further example being 2,2,2-tri F-ethylaminocarbonyl), optionally substituted C₆-C₁₂-aryl (e.g. phenyl, 3-F-phenyl, 4-F-phenyl, 4-Me-phenyl, 3-MeSO₂-phenyl, or 4-MeSO₂-phenyl, a further example being 4-F-2-CF₃-phenyl, 4-F-3-CF₃-phenyl, or 4-F-3-OCF₃-phenyl), C₁-C₆-alkylamine (e.g. n-propylamine), (halogenated C₁-C₆-alkyl)amino (e.g. 2,2,2-triF-ethylamine, a further example being 2,2-diF-ethylamine), optionally substituted C₆-C₁₂-arylamine (e.g. 4-Cl-phenylamine), or optionally substituted C₃-C₁₂-heterocyclyl (e.g. 4-CF₃-pyrid-2-yl or 6-CF₃-pyrimid-4-yl, a further example being 4-Me-pyrid-2-yl); and
- R^{5a}, R^{5b}: are hydrogen.

Additional particular embodiments of pyrrolidine derivatives of formula (Ih) result if:
- R⁶: is C₁-C₄-alkylene (e.g. 1,2-ethylene) that is bound to a carbon atom in R^{3a}, and R^{3a} is an optionally substituted C₆-C₁₂-aryl (e.g. phenyl); and
- R¹, R^{2a}, R^{2b}, R^{3a}, R^{3b}, Y¹, R^{9c}, R^{9d}, n5, R¹⁸, R^{5a} and R^{5b}: are as defined above.

Preferably, R¹ - in the pyrrolidine derivatives of formula (Ih) - is an optionally substituted 5-membered heterocyclic ring as defined herein containing 2 N which, in particular, is optionally substituted 1,3-diazolyl.

According to a further preferred embodiment, R¹ - in the pyrrolidine derivatives of formula (Ih) - is an optionally substituted 5-membered heterocyclic ring as defined herein containing 3 N which, in particular, is optionally substituted 1,2,3-triazolyl.

In connection with R¹ and the pyrrolidine derivatives of formula (Ih), substituted 5-membered heterocyclic ring in particular includes pyrazolyl, imidazolyl, and triazolyl, which are substituted with 1, 2 or 3 substituents independently selected from the group consisting of halogen, C₁-C₆-alkyl, halogenated C₁-C₆-alkyl, C₃-C₆-cycloalkyl, C₁-C₄-alkoxy-carbonyl and C₁-C₆-alkylcarbonylamino. Preferably, the substituent(s) on the heterocyclic ring are independently selected from the group consisting of halogen and C₁-C₆-alkyl. In particular, the substituent(s) on the heterocyclic ring are independently selected from the group consisting of C₁-C₆-alkyl, especially C₁-C₄-alkyl (e.g. methyl). According to a particular embodiment, 1,3-diazolyl is substituted with halogen or C₁-C₆-alkyl as described herein. According to a specific embodiment, R¹ is 1-methyl-1,3-diazol-4-yl.

In connection with R^{3a} and the pyrrolidine derivatives of formula (Ih), substituted C₆-C₁₂-aryl in particular includes C₆-C₁₂-aryl, such as phenyl or naphthyl, which is substituted with 1, 2, or 3 substituents independently selected from the group consisting of halogen, C₁-C₄-alkyl and C₁-C₄-alkoxy. Preferably, the substituent(s) on C₆-C₁₂-aryl are independently selected from the group consisting of halogen.

In connection with R^{3a} and the pyrrolidine derivatives of formula (Ih), substituted C₃-C₁₂-heterocyclyl in particular includes C₃-C₁₂-heterocyclyl, such as azetidinyl, morpholinyl, pyrrolidinyl, piperidinyl, or azepanyl, which is substituted with 1, 2, or 3 substituents independently selected from the group consisting of halogen, C₁-C₄-alkyl and C₁-C₄-alkoxy. Preferably, the substituent(s) on C₃-C₁₂-heterocyclyl are independently selected from the group consisting of halogen and C₁-C₄-alkyl.

According to a preferred embodiment, R^{3a} - in the pyrrolidine derivatives of formula (Ih) - is C₆-C₁₂-aryl optionally substituted with 1, 2, or 3 substituents independently selected from the group consisting of halogen (e.g. 4-F-phenyl), or C₃-C₁₂-heterocyclyl optionally substituted with 1, 2, or 3 substituents independently selected from the group consisting of halogen and C₁-C₄-alkyl (e.g. 3-F-azetidin-1-yl, morpholin-1-yl, pyrrolidin-1-yl, piperidin-1-yl, 2-Me-piperidin-1yl, 3-Me-piperidin-lyl, 4-Me-piperidin-1yl, 4-F-piperidin-1-yl, 4,4-diF-piperidin-1yl, or azepan-1-yl).

In connection with R¹⁸, substituted C₆-C₁₂-aryl in particular includes C₆-C₁₂-aryl, such as phenyl or naphthyl, which is substituted with 1, 2, or 3 substituents independently selected from the group consisting of halogen, C₁-C₄-alkyl and C₁-C₄-alkyl-sulfonyl. Additional substituents may be independently selected from the group consisting of halogenated C₁-C₄-alkyl and halogenated C₁-C₄-alkoxy.

In connection with R¹⁸, substituted C₆-C₁₂-arylamine in particular includes C₆-C₁₂-arylamine, such as phenylamine, which is substituted with 1, 2, or 3 substituents independently selected from the group consisting of halogen.

In connection with R¹⁸, substituted C₃-C₁₂-heterocyclyl in particular includes C₃-C₁₂-heterocyclyl, such as 2-pyridyl or 4-pyrimidyl, which is substituted with 1, 2, or 3 substituents independently selected from the group consisting of halogenated C₁-C₄-alkyl. Additional substituents may be independently selected from the group consisting of C₁-C₄-alkyl.

According to a preferred embodiment, R¹⁸ is hydrogen, C₁-C₈-alkyl (e.g. methyl, ethyl, iso-propyl, or pentyl), C₁-C₆-alkoxycarbonyl (e.g. methoxycabonyl or n-butoxycarbonyl), C₁-C₆-alkylaminocarbonyl (e.g. n-propylaminocarbonyl or n-butylaminocarbonyl), (halogenated C₁-C₄-alkyl)aminocarbonyl (e.g. 2,2-di F-ethylaminocarbonyl, a further example being 2,2,2-tri F-ethylaminocarbonyl), C₆-C₁₂-aryl optionally substituted with 1, 2, or 3 substituents independently selected from the group consisting of halogen, C₁-C₄-alkyl and C₁-C₄-alkyl-sulfonyl, and additionally from the group consisting of halogenated C₁-C₄-alkyl and halogenated C₁-C₄-alkoxy (e.g. phenyl, 3-F-phenyl, 4-F-phenyl, 4-Me-phenyl, 3-MeSO₂-phenyl, or 4-MeSO₂-phenyl, a further example being 4-F-2-CF₃-phenyl, 4-F-3-CF₃-phenyl, or 4-F-3-OCF₃-phenyl), or C₁-C₆-alkylamine (e.g. n-propylamine), (halogenated C₁-C₆-alkyl)amino (e.g. 2,2,2-triF-ethylamine, a further example being 2,2-diF-ethylamine), C₆-C₁₂-arylamine optionally substituted with 1, 2, or 3 substituents independently selected from the group consisting of halogen (e.g. 4-Cl-phenylamine), or C₃-C₁₂-heterocyclyl optionally substituted with 1, 2, or 3 substituents independently selected from the group consisting of halogenated C₁-C₄-alkyl, and additionally from the group consisting of C₁-C₄-alkyl (e.g. 4-CF₃-pyrid-2-yl or 6-CF₃-pyrimid-4-yl, a further example being 4-Me-pyrid-2-yl).

Further preferred embodiments of the pyrrolidine derivatives of formula (Ih) result if:
- R¹: is 1,3-diazolyl optionally substituted with halogen or C₁-C₄-alkyl (e.g. 1-methyl-1,3-diazol-4-yl);
- R^{2a}, R^{2b}: are hydrogen;
- R^{3a}: C₆-C₁₂-aryl optionally substituted with 1, 2, or 3 substituents independently selected from the group consisting of halogen (e.g. 4-F-phenyl), or C₃-C₁₂-heterocyclyl optionally substituted with 1, 2, or 3 substituents independently selected from the group consisting of halogen and C₁-C₄-alkyl (e.g. 3-F-azetidin-1-yl, morpholin-1-yl, pyrrolidin-1-yl, piperidin-1-yl, 2-Me-piperidin-1yl, 3-Me-piperidin-1yl, 4-Me-piperidin-1yl, 4-F-piperidin-1-yl, 4,4-diF-piperidin-lyl, or azepan-1-yl);
- R^{3b}: is hydrogen;
- Y¹: is >CR⁶;
- R⁶: is hydrogen, or
- R⁶: is C₁-C₄-alkylene (e.g. 1,2-ethylene) that is bound to a carbon atom in R^{3a}, and R^{3a} is an optionally substituted C₆-C₁₂-aryl (e.g. phenyl);
- R^{9c}, R^{9d}: are hydrogen;
- n5: is 0, 1, or 2;
- R¹⁸: is hydrogen, C₁-C₈-alkyl (e.g. methyl, ethyl, iso-propyl, or pentyl), C₁-C₆-alkoxycarbonyl (e.g. methoxycabonyl or n-butoxycarbonyl), C₁-C₆-alkylaminocarbonyl (e.g. n-propylaminocarbonyl or n-butylaminocarbonyl), (halogenated C₁-C₄-alkyl)aminocarbonyl (e.g. 2,2-di F-ethylaminocarbonyl, a further example being 2,2,2-tri F-ethylaminocarbonyl), C₆-C₁₂-aryl optionally substituted with 1, 2, or 3 substituents independently selected from the group consisting of halogen, C₁-C₄-alkyl and C₁-C₄-alkyl-sulfonyl, and additionally from the group consisting of halogenated C₁-C₄-alkyl and halogenated C₁-C₄-alkoxy (e.g. phenyl, 3-F-phenyl, 4-F-phenyl, 4-Me-phenyl, 3-MeSO₂-phenyl, or 4-MeSO₂-phenyl, a further example being 4-F-2-CF₃-phenyl, 4-F-3-CF₃-phenyl, or 4-F-3-OCF₃-phenyl), or C₁-C₆-alkylamine (e.g. n-propylamine), (halogenated C₁-C₆-alkyl)amino (e.g. 2,2,2-triF-ethylamine, a further example being 2,2-diF-ethylamine), C₆-C₁₂-arylamine optionally substituted with 1, 2, or 3 substituents independently selected from the group consisting of halogen (e.g. 4-Cl-phenylamine), or C₃-C₁₂-heterocyclyl optionally substituted with 1, 2, or 3 substituents independently selected from the group consisting of halogenated C₁-C₄-alkyl, and additionally from the group consisting of C₁-C₄-alkyl (e.g. 4-CF₃-pyrid-2-yl or 6-CF₃-pyrimid-4-yl, a further example being 4-Me-pyrid-2-yl); and
- R^{5a}, R^{5b}: are hydrogen.

Further preferred embodiments of pyrrolidine derivatives of formula (Ih) result if:
- R⁶: is hydrogen;
- R¹⁸: is hydrogen, C₁-C₈-alkyl (e.g. methyl, ethyl, iso-propyl, or pentyl), C₁-C₆-alkoxycarbonyl (e.g. methoxycabonyl or n-butoxycarbonyl), C₁-C₆-alkylaminocarbonyl (e.g. n-propylaminocarbonyl or n-butylaminocarbonyl), (halogenated C₁-C₄-alkyl)aminocarbonyl (e.g. 2,2-di F-ethylaminocarbonyl, a further example being 2,2,2-tri F-ethylaminocarbonyl), C₆-C₁₂-aryl optionally substituted with 1, 2, or 3 substituents independently selected from the group consisting of halogen, C₁-C₄-alkyl and C₁-C₄-alkyl-sulfonyl (e.g. phenyl, 3-F-phenyl, 4-F-phenyl, 4-Me-phenyl, 3-MeSO₂-phenyl, or 4-MeSO₂-phenyll), or C₁-C₆-alkylamine (e.g. n-propylamine), (halogenated C₁-C₆-alkyl)amino (e.g. 2,2,2-triF-ethylamine, a further example being 2,2-diF-ethylamine), C₆-C₁₂-arylamine optionally substituted with 1, 2, or 3 substituents independently selected from the group consisting of halogen (e.g. 4-Cl-phenylamine), or C₃-C₁₂-heterocyclyl optionally substituted with 1, 2, or 3 substituents independently selected from the group consisting of halogenated C₁-C₄-alkyl (e.g. 4-CF₃-pyrid-2-yl or 6-CF₃-pyrimid-4-yl);and
- R¹, R^{2a}, R^{2b}, R^{3a}, R^{3b}, Y¹, R^{9c}, R^{9d}, n5, R^{5a} and R^{5b}: are as defined above.

According to a further embodiment, R⁴ is -COR¹⁹. Thus, the present invention relates to the pyrrolidine derivatives of the formula (Ii): 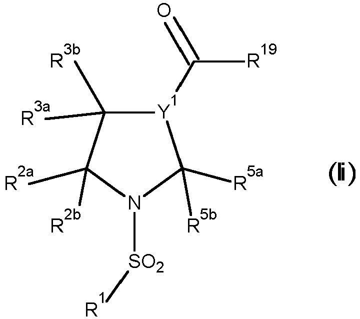 wherein R¹, R^{2a}, R^{2b}, R^{3a}, R^{3b}, Y¹, R^{5a} and R^{5b} are as defined herein, and
- R¹⁹: is optionally substituted C₆-C₁₂-aryl (e.g. phenyl, 3-F-phenyl, 4-F-phenyl, 3-Cl-phenyl, 3,4-diF-phenyl, 3,4-diCl-phenyl, 3,5-diCl-phenyl, 3-F-4-Cl-phenyl, 3-F-5-Cl-phenyl, 3-Me-phenyl, 3-CF₃-phenyl, 3-OCF₃-phenyl, or 4-OCF₃-phenyl) or optionally substituted C₃-C₁₂-heterocyclyl (e.g. 2-pyridyl or 6-pyrimidyl).

In particular, R¹⁹ is optionally substituted C₆-C₁₂-aryl (e.g. phenyl, 3-F-phenyl, 4-F-phenyl, 3-Cl-phenyl, 3,4-diF-phenyl, 3,4-diCl-phenyl, 3,5-diCl-phenyl, 3-F-4-Cl-phenyl, 3-F-5-Cl-phenyl, 3-Me-phenyl, 3-CF₃-phenyl, 3-OCF₃-phenyl, or 4-OCF₃-phenyl).

R^{2a}, R^{2b} - in the pyrrolidine derivatives of formula (Ii) - are, in particular, hydrogen.

R^{3a} - in the pyrrolidine derivatives of formula (Ii) - is, in particular, C₃-C₁₂-cycloalkyl (e.g. cyclopropyl, cyclobutyl, cyclopentyl, or cyclohexyl), optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl), C₃-C₁₂-cycloalkyl-C₁-C₄-alkoxy (e.g. cyclopropylmethoxy or cyclohexylmethoxy), C₂-C₆-alkenyloxy (e.g. allyloxy or 2-methylprop-2-en-1-oxy), C₆-C₁₂-aryl-C₁-C₄-alkoxy (e.g. benzyloxy), optionally substituted C₆-C₁₂-aryloxy (e.g. phenoxy or 4-F-phenoxy), or C₃-C₁₂-heterocyclyl (e.g.pyrid-2-yl). Preferably, R^{3a} is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl). It is further preferred if R^{3a} is C₃-C₁₂-heterocyclyl (e.g. pyrid-2-yl).

R^{3b} - in the pyrrolidine derivatives of formula (Ii) - is, in particular, hydrogen.

Y¹ - in the pyrrolidine derivatives of formula (Ii) - is, in particular, >CR⁶.

R⁶ - in the pyrrolidine derivatives of formula (Ii) - is, in particular, hydrogen.

R^{5a}, R^{5b} - in the pyrrolidine derivatives of formula (Ii) - are, in particular, hydrogen.

Particular embodiments of the pyrrolidine derivatives of formula (Ii) result if
- R¹: is an optionally substituted 5-membered heterocyclic ring as defined herein (e.g. 1,2-diazol-4-yl, 1-methyl-1,2-diazol-4-yl, 1-methyl-1,2-diazol-3-yl, 1-methyl-1,3-diazol-4-yl, 1-methyl-1,2-diazol-5-yl, 1,5-dimethyl-1,2-diazol-4-yl, 1,3-dimethyl-1,2-diazol-4-yl, 1,5-dimethyl-1,2-diazol-4-yl, 1,3-dimethyl-1,2-diazol-5-yl, 1,3-dimethyl-5-Cl-1,2-diazol-4-yl, 1-isopropyl-3-methyl-1,2-diazol-4-yl, 1-Me-3-CF₃-1,2-diazol-4-yl, 1-cyclopentyl-3-Me-1,2-diazol-4-yl, 1,3,5-trimethyl-1,2-diazol-4-yl, 1-CHF₂-3,5-dimethyl-1,2-diazol-4-yl, 1,2-dimethyl-1,3-diazol-4-yl, 1,2-dimethyl-1,3-diazol-5-yl, 1-methyl-5-Cl-1,3-diazol-4-yl, 1-Me-1,2,3-triazol-4-yl, a further example being 1-ethyl-1,3-diazol-4-yl or 1-methyl-1,2,4-triazol-3-yl);
- R^{2a}, R^{2b}: are hydrogen;
- R^{3a}: is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl);
- R^{3b}: is hydrogen;
- Y¹: is >CR⁶;
- R⁶: is hydrogen;
- R¹⁹: is optionally substituted C₆-C₁₂-aryl (e.g. phenyl, 3-F-phenyl, 4-F-phenyl, 3-Cl-phenyl, 3,4-diF-phenyl, 3,4-diCl-phenyl, 3,5-diCl-phenyl, 3-F-4-Cl-phenyl, 3-F-5-Cl-phenyl, 3-Me-phenyl, 3-CF₃-phenyl, 3-OCF₃-phenyl, or 4-OCF₃-phenyl); and
- R^{5a}, R^{5b}: are hydrogen.

Additional particular embodiments of the pyrrolidine derivatives of formula (Ii) result if R^{3a} is C₃-C₁₂-heterocyclyl (e.g. pyrid-2-yl); and
R¹, R^{2a}, R^{2b}, R^{3b}, Y¹, R⁶, R¹⁹, R^{5a} and R^{5b} are as defined above.

Preferably, R¹ - in the pyrrolidine derivatives of formula (Ii) - is an optionally substituted 5-membered heterocyclic ring as defined herein containing 2 N which, in particular, is optionally substituted 1,3-diazolyl.

According to a further preferred embodiment, R¹ - in the pyrrolidine derivatives of formula (Ii) - is an optionally substituted 5-membered heterocyclic ring as defined herein containing 3 N which, in particular, is optionally substituted 1,2,3-triazolyl.

In connection with R¹ and the pyrrolidine derivatives of formula (Ii), substituted 5-membered heterocyclic ring in particular includes pyrazolyl, imidazolyl, and triazolyl, which are substituted with 1, 2 or 3 substituents independently selected from the group consisting of halogen, C₁-C₆-alkyl, halogenated C₁-C₆-alkyl, C₃-C₆-cycloalkyl, C₁-C₄-alkoxy-carbonyl and C₁-C₆-alkylcarbonylamino. Preferably, the substituent(s) on the heterocyclic ring are independently selected from the group consisting of halogen and C₁-C₆-alkyl. In particular, the substituent(s) on the heterocyclic ring are independently selected from the group consisting of C₁-C₆-alkyl, especially C₁-C₄-alkyl (e.g. methyl). According to a particular embodiment, 1,3-diazolyl is substituted with halogen or C₁-C₆-alkyl as described herein. According to a specific embodiment, R¹ is 1-methyl-1,3-diazol-4-yl.

In connection with R^{3a} and the pyrrolidine derivatives of formula (Ii), substituted C₆-C₁₂-aryl in particular includes C₆-C₁₂-aryl, such as phenyl or naphthyl, which is substituted with 1, 2, or 3 substituents independently selected from the group consisting of halogen, C₁-C₄-alkyl and C₁-C₄-alkoxy. Preferably, the substituent(s) on C₆-C₁₂-aryl are independently selected from the group consisting of halogen.

According to a preferred embodiment, R^{3a} - in the pyrrolidine derivatives of formula (Ii) - is C₆-C₁₂-aryl, in particular phenyl, optionally substituted with 1, 2, or 3 substituents independently selected from the group consisting of halogen (e.g. 4-F-phenyl). According to an additional preferred embodiment, R^{3a} - in the pyrrolidine derivatives of formula (Ii) - is C₃-C₁₂-heterocyclyl such as pyrid-2-yl.

In connection with R¹⁹, substituted C₆-C₁₂-aryl in particular includes C₆-C₁₂-aryl, such as phenyl or naphthyl, which is substituted with 1, 2, or 3 substituents independently selected from the group consisting of halogen, C₁-C₄-alkyl, halogenated C₁-C₄-alkyl and halogenated C₁-C₄-alkoxy.

According to a preferred embodiment, R¹⁹ is C₆-C₁₂-aryl optionally substituted with 1, 2, or 3 substituents independently selected from the group consisting of halogen, C₁-C₄-alkyl, halogenated C₁-C₄-alkyl and halogenated C₁-C₄-alkoxy (e.g phenyl, 3-F-phenyl, 4-F-phenyl, 3-Cl-phenyl, 3,4-diF-phenyl, 3,4-diCl-phenyl, 3,5-diCl-phenyl, 3-F-4-Cl-phenyl, 3-F-5-Cl-phenyl, 3-Me-phenyl, 3-CF₃-phenyl, 3-OCF₃-phenyl, or 4-OCF₃-phenyl).

Further preferred embodiments of the pyrrolidine derivatives of formula (Ii) result if:
- R¹: is 1,3-diazolyl optionally substituted with halogen or C₁-C₄-alkyl (e.g. 1-methyl-1,3-diazol-4-yl);
- R^{2a}, R^{2b}: are hydrogen;
- R^{3a}: is C₆-C₁₂-aryl optionally substituted with 1, 2, or 3 substituents independently selected from the group consisting of halogen (e.g. 4-F-phenyl);
- R^{3b}: is hydrogen;
- Y¹: is >CR⁶;
- R⁶: is hydrogen;
- R¹⁹: C₆-C₁₂-aryl optionally substituted with 1, 2, or 3 substituents independently selected from the group consisting of halogen, C₁-C₄-alkyl, halogenated C₁-C₄-alkyl and halogenated C₁-C₄-alkoxy (e.g phenyl, 3-F-phenyl, 4-F-phenyl, 3-Cl-phenyl, 3,4-diF-phenyl, 3,4-diCl-phenyl, 3,5-diCl-phenyl, 3-F-4-Cl-phenyl, 3-F-5-Cl-phenyl, 3-Me-phenyl, 3-CF₃-phenyl, 3-OCF₃-phenyl, or 4-OCF₃-phenyl); and
- R^{5a}, R^{5b}: are hydrogen.

Additional preferred embodiments of the pyrrolidine derivatives of formula (Ii) result if R^{3a} is C₃-C₁₂-heterocyclyl (e.g.pyrid-2-yl); and
R¹, R^{2a}, R^{2b}, R^{3b}, Y¹, R⁶, R¹⁹, R^{5a} and R^{5b} are as defined above.

According to a further particular embodiment R⁴ is -CONR^{20a}R^{20b}. Thus, the present invention relates to the pyrrolidine derivatives of the formula (II): 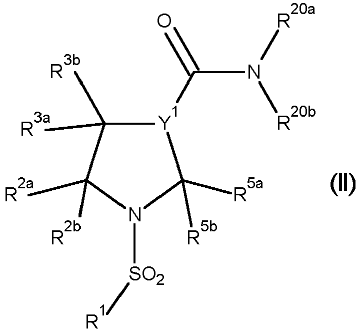 wherein R¹, R^{2a} , R^{2b}, R^{3a}, R^{3b}, Y¹, R^{5a} and R^{5b} are as defined herein, and
- R^{20a}: is C₁-C₈-alkyl (e.g. n-propyl, n-butyl, hexyl, pentyl, or 6-Me-heptyl), (optionally substituted C₃-C₁₂-cycloalkyl)-C₁-C₄-alkyl (e.g. cyclopropyl-methyl), C₁-C₆-alkoxy-C₁-C₆-alkyl (e.g. 2-methoxy-ethyl, 2-ethoxy-ethyl, 2-isopropyloxy-ethyl, 1-methyl-2-methoxy-ethyl, 3-methoxy-propyl, 3-ethoxy-propyl, or 3-isopropyloxy-propyl), (optionally substituted C₆-C₁₂-aryl)-C₁-C₄-alkyl (e.g. benzyl, (4-F-phenyl)methyl, (3-Cl -phenyl)methyl, (4-Cl - phenyl)methyl, (3,4-diF-phenyl)methyl, (3-Cl-4-F-phenyl)methyl, (3-CF₃-phenyl)methyl, (2-Cl-4-CF₃-phenyl)methyl, (2-Cl-5-CF₃-phenyl)methyl, or (3-OCF₃-phenyl)methyl)), (optionally substituted C₃-C₁₂-heterocyclyl)-C₁-C₄-alkyl (e.g. (pyrid-2-yl)-methyl or (4-CF₃-pyrid-2-yl)methyl), optionally substituted C₆-C₁₂-aryl (e.g. phenyl, 4-F-phenyl, 3-Cl-phenyl, 3,5-diCl-phenyl, 3-Cl-4-F-phenyl, 3-Me-phenyl, 3-CF₃-phenyl, 4-CF₃-phenyl, 2-Cl-3-CF₃-phenyl, 3-Cl-4-CF₃-phenyl, 3-CF₃-4-F-phenyl, 3-CN-phenyl, 3-OCF₃-phenyl, 3-OCF₃-4-F-phenyl, or 3-OCF₃-4-Cl-phenyl), or optionally substituted C₃-C₁₂-heterocyclyl (e.g. 4-CF₃-pyrid-2-yl); and
- R^{20b}: is hydrogen or C₁-C₈-alkyl (e.g. methyl, ethyl, or n-butyl).

In particular, R^{20a} is C₁-C₈-alkyl (e.g. n-propyl, n-butyl, hexyl, pentyl, or 6-Me-heptyl), (C₃-C₁₂-cycloalkyl)-C₁-C₄-alkyl (e.g. cyclopropyl-methyl), C₁-C₆-alkoxy-C₁-C₆-alkyl (e.g. 2-methoxy-ethyl, 2-ethoxy-ethyl, 2-isopropyloxy-ethyl, 1-methyl-2-methoxy-ethyl, 3-methoxy-propyl, 3-ethoxy-propyl, or 3-isopropyloxy-propyl), (optionally substituted C₆-C₁₂-aryl)-C₁-C₄-alkyl (e.g. benzyl, (4-F-phenyl)methyl, (3-Cl -phenyl)methyl, (4-Cl -phenyl)methyl, (3,4-diF-phenyl)methyl, (3-Cl-4-F-phenyl)methyl, (3-CF₃-phenyl)methyl, (2-Cl-4-CF₃-phenyl)methyl, (2-Cl-5-CF₃-phenyl)methyl, or (3-OCF₃-phenyl)methyl)), (optionally substituted C₃-C₁₂-heterocyclyl)-C₁-C₄-alkyl (e.g. (pyrid-2-yl)-methyl or (4-CF₃-pyrid-2-yl)methyl), optionally substituted C₆-C₁₂-aryl (e.g. phenyl, 4-F-phenyl, 3-Cl-phenyl, 3,5-diCl-phenyl, 3-Cl-4-F-phenyl, 3-Me-phenyl, 3-CF₃-phenyl, 4-CF₃-phenyl, 2-Cl-3-CF₃-phenyl, 3-Cl-4-CF₃-phenyl, 3-CF₃-4-F-phenyl, 3-CN-phenyl, 3-OCF₃-phenyl, 3-OCF₃-4-F-phenyl, or 3-OCF₃-4-Cl-phenyl), or optionally substituted C₃-C₁₂-heterocyclyl (e.g. 4-CF₃-pyrid-2-yl).

R^{2a,} R^{2b} - in the pyrrolidine derivatives of formula (Il) - are, in particular, hydrogen.

R^{3a} - in the pyrrolidine derivatives of formula (Il) - is, in particular, C₃-C₁₂-cycloalkyl (e.g. cyclopropyl, cyclobutyl, cyclopentyl, or cyclohexyl), optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl), C₃-C₁₂-cycloalkyl-C₁-C₄-alkoxy (e.g. cyclopropylmethoxy or cyclohexylmethoxy), C₂-C₆-alkenyloxy (e.g. allyloxy or 2-methylprop-2-en-1-oxy), C₆-C₁₂-aryl-C₁-C₄-alkoxy (e.g. benzyloxy), optionally substituted C₆-C₁₂-aryloxy (e.g. phenoxy or 4-F-phenoxy), or C₃-C₁₂-heterocyclyl (e.g.pyrid-2-yl). Preferably, R^{3a} is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl).

R^{3b} - in the pyrrolidine derivatives of formula (Il) - is, in particular, hydrogen.

Additionally, R^{3a} and R^{3b} together may be, in particular, optionally substituted C₂-C₅-alkylene (e.g. 1,2-ethylene, 1,3-propylene, 1,4-butylene, or 1,5-pentylene).

Y¹ - in the pyrrolidine derivatives of formula (Il) - is, in particular, >CR⁶.

R⁶ - in the pyrrolidine derivatives of formula (Il) - is, in particular, hydrogen.

R^{5a}, R^{5b} - in the pyrrolidine derivatives of formula (Il) - are, in particular, hydrogen.

Particular embodiments of the pyrrolidine derivatives of formula (Il) result if:
- R¹: is an optionally substituted 5-membered heterocyclic ring as defined herein (e.g. 1,2-diazol-4-yl, 1-methyl-1,2-diazol-4-yl, 1-methyl-1,2-diazol-3-yl, 1-methyl-1,3-diazol-4-yl, 1-methyl-1,2-diazol-5-yl, 1,5-dimethyl-1,2-diazol-4-yl, 1,3-dimethyl-1,2-diazol-4-yl, 1,5-dimethyl-1,2-diazol-4-yl, 1,3-dimethyl-1,2-diazol-5-yl, 1,3-dimethyl-5-Cl-1,2-diazol-4-yl, 1-isopropyl-3-methyl-1,2-diazol-4-yl, 1-Me-3-CF₃-1,2-diazol-4-yl, 1-cyclopentyl-3-Me-1,2-diazol-4-yl, 1,3,5-trimethyl-1,2-diazol-4-yl, 1-CHF₂-3,5-dimethyl-1,2-diazol-4-yl, 1,2-dimethyl-1,3-diazol-4-yl, 1,2-dimethyl-1,3-diazol-5-yl, 1-methyl-5-Cl-1,3-diazol-4-yl, 1-Me-1,2,3-triazol-4-yl, a further example being 1-ethyl-1,3-diazol-4-yl or 1-methyl-1,2,4-triazol-3-yl);
- R^{2a}, R^{2b}: are hydrogen;
- R^{3a}: is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl);
- R^{3b}: is hydrogen;
- Y¹: is >CR⁶;
- R⁶: is hydrogen;
- R^{20a}: is C₁-C₈-alkyl (e.g. n-propyl, n-butyl, hexyl, pentyl, or 6-Me-heptyl), (C₃-C₁₂-cycloalkyl)-C₁-C₄-alkyl (e.g. cyclopropyl-methyl), C₁-C₆-alkoxy-C₁-C₆-alkyl (e.g. 2-methoxy-ethyl, 2-ethoxy-ethyl, 2-isopropyloxy-ethyl, 1-methyl-2-methoxy-ethyl, 3-methoxy-propyl, 3-ethoxy-propyl, or 3-isopropyloxy-propyl), (optionally substituted C₆-C₁₂-aryl)-C₁-C₄-alkyl (e.g. benzyl, (4-F-phenyl)methyl, (3-Cl -phenyl)methyl, (4-Cl -phenyl)methyl, (3,4-diF-phenyl)methyl, (3-Cl-4-F-phenyl)methyl, (3-CF₃-phenyl)methyl, (2-Cl-4-CF₃-phenyl)methyl, (2-Cl-5-CF₃-phenyl)methyl, or (3-OCF₃-phenyl)methyl)), (optionally substituted C₃-C₁₂-heterocyclyl)-C₁-C₄-alkyl (e.g. (pyrid-2-yl)-methyl or (4-CF₃-pyrid-2-yl)methyl), optionally substituted C₆-C₁₂-aryl (e.g. phenyl, 4-F-phenyl, 3-Cl-phenyl, 3,5-diCl-phenyl, 3-Cl-4-F-phenyl, 3-Me-phenyl, 3-CF₃-phenyl, 4-CF₃-phenyl, 2-Cl-3-CF₃-phenyl, 3-Cl-4-CF₃-phenyl, 3-CF₃-4-F-phenyl, 3-CN-phenyl, 3-OCF₃-phenyl, 3-OCF₃-4-F-phenyl, or 3-OCF₃-4-Cl-phenyl), or optionally substituted C₃-C₁₂-heterocyclyl (e.g. 4-CF₃-pyrid-2-yl);
- R^{20b}: is hydrogen or C₁-C₈-alkyl (e.g. methyl, ethyl, or n-butyl); and
- R^{5a}, R^{5b}: are hydrogen.

Additional particular embodiments of the pyrrolidine derivatives of formula (Il) result if
- R^{3a} and R^{3b}: together are optionally substituted C₂-C₅-alkylene (e.g. 1,2-ethylene, 1,3-propylene, 1,4-butylene, or 1,5-pentylene); and
- R¹, R^{2a}, R^{2b}, Y¹, R⁶, R^{20a}, R^{20b}, R^{5a} and R^{5b}: are as defined above.

Preferably, R¹ - in the pyrrolidine derivatives of formula (Il) - is an optionally substituted 5-membered heterocyclic ring as defined herein containing 2 N which, in particular, is optionally substituted 1,3-diazolyl.

According to a further preferred embodiment, R¹ - in the pyrrolidine derivatives of formula (Il) - is an optionally substituted 5-membered heterocyclic ring as defined herein containing 3 N which, in particular, is optionally substituted 1,2,3-triazolyl.

In connection with R¹ and the pyrrolidine derivatives of formula (Il), substituted 5-membered heterocyclic ring in particular includes pyrazolyl, imidazolyl, and triazolyl, which are substituted with 1, 2 or 3 substituents independently selected from the group consisting of halogen, C₁-C₆-alkyl, halogenated C₁-C₆-alkyl, C₃-C₆-cycloalkyl, C₁-C₄-alkoxy-carbonyl and C₁-C₆-alkylcarbonylamino. Preferably, the substituent(s) on the heterocyclic ring are independently selected from the group consisting of halogen and C₁-C₆-alkyl. In particular, the substituent(s) on the heterocyclic ring are independently selected from the group consisting of C₁-C₆-alkyl, especially C₁-C₄-alkyl (e.g. methyl). According to a particular embodiment, 1,3-diazolyl is substituted with halogen or C₁-C₆-alkyl as described herein. According to a specific embodiment, R¹ is 1-methyl-1,3-diazol-4-yl.

In connection with R^{3a} and the pyrrolidine derivatives of formula (II), substituted C₆-C₁₂-aryl in particular includes C₆-C₁₂-aryl, such as phenyl or naphthyl, which is substituted with 1, 2, or 3 substituents independently selected from the group consisting of halogen, C₁-C₄-alkyl and C₁-C₄-alkoxy. Preferably, the substituent(s) on C₆-C₁₂-aryl are independently selected from the group consisting of halogen.

According to a preferred embodiment, R^{3a} - in the pyrrolidine derivatives of formula (Il) - is C₆-C₁₂-aryl, in particular phenyl, optionally substituted with 1, 2, or 3 substituents independently selected from the group consisting of halogen (e.g. 4-F-phenyl).

According to an additional preferred embodiment, R^{3a} and R^{3b} - in the pyrrolidine derivatives of formula (Il) - together may be C₂-C₅-alkylene (e.g. 1,5-pentylene).

In connection with R^{20a}, substituted C₆-C₁₂-aryl-C₁-C₄-alkyl in particular includes C₆-C₁₂-aryl-C₁-C₄-alkyl, such as benzyl, which is substituted with 1, 2, or 3 substituents independently selected from the group consisting of halogen, halogenated C₁-C₄-alkyl and halogenated C₁-C₄-alkoxy.

In connection with R^{20a}, substituted C₃-C₁₂-heterocyclyl-C₁-C₄-alkyl in particular includes C₃-C₁₂-heterocyclyl-C₁-C₄-alkyl, such as pyridyl-methyl, which is substituted with 1, 2, or 3 substituents independently selected from the group consisting of halogenated C₁-C₄-alkyl.

In connection with R^{20a}, substituted C₆-C₁₂-aryl in particular includes C₆-C₁₂-aryl, such as phenyl, which is substituted with 1, 2, or 3 substituents independently selected from the group consisting of halogen, C₁-C₄-alkyl, halogenated C₁-C₄-alkyl, CN and halogenated C₁-C₄-alkoxy.

In connection with R^{20a}, substituted C₃-C₂-heterocyclyl in particular includes C₃-C₂-heterocyclyl, such as pyridyl, which is substituted with 1, 2, or 3 substituents independently selected from the group consisting of halogenated C₁-C₄-alkyl.

According to a preferred embodiment, R^{20a} is C₁-C₈-alkyl (e.g. n-propyl n-butyl, hexyl, pentyl, or 6-Me-heptyl), C₃-C₁₂-cycloalkyl-C₁-C₄-alkyl (e.g. cyclopropyl-methyl), C₁-C₆-alkoxy-C₁-C₆-alkyl (e.g. 2-methoxy-ethyl, 2-ethoxy-ethyl, 2-isopropyloxy-ethyl, 1-methyl-2-methoxy-ethyl, 3-methoxy-propyl, 3-ethoxy-propyl, or 3-isopropyloxy-propyl), C₆-C₁₂-aryl-C₁-C₄-alkyl wherein C₆-C₁₂-aryl is optionally substituted with 1, 2, or 3 substituents independently selected from the group consisting of halogen, halogenated C₁-C₄-alkyl and halogenated C₁-C₄-alkoxy (e.g. benzyl, (4-F-phenyl)methyl, (3-Cl -phenyl)methyl, (4-Cl -phenyl)methyl, (3,4-diF-phenyl)methyl, (3-Cl-4-F-phenyl)methyl, (3-CF₃-phenyl)methyl, (2-Cl-4-CF₃-phenyl)methyl, (2-Cl-5-CF₃-phenyl)methyl, or (3-OCF₃-phenyl)methyl)), or C₃-C₁₂-heterocyclyl-C₁-C₄-alkyl wherein C₃-C₁₂-heterocyclyl is optionally substituted with 1, 2, or 3 substituents independently selected from the group consisting of halogenated C₁-C₄-alkyl (e.g. (pyrid-2-yl)-methyl or (4-CF₃-pyrid-2-yl)methyl), or C₆-C₁₂-aryl optionally substituted with 1, 2, or 3 substituents independently selected from the group consisting of halogen, C₁-C₄-alkyl, halogenated C₁-C₄-alkyl, CN and halogenated C₁-C₄-alkoxy (e.g. phenyl, 4-F-phenyl, 3-Cl-phenyl, 3,5-diCl-phenyl, 3-Cl-4-F-phenyl, 3-Me-phenyl, 3-CF₃-phenyl, 4-CF₃-phenyl, 2-Cl-3-CF₃-phenyl, 3-Cl-4-CF₃-phenyl, 3-CF₃-4-F-phenyl, 3-CN-phenyl, 3-OCF₃-phenyl, 3-OCF₃-4-F-phenyl, or 3-OCF₃-4-Cl-phenyl), or C₃-C₁₂-heterocyclyl optionally substituted with 1, 2, or 3 substituents independently selected from the group consisting of halogenated C₁-C₄-alkyl (e.g. 4-CF₃-pyrid-2-yl).

Further preferred embodiments of the pyrrolidine derivatives of formula (Il) result if:
- R¹: is 1,3-diazolyl optionally substituted with halogen or C₁-C₄-alkyl (e.g. 1-methyl-1,3-diazol-4-yl);
- R^{2a}, R^{2b}: are hydrogen;
- R^{3a}: is C₆-C₁₂-aryl optionally substituted with 1, 2, or 3 substituents independently selected from the group consisting of halogen (e.g. 4-F-phenyl);
- R^{3b}: is hydrogen;
- Y¹: is >CR⁶;
- R⁶: is hydrogen;
- R^{20a}: is C₁-C₈-alkyl (e.g. n-propyl, n-butyl, hexyl, pentyl, or 6-Me-heptyl), C₃-C₂-cycloalkyl-C₁-C₄-alkyl (e.g. cyclopropyl-methyl), C₁-C₆-alkoxy-C₁-C₆-alkyl (e.g. 2-methoxy-ethyl, 2-ethoxy-ethyl, 2-isopropyloxy-ethyl, 1-methyl-2-methoxy-ethyl, 3-methoxy-propyl, 3-ethoxy-propyl, or 3-isopropyloxy-propyl), C₆-C₁₂-aryl-C₁-C₄-alkyl wherein C₆-C₁₂-aryl is optionally substituted with 1, 2, or 3 substituents independently selected from the group consisting of halogen, halogenated C₁-C₄-alkyl and halogenated C₁-C₄-alkoxy (e.g. benzyl, (4-F-phenyl)methyl, (3-Cl -phenyl)methyl, (4-Cl -phenyl)methyl, (3,4-diF-phenyl)methyl, (3-Cl-4-F-phenyl)methyl, (3-CF₃-phenyl)methyl, (2-Cl-4-CF₃-phenyl)methyl, (2-Cl-5-CF₃-phenyl)methyl, or (3-OCF₃-phenyl)methyl)), or C₃-C₁₂-heterocyclyl-C₁-C₄-alkyl wherein C₃-C₁₂-heterocyclyl is optionally substituted with 1, 2, or 3 substituents independently selected from the group consisting of halogenated C₁-C₄-alkyl (e.g. (pyrid-2-yl)-methyl or (4-CF₃-pyrid-2-yl)methyl), or C₆-C₁₂-aryl optionally substituted with 1, 2, or 3 substituents independently selected from the group consisting of halogen, C₁-C₄-alkyl, halogenated C₁-C₄-alkyl, CN and halogenated C₁-C₄-alkoxy (e.g. phenyl, 4-F-phenyl, 3-Cl-phenyl, 3,5-diCl-phenyl, 3-Cl-4-F-phenyl, 3-Me-phenyl, 3-CF₃-phenyl, 4-CF₃-phenyl, 2-Cl-3-CF₃-phenyl, 3-Cl-4-CF₃-phenyl, 3-CF₃-4-F-phenyl, 3-CN-phenyl, 3-OCF₃-phenyl, 3-OCF₃-4-F-phenyl, or 3-OCF₃-4-Cl-phenyl), or C₃-C₁₂-heterocyclyl optionally substituted with 1, 2, or 3 substituents independently selected from the group consisting of halogenated C₁-C₄-alkyl (e.g. 4-CF₃-pyrid-2-yl);
- R^{20b}: is hydrogen or C₁-C₈-alkyl (e.g. n-butyl); and
- R^{5a}, R^{5b}: are hydrogen.

Additional preferred embodiments of the pyrrolidine derivatives of formula (Il) result if
- R^{3a} and R^{3b}: together are C₂-C₅-alkylene (e.g. 1,5-pentylene); and
- R¹, R^{2a}, R^{2b}, Y¹, R⁶, R^{20a}, R^{20b}, R^{5a} and R^{5b}: are as defined above.

According to a further embodiment, R⁴ is -SO₂R²¹. Thus, the present invention relates to the pyrrolidine derivatives of the formula (Im): 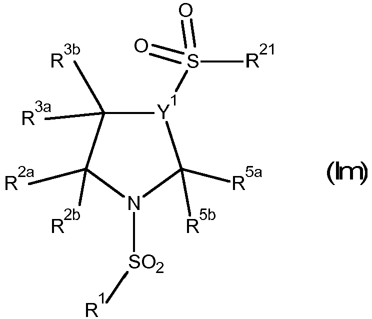 wherein R¹, R^{2a}, R^{2b}, R^{3a}, R^{3b}, Y¹, R^{5a} and R^{5b} are as defined herein, and
- R²¹: is optionally substituted C₆-C₁₂-aryl (e.g. phenyl, 2-Cl-phenyl, or 4-Me-phenyl), or optionally substituted C₃-C₂-heterocyclyl (e.g. 2-pyridyl).

In particular, R²¹ is C₆-C₁₂-aryl (e.g. phenyl).

R^{2a}, R^{2b} - in the pyrrolidine derivatives of formula (Im) - are, in particular, hydrogen.

R^{3a} - in the pyrrolidine derivatives of formula (Im) - is, in particular, C₃-C₂-cycloalkyl (e.g. cyclopropyl, cyclobutyl, cyclopentyl, or cyclohexyl), optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl), C₃-C₁₂-cycloalkyl-C₁-C₄-alkoxy (e.g. cyclopropylmethoxy or cyclohexylmethoxy), C₂-C₆-alkenyloxy (e.g. allyloxy or 2-methylprop-2-en-1-oxy), C₆-C₁₂-aryl-C₁-C₄-alkoxy (e.g. benzyloxy), optionally substituted C₆-C₁₂-aryloxy (e.g. phenoxy or 4-F-phenoxy), or C₃-C₁₂-heterocyclyl (e.g.pyrid-2-yl). Preferably, R^{3a} is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl).

R^{3b} - in the pyrrolidine derivatives of formula (Im) - is, in particular, hydrogen.

Y¹ - in the pyrrolidine derivatives of formula (Im) - is, in particular, >CR⁶.

R⁶ - in the pyrrolidine derivatives of formula (Im) - is, in particular, hydrogen.

R^{5a}, R^{5b} - in the pyrrolidine derivatives of formula (Im) - are, in particular, hydrogen.

Particular embodiments of the pyrrolidine derivatives of formula (Im) result if:
- R¹: is an optionally substituted 5-membered heterocyclic ring as defined herein (e.g. 1,2-diazol-4-yl, 1-methyl-1,2-diazol-4-yl, 1-methyl-1,2-diazol-3-yl, 1-methyl-1,3-diazol-4-yl, 1-methyl-1,2-diazol-5-yl, 1,5-dimethyl-1,2-diazol-4-yl, 1,3-dimethyl-1,2-diazol-4-yl, 1,5-dimethyl-1,2-diazol-4-yl, 1,3-dimethyl-1,2-diazol-5-yl, 1,3-dimethyl-5-Cl-1,2-diazol-4-yl, 1-isopropyl-3-methyl-1,2-diazol-4-yl, 1-Me-3-CF₃-1,2-diazol-4-yl, 1-cyclopentyl-3-Me-1,2-diazol-4-yl, 1,3,5-trimethyl-1,2-diazol-4-yl, 1-CHF₂-3,5-dimethyl-1,2-diazol-4-yl, 1,2-dimethyl-1,3-diazol-4-yl, 1,2-dimethyl-1,3-diazol-5-yl, 1-methyl-5-Cl-1,3-diazol-4-yl, 1-Me-1,2,3-triazol-4-yl, a further example being 1-ethyl-1,3-diazol-4-yl or 1-methyl-1,2,4-triazol-3-yl);
- R^{2a}, R^{2b}: are hydrogen;
- R^{3a}: is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl);
- R^{3b}: is hydrogen;
- Y¹: is >CR⁶;
- R⁶: is hydrogen;
- R^{5a}, R^{5b}: are hydrogen; and
- R²¹: is C₆-C₁₂-aryl (e.g. phenyl).

Preferably, R¹ - in the pyrrolidine derivatives of formula (Im) - is an optionally substituted 5-membered heterocyclic ring as defined herein containing 2 N which, in particular, is optionally substituted 1,3-diazolyl.

According to a further preferred embodiment, R¹ - in the pyrrolidine derivatives of formula (Im) - is an optionally substituted 5-membered heterocyclic ring as defined herein containing 3 N which, in particular, is optionally substituted 1,2,3-triazolyl.

In connection with R¹ and the pyrrolidine derivatives of formula (Im), substituted 5-membered heterocyclic ring in particular includes pyrazolyl, imidazolyl, and triazolyl, which are substituted with 1, 2 or 3 substituents independently selected from the group consisting of halogen, C₁-C₆-alkyl, halogenated C₁-C₆-alkyl, C₃-C₆-cycloalkyl, C₁-C₄-alkoxy-carbonyl and C₁-C₆-alkylcarbonylamino. Preferably, the substituent(s) on the heterocyclic ring are independently selected from the group consisting of halogen and C₁-C₆-alkyl. In particular, the substituent(s) on the heterocyclic ring are independently selected from the group consisting of C₁-C₆-alkyl, especially C₁-C₄-alkyl (e.g. methyl). According to a particular embodiment, 1,3-diazolyl is substituted with halogen or C₁-C₆-alkyl as described herein. According to a specific embodiment, R¹ is 1-methyl-1,3-diazol-4-yl.

According to a preferred embodiment, R^{3a} - in the pyrrolidine derivatives of formula (Im) - is C₆-C₁₂-aryl (e.g. phenyl).

Further preferred embodiments of the pyrrolidine derivatives of formula (Im) result if:
- R¹: is 1,3-diazolyl optionally substituted with halogen or C₁-C₄-alkyl (e.g. 1-methyl-1,3-diazol-4-yl);
- R^{2a}, R^{2b}: are hydrogen;
- R^{3a}: is C₆-C₁₂-aryl (e.g. phenyl);
- R^{3b}: is hydrogen;
- Y¹: is >CR⁶;
- R⁶: is hydrogen;
- R²¹: is C₆-C₁₂-aryl (e.g. phenyl); and
- R^{5a}, R^{5b}: are hydrogen.

According to a further embodiment R⁴ is optionally substituted C₃-C₂-heterocyclyl (e.g. 4-chloroisoindolin-1-one; 7-(trifluoromethyl)-3,4-dihydro-1H-quinazoln-2-on-1-yl, 4-butyl-1,2,3-triazol-1-yl, 1-propyl-1,2,3-triazol-4-yl, 5-(4-CF₃-phenyl)-4,5-dihydroisooxazol-3-yl, 5-(4-CF₃-phenyl)- oxazol-2-yl, or 5-(3-Cl-phenyl)-imidazol-2-yl, a further example being 5-butyl-oxazolidin-2-on-3-yl, 1,4-thiazinan-1,1-dioxide-4-yl, indolinyl, indolin-2-on-1-yl, 6-CF₃-indolin-2-on-1-yl, isoindolinyl, or isoindolin-1-on-2-yl).

R^{3a} - in the pyrrolidine derivatives wherein R⁴ is optionally substituted C₃-C₂-heterocyclyl - is, in particular, C₃-C₂-cycloalkyl (e.g. cyclopropyl, cyclobutyl, cyclopentyl, or cyclohexyl), optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl), C₃-C₁₂-cycloalkyl-C₁-C₄-alkoxy (e.g. cyclopropylmethoxy or cyclohexylmethoxy), C₂-C₆-alkenyloxy (e.g. allyloxy or 2-methylprop-2-en-1-oxy), C₆-C₁₂-aryl-C₁-C₄-alkoxy (e.g. benzyloxy), optionally substituted C₆-C₁₂-aryloxy (e.g. phenoxy or 4-F-phenoxy), or C₃-C₁₂-heterocyclyl (e.g.pyrid-2-yl). Preferably, R^{3a} is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl) or C₃-C₁₂-heterocyclyl (e.g.pyrid-2-yl).

R^{3b} - in the pyrrolidine derivatives wherein R⁴ is optionally substituted C₃-C₂-heterocyclyl - is, in particular, hydrogen.

Y¹ - in the pyrrolidine derivatives wherein R⁴ is optionally substituted C₃-C₂-heterocyclyl - is, in particular, >CR⁶.

R⁶ - in the pyrrolidine derivatives wherein R⁴ is optionally substituted C₃-C₂-heterocyclyl - is, in particular, hydrogen.

R^{5a}, R^{5b} - in the pyrrolidine derivatives wherein R⁴ is optionally substituted C₃-C₂-heterocyclyl - are, in particular, hydrogen.

Particular embodiments of the pyrrolidine derivatives of the invention result if:
- R¹: is an optionally substituted 5-membered heterocyclic ring as defined herein (,e.g. 1,2-diazol-4-yl, 1-methyl-1,2-diazol-4-yl, 1-methyl-1,2-diazol-3-yl, 1-methyl-1,3-diazol-4-yl, 1-methyl-1,2-diazol-5-yl, 1,5-dimethyl-1,2-diazol-4-yl, 1,3-dimethyl-1,2-diazol-4-yl, 1,5-dimethyl-1,2-diazol-4-yl, 1,3-dimethyl-1,2-diazol-5-yl, 1,3-dimethyl-5-Cl-1,2-diazol-4-yl, 1-isopropyl-3-methyl-1,2-diazol-4-yl, 1-Me-3-CF₃-1,2-diazol-4-yl, 1-cyclopentyl-3-Me-1,2-diazol-4-yl, 1,3,5-trimethyl-1,2-diazol-4-yl, 1-CHF₂-3,5-dimethyl-1,2-diazol-4-yl, 1,2-dimethyl-1,3-diazol-4-yl, 1,2-dimethyl-1,3-diazol-5-yl, 1-methyl-5-Cl-1,3-diazol-4-yl, 1-Me-1,2,3-triazol-4-yl, a further example being 1-ethyl-1,3-diazol-4-yl or 1-methyl-1,2,4-triazol-3-yl);
- R^{2a}, R^{2b}: are hydrogen;
- R^{3a}: is optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl) or C₃-C₁₂-heterocyclyl (e.g.pyrid-2-yl);
- R^{3b}: is hydrogen;
- R⁴: is optionally substituted C₃-C₁₂-heterocyclyl (e.g. 4-chloroisoindolin-1-one; 7-(trifluoromethyl)-3,4-dihydro-1H-quinazolin-2-on-1-yl, 4-butyl-1,2,3-triazol-1-yl, 1-propyl-1,2,3-triazol-4-yl, 5-(4-CF₃-phenyl)-4,5-dihydroisooxazol-3-yl, 5-(4-CF₃-phenyl)-oxazol-2-yl, or 5-(3-Cl-phenyl)-imidazol-2-yl, a further example being 5-butyl-oxazolidin-2-on-3-yl, 1,4-thiazinane-1,1-dioxide, indolinyl, indolin-2-on-1-yl, 6-CF₃-indolin-2-on-1-yl, isoindolinyl, or isoindolin-1-on-2-yl);
- Y¹: is >CR⁶;
- R⁶: is hydrogen; and
- R^{5a}, R^{5b}: are hydrogen.

Preferably, R¹ - in the pyrrolidine derivatives wherein R⁴ is optionally substituted C₃-C₁₂-heterocyclyl - is an optionally substituted 5-membered heterocyclic ring as defined herein containing 2 N which, in particular, is optionally substituted 1,3-diazolyl.

According to a further preferred embodiment, R¹ - in the pyrrolidine derivatives wherein R⁴ is optionally substituted C₃-C₁₂-heterocyclyl - is an optionally substituted 5-membered heterocyclic ring as defined herein containing 3 N which, in particular, is optionally substituted 1,2,3-triazolyl.

In connection with R¹ and the pyrrolidine derivatives wherein R⁴ is optionally substituted C₃-C₁₂-heterocyclyl, substituted 5-membered heterocyclic ring in particular includes pyrazolyl, imidazolyl, and triazolyl, which are substituted with 1, 2 or 3 substituents independently selected from the group consisting of halogen, C₁-C₆-alkyl, halogenated C₁-C₆-alkyl, C₃-C₆-cycloalkyl, C₁-C₄-alkoxy-carbonyl and C₁-C₆-alkyl-carbonylamino. Preferably, the substituent(s) on the heterocyclic ring are independently selected from the group consisting of halogen and C₁-C₆-alkyl. In particular, the substituent(s) on the heterocyclic ring are independently selected from the group consisting of C₁-C₆-alkyl, especially C₁-C₄-alkyl (e.g. methyl). According to a particular embodiment, 1,3-diazolyl is substituted with halogen or C₁-C₆-alkyl as described herein. According to a specific embodiment, R¹ is 1-methyl-1,3-diazol-4-yl.

In connection with R^{3a} and and the pyrrolidine derivatives wherein R⁴ is optionally substituted C₃-C₁₂-heterocyclyl, substituted C₆-C₁₂-aryl in particular includes C₆-C₁₂-aryl, such as phenyl or naphthyl, which is substituted with 1, 2, or 3 substituents independently selected from the group consisting of halogen, C₁-C₄-alkyl and C₁-C₄-alkoxy. Preferably, the substituent(s) on C₆-C₁₂-aryl are independently selected from the group consisting of halogen.

In connection with R^{3a} and and the pyrrolidine derivatives wherein R⁴ is optionally substituted C₃-C₁₂-heterocyclyl, substituted C₃-C₁₂-heterocyclyl in particular includes C₃-C₁₂-heterocyclyl, such as pyridyl, which is substituted with 1, 2, or 3 substituents independently selected from the group consisting of halogen, C₁-C₄-alkyl and halogenated C₁-C₄-alkyl.

According to a preferred embodiment, R^{3a} - in the pyrrolidine derivatives wherein R⁴ is optionally substituted C₃-C₁₂-heterocyclyl - is C₆-C₁₂-aryl, in particular phenyl, optionally substituted with 1, 2, or 3 substituents independently selected from the group consisting of halogen (e.g. phenyl, 4-F-phenyl), or C₃-C₁₂-heterocyclyl (e.g. pyrid-2-yl).

In connection with R⁴, substituted C₃-C₁₂-heterocyclyl in particular includes C₃-C₁₂-heterocyclyl, such as triazolyl or isoindolinonyl, and additionally such as oxazodinonyl, isoindolinyl, indolinyl, indolinoyl, or thiazinanyl-1,1-dioxide, which is substituted with 1, 2, or 3 substituents independently selected from the group consisting of halogen, C₁-C₄-alkyl and halogenated C₁-C₄-alkyl.

According to a preferred embodiment, R⁴ - in the pyrrolidine derivatives wherein R⁴ is optionally substituted C₃-C₁₂-heterocyclyl - is C₃-C₂-heterocyclyl substituted with 1, 2, or 3 substituents independently selected from the group consisting of halogen, C₁-C₆-alkyl and halogenated C₁-C₄-alkyl (e.g. 4-chloroisoindolin-1-onyl, 7-(trifluoromethyl)-3,4-dihydro-1H-quinazolin-2-on-1-yl, 4-butyl-1,2,3-triazol-1-yl, or 1-propyl-1,2,3-triazol-4-yl, a further example being 5-butyl-oxazolidin-2-on-3-yl, 1,4-thiazinane-1,1-dioxide, indolinyl, indolin-2-on-1-yl, 6-CF₃-indolin-2-on-1-yl, isoindolinyl, or isoindolin-1-on-2-yl).

Further preferred embodiments of the pyrrolidine derivatives wherein R⁴ is optionally substituted C₃-C₁₂-heterocyclyl result if:
- R¹: is 1,3-diazolyl optionally substituted with halogen or C₁-C₄-alkyl (e.g. 1-methyl-1,3-diazol-4-yl);
- R^{2a}, R^{2b}: are hydrogen;
- R^{3a}: is C₆-C₁₂-aryl optionally substituted with 1, 2, or 3 substituents independently selected from the group consisting of halogen, or C₃-C₁₂-heterocyclyl (e.g. pyrid-2-yl);
- R^{3b}: is hydrogen;
- R⁴: is C₃-C₁₂-heterocyclyl substituted with 1, 2, or 3 substituents independently selected from the group consisting of halogen, C₁-C₆-alkyl and halogenated C₁-C₄-alkyl (e.g. 4-chloroisoindolin-1-one; 7-(trifluoromethyl)-3,4-dihydro-1H-quinazolin-2-on-1-yl, 4-butyl-1,2,3-triazol-1-yl, 1-propyl-1,2,3-triazol-4-yl, 5-(4-CF₃-phenyl)-4,5-dihydroisooxazol-3-yl, 5-(4-CF₃-phenyl)-oxazol-2-yl, or 5-(3-Cl-phenyl)-imidazol-2-yl, a further example being 5-butyl-oxazolidin-2-on-3-yl, 1,4-thiazinane-1,1-dioxide, indolinyl, indolin-2-on-1-yl, 6-CF₃-indolin-2-on-1-yl, isoindolinyl, or isoindolin-1-on-2-yl);
- Y¹: is >CR⁶;
- R⁶: is hydrogen; and
- R^{5a}, R^{5b}: are hydrogen.

Particular embodiments of pyrrolidine derivatives of the invention result if
- R¹: is optionally substituted 5-membered heterocyclic ring as defined herein (e.g. 1-methyl-1,3-diazol-4-yl or 1-methyl-1,2,3-triazol-4-yl);
- R^{2a}, R^{2b}: are hydrogen;
- R^{3a}: is C₃-C₁₂-cycloalkyl (cyclopropyl, cyclobutyl, cyclopentyl, or cyclohexyl), optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl, a further example being 2-Me-phenyl, 3-Me-phenyl, or 4-Me-phenyl), hydroxy, C₁-C₆-alkoxy (e.g.methoxy, ethoxy, n-propyloxy, iso-propyxy, or iso-butoxy), C₃-C₁₂-cycloalkyl-C₁-C₄-alkoxy (cyclopropylmethoxy, cyclohexylmethoxy), C₂-C₆-alkenyloxy (e.g. allyloxy, 2-methylprop-2-en-1-oxy), C₆-C₁₂-aryl-C₁-C₄-alkoxy (e.g. benzyloxy), optionally substituted C₆-C₁₂-aryloxy (e.g. phenoxy, 4-F-phenoxy), or optionally substituted C₃-C₂-heterocyclyl (tetrahydrofuran-2-yl, tetrahydrofuran-3-yl, tetrahydropyran-2-yl, tetrahydropyran-3-yl, 1-cyclopropyl-piperidin-4-yl, 1-cyclopropyl-piperidin-3-yl, 2-pyridyl, 3-pyridyl, 3-F-pyrid-2-yl, 1,3-oxazol-4-yl, 1,3-oxazol-2-yl, 3-F-azetidin-1-yl, morpholin-4-yl, pyrrolidin-1-yl, piperidin-1-yl, 2-Me-piperidin-1yl, 3-Me-piperidin-1yl, 4-Me-piperidin-1yl, 4-F-piperidin-1-yl, 4,4-diF-piperidin-lyl, or azepan-1-yl, a further example being 1,3-dioxan-2-yl, 5,5-dimethyl-1,3-dioxan-2-yl, 4,6-dimethyl-1,3-dioxan-2-yl, 1,3-dioxepan-2-yl, 1,3-dioxolan-2-yl, 5,7-dioxaspiro[2.5]octan-6-yl, morpholin-3-yl, 1-Me-piperidin-2-yl, or 5-F-pyrid-2-yl); or
- R^{3a} and one of R^{2a} or R^{2b}: together with the carbon atoms to which they are bound may form an anellated C₆-C₁₂-aryl (e.g. phenyl);
- R^{3b}: is hydrogen or hydroxy, or
- R^{3a} and R^{3b}: together are C₂-C₅-alkylene (e.g. 1,5-pentylene);
- Y¹: is >CR⁶- or >N-;
- R⁶: is hydrogen, C₁-C₆-alkyl (e.g. methyl or ethyl), C₆-C₁₂-aryl-C₁-C₄-alkyl (e.g. benzyl or phenethyl), hydroxy-C₁-C₆-alkyl (e.g. -CH₂OH, or -(CH₂)₂OH), or hydroxy, or
- R⁶ and R^{3a} or R^{3b}: together are C₁-C₅-alkylene (e.g. 1,4-butylene), or
- R⁶: is C₁-C₄-alkylene (e.g. methylene or 1,2-ethylene) that is bound to a carbon atom in R^{3a}, and
R^{3a} is an optionally substituted C₆-C₁₂-aryl (e.g. phenyl);
- R⁴: is -(CR^{7a}R^{7b})ₙ₁OR¹⁰, -(CR^{7c}R^{7d})ₙ₂NR^{11a}R^{11b}, -(CR^{7e}R^{7f})ₙ₃R¹², optionally substituted C₆-C₁₂-aryl, -NR^{8a}(CR^{9a}R^{9b})ₙ₄R¹³, -NR^{8b}COR¹⁴, -NR^{8c}COOR¹⁵, -NR^{8d}CONR^{16a}R^{16b}, - O(CR^{9c}R^{9d})ₙ₅R¹⁸, -COR¹⁹, -CONR^{20a}R^{20b}, -SO₂R²¹, or optionally substituted C₃-C₁₂-heterocyclyl (e.g. 1-propyl-1,2,3-triazol-4-yl, 4-butyl-1,2,3-triazolyl-1-yl, 4-chloroisoindolin-1-one, or 7-(trifluoromethyl)-3,4-dihydro-1H-quinazolin-2-on-1-yl, a further example being 5-butyl-oxazolidin-2-on-3-yl, 1,4-thiazinane-1,1-dioxide, indolinyl, in-R^{7a}, R^{7b} dolin-2-on-1-yl, 6-CF₃-indolin-2-on-1-yl, isoindolinyl, or isoindolin-1-on-2-yl); are hydrogen, or
- n1: is 1;
- R¹⁰: is hydrogen, optionally substituted C₆-C₁₂-aryl (e. g. phenyl, 3-F-phenyl, 3-Me-phenyl, 3-CF₃-phenyl, 2-Cl-3-CF₃-phenyl, or 3-OCF₃-phenyl, a further example being 2-F-phenyl, 4-F-phenyl, 2-Cl-phenyl, 2,3-diF-phenyl, 2,4-diF-phenyl, 2,5-diF-phenyl, 4-Cl-phenyl, 2-Cl-4-F-phenyl, 4-Cl-3-F-phenyl, 2-Me-phenyl, 4-Me-phenyl, 2-CF₃-phenyl, 4-CF₃-phenyl, 2-Cl-4-CF₃-phenyl, 3-OCHF₂-phenyl, 2-OCF₃-phenyl, 4-OCF₃-phenyl, 3-OMe-phenyl, 2-OEt-phenyl, 2-cyclopentyl-phenyl, 2-cyclohexyl-phenyl, 3-ethynyl-phenyl, 2-CN-phenyl, 4-CN-phenyl, 2-F-4-CN-phenyl, 3-dimethylamino-phenyl, 4-(morpholin-4-yl)-phenyl, 2-pirrolidinyl-phenyl, 2-piperidin-phenyl, indan-5-yl, naphthyl, or tetralin-5-yl), or optionally substituted C₃-C₁₂-heterocyclyl (e.g. 6-CF₃-pyrimid-4-yl, 4-CF₃-pyrid-2-yl, or 2-CF₃-pyrid-4-yl, a further example being pyrid-2-yl, pyrid-3-yl, pyrid-4-yl, pyrazin-2-yl, pyridazin-3-yl, quinolin-6-yl, isoquinolin-5-yl, 1,2-bezoxazol-6-yl, or 2-Me-1,3-benzoxazol-5-y);
- R^{7c}, R^{7d}: are hydrogen, or
- n2: is 1;
- R^{11a}: is C₁-C₈-alkyl (e.g. methyl, ethyl, n-propyl, n-butyl, or n-heptyl), C₃-C₁₂-cycloalkyl-C₁-C₄-alkyl (e.g. cyclopropyl-methyl) C₁-C₆-alkoxy-C₁-C₆-alkyl (2-methoxy-ethyl, 2-ethoxy-ethyl, 2-isopropyloxy-ethyl, 1-methyl-2-methoxy-ethyl, 3-methoxy-propyl, 3-ethoxy-propyl, or 3-isopropyloxy-propyl), C₆-C₁₂-aryl-C₁-C₄-alkyl (e.g. benzyl), optionally substituted C₆-C₁₂-aryl (e.g. phenyl, 4-F-phenyl, 3-Cl-phenyl, 3,5-diCl-phenyl, 3-Cl-4-F-phenyl, 3-Me-phenyl, 3-(aminomethyl)-phenyl, 3-CF₃-phenyl, 4-CF₃-phenyl, 2-Cl-4-CF₃-phenyl, 3-Cl-4-CF₃-phenyl, 3-CF₃-4-F-phenyl, 3-OCF₃-phenyl, 3-OCF₃-4-F-phenyl, 3-OCF₃-4-Cl-phenyl, or 3-(aminocarbonyl)-phenyl);
- R^{11b}: is hydrogen;
- R^{7e}, R^{7f}: are hydrogen, or
- n3: is 1;
- R¹²: is optionally substituted C₆-C₁₂-aryl (e.g. phenyl, 3-Cl-phenyl, or 3-Br-phenyl, a further example being 3-CF₃-phenyl) or optionally substituted C₃-C₁₂-heterocyclyl (e.g. 4-CF₃-pyrid-2-yl or 1-propyl-1,2,3-triazol-4-yl);
- R^{8a}, R^{8b}, R^{8c}, R^{8d}, R^{8e}: are independently hydrogen or C₁-C₆-alkyl (e.g. pentyl or hexyl), or
- R⁶ and one of R^{8a}, R^{8b}, R^{8c}, R^{8d}, or R^{8e}: together are optionally substituted C₁-C₅-alkylene, wherein one or more -CH₂- of C₁-C₅-alkylene may be independently replaced by a an oxygen atom or C=O, or
- R^{3a} and one of R^{8a} or R^{8b}: together are C₁-C₅-alkylene (e.g. 1,3-propylene);
- R^{9a}, R^{9b}: are independently hydrogen, halogen, C₁-C₆-alkyl (e.g. methyl, ethyl, tert-butyl, 2,3-dimethyl-propyl), halogenated C₁-C₆-alkyl (e.g. trifluoro methyl), or C₁-C₆-alkoxy (e.g. methoxy or ethoxy);
- n4: is 0, 1, 2, 3, or 4;
- R¹³: is hydrogen, C₁-C₈-alkyl (e.g. methyl, ethyl, n-butyl, tert-butyl, pentyl, orhexyl), halogenated C₁-C₆-alkyl (e.g. -CF₃ or -CF₂Me), C₁-C₆-alkoxy-C₁-C₆-alkyl (e.g. methoxy-methyl), optionally substituted C₃-C₂-cycloalkyl (e.g. cyclopropyl, 2-propyl-cyclopropyl, 1-(methoxy-methyl)-cyclopropyl, 2-phenyl-cyclopropyl, cyclopentyl, 1-methyl-cyclohexyl, 1-CF₃-cyclopropyl, 4-CF₃-cyclohexyl, or 4,4-diF-cyclohexyl, a further example being 1-phenyl-cyclopropyl, 1-(4-F-phenyl)-cyclopropyl, (3-Cl-phenyl)-cyclopropyl, 2-phenyl-cyclobutyl, 3-phenyl-cyclobutyl, 3-(4-F-phenyl)-cyclobutyl, 3-(2-F-phenyl)-cyclobutyl, 2-Cl-cyclopentyl, 2-Me-cyclopentyl, 3-Me-cyclopentyl, 2-phenyl-cyclopentyl, 3-(2-pyridyl)-cyclopentyl, 3-Me-cyclohexyl, 3,3,5,5-tetraMe-cyclohexyl, 4-Me-cyclohexyl, 4-Et-cyclohexyl, 2-CF₃-cyclohexyl, 2-Me-5-isopropenyl-cyclohexyl, 2-phenyl-cyclohexyl, 3-phenyl-cyclohexyl, 4-phenyl-cyclohexyl, 2-EtO-cyclohexyl, decalin-1-yl, decalin-2-yl, norbornan-2-yl, 1,7,7-trimethyl-norbornan-2-yl, 5-iPr-2-Me-3-bicyclo[3.1.0]hexanyl, bicycle [3.2.1]octanyl, or 3-bicyclo[1.1.1]pentanyl), C₂-C₆-alkenyl (e.g. hex-2-enyl), optionally substituted C₃-C₆-cycloalkenyl (e.g. 1,3,3-trimethylcyclohexen-2-yl), optionally substituted C₆-C₁₂-aryl (e.g. phenyl, 2-F-phenyl, 4-F-phenyl, 3-F-phenyl, 2-Cl-phenyl, 3-Cl-phenyl, 4-Cl-phenyl, 2,3-diF-phenyl, 2,4-diF-phenyl, 2,5-diF-phenyl, 3,4-diF-phenyl, 2,3,4-triF-phenyl, 2,4-diCl-phenyl, 3,4-diCl-phenyl, 3-F-4-Cl-phenyl, 3-Cl-4-F-phenyl, 3-Cl-5-F-phenyl, 3,4-diF-5-Cl-phenyl, 2-Me-phenyl, 3-Me-phenyl, 4-Me-phenyl, 3-iPr-phenyl, 3-tBu-phenyl, 3-Me-4-F-phenyl, 3-Me-4-Cl-phenyl, 3-iPr-4 Cl-phenyl, 3-(1-OH-1-CF₃-Et)-phenyl, 3-CHF₂-4-F-phenyl, 2-CF₃-phenyl, 3-CF₃-phenyl, 4-CF₃-phenyl, 3-Cl-4-CF₃-phenyl, 2-F-3-CF₃-phenyl, 2-F-5-CF₃-phenyl, 3-F-5-CF₃-phenyl, 2-Cl-4-CF₃-phenyl, 2-Cl-3-CF₃-phenyl, 3-CF₃-4-F-phenyl, 3-CF₃-4-Cl-phenyl, 3-Me-4-CF₃-phenyl, 3-CF₃-4-Me-phenyl, 3,4-diCF₃-phenyl, 4-OMe-phenyl, 3-OiPr-phenyl, 3-CF₃-4-OMe-phenyl, 3-OCH₂CF₃-phenyl, 3-OCF₃-phenyl, 2-OCHF₂-5-Cl-phenyl, 3-OCF₃-4-F-phenyl, 3-OCF₃-4-Cl-phenyl, 3-OBn-phenyl, 3-OPh-phenyl, 3-CN-phenyl, 4-CN-phenyl, 2-F-3-CN-phenyl, 2-F-4-CN-phenyl, 3-F-4-CN-phenyl, 2-F-5-CN-phenyl, 3-F-5-CN-phenyl, 3-Cl-4-CN-phenyl, 2-Cl-5-CN-phenyl, 3-Cl-5-CN-phenyl, 3-CN-4-Cl-phenyl, 2-Me-3-CN-phenyl, 2-Me-5-CN-phenyl, 3-Me-5-CN-phenyl, 3-CF₃-4-CN-phenyl, 3-CN-4-OMe-phenyl, 3-CN-4-OCF₃-phenyl, 3-phenyl-phenyl, 3-MeSO₂-phenyl, 3-(piperidin-4-yl)-phenyl, 2-methylcarbonylamino-5-Cl-phenyl, 3-(pyrid-2-yl)-phenyl, 3-(pyrid-3-yl)-4 F-phenyl, 3-(pyrid-4-yl)-4 F-phenyl, 3-(pyrimid-5-yl)-4 F-phenyl, indan-5-yl, or tetralin-6-yl, a further example being 2,4-diF-3-Cl-phenyl, 3-Et-4-Cl-phenyl, 3-(2-methoxyethyl)-phenyl, 3-(2-OH-ethyl)-phenyl, 3-(1-OH-1-Me-ethyl)-phenyl, 3-CHF₂-phenyl, 3-CF₃-2,4-diF-phenyl, 3-(dimethylamino-methyl)-phenyl, 3-(morpholin-4-ylmethyl)-phenyl, 3-cyclopropyl-phenyl, 3-OEt-phenyl, 3-OPr-phenyl, 3-OtBu-phenyl, 3-(cyclopropylmethoxy)-phenyl, 3-(OMe-methoxy)-phenyl, 3-(2-dimethylamino-ethoxy)-phenyl, 3-CF₃-4-OH-phenyl, 3-OCH₂CHF₂-phenyl, 3-OCHF₂-phenyl, 3-OCHF₂-4-F-phenyl, 3-OCF₃-4-OMe-phenyl, 3-cyclopropoxy-phenyl, 3-methylcarbonyl-phenyl, 3-dimethylamino-phenyl, 3-(2-pyridyloxy)-phenyl, 3-(pyrimidin-2-yloxy)-phenyl, indanyl, in-dan-2-yl, 2-F-indanyl, 4-F-indanyl, 5-F-indanyl, 6-F-indanyl, 7-F-indanyl, 3-Me-indanyl, 4-Me-indanyl, 5-Me-indanyl, 6-Me-indanyl, 4-CF₃-indanyl, 5-CF₃-indanyl, 6-CF₃-indanyl, 3,3-dimethyl-indanyl, tetralin-2-yl, 7-F-tetralin-2-yl, 6-F-tetralin-2-yl, tetralinyl, 6-F-tetralinyl, 5-F-tetralinyl, or 7-F-tetralinyl), C₁-C₆-alkoxy (e.g.methoxy, ethoxy, or n-propoxy), C₁-C₆-alkoxy-C₁-C₄-alkoxy (e.g. 2-methoxy-ethoxy), optionally substituted C₆-C₁₂-aryloxy (e.g. 4-F-phenoxy or 4-tertbutyl-phenoxy), optionally substituted C₃-C₁₂-heterocyclyl (e.g. propylfuran-2-yl, 2-CF₃-furan-5-yl, 1,2-dimethyl-5-CN-pyrrol-3-yl, 2,3-diMe-thiophen-5-yl, 2-Methiophen-5-yl, 2-Cl-thiophen-5-yl, 3-Cl-thiophen-2-yl, 2,5-diCl-thiophen-3-yl, 2-tetrahydropyranyl-thiophen-5-yl, 1,3-thiazol-5-yl, 4-Me-1,3-thiazol-2-yl, 2-Me-1,3-thiazol-5-yl, 5-Me-1,3-thiazol-2-yl, 4-Me-1,3-thiazol-5-yl, 2-Me-1,3-thiazol-4-yl, 4-isopropyl-1,3-thiazol-2-yl, 2,4-diMe-1,3-thiazol-5-yl, 2-phenyl-4-Me-1,3-thiazol-5-yl, 4-phenyl-1,3-thiazol-5-yl, 2-(4-Me-phenyl)-1,3-thiazol-5-yl, 4-(4-F-phenyl)-1,3-thiazol-2-yl, 2-Cl-1,3-thiazol-4-yl, 4-Cl-1,3-thiazol-5-yl, 2-Br-1,3-thiazol-5-yl, 4-Br-1,3-thiazol-2-yl, 4-Me-5-Br-1,3-thiazol-2-yl, 2,4-dichloro-1,3-thiazol-5-yl, 1,5-dimethyl-1,2,4-triazol-3-yl, pyrid-2-yl, pyrid-3-yl, pyrid-4-yl, 4-F-pyrid-2-yl, 5-F-pyrid-2-yl, 6-F-pyrid-2-yl, 4-Cl-pyrid-2-yl, 5-F-pyrid-3-yl, 2-F-pyrid-4-yl, 3,5-diCl-pyrid-4-yl, 4,5-diCl-pyrid-2-yl, 2-Cl-3F-pyrid-4-yl, 5-Me-pyrid-2-yl, 4-iPr-pyrid-2-yl, 4-Me-5-F-pyrid-2-yl, 4-CF₃-pyrid-2-yl, 5-CF₃-pyrid-2-yl, 6-CF₃-pyrid-2-yl, 5-CF₃-pyrid-3-yl, 2-CF₃-pyrid-4-yl, 3-F-4-CF₃-pyrid-2-yl, 4-CF₃-5-F-pyrid-2-yl, 4-CF₃-5-Cl-pyrid-2-yl, 3,5-diF-4-CF₃-pyrid-2-yl, 4-OCH₂CF₃-pyrid-2-yl, 4-OCH₂CF₃-5-F-pyrid-2-yl, 4-OCH₂CF₃-5-Cl-pyrid-2-yl, 4-OBn-5-F-pyrid-2-yl, 3-(4-F-phenyl)-pyrid-5-yl, 2-(4-F-phenyl)-pyrid-3-yl, 4-(pyrid-4-yl)-pyrid-2-yl, 4-(pyrid-3-yl)-pyrid-2-yl, 5-cyclopropyl-pyraz-2-yl, 5-cyclobutyl-pyraz-2-yl, 5-pyrrolidin-pyraz-2-yl, pyridaz-3-yl, 4-CF₃-pyridaz-3-yl, 5-CF₃-pyridaz-3-yl, 5-F-pyrimid-2-yl, 6-Cl-pyrimid-4-yl, 6-Me-pyrimid-4-yl, 6-Et-pyrimid-4-yl, 6-Pr-pyrimid-4-yl, 6-iPr-pyrimid-4-yl, 4-CF₃-pyrimid-2-yl, 6-CF₃-pyrimid-4-yl, 2-Me-6-Cl-pyrimid-4-yl, 2-Me-6-CF₃-pyrimid-4-yl, 2-OMe-6-CF₃-pyrimid-4-yl, 2-OMe-pyrimid-4-yl, 6-OMe-pyrimid-4-yl, 6-OEt-pyrimid-4-yl, 6-OPr-pyrimid-4-yl, 6-OiPr-pyrimid-4-yl, 6-OiBu-pyrimid-4-yl, 6-OBu-pyrimid-4-yl, 6-OBn-pyrimid-4-yl, 6-cyclobutylloxy-pyrimid-4-yl, 6-cyclopentyloxy-pyrimid-4-yl, 6-cyclohexyloxy-pyrimid-4-yl, 6-cyclopropyl-pyrimid-4-yl, 6-phenyl-pyrimid-4-yl, 6-(2-F- phenyl)-pyrimid-4-yl, 6-(3-F-phenyl)-pyrimid-4-yl, 6-(4-F- phenyl)-pyrimid-4-yl, 6-Cl-pyrimid-4-yl, 6-(2-Me- phenyl)-pyrimid-4-yl, 6-(3-Me- phenyl)-pyrimid-4-yl, 6-(4-Me- phenyl)-pyrimid-4-yl, 6-(3-Cl- phenyl)-pyrimid-4-yl, 6-(4-Cl- phenyl)-pyrimid-4-yl, 2-(morpholin-1-yl)-pyrimid-4-yl, 6-(azetidin-3-yl-methoxy)-pyrimid-4-yl, 6-(pyrrolidin-3-yl-methoxy)-pyrimid-4-yl, 6-(pyrrolidin-2-yl-methoxy)-pyrimid-4-yl, benzofuran-5-yl, 2-Me-benzofuran-3-yl, 2-Et-benzofuran-3-yl, benzothiophen-5-yl, benzothiophen-6-yl, 5-Me-benzothiophen-2-yl, 5-F-benzothiophen-2-yl, 3-Cl-benzothiophen-2-yl, benzothiazol-2-yl, isoquinolin-6-yl, isoquinolin-7-yl, quinolin-6-yl, quinolin-7-yl, 4,5,6,7-tetrahydro-1,3-benzothiazol-2-yl, 2,3-dihydrobenzofuran-5-yl, 7H-pyrrolo[2,3-d]pyrimidin-4-yl, pyrazolo[1,5-a]pyridine-3-yl, pyrazolo[1,5-a]pyridine-7-yl, imidazo[1,2-a]pyridin-8-yl, 5-methyl-imidazo[1,2-a]pyridin-3-yl, 6-chloro-2-methyl-imidazo[1,2-a]pyridin-3-yl, 6-bromo-2-methyl-imidazo[1,2-a]pyridin-3-yl, thieno[2,3-b]pyridin-2-yl, N-benzyl-indolin-6-yl, indolinon-4-yl, chroman-6-yl, 4,4-dimethylchroman-6-yl, 4,4-dimethyl-1,3-dioxan-2-yl, tetrahydrofuran-3-yl, tetrahydropyran-4-yl, 4-Me-tetrahydropyran-4-yl, 2,2-diMe-tetrahydropyran-4-yl, 1-isopropyl-piperidin-4-yl, or 2-CF₃-piperazin-5-yl, a further example being iBu-pyrazol-4-yl, 1-CHF₂-5-Me-1,2-diazol-4-yl, 1-benzyl-5-F-1,2,4-triazol-3-yl, 2-Me-pyrid-4-yl, 2-iPr-pyrid-4-yl, 4-phenyl-pyrid-2yl, 2-cyclopropyl-pyrid-4-yl, 2-OMe-pyrid-4-yl, 2-OEt-pyrid-4-yl, 2-cyclopropylmethoxy-pyrid-4yl, 2-OiPr-pyrid-4-yl, 2-OCH₂CF₃-pyrid-4-yl, 2-cyclopropoxy-pyrid-4-yl, 2-cyclobutoxypyrid-4-yl, 4,5-diCl-pyrimidin-2yl, benzothiophen-3-yl, 2,2-diF-1,3-benzodioxol-5-yl, 5,6,7,8-tetrahydro-isoquinolin-5-yl, 5,6,7,8-tetrahydro-isoquinolin-8-yl, quinolin-8-yl, 1-Me-3,4-dihydro-2H-quinolin-4-yl, 2-Me-3,4-dihydro-1H-isoquinolin-6-yl, 5,6,7,8-tetrahydroquinolin-5-yl, 5,6,7,8-tetrahydro-quinolin-8-yl, 5,6,7,8-tetrahydro-quinolin-6-yl, 2,3-dihydrobenzofuran-3-yl, pyrazolo[1,5-a]pyrimidin-6-yl, imidazo[1,5-a]pyrazinyl, 3-methylimidazo[1,5-a]pyrazinyl, 3-Me-[1,2,4]triazolo[4,3-a]pyridin-6-yl, imidazo[1,2-a]pyrazin-6-yl, 2-Me-isoindolin-1-on-6-yl, 2,2-dimethyl-7-CF₃-chroman-4-yl, 7-CF₃-chroman-4-yl, 7-OCF₃-chroman-4-yl, isochroman-4-yl, 6,6-dimethyl-1,3-dioxan-2-yl, tetrahydropyran-3-yl, 1-phenyl-pyrrolidin-3-yl, piperidin-3-yl, 1,3-dimethyl-piperidin-4-yl, 1-cyclopropyl-piperidin-4-yl, 1-(tri-F-methyl-carbonyl)-piperidin-3-yl, quinuclidin-2-yl, 6,7-dihydro-5H-cyclopenta[b]pyridine-7-yl, 6,7-dihydro-5H-cyclopenta[b]pyridine-5-yl, 6,7-dihydro-5H-cyclopenta[c]pyridine-7-yl, 6,7-dihydro-5H-cyclopenta[c]pyridine-5-yl, 5,6-dihydro-4H-cyclopenta[b]thiophen-4-yl, or 1,2,3,5,6,7,8,8a-octahydroindolizin), or tri-(C₁-C₄-alkyl)-silyloxy;
- R¹⁴: is C₁-C₈-alkyl (e.g. pentyl, n-butyl, or hexyl), halogenated C₁-C₆-alkyl (e.g. 1,1-diF-butyl, 3,3-diF-butyl, 4,4,4-triF-butyl, 1,1-diF-pentyl, or 4,4-diF-pentyl), (optionally substituted C₃-C₁₂-cycloalkyl)-C₁-C₄-alkyl (e.g. cyclopropyl-methyl), (optionally substituted C₆-C₁₂-aryl)-C₁-C₄-alkyl (e.g. benzyl, a further example being 4-F-benzyl, 3-F-benzyl, 3-CF₃-benzyl, 4-F-3-CF₃-benzyl, or 3-OCF₃-benzyl), hydroxy-C₁-C₆-alkyl (e.g. hydroxyl-methyl or 1-hydroxy-pentyl), (optionally substituted C₆-C₁₂-aryloxy)-C₁-C₄-alkyl (e.g. phenoxy-methyl or (4-F-phenoxy)-methyl), C₁-C₆-alkylcarbonyl-C₁-C₄-alkyl (e.g. 2-methylcarbonyl-ethyl or 3-methylcarbonyl-propyl), C₁-C₆-alkoxycarbonyl-C₁-C₄-alkyl (e.g. 3-methoxycarbonylpropyl), optionally substituted C₃-C₁₂-cycloalkyl (e.g. 1-CF₃-cyclopropyl or 4-CF₃-cyclohexyl, a further example being 1-phenyl-cyclopropyl, 1-(4-F-phenyl)cyclopropyl, 1-(3-F-phenyl)cyclopropyl, 1-(3-Cl-phenyl)cyclopropyl, 1-(3-CF₃-phenyl)cyclopropyl, 3-hydroxymethyl-bycyclo [1.1.1]pentyl, 3-methoxymethyl-bycyclo [1.1.1]pentyl, 3-ethoxymethyl-bycyclo[1.1.1]pentyl, or 3-methoxycarbonyl-bycyclo[1.1.1]pentyl), optionally substituted C₆-C₁₂-aryl (e.g. phenyl, 2-F-phenyl, 3-F-phenyl, 4-F-phenyl, 2-Cl-phenyl, 3-Cl-phenyl, 4-Cl-phenyl, 2,3-diCl-phenyl, 2,4-diCl-phenyl, 3,5-diCl-phenyl, 2-Cl-4-F-phenyl, 2-Me-phenyl, 3-Me-phenyl, 4-Me-phenyl, 2-CF₃-phenyl, 3-CF₃-phenyl, 4-CF₃-phenyl, 3-Cl-4-CF₃-phenyl, 2-Cl-3-CF₃-phenyl, 2-Cl-4-CF₃-phenyl, 2-OMe-phenyl, 3-OMe-phenyl, 2-CN-phenyl, or 3-CN-phenyl, 4-CN-phenyl), or optionally substituted C₃-C₁₂-heterocyclyl (e.g. 3-Cl-pyridazin-6-yl, 2-Cl-pyrazin-5-yl, or 2-CF₃-pyrazin-5-yl);
- R¹⁵: is C₁-C₆-alkyl (e.g. ethyl, n-propyl, n-butyl, iso-butyl, or tert-bu) or C₆-C₁₂-aryl (e.g. phenyl);
- R^{16a}: is (optionally substituted C₆-C₁₂-aryl)-C₁-C₄-alkyl (e.g. benzyl or (2-Cl-phenyl)-methyl) or optionally substituted C₆-C₁₂-aryl (e.g. phenyl, 4-F-phenyl, 3-F-phenyl, 2F-phenyl, 4-Cl-phenyl, or 2-Cl-phenyl, a further example being 3-Cl-phenyl);
- R^{16b}: is hydrogen;
- R^{9c}, R^{9d}: are hydrogen;
- n5: is 0, 1, or 2;
- R¹⁸: is hydrogen, optionally substituted C₁-C₈-alkyl (e.g. methyl, ethyl, iso-propyl, or pentyl), C₁-C₆-alkoxycarbonyl (e.g. methoxycabonyl or n-butoxycarbonyl), C₁-C₆-alkylaminocarbonyl (e.g. n-propylaminocarbonyl or n-butylaminocarbonyl), (halogenated C₁-C₄-alkyl)aminocarbonyl (e.g. 2,2-di F-ethylaminocarbonyl, a further example being 2,2,2-tri F-ethylaminocarbonyl), optionally substituted C₆-C₁₂-aryl (e.g. phenyl, 3-F-phenyl, 4-F-phenyl, 4-Me-phenyl, 3-MeSO₂-phenyl, or 4-MeSO₂-phenyl, a further example being 4-F-2-CF₃-phenyl, 4-F-3-CF₃-phenyl, or 4-F-3-OCF₃-phenyl), C₁-C₆-alkylamine (e.g. propylamine), (halogenated C₁-C₆-alkyl)amino (e.g. 2,2,2-triF-ethylamine, a further example being 2,2-diF-ethylamine), optionally substituted C₆-C₁₂-arylamine (e.g. 4-Cl-phenylamine), or optionally substituted C₃-C₁₂-heterocyclyl (e.g. 4-CF₃-pyrid-2-yl or 6-CF₃-pyrimid-4-yl, a further example being 4-Me-pyrid-2-yl);
- R¹⁹: is optionally substituted C₆-C₁₂-aryl (e.g. phenyl, 3-F-phenyl, 4-F-phenyl, 3-Cl-phenyl, 3,4-diF-phenyl, 3,4-diCl-phenyl, 3,5-diCl-phenyl, 3-F-4-Cl-phenyl, 3-F-5-Cl-phenyl, 3-Me-phenyl, 3-CF₃-phenyl, 3-OCF₃-phenyl, or 4-OCF₃-phenyl);
- R^{20a}: is C₁-C₈-alkyl (e.g. n-propyl, n-butyl, hexyl, pentyl, or 6-Me-heptyl), (C₃-C₁₂-cycloalkyl)-C₁-C₄-alkyl (e.g. cyclopropyl-methyl), C₁-C₆-alkoxy-C₁-C₆-alkyl (e.g. 2-methoxy-ethyl, 2-ethoxy-ethyl, 2-isopropoxy-ethyl, 1-methyl-2-methoxy-ethyl, 3-methoxy-propyl, 3-ethoxy-propyl, or 3-isopropoxy-propyl), (optionally substituted C₆-C₁₂-aryl)-C₁-C₄-alkyl (e.g. benzyl, (4-F-phenyl)methyl, (3-Cl -phenyl)methyl, (4-Cl -phenyl)methyl, (3,4-diF-phenyl)methyl, (3-Cl-4-F-phenyl)methyl, (3-CF₃-phenyl)methyl, (2-Cl-4-CF₃-phenyl)methyl, (2-Cl-5-CF₃-phenyl)methyl, or (3-OCF₃-phenyl)methyl)), (optionally substituted C₃-C₁₂-heterocyclyl)-C₁-C₄-alkyl (e.g. (pyrid-2-yl)-methyl or (4-CF₃-pyrid-2-yl)methyl), optionally substituted C₆-C₁₂-aryl (e.g. phenyl, 4-F-phenyl, 3-Cl-phenyl, 3,5-diCl-phenyl, 3-Cl-4-F-phenyl, 3-Me-phenyl, 3-CF₃-phenyl, 4-CF₃-phenyl, 2-Cl-3-CF₃-phenyl, 3-Cl-4-CF₃-phenyl, 3-CF₃-4-F-phenyl, 3-CN-phenyl, 3-OCF₃-phenyl, 3-OCF₃-4-F-phenyl, or 3-OCF₃-4-Cl-phenyl), or optionally substituted C₃-C₁₂-heterocyclyl (e.g. 4-CF₃-pyrid-2-yl).
- R^{20b}: is hydrogen or C₁-C₈-alkyl (e.g. methyl, ethyl, or n-butyl);
- R²¹: is C₆-C₁₂-aryl (e. g phenyl); and
- R^{5a}, R^{5b}: are hydrogen; or
- R^{5a}, R^{5b}: together with the carbon atom to which they are bound may form a C=O, or . one of R^{2a} or R^{2b} and one of R^{5a} or R^{5b}
together are C₁-C₅-alkylene (e.g. 1,2-ethylene).

Further particular embodiments of pyrrolidine derivatives of the invention result if
- R¹: is optionally substituted 5-membered heterocyclic ring containing 2 or 3 N heteroatoms (e.g. 1-methyl-1,3-diazol-4-yl or 1-methyl-1,2,3-triazol-4-yl);
- R^{2a}, R^{2b}: are hydrogen;
- R^{3a}: is C₃-C₁₂-cycloalkyl (cyclopropyl, cyclobutyl, cyclopentyl, or cyclohexyl), optionally substituted C₆-C₁₂-aryl (e.g. phenyl, 2-Cl-phenyl, 2-Br-phenyl, 3-Cl-phenyl, 4-Cl-phenyl, 2-F-phenyl, 3-F-phenyl, 4-F-phenyl, 4-OMe-phenyl, or 2,4,5-trifluoro-phenyl, a further example being 2-Me-phenyl, 3-Me-phenyl, or 4-Me-phenyl) hydroxy, C₁-C₆-alkoxy (e.g.methoxy, ethoxy, n-propyloxy, iso-propyxy, or iso-butoxy) C₃-C₁₂-cycloalkyl-C₁-C₄-alkoxy (cyclo-propylmethoxy, cyclohexylmethoxy), C₂-C₆-alkenyloxy (e.g. allyloxy, 2-methylprop-2-en-1-oxy), C₆-C₁₂-aryl-C₁-C₄-alkoxy (e.g. benzyloxy), or optionally substituted C₆-C₁₂-aryloxy (e.g. phenoxy, 4-F-phenoxy);
- R^{3b}: is hydrogen or hydroxy;
- Y¹: is >CR⁶- or >N-;
- R⁶: is hydrogen, C₁-C₆-alkyl (e.g. methyl or ethyl), C₆-C₁₂-aryl-C₁-C₄-alkyl (e.g. benzyl or phenethyl), hydroxy-C₁-C₆-alkyl (e.g.-CH₂OH, or -(CH₂)₂OH), or hydroxy;
- R⁴: is -(CR^{7a}R^{7b})ₙ₁OR¹⁰, -(CR^{7c}R^{7d})ₙ₂NR^{11a}R^{11b}, -(CR^{7e}R^{7f})ₙ₃R¹², optionally substituted C₆-C₁₂-aryl, -NR^{8a}(CR^{9a}R^{9b})ₙ₄R¹³, -NR^{8b}COR¹⁴, -NR^{8c}COOR¹⁵, -NR^{8d}CONR^{16a}R^{16b},-O(CR^{9c}R^{9d})ₙ₅R¹⁸, -COR¹⁹, -CONR^{20a}R^{20b}, -SO₂R²¹, or optionally substituted C₃-C₁₂-heterocyclyl (e.g. 1-propyl-1,2,3-triazol-4-yl, 4-butyl-1,2,3-triazolyl-1-yl, 4-chloroisoindolin-1-one, or 7-(trifluoromethyl)-3,4-dihydro-1H-quinazolin-2-on-1-yl, a further example being 5-butyl-oxazolidin-2-on-3-yl, 1,4-thiazinane-1,1-dioxide, indolinyl, indolin-2-on-1-yl, 6-CF₃-indolin-2-on-1-yl, isoindolinyl, or isoindolin-1-on-2-yl);
- R^{7a}, R^{7b}: are hydrogen, or
- n1: is 1;
- R¹⁰: is hydrogen, optionally substituted C₆-C₁₂-aryl (e. g. phenyl, 3-F-phenyl, 3-Me-phenyl, 3-CF₃-phenyl, 2-Cl-3-CF₃-phenyl, or 3-OCF₃-phenyl, a further example being 2-F-phenyl, 4-F-phenyl, 2-Cl-phenyl, 2,3-diF-phenyl, 2,4-diF-phenyl, 2,5-diF-phenyl, 4-Cl-phenyl, 2-Cl-4-F-phenyl, 4-Cl-3-F-phenyl, 2-Me-phenyl, 4-Me-phenyl, 2-CF₃-phenyl, 4-CF₃-phenyl, 2-Cl-4-CF₃-phenyl, 3-OCHF₂-phenyl, 2-OCF₃-phenyl, 4-OCF₃-phenyl, 3-OMe-phenyl, 2-OEt-phenyl, 2-cyclopentyl-phenyl, 2-cyclohexyl-phenyl, 3-ethynyl-phenyl, 2-CN-phenyl, 4-CN-phenyl, 2-F-4-CN-phenyl, 3-dimethylamino-phenyl, 4-(morpholin-4-yl)-phenyl, 2-pirrolidinyl-phenyl, 2-piperidin-phenyl, indan-5-yl, naphthyl, or tetralin-5-yl), or optionally substituted C₃-C₁₂-heterocyclyl (e.g. 6-CF₃-pyrimid-4-yl, 4-CF₃-pyrid-2-yl, or 2-CF₃-pyrid-4-yl, a further example being pyrid-2-yl, pyrid-3-yl, pyrid-4-yl, pyrazin-2-yl, pyridazin-3-yl, quinolin-6-yl, isoquinolin-5-yl, 1,2-bezoxazol-6-yl, or 2-Me-1,3-benzoxazol-5-y);
- R^{7c}, R^{7d}: are hydrogen, or
- n2: is 1;
- R^{11a}: is C₁-C₈-alkyl (e.g. methyl, ethyl, n-propyl, n-butyl, or n-heptyl), C₃-C₁₂-cycloalkyl-C₁-C₄-alkyl (e.g. cyclopropyl-methyl) C₁-C₆-alkoxy-C₁-C₆-alkyl (2-methoxy-ethyl, 2-ethoxy-ethyl, 2-isopropyloxy-ethyl, 1-methyl-2-methoxy-ethyl, 3-methoxy-propyl, 3-ethoxy-propyl, or 3-isopropyloxy-propyl), C₆-C₁₂-aryl-C₁-C₄-alkyl (e.g. benzyl), optionally substituted C₆-C₁₂-aryl (e.g. phenyl, 4-F-phenyl, 3-Cl-phenyl, 3,5-diCl-phenyl, 3-Cl-4-F-phenyl, 3-Me-phenyl, 3-(aminomethyl)-phenyl, 3-CF₃-phenyl, 4-CF₃-phenyl, 2-Cl-4-CF₃-phenyl, 3-Cl-4-CF₃-phenyl, 3-CF₃-4-F-phenyl, 3-OCF₃-phenyl, 3-OCF₃-4-F-phenyl, 3-OCF₃-4-Cl-phenyl, or 3-(aminocarbonyl)-phenyl);
- R^{11b}: is hydrogen;
- R^{7e}, R^{7f}: are hydrogen, or
- n3: is 1;
- R¹²: is optionally substituted C₆-C₁₂-aryl (e.g. phenyl, 3-Cl-phenyl, or 3-Br-phenyl, a further example being 3-CF₃-phenyl) or optionally substituted C₃-C₂-heterocyclyl (e.g. 4-CF₃-pyrid-2-yl or 1-propyl-1,2,3-triazol-4-yl);
- R^{8a}, R^{8b}, R^{8c}, R^{8d}, R^{8e}: are independently hydrogen or C₁-C₆-alkyl (e.g. pentyl or hexyl), or
- R⁶ and one of R^{8a}, R^{8b}, R^{8c}, R^{8d}, or R^{8e}: together are optionally substituted C₁-C₅-alkylene, wherein one or more -CH₂- of C₁-C₅-alkylene may be independently replaced by a an oxygen atom or C=O;
- R^{9a}, R^{9b}: are independently hydrogen, halogen, C₁-C₆-alkyl (e.g. methyl, ethyl, tert-butyl, 2,3-dimethyl-propyl), or C₁-C₆-alkoxy (e.g. methoxy or ethoxy);
- n4: is 0, 1, 2, 3, or 4;
- R¹³: is hydrogen, C₁-C₈-alkyl (e.g. methyl, ethyl, n-butyl, tert-butyl, pentyl, or hexyl), halogenated C₁-C₆-alkyl (e.g. -CF₃ or -CF₂Me), C₁-C₆-alkoxy-C₁-C₆-alkyl (e.g. methoxy-methyl), optionally substituted C₃-C₁₂-cycloalkyl (e.g. cyclopropyl, 2-propyl-cyclopropyl, 1-(methoxy-methyl)-cyclopropyl, 2-phenyl-cyclopropyl, cyclopentyl, 1-methyl-cyclohexyl, 1-CF₃-cyclopropyl, 4-CF₃-cyclohexyl,or 4,4-diF-cyclohexyl, a further example being 1-phenyl-cyclopropyl, 1-(4-F-phenyl)-cyclopropyl, (3-Cl-phenyl)-cyclopropyl, 2-phenyl-cyclobutyl, 3-phenyl-cyclobutyl, 3-(4-F-phenyl)-cyclobutyl, 3-(2-F-phenyl)-cyclobutyl, 2-Cl-cyclopentyl, 2-Me-cyclopentyl, 3-Me-cyclopentyl, 2-phenyl-cyclopentyl, 3-(2-pyridyl)-cyclopentyl, 3-Me-cyclohexyl, 3,3,5,5-tetraMe-cyclohexyl, 4-Me-cyclohexyl, 4-Et-cyclohexyl, 2-CF₃-cyclohexyl, 2-Me-5-isopropenyl-cyclohexyl, 2-phenyl-cyclohexyl, 3-phenyl-cyclohexyl, 4-phenyl-cyclohexyl, 2-EtO-cyclohexyl, decalin-1-yl, decalin-2-yl, norbornan-2-yl, 1,7,7-trimethyl-norbornan-2-yl, 5-iPr-2-Me-3-bicyclo[3.1.0]hexanyl, bicycle [3.2.1]octanyl, or 3-bicyclo[1.1.1]pentanyl), C₂-C₆-alkenyl (e.g. hex-2-enyl), optionally substituted C₃-C₆-cycloalkenyl (e.g. 1,3,3-trimethylcyclohexen-2-yl), optionally substituted C₆-C₁₂-aryl (e.g. phenyl, 2-F-phenyl, 4-F-phenyl, 3-F-phenyl, 2-Cl-phenyl, 3-Cl-phenyl, 4-Cl-phenyl, 2,3-diF-phenyl, 2,4-diF-phenyl, 2,5-diF-phenyl, 3,4-diF-phenyl, 2,3,4-triF-phenyl, 2,4-diCl-phenyl, 3,4-diCl-phenyl, 3-F-4-Cl-phenyl, 3-Cl-4-F-phenyl, 3-Cl-5-F-phenyl, 3,4-diF-5-Cl-phenyl, 2-Me-phenyl, 3-Me-phenyl, 4-Me-phenyl, 3-iPr-phenyl, 3-tBu-phenyl, 3-Me-4-F-phenyl, 3-Me-4-Cl-phenyl, 3-iPr-4 Cl-phenyl, 3-(1-OH-1-CF₃-Et)-phenyl, 3-CHF₂-4-F-phenyl, 2-CF₃-phenyl, 3-CF₃-phenyl, 4-CF₃-phenyl, 3-Cl-4-CF₃-phenyl, 2-F-3-CF₃-phenyl, 2-F-5-CF₃-phenyl, 3-F-5-CF₃-phenyl, 2-Cl-4-CF₃-phenyl, 2-Cl-3-CF₃-phenyl, 3-CF₃-4-F-phenyl, 3-CF₃-4-Cl-phenyl, 3-Me-4-CF₃-phenyl, 3-CF₃-4-Me-phenyl, 3,4-diCF₃-phenyl, 4-OMe-phenyl, 3-OiPr-phenyl, 3-CF₃-4-OMe-phenyl, 3-OCH₂CF₃-phenyl, 3-OCF₃-phenyl, 2-OCHF₂-5-Cl-phenyl, 3-OCF₃-4-F-phenyl, 3-OCF₃-4-Cl-phenyl, 3-OBn-phenyl, 3-OPh-phenyl, 3-CN-phenyl, 4-CN-phenyl, 2-F-3-CN-phenyl, 2-F-4-CN-phenyl, 3-F-4-CN-phenyl, 2-F-5-CN-phenyl, 3-F-5-CN-phenyl, 3-Cl-4-CN-phenyl, 2-Cl-5-CN-phenyl, 3-Cl-5-CN-phenyl, 3-CN-4-Cl-phenyl, 2-Me-3-CN-phenyl, 2-Me-5-CN-phenyl, 3-Me-5-CN-phenyl, 3-CF₃-4-CN-phenyl, 3-CN-4-OMe-phenyl, 3-CN-4-OCF₃-phenyl, 3-phenyl-phenyl, 3-MeSO₂-phenyl, 3-(piperidin-4-yl)-phenyl, 2-methylcarbonylamino-5-Cl-phenyl, 3-(pyrid-2-yl)-phenyl, 3-(pyrid-3-yl)-4 F-phenyl, 3-(pyrid-4-yl)-4 F-phenyl, 3-(pyrimid-5-yl)-4 F-phenyl, indan-5-yl, or tetralin-6-yl, a further example being 2,4-diF-3-Cl-phenyl, 3-Et-4-Cl-phenyl, 3-(2-methoxyethyl)-phenyl, 3-(2-OH-ethyl)-phenyl, 3-(1-OH-1-Me-ethyl)-phenyl, 3-CHF₂-phenyl, 3-CF₃-2,4-diF-phenyl, 3-(dimethylamino-methyl)-phenyl, 3-(morpholin-4-yl-methyl)-phenyl, 3-cyclopropyl-phenyl, 3-OEt-phenyl, 3-OPr-phenyl, 3-OtBu-phenyl, 3-(cyclopropylmethoxy)-phenyl, 3-(OMe-methoxy)-phenyl, 3-(2-dimethylamino-ethoxy)-phenyl, 3-CF₃-4-OH-phenyl, 3-OCH₂CHF₂-phenyl, 3-OCHF₂-phenyl, 3-OCHF₂-4-F-phenyl, 3-OCF₃-4-OMe-phenyl, 3-cyclopropoxy-phenyl, 3-methylcarbonyl-phenyl, 3-dimethylamino-phenyl, 3-(2-pyridyloxy)-phenyl, 3-(pyrimidin-2-yloxy)-phenyl, indanyl, indan-2-yl, 2-F-indanyl, 4-F-indanyl, 5-F-indanyl, 6-F-indanyl, 7-F-indanyl, 3-Me-indanyl, 4-Me-indanyl, 5-Me-indanyl, 6-Me-indanyl, 4-CF₃-indanyl, 5-CF₃-indanyl, 6-CF₃-indanyl, 3,3-dimethyl-indanyl, tetralin-2-yl, 7-F-tetralin-2-yl, 6-F-tetralin-2-yl, tetralinyl, 6-F-tetralinyl, 5-F-tetralinyl, or 7-F-tetralinyl), C₁-C₆-alkoxy (e.g.methoxy, ethoxy, or n-propoxy), C₁-C₆-alkoxy-C₁-C₄-alkoxy (e.g. 2-methoxy-ethoxy), optionally substituted C₆-C₁₂-aryloxy (e.g. 4-F-phenoxy or 4-tertbutyl-phenoxy), optionally substituted C₃-C₁₂-heterocyclyl (e.g. propyl-furan-2-yl, 2-CF₃-furan-5-yl, 1,2-dimethyl-5-CN-pyrrol-3-yl, 2,3-diMe-thiophen-5-yl, 2-Methiophen-5-yl, 2-Cl-thiophen-5-yl, 3-Cl-thiophen-2-yl, 2,5-diCl-thiophen-3-yl, 2-tetrahydropyranyl-thiophen-5-yl, 1,3-thiazol-5-yl, 4-Me-1,3-thiazol-2-yl, 2-Me-1,3-thiazol-5-yl, 5-Me-1,3-thiazol-2-yl, 4-Me-1,3-thiazol-5-yl, 2-Me-1,3-thiazol-4-yl, 4-isopropyl-1,3-thiazol-2-yl, 2,4-diMe-1,3-thiazol-5-yl, 2-phenyl-4-Me-1,3-thiazol-5-yl, 4-phenyl-1,3-thiazol-5-yl, 2-(4-Me-phenyl)-1,3-thiazol-5-yl, 4-(4-F-phenyl)-1,3-thiazol-2-yl, 2-Cl-1,3-thiazol-4-yl, 4-Cl-1,3-thiazol-5-yl, 2-Br-1,3-thiazol-5-yl, 4-Br-1,3-thiazol-2-yl, 4-Me-5-Br-1,3-thiazol-2-yl, 2,4-dichloro-1,3-thiazol-5-yl, 1,5-dimethyl-1,2,4-triazol-3-yl, pyrid-2-yl, pyrid-3-yl, pyrid-4-yl, 4-F-pyrid-2-yl, 5-F-pyrid-2-yl, 6-F-pyrid-2-yl, 4-Cl-pyrid-2-yl, 5-F-pyrid-3-yl, 2-F-pyrid-4-yl, 3,5-diCl-pyrid-4-yl, 4,5-diCl-pyrid-2-yl, 2-Cl-3F-pyrid-4-yl, 5-Me-pyrid-2-yl, 4-iPr-pyrid-2-yl, 4-Me-5-F-pyrid-2-yl, 4-CF₃-pyrid-2-yl, 5-CF₃-pyrid-2-yl, 6-CF₃-pyrid-2-yl, 5-CF₃-pyrid-3-yl, 2-CF₃-pyrid-4-yl, 3-F-4-CF₃-pyrid-2-yl, 4-CF₃-5-F-pyrid-2-yl, 4-CF₃-5-Cl-pyrid-2-yl, 3,5-diF-4-CF₃-pyrid-2-yl, 4-OCH₂CF₃-pyrid-2-yl, 4-OCH₂CF₃-5-F-pyrid-2-yl, 4-OCH₂CF₃-5-Cl-pyrid-2-yl, 4-OBn-5-F-pyrid-2-yl, 3-(4-F-phenyl)-pyrid-5-yl, 2-(4-F-phenyl)-pyrid-3-yl, 4-(pyrid-4-yl)-pyrid-2-yl, 4-(pyrid-3-yl)-pyrid-2-yl, 5-cyclopropyl-pyraz-2-yl, 5-cyclobutyl-pyraz-2-yl, 5-pyrrolidin-pyraz-2-yl, pyridaz-3-yl, 4-CF₃-pyridaz-3-yl, 5-CF₃-pyridaz-3-yl, 5-F-pyrimid-2-yl, 6-Cl-pyrimid-4-yl, 6-Me-pyrimid-4-yl, 6-Et-pyrimid-4-yl, 6-Pr-pyrimid-4-yl, 6-iPr-pyrimid-4-yl, 4-CF₃-pyrimid-2-yl, 6-CF₃-pyrimid-4-yl, 2-Me-6-Cl-pyrimid-4-yl, 2-Me-6-CF₃-pyrimid-4-yl, 2-OMe-6-CF₃-pyrimid-4-yl, 2-OMe-pyrimid-4-yl, 6-OMe-pyrimid-4-yl, 6-OEt-pyrimid-4-yl, 6-OPr-pyrimid-4-yl, 6-OiPr-pyrimid-4-yl, 6-OiBu-pyrimid-4-yl, 6-OBu-pyrimid-4-yl, 6-OBn-pyrimid-4-yl, 6-cyclobutylloxy-pyrimid-4-yl, 6-cyclopentyloxy-pyrimid-4-yl, 6-cyclohexyloxy-pyrimid-4-yl, 6-cyclopropyl-pyrimid-4-yl, 6-phenyl-pyrimid-4-yl, 6-(2-F- phenyl)-pyrimid-4-yl, 6-(3-F-phenyl)-pyrimid-4-yl, 6-(4-F- phenyl)-pyrimid-4-yl, 6-Cl-pyrimid-4-yl, 6-(2-Me- phenyl)-pyrimid-4-yl, 6-(3-Me- phenyl)-pyrimid-4-yl, 6-(4-Me- phenyl)-pyrimid-4-yl, 6-(3-Cl- phenyl)-pyrimid-4-yl, 6-(4-Cl- phenyl)-pyrimid-4-yl, 2-(morpholin-1-yl)-pyrimid-4-yl, 6-(azetidin-3-yl-methoxy)-pyrimid-4-yl, 6-(pyrrolidin-3-yl-methoxy)-pyrimid-4-yl, 6-(pyrrolidin-2-yl-methoxy)-pyrimid-4-yl, benzofuran-5-yl, 2-Me-benzofuran-3-yl, 2-Et-benzofuran-3-yl, benzothiophen-5-yl, benzothiophen-6-yl, 5-Me-benzothiophen-2-yl, 5-F-benzothiophen-2-yl, 3-Cl-benzothiophen-2-yl, benzothiazol-2-yl, isoquinolin-6-yl, isoquinolin-7-yl, quinolin-6-yl, quinolin-7-yl, 4,5,6,7-tetrahydro-1,3-benzothiazol-2-yl, 2,3-dihydrobenzofuran-5-yl, 7H-pyrrolo[2,3-d]pyrimidin-4-yl, pyrazolo[1,5-a]pyridine-3-yl, pyrazolo[1,5-a]pyridine-7-yl, imidazo[1,2-a]pyridin-8-yl, 5-methyl-imidazo[1,2-a]pyridin-3-yl, 6-chloro-2-methyl-imidazo[1,2-a]pyridin-3-yl, 6-bromo-2-methyl-imidazo[1,2-a]pyridin-3-yl, thieno[2,3-b]pyridin-2-yl, N-benzyl-indolin-6-yl, indolinon-4-yl, chroman-6-yl, 4,4-dimethylchroman-6-yl, 4,4-dimethyl-1,3-dioxan-2-yl, tetrahydrofuran-3-yl, tetrahydropyran-4-yl, 4-Me-tetrahydropyran-4-yl, 2,2-diMe-tetrahydropyran-4-yl, 1-isopropyl-piperidin-4-yl, or 2-CF₃-piperazin-5-yl, a further example being iBu-pyrazol-4-yl, 1-CHF₂-5-Me-1,2-diazol-4-yl, 1-benzyl-5-F-1,2,4-triazol-3-yl, 2-Me-pyrid-4-yl, 2-iPr-pyrid-4-yl, 4-phenyl-pyrid-2yl, 2-cyclopropyl-pyrid-4-yl, 2-OMe-pyrid-4-yl, 2-OEt-pyrid-4-yl, 2-cyclopropylmethoxy-pyrid-4yl, 2-OiPr-pyrid-4-yl, 2-OCH₂CF₃-pyrid-4-yl, 2-cyclopropoxy-pyrid-4-yl, 2-cyclobutoxy-pyrid-4-yl, 4,5-diCl-pyrimidin-2yl, benzothiophen-3-yl, 2,2-diF-1,3-benzodioxol-5-yl, 5,6,7,8-tetrahydro-isoquinolin-5-yl, 5,6,7,8-tetrahydro-isoquinolin-8-yl, quinolin-8-yl, 1-Me-3,4-dihydro-2H-quinolin-4-yl, 2-Me-3,4-dihydro-1H-isoquinolin-6-yl, 5,6,7,8-tetrahydroquinolin-5-yl, 5,6,7,8-tetrahydro-quinolin-8-yl, 5,6,7,8-tetrahydro-quinolin-6-yl, 2,3-dihydrobenzofuran-3-yl, pyrazolo[1,5-a]pyrimidin-6-yl, imidazo[1,5-a]pyrazinyl, 3-methylimidazo[1,5-a]pyrazinyl, 3-Me-[1,2,4]triazolo[4,3-a]pyridin-6-yl, imidazo[1,2-a]pyrazin-6-yl, 2-Me-isoindolin-1-on-6-yl, 2,2-dimethyl-7-CF₃-chroman-4-yl, 7-CF₃-chroman-4-yl, 7-OCF₃-chroman-4-yl, isochroman-4-yl, 6,6-dimethyl-1,3-dioxan-2-yl, tetrahydropyran-3-yl, 1-phenyl-pyrrolidin-3-yl, piperidin-3-yl, 1,3-dimethyl-piperidin-4-yl, 1-cyclopropyl-piperidin-4-yl, 1-(tri-F-methyl-carbonyl)-piperidin-3-yl, quinuclidin-2-yl, 6,7-dihydro-5H-cyclopenta[b]pyridine-7-yl, 6,7-dihydro-5H-cyclopenta[b]pyridine-5-yl, 6,7-dihydro-5H-cyclopenta[c]pyridine-7-yl, 6,7-dihydro-5H-cyclopenta[c]pyridine-5-yl, 5,6-dihydro-4H-cyclopenta[b]thiophen-4-yl, or 1,2,3,5,6,7,8,8a-octahydroindolizin), or tri-(C₁-C₄-alkyl)-silyloxy;
- R¹⁴: is C₁-C₈-alkyl (e.g. pentyl, n-butyl, or hexyl), halogenated C₁-C₆-alkyl (e.g. 1,1-diF-butyl, 3,3-diF-butyl, 4,4,4-triF-butyl, 1,1-diF-pentyl, or 4,4-diF-pentyl), (optionally substituted C₃-C₁₂-cycloalkyl)-C₁-C₄-alkyl (e.g. cyclopropyl-methyl), hydroxy-C₁-C₆-alkyl (e.g. hydroxylmethyl or 1-hydroxy-pentyl), (optionally substituted C₆-C₁₂-aryloxy)-C₁-C₄-alkyl (e.g. phenoxy-methyl or (4-F-phenoxy)-methyl), C₁-C₆-alkylcarbonyl-C₁-C₄-alkyl (e.g. 2-methylcarbonyl-ethyl or 3-methylcarbonyl-propyl), C₁-C₆-alkoxycarbonyl-C₁-C₄-alkyl (e.g. 3-methoxycarbonyl-propyl), optionally substituted C₃-C₁₂-cycloalkyl (e.g.1-CF₃-cyclopropyl or 4-CF₃-cyclohexyl, a further example being 1-phenyl-cyclopropyl, 1-(4-F-phenyl)cyclopropyl, 1-(3-F-phenyl)cyclopropyl, 1-(3-Cl-phenyl)cyclopropyl, 1-(3-CF₃-phenyl)cyclopropyl, 3-hydroxymethyl-bycyclo[1.1.1]pentyl, 3-methoxymethyl-bycyclo[1.1.1]pentyl, 3-ethoxymethyl-bycyclo[1.1.1]pentyl, or 3-methoxycarbonyl-bycyclo[1.1.1]pentyl), optionally substituted C₆-C₁₂-aryl (e.g. phenyl, 2-F-phenyl, 3-F-phenyl, 4-F-phenyl, 2-Cl-phenyl, 3-Cl-phenyl, 4-Cl-phenyl, 2,3-diCl-phenyl, 2,4-diCl-phenyl, 3,5-diCl-phenyl, 2-Cl-4-F-phenyl, 2-Me-phenyl, 3-Me-phenyl, 4-Me-phenyl, 2-CF₃-phenyl, 3-CF₃-phenyl, 4-CF₃-phenyl, 3-Cl-4-CF₃-phenyl, 2-Cl-3-CF₃-phenyl, 2-Cl-4-CF₃-phenyl, 2-OMe-phenyl, 3-OMe-phenyl, 2-CN-phenyl, or 3-CN-phenyl, 4-CN-phenyl), or optionally substituted C₃-C₁₂-heterocyclyl (e.g. 3-Cl-pyridazin-6-yl, 2-Cl-pyrazin-5-yl, or 2-CF₃-pyrazin-5-yl);
- R¹⁵: is C₁-C₆-alkyl (e.g. ethyl, n-propyl, n-butyl, iso-butyl, or tert-bu) or C₆-C₁₂-aryl (e.g. phenyl);
- R^{16a}: is (optionally substituted C₆-C₁₂-aryl)-C₁-C₄-alkyl (e.g. benzyl or (2-Cl-phenyl)-methyl) or optionally substituted C₆-C₁₂-aryl (e.g. phenyl, 4-F-phenyl, 3-F-phenyl, 2F-phenyl, 4-Cl-phenyl, or 2-Cl-phenyl, a further example being 3-Cl-phenyl);
- R^{16b}: is hydrogen;
- R^{9c}, R^{9d}: are hydrogen;
- n5: is 0, 1, or 2;
- R¹⁸: is hydrogen, optionally substituted C₁-C₈-alkyl (e.g. methyl, ethyl, iso-propyl, or pentyl), C₁-C₆-alkoxycarbonyl (e.g. methoxycabonyl or n-butoxycarbonyl), C₁-C₆-alkylaminocarbonyl (e.g. n-propylaminocarbonyl or n-butylaminocarbonyl), (halogenated C₁-C₄-alkyl)aminocarbonyl (e.g. 2,2-di F-ethylaminocarbonyl, a further example being 2,2,2-tri F-ethylaminocarbonyl), optionally substituted C₆-C₁₂-aryl (e.g. phenyl, 3-F-phenyl, 4-F-phenyl, 4-Me-phenyl, 3-MeSO₂-phenyl, or 4-MeSO₂-phenyl, a further example being 4-F-2-CF₃-phenyl, 4-F-3-CF₃-phenyl, or 4-F-3-OCF₃-phenyl,), C₁-C₆-alkylamine (e.g. propylamine), (halogenated C₁-C₆-alkyl)amino (e.g. 2,2,2-triF-ethylamine, a further example being 2,2-diF-ethylamine), optionally substituted C₆-C₁₂-arylamine (e.g. 4-Cl-phenylamine), or optionally substituted C₃-C₁₂-heterocyclyl (e.g. 4-CF₃-pyrid-2-yl or 6-CF₃-pyrimid-4-yl, a further example being 4-Me-pyrid-2-yl);
- R¹⁹: is optionally substituted C₆-C₁₂-aryl (e.g. phenyl, 3-F-phenyl, 4-F-phenyl, 3-Cl-phenyl, 3,4-diF-phenyl, 3,4-diCl-phenyl, 3,5-diCl-phenyl, 3-F-4-Cl-phenyl, 3-F-5-Cl-phenyl, 3-Me-phenyl, 3-CF₃-phenyl, 3-OCF₃-phenyl, or 4-OCF₃-phenyl);
- R^{20a}: is C₁-C₈-alkyl (e.g. n-propyl, n-butyl, hexyl, pentyl, or 6-Me-heptyl), (C₃-C₁₂-cycloalkyl)-C₁-C₄-alkyl (e.g. cyclopropyl-methyl), C₁-C₆-alkoxy-C₁-C₆-alkyl (e.g. 2-methoxy-ethyl, 2-ethoxy-ethyl, 2-isopropoxy-ethyl, 1-methyl-2-methoxy-ethyl, 3-methoxy-propyl, 3-ethoxy-propyl, or 3-isopropoxy-propyl), (optionally substituted C₆-C₁₂-aryl)-C₁-C₄-alkyl (e.g. benzyl, (4-F-phenyl)methyl, (3-Cl -phenyl)methyl, (4-Cl -phenyl)methyl, (3,4-diF-phenyl)methyl, (3-Cl-4-F-phenyl)methyl, (3-CF₃-phenyl)methyl, (2-Cl-4-CF₃-phenyl)methyl, (2-Cl-5-CF₃-phenyl)methyl, or (3-OCF₃-phenyl)methyl)), (optionally substituted C₃-C₁₂-heterocyclyl)-C₁-C₄-alkyl (e.g. (pyrid-2-yl)-methyl or (4-CF₃-pyrid-2-yl)methyl), optionally substituted C₆-C₁₂-aryl (e.g. phenyl, 4-F-phenyl, 3-Cl-phenyl, 3,5-diCl-phenyl, 3-Cl-4-F-phenyl, 3-Me-phenyl, 3-CF₃-phenyl, 4-CF₃-phenyl, 2-Cl-3-CF₃-phenyl, 3-Cl-4-CF₃-phenyl, 3-CF₃-4-F-phenyl, 3-CN-phenyl, 3-OCF₃-phenyl, 3-OCF₃-4-F-phenyl, or 3-OCF₃-4-Cl-phenyl), or optionally substituted C₃-C₁₂-heterocyclyl (e.g. 4-CF₃-pyrid-2-yl).
- R^{20b}: is hydrogen or C₁-C₈-alkyl (e.g. methyl, ethyl, or n-butyl);
- R²¹: is C₆-C₁₂-aryl (e. g phenyl); and
- R^{5a}, R^{5b}: are hydrogen; or
- R^{5a}, R^{5b}: together with the carbon atom to which they are bound may form a C=O.

Further particular compounds of the present invention are the individual pyrrolidine derivatives of the formula (Id2) as listed in the following tables 1 to 8 and physiologically tolerated salts thereof: 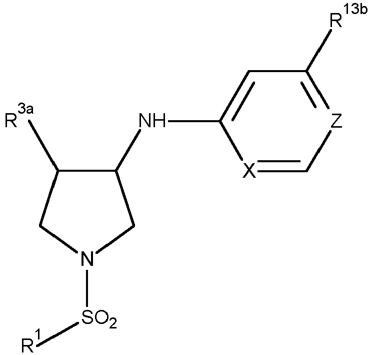
Table 1 Compounds of the formula (Id2) wherein X is =CH-, Z is =CR^{13c}-, R^{13c} is H and the combination of R¹, R^{3a} and R^{13b} for a compound in each case corresponds to one line of Table A (A-1 to A-344).
Table 2 Compounds of the formula (Id2) wherein X is =CH-, Z is =CR^{13c}-, R^{13c} is F and the combination of R¹, R^{3a} and R^{13b} for a compound in each case corresponds to one line of Table A (A-1 to A-344).
Table 3 Compounds of the formula (Id2) wherein X is =CH-, Z is =CR^{13c}-, R^{13c} is Cl and the combination of R¹, R^{3a} and R^{13b} for a compound in each case corresponds to one line of Table A (A-1 to A-344).
Table 4 Compounds of the formula (Id2) wherein X is =N-, Z is =CR^{13c}-, R^{13c} is H and the combination of R¹, R^{3a} and R^{13b} for a compound in each case corresponds to one line of Table A (A-1 to A-344).
Table 5 Compounds of the formula (Id2) wherein X is =N-, Z is =CR^{13c}-, R^{13c} is F and the combination of R¹, R^{3a} and R^{13b} for a compound in each case corresponds to one line of Table A (A-1 to A-344).
Table 6 Compounds of the formula (Id2) wherein X is =N-, Z is =CR^{13c}-, R^{13c} is Cl and the combination of R¹, R^{3a} and R^{13b} for a compound in each case corresponds to one line of Table A (A-1 to A-44).
Table 7 Compounds of the formula (Id2) wherein X is =N-, Z is =N- and the combination of R¹, R^{3a} and R^{13b} for a compound in each case corresponds to one line of Table A (A-1 to A-344).

| | R¹ | R^{3a} | R^{13b} |
|---|---|---|---|
| A-1. | 1-methyl-1,3-diazol-4-yl | Ph | H |
| A-2. | 1-methyl-1,3-diazol-4-yl | 4-F-Ph | H |
| A-3. | 1-methyl-1,3-diazol-4-yl | 2-THP | H |
| A-4. | 1-methyl-1,3-diazol-4-yl | 2-THF | H |
| A-5. | 1-methyl-1,2,3-triazol-4-yl | Ph | H |
| A-6. | 1-methyl-1,2,3-triazol-4-yl | 4-F-Ph | H |
| A-7. | 1-methyl-1,2,3-triazol-4-yl | 2-THP | H |
| A-8. | 1-methyl-1,2,3-triazol-4-yl | 2-THF | H |
| A-9. | 1-methyl-1,3-diazol-4-yl | Ph | F |
| A-10. | 1-methyl-1,3-diazol-4-yl | 4-F-Ph | F |
| A-11. | 1-methyl-1,3-diazol-4-yl | 2-THP | F |
| A-12. | 1-methyl-1,3-diazol-4-yl | 2-THF | F |
| A-13. | 1-methyl-1,2,3-triazol-4-yl | Ph | F |
| A-14. | 1-methyl-1,2,3-triazol-4-yl | 4-F-Ph | F |
| A-15. | 1-methyl-1,2,3-triazol-4-yl | 2-THP | F |
| A-16. | 1-methyl-1,2,3-triazol-4-yl | 2-THF | F |
| A-17. | 1-methyl-1,3-diazol-4-yl | Ph | Cl |
| A-18. | 1-methyl-1,3-diazol-4-yl | 4-F-Ph | Cl |
| A-19. | 1-methyl-1,3-diazol-4-yl | 2-THP | Cl |
| A-20. | 1-methyl-1,3-diazol-4-yl | 2-THF | Cl |
| A-21. | 1-methyl-1,2,3-triazol-4-yl | Ph | Cl |
| A-22. | 1-methyl-1,2,3-triazol-4-yl | 4-F-Ph | Cl |
| A-23. | 1-methyl-1,2,3-triazol-4-yl | 2-THP | Cl |
| A-24. | 1-methyl-1,2,3-triazol-4-yl | 2-THF | Cl |
| A-25. | 1-methyl-1,3-diazol-4-yl | Ph | -CH₃ |
| A-26. | 1-methyl-1,3-diazol-4-yl | 4-F-Ph | -CH₃ |
| A-27. | 1-methyl-1,3-diazol-4-yl | 2-THP | -CH₃ |
| A-28. | 1-methyl-1,3-diazol-4-yl | 2-THF | -CH₃ |
| A-29. | 1-methyl-1,2,3-triazol-4-yl | Ph | -CH₃ |
| A-30. | 1-methyl-1,2,3-triazol-4-yl | 4-F-Ph | -CH₃ |
| A-31. | 1-methyl-1,2,3-triazol-4-yl | 2-THP | -CH₃ |
| A-32. | 1-methyl-1,2,3-triazol-4-yl | 2-THF | -CH₃ |
| A-33. | 1-methyl-1,3-diazol-4-yl | Ph | -CH₂CH₃ |
| A-34. | 1-methyl-1,3-diazol-4-yl | 4-F-Ph | -CH₂CH₃ |
| A-35. | 1-methyl-1,3-diazol-4-yl | 2-THP | -CH₂CH₃ |

| | R¹ | R^{3a} | R^{13b} |
|---|---|---|---|
| A-36. | 1-methyl-1,3-diazol-4-yl | 2-THF | -CH₂CH₃ |
| A-37. | 1-methyl-1,2,3-triazol-4-yl | Ph | -CH₂CH₃ |
| A-38. | 1-methyl-1,2,3-triazol-4-yl | 4-F-Ph | -CH₂CH₃ |
| A-39. | 1-methyl-1,2,3-triazol-4-yl | 2-THP | -CH₂CH₃ |
| A-40. | 1-methyl-1,2,3-triazol-4-yl | 2-THF | -CH₂CH₃ |
| A-41. | 1-methyl-1,3-diazol-4-yl | Ph | -CH₂CH₂CH₃ |
| A-42. | 1-methyl-1,3-diazol-4-yl | 4-F-Ph | -CH₂CH₂CH₃ |
| A-43. | 1-methyl-1,3-diazol-4-yl | 2-THP | -CH₂CH₂CH₃ |
| A-44. | 1-methyl-1,3-diazol-4-yl | 2-THF | -CH₂CH₂CH₃ |
| A-45. | 1-methyl-1,2,3-triazol-4-yl | Ph | -CH₂CH₂CH₃ |
| A-46. | 1-methyl-1,2,3-triazol-4-yl | 4-F-Ph | -CH₂CH₂CH₃ |
| A-47. | 1-methyl-1,2,3-triazol-4-yl | 2-THP | -CH₂CH₂CH₃ |
| A-48. | 1-methyl-1,2,3-triazol-4-yl | 2-THF | -CH₂CH₂CH₃ |
| A-49. | 1-methyl-1,3-diazol-4-yl | Ph | -CH(CH₃)₂ |
| A-50. | 1-methyl-1,3-diazol-4-yl | 4-F-Ph | -CH(CH₃)₂ |
| A-51. | 1-methyl-1,3-diazol-4-yl | 2-THP | -CH(CH₃)₂ |
| A-52. | 1-methyl-1,3-diazol-4-yl | 2-THF | -CH(CH₃)₂ |
| A-53. | 1-methyl-1,2,3-triazol-4-yl | Ph | -CH(CH₃)₂ |
| A-54. | 1-methyl-1,2,3-triazol-4-yl | 4-F-Ph | -CH(CH₃)₂ |
| A-55. | 1-methyl-1,2,3-triazol-4-yl | 2-THP | -CH(CH₃)₂ |
| A-56. | 1-methyl-1,2,3-triazol-4-yl | 2-THF | -CH(CH₃)₂ |
| A-57. | 1-methyl-1,3-diazol-4-yl | Ph | -C(CH₃)₃ |
| A-58. | 1-methyl-1,3-diazol-4-yl | 4-F-Ph | -C(CH₃)₃ |
| A-59. | 1-methyl-1,3-diazol-4-yl | 2-THP | -C(CH₃)₃ |
| A-60. | 1-methyl-1,3-diazol-4-yl | 2-THF | -C(CH₃)₃ |
| A-61. | 1-methyl-1,2,3-triazol-4-yl | Ph | -C(CH₃)₃ |
| A-62. | 1-methyl-1,2,3-triazol-4-yl | 4-F-Ph | -C(CH₃)₃ |
| A-63. | 1-methyl-1,2,3-triazol-4-yl | 2-THP | -C(CH₃)₃ |
| A-64. | 1-methyl-1,2,3-triazol-4-yl | 2-THF | -C(CH₃)₃ |
| A-65. | 1-methyl-1,3-diazol-4-yl | Ph | -CF₃ |
| A-66. | 1-methyl-1,3-diazol-4-yl | 4-F-Ph | -CF₃ |
| A-67. | 1-methyl-1,3-diazol-4-yl | 2-THP | -CF₃ |
| A-68. | 1-methyl-1,3-diazol-4-yl | 2-THF | -CF₃ |
| A-69. | 1-methyl-1,2,3-triazol-4-yl | Ph | -CF₃ |
| A-70. | 1-methyl-1,2,3-triazol-4-yl | 4-F-Ph | -CF₃ |
| A-71. | 1-methyl-1,2,3-triazol-4-yl | 2-THP | -CF₃ |

| | | | |
|---|---|---|---|
| | R¹ | R^{3a} | R^{13b} |
| A-72. | 1-methyl-1,2,3-triazol-4-yl | 2-THF | -CF₃ |
| A-73. | 1-methyl-1,3-diazol-4-yl | Ph | -CHF₂ |
| A-74. | 1-methyl-1,3-diazol-4-yl | 4-F-Ph | -CHF₂ |
| A-75. | 1-methyl-1,3-diazol-4-yl | 2-THP | -CHF₂ |
| A-76. | 1-methyl-1,3-diazol-4-yl | 2-THF | -CHF₂ |
| A-77. | 1-methyl-1,2,3-triazol-4-yl | Ph | -CHF₂ |
| A-78. | 1-methyl-1,2,3-triazol-4-yl | 4-F-Ph | -CHF₂ |
| A-79. | 1-methyl-1,2,3-triazol-4-yl | 2-THP | -CHF₂ |
| A-80. | 1-methyl-1,2,3-triazol-4-yl | 2-THF | -CHF₂ |
| A-81. | 1-methyl-1,3-diazol-4-yl | Ph | -CH(OH)(CF₃)CH₃ |
| A-82. | 1-methyl-1,3-diazol-4-yl | 4-F-Ph | -CH(OH)(CF₃)CH₃ |
| A-83. | 1-methyl-1,3-diazol-4-yl | 2-THP | -CH(OH)(CF₃)CH₃ |
| A-84. | 1-methyl-1,3-diazol-4-yl | 2-THF | -CH(OH)(CF₃)CH₃ |
| A-85. | 1-methyl-1,2,3-triazol-4-yl | Ph | -CH(OH)(CF₃)CH₃ |
| A-86. | 1-methyl-1,2,3-triazol-4-yl | 4-F-Ph | -CH(OH)(CF₃)CH₃ |
| A-87. | 1-methyl-1,2,3-triazol-4-yl | 2-THP | -CH(OH)(CF₃)CH₃ |
| A-88. | 1-methyl-1,2,3-triazol-4-yl | 2-THF | -CH(OH)(CF₃)CH₃ |
| A-89. | 1-methyl-1,3-diazol-4-yl | Ph | cyclopropyl |
| A-90. | 1-methyl-1,3-diazol-4-yl | 4-F-Ph | cyclopropyl |
| A-91. | 1-methyl-1,3-diazol-4-yl | 2-THP | cyclopropyl |
| A-92. | 1-methyl-1,3-diazol-4-yl | 2-THF | cyclopropyl |
| A-93. | 1-methyl-1,2,3-triazol-4-yl | Ph | cyclopropyl |
| A-94. | 1-methyl-1,2,3-triazol-4-yl | 4-F-Ph | cyclopropyl |
| A-95. | 1-methyl-1,2,3-triazol-4-yl | 2-THP | cyclopropyl |
| A-96. | 1-methyl-1,2,3-triazol-4-yl | 2-THF | cyclopropyl |
| A-97. | 1-methyl-1,3-diazol-4-yl | Ph | phenyl |
| A-98. | 1-methyl-1,3-diazol-4-yl | 4-F-Ph | phenyl |
| A-99. | 1-methyl-1,3-diazol-4-yl | 2-THP | phenyl |
| A-100. | 1-methyl-1,3-diazol-4-yl | 2-THF | phenyl |
| A-101. | 1-methyl-1,2,3-triazol-4-yl | Ph | phenyl |
| A-102. | 1-methyl-1,2,3-triazol-4-yl | 4-F-Ph | phenyl |
| A-103. | 1-methyl-1,2,3-triazol-4-yl | 2-THP | phenyl |
| A-104. | 1-methyl-1,2,3-triazol-4-yl | 2-THF | phenyl |
| A-105. | 1-methyl-1,3-diazol-4-yl | Ph | 4-Cl-phenyl |
| A-106. | 1-methyl-1,3-diazol-4-yl | 4-F-Ph | 4-Cl-phenyl |
| A-107. | 1-methyl-1,3-diazol-4-yl | 2-THP | 4-Cl-phenyl |

| | R¹ | R^{3a} | R^{13b} |
|---|---|---|---|
| A-108. | 1-methyl-1,3-diazol-4-yl | 2-THF | 4-Cl-phenyl |
| A-109. | 1-methyl-1,2,3-triazol-4-yl | Ph | 4-Cl-phenyl |
| A-110. | 1-methyl-1,2,3-triazol-4-yl | 4-F-Ph | 4-Cl-phenyl |
| A-111. | 1-methyl-1,2,3-triazol-4-yl | 2-THP | 4-Cl-phenyl |
| A-112. | 1-methyl-1,2,3-triazol-4-yl | 2-THF | 4-Cl-phenyl |
| A-113. | 1-methyl-1,3-diazol-4-yl | Ph | 3-Cl-phenyl |
| A-114. | 1-methyl-1,3-diazol-4-yl | 4-F-Ph | 3-Cl-phenyl |
| A-115. | 1-methyl-1,3-diazol-4-yl | 2-THP | 3-Cl-phenyl |
| A-116. | 1-methyl-1,3-diazol-4-yl | 2-THF | 3-Cl-phenyl |
| A-117. | 1-methyl-1,2,3-triazol-4-yl | Ph | 3-Cl-phenyl |
| A-118. | 1-methyl-1,2,3-triazol-4-yl | 4-F-Ph | 3-Cl-phenyl |
| A-119. | 1-methyl-1,2,3-triazol-4-yl | 2-THP | 3-Cl-phenyl |
| A-120. | 1-methyl-1,2,3-triazol-4-yl | 2-THF | 3-Cl-phenyl |
| A-121. | 1-methyl-1,3-diazol-4-yl | Ph | 4-F-phenyl |
| A-122. | 1-methyl-1,3-diazol-4-yl | 4-F-Ph | 4-F-phenyl |
| A-123. | 1-methyl-1,3-diazol-4-yl | 2-THP | 4-F-phenyl |
| A-124. | 1-methyl-1,3-diazol-4-yl | 2-THF | 4-F-phenyl |
| A-125. | 1-methyl-1,2,3-triazol-4-yl | Ph | 4-F-phenyl |
| A-126. | 1-methyl-1,2,3-triazol-4-yl | 4-F-Ph | 4-F-phenyl |
| A-127. | 1-methyl-1,2,3-triazol-4-yl | 2-THP | 4-F-phenyl |
| A-128. | 1-methyl-1,2,3-triazol-4-yl | 2-THF | 4-F-phenyl |
| A-129. | 1-methyl-1,3-diazol-4-yl | Ph | 3-F-phenyl |
| A-130. | 1-methyl-1,3-diazol-4-yl | 4-F-Ph | 3-F-phenyl |
| A-131. | 1-methyl-1,3-diazol-4-yl | 2-THP | 3-F-phenyl |
| A-132. | 1-methyl-1,3-diazol-4-yl | 2-THF | 3-F-phenyl |
| A-133. | 1-methyl-1,2,3-triazol-4-yl | Ph | 3-F-phenyl |
| A-134. | 1-methyl-1,2,3-triazol-4-yl | 4-F-Ph | 3-F-phenyl |
| A-135. | 1-methyl-1,2,3-triazol-4-yl | 2-THP | 3-F-phenyl |
| A-136. | 1 -methyl- 1,2,3 -triazol-4-yl | 2-THF | 3-F-phenyl |
| A-137. | 1-methyl-1,3-diazol-4-yl | Ph | 2-F-phenyl |
| A-138. | 1-methyl-1,3-diazol-4-yl | 4-F-Ph | 2-F-phenyl |
| A-139. | 1-methyl-1,3-diazol-4-yl | 2-THP | 2-F-phenyl |
| A-140. | 1-methyl-1,3-diazol-4-yl | 2-THF | 2-F-phenyl |
| A-141. | 1-methyl-1,2,3-triazol-4-yl | Ph | 2-F-phenyl |
| A-142. | 1-methyl-1,2,3-triazol-4-yl | 4-F-Ph | 2-F-phenyl |
| A-143. | 1-methyl-1,2,3-triazol-4-yl | 2-THP | 2-F-phenyl |

| | R¹ | R^{3a} | R^{13b} |
|---|---|---|---|
| A-144. | 1-methyl-1,2,3-triazol-4-yl | 2-THF | 2-F-phenyl |
| A-145. | 1-methyl-1,3-diazol-4-yl | Ph | 2-Me-phenyl |
| A-146. | 1-methyl-1,3-diazol-4-yl | 4-F-Ph | 2-Me-phenyl |
| A-147. | 1-methyl-1,3-diazol-4-yl | 2-THP | 2-Me-phenyl |
| A-148. | 1-methyl-1,3-diazol-4-yl | 2-THF | 2-Me-phenyl |
| A-149. | 1-methyl-1,2,3-triazol-4-yl | Ph | 2-Me-phenyl |
| A-150. | 1-methyl-1,2,3-triazol-4-yl | 4-F-Ph | 2-Me-phenyl |
| A-151. | 1-methyl-1,2,3-triazol-4-yl | 2-THP | 2-Me-phenyl |
| A-152. | 1-methyl-1,2,3-triazol-4-yl | 2-THF | 2-Me-phenyl |
| A-153. | 1-methyl-1,3-diazol-4-yl | Ph | 3-Me-phenyl |
| A-154. | 1-methyl-1,3-diazol-4-yl | 4-F-Ph | 3-Me-phenyl |
| A-155. | 1-methyl-1,3-diazol-4-yl | 2-THP | 3-Me-phenyl |
| A-156. | 1-methyl-1,3-diazol-4-yl | 2-THF | 3-Me-phenyl |
| A-157. | 1-methyl-1,2,3-triazol-4-yl | Ph | 3-Me-phenyl |
| A-158. | 1-methyl-1,2,3-triazol-4-yl | 4-F-Ph | 3-Me-phenyl |
| A-159. | 1-methyl-1,2,3-triazol-4-yl | 2-THP | 3-Me-phenyl |
| A-160. | 1-methyl-1,2,3-triazol-4-yl | 2-THF | 3-Me-phenyl |
| A-161. | 1-methyl-1,3-diazol-4-yl | Ph | 4-Me-phenyl |
| A-162. | 1-methyl-1,3-diazol-4-yl | 4-F-Ph | 4-Me-phenyl |
| A-163. | 1-methyl-1,3-diazol-4-yl | 2-THP | 4-Me-phenyl |
| A-164. | 1-methyl-1,3-diazol-4-yl | 2-THF | 4-Me-phenyl |
| A-165. | 1-methyl-1,2,3-triazol-4-yl | Ph | 4-Me-phenyl |
| A-166. | 1-methyl-1,2,3-triazol-4-yl | 4-F-Ph | 4-Me-phenyl |
| A-167. | 1-methyl-1,2,3-triazol-4-yl | 2-THP | 4-Me-phenyl |
| A-168. | 1-methyl-1,2,3-triazol-4-yl | 2-THF | 4-Me-phenyl |
| A-169. | 1-methyl-1,3-diazol-4-yl | Ph | -CN |
| A-170. | 1-methyl-1,3-diazol-4-yl | 4-F-Ph | -CN |
| A-171. | 1-methyl-1,3-diazol-4-yl | 2-THP | -CN |
| A-172. | 1-methyl-1,3-diazol-4-yl | 2-THF | -CN |
| A-173. | 1-methyl-1,2,3-triazol-4-yl | Ph | -CN |
| A-174. | 1-methyl-1,2,3-triazol-4-yl | 4-F-Ph | -CN |
| A-175. | 1-methyl-1,2,3-triazol-4-yl | 2-THP | -CN |
| A-176. | 1-methyl-1,2,3-triazol-4-yl | 2-THF | -CN |
| A-177. | 1-methyl-1,3-diazol-4-yl | Ph | -OCH₃ |
| A-178. | 1-methyl-1,3-diazol-4-yl | 4-F-Ph | -OCH₃ |
| A-179. | 1-methyl-1,3-diazol-4-yl | 2-THP | -OCH₃ |

| | R¹ | R^{3a} | R^{13b} |
|---|---|---|---|
| A-180. | 1-methyl-1,3-diazol-4-yl | 2-THF | -OCH₃ |
| A-181. | 1-methyl-1,2,3-triazol-4-yl | Ph | -OCH₃ |
| A-182. | 1-methyl-1,2,3-triazol-4-yl | 4-F-Ph | -OCH₃ |
| A-183. | 1-methyl-1,2,3-triazol-4-yl | 2-THP | -OCH₃ |
| A-184. | 1-methyl-1,2,3-triazol-4-yl | 2-THF | -OCH₃ |
| A-185. | 1-methyl-1,3-diazol-4-yl | Ph | -OCH₂CH₃ |
| A-186. | 1-methyl-1,3-diazol-4-yl | 4-F-Ph | -OCH₂CH₃ |
| A-187. | 1-methyl-1,3-diazol-4-yl | 2-THP | -OCH₂CH₃ |
| A-188. | 1-methyl-1,3-diazol-4-yl | 2-THF | -OCH₂CH₃ |
| A-189. | 1-methyl-1,2,3-triazol-4-yl | Ph | -OCH₂CH₃ |
| A-190. | 1-methyl-1,2,3-triazol-4-yl | 4-F-Ph | -OCH₂CH₃ |
| A-191. | 1-methyl-1,2,3-triazol-4-yl | 2-THP | -OCH₂CH₃ |
| A-192. | 1-methyl-1,2,3-triazol-4-yl | 2-THF | -OCH₂CH₃ |
| A-193. | 1-methyl-1,3-diazol-4-yl | Ph | -O(CH₂)₂CH₃ |
| A-194. | 1-methyl-1,3-diazol-4-yl | 4-F-Ph | -O(CH₂)₂CH₃ |
| A-195. | 1-methyl-1,3-diazol-4-yl | 2-THP | -O(CH₂)₂CH₃ |
| A-196. | 1-methyl-1,3-diazol-4-yl | 2-THF | -O(CH₂)₂CH₃ |
| A-197. | 1-methyl-1,2,3-triazol-4-yl | Ph | -O(CH₂)₂CH₃ |
| A-198. | 1-methyl-1,2,3-triazol-4-yl | 4-F-Ph | -O(CH₂)₂CH₃ |
| A-199. | 1-methyl-1,2,3-triazol-4-yl | 2-THP | -O(CH₂)₂CH₃ |
| A-200. | 1-methyl-1,2,3-triazol-4-yl | 2-THF | -O(CH₂)₂CH₃ |
| A-201. | 1-methyl-1,3-diazol-4-yl | Ph | -O(CH₂)₃CH₃ |
| A-202. | 1-methyl-1,3-diazol-4-yl | 4-F-Ph | -O(CH₂)₃CH₃ |
| A-203. | 1-methyl-1,3-diazol-4-yl | 2-THP | -O(CH₂)₃CH₃ |
| A-204. | 1-methyl-1,3-diazol-4-yl | 2-THF | -O(CH₂)₃CH₃ |
| A-205. | 1-methyl-1,2,3-triazol-4-yl | Ph | -O(CH₂)₃CH₃ |
| A-206. | 1-methyl-1,2,3-triazol-4-yl | 4-F-Ph | -O(CH₂)₃CH₃ |
| A-207. | 1-methyl-1,2,3-triazol-4-yl | 2-THP | -O(CH₂)₃CH₃ |
| A-208. | 1-methyl-1,2,3-triazol-4-yl | 2-THF | -O(CH₂)₃CH₃ |
| A-209. | 1-methyl-1,3-diazol-4-yl | Ph | -OCH(CH₃)₂ |
| A-210. | 1-methyl-1,3-diazol-4-yl | 4-F-Ph | -OCH(CH₃)₂ |
| A-211. | 1-methyl-1,3-diazol-4-yl | 2-THP | -OCH(CH₃)₂ |
| A-212. | 1-methyl-1,3-diazol-4-yl | 2-THF | -OCH(CH₃)₂ |
| A-213. | 1-methyl-1,2,3-triazol-4-yl | Ph | -OCH(CH₃)₂ |
| A-214. | 1-methyl-1,2,3-triazol-4-yl | 4-F-Ph | -OCH(CH₃)₂ |
| A-215. | 1-methyl-1,2,3-triazol-4-yl | 2-THP | -OCH(CH₃)₂ |

| | R¹ | R^{3a} | R^{13b} |
|---|---|---|---|
| A-216. | 1-methyl-1,2,3-triazol-4-yl | 2-THF | -OCH(CH₃)₂ |
| A-217. | 1-methyl-1,3-diazol-4-yl | Ph | -OCH₂(CH₃)₂ |
| A-218. | 1-methyl-1,3-diazol-4-yl | 4-F-Ph | -OCH₂(CH₃)₂ |
| A-219. | 1-methyl-1,3-diazol-4-yl | 2-THP | -OCH₂(CH₃)₂ |
| A-220. | 1-methyl-1,3-diazol-4-yl | 2-THF | -OCH₂(CH₃)₂ |
| A-221. | 1-methyl-1,2,3-triazol-4-yl | Ph | -OCH₂(CH₃)₂ |
| A-222. | 1-methyl-1,2,3-triazol-4-yl | 4-F-Ph | -OCH₂(CH₃)₂ |
| A-223. | 1-methyl-1,2,3-triazol-4-yl | 2-THP | -OCH₂(CH₃)₂ |
| A-224. | 1-methyl-1,2,3-triazol-4-yl | 2-THF | -OCH₂(CH₃)₂ |
| A-225. | 1-methyl-1,3-diazol-4-yl | Ph | -OCH₂(pyrrolyd-3yl) |
| A-226. | 1-methyl-1,3-diazol-4-yl | 4-F-Ph | -OCH₂(pyrrolyd-3yl) |
| A-227. | 1-methyl-1,3-diazol-4-yl | 2-THP | -OCH₂(pyrrolyd-3yl) |
| A-228. | 1-methyl-1,3-diazol-4-yl | 2-THF | -OCH₂(pyrrolyd-3yl) |
| A-229. | 1-methyl-1,2,3-triazol-4-yl | Ph | -OCH₂(pyrrolyd-3yl) |
| A-230. | 1-methyl-1,2,3-triazol-4-yl | 4-F-Ph | -OCH₂(pyrrolyd-3yl) |
| A-231. | 1-methyl-1,2,3-triazol-4-yl | 2-THP | -OCH₂(pyrrolyd-3yl) |
| A-232. | 1-methyl-1,2,3-triazol-4-yl | 2-THF | -OCH₂(pyrrolyd-3yl) |
| A-233. | 1-methyl-1,3-diazol-4-yl | Ph | -OCH₂(pyrrolyd-2yl) |
| A-234. | 1-methyl-1,3-diazol-4-yl | 4-F-Ph | -OCH₂(pyrrolyd-2yl) |
| A-235. | 1-methyl-1,3-diazol-4-yl | 2-THP | -OCH₂(pyrrolyd-2yl) |
| A-236. | 1-methyl-1,3-diazol-4-yl | 2-THF | -OCH₂(pyrrolyd-2yl) |
| A-237. | 1-methyl-1,2,3-triazol-4-yl | Ph | -OCH₂(pyrrolyd-2yl) |
| A-238. | 1-methyl-1,2,3-triazol-4-yl | 4-F-Ph | -OCH₂(pyrrolyd-2yl) |
| A-239. | 1-methyl-1,2,3-triazol-4-yl | 2-THP | -OCH₂(pyrrolyd-2yl) |
| A-240. | 1-methyl-1,2,3-triazol-4-yl | 2-THF | -OCH₂(pyrrolyd-2yl) |
| A-241. | 1-methyl-1,3-diazol-4-yl | Ph | -OCH₂(azetid-3yl) |
| A-242. | 1-methyl-1,3-diazol-4-yl | 4-F-Ph | -OCH₂(azetid-3yl) |
| A-243. | 1-methyl-1,3-diazol-4-yl | 2-THP | -OCH₂(azetid-3yl) |
| A-244. | 1-methyl-1,3-diazol-4-yl | 2-THF | -OCH₂(azetid-3yl) |
| A-245. | 1-methyl-1,2,3-triazol-4-yl | Ph | -OCH₂(azetid-3yl) |
| A-246. | 1-methyl-1,2,3-triazol-4-yl | 4-F-Ph | -OCH₂(azetid-3yl) |
| A-247. | 1-methyl-1,2,3-triazol-4-yl | 2-THP | -OCH₂(azetid-3yl) |
| A-248. | 1-methyl-1,2,3-triazol-4-yl | 2-THF | -OCH₂(azetid-3yl) |
| A-249. | 1-methyl-1,3-diazol-4-yl | Ph | -OCF₃ |
| A-250. | 1-methyl-1,3-diazol-4-yl | 4-F-Ph | -OCF₃ |
| A-251. | 1-methyl-1,3-diazol-4-yl | 2-THP | -OCF₃ |

| | R¹ | R^{3a} | R^{13b} |
|---|---|---|---|
| A-252. | 1-methyl-1,3-diazol-4-yl | 2-THF | -OCF₃ |
| A-253. | 1-methyl-1,2,3-triazol-4-yl | Ph | -OCF₃ |
| A-254. | 1-methyl-1,2,3-triazol-4-yl | 4-F-Ph | -OCF₃ |
| A-255. | 1-methyl-1,2,3-triazol-4-yl | 2-THP | -OCF₃ |
| A-256. | 1-methyl-1,2,3-triazol-4-yl | 2-THF | -OCF₃ |
| A-257. | 1-methyl-1,3-diazol-4-yl | Ph | -OCH₂CF₃ |
| A-258. | 1-methyl-1,3-diazol-4-yl | 4-F-Ph | -OCH₂CF₃ |
| A-259. | 1-methyl-1,3-diazol-4-yl | 2-THP | --OCH₂CF₃ |
| A-260. | 1-methyl-1,3-diazol-4-yl | 2-THF | -OCH₂CF₃ |
| A-261. | 1-methyl-1,2,3-triazol-4-yl | Ph | -OCH₂CF₃ |
| A-262. | 1-methyl-1,2,3-triazol-4-yl | 4-F-Ph | -OCH₂CF₃ |
| A-263. | 1-methyl-1,2,3-triazol-4-yl | 2-THP | -OCH₂CF₃ |
| A-264. | 1-methyl-1,2,3-triazol-4-yl | 2-THF | -OCH₂CF₃ |
| A-265. | 1-methyl-1,3-diazol-4-yl | Ph | -O-cyclobutyl |
| A-266. | 1-methyl-1,3-diazol-4-yl | 4-F-Ph | -O-cyclobutyl |
| A-267. | 1-methyl-1,3-diazol-4-yl | 2-THP | -O-cyclobutyl |
| A-268. | 1-methyl-1,3-diazol-4-yl | 2-THF | -O-cyclobutyl |
| A-269. | 1-methyl-1,2,3-triazol-4-yl | Ph | -O-cyclobutyl |
| A-270. | 1-methyl-1,2,3-triazol-4-yl | 4-F-Ph | -O-cyclobutyl |
| A-271. | 1-methyl-1,2,3-triazol-4-yl | 2-THP | -O-cyclobutyl |
| A-272. | 1-methyl-1,2,3-triazol-4-yl | 2-THF | -O-cyclobutyl |
| A-273. | 1-methyl-1,3-diazol-4-yl | Ph | -O-cyclopentyl |
| A-274. | 1-methyl-1,3-diazol-4-yl | 4-F-Ph | -O-cyclopentyl |
| A-275. | 1-methyl-1,3-diazol-4-yl | 2-THP | -O-cyclopentyl |
| A-276. | 1-methyl-1,3-diazol-4-yl | 2-THF | -O-cyclopentyl |
| A-277. | 1-methyl-1,2,3-triazol-4-yl | Ph | -O-cyclopentyl |
| A-278. | 1-methyl-1,2,3-triazol-4-yl | 4-F-Ph | -O-cyclopentyl |
| A-279. | 1-methyl-1,2,3-triazol-4-yl | 2-THP | -O-cyclopentyl |
| A-280. | 1-methyl-1,2,3-triazol-4-yl | 2-THF | -O-cyclopentyl |
| A-281. | 1-methyl-1,3-diazol-4-yl | Ph | -O-cyclohexyl |
| A-282. | 1-methyl-1,3-diazol-4-yl | 4-F-Ph | -O-cyclohexyl |
| A-283. | 1-methyl-1,3-diazol-4-yl | 2-THP | -O-cyclohexyl |
| A-284. | 1-methyl-1,3-diazol-4-yl | 2-THF | -O-cyclohexyl |
| A-285. | 1-methyl-1,2,3-triazol-4-yl | Ph | -O-cyclohexyl |
| A-286. | 1-methyl-1,2,3-triazol-4-yl | 4-F-Ph | -O-cyclohexyl |
| A-287. | 1-methyl-1,2,3-triazol-4-yl | 2-THP | -O-cyclohexyl |

| | R¹ | R^{3a} | R^{13b} |
|---|---|---|---|
| A-288. | 1-methyl-1,2,3-triazol-4-yl | 2-THF | -O-cyclohexyl |
| A-289. | 1-methyl-1,3-diazol-4-yl | Ph | -OCH₂Ph |
| A-290. | 1-methyl-1,3-diazol-4-yl | 4-F-Ph | -OCH₂Ph |
| A-291. | 1-methyl-1,3-diazol-4-yl | 2-THP | -OCH₂Ph |
| A-292. | 1-methyl-1,3-diazol-4-yl | 2-THF | -OCH₂Ph |
| A-293. | 1-methyl-1,2,3-triazol-4-yl | Ph | -OCH₂Ph |
| A-294. | 1-methyl-1,2,3-triazol-4-yl | 4-F-Ph | -OCH₂Ph |
| A-295. | 1-methyl-1,2,3-triazol-4-yl | 2-THP | -OCH₂Ph |
| A-296. | 1-methyl-1,2,3-triazol-4-yl | 2-THF | -OCH₂Ph |
| A-297. | 1-methyl-1,3-diazol-4-yl | Ph | -SO₂Me |
| A-298. | 1-methyl-1,3-diazol-4-yl | 4-F-Ph | -SO₂Me |
| A-299. | 1-methyl-1,3-diazol-4-yl | 2-THP | -SO₂Me |
| A-300. | 1-methyl-1,3-diazol-4-yl | 2-THF | -SO₂Me |
| A-301. | 1-methyl-1,2,3-triazol-4-yl | Ph | -SO₂Me |
| A-302. | 1-methyl-1,2,3-triazol-4-yl | 4-F-Ph | -SO₂Me |
| A-303. | 1-methyl-1,2,3-triazol-4-yl | 2-THP | -SO₂Me |
| A-304. | 1-methyl-1,2,3-triazol-4-yl | 2-THF | -SO₂Me |
| A-305. | 1-methyl-1,3-diazol-4-yl | Ph | 2-pyridyl |
| A-306. | 1-methyl-1,3-diazol-4-yl | 4-F-Ph | 2-pyridyl |
| A-307. | 1-methyl-1,3-diazol-4-yl | 2-THP | 2-pyridyl |
| A-308. | 1-methyl-1,3-diazol-4-yl | 2-THF | 2-pyridyl |
| A-309. | 1-methyl-1,2,3-triazol-4-yl | Ph | 2-pyridyl |
| A-310. | 1-methyl-1,2,3-triazol-4-yl | 4-F-Ph | 2-pyridyl |
| A-311. | 1-methyl-1,2,3-triazol-4-yl | 2-THP | 2-pyridyl |
| A-312. | 1-methyl-1,2,3-triazol-4-yl | 2-THF | 2-pyridyl |
| A-313. | 1-methyl-1,3-diazol-4-yl | Ph | 3-pyridyl |
| A-314. | 1-methyl-1,3-diazol-4-yl | 4-F-Ph | 3-pyridyl |
| A-315. | 1-methyl-1,3-diazol-4-yl | 2-THP | 3-pyridyl |
| A-316. | 1-methyl-1,3-diazol-4-yl | 2-THF | 3-pyridyl |
| A-317. | 1-methyl-1,2,3-triazol-4-yl | Ph | 3-pyridyl |
| A-318. | 1-methyl-1,2,3-triazol-4-yl | 4-F-Ph | 3-pyridyl |
| A-319. | 1-methyl-1,2,3-triazol-4-yl | 2-THP | 3-pyridyl |
| A-320. | 1-methyl-1,2,3-triazol-4-yl | 2-THF | 3-pyridyl |
| A-321. | 1-methyl-1,3-diazol-4-yl | Ph | 4-pyridyl |
| A-322. | 1-methyl-1,3-diazol-4-yl | 4-F-Ph | 4-pyridyl |
| A-323. | 1-methyl-1,3-diazol-4-yl | 2-THP | 4-pyridyl |

| | R¹ | R^{3a} | R^{13b} |
|---|---|---|---|
| A-324. | 1-methyl-1,3-diazol-4-yl | 2-THF | 4-pyridyl |
| A-325. | 1-methyl-1,2,3-triazol-4-yl | Ph | 4-pyridyl |
| A-326. | 1-methyl-1,2,3-triazol-4-yl | 4-F-Ph | 4-pyridyl |
| A-327. | 1-methyl-1,2,3-triazol-4-yl | 2-THP | 4-pyridyl |
| A-328. | 1-methyl-1,2,3-triazol-4-yl | 2-THF | 4-pyridyl |
| A-329. | 1-methyl-1,3-diazol-4-yl | Ph | 5-pyrimidyl |
| A-330. | 1-methyl-1,3-diazol-4-yl | 4-F-Ph | 5-pyrimidyl |
| A-331. | 1-methyl-1,3-diazol-4-yl | 2-THP | 5-pyrimidyl |
| A-332. | 1-methyl-1,3-diazol-4-yl | 2-THF | 5-pyrimidyl |
| A-333. | 1-methyl-1,2,3-triazol-4-yl | Ph | 5-pyrimidyl |
| A-334. | 1-methyl-1,2,3-triazol-4-yl | 4-F-Ph | 5-pyrimidyl |
| A-335. | 1-methyl-1,2,3-triazol-4-yl | 2-THP | 5-pyrimidyl |
| A-336. | 1-methyl-1,2,3-triazol-4-yl | 2-THF | 5-pyrimidyl |
| A-337. | 1-methyl-1,3-diazol-4-yl | Ph | 4-piperidyl |
| A-338. | 1-methyl-1,3-diazol-4-yl | 4-F-Ph | 4-piperidyl |
| A-339. | 1-methyl-1,3-diazol-4-yl | 2-THP | 4-piperidyl |
| A-340. | 1-methyl-1,3-diazol-4-yl | 2-THF | 4-piperidyl |
| A-341. | 1-methyl-1,2,3-triazol-4-yl | Ph | 4-piperidyl |
| A-342. | 1-methyl-1,2,3-triazol-4-yl | 4-F-Ph | 4-piperidyl |
| A-343. | 1-methyl-1,2,3-triazol-4-yl | 2-THP | 4-piperidyl |
| A-344. | 1-methyl-1,2,3-triazol-4-yl | 2-THF | 4-piperidyl |

Further particular compounds of the present invention are the pyrrolidine derivatives disclosed in preparation examples and physiologically tolerated salts thereof. These include for each preparation example the exemplified compound as well as the corresponding free base and any other physiologically tolerated salts of the free base (if the exemplified compound is a salt), or any physiologically tolerated salt of the free base (if the exemplified compound is a free base). These further include enantiomers, diastereomers, tautomers and any other isomeric forms of said compounds, be they explicitly or implicitly disclosed.

It is noted that some pyrrolidine derivatives of the formula (I) are described in the prior art and therefore the present invention does not encompass these compounds (and physiologically tolerated salts thereof) *per se* as defined at the end of claim 1. However, the present invention does encompass pharmaceutical compositions comprising such pyrrolidine derivatives, and such pyrrolidine derivatives for therapeutic purposes, as described herein.

The compounds of the formula (I) can be prepared by analogy to methods which are well known in the art. Suitable methods for the preparation of compounds of formula (I) are outlined in the following schemes.

The process depicted in scheme 1 is useful for obtaining pyrrolidine derivatives, wherein Y¹ is >CH- and R⁴ is -N(CR^{9a}R^{9b})ₙ₄R¹³, -NHCOR¹⁴ or -NHCONHR^{16a}.

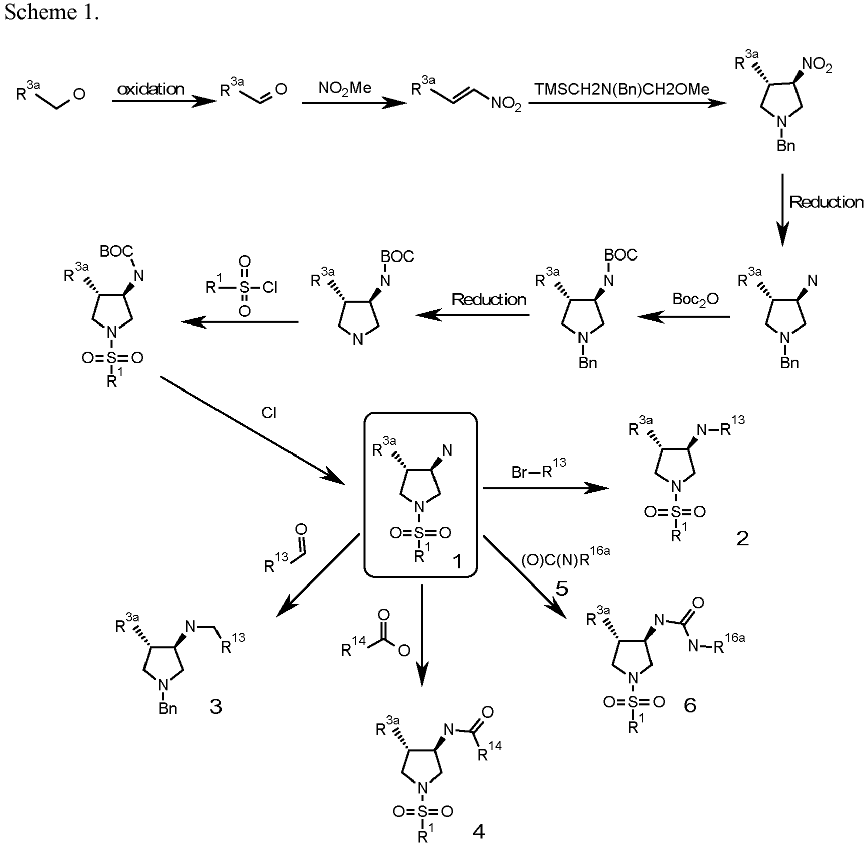

In scheme 1, the variables R¹, R^{3a}, R¹³, R¹⁴, R^{16a} are as defined herein.

As shown in the above scheme 1, the intermediate of general formula 1 can be alkylated to give compounds of formula 2. Alternatively, 1 can be transformed to compounds 3 via reductive amination or can yield amides of formula 4 by coupling reaction with the corresponding acid. Reaction with isocyanates of formula 5 affords compounds of formula 6.

The process depicted in scheme 2 is useful for obtaining pyrrolidine derivatives, wherein Y¹ is >CH- and R⁴ is -(CR^{7c}R^{7d})ₙ₂NR^{11a}R^{11b} or -CONR^{20a}R^{20b}.

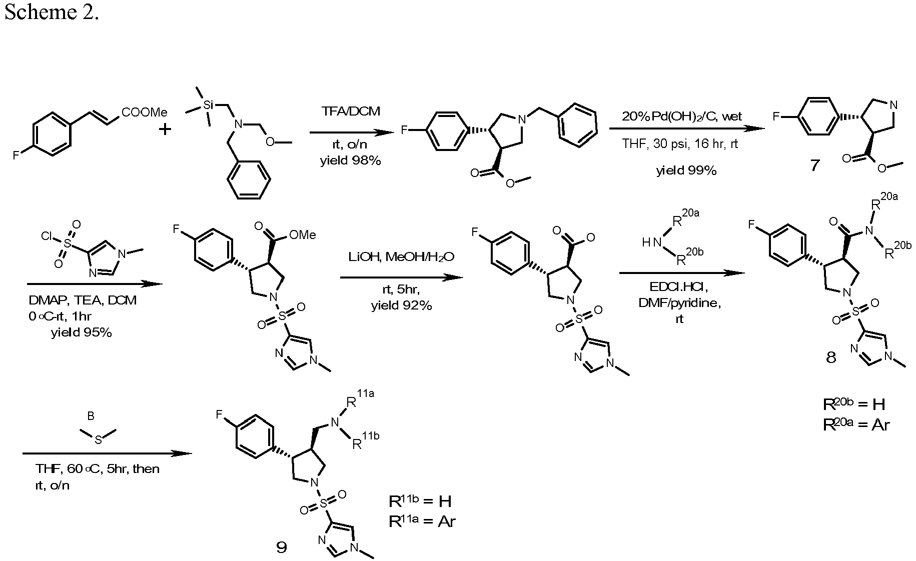

As shown in the above scheme 2, cycloaddition followed by benzyl deprotection afford pyrrolidine 7. Sulfonylation, methylester hydrolysis and coupling with amines yield compounds of formula 8. Reduction of compounds of formula 8 affords the corresponding amines 9.

The process depicted in scheme 3 is useful for obtaining pyrrolidine derivatives, wherein Y¹ is >CH- and R⁴ is -(CR^{7a}R^{7b})ₙ₁NR¹⁰.

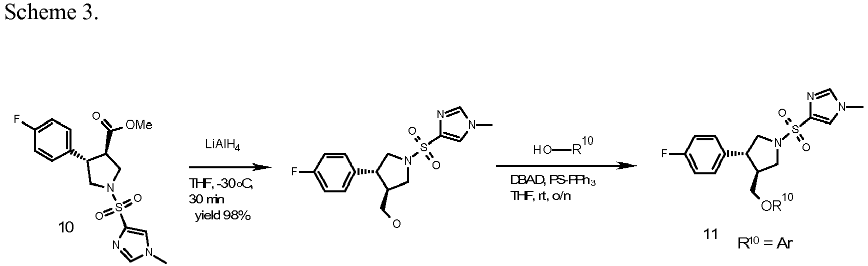

As shown in the above scheme 3, reduction with LiAlH₄ of intermediate 10 affords the corresponding alcohol which then can be transformed in the alkoxy derivatives of formula 11 via Mitsunobu coupling.

The process depicted in scheme 4 is useful for obtaining pyrrolidine derivatives, wherein Y¹ is >CH- and R⁴ is -COR¹⁹.

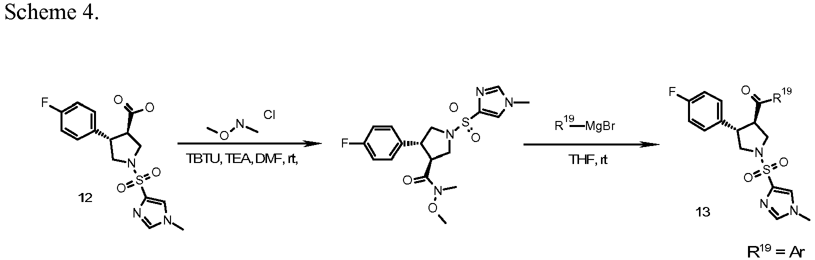

As shown in the above scheme 4, the pyrrolidine intermediate 12 can be converted in the corresponding Weinreb amide. Treatment of the Weinreb amide with Grignard reagents affords the ketones of formula 13.

The process depicted in scheme 5 is useful for obtaining pyrrolidine derivatives, wherein Y¹ is >CH- and R⁴ is -O(CR^{9c}R^{9d})ₙ₅R¹⁸.

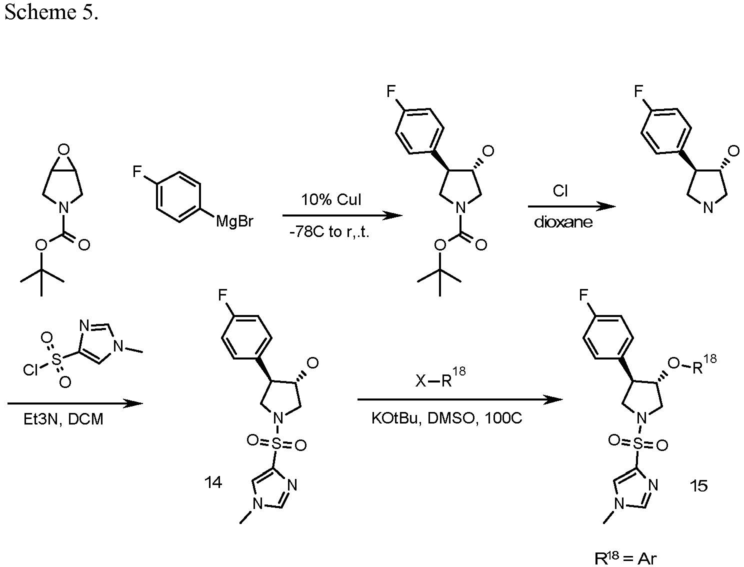

As shown in the above scheme 5, alkylation of alcohol 14 affords the compounds of formula 15.

The process depicted in scheme 6 is useful for obtaining pyrrolidine derivatives, wherein Y¹ is >CH-, R⁴ is -N(CR^{9a}R⁹b)ₙ₄R¹³ and R^{3a} or R^{3b} is C₃-C₁₂-heterocyclyl.

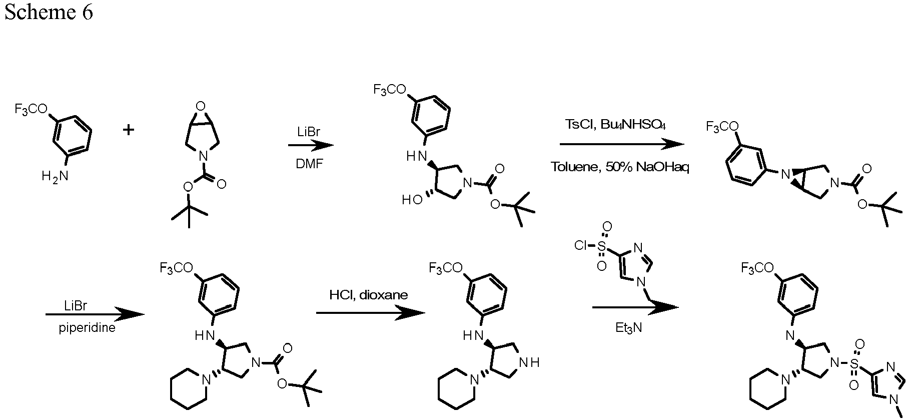

The process depicted in scheme 7 is useful for obtaining pyrrolidine derivatives, wherein Y¹ is >CH-, and R^{3a} or R^{3b} is optionally substituted C₃-C₁₂-heterocyclyl.

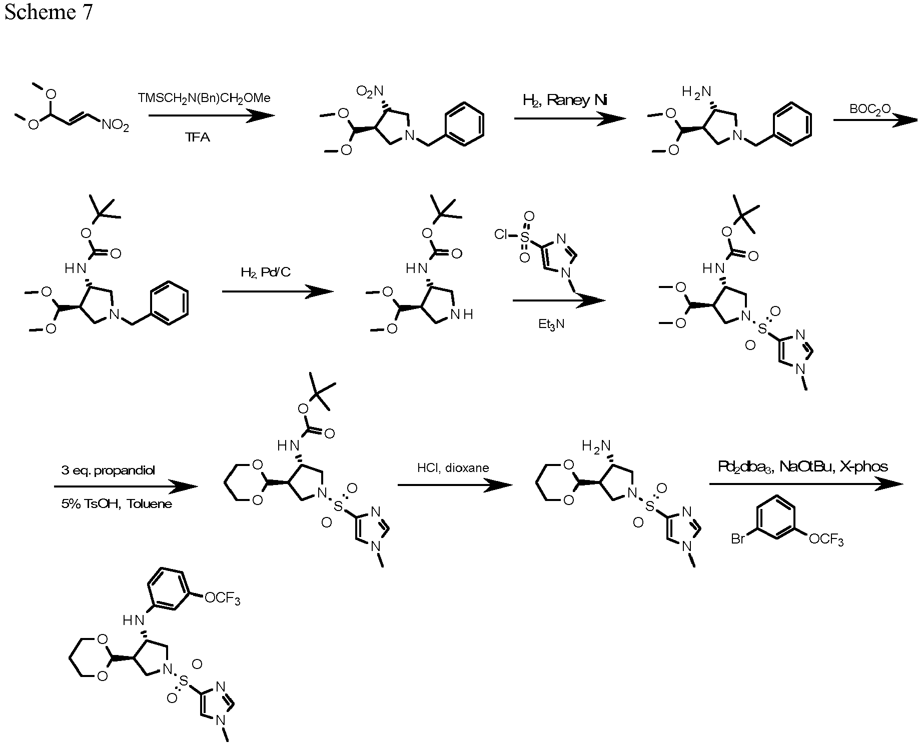

The process depicted in scheme 8 is useful for obtaining pyrrolidine derivatives, wherein Y¹ is >CR⁶- and R⁶ and R^{3a} or R^{3b} together are optionally substituted C₁-C₅-alkylene.

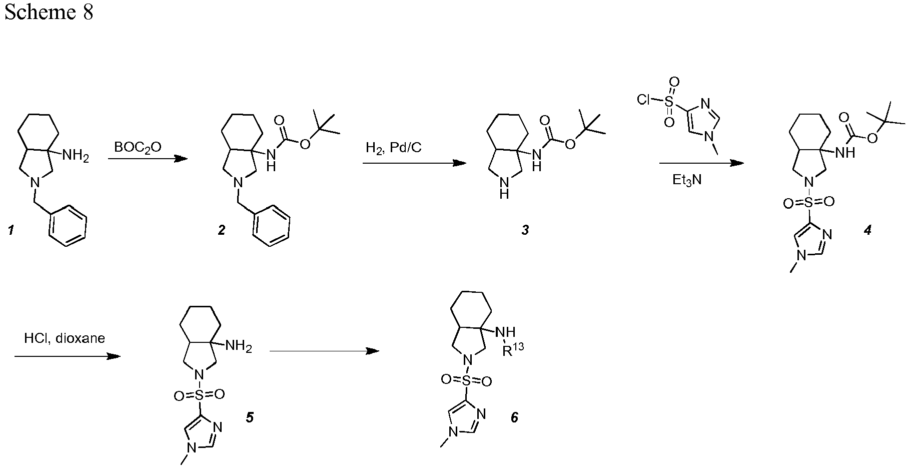

The processes depicted in schemes 9 and 10 are useful for obtaining pyrrolidine derivatives, wherein Y¹ is >CR⁶- and R⁶ is C₁-C₄-alkylene that is bound to a carbon atom in R^{3a}, and R^{3a} is an optionally substituted C₆-C₁₂-aryl or an optionally substituted C₃-C₁₂-heterocyclyl.

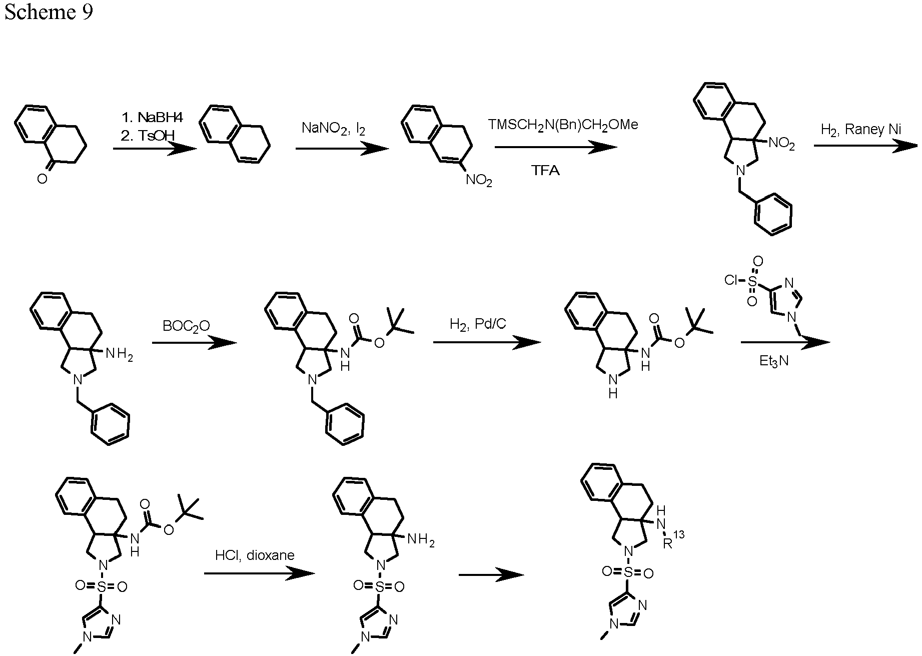

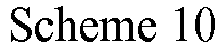

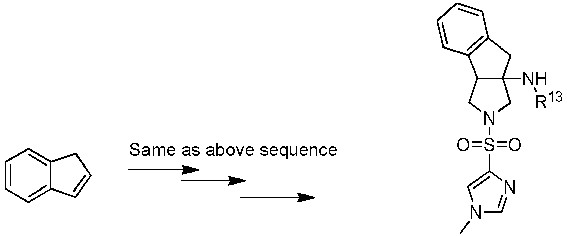

The process depicted in scheme 11 is useful for obtaining pyrrolidine derivatives, wherein Y¹ is >CH-, R⁴ is -COR¹⁹ and R^{3a} and R^{3b} together are optionally substituted C₂-C₅-alkylene.

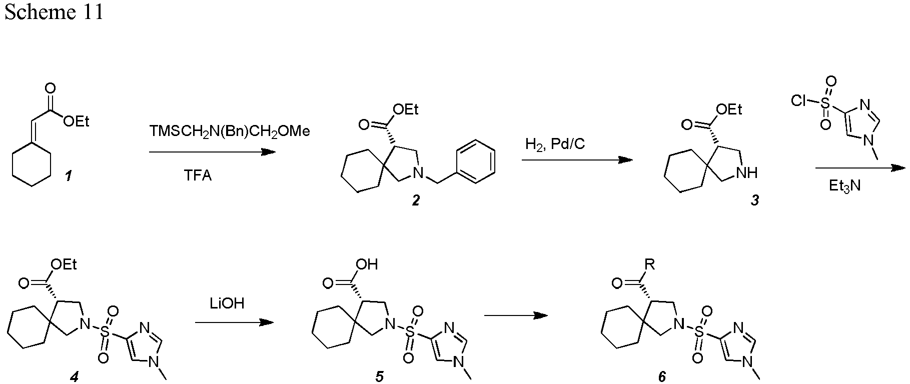

The process depicted in scheme 12 is useful for obtaining pyrrolidine derivatives, wherein Y¹ is >CH-, R⁴ is -N(CR^{9a}R⁹b)ₙ₄R¹³ and one of R^{2a} or R^{2b} and one of R^{5a} or R^{5b} together are optionally substituted C₁-C₅-alkylene.

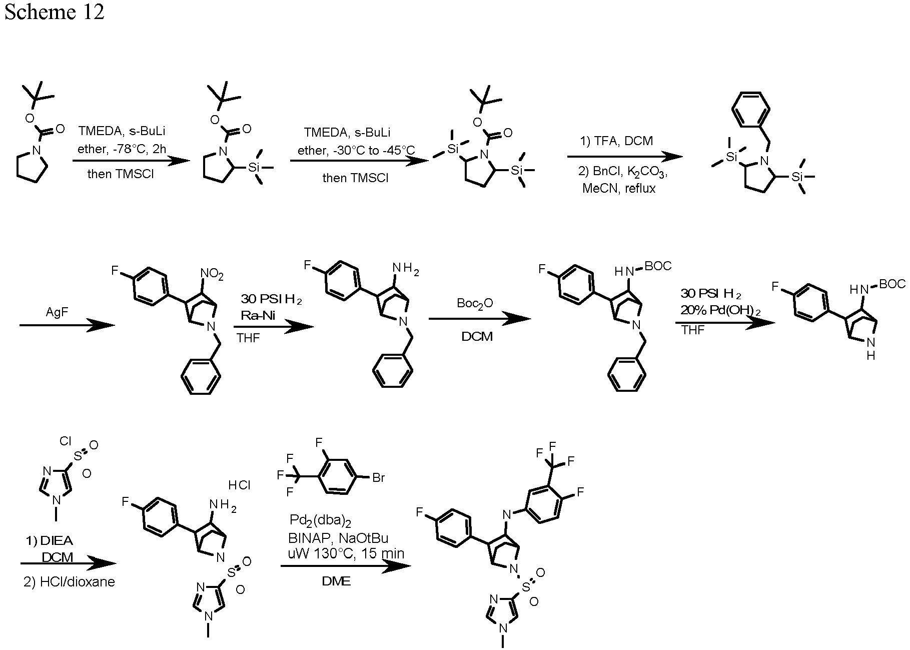

The process depicted in scheme 13 is useful for obtaining pyrrolidine derivatives, wherein Y¹ is >CH-, R⁴ is -N(CR^{9a}R⁹b)ₙ₄R¹³ and R^{3a} or R^{3b} is optionally substituted C₃-C₁₂-heterocyclyl.

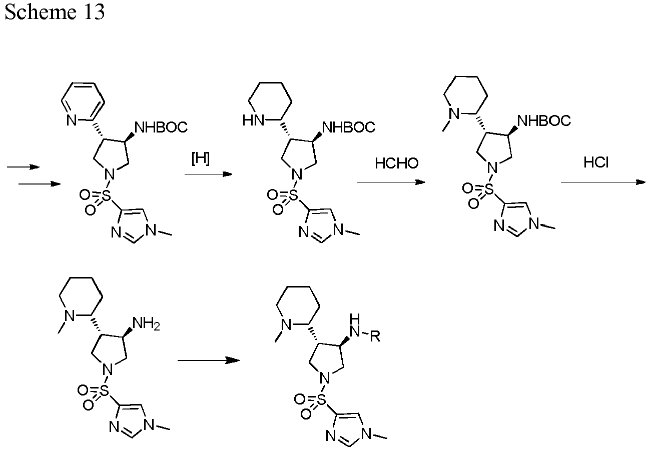

The process depicted in scheme 14 is useful for obtaining pyrrolidine derivatives, wherein Y¹ is >CH- and R^{3a} and one of R^{2a} or R^{2b} together with the carbon atoms to which they are bound form an anellated C₆-C₁₂-aryl.

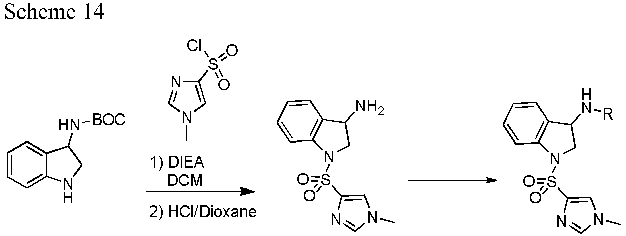

Schemes 1 to 14 refer also to the preparation of the enantiomers, diastereomers, tautomers and any other isomeric forms of said compounds, be they explicitly or implicitly disclosed.

The acid addition salts of the pyrrolidine derivatives of formula (I) are prepared in a customary manner by mixing the free base with a corresponding acid, optionally in solution in an organic solvent, for example a lower alcohol, such as methanol, ethanol or propanol, an ether, such as methyl tert-butyl ether or diisopropyl ether, a ketone, such as acetone or methyl ethyl ketone, or an ester, such as ethyl acetate.

The compounds of the formula (I) are capable of inhibiting the activity of glycine transporter, in particular glycine transporter 1 (GlyT1).

The utility of the compounds in accordance with the present invention as inhibiting the glycine transporter activity, in particular GlyT1 activity, may be demonstrated by methodology known in the art. For instance, human GlyT1c expressing recombinant hGlyT1c_5_CHO cells can be used for measuring glycine uptake and its inhibition (IC₅₀) by a compound of formula (I).

Amongst the compounds of the formula (I) those are preferred which achieve effective inhibition at low concentrations. In particular, compounds of the formula (I) are preferred which inhibit glycine transporter 1 (GlyT1) at a level of IC₅₀ < 1 µMol, more preferably at a level of IC₅₀ < 0.5 µMol, particularly preferably at a level of IC₅₀ < 0.2 µMol and most preferably at a level of IC₅₀ < 0.1 µMοl.

The compounds of formula (I) display good to moderate metabolic stability.

The metabolic stability of a compound can be measured for example by incubating a solution of this compound with liver microsomes from particular species (for example rat, dog or human) and determining the half-life of the compound under these conditions (RS Obach, Curr Opin Drug Discov Devel. 2001, 4, 36-44). It is possible in this connection to conclude from an observed longer half-life that the metabolic stability of the compound is improved. The stability in the presence of human liver microsomes is of particular interest because it makes it possible to predict the metabolic degradation of the compound in the human liver. Compounds with increased metabolic stability (measured in the liver microsome test) are therefore probably also degraded more slowly in the liver. The slower metabolic degradation in the liver may lead to higher and/or longer-lasting concentrations (active levels) of the compound in the body, so that the elimination half-life of the compounds of the invention is increased. Increased and/or longer-lasting active levels may lead to a better activity of the compound in therapeutic treatment. In addition, an improved metabolic stability may lead to an increased bioavailability after oral administration, because the compound is subject, after absorption in the intestine, to less metabolic degradation in the liver (so-called first pass effect). An increased oral bioavailability may, owing to an increased concentration (active level) of the compound, lead to a better activity of the compound after oral administration.

Amongst the compounds of the formula (I) those are particularly preferred which display good to moderate metabolic stability towards human liver microsomes. In particular, compounds of the formula (I) are preferred which display a microsomal clearance at a level of mCl < 1000 µl/min/mg (mClint,u < 500 L/h/kg), more preferably at a level of mCl < 500 µl/min/mg (mClint,u < 100L/h/kg), particularly preferably at a level of mCl < 100 µl/min/mg (mClint,u < 50L/h/kg) and most preferably at a level of mCl < 50 µl/min/mg (mClint,u < 5 L/h/kg).

Further, compounds of formula (I) exhibit favorable efflux properties which may lead to enhanced oral bioavailability and/or increased brain availability. According to a particular embodiment, compounds of the invention combine high affinity and high metabolic stability with favorable efflux properties.

The efflux properties of a compound can be measured in well-known assays (e.g. Caco-2, MDCK assay).

The compounds of the formula (I) according to the present invention are thus useful as pharmaceuticals.

The present invention therefore also relates to pharmaceutical compositions which comprise an inert carrier and a compound of the formula (I).

The present invention also relates to the use of the compounds of the formula (I) in the manufacture of a medicament for inhibiting the glycine transporter GlyT1, and to corresponding methods of inhibiting the glycine transporter GlyT1.

The NMDA receptor is central to a wide range of CNS processes, and its role in a variety of diseases in humans or other species has been described. GlyT1 inhibitors slow the removal of glycine from the synapse, causing the level of synaptic glycine to rise. This in turn increases the occupancy of the glycine binding site on the NMDA receptor, which increases activation of the NMDA receptor following glutamate release from the presynaptic terminal. Glycine transport inhibitors and in particular inhibitors of the glycine transporter GlyT1 are thus known to be useful in treating a variety of neurologic and psychiatric disorders. Further, glycine A receptors play a role in a variety of diseases in humans or other species. Increasing extracellular glycine concentrations by inhibiting glycine transport may enhance the activity of glycine A receptors. Glycine transport inhibitors and in particular inhibitors of the glycine transporter GlyT1 are thus useful in treating a variety of neurologic and psychiatric disorders.

The present invention thus further relates to the use of the compounds of the formula (I) for the manufacture of a medicament for treating a neurologic or psychiatric disorder, and to compounds of formula (I) for use in corresponding methods of treating said disorders.

According to a particular embodiment, the disorder is associated with glycinergic or glutamatergic neurotransmission dysfunction.

According to a further particular embodiment, the disorder is one or more of the following conditions or diseases: schizophrenia or a psychotic disorder including schizophrenia (paranoid, disorganized, catatonic or undifferentiated), schizophreniform disorder, schizoaffective disorder, delusional disorder, brief psychotic disorder, shared psychotic disorder, psychotic disorder due to a general medical condition and substance- induced psychotic disorder, including both the positive and the negative symptoms of schizophrenia and other psychoses; cognitive disorders including dementia (associated with Alzheimer's disease, ischemia, multi-infarct dementia, trauma, vascular problems or stroke, HIV disease, Parkinson's disease, Huntington's disease, Pick's disease, Creutz-feldt-Jacob disease, perinatal hypoxia, other general medical conditions or substance abuse); delirium, amnestic disorders or cognitive impairment including age related cognitive decline; anxiety disorders including acute stress disorder, agoraphobia, generalized anxiety disorder, obsessive-compulsive disorder, panic attack, panic disorder, post-traumatic stress disorder, separation anxiety disorder, social phobia, specific phobia, substance-induced anxiety disorder and anxiety due to a general medical condition; substance-related disorders and addictive behaviors (including substance-induced delirium, persisting dementia, persisting amnestic disorder, psychotic disorder or anxiety disorder; tolerance, dependence or withdrawal from substances including alcohol, amphetamines, cannabis, cocaine, hallucinogens, inhalants, nicotine, opioids, phencyclidine, sedatives, hypnotics or anxiolytics); obesity, bulimia nervosa and compulsive eating disorders; bipolar disorders, mood disorders including depressive disorders; depression including unipolar depression, seasonal depression and post-partum depression, premenstrual syndrome (PMS) and premenstrual dysphoric disorder (PDD), mood disorders due to a general medical condition, and substance-induced mood disorders; learning disorders, pervasive developmental disorder including autistic disorder, attention deficit disorders including attention-deficit hyperactivity disorder (ADHD) and conduct disorder; movement disorders, including akinesias and akinetic-rigid syndromes (including Parkinson's disease, drug-induced parkinsonism, postencephalitic parkinsonism, progressive supranuclear palsy, multiple system atrophy, corticobasal degeneration, parkinsonism-ALS dementia complex and basal ganglia calcification), medication-induced parkinsonism (such as neuroleptic-induced parkinsonism, neuroleptic malignant syndrome, neuroleptic-induced acute dystonia, neuroleptic-induced acute akathisia, neuroleptic-induced tardive dyskinesia and medication-induced postural tremor), Gilles de la Tourette's syndrome, epilepsy, muscular spasms and disorders associated with muscular spasticity or weakness including tremors; dyskinesias [including tremor (such as rest tremor, postural tremor and intention tremor), chorea (such as Sydenham's chorea, Huntington's disease, benign hereditary chorea, neuroacanthocytosis, symptomatic chorea, drug-induced chorea and hemiballism), myoclonus (including generalised myoclonus and focal myoclonus), tics (including simple tics, complex tics and symptomatic tics), and dystonia (including generalised dystonia such as iodiopathic dystonia, drug-induced dystonia, symptomatic dystonia and paroxymal dystonia, and focal dystonia such as blepharospasm, oromandibular dystonia, spasmodic dysphonia, spasmodic torticollis, axial dystonia, dystonic writer's cramp and hemiplegic dystonia)]; urinary incontinence; neuronal damage including ocular damage, retinopathy or macular degeneration of the eye, tinnitus, hearing impairment and loss, and brain edema; emesis; and sleep disorders including insomnia and narcolepsy.

According to a further particular embodiment, the disorder is pain, in particular chronic pain and especially neuropathic pain.

Pain can be classified as acute and chronic pain. Acute pain and chronic pain differ in their etiology, pathophysiology, diagnosis and treatment.

Acute pain, which occurs following tissue injury, is self-limiting, serves as an alert to ongoing tissue damage and following tissue repair it will usually subside. There are minimal psychological symptoms associated with acute pain apart from mild anxiety. Acute pain is nociceptive in nature and occurs following chemical, mechanical and thermal stimulation of A-delta and C-polymodal pain receptors.

Chronic pain, on the other hand, serves no protective biological function. Rather than being the symptom of tissue damage it is a disease in its own right. Chronic pain is unrelenting and not self-limiting and can persist for years, perhaps decades after the initial injury. Chronic pain can be refractory to multiple treatment regimes. Psychological symptoms associated with chronic pain include chronic anxiety, fear, depression, sleeplessness and impairment of social interaction. Chronic non-malignant pain is predominantly neuropathic in nature and involves damage to either the peripheral or central nervous systems.

Acute pain and chronic pain are caused by different neuro-physiological processes and therefore tend to respond to different types of treatments. Acute pain can be somatic or visceral in nature. Somatic pain tends to be a well localised, constant pain and is described as sharp, aching, throbbing or gnawing. Visceral pain, on the other hand, tends to be vague in distribution, paroxysmal in nature and is usually described as deep, aching, squeezing or colicky in nature. Examples of acute pain include post-operative pain, pain associated with trauma and the pain of arthritis. Acute pain usually responds to treatment with opioids or non-steroidal anti-inflammatory drugs.

Chronic pain, in contrast to acute pain, is described as burning, electric, tingling and shooting in nature. It can be continuous or paroxysmal in presentation. The hallmarks of chronic pain are chronic allodynia and hyperalgesia. Allodynia is pain resulting from a stimulus that normally does not ellicit a painful response, such as a light touch. Hyperalgesia is an increased sensitivity to normally painful stimuli. Primary hyperalgesia occurs immediately within the area of the injury. Secondary hyperalgesia occurs in the undamaged area surrounding the injury. Examples of chronic pain include complex regional pain syndrome, pain arising from peripheral neuropathies, post-operative pain, chronic fatigue syndrome pain, tension-type headache, pain arising from mechanical nerve injury and severe pain associated with diseases such as cancer, metabolic disease, neuro-tropic viral disease, neurotoxicity, inflammation, multiple sclerosis or any pain arising as a consequence of or associated with stress or depressive illness.

Although opioids are cheap and effective, serious and potentially life-threatening side effects occur with their use, most notably respiratory depression and muscle rigidity. In addition the doses of opioids which can be administered are limited by nausea, emesis, constipation, pruritis and urinary retention, often resulting in patients electing to receive sub-optimal pain control rather than suffer these distressing side-effects. Furthermore, these side-effects often result in patients requiring extended hospitalisation. Opioids are highly addictive and are scheduled drugs in many territories.

The compounds of formula (I) are particularly useful in the treatment of schizophrenia, bipolar disorder, depression including unipolar depression, seasonal depression and post-partum depression, premenstrual syndrome (PMS) and premenstrual dysphoric disorder (PDD), learning disorders, pervasive developmental disorder including autistic disorder, attention deficit disorders including Attention-Deficit/Hyperactivity Disorder, tic disorders including Tourette's disorder, anxiety disorders including phobia and post traumatic stress disorder, cognitive disorders associated with dementia, AIDS dementia, Alzheimer's, Parkinson's, Huntington's disease, spasticity, myoclonus, muscle spasm, tinnitus and hearing impairment and loss are of particular importance.

Particular cognitive disorders are dementia, delirium, amnestic disorders and cognitive impartment including age-related cognitive decline.

Particular anxiety disorders are generalized anxiety disorder, obsessive-compulsive disorder and panic attack.

Particular schizophrenia or psychosis pathologies are paranoid, disorganized, catatonic or undifferentiated schizophrenia and substance-induced psychotic disorder.

Particular neurologic disorders that can be treated with the compounds of of the formula (I) include in particular a cognitive disorder such as dementia, cognitive impairment, attention deficit hyperactivity disorder.

Particular psychiatric disorders that can be treated with the compounds of of the formula (I) include in particular an anxiety disorder, a mood disorder such as depression or a bipolar disorder, schizophrenia, a psychotic disorder.

Within the context of the treatment, the use according to the invention of the compounds of the formula (I) involves a method. In this method, an effective quantity of one or more compounds or the formula (I), as a rule formulated in accordance with pharmaceutical and veterinary practice, is administered to the individual to be treated, preferably a mammal, in particular a human being. Whether such a treatment is indicated, and in which form it is to take place, depends on the individual case and is subject to medical assessment (diagnosis) which takes into consideration signs, symptoms and/or malfunctions which are present, the risks of developing particular signs, symptoms and/or malfunctions, and other factors.

As a rule, the treatment is effected by means of single or repeated daily administration, where appropriate together, or alternating, with other drugs or drug-containing preparations.

The invention also relates to the manufacture of pharmaceutical compositions for treating an individual, preferably a mammal, in particular a human being. Thus, the compounds of the formula (I) are customarily administered in the form of pharmaceutical compositions which comprise an inert carrier (e.g. a pharmaceutically acceptable excipient) together with at least one compound according to the invention and, where appropriate, other drugs. These compositions can, for example, be administered orally, rectally, transdermally, subcutaneously, intravenously, intramuscularly or intranasally.

Examples of suitable pharmaceutical formulations are solid medicinal forms, such as powders, granules, tablets, in particular film tablets, lozenges, sachets, cachets, sugar-coated tablets, capsules, such as hard gelatin capsules and soft gelatin capsules, suppositories or vaginal medicinal forms, semisolid medicinal forms, such as ointments, creams, hydrogels, pastes or plasters, and also liquid medicinal forms, such as solutions, emulsions, in particular oil-in-water emulsions, suspensions, for example lotions, injection preparations and infusion preparations, and eyedrops and eardrops. Implanted release devices can also be used for administering inhibitors according to the invention. In addition, it is also possible to use liposomes or microspheres.

When producing the compositions, the compounds according to the invention are optionally mixed or diluted with one or more carriers (excipients). Carriers (excipients) can be solid, semisolid or liquid materials which serve as vehicles, carriers or medium for the active compound.

Suitable carriers (excipients) are listed in the specialist medicinal monographs. In addition, the formulations can comprise pharmaceutically acceptable auxiliary substances, such as wetting agents; emulsifying and suspending agents; preservatives; antioxidants; antiirritants; chelating agents; coating auxiliaries; emulsion stabilizers; film formers; gel formers; odor masking agents; taste corrigents; resin; hydrocolloids; solvents; solubilizers; neutralizing agents; diffusion accelerators; pigments; quaternary ammonium compounds; refatting and overfatting agents; raw materials for ointments, creams or oils; silicone derivatives; spreading auxiliaries; stabilizers; sterilants; suppository bases; tablet auxiliaries, such as binders, fillers, glidants, disintegrants or coatings; propellants; drying agents; opacifiers; thickeners; waxes; plasticizers and white mineral oils. A formulation in this regard is based on specialist knowledge as described, for example, in Fiedler, H.P., Lexikon der Hilfsstoffe für Pharmazie, Kosmetik und angrenzende Gebiete [Encyclopedia of auxiliary substances for pharmacy, cosmetics and related fields], 4th edition, Aulendorf: ECV-Editio-Cantor-Verlag, 1996.

The compounds of formula (I) may also be suitable for combination with other therapeutic agents.

Thus, the present invention also provides:
i) a combination comprising a compound of formula (I) with one or more further therapeutic agents;
ii) a pharmaceutical composition comprising a combination product as defined in i) above and at least one carrier, diluent or excipient;
iii) the use of a combination as defined in i) above in the manufacture of a medicament for treating or preventing a disorder, disease or condition as defined herein;
iv) a combination as defined in i) above for use in treating or preventing a disorder, disease or condition as defined herein;
v) a kit-of-parts for use in the treatment of a disorder, disease or condition as defined herein, comprising a first dosage form comprising a compound of formula (I) and one or more further dosage forms each comprising one or more further therapeutic agents for simultaneous therapeutic administration,
vi) a combination as defined in i) above for use in therapy;
vii) a method of treatment or prevention of a disorder, disease or condition as defined herein comprising administering an effective amount of a combination as defined in i) above;
viii) a combination as defined in i) above for treating or preventing a disorder, disease or condition as defined herein.

The combination therapies of the invention may be administered adjunctively. By adjunctive administration is meant the coterminous or overlapping administration of each of the components in the form of separate pharmaceutical compositions or devices. This regime of therapeutic administration of two or more therapeutic agents is referred to generally by those skilled in the art and herein as adjunctive therapeutic administration; it is also known as add-on therapeutic administration. Any and all treatment regimes in which a patient receives separate but coterminous or overlapping therapeutic administration of the compounds of formula (I) and at least one further therapeutic agent are within the scope of the current invention. In one embodiment of adjunctive therapeutic administration as described herein, a patient is typically stabilized on a therapeutic administration of one or more of the components for a period of time and then receives administration of another component.

The combination therapies of the invention may also be administered simultaneously. By simultaneous administration is meant a treatment regime wherein the individual components are administered together, either in the form of a single pharmaceutical composition or device comprising or containing both components, or as separate compositions or devices, each comprising one of the components, administered simultaneously. Such combinations of the separate individual components for simultaneous combination may be provided in the form of a kit-of-parts.

In a further aspect, the invention provides compounds of formula (I) for use in a method of treatment of a psychotic disorder by adjunctive therapeutic administration of compounds of formula (I) to a patient receiving therapeutic administration of at least one antipsychotic agent. In a further aspect, the invention provides the use of compounds of formula (I) in the manufacture of a medicament for adjunctive therapeutic administration for the treatment of a psychotic disorder in a patient receiving therapeutic administration of at least one antipsychotic agent. The invention further provides compounds of formula (I) for use for adjunctive therapeutic administration for the treatment of a psychotic disorder in a patient receiving therapeutic administration of at least one antipsychotic agent.

In a further aspect, the invention provides compounds of formula (I) for use in a method of treatment of a psychotic disorder by adjunctive therapeutic administration of at least one antipsychotic agent to a patient receiving therapeutic administration of compounds of formula (I). In a further aspect, at least one antipsychotic agent is used in the manufacture of a medicament for adjunctive therapeutic administration for the treatment of a psychotic disorder in a patient receiving therapeutic administration of compounds of formula (I). Further at least one antipsychotic agent is used for adjunctive therapeutic administration for the treatment of a psychotic disorder in a patient receiving therapeutic administration of compounds of formula (I).

In a further aspect, the invention provides compounds of formula (I) for use in a method of treatment of a psychotic disorder by simultaneous therapeutic administration of compounds of formula (I) in combination with at least one antipsychotic agent. The invention further provides the use of a combination of compounds of formula (I) and at least one antipsychotic agent in the manufacture of a medicament for simultaneous therapeutic administration in the treatment of a psychotic disorder. The invention further provides a combination of compounds of formula (I) and at least one antipsychotic agent for simultaneous therapeutic administration in the treatment of a psychotic disorder. The invention further provides the use of compounds of formula (I) in the manufacture of a medicament for simultaneous therapeutic administration with at least one antipsychotic agent in the treatment of a psychotic disorder. The invention further provides compounds of formula (I) for use for simultaneous therapeutic administration with at least one antipsychotic agent in the treatment of a psychotic disorder. Further least one antipsychotic agent is used in the manufacture of a medicament for simultaneous therapeutic administration with compounds of formula (I) in the treatment of a psychotic disorder. Further at least one antipsychotic agent is used for simultaneous therapeutic administration with compounds of formula (I) in the treatment of a psychotic disorder. In further aspects, the invention provides compounds of formula (I) for use in a method of treatment of a psychotic disorder by simultaneous therapeutic administration of a pharmaceutical composition comprising compounds of formula (I) and at least one mood stabilising or antimanic agent, a pharmaceutical composition comprising compounds of formula (I) and at least one mood stabilising or antimanic agent, the use of a pharmaceutical composition comprising compounds of formula (I) and at least one mood stabilising or antimanic agent in the manufacture of a medicament for the treatment of a psychotic disorder, and a pharmaceutical composition comprising compounds of formula (I) and at least one mood stabilising or antimanic agent for use in the treatment of a psychotic disorder.

Antipsychotic agents include both typical and atypical antipsychotic drugs. Examples of antipsychotic drugs that are useful in the present invention include, but are not limited to: butyrophenones, such as haloperidol, pimozide, and droperidol; phenothiazines, such as chlorpromazine, thioridazine, mesoridazine, trifluoperazine, perphenazine, fluphenazine, thiflupromazine, prochlorperazine, and acetophenazine; thioxanthenes, such as thiothixene and chlorprothixene; thienobenzodiazepines; dibenzodiazepines; benzisoxazoles; dibenzothiazepines; imidazolidinones; benziso- thiazolyl-piperazines; triazine such as lamotrigine; dibenzoxazepines, such as loxapine; dihydroindolones, such as molindone; aripiprazole; and derivatives thereof that have antipsychotic activity.

Examples of tradenames and suppliers of selected antipsychotic drugs are as follows: clozapine (available under the tradename CLOZARIL®, from Mylan, Zenith Goldline, UDL, Novartis); olanzapine (available under the tradename ZYPREX®, from Lilly); ziprasidone (available under the tradename GEODON®, from Pfizer); risperidone (available under the tradename RISPERDAL®, from Janssen); quetiapine fumarate (available under the tradename SEROQ-UEL®, from AstraZeneca); haloperidol (available under the tradename HALDOL®, from Ortho-McNeil); chlorpromazine (available under the tradename THORAZINE®, from SmithKline Beecham (GSK)); fluphenazine (available under the tradename PROLIXIN®, from Apothecon, Copley, Schering, Teva, and American Pharmaceutical Partners, Pasadena); thiothixene (available under the tradename NAVANE®, from Pfizer); trifluoperazine (10-[3-(4-methyl-1-piperazinyl)propyl]-2- (trifluoromethyl)phenothiazine dihydrochloride, available under the tradename STELAZINE®, from Smith Klein Beckman); perphenazine (available under the tradename TRILAFON®; from Schering); thioridazine (available under the tradename MELLARIL®; from Novartis, Roxane, HiTech, Teva, and Alpharma) ; molindone (available under the tradename MOBAN®, from Endo); and loxapine (available under the tradename LOXITANE(D; from Watson). Furthermore, benperidol (Glianimon®), perazine (Taxilan®) or melperone (Eunerpan®) may be used. Other antipsychotic drugs include promazine (available under the tradename SPAR-INE®), triflurpromazine (available under the tradename VESPRI N®), chlorprothixene (available under the tradename TARACTAN®), droperidol (available under the tradename INAPSINE®), acetophenazine (available under the tradename TINDAL®), prochlorperazine (available under the tradename COMPAZINE®), methotrimeprazine (available under the tradename NOZINAN®), pipotiazine (available under the tradename PIPOTRIL®), ziprasidone, and hoperidone.

In a further aspect, the invention provides compounds of formula (I) for use in a method of treatment of a neurodegenerative disorder such as Alzheimer Disease by adjunctive therapeutic administration of compounds of formula (I) to a patient receiving therapeutic administration of at least one agent suitable for the treatment of a neurodegenerative disorder such as Alzheimer Disease. In a further aspect, the invention provides the use of compounds of formula (I) in the manufacture of a medicament for adjunctive therapeutic administration for the treatment of a neurodegenerative disorder such as Alzheimer Disease in a patient receiving therapeutic administration of at least one agent suitable for the treatment of a neurodegenerative disorder such as Alzheimer Disease. The invention further provides compounds of formula (I) for use for adjunctive therapeutic administration for the treatment of a neurodegenerative disorder such as Alzheimer Disease in a patient receiving therapeutic administration of at least one agent suitable for the treatment of a neurodegenerative disorder such as Alzheimer Disease.

In a further aspect, a method of treatment of a neurodegenerative disorder such as Alzheimer Disease by adjunctive therapeutic administration of at least one agent suitable for the treatment of a neurodegenerative disorder such as Alzheimer Disease to a patient receiving therapeutic administration of compounds of formula (I) is disclosed. In a further aspect, the use of at least one agent suitable for the treatment of a neurodegenerative disorder such as Alzheimer Disease in the manufacture of a medicament for adjunctive therapeutic administration for the treatment of a neurodegenerative disorder such as Alzheimer Disease in a patient receiving therapeutic administration of compounds of formula (I) is disclosed. Further at least one agent suitable for the treatment of a neurodegenerative disorder such as Alzheimer Disease for adjunctive therapeutic administration for the treatment of a neurodegenerative disorder such as Alzheimer Disease in a patient receiving therapeutic administration of compounds of formula (I) is disclosed.

In a further aspect, the invention provides compounds of formula (I) for use in a method of treatment of a neurodegenerative disorder such as Alzheimer Disease by simultaneous therapeutic administration of compounds of formula (I) in combination with at least one agent suitable for the treatment of a neurodegenerative disorder such as Alzheimer Disease. The invention further provides the use of a combination of compounds of formula (I) and at least one agent suitable for the treatment of a neurodegenerative disorder such as Alzheimer Disease in the manufacture of a medicament for simultaneous therapeutic administration in the treatment of a neurodegenerative disorder such as Alzheimer Disease. The invention further provides a combination of compounds of formula (I) and at least one agent suitable for the treatment of a neurodegenerative disorder such as Alzheimer Disease for simultaneous therapeutic administration in the treatment of a neurodegenerative disorder such as Alzheimer Disease. The invention further provides the use of compounds of formula (I) in the manufacture of a medicament for simultaneous therapeutic administration with at least one agent suitable for the treatment of a neurodegenerative disorder such as Alzheimer Disease in the treatment of a neurodegenerative disorder such as Alzheimer Disease. The invention further provides compounds of formula (I) for use for simultaneous therapeutic administration with at least one agent suitable for the treatment of a neurodegenerative disorder such as Alzheimer Disease in the treatment of a neurodegenerative disorder such as Alzheimer Disease. Further the use of at least one agent suitable for the treatment of a neurodegenerative disorder such as Alzheimer Disease in the manufacture of a medicament for simultaneous therapeutic administration with compounds of formula (I) in the treatment of a neurodegenerative disorder such as Alzheimer Disease is disclosed. Further at least one agent suitable for the treatment of a neurodegenerative disorder such as Alzheimer Disease for simultaneous therapeutic administration with compounds of formula (I) in the treatment of a neurodegenerative disorder such as Alzheimer Disease is disclosed.

Examples of agents suitable for the treatment of a neurodegenerative disorder such as Alzheimer Disease that are useful in the present invention include, but are not limited to: cholinesterase inhibitors, agents targeting nicotinic or muscarinic acethylcholine receptors, NMDA receptors, amyloid formation, mitochondrial dysfunctions, disease associated calpain activity, neuroinflamation, tumor necrosis factor receptors, NF-kappaB, peroxisome proliferator activator receptor gamma, Apolipoprotein E variant 4 (ApoE4), disease-associated increase of the HPA axis, epileptic discharges, vascular dysfunction, vascular risk factors, and oxidative stress.

Suitable cholinesterase inhibitors which may be used in combination with the compounds of the inventions include for example tacrine, donepezil, galantamine and rivastigmine.

Suitable NMDA receptors targeting agents which may be used in combination with the compounds of the inventions include for example memantine.

Suitable agents affecting increased HPA axis activity which may be used in combination with the compounds of the inventions include for example CRF1 antagonists or Vlb antagonists.

In a further aspect therefore, the invention provides compounds of formula (I) for use in a method of treatment of pain by adjunctive therapeutic administration of compounds of formula (I) to a patient receiving therapeutic administration of at least one agent suitable for the treatment of pain. In a further aspect, the invention provides the use of compounds of formula (I) in the manufacture of a medicament for adjunctive therapeutic administration for the treatment of pain in a patient receiving therapeutic administration of at least one agent suitable for the treatment of pain. The invention further provides compounds of formula (I) for use for adjunctive therapeutic administration for the treatment of pain in a patient receiving therapeutic administration of at least one agent suitable for the treatment of pain.

In a further aspect, a method of treatment of pain by adjunctive therapeutic administration of at least one agent suitable for the treatment of pain to a patient receiving therapeutic administration of compounds of formula (I) is disclosed. Further the use of at least one agent suitable for the treatment of pain in the manufacture of a medicament for adjunctive therapeutic administration for the treatment of pain in a patient receiving therapeutic administration of compounds of formula (I) is disclosed. Further at least one agent suitable for the treatment of pain for adjunctive therapeutic administration for the treatment of pain in a patient receiving therapeutic administration of compounds of formula (I) is disclosed.

In a further aspect, the invention provides compounds of formula (I) for use in a method of treatment of pain by simultaneous therapeutic administration of compounds of formula (I) in combination with at least one agent suitable for the treatment of pain. The invention further provides the use of a combination of compounds of formula (I) and at least one agent suitable for the treatment of pain in the manufacture of a medicament for simultaneous therapeutic administration in the treatment of pain. The invention further provides a combination of compounds of formula (I) and at least one agent suitable for the treatment of pain for simultaneous therapeutic administration in the treatment of pain. The invention further provides the use of compounds of formula (I) in the manufacture of a medicament for simultaneous therapeutic administration with at least one agent suitable for the treatment of pain in the treatment of pain. The invention further provides compounds of formula (I) for use for simultaneous therapeutic administration with at least one agent suitable for the treatment of pain in the treatment of pain. Further the use of at least one agent suitable for the treatment of pain in the manufacture of a medicament for simultaneous therapeutic administration with compounds of formula (I) in the treatment of pain is disclosed. Further at least one agent suitable for the treatment of pain for simultaneous therapeutic administration with compounds of formula (I) in the treatment of pain is disclosed.

Examples of agents suitable for the treatment of pain that are useful in the present invention include, but are not limited to: NSAIDs (Nonsteroidal Antiinflammatory Drugs), anticonvulsant drugs such as carbamazepine and gabapentin, sodium channel blockers, antidepressant drugs, cannabinoids and local anaesthetics.

Suitable agents used in combination with the compounds of the inventions include for example celecoxib, etoricoxib, lumiracoxib, paracetamol, tramadol, methadone, venlafaxine, imipramine, duloxetine, bupropion, gabapentin, pregabalin, lamotrigine, fentanyl, parecoxib, nefopam, remifentanil, pethidine, diclofenac, rofecoxib, nalbuphine, sufentanil, pethidine, diamorphine and butorphanol.

It will be appreciated by those skilled in the art that the compounds according to the invention may advantageously be used in conjunction with one or more other therapeutic agents, for instance, antidepressant agents such as 5HT3 antagonists, serotonin agonists, NK-1 antagonists, selective serotonin reuptake inhibitors (SSRI), noradrenaline re-uptake inhibitors (SNRI), tricyclic antidepressants, dopaminergic antidepressants, H3 antagonists, 5HT1A antagonists, 5HT1B antagonists, 5HT1D antagonists, D1 agonists, M1 agonists and/or anticonvulsant agents, as well as cognitive enhancers.

Suitable 5HT3 antagonists which may be used in combination of the compounds of the inventions include for example ondansetron, granisetron, metoclopramide.

Suitable serotonin agonists which may be used in combination with the compounds of the invention include sumatriptan, rauwolscine, yohimbine, metoclopramide.

Suitable SSRIs which may be used in combination with the compounds of the invention include fluoxetine, citalopram, femoxetine, fluvoxamine, paroxetine, indalpine, sertraline, zimeldine.

Suitable SNRIs which may be used in combination with the compounds of the invention include venlafaxine and reboxetine.

Suitable tricyclic antidepressants which may be used in combination with a compound of the invention include imipramine, amitriptiline, chlomipramine and nortriptiline.

Suitable dopaminergic antidepressants which may be used in combination with a compound of the invention include bupropion and amineptine.

Suitable anticonvulsant agents which may be used in combination of the compounds of the invention include for example divalproex, carbamazepine and diazepam.

The following examples serve to explain the invention without limiting it.

The compounds were characterized by mass spectrometry, generally recorded via HPLC-MS in a fast gradient on C18-material (electrospray-ionisation (ESI) mode).

### Preparation Examples

**Abbreviations:** APCI for atmospheric pressure chemical ionization; AcOH for acetic acid; Boc for *tert*-butoxy carbonyl; Bu for butyl; DCI for desorption chemical ionization; DCM for dichloromethane; dimethylsulfoxide for dimethyl sulfoxide; eq for equivalent(s); ESI for electrospray ionization; EtOAc for ethyl acetate; HATU for [o-(azabenzotriazol-1-yl)-1,1,3,3-tetramethyluronium hexafluorophosphate]; HCl for hydrochloric acid; HPLC for high performance liquid chromatography; id for internal diameter; LC/MS for liquid chromatography/mass spectrometry; MeOH for methanol; MgSO4 for magnesium sulfate; MP for macroporous resin; NaOAc for sodium acetate; PS for polymer supported; psi for pounds per square inch; SFC for supercritical fluid chromatography; SPE for solid phase extraction, and tBu for *tert*-butyl.

**Definitions:** Similarly indicates that that reactants may be substituted for other the reactants described, the temperature may vary by 50 °C, the equivilents may differ by upto 2 fold, or any combination thereof.

**Preparative HPLC Procedure:** Samples were purified by preparative HPLC on a Phenomenex® Luna® C8(2) 5 µm 100Å AXIA™ column (30 mm × 75 mm). A gradient of acetonitrile (A) and 0.1% trifluoroacetic acid in water (B) was used, at a flow rate of 50 mL/minute (0-0.5 minutes 10% A, 0.5-7.0 minutes linear gradient 10-95% A, 7.0-10.0 minutes 95% A, 10.0-12.0 minutes linear gradient 95-10% A). Samples were injected in 1.5 mL dimethyl sulfoxide:methanol (1:1). With specified samples, ammonium acetate was used instead of trifluoroacetic acid. A custom purification system was used, consisting of the following modules: Waters LC4000 preparative pump; Waters 996 diode-array detector; Waters 717+ autosampler; Waters SAT/IN module, Alltech Varex III evaporative light-scattering detector; Gilson 506C interface box; and two Gilson FC204 fraction collectors. The system was controlled using Waters Millennium32 software, automated using an Abbott developed Visual Basic application for fraction collector control and fraction tracking. Fractions were collected based upon UV signal threshold and selected fractions subsequently analyzed by flow injection analysis mass spectrometry using positive APCI ionization on a Finnigan LCQ using 70:30 methanol: 10 mM NH₄OH(aqueous) at a flow rate of 0.8 mL/minute. Loop-injection mass spectra were acquired using a Finnigan LCQ running LCQ Navigator 1.2 software and a Gilson 215 liquid handler for fraction injection controlled by an Abbott developed Visual Basic application.

For chiral compounds the absolute configuration is indicated in their chemical names. A chemical name with "trans" or no stereochemistry information does not refer to a chiral compound (even if the corresponding formula depicts a chiral compound).

### Example 1

### 2-chloro-N-{trans-1-[(1-methyl-1H-imidazol-4-yl)sulfonyl]-4-phenylpyrrolidin-3-yl}-3-(trifluoromethyl)benzamide

### Example 1A

To a solution of trans-tert-butyl 3-amino-4-phenylpyrrolidine-1-carboxylate (0.305 g, 1.163 mmol) and 2-chloro-3-(trifluoromethyl)benzoic acid (0.261 g, 1.163 mmol) in dichloromethane (4.65 ml) was added triethylamine (0.486 ml, 3.49 mmol) and HATU (0.663 g, 1.744 mmol) and the mixture was stirred at room temperature for 2 hours. The reaction mixture was partitioned with water, the organic fraction was collected, and the aqueous fraction was washed with dichloromethane. The organic fractions were combined, dried over sodium sulfate and concentrated. The crude product was purified by silica gel chromatography (Analogix IntelliFlash 280, SF15-12) eluting with 30% ethyl acetate / hexanes to afford the title compound. MS (ESI) *m*/*z* 469.3 [M+H]⁺

### Example 1B

### 2-chloro-N-(trans-4-phenylpyrrolidin-3-yl)-3-(trifluoromethyl)benzamide hydrochloride

To a solution of Example 1A (0.32 g, 0.682 mmol) in dioxane (0.682 ml) was added HCl in dioxane (4M, 1.706 ml, 6.82 mmol) and the mixture was stirred at room temperature for 2 hours. The solvent was evaporated and the solid was triturated twice with dichloromethane and the solvent was evaporated to afford the title compound. MS (ESI) *m*/*z* 369.2 [M+H]+

### Example 1C

### 2-chlor0-N-(trans-1-(1-methyl-1H-imidazol-4-ylsulfonyl)-4-phenylpyrrolidin-3-yl)-3-(trifluoromethyl)benzamide

To a solution of Example 1B (0.075 g, 0.185 mmol) in dichloromethane (0.740 ml) was added triethylamine (0.077 ml, 0.555 mmol) and 1-methyl-1H-imidazole-4-sulfonyl chloride (0.033 g, 0.185 mmol). The mixture was stirred at room temperature for 1 hour. The solvent was evaporated. The crude product was purified by HPLC to afford the title compound. MS (ESI) *m*/*z* 513.1 [M+H]+

### Example 2

### 2-chloro-N-{trans-4-(4-methoxyphenyl)-1-[(1-methyl-1H-imidazol-4-yl)sulfonyl]pyrrolidin-3-yl}-3-(trifluoromethyl)benzamide

### Example 2A

### trans-1 -benzyl-3 -(4-methoxyphenyl)-4-nitropyrrolidine

To a solution of (E)-1-methoxy-4-(2-nitrovinyl)benzene (5.09g, 28.4 mmol) and N-(methoxymethyl)-N-(trimethylsilylmethyl)benzylamine (8.07g, 34.0 mmol) in 75 mL of dichloromethane at 0 °C under nitrogen was added trifluoroacetic acid (388 mg, 3.4 mmol) in one portion. The reaction was allowed to warm to ambient temperature and stirred for 16 hours. The reaction was then partitioned between dichloromethane and saturated sodium bicarbonate solution. The organic fraction was collected. The aqueous portion was washed with additional dichloromethane and the combined organic fractions were washed with water, brine and dried over sodium sulfate. The mixture was filtered, concentrated and purified on a silica gel flash column (7:3 hexane:ethyl acetate) to afford the title compound. MS (DCI) *m*/*z* 313.1 (M+H)⁺.

### Example 2B

### trans-1 -benzyl-4-(4-methoxyphenyl)pyrrolidin-3 -amine

Example 2A (4.85 mg, 15.5 mmol) and tetrahydrofuran (100 mL) were added to a Raney nickel water slurry (Grace 2800, 5.00 g) in a stainless steel reactor. The vessel was pressurized with 30 psi of hydrogen and shaken at room temperature for 16 hours. The mixture was filtered through a nylon membrane concentrated and purified on a silica gel flash column (95:5 dicloromethane: 2N ammonia in methanol) to afford the title compound as colorless oil. MS (DCI) *m*/*z* 283.1(M+H)⁺.

### Example 2C

### tert-Butyl trans-1 -benzyl-4-(4-methoxyphenyl)pyrrolidin-3 -ylcarbamate

To a solution of 2.5 g (8.86 mmol) of Example 2B in tetrahydrofuran (20 ml) was added saturated sodium bicarbonate solution (20 ml) followed by di-tert-butyl dicarbonate (1.0 M solution in tetrahyrofuran, 10 ml, 10.0 mmol) at room temperature under nitrogen. The reaction was stirred for 1 hour and then partitioned between ethyl acetate and water. The organic fraction was collected. The aqueous portion was washed several additional times with ethyl acetate and the combined organic extracts were washed with brine and dried over sodium sulfate. The mixture was filtered, concentrated and purified on a silica gel flash column (3:2 ethyl acetate hexane) to afford the title compound. MS (DCI) *m*/*z* 383.2 (M+H)⁺.

### Example 2D

### tert-butyl (3S,4R)-4-(4-methoxyphenyl)pyrrolidin-3-ylcarbamate

Example 2C (2.65 mg, 6.93 mmol) and 2,2,2-trifluoroethanol or tetrahydrofuran (40 mL) were added to 20% Pd(OH)₂/C (50% water, 0.530 g) in a stainless steel reactor. The vessel was pressurized with 30 psi of hydrogen and shaken at 50 °C for 30 minutes. The mixture was filtered through a nylon membrane, and the product was purified on a silica gel column (95:5 dichloromethane:2N ammonia in methanol) to afford the title compound. MS (DCI) *m*/*z* 293.1 (M+H)⁺.

### Example 2E

### tert-butyl (trans-4-(4-methoxyphenyl)-1-((1-methyl-1H-imidazol-4-yl)sulfonyl)pyrrolidin-3-yl)carbamate

To a solution of the product from Example 2D (154 mg, 0.53 mmol), and triethylamine (152 mg, 1.5 mmol) in dichloromethane (8 ml) was added 1-methyl-1H-imidazole-4-sulfonyl chloride (108 mg, 0.60 mmol) in one portion at room temperature. 4-(dimethylamino)-pyridine (4 mg, 0.03 mmol) was added and the reaction stirred for two hours at room temperature. The reaction was concentrated and purified on a silica gel flash column (97:3 dichloromethane: 2N ammonia in methanol) to afford the title compound. MS (DCI) *m*/*z* 425.1(M+H)⁺.

### Example 2F

### trans-4-(4-methoxyphenyl)-1-((1-methyl-1H-imidazol-4-yl)sulfonyl)pyrrolidin-3 -amine

The product from Example 2E (195 mg, 0.46 mmol) was stirred with 1,4-dioxane (4 ml) and hydrogen chloride in 1,4-dioxane (4M, 4 ml, 16.0 mmol) at room temperature under nitrogen overnight. The reaction was concentrated and concentrated to afford the title compound as the hydrochloride salt. MS (DCI) *m*/*z* 325.0 (M+H)⁺.

### Example 2G

### 2-chloro-N- {trans-1 -[(1 -methyl-1H-imidazol-4-yl)sulfonyl]-4-phenylpyrrolidin-3 -yl} -3 - (trifluoromethyl)benzamide

A mixture of Example 2F, 2-chloro-3-(trifluoromethyl)benzoic acid (101 mg, 0.45 mmol), and N-(3-Dimethylaminopropyl)-N-ethylcarbodiimide hydrochloride (86 mg, 0.45 mmol) was stirred in a 1:1 solution of pyridine and N,N-dimethylformamide (5 ml) at room temperature under nitrogen for 16 hours. The reaction was concentrated and the residue was partitioned between ethyl acetate and water. The organic fraction was collected and the aqueous portion was washed with additional ethyl acetate. The combined organic fractions were washed with water, brine and dried over sodium sulfate. The mixture was filtered, concentrated and purified on a silica gel flash column (95:5 dichloromethane 2N ammonia in methanol) to afford the title compound. MS (ESI) *m*/*z* 543.2 (M+H)⁺.

### Example 3

### 2-chloro-N-{trans-4-(4-fluorophenyl)-1-[(1-methyl-1H-imidazol-4-yl)sulfonyl]pyrrolidin-3-yl}-3-(trifluoromethyl)benzamide

### Example 3A

### trans-4-(4-fluorophenyl)-1-(1-methyl-1H-imidazol-4-ylsulfonyl)pyrrolidin-3-amine

The title compound was prepared as an HCl salt similarly to the procedures described in Example 2A-2F substituting (E)-1-fluoro-4-(2-nitrovinyl)benzene for (E)-1-methoxy-4-(2-nitrovinyl)benzene.

### Example 3B

### 2-chloro-N-{trans-4-(4-fluorophenyl)-1-[(1-methyl-1H-imidazol-4-yl)sulfonyl]pyrrolidin-3-yl}-3-(trifluoromethyl)benzamide

The title compound was prepared similarly to the procedure described in Example 2G substituting Example 3A for Example 2F. MS (ESI) *m*/*z* 531 (M+H)⁺.

### Reference Example 4

### 2-chloro-N-[trans-1-(methylsulfonyl)-4-(2,4,5-trifluorophenyl)pyrrolidin-3-yl]-3-(trifluoromethyl)benzamide

### Example 4A

### (E)-1,2,4-trifluoro-5-(2-nitrovinyl)benzene

A stirred solution containing 2,4,5-trifluorobenzaldehyde (16.2 g, 80.1 mmol), ammonium acetate (17.3 g, 225 mmol), nitromethane (12.2 mL, 225 mmol), and acetic acid (75 mL) was heated to 100 °C for 3 hours. The mixture was cooled to room temperature and partitioned between water and dichloromethane. The organic fraction was collected and the aqueous fraction was washed with dichloromethane. The organic fractions were combined and washed with sodium bicarbonate (aq.), brine, and water. The organic fraction was dried with sodium sulfate and purified via flash chromatography (0-100%EtOAc/hexanes) to provide the title compound.

### Example 4B

### trans-1-benzyl-4-(2,4,5-trifluorophenyl)pyrrolidin-3-amine

The title compound was prepared similarly to the conditions described in Examples 2A-2B substituting Example 4A for (E)-1-methoxy-4-(2-nitrovinyl)benzene.

### Example 4C

N-(trans-1-benzyl-4-(2,4,5-trifluorophenyl)pyrrolidin-3-yl)-2-chloro-3-(trifluoromethyl)benzamide To a stirred solution of Example 4B (3.3 g, 10.7 mmol) in a 1:1 dimethylformamide:pyridine solution (20 mL) was added 2-chloro-3-(trifluoromethyl)benzoic acid (2.9 g, 12.9 mmol) and N-(3-Dimethylaminopropyl)-N'-ethylcarbodiimide hydrochloride (2.85 g, 15 mmol). The reaction was stirred at room temperature for 18 hours. The reaction mixture was concentrate. The reaction mixture was partitioned between, EtOAc (100 mL) and 1 M HCl (200 mL). The organic layer was collected.

The aqueous fraction was washed with EtOAc (100 mL). The organic fractions were combined. Purification via flash chromatography (0-100% EtOAc/hexanes) provided the title compound.

### 2-chloro-3-(trifluoromethyl)-N-(trans-4-(2,4,5-trifluorophenyl)pyrrolidin-3-yl)benzamide

To a stirred solution of Example 4C (3.4 g, 6.63 mmol) in dichloroethane was added 1-chloroethyl carbonochloridate (1.0 g, 7.29 mmol). The reaction mixture was stirred at 80 °C for 2 hours. Some precipitate was collected via filtration to provide the title compound. The filtrate was concentrated and purified via flash chromatography (0-30 % methanol/DCM) to provide the title compound.

### Example 4E

### 2-chloro-N-[(trans-1-(methylsulfonyl)-4-(2,4,5-trifluorophenyl)pyrrolidin-3-yl]-3-(trifluoromethyl)benzamide

To a stirred solution of Example 4D (50 mg, 0.18 mmol) in pyridine (2 mL) was added methanesulfonyl chloride (16 mg, 0.14 mmol). The reaction mixture stirred at 60 °C for 18 hours. The reaction mixture was then concentrated and purified via HPLC. MS (ESI) *m*/*z* 501 (M+H)⁺.

### Example 13

### 2-chloro-N-{trans-4-(4-chlorophenyl)-1-[(1-methyl-1H-imidazol-4-yl)sulfonyl]pyrrolidin-3-yl}-3-(trifluoromethyl)benzamide

The title compound was prepared similarly to the procedures described in Example 2 substituting (E)-1-chloro-4-(2-nitrovinyl)benzene for (E)-1-methoxy-4-(2-nitrovinyl)benzene. MS (DCI) *m*/*z* 547.0 (M+H)⁺

### Example 14

### 2-chloro-N-{trans-1-[(1-methyl-1H-imidazol-4-yl)sulfonyl]-4-phenylpyrrolidin-3-yl}benzamide

### Example 14A

### trans-1-(1-methyl-1H-imidazol-4-ylsulfonyl)-4-phenylpyrrolidin-3-amine

The title compound was prepared as an HCl salt similarly to the conditions described in Example 2A -2F substituting (E)-(2-nitrovinyl)benzene for (E)-1-methoxy-4-(2-nitrovinyl)benzene.

### Example 14B

### 2-chloro-N-{trans-1-[(1-methyl-1H-imidazol-4-yl)sulfonyl]-4-phenylpyrrolidin-3-yl}benzamide

The title compound was prepared similarly to the conditions described in Example 4C substituting Example 14A for Example 4B and substituting 2-chloro-benzoic acid for 2-chloro-3-(trifluoromethyl)benzoic acid. MS (ESI) *m*/*z* 445 (M+H)⁺.

### Example 15

### 3-chloro-N-{trans-1-[(1-methyl-1H-imidazol-4-yl)sulfonyl]-4-phenylpyrrolidin-3-yl}benzamide

The title compound was prepared similarly to the conditions described in Example 4C substituting Example 14A for Example 4B and substituting 3-chloro-benzoic acid for 2-chloro-3-(trifluoromethyl)benzoic acid. MS (ESI) *m*/*z* 445 (M+H)⁺.

### Example 16

### 4-chloro-N-{trans-1-[(1-methyl-1H-imidazol-4-yl)sulfonyl]-4-phenylpyrrolidin-3-yl}benzamide

The title compound was prepared similarly to the conditions described in Example 4C substituting Example 14A for Example 4B and substituting 4-chloro-benzoic acid for 2-chloro-3-(trifluoromethyl)benzoic acid. MS (ESI) *m*/*z* 445 (M+H)⁺.

### Example 17

### 2-fluoro-N-{trans-1-[(1-methyl-1H-imidazol-4-yl)sulfonyl]-4-phenylpyrrolidin-3-yl}benzamide

The title compound was prepared similarly to the conditions described in Example 4C substituting Example 14A for Example 4B and substituting 2-fluoro-benzoic acid for 2-chloro-3-(trifluoromethyl)benzoic acid. MS (ESI) *m*/*z* 429 (M+H)⁺.

### Example 18

### 3 -fluoro-N-{trans-1-[(1-methyl-1H-imidazol-4-yl)sulfonyl]-4-phenylpyrrolidin-3-yl}benzamide

The title compound was prepared similarly to the conditions described in Example 4C substituting Example 14A for Example 4B and substituting 3-fluoro-benzoic acid for 2-chloro-3-(trifluoromethyl)benzoic acid. MS (ESI) *m*/*z* 429 (M+H)⁺.

### Example 19

### 4-fluoro-N-{trans-1-[(1-methyl-1H-imidazol-4-yl)sulfonyl]-4-phenylpyrrolidin-3-yl}benzamide

The title compound was prepared similarly to the conditions described in Example 4C substituting Example 14A for Example 4B and substituting 4-fluoro-benzoic acid for 2-chloro-3-(trifluoromethyl)benzoic acid. MS (ESI) *m*/*z* 429 (M+H)⁺.

### Example 20

### 2-methoxy-N-{trans-1-[(1-methyl-1H-imidazol-4-yl)sulfonyl]-4-phenylpyrrolidin-3-yl}benzamide

The title compound was prepared similarly to the conditions described in Example 4C substituting Example 14A for Example 4B and substituting 2-methoxy-benzoic acid for 2-chloro-3-(trifluoromethyl)benzoic acid. MS (ESI) *m*/*z* 441 (M+H)⁺.

### Example 21

### 3-methoxy-N-{trans-1-[(1-methyl-1H-imidazol-4-yl)sulfonyl]-4-phenylpyrrolidin-3-yl}benzamide

The title compound was prepared similarly to the conditions described in Example 4C substituting Example 14A for Example 4B and substituting 3-methoxy-benzoic acid for 2-chloro-3-(trifluoromethyl)benzoic acid. MS (ESI) *m*/*z* 441 (M+H)⁺.

### Example 22

### 2-methyl-N-{trans-1-[(1-methyl-1H-imidazol-4-yl)sulfonyl]-4-phenylpyrrolidin-3-yl}benzamide

The title compound was prepared similarly to the conditions described in Example 4C substituting Example 14A for Example 4B and substituting 2-methyl-benzoic acid for 2-chloro-3-(trifluoromethyl)benzoic acid. MS (ESI) *m*/*z* 425 (M+H)⁺.

### Example 23

### 3-methyl-N-{trans-1-[(1-methyl-1H-imidazol-4-yl)sulfonyl]-4-phenylpyrrolidin-3-yl}benzamide

The title compound was prepared similarly to the conditions described in Example 4C substituting Example 14A for Example 4B and substituting 3-methyl-benzoic acid for 2-chloro-3-(trifluoromethyl)benzoic acid. MS (ESI) *m*/*z* 425 (M+H)⁺.

### Example 24

### 4-methyl-N-{trans-1-[(1-methyl-1H-imidazol-4-yl)sulfonyl]-4-phenylpyrrolidin-3-yl}benzamide

The title compound was prepared similarly to the conditions described in Example 4C substituting Example 14A for Example 4B and substituting 4-methyl-benzoic acid for 2-chloro-3-(trifluoromethyl)benzoic acid. MS (ESI) *m*/*z* 425 (M+H)⁺.

### Example 25

### N-{trans-1-[(1-methyl-1H-imidazol-4-yl)sulfonyl]-4-phenylpyrrolidin-3-yl}benzamide

The title compound was prepared similarly to the conditions described in Example 4C substituting Example 14A for Example 4B and substituting benzoic acid for 2-chloro-3-(trifluoromethyl)benzoic acid. MS (ESI) *m*/*z* 411 (M+H)⁺.

### Example 26

### 2,4-dichloro-N-{trans-1-[(1-methyl-1H-imidazol-4-yl)sulfonyl]-4-phenylpyrrolidin-3-yl}benzamide

The title compound was prepared similarly to the conditions described in Example 4C substituting Example 14A for Example 4B and substituting 2,4-dichlorobenzoic acid for 2-chloro-3-(trifluoromethyl)benzoic acid. MS (ESI) *m*/*z* 479 (M+H)⁺.

### Example 27

### 2-chloro-4-fluoro-N-{trans-1-[(1-methyl-1H-imidazol-4-yl)sulfonyl]-4-phenylpyrrolidin-3-yl}benzamide

The title compound was prepared similarly to the conditions described in Example 4C substituting Example 14A for Example 4B and substituting 2-chloro-4-fluorobenzoic acid for 2-chloro-3-(trifluoromethyl)benzoic acid. MS (ESI) *m*/*z* 463 (M+H)⁺.

### Example 29

### trans-1-[(1-methyl-1H-imidazol-4-yl)sulfonyl]-N,4-diphenylpyrrolidin-3-amine

Under nitrogen, a pressure vial was charged with Example 14A (50 mg, 0.15 mmol), bromobenzene (23 mg, 0.15 mmol), 2'-(di-tert-butylphosphino)-N,N-dimethylbiphenyl-2-amine (5.0 mg, 0.02 mmol), tris(dibenzylidene-acetone)dipalladium(0) (6.7 mg, 7.3 µmol), sodium tert-butoxide (18 mg, 0.19 mmol), and toluene (1 mL). The reaction mixture was stirred at 80 °C for 3 hours. Then, the reaction mixture was concentrated. Purification via HPLC provided the title compound. MS (ESI) *m*/*z* 383 (M+H)⁺.

### Example 30

### 3,5-dichloro-N-{trans-1-[(1-methyl-1H-imidazol-4-yl)sulfonyl]-4-phenylpyrrolidin-3-yl}benzamide

The title compound was prepared similarly to the conditions described in Example 4C substituting Example 14A for Example 4B and substituting 3,5-dichlorobenzoic acid for 2-chloro-3-(trifluoromethyl)benzoic acid. MS (ESI) *m*/*z* 479 (M+H)⁺.

### Example 31

### 2,3-dichloro-N-{trans-1-[(1-methyl-1H-imidazol-4-yl)sulfonyl]-4-phenylpyrrolidin-3-yl}benzamide

The title compound was prepared similarly to the conditions described in Example 4C substituting Example 14A for Example 4B and substituting 2,3-dichlorobenzoic acid for 2-chloro-3-(trifluoromethyl)benzoic acid. MS (ESI) *m*/*z* 479 (M+H)⁺.

### Example 32

### 2-cyano-N- {trans-1 -[(1 -methyl-1H-imidazol-4-yl)sulfonyl]-4-phenylpyrrolidin-3 -yl} benzamide

The title compound was prepared similarly to the conditions described in Example 4C substituting Example 14A for Example 4B and substituting 2-cyanobenzoic acid for 2-chloro-3-(trifluoromethyl)benzoic acid. MS (ESI) *m*/*z* 436 (M+H)⁺.

### Example 33

### 3-cyano-N-{trans-1-[(1-methyl-1H-imidazol-4-yl)sulfonyl]-4-phenylpyrrolidin-3-yl}benzamide

The title compound was prepared similarly to the conditions described in Example 4C substituting Example 14A for Example 4B and substituting 3-cyanobenzoic acid for 2-chloro-3-(trifluoromethyl)benzoic acid. MS (ESI) *m*/*z* 436 (M+H)⁺.

### Example 34

### 4-cyano-N- {trans-1 -[(1 -methyl-1H-imidazol-4-yl)sulfonyl]-4-phenylpyrrolidin-3 -yl} benzamide

The title compound was prepared similarly to the conditions described in Example 4C substituting Example 14A for Example 4B and substituting 4-cyanobenzoic acid for 2-chloro-3-(trifluoromethyl)benzoic acid. MS (ESI) *m*/*z* 436 (M+H)⁺.

### Example 35

### N-{trans-1-[(1-methyl-1H-imidazol-4-yl)sulfonyl]-4-phenylpyrrolidin-3-yl}-2-(trifluoromethyl)benzamide

The title compound was prepared similarly to the conditions described in Example 4C substituting Example 14A for Example 4B and substituting 2-(trifluoromethyl)benzoic acid for 2-chloro-3-(trifluoromethyl)benzoic acid. MS (ESI) *m*/*z* 479 (M+H)⁺.

### Example 36

### N-{trans-1-[(1-methyl-1H-imidazol-4-yl)sulfonyl]-4-phenylpyrrolidin-3-yl}-3-(trifluoromethyl)benzamide

The title compound was prepared similarly to the conditions described in Example 4C substituting Example 14A for Example 4B and substituting 3-(trifluoromethyl)benzoic acid for 2-chloro-3-(trifluoromethyl)benzoic acid. MS (ESI) *m*/*z* 479 (M+H)⁺.

### Example 37

### N-{trans-1-[(1-methyl-1H-imidazol-4-yl)sulfonyl]-4-phenylpyrrolidin-3-yl}-4-(trifluoromethyl)benzamide

The title compound was prepared similarly to the conditions described in Example 4C substituting Example 14A for Example 4B and substituting 4-(trifluoromethyl)benzoic acid for 2-chloro-3-(trifluoromethyl)benzoic acid. MS (ESI) *m*/*z* 479 (M+H)⁺.

### Example 38

### 2-chloro-N-[trans-1-{[1-methyl-3-(trifluoromethyl)-1H-pyrazol-4-yl]sulfonyl}-4-phenylpyrrolidin-3-yl]-3-(trifluoromethyl)benzamide

### Example 38A

### (trans)-1-benzyl-4-phenylpyrrolidin-3-amine 2-chloro-N-(trans-4-phenylpyrrolidin-3-yl)-3-(trifluoromethyl)benzamide

The title compound was prepared similarly to the procedures described in Examples 2A-2B substituting (E)-1-fluoro-4-(2-nitrovinyl)benzene for (E)-1-methoxy-4-(2-nitrovinyl)benzene.

### Example 38B

### 2-chloro-N-(trans-4-phenylpyrrolidin-3-yl)-3-(trifluoromethyl)benzamide

The title compound was prepared similarly to Example 4C-4D substituting Example 38A for Example 4B.

### Example 38C

### 2-chloro-N-[trans-1-{[1-methyl-3-(trifluoromethyl)-1H-pyrazol-4-yl]sulfonyl}-4-phenylpyrrolidin-3-yl]-3-(trifluoromethyl)benzamide

The title compound was prepared similarly to the procedure described in Example 4E substituting Example 38B for Example 4D and substituting 1-methyl-3-(trifluoromethyl)-1H-pyrazole-4-sulfonyl chloride for methanesulfonyl chloride. MS (ESI) *m*/*z* 581 (M+H)⁺.

### Example 39

### 2-chloro-N-[trans-1-{[1-(difluoromethyl)-3,5-dimethyl-1H-pyrazol-4-yl]sulfonyl}-4-phenylpyrrolidin-3-yl]-3-(trifluoromethyl)benzamide

The title compound was prepared similarly to the conditions described in Example 4E substituting Example 38B for tert-butyl 3-amino-4-phenylpyrrolidine-1-carboxylate and substituting 1-(difluoromethyl)-3,5-dimethyl-1H-pyrazole-4-sulfonyl chloride for methanesulfonyl chloride. MS (ESI) *m*/*z* 577 (M+H)⁺.

### Example 40

### 2-chloro-N-{trans-1-[(5-chloro-1,3-dimethyl-1H-pyrazol-4-yl)sulfonyl]-4-phenylpyrrolidin-3-yl}-3-(trifluoromethyl)benzamide

The title compound was prepared similarly to the conditions described in Example 4E substituting Example 38B for tert-butyl 3-amino-4-phenylpyrrolidine-1-carboxylate and substituting 5-chloro-1,3-dimethyl-1H-pyrazole-4-sulfonyl chloride for methanesulfonyl chloride. MS (ESI) *m*/*z* 561 (M+H)⁺.

### Example 41

### 2-chloro-N-{trans-1-[(1,5-dimethyl-1H-pyrazol-4-yl)sulfonyl]-4-phenylpyrrolidin-3-yl}-3-(trifluoromethyl)benzamide

The title compound was prepared similarly to the conditions described in Example 4E substituting Example 38B for tert-butyl 3-amino-4-phenylpyrrolidine-1-carboxylate and substituting 1,5-dimethyl-1H-pyrazole-4-sulfonyl chloride for methanesulfonyl chloride. MS (ESI) *m*/*z* 527 (M+H)⁺.

### Example 42

### 2-chloro-N-{trans-1-[(1,3-dimethyl-1H-pyrazol-4-yl)sulfonyl]-4-phenylpyrrolidin-3-yl}-3-(trifluoromethyl)benzamide

The title compound was prepared similarly to the conditions described in Example 4E substituting Example 38B for tert-butyl 3-amino-4-phenylpyrrolidine-1-carboxylate and substituting 1,3-dimethyl-1H-pyrazole-4-sulfonyl chloride for methanesulfonyl chloride. MS (ESI) *m*/*z* 527 (M+H)⁺.

### Example 43

### 2-chloro-N-{trans-1-[(1-methyl-1H-pyrazol-4-yl)sulfonyl]-4-phenylpyrrolidin-3-yl}-3-(trifluoromethyl)benzamide

The title compound was prepared similarly to the conditions described in Example 4E substituting Example 38B for tert-butyl 3-amino-4-phenylpyrrolidine-1-carboxylate and substituting 1-methyl-1H-pyrazole-4-sulfonyl chloride for methanesulfonyl chloride. MS (ESI) *m*/*z* 513 (M+H)⁺.

### Reference Example 44

### 2-chloro-N-{trans-1-[(6-methoxypyridin-3-yl)sulfonyl}-4-phenylpyrrolidin-3-yl}-3-(trifluoromethyl)benzamide

The title compound was prepared similarly to the conditions described in Example 4E substituting Example 38B for tert-butyl 3-amino-4-phenylpyrrolidine-1-carboxylate and substituting 6-methoxypyridine-3-sulfonyl chloride for methanesulfonyl chloride. MS (ESI) *m*/*z* 540 (M+H)⁺.

### Reference Example 45

### 2-chloro-N-[trans-4-phenyl-1-(pyridin-3-ylsulfonyl)pyrrolidin-3-yl]-3-(trifluoromethyl)benzamide

The title compound was prepared similarly to the conditions described in Example 4E substituting Example 38B for tert-butyl 3-amino-4-phenylpyrrolidine-1-carboxylate and substituting pyridine-3-sulfonyl chloride for methanesulfonyl chloride. MS (ESI) *m*/*z* 510 (M+H)⁺. Example 46 2-chloro-N-[trans-4-phenyl-1-(1H-pyrazol-4-ylsulfonyl)pyrrolidin-3-yl]-3-(trifluoromethyl)benzamide

The title compound was prepared similarly to the conditions described in Example 4E substituting Example 38B for tert-butyl 3-amino-4-phenylpyrrolidine-1-carboxylate and substituting 1H-pyrazole-4-sulfonyl chloride for methanesulfonyl chloride. MS (ESI) *m*/*z* 499 (M+H)⁺. Example 47

### 2-chloro-N-{trans-1-[(1-methyl-1H-pyrazol-5-yl)sulfonyl]-4-phenylpyrrolidin-3-yl}-3-(trifluoromethyl)benzamide

The title compound was prepared similarly to the conditions described in Example 4E substituting Example 38B for tert-butyl 3-amino-4-phenylpyrrolidine-1-carboxylate and substituting 1-methyl-1H-pyrazole-5-sulfonyl chloride for methanesulfonyl chloride. MS (ESI) *m*/*z* 513 (M+H)⁺.

### Example 48

### 2-chloro-N-{trans-1-[(5-chloro-1-methyl-1H-imidazol-4-yl)sulfonyl]-4-phenylpyrrolidin-3-yl}-3-(trifluoromethyl)benzamide

The title compound was prepared similarly to the conditions described in Example 4E substituting Example 38B for tert-butyl 3-amino-4-phenylpyrrolidine-1-carboxylate and substituting 5-chloro-1-methyl-1H-imidazole-4-sulfonyl chloride for methanesulfonyl chloride. MS (ESI) *m*/*z* 547 (M+H)⁺. Example 49

### 2-chloro-N-{trans-1-[(1-methyl-1H-pyrazol-3-yl)sulfonyl]-4-phenylpyrrolidin-3-yl}-3-(trifluoromethyl)benzamide

The title compound was prepared similarly to the conditions described in Example 4E substituting Example 38B for tert-butyl 3-amino-4-phenylpyrrolidine-1-carboxylate and substituting 1-methyl-1H-pyrazole-3-sulfonyl chloride for methanesulfonyl chloride. MS (ESI) *m*/*z* 513 (M+H)⁺.

### Example 50

### trans-N-(4-methoxyphenyl)-1-[(1-methyl-1H-imidazol-4-yl)sulfonyl]-4-phenylpyrrolidin-3-amine

The title compound was prepared similarly to the conditions described in Example 29 substituting 1-bromo-4-methoxybenzene for bromobenzene. MS (ESI) *m*/*z* 413 (M+H)⁺.

### Example 51

### 3-({trans-1-[(1-methyl-1H-imidazol-4-yl)sulfonyl]-4-phenylpyrrolidin-3-yl}amino)benzonitrile

The title compound was prepared similarly to the conditions described in Example 29 substituting 3-bromobenzenenitrile for bromobenzene. MS (ESI) *m*/*z* 408 (M+H)⁺.

### Example 52

### 4-({trans-1-[(1-methyl-1H-imidazol-4-yl)sulfonyl]-4-phenylpyrrolidin-3-yl}amino)benzonitrile

The title compound was prepared similarly to the conditions described in Example 29 substituting 4-bromobenzenenitrile for bromobenzene. MS (ESI) *m*/*z* 408 (M+H)⁺.

### Example 53

### 3-fluoro-4-({trans-1-[(1-methyl-1H-imidazol-4-yl)sulfonyl]-4-phenylpyrrolidin-3-yl}amino)benzonitrile

The title compound was prepared similarly to the conditions described in Example 29 substituting 4-bromo-3-fluorobenzonitrile for bromobenzene. MS (ESI) *m*/*z* 426 (M+H)⁺.

### Example 54

### 2-chloro-N-{trans-4-(2-fluorophenyl)-1-[(1-methyl-1H-imidazol-4-yl)sulfonyl]pyrrolidin-3-yl}-3-(trifluoromethyl)benzamide

The title compound was prepared similarly to the procedures described in Example 2 substituting (E)-1-fluoro-2-(2-nitrovinyl)benzene for (E)-1-methoxy-4-(2-nitrovinyl)benzene in Example 2A. MS (DCI) *m*/*z* 530.9 (M+H)⁺

### Example 55

### 2-chloro-N-{trans-4-(3-fluorophenyl)-1-[(1-methyl-1H-imidazol-4-yl)sulfonyl]pyrrolidin-3-yl}-3-(trifluoromethyl)benzamide

The title compound was prepared similarly to the procedures as described in Example 2 substituting (E)-1-fluoro-3-(2-nitrovinyl)benzene for (E)-1-methoxy-4-(2-nitrovinyl)benzene in Example 2A. MS (DCI) *m*/*z* 531.2 (M+H)⁺

### Example 56

### 2-chloro-N-{trans-4-(3-chlorophenyl)-1-[(1-methyl-1H-imidazol-4-yl)sulfonyl]pyrrolidin-3-yl}-3-(trifluoromethyl)benzamide

The title compound was prepared similarly to procedures described in Example 2 substituting (E)-1-chloro-3-(2-nitrovinyl)benzene for (E)-1-methoxy-4-(2-nitrovinyl)benzene in Example 2A. MS (DCI) *m*/*z* 547.5 (M+H)⁺

### Example 57

### trans-1-[(1-methyl-1H-imidazol-4-yl)sulfonyl]-N-(2-methylphenyl)-4-phenylpyrrolidin-3-amine

Under nitrogen, a pressure vial was charged with Example 14A as a hydrochloric salt (100 mg, 0.30 mmol), 1-bromo-2-methylbenzene (55 mg, 0.32 mmol), 2'-(di-tert-butylphosphino)-N,N-dimethylbiphenyl-2-amine (10 mg, 0.03 mmol), tris(dibenzylidene-acetone)dipalladium(0) (13 mg, 0.02 mmol), sodium tert-butoxide (70 mg, 0.729 mmol), and toluene (2 mL). The reaction mixture was stirred at 80 °C for 6 hours. Then, the reaction mixture was concentrated. Purification via HPLC provided the title compound. MS (ESI) *m*/*z* 397 (M+H)⁺.

### Example 58

### trans-1-[(1-methyl-1H-imidazol-4-yl)sulfonyl]-N-(3-methylphenyl)-4-phenylpyrrolidin-3-amine

The title compound was prepared similarly to the conditions described in Example 57 substituting 1-bromo-3-methylbenzene for 1-bromo-2-methylbenzene. MS (ESI) *m*/*z* 397 (M+H)⁺.

### Example 59

### trans-1-[(1-methyl-1H-imidazol-4-yl)sulfonyl]-N-(4-methylphenyl)-4-phenylpyrrolidin-3-amine

The title compound was prepared similarly to the conditions described in Example 57 substituting 1-bromo-4-methylbenzene for 1-bromo-2-methylbenzene. MS (ESI) *m*/*z* 397 (M+H)⁺.

### Example 60

### 2-chloro-N-{trans-1-[(1-methyl-1H-imidazol-4-yl)sulfonyl]-4-phenylpyrrolidin-3-yl}-4-(trifluoromethyl)benzamide

The title compound was prepared similarly to the conditions described in Example 4C substituting Example 14A for Example 4B and substituting 2-chloro-4-(trifluoromethyl)benzoic acid for 2-chloro-3-(trifluoromethyl)benzoic acid. MS (ESI) *m*/*z* 513 (M+H)⁺. Example 61

### 3-chloro-N-{trans-1-[(1-methyl-1H-imidazol-4-yl)sulfonyl]-4-phenylpyrrolidin-3-yl}-4-(trifluoromethyl)benzamide

The title compound was prepared similarly to the conditions described in Example 4C substituting Example 14A for Example 4B and substituting 3-chloro-4-(trifluoromethyl)benzoic acid for 2-chloro-3-(trifluoromethyl)benzoic acid. MS (ESI) *m*/*z* 513 (M+H)⁺.

### Example 62

### 2-chloro-N-{trans-4-(4-fluorophenyl)-1-[(1-methyl-1H-imidazol-4-yl)sulfonyl]pyrrolidin-3-yl}-4-(trifluoromethyl)benzamide

The title compound was prepared similarly to the conditions described in Example 4C substituting Example 3A for Example 4B and substituting 2-chloro-4-(trifluoromethyl)benzoic acid for 2-chloro-3-(trifluoromethyl)benzoic acid. MS (ESI) *m*/*z* 531 (M+H)⁺.

### Example 63

### 3-chloro-N-{trans-4-(4-fluorophenyl)-1-[(1-methyl-1H-imidazol-4-yl)sulfonyl]pyrrolidin-3-yl}-4-(trifluoromethyl)benzamide

The title compound was prepared similarly to the conditions described in Example 4C substituting Example 3A for Example 4B and substituting 3-chloro-4-(trifluoromethyl)benzoic acid for 2-chloro-3-(trifluoromethyl)benzoic acid. MS (ESI) *m*/*z* 531 (M+H)⁺.

### Example 64

### N-{trans-4-(4-fluorophenyl)-1-[(1-methyl-1H-imidazol-4-yl)sulfonyl]pyrrolidin-3-yl}-4-(trifluoromethyl)benzamide

The title compound was prepared similarly to the conditions described in Example 4C substituting Example 3A for Example 4B and substituting 4-(trifluoromethyl)benzoic acid for 2-chloro-3-(trifluoromethyl)benzoic acid. MS (ESI) *m*/*z* 497 (M+H)⁺.

### Example 65

### 3,5-dichloro-N-{trans-4-(4-fluorophenyl)-1-[(1-methyl-1H-imidazol-4-yl)sulfonyl]pyrrolidin-3-yl}benzamide

The title compound was prepared similarly to the conditions described in Example 4C substituting Example 3A for Example 4B and substituting 3,5-dichlorobenzoic acid for 2-chloro-3-(trifluoromethyl)benzoic acid. MS (ESI) *m*/*z* 497 (M+H)⁺.

### Example 66

### trans-1-[(1-methyl-1H-imidazol-4-yl)sulfonyl]-4-phenyl-N-[2-(trifluoromethyl)phenyl]pyrrolidin-3-amine

The title compound was prepared similarly to the conditions described in Example 57 substituting 1-bromo-2-(trifluoromethyl)benzene for 1-bromo-2-methylbenzene. MS (ESI) *m*/*z* 451 (M+H)⁺.

### Example 67

### trans-1-[(1-methyl-1H-imidazol-4-yl)sulfonyl]-4-phenyl-N-[3-(trifluoromethyl)phenyl]pyrrolidin-3-amine

The title compound was prepared similarly to the conditions described in Example 57 substituting 1-bromo-3-(trifluoromethyl)benzene for 1-bromo-2-methylbenzene. MS (ESI) *m*/*z* 451 (M+H)⁺.

### Example 68

### trans-1-[(1-methyl-1H-imidazol-4-yl)sulfonyl]-4-phenyl-N-[4-(trifluoromethyl)phenyl]pyrrolidin-3-amine

The title compound was prepared similarly to the conditions described in Example 57 substituting 1-bromo-4-(trifluoromethyl)benzene for 1-bromo-2-methylbenzene. MS (ESI) *m*/*z* 451 (M+H)⁺.

### Example 69

### trans-N-(2-fluorophenyl)-1-[(1-methyl-1H-imidazol-4-yl)sulfonyl]-4-phenylpyrrolidin-3-amine

The title compound was prepared similarly to the conditions described in Example 57 substituting 1-bromo-2-fluorobenzene for 1-bromo-2-methylbenzene. MS (ESI) *m*/*z* 401 (M+H)⁺.

### Example 70

### trans-N-(3-fluorophenyl)-1-[(1-methyl-1H-imidazol-4-yl)sulfonyl]-4-phenylpyrrolidin-3-amine

The title compound was prepared similarly to the conditions described in Example 57 substituting 1-bromo-3-fluorobenzene for 1-bromo-2-methylbenzene. MS (APCI) *m*/*z* 401 (M+H)⁺.

### Example 71

### trans-N-(4-fluorophenyl)-1-[(1-methyl-1H-imidazol-4-yl)sulfonyl]-4-phenylpyrrolidin-3-amine

The title compound was prepared similarly to the conditions described in Example 57 substituting 1-bromo-4-fluorobenzene for 1-bromo-2-methylbenzene. MS (ESI) *m*/*z* 401 (M+H)⁺.

### Example 72

### 3-({trans-4-(4-fluorophenyl)-1-[(1-methyl-1H-imidazol-4-yl)sulfonyl]pyrrolidin-3-yl}amino)benzonitrile

The title compound was prepared similarly to the conditions described in Example 57 substituting Example 3A for Example 14A and substituting 3-bromo-benzenonitrile for 1-bromo-2-methylbenzene. MS (ESI) *m*/*z* 426 (M+H)⁺.

### Example 73

### trans-N-(2-chlorophenyl)-1-[(1-methyl-1H-imidazol-4-yl)sulfonyl]-4-phenylpyrrolidin-3-amine

The title compound was prepared similarly to the conditions described in Example 57 substituting 1-bromo-2-chlorobenzene for 1-bromo-2-methylbenzene. MS (APCI) *m*/*z* 418 (M+H)⁺.

### Example 74

### trans-N-(3-chlorophenyl)-1-[(1-methyl-1H-imidazol-4-yl)sulfonyl]-4-phenylpyrrolidin-3-amine

The title compound was prepared similarly to the conditions described in Example 57 substituting 1-bromo-3-chlorobenzene for 1-bromo-2-methylbenzene. MS (ESI) *m*/*z* 417 (M+H)⁺.

### Example 75

### trans-N-(4-chlorophenyl)-1-[(1-methyl-1H-imidazol-4-yl)sulfonyl]-4-phenylpyrrolidin-3-amine The title compound was prepared similarly to the conditions described in Example 57 substituting 1-bromo-4-chlorobenzene for 1-bromo-2-methylbenzene. MS (ESI) m/z 417 (M+H)⁺.

### Example 76

### 4-fluoro-3-({trans-1-[(1-methyl-1H-imidazol-4-yl)sulfonyl]-4-phenylpyrrolidin-3-yl}amino)benzonitrile

The title compound was prepared similarly to the conditions described in Example 57 substituting 3-bromo-4-fluorobenzonitrile for 1-bromo-2-methylbenzene. MS (APCI) *m*/*z* 426 (M+H)⁺.

### Example 77

### 2-fluoro-3-({trans-1-[(1-methyl-1H-imidazol-4-yl)sulfonyl]-4-phenylpyrrolidin-3-yl}amino)benzonitrile

The title compound was prepared similarly to the conditions described in Example 57 substituting 3-bromo-2-fluorobenzonitrile for 1-bromo-2-methylbenzene. MS (ESI) *m*/*z* 426 (M+H)⁺.

### Example 78

### 3-fluoro-5-({trans-1-[(1-methyl-1H-imidazol-4-yl)sulfonyl]-4-phenylpyrrolidin-3-yl}amino)benzonitrile

The title compound was prepared similarly to the conditions described in Example 57 substituting 3-bromo-5-fluorobenzonitrile for 1-bromo-2-methylbenzene. MS (ESI) *m*/*z* 426 (M+H)⁺.

### Example 79

### 2-chloro-5-({trans-1-[(1-methyl-1H-imidazol-4-yl)sulfonyl]-4-phenylpyrrolidin-3-yl}amino)benzonitrile

The title compound was prepared similarly to the conditions described in Example 57 substituting 5-bromo-2-chlorobenzonitrile for 1-bromo-2-methylbenzene. MS (ESI) *m*/*z* 442 (M+H)⁺.

### Example 80

### 3-chloro-5-({trans-1-[(1-methyl-1H-imidazol-4-yl)sulfonyl]-4-phenylpyrrolidin-3-yl}amino)benzonitrile

The title compound was prepared similarly to the conditions described in Example 57 substituting 3-bromo-5-chlorobenzonitrile for 1-bromo-2-methylbenzene. MS (ESI) *m*/*z* 442 (M+H)⁺.

### Example 81

### 4-chloro-3-({trans-1-[(1-methyl-1H-imidazol-4-yl)sulfonyl]-4-phenylpyrrolidin-3-yl}amino)benzonitrile

The title compound was prepared similarly to the conditions described in Example 57 substituting 3-bromo-4-chlorobenzonitrile for 1-bromo-2-methylbenzene. MS (ESI) *m*/*z* 442 (M+H)⁺.

### Example 82

### 4-methyl-3-({trans-1-[(1-methyl-1H-imidazol-4-yl)sulfonyl]-4-phenylpyrrolidin-3-yl}amino)benzonitrile

The title compound was prepared similarly to the conditions described in Example 57 substituting 3-bromo-4-methylbenzonitrile for 1-bromo-2-methylbenzene. MS (ESI) *m*/*z* 422 (M+H)⁺.

### Example 83

### 2-methyl-3-({trans-1-[(1-methyl-1H-imidazol-4-yl)sulfonyl]-4-phenylpyrrolidin-3-yl}amino)benzonitrile

The title compound was prepared similarly to the conditions described in Example 57 substituting 3-bromo-2-methylbenzonitrile for 1-bromo-2-methylbenzene. MS (ESI) *m*/*z* 422 (M+H)⁺.

### Example 84

### trans-4-(4-fluorophenyl)-1-[(1-methyl-1H-imidazol-4-yl)sulfonyl]-N-(2-methylphenyl)pyrrolidin-3-amine

The title compound was prepared similarly to the conditions described in Example 57 substituting Example 3A for Example 14A. MS (ESI) *m*/*z* 415 (M+H)⁺.

### Example 85

### trans-4-(4-fluorophenyl)-1-[(1-methyl-1H-imidazol-4-yl)sulfonyl]-N-(3-methylphenyl)pyrrolidin-3-amine

The title compound was prepared similarly to the conditions described in Example 57 substituting Example 3A for Example 14A and substituting 1-bromo-3-methylbenzene for 1-bromo-2-methylbenzene. MS (ESI) *m*/*z* 415 (M+H)⁺.

### Example 86

### trans-4-(4-fluorophenyl)-1-[(1-methyl-1H-imidazol-4-yl)sulfonyl]-N-(4-methylphenyl)pyrrolidin-3-amine

The title compound was prepared similarly to the conditions described in Example 57 substituting Example 3A for Example 14A and substituting 1-bromo-4-methylbenzene for 1-bromo-2-methylbenzene. MS (ESI) *m*/*z* 415 (M+H)⁺.

### Example 87

### trans-4-(4-fluorophenyl)-1-[(1-methyl-1H-imidazol-4-yl)sulfonyl]-N-[2-(trifluoromethyl)phenyl]pyrrolidin-3 -amine

The title compound was prepared similarly to the conditions described in Example 57 substituting Example 3A for Example 14A and substituting 1-bromo-2-(trifluoromethyl)benzene for 1-bromo-2-methylbenzene. MS (ESI) *m*/*z* 469 (M+H)⁺.

### Example 88

### trans-4-(4-fluorophenyl)-1-[(1-methyl-1H-imidazol-4-yl)sulfonyl]-N-[3-(trifluoromethyl)phenyl]pyrrolidin-3 -amine

The title compound was prepared similarly to the conditions described in Example 57 substituting Example 3A for Example 14A and substituting 1-bromo-3-(trifluoromethyl)benzene for 1-bromo-2-methylbenzene. MS (ESI) *m*/*z* 469 (M+H)⁺.

### Example 89

### trans-4-(4-fluorophenyl)-1-[(1-methyl-1H-imidazol-4-yl)sulfonyl]-N-[4-(trifluoromethyl)phenyl]pyrrolidin-3 -amine

The title compound was prepared similarly to the conditions described in Example 57 substituting Example 3A for Example 14A and substituting 1-bromo-4-(trifluoromethyl)benzene for 1-bromo-2-methylbenzene. MS (ESI) *m*/*z* 469 (M+H)⁺.

### Example 90

### trans-N-(2-fluorophenyl)-4-(4-fluorophenyl)-1-[(1-methyl-1H-imidazol-4-yl)sulfonyl]pyrrolidin-3-amine

The title compound was prepared similarly to the conditions described in Example 57 substituting Example 3A for Example 14A and substituting 1-bromo-2-fluorobenzene for 1-bromo-2-methylbenzene. MS (ESI) *m*/*z* 419 (M+H)⁺.

### Example 91

### trans-N-(3-fluorophenyl)-4-(4-fluorophenyl)-1-[(1-methyl-1H-imidazol-4-yl)sulfonyl]pyrrolidin-3-amine

The title compound was prepared similarly to the conditions described in Example 57 substituting Example 3A for Example 14A and substituting 1-bromo-3-fluorobenzene for 1-bromo-2-methylbenzene. MS (ESI) *m*/*z* 419 (M+H)⁺.

### Example 92

### trans-N,4-bis(4-fluorophenyl)-1-[(1-methyl-1H-imidazol-4-yl)sulfonyl]pyrrolidin-3-amine

The title compound was prepared similarly to the conditions described in Example 57 substituting Example 3A for Example 14A and substituting 1-bromo-4-fluorobenzene for 1-bromo-2-methylbenzene. MS (ESI) *m*/*z* 419 (M+H)⁺.

### Example 93

trans-N-(2-chlorophenyl)-4-(4-fluorophenyl)-1-[(1-methyl-1H-imidazol-4-yl)sulfonyl]pyrrolidin-3-amine

The title compound was prepared similarly to the conditions described in Example 57 substituting Example 3A for Example 14A and substituting 1-bromo-2-chloroobenzene for 1-bromo-2-methylbenzene. MS (ESI) *m*/*z* 435 (M+H)⁺.

### Example 94

### trans-N-(3-chlorophenyl)-4-(4-fluorophenyl)-1-[(1-methyl-1H-imidazol-4-yl)sulfonyl]pyrrolidin-3-amine

The title compound was prepared similarly to the conditions described in Example 57 substituting Example 3A for Example 14A and substituting 1-bromo-3-chlorobenzene for 1-bromo-2-methylbenzene. MS (ESI) *m*/*z* 435 (M+H)⁺.

### Example 95

### trans-N-(4-chlorophenyl)-4-(4-fluorophenyl)-1-[(1-methyl-1H-imidazol-4-yl)sulfonyl]pyrrolidin-3 - amine

The title compound was prepared similarly to the conditions described in Example 57 substituting Example 3A for Example 14A and substituting 1-bromo-4-chlorobenzene for 1-bromo-2-methylbenzene. MS (ESI) *m*/*z* 435 (M+H)⁺.

### Example 100

### 2-methoxy-5-({trans-1-[(1-methyl-1H-imidazol-4-yl)sulfonyl]-4-phenylpyrrolidin-3-yl}amino)benzonitrile

The title compound was prepared similarly to the conditions described in Example 57 substituting 5-bromo-2-methoxybenzonitrile for 1-bromo-2-methylbenzene. MS (ESI) *m*/*z* 438 (M+H)⁺.

### Example 101

### 3-methyl-5-({trans-1-[(1-methyl-1H-imidazol-4-yl)sulfonyl]-4-phenylpyrrolidin-3-yl}amino)benzonitrile

The title compound was prepared similarly to the conditions described in Example 57 substituting 3-bromo-5-methylbenzonitrile for 1-bromo-2-methylbenzene. MS (ESI) *m*/*z* 422 (M+H)⁺.

### Example 102

### 5-({trans-1-[(1-methyl-1H-imidazol-4-yl)sulfonyl]-4-phenylpyrrolidin-3-yl}amino)-2-(trifluoromethoxy)benzonitrile

The title compound was prepared similarly to the conditions described in Example 57 substituting 5-bromo-2-(trifluoromethoxy)benzonitrile for 1-bromo-2-methylbenzene. MS (ESI) *m*/*z* 476 (M-CH₃)⁺.

### Example 115

### 2-chloro-N-{trans-4-(2-chlorophenyl)-1-[(1-methyl-1H-imidazol-4-yl)sulfonyl]pyrrolidin-3-yl}-3-(trifluoromethyl)benzamide

The title compound was prepared similarly to the procedures described in Example 2 substituting (E)-1-chloro-2-(2-nitrovinyl)benzene for (E)-1-methoxy-4-(2-nitrovinyl)benzene in Example 2A. MS (DCI) *m*/*z* 547.2 (M+H)⁺.

### Reference Example 122

### N-[trans-1-(ethylsulfonyl)-4-phenylpyrrolidin-3-y]-4-(trifluoromethyl)benzamide

### Example 122A

### trans-1-(ethylsulfonyl)-4-phenylpyrrolidin-3-amine

The title compound was prepared as an HCl salt similarly to Example 2A-2F substituting (E)-(2-nitrovinyl)benzene for (E)-1-methoxy-4-(2-nitrovinyl)benzene and substituting ethanesulfonyl chloride for 1-methyl-1H-imidazole-4-sulfonyl chloride.

### Example 122B

### N-[trans-1-(ethylsulfonyl)-4-phenylpyrrolidin-3-yl]-4-(trifluoromethyl)benzamide

The title compound was prepared similarly to Example 4C substituting Example 122A for Example 4B and substituting 4-(trifluoromethyl)benzoic acid for 2-chloro-3-(trifluoromethyl)benzoic acid. MS (ESI) *m*/*z* 427 (M+H)⁺.

### Reference Example 123

### 3,5-dichloro-N-[trans-1-(ethylsulfonyl)-4-phenylpyrrolidin-3-yl]benzamide

The title compound was prepared similarly to Example 4C substituting Example 122A for Example 4B and substituting 3,5-dichlorobenzoic acid for 2-chloro-3-(trifluoromethyl)benzoic acid. MS (ESI) *m*/*z* 427 (M+H)⁺.

### Reference Example 124

### 2-chloro-N-[trans-1-(ethylsulfonyl)-4-phenylpyrrolidin-3-yl]-3-(trifluoromethyl)benzamide

The title compound was prepared similarly to Example 4C substituting Example 122A for Example 4B. MS (ESI) *m*/*z* 461 (M+H)⁺.

### Reference Example 125

### N-[trans-4-phenyl-1-(propylsulfonyl)pyrrolidin-3-yl]-4-(trifluoromethyl)benzamide

### Example 125A

The title compound was prepared as an HCl salt similarly to the procedures described for Examples 2A-2F substituting (E)-(2-nitrovinyl)benzene for (E)-1-methoxy-4-(2-nitrovinyl)benzene and substituting propanesulfonyl chloride for 1-methyl-1H-imidazole-4-sulfonyl chloride.

### Example 125B

### N-[trans-4-phenyl-1-(propylsulfonyl)pyrrolidin-3-yl]-4-(trifluoromethyl)benzamide

The title compound was prepared similarly to the procedure described in Example 4C substituting Example 125A for Example 4B and substituting 4-(trifluoromethyl)benzoic acid for 2-chloro-3-(trifluoromethyl)benzoic acid. MS (ESI) *m*/*z* 441 (M+H)⁺.

### Reference Example 126

### 3,5-dichloro-N-[trans-4-phenyl-1-(propylsulfonyl)pyrrolidin-3-yl]benzamide

The title compound was prepared similarly to Example 4C substituting Example 125A for Example 4B and substituting 3,5-dichlorobenzoic acid for 2-chloro-3-(trifluoromethyl)benzoic acid. MS (ESI) *m*/*z* 441 (M+H)⁺.

### Reference Example 127

### 2-chloro-N-[trans-4-phenyl-1-(propylsulfonyl)pyrrolidin-3-yl]-3-(trifluoromethyl)benzamide

The title compound was prepared similarly to Example 4C substituting Example 125A for Example 4B. MS (ESI) *m*/*z* 475 (M+H)⁺.

### Reference Example 128

### N-{trans-1-[(cyclopropylmethyl)sulfonyl]-4-phenylpyrrolidin-3-yl}-4-(trifluoromethyl)benzamide

### Example 128A

### trans-1-(cyclopropylmethylsulfonyl)-4-phenylpyrrolidin-3-amine

The title compound was prepared as an HCl salt similarly to the procedure described in Example 2A-2F substituting (E)-(2-nitrovinyl)benzene for (E)-1-methoxy-4-(2-nitrovinyl)benzene and substituting cyclopropylmethanesulfonyl chloride for 1-methyl-1H-imidazole-4-sulfonyl chloride.

### Example 128B

### N-{trans-1-[(cyclopropylmethyl)sulfonyl]-4-phenylpyrrolidin-3-yl}-4-(trifluoromethyl)benzamide

The title compound was prepared similarly to Example 4C substituting Example 128A for Example 4B and substituting 4-(trifluoromethyl)benzoic acid for 2-chloro-3-(trifluoromethyl)benzoic acid. MS (ESI) *m*/*z* 453 (M+H)⁺.

### Reference Example 129

### 3,5-dichloro-N-{trans-1-[(cyclopropylmethyl)sulfonyl]-4-phenylpyrrolidin-3-yl}benzamide

The title compound was prepared similarly to Example 4C substituting Example 128A for Example 4B and substituting 3,5-dichlorobenzoic acid for 2-chloro-3-(trifluoromethyl)benzoic acid. MS (ESI) *m*/*z* 453 (M+H)⁺.

### Reference Example 130

### 2-chloro-N-{trans-1-[(cyclopropylmethyl)sulfonyl]-4-phenylpyrrolidin-3-yl}-3-(trifluoromethyl)benzamide

The title compound was prepared similarly to Example 4C substituting Example 128A for Example 4B. MS (ESI) *m*/*z* 487 (M+H)⁺.

### Reference Example 131

### 3-{[trans-1-(ethylsulfonyl)-4-phenylpyrrolidin-3-yl]amino}benzonitrile

The title compound was prepared similarly to the procedure described in Example 57 substituting Example 122A for Example 14A and substituting 3-bromo-benzonitrile for 1-bromo-2-methylbenzene. MS (ESI) *m*/*z* 356 (M+H)⁺.

### Reference Example 132

### 3-{[trans-4-phenyl-1-(propylsulfonyl)pyrrolidin-3-yl]amino}benzonitrile

The title compound was prepared similarly to the procedure described in Example 57 substituting Example 125A for Example 14A and substituting 3-bromo-benzonitrile for 1-bromo-2-methylbenzene. MS (ESI) *m*/*z* 370 (M+H)⁺.

### Reference Example 133

### 3-({trans-1-[(cyclopropylmethyl)sulfonyl]-4-phenylpyrrolidin-3-yl}amino)benzonitrile

The title compound was prepared similarly to the procedure described in Example 57 substituting Example 128A for Example 14A and substituting 3-bromo-benzonitrile for 1-bromo-2-methylbenzene. MS (ESI) *m*/*z* 382 (M+H)⁺.

### Reference Example 134

### N-{trans-1-[(4-methoxyphenyl)sulfonyl]-4-phenylpyrrolidin-3-yl}-4-(trifluoromethyl)benzamide

### Example 134A

### trans-1-(4-methoxyphenylsulfonyl)-4-phenylpyrrolidin-3-amine

The title compound was prepared as an HCl salt similarly to Example 2A-2F substituting (E)-(2-nitrovinyl)benzene for (E)-1-methoxy-4-(2-nitrovinyl)benzene and substituting 4-methoxybenzene-1-sulfonyl chloride for 1-methyl-1H-imidazole-4-sulfonyl chloride.

### Example 134B

### N-{trans-1-[(4-methoxyphenyl)sulfonyl]-4-phenylpyrrolidin-3-yl}-4-(trifluoromethyl)benzamide

The title compound was prepared similarly to the procedure described in Example 4C substituting Example 134A for Example 4B and substituting 4-(trifluoromethyl)benzoic acid for 2-chloro-3-(trifluoromethyl)benzoic acid. MS (ESI) *m*/*z* 505 (M+H)⁺.

### Reference Example 135

### 3,5-dichloro-N-{trans-1-[([(4-methoxyphenyl)sulfonyl]-4-phenylpyrrolidin-3-yl}benzamide

The title compound was prepared similarly to the procedure described in Example 4C substituting Example 134A for Example 4B and substituting 3,5-dichlorobenzoic acid for 2-chloro-3-(trifluoromethyl)benzoic acid. MS (ESI) *m*/*z* 505 (M+H)⁺,

### Reference Example 136

### 2-chloro-N-{trans-1-[(4-methoxyphenyl)sulfonyl]-4-phenylpyrrolidin-3-yl}-3-(trifluoromethyl)benzamide

The title compound was prepared similarly to the procedure described in Example 4C substituting Example 134A for Example 4B. MS (ESI) *m*/*z* 539 (M+H)⁺.

### Reference Example 137

### 3-({trans-1-[(4-methoxyphenyl)sulfonyl]-4-phenylpyrrolidin-3-yl}amino)benzonitrile

The title compound was prepared similarly to the procedure described in Example 57 substituting Example 134A for Example 14A and substituting 3-bromo-benzonitrile for 1-bromo-2-methylbenzene.. MS (ESI) *m*/*z* 434 (M+H)⁺.

### Reference Example 142

### 3,5-dichloro-N-[trans-4-phenyl-1-{[4-(trifluoromethyl)phenyl]sulfonyl}pyrrolidin-3-yl]benzamide

### Example 142A

### trans-4-phenyl-1-(4-(trifluoromethyl)phenylsulfonyl)pyrrolidin-3-amine

The title compound was prepared as an HCl salt similarly to Example 2A-2F substituting (E)-(2-nitrovinyl)benzene for (E)-1-methoxy-4-(2-nitrovinyl)benzene and substituting 4-(trifluoromethyl)benzene-1-sulfonyl chloride for 1-methyl-1H-imidazole-4-sulfonyl chloride.

### Example 142B

### 3,5-dichloro-N-[trans-4-phenyl-1-{[4-(trifluoromethyl)phenyl]sulfonyl}pyrrolidin-3-yl]benzamide

The title compound was prepared similarly to the procedure described in Example 4C substituting Example 142A for Example 4B and substituting 3,5-dichlorobenzoic acid for 2-chloro-3-(trifluoromethyl)benzoic acid. MS (ESI) *m*/*z* 543 (M+H)⁺.

### Reference Example 143

### 2-chloro-N-[trans-4-phenyl-1-{[4-(trifluoromethyl)phenyl]sulfonyl}pyrrolidin-3-yl]-3-(trifluoromethyl)benzamide

The title compound was prepared similarly to the procedure described in Example 4C substituting Example 142A for Example 4B. MS (ESI) *m*/*z* 577 (M+H)⁺.

### Reference Example 144

### 3-{[trans-4-phenyl-1-{[4-(trifluoromethyl)phenyl]sulfonyl}pyrrolidin-3-yl]amino}benzonitrile

The title compound was prepared similarly to the procedure described in Example 57 substituting Example 142A for Example 14A and substituting 3-bromo-benzonitrile for 1-bromo-2-methylbenzene. MS (ESI) *m*/*z* 472 (M+H)⁺.

### Example 145

### 4-{[trans-3-benzyl-4-phenylpyrrolidin-1-yl]sulfonyl}-1-methyl-1H-imidazole

### Example 145A

### (1-benzyl-4-phenylpyrrolidin-3-yl)(phenyl)methanone

A solution of N-benzyl-1-methoxy-N-((trimethylsilyl)methyl)methanamine (5.9 g, 0.025 mole) in dichloromethane (30 mL) was stirred under nitrogen, in an ice/methanol bath. Chalcone (4.16g, 0.02 mole) was added dropwise and the mixture was stirred in the cold bath overnight The reaction mixture was washed with aqueous sodium bicarbonate, brine, and dried over sodium sulfate. The solvent was evaporated to provide the title compound. MS (ESI) *m*/*z* 342 [M+H]⁺.

### Example 145B

### 3-benzyl-4-phenylpyrrolidine hydrochloride

Example 145A (8.5 g, 0.025 mole) was hydrogenated with 10% Pd/C (0.25 g) in methanol (200 mL). The reaction mixture was filtered, dissolved in methanol (100 mL) and passed through Amberlite resin. The filtrate was concentrated, redissolved in toluene and concentrated. This procedure was repeated several times until the residue was dry. MS (ESI) *m*/*z* 338 [M+H]⁺.

### Example 145C

### 4-(trans-3-benzyl-4-phenylpyrrolidin-1-ylsulfonyl)-1-methyl-1H-imidazole

To a solution of Example 145B (.0767 g, 0.280 mmol) in dichloromethane (1.121 ml) was added triethylamine (0.156 ml, 1.121 mmol) and 1-methyl-1H-imidazole-4-sulfonyl chloride (0.051 g, 0.280 mmol), The mixture was stirred at room temperature for 15 minutes. The solvent was evaporated. The crude material was purified by HPLC to afford the title compound. MS (ESI) *m*/*z* 382.2 [M+H]⁺.

### Example 148

### trans-N-benzyl-4-(4-fluorophenyl)-1-[(1-methyl-1H-imidazol-4-yl)sulfonyl]pyrrolidin-3-amine

To a stirred solution of Example 3A (100 mg, 0.28 mmol) in a buffer 4 pH solution (5 mL, made from 48 g AcOH and 30.5 g NaOAc in 1 L methanol) was added benzaldehyde (24 mg, .22 mmol) and MgSO₄ (334 mg, 2.77 mmol). The reaction was allowed to stir for 1 hour at room temperature before MgSO₄ was removed via filtration. To the filtrate was added MP-cyanoborohydride (2.19 mmol/g, 380 mg, 0.83 mmol) and the reaction was allowed to stir for 24 hours. The resin was removed via filtration and purification via HPLC provided the title compound. MS (ESI) *m*/*z* 415 (M+H)⁺.

### Example 149

### trans-N-[2-chloro-3-(trifluoromethyl)benzyl]-4-(4-fluorophenyl)-1-[(1-methyl-1H-imidazol-4-yl)sulfonyl]pyrrolidin-3-amine

The title compound was prepared similarly to the procedure described in Example 148 substituting 2-chloro-3-(trifluoromethyl)benzaldehyde for benzaldehyde. MS (ESI) *m*/*z* 517 (M+H)⁺.

### Example 150

### trans-N-[2-chloro-4-(trifluoromethyl)benzyl]-4-(4-fluorophenyl)-1-[(1-methyl-1H-imidazol-4-yl)sulfonyl]pyrrolidin-3 -amine

The title compound was prepared similarly to the procedure described in Example 148 substituting 2-chloro-4-(trifluoromethyl)benzaldehyde for benzaldehyde. MS (ESI) *m*/*z* 517 (M+H)⁺.

### Example 151

### trans-N-[3-chloro-4-(trifluoromethyl)benzyl]-4-(4-fluorophenyl)-1-[(1-methyl-1H-imidazol-4-yl)sulfonyl]pyrrolidin-3 -amine

The title compound was prepared similarly to the procedure described in Example 148 substituting 3-chloro-4-(trifluoromethyl)benzaldehyde for benzaldehyde. MS (ESI) *m*/*z* 517 (M+H)⁺.

### Example 152

### 3-[({trans-4-(4-fluorophenyl)-1-[(1-methyl-1H-imidazol-4-yl)sulfonyl]pyrrolidin-3-yl}amino)methyl]benzonitrile

The title compound was prepared similarly to the procedure described in Example 148 substituting 3-cyanobenzaldehyde for benzaldehyde. MS (ESI) *m*/*z* 440 (M+H)⁺.

### Example 153

### trans-4-(4-fluorophenyl)-N-(2-methylbenzyl)-1-[(1-methyl-1H-imidazol-4-yl)sulfonyl]pyrrolidin-3-amine

The title compound was prepared similarly to the procedure described in Example 148 substituting 2-methylbenzaldehyde for benzaldehyde. MS (ESI) *m*/*z* 429 (M+H)⁺.

### Example 154

### trans-4-(4-fluorophenyl)-N-(3-methylbenzyl)-1-[(1-methyl-1H-imidazol-4-yl)sulfonyl]pyrrolidin-3-amine

The title compound was prepared similarly to the procedure described in Example 148 substituting 3-methylbenzaldehyde for benzaldehyde. MS (ESI) *m*/*z* 429 (M+H)⁺.

### Example 155

### trans-4-(4-fluorophenyl)-N-(4-methylbenzyl)-1-[(1-methyl-1H-imidazol-4-yl)sulfonyl]pyrrolidin-3-amine

The title compound was prepared similarly to the procedure described in Example 148 substituting 4-methylbenzaldehyde for benzaldehyde. MS (ESI) *m*/*z* 429 (M+H)⁺.

### Example 156

### trans-N-(4-fluorobenzyl)-4-(4-fluorophenyl)-1-[(1-methyl-1H-imidazol-4-yl)sulfonyl]pyrrolidin-3-amine

The title compound was prepared similarly to the procedure described in Example 148 substituting 4-fluorobenzaldehyde for benzaldehyde. MS (ESI) *m*/*z* 433 (M+H)⁺.

### Example 157

### (cis)-N-benzyl-1-[(1-methyl-1H-imidazol-4-yl)sulfonyl]-4-phenylpyrrolidin-3-amine

### Example 157A

### 1-benzyl-3-(benzylamino)-4-chloro-1H-pyrrole-2,5 -dione

To a stirred solution of 3,4-dichlorofuran-2,5-dione (5 g, 30 mmol) in acetic acid (17 mL) was added benzylamine (3.2 g, 30 mmol) and the reaction was stirred at room temperature for 1 hour. More benzylamine (3.2 g, 30 mmol) was added and the reaction was heated to 100 °C for 2 hours. The reaction mixture was concentrated. The mixture was neutralized with aqueous sodium hydroxide and partitioned with ethyl acetate. The organic fraction was collected and concentrated. Purification via flash chromatography (0-60%EtOAc/hexanes) provided the title compound.

### Example 157B

### 1-benzyl-3-(benzylamino)-4-chloro-1H-pyrrole-2,5 -dione

To a solution of Example 157A (5.5 g, 16.8 mmol) in 1,2-dimethoxyethane (60 mL) was added phenylboronic acid (2.46 g, 20.2 mmol), dicyclohexyl(2',6'-dimethoxybiphenyl-2-yl)phosphine (691 mg, 1.68 mmol), palldium(II) acetate (189 mg, 0.848 mmol), and potassium carbonate (2 M, 16.8 mL, 33.7 mmol). The reaction mixture was refluxed for 2 hours. The reaction was partitioned between water and EtOAc. The organic layer was collected and concentrated. Purification via flash chromatography (0-80%EtOAc/hexanes) provided the title compound.

### Example 157C

### tert-butyl benzyl(1-benzyl-2,5-dioxo-4-phenyl-2,5-dihydro-1H-pyrrol-3-yl)carbamate

To a stirred solution of Example 157B (4.13 g, 11.2 mmol) and triethylamine (113 mg, 1.12 mmol) in dichloromethane (50 mL) at 0 °C was added di-tert-butyl dicarbonate (1 M in tetrahydrofuran, 12.3 mL, 12.3 mmol) and N,N-dimethylpyridin-4-amine (137 mg, 1.12 mmol). The reaction was allowed to warm to room temperature after 1 hour and stirred at room temperature for 24 hours. More di-tert-butyl dicarbonate (1 M in tetrahydrofuran, 3 mL, 3 mmol) was added and the reaction mixture stirred for 2 more hours. The reaction mixture was then concentrated. Purification via flash chromatography (0-10% methanol/DCM) provided the title compound.

### Example 157D

### tert-butyl benzyl(cis-1-benzyl-2,5-dioxo-4-phenylpyrrolidin-3-yl)carbamate

To a solution of Example 157C (25 mg, 0.053 mmol) in trifluoroethane (4 ml) in a 50 ml pressure bottle was added 20% Pd(OH)2/C, wet (6.25 mg, 0.045 mmol). The reaction mixture stirred for 16 hours at 30 psi hydrogen and 50 °C. The mixture was filtered through a nylon membrane and the filtrate was concentrated to provide the title compound.

### Example 157E

### tert-butyl benzyl((cis)-1-benzyl-4-phenylpyrrolidin-3-yl)carbamate

To a solution of Example 157D (2.5 g, 5.3 mmol) in tetrahydrofuran (50 mL) was added borane (1 M in tetrahydrofuran, 21.3 mL, 21.3 mmol). The reaction mixture was heated to 70 °C for 18 hours. The reaction was cooled to 50 °C and methanol (20 mL) was added slowly. The reaction mixture stirred for 1 hour. The reaction mixture was concentrated. Purification via flash chromatography (0-100 % EtOAc/hexanes) provided the title compound.

### Example 157F

### tert-butyl benzyl((cis)-4-phenylpyrrolidin-3 -yl)carbamate

To a pressure vial was added Example 157E (801 mg, 1.810 mmol), tetrahydrofuran (20 ml) and Pd(OH)₂/C, (20%, wet, 160 mg, 1.141 mmol) and the reaction mixture was stirred for 16 hours at 30 psi of hydrogen at 50 °C. The mixture was filtered through a nylon membrane and the filtrate was concentrated to provide the title compound.

### Example 157G

### (cis)-N-benzyl-1-(1-methyl-1H-imidazol-4-ylsulfonyl)-4-phenylpyrrolidin-3-amine

The title compound was prepared similarly to the procedures described in Example 2E-2F substituting Example 157F for Example 2D. MS (ESI) *m*/*z* 397 (M+H)⁺.

### Example 158

### trans-N-(3,4-difluorophenyl)-4-(4-fluorophenyl)-1-[(1-methyl-1H-imidazol-4-yl)sulfonyl]pyrrolidin-3 -amine

The title compound was prepared similarly to the procedure described in Example 57 substituting Example 3A for Example 14A and substituting 4-bromo-1,2-difluorobenzene for 1-bromo-2-methylbenzene. MS (ESI) *m*/*z* 437 (M+H)⁺.

### Example 159

### trans-4-(4-fluorophenyl)-N-[4-fluoro-3-(trifluoromethyl)phenyl]-1-[(1-methyl-1H-imidazol-4-yl)sulfonyl]pyrrolidin-3 -amine

The title compound was prepared similarly to the procedure described in Example 57 substituting Example 3A for Example 14A and substituting 4-bromo-1-fluoro-2-(trifluoromethyl)benzene for 1-bromo-2-methylbenzene. MS (ESI) *m*/*z* 437 (M+H)⁺.

### Example 160

### trans-N-(3-chloro-4-fluorophenyl)-4-(4-fluorophenyl)-1-[(1-methyl-1H-imidazol-4-yl)sulfonyl]pyrrolidin-3 -amine

The title compound was prepared similarly to the procedure described in Example 57 substituting Example 3A for Example 14A and substituting 4-bromo-2-chloro-1-fluorobenzene for 1-bromo-2-methylbenzene. MS (ESI) *m*/*z* 453 (M+H)⁺.

### Example 161

### trans-N-(3,4-dichlorophenyl)-4-(4-fluorophenyl)-1-[(1-methyl-1H-imidazol-4-yl)sulfonyl]pyrrolidin-3-amine

The title compound was prepared similarly to the procedure described in Example 57 substituting Example 3A for Example 14A and substituting 4-bromo-1,2-dichlorobenzene for 1-bromo-2-methylbenzene. MS (ESI) *m*/*z* 469 (M+H)⁺.

### Example 162

### trans-N-(4-chloro-3-fluorophenyl)-4-(4-fluorophenyl)-1-[(1-methyl-1H-imidazol-4-yl)sulfonyl]pyrrolidin-3 -amine

The title compound was prepared similarly to the procedure described in Example 57 substituting Example 3A for Example 14A and substituting 4-bromo-1-chloro-2-fluorobenzene for 1-bromo-2-methylbenzene. MS (ESI) *m*/*z* 453 (M+H)⁺.

### Example 163

### trans-N-(2,3-difluorophenyl)-4-(4-fluorophenyl)-1-[(1-methyl-1H-imidazol-4-yl)sulfonyl]pyrrolidin-3-amine

The title compound was prepared similarly to the procedure described in Example 57 substituting Example 3A for Example 14A and substituting 1-bromo-2,3-difluorobenzene for 1-bromo-2-methylbenzene. MS (ESI) *m*/*z* 437 (M+H)⁺.

### Example 164

### trans-N-(2,5-difluorophenyl)-4-(4-fluorophenyl)-1-[(1-methyl-1H-imidazol-4-yl)sulfonyl]pyrrolidin-3-amine

The title compound was prepared similarly to the procedure described in Example 57 substituting Example 3A for Example 14A and substituting 1-bromo-2,5-difluorobenzene for 1-bromo-2-methylbenzene. MS (ESI) *m*/*z* 437 (M+H)⁺.

### Example 165

### trans-N-(2,4-difluorophenyl)-4-(4-fluorophenyl)-1-[(1-methyl-1H-imidazol-4-yl)sulfonyl]pyrrolidin-3-amine

The title compound was prepared similarly to the procedure described in Example 57 substituting Example 3A for Example 14A and substituting 1-bromo-2,4-difluorobenzene for 1-bromo-2-methylbenzene. MS (ESI) *m*/*z* 437 (M+H)⁺.

### Example 166

### trans-4-(4-fluorophenyl)-1-[(1 -methyl-1H-imidazol-4-yl)sulfonyl]-N-(2,3,4-trifluorophenyl)pyrrolidin-3 -amine

The title compound was prepared similarly to the procedure described in Example 57 substituting Example 3A for Example 14A and substituting 1-bromo-2,3,4-trifluorobenzene for 1-bromo-2-methylbenzene. MS (ESI) *m*/*z* 455 (M+H)⁺.

### Example 167

### trans-4-(4-fluorophenyl)-N-[2-fluoro-3-(trifluoromethyl)phenyl]-1-[(1-methyl-1H-imidazol-4-yl)sulfonyl]pyrrolidin-3 -amine

The title compound was prepared similarly to the procedure described in Example 57 substituting Example 3A for Example 14A and substituting 1-bromo-2-fluoro-3-(trifluoromethyl)benzene for 1-bromo-2-methylbenzene. MS (ESI) *m*/*z* 487 (M+H)⁺.

### Example 168

### trans-4-(4-fluorophenyl)-N-[3-fluoro-5-(trifluoromethyl)phenyl]-1-[(1-methyl-1H-imidazol-4-yl)sulfonyl]pyrrolidin-3 -amine

The title compound was prepared similarly to the procedure described in Example 57 substituting Example 3A for Example 14A and substituting 1-bromo-3-fluoro-5-(trifluoromethyl)benzene for 1-bromo-2-methylbenzene. MS (ESI) *m*/*z* 487 (M+H)⁺.

### Example 169

### trans-4-(4-fluorophenyl)-N-[2-fluoro-5-(trifluoromethyl)phenyl]-1-[(1-methyl-1H-imidazol-4-yl)sulfonyl]pyrrolidin-3 -amine

The title compound was prepared similarly to the procedure described in Example 57 substituting Example 3A for Example 14A and substituting 1-bromo-2-fluoro-5-(trifluoromethyl)benzene for 1-bromo-2-methylbenzene. MS (ESI) *m*/*z* 487 (M+H)⁺.

### Example 170

### trans-4-(4-fluorophenyl)-1-[(1-methyl-1H-imidazol-4-yl)sulfonyl]-N-[3-(propan-2-yl)phenyl]pyrrolidin-3 -amine

The title compound was prepared similarly to the procedure described in Example 57 substituting Example 3A for Example 14A and substituting 1-bromo-3-isopropylbenzene for 1-bromo-2-methylbenzene. MS (ESI) *m*/*z* 443 (M+H)⁺.

### Example 171

### trans-N-(3-tert-butylphenyl)-4-(4-fluorophenyl)-1-[(1-methyl-1H-imidazol-4-yl)sulfonyl]pyrrolidin-3-amine

The title compound was prepared similarly to the procedure described in Example 57 substituting Example 3A for Example 14A and substituting 1-bromo-3-tert-butylbenzene for 1-bromo-2-methylbenzene. MS (ESI) *m*/*z* 457 (M+H)⁺.

### Example 172

### trans-N-[4-chloro-3-(trifluoromethyl)phenyl]-4-(4-fluorophenyl)-1-[(1-methyl-1H-imidazol-4-yl)sulfonyl]pyrrolidin-3 -amine

The title compound was prepared similarly to the procedure described in Example 57 substituting Example 3A for Example 14A and substituting 4-bromo-1-chloro-2-(trifluoromethyl)benzene for 1-bromo-2-methylbenzene. MS (ESI) *m*/*z* 503 (M+H)⁺.

### Example 173

### trans-N-[3-chloro-4-(trifluoromethyl)phenyl]-4-(4-fluorophenyl)-1-[(1-methyl-1H-imidazol-4-yl)sulfonyl]pyrrolidin-3 -amine

The title compound was prepared similarly to the procedure described in Example 57 substituting Example 3A for Example 14A and substituting 4-bromo-2-chloro-1-(trifluoromethyl)benzene for 1-bromo-2-methylbenzene. MS (ESI) *m*/*z* 503 (M+H)⁺.

### Example 174

### trans-N-(3-chloro-5-fluorophenyl)-4-(4-fluorophenyl)-1-[(1-methyl-1H-imidazol-4-yl)sulfonyl]pyrrolidin-3 -amine

The title compound was prepared similarly to the procedure described in Example 57 substituting Example 3A for Example 14A and substituting 1-bromo-3-chloro-5-fluorobenzene for 1-bromo-2-methylbenzene. MS (ESI) *m*/*z* 453 (M+H)⁺.

### Example 175

### trans-N-(3-chloro-4,5-difluorophenyl)-4-(4-fluorophenyl)-1-[(1-methyl-1H-imidazol-4-yl)sulfonyl]pyrrolidin-3 -amine

The title compound was prepared similarly to the procedure described in Example 57 substituting Example 3A for Example 14A and substituting 1-bromo-3-chloro-4,5-difluorobenzene for 1-bromo-2-methylbenzene. MS (ESI) *m*/*z* 471 (M+H)⁺.

### Example 176

### trans-4-(4-fluorophenyl)-1-[(1-methyl-1H-imidazol-4-yl)sulfonyl]-N-[3-(trifluoromethoxy)phenyl]pyrrolidin-3-amine

The title compound was prepared similarly to the procedure described in Example 57 substituting Example 3A for Example 14A and substituting 1-bromo-3-(tirfluoromethoxy)benzene for 1-bromo-2-methylbenzene. MS (ESI) *m*/*z* 485 (M+H)⁺.

### Example 177

### N-{trans-4-(4-fluorophenyl)-1-[(1-methyl-1H-imidazol-4-yl)sulfonyl]pyrrolidin-3-yl}pyridin-4-amine

The title compound was prepared similarly to the procedure described in Example 57 substituting Example 3A for Example 14A and substituting 4-bromopyridine hydrochloride for 1-bromo-2-methylbenzene. MS (ESI) *m*/*z* 402 (M+H)⁺.

### Example 178

### N-{trans-4-(4-fluorophenyl)-1-[(1-methyl-1H-imidazol-4-yl)sulfonyl]pyrrolidin-3-yl}pyridin-2-amine

The title compound was prepared similarly to the procedure described in Example 57 substituting Example 3A for Example 14A and substituting 2-bromopyridine for 1-bromo-2-methylbenzene. MS (ESI) *m*/*z* 402 (M+H)⁺.

### Example 179

### N-{trans-4-(4-fluorophenyl)-1-[(1-methyl-1H-imidazol-4-yl)sulfonyl]pyrrolidin-3-yl}pyridin-3-amine

The title compound was prepared similarly to the procedure described in Example 57 substituting Example 3A for Example 14A and substituting 3-bromopyridine for 1-bromo-2-methylbenzene. MS (ESI) *m*/*z* 402 (M+H)⁺.

### Example 180

### N-{trans-4-(4-fluorophenyl)-1-[(1-methyl-1H-imidazol-4-yl)sulfonyl]pyrrolidin-3-yl}-6-(trifluoromethyl)pyridin-2-amine

The title compound was prepared similarly to the procedure described in Example 57 substituting Example 3A for Example 14A and substituting 2-bromo-6-(trifluoromethyl)pyridine for 1-bromo-2-methylbenzene. MS (ESI) *m*/*z* 470 (M+H)⁺.

### Example 181

### N-{trans-4-(4-fluorophenyl)-1-[(1-methyl-1H-imidazol-4-yl)sulfonyl]pyrrolidin-3-yl} -5-(trifluoromethyl)pyridin-3 -amine

The title compound was prepared similarly to the procedure described in Example 57 substituting Example 3A for Example 14A and substituting 3-bromo-5-(trifluoromethyl)pyridine for 1-bromo-2-methylbenzene. MS (ESI) *m*/*z* 470 (M+H)⁺.

### Example 182

### 4-{[3-(3-chlorobenzyl)-4-(4-fluorophenyl)pyrrolidin-1-yl]sulfonyl}-1-methyl-1H-imidazole

### Example 182A

### 2-(3 -chlorobenzyl)-3 -(4-fluorophenyl)-4-nitrobutanal

To a solution of (E)-1-fluoro-4-(2-nitrovinyl)benzene (1 g, 6.0 mmol) in dichloromethane (10 mL) was added 3-(3-chlorophenyl)propanal (1 g, 5.9 mmol), triethylamine (50 mL), and L-proline (50 mg, 0.43 mmol). The mixture was allowed to stir for 16 hours and the crude material was purified by silica gel column chromatography e(25% ethyl acetate/hexanes) to provide the title compound (4:1 mixture of 2 diastereomers).

### Example 182B

### 3-(3-chlorobenzyl)-4-(4-fluorophenyl)pyrrolidine

To a solution of Example 182A (0.92 g, 2.7 mmol) in methanol (5 mL) was added 50% acetic acid (aq) (5 mL) followed by zinc (6x200 mg, 19 mmol) and sodium cyanoborohydride (120 mg, 1.9 mmol). The mixture was allowed to stir for 30 minutes. Sodium hydroxide (1 M) was added to adjust the pH value to 8. Ethyl acetate was added and the solution partitioned. The organic fraction was collected and concentrated to provide the crude title compound.

### Example 182C

### 4-{[3-(3-chlorobenzyl)-4-(4-fluorophenyl)pyrrolidin-1-yl]sulfonyl}-1-methyl-1H-imidazole

To a solution of Example 182B (289 mg, 1.0 mmol) in dichloromethane (5 mL) was added triethylamine (130 mg, 1.3 mmol), and 1-methyl-1H-imidazole-4-sulfonyl chloride (180 mg, 1.0 mmol). The mixture was allowed to stir for 20 minutes and purified by silica gel column chromatography (100% ethyl acetate). The product was recrystallized in ethyl acetate to provide the title compound. MS (ESI) *m*/*z* 434/436 (3:1) (M+H)⁺.

### Example 183

### 2-chloro-N-{(cis)-1-[(1-methyl-1H-imidazol-4-yl)sulfonyl]-4-phenylpyrrolidin-3-yl}-3-(trifluoromethyl)benzamide

### Example 183A

A 20 mL pressure vial was charged with Example 157 (891.1 mg, 2.247 mmol), ethanol (6 mL) and dihydroxypalladium (60.2 mg, 0.429 mmol). The mixture was stirred under 60 psi of hydrogen at 50 °C for 1.5 hours. Another 60 mg of catalyst was added, and the hydrogenation was continued for 2 hours more. The mixture was filtered through a polypropylene membrane and concentrated to provide the title compound.

### Example 183B

### 2-chloro-N-{(cis)-1-[(1-methyl-1H-imidazol-4-yl)sulfonyl]-4-phenylpyrrolidin-3-yl}-3-(trifluoromethyl)benzamide

The title compound was prepared similarly to the procedure described in Example 4C substituting Example 183A for Example 4B. MS (ESI) *m*/*z* 513 (M+H)⁺.

### Example 184

### (cis)-1-[(1-methyl-1H-imidazol-4-yl)sulfonyl]-N,4-diphenylpyrrolidin-3-amine

The title compound was prepared similarly to the procedure described in Example 57 substituting Example 183A for Example 14A and substituting bromobenzene for 1-bromo-2-methylbenzene. MS (ESI) *m*/*z* 383 (M+H)⁺.

### Example 185

### trans-4-(3-fluorophenyl)-1-[(1-methyl-1H-imidazol-4-yl)sulfonyl]-N-phenylpyrrolidin-3-amine

### Example 185A

### (E)-1-fluoro-3 -(2-nitrovinyl)benzene

A stirred solution containing 3-fluorobenzaldehyde (9.95 g, 80.1 mmol), ammonium acetate (17.3 g, 225 mmol), nitromethane (12.2 mL, 225 mmol), and acetic acid (75 mL) was heated to 100 °C for 3 hours. The mixture was cooled to room temperature and partitioned between water and dichloromethane. The organic fraction was collected and the aqueous fraction was washed with dichloromethane. The organic fractions were combined and washed with sodium bicarbonate (aq.), brine, and water. The organic fraction was dried with sodium sulfate and purification via flash chromatography (10%EtOAc/hexanes) provided the title compound.

### Example 185B

### trans-4-(3-fluorophenyl)-1-(1-methyl-1H-imidazol-4-ylsulfonyl)pyrrolidin-3 -amine

The title compound was prepared as the HCl salt similarly to the procedures described in Example 2A-2F substituting Example 185A for (E)-1-methoxy-4-(2-nitrovinyl)benzene.

### Example 185C

### trans-4-(3-fluorophenyl)-1-[(1-methyl-1H-imidazol-4-yl)sulfonyl]-N-phenylpyrrolidin-3-amine

The title compound was prepared similarly to the procedure described in Example 57 substituting Example 185B for Example 14A and substituting bromobenzene for 1-bromo-2-methylbenzene. MS (ESI) *m*/*z* 401 (M+H)⁺.

### Example 186

### trans-N,4-bis(3-fluorophenyl)-1-[(1-methyl-1H-imidazol-4-yl)sulfonyl]pyrrolidin-3-amine

The title compound was prepared similarly to the procedure described in Example 57 substituting Example 185B for Example 14A and substituting 1-bromo-3-fluorobenzene for 1-bromo-2-methylbenzene. MS (ESI) *m*/*z* 419 (M+H)⁺.

### Example 187

### trans-4-(3-fluorophenyl)-N-(4-fluorophenyl)-1-[(1-methyl-1H-imidazol-4-yl)sulfonyl]pyrrolidin-3-amine

The title compound was prepared similarly to the procedure described in Example 57 substituting Example 185B for Example 14A and substituting 1-bromo-4-fluorobenzene for 1-bromo-2-methylbenzene. MS (ESI) *m*/*z* 419 (M+H)⁺.

### Example 188

### trans-N-(3-chlorophenyl)-4-(3-fluorophenyl)-1-[(1-methyl-1H-imidazol-4-yl)sulfonyl]pyrrolidin-3-amine

The title compound was prepared similarly to the procedure described in Example 57 substituting Example 185B for Example 14A and substituting 1-bromo-3-chlorobenzene for 1-bromo-2-methylbenzene. MS (ESI) *m*/*z* 435 (M+H)⁺.

### Example 189

### trans-4-(3-fluorophenyl)-1-[(1-methyl-1H-imidazol-4-yl)sulfonyl]-N-[3-(trifluoromethyl)phenyl]pyrrolidin-3 -amine

The title compound was prepared similarly to the procedure described in Example 57 substituting Example 185B for Example 14A and substituting 1-bromo-3-(trifluoromethane)benzene for 1-bromo-2-methylbenzene. MS (ESI) *m*/*z* 469 (M+H)⁺.

### Example 190

### trans-4-(3 -fluorophenyl)-1 -[(1 -methyl-1H-imidazol-4-yl)sulfonyl] -N- [4-(trifluoromethyl)phenyl]pyrrolidin-3 -amine

The title compound was prepared similarly to the procedure described in Example 57 substituting Example 185B for Example 14A and substituting 1-bromo-4-(trifluoromethane)benzene for 1-bromo-2-methylbenzene. MS (ESI) *m*/*z* 469 (M+H)⁺.

### Example 191

### N-{trans-4-(3-fluorophenyl)-1-[(1-methyl-1H-imidazol-4-yl)sulfonyl]pyrrolidin-3-yl}pyridin-4-amine

The title compound was prepared similarly to the procedure described in Example 57 substituting Example 185B for Example 14A and substituting 4-bromopyridine hydrochloride for 1-bromo-2-methylbenzene. MS (ESI) *m*/*z* 402 (M+H)⁺.

### Example 192

### trans-N-(3,4-dichlorophenyl)-4-(3-fluorophenyl)-1-[(1-methyl-1H-imidazol-4-yl)sulfonyl]pyrrolidin-3 -amine

The title compound was prepared similarly to the procedure described in Example 57 substituting Example 185B for Example 14A and substituting 1-bromo-3,4-dichlorobenzene for 1-bromo-2-methylbenzene. MS (ESI) *m*/*z* 469 (M+H)⁺.

### Example 193

### trans-N-(4-chloro-3-fluorophenyl)-4-(3-fluorophenyl)-1-[(1-methyl-1H-imidazol-4-yl)sulfonyl]pyrrolidin-3 -amine

The title compound was prepared similarly to the procedure described in Example 57 substituting Example 185B for Example 14A and substituting 1-bromo-4-chloro-3-fluorobenzene for 1-bromo-2-methylbenzene. MS (ESI) *m*/*z* 453 (M+H)⁺.

### Example 194

### trans-N-(3-chloro-4-fluorophenyl)-4-(3-fluorophenyl)-1-[(1-methyl-1H-imidazol-4-yl)sulfonyl]pyrrolidin-3 -amine

The title compound was prepared similarly to the procedure described in Example 57 substituting Example 185B for Example 14A and substituting 1-bromo-3-chloro-4-fluorobenzene for 1-bromo-2-methylbenzene. MS (ESI) *m*/*z* 453 (M+H)⁺.

### Example 195

### N-{trans-4-(3-fluorophenyl)-1-[(1-methyl-1H-imidazol-4-yl)sulfonyl]pyrrolidin-3-yl} -6-(trifluoromethyl)pyridin-2-amine

The title compound was prepared similarly to the procedure described in Example 57 substituting Example 185B for Example 14A and substituting 2-bromo-6-(trifluoromethyl)pyridine for 1-bromo-2-methylbenzene. MS (ESI) *m*/*z* 470 (M+H)⁺.

### Example 196

### N-{trans-4-(3-fluorophenyl)-1-[(1-methyl-1H-imidazol-4-yl)sulfonyl]pyrrolidin-3-yl}-5-(trifluoromethyl)pyridin-3 -amine

The title compound was prepared similarly to the procedure described in Example 57 substituting Example 185B for Example 14A and substituting 3-bromo-5-(trifluoromethyl)pyridine for 1-bromo-2-methylbenzene. MS (ESI) *m*/*z* 470 (M+H)⁺.

### Example 197

### trans-N-(3,4-difluorophenyl)-4-(3-fluorophenyl)-1-[(1-methyl-1H-imidazol-4-yl)sulfonyl]pyrrolidin-3 -amine

The title compound was prepared similarly to the procedure described in Example 57 substituting Example 185B for Example 14A and substituting 1-bromo-3,4-difluorobenzene for 1-bromo-2-methylbenzene. MS (ESI) *m*/*z* 437 (M+H)⁺.

### Example 198

### trans-4-(2-fluorophenyl)-1-[(1-methyl-1H-imidazol-4-yl)sulfonyl]-N-phenylpyrrolidin-3-amine

### Example 198A

### trans-4-(2-fluorophenyl)-1-(1-methyl-1H-imidazol-4-ylsulfonyl)pyrrolidin-3 -amine

The title compound was prepared similarly to the procedures described in Example 2A-2F substituting (E)-1-fluoro-2-(2-nitrovinyl)benzene for (E)-(2-nitrovinyl)benzene.

### Example 198B

### trans-4-(2-fluorophenyl)-1-[(1-methyl-1H-imidazol-4-yl)sulfonyl]-N-phenylpyrrolidin-3-amine

The title compound was prepared similarly to the procedure described in Example 57 substituting Example 198A for Example 14A and substituting bromobenzene for 1-bromo-2-methylbenzene. MS (ESI) *m*/*z* 401 (M+H)⁺.

### Example 199

### trans-4-(2-fluorophenyl)-N-(3-fluorophenyl)-1-[(1-methyl-1H-imidazol-4-yl)sulfonyl]pyrrolidin-3-amine

The title compound was prepared similarly to the procedure described in Example 57 substituting Example 198A for Example 14A and substituting 1-bromo-3-fluorobenzene for 1-bromo-2-methylbenzene. MS (ESI) *m*/*z* 419 (M+H)⁺.

### Example 200

### trans-4-(2-fluorophenyl)-N-(4-fluorophenyl)-1-[(1-methyl-1H-imidazol-4-yl)sulfonyl]pyrrolidin-3-amine

The title compound was prepared similarly to the procedure described in Example 57 substituting Example 198A for Example 14A and substituting 1-bromo-4-fluorobenzene for 1-bromo-2-methylbenzene. MS (ESI) *m*/*z* 419 (M+H)⁺.

### Example 201

### trans-N-(3-chlorophenyl)-4-(2-fluorophenyl)-1-[(1-methyl-1H-imidazol-4-yl)sulfonyl]pyrrolidin-3-amine

The title compound was prepared similarly to the procedure described in Example 57 substituting Example 198A for Example 14A and substituting 1-bromo-3-chlorobenzene for 1-bromo-2-methylbenzene. MS (ESI) *m*/*z* 435 (M+H)⁺.

### Example 202

### trans-N-(4-chlorophenyl)-4-(2-fluorophenyl)-1-[(1-methyl-1H-imidazol-4-yl)sulfonyl]pyrrolidin-3-amine

The title compound was prepared similarly to the procedure described in Example 57 substituting Example 198A for Example 14A and substituting 1-bromo-4-chlorobenzene for 1-bromo-2-methylbenzene. MS (ESI) *m*/*z* 435 (M+H)⁺.

### Example 203

### N-{trans-4-(2-fluorophenyl)-1-[(1-methyl-1H-imidazol-4-yl)sulfonyl]pyrrolidin-3-yl}-6-(trifluoromethyl)pyridin-2-amine

The title compound was prepared similarly to the procedure described in Example 57 substituting Example 198A for Example 14A and substituting 2-bromo-6-(trifluoromethyl)pyridine for 1-bromo-2-methylbenzene. MS (ESI) *m*/*z* 470 (M+H)⁺.

### Example 204

### N-{trans-4-(2-fluorophenyl)-1-[(1-methyl-1H-imidazol-4-yl)sulfonyl]pyrrolidin-3-yl}-2-(trifluoromethyl)pyridin-4-amine

The title compound was prepared similarly to the procedure described in Example 57 substituting Example 198A for Example 14A and substituting 4-bromo-2-(trifluoromethyl)pyridine for 1-bromo-2-methylbenzene. MS (ESI) *m*/*z* 470 (M+H)⁺.

### Example 205

### 4-{[3-(2-bromobenzyl)-4-(2-bromophenyl)pyrrolidin-1-yl]sulfonyl}-1-methyl-1H-imidazole

### Example 205A

### 2-(2-bromobenzyl)-3-(2-bromophenyl)-4-nitrobutanal

The title compound was prepared using the conditions described in Example 182A substituting (E)-1-bromo-2-(2-nitrovinyl)benzene for (E)-1-fluoro-4-(2-nitrovinyl)benzene and 3-(2-bromophenyl)propanal for 3-(3-chlorophenyl)propanal.

### Example 205B

### 3-(2-bromobenzyl)-4-(2-bromophenyl)pyrrolidine

The title compound was prepared using the conditions described in Example 182B substituting Example 205A for Example 182A.

### Example 205C

### 4-{[3-(2-bromobenzyl)-4-(2-bromophenyl)pyrrolidin-1-yl]sulfonyl}-1-methyl-1H-imidazole

The title compound was prepared using the conditions described in Example 182C substituting Example 205B for Example 182B. MS (ESI) *m*/*z* 537/539/541(1:2:1) (M+H)⁺.

### Example 206

{Trans-4-(4-fluorophenyl)-1-[(1-methyl-1H-imidazol-4-yl)sulfonyl]pyrrolidin-3-yl}methanol To a solution of Example 242C (620 mg, 1.69 mmol) in 6 mL of tetrahydrofuran at -30 °C was added lithium aluminum hydride (2N in tetrahydrofuran, 0.93 mL, 1.86 mmol),. The reaction mixture stirred at -30°C for 30 min. The reaction mixture was quenched with a saturated sodium bicarbonate solution, and the solution was partitioned with EtOAc. The organic fraction was collected, washed with water dried over sodium sulfate, and concentrated to give the title compound. MS (ESI) *m*/*z* 340.0 (M+H)⁺.

### Example 207

### N-{trans-1-[(1-methyl-1H-imidazol-4-yl)sulfonyl]-4-phenylpyrrolidin-3-yl}pyridin-2-amine

The title compound was prepared similarly to the procedure described in Example 57 substituting 2-bromopyridine for 1-bromo-2-methylbenzene. MS (ESI) *m*/*z* 384 (M+H)⁺.

### Example 208

### N-{trans-1-[(1-methyl-1H-imidazol-4-yl)sulfonyl]-4-phenylpyrrolidin-3-yl}pyridin-3-amine

The title compound was prepared similarly to the procedure described in Example 57 substituting 3-bromopyridine for 1-bromo-2-methylbenzene. MS (ESI) *m*/*z* 384 (M+H)⁺.

### Example 209

### N-{trans-1-[(1-methyl-1H-imidazol-4-yl)sulfonyl]-4-phenylpyrrolidin-3-yl}pyridin-4-amine

The title compound was prepared similarly to the procedure described in Example 57 substituting 4-bromopyridine for 1-bromo-2-methylbenzene. MS (ESI) *m*/*z* 384 (M+H)⁺.

### Example 210

### N-{trans-1-[(1-methyl-1H-imidazol-4-yl)sulfonyl]-4-phenylpyrrolidin-3-yl}-6-(trifluoromethyl)pyridin-2-amine

The title compound was prepared similarly to the procedure described in Example 57 substituting 2-bromo-6-(trifluoromethyl)pyridine for 1-bromo-2-methylbenzene. MS (ESI) *m*/*z* 452 (M+H)⁺.

### Example 211

### N-{trans-1-[(1-methyl-1H-imidazol-4-yl)sulfonyl]-4-phenylpyrrolidin-3-yl}-2-(trifluoromethyl)pyridin-4-amine

The title compound was prepared similarly to the procedure described in Example 57 substituting 4-bromo-2-(trifluoromethyl)pyridine for 1-bromo-2-methylbenzene. MS (ESI) *m*/*z* 452 (M+H)⁺.

### Example 212

### N-{trans-1-[(1-methyl-1H-imidazol-4-yl)sulfonyl]-4-phenylpyrrolidin-3-yl}-4-(trifluoromethyl)pyridin-2-amine

The title compound was prepared similarly to the procedure described in Example 57 substituting 2-bromo-4-(trifluoromethyl)pyridine for 1-bromo-2-methylbenzene. MS (ESI) *m*/*z* 452 (M+H)⁺.

### Example 213

### N- {trans-1-[(1-methyl-1H-imidazol-4-yl)sulfonyl]-4-phenylpyrrolidin-3 -yl} -5 - (trifluoromethyl)pyridin-3 -amine

The title compound was prepared similarly to the procedure described in Example 57 substituting 3-bromo-5-(trifluoromethyl)pyridine for 1-bromo-2-methylbenzene. MS (ESI) *m*/*z* 452 (M+H)⁺.

### Example 214

### trans-N-[4-fluoro-3-(trifluoromethyl)phenyl]-1-[(1-methyl-1H-imidazol-4-yl)sulfonyl]-4-phenylpyrrolidin-3 -amine

The title compound was prepared similarly to the procedure described in Example 57 substituting 4-bromo-1-fluoro-2-(trifluoromethyl)benzene for 1-bromo-2-methylbenzene. MS (ESI) *m*/*z* 469 (M+H)⁺.

### Example 215

### trans-N-(3-chloro-4-fluorophenyl)-1-[(1-methyl-1H-imidazol-4-yl)sulfonyl]-4-phenylpyrrolidin-3-amine

The title compound was prepared similarly to the procedure described in Example 57 substituting 4-bromo-2-chloro-1-fluorobenzene for 1-bromo-2-methylbenzene. MS (ESI) *m*/*z* 435 (M+H)⁺.

### Example 216

### trans-N-(3,4-difluorophenyl)-1-[(1-methyl-1H-imidazol-4-yl)sulfonyl]-4-phenylpyrrolidin-3-amine

The title compound was prepared similarly to the procedure described in Example 57 substituting 4-bromo-1,2-difluorobenzene for 1-bromo-2-methylbenzene. MS (ESI) *m*/*z* 419 (M+H)⁺.

### Example 217

### trans-N-[4-chloro-3-(trifluoromethyl)phenyl]-1-[(1-methyl-1H-imidazol-4-yl)sulfonyl]-4-phenylpyrrolidin-3 -amine

The title compound was prepared similarly to the procedure described in Example 57 substituting 4-bromo-1-chloro-2-(trifluoromethyl)benzene for 1-bromo-2-methylbenzene. MS (ESI) *m*/*z* 485 (M+H)⁺.

### Example 218

### trans-N-(3,4-dichlorophenyl)-1-[(1-methyl-1H-imidazol-4-yl)sulfonyl]-4-phenylpyrrolidin-3-amine

The title compound was prepared similarly to the procedure described in Example 57 substituting 4-bromo-1,2-dichlorobenzene for 1-bromo-2-methylbenzene. MS (ESI) *m*/*z* 451 (M+H)⁺.

### Example 219

### trans-N-(3-chloro-4,5-difluorophenyl)-1-[(1-methyl-1H-imidazol-4-yl)sulfonyl]-4-phenylpyrrolidin-3-amine

The title compound was prepared similarly to the procedure described in Example 57 substituting 5-bromo-1-chloro-2,3-difluorobenzene for 1-bromo-2-methylbenzene. MS (ESI) *m*/*z* 453 (M+H)⁺.

### Example 220

### trans-1-[(1-methyl-1H-imidazol-4-yl)sulfonyl]-4-phenyl-N-[3-(propan-2-yl)phenyl]pyrrolidin-3-amine

The title compound was prepared similarly to the procedure described in Example 57 substituting 1-bromo-3-isopropylbenzene for 1-bromo-2-methylbenzene. MS (ESI) *m*/*z* 425 (M+H)⁺.

### Example 221

### trans-N-(3-tert-butylphenyl)-1-[(1-methyl-1H-imidazol-4-yl)sulfonyl] -4-phenylpyrrolidin-3 -amine

The title compound was prepared similarly to the procedure described in Example 57 substituting 1-bromo-3-tert-butyl-benzene for 1-bromo-2-methylbenzene. MS (ESI) *m*/*z* 439 (M+H)⁺.

### Example 222

### trans-N-[2-fluoro-3-(trifluoromethyl)phenyl]-1-[(1-methyl-1H-imidazol-4-yl)sulfonyl]-4-phenylpyrrolidin-3 -amine

The title compound was prepared similarly to the procedure described in Example 57 substituting 1-bromo-2-fluoro-3-(trifluoromethyl)benzene for 1-bromo-2-methylbenzene. MS (ESI) *m*/*z* 469 (M+H)⁺.

### Example 223

### trans-N-[3-fluoro-5-(trifluoromethyl)phenyl]-1-[(1-methyl-1H-imidazol-4-yl)sulfonyl]-4-phenylpyrrolidin-3 -amine

The title compound was prepared similarly to the procedure described in Example 57 substituting 1-bromo-3-fluoro-5-(trifluoromethyl)benzene for 1-bromo-2-methylbenzene. MS (ESI) *m*/*z* 469 (M+H)⁺.

### Example 224

### trans-N-[2-fluoro-5-(trifluoromethyl)phenyl]-1-[(1-methyl-1H-imidazol-4-yl)sulfonyl]-4-phenylpyrrolidin-3 -amine

The title compound was prepared similarly to the procedure described in Example 57 substituting 1-bromo-2-fluoro-5-(trifluoromethyl)benzene for 1-bromo-2-methylbenzene. MS (ESI) *m*/*z* 469 (M+H)⁺.

### Example 225

### trans-N-(3-chloro-5-fluorophenyl)-1-[(1-methyl-1H-imidazol-4-yl)sulfonyl]-4-phenylpyrrolidin-3-amine

The title compound was prepared similarly to the procedure described in Example 57 substituting 1-bromo-3-chloro-5-fluorobenzene for 1-bromo-2-methylbenzene. MS (ESI) *m*/*z* 435 (M+H)⁺.

### Example 226

### trans-1-[(1-methyl-1H-imidazol-4-yl)sulfonyl]-4-phenyl-N-[3-(trifluoromethoxy)phenyl]pyrrolidin-3-amine

The title compound was prepared similarly to the procedure described in Example 57 substituting 1-bromo-3-(trifluoromethoxy)benzene for 1-bromo-2-methylbenzene. MS (ESI) *m*/*z* 467 (M+H)⁺.

### Example 227

### N-{trans-1-[(1-methyl-1H-imidazol-4-yl)sulfonyl]-4-phenylpyrrolidin-3-yl}-4-(trifluoromethyl)pyrimidin-2-amine

The title compound was prepared similarly to the procedure described in Example 57 substituting 2-bromo-4-(trifluoromethyl)pyrimidine for 1-bromo-2-methylbenzene. MS (ESI) *m*/*z* 453 (M+H)⁺.

### Example 228

### N-{trans-1-[(1-methyl-1H-imidazol-4-yl)sulfonyl]-4-phenylpyrrolidin-3-yl}quinolin-7-amine

The title compound was prepared similarly to the procedure described in Example 57 substituting 7-bromoquinoline for 1-bromo-2-methylbenzene. MS (ESI) *m*/*z* 434 (M+H)⁺.

### Example 229

### N-{trans-1-[(1-methyl-1H-imidazol-4-yl)sulfonyl]-4-phenylpyrrolidin-3-yl}quinolin-6-amine

The title compound was prepared similarly to the procedure described in Example 57 substituting 6-bromoquinoline for 1-bromo-2-methylbenzene. MS (ESI) *m*/*z* 434 (M+H)⁺.

### Example 230

### N-{trans-1-[(1-methyl-1H-imidazol-4-yl)sulfonyl]-4-phenylpyrrolidin-3-yl}isoquinolin-6-amine

The title compound was prepared similarly to the procedure described in Example 57 substituting 6-bromoisoquinoline for 1-bromo-2-methylbenzene. MS (ESI) *m*/*z* 434 (M+H)⁺.

### Example 231

### trans-N-(2,3-difluorophenyl)-1-[(1-methyl-1H-imidazol-4-yl)sulfonyl]-4-phenylpyrrolidin-3-amine

The title compound was prepared similarly to the procedure described in Example 57 substituting 1-bromo-2,3-difluorobenzene for 1-bromo-2-methylbenzene. MS (ESI) *m*/*z* 419 (M+H)⁺.

### Example 232

### trans-N-(2,5-difluorophenyl)-1-[(1-methyl-1H-imidazol-4-yl)sulfonyl]-4-phenylpyrrolidin-3-amine

The title compound was prepared similarly to the procedure described in Example 57 substituting 1-bromo-2,5-difluorobenzene for 1-bromo-2-methylbenzene. MS (ESI) *m*/*z* 419 (M+H)⁺.

### Example 233

### trans-N-(2,4-difluorophenyl)-1-[(1-methyl-1H-imidazol-4-yl)sulfonyl]-4-phenylpyrrolidin-3-amine

The title compound was prepared similarly to the procedure described in Example 57 substituting 1-bromo-2,4-difluorobenzene for 1-bromo-2-methylbenzene. MS (ESI) *m*/*z* 419 (M+H)⁺.

### Example 234

### trans-1-[(1-methyl-1H-imidazol-4-yl)sulfonyl]-4-phenyl-N-(2,3,4-trifluorophenyl)pyrrolidin-3-amine

The title compound was prepared similarly to the procedure described in Example 57 substituting 1-bromo-2,3,4-trifluorobenzene for 1-bromo-2-methylbenzene. MS (ESI) *m*/*z* 437 (M+H)⁺.

### Example 235

### 6-fluoro-N-{trans-1-[(1-methyl-1H-imidazol-4-yl)sulfonyl]-4-phenylpyrrolidin-3-yl}pyridin-2-amine

The title compound was prepared similarly to the procedure described in Example 57 substituting 2-bromo-6-fluoropyridine for 1-bromo-2-methylbenzene. MS (ESI) *m*/*z* 402 (M+H)⁺.

### Example 236

### 2-fluoro-N-{trans-1-[(1-methyl-1H-imidazol-4-yl)sulfonyl]-4-phenylpyrrolidin-3-yl}pyridin-4-amine

The title compound was prepared similarly to the procedure described in Example 57 substituting 4-bromo-2-fluoropyridine for 1-bromo-2-methylbenzene. MS (ESI) *m*/*z* 402 (M+H)⁺.

### Example 237

### 5-fluoro-N-{trans-1-[(1-methyl-1H-imidazol-4-yl)sulfonyl]-4-phenylpyrrolidin-3-yl}pyridin-3-amine

The title compound was prepared similarly to the procedure described in Example 57 substituting 3-bromo-5-fluoropyridine for 1-bromo-2-methylbenzene. MS (ESI) *m*/*z* 402 (M+H)⁺.

### Example 238

### 6-fluoro-N-{trans-4-(4-fluorophenyl)-1-[(1-methyl-1H-imidazol-4-yl)sulfonyl]pyrrolidin-3-yl}pyridin-2-amine

The title compound was prepared similarly to the procedure described in Example 57 substituting Example 3A for Example 14A and substituting 2-bromo-6-fluoropyridine for 1-bromo-2-methylbenzene. MS (ESI) *m*/*z* 420 (M+H)⁺.

### Example 239

### 2-fluoro-N-{trans-4-(4-fluorophenyl)-1-[(1-methyl-1H-imidazol-4-yl)sulfonyl]pyrrolidin-3-yl} pyridin-4-amine

The title compound was prepared similarly to the procedure described in Example 57 substituting Example 3A for Example 14A and substituting 4-bromo-2-fluoropyridine for 1-bromo-2-methylbenzene. MS (ESI) *m*/*z* 420 (M+H)⁺.

### Example 240

### N- {trans-1-[(1-methyl-1H-imidazol-4-yl)sulfonyl]-4-phenylpyrrolidin-3 -yl} -6-(trifluoromethyl)pyrimidin-4-amine

The title compound was prepared similarly to the procedure described in Example 57 substituting 4-bromo-6-(trifluoromethyl)pyrimidine for 1-bromo-2-methylbenzene. MS (ESI) *m*/*z* 453 (M+H)⁺.

### Example 241

### N-{trans-1-[(1-methyl-1H-imidazol-4-yl)sulfonyl]-4-phenylpyrrolidin-3-yl}isoquinolin-7-amine

The title compound was prepared similarly to the procedure described in Example 57 substituting 7-bromoisoquinoline for 1-bromo-2-methylbenzene. MS (ESI) *m*/*z* 434 (M+H)⁺.

### Example 242

### trans-4-(4-fluorophenyl)-1-[(1-methyl-1H-imidazol-4-yl)sulfonyl]-N-phenylpyrrolidine-3-carboxamide

### Example 242A

A solution of (E)-methyl 3-(4-fluorophenyl)acrylate (10.52 g, 58.4 mmol) and N-benzyl-1-methoxy-N-((trimethylsilyl)methyl)methanamine (19.41 g, 81.8 mmol) in 110 mL of dichloromethane was cooled to 0°C. Trifluoroacetic acid (0.495 ml, 6.42 mmol) was slowly added under N₂. The reaction mixture was stirred at 0°C for 1 hour, then stirred at room temperature for 18 hours. The reaction mixture was partitioned with saturated sodium bicarbonate. The organic fraction was collected, concentrated, and purified by flash-chromatography on silica gel (20-30% ethyl acetate in hexane) to provide the title compound.. MS (ESI) *m*/*z* 314.3 (M+H)⁺

### Example 242B

### Trans-methyl 4-(4-fluorophenyl)pyrrolidine-3 -carboxylate

Example 242A (17.98 g, 57.4 mmol) and tetrahydrofuran (10 ml) were added to 20% Pd(OH)₂/C, wet (3.60 g, 25.6 mmol) in a 250 mL SS pressure bottle and stirred for 16 hours under 30 psi of hydrogen at room temperature. The mixture was filtered through a nylon membrane and concentrated to provide the title compound. MS (ESI) *m*/*z* 224.0 (M+H)⁺.

### Example 242C

### Trans-methyl 4-(4-fluorophenyl)-1-(1-methyl-1H-imidazol-4-ylsulfonyl)pyrrolidine-3 -carboxylate

To Example 242 B (12.74 g, 57.1 mmol) in 15 mL of dichloromethane were added triethylamine (12.13 g, 120 mmol) and 4-dimethylaminopyridine (0.35 g, 2.85 mmol). The reaction mixture was cooled to 0 °C. 1-methyl-1H-imidazole-4-sulfonyl chloride (10.82 g, 59.9 mmol) was added portion wise at 0 °C. The reaction mixture was slowly warmed up to room temperature and stirred for 1 hour. The reaction mixture was partitioned with dichloromethane, and water. The organic fraction was collected, washed with water, concentrated, and purified by flash-chromatography on silica gel (100% ethyl acetate) to afford the title compound. MS (ESI) *m*/*z* 368.0 (M+H)⁺.

### Example 242D

### Trans-4-(4-fluorophenyl)-1-(1-methyl-1H-imidazol-4-ylsulfonyl)pyrrolidine-3-carboxylic acid

To Example 242C (3.67 g, 9.99 mmol) was added 4 mL of methanol. To this solution was added lithium hydroxide (1 M, methanol:water=5:3, 15 mL, 15 mmol). The reaction mixture was stirred at room temperature for 5 hours. The reaction mixture was concentrated, and the residue was then treated with hydrochloric acid (1 M, aq.) until pH=5. The reaction mixture was partitioned with ethyl acetate and the organic fraction was collected. The aqueous fraction was washed with ethyl acetate (3x), and the organic fractions were combined and concentrated to provide the title product. MS (ESI) *m*/*z* 354.0 (M+H)⁺.

### Example 242E

### Trans-4-(4-fluorophenyl)-1-[(1-methyl-1H-imidazol-4-yl)sulfonyl]-N-phenylpyrrolidine-3-carboxamide

To solution of Example 242D (150 mg, 0.42 mmol) in dimethylformamide/pyridine (1:1. 15 mL) was added 1-(3-dimethylaminopropyl)-3-ethylcarbodiimide hydrochloride (85 mg, 0.45 mmol) and aniline (41.5 mg, 0.45 mmol). The reaction mixture was stirred at room temperature for 18 hours. The reactiom mixture was concentrated purified by HPLC to provide the title product. MS (ESI) *m*/*z* 429.1 (M+H)⁺.

### Example 243

### Trans-4-(4-fluorophenyl)-1-[(1-methyl-1H-imidazol-4-yl)sulfonyl]-N-[4-(trifluoromethyl)phenyl]pyrrolidine-3-carboxamide

The title compound was prepared using the same sequence of steps described in Example 242 substituting 4-(trifluoromethyl)aniline for aniline. MS (ESI) *m*/*z* 497.0 (M+H)⁺.

### Example 244

### Trans-N-(3,5-dichlorophenyl)-4-(4-fluorophenyl)-1-[(1-methyl-1H-imidazol-4-yl)sulfonyl]pyrrolidine-3-carboxamide

The title compound was prepared using the procedures described in Example 242 substituting 3,5-dichloroaniline for aniline. MS (ESI) *m*/*z* 497.1 (M+H)⁺.

### Example 245

4-{[trans-3-(4-fluorophenyl)-4-(phenoxymethyl)pyrrolidin-1-yl]sulfonyl}-1-methyl-1H-imidazole To a solution of Example 206 (52 mg, 0.15 mmol) in tetrahydrofuran (1 mL) was added phenol (21.6 mg, 0.23 mmol), PS-triphenylphosphine (105 mg, 0.34 mmol, 3.2 mmol/g), and di-tert-butylazodicarboxylate (70.6 mg, 0.31 mmol). The reaction mixture was stirred at room temperature for 18 hours. The reaction mixture was filtered and washed with methanol. The filtrate was concentrated and purified by HPLC to provide the title compound. MS (ESI) *m*/*z* 416.1 (M+H)⁺.

### Example 246

### 4-{[trans-3-[(2,4-dichlorophenoxy)methyl]-4-(4-fluorophenyl)pyrrolidin-1-yl]sulfonyl}-1-methyl-1H-imidazole

The title compound was prepared using the procedure described in Example 245 substituting 2,4-dichlorophenol for phenol. MS (ESI) *m*/*z* 484.1 (M+H)⁺.

### Example 247

### 4-{[trans-3-(4-fluorophenyl)-4-{[3-(trifluoromethoxy)phenoxy]methyl}pyrrolidin-1-yl]sulfonyl}-1-methyl-1H-imidazole

The title compound was prepared using the procedure described in Example 245 substituting 3-(trifluoromethoxy)phenol for phenol. MS (ESI) *m*/*z* 500.1 (M+H)⁺.

### Example 248

### 4-{[trans-3-[(3-chlorophenoxy)methyl]-4-(4-fluorophenyl)pyrrolidin-1-yl]sulfonyl}-1-methyl-1H-imidazole

The title compound was prepared using the procedure described in Example 245 substituting 3-chlorophenol for phenol. MS (ESI) *m*/*z* 450.1 (M+H)⁺.

### Example 249

### 4-{[trans-3-{[4-chloro-3-(trifluoromethyl)phenoxy]methyl}-4-(4-fluorophenyl)pyrrolidin-1-yl]sulfonyl}-1-methyl-1H-imidazole

The title compound was prepared using the procedures described in Example 245 substituting 4-chloro-3-(trifluoromethyl)phenol for phenol. MS (ESI) *m*/*z* 518.2 (M+H)⁺.

### Example 250

### 4-{[trans-3-{[2-chloro-3-(trifluoromethyl)phenoxy]methyl}-4-(4-fluorophenyl)pyrrolidin-1-yl]sulfonyl}-1-methyl-1H-imidazole

The title compound was prepared using the procedures as in Example 245 substituting 2-chloro-3-(trifluoromethyl)phenol for phenol. MS (ESI) *m*/*z* 518.1 (M+H)⁺.

### Example 251

### 4-{[trans-3-[(3-fluorophenoxy)methyl]-4-(4-fluorophenyl)pyrrolidin-1-yl]sulfonyl}-1-methyl-1H-imidazole

The title compound was prepared using the procedure described in Example 245 substituting 3-fluorophenol for phenol. MS (ESI) *m*/*z* 434.1 (M+H)⁺.

### Example 252

### trans-4-(2-chlorophenyl)-N-(3-fluorophenyl)-1-[(1-methyl-1H-imidazol-4-yl)sulfonyl]pyrrolidin-3-amine

### Example 252A

### trans-4-(2-chlorophenyl)-1-(1-methyl-1H-imidazol-4-ylsulfonyl)pyrrolidin-3-amine

The title compound was prepared similarly to the procedures described in Example 2A-2F substituting (E)-1-chloro-2-(2-nitrovinyl)benzene for (E)-1-methoxy-4-(2-nitrovinyl)benzene.

### Example 252B

### trans-4-(2-chlorophenyl)-N-(3-fluorophenyl)-1-[(1-methyl-1H-imidazol-4-yl)sulfonyl]pyrrolidin-3-amine

The title compound was prepared similarly to the procedure described in Example 57 substituting Example 252A for Example 14A and substituting 1-bromo-3-fluorobenzene for 1-bromo-2-methylbenzene. MS (ESI) *m*/*z* 435 (M+H)⁺.

### Example 253

### trans-4-(2-chlorophenyl)-N-(4-fluorophenyl)-1-[(1-methyl-1H-imidazol-4-yl)sulfonyl]pyrrolidin-3-amine

### Example 253A

### trans-4-(2-chlorophenyl)-1-(1-methyl-1H-imidazol-4-ylsulfonyl)pyrrolidin-3-amine

The title compound was prepared similarly to the procedure described in Example 3A substituting (E)-1-chloro-2-(2-nitrovinyl)benzene for (E)-(2-nitrovinyl)benzene.

### Example 253B

### trans-4-(2-chlorophenyl)-N-(4-fluorophenyl)-1-[(1-methyl-1H-imidazol-4-yl)sulfonyl]pyrrolidin-3-amine

The title compound was prepared similarly to the procedure described in Example 57 substituting Example 253A for Example 14A and substituting 1-bromo-4-fluorobenzene for 1-bromo-2-methylbenzene. MS (ESI) *m*/*z* 435 (M+H)⁺.

### Example 254

### trans-4-(2-chlorophenyl)-N-(4-chlorophenyl)-1-[(1-methyl-1H-imidazol-4-yl)sulfonyl]pyrrolidin-3-amine

The title compound was prepared similarly to the procedure described in Example 57 substituting Example 253A for Example 14A and substituting 1-bromo-4-chlorobenzene for 1-bromo-2-methylbenzene. MS (ESI) *m*/*z* 451 (M+H)⁺.

### Example 255

### trans-4-(2-chlorophenyl)-1-[(1-methyl-1H-imidazol-4-yl)sulfonyl]-N-[3-(trifluoromethyl)phenyl]pyrrolidin-3 -amine

The title compound was prepared similarly to the procedure described in Example 57 substituting Example 253A for Example 14A and substituting 1-bromo-3-(trifluoromethyl)benzene for 1-bromo-2-methylbenzene. MS (ESI) *m*/*z* 485 (M+H)⁺.

### Example 256

### trans-4-(2-chlorophenyl)-1-[(1-methyl-1H-imidazol-4-yl)sulfonyl]-N-[4-(trifluoromethyl)phenyl]pyrrolidin-3 -amine

The title compound was prepared similarly to the procedure described in Example 57 substituting Example 253A for Example 14A and substituting 1-bromo-4-(trifluoromethyl)benzene for 1-bromo-2-methylbenzene. MS (ESI) *m*/*z* 485 (M+H)⁺.

### Example 257

### trans-N-(3-chloro-4-fluorophenyl)-4-(2-chlorophenyl)-1-[(1-methyl-1H-imidazol-4-yl)sulfonyl]pyrrolidin-3 -amine

The title compound was prepared similarly to the procedure described in Example 57 substituting Example 253A for Example 14A and substituting 1-bromo-3-chloro-4-fluorobenzene for 1-bromo-2-methylbenzene. MS (ESI) *m*/*z* 469 (M+H)⁺.

### Example 258

### trans-4-(2-chlorophenyl)-N-[4-fluoro-3-(trifluoromethyl)phenyl]-1-[(1-methyl-1H-imidazol-4-yl)sulfonyl]pyrrolidin-3 -amine

The title compound was prepared similarly to the procedure described in Example 57 substituting Example 253A for Example 14A and substituting 1-bromo-4-fluoro-3-(trifluoromethyl)benzene for 1-bromo-2-methylbenzene. MS (ESI) *m*/*z* 503 (M+H)⁺.

### Example 259

### N-{trans-4-(2-chlorophenyl)-1-[(1-methyl-1H-imidazol-4-yl)sulfonyl]pyrrolidin-3-yl}-2-(trifluoromethyl)pyridin-4-amine

The title compound was prepared similarly to the procedure described in Example 57 substituting Example 253A for Example 14A and substituting 4-bromo-2-(trifluoromethyl)pyridine for 1-bromo-2-methylbenzene. MS (ESI) *m*/*z* 486 (M+H)⁺.

### Example 260

### trans-N-(2-chlorobenzyl)-4-(4-fluorophenyl)-1-[(1-methyl-1H-imidazol-4-yl)sulfonyl]pyrrolidin-3-amine

The title compound was prepared similarly to the procedure described in Example 148 substituting 2-chlorobenzaldehyde for benzaldehyde. MS (ESI) *m*/*z* 449 (M+H)⁺.

### Example 261

### trans-N-(3-chlorobenzyl)-4-(4-fluorophenyl)-1-[(1-methyl-1H-imidazol-4-yl)sulfonyl]pyrrolidin-3-amine

The title compound was prepared similarly to the procedure described in Example 148 substituting 3-chlorobenzaldehyde for benzaldehyde. MS (ESI) *m*/*z* 449 (M+H)⁺.

### Example 262

### trans-N-(4-chlorobenzyl)-4-(4-fluorophenyl)-1-[(1-methyl-1H-imidazol-4-yl)sulfonyl]pyrrolidin-3-amine

The title compound was prepared similarly to the procedure described in Example 148 substituting 4-chlorobenzaldehyde for benzaldehyde. MS (ESI) *m*/*z* 449 (M+H)⁺.

### Example 263

### trans-N-(2-fluorobenzyl)-4-(4-fluorophenyl)-1-[(1-methyl-1H-imidazol-4-yl)sulfonyl]pyrrolidin-3-amine

The title compound was prepared similarly to the procedure described in Example 148 substituting 2-fluorobenzaldehyde for benzaldehyde. MS (ESI) *m*/*z* 433 (M+H)⁺.

### Example 264

### trans-N-(3-fluorobenzyl)-4-(4-fluorophenyl)-1-[(1-methyl-1H-imidazol-4-yl)sulfonyl]pyrrolidin-3-amine

The title compound was prepared similarly to the procedure described in Example 148 substituting 3-fluorobenzaldehyde for benzaldehyde. MS (ESI) *m*/*z* 433 (M+H)⁺.

### Example 265

### trans-4-(4-fluorophenyl)-1-[(1-methyl-1H-imidazol-4-yl)sulfonyl]-N-[2-(trifluoromethyl)benzyl]pyrrolidin-3 -amine

The title compound was prepared similarly to the procedure described in Example 148 substituting 2-(trifluoromethyl)benzaldehyde for benzaldehyde. MS (ESI) *m*/*z* 483 (M+H)⁺.

### Example 266

### trans-4-(4-fluorophenyl)-1-[(1-methyl-1H-imidazol-4-yl)sulfonyl]-N-[3-(trifluoromethyl)benzyl]pyrrolidin-3 -amine

The title compound was prepared similarly to the procedure described in Example 148 substituting 3-(trifluoromethyl)benzaldehyde for benzaldehyde. MS (ESI) *m*/*z* 483 (M+H)⁺.

### Example 267

### trans-4-(4-fluorophenyl)-1-[(1-methyl-1H-imidazol-4-yl)sulfonyl]-N-[4-(trifluoromethyl)benzyl]pyrrolidin-3-amine

The title compound was prepared similarly to the procedure described in Example 148 substituting 4-(trifluoromethyl)benzaldehyde for benzaldehyde. MS (ESI) *m*/*z* 483 (M+H)⁺.

### Example 268

### trans-4-(4-fluorophenyl)-1-[(1-methyl-1H-imidazol-4-yl)sulfonyl]-N-(pyridin-2-ylmethyl)pyrrolidin-3 -amine

The title compound was prepared similarly to the procedure described in Example 148 substituting picolinaldehyde for benzaldehyde. MS (ESI) *m*/*z* 416 (M+H)⁺.

### Example 269

### trans-4-(4-fluorophenyl)-1-[(1-methyl-1H-imidazol-4-yl)sulfonyl]-N-(pyridin-3-ylmethyl)pyrrolidin-3 -amine

The title compound was prepared similarly to the procedure described in Example 148 substituting nicotinaldehyde for benzaldehyde. MS (ESI) *m*/*z* 416 (M+H)⁺.

### Example 270

### trans-4-(4-fluorophenyl)-1-[(1-methyl-1H-imidazol-4-yl)sulfonyl]-N-(pyridin-4-ylmethyl)pyrrolidin-3 -amine

The title compound was prepared similarly to the procedure described in Example 148 substituting isonicotinaldehyde for benzaldehyde. MS (ESI) *m*/*z* 416 (M+H)⁺.

### Example 271

### {trans-4-(4-fluorophenyl)-1-[(1-methyl-1H-imidazol-4-yl)sulfonyl]pyrrolidin-3-yl}(phenyl)methanone

### Example 271A

### Trans-4-(4-fluorophenyl)-N-methoxy-N-methyl-1-(1-methyl-1H-imidazol-4-ylsulfonyl)pyrrolidine-3-carboxamide

To a solution of Example 242D (610 mg, 1.73 mmol) in dimethylformamide (5 mL) was added triethylamine (0.52 mL, 3.71 mmol), N,O-dimethylhydroxylamine hydrochloride (236 mg. 2.42 mmol), and 2-(1H-benzo[d][1,2,3]triazol-1-yl)-1,1,3,3-tetramethylisouronium tetrafluoroborate (665 mg, 2.07 mmol). The mixture was stirred at room temperature for 4 hours.. The reaction mixture was partitioned between water and ethyl acetate. The organic fraction was collected. The aqueous fraction was washed with ethyl acetate 3 more times. The combined organic fractions were dried over sodium sulfate, concentrated, and purified by flash-chromatography on silica gel (5-10% methanol in dichloromethane (with 0.5% volume triethylamine added)) to afford the title compound. MS (ESI) *m*/*z* 397.0 (M+H)⁺.

### Example 271B

### {Trans-4-(4-fluorophenyl)-1-[(1-methyl-1H-imidazol-4-yl)sulfonyl]pyrrolidin-3-yl} (phenyl)methanone

To a solution of Example 271A (113 mg, 0.29 mmol) in tetrahydrofuran (0.5 mL) was added phenylmagnesium bromide (0.57 mL, 0.57 mmol, 1.0 M in tetrahydrofuran) slowly at room temperature. The solution stirred for 1 hour. The reaction mixture was partitioned with saturated ammonium chloride (aq.) and the organic fraction was collected. The organic fraction was dried over sodium sulfate, concentrated, and purified by HPLC to afford the title compound. MS (ESI) *m*/*z* 414.1 (M+H)⁺.

### Example 272

### Trans-N- [2-chloro-3 -(trifluoromethyl)phenyl] -4-(4-fluorophenyl)-1 - [(1 -methyl-1H-imidazol-4-yl)sulfonyl]pyrrolidine-3-carboxamide

The title compound was prepared using the procedure described in Example 242E substituting 2-chloro-3-(trifluoromethyl)aniline for aniline. MS (ESI) *m*/*z* 531.1 (M+H)⁺.

### Example 273

### Trans-4-(4-fluorophenyl)-1-[(1-methyl-1H-imidazol-4-yl)sulfonyl]-N-[3-(trifluoromethoxy)phenyl]pyrrolidine-3-carboxamide

The title compound was prepared using the procedure described in Example 242E substituting 3-(trifluoromethoxy)aniline for aniline. MS (ESI) *m*/*z* 513.1 (M+H)⁺.

### Example 274

### trans-4-(4-fluorophenyl)-N-[4-fluoro-3-(trifluoromethyl)phenyl]-1-[(1-methyl-1H-imidazol-4-yl)sulfonyl]pyrrolidine-3-carboxamide

The title compound was prepared similarly to the procedure described in Example 242E substituting 4-fluoro-3-(trifluoromethyl)aniline for aniline. MS (ESI) *m*/*z* 515.1 (M+H)⁺.

### Example 275

### trans-N-(3-chloro-4-fluorophenyl)-4-(4-fluorophenyl)-1-[(1-methyl-1H-imidazol-4-yl)sulfonyl]pyrrolidine-3-carboxamide

The title compound was prepared using the procedure described in Example 242E substituting 3-chloro-4-fluoroaniline for aniline. MS (ESI) *m*/*z* 481.1 (M+H)⁺.

### Example 276

### trans-N-[3-chloro-4-(trifluoromethyl)phenyl]-4-(4-fluorophenyl)-1-[(1-methyl-1H-imidazol-4-yl)sulfonyl]pyrrolidine-3-carboxamide

The title compound was prepared using the procedure described in Example 242E substituting 3-chloro-4-(trifluoromethyl)aniline for aniline. MS (ESI) *m*/*z* 531.1 (M+H)⁺.

### Example 277

### 1-methyl-4-[(3-phenoxy-4-phenylpyrrolidin-1-yl)sulfonyl]-1H-imidazole

To a stirred solution of tert-butyl 3-hydroxy-4-phenylpyrrolidine-1-carboxylate (200 mg, 0.76 mmol) in tetrahydrofuran (5 mL) was added (Z)-di-tert-butyl diazene-1,2-dicarboxylate (262 mg, 1.1 mmol), phenol (72 mg, 0.76 mmol), and polystyrene triphenylphosphine resin (2.37 mmol/g, 961 mg, 2.3 mmol). The reaction mixture was stirred at room temperature for 18 hours before the resin was filtered off. The filtrate was concentrated. The concentrate was dissolved in dichloromethane (2mL) and HCl (4M in dioxane, 2 mL, 8 mmol) was added. When LCMS showed that the reaction was complete the reaction mixture was concentrated. To a stirred solution of the concentrate in pyridine (4 mL) was added 1-methyl-1H-imidazole-4-sulfonyl chloride (165 mg, 0.91 mmol). The reaction mixture was warmed to 60 °C for 18 hours. The reaction was then concentrated. Purification via HPLC provided the title compound.

### Example 278

### trans-4-(2-chlorophenyl)-N-(3-chlorophenyl)-1-[(1-methyl-1H-imidazol-4-yl)sulfonyl]pyrrolidin-3-amine

The title compound was prepared similarly to the procedure described in Example 57 substituting Example 252A for Example 14A and substituting 1-bromo-3-chlorobenzene for 1-bromo-2-methylbenzene. MS (ESI) *m*/*z* 451 (M+H)⁺.

### Example 279

### trans-N-(2,4-dichlorobenzyl)-4-(4-fluorophenyl)-1-[(1-methyl-1H-imidazol-4-yl)sulfonyl]pyrrolidin-3 -amine

The title compound was prepared similarly to the procedure described in Example 148 substituting 2,4-dichlorobenzaldehyde for benzaldehyde. MS (ESI) *m*/*z* 483 (M+H)⁺.

### Example 280

### trans-N-(2,4-dichlorobenzyl)-1-[(1-methyl-1H-imidazol-4-yl)sulfonyl]-4-phenylpyrrolidin-3-amine

The title compound was prepared similarly to the procedure described in Example 148 substituting Example 14A for Example 3A and substituting 2,4-dichlorobenzaldehyde for benzaldehyde. MS (ESI) *m*/*z* 465 (M+H)⁺.

### Example 281

### trans-N-[2-chloro-4-(trifluoromethyl)benzyl]-1-[(1-methyl-1H-imidazol-4-yl)sulfonyl]-4-phenylpyrrolidin-3 -amine

The title compound was prepared similarly to the procedure described in Example 148 substituting Example 14A for Example 3A and substituting 2-chloro-4-(trifluoromethyl)benzaldehyde for benzaldehyde. MS (ESI) *m*/*z* 499 (M+H)⁺.

### Example 282

trans-N-(2,4-dichlorobenzyl)-4-(2-fluorophenyl)-1-[(1-methyl-1H-imidazol-4-yl)sulfonyl]pyrrolidin-3 -amine

### Example 282A

### trans-4-(2-fluorophenyl)-1-(1-methyl-1H-imidazol-4-ylsulfonyl)pyrrolidin-3 -amine

The title compound was prepared as the HCl salt similarly to the procedure described in Example 2A-2F substituting (E)-1-fluoro-2-(2-nitrovinyl)benzene for (E)-1-methoxy-4-(2-nitrovinyl)benzene.

### Example 282B

### trans-N-(2,4-dichlorobenzyl)-4-(2-fluorophenyl)-1-[(1-methyl-1H-imidazol-4-yl)sulfonyl]pyrrolidin-3 -amine

The title compound was prepared similarly to the procedure described in Example 148 substituting Example 282A for Example 3A and substituting 2,4-dichlorobenzaldehyde for benzaldehyde. MS (ESI) *m*/*z* 483 (M+H)⁺.

### Example 283

### trans-N- [2-chloro-4-(trifluoromethyl)benzyl] -4-(2-fluorophenyl)-1-[(1 -methyl-1H-imidazol-4-yl)sulfonyl]pyrrolidin-3 -amine

The title compound was prepared similarly to the procedure described in Example 148 substituting Example 282A for Example 3A and substituting 2-chloro-4-(trifluoromethyl)benzaldehyde for benzaldehyde. MS (ESI) *m*/*z* 517 (M+H)⁺.

### Example 284

### trans-4-(2-chlorophenyl)-N-(2,4-dichlorobenzyl)-1-[(1-methyl-1H-imidazol-4-yl)sulfonyl]pyrrolidin-3 -amine

The title compound was prepared similarly to the procedure described in Example 148 substituting Example 252A for Example 3A and substituting 2,4-dichlorobenzaldehyde for benzaldehyde. MS (ESI) *m*/*z* 501 (M+H)⁺.

### Example 285

### trans-4-(2-chlorophenyl)-N-[2-chloro-4-(trifluoromethyl)benzyl]-1-[(1-methyl-1H-imidazol-4-yl)sulfonyl]pyrrolidin-3 -amine

The title compound was prepared similarly to the procedure described in Example 148 substituting Example 252A for Example 3A and substituting 2-chloro-4-(trifluoromethyl)benzaldehyde for benzaldehyde. MS (ESI) *m*/*z* 533 (M+H)⁺.

### Example 286

### trans-N-(2,4-dichlorobenzyl)-1-[(1-methyl-1H-imidazol-4-yl)sulfonyl]-4-(pyridin-3-yl)pyrrolidin-3-amine

### Example 286A

### trans-1-(1-methyl-1H-imidazol-4-ylsulfonyl)-4-(pyridin-3-yl)pyrrolidin-3-amine

The title compound was prepared as an HCl salt similarly to the conditions described in Example 2A -2F substituting (E)-3-(2-nitrovinyl)pyridine for (E)-1-methoxy-4-(2-nitrovinyl)benzene.

### Example 286B

### trans-N-(2,4-dichlorobenzyl)-1-[(1-methyl-1H-imidazol-4-yl)sulfonyl]-4-(pyridin-3-yl)pyrrolidin-3-amine

The title compound was prepared similarly to the procedure described in Example 148 substituting 286A for Example 3A and substituting 2,4-dichlorobenzaldehyde for benzaldehyde. MS (ESI) *m*/*z* 466 (M+H)⁺.

### Example 287

### N-{trans-4-(4-fluorophenyl)-1-[(1-methyl-1H-imidazol-4-yl)sulfonyl]pyrrolidin-3-yl}-4-(trifluoromethyl)pyridin-2-amine

The title compound was prepared similarly to the procedure described in Example 57 substituting Example 3A for Example 14A and substituting 2-bromo-4-(trifluoromethyl)pyridine for 1-bromo-2-methylbenzene. MS (ESI) *m*/*z* 470 (M+H)⁺.

### Example 288

### N-{trans-4-(4-fluorophenyl)-1-[(1-methyl-1H-imidazol-4-yl)sulfonyl]pyrrolidin-3-yl}-2-(trifluoromethyl)pyridin-4-amine

The title compound was prepared similarly to the procedure described in Example 57 substituting Example 3A for Example 14A and substituting 4-bromo-2-(trifluoromethyl)pyridine for 1-bromo-2-methylbenzene. MS (ESI) *m*/*z* 470 (M+H)⁺.

### Example 289

### Trans-N-benzyl-4-(4-fluorophenyl)-1-[(1-methyl-1H-imidazol-4-yl)sulfonyl]pyrrolidine-3-carboxamide

The title compound was prepared using the procedure described in Example 242E substituting phenylmethanamine for aniline. MS (ESI) *m*/*z* 443.1 (M+H)⁺.

### Example 290

### N-({trans-4-(4-fluorophenyl)-1-[(1-methyl-1H-imidazol-4-yl)sulfonyl]pyrrolidin-3-yl}methyl)aniline

To a solution of Example 242E (106.7 mg, 0.25 mmol) in dry tetrahydrofuran (0.7 mL) under argon, was added borane dimethyl sulfide complex (0.5 mL, 1.0 mmol, 2M in tetrahydrofuran). The reaction mixture was stirred at 60 °C for 5 hours and stirred at room temperature for 18 hours. Hydrochloric acid (0.5 mL of 0.5 N HCl) was carefully added. The reaction mixture was refluxed for 2 hours, then treated with sodium hydroxide (1N) to pH=8-9. The reaction mixture was portioned with ethyl acetate. The organic fraction was collected. The aqueous fraction was washed with dichloromethane. The organic fractions were combined, dried over sodium sulfate, concentrated, and purified by HPLC to afford the title compound. MS (ESI) *m*/*z* 415.1 (M+H)⁺.

### Example 291

### N-({trans-4-(4-fluorophenyl)-1-[(1-methyl-1H-imidazol-4-yl)sulfonyl]pyrrolidin-3-yl}methyl)-4-(trifluoromethyl)aniline

The title compound was prepared using the procedure described in Example 290 substituting Example 243 for Example 242E. MS (ESI) *m*/*z* 483.1 (M+H)⁺.

### Example 292

### 3,5-dichloro-N-({trans-4-(4-fluorophenyl)-1-[(1-methyl-1H-imidazol-4-yl)sulfonyl]pyrrolidin-3-yl} methyl)aniline

The title compound was prepared using the procedure described in Example 290 substituting Example 244 for Example 242E. MS (ESI) *m*/*z* 483.1 (M+H)⁺.

### Example 293

### 4-{[trans-3-(4-fluorophenyl)-4-{[3-(trifluoromethyl)phenoxy]methyl}pyrrolidin-1-yl]sulfonyl}-1-methyl-1H-imidazole

The title compound was prepared similarly to the procedure described in Example 245 substituting 3-(trifluoromethyl)phenol for phenol. MS (ESI) *m*/*z* 484.1 (M+H)⁺

### Example 294

### 4-{[trans-3-{[2-chloro-4-(trifluoromethyl)phenoxy]methyl}-4-(4-fluorophenyl)pyrrolidin-1-yl]sulfonyl}-1-methyl-1H-imidazole

The title compound was prepared similarly to the procedure described in Example 245 substituting 3-chloro-4-hydroxybenzotrifluoride for phenol. MS (ESI) *m*/*z* 518.1 (M+H)⁺

### Example 295

### 4-{[trans-3-(4-fluorophenyl)-4-{[4-fluoro-3-(trifluoromethyl)phenoxy]methyl}pyrrolidin-1-yl]sulfonyl}-1-methyl-1H-imidazole

The title compound was prepared similarly to the procedure described in Example 245 substituting 4-fluoro-3-(trifluoromethyl)phenol for phenol. MS (ESI) *m*/*z* 502.1 (M+H)⁺.

### Example 296

### 4-({trans-3-(4-fluorophenyl)-4-[(3-methylphenoxy)methyl]pyrrolidin-1-yl}sulfonyl)-1-methyl-1H-imidazole

The title compound was prepared similarly to the procedure described in Example 245 substituting m-cresol for phenol. MS (ESI) *m*/*z* 430.1 (M+H)⁺.

### Example 297

### (3-chlorophenyl){trans-4-(4-fluorophenyl)-1-[(1-methyl-1H-imidazol-4-yl)sulfonyl]pyrrolidin-3-yl}methanone

The title compound was prepared similarly to the procedure described in Example 271B substituting (3-chlorophenyl)magnesium bromide for phenylmagnesium bromide. MS (ESI) *m*/*z* 448.1 (M+H)⁺.

### Example 298

### trans-N- [4-fluoro-3 -(trifluoromethoxy)phenyl]-1-[(1-methyl-1H-imidazol-4-yl)sulfonyl] -4-phenylpyrrolidin-3 -amine

The title compound was prepared similarly to the conditions described in Example 57 substituting 1-bromo-4-fluoro-3-(trifluoromethoxy)benzene for 1-bromo-2-methylbenzene. MS (ESI) *m*/*z* 485 (M+H)⁺.

### Example 299

### trans-N-[4-chloro-3-(trifluoromethoxy)phenyl]-1-[(1-methyl-1H-imidazol-4-yl)sulfonyl]-4-phenylpyrrolidin-3 -amine

The title compound was prepared similarly to the conditions described in Example 57 substituting 1-bromo-4-chloro-3-(trifluoromethoxy)benzene for 1-bromo-2-methylbenzene. MS (ESI) *m*/*z* 501 (M+H)⁺.

### Example 300

### {trans-4-(4-fluorophenyl)-1-[(1-methyl-1H-imidazol-4-yl)sulfonyl]pyrrolidin-3-yl}(3-methylphenyl)methanone

The title compound was prepared similarly to the procedure described in Example 271B substituting m-tolylmagnesium bromide for phenylmagnesium bromide. MS (ESI) *m*/*z* 428.0 (M+H)⁺.

### Example 301

### (3-fluorophenyl){(trans-4-(4-fluorophenyl)-1-[(1-methyl-1H-imidazol-4-yl)sulfonyl]pyrrolidin-3-yl}methanone

The title compound was prepared similarly to the procedure described in Example 271B substituting (3-fluorophenyl)magnesium bromide for phenylmagnesium bromide. MS (ESI) *m*/*z* 432.1 (M+H)⁺.

### Example 302

### {trans-4-(4-fluorophenyl)-1-[(1-methyl-1H-imidazol-4-yl)sulfonyl]pyrrolidin-3-yl}[3-(trifluoromethyl)phenyl]methanone

The title compound was prepared similarly to the procedure described in Example 271B substituting (3-(trifluoromethyl)phenyl)magnesium bromide for phenylmagnesium bromide. MS (ESI) *m*/*z* 482.1 (M+H)⁺.

### Example 303

### (4-fluorophenyl){trans-4-(4-fluorophenyl)-1-[(1-methyl-1H-imidazol-4-yl)sulfonyl]pyrrolidin-3-yl}methanone

The title compound was prepared similarly to the procedure described in Example 271B substituting (4-fluorophenyl) magnesium bromide for phenylmagnesium bromide. MS (ESI) *m*/*z* 432.1 (M+H)⁺.

### Example 304

### trans-N-(3-chlorophenyl)-4-(4-fluorophenyl)-1-[(1-methyl-1H-imidazol-4-yl)sulfonyl]pyrrolidine-3-carboxamide

The title compound was prepared similarly to the procedure described in Example 242E substituting 3-chloro aniline for aniline. MS (ESI) *m*/*z* 463.0 (M+H)⁺.

### Example 305

### trans-4-(4-fluorophenyl)-N-[4-fluoro-3-(trifluoromethoxy)phenyl]-1-[(1-methyl-1H-imidazol-4-yl)sulfonyl]pyrrolidin-3 -amine

The title compound was prepared similarly to the conditions described in Example 57 substituting Example 3A For Example 14A and substituting 1-bromo-4-fluoro-3-(trifluoromethoxy)benzene for 1-bromo-2-methylbenzene. MS (ESI) *m*/*z* 503 (M+H)⁺.

### Example 306

### trans-N-[4-chloro-3-(trifluoromethoxy)phenyl]-4-(4-fluorophenyl)-1-[(1-methyl-1H-imidazol-4-yl)sulfonyl]pyrrolidin-3 -amine

The title compound was prepared similarly to the conditions described in Example 57 substituting Example 3A For Example 14A and substituting 1-bromo-4-chloro-3-(trifluoromethoxy)benzene for 1-bromo-2-methylbenzene. MS (ESI) *m*/*z* 519 (M+H)⁺.

### Example 307

### trans-N-(2,4-dichlorobenzyl)-1-[(1-methyl-1H-imidazol-4-yl)sulfonyl]-4-(pyridin-2-yl)pyrrolidin-3-amine

### Example 307A

### (E)-2-(2-nitrovinyl)pyridine

To a stirred solution of nitromethane (15.2 mL, 280 mmol) and 2-pyridinecarboxaldeyde (8.9 mL, 93 mmol) in toluene (100 mL) at room temperature was added 1,1,2,2,-tetramethylguanidine (1.2 mL, 9.3 mmol) followed by methanesulfonyl chloride (14.5 mL, 187 mmol). The reaction mixture was stirred for 5 minutes before triethylamine (26.1 mL, 187 mmol) was added. The reaction mixture was stirred for 30 minutes, then quenched with sodium bicarbonate (aq.) The organic fraction was collected. The aqueous fraction was washed with dichloromethane. All organic fractions were combined. Purification via flash chromatography provided the title compound.

### Example 307B

### trans-1-(1-methyl-1H-imidazol-4-ylsulfonyl)-4-(pyridin-2-yl)pyrrolidin-3-amine

The title compound was prepared as an HCl salt similarly to the procedures described in Example 2A-2Fsubstituting Example 307A for (E)-1-methoxy-4-(2-nitrovinyl)benzene.

### Example 307C

### trans-N-(2,4-dichlorobenzyl)-1-[(1-methyl-1H-imidazol-4-yl)sulfonyl]-4-(pyridin-2-yl)pyrrolidin-3-amine

The title compound was prepared similarly to the procedure described in Example 148 substituting Example 307B for Example 3A and substituting 2,4-dichlorobenzaldehyde for benzaldehyde. MS (ESI) *m*/*z* 466 (M+H)⁺.

### Example 308

### trans-1-[(1-methyl-1H-imidazol-4-yl)sulfonyl]-4-(pyridin-3-yl)-N-[3-(trifluoromethyl)phenyl]pyrrolidin-3 -amine

The title compound was prepared similarly to the procedure described in Example 57 substituting Example 286A for Example 14A and substituting 1-bromo-3-(trifluoromethyl)benzene for 1-bromo-2-methylbenzene. MS (ESI) *m*/*z* 452 (M+H)⁺.

### Example 309

### 4-{[3-(3-chlorobenzyl)-4-cyclopropylpyrrolidin-1-yl]sulfonyl}-1-methyl-1H-imidazole

### Example 309A

### 2-(3-chlorobenzyl)-3-cyclopropyl-4-nitrobutanal

The title compound was prepared using the conditions described in Example 182A substituting (E)-(2-nitrovinyl)cyclopropane for (E)-1-fluoro-4-(2-nitrovinyl)benzene.

### Example 309B

### 3-(3-chlorobenzyl)-4-cyclopropylpyrrolidine

The title compound was prepared using the conditions described in Example 182B substituting Example 309A for Example 182A.

### Example 309

### 4-{[3-(3-chlorobenzyl)-4-cyclopropylpyrrolidin-1-yl]sulfonyl}-1-methyl-1H-imidazole

The title compound was prepared using the conditions described in Example 182C substituting Example 309B for Example 182B. MS (ESI) *m*/*z* 380/382(3:1) (M+H)⁺.

### Example 310

### N-[3-(difluoromethyl)-4-fluorophenyl]-4-(4-fluorophenyl)-1-[(1-methyl-1H-imidazol-4-yl)sulfonyl]pyrrolidin-3 -amine

To a 10 mL microwave vial was added 4-(4-fluorophenyl)-1-(1-methyl-1H-imidazol-4-ylsulfonyl)pyrrolidin-3-amine hydrochloride (100 mg, 0.277 mmol) and premixed tris(dibenzylideneacetone)dipalladium(0)/sodium tert-butoxide/ 2,2'-bis(diphenylphosphino)-1,1'-binaphthyl (100 mg, 0.05:0.15:2), aldrich 715530). The solids were suspended in 1,2-dimethoxyethane, and 4-bromo-2-(difluoromethyl)-1-fluorobenzene (74.8 mg, 0.333 mmol) was added. The reaction vessel was capped and the reaction mixture was heated in a microwave (Biotage Initiator™, maximum 400 Watts) for 15 min at 130°C. The mixture was filtered through celite, concentrated, dissolved in 2 ml 50% methanol/dimethylsulfoxide, and purified by HPLC to provide the title compound. MS (ESI) *m*/*z* 469 (M+H)⁺.

### Example 311

### 3-chloro-N-({trans-4-(4-fluorophenyl)-1-[(1-methyl-1H-imidazol-4-yl)sulfonyl]pyrrolidin-3-yl}methyl)-4-(trifluoromethyl)aniline

The title compound was prepared similarly to the procedure described in Example 290 substituting Example 276 for Example 242E. MS (ESI) *m*/*z* 517.0 (M+H)⁺.

### Example 312

### N-({trans-4-(4-fluorophenyl)-1-[(1-methyl-1H-imidazol-4-yl)sulfonyl]pyrrolidin-3-yl}methyl)-3-(trifluoromethoxy)aniline

The title compound was prepared similarly to the procedure described in Example 290 substituting Example 273 for Example 242E. MS (ESI) *m*/*z* 499 (M+H)⁺.

### Example 313

### 4-fluoro-N-({trans-4-(4-fluorophenyl)-1-[(1-methyl-1H-imidazol-4-yl)sulfonyl]pyrrolidin-3-yl}methyl)-3 -(trifluoromethyl)aniline

The title compound was prepared similarly to the procedure described in Example 290 substituting Example 274 for Example 242E. MS (ESI) *m*/*z* 501.1 (M+H)⁺.

### Example 314

### 3-chloro-4-fluoro-N-({trans-4-(4-fluorophenyl)-1-[(1-methyl-1H-imidazol-4-yl)sulfonyl]pyrrolidin-3-yl} methyl)aniline

The title compound was prepared similarly to the procedure described in Example 290 substituting Example 275 for Example 242E. MS (ESI) *m*/*z* 467.1 (M+H)⁺.

### Example 315

### N-benzyl-1-{trans-4-(4-fluorophenyl)-1-[(1-methyl-1H-imidazol-4-yl)sulfonyl]pyrrolidin-3-yl}methanamine

The title compound was prepared similarly to the procedure described in Example 290 substituting Example 289 for Example 242E. MS (ESI) *m*/*z* 429.1 (M+H)⁺.

### Example 316

### trans-4-(4-fluorophenyl)-1-[(1-methyl-1H-imidazol-4-yl)sulfonyl]-N-(3-methylphenyl)pyrrolidine-3-carboxamide

The title compound was prepared similarly to the procedure described in Example 242E substituting m-toluidine for aniline. MS (ESI) *m*/*z* 443.0 (M+H)⁺

### Example 317

### Trans-4-(4-fluorophenyl)-1-[(1-methyl-1H-imidazol-4-yl)sulfonyl]-N-[3-(trifluoromethyl)phenyl]pyrrolidine-3-carboxamide

The title compound was prepared using the procedure described in Example 242E substituting 3-(trifluoromethyl)aniline for aniline. MS (ESI) *m*/*z* 497.0 (M+H)⁺.

### Example 318

### 4-{[Trans-3-[(3-chloro-4-fluorophenoxy)methyl]-4-(4-fluorophenyl)pyrrolidin-1-yl]sulfonyl}-1-methyl-1H-imidazole

The title compound was prepared similarly to the procedure described in Example 245 substituting 3-chloro-4-fluorophenol for phenol. MS (ESI) *m*/*z* 468.1 (M+H)⁺.

### Example 319

### (4-Chloro-3 -fluorophenyl) {trans-4-(4-fluorophenyl)-1-[(1-methyl-1H-imidazol-4-yl)sulfonyl]pyrrolidin-3 -yl} methanone

The title compound was prepared similarly to the procedure described in Example 271B substituting (4-chloro-3-fluorophenyl) magnesium bromide for phenylmagnesium bromide. MS (ESI) *m*/*z* 466.1 (M+H)⁺.

### Example 320

### trans-N-[2-chloro-4-(trifluoromethyl)benzyl]-1-[(1-methyl-1H-imidazol-4-yl)sulfonyl]-4-(pyridin-2-yl)pyrrolidin-3-amine

The title compound was prepared similarly to the procedure described in Example 148 substituting Example 286A for Example 3A and substituting 2-chloro-4-(trifluoromethyl)-benzaldehyde for benzaldehyde. MS (ESI) *m*/*z* 500 (M+H)⁺.

### Example 321

### (3S,4R)-N-[2-chloro-4-(trifluoromethyl)benzyl]-4-(4-fluorophenyl)-1-[(1-methyl-1H-imidazol-4-yl)sulfonyl]pyrrolidin-3 -amine

### Example 321A

### tert-butyl (3S,4R)-4-(4-fluorophenyl)-1-(1-methyl-1H-imidazol-4-ylsulfonyl)pyrrolidin-3-ylcarbamate

The title compound was prepared similarly to the conditions described in Example 2A-2E substituting (E)-1-fluoro-4-(2-nitrovinyl)benzene for (E)-1-methoxy-4-(2-nitrovinyl)benzene. A Chiral SFC separation provided the title compound. (Instrument: SFC200 Column: AD-H, 50 ×250 mm, 5 µm; Column Temperature: 35 °C; Mobile Phase: CO₂/methanol/diethylamine =80/20/0.1; Flow rate: 180 g/min; Back Pressure: 100 Bar; Wavelength: 214 nm; Cycle time: 5.1 min; Injection: 2.0 mL Sample solution: 55 g in 500 mL methanol). Retention Time = 4.03 minutes.

### Example 321B

### (3S,4R)-4-(4-fluorophenyl)-1-(1-methyl-1H-imidazol-4-ylsulfonyl)pyrrolidin-3-amine

The title compound was prepared as an HCl salt similarly to the procedure described in Example 2F substituting Example 321A for Example 2E.

### Example 321C

### (3S,4R)-N-[2-chloro-4-(trifluoromethyl)benzyl]-4-(4-fluorophenyl)-1-[(1-methyl-1H-imidazol-4-yl)sulfonyl]pyrrolidin-3 -amine

The title compound was prepared similarly to the procedure described in Example 148 substituting Example 321B for Example 3A and substituting 2-chloro-4-(trifluoromethyl)benzaldehyde for benzaldehyde. MS (ESI) *m*/*z* 517 (M+H)⁺.

### Example 322

### (3R,4S)-N-[2-chloro-4-(trifluoromethyl)benzyl]-4-(4-fluorophenyl)-1-[(1-methyl-1H-imidazol-4-yl)sulfonyl]pyrrolidin-3 -amine

### Example 322A

### tert-butyl (3R,4S)-4-(4-fluorophenyl)-1-(1-methyl-1H-imidazol-4-ylsulfonyl)pyrrolidin-3-ylcarbamate

The title compound was prepared similarly to Example 321A. RT = 5.73 minutes.

### Example 322B

### (3R,4S)-4-(4-fluorophenyl)-1-(1-methyl-1H-imidazol-4-ylsulfonyl)pyrrolidin-3-amine

The title compound was prepared as an HCl salt similarly to the procedure described in Example 2F substituting Example 322A for Example 2E.

### Example 322C

### (3R,4S)-N-[2-chloro-4-(trifluoromethyl)benzyl]-4-(4-fluorophenyl)-1-[(1-methyl-1H-imidazol-4-yl)sulfonyl]pyrrolidin-3 -amine

The title compound was prepared similarly to the procedure described in Example 148 substituting Example 322B for Example 3A and substituting 2-chloro-4-(trifluoromethyl)benzaldehyde for benzaldehyde. MS (ESI) *m*/*z* 517 (M+H)⁺.

### Example 323

### (3S,4R)-4-(4-fluorophenyl)-1-[(1-methyl-1H-imidazol-4-yl)sulfonyl]-N-[3-(trifluoromethoxy)phenyl]pyrrolidin-3-amine

Under nitrogen, a pressure vial was charged with Example 321B (100 mg, 0.27 mmol), 1-bromo-3-(trifluoromethoxy)benzene (67 mg, 0.27 mmol), 2'-(di-tert-butylphosphino)-N,N-dimethylbiphenyl-2-amine (9.5 mg, 0.03 mmol), tris(dibenzylidene-acetone)dipalladium(0) (13 mg, 0.014 mmol), sodium tert-butoxide (107 mg, 1.1 mmol), and dioxane (4 mL). The reaction mixture was stirred at 80 °C for 2 hours. Then, the reaction mixture was concentrated. Purification via HPLC provided the title compound. MS (ESI) *m*/*z* 485 (M+H)⁺.

### Example 324

### (3S,4R)-N,4-bis(4-fluorophenyl)-1-[(1-methyl-1H-imidazol-4-yl)sulfonyl]pyrrolidin-3-amine

The title compound was prepared similarly to the procedure described in Example 323 substituting 1-bromo-4-fluorobenzene for bromo-3-(trifluoromethoxy)benzene. MS (ESI) *m*/*z* 419 (M+H)⁺.

### Example 325

### (3S,4R)-N-(3-chlorophenyl)-4-(4-fluorophenyl)-1-[(1-methyl-1H-imidazol-4-yl)sulfonyl]pyrrolidin-3-amine

The title compound was prepared similarly to the procedure described in Example 323 substituting 1-bromo-3-chlorobenzene for bromo-3-(trifluoromethoxy)benzene. MS (ESI) *m*/*z* 435 (M+H)⁺.

### Example 326

### (3 S,4R)-4-(4-fluorophenyl)-1-[(1-methyl-1H-imidazol-4-yl)sulfonyl]-N-[3-(trifluoromethyl)phenyl]pyrrolidin-3 -amine

The title compound was prepared similarly to the procedure described in Example 323 substituting 1-bromo-3-(trifluoromethyl) benzene for bromo-3-(trifluoromethoxy)benzene. MS (ESI) *m*/*z* 469 (M+H)⁺.

### Example 327

### (3R,4S)-4-(4-fluorophenyl)-1-[(1-methyl-1H-imidazol-4-yl)sulfonyl]-N-[3-(trifluoromethyl)phenyl]pyrrolidin-3 -amine

Under nitrogen, a pressure vial was charged with Example 322B (100 mg, 0.28 mmol), 1-bromo-3-(trifluoromethyl)benzene (62 mg, 0.28 mmol), 2'-(di-tert-butylphosphino)-N,N-dimethylbiphenyl-2-amine (9.5 mg, 0.03 mmol), tris(dibenzylidene-acetone)dipalladium(0) (13 mg, 0.014 mmol), sodium tert-butoxide (107 mg, 1.1 mmol), and dioxane (4 mL). The reaction mixture was stirred at 80 °C for 2 hours. Then, the reaction mixture was concentrated. Purification via HPLC provided the title compound. MS (ESI) *m*/*z* 469 (M+H)⁺.

### Example 328

### (3R,4S)-N-(3-chlorophenyl)-4-(4-fluorophenyl)-1-[(1-methyl-1H-imidazol-4-yl)sulfonyl]pyrrolidin-3-amine

The title compound was prepared similarly to the procedure described in Example 327 substituting 1-bromo-3-chlorobenzene for 1-bromo-3-(trifluoromethyl)benzene, but the reaction only stirred for 1 hour. MS (ESI) *m*/*z* 435 (M+H)⁺.

### Example 329

### (3R,4S)-4-(4-fluorophenyl)-1-[(1-methyl-1H-imidazol-4-yl)sulfonyl]-N-[3-(trifluoromethoxy)phenyl]pyrrolidin-3-amine

The title compound was prepared similarly to the procedure described in Example 327 substituting 1-bromo-3-(trifluoromethoxy)benzene for 1-bromo-3-(trifluoromethyl)benzene. MS (ESI) *m*/*z* 485 (M+H)⁺.

### Example 330

### (3R,4S)-N,4-bis(4-fluorophenyl)-1-[(1-methyl-1H-imidazol-4-yl)sulfonyl]pyrrolidin-3-amine

The title compound was prepared similarly to the procedure described in Example 327 substituting 1-bromo-4-fluorobenzene for 1-bromo-3-(trifluoromethoxy)benzene. MS (ESI) *m*/*z* 419 (M+H)⁺.

### Example 331

### 3-(3-chlorobenzyl)-1-[(1-methyl-1H-imidazol-4-yl)sulfonyl]-4-phenylpyrrolidin-2-one

### Example 331A

### methyl 2-(3-chlorobenzyl)-4-nitro-3-phenylbutanoate

To a solution of diisopropylamine (210 mg, 2 mmol) in tetrahydrofurane (1 mL) at -78 °C was added butyllithium (1 mL, 2N, 2 mmol). The mixture was allowed to stir for 10 minutes. Then methyl 3-(3-chlorophenyl)propanoate in tetrahydrofurane (1 mL) was added. The mixture was allowed to stir for 30 minutes. (E)-(2-nitrovinyl)benzene in tetrahydrofuran (1 mL) was added. The mixture was allowed to stir for 1 hour, and partitioned with ammonium chloride (aq) and ethyl acetate. The organic fraction was collected and concentrated to provide the crude title compound.

### Example 331B

### 3-(3-chlorobenzyl)-4-phenylpyrrolidin-2-one

To a solution of Example 331A in methanol (2 mL) was added an acetic acid solution (2 mL. 50% aq.), and zinc (6x130mg, 12 mmol). The reaction mixture stirred for 2 hours. The reaction mixture was concentrated and partitioned between ethyl acetate and enough NaOH(1M aq.) to adjust the pH value to 10. The insoluble material was filtered off through celite. The organic fraction was collected,and dried over sodium hydroxide pellets for 16 hours. The mixture was washed with HCl(aq) and purified by silica gel flash chromatography (100% ethyl acetate) to afford the title compound.

### Example 331C

### 3-(3-chlorobenzyl)-1-[(1-methyl-1H-imidazol-4-yl)sulfonyl]-4-phenylpyrrolidin-2-one

To a solution of Example 331B (150 mg, 0.53 mmol) in tetrahydrofuran (1 mL) at -78 °C was added butyllithium (0.3 mL, 2 N, 0.6 mmol). The mixture was allowed to stir for 10 minutes. 1-Methyl-1H-imidazole-4-sulfonyl chloride (95 mg, 0.53 mmol) was added. The reaction stirred for 2 hours. The mixture was warmed to 20 °C and quenched with ammonium chloride (aq.) The mixture was extracted with ethyl acetate and purified by flash chromatography (10% methanol/ethyl acetate) followed by trituration in methanol. The precipitates were collected to afford the title compound. MS (ESI) *m*/*z* 430/432(3:1) (M+H)⁺.

### Example 332

### 2-Chloro-N-({trans-4-(4-fluorophenyl)-1-[(1-methyl-1H-imidazol-4-yl)sulfonyl]pyrrolidin-3-yl}methyl)-4-(trifluoromethyl)aniline

### Example 332A

### Trans-4-(4-fluorophenyl)-1-(1-methyl-1H-imidazol-4-ylsulfonyl)pyrrolidine-3-carbaldehyde

To a solution of Example 206 (180 mg, 0.53 mmol) in dichloromethane (2 mL) was added Dess-MartinPeriodinane (450 mg, 1.06 mmol). The reaction mixture was stirred at room temperature for 16 hours. The reaction mixture was filtered and washed with dichloromethane. The filtrate was concentrated to afford the title compound. MS (ESI) *m*/*z* 338.0 (M+H)⁺.

### Example 332B

### 2-Chloro-N-({trans-4-(4-fluorophenyl)-1-[(1-methyl-1H-imidazol-4-yl)sulfonyl]pyrrolidin-3-yl}methyl)-4-(trifluoromethyl)aniline

To Example 332A (150 mg, 0.45 mmol) in methanol acetate buffer (2 mL, made from 48 g AcOH and 30.5 g NaOAc in 1L methanol) was added 2-chloro-4-(trifluoromethyl)aniline (104 mg, 0.53 mmol) and sodium cyanoborohydride (42 mg, 0.67 mmol). The mixture was allowed to stir overnight. It was partitioned between dichoromethane and saturated sodium bicarbonate. Purification via HPLC afford the title compound. MS (ESI) m/z 517.0 (M+H)⁺.

### Example 333

### 3-Chloro-N-({trans-4-(4-fluorophenyl)-1-[(1-methyl-1H-imidazol-4-yl)sulfonyl]pyrrolidin-3-yl} methyl)aniline

The title compound was prepared similarly to the procedure described in Example 290 substituting Example 304 for Example 242E. MS (ESI) *m*/*z* 449.1 (M+H)⁺.

### Example 334

### N-({Trans-4-(4-fluorophenyl)-1-[(1-methyl-1H-imidazol-4-yl)sulfonyl]pyrrolidin-3-yl}methyl)-3-methylaniline

The title compound was prepared similarly to the procedure described in Example 290 substituting Example 316 for Example 242E. MS (ESI) *m*/*z* 429.1 (M+H)⁺.

### Example 335

### N-({Trans-4-(4-fluorophenyl)-1-[(1-methyl-1H-imidazol-4-yl)sulfonyl]pyrrolidin-3-yl}methyl)-3-(trifluoromethyl)aniline

The title compound was prepared using the procedure as described in Example 290 substituting Example 317 for Example 242E. MS (ESI) *m*/*z* 483.1 (M+H)⁺.

### Example 336

### 4-({Trans-4-(4-fluorophenyl)-1-[(1-methyl-1H-imidazol-4-yl)sulfonyl]pyrrolidin-3-yl}methoxy)-6-(trifluoromethyl)pyrimidine

The title compound was prepared using the procedure as described in Example 245 substituting 6-(trifluoromethyl)pyrimidin-4-ol for phenol. MS (ESI) *m*/*z* 486.1 (M+H)⁺.

### Example 337

### Trans-4-cyclopropyl-1-[(1-methyl-1H-imidazol-4-yl)sulfonyl]-N-[3-(trifluoromethyl)phenyl]pyrrolidin-3 -amine

### Example 337A

### Trans-1 -benzyl-3 -cyclopropyl-4-nitropyrrolidine

The title compound was prepared similarly to the procedure described in Example 2A substituting (E)-(2-nitrovinyl)cyclopropane for trans-4-methoxy-beta-nitrostyrene.

### Example 337B

### Trans-1-benzyl-4-cyclopropylpyrrolidin-3-amine

The title compound was prepared similarly to the procedure described in Example 2B substituting Example 337A for Example 2A.

### Example 337C

### tert-butyl (trans-1-benzyl-4-cyclopropylpyrrolidin-3-yl)carbamate

To a solution of Example 337B (1.0 g, 4.6 mmol) in ethyl acetate (10 mL) was added di-tert-butyl dicarbonate (1.4 g, 6.4 mmol). The mixture was allowed to stir for 1 hour. The mixture was concentrated and purified by silica gel column chromatography (50% ethyl acetate/hexanes) to afford the title compound.

### Example 337D

### tert-butyl ((trans-4-cyclopropylpyrrolidin-3-yl)carbamate

The title compound was prepared similarly to the procedure described in Example 2D substituting Example 337C for 2C.

### Example 337E

### tert-butyl ((trans-4-cyclopropyl-1-((1-methyl-1H-imidazol-4-yl)sulfonyl)pyrrolidin-3 -yl)carbamate

The title compound was prepared similarly to the procedure described in Example 2E substituting Example 337D for Example 2D.

### Example 337F

### Trans-4-cyclopropyl-1-((1-methyl-1H-imidazol-4-yl)sulfonyl)pyrrolidin-3-amine

To Example 337E (0.82 g, 2.2 mmol) in dioxane (5 mL), was added hydrochloric acid (4N in dioxane, 3 mL). The mixture was stirred overnight, concentrated, and partitioned between ethyl acetate and sodium hydroxide (1M). The organic fraction was dried over potassium carbonate, filtered, and concentrated to provide the title compound.

### Example 337G

### Trans-4-cyclopropyl-1-[(1-methyl-1H-imidazol-4-yl)sulfonyl]-N-[3-(trifluoromethyl)phenyl]pyrrolidin-3 -amine

To a solution of Example 337F (135 mg, 0.5 mmol) in dioxane (1 mL) was added 1-bromo-3-(trifluoromethyl)benzene (101 mg, 0.5 mmol), tris(dibenzylidene-acetone)dipalladium(0) (46 mg, 0.05 mmol), dicyclohexyl(2',4',6'-triisopropyl-[1,1'-biphenyl]-2-yl)phosphine (48 gm, 0.1 mmol), and sodium 2-methylpropan-2-olate (48 mg, 0.5 mmol). The mixture was heated at 110 °C for 2 hours, partitioned between ethyl acetate and ammonium chloride (aq), and purified by HPLC to give the title compound. MS (ESI) *m*/*z* 415 (M+H)⁺.

### Example 338

### Trans-N-(3-chlorophenyl)-4-cyclopropyl-1-[(1-methyl-1H-imidazol-4-yl)sulfonyl]pyrrolidin-3-amine

The title compound was prepared similarly to the procedure described in Example 337G substituting 1-bromo-3-chlorobenzene for 1-bromo-3-(trifluoromethyl)benzene. MS (ESI) *m*/*z* 381/383(3:1) (M+H)⁺.

### Example 339

### 4-{[3-(2-chlorophenyl)-4-(4-fluorophenyl)pyrrolidin-1-yl]sulfonyl}-1-methyl-1H-imidazole

### Example 339A

### 2-(2-chlorophenyl)acetaldehyde

To 2-(2-chlorophenyl)ethanol (1 g, 6.39 mmol) in 10 mL of dichloromethane was added Dess-MartinPeriodinane (3.52 g, 8.3 mmol) at 0°C. This mixture was stirred at 0°C for 1 hour. The mixture was filtered and washed with dichloromethane. The filtrate was concentrated to afford the title product.

### Example 339B

### 2-(2-Chlorophenyl)-3-(4-fluorophenyl)-4-nitrobutanal

The title compound was prepared similarly to the procedure described in Example 182A substituting Example 339A for 3-(3-chlorophenyl)propanal.

### Example 339C

### Trans-3-(2-chlorophenyl)-4-(4-fluorophenyl)pyrrolidine

The title compound was prepared similarly to the procedure described in Example 182B substituting Example 339B for Example 182A.

### Example 339D

### 4-{[3-(2-chlorophenyl)-4-(4-fluorophenyl)pyrrolidin-1-yl]sulfonyl}-1-methyl-1H-imidazole

The title compound was prepared similarly to the procedure described in Example 182C substituting Example 339C for Example 182 B. MS (ESI) *m*/*z* 420.3 (M+H)⁺.

### Example 340

### 4-{[3-(4-chlorophenyl)-4-(4-fluorophenyl)pyrrolidin-1-yl]sulfonyl}-1-methyl-1H-imidazole

### Example 340A

### 2-(4-Chlorophenyl)acetaldehyde

The title compound was prepared similarly to the procedure described in Example 339A substituting 2-(4-chlorophenyl)ethanol for 2-(2-chlorophenyl)ethanol.

### Example 340B

### 2-(4-Chlorophenyl)-3-(4-fluorophenyl)-4-nitrobutanal

The title compound was prepared similarly to the procedure described in Example 182A substituting Example 340A for 3-(3-chlorophenyl)propanal.

### Example 340C

The title compound was prepared similarly to the procedure described in Example 182B substituting Example 340B for Example 182A.

### Example 340D

### 4-{[3-(4-chlorophenyl)-4-(4-fluorophenyl)pyrrolidin-1-yl]sulfonyl}-1-methyl-1H-imidazole

The title compound was prepared similarly to the procedure described in Example 182C substituting Example 340C for Example 182B. MS (ESI) *m*/*z* 420.2 (M+H)⁺.

### Example 341

### 2-chloro-4-({trans-4-(4-fluorophenyl)-1-[(1-methyl-1H-imidazol-4-yl)sulfonyl]pyrrolidin-3-yl}amino)benzonitrile

A 10 mL microwave vial was charged with Example 322B (100 mg, 0.277 mmol) and 100 mg of premixed tris(dibenzylidene-acetone)dipalladium(0)/sodium tert-butoxide/ 2,2'-bis(diphenylphosphino)-1,1'-binaphthyl (0.05:0.15:2), aldrich 715530). The solids were suspended in 1,2-dimethoxyethane and 4-bromo-2-chlorobenzonitrile (72.1 mg, 0.333 mmol) was added. The reaction vessel was capped and the reaction mixture heated under microwave conditions for 15 min at 130 °C. The mixture was filtered through celite, concentrated, dissolved in 2 ml 50% MeOH/dimethylsulfoxide, and purified by reverse phase HPLC to obtain the title compound. MS (ESI) *m*/*z* 460 (M+H)⁺.

### Example 342

### 2-fluoro-4-({trans-4-(4-fluorophenyl)-1-[(1-methyl-1H-imidazol-4-yl)sulfonyl]pyrrolidin-3-yl}amino)benzonitrile

The title compound was prepared similarly to the procedure described in Example 341 substituting 4-bromo-2-fluorobenzonitrile for 4-bromo-2-(difluoromethyl)benzonitrile. MS (ESI) *m*/*z* 444 (M+H)⁺.

### Example 343

### 3-(3-chlorobenzyl)-1-[(1-methyl-1H-imidazol-4-yl)sulfonyl]-4-phenylimidazolidin-2-one

### Example 343A

### N1-(3-chlorobenzyl)-1-phenylethane-1,2-diamine

To (3-chlorophenyl)methanamine (0.28 g, 2 mmol) in methanol (5 mL) was added (E)-(2-nitrovinyl)benzene (0.30 g, 2 mmol). The mixture was allowed to stir for 4 hours. A solution of acetic acid in water (50%, 2 mL) was added, followed by zinc (5x130 mg, 10 mmol). The mixture was allowed to stir for 1 hour and concentrated. NaOH(1 M) was added to adjust the pH to 10. Ethyl acetate was added and the solution partitioned. The organic fraction was collected, dried over potassium carbonate, filtered, and concentrated to provide the title compound.

### Example 343B

### 1-(3-chlorobenzyl)-5-phenylimidazolidin-2-one

To a solution of Example 343A in dichloromethane (5 mL) at -78 °C was added triethylamine (100 mg, 1 mmol) and triphosgene (60 mg, 0.2 mmol). The reaction mixture was allowed to warm to room temperature and stir for 18 hours. The crude material was purified by flash chromatography (100% ethyl acetate) to afford the title compound.

### Example 343C

### 3-(3-chlorobenzyl)-1-[(1-methyl-1H-imidazol-4-yl)sulfonyl]-4-phenylimidazolidin-2-one

To a solution of Example 343B (60 mg, 0.2 mmol) in tetrahydrofuran (1 mL) at 0 °C was added sodium hydride (40 mg, 60%, 1 mmol) and 1-methyl-1H-imidazole-4-sulfonyl chloride (38 mg, 0.2 mmol). The mixture was stirred for 18 hours, quenched with ammonium chloride (aq). Ethyl acetate was added then the solution partitioned. The organic fraction was collected and concentrated. Purification via HPLC provided the title compound. MS (ESI) *m*/*z* 431/433 (3:1) (M+H)⁺.

### Example 344

### trans-1-[(1-methyl-1H-imidazol-4-yl)sulfonyl]-4-(pyridin-2-yl)-N-[3-(trifluoromethyl)phenyl]pyrrolidin-3 -amine

The title compound was prepared similarly to the procedure described in Example 337G substituting Example 307B for Example 337F. MS (ESI) *m*/*z* 452 (M+H)⁺.

### Example 345

### Trans-N-(3-chlorophenyl)-1-[(1-methyl-1H-imidazol-4-yl)sulfonyl]-4-(pyridin-2-yl)pyrrolidin-3-amine

The title compound was prepared similarly to the procedure described in Example 337G substituting Example 307B for Example 337F and substituting 1-bromo-3-chlorobenzene for 1-bromo-3-(trifluoromethyl)benzene. MS (ESI) *m*/*z* 418/420 (3:1) (M+H)⁺.

### Example 346

### 2-({Trans-4-(4-fluorophenyl)-1-[(1-methyl-1H-imidazol-4-yl)sulfonyl]pyrrolidin-3-yl}methoxy)-4-(trifluoromethyl)pyridine

The title compound was prepared similarly to the procedure described in Example 245 substituting 4-(trifluoromethyl)pyridin-2-ol for phenol. MS (ESI) *m*/*z* 485.1 (M+H)⁺.

### Example 347

### Trans-N-(3-cyanophenyl)-4-(4-fluorophenyl)-1-[(1-methyl-1H-imidazol-4-yl)sulfonyl]pyrrolidine-3-carboxamide

The title compound was prepared similarly to the procedure described in Example 242E substituting 3-aminobenzonitrile for aniline. MS (ESI) *m*/*z* 454.1 (M+H)⁺.

### Example 348

### 4-{[Trans-3-[(4-fluorophenoxy)methyl]-4-(4-fluorophenyl)pyrrolidin-1-yl]sulfonyl}-1-methyl-1H-imidazole

The title compound was prepared similarly to the procedure described in Example 245 substituting 4-fluorophenol for phenol. MS (ESI) *m*/*z* 434.1 (M+H)⁺.

### Example 349

### 4-(4-fluorophenyl)-1-[(1-methyl-1H-imidazol-4-yl)sulfonyl]-N-[3-(propan-2-yloxy)phenyl]pyrrolidin-3 -amine

The title compound was prepared similarly to the procedure described in Example 341 substituting 1-bromo-3-isopropoxybenzene for 4-bromo-2-(difluoromethyl)-1-fluorobenzene. MS (ESI) *m*/*z* 459 (M+H)⁺

### Example 350

### N-{4-(4-fluorophenyl)-1-[(1-methyl-1H-imidazol-4-yl)sulfonyl]pyrrolidin-3-yl}pyridazin-3-amine

### Example 350A

### 6-chloro-N-(4-(4-fluorophenyl)-1-(1-methyl-1H-imidazol-4-ylsulfonyl)pyrrolidin-3-yl)pyridazin-3 -amine.

4-(4-fluorophenyl)-1-(1-methyl-1H-imidazol-4-ylsulfonyl)pyrrolidin-3-amine (100 mg, 0.308 mmol), 3,6-dichloro-pyridazine (46 mg, 0.308 mmol), N,N-diisopropylethyl amine (53.8 µl, 0.308 mmol), and 4 mL ethanol were placed in a 2-5 mL microwave vial and irradiated in a microwave (Biotage Initiator™, maximum 400 Watts) for 1 hour at 175°C. The reaction contents were concentrated, reddissolved in 2 mL 50% dimethylsulfoxide/methanol and purified via reverse phase HPLC to yield the title compound.

### Example 350B

### N-{4-(4-fluorophenyl)-1-[(1-methyl-1H-imidazol-4-yl)sulfonyl]pyrrolidin-3-yl}pyridazin-3-amine

Example 350A (18 mg, 0.041 mmol) and methanol (4 ml) were added to 5% Pd/C, wet (4 mg, 0.940 µmol) and triethyl amine (8 mg, 0.082 mmol) in a 50 ml pressure bottle. The mixture stirred for 2 hours under 30 psi of hydrogen until HPLC indicated complete conversion. The mixture was filtered through a nylon membrane. Purification via HPLC provided the title compound. MS (ESI) *m*/*z* 459 (M+H)⁺.

### Example 351

### N-{(3R,4S)-4-(4-fluorophenyl)-1-[(1-methyl-1H-imidazol-4-yl)sulfonyl]pyrrolidin-3-yl}-6-(trifluoromethyl)pyridin-2-amine

The title compound was prepared similarly to the procedure described in Example 341 substituting 2-bromo-6-(trifluoromethyl)pyridine for 4-bromo-2-(difluoromethyl)-1-fluorobenzene. MS (ESI) *m*/*z* 470 (M+H)⁺.

### Example 352

### (3R,4S)-N-(3-chloro-4-fluorophenyl)-4-(4-fluorophenyl)-1-[(1-methyl-1H-imidazol-4-yl)sulfonyl]pyrrolidin-3 -amine

The title compound was prepared similarly to the procedure described in Example 341 substituting 4-bromo-2-chloro-1-fluorobenzene for 4-bromo-2-(difluoromethyl)-1-fluorobenzene. MS (ESI) *m*/*z* 453 (M+H)⁺.

### Example 353

### (3R,4S)-N-(3-fluorophenyl)-4-(4-fluorophenyl)-1-[(1-methyl-1H-imidazol-4-yl)sulfonyl]pyrrolidin-3-amine

The title compound was prepared similarly to the procedure described in Example 341 substituting 1-bromo-3-fluorobenzene for 4-bromo-2-(difluoromethyl)-1-fluorobenzene. MS (ESI) *m*/*z* 419 (M+H)⁺.

### Example 354

### Trans-N-(4-chlorobenzyl)-3-methyl-1-[(1-methyl-1H-imidazol-4-yl)sulfonyl]-4-phenylpyrrolidin-3-amine

### Example 354A

### Trans-1 -benzyl-3 -methyl-3 -nitro-4-phenylpyrrolidine

The title compound was prepared similarly to the procedure described in Example 2A substituting Example (E)-(2-nitroprop-1-en-1-yl)benzene for 4-methoxy-beta-nitrostyrene.

### Example 354B

### Trans-1-benzyl-3-methyl-4-phenylpyrrolidin-3-amine

The title compound was prepared similarly to the procedure described in Example 2B substituting Example 354A for Example 2A.

### Example 354C

### Trans-3 -methyl-4-phenylpyrrolidin-3 -amine

The title compound was prepared similarly to the procedure described in Example 2D substituting Example 354B for 2C.

### Example 354D

### Trans-3 -methyl-1-((1-methyl-1H-imidazol-4-yl)sulfonyl)-4-phenylpyrrolidin-3 -amine

The title compound was prepared similarly to the procedure described in Example 182C substituting Example 354C for Example 182B.

### Example 354E

### Trans-N-(4-chlorobenzyl)-3-methyl-1-[(1-methyl-1H-imidazol-4-yl)sulfonyl]-4-phenylpyrrolidin-3-amine

To a solution of Example 354D (100 mg, 0.28 mmol) in methanol acetate buffer (pH4, 1M, 1 mL, made from 48 g AcOH and 30.5 g NaOAc in 1 L methanol) was added 4-chlorobenzaldehyde (40 mg, 0.28 mmol) and sodium cyanoborohydride (40 mg, 0.65 mmol). The mixture was stirred for 18 hours and partitioned between ethyl acetate and NaOH(1 M). The organic fraction was collected, concentrated, and purified by HPLC to afford the title compound. MS (ESI) *m*/*z* 445/447 (3:1) (M+H)⁺.

### Example 355

### Trans-3-methyl-1-[(1-methyl-1H-imidazol-4-yl)sulfonyl]-4-phenyl-N-[4-(trifluoromethyl)benzyl]pyrrolidin-3 -amine

The title compound was prepared similarly to the procedure described in Example 354E substituting 4-(trifluoromethyl)benzaldehyde for 4-chlorobenzaldehyde. MS (ESI) *m*/*z* 479 (M+H)⁺.

### Example 356

### Trans-N-[2-chloro-4-(trifluoromethyl)benzyl]-3-methyl-1-[(1-methyl-1H-imidazol-4-yl)sulfonyl]-4-phenylpyrrolidin-3 -amine

The title compound was prepared similarly to the procedure described in Example 354E substituting 2-chloro-4-(trifluoromethyl)benzaldehyde for 4-chlorobenzaldehyde. MS (ESI) *m*/*z* 514/516 (3:1) (M+H)⁺.

### Example 357

### (3,4-Difluorophenyl){trans-4-(4-fluorophenyl)-1-[(1-methyl-1H-imidazol-4-yl)sulfonyl]pyrrolidin-3-yl}methanone

The title compound was prepared similarly to the procedure described in Example 271B substituting (3,4-difluorophenyl)magnesium bromide for phenylmagnesium bromide. MS (ESI) *m*/*z* 450.2 (M+H)⁺.

### Example 358

### Trans-N,4-bis(4-fluorophenyl)-1-[(1-methyl-1H-imidazol-4-yl)sulfonyl]pyrrolidine-3-carboxamide

The title compound was prepared similarly to the procedure described in Example 242E substituting 4-fluoroaniline for aniline. MS (ESI) *m*/*z* 447.1 (M+H)⁺.

### Example 359

### (3,4-Dichlorophenyl){trans-4-(4-fluorophenyl)-1-[(1-methyl-1H-imidazol-4-yl)sulfonyl]pyrrolidin-3-yl} methanone

The title compound was prepared similarly to the procedure described in Example 271B substituting (3,4-dichlorophenyl)magnesium bromide for phenylmagnesium bromide. MS (ESI) *m*/*z* 482.1 (M+H)⁺.

### Example 360

### (3,5-Dichlorophenyl){trans-4-(4-fluorophenyl)-1-[(1-methyl-1H-imidazol-4-yl)sulfonyl]pyrrolidin-3-yl} methanone

The title compound was prepared similarly to the procedure described in Example 271B substituting (3,5-dichlorophenyl)magnesium bromide for phenylmagnesium bromide. MS (ESI) *m*/*z* 482.1 (M+H)⁺.

### Example 361

### (3-Chloro-5-fluorophenyl){trans-4-(4-fluorophenyl)-1-[(1-methyl-1H-imidazol-4-yl)sulfonyl]pyrrolidin-3 -yl} methanone

The title compound was prepared similarly to the procedure described in Example 271B substituting (3-chloro-5-fluorophenyl)magnesium bromide for phenylmagnesium bromide. MS (ESI) *m*/*z* 466.2 (M+H)⁺.

### Example 362

### {Trans-4-(4-fluorophenyl)-1-[(1-methyl-1H-imidazol-4-yl)sulfonyl]pyrrolidin-3-yl}[3-(trifluoromethoxy)phenyl]methanone

The title compound was prepared similarly to the procedure described in Example 271B substituting (3-(trifluoromethoxy)phenyl)magnesium bromide for phenylmagnesium bromide. MS (ESI) *m*/*z* 498.1 (M+H)⁺.

### Example 363

### {Trans-4-(4-fluorophenyl)-1-[(1-methyl-1H-imidazol-4-yl)sulfonyl]pyrrolidin-3-yl}[4-(trifluoromethoxy)phenyl]methanone

The title compound was prepared similarly to the procedure described in Example 271B substituting (4-(trifluoromethoxy)phenyl)magnesium bromide for phenylmagnesium bromide. MS (ESI) *m*/*z* 498.1 (M+H)⁺.

### Example 364

### 3-[({Trans-4-(4-fluorophenyl)-1-[(1-methyl-1H-imidazol-4-yl)sulfonyl]pyrrolidin-3-yl}methyl)amino]benzamide

The title compound was prepared similarly to the procedure described in Example 290 substituting Example 347 for Example 242E. MS (ESI) *m*/*z* 458.1 (M+H)⁺.

### Example 365

### 3-(Aminomethyl)-N-({Trans-4-(4-fluorophenyl)-1-[(1-methyl-1H-imidazol-4-yl)sulfonyl]pyrrolidin-3-yl}methyl)aniline

The title compound was prepared similarly to the procedure described in Example 290 substituting Example 347 for Example 242E. MS (ESI) *m*/*z* 444.1 (M+H)⁺.

### Example 366

### 4-({Trans-4-(4-fluorophenyl)-1-[(1-methyl-1H-imidazol-4-yl)sulfonyl]pyrrolidin-3-yl}methoxy)-2-(trifluoromethyl)pyridine

The title compound was prepared similarly to the procedure described in Example 245 substituting 2-(trifluoromethyl)pyridin-4-ol for phenol. MS (ESI) *m*/*z* 485.1 (M+H)⁺.

### Example 367

### 4-Fluoro-N-({trans-4-(4-fluorophenyl)-1-[(1-methyl-1H-imidazol-4-yl)sulfonyl]pyrrolidin-3-yl}methyl)aniline

The title compound was prepared similarly to the procedure described in Example 290 substituting Example 358 for Example 242E. MS (ESI) *m*/*z* 433.1 (M+H)⁺.

### Example 368

### N-{(3R,4S)-4-(4-fluorophenyl)-1-[(1-methyl-1H-imidazol-4-yl)sulfonyl]pyrrolidin-3-yl}-4-(trifluoromethyl)pyridin-2-amine

The title compound was prepared similarly to the procedure described in Example 327 substituting 2-bromo-4-(trifluoromethyl)benzene for 1-bromo-3-(trifluoromethoxy)benzene. MS (ESI) *m*/*z* 470 (M+H)⁺.

### Example 369

### Trans-4-(4-fluorophenyl)-1-[(1-methyl-1H-imidazol-4-yl)sulfonyl]-N-[4-(trifluoromethyl)pyridin-2-yl]pyrrolidine-3 -carboxamide

The title compound was prepared similarly to the procedure described in Example 242E substituting 4-(trifluoromethyl)pyridin-2-amine for aniline. MS (ESI) *m*/*z* 498.1 (M+H)⁺.

### Example 370

### Trans-4-(4-fluorophenyl)-N-[4-fluoro-3-(trifluoromethoxy)phenyl]-1-[(1-methyl-1H-imidazol-4-yl)sulfonyl]pyrrolidine-3-carboxamide

The title compound was prepared similarly to the procedure described in Example 242E substituting 4-fluoro-3-(trifluoromethoxy)aniline for aniline. MS (ESI) *m*/*z* 531.1 (M+H)⁺.

### Example 371

### Trans-N-[4-chloro-3-(trifluoromethoxy)phenyl]-4-(4-fluorophenyl)-1-[(1-methyl-1H-imidazol-4-yl)sulfonyl]pyrrolidine-3-carboxamide

The title compound was prepared similarly to the procedure described in Example 242E substituting 4-chloro-3-(trifluoromethoxy)aniline for aniline. MS (ESI) *m*/*z* 547.1 (M+H)⁺.

### Example 372

### N-[4-chloro-2-({Trans-4-(4-fluorophenyl)-1-[(1-methyl-1H-imidazol-4-yl)sulfonyl]pyrrolidin-3-yl}amino)phenyl]acetamide

The title compound was prepared similarly to the procedure described in Example 337G substituting Example 3A for Example 337F and N-(2-bromo-4-chlorophenyl)acetamide for 1-bromo-3-(trifluoromethyl)benzene. MS (ESI) *m*/*z* 492/494 (3:1) (M+H)⁺.

### Example 373

### Trans-4-[(3-chlorophenyl)amino]-1-[(1-methyl-1H-imidazol-4-yl)sulfonyl]pyrrolidin-3-ol

### Example 373A

### (trans)-tert-butyl 3-((3-chlorophenyl)amino)-4-hydroxypyrrolidine-1-carboxylate

A mixture of 3-chloroaniline (0.38 g, 3 mmol) and tert-butyl 6-oxa-3-azabicyclo[3.1.0]hexane-3-carboxylate (0.56 g, 3 mmol) in ethanol (3 mL) was heated at 100 °C for 3 days. The mixture was concentrated and purified by flash chromatography (50% ethyl acetate/hexanes) to afford the title compound.

### Example 373B

### Trans-4-((3-chlorophenyl)amino)pyrrolidin-3-ol

To a solution of Example 373A (0.97 g, 3.1 mmol) in dioxane (3 mL) at 0 °C was added hydrochloric acid (4N in dioxane, 5 mL). The mixture was allowed to stir for 5 hours. Concentration provided the title compound as the hydrochloride salt.

### Example 373C

### Trans-4-[(3-chlorophenyl)amino]-1-[(1-methyl-1H-imidazol-4-yl)sulfonyl]pyrrolidin-

To a solution of Example 373B in dichloromethane (5 mL) was added triethylamine (1 g, 10 mmol) and 1-methyl-1H-imidazole-4-sulfonyl chloride (540 mg, 3.0 mmol). The mixture was allowed to stir for 16 hours. The reaction mixuture was concentrated and partitioned between ethyl acetate and NaOH(1 M). The organic fraction was collected, concentrated, and purified by flash chromatography (100% ethyl acetate) to afford the title compound. MS (ESI) *m*/*z* 357/359 (3:1) (M+H)⁺.

### Example 374

### N-[Trans-1-[(1-methyl-1H-imidazol-4-yl)sulfonyl]-4-(pyridin-2-yl)pyrrolidin-3-yl]-1-(trifluoromethyl)cyclopropanecarboxamide

The title compound was prepared similarly to the procedure described in Example 242E substituting Example 307B for 242D and substituting 1-(trifluoromethyl)cyclopropanecarboxylic acid for aniline. MS (ESI) *m*/*z* 444.0 (M+H)⁺.

### Example 375

### N-{(3R,4S)-4-(4-fluorophenyl)-1-[(1-methyl-1H-imidazol-4-yl)sulfonyl]pyrrolidin-3-yl}-1-(trifluoromethyl)cyclopropanecarboxamide

The title compound was prepared similarly to the procedure described in Example 242E substituting Example 322B for Example 242D and substituting 1-(trifluoromethyl)cyclopropanecarboxylic acid for aniline. MS (ESI) *m*/*z* 461.1 (M+H)⁺.

### Example 376

### 4-fluoro-N-({trans-4-(4-fluorophenyl)-1-[(1-methyl-1H-imidazol-4-yl)sulfonyl]pyrrolidin-3-yl}methyl)-3-(trifluoromethoxy)aniline

The title compound was prepared similarly to the procedure described in Example 290 substituting Example 370 for Example 242E. MS (ESI) *m*/*z* 517.2 (M+H)⁺.

### Example 377

### 4-chloro-N-({trans-4-(4-fluorophenyl)-1-[(1-methyl-1H-imidazol-4-yl)sulfonyl]pyrrolidin-3-yl}methyl)-3-(trifluoromethoxy)aniline

The title compound was prepared similarly to the procedure described in Example 290 substituting Example 371 for Example 242E. MS (ESI) *m*/*z* 533.2 (M+H)⁺.

### Example 378

### Trans-N-(3-chlorophenyl)-4-(4-fluorophenoxy)-1-[(1-methyl-1H-imidazol-4-yl)sulfonyl]pyrrolidin-3-amine

To a solution of Example 373 (142 mg, 0.4 mmol) in dimethylformamide (0.2 mL) was added copper(I) iodide (40 mg, 0.2 mmol), cesium carbonate (300 mg, 0.92 mmol), and 1-fluoro-4-iodobenzene (88 mg, 0.4 mmol). The mixture was stirred at 110 °C for 16 hours, then partitioned between ethyl acetate and water. The organic fraction was collected, concentrated,, and purified by HPLC to provide the title compound. MS (ESI) *m*/*z* 452/454 (3:1) (M+H)⁺.

### Example 379

### 2-({trans-4-(4-fluorophenyl)-1-[(1-methyl-1H-imidazol-4-yl)sulfonyl]pyrrolidin-3-yl}oxy)-4-(trifluoromethyl)pyridine

### Example 379A

### Trans-tert-butyl 3 -(4-fluorophenyl)-4-hydroxypyrrolidine-1 -carboxylate

To a solution of tert-butyl 6-oxa-3-azabicyclo[3.1.0]hexane-3-carboxylate (0.93 g, 5 mmol) in tetrahydrofuran (5 mL) at -78 °C was added copper(I) iodide (85 mg, 0.5 mmol) then (4-fluorophenyl)magnesium bromide (7 mL, 1 M, 7 mol). The mixture was warmed to room temperature and, allowed to stir for 3 hours. The reaction mixture was partitioned with ammonium chloride (aq). The organic fraction was collected,, dried over MgSO₄, filtered, and concentrated to provide the title compound.

### Example 379B

### Trans-4-(4-fluorophenyl)pyrrolidin-3-ol

The title compound was prepared as the hydrochloride salt similarly to the procedure described in Example 373B substituting Example 379A for Example 373A.

### Example 379C

### Trans-4-(4-fluorophenyl)-1-((1-methyl-1H-imidazol-4-yl)sulfonyl)pyrrolidin-3-ol

The title compound was prepared similarly to the procedure described in Example 373C substituting Example 379B for Example 373B.

### Example 379D

### 2-({Trans-4-(4-fluorophenyl)-1-[(1-methyl-1H-imidazol-4-yl)sulfonyl]pyrrolidin-3-yl}oxy)-4-(trifluoromethyl)pyridine

To a solution of Example 379C (110 mg, 0.34 mmol) in dimethylsulfoxide (0.3 mL) was added potassium 2-methylpropan-2-olate (50 mg, 0.45 mmol) and 2-fluoro-4-(trifluoromethyl)pyridine (70 mg, 0.43 mmol). The mixture was stirred at 100 °C for 16 hours, then partitioned between ethyl acetate and water. Purification by HPLC provided the title compound. MS (ESI) *m*/*z* 471 (M+H)⁺.

### Example 380

### 3-({(3R,4S)-4-(4-fluorophenyl)-1-[(1-methyl-1H-imidazol-4-yl)sulfonyl]pyrrolidin-3-yl}amino)benzonitrile

### Example 380A

### (3R,4S)-4-(4-fluorophenyl)-1-(1-methyl-1H-imidazol-4-ylsulfonyl)pyrrolidin-3-amine

Example 322B (2.53 g, 7.03 mmol) was partitioned between ethyl acetate (25 mL) and saturated sodium bicarbonate (aq. 10 mL). The organic fraction was collected. The aqueous fraction was washed with ethyl acetate 3 times. The combined organic fractions were dried over sodium sulfate and concentrated to give the title compound. MS (ESI) *m*/*z* 325.3 (M+H)⁺.

### Example 380B

### 3-({(3R,4S)-4-(4-fluorophenyl)-1-[(1-methyl-1H-imidazol-4-yl)sulfonyl]pyrrolidin-3-yl}amino)benzonitrile

The title compound was prepared similarly to the procedure described in Example 337G substituting Example 380A for Example 337F and substituting 3-bromobenzonitrile for 1-bromo-3-(trifluoromethyl)benzene. MS (ESI) *m*/*z* 426.1 (M+H)⁺.

### Example 381

### 6-chloro-2-[Trans-1-[(1-methyl-1H-imidazol-4-yl)sulfonyl]-4-(pyridin-2-yl)pyrrolidin-3-yl]-2,3-dihydro-1H-isoindol-1-one

### Example 381A

### Trans-N-(2-bromo-4-chlorobenzyl)-1-((1-methyl-1H-imidazol-4-yl)sulfonyl)-4-(pyridin-2-yl)pyrrolidin-3-amine

The title compound was prepared similarly to the procedure described in Example 354E substituting Example 307B for Example 354D and 2-bromo-4-chlorobenzaldehyde for 4-chlorobenzaldehyde.

### Example 381B

### 6-chloro-2-[Trans-1-[(1-methyl-1H-imidazol-4-yl)sulfonyl]-4-(pyridin-2-yl)pyrrolidin-3-yl]-2,3-dihydro-1H-isoindol-1-one

To a solution of Example 381A (500 mg, 0.98 mmol) in methanol (5 mL) in a 50 mL pressure bottle was added 1,1'-Bis(diphenylphosphino)ferrocene]dichloropalladium(II), (36 mg, 0.048 mmol) and triethylamine (0.27 mL, 2 mmol). The mixture was pressurized with Carbon Monoxide (60 psi), and stirred for 4 hours at 80 °C. The insoluble materials were filtered off and the filtrate was purified by HPLC to provide the title compound. MS (ESI) *m*/*z* 458/460 (3:1) (M+H)⁺.

### Example 382

### 3-benzyl-4-(4-fluorophenyl)-1-[(1-methyl-1H-imidazol-4-yl)sulfonyl]pyrrolidin-3-ol

### Example 382A

### 4-(4-fluorophenyl)-1-((1-methyl-1H-imidazol-4-yl)sulfonyl)pyrrolidin-3-one

To a solution of Example 379C (0.43 g, 1.3 mmol) in dichloromethane (3 mL) was added Dess-Martin periodinane (0.67 g, 1.6 mmol). The mixture was stirred overnight and partitioned between ethyl acetate and sodium hydroxide (1M aq.) . The organic fraction was collected, dried over MgSO₄, filtered, and concentrated to provide the title compound.

### Example 382B

### 3-benzyl-4-(4-fluorophenyl)-1-[(1-methyl-1H-imidazol-4-yl)sulfonyl]pyrrolidin-

To a solution of Example 382A (50 mg) in tetrahydrofuran (0.5 mL) at -78 °C was added benzylmagnesiumbromide (1 M, 1 mL, 1 mmol). The mixture was allowed to stir at room temperature for 1 hour. The reaction mxture was partitioned between Ammonium chloride (aq) and ethyl acetate. The organic frantion was collected, concentrated, and purified by flash chromatography (100% ethyl acetate) to afford the title compound..MS (ESI) *m*/*z* 416 (M+H)⁺.

### Example 383

### N-{trans-4-(4-fluorophenyl)-1-[(1-methyl-1H-imidazol-4-yl)sulfonyl]pyrrolidin-3 -yl} -5 - (trifluoromethyl)pyridazin-3 -amine

### Example 383A

### Trans-6-chloro-N-(4-(4-fluorophenyl)-1-(1-methyl-1H-imidazol-4-ylsulfonyl)pyrrolidin-3 -yl)-5 -(trifluoromethyl)pyridazin-3 -amine

Example 3A (100 mg, 0.308 mmol), 3,6-dichloro-4-(trifluoromethyl)pyridazine (67 mg, 0.308 mmol), diisopropylethylamine (53.8 µl, 0.308 mmol), and ethanol (4 mL) were placed in a 2-5 mL microwave vial and irradiated for 1 hr at 175°C. The reaction contents were concentrated, reddissolved in 2 mL 50% dimethylsulfoxide/methanol and purified via HPLC to provide the title compound.. MS (ESI) *m*/*z* 505 (M+H)⁺

### Example 383B

### N-{(3R,4S)-4-(4-fluorophenyl)-1-[(1-methyl-1H-imidazol-4-yl)sulfonyl]pyrrolidin-3-yl}-5-(trifluoromethyl)pyridazin-3 -amine

The title compound was prepared similarly to the procedure described in Example 350B substituting 6-chloro-N-(4-(4-fluorophenyl)-1-(1-methyl-1H-imidazol-4-ylsulfonyl)pyrrolidin-5-yl)-4-(trifluoromethyl)pyridazin-3-amine for 6-chloro-N-(4-(4-fluorophenyl)-1-(1 -methyl-1H-imidazol-4-ylsulfonyl)pyrrolidin-3-yl)pyridazin-3-amine. MS (ESI) *m*/*z* 471 (M+H)+

### Example 384

### N-{4-(4-fluorophenyl)-1-[(1-methyl-1H-imidazol-4-yl)sulfonyl]pyrrolidin-3 -yl} -4-(trifluoromethyl)pyridazin-3 -amine

The title compound was prepared similarly to the procedure described in Example 350A-350B As both isomers are formed in 350A. MS (ESI) *m*/*z* 471 (M+H)+

### Example 385

### 1-methyl-4-{[trans-3-phenyl-4-(phenylsulfonyl)pyrrolidin-1-yl]sulfonyl}-1H-imidazole

### Example 385A

### Trans-1-benzyl-3-phenyl-4-(phenylsulfonyl)pyrrolidine

The title compound was prepared similarly to the procedure described in Example 2A substituting phenyl trans-styryl sulfone for 4-methoxy-beta-nitrostyrene. MS (ESI) *m*/*z* 378.1 (M+H)⁺.

### Example 385B

### Trans-3-phenyl-4-(phenylsulfonyl)pyrrolidine

The title compound was prepared similarly to the procedure described in Example 2D substituting Example 385A for 2C. MS (ESI) *m*/*z* 288.1 (M+H)⁺.

### Example 385C

### 1-Methyl-4-{trans-3-phenyl-4-(phenylsulfonyl)pyrrolidin-1-yl]sulfonyl}-1H-imidazole

The title compound was prepared similarly to the procedure described in Example 2E substituting Example 385B for Example 2D. MS (ESI) *m*/*z* 432.1 (M+H)⁺.

### Example 386

### (3R,4S)-4-(4-fluorophenyl)-1-[(1-methyl-1H-imidazol-4-yl)sulfonyl]-N-{[-(trifluoromethyl)cyclopropyl]methyl}pyrrolidin-3-amine

The title compound was prepared similarly to the procedure described in Example 290 substituting Example 375 for Example 242E. MS (ESI) *m*/*z* 447.1 (M+H)⁺.

### Example 387

### (3S,4R)-N-(3-chloro-4-fluorophenyl)-4-(4-fluorophenyl)-1-[(1-methyl-1H-imidazol-4-yl)sulfonyl]pyrrolidin-3 -amine

The title compound was prepared similarly to the procedure described in Example 337G substituting Example 389A for Example 337F and 4-bromo-2-chloro-1-fluorobenzene for 1-bromo-3-(trifluoromethyl)benzene. MS (ESI) *m*/*z* 453.2 (M+H)⁺.

### Example 388

### N-{Trans-3-methyl-1-[(1-methyl-1H-imidazol-4-yl)sulfonyl]-4-phenylpyrrolidin-3-yl}-4-(trifluoromethyl)pyridin-2-amine

The title compound was prepared similarly to the procedure described in Example 337G substituting Example 354D for Example 337F and 2-bromo-4-(trifluoromethyl)pyridine for 1-bromo-3-(trifluoromethyl)benzene. MS (ESI) *m*/*z* 466 (M+H)⁺.

### Example 389

### 3-({(3S,4R)-4-(4-fluorophenyl)-1-[(1-methyl-1H-imidazol-4-yl)sulfonyl]pyrrolidin-3-yl}amino)benzonitrile

### Example 389A

### (3S,4R)-4-(4-fluorophenyl)-1-(1-methyl-1H-imidazol-4-ylsulfonyl)pyrrolidin-3-amine

The title compound was prepared similarly to the procedure described in Example 380A substituting Example 321B for Example 322B. MS (ESI) *m*/*z* 307.1 (M+H)⁺.

### Example 389B

### 3-({(3S,4R)-4-(4-fluorophenyl)-1-[(1-methyl-1H-imidazol-4-yl)sulfonyl]pyrrolidin-3-yl}amino)benzonitrile

The title compound was prepared similarly to the procedure described in Example 337G substituting 389A for Example 337F and substituting 3-bromobenzonitrile for 1-bromo-3-(trifluoromethyl)benzene. MS (ESI) *m*/*z* 426.3 (M+H)⁺.

### Example 390

### N-{(3R,4S)-4-(4-fluorophenyl)-1-[(1-methyl-1H-imidazol-4-yl)sulfonyl]pyrrolidin-3-yl}-6-(trifluoromethyl)pyrimidin-4-amine

The title compound was prepared similarly to the procedure described in Example 337G substituting Example 380A for Example 337F and substituting 4-bromo-6-(trifluoromethyl)pyrimidine for 1-bromo-3-(trifluoromethyl)benzene. MS (ESI) *m*/*z* 471.2 (M+H)⁺.

### Example 391

### (3R,4S)-4-(4-fluorophenyl)-1-[(1-methyl-1H-imidazol-4-yl)sulfonyl]-N-{[5-(trifluoromethyl)furan-2-yl]methyl}pyrrolidin-3-amine

The title compound was prepared similarly to the procedure described in Example 354E substituting Example 380A for Example 354D and substituting 5-(trifluoromethyl)furan-2-carbaldehyde for 4-chlorobenzaldehyde. MS (ESI) *m*/*z* 473.1 (M+H)⁺.

### Example 392

### 1,1,1-trifluoro-2-[3-({(3R,4S)-4-(4-fluorophenyl)-1-[(1-methyl-1H-imidazol-4-yl)sulfonyl]pyrrolidin-3-yl}amino)phenyl]propan-2-ol

### Example 392A

### 2-(3-Bromophenyl)-1,1,1-trifluoropropan-2-ol

To 1-(3-bromophenyl)ethanone (364 mg, 1.83 mmol) and (trifluoromethyl)trimethylsilane (3 mL, 1.46 mmol, 0.5 M in tetrahydrofuran) was added tetrabutylammonium fluoride (1 M in tetrahydrofurane, 2.0 mL, 2.0 mmol) at 0 °C. The reaction mixture stirred at room temperature for 3 hours. The reaction mixture was partitioned with saturated sodium carbonate (aq.) and dichloromethane. The organic fraction was collected. The aqueous fraction was washed with dichloromethane 3 times. The combined organic fractions were washed with saturated brine, dried over sodium sulfate, concentrated, and purified by flash chromatography (5:1 EtOAc/hexanes) to afford the title compound.

### Example 392B

### 1,1,1-trifluoro-2-[3-({(3R,4S)-4-(4-fluorophenyl)-1-[(1-methyl-1H-imidazol-4-yl)sulfonyl]pyrrolidin-3-yl}amino)phenyl]propan-2-ol

The title compound was prepared similarly to the procedure described in Example 337G substituting Example 380A for Example 337F and susbstituting Example 392A for 1-bromo-3-(trifluoromethyl)benzene. MS (ESI) *m*/*z* 513.3 (M+H)⁺.

### Example 393

### (3R,4S)-N-[(5-chlorothiophen-2-yl)methyl]-4-(4-fluorophenyl)-1-[(1-methyl-1H-imidazol-4-yl)sulfonyl]pyrrolidin-3 -amine

The title compound was prepared similarly to the procedure described in Example 354E substituting Example 380A for Example 354D and substituting 5-chlorothiophene-2-carbaldehyde for 4-chlorobenzaldehyde and. MS (ESI) *m*/*z* 455.3 (M+H)⁺.

### Example 394

### (3R,4S)-N-[2-chloro-4-(trifluoromethyl)benzyl]-1-[(1-methyl-1H-imidazol-4-yl)sulfonyl]-4-phenylpyrrolidin-3 -amine

The title compound was prepared similarly to the procedure described in Example 354E substituting Example 380A for Example 354D and substituting 2-chloro-4-(trifluoromethyl)benzaldehyde for 4-chlorobenzaldehyde. MS (ESI) *m*/*z* 455.3 (M+H)⁺.

### Example 395

### (3S,4R)-N-[2-chloro-4-(trifluoromethyl)benzyl]-1-[(1-methyl-1H-imidazol-4-yl)sulfonyl]-4-phenylpyrrolidin-3 -amine

### Example 395A

### (3S,4R)-1-(1-methyl-1H-imidazol-4-ylsulfonyl)-4-phenylpyrrolidin-3-amine hydrochloride

The title compound was prepared similarly to the conditions described in Example 2A-2F substituting (E)-4-(2-nitrovinyl)benzene for (E)-1-methoxy-4-(2-nitrovinyl)benzene and separating the enantiomers produced from the procedure of Example 2C via Chiral SFC separation (SFC 200 Column: Whelk-O1, 50 × 250 mm, 5 µm Column Temperature: 35 °C Mobile Phase: CO₂/IPA/DEA = 70/30/0.1 Flow rate: 70 g/min Back Pressure: 100 Bar Wavelength: 214 nm Cycle time: 5.0 min Injection: 4.0 mL Sample solution: 41000 mg in 250 mL methanol) RT = 6.00 minutes.

### Example 395B

### (3S,4R)-1-(1-methyl-1H-imidazol-4-ylsulfonyl)-4-phenylpyrrolidin-3-amine

The title compound was prepared similarly to the procedure described in Example 380A substituting Example 395A for Example 322B. MS (ESI) *m*/*z* 307.1 (M+H)⁺.

### Example 395 C

### (3S,4R)-N-[2-chloro-4-(trifluoromethyl)benzyl]-1-[(1-methyl-1H-imidazol-4-yl)sulfonyl]-4-phenylpyrrolidin-3 -amine

The title compound was prepared similarly to the procedure described in Example 354E substituting Example 395B for Example 354D and substituting 2-chloro-4-(trifluoromethyl)benzaldehyde for 4-chlorobenzaldehyde. MS (ESI) *m*/*z* 499.2 (M+H)⁺.

### Example 396

### N-{(3R,4S)-1-[(1-methyl-1H-imidazol-4-yl)sulfonyl]-4-phenylpyrrolidin-3-yl}-4-(trifluoromethyl)pyridin-2-amine

### Example 396A

### N-{(3R,4S)-1-[(1-methyl-1H-imidazol-4-yl)sulfonyl]-4-phenylpyrrolidin-3-yl}-4-(trifluoromethyl)pyridin-2-amine

The title compound was prepared similarly to the procedure described in Example 380A substituting Example 574A for Example 322B. MS (ESI) *m*/*z* 307.1 (M+H)⁺.

### Example 396B

### N-{(3R,4S)-1-[(1-methyl-1H-imidazol-4-yl)sulfonyl]-4-phenylpyrrolidin-3-yl}-4-(trifluoromethyl)pyridin-2-amine

The title compound was prepared similarly to the procedure described in Example 337G substituting Example 396A for Example 337F and 2-bromo-4-(trifluoromethyl)pyridine for 1-bromo-3-(trifluoromethyl)benzene. MS (ESI) *m*/*z* 452.2 (M+H)⁺.

### Example 397

### (3R,4S)-1-[(1-methyl-1H-imidazol-4-yl)sulfonyl]-4-phenyl-N-[3-(trifluoromethyl)phenyl]pyrrolidin-3 -amine

The title compound was prepared similarly to the procedure described in Example 337G substituting Example 396A for Example 337F. MS (ESI) *m*/*z* 452.2 (M+H)⁺.

### Example 398

### (3R,4S)-1-[(1-methyl-1H-imidazol-4-yl)sulfonyl]-4-phenyl-N-[4-(trifluoromethyl)benzyl]pyrrolidin-3 -amine

The title compound was prepared similarly to the procedure described in Example 354E substituting 4-(trifluoromethyl)benzaldehyde for 4-chlorobenzaldehyde and Example 396A Example 354D. MS (ESI) *m*/*z* 465.1 (M+H)⁺.

### Example 399

### 1,1,1-trifluoro-2-[3-({(3R,4S)-1-[(1-methyl-1H-imidazol-4-yl)sulfonyl]-4-phenylpyrrolidin-3-yl}amino)phenyl]propan-2-ol

The title compound was prepared similarly to the procedure described in Example 337G substituting Example 396A for Example 337F and Example 392A for 1-bromo-3-(trifluoromethyl)benzene. MS (ESI) *m*/*z* 495.1 (M+H)⁺.

### Example 400

### Trans-4-(benzyloxy)-N-(3-chlorophenyl)-1-[(1-methyl-1H-imidazol-4-yl)sulfonyl]pyrrolidin-3-amine

To a solution of Example 373C (71 mg, 0.2 mmol) in tetrahydrofuran (0.5 mL) was added potassium tert-butoxide (22 mg, 0.2 mmol) and benzyl bromide (40 mg, 0.23 mmol). The mixture was allowed to stir at room temperature for 2 hours. The reaction mixture was then partitioned with water and ethyl acetate. The organic fractin was collected, concentrate,and purified by HPLC to provide the title compound. MS (ESI) *m*/*z* 447/449 (3:1) (M+H)⁺.

### Example 401

### Trans-1-[(1-methyl-1H-imidazol-4-yl)sulfonyl]-4-phenyl-3-{[4-(trifluoromethyl)benzyl]amino}pyrrolidin-3-yl]methanol

### Example 401A

### (E)-2-nitro-3-phenylprop-2-en-1-ol

To (E)-(2-nitrovinyl)benzene (1.5 g, 10 mmol) in tetrahydrofuran (10 mL) was added formaldehyde (37%, aq., 10 mL), imidazole (0.68 g, 10 mmol), and antranilic acid (14 mg, 0.1 mmol). The mixture was allowed to stir for 16 hours. The reaction mixture was partitioned between ethyl acetate and hydrochloric acid (1N). The organic fraction was collected, dried over MgSO₄, filtered, and concentrated to provide the title compound.

### Example 401B

### (E)-((2-nitro-3-phenylallyl)oxy)methyl acetate

To a solution of Example 401A (900 mg, 5 mmol) in dichloromethane (5 mL) was added triethylamine (600 mg, 6 mmol) and acetyl chloride (400 mg, 5.1 mmol) at 0 °C. The mixture was allowed to stir for 1hour. Water was added and the solution partitioned. The organic fraction was collected, concentrated, and purified by flash chromatography (15% ethyl acetate/hexanes) to afford the title compound.

### Example 401C

### ((1-benzyl-3-nitro-4-phenylpyrrolidin-3-yl)methoxy)methyl acetate

The title compound was prepared similarly to the procedure described in Example 2A substituting Example 401B for 4-methoxy-beta-nitrostyrene.

### Example 401D

### 7-benzyl-9-phenyl-3-oxa-1,7-diazaspiro[4.4]nonane

The title compound was prepared similarly to the procedure described in Example 2B substituting Example 401C for Example 2A.

### Example 401E

### tert-butyl 7-benzyl-9-phenyl-3-oxa-1,7-diazaspiro[4.4]nonane-1-carboxylate

The title compound was prepared similarly to the procedure described in Example 337C substituting Example 401D for Example 337B.

### Example 401F

### tert-butyl 9-phenyl-3-oxa-1,7-diazaspiro[4.4]nonane-1-carboxylate

The title compound was prepared similarly to the procedure described in Example 2D substituting Example 401E for 2C.

### Example 401G

### tert-butyl 7-((1-methyl-1H-imidazol-4-yl)sulfonyl)-9-phenyl-3-oxa-1,7-diazaspiro[4.4]nonane-1-carboxylate

The title compound was prepared similarly to the procedure described in Example 373C substituting Example 401F for Example 373B.

### Example 401H

### 7-((1-methyl-1H-imidazol-4-yl)sulfonyl)-9-phenyl-3-oxa-1,7-diazaspiro[4.4]nonane

The title compound was prepared as the hydrochloride salt similarly to the procedure described in Example 373B substituting Example 401G for Example 373A

### Example 401I

### [Trans-1-[(1-methyl-1H-imidazol-4-yl)sulfonyl]-4-phenyl-3- {[4-(trifluoromethyl)benzyl]amino}pyrrolidin-3-yl]methanol

The title compound was prepared similarly to the procedure described in Example 354E substituting 4-(trifluoromethyl)benzaldehyde for 4-chlorobenzaldehyde and Example 401H for Example 354D MS (ESI) *m*/*z* 495 (M+H)⁺.

### Example 402

### Trans-N-(3-chlorophenyl)-1-[(1-methyl-1H-imidazol-4-yl)sulfonyl]-4-(prop-2-en-1-yloxy)pyrrolidin-3 -amine

The title compound was prepared similarly to the procedure described in Example 400 substituting allyl bromide for benzyl bromide. MS (ESI) *m*/*z* 397/399 (3:1) (M+H)⁺.

### Example 403

### Trans-N-(3-chlorophenyl)-1-[(1-methyl-1H-imidazol-4-yl)sulfonyl]-4-[(2-methylprop-2-en-1-yl)oxy]pyrrolidin-3 -amine

The title compound was prepared similarly to the procedure described in Example 400 substituting 3-bromo-2-methylprop-1-ene for benzyl bromide. MS (ESI) *m*/*z* 411/413 (3:1) (M+H)⁺.

### Example 404

### 3-{Trans-1-[(1-methyl-1H-imidazol-4-yl)sulfonyl]-4-phenylpyrrolidin-3-yl}-6-(trifluoromethyl)-3,4-dihydroquinazolin-2(1H)-one

### Example 404A

### N-(Trans-1-((1-methyl-1H-imidazol-4-yl)sulfonyl)-4-phenylpyrrolidin-3-yl)-2-nitro-5-(trifluoromethyl)benzamide

To Example 14A (140 mg, 0.4 mmol) in dimethylformamide (0.5 mL) was added 2-nitro-5-(trifluoromethyl)benzoic acid (96 mg, 0.4 mmol), triethylamine (100 mg, 1 mmol), and *O-*(Benzotriazol-l-yl)-*N,N,N',N'*-tetramethyluronium tetrafluoroborate (160 mg, 0.5 mmol). The mixture was allowed to stir overnight. Water was added and the precipitates were collected to provide the title compound.

### Example 404B

### Trans-N-(2-amino-5-(trifluoromethyl)benzyl)-1-((1-methyl-1H-imidazol-4-yl)sulfonyl)-4-phenylpyrrolidin-3 -amine

To a solution of Example 404A (110 mg, 0.21 mmol) in tetrahydrofuran (1 mL) was added borane dimethyl sulfide complex (2N, 1.4 mL, 2.8 mmol). The mixture was stirred at 60 °C for 2 days and quenched with hydrochloric acid (1 M aq.) . The mixture was allowed to stir for 30 minutes. Sodium hydroxide (1 M, aq.) was added to adjust the pH value to 10. The mixture was partitioned with ethyl acetate. The organic fraction was collected and dried over potassium carbonate, filtered, and concentrated to provide the title compound.

### Example 404C

### 3-{Trans-1-[(1-methyl-1H-imidazol-4-yl)sulfonyl]-4-phenylpyrrolidin-3-yl}-6-(trifluoromethyl)-3,4-dihydroquinazolin-2(1H)-one

To a solution of Example 404B (130 mg, 0.27 mmol) in dichloromethane (10 mL) was added triethylamine (100 mg, 1 mmol) and triphosegene (28 mg, 0.094 mmol). The mixture was allowed to stir for 1 hour, and then methanol was added. The mixture was concentrated and purified by HPLC to provide the title compound. MS (ESI) *m*/*z* 506 (M+H)⁺.

### Example 405

### 4-fluoro-N-{(3R,4S)-1-[(1-methyl-1H-imidazol-4-yl)sulfonyl]-4-phenylpyrrolidin-3-yl}pyridin-2-amine

The title compound was prepared similarly to the procedure described in Example 337G substituting Example 396A for Example 337F and 2-bromo-4-fluoropyridine for 1-bromo-3-(trifluoromethyl)benzene. MS (ESI) *m*/*z* 402.1 (M+H)⁺.

### Example 406

### N-{(3R,4S)-1-[(1-methyl-1H-imidazol-4-yl)sulfonyl]-4-phenylpyrrolidin-3-yl}-7H-pyrrolo[2,3-d]pyrimidin-4-amine

The title compound was prepared similarly to the procedure described in Example 337G substituting Example 396A for Example 337F and substituting 4-bromo-7H-pyrrolo[2,3-D]pyrimidine for 1-bromo-3-(trifluoromethyl)benzene. MS (ESI) *m*/*z* 424.2 (M+H)⁺.

### Example 407

### Trans-7-[(1-methyl-1H-imidazol-4-yl)sulfonyl]-9-phenyl-1-[4-(trifluoromethyl)benzyl]-3-oxa-1,7-diazaspiro[4.4]nonan-2-one

The title compound was prepared similarly to the procedure described in Example 404C substituting Example 401I for Example 404B. MS (ESI) *m*/*z* 521 (M+H)⁺.

### Example 408

### 3,5-difluoro-N-{(3R,4S)-1-[(1-methyl-1H-imidazol-4-yl)sulfonyl]-4-phenylpyrrolidin-3-yl}-4-(trifluoromethyl)pyridin-2-amine

To Example 396A (100 mg, 0.33 mmol) in dimethylsulfoxide (0.5 mL) was added potassium tert-butoxide (50 mg, 0.45 mmol) and 2,3,5-trifluoro-4-(trifluoromethyl)pyridine (80 mg, 0.4 mmol). The mixture was stirred at 80 °C for 2hours, and then partitioned between water and ethyl acetate. The organic fraction was collected, dried over potassium carbonate, filtered, concentrated, and purified by reversed phase HPLC to provide the title compound. MS (ESI) *m*/*z* 488 (M+H)⁺.

### Example 409

### (3R,4S)-1-[(1-methyl-1H-imidazol-4-yl)sulfonyl]-4-phenyl-N-{[5-(trifluoromethyl)pyridin-2-yl]methyl}pyrrolidin-3-amine

The title compound was prepared similarly to the procedure described in Example 354E substituting Example 574A for Example 354D and substituting 5-(trifluoromethyl)picolinaldehyde for 4-chlorobenzaldehyde. MS (ESI) *m*/*z* 466.1 (M+H)⁺.

### Example 410

### 5-cyclopropyl-N-{(3R,4S)-1-[(1-methyl-1H-imidazol-4-yl)sulfonyl]-4-phenylpyrrolidin-3-yl}pyrazin-2-amine

The title compound was prepared similarly to the procedure described in Example 337G substituting Example 396A for Example 337F and 2-bromo-5-cyclopropylpyrazine for 1-bromo-3-(trifluoromethyl)benzene. MS (ESI) *m*/*z* 425.3 (M+H)⁺.

### Example 411

### 5-cyclobutyl-N-{(3R,4S)-1-[(1-methyl-1H-imidazol-4-yl)sulfonyl]-4-phenylpyrrolidin-3-yl}pyrazin-2-amine

The title compound was prepared similarly to the procedure described in Example 337G substituting Example 396A for Example 337F and 2-bromo-5-cyclobutylpyrazine for 1-bromo-3-(trifluoromethyl)benzene. MS (ESI) *m*/*z* 439.3 (M+H)⁺.

### Example 412

### Trans-N-(3-chlorophenyl)-1-[(1-methyl-1H-imidazol-4-yl)sulfonyl]-4-(2-methylpropoxy)pyrrolidin-3-amine

To Example 403 (17mg, 0.041 mmol) in tetrahydrofuran (4 mL) was added 5% Pt/C (20 mg, 0.041 mmol) . The mixture was stirred for 45 minutes under 30 psi of hydrogen at room temperature. The mixture was filtered through a nylon membrane and purified by reversed phase HPLC to provide the title compound. MS (ESI) *m*/*z* 413/415 (3:1) (M+H)⁺.

### Example 413

### N-{(3R,4S)-1-[(1-methyl-1H-imidazol-4-yl)sulfonyl]-4-phenylpyrrolidin-3-yl}-5-(pyrrolidin-1-yl)pyrazin-2-amine

The title compound was prepared similarly to the procedure described in Example 337G substituting Example 396A for Example 337F and substituting 2-bromo-5-(pyrrolidin-1-yl)pyrazine for 1-bromo-3-(trifluoromethyl)benzene. MS (ESI) *m*/*z* 454.3 (M+H)⁺.

### Example 414

### N-{(3R,4S)-1-[(1-methyl-1H-imidazol-4-yl)sulfonyl]-4-phenylpyrrolidin-3-yl}-5-(trifluoromethyl)pyrazine-2-carboxamide

The title compound was prepared similarly to the procedure described in Example 242E substituting Example 574A for aniline and 5-(trifluoromethyl)pyrazine-2-carboxylic acid for Example 242D. MS (ESI) *m*/*z* 481.2 (M+H)⁺.

### Example 415

### 5-chloro-N-{(3R,4S)-1-[(1-methyl-1H-imidazol-4-yl)sulfonyl]-4-phenylpyrrolidin-3-yl}pyrazine-2-carboxamide

The title compound was prepared similarly to the procedure described in Example 242E substituting Example 574A for aniline and 5-chloropyrazine-2-carboxylic acid for Example 242D. MS (ESI) *m*/*z* 447.1 (M+H)⁺.

### Example 416

### (3R,4S)-N-(4-chlorobenzyl)-1-[(1-methyl-1H-imidazol-4-yl)sulfonyl]-4-phenylpyrrolidin-3-amine

The title compound was prepared similarly to the procedure described in Example 354E substituting Example 574A for Example 354D. MS (ESI) *m*/*z* 431.1(M+H)⁺

### Example 417

### (3R,4S)-N-(3-chloro-4-fluorophenyl)-1-[(1-methyl-1H-imidazol-4-yl)sulfonyl]-4-phenylpyrrolidin-3-amine

The title compound was prepared similarly to the procedure described in Example 337G substituting Example 396A for Example 337F and substituting 4-bromo-2-chloro-1-fluorobenzene for 1-bromo-3-(trifluoromethyl)benzeneMS (ESI) *m*/*z* 435.1 (M+H)⁺.

### Example 418

### (3R,4S)-N-(3,4-difluorophenyl)-4-(4-fluorophenyl)-1-[(1-methyl-1H-imidazol-4-yl)sulfonyl]pyrrolidin-3 -amine

The title compound was prepared similarly to the procedure described in Example 337G substituting Example 380A for Example 337F and substituting 1-bromo-3,4-difluorobenzene for 1-bromo-3-(trifluoromethyl)benzene. MS (ESI) *m*/*z* 437.1 (M+H)⁺.

### Example 419

### 6-chloro-N-{(3R,4S)-1-[(1-methyl-1H-imidazol-4-yl)sulfonyl]-4-phenylpyrrolidin-3-yl}pyridazine-3-carboxamide

The title compound was prepared similarly to the procedure described in Example 242E substituting Example 574A for aniline and substituting 6-chloropyridazine-3-carboxylic acid for Example 242D. MS (ESI) *m*/*z* 447.2 (M+H)⁺.

### Example 420

### N-{(3R,4S)-1-[(1-methyl-1H-imidazol-4-yl)sulfonyl]-4-phenylpyrrolidin-3-yl}-2-(morpholin-4-yl)pyrimidin-4-amine

The title compound was prepared similarly to the procedure described in Example 337G substituting Example 396A for Example 337F and substituting 4-(4-bromopyrimidin-2-yl)morpholine for 1-bromo-3-(trifluoromethyl)benzene. MS (ESI) *m*/*z* 470.2 (M+H)⁺.

### Example 421

### 4-({(3R,4S)-1-[(1-methyl-1H-imidazol-4-yl)sulfonyl]-4-phenylpyrrolidin-3-yl}amino)-2,3-dihydro-1H-isoindol-1-one

The title compound was prepared similarly to the procedure described in Example 337G substituting Example 396A for Example 337F and substituting 4-(4-bromopyrimidin-2-yl)morpholine for 1-bromo-3-(trifluoromethyl)benzene. MS (ESI) *m*/*z* 470.2 (M+H)⁺.

### Example 422

### (3R,4S)-1-[(1-methyl-1H-imidazol-4-yl)sulfonyl]-N-[3-(methylsulfonyl)phenyl]-4-phenylpyrrolidin-3 -amine

The title compound was prepared similarly to the procedure described in Example 337G substituting Example 396A for Example 337F and substituting 1-bromo-3-(methylsulfonyl)benzene for 1-bromo-3-(trifluoromethyl)benzene. MS (ESI) *m*/*z* 461.2 (M+H)⁺.

### Example 423

### (3R,4S)-1-[(1-methyl-1H-imidazol-4-yl)sulfonyl]-4-phenyl-N-{[5-(trifluoromethyl)piperazin-2-yl]methyl}pyrrolidin-3-amine

The title compound was prepared using the same procedures as described in Example 290 substituting Example 414 for Example 242E. MS (ESI) *m*/*z* 473.2 (M+H)⁺.

### Example 424

### 5-fluoro-N-{(3R,4S)-1-[(1-methyl-1H-imidazol-4-yl)sulfonyl]-4-phenylpyrrolidin-3-yl}pyrimidin-2-amine

The title compound was prepared similarly to the procedure described in Example 57 substituting 2-bromo-5- fluoropyrimidine for 1-bromo-2-methylbenzene MS (ESI) *m*/*z* 403.2 (M+H)⁺

### Example 425

### Trans-4-cyclohexyl-1-[(1-methyl-1H-imidazol-4-yl)sulfonyl]-N-[3-(trifluoromethoxy)phenyl]pyrrolidin-3-amine

### Example 425A

### (E)-(2-nitrovinyl)cyclohexane

A solution of cyclohexane carboxaldehyde (5.0 g, 44.6 mmol) and nitromethane (4.1 g, 66.9 mmol) in tetrahydrofuran (25 ml) and tert butanol (25 ml) was stirred and chilled in an ice bath under nitrogen. Potassium-t-butoxide (1.0 g,9.0 mmol) was added as a solid in one portion and the reaction was allowed to stir and warm to room temperature over one hour. The reaction was stirred for an additional four hours and was then partitioned with a saturated solution of ammonium chloride. The organic fraction was collected, and the aqueous portion was washed with dichloromethane. The combined organic fractions were washed with brine, dried over sodium sulfate, filtered, and concentrated. The resulting liquid was dissolved in dichloromethane (100 ml) and chilled in an ice bath. Trifluoroacetic anhydride (9.8 g, 46.7 mmol) was added in one portion, and the solution stirred for five minutes. Triethylamine (9.5 g, 93.5 mmol) was added dropwise. Stirring was continued for one hour, and then the reaction was allowed to warm to room temperature and stir for two hours. The reaction was partitioned with a solution of saturated ammonium chloride. The organic fraction was collected. The aqueous portion was washed with dichloromethane. The organic fractions were combined and washed with water and brine, and dried over sodium sulfate. The mixture was filtered, concentrated and chromatograhed on a silica gel flah column eluting with 98:2 heptane:ethyl acetate to afford 5.1 g of a slightly tinted liquid. MS (DCI) *m*/*z* 173.1 (M+NH₄)⁺

### Example 425B

### trans-4-cyclohexyl-1-(1-methyl-1H-imidazol-4-ylsulfonyl)pyrrolidin-3-amine

The title compound was prepared as an HCl salt similarly to the procedures described in Examples 2A-2F substituting Example 425A for trans-4-methoxy-beta-nitrostyrene in Example 2A. MS (DCI) *m*/*z* 313.1 (M+H)⁺

### Example 425C

### trans-4-cyclohexyl-1-[(1-methyl-1H-imidazol-4-yl)sulfonyl]-N-[3-(trifluoromethoxy)phenyl]pyrrolidin-3-amine

The title compound was prepared similarly to the procedures described in Example 57 substituting Example 425B for Example 14A and substituting 1-bromo-3-(trifluoromethyoxy)benzene for 1-bromo-2-methylbenzene. MS (ESI) *m*/*z* 473.2 (M+H)⁺.

### Example 426

### (3R,4S)-1-[(1-methyl-1H-imidazol-4-yl)sulfonyl]-4-phenyl-N-(4,4,4-trifluorobutyl)pyrrolidin-3-amine

The title compound was prepared similarly to the procedure described in Example 354E substituting 4,4,4-trifluorobutanal for 4-chlorobenzaldehyde and substituting Example 574A for Example 354D. MS (ESI) *m*/*z* 417.0 (M+H)⁺

### Example 427

### (3R,4S)-N-(2-cyclopentylethyl)-1-[(1-methyl-1H-imidazol-4-yl)sulfonyl]-4-phenylpyrrolidin-3-amine

The title compound was prepared similarly to the procedure described in Example 354E substituting 2-cyclopentylacetaldehyde for 4-chlorobenzaldehyde and substituting Example 574A for Example 354D. MS (ESI) *m*/*z* 403.0 (M+H)⁺

### Example 428

### (3R,4S)-1-[(1-methyl-1H-imidazol-4-yl)sulfonyl]-4-phenyl-N-[(5-propylfuran-2-yl)methyl]pyrrolidin-3 -amine

The title compound was prepared similarly to the procedure described in Example 354E substituting 5-propylfuran-2-carbaldehyde for 4-chlorobenzaldehyde and substituting Example 574A for Example 354D. MS (ESI) *m*/*z* 429.0 (M+H)⁺.

### Example 429

### (3R,4S)-1-[(1-methyl-1H-imidazol-4-yl)sulfonyl]-N-[(4-methyltetrahydro-2H-pyran-4-yl)methyl]-4-phenylpyrrolidin-3 -amine

The title compound was prepared similarly to the procedure described in Example 354E substituting 4-methyltetrahydro-2H-pyran-4-carbaldehyde for 4-chlorobenzaldehyde and substituting Example 574A for Example 354D. MS (ESI) *m*/*z* 419.0 (M+H)⁺.

### Example 430

### (3R,4S)-1-[(1-methyl-1H-imidazol-4-yl)sulfonyl]-4-phenyl-N-[(4-phenyl-1,3-thiazol-5-yl)methyl]pyrrolidin-3 -amine

The title compound was prepared similarly to the procedure described in Example 354E substituting 4-phenylthiazole-5-carbaldehyde for 4-chlorobenzaldehyde and substituting Example 574A for Example 354D. MS (ESI) *m*/*z* 480.0 (M+H)⁺

### Example 431

### (3R,4S)-1-[(1-methyl-1H-imidazol-4-yl)sulfonyl]-N-[(5-methyl-1,3-thiazol-2-yl)methyl]-4-phenylpyrrolidin-3 -amine

The title compound was prepared similarly to the procedure described in Example 354E substituting 5-methylthiazole-2-carbaldehyde for 4-chlorobenzaldehyde and substituting Example 574A for Example 354D. MS (ESI) *m*/*z* 418.0 (M+H)⁺

### Example 432

### (3R,4S)-N-[(3,5-dichloropyridin-4-yl)methyl]-1-[(1-methyl-1H-imidazol-4-yl)sulfonyl]-4-phenylpyrrolidin-3 -amine

The title compound was prepared similarly to the procedure described in Example 354E substituting 3,5-dichloroisonicotinaldehyde for 4-chlorobenzaldehyde and substituting Example 574A for Example 354D. MS (ESI) *m*/*z* 467.0 (M+H)⁺

### Example 433

### (3R,4S)-N-(2-ethylhexyl)-1-[(1-methyl-1H-imidazol-4-yl)sulfonyl]-4-phenylpyrrolidin-3-amine

The title compound was prepared similarly to the procedure described in Example 354E substituting 2-ethylhexanal for 4-chlorobenzaldehyde and substituting Example 574A for Example 354D. MS (ESI) *m*/*z* 419.0 (M+H)⁺

### Example 434

### (3R,4S)-1-[(1-methyl-1H-imidazol-4-yl)sulfonyl]-N-[(4-methyl-1,3-thiazol-5-yl)methyl]-4-phenylpyrrolidin-3 -amine

The title compound was prepared similarly to the procedure described in Example 354E substituting 4-methylthiazole-5-carbaldehyde for 4-chlorobenzaldehyde and substituting Example 574A for Example 354D. MS (ESI) *m*/*z* 418.0 (M+H)⁺

### Example 435

### (3R,4S)-N-[(2,4-dichloro-1,3-thiazol-5-yl)methyl]-1-[(1-methyl-1H-imidazol-4-yl)sulfonyl]-4-phenylpyrrolidin-3 -amine

The title compound was prepared similarly to the procedure described in Example 354E substituting 2,4-dichlorothiazole-5-carbaldehyde for 4-chlorobenzaldehyde and substituting Example 574A for Example 354D. MS (ESI) *m*/*z* 473.0 (M+H)⁺

### Example 436

### (3R,4S)-N-(2,2-dimethylpropyl)-1-[(1-methyl-1H-imidazol-4-yl)sulfonyl]-4-phenylpyrrolidin-3-amine

The title compound was prepared similarly to the procedure described in Example 354E substituting pivalaldehyde for 4-chlorobenzaldehyde and substituting Example 574A for Example 354D. MS (ESI) *m*/*z* 377.0 (M+H)⁺

### Example 437

### (3R,4S)-N-[(5-methyl-1-benzothiophen-2-yl)methyl]-1-[(1-methyl-1H-imidazol-4-yl)sulfonyl]-4-phenylpyrrolidin-3 -amine

The title compound was prepared similarly to the procedure described in Example 354E substituting 5-methylbenzo[b]thiophene-2-carbaldehyde for 4-chlorobenzaldehyde and substituting Example 574A for Example 354D. MS (ESI) *m*/*z* 467.0 (M+H)⁺.

### Example 438

### (3R,4S)-N-[(2-bromo-1,3-thiazol-5 -yl)methyl]-1-[(1-methyl-1H-imidazol-4-yl)sulfonyl]-4-phenylpyrrolidin-3 -amine

The title compound was prepared similarly to the procedure described in Example 354E substituting 2-bromothiazole-5-carbaldehyde for 4-chlorobenzaldehyde and substituting Example 574A for Example 354D. MS (ESI) *m*/*z* 483.0 (M+H)⁺.

### Example 439

### (3R,4S)-1-[(1-methyl-1H-imidazol-4-yl)sulfonyl]-N-(2-methylpropyl)-4-phenylpyrrolidin-3-amine

The title compound was prepared similarly to the procedure described in Example 354E substituting isobutyraldehyde for 4-chlorobenzaldehyde and substituting Example 574A for Example 354D. MS (ESI) *m*/*z* 363.0 (M+H)⁺.

### Example 440

### (3R,4S)-N-[(3-chloro-1-benzothiophen-2-yl)methyl]-1-[(1-methyl-1H-imidazol-4-yl)sulfonyl]-4-phenylpyrrolidin-3 -amine

The title compound was prepared similarly to the procedure described in Example 354E substituting 3-chlorobenzo[b]thiophene-2-carbaldehyde for 4-chlorobenzaldehyde and substituting Example 574A for Example 354D. MS (ESI) *m*/*z* 487.0 (M+H)⁺.

### Example 441

### (3R,4S)-N-[(2,4-dimethyl-1,3-thiazol-5-yl)methyl]-1-[(1-methyl-1H-imidazol-4-yl)sulfonyl]-4-phenylpyrrolidin-3 -amine

The title compound was prepared similarly to the procedure described in Example 354E substituting 2,4-dimethylthiazole-5-carbaldehyde for 4-chlorobenzaldehyde and substituting Example 574A for Example 354D. MS (ESI) *m*/*z* 432.0 (M+H)⁺.

### Example 442

### (3R,4S)-1-[(1-methyl-1H-imidazol-4-yl)sulfonyl]-4-phenyl-N-(thieno[2,3-b]pyridin-2-ylmethyl)pyrrolidin-3 -amine

The title compound was prepared similarly to the procedure described in Example 354E substituting thieno [2,3-b]pyridine-2-carbaldehyde for 4-chlorobenzaldehyde and Example 574A for Example 354D. MS (ESI) *m*/*z* 454.0 (M+H)⁺.

### Example 443

### (3R,4S)-N-[(2-methyl-1-benzofuran-3-yl)methyl]-1-[(1-methyl-1H-imidazol-4-yl)sulfonyl]-4-phenylpyrrolidin-3 -amine

The title compound was prepared similarly to the procedure described in Example 354E substituting 2-methylbenzofuran-3-carbaldehyde for 4-chlorobenzaldehyde and substituting Example 574A for Example 354D. MS (ESI) *m*/*z* 451.0 (M+H)⁺.

### Example 444

### (3R,4S)-N-[(6-chloro-2-methylimidazo[1,2-a]pyridin-3-yl)methyl]-1-[(1-methyl-1H-imidazol-4-yl)sulfonyl] -4-phenylpyrrolidin-3 -amine

The title compound was prepared similarly to the procedure described in Example 354E substituting 6-chloro-2-methylimidazo[1,2-a]pyridine-3-carbaldehyde for 4-chlorobenzaldehyde and substituting Example 574A for Example 354D. MS (ESI) *m*/*z* 485.0 (M+H)⁺.

### Example 445

### (3R,4S)-N-{[5-(4-fluorophenyl)pyridin-3-yl]methyl}-1-[(1-methyl-1H-imidazol-4-yl)sulfonyl]-4-phenylpyrrolidin-3 -amine

The title compound was prepared similarly to the procedure described in Example 354E substituting 5-(4-fluorophenyl) nicotinaldehyde for 4-chlorobenzaldehyde and substituting Example 574A for Example 354D. MS (ESI) *m*/*z* 492.0 (M+H)⁺.

### Example 446

### (3R,4S)-1-[(1-methyl-1H-imidazol-4-yl)sulfonyl]-N-(2-methylpentyl)-4-phenylpyrrolidin-3-amine

The title compound was prepared similarly to the procedure described in Example 354E substituting 2-methylpentanal for 4-chlorobenzaldehyde and substituting Example 574A for Example 354D. MS (ESI) *m*/*z* 391.0 (M+H)⁺

### Example 447

### (3R,4S)-1-[(1-methyl-1H-imidazol-4-yl)sulfonyl]-4-phenyl-N-(tetrahydrofuran-3-ylmethyl)pyrrolidin-3 -amine

The title compound was prepared similarly to the procedure described in Example 354E substituting tetrahydrofuran-3-carbaldehyde for 4-chlorobenzaldehyde and substituting Example 574A for Example 354D. MS (ESI) *m*/*z* 391.0 (M+H)⁺

### Example 448

### (3R,4S)-1-[(1-methyl-1H-imidazol-4-yl)sulfonyl]-N-pentyl-4-phenylpyrrolidin-3 -amine

The title compound was prepared similarly to the procedure described in Example 354E substituting pentanal for 4-chlorobenzaldehyde and substituting Example 574A for Example 354D. MS (ESI) *m*/*z* 377.0 (M+H)⁺.

### Example 449

### (3R,4S)-N-(2-ethylbutyl)-1-[(1-methyl-1H-imidazol-4-yl)sulfonyl]-4-phenylpyrrolidin-3-amine

The title compound was prepared similarly to the procedure described in Example 354E substituting 2-ethylbutanal for 4-chlorobenzaldehyde and substituting Example 574A for Example 354D. MS (ESI) *m*/*z* 391.0 (M+H)⁺

### Example 450

### (3R,4S)-N-(2,2-dimethylbutyl)-1-[(1-methyl-1H-imidazol-4-yl)sulfonyl]-4-phenylpyrrolidin-3-amine

The title compound was prepared similarly to the procedure described in Example 354E substituting 2,2-dimethylbutanal for 4-chlorobenzaldehyde and substituting Example 574A for Example 354D. MS (ESI) *m*/*z* 391.0 (M+H)⁺.

### Example 451

### (3R,4S)-1-[(1-methyl-1H-imidazol-4-yl)sulfonyl]-N-[(2-methyl-1,3-thiazol-5-yl)methyl]-4-phenylpyrrolidin-3 -amine

The title compound was prepared similarly to the procedure described in Example 354E substituting 2-methylthiazole-5-carbaldehyde for 4-chlorobenzaldehyde and substituting Example 574A for Example 354D. MS (ESI) *m*/*z* 418.0 (M+H)⁺.

### Example 452

### (3R,4S)-1-[(1-methyl-1H-imidazol-4-yl)sulfonyl]-4-phenyl-N-[2-(tetrahydro-2H-pyran-4-yl)ethyl]pyrrolidin-3 -amine

The title compound was prepared similarly to the procedure described in Example 354E substituting 2-(tetrahydro-2H-pyran-4-yl)acetaldehyde for 4-chlorobenzaldehyde and substituting Example 574A for Example 354D. MS (ESI) *m*/*z* 419.0 (M+H)⁺.

### Example 453

### (3R,4S)-N-(1,3-benzothiazol-2-ylmethyl)-1-[(1-methyl-1H-imidazol-4-yl)sulfonyl]-4-phenylpyrrolidin-3 -amine

The title compound was prepared similarly to the procedure described in Example 354E substituting benzo[d]thiazole-2-carbaldehyde for 4-chlorobenzaldehyde and substituting Example 574A for Example 354D. MS (ESI) *m*/*z* 454.0 (M+H)⁺.

### Example 454

### (3R,4S)-1-[(1-methyl-1H-imidazol-4-yl)sulfonyl]-N-[(4-methyl-1,3-thiazol-2-yl)methyl]-4-phenylpyrrolidin-3 -amine

The title compound was prepared similarly to the procedure described in Example 354E substituting 4-methylthiazole-2-carbaldehyde for 4-chlorobenzaldehyde and substituting Example 574A for Example 354D. MS (ESI) *m*/*z* 418.0 (M+H)⁺.

### Example 455

### (3R,4S)-N-[(4,5-dimethylthiophen-2-yl)methyl]-1-[(1-methyl-1H-imidazol-4-yl)sulfonyl]-4-phenylpyrrolidin-3 -amine

The title compound was prepared similarly to the procedure described in Example 354E substituting 4,5-dimethylthiophene-2-carbaldehyde for 4-chlorobenzaldehyde and substituting Example 574A for Example 354D. MS (ESI) *m*/*z* 431.0 (M+H)⁺.

### Example 456

### (3R,4S)-1-[(1-methyl-1H-imidazol-4-yl)sulfonyl]-4-phenyl-N-(4,5,6,7-tetrahydro-1,3-benzothiazol-2-ylmethyl)pyrrolidin-3 -amine

The title compound was prepared similarly to the procedure described in Example 354E substituting 4,5,6,7-tetrahydrobenzo[d]thiazole-2-carbaldehyde for 4-chlorobenzaldehyde and substituting Example 574A for Example 354D. MS (ESI) *m*/*z* 458.0 (M+H)⁺.

### Example 457

### (3R,4S)-1-[(1-methyl-1H-imidazol-4-yl)sulfonyl]-4-phenyl-N-(1,3 -thiazol-5-ylmethyl)pyrrolidin-3-amine

The title compound was prepared similarly to the procedure described in Example 354E substituting thiazole-5-carbaldehyde for 4-chlorobenzaldehyde and substituting Example 574A for Example 354D. MS (ESI) *m*/*z* 404.0 (M+H)⁺.

### Example 458

### (3R,4S)-1-[(1-methyl-1H-imidazol-4-yl)sulfonyl]-4-phenyl-N-[2-(2,6,6-trimethylcyclohex-1-en-1-yl)ethyl]pyrrolidin-3 -amine

The title compound was prepared similarly to the procedure described in Example 354E substituting 2-(2,6,6-trimethylcyclohex-1-en-1-yl)acetaldehyde for 4-chlorobenzaldehyde and substituting Example 574A for Example 354D. MS (ESI) *m*/*z* 457.0 (M+H)⁺.

### Example 459

### (3R,4S)-1-[(1-methyl-1H-imidazol-4-yl)sulfonyl]-4-phenyl-N-{[1-(propan-2-yl)piperidin-4-yl]methyl}pyrrolidin-3-amine

The title compound was prepared similarly to the procedure described in Example 354E substituting 1-isopropylpiperidine-4-carbaldehyde for 4-chlorobenzaldehyde and substituting Example 574A for Example 354D. MS (ESI) *m*/*z* 446.0 (M+H)⁺.

### Example 460

### (3R,4S)-N-{[4-(4-fluorophenyl)-1,3-thiazol-2-yl]methyl}-1-[(1-methyl-1H-imidazol-4-yl)sulfonyl]-4-phenylpyrrolidin-3 -amine

The title compound was prepared similarly to the procedure described in Example 354E substituting 4-(4-fluorophenyl)thiazole-2-carbaldehyde for 4-chlorobenzaldehyde and substituting Example 574A for Example 354D. MS (ESI) *m*/*z* 498.0 (M+H)⁺.

### Example 461

### (3R,4S)-1-[(1-methyl-1H-imidazol-4-yl)sulfonyl]-N-[2-(4-methylphenyl)ethyl]-4-phenylpyrrolidin-3-amine

The title compound was prepared similarly to the procedure described in Example 354E substituting 2-(p-tolyl)acetaldehyde for 4-chlorobenzaldehyde and substituting Example 574A for Example 354D. MS (ESI) *m*/*z* 425.0 (M+H)⁺.

### Example 462

### (3R,4S)-1-[(1-methyl-1H-imidazol-4-yl)sulfonyl]-N-[(5-methylthiophen-2-yl)methyl]-4-phenylpyrrolidin-3 -amine

The title compound was prepared similarly to the procedure described in Example 354E substituting 5-methylthiophene-2-carbaldehyde for 4-chlorobenzaldehyde and substituting Example 574A for Example 354D. MS (ESI) *m*/*z* 417.0 (M+H)⁺.

### Example 463

### (3R,4S)-N-(3-methylbutyl)-1-[(1-methyl-1H-imidazol-4-yl)sulfonyl]-4-phenylpyrrolidin-3-amine

The title compound was prepared similarly to the procedure described in Example 354E substituting 3-methylbutanal for 4-chlorobenzaldehyde and substituting Example 574A for Example 354D. MS (ESI) *m*/*z* 377.0 (M+H)⁺.

### Example 464

### (3R,4S)-1-[(1-methyl-1H-imidazol-4-yl)sulfonyl]-4-phenyl-N-(3,5,5-trimethylhexyl)pyrrolidin-3-amine

The title compound was prepared similarly to the procedure described in Example 354E substituting 3,5,5-trimethylhexanal for 4-chlorobenzaldehyde and Example 574A for Example 354D. MS (ESI) *m*/*z* 433.0 (M+H)⁺.

### Example 465

### (3R,4S)-1-[(1-methyl-1H-imidazol-4-yl)sulfonyl]-4-phenyl-N-(2-phenylpropyl)pyrrolidin-3-amine

The title compound was prepared similarly to the procedure described in Example 354E substituting 2-phenylpropanal for 4-chlorobenzaldehyde and substituting Example 574A for Example 354D. MS (ESI) *m*/*z* 425.0 (M+H)⁺.

### Example 466

### (3R,4S)-N-(3,3-dimethylbutyl)-1-[(1-methyl-1H-imidazol-4-yl)sulfonyl]-4-phenylpyrrolidin-3 -amine

The title compound was prepared similarly to the procedure described in Example 354E substituting 3,3-dimethylbutanal for 4-chlorobenzaldehyde and substituting Example 574A for Example 354D. MS (ESI) *m*/*z* 391.0 (M+H)⁺.

### Example 467

### (3R,4S)-N-[(1-methylcyclohexyl)methyl]-1-[(1-methyl-1H-imidazol-4-yl)sulfonyl]-4-phenylpyrrolidin-3 -amine

The title compound was prepared similarly to the procedure described in Example 354E substituting 1-methylcyclohexanecarbaldehyde for 4-chlorobenzaldehyde and substituting Example 574A for Example 354D. MS (ESI) *m*/*z* 417.0 (M+H)⁺.

### Example 468

### (3R,4S)-N-[2-(3-chlorophenyl)ethyl]-1-[(1-methyl-1H-imidazol-4-yl)sulfonyl]-4-phenylpyrrolidin-3-amine

The title compound was prepared similarly to the procedure described in Example 354E substituting 2-(3-chlorophenyl)acetaldehyde for 4-chlorobenzaldehyde and substituting Example 574A for Example 354D. MS (ESI) *m*/*z* 445.0 (M+H)⁺.

### Example 469

### (3R,4S)-N-[2-(4-tert-butylphenoxy)ethyl]-1-[(1-methyl-1H-imidazol-4-yl)sulfonyl]-4-phenylpyrrolidin-3-amine

The title compound was prepared similarly to the procedure described in Example 354E substituting 2-(4-(tert-butyl)phenoxy)acetaldehyde for 4-chlorobenzaldehyde and substituting Example 574A for Example 354D. MS (ESI) *m*/*z* 483.0 (M+H)⁺.

### Example 470

### (3R,4S)-N-(2-ethyl-3-methylbutyl)-1-[(1-methyl-1H-imidazol-4-yl)sulfonyl]-4-phenylpyrrolidin-3-amine

The title compound was prepared similarly to the procedure described in Example 354E substituting 2-ethyl-3-methylbutanal for 4-chlorobenzaldehyde and substituting Example 574A for Example 354D. MS (ESI) *m*/*z* 405.0 (M+H)⁺.

### Example 471

### (3R,4S)-N-[3-(3-chlorophenyl)propyl]-1-[(1-methyl-1H-imidazol-4-yl)sulfonyl]-4-phenylpyrrolidin-3-amine

The title compound was prepared similarly to the procedure described in Example 354E substituting 3-(3-chlorophenyl) propanal for 4-chlorobenzaldehyde and substituting Example 574A for Example 354D. MS (ESI) *m*/*z* 459.0 (M+H)⁺.

### Example 472

### (3R,4S)-N-[(4-bromo-1,3-thiazol-2-yl)methyl]-1-[(1-methyl-1H-imidazol-4-yl)sulfonyl]-4-phenylpyrrolidin-3-amine

The title compound was prepared similarly to the procedure described in Example 354E substituting 4-bromothiazole-2-carbaldehyde for 4-chlorobenzaldehyde and substituting Example 574A for Example 354D. MS (ESI) *m*/*z* 482 (M+H)⁺.

### Example 473

### (3R,4S)-1-[(1-methyl-1H-imidazol-4-yl)sulfonyl]-N-[(4-methyl-2-phenyl-1,3-thiazol-5-yl)methyl]-4-phenylpyrrolidin-3-amine

The title compound was prepared similarly to the procedure described in Example 354E substituting 4-methyl-2-phenylthiazole-5-carbaldehyde for 4-chlorobenzaldehyde and substituting Example 574A for Example 354D. MS (ESI) *m*/*z* 494 (M+H)⁺.

### Reference Example 474

### N-(3,4-difluorophenyl)-1-[(2,4-dimethyl-1,3-thiazol-5-yl)sulfonyl]-4-phenylpyrrolidin-3-amine

### Example 474A

### N-(3,4-difluorophenyl)-4-phenylpyrrolidin-3-amine

To a 100 mL round bottom flask with magnetic stir bar under argon was added cesium carbonate (28.7 g, 88 mmol)], 4-bromo-1,2-difluorobenzene (5.66 g, 29.3 mmol), 1-benzyl-4-phenylpyrrolidin-3-amine (7.4 g, 29.3 mmol), and Dichloro[1,3-bis(2,6-Di-3-pentylphenyl)imidazol-2-ylidene](3-chloropyridyl)palladium(II), (0.928 g, 1.173 mmol). 1,2-dimethoxyethane (25mL) was added and the reaction mixture was stirred at 80°C for 18 hours. The reaction mixture was cooled, concentrated onto celite, and purified via flash chromatography (5-50% EtOAc/Hex, 220g column) to provide the title compound.

### Example 474B

### N-(3,4-difluorophenyl)-1-[(2,4-dimethyl-1,3-thiazol-5-yl)sulfonyl]-4-phenylpyrrolidin-3-amine

A 4 mL vial with a magnetic stir bar was charged with Example 474A (40 mg, 0.146 mmol) and a solution of diisopropylethylamine in dichloromethane (1 mL of a 5 wt% diisopropylethylamine). A solution of 2,4-dimethylthiazole-5-sulfonyl chloride (972 µl, 0.292 mmol, 0.3 mM in 5% diisopropylethylamine in dichloromethane) was added and the reaction mixture was allowed to stir for 4 hours. The reaction was concentrated, redissolved in 2 mL of 50% dimethylsulfoxide/methanol and purified via HPLC to provide the title compound. MS (ESI) *m*/*z* 450 (M+H)⁺.

### Example 475

### N-(3,4-difluorophenyl)-1-{[3-methyl-1-(propan-2-yl)-1H-pyrazol-4-yl]sulfonyl}-4-phenylpyrrolidin-3-amine

The title compound was prepared similarly to the procedure described in Example 474B substituting 1-isopropyl-3-methyl-1H-pyrazole-4-sulfonyl chloride for 2,4-dimethylthiazole-5-sulfonyl chloride. MS (ESI) *m*/*z* 461 (M+H)⁺.

### Example 476

### N-(3,4-difluorophenyl)-1-{[1-methyl-3-(trifluoromethyl)-1H-pyrazol-4-yl]sulfonyl}-4-phenylpyrrolidin-3-amine

The title compound was prepared similarly to the procedure described in Example 474B substituting 1-methyl-3-(trifluoromethyl)-1H-pyrazole-4-sulfonyl chloride for 2,4-dimethylthiazole-5-sulfonyl chloride. MS (ESI) *m*/*z* 487 (M+H)⁺.

### Reference Example 477

### N-(3,4-difluorophenyl)-4-phenyl-1-(pyridin-3-ylsulfonyl)pyrrolidin-3-amine

The title compound was prepared similarly to the procedure described in Example 474B substituting pyridine-3-sulfonyl chloride for 2,4-dimethylthiazole-5-sulfonyl chloride. MS (ESI) *m*/*z* 416 (M+H)⁺.

### Reference Example 478

### 5-({3-[(3,4-difluorophenyl)amino]-4-phenylpyrrolidin-1-yl}sulfonyl)-3,3-dimethyl-1,3-dihydro-2H-indol-2-one

The title compound was prepared similarly to the procedure described in Example 474B substituting 3,3-dimethyl-2-oxoindoline-5-sulfonyl chloride for 2,4-dimethylthiazole-5-sulfonyl chloride. MS (ESI) *m*/*z* 498 (M+H)⁺.

### Example 479

### 1-[(1-cyclopentyl-3-methyl-1H-pyrazol-4-yl)sulfonyl]-N-(3,4-difluorophenyl)-4-phenylpyrrolidin-3-amine

The title compound was prepared similarly to the procedure described in Example 474B substituting 1-cyclopentyl-3-methyl-1H-pyrazole-4-sulfonyl chloride for 2,4-dimethylthiazole-5-sulfonyl chloride. MS (ESI) *m*/*z* 487 (M+H)⁺.

### Reference Example 480

### 6-({3-[(3,4-difluorophenyl)amino]-4-phenylpyrrolidin-1-yl}sulfonyl)quinazoline-2,4(1H,3H)-dione

The title compound was prepared similarly to the procedure described in Example 474B substituting 2,4-dioxo-1,2,3,4-tetrahydroquinazoline-6-sulfonyl chloride for 2,4-dimethylthiazole-5-sulfonyl chloride. MS (ESI) *m*/*z* 499 (M+H)⁺.

### Reference Example 481

### 7-({3-[(3,4-difluorophenyl)amino]-4-phenylpyrrolidin-1-yl}sulfonyl)-6-methyl-2H-1,4-benzoxazin-3(4H)-one

The title compound was prepared similarly to the procedure described in Example 474B substituting 6-methyl-3-oxo-3,4-dihydro-2H-benzo[b][1,4]oxazine-7-sulfonyl chloride for 2,4-dimethylthiazole-5-sulfonyl chloride. MS (ESI) *m*/*z* 500 (M+H)⁺.

### Example 482

### N-(3,4-difluorophenyl)-1-[(1,3-dimethyl-1H-pyrazol-5-yl)sulfonyl]-4-phenylpyrrolidin-3-amine

The title compound was prepared similarly to the procedure described in Example 474B substituting 1,3-dimethyl-1H-pyrazole-5-sulfonyl chloride for 2,4-dimethylthiazole-5-sulfonyl chloride. MS (ESI) *m*/*z* 433 (M+H)⁺.

### Example 483

### N-(3,4-difluorophenyl)-1-[(1,5-dimethyl-1H-pyrazol-4-yl)sulfonyl]-4-phenylpyrrolidin-3-amine

The title compound was prepared similarly to the procedure described in Example 474B substituting 1,5-dimethyl-1H-pyrazole-4-sulfonyl chloride for 2,4-dimethylthiazole-5-sulfonyl chloride. MS (ESI) *m*/*z* 433 (M+H)⁺.

### Reference Example 484

### N-(3,4-difluorophenyl)-1-[(4-methylthiophen-2-yl)sulfonyl]-4-phenylpyrrolidin-3-amine

The title compound was prepared similarly to the procedure described in Example 474B substituting 4-methylthiophene-2-sulfonyl chloride for 2,4-dimethylthiazole-5-sulfonyl chloride. MS (ESI) *m*/*z* 435 (M+H)⁺.

### Reference Example 485

### N-(3,4-difluorophenyl)-4-phenyl-1-(pyrrolidin-1-ylsulfonyl)pyrrolidin-3-amine

The title compound was prepared similarly to the procedure described in Example 474B substituting pyrrolidine-1-sulfonyl chloride for 2,4-dimethylthiazole-5-sulfonyl chloride. MS (ESI) *m*/*z* 408 (M+H)⁺.

### Example 486

### 1-[(5-chloro-1,3-dimethyl-1H-pyrazol-4-yl)sulfonyl]-N-(3,4-difluorophenyl)-4-phenylpyrrolidin-3-amine

The title compound was prepared similarly to the procedure described in Example 474B substituting 5-chloro-1,3-dimethyl-1H-pyrazole-4-sulfonyl chloride for 2,4-dimethylthiazole-5-sulfonyl chloride. MS (ESI) *m*/*z* 467 (M+H)⁺.

### Reference Example 487

### N-(3,4-difluorophenyl)-1-[(5-methyl-1,2-oxazol-4-yl)sulfonyl]-4-phenylpyrrolidin-3-amine

The title compound was prepared similarly to the procedure described in Example 474B substituting 5-methylisoxazole-4-sulfonyl chloride for 2,4-dimethylthiazole-5-sulfonyl chloride. MS (ESI) *m*/*z* 420(M+H)⁺.

### Reference Example 488

### N-(3,4-difluorophenyl)-1-[(2,5-dimethylfuran-3-yl)sulfonyl]-4-phenylpyrrolidin-3-amine

The title compound was prepared similarly to the procedure described in Example 474B substituting 2,5-dimethylfuran-3-sulfonyl chloride for 2,4-dimethylthiazole-5-sulfonyl chloride. MS (ESI) *m*/*z* 433 (M+H)⁺.

### Reference Example 489

### methyl 3-({3-[(3,4-difluorophenyl)amino]-4-phenylpyrrolidin-1-yl}sulfonyl)thiophene-2-carboxylate

The title compound was prepared similarly to the procedure described in Example 474B substituting methyl 3-(chlorosulfonyl)thiophene-2-carboxylate for 2,4-dimethylthiazole-5-sulfonyl chloride. MS (ESI) *m*/*z* 479 (M+H)⁺.

### Example 490

### N-(3,4-difluorophenyl)-1-[(1,2-dimethyl-1H-imidazol-5-yl)sulfonyl]-4-phenylpyrrolidin-3-amine

The title compound was prepared similarly to the procedure described in Example 474B substituting 1,2-dimethyl-1H-imidazole-5-sulfonyl chloride for 2,4-dimethylthiazole-5-sulfonyl chloride. MS (ESI) *m*/*z* 433 (M+H)⁺.

### Reference Example 491

### methyl 5-({3-[(3,4-difluorophenyl)amino]-4-phenylpyrrolidin-1-yl}sulfonyl)-4-methoxythiophene-3-carboxylate

The title compound was prepared similarly to the procedure described in Example 474B substituting methyl 5-(chlorosulfonyl)-4-methoxythiophene-3-carboxylate for 2,4-dimethylthiazole-5-sulfonyl chloride. MS (ESI) *m*/*z* 509(M+H)⁺.

### Reference Example 492

### N-(3,4-difluorophenyl)-1-[(5-ethylthiophen-2-yl)sulfonyl]-4-phenylpyrrolidin-3-amine

The title compound was prepared similarly to the procedure described in Example 474B substituting 5-ethylthiophene-2-sulfonyl chloride for 2,4-dimethylthiazole-5-sulfonyl chloride. MS (ESI) *m*/*z* 449 (M+H)⁺.

### Reference Example 493

### N-(3,4-difluorophenyl)-1-[(4-methylpiperidin-1-yl)sulfonyl]-4-phenylpyrrolidin-3-amine

The title compound was prepared similarly to the procedure described in Example 474B substituting 4-methylpiperidine-1-sulfonyl chloride for 2,4-dimethylthiazole-5-sulfonyl chloride. MS (EST) *m*/*z* 436 (M+H)⁺.

### Reference Example 494

### N-(3,4-difluorophenyl)-1-{[6-(morpholin-4-yl)pyridin-3-yl]sulfonyl}-4-phenylpyrrolidin-3-amine

The title compound was prepared similarly to the procedure described in Example 474B substituting 6-morpholinopyridine-3-sulfonyl chloride for 2,4-dimethylthiazole-5-sulfonyl chloride. MS (ESI) *m*/*z* 501(M+H)⁺.

### Reference Example 495

### 1-(1,3-benzodioxol-5-ylsulfonyl)-N-(3,4-difluorophenyl)-4-phenylpyrrolidin-3-amine

The title compound was prepared similarly to the procedure described in Example 474B substituting benzo[d][1,3]dioxole-5-sulfonyl chloride for 2,4-dimethylthiazole-5-sulfonyl chloride. MS (ESI) *m*/*z* 459 (M+H)⁺.

### Reference Example 496

### N-(3,4-difluorophenyl)-1-(2,3-dihydro-1,4-benzodioxin-6-ylsulfonyl)-4-phenylpyrrolidin-3-amine

The title compound was prepared similarly to the procedure described in Example 474B substituting 2,3-dihydrobenzo[b][1,4]dioxine-6-sulfonyl chloride for 2,4-dimethylthiazole-5-sulfonyl chloride. MS (ESI) *m*/*z* 473 (M+H)⁺.

### Reference Example 497

### N-(3,4-difluorophenyl)-1-(6,7-dihydro-5H-pyrrolo[1,2-a]imidazol-3-ylsulfonyl)-4-phenylpyrrolidin-3-amine

The title compound was prepared similarly to the procedure described in Example 474B substituting 6,7-dihydro-5H-pyrrolo[1,2-a]imidazole-3-sulfonyl chloride for 2,4-dimethylthiazole-5-sulfonyl chloride. MS (ESI) *m*/*z* 445 (M+H)⁺.

### Reference Example 498

### N-(3,4-difluorophenyl)-4-phenyl-1-[(tetrahydro-2H-pyran-2-ylmethyl)sulfonyl]pyrrolidin-3-amine

The title compound was prepared similarly to the procedure described in Example 474B substituting (tetrahydro-2H-pyran-2-yl)methanesulfonyl chloride for 2,4-dimethylthiazole-5-sulfonyl chloride. MS (ESI) *m*/*z* 437 (M+H)⁺.

### Reference Example 499

### N-[5-({3-[(3,4-difluorophenyl)amino]-4-phenylpyrrolidin-1-yl}sulfonyl)-1,3-thiazol-2-yl]acetamide

The title compound was prepared similarly to the procedure described in Example 474B substituting 2-acetamidothiazole-5-sulfonyl chloride for 2,4-dimethylthiazole-5-sulfonyl chloride. MS (ESI) *m*/*z* 479 (M+H)⁺.

### Reference Example 500

### N-(3,4-difluorophenyl)-4-phenyl-1-{[6-(trifluoromethyl)pyridin-3-yl]sulfonyl}pyrrolidin-3-amino

The title compound was prepared similarly to the procedure described in Example 474B substituting 6-(trifluoromethyl)pyridine-3-sulfonyl chloride for 2,4-dimethylthiazole-5-sulfonyl chloride. MS (ESI) *m*/*z* 484 (M+H)⁺.

### Reference Example 501

### 6-({3-[(3,4-difluorophenyl)amino]-4-phenylpyrrolidin-1-yl}sulfonyl)-1,4-dihydroquinoxaline-2,3-dione

The title compound was prepared similarly to the procedure described in Example 474B substituting 2,3-dioxo-1,2,3,4-tetrahydroquinoxaline-6-sulfonyl chloride for 2,4-dimethylthiazole-5-sulfonyl chloride. MS (ESI) *m*/*z* 499 (M+H)⁺.

### Example 502

### N-(3,4-difluorophenyl)-1-[(1-methyl-1H-pyrazol-3-yl)sulfonyl]-4-phenylpyrrolidin-3-amine

The title compound was prepared similarly to the procedure described in Example 474B substituting 1-methyl-1H-pyrazole-3-sulfonyl chloride for 2,4-dimethylthiazole-5-sulfonyl chloride. MS (ESI) *m*/*z* 419 (M+H)⁺.

### Reference Example 503

### N-(3,4-difluorophenyl)-1-[(1-methyl-1H-indol-5-yl)sulfonyl]-4-phenylpyrrolidin-3-amine

The title compound was prepared similarly to the procedure described in Example 474B substituting 1-methyl-1H-indole-5-sulfonyl chloride for 2,4-dimethylthiazole-5-sulfonyl chloride. MS (ESI) *m*/*z* 468 (M+H)⁺.

### Reference Example 504

### methyl 5-({3-[(3,4-difluorophenyl)amino]-4-phenylpyrrolidin-1-yl}sulfonyl)-1-methyl-1H-pyrrole-2-carboxylate

The title compound was prepared similarly to the procedure described in Example 474B substituting methyl 5-(chlorosulfonyl)-1-methyl-1H-pyrrole-2-carboxylate 2,4-dimethylthiazole-5-sulfonyl chloride. MS (ESI) *m*/*z* 476 (M+H)⁺.

### Reference Example 505

### 1-(1,2-benzoxazol-5-ylsulfonyl)-N-(3,4-difluorophenyl)-4-phenylpyrrolidin-3-amine

The title compound was prepared similarly to the procedure described in Example 474B substituting benzo[d]isoxazole-5-sulfonyl chloride for 2,4-dimethylthiazole-5-sulfonyl chloride. MS (ESI) *m*/*z* 456 (M+H)⁺.

### Reference Example 506

### 5-({3-[(3,4-difluorophenyl)amino]-4-phenylpyrrolidin-1-yl}sulfonyl)-1,3-dihydro-2H-benzimidazol-2-one

The title compound was prepared similarly to the procedure described in Example 474B substituting 2-oxo-2,3-dihydro-1H-benzo[d]imidazole-5-sulfonyl chloride for 2,4-dimethylthiazole-5-sulfonyl chloride. MS (ESI) *m*/*z* 471 (M+H)⁺.

### Reference Example 507

### 5-({3-[(3,4-difluorophenyl)amino]-4-phenylpyrrolidin-1-yl}sulfonyl)-1,3-dihydro-2H-indol-2-one

The title compound was prepared similarly to the procedure described in Example 474B substituting 2-oxoindoline-5-sulfonyl chloride for 2,4-dimethylthiazole-5-sulfonyl chloride. MS (ESI) *m*/*z* 470 (M+H)⁺.

### Example 508

### N-(3,4-difluorophenyl)-4-phenyl-1-[(1,3,5-trimethyl-1H-pyrazol-4-yl)sulfonyl]pyrrolidin-3-amine

The title compound was prepared similarly to the procedure described in Example 474B substituting 1,3,5-trimethyl-1H-pyrazole-4-sulfonyl chloride for 2,4-dimethylthiazole-5-sulfonyl chloride. MS (ESI) *m*/*z* 447(M+H)⁺.

### Reference Example 509

### 1-(cyclobutylsulfonyl)-N-(3,4-difluorophenyl)-4-phenylpyrrolidin-3-amine

The title compound was prepared similarly to the procedure described in Example 474B substituting cyclobutanesulfonyl chloride for 2,4-dimethylthiazole-5-sulfonyl chloride. MS (ESI) *m*/*z* 393 (M+H)⁺.

### Reference Example 510

### 1-(cyclohexylsulfonyl)-N-(3,4-difluorophenyl)-4-phenylpyrrolidin-3-amine

The title compound was prepared similarly to the procedure described in Example 474B substituting cyclohexanesulfonyl chloride for 2,4-dimethylthiazole-5-sulfonyl chloride. MS (ESI) *m*/*z* 421 (M+H)⁺.

### Reference Example 511

### 1-[(5-chlorothiophen-2-yl)sulfonyl]-N-{3,4-difluorophenyl}-4-phenylpyrrolidin-3-amine

The title compound was prepared similarly to the procedure described in Example 474B substituting 5-chlorothiophene-2-sulfonyl chloride for 2,4-dimethylthiazole-5-sulfonyl chloride. MS (ESI) *m*/*z* 455 (M+H)⁺.

### Reference Example 512

### 6-({3-[(3,4-difluorophenyl)aminol-4-phenylpyrrolidin-1-yl}sulfonyl)-3,4-dihydroquinolin-2(1H)-one

The title compound was prepared similarly to the procedure described in Example 474B substituting 2-oxo-1,2,3,4-tetrahydroquinoline-6-sulfonyl chloride for 2,4-dimethylthiazole-5-sulfonyl chloride. MS (ESI) *m*/*z* 484 (M+H)⁺.

### Reference Example 513

### N-(3,4-difluorophenyl)-1-[(3,5-dimethyl-1,2-oxazol-4-yl)sulfonyl]-4-phenylpyrrolidin-3-amine

The title compound was prepared similarly to the procedure described in Example 474B substituting 3,5-dimethylisoxazole-4-sulfonyl chloride for 2,4-dimethylthiazole-5-sulfonyl chloride. MS (ESI) *m*/*z* 434 (M+H)⁺.

### Reference Example 514

### 5-({3-[(3,4-difluorophenyl)amino]-4-phenylpyrrolidin-1-yl}sulfonyl)-1,3-benzoxazol-2(3H)-one

The title compound was prepared similarly to the procedure described in Example 474B substituting 2-oxo-2,3-dihydrobenzo[d]oxazole-5-sulfonyl chloride for 2,4-dimethylthiazole-5-sulfonyl chloride. MS (ESI) *m*/*z* 472 (M+H)⁺.

### Reference Example 515

### N-(3,4-difluorophenyl)-1-(isoquinolin-5-ylsulfonyl)-4-phenylpyrrolidin-3-amine

The title compound was prepared similarly to the procedure described in Example 474B substituting isoquinoline-5-sulfonyl chloride for 2,4-dimethylthiazole-5-sulfonyl chloride. MS (ESI) *m*/*z* 466 (M+H)⁺.

### Example 516

### N-(3,4-difluorophenyl)-1-[(1-methyl-1H-pyrazol-5-yl)sulfonyl]-4-phenylpyrrolidin-3-amine

The title compound was prepared similarly to the procedure described in Example 474B substituting 1-methyl-1H-pyrazole-5-sulfonyl chloride for 2,4-dimethylthiazole-5-sulfonyl chloride. MS (ESI) *m*/*z* 419 (M+H)⁺.

### Reference Example 517

### N-(3,4-difluorophenyl)-1-[(2,5-dimethylthiophen-3-yl)sulfonyl]-4-phenylpyrrolidin-3-amine

The title compound was prepared similarly to the procedure described in Example 474B substituting 2,5-dimethylthiophene-3-sulfonyl chloride for 2,4-dimethylthiazole-5-sulfonyl chloride. MS (ESI) *m*/*z* 449 (M+H)⁺.

### Reference Example 518

### N-(3,4-difluorophenyl)-1-[(5-methylthiophen-2-yl)sulfonyl]-4-phenylpyrrolidin-3-amine

The title compound was prepared similarly to the procedure described in Example 474B substituting 5-methylthiophene-2-sulfonyl chloride for 2,4-dimethylthiazole-5-sulfonyl chloride. MS (ESI) *m*/*z* 435 (M+H)⁺.

### Reference Example 519

### N-(3,4-difluorophenyl)-1-(2,3-dihydro-1-benzofuran-5-ylsulfonyl)-4-phenylpyrrolidin-3-amine

The title compound was prepared similarly to the procedure described in Example 474B substituting 2,3-dihydrobenzofuran-5-sulfonyl chloride for 2,4-dimethylthiazole-5-sulfonyl chloride. MS (ESI) *m*/*z* 457 (M+H)⁺.

### Reference Example 520

### 1-[(6-chloroimidazo[2,1-b][1,3]thiazol-5-yl)sulfonyl]-N-(3,4-difluorophenyl)-4-phenylpyrrolidin-3-amine

The title compound was prepared similarly to the procedure described in Example 474B substituting 6-chloroimidazo[2,1-b]thiazole-5-sulfonyl chloride for for 2,4-dimethylthiazole-5-sulfonyl chloride. MS (ESI) *m*/*z* 495 (M+H)⁺.

### Reference Example 521

### N-(3,4-difluorophenyl)-1-(morpholin-4-ylsulfonyl)-4-phenylpyrrolidin-3-amine

The title compound was prepared similarly to the procedure described in Example 474B substituting morpholine-4-sulfonyl chloride for for 2,4-dimethylthiazole-5-sulfonyl chloride. MS (ESI) *m*/*z* 424 (M+H)⁺.

### Reference Example 522

### 1-[5-({3-[(3,4-difluorophenyl)amino]-4-phenylpyrrolidin-1-yl}sulfonyl)-2,3-dihydro-1H-indol-1-yl]ethanone

The title compound was prepared similarly to the procedure described in Example 474B substituting 1-acetylindoline-5-sulfonyl chloride for 2,4-dimethylthiazole-5-sulfonyl chloride. MS (ESI) *m*/*z* 498 (M+H)⁺.

### Reference Example 523

### tert-butyl {trans-1-[(2-cyanophenyl)sulfonyl]-4-phenylpyrrolidin-3-yl}carbamate

### Example 523A

### Tert-butyl-trans-4-phenylpyrrolidin-3-ylcarbamate

The title compound was prepared similarly to the procedures described in Example 2A-2D substituting (E)-(2-nitrovinyl)benzene for trans-4-methoxy-beta-nitrostyrene.

### Example 523B

### Tert-butyl {trans-1-[(2-cyanophenyl)sulfonyl]-4-phenylpyrrolidin-3-yl}carbamate

The title compound was prepared similarly to the procedure described in Example 2E substituting Example 523A for Example 2D and substituting 2-cyanobenzene-1-sulfonyl chloride for 1-methyl-1H-imidazole-4-sulfonyl chloride. MS (DCI) *m*/*z* 444.8 (M+NH4)⁺.

### Reference Example 524

### tert-butyl [trans-1-[(2-cyanophenyl)sulfonyl]-4-(4-fluorophenyl)pyrrolidin-3-yl]carbamate

### Example 524A

### Tert-butyl-trans-4-(4-fluorophenyl)pyrrolidin-3-ylcarbamate

The title compound was prepared similarly to the procedures described in Example 2A-2D substituting (E)-1-fluoro-4-(2-nitrovinyl)benzene for trans-4-methoxy-beta-nitrostyrene.

### Example 524 B

### Tert-butyl [trans-1-[(2-cyanophenyl)sulfonyl]-4-(4-fluorophenyl)pyrrolidin-3-yl]carbamate

The title compound was prepared similarly to the procedure described in Example 2E substituting Example 524A for Example 2D and substituting 2-cyanobenzene-1-sulfonyl chloride for 1-methyl-1H-imidazole-4-sulfonyl chloride. MS (DCI) *m*/*z* 444.4 (M-H)⁻.

### Reference Example 525

### 2-{[trans-3-phenyl-4-{[4-(trifluoromethyl)pyridin-2-yl]amino}pyrrolidin-1-yl]sulfonyl}benzonitrile

The title compound was prepared similarly to the procedure described in Example 337G substituting Example 527 for Example 337F and substituting 2-bromo-4-(trifluoromethyl)pyridine for 1-bromo-3-(trifluoromethyl)benzene. MS (ESI) *m*/*z* 473.2 (M+H)⁺.

### Reference Example 526

### 2-{[trans-3-phenyl-4-{[4-(trifluoromethyl)pyridin-2-yl]amino}pyrrolidin-1-yl]sulfonyl}benzamide

The title compound was prepared similarly to the procedure described in Example 337G substituting Example 527 for Example 337F and substituting 2-bromo-4-(trifluoromethyl)pyridine for 1-bromo-3-(trifluoromethyl)benzene. MS (ESI) *m*/*z* 491.2 (M+H)⁺.

### Reference Example 527

### 2-(Trans-3-amino-4-phenylpyrrolidin-1-ylsulfonyl)benzonitrile

The title compound was prepared similarly to the procedure described in Example 380A substituting Example 523 for Example 322B. MS (ESI) MS *m*/*z* 328.2(M+H)⁺.

### Reference Example 528

### 2-({Trans-3-[(3-chlorophenyl)amino]-4-phenylpyrrolidin-1-yl}sulfonyl)benzonitrile

The title compound was prepared similarly to the procedure described in Example 337G substituting Example 527 for Example 337F and substituting 1-bromo-3-chlorobenzene for 1-bromo-3-(trifluoromethyl)benzene. MS (ESI) *m*/*z* 438.2 (M+H)⁺.

### Reference Example 529

### 2-({Trans-3-[(4-fluorophenyl)amino]-4-phenylpyrrolidin-1-yl}sulfonyl)benzonitrile

The title compound was prepared similarly to the procedure described in Example 337G substituting Example 527 for Example 337F and substituting 1-bromo-4-fluorobenzene for 1-bromo-3-(trifluoromethyl)benzene. MS (ESI) *m*/*z* 422.2 (M+H)⁺.

### Example 530

### Trans-N-(3-chlorophenyl)-4-(cyclopropylmethoxy)-1-[(1-methyl-1H-imidazol-4-yl)sulfonyl]pyrrolidin-3-amine

The title compound was prepared similarly to the procedure described in Example 400 substituting (bromomethyl)cyclopropane for benzyl bromide. MS (ESI) *m*/*z* 411/413 (3:1) (M+H)⁺.

### Example 531

### N-{trans-4-cyclohexyl-1-[(1-methyl-1H-imidazol-4-yl)sulfonyl]pyrrolidin-3-yl}-6-(trifluoromethyl)pyrimidin-4-amine

The title compound was prepared similarly to the procedure described in Example 425C substituting 4-bromo-6-trifluoromethylpyrimidine for 1-bromo-3-trifluoromethylbenzene. MS (ESI) *m*/*z* 459.4 (M+H)⁺.

### Example 532

### 2-methoxy-N-{(3R,4S)-1-[(1-methyl-1H-imidazol-4-yl)sulfonyl]-4-phenylpyrrolidin-3-yl}pyrimidin-4-amine

The title compound was prepared similarly to the procedure described in Example 337G substituting Example 396A for Example 337F and substituting 4-bromo-2-methoxypyrimidine for 1-bromo-3-(trifluoromethyl)benzene. MS (ESI) *m*/*z* 415.2 (M+H)⁺.

### Example 533

### (3R,4S)-1-[(1-methyl-1H-imidazol-4-yl)sulfonyl]-N-[(2-methyl-1,3-thiazol-4-yl)methyl]-4-phenylpyrrolidin-3-amine

The title compound was prepared similarly to the procedure described in Example 354E substituting 2-methylthiazole-4-carbaldehyde for 4-chlorobenzaldehyde and substituting Example 574A for Example 354D. MS (ESI) *m*/*z* 418 (M+H)⁺.

### Example 534

### (3R,4S)-N-[(2-ethyl-1-benzofuran-3-yl)methyl]-1-[(1-methyl-1H-imidazol-4-yl)sulfonyl]-4-phenylpyrrolidin-3-amine

The title compound was prepared similarly to the procedure described in Example 354E substituting 2-ethylbenzofuran-3-carbaldehyde for 4-chlorobenzaldehyde and substituting Example 574A for Example 354D. MS (ESI) *m*/*z* 465 (M+H)⁺.

### Example 535

### (3R,4S)-N-(imidazo[1,2-a]pyridin-8-ylmethyl)-1-[(1-methyl-1H-imidazol-4-yl)sulfonyl]-4-phenylpyrrolidin-3-amine

The title compound was prepared similarly to the procedure described in Example 354E substituting imidazo[1,2-a]pyridine-8-carbaldehyde for 4-chlorobenzaldehyde and substituting Example 574A for Example 354D. MS (ESI) *m*/*z* 437 (M+H)⁺.

### Example 536

### (3R,4S)-1-[(1-methyl-1H-imidazol-4-yl)sulfonyl]-N-[(5-methylpyridin-2-yl)methyl]-4-phenylpyrrolidin-3-amine

The title compound was prepared similarly to the procedure described in Example 354E substituting 5-methylpicolinaldehyde for 4-chlorobenzaldehyde and substituting Example 574A for Example 354D. MS (ESI) *m*/*z* 412 (M+H)⁺.

### Example 537

### (3R,4S)-1-[(1-methyl-1H-imidazol-4-yl)sulfonyl]-4-phenyl-N-(pyrazolo[1,5-a]pyridin-3-ylmethyl)pyrrolidin-3-amine

The title compound was prepared similarly to the procedure described in Example 354E substituting pyrazolo[1,5-a]pyridine-3-carbaldehyde for 4-chlorobenzaldehyde and substituting Example 574A for Example 354D. MS (ESI) *m*/*z* 437 (M+H)⁺.

### Example 538

### N-{Trans-4-hydroxy-1-[(1-methyl-1H-imidazol-4-yl)sulfonyl]-4-phenylpyrrolidin-3-yl}-4-(trifluoromethyl)benzamide

### Example 538A

### Trans-tert-butyl 3-hydroxy-4-(4-(trifluoromethyl)benzamido)pyrrolidine-1-carboxylate

To a solution of trans-tert-butyl 3-amino-4-hydroxypyrrolidine-1-carboxylate (600 mg, 3 mmol) in dichloromethane (10 mL) at 0 °C was added triethylamine (350 mg, 3.5 mmol) and 4-(trifluoromethyl)benzoyl chloride (620 mg, 3 mmol). The mixture was allowed to stir for 16 hours. The mixture was concentrated and dissolved in methanol (5 mL). Sodium methoxide (21% in methanol, 0.5 mL) was added. The mixture was stirred for 6 hours, concentrated, and partitioned between ethyl acetate and water. The organic fractions were collected, dried over MgSO₄, filtered, and concentrated to provide the title compound.

### Example 538B

### N-(trans-4-hydroxypyrrolidin-3-yl)-4-(trifluoromethyl)benzamide.

The title compound was prepared as the hydrochloride salt similarly to the procedure described in Example 373B substituting Example 538A for Example 373A.

### Example 538C

### N-(trans-4-hydroxy-1-((1-methyl-1H-imidazol-4-yl)sulfonyl)pyrrolidin-3-yl)-4-(trifluoromethyl)benzamide

The title compound was prepared similarly to the procedure described in Example 373C substituting Example 538B for Example 373B.

### Example 538D

### N-(1-((1-methyl-1H-imidazol-4-yl)sulfonyl)-4-oxopyrrolidin-3-yl)-4-(trifluoromethyl)benzamide

The title compound was prepared similarly to the procedure described in Example 382A substituting Example 538C for Example 379C.

### Example 538E

### N-{Trans-4-hydroxy-1-[(1-methyl-1H-imidazol-4-yl)sulfonyl]-4-phenylpyrrolidin-3-yl}-4-(trifluoromethyl)benzamide

The title compound was prepared similarly to the procedure described in Example 382B substituting Example 538D for Example 382A and phenylmagnesium bromide for benzylmagnesium bromide. MS (ESI) *m*/*z* 495 (M+H)⁺.

### Example 539

### 1-[(1-methyl-1H-imidazol-4-yl)sulfonyl]-3-phenyl-4-{[4-(trifluoromethyl)benzyl]amino}pyrrolidin-3-ol

The title compound was prepared similarly to the procedure described in Example 404B substituting Example 538E for Example 404A. MS (ESI) *m*/*z* 481 (M+H)⁺.

### Example 540

### (3R,4S)-N-[4-chloro-3-(trifluoromethyl)phenyl]-4-(4-fluorophenyl)-1-[(1-methyl-1H-imidazol-4-yl)sulfonyl]pyrrolidin-3-amine

The title compound was prepared similarly to the procedure described in Example 327 substituting 1-bromo-4-chloro-3-(trifluoromethyl)benzene for 1-bromo-3-(trifluoromethoxy)benzene, but only heating for 1 hour. MS (ESI) *m*/*z* 503 (M+H)⁺.

### Example 541

### ethyl {(3R,4S)-1-[(1-methyl-1H-imidazol-4-yl)sulfonyl]-4-phenylpyrrolidin-3-yl}carbamate

To Example 396A (50 mg, 0.16 mmol) in dichloromethane (0.5 mL) was added triethylamine (20 mg, 0.2 mmol) and ethyl chloroformate (22 mg, 0.2 mmol). The mixture was stirred for 1 hour, concentrated, and purified by HPLC to provide the title compound. MS (ESI) *m*/*z* 379 (M+H)⁺.

### Example 542

### propyl {(3R,4S)-1-[(1-methyl-1H-imidazol-4-yl)sulfonyl]-4-phenylpyrrolidin-3-yl}carbamate

The title compound was prepared similarly to the procedure described in Example 541 substituting propyl chloroformate for ethyl chloroformate. MS (ESI) *m*/*z* 393 (M+H)⁺.

### Example 543

### 2-methylpropyl {(3R,4S)-1-[(1-methyl-1H-imidazol-4-yl)sulfonyl]-4-phenylpyrrolidin-3-yl}carbamate

The title compound was prepared similarly to the procedure described in Example 541 substituting isobutyl chloroformate for ethyl chloroformate. MS (ESI) *m*/*z* 407 (M+H)⁺.

### Example 544

### butyl {(3R,4S)-1-[(1-methyl-1H-imidazol-4-yl)sulfonyl]-4-phenylpyrrolidin-3-yl}carbamate

The title compound was prepared similarly to the procedure described in Example 541 substituting butyl chloroformate for ethyl chloroformate. MS (ESI) *m*/*z* 407 (M+H)⁺.

### Example 545

### (3R,4S)-N-(2-cyclopropylethyl)-1-[(1-methyl-1H-imidazol-4-yl)sulfonyl]-4-phenylpyrrolidin-3-amine

The title compound was prepared similarly to the procedure described in Example 404B substituting Example 546 for Example 404A. MS (ESI) *m*/*z* 375 (M+H)⁺.

### Example 546

### 2-cyclopropyl-N-{(3R,4S)-1-[(1-methyl-1H-imidazol-4-yl)sulfonyl]-4-phenylpyrrolidin-3-yl}acetamide

The title compound was prepared similarly to the procedure described in Example 404A substituting Example 396A for Example 14A and substituting 2-cyclopropylacetic acid for 2-nitro-5-(trifluoromethyl)benzoic acid. MS (ESI) *m*/*z* 388 (M+H)⁺.

### Example 547

### (3R,4S)-N-[(2E)-hex-2-en-1-yl]-1-[(1-methyl-1H-imidazol-4-yl)sulfonyl]-4-phenylpyrrolidin-3-amine

The title compound was prepared similarly to the procedure described in Example 354E substituting (E)-hex-2-enal for 4-chlorobenzaldehyde and substituting Example 396A for Example 354D. Both Example 547 and Example 548 were isolated. MS (ESI) *m*/*z* 389 (M+H)⁺.

### Example 548

### (3R,4S)-N-hexyl-1-[(1-methyl-1H-imidazol-4-yl)sulfonyl]-4-phenylpyrrolidin-3-amine

The title compound was prepared similarly to the procedure described in Example 354E substituting (E)-hex-2-enal for 4-chlorobenzaldehyde and Example 396A for Example 354D. Both Example 547 and Example 548 were isolated. MS (ESI) *m*/*z* 391 (M+H)⁺.

### Example 549

### N-{(3R,4S)-1-[(1-methyl-1H-imidazol-4-yl)sulfonyl]-4-phenylpyrrolidin-3-yl}pentanamide

The title compound was prepared similarly to the procedure described in Example 404A substituting Example 396A for Example 14A and pentanoic acid for 2-nitro-5-(trifluoromethyl)benzoic acid. MS (ESI) *m*/*z* 391 (M+H)⁺.

### Example 550

### N-{(3R,4S)-1-[(1-methyl-1H-imidazol-4-yl)sulfonyl]-4-phenylpyrrolidin-3-yl}hexanamide

The title compound was prepared similarly to the procedure described in Example 404A substituting Example 396A for Example 14A and substituting hexanoic acid for 2-nitro-5-(trifluoromethyl)benzoic acid. MS (ESI) *m*/*z* 405 (M+H)⁺.

### Example 551

### (3R,4S)-N-(2-{[tert-butyl(dimethyl)silyl]oxy}ethyl)-1-[(1-methyl-1H-imidazol-4-yl)sulfonyl]-4-phenylpyrrolidin-3-amine

The title compound was prepared similarly to the procedure described in Example 354E substituting (tert-butyldimethylsilyloxy) acetaldehyde for 4-chlorobenzaldehyde and substituting Example 396A for Example 354D. MS (ESI) *m*/*z* 465.2 (M+H)⁺.

### Example 552

### (3R,4S)-1-[(1-methyl-1H-imidazol-4-yl)sulfonyl]-4-phenyl-N-1[6-(trifluoromethyl)pyridin-2-yl]methyl}pyrrolidin-3-amine

The title compound was prepared similarly to the procedure described in Example 354E substituting 6-(trifluoromethyl)picolinaldehyde for 4-chlorobenzaldehyde and substituting Example 574A for Example 354D. MS (ESI) *m*/*z* 466 (M+H)⁺.

### Example 553

### (3R,4S)-N-[(3-chlorothiophen-2-yl)methyl]-1-[(1-methyl-1H-imidazol-4-yl)sulfonyl]-4-phenylpyrrolidin-3-amine

The title compound was prepared similarly to the procedure described in Example 354E substituting 3-chlorothiophene-2-carbaldehyde for 4-chlorobenzaldehyde and substituting Example 574A for Example 354D. MS (ESI) *m*/*z* 437 (M+H)⁺.

### Example 554

### (3R,4S)-N-[(4,4-difluorocyclohexyl)methyl]-1-[(1-methyl-1H-imidazol-4-yl)sulfonyl]-4-phenylpyrrolidin-3-amine

The title compound was prepared similarly to the procedure described in Example 354E substituting 4, 4-difluorocyclohexanecarbaldehyde for 4-chlorobenzaldehyde and substituting Example 574A for Example 354D. MS (ESI) *m*/*z* 439 (M+H)⁺.

### Example 555

### (3R,4S)-1-[(1-methyl-1H-imidazol-4-yl)sulfonyl]-4-phenyl-N-(pyrazolo[1,5-a]pyridin-7-ylmethyl)pyrrolidin-3-amine

The title compound was prepared similarly to the procedure described in Example 354E substituting pyrazolo[1,5-a]pyridine-7-carbaldehyde for 4-chlorobenzaldehyde and Example 574A for Example 354D. MS (ESI) *m*/*z* 437 (M+H)⁺.

### Example 556

### (3R,4S)-1-[(1-methyl-1H-imidazol-4-yl)sulfonyl]-4-phenyl-N-{[4-(propan-2-yl)-1,3-thiazol-2-yl]methyl}pyrrolidin-3-amine

The title compound was prepared similarly to the procedure described in Example 354E substituting 4-isopropylthiazole-2-carbaldehyde for 4-chlorobenzaldehyde and substituting Example 574A for Example 354D. MS (ESI) *m*/*z* 446 (M+H)⁺.

### Example 557

### (3R,4S)-N-[(2,2-dimethyltetrahydro-2H-pyran-4-yl)methyl]-1-[(1-methyl-1H-imidazol-4-yl)sulfonyl]-4-phenylpyrrolidin-3-amine

The title compound was prepared similarly to the procedure described in Example 354E substituting 2,2-dimethyltetrahydro-2H-pyran-4-carbaldehyde for 4-chlorobenzaldehyde and substituting Example 574A for Example 354D. MS (ESI) *m*/*z* 433 (M+H)⁺.

### Example 558

### (3R,4S)-1-[(1-methyl-1H-imidazol-4-yl)sulfonyl]-4-phenyl-N-{[5-(tetrahydro-2H-pyran-2-yl)thiophen-2-yl]methyl}pyrrolidin-3-amine

The title compound was prepared similarly to the procedure described in Example 354E substituting 5-(tetrahydro-2H-pyran-2-yl)thiophene-2-carbaldehyde for 4-chlorobenzaldehyde and substituting Example 574A for Example 354D. MS (ESI) *m*/*z* 487 (M+H)⁺.

### Example 559

### (3R,4S)-N-[(5-bromo-4-methyl-1,3-thiazol-2-yl)methyl]-1-[(1-methyl-1H-imidazol-4-yl)sulfonyl]-4-phenylpyrrolidin-3-amine

The title compound was prepared similarly to the procedure described in Example 354E substituting 5-bromo-4-methylthiazole-2-carbaldehyde for 4-chlorobenzaldehyde and substituting Example 574A for Example 354D. MS (ESI) *m*/*z* 497 (M+H)⁺.

### Example 560

### (3R,4S)-N-(imidazo[1,5-a]pyridin-5-ylmethyl)-1-[(1-methyl-1H-imidazol-4-yl)sulfonyl]-4-phenylpyrrolidin-3-amine

The title compound was prepared similarly to the procedure described in Example 354E substituting imidazo[1,5-a]pyridine-5-carbaldehyde for 4-chlorobenzaldehyde and substituting Example 574A for Example 354D. MS (ESI) *m*/*z* 437 (M+H)⁺.

### Example 561

### (3R,4S)-N-(2,3-dimethylpentyl)-1-[(1-methyl-1H-imidazol-4-yl)sulfonyl]-4-phenylpyrrolidin-3-amine

The title compound was prepared similarly to the procedure described in Example 354E substituting 2,3-dimethylpentanal for 4-chlorobenzaldehyde and substituting Example 574A for Example 354D. MS (ESI) *m*/*z* 405 (M+H)⁺.

### Example 562

### (3R,4S)-N-[(2-chloro-1,3-thiazol-4-yl)methyl]-1-[(1-methyl-1H-imidazol-4-yl)sulfonyl]-4-phenylpyrrolidin-3-amine

The title compound was prepared similarly to the procedure described in Example 354E substituting 2-chlorothiazole-4-carbaldehyde for 4-chlorobenzaldehyde and substituting Example 574A for Example 354D. MS (ESI) *m*/*z* 438 (M+H)⁺.

### Example 563

### (3R,4S)-1-[(1-methyl-1H-imidazol-4-yl)sulfonyl]-N-[(5-methylimidazo[1,2-a]pyridin-3-yl)methyl]-4-phenylpyrrolidin-3-amine

The title compound was prepared similarly to the procedure described in Example 354E substituting 5-methylimidazo[1,2-a]pyridine-3-carbaldehyde for 4-chlorobenzaldehyde and substituting Example 574A for Example 354D. MS (ESI) *m*/*z* 451 (M+H)⁺.

### Example 564

### (3R,4S)-1-[(1-methyl-1H-imidazol-4-yl)sulfonyl]-4-phenyl-N-[(2-phenylcyclopropyl)methyl]pyrrolidin-3-amine

The title compound was prepared similarly to the procedure described in Example 354E substituting 2-phenylcyclopropanecarbaldehyde for 4-chlorobenzaldehyde and substituting Example 574A for Example 354D. MS (ESI) *m*/*z* 437 (M+H)⁺.

### Example 565

### (3R,4S)-N-[(4-chloro-1,3-thiazol-5-yl)methyl]-1-[(1-methyl-1H-imidazol-4-yl)sulfonyl]-4-phenylpyrrolidin-3-amine

The title compound was prepared similarly to the procedure described in Example 354E substituting 4-chlorothiazole-5-carbaldehyde for 4-chlorobenzaldehyde and substituting Example 574A for Example 354D. MS (ESI) *m*/*z* 438 (M+H)⁺.

### Example 566

### (3R,4S)-1-[(1-methyl-1H-imidazol-4-yl)sulfonyl]-4-phenyl-N-(3,3,3-trifluoro-2-methylpropyl)pyrrolidin-3-amine

The title compound was prepared similarly to the procedure described in Example 354E substituting 3,3,3-trifluoro-2-methylpropanal for 4-chlorobenzaldehyde and substituting Example 574A for Example 354D. MS (ESI) *m*/*z* 417 (M+H)⁺.

### Example 567

### (3R,4S)-N-[(2-chloro-3-fluoropyridin-4-yl)methyl]-1-[(1-methyl-1H-imidazol-4-yl)sulfonyl]-4-phenylpyrrolidin-3-amine

The title compound was prepared similarly to the procedure described in Example 354E substituting 2-chloro-3-fluoroisonicotinaldehyde for 4-chlorobenzaldehyde and substituting Example 574A for Example 354D. MS (ESI) *m*/*z* 450 (M+H)⁺.

### Example 568

### (3R,4S)-N-[(5-fluoro-1-benzothiophen-2-yl)methyl]-1-[(1-methyl-1H-imidazol-4-yl)sulfonyl]-4-phenylpyrrolidin-3-amine

The title compound was prepared similarly to the procedure described in Example 354E substituting 5-fluorobenzo[b]thiophene-2-carbaldehyde for 4-chlorobenzaldehyde and substituting Example 574A for Example 354D. MS (ESI) *m*/*z* 471 (M+H)⁺.

### Example 569

### (3R,4S)-N-[5-chloro-2-(difluoromethoxy)benzyl]-1-[(1-methyl-1H-imidazol-4-yl)sulfonyl]-4-phenylpyrrolidin-3-amine

The title compound was prepared similarly to the procedure described in Example 354E substituting 5-chloro-2-(difluoromethoxy)benzaldehyde for 4-chlorobenzaldehyde and substituting Example 574A for Example 354D. MS (ESI) *m*/*z* 497 (M+H)⁺.

### Example 570

### (3R,4S)-N-[(2,5-dichlorothiophen-3-yl)methyl]-1-[(1-methyl-1H-imidazol-4-yl)sulfonyl]-4-phenylpyrrolidin-3-amine

The title compound was prepared similarly to the procedure described in Example 354E substituting 2,5-dichlorothiophene-3-carbaldehyde for 4-chlorobenzaldehyde and substituting Example 574A for Example 354D. MS (ESI) *m*/*z* 471 (M+H)⁺.

### Example 571

### (3R,4S)-N-{[2-(4-fluorophenyl)pyridin-3-yl]methyl}-1-[(1-methyl-1H-imidazol-4-yl)sulfonyl]-4-phenylpyrrolidin-3-amine

The title compound was prepared similarly to the procedure described in Example 354E substituting 2-(4-fluorophenyl)nicotinaldehyde for 4-chlorobenzaldehyde and substituting Example 574A for Example 354D. MS (ESI) *m*/*z* 492 (M+H)⁺.

### Example 572

### (3R,4S)-1-[(1-methyl-1H-imidazol-4-yl)sulfonyl]-N-{[2-(4-methylphenyl)-1,3-thiazol-5-yl]methyl}-4-phenylpyrrolidin-3-amine

The title compound was prepared similarly to the procedure described in Example 354E substituting 2-(p-tolyl)thiazole-5-carbaldehyde for 4-chlorobenzaldehyde and substituting Example 574A for Example 354D. MS (ESI) *m*/*z* 494 (M+H)⁺.

### Example 573

### (3R,4S)-N-(3-cyclopropylpropyl)-1-[(1-methyl-1H-imidazol-4-yl)sulfonyl]-4-phenylpyrrolidin-3-amine

### Example 573A

### 3-cyclopropyl-N-((3R,4S)-1-((1-methyl-1H-imidazol-4-yl)sulfonyl)-4-phenylpyrrolidin-3-yl)propanamide

The title compound was prepared similarly to the procedure described in Example 404A substituting Example 396A for Example 14A and substituting 3-cyclopropylpropanoic acid for 2-nitro-5 -(trifluoromethyl)benzoic acid.

### Example 573B

### (3R,4S)-N-(3-cyclopropylpropyl)-1-[(1-methyl-1H-imidazol-4-yl)sulfonyl]-4-phenylpyrrolidin-3-amine

The title compound was prepared similarly to the procedure described in Example 404B substituting Example 573A for Example 404A. MS (ESI) *m*/*z* 389 (M+H)⁺.

### Example 574

### 5-fluoro-4-methyl-N-{(3R,4S)-1-[(1-methyl-1H-imidazol-4-yl)sulfonyl]-4-phenylpyrrolidin-3-yl}pyridin-2-amine

### Example 574A

### (3R,4S)-1-(1-methyl-1H-imidazol-4-ylsulfonyl)-4-phenylpyrrolidin-3-amine

The title compound was prepared as an HCl salt similarly to the conditions described in Example 2A-2F substituting (E)-4-(2-nitrovinyl)benzene for (E)-1-methoxy-4-(2-nitrovinyl)benzene and separating the enantiomers produced from the procedure of Example 2C via Chiral SFC separation (SFC 200 Column: Whelk-O1, 50 × 250 mm, 5 µm Column Temperature: 35 °C Mobile Phase: CO₂/IPA/DEA = 70/30/0.1 Flow rate: 70 g/min Back Pressure: 100 Bar Wavelength: 214 nm Cycle time: 5.0 min Injection: 4.0 mL Sample solution: 41000 mg in 250 mL methanol) The enantiomer with a retention time of 4.41 minutes was used to make the title compound.

### Example 574B

### 5-fluoro-4-methyl-N-{(3R,4S)-1-[(1-methyl-1H-imidazol-4-yl)sulfonyl]-4-phenylpyrrolidin-3-yl}pyridin-2-amine

The title compound was prepared similarly to the procedure described in Example 327 substituting Example 574A for Example 322B and substituting 2-bromo-4-methyl-5-fluoropyridine for 1-bromo-3-(trifluoromethoxy)benzene. MS (APCI) *m*/*z* 416 (M+H)⁺.

### Example 575

### 1,5-dimethyl-N-{(3R,4S)-1-[(1-methyl-1H-imidazol-4-yl)sulfonyl]-4-phenylpyrrolidin-3-yl}-1H-1,2,4-triazol-3-amine

The title compound was prepared similarly to the procedure described in Example 327 substituting Example 574A for Example 322B and substituting 3-bromo-1,5-dimethyl-1H-1,2,4-triazole for 1-bromo-3-(trifluoromethoxy)benzene. MS (ESI) *m*/*z* 402 (M+H)⁺.

### Example 576

### 1,5-dimethyl-4-({(3R,4S)-1-[(1-methyl-1H-imidazol-4-yl)sulfonyl]-4-phenylpyrrolidin-3-yl}amino)-1H-pyrrole-2-carbonitrile

The title compound was prepared similarly to the procedure described in Example 327 substituting Example 574A for Example 322B and substituting 4-bromo-1,5-dimethyl-1H-pyrrole-2-carbonitrile for 1-bromo-3-(trifluoromethoxy)benzene. MS (APCI) *m*/*z* 425 (M+H)⁺.

### Example 577

### (3S,4R)-4-(4-fluorophenyl)-1-[(1-methyl-1H-imidazol-4-yl)sulfonyl]-N-[3-(propan-2-yloxy)propyl]pyrrolidine-3-carboxamide

The title compound was prepared similarly to the procedure described in Example 242E substituting 3-isopropoxypropylamine for aniline. MS (ESI) *m*/*z* 453.2 (M+H)⁺.

### Example 578

### (3S,4R)-N-(3-ethoxypropyl)-4-(4-fluorophenyl)-1-[(1-methyl-1H-imidazol-4-yl)sulfonyl]pyrrolidine-3-carboxamide

The title compound was prepared similarly to the procedure described in Example 242E substituting 3-ethoxypropylamine for aniline. MS (ESI) *m*/*z* 439.2 (M+H)⁺.

### Example 579

### (3S,4R)-4-(4-fluorophenyl)-N-(2-methoxyethyl)-1-[(1-methyl-1H-imidazol-4-yl)sulfonyl]pyrrolidine-3-carboxamide

The title compound was prepared similarly to the procedure described in Example 242E substituting 2-methoxyethylamine for aniline. MS (ESI) *m*/*z* 411.2 (M+H)⁺.

### Example 580

### (3S,4R)-4-(4-fluorophenyl)-1-[(1-methyl-1H-imidazol-4-yl)sulfonyl]-N-propylpyrrolidine-3-carboxamide

The title compound was prepared similarly to the procedure described in Example 242E substituting propylamine for aniline. MS (ESI) *m*/*z* 395.2 (M+H)⁺.

### Example 581

### (3S,4R)-N-butyl-4-(4-fluorophenyl)-1-[(1-methyl-1H-imidazol-4-yl)sulfonyl]pyrrolidine-3-carboxamide

The title compound was prepared similarly to the procedure described in Example 242E substituting butylamine for aniline. MS (ESI) *m*/*z* 409.2 (M+H)⁺.

### Example 582

### (3S,4R)-4-(4-fluorophenyl)-N-[1-methoxypropan-2-yl]-1-[(1-methyl-1H-imidazol-4-yl)sulfonyl]pyrrolidine-3-carboxamide

The title compound was prepared similarly to the procedure described in Example 242E substituting 2-methoxyisopropylamine for aniline. MS (ESI) *m*/*z* 425.2 (M+H)⁺.

### Example 583

### (3R,4S)-4-(4-fluorophenyl)-N-(3-methoxypropyl)-1-[(1-methyl-1H-imidazol-4-yl)sulfonyl]pyrrolidine-3-carboxamide

The title compound was prepared similarly to the procedure described in Example 242E substituting 3-methoxypropylamine for aniline. MS (ESI) *m*/*z* 425.2 (M+H)⁺.

### Example 584

### (3S,4R)-N-(2-ethoxyethyl)-4-(4-fluorophenyl)-1-[(1-methyl-1H-imidazol-4-yl)sulfonyl]pyrrolidine-3-carboxamide

The title compound was prepared similarly to the procedure described in Example 242E substituting 2-ethoxyethylamine for aniline. MS (ESI) *m*/*z* 425.2 (M+H)⁺.

### Example 585

### (3R,4S)-N-[4-chloro-3-(trifluoromethoxy)phenyl]-4-(4-fluorophenyl)-1-[(1-methyl-1H-imidazol-4-yl)sulfonyl]pyrrolidin-3-amine

The title compound was prepared similarly to Example 327 substituting 1-bromo-4-chloro-3-(trifluoromethoxy)benzene for 1-bromo-3-(trifluoromethyl)benzene, but the reaction mixture was only heated for 1 hour. MS (APCI) *m*/*z* 519 (M+H)⁺.

### Example 586

### (3S,4R)-N-(cyclopropylmethyl)-4-(4-fluorophenyl)-1-[(1-methyl-1H-imidazol-4-yl)sulfonyl]pyrrolidine-3-carboxamide

The title compound was prepared similarly to the procedure described in Example 242E substituting cyclopropylmethylamine for aniline. MS (ESI) *m*/*z* 407.1 (M+H)⁺.

### Example 587

### (3S,4R)-4-(4-fluorophenyl)-1-[(1-methyl-1H-imidazol-4-yl)sulfonyl]-N-[2-(propan-2-yloxy)ethyl]pyrrolidine-3-carboxamide

The title compound was prepared similarly to the procedure described in Example 242E substituting 2-isopropoxy-ethylamine for aniline. MS (ESI) *m*/*z* 439.1 (M+H)⁺.

### Example 588

### (3R,4S)-N-(3-methoxypropyl)-1-[(1-methyl-1H-imidazol-4-yl)sulfonyl]-4-phenylpyrrolidin-3-amine

### Example 588A

### (3R,4S)-N-(3-(benzyloxy)propyl)-1-((1-methyl-1H-imidazol-4-yl)sulfonyl)-4-phenylpyrrolidin-3-amine

The title compound was prepared similarly to the procedure described in Example 354E substituting Example 396A for Example 354D and substituting 3-(benzyloxy)propanal for 4-chlorobenzaldehyde.

### Example 588B

### tert-butyl (3-(benzyloxy)propyl)((3R,4S)-1-((1-methyl-1H-imidazol-4-yl)sulfonyl)-4-phenylpyrrolidin-3-yl)carbamate

The title compound was prepared similarly to the procedure described in Example 337C substituting Example 588A for Example 337B.

### Example 588C

### tert-butyl (3-hydroxypropyl)((3R,4S)-1-((1-methyl-1H-imidazol-4-yl)sulfonyl)-4-phenylpyrrolidin-3-yl)carbamate

To Example 588B (200 mg, 0.36 mmol) in tetrahydrofuran (10 mL) was added 20% palladium hydroxide on carbon, (80 mg, 0.058 mmol). The mixture was allowed to stir under 30 psi of hydrogen at 50 °C for 16 hours. The mixture was filtered through a nylon membrane and concentrated to provide the title compound.

### Example 588D

### (3R,4S)-N-(3-methoxypropyl)-1-[(1-methyl-1H-imidazol-4-yl)sulfonyl]-4-phenylpyrrolidin-3-amine

To Example 588C (60 mg, 0.13 mmol) in tetrahydrofuran (0.5 mL) was added iodomethane (70 mg, 0.5 mmol) and sodium hydride (60%, 20 mg, 0.5 mmol). The mixture was stirred at 60 °C for 2 hours. Hydrochloric acid (4N in dioxane, 1 mL) was added. The mixture was stirred for 2 hours, concentrated, and partitioned between ethyl acetate and NaOH (1 M aq). The organic fraction was collected, concentrated, and purified HPLC to provide the title compound. MS (ESI) *m*/*z* 379 (M+H)⁺.

### Example 589

### (3R,4S)-N-(3-ethoxypropyl)-1-[(1-methyl-1H-imidazol-4-yl)sulfonyl]-4-phenylpyrrolidin-3-amine

The title compound was prepared similarly to the procedure described in Example 588D substituting iodoethane for ioidomethane. MS (ESI) *m*/*z* 393 (M+H)⁺.

### Example 590

### (3R,4S)-N-{[1-(methoxymethyl)cyclopropyl]methyl}-1-[(1-methyl-1H-imidazol-4-yl)sulfonyl]-4-phenylpyrrolidin-3-amine

### Example 590A

### 1-(methoxymethyl)-N-((3R,4S)-1-((1-methyl-1H-imidazol-4-yl)sulfonyl)-4-phenylpyrrolidin-3-yl)cyclopropanecarboxamide

The title compound was prepared similarly to the procedure described in Example 404A substituting Example 396A for Example 14A and substituting 1-(methoxymethyl)cyclopropanecarboxylic acid for 2-nitro-5-(trifluoromethyl)benzoic acid.

### Example 590

### (3R,4S)-N-{[1-(methoxymethyl)cyclopropyl]methyl}-1-[(1-methyl-1H-imidazol-4-yl)sulfonyl]-4-phenylpyrrolidin-3-amine

The title compound was prepared similarly to the procedure described in Example 404B substituting Example 590A for Example 404A. MS (ESI) *m*/*z* 405 (M+H)⁺.

### Example 591

### N-({trans-4-(4-fluorophenyl)-1-[(1-methyl-1H-imidazol-4-yl)sulfonyl]pyrrolidin-3-yl}methyl)-3-(propan-2-yloxy)propan-1-amine

The title compound was prepared similarly to the procedure described in Example 290 substituting Example 577 for Example 242E. MS (ESI) *m*/*z* 439.1 (M+H)⁺.

### Example 592

### 3-ethoxy-N-({trans-4-(4-fluorophenyl)-1-[(1-methyl-1H-imidazol-4-yl)sulfonyl]pyrrolidin-3-yl}methyl)propan-1-amine

The title compound was prepared similarly to the procedure described in Example 290 substituting Example 578 for Example 242E. MS (ESI) *m*/*z* 425.1 (M+H)⁺.

### Example 593

### N-({trans-4-(4-fluorophenyl)-1-[(1-methyl-1H-imidazol-4-yl)sulfonyl]pyrrolidin-3-yl}methyl)-2-methoxyethanamine

The title compound was prepared similarly to the procedure described in Example 290 substituting Example 579 for Example 242E. MS (ESI) *m*/*z* 397.1 (M+H)⁺.

### Example 594

### N-({trans-4-(4-fluorophenyl)-1-[(1-methyl-1H-imidazol-4-yl)sulfonyl]pyrrolidin-3-yl}methyl)propan-1-amine

The title compound was prepared similarly to the procedure described in Example 290 substituting Example 580 for Example 242E. MS (ESI) *m*/*z* 381.1 (M+H)⁺.

### Example 595

### N-({trans-4-(4-fluorophenyl)-1-[(1-methyl-1H-imidazol-4-yl)sulfonyl]pyrrolidin-3-yl}methyl)butan-1-amine

The title compound was prepared similarly to the procedure described in Example 290 substituting Example 581 for Example 242E. MS (ESI) *m*/*z* 394.1 (M+H)⁺.

### Example 596

### (2S)-N-({trans-4-(4-fluorophenyl)-1-[(1-methyl-1H-imidazol-4-yl)sulfonyl]pyrrolidin-3-yl}methyl)-1-methoxypropan-2-amine

The title compound was prepared similarly to the procedure described in Example 290 substituting Example 582 for Example 242E. MS (ESI) *m*/*z* 411.1 (M+H)⁺.

### Example 597

### N-({trans-4-(4-fluorophenyl)-1-[(1-methyl-1H-imidazol-4-yl)sulfonyl]pyrrolidin-3-yl}methyl)-3-methoxypropan-1-amine

The title compound was prepared similarly to the procedure described in Example 290 substituting Example 583 for Example 242E. MS (ESI) *m*/*z* 411.1 (M+H)⁺.

### Example 598

### 2-ethoxy-N-({trans-4-(4-fluorophenyl)-1-[(1-methyl-1H-imidazol-4-yl)sulfonyl]pyrrolidin-3-yl}methyl)ethanamine

The title compound was prepared similarly to the procedure described in Example 290 substituting Example 584 for Example 242E. MS (ESI) *m*/*z* 411.1 (M+H)⁺.

### Example 599

### N-({trans-4-(4-fluorophenyl)-1-[(1-methyl-1H-imidazol-4-yl)sulfonyl]pyrrolidin-3-yl}methyl)-2-(propan-2-yloxy)ethanamine

The title compound was prepared similarly to the procedure described in Example 290 substituting Example 587 for Example 242E. MS (ESI) *m*/*z* 425.1 (M+H)⁺.

### Example 600

### (3R,4S)-N-(2-methoxyethyl)-1-[(1-methyl-1H-imidazol-4-yl)sulfonyl]-4-phenylpyrrolidin-3-amine

### Example 600A

### Tert-butyl 2-(tert-butyldimethylsilyloxy)ethyl((3R,4S)-1-(1-methyl-1H-imidazol-4-ylsulfonyl)-4-phenylpyrrolidin-3-yl)carbamate

To a solution of Example 551 (686 mg, 1.48 mmol) in ethyl acetate (7 mL), was added di-tert-butyldicarbonate (644 mg, 2.95 mmol). This solution was stirred at room temperature for 16 hours. The solution was then concentrated and purified by flash-chromatography (30-50% ethyl acetate/heptanes) to provide the title compound. MS (ESI) *m*/*z* 565.0 (M+H)⁺.

### Example 600 B

### Tert-butyl 2-hydroxyethyl((3R,4S)-1-(1-methyl-1H-imidazol-4-ylsulfonyl)-4-phenylpyrrolidin-3-yl)carbamate

To a solution of Example 600A (626 mg, 1.11 mmol) in 8 mL of dichloromethane (5 mL) was added tetrabutylammonium fluoride hydrate (620 mg, 2.22 mmol). This solution was stirred at room temperature for 16 hours, concentrated, and purified by flash-chromatography (5% methanol/dichloromethane with 0.5% triethylamine added) to provide the title compound. MS (ESI) *m*/*z* 450.8 (M+H)⁺.

### Example 600 C

### (3R,4S)-N-(2-methoxyethyl)-1-[(1-methyl-1H-imidazol-4-yl)sulfonyl]-4-phenylpyrrolidin-3-amine

Powdered potassium hydroxide (30 mg, 0.53 mmol) was added dimethylsulfoxide (7 mL) and the mixture was stirred for 2 minutes. To this solution was added Example 600 B (60 mg, 0.13 mmol) and iodomethane (66.2 mg, 0.47 mmol). This mixture was stirred at room temperature for 16 hours then partitioned between ethyl acetate and water, The organic fraction was collected, and the aqueous fraction was washed with ethyl acetate 3 times. All organic fractions were combined and concentrated. To this concentratewas added dichloromethane (1 mL) and trifluoroacetic acid (0.3 mL), and the solution stirred at room temperature for 2 hours. The mixture was concentrated and purified by HPLC to provide the title compound. MS (ESI) *m*/*z* 365.1 (M+H)⁺.

### Example 601

### 1-[(1-methyl-1H-imidazol-4-yl)sulfonyl]-4-(tetrahydrofuran-2-yl)-N-[3-(trifluoromethoxy)phenyl]pyrrolidin-3-amine

### Example 601A

### (E)-2-(2-nitrovinyl)tetrahydrofuran

A solution of terahydrofuran-2-carboxaldehyde (2.0 g, 20.0 mmol) and nitromethane (3.66 g, 60.0 mmol) in tetrahydrofuran (20 ml) was stirred at room temperature under nitrogen and 1,1,3,3-tetramethylguanidine (230 mg, 2.0 mmol) was added in one portion. The reaction mixture was stirred for thirty minutes. The mixture was then chilled in an ice bath to 0 °C. Trifluoroacetic anhydride (5.25 g, 25 mmol) was added in one portion. The reaction mixture was stirred for thirty minutes after which time triethylamine (5.06 g, 50.0 mmol) was added dropwise. The reaction was stirred for 30 minutes and then partitioned between ethyl acetate and a saturated solution of ammonium chloride. The organic fraction was collected, and the aqueous portion was washed with additional ethyl acetate. The combined organic extracts were dried over sodium sulfate, filtered, concentrated, and purified via flash column (1:2 ethyl acetate:heptanes) to afford the title compound.

### Examample 601B

### 1-(1-methyl-1H-imidazol-4-ylsulfonyl)-4-((S)-tetrahydrofuran-2-yl)pyrrolidin-3-amine

The title compound was prepared similarly to the procedures described in Example 2A-2F substituting Example 601A for trans-4-methoxy-beta nitrostyrene.

### Example 601C

### 1-[(1-methyl-1H-imidazol-4-yl)sulfonyl]-4-(tetrahydrofuran-2-yl)-N-[3-(trifluoromethoxy)phenyl]pyrrolidin-3-amine

The title compound was prepared similarly to the procedure described in Example 57 substituting Example 601B for Example 14A. Purification via flash chromatography (4:1 ethyl acetate:heptanes) provided the title compound. MS (ESI) *m*/*z* 461.1 (M+H)⁺.

### Example 602

### 1-[(1-methyl-1H-imidazol-4-yl)sulfonyl]-4-(tetrahydrofuran-2-yl)-N-[3-(trifluoromethoxy)phenyl]pyrrolidin-3-amine

The title compound was prepared similarly to the procedure described in Example 57 substituting Example 601B for Example 14A. Purification via flash chromatography (4:1 ethyl acetate:heptanes) provided the title compound. MS (ESI) *m*/*z* 461.1 (M+H)⁺.

### Example 603

### 1-[(1-methyl-1H-imidazol-4-yl)sulfonyl]-4-(tetrahydrofuran-2-yl)-N-[3-(trifluoromethyl)phenyl]pyrrolidin-3-amine

The title compound was prepared similarly to the procedure described in Example 57 substituting Example 601B for Example 14A and substituting 1-bromo-3-trifluoromethylbenzene for 1-bromo-2-methylbenzene. Purification via flash chromatography (3:1 ethyl acetate:heptane) provided the title compound. MS (ESI) *m*/*z* 445.1 (M+H)⁺.

### Example 604

### (3R,4S)-1-[(1-methyl-1H-imidazol-4-yl)sulfonyl]-4-(tetrahydrofuran-2-yl)-N-[3-(trifluoromethyl)phenyl]pyrrolidin-3-amine

The title compound was prepared similarly to the procedure described in Example 57 substituting Example 601B for Example 14A and substituting 1-bromo-3-trifluoromethylbenzene for 1-bromo-2-methylbenzene. Purification via flash chromatography (3:1 ethyl acetate:heptane) provided the title compound. MS (ESI) *m*/*z* 445.1 (M+H)⁺.

### Example 605

### 2-methoxy-N- {(3R,4S)-1-[(1-methyl-1H-imidazol-4-yl)sulfonyl]-4-phenylpyrrolidin-3-yl} -6-(trifluoromethyl)pyrimidin-4-amine

### Example 605A

### N-((3R,4S)-1-((1-methyl-1H-imidazol-4-yl)sulfonyl)-4-phenylpyrrolidin-3-yl)-2-(methylthio)-6-(trifluoromethyl)pyrimidin-4-amine

To Example 396A (80 mg, 0.26 mmol) in dimethylsulfoxide (0.3 mL) was added 4-chloro-2-(methylthio)-6-(trifluoromethyl)pyrimidine (80 mg, 0.35 mmol) and cesium carbonate (120 mg, 0.37). The mixture was stirred at 120 °C for 4 hours and then partitioned between ethyl acetate and water. The organic fraction was dried over MgSO₄, filtered, and concentrated to provide the crude title compound.

### Example 605B

### N-((3R,4S)-1-((1-methyl-1H-imidazol-4-yl)sulfonyl)-4-phenylpyrrolidin-3-yl)-2-(methylsulfonyl)-6-(trifluoromethyl)pyrimidin-4-amine

To Example 605A in dichloromethane (1 mL) was added meta-chloroperbenzoic acid (<77%, 320 mg, 1.4 mmol). The mixture was allowed to stir for 3 days. Then the mixture was partition between sodium metabisulfite (aq.) and ethyl acetate. The organic fraction was collected, dried over MgSO₄, filtered, and concentrated to provide the crude title compound.

### Example 605C

### 2-methoxy-N- {(3R,4S)-1-[(1-methyl-1H-imidazol-4-yl)sulfonyl]-4-phenylpyrrolidin-3-yl} -6-(trifluoromethyl)pyrimidin-4-amine

To a solution of Example 605B (0.19 g, 0.36 mmol) in methanol (1 mL) was added sodium methoxide (21 % in methanol, 1 mL). The mixture was stirred at 100 °C for 2 hours and, partitioned between ethyl acetate and water. The organic fraction was collected, concentrated, and purified by HPLC to provide the title compound. MS (ESI) *m*/*z* 483 (M+H)⁺.

### Example 606

### 6-chloro-N-{(3R,4S)-1-[(1-methyl-1H-imidazol-4-yl)sulfonyl]-4-phenylpyrrolidin-3-yl}pyrimidin-4-amine

To a solution of Example 574A (586 mg, 1.85 mmol) in methanol (10 mL) was added 4,6-dichloropyrimidine (829 mg, 5.6 mmol) and triethylamine (0.52 mL, 3.7 mmol). The reaction heated at 50 C for 1 hour. More 4,6-dichloropyrimidine (800 mg) was added and the reaction stirred for 18 hours. The reaction mixture was concentrated. Purification via flash chromatography (0-100% EtOAc/Hexanes) provided the title compound. MS (ESI) *m*/*z* 419 (M+H)⁺.

### Example 607

### 1-cyclopropyl-N-({(3R,4R)-4-(4-fluorophenyl)-1-[(1-methyl-1H-imidazol-4-yl)sulfonyl]pyrrolidin-3-yl}methyl)methanamine

The title compound was prepared similarly to the procedure described in Example 290 substituting Example 586 for Example 242E. MS (ESI) *m*/*z* 393.1 (M+H)⁺.

### Example 608

### (3R,4S)-N-(2-ethoxyethyl)-1-[(1-methyl-1H-imidazol-4-yl)sulfonyl]-4-phenylpyrrolidin-3-amine

To a solution of Example 600B (70 mg, 0.16 mmol) in tetrahydrofuran (7 ml) under nitrogen was added iodoethane (43.6 mg, 0.28 mmol) and sodium hydride (18.6 mg, 0.47 mmol). This solution was stirred at 60 °C for 1hour, and then it was cooled to room temperature. To the reaction mixture was added hydrochloric acid (4N in dioxane 0.7 mL) and the solution stirred at room temperature for 1 hour. The reaction mixture was then concentrated and partitioned between ethyl acetate and saturated sodium bicarbonate (aq.). The organic fraction was collected, concentrated, and purified by HPLC to afford the title product. MS (ESI) *m*/*z* 379.1 (M+H)⁺.

### Example 609

### (3R,4S)-1-[(1-methyl-1H-imidazol-4-yl)sulfonyl]-4-phenyl-N-(2-propoxyethyl)pyrrolidin-3-amine

### Example 609A

### Tert-butyl 2-(allyloxy)ethyl((3R,4S)-1-(1-methyl-1H-imidazol-4-ylsulfonyl)-4-phenylpyrrolidin-3-yl)carbamate

To a solution of Example 600B (73 mg, 0.16 mmol) in dimethylformamide (0.5 mL) under nitrogen at 0°C was added allyl bromide (98 mg, 0.81 mmol) and sodium hydride (19.44 mg, 0.49 mmol). This mixture was stirred at 0°C for 30 minutes, partitioned between ethyl acetate and water. The organic fraction was collected and concentrated to afford the title compound. MS (ESI) *m*/*z* 491.3 (M+H)⁺.

### Example 609B

### (3R,4S)-1-[(1-methyl-1H-imidazol-4-yl)sulfonyl]-4-phenyl-N-(2-propoxyethyl)pyrrolidin-3-amine

To a solution of Example 609A (79 mg, 0.161 mmol) in tetrahydrofuran (15 ml) in a 50 ml pressure bottle was added 5% Pd/C, wet (8 mg, 1.879 µmol). The solution was stirred for 30 minutes under 50 psi of hydrogen at room temperature. The mixture was filtered through a nylon membrane and concentrated. The concentrate was dissolved in dichloromethane (0.5 mL) and trifluoroacetic acid (0.3 mL) was added. This solution was stirred at room temperature for 1 hour, concentrated, and purified via HPLC to afford the title compound. MS (ESI) *m*/*z* 393.1 (M+H)⁺.

### Example 610

### N-(3,4-difluorophenyl)-1-[(1-methyl-1H-imidazol-4-yl)sulfonyl]-4-[-tetrahydrofuran-2-yl]pyrrolidin-3-amine

The title compound was prepared similarly to the procedure described in Example 57 substituting Example 601B for Example 14A and substituting 3,4-difluoro-1-bromobenzene for 1-bromo-2-methylbenzene. Purification via flash chromatography (100% ethyl acetate) provided the title compound. MS (ESI) *m*/*z* 413.0 (M+H)⁺.

### Example 611

### N-(3,4-difluorophenyl)-1-[(1-methyl-1H-imidazol-4-yl)sulfonyl]-4-(tetrahydrofuran-2-yl)pyrrolidin-3-amine

The title compound was prepared similarly to the procedure described in Example 57 substituting Example 601B for Example 14A and substituting 3,4-difluoro-1-bromobenzene for 1-bromo-2-methylbenzene. Purification via flash chromatography (100% ethyl acetate) provided the title compound. MS (ESI) *m*/*z* 413.0 (M+H)⁺.

### Example 612

### 6-cyclopropyl-N-{(3R,4S)-1-[(1-methyl-1H-imidazol-4-yl)sulfonyl]-4-phenylpyrrolidin-3-yl}pyrimidin-4-amine

The title compound was prepared similarly to the procedure described in Example 327 substituting Example 574A for Example 322B and substituting 4-bromo-6-cyclopropylpyrimidine for 1-bromo-3-(trifluoromethyl)benzene. MS (ESI) *m*/*z* 425 (M+H)⁺.

### Example 613

### 6-ethyl-N-{(3R,4S)-4-(4-fluorophenyl)-1-[(1-methyl-1H-imidazol-4-yl)sulfonyl]pyrrolidin-3-yl}pyrimidin-4-amine

The title compound was prepared similarly to the procedure described in Example 337G substituting Example 380A for Example 337F and substituting 4-bromo-6-ethylpyrimidine for 1-bromo-3-(trifluoromethyl)benzene. MS (ESI) *m*/*z* 431.1 (M+H)⁺.

### Example 614

### N-{(3R,4S)-4-(4-fluorophenyl)-1-[(1-methyl-1H-imidazol-4-yl)sulfonyl]pyrrolidin-3-yl}-6-(propan-2-yl)pyrimidin-4-amine

The title compound was prepared similarly to the procedure described in Example 337G substituting Example 380A for Example 337F and substituting 4-bromo-6-isopropylpyrimidine for 1-bromo-3-(trifluoromethyl)benzene. MS (ESI) *m*/*z* 445.1 (M+H)⁺.

### Example 615

### N-{(3R,4S)-4-(4-fluorophenyl)-1-[(1-methyl-1H-imidazol-4-yl)sulfonyl]pyrrolidin-3-yl}-2-methyl-6-(trifluoromethyl)pyrimidin-4-amine

The title compound was prepared similarly to the procedure described in Example 605A substituting Example 380A for Example 396A and substituting 4-chloro-2-methyl-6-(trifluoromethyl)pyrimidine for 4-chloro-2-(methylthio)-6-(trifluoromethyl)pyrimidine. MS (ESI) *m*/*z* 485 (M+H)⁺.

### Example 616

### N-{(3R,4S)-4-(4-fluorophenyl)-1-[(1-methyl-1H-imidazol-4-yl)sulfonyl]pyrrolidin-3-yl}-6-propylpyrimidin-4-amine

The title compound was prepared similarly to the procedure described in Example 605A substituting Example 380A for Example 396A and substituting 4-chloro-6-propylpyrimidine for 4-chloro-2-(methylthio)-6-(trifluoromethyl)pyrimidine. MS (ESI) *m*/*z* 445 (M+H)⁺.

### Example 617

### 5-chloro-N-{(3R,4S)-4-(4-fluorophenyl)-1-[(1-methyl-1H-imidazol-4-yl)sulfonyl]pyrrolidin-3-yl}-4-(trifluoromethyl)pyridin-2-amine

The title compound was prepared similarly to the procedure described in Example 605A substituting Example 380A for Example 396A and substituting 2,5-dichloro-4-(trifluoromethyl)pyridine for 4-chloro-2-(methylthio)-6-(trifluoromethyl)pyrimidine. MS (ESI) *m*/*z* 504/506 (3:1) (M+H)⁺.

### Example 618

### (3S,4R)-N-[4-chloro-3-(trifluoromethyl)phenyl]-1-[(1-methyl-1H-imidazol-4-yl)sulfonyl]-4-(tetrahydrofuran-2-yl)pyrrolidin-3-amine

The title compound was prepared similarly to the procedure described in Example 57 substituting Example 601B for Example 14A and substituting 1-bromo-4-chloro-3-trifluoromethylbenzene for 1-bromo-2-methylbenzene. Purification via flash chromatography (3:1 ethyl acetate:heptane) provided the title compound. MS (ESI) *m*/*z* 479.3 (M+H)⁺.

### Example 619

### (3R,4S)-N-[4-chloro-3-(trifluoromethyl)phenyl]-1-[(1-methyl-1H-imidazol-4-yl)sulfonyl]-4-(tetrahydrofuran-2-yl)pyrrolidin-3-amine

The title compound was prepared similarly to the procedure described in Example 57 substituting Example 601B for Example 14A and substituting 1-bromo-4-chloro-3-trifluoromethylbenzene for 1-bromo-2-methylbenzene. Purification via flash chromatography (3:1 ethyl acetate:heptane) provided the title compound. MS (ESI) *m*/*z* 479.3 (M+H)⁺.

### Example 620

### 6-ethoxy-N-{(3R,4S)-1-[(1-methyl-1H-imidazol-4-yl)sulfonyl]-4-phenylpyrrolidin-3-yl}pyrimidin-4-amine

A microwave vial was charged with Example 606 (100 mg, 0.24 mmol), ethanol (2 mL), and sodium tert-butoxide (115 mg, 1.2 mmol). The reaction mixture was heated in a microwave (Biotage Initiator™, maximum 400 Watts) at 120 °C for 10 minutes. Purification via HPLC provided the title compound. MS (ESI) *m*/*z* 429 (M+H)⁺.

### Example 621

### 6-methoxy-N-{(3R,4S)-1-[(1-methyl-1H-imidazol-4-yl)sulfonyl]-4-phenylpyrrolidin-3-yl}pyrimidin-4-amine

The title compound was prepared similarly to Example 620 substituting methanol for ethanol. MS (ESI) *m*/*z* 415 (M+H)⁺.

### Example 622

### N-{(3R,4S)-1-[(1-methyl-1H-imidazol-4-yl)sulfonyl]-4-phenylpyrrolidin-3-yl}-6-propoxypyrimidin-4-amine

The title compound was prepared similarly to Example 620 substituting propanol for ethanol. MS (ESI) *m*/*z* 443 (M+H)⁺.

### Example 623

### 6-butoxy-N- {(3R,4S)-1-[(1-methyl-1H-imidazol-4-yl)sulfonyl]-4-phenylpyrrolidin-3-yl}pyrimidin-4-amine

The title compound was prepared similarly to Example 620 substituting butanol for ethanol. MS (ESI) *m*/*z* 457 (M+H)⁺.

### Example 624

### N-{(3R,4S)-1-[(1-methyl-1H-imidazol-4-yl)sulfonyl]-4-phenylpyrrolidin-3-yl}-6-(2-methylpropoxy)pyrimidin-4-amine

The title compound was prepared similarly to Example 620 substituting isobutanol for ethanol. MS (ESI) *m*/*z* 457 (M+H)⁺.

### Example 625

### 4-{[(3S,4R)-3-(4-fluorophenyl)-4-(hexyloxy)pyrrolidin-1-yl]sulfonyl}-1-methyl-1H-imidazole

### Example 625A

### Trans-tert-butyl 3 -(4-fluorophenyl)-4-(hexyloxy)pyrrolidine-1 -carboxylate

To Example 379A (140 mg, 0.5 mmol) in dimethylformamide (0.5 mL) was added sodium hydride (35 mg, 60%, 0.9 mmol) and 1-bromohexane (150 mg, 0.91 mmol). The mixture was stirred at 100 °C for 2 hours. The mixture was partitioned between ethyl acetate and water. The organic fraction was collected, and the aqueous fraction was washed with ethyl acetate 3 time. The organic fractions were combined, dried over MgSO₄, filtered, and concentrated to provide the title compound..

### Example 625B

### Trans-3-(4-fluorophenyl)-4-(hexyloxy)pyrrolidine

The title compound was prepared as the hydrochloride salt similarly to the procedure described in Example 373B substituting Example 625A for Example 373A.

### Example 625C

### 4-{[Trans-3-(4-fluorophenyl)-4-(hexyloxy)pyrrolidin-1-yl]sulfonyl}-1-methyl-1H-imidazole

The title compound was prepared similarly to the procedure described in Example 373C substituting Example 625B for Example 373B. MS (ESI) *m*/*z* 410 (M+H)⁺.

### Example 626

### Trans-4-(4-fluorophenyl)-1-[(1-methyl-1H-imidazol-4-yl)sulfonyl]-N-pentylpyrrolidine-3-carboxamide

The title compound was prepared similarly to the procedure described in Example 242E substituting pentan-1-amine for aniline. MS (ESI) *m*/*z* 423.1 (M+H)⁺.

### Example 627

### Trans-4-(4-fluorophenyl)-N-hexyl-1-[(1-methyl-1H-imidazol-4-yl)sulfonyl]pyrrolidine-3-carboxamide

The title compound was prepared similarly to the procedure described in Example 242E substituting hexan-1-amine for aniline. MS (ESI) *m*/*z* 437.1 (M+H)⁺.

### Example 628

### Trans-N-(3-chlorobenzyl)-4-(4-fluorophenyl)-1-[(1-methyl-1H-imidazol-4-yl)sulfonyl]pyrrolidine-3-carboxamide

The title compound was prepared similarly to the procedure described in Example 242E substituting 3-chlorobenzylamine for aniline. MS (ESI) *m*/*z* 477.2 (M+H)⁺.

### Example 629

### Trans-N-(4-chlorobenzyl)-4-(4-fluorophenyl)-1-[(1-methyl-1H-imidazol-4-yl)sulfonyl]pyrrolidine-3-carboxamide

The title compound was prepared similarly to the procedure described in Example 242E substituting 4-chlorobenzylamine for aniline. MS (ESI) *m*/*z* 477.2 (M+H)⁺.

### Example 630

### Trans-N-(4-fluorobenzyl)-4-(4-fluorophenyl)-1-[(1-methyl-1H-imidazol-4-yl)sulfonyl]pyrrolidine-3-carboxamide

The title compound was prepared similarly to the procedure described in Example 242E substituting 4-fluorobenzylamine for aniline. MS (ESI) *m*/*z* 461 (M+H)⁺.

### Example 631

### Trans-N-(3,4-difluorobenzyl)-4-(4-fluorophenyl)-1-[(1-methyl-1H-imidazol-4-yl)sulfonyl]pyrrolidine-3-carboxamide

The title compound was prepared similarly to the procedure described in Example 242E substituting (3,4-difluorophenyl)methanamine for aniline. MS (ESI) *m*/*z* 479.3 (M+H)⁺.

### Example 632

### Trans-4-(4-fluorophenyl)-1-[(1-methyl-1H-imidazol-4-yl)sulfonyl]-N-[3-(trifluoromethyl)benzyl]pyrrolidine-3-carboxamide

The title compound was prepared similarly to the procedure described in Example 242E substituting (3-(trifluoromethyl)phenyl)methanamine for aniline. 45 mg (31%) of the product was obtained. MS (ESI) *m*/*z* 511.3 (M+H)⁺.

### Example 633

### Trans-N-[2-chloro-5-(trifluoromethyl)benzyl]-4-(4-fluorophenyl)-1-[(1-methyl-1H-imidazol-4-yl)sulfonyl]pyrrolidine-3-carboxamide

The title compound was prepared similarly to the procedure described in Example 242E substituting (2-chloro-5-(trifluoromethyl)phenyl)methanamine for aniline. MS (ESI) *m*/*z* 545.3 (M+H)⁺.

### Example 634

### Trans-4-(4-fluorophenyl)-1-[(1-methyl-1H-imidazol-4-yl)sulfonyl] -N-(pyridin-2-ylmethyl)pyrrolidine-3 -carboxamide

The title compound was prepared similarly to the procedure described in Example 242E substituting pyridin-2-ylmethanamine for aniline. MS (ESI) *m*/*z* 444.2 (M+H)⁺.

### Example 635

### Trans-4-(4-fluorophenyl)-1-[(1-methyl-1H-imidazol-4-yl)sulfonyl]-N-{[4-(trifluoromethyl)pyridin-2-yl]methyl}pyrrolidine-3-carboxamide

The title compound was prepared similarly to the procedure described in Example 242E substituting (4-(trifluoromethyl)pyridin-2-yl)methanamine hydrochloride for aniline. MS (ESI) *m*/*z* 512.2 (M+H)⁺.

### Example 636

### Trans-N,N-dibutyl-4-(4-fluorophenyl)-1-[(1-methyl-1H-imidazol-4-yl)sulfonyl]pyrrolidine-3-carboxamide

The title compound was prepared similarly to the procedure described in Example 242E substituting dibutylamine for aniline. MS (ESI) *m*/*z* 465.2 (M+H)⁺.

### Example 637

### 6-methyl-N-{(3R,4S)-1-[(1-methyl-1H-imidazol-4-yl)sulfonyl]-4-phenylpyrrolidin-3-yl}pyrimidin-4-amine

The title compound was prepared similarly to the procedure described in Example 327 substituting Example 574A for Example 322B and substituting 4-bromo-6-methylpyrimidine for 1-bromo-3-(trifluoromethyl)benzene. MS (ESI) *m*/*z* 399 (M+H)⁺.

### Example 638

### (3R,4S)-4-(4-fluorophenyl)-1-[(1-methyl-1H-imidazol-4-yl)sulfonyl]-N-[4-methyl-3-(trifluoromethyl)phenyl]pyrrolidin-3-amine

The title compound was prepared similarly to the procedure described in Example 327 substituting 1-bromo-4-methyl-3-(trifluoromethyl)benzene for 1-bromo-3-(trifluoromethyl)benzene. MS (ESI) *m*/*z* 483 (M+H)⁺.

### Example 639

### 4-({(3R,4S)-4-(4-fluorophenyl)-1-[(1-methyl-1H-imidazol-4-yl)sulfonyl]pyrrolidin-3-yl}amino)-2-(trifluoromethyl)benzonitrile

The title compound was prepared similarly to the procedure described in Example 327 substituting 4-bromo-2-(trifluoromethyl)benzonitrile for 1-bromo-3-(trifluoromethyl)benzene. MS (ESI) *m*/*z* 494 (M+H)⁺.

### Example 640

### 4-{(3R,4S)-4-(4-fluorophenyl)-1-[(1-methyl-1H-imidazol-4-yl)sulfonyl]pyrrolidin-3-yl}-1-propyl-1H-1,2,3-triazole

### Example 640A

### 4-((trans-3-(2,2-dibromovinyl)-4-(4-fluorophenyl)pyrrolidin-1-yl)sulfonyl)-1-methyl-1H-imidazole

To a solution of Example 332A (0.39 g, 1.2 mmol) in dichloromethane (10 mL) was added carbon tetrabromide (0.38 g, 1.2 mmol) and triphenylphosphine (0.61 g, 2.3 mmol) in portions. The mixture was stirred for 2 hours, concentrated, and purified by flash chromatography (100% ethyl acetate) to afford the title compound.

### Example 640B

### 4-(((3S,4S)-3-ethynyl-4-(4-fluorophenyl)pyrrolidin-1-yl)sulfonyl)-1-methyl-1H-imidazole

To a solution of Example 640A (0.24 g, 0.49 mmol) in tetrahydrofuran (1 mL) at -78 °C was added butyllithium (2N, 0.5 mL, 1 mmol). The mixture was stirred for 1 hour and then warmed to room temperature.. The mixture was stirred for 1 hour at room temperature, and then partitioned between ammonium chloride (aq.) and ethyl acetate. The organic fraction was collected, dried over MgSO₄, filtered, and concentrated to provide the title compound.

### Example 640C

### 1-azidopropane

To a solution of 1-bromo propane (2.4 g, 20 mmol) in tetrahydrofuran (15 mL) was added sodium azide (1.3 g, 20 mmol) and water (1mL). The mixture was stirred at 70 °C for 2 hours and this solution containing the title compound was used for Example 604D.

### Example 640D

### 4-{Trans-4-(4-fluorophenyl)-1-[(1-methyl-1H-imidazol-4-yl)sulfonyl]pyrrolidin-3-yl}-1-propyl-1H-1,2,3-triazole

To Example 640B (0.18 g, 0.54 mmol) in half of Example 604C solution was added CuI (90 mg, 0.5 mmol). The mixture was heated at 70 °C for 5 hours, concentrated, and partitioned between ethyl acetate and water. The organic fraction was collected, concentrated, and purified by HPLC to provide the title compound. MS (ESI) *m*/*z* 419 (M+H)⁺.

### Example 641

### 4-[-1-cyclopropylpiperidin-3-yl]-1-[(1-methyl-1H-imidazol-4-yl)sulfonyl]-N-[3-(trifluoromethoxy)phenyl]pyrrolidin-3-amine

### Example 641A

### tert-Butyl (-1-benzyl-4-(pyridin-3-yl)pyrrolidin-3-yl)carbamate

The title compound was prepared in similarly to the procedures described in Example 2A-2C substituting 3-(2-nitroethenyl)pyridine for trans-4-methoxy-beta-nitrostyrene. MS (DCI) *m*/*z* 354.2 (+H)⁺.

### Example 641B

### tert-butyl 1-benzyl-4-(piperidin-3-yl)pyrrolidin-3-ylcarbamate

A solution of Example 641A (3.9 g, 11.03 mmol) in acetic acid (80 mL) was added to 5% platinum on carbon (50% water, 1.1 g) in a stainless steel pressure bottle. The vessel was pressurized with 30 psi of hydrogen and shaken for 8 hours at room temperature. The mixture was filtered through a nylon membrane and concentrated to provide the title compound.

### Example 641C

### tert-butyl 1-benzyl-4-(1-cyclopropylpiperidin-3-yl)pyrrolidin-3-ylcarbamate

641B (1.0 g, 2.78 mmol), 1-ethoxycyclopropoxy-trimethylsilane (1.16 g, 6.68 mmol) and sodium cyanoborohydride (327 mg, 5.2 mmol) in methanol (10 ml) was stirred and heated to 60 °C under nitrogen for six hours. The reaction was then concentrated and the residue was taken up in ethyl acetate. The organic solution was washed with water, brine, and dried over sodium sulfate. The organic mixture was filtered, concentrated, and purified via flash chromatography (97.5:2.5 dichloromethane:2N ammonia in methanol) to afford the title compound.

### Example 641D

### 4-(1-cyclopropylpiperidin-3-yl)-1-(1-methyl-1H-1,2,3-triazol-4-ylsulfonyl)pyrrolidin-3-amine

The title compound was prepared similarly to the procedures described in Examples 2D-2F substituting the 641C for Example 2C.

### Example 641E

### 4-[-1-cyclopropylpiperidin-3-yl]-1-[(1-methyl-1H-imidazol-4-yl)sulfonyl]-N-[3-(trifluoromethoxy)phenyl]pyrrolidin-3-amine

The title compound was prepared similarly to the procedure described in Example 57 substituting Example 641D for Example 14A and substituting 1-bromo-3-trifluoromethylbenzene for 1-bromo-2-methylbenzene.. MS (ESI) *m*/*z* 514.2 (M+H)⁺.

### Example 642

### Trans-N-(3-chloro-4-fluorobenzyl)-4-(4-fluorophenyl)-1-[(1-methyl-1H-imidazol-4-yl)sulfonyl]pyrrolidine-3-carboxamide

The title compound was prepared similarly to the procedure described in Example 242E substituting (3-chloro-4-fluorophenyl)methanamine for aniline. MS (ESI) *m*/*z* 495.2 (M+H)⁺.

### Example 643

### Trans-4-(4-fluorophenyl)-N-(6-methylheptyl)-1-[(1-methyl-1H-imidazol-4-yl)sulfonyl]pyrrolidine-3-carboxamide

The title compound was prepared similarly to the procedure described in Example 242E substituting 6-methylheptan-1-amine for aniline. MS (ESI) *m*/*z* 465.2 (M+H)⁺.

### Example 644

### 2-(4-fluorophenoxy)-N-{(3R,4S)-4-(4-fluorophenyl)-1-[(1-methyl-1H-imidazol-4-yl)sulfonyl]pyrrolidin-3-yl}acetamide

The title compound was prepared similarly to the procedure described in Example 242E substituting Example 380A for aniline and substituting 4-fluorophenoxyacetic acid for Example 242D. MS (ESI) *m*/*z* 477.5 (M+H)⁺.

### Example 645

### (3R,4S)-N-[4-fluoro-3-(trifluoromethyl)phenyl]-1-[(1-methyl-1H-imidazol-4-yl)sulfonyl]-4-phenylpyrrolidin-3-amine

The title compound was prepared similarly to the procedure described in Example 327 substituting Example 574A for Example 322B and substituting 4-bromo-1-fluoro-2-(trifluoromethyl)benzene for 1-bromo-3-(trifluoromethyl)benzene. MS (ESI) *m*/*z* 469 (M+H)⁺.

### Example 646

### (3R,4S)-N-[4-fluoro-3-(trifluoromethoxy)phenyl]-1-[(1-methyl-1H-imidazol-4-yl)sulfonyl]-4-phenylpyrrolidin-3-amine

The title compound was prepared similarly to the procedure described in Example 327 substituting Example 574A for Example 322B and substituting 4-bromo-1-fluoro-2-(trifluoromethoxy)benzene for 1-bromo-3-(trifluoromethyl)benzene. MS (ESI) *m*/*z* 485 (M+H)⁺.

### Example 647

### (3R,4S)-N-(4-methoxybutyl)-1-[(1-methyl-1H-imidazol-4-yl)sulfonyl]-4-phenylpyrrolidin-3-amine

### Example 647A

### methyl 4-(((3R,4S)-1-((1-methyl-1H-imidazol-4-yl)sulfonyl)-4-phenylpyrrolidin-3-yl)amino)butanoate

The title compound was prepared similarly to the procedure described in Example 354E substituting methyl 4-oxobutanoate for 4-chlorobenzaldehyde and substituting Example 396A for Example 354D.

### Example 647B

### methyl 4-((tert-butoxycarbonyl)((3R,4S)-1-((1-methyl-1H-imidazol-4-yl)sulfonyl)-4-phenylpyrrolidin-3-yl)amino)butanoate

The title compound was prepared similarly to the procedure described in Example 337C substituting Example 647A for Example 337B.

### Example 647C

### tert-butyl (4-hydroxybutyl)((3R,4S)-1-((1-methyl-1H-imidazol-4-yl)sulfonyl)-4-phenylpyrrolidin-3-yl)carbamate

To a solution of Example 647B (140 mg, 0.28 mmol) in dichloromethane (1 mL) at -78 °C was added diisobutylaluminum hydride (0.5 mL, IN in hexanes, 0.5 mmol). The mixture was allowed to stir for 45 minutes. The mixture was quenched and partitioned with ethyl acetate and sodium hydrideCO₃(aq). The organic fraction was collected, dried over MgSO₄, filtered, and concentrated to provide the crude title compound.

### Example 647D

### (3R,4S)-N-(4-methoxybutyl)-1-[(1-methyl-1H-imidazol-4-yl)sulfonyl]-4-phenylpyrrolidin-3-amine

The title compound was prepared similarly to the procedure described in Example 588D substituting Example 647C for Example 588C. MS (ESI) *m*/*z* 393 (M+H)⁺.

### Example 648

### (3R,4S)-N-(4-methoxyhexyl)-1-[(1-methyl-1H-imidazol-4-yl)sulfonyl]-4-phenylpyrrolidin-3-amine

### Example 648A

### tert-butyl ((3R,4S)-1-((1-methyl-1H-imidazol-4-yl)sulfonyl)-4-phenylpyrrolidin-3-yl)(4-oxobutyl)carbamate

To a solution of Example 647B (140 mg, 0.28 mmol) in dichloromethane (1 mL) at -78 °C was added Diisobutylaluminum hydride (0.5 mL, IN in hexanes, 0.5 mmol). The mixture was stirred for 45 minutes, and then quenched with ethyl acetate and sodium bicarbonate. The organic fraction was collected, dried over MgSO₄, filtered, and concentrated to provide the crude title compound.

### Example 648B

### tert-butyl (4-hydroxyhexyl)((3R,4S)-1-((1-methyl-1H-imidazol-4-yl)sulfonyl)-4-phenylpyrrolidin-3-yl)carbamate

The title compound was prepared similarly to the procedure described in Example 382B substituting Example 648A for Example 382A and substituting EtMgBr for BnMgBr.

### Example 648C

### (3R,4S)-N-(4-methoxyhexyl)-1-[(1-methyl-1H-imidazol-4-yl)sulfonyl]-4-phenylpyrrolidin-3-amine

The title compound was prepared similarly to the procedure described in Example 588D substituting Example 648B for Example 588C. MS (ESI) *m*/*z* 421 (M+H)⁺.

### Example 649

### (3R,4S)-1-[(1-methyl-1H-imidazol-4-yl)sulfonyl]-4-phenyl-N-[(2-propylcyclopropyl)methyl]pyrrolidin-3-amine

### Example 649A

### Cis-2-propylcyclopropyl)methanol

To a solution of (Z)-hex-2-en-1-ol (200 mg, 2 mmol) in dichloromethane (4 mL) was added diiodomethane (0.66 g, 2.5 mmol), then diethylzinc (4 mL, 1N), 4 mmol) dropwise under nitrogen. The mixture was allowed to stir for 16 hours, and was then quenched with ammonium chloride (aq) hydrochloric acid (aq). Ethyl acetate was added and the mixture partitioned. The organic fraction was collected, dried over MgSO₄, filtered, and concentrated to provide the title compound.

### Example 649B

### Cis-2-propylcyclopropanecarbaldehyde

To a solution of Example 649A (200 mg, 1.75 mmol) in dichloromethane (1 mL) was added Dess-Martin periodinane (424 mg, 1 mmol). The mixture was stirred for 1 hour, and then quenched with sodium bisulfite (aq), and extracted with dichloromethane. The organic extracts were dried over MgSO₄, filtered, and concentrated to nearly dry to provide a concentrated dichloromethane solution of the title compound.

### Example 649C

### (3R,4S)-1-[(1-methyl-1H-imidazol-4-yl)sulfonyl]-4-phenyl-N-[(2-propylcyclopropyl)methyl]pyrrolidin-3-amine

The title compound was prepared similarly to the procedure described in Example 354E substituting Example 649B for 4-chlorobenzaldehyde and Example 396A for Example 354D. MS (ESI) *m*/*z* 403 (M+H)⁺.

### Example 650

### (3R,4S)-1-[(1-methyl-1H-imidazol-4-yl)sulfonyl]-4-phenyl-N-[(2-propylcyclopropyl)methyl]pyrrolidin-3-amine

### Example 650A

### (Trans-2-propylcyclopropyl)methanol

The title compound was prepared similarly to the procedure described in Example 649A substituting (E)-hex-2-en-1-ol for (Z)-hex-2-en-1-ol.

### Example 650B

### Trans-2-propylcyclopropanecarbaldehyde

The title compound was prepared similarly to the procedure described in Example 649B substituting Example 650A for Example 649A.

### Example 650C

### (3R,4S)-1-[(1-methyl-1H-imidazol-4-yl)sulfonyl]-4-phenyl-N-[(2-propylcyclopropyl)methyl]pyrrolidin-3-amine

The title compound was prepared similarly to the procedure described in Example 354E substituting Example 650B for 4-chlorobenzaldehyde and substituting Example 396A for Example 354D. MS (ESI) *m*/*z* 403 (M+H)⁺

### Example 651

### (3R,4S)-4-(4-fluorophenyl)-N-[4-fluoro-3-(trifluoromethoxy)phenyl]-1-[(1-methyl-1H-imidazol-4-yl)sulfonyl]pyrrolidin-3-amine

The title compound was prepared similarly to the procedure described in Example 327 substituting 4-bromo-1-fluoro-2-(trifluoromethoxy)benzene for 1-bromo-3-(trifluoromethyl)benzene. MS (ESI) *m*/*z* 503 (M+H)⁺.

### Example 652

### (3R,4S)-4-(4-fluorophenyl)-N-[4-fluoro-3-(trifluoromethyl)phenyl]-1-[(1-methyl-1H-imidazol-4-yl)sulfonyl]pyrrolidin-3-amine

The title compound was prepared similarly to the procedure described in Example 327 substituting 4-bromo-1-fluoro-2-(trifluoromethyl)benzene for 1-bromo-3-(trifluoromethyl)benzene. MS (ESI) *m*/*z* 503 (M+H)⁺.

### Example 653

### (3R,4S)-N-[4-methoxy-3-(trifluoromethyl)phenyl]-1-[(1-methyl-1H-imidazol-4-yl)sulfonyl]-4-phenylpyrrolidin-3-amine

The title compound was prepared similarly to the procedure described in Example 327 substituting Example 574A for Example 322B and substituting 4-bromo-1-methoxy-2-(trifluoromethyl)benzene for 1-bromo-3-(trifluoromethyl)benzene. MS (ESI) *m*/*z* 481 (M+H)⁺.

### Example 654

### (3R,4S)-N-(1-benzothiophen-5-yl)-1-[(1-methyl-1H-imidazol-4-yl)sulfonyl]-4-phenylpyrrolidin-3-amine

The title compound was prepared similarly to the procedure described in Example 327 substituting Example 574A for Example 322B and substituting 5-bromobenzo[b]thiophene for 1-bromo-3-(trifluoromethyl)benzene. MS (ESI) *m*/*z* 439 (M+H)⁺.

### Example 655

### (3R,4S)-N-(2,3-dihydro-1H-inden-5-yl)-1-[(1-methyl-1H-imidazol-4-yl)sulfonyl]-4-phenylpyrrolidin-3-amine

The title compound was prepared similarly to the procedure described in Example 327 substituting Example 574A for Example 322B and substituting 5-bromo-2,3-dihydro-1H-indene for 1-bromo-3-(trifluoromethyl)benzene. MS (ESI) *m*/*z* 423 (M+H)⁺.

### Example 656

### (3R,4S)-N-[3,4-bis(trifluoromethyl)phenyl]-1-[(1-methyl-1H-imidazol-4-yl)sulfonyl]-4-phenylpyrrolidin-3-amine

The title compound was prepared similarly to the procedure described in Example 327 substituting Example 574A for Example 322B and substituting 1-bromo-3,4-bis(trifluoromethyl)benzene for 1-bromo-3-(trifluoromethyl)benzene. MS (ESI) *m*/*z* 519 (M+H)⁺.

### Example 657

### (3R,4S)-1-[(1-methyl-1H-imidazol-4-yl)sulfonyl]-4-phenyl-N-(5,6,7,8-tetrahydronaphthalen-2-yl)pyrrolidin-3-amine

The title compound was prepared similarly to the procedure described in Example 327 substituting Example 574A for Example 322B and substituting 6-bromo-1,2,3,4-tetrahydronaphthalene for 1-bromo-3-(trifluoromethyl)benzene. MS (ESI) *m*/*z* 437 (M+H)⁺.

### Example 658

### (3R,4S)-N-(3,4-dihydro-2H-chromen-6-yl)-1-[(1-methyl-1H-imidazol-4-yl)sulfonyl]-4-phenylpyrrolidin-3-amine

The title compound was prepared similarly to the procedure described in Example 327 substituting Example 574A for Example 322B and substituting 6-bromochroman for 1-bromo-3-(trifluoromethyl)benzene. MS (ESI) *m*/*z* 439 (M+H)⁺.

### Example 659

### 4-({(3S,4R)-1-[(1-methyl-1H-imidazol-4-yl)sulfonyl]-4-phenylpyrrolidin-3-yl}methyl)-1-propyl-1H-1,2,3-triazole

### Example 659A

### 1-benzyl-4-phenylpyrrolidin-2-one

To a solutuion of 4-phenylpyrrolidin-2-one (1 g, 6.2 mmol) in tetrahydrofuran (6 mL) was added sodium hydride (60%, 280 mg, 7 mmol). Benzylbromide was added after 10 minutes. The mixture was allowed to stir at. 65 °C for 2 hours. The reaction mixture was, quenched with ammonium chloride (aq) and partitioned with ethyl acetate. The organic fraction was collected, dried over MgSO₄, filtered, and concentrated to provide the title compound.

### Example 659B

### Trans-1 -benzyl-4-phenyl-3 -(prop-2-yn-1 -yl)pyrrolidin-2-one

To a solution of diisopropylamine (700 mg, 7 mmol) in tetrahydrofuran (7 mL) at -78 °C was added butyllithium (2N, 3.1 mL, 6.2 mmol). The mixture was allowed to stir for 10 minutes. Example 659A (1.56 g, 6.2 mmol) in tetrahydrofuran (5 mL) was added. The mixture was allowed to stir for 30 minutes. Propargyl bromide (0.7 mL, 80% toluene solution, 6.2 mmol) in tetrahydrofuran (3 mL) was added. The mixture was allowed to stir for 1 hour, quenched with ammonium chlorid (aq), and partitioned with ethyl acetate. The organic fraction was collected, concentrated, and purified via flash chromatography (50% ethyl acetate/hexanes) to afford the title compound.

### Example 659C

### Trans-1-benzyl-4-phenyl-3-((1-propyl-1H-1,2,3-triazol-4-yl)methyl)pyrrolidin-2-one

The title compound was prepared similarly to the procedure described in Example 640D substituting Example 659B for Example 640B.

### Example 659D

### 4-((trans-1-benzyl-4-phenylpyrrolidin-3-yl)methyl)-1-propyl-1H-1,2,3-triazole

To a solution of Example 659C (0.32 g, 0.86 mmol) in tetrahydrofuran (3 mL) was added lithium aluminumhydride in tetrahydrofuran (2N, 1 mL, 2 mmol). The mixture was allowed to stir for 3 hours. It was then quenched with methanol, followed by sodium bicarbonate (aq). The mixture was partitioned with ethyl acetate. The organic fraction was dried over potassium carbonate, filtered, and concentrated to provide the title compound.

### Example 659E

### 4-((trans-4-phenylpyrrolidin-3-yl)methyl)-1-propyl-1H-1,2,3-triazole

The title compound was prepared similarly to the procedure described in Example 2D substituting Example 659D for Example 2C.

### Example 659F

### 4-({Trans-1-[(1-methyl-1H-imidazol-4-yl)sulfonyl]-4-phenylpyrrolidin-3-yl}methyl)-1-propyl-1H-1,2,3-triazole

The title compound was prepared similarly to the procedure described in Example 373C substituting Example 659E for Example 373B. MS (ESI) *m*/*z* 415 (M+H)⁺.

### Example 660

### (3R,4S)-N-(1-benzofuran-5-yl)-1-[(1-methyl-1H-imidazol-4-yl)sulfonyl]-4-phenylpyrrolidin-3-amine

The title compound was prepared similarly to the procedure described in Example 327 substituting Example 574A for Example 322B and substituting 5-bromobenzofuran for 1-bromo-3-(trifluoromethyl)benzene. MS (ESI) *m*/*z* 423 (M+H)⁺.

### Example 661

### Trans-4-(4-fluorophenyl)-1-[(1-methyl-1H-imidazol-4-yl)sulfonyl]-N-[3-(trifluoromethoxy)benzyl]pyrrolidine-3-carboxamide

The title compound was prepared similarly to the procedure described in Example 242E substituting (3-(trifluoromethoxy)phenyl)methanamine for aniline. MS (ESI) *m*/*z* 527.2 (M+H)⁺.

### Example 662

### methyl 5-({(3R,4S)-4-(4-fluorophenyl)-1-[(1-methyl-1H-imidazol-4-yl)sulfonyl]pyrrolidin-3-yl}amino)-5-oxopentanoate

The title compound was prepared similarly to the procedure described in Example 242E substituting Example 380A for aniline, methyl hydrogen glutarate for Example 242D. MS (ESI) *m*/*z* 453.3 (M+H)⁺.

### Example 663

### (3R,4S)-N-[2-chloro-4-(trifluoromethyl)benzyl]-4-(4-fluorophenyl)-1-[(1-methyl-1H-imidazol-4-yl)sulfonyl]pyrrolidine-3-carboxamide

The title compound was prepared similarly to the procedure described in Example 242E substituting (2-chloro-4-(trifluoromethyl)phenyl)methanamine for aniline. MS (ESI) *m*/*z* 545.3 (M+H)⁺.

### Example 664

### (3R,4S)-4-(4-fluorophenyl)-1-[(1-methyl-1H-imidazol-4-yl)sulfonyl]-N-pentylpyrrolidin-3-amine

The title compound was prepared similarly to the procedure described in Example 354E substituting valeraldehyde for 4-chlorobenzaldehyde and substituting Example 380A for Example 354D. MS (ESI) *m*/*z* 395.2 (M+H)⁺.

### Example 665

### (3R,4S)-4-(4-fluorophenyl)-N-hexyl-1-[(1-methyl-1H-imidazol-4-yl)sulfonyl]pyrrolidin-3-amine

The title compound was prepared similarly to the procedure described in Example 354E substituting hexanal for 4-chlorobenzaldehyde and substituting Example 380A for Example 354D. MS (ESI) *m*/*z* 409.2 (M+H)⁺.

### Example 666

### (3R,4S)-N-[2-(2-methoxyethoxy)ethyl]-1-[(1-methyl-1H-imidazol-4-yl)sulfonyl]-4-phenylpyrrolidin-3-amine

### Example 666A

### 2-(2-methoxyethoxy)-N-((3R,4S)-1-((1-methyl-1H-imidazol-4-yl)sulfonyl)-4-phenylpyrrolidin-3-yl)acetamide

The title compound was prepared similarly to the procedure described in Example 404A substituting Example 396A for Example 14A and substituting 2-(2-methoxyethoxy)acetic acid for 2-nitro-5 -(trifluoromethyl)benzoic acid.

### Example 666B

### (3R,4S)-N-[2-(2-methoxyethoxy)ethyl]-1-[(1-methyl-1H-imidazol-4-yl)sulfonyl]-4-phenylpyrrolidin-3-amine

The title compound was prepared similarly to the procedure described in Example 404B substituting Example 666A for Example 404A. MS (ESI) *m*/*z* 409 (M+H)⁺.

### Example 667

### (3R,4S)-N-[2-(4-fluorophenoxy)ethyl]-4-(4-fluorophenyl)-1-[(1-methyl-1H-imidazol-4-yl)sulfonyl]pyrrolidin-3-amine

The title compound was prepared similarly to the procedure described in Example 290 substituting Example 644 for Example 242E. MS (ESI) *m*/*z* 463.1 (M+H)⁺.

### Example 668

### 4-butyl-1-{(3R,4S)-1-[(1-methyl-1H-imidazol-4-yl)sulfonyl]-4-phenylpyrrolidin-3-yl}-1H-1,2,3-triazole

### Example 668A

### Ethyl 2-(4-butyl-1H-1,2,3-triazol-1-yl)acetate

The title compound was prepared similarly to the procedure described in Example 640D substituting hex-1-yne for Example 640B and substituting ethyl 2-azidoacetate for Example 640C.

### Example 668B

### ethyl 2-(4-butyl-1H-1,2,3-triazol-1-yl)-4-nitro-3-phenylbutanoate

The title compound was prepared similarly to the procedure described in Example 331A substituting Example 668A for methyl 3-(3-chlorophenyl)propanoate.

### Example 668C

### Trans-3-(4-butyl-1H-1,2,3-triazol-1-yl)-4-phenylpyrrolidin-2-one

To Example 668B (530 mg, 1.5 mmol) in ethanol (10 mL) was added Raney nickel, water slurry (616 mg, 10.5 mmol) in a 20 mL pressure bottle. The mixture was stirred under 60 psi of hydrogen at room temperatue for 2 days. The mixture was filtered through a polypropylene membrane and concentrated to provide the title compound.

### Example 668D

### 4-Butyl-1-(Trans-4-phenylpyrrolidin-3-yl)-1H-1,2,3-triazole

The title compound was prepared similarly to the procedure described in Example 404B substituting Example 668C for Example 404A.

### Example 668E

### 4-butyl-1-{Trans-1-[(1-methyl-1H-imidazol-4-yl)sulfonyl]-4-phenylpyrrolidin-3-yl}-1H-1,2,3-triazole

The title compound was prepared similarly to the procedure described in Example 373C substituting Example 668D for Example 373B. MS (ESI) *m*/*z* 375 (M+H)⁺. MS (ESI) *m*/*z* 415 (M+H)⁺.

### Example 669

### (3R,4S)-N-(4-chloro-3-methylphenyl)-1-[(1-methyl-1H-imidazol-4-yl)sulfonyl]-4-phenylpyrrolidin-3-amine

The title compound was prepared similarly to the procedure described in Example 327 substituting Example 574A for Example 322B and substituting 1-bromo-4-chloro-3-methylbenzene for 1-bromo-3-(trifluoromethyl)benzene. MS (APCI) *m*/*z* 431 (M+H)⁺.

### Example 670

### (3R,4S)-1-[(1-methyl-1H-imidazol-4-yl)sulfonyl]-4-phenyl-N-[3-(2,2,2-trifluoroethoxy)phenyl]pyrrolidin-3-amine

The title compound was prepared similarly to the procedure described in Example 327 substituting Example 574A for Example 322B and substituting 1-bromo-3-(2,2,2-trifluoroethoxy)benzene for 1-bromo-3-(trifluoromethyl)benzene. MS (ESI) *m*/*z* 481 (M+H)⁺.

### Example 671

### (3R,4S)-N-(2,3-dihydro-1-benzofuran-5-yl)-1-[(1-methyl-1H-imidazol-4-yl)sulfonyl]-4-phenylpyrrolidin-3-amine

The title compound was prepared similarly to the procedure described in Example 327 substituting Example 574A for Example 322B and substituting 5-bromo-2,3-dihydrobenzofuran for 1-bromo-3-(trifluoromethyl)benzene. MS (ESI) *m*/*z* 425 (M+H)⁺.

### Example 672

### (3R,4S)-N-(4,4-dimethyl-3,4-dihydro-2H-chromen-6-yl)-4-(4-fluorophenyl)-1-[(1-methyl-1H-imidazol-4-yl)sulfonyl]pyrrolidin-3-amine

The title compound was prepared similarly to the procedure described in Example 327 substituting 6-bromo-4,4-dimethylchroman for 1-bromo-3-(trifluoromethyl)benzene. MS (ESI) *m*/*z* 485 (M+H)⁺.

### Example 673

### N-{(3R,4S)-4-(4-fluorophenyl)-1-[(1-methyl-1H-imidazol-4-yl)sulfonyl]pyrrolidin-3-yl}-2-hydroxyhexanamide

The title compound was prepared similarly to the procedure described in Example 242E substituting Example 380A for aniline and substituting DL-alpha-hydroxycaproic acid for Example 242D. MS (ESI) *m*/*z* 439.2 (M+H)⁺.

### Example 674

### (3R,4S)-N-(3-methoxyhexyl)-1-[(1-methyl-1H-imidazol-4-yl)sulfonyl]-4-phenylpyrrolidin-3-amine

### Example 674A

### tert-butyl (3-hydroxyhexyl)((3R,4S)-1-((1-methyl-1H-imidazol-4-yl)sulfonyl)-4-phenylpyrrolidin-3-yl)carbamate

A mixture of hex-1-en-3-one (38 mg, 0.4 mmol) and Example 396A (120 mg, 0.4 mmol) in methanol (0.5 mL) was allowed to stir overnight. Sodium borohydride (15 mg, 0.4 mmol) was added to the solution. The mixture was stirred for 30 minutes, di-tert-butyl dicarbonate (100 mg, 0.45 mmol) was added. The mixture was stirred for 2 hours and partitioned between ethyl acetate and water. The organic fraction was collected, concentrated, and purified by flash chromatography (100% ethyl acetate).to afford the title compound.

### Example 674B

### (3R,4S)-N-(3-methoxyhexyl)-1-[(1-methyl-1H-imidazol-4-yl)sulfonyl]-4-phenylpyrrolidin-3-amine

The title compound was prepared similarly to the procedure described in Example 588D substituting Example 674A for Example 588C. MS (ESI) *m*/*z* 421 (M+H)⁺.

### Example 675

### (3R,4S)-N-[4-chloro-3-(propan-2-yl)phenyl]-4-(4-fluorophenyl)-1-[(1-methyl-1H-imidazol-4-yl)sulfonyl]pyrrolidin-3-amine

The title compound was prepared similarly to the procedure described in Example 327 substituting 4-bromo-1-chloro-2-isopropylbenzene for 1-bromo-3-(trifluoromethyl)benzene. MS (ESI) *m*/*z* 477 (M+H)⁺.

### Example 676

### (3R,4S)-4-(4-fluorophenyl)-1-[(1-methyl-1H-imidazol-4-yl)sulfonyl]-N-[3-methyl-4-(trifluoromethyl)phenyl]pyrrolidin-3-amine

The title compound was prepared similarly to the procedure described in Example 327 substituting 4-bromo-3-methyl-1-(trifluoromethyl)benzene for 1-bromo-3-(trifluoromethyl)benzene. MS (ESI) *m*/*z* 483 (M+H)⁺.

### Example 677

### (3R,4S)-N-(1-benzothiophen-6-yl)-4-(4-fluorophenyl)-1-[(1-methyl-1H-imidazol-4-yl)sulfonyl]pyrrolidin-3-amine

The title compound was prepared similarly to the procedure described in Example 327 substituting 6-bromobenzo[b]thiophene for 1-bromo-3-(trifluoromethyl)benzene. MS (ESI) *m*/*z* 457 (M+H)⁺.

### Example 678

### -4-(1-cyclopropylpiperidin-4-yl)-1-[(1-methyl-1H-imidazol-4-yl)sulfonyl]-N-[3-(trifluoromethoxy)phenyl]pyrrolidin-3-amine

### Example 678A

### (E)-tert-butyl 4-(2-nitrovinyl)piperidine-1-carboxylate

The title compound was prepared similarly to the procedure described in Example 601A substituting 4-formylpiperidine-1-carboxylic acid-t-butyl ester for tetrahydrofuran-2-carboxaldehyde. MS (DCI) *m*/*z* 274.1 (M+NH₄)⁺

### Example 678B

### 4-(1-cyclopropylpiperidin-4-yl)-1-(1-methyl-1H-1,2,3-triazol-4-ylsulfonyl)pyrrolidin-3-amine

The title compound was prepared similarly to the procedures described in Example 2A-2F substituting Example 678A for (E)-1-methoxy-4-(2-nitrovinyl)benzene.

### Example 678C

### -4-(1-cyclopropylpiperidin-4-yl)-1-[(1-methyl-1H-imidazol-4-yl)sulfonyl]-N-[3-(trifluoromethoxy)phenyl]pyrrolidin-3-amine

The title compound was prepared similarly to the procedure described in Example 57 substituting 1-bromo-3-(trifluoromethoxy)benzene for 1-bromo-2-methylbenzene. MS (ESI) *m*/*z* 514.2 (M+H)+

### Example 679

### (3R,4S)-N-hexyl-1-[(1-methyl-1H-imidazol-4-yl)sulfonyl]-4-(tetrahydro-2H-pyran-2-yl)pyrrolidin-3-amine

### Example 679A

### tetrahydro-2H-pyran-2-carbaldehyde

To a solution of oxalyl chloride (1.3 g, 10 mmol) in dichloromethane (20 mL) at -78 °C was added dimethylsufoxide (1.3 g, 17 mmol). The mixture was stirred for 10 minutes. A solution of (tetrahydro-2H-pyran-2-yl)methanol (1.2 g, 10 mmol) in dichloromethane (20 mL) was added. The mixture was stirred for 15 minutes at room temperature. The mixture was washed with water. The organic fraction was dried over MgSO₄, filtered, and concentrated to provide the title compound.

### Example 679B

### (E)-2-(2-nitrovinyl)tetrahydro-2H-pyran

To a solution of Example 679A (1.2 g, 10 mmol) in nitromethane (5 mL) was added pyrrolidine (100 mL). The mixture was allowed to stir overnight and concentrated to afford the title compound.

### Example 679C

### Trans-1-benzyl-3-nitro-4-(-tetrahydro-2H-pyran-2-yl)pyrrolidine.

The title compound was prepared similarly to the procedure described in Example 2A substituting Example 679B for 4-methoxy-beta-nitrostyrene.

### Example 679D

### Trans-1-benzyl-4-(tetrahydro-2H-pyran-2-yl)pyrrolidin-3-amine

The title compound was prepared similarly to the procedure described in Example 2B substituting Example 679C for Example 2A

### Example 679E

### tert-butyl (trans-1-benzyl-4-(tetrahydro-2H-pyran-2-yl)pyrrolidin-3-yl)carbamate

The title compound was prepared similarly to the procedure described in Example 337C substituting Example 679D for Example 337B.

### Example 679F

### tert-butyl (trans-4-(tetrahydro-2H-pyran-2-yl)pyrrolidin-3-yl)carbamate

The title compound was prepared similarly to the procedure described in Example 2D substituting Example 679E for 2C

### Example 679G

### tert-butyl (trans-1-((1-methyl-1H-imidazol-4-yl)sulfonyl)-4-(tetrahydro-2H-pyran-2-yl)pyrrolidin-3 -yl)carbamate

The title compound was prepared similarly to the procedure described in Example 373C substituting Example 679F for Example 373B

### Example 679H

### Trans-1-((1-methyl-1H-imidazol-4-yl)sulfonyl)-4-(tetrahydro-2H-pyran-2-yl)pyrrolidin-3-amine

The title compound was prepared similarly to the procedure described in Example 337F substituting Example 679G for Example 337E.

### Example 6791

### Trans-N-hexyl-1-[(1-methyl-1H-imidazol-4-yl)sulfonyl]-4-(tetrahydro-2H-pyran-2-yl)pyrrolidin-3-amine

The title compound was prepared similarly to the procedure described in Example 354E substituting hexanal for 4-chlorobenzaldehyde and substituting Example 679H for Example 354D. MS (ESI) *m*/*z* 399 (M+H)⁺.

### Example 680

### Trans-N-[2-chloro-4-(trifluoromethyl)benzyl]-1-[(1-methyl-1H-imidazol-4-yl)sulfonyl]-4-(tetrahydro-2H-pyran-2-yl)pyrrolidin-3-amine

The title compound was prepared similarly to the procedure described in Example 354E substituting 2-chloro-4-(trifluoromethyl)benzaldehyde for 4-chlorobenzaldehyde and substituting Example 679H for Example 354D. MS (ESI) *m*/*z* 507/509 (3:1) (M+H)⁺.

### Example 681

### Trans-1-[(1-methyl-1H-imidazol-4-yl)sulfonyl]-4-(tetrahydro-2H-pyran-2-yl)-N-[3-(trifluoromethyl)phenyl]pyrrolidin-3-amine

The title compound was prepared similarly to the procedure described in Example 337G substituting Example 679H for Example 337F. MS (ESI) *m*/*z* 459 (M+H)⁺.

### Example 682

### N-{Trans-1-[(1-methyl-1H-imidazol-4-yl)sulfonyl]-4-[tetrahydro-2H-pyran-2-yl]pyrrolidin-3-yl}-4-(trifluoromethyl)pyridin-2-amine

The title compound was prepared similarly to the procedure described in Example 337G substituting Example 679H for Example 337F and substituting 2-bromo-4-(trifluoromethyl)pyridine for 1-bromo-3-(trifluoromethyl)benzene. MS (ESI) *m*/*z* 460 (M+H)⁺.

### Example 683

### N-{Trans-1-[(1-methyl-1H-imidazol-4-yl)sulfonyl]-4-[tetrahydro-2H-pyran-2-yl]pyrrolidin-3-yl}-4-(trifluoromethyl)pyridin-2-amine

The title compound was prepared similarly to the procedure described in Example 337G substituting Example 679H for Example 337F and substituting 2-bromo-4-(trifluoromethyl)pyridine for 1-bromo-3-(trifluoromethyl)benzene. MS (ESI) *m*/*z* 460 (M+H)⁺.

### Example 684

### (3R,4S)-N-[4-(4,4-dimethyl-1,3-dioxan-2-yl)butyl]-4-(4-fluorophenyl)-1-[(1-methyl-1H-imidazol-4-yl)sulfonyl]pyrrolidin-3-amine

The title compound was prepared similarly to the procedure described in Example 354E substituting 5,5-dimethyl-2-(3'-formylpropyl)-1,3-dioxane for 4-chlorobenzaldehyde and substituting Example 380A for Example 354D. MS (ESI) *m*/*z* 495.1 (M+H)⁺.

### Example 685

### Trans-1-[(1-methyl-1H-imidazol-4-yl)sulfonyl]-4-(tetrahydro-2H-pyran-2-yl)-N-[3-(trifluoromethoxy)phenyl]pyrrolidin-3-amine

The title compound was prepared similarly to the procedure described in Example 337G substituting Example 679H for Example 337F and substituting 1-bromo-3-(trifluoromethoxy)benzene for 1-bromo-3-(trifluoromethyl)benzene. MS (ESI) *m*/*z* 475 (M+H)⁺.

### Example 686

### Trans-1-[(1-methyl-1H-imidazol-4-yl)sulfonyl]-4-[tetrahydro-2H-pyran-2-yl]-N-[3-(trifluoromethoxy)phenyl]pyrrolidin-3-amine

The title compound was prepared similarly to the procedure described in Example 337G substituting Example 679H for Example 337F and substituting 1-bromo-3-(trifluoromethoxy)benzene for 1-bromo-3-(trifluoromethyl)benzene. MS (ESI) *m*/*z* 475 (M+H)⁺.

### Example 687

### N-[Trans-1-[(1-methyl-1H-imidazol-4-yl)sulfonyl]-4-(tetrahydro-2H-pyran-2-yl)pyrrolidin-3-yl]-6-(trifluoromethyl)pyrimidin-4-amine

The title compound was prepared similarly to the procedure described in Example 337G substituting Example 679H for Example 337F and substituting 4-bromo-6-(trifluoromethyl)pyrimidine for 1-bromo-3-(trifluoromethyl)benzene. MS (ESI) *m*/*z* 461 (M+H)⁺.

### Example 688

### N-[2-({Trans-4-(4-fluorophenyl)-1-[(1-methyl-1H-imidazol-4-yl)sulfonyl]pyrrolidin-3-yl}oxy)ethyl]propan-1-amine

The title compound was prepared similarly to the procedure described in Example 404B substituting Example 689E for Example 404A. MS (ESI) *m*/*z* 411 (M+H)⁺.

### Example 689

### 2-({Trans-4-(4-fluorophenyl)-1-[(1-methyl-1H-imidazol-4-yl)sulfonyl]pyrrolidin-3-yl}oxy)-N-propylacetamide

### Example 689A

### Trans-tert-butyl 3-(4-fluorophenyl)-4-(2-methoxy-2-oxoethoxy)pyrrolidine-1-carboxylate

To a solution of Example 379A (0.56 g, 2 mmol) in dimethylformamide (2 mL) was added methyl 2-bromoacetate (0.38 g, 2.5 mmol) and sodium hydride (60%, 120 mg, 3 mmol). The mixture was stirred at 80 °C for 4 hours. The mixture was partitioned with water and ethyl acetate. The organic fraction was collected, concentrated, and purified by flash chromatography (50% ethyl acetate/hexanes) to afford the title compound.

### Example 689B

### methyl 2-((trans-4-(4-fluorophenyl)pyrrolidin-3-yl)oxy)acetate

The title compound was prepared as the hydrochloride salt similarly to the procedure described in Example 373B substituting Example 689A for Example 373A.

### Example 689C

### methyl 2-((trans-4-(4-fluorophenyl)-1-((1-methyl-1H-imidazol-4-yl)sulfonyl)pyrrolidin-3-yl)oxy)acetate

The title compound was prepared similarly to the procedure described in Example 373C substituting Example 689B for Example 373B.

### Example 689D

### 2-((Trans-4-(4-fluorophenyl)-1-((1-methyl-1H-imidazol-4-yl)sulfonyl)pyrrolidin-3-yl)oxy)acetic acid

Example 689C (0.11 g, 0.28 mmol) was dissolved in a solution of litium hydroxide (methanol:water. 5:3, IN, 3 mL). The mixture was stirred for 2 hours. Hydrochloric acid (aq) was added to adjust the pH value to 2. The mixture was partitioned with ethyl acetate. The organic fraction was collected, dried over MgSO₄, filtered, and concentrated to provide the title compound.

### Example 689E

### 2-({Trans-4-(4-fluorophenyl)-1-[(1-methyl-1H-imidazol-4-yl)sulfonyl]pyrrolidin-3-yl}oxy)-N-propylacetamide

The title compound was prepared similarly to the procedure described in Example 404A substituting propyl amine for Example 14A and Example 689D for 2-nitro-5-(trifluoromethyl)benzoic acid. MS (ESI) *m*/*z* 425 (M+H)⁺.

### Example 690

### 6-chloro-N-{(3R,4S)-4-(4-fluorophenyl)-1-[(1-methyl-1H-imidazol-4-yl)sulfonyl]pyrrolidin-3-yl}-2-methylpyrimidin-4-amine

The title compound was prepared similarly to the procedure described in Example 606 substituting Example 322B for Example 574A and substituting 4,6-dichloro-2-methylpyrimidine for 4,6-dichloropyrimidine. MS (ESI) *m*/*z* 451 (M+H)⁺.

### Example 691

### 6-chloro-N-{(3R,4S)-4-(4-fluorophenyl)-1-[(1-methyl-1H-imidazol-4-yl)sulfonyl]pyrrolidin-3-yl}pyrimidin-4-amine

The title compound was prepared similarly to the procedure described in Example 606 substituting Example 322B for Example 574A but heated at 120 °C for 10 minutes in a microwave (Biotage Initiator™, maximum 400 Watts). MS (ESI) *m*/*z* 437 (M+H)⁺.

### Example 692

### 1-{(3R,4S)-4-(4-fluorophenyl)-1-[(1-methyl-1H-imidazol-4-yl)sulfonyl]pyrrolidin-3-yl}-3-phenylurea

A microwave vial was charged with Example 322B (50 mg, 0.15 mmol), isocyanatobenzene (20 mg, 0.17 mmol), triethylamine (65 µL, 0.46 mmol) and tetrahydrofuran (2 mL). The reaction mixture was heated in a microwave (Biotage Initiator™, maximum 400 Watts) at 100 °C for 10 minutes. Purification via HPLC provided the title compound. MS (ESI) *m*/*z* 444 (M+H)⁺.

### Example 693

### phenyl {(3R,4S)-4-(4-fluorophenyl)-1-[(1-methyl-1H-imidazol-4-yl)sulfonyl]pyrrolidin-3-yl}carbamate

The title compound was prepared similarly to Example 692 substituting phenyl carbonochloridate for isocyanatobenzene. MS (ESI) *m*/*z* 445 (M+H)⁺.

### Example 694

### 1-(2-chlorophenyl)-3-{(3R,4S)-4-(4-fluorophenyl)-1-[(1-methyl-1H-imidazol-4-yl)sulfonyl]pyrrolidin-3-yl}urea

The title compound was prepared similarly to Example 692 substituting 1-chloro-2-isocyanatobenzene for isocyanatobenzene. MS (ESI) *m*/*z* 478 (M+H)⁺.

### Example 695

### N-{(3R,4S)-4-(4-fluorophenyl)-1-[(1-methyl-1H-imidazol-4-yl)sulfonyl]pyrrolidin-3-yl}-5-oxohexanamide

The title compound was prepared similarly to the procedure described in Example 242E substituting Example 380A for aniline and substituting 4-acetylbutyric acid for Example 242D. MS (ESI) *m*/*z* 437.1 (M+H)⁺.

### Example 696

### N-{(3R,4S)-4-(4-fluorophenyl)-1-[(1-methyl-1H-imidazol-4-yl)sulfonyl]pyrrolidin-3-yl}-4-(trifluoromethyl)cyclohexanecarboxamide

The title compound was prepared similarly to the procedure described in Example 242E substituting Example 380A for aniline and substituting 4-(trifluoromethyl)cyclohexanecarboxylic acid for Example 242D. MS (ESI) *m*/*z* 503.3 (M+H)⁺.

### Example 697

### butyl (3S,4R)-4-(4-fluorophenyl)-1-[(1-methyl-1H-imidazol-4-yl)sulfonyl]pyrrolidin-3-yl carbonate

### Example 697A

### Trans-tert-butyl 3-(butoxycarbonyloxy)-4-(4-fluorophenyl)pyrrolidine-1-carboxylate

The title compound was prepared similarly to the procedure described in Example 699A substituting butyl carbonochloridate for 1-isocyanatobutane.

### Example 697B

### Butyl-trans-4-(4-fluorophenyl)pyrrolidin-3-yl carbonate

The title compound was prepared similarly to the procedure described in Example 699B substituting Example 697A for Example 699A.

### Example 697C

### Butyl-trans-4-(4-fluorophenyl)-1-[(1-methyl-1H-imidazol-4-yl)sulfonyl]pyrrolidin-3-yl carbonate

The title compound was prepared similarly to the procedure described in Example 699C substituting Example 697B for Example 699B. MS (ESI) *m*/*z* 426.0 (M+H)⁺.

### Example 698

### 5,5,5-trifluoro-N-{(3R,4S)-4-(4-fluorophenyl)-1-[(1-methyl-1H-imidazol-4-yl)sulfonyl]pyrrolidin-3-yl}pentanamide

The title compound was prepared similarly to the procedure described in Example 242E substituting Example 380A for aniline and substituting 5,5,5-trifluoropentanoic acid for Example 242D. MS (ESI) *m*/*z* 463.1 (M+H)⁺.

### Example 699

### (3S,4R)-4-(4-fluorophenyl)-1-[(1-methyl-1H-imidazol-4-yl)sulfonyl]pyrrolidin-3-ylbutylcarbamate

### Example 699A

### Trans-tert-butyl 3-(butylcarbamoyloxy)-4-(4-fluorophenyl)pyrrolidine-1-carboxylate

To a solution of Example 379A (155 mg, 0.55 mmol) in dichloromethane (1.5 mL) was added triethylamine (167 mg, 1.65 mmol) and 1-isocyanatobutane (109 mg, 1.10 mmol). The solution was stirred at room temperature for 16 hours. The mixture was partitioned between dichloromethane and water. The organic fraction was collected, dried over sodium sulfate, concentrated, and purified by flash chromatography (25% ethyl acetate/hexanes) to provide the title compound.

### Example 699B

### Trans-4-(4-fluorophenyl)pyrrolidin-3-yl butylcarbamate

To a solution of Example 699A (85.5 mg, 0.23 mmol) in dichloromethane (1 mL) was added trifluoroacetic acid (0.4 mL). The mixture was stirred at room temperature for 1 hour, and was then concentrated to afford title compound.

### Example 699C

### Trans-4-(4-fluorophenyl)-1-[(1-methyl-1H-imidazol-4-yl)sulfonyl]pyrrolidin-3-ylbutylcarbamate

To a solution of Example 699B (63 mg, 0.23 mmol) in dichloromethane (1 mL) was added triethylamine (68.2 mg, 0,67 mmol) and 4-dimethylaminopyridine (1.4 mg, 0.011 mmol). The solution was cooled 0°C. To the chilled solution was added 1-methyl-1H-imidazole-4-sulfonyl chloride (42.6 mg, 0.24 mmol) portionwise. This mixture was slowly warmed up to room temperature over 1 hour and stirred at room temperature for 1 hour. The reaction mixture was partitioned with dichloromethane and water. The organic layer was collected, concentrated, and purified via HPLC to provide the title compound. MS (ESI) *m*/*z* 425.1 (M+H)⁺.

### Example 700

### 2,2-difluoro-N-{(3R,4S)-4-(4-fluorophenyl)-1-[(1-methyl-1H-imidazol-4-yl)sulfonyl]pyrrolidin-3-yl}pentanamide

The title compound was prepared similarly to the procedure described in Example 242E substituting Example 380A for aniline and substituting 2,2-difluorohexanoic acid for Example 242D. MS (ESI) *m*/*z* 459.2 (M+H)⁺.

### Example 701

### 1-(4-chlorophenyl)-3-{(3R,4S)-4-(4-fluorophenyl)-1-[(1-methyl-1H-imidazol-4-yl)sulfonyl]pyrrolidin-3-yl}urea

The title compound was prepared similarly to Example 692 substituting 1-chloro-4-isocyanatobenzene for isocyanatobenzene. MS (ESI) *m*/*z* 478 (M+H)⁺.

### Example 702

### 1-(2-fluorophenyl)-3-{(3R,4S)-4-(4-fluorophenyl)-1-[(1-methyl-1H-imidazol-4-yl)sulfonyl]pyrrolidin-3-yl}urea

The title compound was prepared similarly to Example 692 substituting 1-fluoro-2-isocyanatobenzene for isocyanatobenzene. MS (ESI) *m*/*z* 462 (M+H)⁺.

### Example 703

### 1-(3-fluorophenyl)-3-{(3R,4S)-4-(4-fluorophenyl)-1-[(1-methyl-1H-imidazol-4-yl)sulfonyl]pyrrolidin-3-yl}urea

The title compound was prepared similarly to Example 692 substituting 1-fluoro-3-isocyanatobenzene for isocyanatobenzene. MS (ESI) *m*/*z* 462 (M+H)⁺.

### Example 704

### 1-(4-fluorophenyl)-3-{(3R,4S)-4-(4-fluorophenyl)-1-[(1-methyl-1H-imidazol-4-yl)sulfonyl]pyrrolidin-3-yl}urea

The title compound was prepared similarly to Example 692 substituting 1-fluoro-4-isocyanatobenzene for isocyanatobenzene. MS (ESI) *m*/*z* 462 (M+H)⁺.

### Example 705

### 1-(2-chlorobenzyl)-3-{(3R,4S)-4-(4-fluorophenyl)-1-[(1-methyl-1H-imidazol-4-yl)sulfonyl]pyrrolidin-3-yl}urea

The title compound was prepared similarly to Example 692 substituting 1-chloro-2-(isocyanatomethyl)benzene for isocyanatobenzene. MS (ESI) *m*/*z* 492 (M+H)⁺.

### Example 706

### 4,5-dichloro-N-{(3R,4S)-4-(4-fluorophenyl)-1-[(1-methyl-1H-imidazol-4-yl)sulfonyl]pyrrolidin-3-yl}pyridin-2-amine

To a stirred solution of Example 322B (100 mg, 0.31 mmol) in dimethylsulfoxide(4 mL) was added 2,4,5-trichloropyridine (112 mg, 0.62 mmol) and sodium tert-butoxide (59 mg, .062 mmol). The reaction mixture was heated at 90 °C for 18 hours, then concentrated. Purification via HPLC provided the title compound. MS (ESI) *m*/*z* 470.5 (M+H)⁺.

### Example 707

### (3R,4S)-4-(4-fluorophenyl)-1-[(1-methyl-1H-imidazol-4-yl)sulfonyl]-N-{[4-(trifluoromethyl)cyclohexyl]methyl}pyrrolidin-3-amine

The title compound was prepared similarly to the procedure described in Example 290 substituting Example 696 for Example 242E. MS (ESI) *m*/*z* 489.1 (M+H)⁺.

### Example 708

### 2,2-difluoro-N-{(3R,4S)-4-(4-fluorophenyl)-1-[(1-methyl-1H-imidazol-4-yl)sulfonyl]pyrrolidin-3-yl}hexanamide

The title compound was prepared similarly to the procedure described in Example 242E substituting Example 380A for aniline and substituting 2,2-difluorohexanoic acid for Example 242D. MS (ESI) *m*/*z* 459.2 (M+H)⁺.

### Example 709

### Trans-N-[3,4-difluorophenyl]-1-[(1-methyl-1H-imidazol-4-yl)sulfonyl]-4-(1,3-oxazol-2-yl)pyrrolidin-3-amine

### Example 709A

### (E)-2-(2-nitrovinyl)oxazole

The title compound was prepared similarly to the procedure described in Example 601A substituting oxazole-2-carboxaldehyde for tetrahydrofuran-2-carboxaldehyde. MS (DCI) m/z 141.0 (M+H)⁺

### Example 709B

### (3R,4S)-1-(1-methyl-1H-imidazol-4-ylsulfonyl)-4-(oxazol-2-yl)pyrrolidin-3-amine

The title compound was prepared similarly to the procedures described in Examples 2A-2F substituting Example 709A for (E)-1-methoxy-4-(2-nitrovinyl)benzene.

### Example 709C

### Trans-N-[3,4-difluorophenyl]-1-[(1-methyl-1H-imidazol-4-yl)sulfonyl]-4-(1,3-oxazol-2-yl)pyrrolidin-3 -amine

The title compound was prepared similarly to the procedure described in Example 57 substituting Example 709C for Example 14A and substituting 1-bromo-3,4-difluorobenzene for 1-bromo-2-methylbenzene. MS (ESI) m/z 410.1 (M+H)⁺.

### Example 710

### N-[4-chloro-3-(trifluoromethyl)phenyl]-1-[(1-methyl-1H-imidazol-4-yl)sulfonyl]-4-(1,3-oxazol-2-yl)pyrrolidin-3-amine

The title compound was prepared as described in Example 709C substituting 1-bromo-4-chloro-3-(trifluoromethyl)benzene for 1-bromo-3,4-difluorobenzene. MS (ESI) m/z 476.0 (M+H)⁺

### Example 711

### 4-{[trans-3-[(4-fluorobenzyl)oxy]-4-(4-fluorophenyl)pyrrolidin-1-yl]sulfonyl}-1-methyl-1H-imidazole

The title compound was prepared similarly to the procedure described in Example 625A substituting Example 379C for Example 379A and substituting 4-fluorobenzyl bromide for 1-bromohexane. MS (ESI) *m*/*z* 434 (M+H)⁺.

### Example 712

### 4-{[trans-3-[(3-fluorobenzyl)oxy]-4-(4-fluorophenyl)pyrrolidin-1-yl]sulfonyl}-1-methyl-1H-imidazole

The title compound was prepared similarly to the procedure described in Example 625A substituting Example 379C for Example 379A and substituting 3-fluorobenzyl bromide for 1-bromohexane. MS (ESI) *m*/*z* 434 (M+H)⁺.

### Example 713

### 4-{[trans-3-(4-fluorophenyl)-4-{[4-(methylsulfonyl)benzyl]oxy}pyrrolidin-1-yl]sulfonyl}-1-methyl-1H-imidazole

The title compound was prepared similarly to the procedure described in Example 625A substituting Example 379C for Example 379A and substituting 4-methylsulfonylbenzyl bromide for 1-bromohexane. MS (ESI) *m*/*z* 494 (M+H)⁺.

### Example 714

### 4-{[trans-3-(4-fluorophenyl)-4-{[3-(methylsulfonyl)benzyl]oxy}pyrrolidin-1-yl]sulfonyl}-1-methyl-1H-imidazole

The title compound was prepared similarly to the procedure described in Example 625A substituting Example 379C for Example 379A and substituting 3-methylsulfonylbenzyl bromide for 1-bromohexane. MS (ESI) *m*/*z* 494 (M+H)⁺.

### Example 715

### 6-ethoxy-N-{(3R,4S)-4-(4-fluorophenyl)-1-[(1-methyl-1H-imidazol-4-yl)sulfonyl]pyrrolidin-3-yl}pyrimidin-4-amine

The title compound was prepared similarly to the procedure described in Example 620 substituting Example 691 for Example 606. MS (ESI) *m*/*z* 447 (M+H)⁺.

### Example 716

### N-{(3R,4S)-4-(4-fluorophenyl)-1-[(1-methyl-1H-imidazol-4-yl)sulfonyl]pyrrolidin-3-yl}-6-(propan-2-yloxy)pyrimidin-4-amine

The title compound was prepared similarly to the procedure described in Example 620 substituting Example 691 for Example 606 and substituting isopropanol for ethanol. MS (ESI) *m*/*z* 461 (M+H)⁺.

### Example 717

### 6-butoxy-N-{(3R,4S)-4-(4-fluorophenyl)-1-[(1-methyl-1H-imidazol-4-yl)sulfonyl]pyrrolidin-3-yl}pyrimidin-4-amine

The title compound was prepared similarly to the procedure described in Example 620 substituting Example 691 for Example 606 and substituting n-butanol for ethanol. MS (ESI) *m*/*z* 473 (M+H)⁺.

### Example 718

### N-{(3R,4S)-4-(4-fluorophenyl)-1-[(1-methyl-1H-imidazol-4-yl)sulfonyl]pyrrolidin-3-yl}-6-(2-methylpropoxy)pyrimidin-4-amine

The title compound was prepared similarly to the procedure described in Example 620 substituting Example 691 for Example 606 and substituting iso-butanol for ethanol. MS (ESI) *m*/*z* 473 (M+H)⁺.

### Example 719

### 6-(cyclobutyloxy)-N-{(3R,4S)-4-(4-fluorophenyl)-1-[(1-methyl-1H-imidazol-4-yl)sulfonyl]pyrrolidin-3-yl}pyrimidin-4-amine

The title compound was prepared similarly to the procedure described in Example 620 substituting Example 691 for Example 606 and substituting cyclobutanol for ethanol. MS (ESI) *m*/*z* 473 (M+H)⁺.

### Example 720

### 6-(cyclopentyloxy)-N-{(3R,4S)-4-(4-fluorophenyl)-1-[(1-methyl-1H-imidazol-4-yl)sulfonyl]pyrrolidin-3-yl}pyrimidin-4-amine

The title compound was prepared similarly to the procedure described in Example 620 substituting Example 691 for Example 606 and substituting cyclopentanol for ethanol. MS (ESI) *m*/*z* 487 (M+H)⁺.

### Example 721

### 6-(cyclohexyloxy)-N-{(3R,4S)-4-(4-fluorophenyl)-1-[(1-methyl-1H-imidazol-4-yl)sulfonyl]pyrrolidin-3-yl}pyrimidin-4-amine

The title compound was prepared similarly to the procedure described in Example 620 substituting Example 691 for Example 606 and substituting cyclohexanol for ethanol. MS (ESI) *m*/*z* 487 (M+H)⁺.

### Example 722

### N-{(3R,4S)-4-(4-fluorophenyl)-1-[(1-methyl-1H-imidazol-4-yl)sulfonyl]pyrrolidin-3-yl}-6-phenylpyrimidin-4-amine

A microwave vial was charged with Example 722 (50 mg, 0.11 mmol), phenylboronic acid (14 mg, 0.12 mmol), dicyclohexyl(2',6'-dimethoxy-[1,1'-biphenyl]-2-yl)phosphine (4.7 mg, 11 µmol), potassium carbonate (2M, 57 µL, 0.14 mmol)), palladium(II) acetate (1.3 mg, 5.7 µmol), and 1,2-dimethoxyethane (2 mL). The reaction mixture was heated to 100 °C in a microwave (Biotage Initiator™, maximum 400 Watts) for 10 minutes. The reaction mixture was concentrated. Purification via HPLC provided the title compound. MS (ESI) *m*/*z* 479 (M+H)⁺.

### Example 723

### N-{(3R,4S)-4-(4-fluorophenyl)-1-[(1-methyl-1H-imidazol-4-yl)sulfonyl]pyrrolidin-3-yl}-6-(2-methylphenyl)pyrimidin-4-amine

The title compound was prepared similarly to the procedure described in Example 722 substituting o-tolylboronic acid for phenylboronic acid. MS (ESI) *m*/*z* 493 (M+H)⁺.

### Example 724

### N-{(3R,4S)-4-(4-fluorophenyl)-1-[(1-methyl-1H-imidazol-4-yl)sulfonyl]pyrrolidin-3-yl}-6-(3-methylphenyl)pyrimidin-4-amine

The title compound was prepared similarly to the procedure described in Example 722 substituting m-tolylboronic acid for phenylboronic acid. MS (ESI) *m*/*z* 493 (M+H)⁺.

### Example 725

### N-{(3R,4S)-4-(4-fluorophenyl)-1-[(1-methyl-1H-imidazol-4-yl)sulfonyl]pyrrolidin-3-yl}-6-(4-methylphenyl)pyrimidin-4-amine

The title compound was prepared similarly to the procedure described in Example 722 substituting p-tolylboronic acid for phenylboronic acid. MS (ESI) *m*/*z* 493 (M+H)⁺.

### Example 726

### 6-(2-fluorophenyl)-N-{(3R,4S)-4-(4-fluorophenyl)-1-[(1-methyl-1H-imidazol-4-yl)sulfonyl]pyrrolidin-3-yl}pyrimidin-4-amine

The title compound was prepared similarly to the procedure described in Example 722 substituting 2-(fluorophenyl)boronic acid for phenylboronic acid. MS (ESI) *m*/*z* 497 (M+H)⁺.

### Example 727

### 6-(3-fluorophenyl)-N-{(3R,4S)-4-(4-fluorophenyl)-1-[(1-methyl-1H-imidazol-4-yl)sulfonyl]pyrrolidin-3-yl}pyrimidin-4-amine

The title compound was prepared similarly to the procedure described in Example 722 substituting 3-(fluorophenyl)boronic acid for phenylboronic acid. MS (ESI) *m*/*z* 497 (M+H)⁺.

### Example 728

### 6-(4-fluorophenyl)-N-{(3R,4S)-4-(4-fluorophenyl)-1-[(1-methyl-1H-imidazol-4-yl)sulfonyl]pyrrolidin-3-yl}pyrimidin-4-amine

The title compound was prepared similarly to the procedure described in Example 722 substituting 4-(fluorophenyl)boronic acid for phenylboronic acid. MS (ESI) *m*/*z* 497 (M+H)⁺.

### Example 729

### 6-(3-chlorophenyl)-N-{(3R,4S)-4-(4-fluorophenyl)-1-[(1-methyl-1H-imidazol-4-yl)sulfonyl]pyrrolidin-3-yl}pyrimidin-4-amine

The title compound was prepared similarly to the procedure described in Example 722 substituting 3-(chlorophenyl)boronic acid for phenylboronic acid. MS (ESI) *m*/*z* 513 (M+H)⁺.

### Example 730

### 6-(4-chlorophenyl)-N-{(3R,4S)-4-(4-fluorophenyl)-1-[(1-methyl-1H-imidazol-4-yl)sulfonyl]pyrrolidin-3-yl}pyrimidin-4-amine

The title compound was prepared similarly to the procedure described in Example 722 substituting 4-(chlorophenyl)boronic acid for phenylboronic acid. MS (ESI) *m*/*z* 513 (M+H)⁺.

### Example 731

### 4-({(3S,4R)-3-(4-fluorophenyl)-4-[(4-methylbenzyl)oxy]pyrrolidin-1-yl}sulfonyl)-1-methyl-1H-imidazole

The title compound was prepared similarly to the procedure described in Example 625A substituting Example 379C for Example 379A and substituting 4-methylbenzyl bromide for 1-bromohexane. MS (ESI) *m*/*z* 430 (M+H)⁺.

### Example 732

### (3R,4S)-4-(4-fluorophenyl)-1-[(1-methyl-1H-imidazol-4-yl)sulfonyl]-N-(5,5,5-trifluoropentyl)pyrrolidin-3 -amine

The title compound was prepared similarly to the procedure described in Example 290 substituting Example 698 for Example 242E. MS (ESI) *m*/*z* 449.1 (M+H)⁺.

### Example 733

### (3R,4S)-N-(2,2-difluorohexyl)-4-(4-fluorophenyl)-1-[(1-methyl-1H-imidazol-4-yl)sulfonyl]pyrrolidin-3 -amine

The title compound was prepared similarly to the procedure described in Example 290 substituting Example 700 for Example 242E. MS (ESI) *m*/*z* 445.1 (M+H)⁺.

### Example 734

### (3R,4S)-N-(2,2-difluoropentyl)-4-(4-fluorophenyl)-1-[(1-methyl-1H-imidazol-4-yl)sulfonyl]pyrrolidin-3-amine

The title compound was prepared similarly to the procedure described in Example 290 substituting Example 700 for Example 242E. MS (ESI) *m*/*z* 431.1 (M+H)⁺.

### Example 735

### 5,5-difluoro-N-{(3R,4S)-4-(4-fluorophenyl)-1-[(1-methyl-1H-imidazol-4-yl)sulfonyl]pyrrolidin-3-yl}hexanamide

To a solution of Example 695 (200 mg, 0.46 mmol) in dichloromethane (2.5 mL) at 0°C was added diethylaminosulfur trifluoride (258 mg, 1.60 mmol)., The reaction mixture stirred at 0°C for 1 hour and then room temoperature for 2 days. The reaction mixuture was partitioned between dichloromethane and aqueous sodium bicarbonate. The organic fraction was collected, washed with water, concentrated, and purified by HPLC to provide the title compound. MS (ESI) *m*/*z* 459.1 (M+H)⁺.

### Example 736

### 4-fluoro-N-{(3R,4S)-4-(4-fluorophenyl)-1-[(1-methyl-1H-imidazol-4-yl)sulfonyl]pyrrolidin-3-yl}pyridin-2-amine

The title compound was prepared similarly to the procedure described in Example 327 substituting 2-chloro-4-fluoropyridine for 1-bromo-3-(trifluoromethyl)benzene. MS (DCI) *m*/*z* 420.1 (M+H)⁺.

### Example 737

### 5-fluoro-N-{(3R,4S)-4-(4-fluorophenyl)-1-[(1-methyl-1H-imidazol-4-yl)sulfonyl]pyrrolidin-3-yl}pyridin-2-amine

The title compound was prepared similarly to the procedure described in Example 327 substituting 2-chloro-5-fluoropyridine for 1-bromo-3-(trifluoromethyl)benzene. MS (DCI) *m*/*z* 420 (M+H)⁺.

### Example 738

### N-{(3R,4S)-4-(4-fluorophenyl)-1-[(1-methyl-1H-imidazol-4-yl)sulfonyl]pyrrolidin-3-yl}-4-oxopentanamide

The title compound was prepared similarly to the procedure described in Example 242E substituting Example 380A for aniline and substituting 4-oxopentanoic acid for Example 242D. MS (ESI) *m*/*z* 423.0 (M+H)⁺.

### Example 739

### (3R,4S)-N-(5,5-difluorohexyl)-4-(4-fluorophenyl)-1-[(1-methyl-1H-imidazol-4-yl)sulfonyl]pyrrolidin-3-amine

The title compound was prepared similarly to the procedure described in Example 290 substituting Example 735 for Example 242E. MS (ESI) *m*/*z* 445.2 (M+H)⁺

### Example 740

### 4-chloro-N-[2-({(3R,4S)-4-(4-fluorophenyl)-1-[(1-methyl-1H-imidazol-4-yl)sulfonyl]pyrrolidin-3-yl}oxy)ethyl]aniline

### Example 740A

### N-(4-fluorophenyl)-2-((trans-4-(4-fluorophenyl)-1-((1-methyl-1H-imidazol-4-yl)sulfonyl)pyrrolidin-3-yl)oxy)acetamide

The title compound was prepared similarly to the procedure described in Example 404A substituting 4-fluoroaniline for Example 14A and substituting Example 689D for 2-nitro-5-(trifluoromethyl)benzoic acid.

### Example 740B

### 4-chloro-N-[2-({Trans-4-(4-fluorophenyl)-1-[(1-methyl-1H-imidazol-4-yl)sulfonyl]pyrrolidin-3-yl}oxy)ethyl]aniline

The title compound was prepared similarly to the procedure described in Example 404B substituting Example 740A for Example 404A. MS (ESI) *m*/*z* 479/481 (3:1) (M+H)⁺.

### Example 741

### (3S,4R)-N-[4-fluoro-3-(trifluoromethoxy)phenyl]-1-[(1-methyl-1H-imidazol-4-yl)sulfonyl]-4-(tetrahydro-2H-pyran-2-yl)pyrrolidin-3-amine

The title compound was prepared similarly to the procedure described in Example 337G substituting Example 679H for Example 337F and substituting 4-bromo-1-fluoro-2-(trifluoromethoxy)benzene for 1-bromo-3-(trifluoromethyl)benzene. MS (ESI) *m*/*z* 493 (M+H)⁺.

### Example 742

### 1-benzyl-3-{(3R,4S)-4-(4-fluorophenyl)-1-[(1-methyl-1H-imidazol-4-yl)sulfonyl]pyrrolidin-3-yl}urea

The title compound was prepared similarly to the procedure described in Example 692 substituting (isocyanatomethyl)benzene for isocyanatobenzene. MS (ESI) *m*/*z* 458 (M+H)⁺.

### Example 743

### (3R,4S)-N-(4-chloro-3-methylphenyl)-4-(4-fluorophenyl)-1-[(1-methyl-1H-imidazol-4-yl)sulfonyl]pyrrolidin-3-amine

The title compound was prepared similarly to Example 327 substituting 1-bromo-4-chloro-3-methylbenzene for 1-bromo-3-(trifluoromethyl)benzene. MS (ESI) *m*/*z* 449 (M+H)⁺.

### Example 744

### (3R,4S)-4-(4-fluorophenyl)-1-[(1-methyl-1H-imidazol-4-yl)sulfonyl]-N-[3-(2,2,2-trifluoroethoxy)phenyl]pyrrolidin-3-amine

The title compound was prepared similarly to Example 327 substituting 1-bromo-3-(2,2,2-trifluoroethoxy)benzene for 1-bromo-3-(trifluoromethyl)benzene. MS (ESI) *m*/*z* 499 (M+H)⁺.

### Example 745

### (3R,4S)-4-(4-fluorophenyl)-N-[4-fluoro-3-(pyridin-3-yl)phenyl]-1-[(1-methyl-1H-imidazol-4-yl)sulfonyl]pyrrolidin-3-amine

A solution of Example 352 (100 mg, 0.22 mmol), pyridine-3-ylboronic acid (41 mg, 0.33 mmol), tricyclohexylphosphine (6.2 mg, 22 µmol), potassium phosphate (1.2 M (aq.), 0.36 mL, 442 mmol)) and palladium(II) acetate (10 mg, 11 µmol) in dioxane (2 mL) was stirred at 100 °C for 18 hours. The reaction mixture was concentrated. Purification via HPLC provided the title compound. MS (ESI) *m*/*z* 496 (M+H)⁺.

### Example 746

### (3R,4S)-4-(4-fluorophenyl)-N-[4-fluoro-3-(pyridin-4-yl)phenyl]-1-[(1-methyl-1H-imidazol-4-yl)sulfonyl]pyrrolidin-3-amine

The title compound was prepared similarly to the procedure described in Example 745 substituting pyridin-4-ylboronic acid for pyridine-3-ylboronic acid. MS (ESI) *m*/*z* 496 (M+H)⁺.

### Example 747

### (3R,4S)-N-(4-fluoro-3-methylphenyl)-4-(4-fluorophenyl)-1-[(1-methyl-1H-imidazol-4-yl)sulfonyl]pyrrolidin-3-amine

The title compound was prepared similarly to the procedure described in Example 327 substituting 1-bromo-4-fluoro-3-methylbenzene for 1-bromo-3-(trifluoromethyl)benzene. MS (ESI) *m*/*z* 433 (M+H)⁺.

### Example 748

### 5-fluoro-N-{(3R,4S)-4-(4-fluorophenyl)-1-[(1-methyl-1H-imidazol-4-yl)sulfonyl]pyrrolidin-3-yl}-4-methylpyridin-2-amine

The title compound was prepared similarly to the procedure described in Example 327 substituting 2-bromo-5-fluoro-4-methylpyridine for 1-bromo-3-(trifluoromethyl)benzene. MS (ESI) *m*/*z* 434 (M+H)⁺.

### Example 749

### (3R,4S)-4-(4-fluorophenyl)-N-[4-fluoro-3-(pyrimidin-5-yl)phenyl]-1-[(1-methyl-1H-imidazol-4-yl)sulfonyl]pyrrolidin-3-amine

The title compound was prepared similarly to the procedure described in Example 745 substituting pyrimidin-5-ylboronic acid for pyridine-3-ylboronic acid. MS (ESI) *m*/*z* 497 (M+H)⁺.

### Example 750

### N-{(3R,4S)-4-(4-fluorophenyl)-1-[(1-methyl-1H-imidazol-4-yl)sulfonyl]pyrrolidin-3-yl}-3,4'-bipyridin-2'-amine

The title compound was prepared similarly to the procedure described in Example 745 substituting Example 759 for Example 352. MS (ESI) *m*/*z* 479 (M+H)⁺.

### Example 751

### N-{(3R,4S)-4-(4-fluorophenyl)-1-[(1-methyl-1H-imidazol-4-yl)sulfonyl]pyrrolidin-3-yl}-4,4'-bipyridin-2-amine

The title compound was prepared similarly to the procedure described in Example 745 substituting Example 759 for Example 352 and substituting pyridin-4-ylboronic acid for pyridine-3-ylboronic acid. MS (ESI) *m*/*z* 479 (M+H)⁺.

### Example 752

### 4-({(3S,4R)-4-(4-fluorophenyl)-1-[(1-methyl-1H-imidazol-4-yl)sulfonyl]pyrrolidin-3-yl}oxy)-6-(trifluoromethyl)pyrimidine

The title compound was prepared similarly to the procedure described in Example 379D substituting 4-bromo-6-(trifluoromethyl)pyrimidine for 2-fluoro-4-(trifluoromethyl)pyridine. MS (ESI) *m*/*z* 472 (M+H)⁺.

### Example 753

### chloro-3-(trifluoromethyl)phenyl]-1-[(1-methyl-1H-imidazol-4-yl)sulfonyl]-4-(1,3-oxazol-4-yl)pyrrolidin-3-amine

### Example 753A

### (E)-4-(2-nitrovinyl)oxazole

The title compound was prepared similarly to the procedure described in Example 601A substituting 4-oxazolecarboxaldehyde for tetrahydrofuran-2-carboxaldehyde.

### Example 753B

### (trans)-1-(1-methyl-1H-imidazol-4-ylsulfonyl)-4-(oxazol-4-yl)pyrrolidin-3-amine

The title compound was prepared similarly to the procedures described in Exmaple 2A-2F substituting Example 753A for (E)-1-methoxy-4-(2-nitrovinyl)benzene.

### Example 753C

### chloro-3-(trifluoromethyl)phenyl]-1-[(1-methyl-1H-imidazol-4-yl)sulfonyl]-4-(1,3-oxazol-4-yl)pyrrolidin-3-amine

The title compound was prepared similarly to the procedure described in Example 57 substituting Example 753B for Example 14a and substituting 1-bromo-4-chloro-3-(trifluoromethyl)benzene for 1-bromo-2-methylbenzene. MS (ESI) *m*/*z* 476.0 (M+H)⁺.

### Example 754

### 4,4-difluoro-N-{(3R,4S)-4-(4-fluorophenyl)-1-[(1-methyl-1H-imidazol-4-yl)sulfonyl]pyrrolidin-3-yl}pentanamide

The title compound was prepared similarly to the procedure described in Example 735 substituting Example 738 for Example 695. MS (ESI) *m*/*z* 445.1 (M+H)⁺.

### Example 755

### (3R,4S)-N-(3,4-difluorophenyl)-1-[(1,2-dimethyl-1H-imidazol-4-yl)sulfonyl]-4-(4-fluorophenyl)pyrrolidin-3-amine

### Example 755 A

### (3R,4S)-1-(1,2-dimethyl-1H-imidazol-4-ylsulfonyl)-4-(4-fluorophenyl)pyrrolidin-3-amine hydrochloride

The title compound was prepared similarly to the procedures described in Example 2A-2F substituting (E)-1-fluoro-4-(2-nitrovinyl)benzene for (E)-1-methoxy-4-(2-nitrovinyl)benzene and substituting 1,2-dimethyl-1H-imidazole-4-sulfonyl chloride for 1-methyl-1H-imidazole-4-sulfonyl chloride.

### Example 755 B

### (3R,4S)-1-(1,2-dimethyl-1H-imidazol-4-ylsulfonyl)-4-(4-fluorophenyl)pyrrolidin-3-amine

The title compound was prepared similarly to the procedure described in Example 380A substituting Example 755 A for Example 322B. MS (ESI) *m*/*z* 339.1 (M+H)⁺.

### Example 755 C

### (3R,4S)-N-(3,4-difluorophenyl)-1-[(1,2-dimethyl-1H-imidazol-4-yl)sulfonyl]-4-(4-fluorophenyl)pyrrolidin-3-amine

The title compound was prepared similarly to the procedure described in Example 337G substituting Example 755B for Example 337F and substituting 4-bromo-1,2-difluorobenzene for 1-bromo-3-(trifluoromethyl)benzene. MS (ESI) *m*/*z* 451.2 (M+H)⁺.

### Example 756

### (3R,4S)-1-[(1,2-dimethyl-1H-imidazol-4-yl)sulfonyl]-4-(4-fluorophenyl)-N-[4-fluoro-3-(trifluoromethyl)phenyl]pyrrolidin-3-amine

The title compound was prepared similarly to the procedure described in Example 337G substituting Example 755B for Example 337F and substituting 4-bromo-1-fluoro-2-(trifluoromethyl)benzene for 1-bromo-3-(trifluoromethyl)benzene. MS (ESI) *m*/*z* 501.1 (M+H)⁺.

### Example 757

### (3R,4S)-N-(3,4-difluorophenyl)-1-[(1,2-dimethyl-1H-imidazol-5-yl)sulfonyl]-4-(4-fluorophenyl)pyrrolidin-3-amine

### Example 757 A

### (3R,4S)-1-(1,2-dimethyl-1H-imidazol-5-ylsulfonyl)-4-(4-fluorophenyl)pyrrolidin-3-amine hydrochloride

The title compound was prepared similarly to the conditions described in Example 2A-2F substituting (E)-1-fluoro-4-(2-nitrovinyl)benzene for (E)-1-methoxy-4-(2-nitrovinyl)benzene and substituting 1,2-dimethyl-1H-imidazole-5-sulfonyl chloride for 1-methyl-1H-imidazole-4-sulfonyl chloride.

### Example 757 B

### (3R,4S)-1-(1,2-dimethyl-1H-imidazol-5-ylsulfonyl)-4-(4-fluorophenyl)pyrrolidin-3-amine

The title compound was prepared similarly to the procedure described in Example 380A substituting Example 757A for Example 322B. MS (ESI) *m*/*z* 339.1 (M+H)⁺.

### Example 757 C

### (3R,4S)-N-(3,4-difluorophenyl)-1-[(1,2-dimethyl-1H-imidazol-5-yl)sulfonyl]-4-(4-fluorophenyl)pyrrolidin-3-amine

The title compound was prepared similarly to the procedure described in Example 337G substituting Example 757B for Example 337F and substituting 4-bromo-1,2-difluorobenzene for 1-bromo-3-(trifluoromethyl)benzene. MS (ESI) *m*/*z* 451.2 (M+H)⁺

### Example 758

### (3R,4S)-1-[(1,2-dimethyl-1H-imidazol-5-yl)sulfonyl]-4-(4-fluorophenyl)-N-[4-fluoro-3-(trifluoromethyl)phenyl]pyrrolidin-3-amine

The title compound was prepared similarly to the procedure described in Example 337G substituting Example 757B for Example 337F and substituting 4-bromo-1-fluoro-2-(trifluoromethyl)benzene for 1-bromo-3-(trifluoromethyl)benzene. MS (ESI) *m*/*z* 501.1 (M+H)⁺

### Example 759

### 4-chloro-N-{(3R,4S)-4-(4-fluorophenyl)-1-[(1-methyl-1H-imidazol-4-yl)sulfonyl]pyrrolidin-3-yl}pyridin-2-amine

The title compound was prepared similarly to the procedure described in Example 327 substituting 2-bromo-4-chloropyrdine for 1-bromo-3-(trifluoromethyl)benzene. MS (ESI) *m*/*z* 436 (M+H)⁺.

### Example 760

### 5-chloro-N-[(3R,4S)-1-[(1,2-dimethyl-1H-imidazol-4-yl)sulfonyl]-4-(4-fluorophenyl)pyrrolidin-3-yl]-4-(trifluoromethyl)pyridin-2-amine

The title compound was prepared similarly to the procedure described in Example 848 substituting Example 755B for Example 679H. MS (ESI) *m*/*z* 518.1 (M+H)⁺

### Example 761

### Trans-chloro-4-(trifluoromethyl)benzyl]-4-(3-fluoropyridin-2-yl)-1-[(1-methyl-1H-imidazol-4-yl)sulfonyl]pyrrolidin-3 -amine

### Example 761A

### Trans-4-(3-fluoropyridin-2-yl)-1-(1-methyl-1H-imidazol-4-ylsulfonyl)pyrrolidin-3-amine hydrochloride

The title compound was prepared similarly to the procedures described in Examples 2A-2F substituting (E)-3-fluoro-2-(2-nitrovinyl)pyridine for (E)-1-methoxy-4-(2-nitrovinyl)benzene.

### Example 761B

### Trans-4-(3-fluoropyridin-2-yl)-1-(1-methyl-1H-imidazol-4-ylsulfonyl)pyrrolidin-3-amine

The title compound was prepared similarly to the procedure described in Example 380A substituting Example 761A for Example 322B.

### Example 761 C

### Trans-N-[2-chloro-4-(trifluoromethyl)benzyl]-4-(3-fluoropyridin-2-yl)-1-[(1-methyl-1H-imidazol-4-yl)sulfonyl]pyrrolidin-3-amine

The title compound was prepared similarly to the procedure described in Example 354E substituting 2-chloro-4-(trifluoromethyl)benzaldehyde for 4-chlorobenzaldehyde, Example 761 A for 354D amine. MS (ESI) *m*/*z* 517.9 (M+H)⁺

### Example 762

### Trans-4-(3-fluoropyridin-2-yl)-N-hexyl-1-[(1-methyl-1H-imidazol-4-yl)sulfonyl]pyrrolidin-3-amine

The title compound was prepared similarly to the procedure described in Example 354E substituting hexanal for 4-chlorobenzaldehyde and substituting Example 761 A for 354D amine. MS (ESI) *m*/*z* 410.1 (M+H)⁺.

### Example 763

### Trans-4-(3-fluoropyridin-2-yl)-N,N-dihexyl-1-[(1-methyl-1H-imidazol-4-yl)sulfonyl]pyrrolidin-3-amine

The title compound was prepared similarly to the procedure described in Example 354E substituting hexanal for 4-chlorobenzaldehyde and substituting Example 761A for 354D. MS (ESI) *m*/*z* 494.1 (M+H)⁺

### Example 764

### Trans-4-(3-fluoropyridin-2-yl)-1-[(1-methyl-1H-imidazol-4-yl)sulfonyl]-N,N-dipentylpyrrolidin-3-amine

The title compound was prepared similarly to the procedure described in Example 354E substituting pentanal for 4-chlorobenzaldehyde and substituting Example 761 A for 354D. MS (ESI) *m*/*z* 466.1 (M+H)⁺

### Example 765

### Trans-4-(3-fluoropyridin-2-yl)-1-[(1-methyl-1H-imidazol-4-yl)sulfonyl]-N-pentylpyrrolidin-3-amine

The title compound was prepared similarly to the procedure described in Example 354E substituting pentanal for 4-chlorobenzaldehyde and substituting Example 761 A for 354D. MS (ESI) *m*/*z* 396.1 (M+H)⁺

### Example 766

### Trans-N-(3,4-difluorophenyl)-4-(3-fluoropyridin-2-yl)-1-[(1-methyl-1H-imidazol-4-yl)sulfonyl]pyrrolidin-3-amine

The title compound was prepared similarly to the procedure described in Example 337G substituting Example 761 B for Example 337F and substituting 4-bromo-1,2-difluorobenzene for 1-bromo-3-(trifluoromethyl)benzene. MS (ESI) *m*/*z* 438.0 (M+H)⁺

### Example 767

### Trans-4-(3-fluoropyridin-2-yl)-N-[4-fluoro-3-(trifluoromethyl)phenyl]-1-[(1-methyl-1H-imidazol-4-yl)sulfonyl]pyrrolidin-3-amine

The title compound was prepared similarly to the procedure described in Example 337G substituting Example 761B for Example 337F and substituting 4-bromo-1-fluoro-2-(trifluoromethyl)benzene for 1-bromo-3-(trifluoromethyl)benzene. MS (ESI) *m*/*z* 488.0 (M+H)⁺

### Example 768

### Trans-4-(3-fluoropyridin-2-yl)-N-[4-fluoro-3-(trifluoromethoxy)phenyl]-1-[(1-methyl-1H-imidazol-4-yl)sulfonyl]pyrrolidin-3-amine

The title compound was prepared similarly to the procedure described in Example 337G substituting Example 761B for Example 337F and substituting 4-bromo-1-fluoro-2-(trifluoromethoxy)benzene for 1-bromo-3-(trifluoromethyl)benzene. MS (ESI) *m*/*z* 504.0 (M+H)⁺

### Example 769

### 5-fluoro-N-{trans-4-(3-fluoropyridin-2-yl)-1-[(1-methyl-1H-imidazol-4-yl)sulfonyl]pyrrolidin-3-yl}-4-methylpyridin-2-amine

The title compound was prepared similarly to the procedure described in Example 337G substituting Example 761B for Example 337F and 2-bromo-5-fluoro-4-methylpyridine for 1-bromo-3-(trifluoromethyl)benzene. MS (ESI) *m*/*z* 435.1 (M+H)⁺

### Example 770

### N-{Trans-4-(3-fluoropyridin-2-yl)-1-[(1-methyl-1H-imidazol-4-yl)sulfonyl]pyrrolidin-3-yl}-6-(trifluoromethyl)pyrimidin-4-amine

The title compound was prepared similarly to the procedure described in Example 337G substituting Example 761B for Example 337F and substituting 4-bromo-6-(trifluoromethyl)pyrimidine for 1-bromo-3-(trifluoromethyl)benzene. MS (ESI) *m*/*z* 472.0 (M+H)⁺

### Example 771

### Trans-N-(3-chloro-4-fluorophenyl)-4-(3-fluoropyridin-2-yl)-1-[(1-methyl-1H-imidazol-4-yl)sulfonyl]pyrrolidin-3-amine

The title compound was prepared similarly to the procedure described in Example 337G substituting Example 761B for Example 337F and substituting 4-bromo-2-chloro-1-fluorobenzene for 1-bromo-3-(trifluoromethyl)benzene. MS (ESI) *m*/*z* 453.9 (M+H)⁺

### Example 772

### 6-chloro-N-{trans-4-(3-fluoropyridin-2-yl)-1-[(1-methyl-1H-imidazol-4-yl)sulfonyl]pyrrolidin-3-yl}pyrimidin-4-amine

To a microwave vial charged with Example 761B (100 mg, 0.31 mmol) and methanol (1 mL) was added triethylamine (62.2 mg, 0.62 mmol) and 4,6-dichloropyrimidine (137 mg, 0.93 mmol). The reaction mixture was stirred in a microwave at 120 °C for 1 hour. The mixture was concentrated and purified via HPLC to afford the title compound. MS (ESI) *m*/*z* 438.0 (M+H)⁺

### Example 773

### (3R,4S)-N-[3-(benzyloxy)phenyl]-1-[(1-methyl-1H-imidazol-4-yl)sulfonyl]-4-phenylpyrrolidin-3-amine

The title compound was prepared similarly to the procedure described in Example 327 substituting Example 574A for Example322B and substituting 1-(benzyloxy)-3-bromobenzene for 1-bromo-3-(trifluoromethyl)benzene. MS (APCI) *m*/*z* 489 (M+H)⁺.

### Example 774

### 6-(benzyloxy)-N-{(3R,4S)-4-(4-fluorophenyl)-1-[(1-methyl-1H-imidazol-4-yl)sulfonyl]pyrrolidin-3-yl}pyrimidin-4-amine

The title compound was prepared similarly to the procedure described in Example 327 substituting 4-(benzyloxy)-6-bromopyrimidine for 1-bromo-3-(trifluoromethyl)benzene. MS (ESI) *m*/*z* 509 (M+H)⁺.

### Example 775

### Trans-N-(4-fluoro-3-methylphenyl)-4-(3-fluoropyridin-2-yl)-1-[(1-methyl-1H-imidazol-4-yl)sulfonyl]pyrrolidin-3-amine

The title compound was prepared similarly to the procedure described in Example 337G substituting Example 761B for Example 337F and substituting 4-bromo-1-fluoro-2-methylbenzene for 1-bromo-3-(trifluoromethyl)benzene. MS (ESI) *m*/*z* 434.0 (M+H)⁺

### Example 776

### 4-(benzyloxy)-5-fluoro-N-{(3R,4S)-1-[(1-methyl-1H-imidazol-4-yl)sulfonyl]-4-phenylpyrrolidin-3-yl}pyridin-2-amine

### Example 776A

### 4-(benzyloxy)-2-bromo-5-fluoropyridine

To a stirred solution of 2-bromo-5-fluoropyridin-4-ol (100 mg, 0.52 mmol) in dimethylformamide (4 mL) was added (bromomethyl)benzene (89 mg, 0.52 mmol) and potassium carbonate (144 mg, 1.0 mmol). The reaction mixture was stirred at 60 °C for 3 hours. Water and EtOAc were added. The organic fraction was collected and concentrated. Purification via flash chromatography (0-50% EtOAc/heptanes) provided the title compound.

### Example 776B

### 4-(benzyloxy)-5-fluoro-N-{(3R,4S)-1-[(1-methyl-1H-imidazol-4-yl)sulfonyl]-4-phenylpyrrolidin-3-yl}pyridin-2-amine

The title compound was prepared similarly to the procedure described in Example 327 substituting Example 574A for Example 322B and substituting Example 776A for 1-bromo-3-(trifluoromethyl)benzene. MS (ESI) *m*/*z* 508 (M+H)⁺.

### Example 777

### (3R,4S)-1-[(1-methyl-1H-imidazol-4-yl)sulfonyl]-4-phenyl-N-[3-(piperidin-4-yl)phenyl]pyrrolidin-3-amine

The title compound was prepared similarly to the procedure described in Example 327 substituting Example 574A for Example 322B and substituting tert-butyl 4-(3-bromophenyl)piperidine-1-carboxylate for 1-bromo-3-(trifluoromethyl)benzene followed by dissolving the crude mixture in dichloromethane (5 mL) and treating the crude mixture with HCl (4 M, 3 mL) and purifying via HPLC. MS (APCI) *m*/*z* 466 (M+H)⁺.

### Example 778

### 6-(azetidin-3-ylmethoxy)-N-{(3R,4S)-4-(4-fluorophenyl)-1-[(1-methyl-1H-imidazol-4-yl)sulfonyl]pyrrolidin-3-yl}pyrimidin-4-amine

Tert-butyl 3-((6-((3R,4S)-4-(4-fluorophenyl)-1-(1-methyl-1H-imidazol-4-ylsulfonyl)pyrrolidin-3-ylamino)pyrimidin-4-yloxy)methyl)azetidine-1-carboxylate was prepared similarly to the procedure described in Example 691 substituting Example 606 for Example 691 and substituting tert-butyl 3-(hydroxymethyl)azetidine-1-carboxylate (3 eq.) for ethanol and adding tert-butanol (5 mL). The reaction mixture was concentrated, dissolved in dichloromethane, and treated with HCl (4 M, 10 eq.). Purification via HPLC provided the title compound. MS (ESI) *m*/*z* 488 (M+H)⁺.

### Example 779

### N-{(3R,4S)-4-(4-fluorophenyl)-1-[(1-methyl-1H-imidazol-4-yl)sulfonyl]pyrrolidin-3-yl}-6-(pyrrolidin-3-ylmethoxy)pyrimidin-4-amine

The title compound was prepared similarly to the procedure described in Example 778 substituting tert-butyl-3-hydroxymethyl-pyrrolidine-1-carboxylate for tert-butyl 3-(hydroxymethyl)azetidine-1-carboxylate. MS (ESI) *m*/*z* 502 (M+H)⁺.

### Example 780

### N-{(3R,4S)-4-(4-fluorophenyl)-1-[(1-methyl-1H-imidazol-4-yl)sulfonyl]pyrrolidin-3-yl}-6-(pyrrolidin-2-ylmethoxy)pyrimidin-4-amine

The title compound was prepared similarly to the procedure described in Example 778 substituting tert-butyl 2-(hydroxymethyl)pyrrolidine-1-carboxylate for tert-butyl 3-(hydroxymethyl)azetidine-1-carboxylate. MS (ESI) *m*/*z* 502 (M+H)⁺.

### Example 781

### trans-1-[(1-methyl-1H-imidazol-4-yl)sulfonyl]-N-(3-methylphenyl)-4-(tetrahydro-2H-pyran-3-yl)pyrrolidin-3-amine

### Example 781A

### (E)-3-(2-nitrovinyl)tetrahydro-2H-pyran

The title compound was prepared similarly to the procedure described in Example 307A substituting tetrahydropyran-3-carbaldehyde for 2-pyridinecarboxaldeyde.

### Example 781B

### trans-1-(1-methyl-1H-imidazol-4-ylsulfonyl)-4-((S)-tetrahydro-2H-pyran-3-yl)pyrrolidin-3-amine

The title compound was prepared as an HCl salt similarly to the procedures described in Examples 2A-2F substituting Example 781A for (E)-1-methoxy-4-(2-nitrovinyl)benzene.

### Example 781C

### trans -1-[(1-methyl-1H-imidazol-4-yl)sulfonyl]-N-(3-methylphenyl)-4-(tetrahydro-2H-pyran-3-yl)pyrrolidin-3-amine

The title compound was prepared similarly to the procedure described in Example 327 substituting Example 781B for Example 322B and substituting 1-bromo-3-methylbenzene for 1-bromo-3-(trifluoromethyl)benzene. MS (ESI) *m*/*z* 405 (M+H)⁺.

### Example 782

### trans-1-[(1-methyl-1H-imidazol-4-yl)sulfonyl]-4-(tetrahydro-2H-pyran-3-yl)-N-[3-(trifluoromethyl)phenyl]pyrrolidin-3-amine

The title compound was prepared similarly to the procedure described in Example 327 substituting Example 781B for Example 322B.. MS (ESI) *m*/*z* 459 (M+H)⁺.

### Example 783

### trans -N-(3 -chlorophenyl)-1-[(1-methyl-1H-imidazol-4-yl)sulfonyl] -4-(tetrahydro-2H-pyran-3 - yl)pyrrolidin-3-amine

The title compound was prepared similarly to the procedure described in Example 327 substituting Example 781B for Example 322B and substituting 1-bromo-3-chlorobenzene for 1-bromo-3-(trifluoromethyl)benzene. MS (ESI) *m*/*z* 425 (M+H)⁺.

### Example 784

### trans-1-[(1-methyl-1H-imidazol-4-yl)sulfonyl]-4-[(3S)-tetrahydro-2H-pyran-3-yl]-N-[3-(trifluoromethoxy)phenyl]pyrrolidin-3-amine

The title compound was prepared similarly to the procedure described in Example 327 substituting Example 781B for Example 322B and substituting 1-bromo-3-(trifluoromethoxy)benzene for 1-bromo-3-(trifluoromethyl)benzene. MS (ESI) *m*/*z* 475 (M+H)⁺.

### Example 785

### trans -1-[(1-methyl-1H-imidazol-4-yl)sulfonyl]-4-[(3S)-tetrahydro-2H-pyran-3-yl]-N-[3-(2,2,2-trifluoroethoxy)phenyl]pyrrolidin-3-amine

The title compound was prepared similarly to the procedure described in Example 327 substituting Example 781B for Example 322B and substituting 1-bromo-3-(2,2,2-trifluoroethoxy)benzene for 1-bromo-3-(trifluoromethyl)benzene. MS (ESI) *m*/*z* 489 (M+H)⁺.

### Example 786

### trans-1-[(1-methyl-1H-imidazol-4-yl)sulfonyl]-N-(3-phenoxyphenyl)-4-[(3S)-tetrahydro-2H-pyran-3-yl]pyrrolidin-3 -amine

The title compound was prepared similarly to the procedure described in Example 327 substituting Example 781B for Example 322B and substituting 1-bromo-3-phenoxybenzene for 1-bromo-3-(trifluoromethyl)benzene. MS (ESI) *m*/*z* 483 (M+H)⁺.

### Example 787

### trans-N-(biphenyl-3-yl)-1-[(1-methyl-1H-imidazol-4-yl)sulfonyl]-4-[(3S)-tetrahydro-2H-pyran-3-yl]pyrrolidin-3-amine

The title compound was prepared similarly to the procedure described in Example 327 substituting Example 781B for Example 322B and substituting 3-bromo-1,1'-biphenyl for 1-bromo-3-(trifluoromethyl)benzene. MS (ESI) *m*/*z* 467 (M+H)⁺.

### Example 788

### Trans-N-[4-fluoro-3-(trifluoromethoxy)phenyl]-1-[(1-methyl-1H-imidazol-4-yl)sulfonyl]-4-[(3S)-tetrahydro-2H-pyran-3-yl]pyrrolidin-3-amine

The title compound was prepared similarly to the procedure described in Example 327 substituting Example 781B for Example 322B and substituting 4-bromo-1-fluoro-2-(trifluoromethoxy)benzene for 1-bromo-3-(trifluoromethyl)benzene. MS (ESI) *m*/*z* 493 (M+H)⁺.

### Example 789

### Trans-N-(3-chloro-4-fluorophenyl)-1-[(1-methyl-1H-imidazol-4-yl)sulfonyl]-4-[(3S)-tetrahydro-2H-pyran-3-yl]pyrrolidin-3-amine

The title compound was prepared similarly to the procedure described in Example 327 substituting Example 781B for Example 322B and substituting 4-bromo-1-fluoro-2-chlorobenzene for 1-bromo-3-(trifluoromethyl)benzene. MS (ESI) *m*/*z* 443 (M+H)⁺.

### Example 790

### Trans-N-(4-fluoro-3-methylphenyl)-1-[(1-methyl-1H-imidazol-4-yl)sulfonyl]-4-[(3S)-tetrahydro-2H-pyran-3-yl]pyrrolidin-3-amine

The title compound was prepared similarly to the procedure described in Example 327 substituting Example 781B for Example 322B and substituting 4-bromo-1-fluoro-2-methylbenzene for 1-bromo-3-(trifluoromethyl)benzene. MS (ESI) *m*/*z* 423 (M+H)⁺.

### Example 791

### Trans-N-[4-fluoro-3-(trifluoromethyl)phenyl]-1-[(1-methyl-1H-imidazol-4-yl)sulfonyl]-4-[(3S)-tetrahydro-2H-pyran-3-yl]pyrrolidin-3-amine

The title compound was prepared similarly to the procedure described in Example 327 substituting Example 781B for Example 322B and substituting 4-bromo-1-fluoro-2-(trifluoromethyl)benzene for 1-bromo-3-(trifluoromethyl)benzene. MS (ESI) *m*/*z* 477 (M+H)⁺.

### Example 792

### Trans-N-[4-chloro-3-(trifluoromethyl)phenyl]-1-[(1-methyl-1H-imidazol-4-yl)sulfonyl]-4-[(3S)-tetrahydro-2H-pyran-3-yl]pyrrolidin-3-amine

The title compound was prepared similarly to the procedure described in Example 327 substituting Example 781B for Example 322B and substituting 4-bromo-1-chloro-2-(trifluoromethyl)benzene for 1-bromo-3-(trifluoromethyl)benzene. MS (ESI) *m*/*z* 509 (M+H)⁺.

### Example 793

### Trans-N-[4-chloro-3-(trifluoromethoxy)phenyl]-1-[(1-methyl-1H-imidazol-4-yl)sulfonyl]-4-[(3S)-tetrahydro-2H-pyran-3-yl]pyrrolidin-3-amine

The title compound was prepared similarly to the procedure described in Example 327 substituting Example 781B for Example 322B and substituting 4-bromo-1-chloro-2-(trifluoromethoxy)benzene for 1-bromo-3-(trifluoromethyl)benzene. MS (ESI) *m*/*z* 497 (M+H)⁺.

### Example 794

### Trans-N-[3-(benzyloxy)phenyl]-1-[(1-methyl-1H-imidazol-4-yl)sulfonyl]-4-[(3S)-tetrahydro-2H-pyran-3-yl]pyrrolidin-3-amine

The title compound was prepared similarly to the procedure described in Example 327 substituting Example 781B for Example 322B and substituting 1-(benzyloxy)-3-bromobenzene for 1-bromo-3-(trifluoromethyl)benzene. MS (ESI) *m*/*z* 497 (M+H)⁺.

### Example 795

### 5-fluoro-N-{(3R,4S)-4-(4-fluorophenyl)-1-[(1-methyl-1H-imidazol-4-yl)sulfonyl]pyrrolidin-3-yl}-4-(trifluoromethyl)pyridin-2-amine

To a solution of Example 368 (0.12 g, 0.26 mmol) in dimethylformamide (0.4 mL) was added 1-(chloromethyl)-4-fluoro-1,4-diazabicyclo[2.2.2]octane-1,4-diium tetrafluoroborate (SelectFluo, 91 mg, 0.26 mmol). The mixture was stirred at room temperature for 16 hours. More 1-(chloromethyl)-4-fluoro-1,4-diazabicyclo[2.2.2]octane-1,4-diium tetrafluoroborate (60 mg) was added. The mixture was stirred at room temperature for another 16 hours, concentrated, and purified by HPLC to afford the title compound. MS (ESI) *m*/*z* 488 (M+H)⁺.

### Example 796

### 3-fluoro-N- {(3R,4S)-4-(4-fluorophenyl)-1-[(1-methyl-1H-imidazol-4-yl)sulfonyl]pyrrolidin-3-yl}-4-(trifluoromethyl)pyridin-2-amine

To a solution of Example 368 (0.12 g, 0.26 mmol) in dimethylformamide (0.4 mL) was added 1-(chloromethyl)-4-fluoro-1,4-diazabicyclo[2.2.2]octane-1,4-diium tetrafluoroborate (SelectFluo, 91 mg, 0.26 mmol). The mixture was stirred at room temperature for 16 hours. More 1-(chloromethyl)-4-fluoro-1,4-diazabicyclo[2.2.2]octane-1,4-diium tetrafluoroborate (60 mg) was added. The mixture was stirred at room temperature for another 16 hours, concentrated, and purified by HPLC to afford the title compound. MS (ESI) *m*/*z* 488 (M+H)⁺.

### Example 797

### N-(2,2-difluoroethyl)-2-({(3R,4S)-4-(4-fluorophenyl)-1-[(1-methyl-1H-imidazol-4-yl)sulfonyl]pyrrolidin-3-yl}oxy)acetamide

The title compound was prepared similarly to the procedure described in Example 242E substituting 2,2-difluoroethanamine for aniline and substituting Example 689D for Example 242D. MS (ESI) *m*/*z* 447.0 (M+H)⁺

### Example 798

### Trans-4-(3-fluoropyridin-2-yl)-1-[(1-methyl-1H-imidazol-4-yl)sulfonyl]-N-[3-(trifluoromethyl)phenyl]pyrrolidin-3-amine

The title compound was prepared similarly to the procedure described in Example 337G substituting Example 761 B for Example 337F. MS (ESI) *m*/*z* 470.0 (M+H)⁺

### Example 799

### Trans-4-(3-fluoropyridin-2-yl)-1-[(1-methyl-1H-imidazol-4-yl)sulfonyl]-N-[3-(trifluoromethoxy)phenyl]pyrrolidin-3-amine

The title compound was prepared similarly to the procedure described in Example 337G substituting Example 761 B for Example 337F and substituting 1-bromo-3-(trifluoromethoxy)benzene for 1-bromo-3-(trifluoromethyl)benzene. MS (ESI) *m*/*z* 486.0 (M+H)⁺

### Example 800

### Trans-4-(3-fluoropyridin-2-yl)-1-[(1-methyl-1H-imidazol-4-yl)sulfonyl]-N-[3-(2,2,2-trifluoroethoxy)phenyl]pyrrolidin-3-amine

The title compound was prepared similarly to the procedure described in Example 337G substituting Example 761B for Example 337F and substituting 1-bromo-3-(2,2,2-trifluoroethoxy)benzene for 1-bromo-3-(trifluoromethyl)benzene. MS (ESI) *m*/*z* 500.0 (M+H)⁺

### Example 801

### (3R,4S)-4-(4-fluorophenyl)-1-[(1-methyl-1H-imidazol-4-yl)sulfonyl]-N-[3-(pyridin-2-yl)phenyl]pyrrolidin-3-amine

The title compound was prepared similarly to Example 327 substituting 2-(3-bromophenyl)pyridine for 1-bromo-3-(trifluoromethyl)benzene. MS (ESI) *m*/*z* 478 (M+H)⁺.

### Example 802

### (3R,4S)-1-[(1-methyl-1H-imidazol-4-yl)sulfonyl]-4-phenyl-N-[3-(pyridin-2-yl)phenyl]pyrrolidin-3-amine

The title compound was prepared similarly to Example 327 substituting Example 574A for Example 322B and substituting 2-(3-bromophenyl)pyridine. MS (ESI) *m*/*z* 460 (M+H)⁺.

### Example 803

### 5-fluoro-N-{(3R,4S)-1-[(1-methyl-1H-imidazol-4-yl)sulfonyl]-4-phenylpyrrolidin-3-yl}-4-(2,2,2-trifluoroethoxy)pyridin-2-amine

### Example 803A

### 2-bromo-5-fluoro-4-(2,2,2-trifluoroethoxy)pyridine

A microwave vial was charged with 2-bromo-5-fluoropyridin-4-ol (1 g, 5.2 mmol), 1,1,1-trifluoro-2-iodoethane (3.3 g, 15.6 mmol), potassium carbonate (1.44 g, 10.4 mmol), and dimethylsulfoxide (10 mL). The reaction mixture was stirred in a microwave (Biotage Initiator™, maximum 400 Watts) at 150 °C for 1 hour. The reaction mixture was partitioned between water and EtOAc. The organic fraction was collected and concentrated. Flash chromatography (0-50% EtOAc/heptanes) provided the title compound.

### Example 803B

### 5-fluoro-N-{(3R,4S)-1-[(1-methyl-1H-imidazol-4-yl)sulfonyl]-4-phenylpyrrolidin-3-yl}-4-(2,2,2-trifluoroethoxy)pyridin-2-amine

The title compound was prepared similarly to the procedure described in Example 327 substituting Example 574A for Example 322B and substituting Example 803A for 1-bromo-3-(trifluoromethyl)benzene. MS (ESI) *m*/*z* 500 (M+H)⁺.

### Example 804

### 5-Chloro-N-{trans-4-(3-fluoropyridin-2-yl)-1-[(1-methyl-1H-imidazol-4-yl)sulfonyl]pyrrolidin-3-yl}-4-(trifluoromethyl)pyridin-2-amine

The title compound was prepared as described in Example 848 substituting Example 761B for Example 679H. MS (ESI) *m*/*z* 505.0 (M+H)⁺

### Example 805

### Trans-N-(3-fluorophenyl)-4-(3-fluoropyridin-2-yl)-1-[(1-methyl-1H-imidazol-4-yl)sulfonyl]pyrrolidin-3-amine

The title compound was prepared similarly to the procedure described in Example 337G substituting Example 761B for Example 337F and substituting 1-bromo-3-fluorobenzene for 1-bromo-3-(trifluoromethyl)benzene. MS (ESI) *m*/*z* 420.0 (M+H)⁺

### Example 806

### 5-chloro-N-{(3R,4S)-4-(4-fluorophenyl)-1-[(1-methyl-1H-imidazol-4-yl)sulfonyl]pyrrolidin-3-yl}-4-(2,2,2-trifluoroethoxy)pyridin-2-amine

### Example 806A

### 2-bromo-5-chloro-4-(2,2,2-trifluoroethoxy)pyridine

The title compound was prepared similarly to Example 803A substituting 2-bromo-5-chloropyridin-4-ol for 2-bromo-5-fluoropyridin-4-ol.

### Example 806B

### 5-chloro-N-{(3R,4S)-4-(4-fluorophenyl)-1-[(1-methyl-1H-imidazol-4-yl)sulfonyl]pyrrolidin-3-yl}-4-(2,2,2-trifluoroethoxy)pyridin-2-amine

The title compound was prepared similarly to the procedure described in Example 327 substituting Example 806A for 1-bromo-3-(trifluoromethyl)benzene. MS (ESI) *m*/*z* 534 (M+H)⁺.

### Example 807

### N-{(3R,4S)-4-(4-fluorophenyl)-1-[(1-methyl-1H-imidazol-4-yl)sulfonyl]pyrrolidin-3-yl}-4-(2,2,2-trifluoroethoxy)pyridin-2-amine

### Example 807A

### 2-bromo-4-(2,2,2-trifluoroethoxy)pyridine

The title compound was prepared similarly to the procedure described in Example 803A substituting 2-bromopyridin-4-ol for 2-bromo-5-fluoropyridin-4-ol.

### Example 807B

### N-{(3R,4S)-4-(4-fluorophenyl)-1-[(1-methyl-1H-imidazol-4-yl)sulfonyl]pyrrolidin-3-yl}-4-(2,2,2-trifluoroethoxy)pyridin-2-amine

The title compound was prepared similarly to the procedure described in Example 327 substituting Example 807A for 1-bromo-3-(trifluoromethyl)benzene. MS (ESI) *m*/*z* 500 (M+H)⁺.

### Example 808

### 5-chloro-N-{(3R,4S)-1-[(1-methyl-1H-imidazol-4-yl)sulfonyl]-4-phenylpyrrolidin-3-yl}-4-(2,2,2-trifluoroethoxy)pyridin-2-amine

The title compound was prepared similarly to the procedure described in Example 327 substituting Example 574A for Example 322B and substituting Example 806A for 1-bromo-3-(trifluoromethyl)benzene. MS (ESI) *m*/*z* 516 (M+H)⁺.

### Example 809

### N-{(3R,4S)-1-[(1-methyl-1H-imidazol-4-yl)sulfonyl]-4-phenylpyrrolidin-3-yl}-4-(2,2,2-trifluoroethoxy)pyridin-2-amine

The title compound was prepared similarly to the procedure described in Example 327 substituting Example 574A for Example 322B and substituting Example 807A for 1-bromo-3-(trifluoromethyl)benzene. MS (ESI) *m*/*z* 482 (M+H)⁺.

### Example 810

### 5-fluoro-N-{(3R,4S)-4-(4-fluorophenyl)-1-[(1-methyl-1H-imidazol-4-yl)sulfonyl]pyrrolidin-3-yl}-4-(2,2,2-trifluoroethoxy)pyridin-2-amine

The title compound was prepared similarly to the procedure described in Example 327 substituting Example 803A for 1-bromo-3-(trifluoromethyl)benzene. MS (ESI) *m*/*z* 518 (M+H)⁺.

### Example 811

### Trans-4-(3-fluoropyridin-2-yl)-N-hexyl-1-[(1-methyl-1H-1,2,3-triazol-4-yl)sulfonyl]pyrrolidin-3-amine

### Example 811 A

### Trans-4-(3-fluoropyridin-2-yl)-1-(1-methyl-1H-1,2,3-triazol-4-ylsulfonyl)pyrrolidin-3-amine hydrochloride

The title compound was prepared similarly to the conditions described in Example 2A-2F substituting (E)-3-fluoro-2-(2-nitrovinyl)pyridine for (E)-1-methoxy-4-(2-nitrovinyl)benzene and 1-methyl-1H-1,2,3-triazole-4-sulfonyl chloride for 1-methyl-1H-imidazole-4-sulfonyl chloride.

### Example 811 B

### Trans-4-(3-fluoropyridin-2-yl)-N-hexyl-1-[(1-methyl-1H-1,2,3-triazol-4-yl)sulfonyl]pyrrolidin-3-amine

The title compound was prepared similarly to the procedure described in Example 354E substituting hexanal for 4-chlorobenzaldehyde and substituting Example 811 A for 354D. MS (ESI) *m*/*z* 411.0 (M+H)⁺

### Example 812

### N-{(3R,4S)-4-(4-fluorophenyl)-1-[(1-methyl-1H-imidazol-4-yl)sulfonyl]pyrrolidin-3-yl}-4-(propan-2-yl)pyridin-2-amine

The title compound was prepared similarly to the procedure described in Example 327 substituting 2-bromo-4-isopropylpyridine for 1-bromo-3-(trifluoromethyl)benzene. MS (ESI) *m*/*z* 444 (M+H)⁺.

### Example 813

### N-{(3R,4S)-1-[(1-methyl-1H-imidazol-4-yl)sulfonyl]-4-phenylpyrrolidin-3-yl}-4-(propan-2-yl)pyridin-2-amine

The title compound was prepared similarly to the procedure described in Example 327 substituting Example 574A for Example 322B and substituting 2-bromo-4-isopropylpyridine for 1-bromo-3-(trifluoromethyl)benzene. MS (ESI) *m*/*z* 426 (M+H)⁺.

### Example 814

### 2,2,2-trifluoro-N-[2-({trans-4-(4-fluorophenyl)-1-[(1-methyl-1H-imidazol-4-yl)sulfonyl]pyrrolidin-3-yl}oxy)ethyl]ethanamine

The title compound was prepared similarly to the procedure described in Example 290 substituting Example 797 for Example 242E. MS (ESI) *m*/*z* 451.1 (M+H)⁺

### Example 815

### Trans-N-(3-chloro-4-fluorophenyl)-1-[(1-methyl-1H-imidazol-4-yl)sulfonyl]-4-(tetrahydro-2H-pyran-2-yl)pyrrolidin-3-amine

The title compound was prepared similarly to the procedure described in Example 337G substituting Example 679H for Example 337F and substituting 4-bromo-2-chloro-1-fluorobenzene for 1-bromo-3-trifluoromethylbenzene. MS (ESI) *m*/*z* 443.0 (M+H)⁺

### Example 816

### Trans-1-[(1-methyl-1H-imidazol-4-yl)sulfonyl]-4-(tetrahydro-2H-pyran-2-yl)-N-[3-(2,2,2-trifluoroethoxy)phenyl]pyrrolidin-3-amine

The title compound was prepared similarly to the procedure described in Example 337G substituting Example 679H for Example 337F and substituting 1-bromo-3-(2,2,2-trifluoroethoxy)benzene for 1-bromo-3-trifluoromethylbenzene. MS (ESI) *m*/*z* 489.1 (M+H)⁺

### Example 817

### 2-({1-[(1-methyl-1H-imidazol-4-yl)sulfonyl]-4-phenylpyrrolidin-3-yl}methyl)-4-(trifluoromethyl)pyridine

### Example 817A

### (E)-ethyl 3-(4-(trifluoromethyl)pyridin-2-yl)acrylate

To a solution of ethyl 2-(diethoxyphosphoryl)acetate (0.45 g, 2 mmol) in tetrahydrofuran (3 mL) at -20 °C was added sodium hydride (100 mg, 60%, 2.5 mmol) followed by the addition of 4-(trifluoromethyl)picolinaldehyde in tetrahydrofuran (1 mL). The mixture was warmed to room temperature over 1 hour. Aqueous ammonium chloride was added and the solution was partitioned with ethyl acetate. The organic fraction was collected, concentrated, and purified by flash chromatography (25% ethyl acetate/hexanes) to afford the title compound.

### Example 817B

### ethyl 3-(4-(trifluoromethyl)pyridin-2-yl)propanoate

To a solution of Example 817A (460 mg, 1.8 mmol) in ethanol (20 mL) in a 50 mL pressure bottle was added 5% palladium on carbon, (95 mg, 0.022 mmol). The mixture was stirred for 2 hours under 30 psi of hydrogen at room temperature. The mixture was filtered through a nylon membrane and concentrated to afford the title compound.

### Example 817C

### ethyl 4-nitro-3-phenyl-2-((4-(trifluoromethyl)pyridin-2-yl)methyl)butanoate

The title compound was prepared similarly to the procedure described in Example 331A substituting Example 817B for methyl 3-(3-chlorophenyl)propanoate.

### Example 817D

### 4-phenyl-3-((4-(trifluoromethyl)pyridin-2-yl)methyl)pyrrolidin-2-one

The title compound was prepared similarly to the procedure described in Example 331B substituting Example 817C for Example 331A.

### Example 817E

### 2-((4-phenylpyrrolidin-3-yl)methyl)-4-(trifluoromethyl)pyridine

To a solution of Example 817D (450 mg, 1.4 mmol) in tetrahydrofuran (3 mL) was added lithiumaluminum hydride (2N, 1.5 mL, 3 mmol). The mixture was stirred for 16 hours. Aqueous sodium bicarbonate followed by ethyl acetate was added. The organic fraction was collected, dried over potassium carbonate, filtered, and concentrated to provide the title compound.

### Example 817F

### 2-({1-[(1-methyl-1H-imidazol-4-yl)sulfonyl]-4-phenylpyrrolidin-3-yl}methyl)-4-(trifluoromethyl)pyridine

The title compound was prepared similarly to the procedure described in Example 182C substituting Example 817E for Example 182B. MS (ESI) *m*/*z* 451 (M+H)⁺.

### Example 818

### Trans-N-[2-chloro-4-(trifluoromethyl)benzyl]-4-(3-fluoropyridin-2-yl)-1-[(1-methyl-1H-1,2,3-triazol-4-yl)sulfonyl]pyrrolidin-3-amine

The title compound was prepared similarly to the procedure described in Example 354E substituting 2-chloro-4-(trifluoromethyl)benzaldehyde for 4-chlorobenzaldehyde and substituting example 811A for 354D. MS (ESI) *m*/*z* 519.0 (M+H)⁺

### Example 819

### Trans-1-[(1-methyl-1H-imidazol-4-yl)sulfonyl]-4-(tetrahydrofuran-2-yl)-N-[3-(2,2,2-trifluoroethoxy)phenyl]pyrrolidin-3-amine

The title compound was prepared as described in Example 603 substituting 1 -bromo-3 -(2,2,2-trifluoroethoxy)benzene for 1-bromo-3-trifluoromethylbenzene. MS (ESI) *m*/*z* 475.1 (M+H)⁺

### Example 820

### N-Hexyl-1-[(1-methyl-1H-imidazol-4-yl)sulfonyl]-4-[(2R)-tetrahydrofuran-2-yl]pyrrolidin-3-amine

The title compound was prepared similarly to the procedure described in Example 354E substituting hexanal for 4-chlorobenzaldehyde and substituting example 601B for 354D. MS (ESI) *m*/*z* 385.2 (M+H)⁺.

### Example 821

### N-hexyl-1-[(1-methyl-1H-imidazol-4-yl)sulfonyl]-4-[(2S)-tetrahydrofuran-2-yl]pyrrolidin-3-amine

The title compound was prepared similarly to the procedure described in Example 354E substituting hexanal for 4-chlorobenzaldehyde and substituting example 601B for 354D. MS (ESI) *m*/*z* 385.2 (M+H)⁺.

### Example 822

### Trans-N-[2-chloro-4-(trifluoromethyl)benzyl]-1-[(1-methyl-1H-imidazol-4-yl)sulfonyl]-4-(tetrahydrofuran-2-yl)pyrrolidin-3-amine

The title compound was prepared similarly to the procedure described in Example 354E substituting 2-chloro-4-(trifluoromethyl)benzaldehyde for 4-chlorobenzaldehyde and substituting example 601B for 354D. MS (ESI) *m*/*z* 493.1 (M+H)⁺.

### Example 823

### N-[2-chloro-4-(trifluoromethyl)benzyl]-1-[(1-methyl-1H-imidazol-4-yl)sulfonyl]-4-[(2S)-tetrahydrofuran-2-yl]pyrrolidin-3-amine

The title compound was prepared similarly to the procedure described in Example 354E substituting 2-chloro-4-(trifluoromethyl)benzaldehyde for 4-chlorobenzaldehyde and substituting example 601B for 354D. MS (ESI) *m*/*z* 493.1 (M+H)⁺.

### Example 824

### Trans-N-(3-chloro-4-fluorophenyl)-1-[(1-methyl-1H-imidazol-4-yl)sulfonyl]-4-(tetrahydrofuran-2-yl)pyrrolidin-3-amine

The title compound was prepared as described in Example 603 substituting 4-bromo-2-chloro-1-fluorobenzene for 1-bromo-3-trifluoromethylbenzene. MS (ESI) *m*/*z* 429.1 (M+H)⁺

### Example 825

### N-(3-chloro-4-fluorophenyl)-1-[(1-methyl-1H-imidazol-4-yl)sulfonyl]-4-[(2S)-tetrahydrofuran-2-yl]pyrrolidin-3 -amine

The title compound was prepared as described in Example 603 substituting 4-bromo-2-chloro-1-fluorobenzene for 1-bromo-3-trifluoromethylbenzene. MS (ESI) *m*/*z* 429.1 (M+H)⁺

### Example 826

### 1-benzyl-N-{(3R,4S)-1-[(1-methyl-1H-imidazol-4-yl)sulfonyl]-4-phenylpyrrolidin-3-yl}-2,3-dihydro-1H-indol-6-amine

### Example 826A

### 1-benzyl-6-bromoindoline

To a stirred solution of 6-bromoindoline (100 mg, 0.50 mmol) in dimethylformamide (5 mL) was added (bromomethyl)benzene (95 mg, 0.55 mmol) and potassium carbonate (140 mg, 1.0 mmol). The reaction was stirred at 60 °C for 18 hours. The reaction mixture was partitioned between water and ethyl acetate. The organic fraction was collected, washed with brine, dried with magnesium sulfate, and concentrated to provide the title compound.

### Example 826B

### 1-benzyl-N-{(3R,4S)-1-[(1-methyl-1H-imidazol-4-yl)sulfonyl]-4-phenylpyrrolidin-3-yl}-2,3-dihydro-1H-indol-6-amine

The title compound was prepared similarly to the procedure described in Example 327 substituting Example 574A for Example 322B and substituting Example 826A for 1-bromo-3-(trifluoromethyl)benzene. MS (ESI) *m*/*z* 514 (M+H)⁺.

### Example 827

### trans-1-[(1-methyl-1H-imidazol-4-yl)sulfonyl]-4-(tetrahydrofuran-3 -yl)-N-[3 - (trifluoromethyl)phenyl]pyrrolidin-3 -amine

### Example 827A

### (E)-3 -(2-nitrovinyl)tetrahydrofuran

To a solution of oxalyl chloride (5.1 mL, 60 mmol) in dichloromethane (100 mL) under a nitrogen atmosphere at -78 °C was added dropwise dimethylsulfoxide (4.6 mL, 65 mmol) in dichloromethane (10 mL). The reaction mixture stirred for 10 minutes. Tetrahydro-3-furan methanol (5.5 g, 54 mmol) was added in dichloromethane (20 mL) over several minutes, and the reaction mixture was stirred for 10 minutes. Triethylamine (17.4 mL, 125 mmol) was added drop wise. The reaction mixture was allowed to warm to room temperature. Diethyl ether was added and the solution was filtered. The filtrate was concentrated to provide crude tetrahydrofuran-3-carbaldehyde.

To a stirred solution of this crude tetrahydrofuran-3-carbaldehyde in tetrahydrofuran (50 mL) under a nitrogen atmosphere was added nitromethane (8.1 mL, 150 mmol), 1,1,2,2-tetramethyl guanidine (0.69 mL, 5.5 mmol). The reaction was stirred at room temperature for 1 hour. The reaction mixture was then cooled to 0 °C and trifluoroacetic anhydride (6.3 mL, 100 mmol) was added. The reaction mixture stirred for 15 minutes. Triethylamine (13.9 mL, 100 mmol) was added dropwise and the reaction mixture stirred for 1 hour. The reaction mixture was concentrated and partitioned between ethyl acetate and water. The organic fraction was collected, washed with brine, and concentrated. Purification via flash chromatography (40 % EtOAc/heptanes) provided the title compound.

### Example 827B

### trans-1-(1-methyl-1H-imidazol-4-ylsulfonyl)-4-((S)-tetrahydrofuran-3-yl)pyrrolidin-3-amine

The title compound was prepare as an HCl salt similarly to the procedures described in Example 2A-2F substituting Example 827A for (E)-1-methoxy-4-(2-nitrovinyl)benzene.

### Example 827C

### trans-1-[(1-methyl-1H-imidazol-4-yl)sulfonyl]-4-(tetrahydrofuran-3-yl)-N-[3-(trifluoromethyl)phenyl]pyrrolidin-3 -amine

The title compound was prepared similarly to the procedure described in Example 327 substituting Example 827B for Example 322B. MS (ESI) *m*/*z* 445 (M+H)⁺.

### Example 828

### trans-1-[(1-methyl-1H-imidazol-4-yl)sulfonyl]-N-(3-methylphenyl)-4-(tetrahydrofuran-3-yl)pyrrolidin-3-amine

The title compound was prepared similarly to the procedure described in Example 327 substituting Example 827B for Example 322B and substituting 1-bromo-3-methylbenzene for 1-bromo-3-(trifluoromethyl)benzene. MS (ESI) *m*/*z* 391 (M+H)⁺.

### Example 829

### trans-N-(3-chlorophenyl)-1-[(1-methyl-1H-imidazol-4-yl)sulfonyl]-4-(tetrahydrofuran-3-yl)pyrrolidin-3-amine

The title compound was prepared similarly to the procedure described in Example 327 substituting Example 827B for Example 322B and substituting 1-bromo-3-chlorobenzene for 1-bromo-3-(trifluoromethyl)benzene. MS (ESI) *m*/*z* 411 (M+H)⁺.

### Example 830

### trans -N-(3 -chloro-4-fluorophenyl)-1-[(1-methyl-1H-imidazol-4-yl)sulfonyl] -4-(tetrahydrofuran-3 - yl)pyrrolidin-3-amine

The title compound was prepared similarly to the procedure described in Example 327 substituting Example 827B for Example 322B and substituting 1-bromo-3-chloro-4-fluorobenzene for 1-bromo-3-(trifluoromethyl)benzene, but only stirring for 1 hour. MS (APCI) *m*/*z* 429 (M+H)⁺.

### Example 831

### trans -N- [4-fluoro-3 -(trifluoromethoxy)phenyl]-1-[(1-methyl-1 H-imidazol-4-yl)sulfonyl] -4-(tetrahydrofuran-3 -yl)pyrrolidin-3 -amine

The title compound was prepared similarly to the procedure described in Example 327 substituting Example 827B for Example 322B and substituting 1-bromo-4-fluoro-3-(trifluoromethoxy)benzene for 1-bromo-3-(trifluoromethyl)benzene. MS (ESI) *m*/*z* 479 (M+H)⁺.

### Example 832

### trans-N-[4-fluoro-3-(trifluoromethyl)phenyl]-1-[(1-methyl-1H-imidazol-4-yl)sulfonyl]-4-(tetrahydrofuran-3-yl)pyrrolidin-3-amine

The title compound was prepared similarly to the procedure described in Example 327 substituting Example 827B for Example 322B and substituting 1-bromo-4-fluoro-3-(trifluoromethyl)benzene for 1-bromo-3-(trifluoromethyl)benzene. MS (APCI) *m*/*z* 463 (M+H)⁺.

### Example 833

### trans-N-[4-chloro-3-(trifluoromethyl)phenyl]-1-[(1-methyl-1H-imidazol-4-yl)sulfonyl]-4-(tetrahydrofuran-3-yl)pyrrolidin-3-amine

The title compound was prepared similarly to the procedure described in Example 327 substituting Example 827B for Example 322B and substituting 1-bromo-4-chloro-3-(trifluoromethyl)benzene for 1-bromo-3-(trifluoromethyl)benzene. MS (ESI) *m*/*z* 479 (M+H)⁺.

### Example 834

### trans-1-[(1-methyl-1H-imidazol-4-yl)sulfonyl]-4-(tetrahydrofuran-3-yl)-N-[3-(2,2,2-trifluoroethoxy)phenyl]pyrrolidin-3-amine

The title compound was prepared similarly to the procedure described in Example 327 substituting Example 827B for Example 322B and substituting 1-bromo-3-(2,2,2-trifluoroethoxy)benzene for 1-bromo-3-(trifluoromethyl)benzene. MS (ESI) *m*/*z* 475 (M+H)⁺.

### Example 835

### trans-1-[(1-methyl-1H-imidazol-4-yl)sulfonyl]-4-(tetrahydrofuran-3-yl)-N-[3-(trifluoromethoxy)phenyl]pyrrolidin-3-amine

The title compound was prepared similarly to the procedure described in Example 327 substituting Example 827B for Example 322B and substituting 1-bromo-3-(trifluoromethoxy)benzene for 1-bromo-3-(trifluoromethyl)benzene. MS (ESI) *m*/*z* 461 (M+H)⁺.

### Example 836

### trans-4-(4-fluorophenyl)-1-[(1-methyl-1H-1,2,3-triazol-4-yl)sulfonyl]-N-[3-(2,2,2-trifluoroethoxy)phenyl]pyrrolidin-3-amine

### Example 836A

### 1-methyl-1H-1,2,3-triazole-4-sulfonyl chloride

To an ice-cooled solution of sodium 1H-1,2,3-triazole-4-thiolate (34.5 g, 280 mmol) in ethanol (400 mL) was added benzylbromide (18.8 mL, 158 mmol) drop-wise. The mixture stirred at 25 °C for 2 hours, and was then diluted with EtOAc (600 mL), washed with water (500 mL), brine (500 mL), dried over sodium sulfate filtered, and concentrated under reduced pressure to afford 4-(benzylthio)-1H-1,2,3-triazole.

To a solution of 4-(benzylthio)-1H-1,2,3-triazole (55.0 g, 288 mmol) in dimethylformamide (550 mL) at 0 °C was added potassium carbonate (87.0 g, 630 mmol) followed by dimethyl sulfate (55.0 mL, 575 mmol) dropwise. Then the reaction mixture was stirred at 20°C for 16 hours. After the reaction was completed, the reaction mixture was concentrated under reduced pressure. The residue was dissolved in dichloromethane (500mL) and washed with water (500 mL). The organic fraction was dried with sodium sulfate, and concentrated. The residue was purified via flash chromatography (Petroleum ether:EtOAc = 1:0, 10:1, 5:1 to 3:1) to afford 4-(benzylthio)-1-methyl-1H-1,2,3-triazole.

1,3-dichloro-5,5-dimethylimidazolidine-2,4-dione (40.3 g, 205 mmol) was added portion wise to a mixture of 4-(benzylthio)-1-methyl-1H-1,2,3-triazole (28.0 g, 137 mmol) in acetonitrile (1200 mL), AcOH (50 mL) and water (34 mL), which had been cooled to 0 °C. The addition was slow enough to maintain the internal temperature of the reaction mixture below 5 °C. Upon complete addition, the mixture stirred for an additional 2 hours at 0 °C. The reaction mixture was then quenched slowly with aqueous sodium hydrideCO₃ solution (5%, 700 mL). The resulting mixture was stirred for 15 minutes and then diluted with dichloromethane (2000 mL). The organic fraction was collected, washed with brine (1000 mL), dried over sodiumsulfate, filtered and concentrated under reduced pressure. The residue was purified by flash column chromatography (petroleum ether/EtOAc = 1:0 to 5:1) to afford the title compound.

### Example 836B

### trans-4-(4-fluorophenyl)-1-(1-methyl-1H-1,2,3-triazol-4-ylsulfonyl)pyrrolidin-3-amine

The title compound was prepared as an HCl salt similarly to the procedures described in Examples 2A-2F substituting (E)-1-fluoro-4-(2-nitrovinyl)benzene for (E)-1-methoxy-4-(2-nitrovinyl)benzene and substituting 1-methyl-1H-1,2,3-triazole-4-sulfonyl chloride for 1-methyl-1H-imidazole-4-sulfonyl chloride.

### Example 836C

### trans-4-(4-fluorophenyl)-1-[(1-methyl-1H-1,2,3-triazol-4-yl)sulfonyl]-N-[3-(2,2,2-trifluoroethoxy)phenyl]pyrrolidin-3-amine

The title compound was prepared similarly to the procedure described in Example 327 substituting Example 836B for Example 322B and substituting 1-bromo-3-(2,2,2-trifluoroethoxy)benzene for 1-bromo-3-(trifluoromethyl)benzene. MS (ESI) *m*/*z* 500 (M+H)⁺.

### Example 837

### (3R,4S)-4-(4-fluorophenyl)-1-[(1-methyl-1H-1,2,3-triazol-4-yl)sulfonyl]-N-[3-(trifluoromethoxy)phenyl]pyrrolidin-3-amine

The title compound was prepared similarly to the procedure described in Example 327 substituting Example 836B for Example 322B and substituting 1-bromo-3-(trifluoromethoxy)benzene for 1-bromo-3-(trifluoromethyl)benzene. MS (ESI) *m*/*z* 486 (M+H)⁺.

### Example 838

### (3R,4S)-4-(4-fluorophenyl)-N-[4-fluoro-3-(trifluoromethoxy)phenyl]-1-[(1-methyl-1H-1,2,3-triazol-4-yl)sulfonyl]pyrrolidin-3-amine

The title compound was prepared similarly to the procedure described in Example 327 substituting Example 836B for Example 322B and substituting 1-bromo-4-fluoro-3-(trifluoromethoxy)benzene for 1-bromo-3-(trifluoromethyl)benzene. MS (ESI) *m*/*z* 504 (M+H)⁺.

### Example 839

### (3R,4S)-4-(4-fluorophenyl)-1-[(1-methyl-1H-1,2,3-triazol-4-yl)sulfonyl]-N-[3-(trifluoromethyl)phenyl]pyrrolidin-3-amine

The title compound was prepared similarly to the procedure described in Example 327 substituting Example 836B for Example 322B and substituting 1-bromo-3-(trifluoromethyl)benzene for 1-bromo-3-(trifluoromethyl)benzene. MS (ESI) *m*/*z* 470 (M+H)⁺.

### Example 840

### (3R,4S)-4-(4-fluorophenyl)-N-[4-fluoro-3-(trifluoromethyl)phenyl]-1-[(1-methyl-1H-1,2,3-triazol-4-yl)sulfonyl]pyrrolidin-3-amine

The title compound was prepared similarly to the procedure described in Example 327 substituting Example 836B for Example 322B and substituting 1-bromo-4-fluoro-3-(trifluoromethyl)benzene for 1-bromo-3-(trifluoromethyl)benzene. MS (ESI) *m*/*z 488* (M+H)⁺.

### Example 841

### (3R,4S)-N-(3-chlorophenyl)-4-(4-fluorophenyl)-1-[(1-methyl-1H-1,2,3-triazol-4-yl)sulfonyl]pyrrolidin-3-amine

The title compound was prepared similarly to the procedure described in Example 327 substituting Example 836B for Example 322B and substituting 1-bromo-3-chlorobenzene for 1-bromo-3-(trifluoromethyl)benzene. MS (ESI) *m*/*z* 436 (M+H)⁺.

### Example 842

### (3R,4S)-N-(3-chloro-4-fluorophenyl)-4-(4-fluorophenyl)-1-[(1-methyl-1H-1,2,3-triazol-4-yl)sulfonyl]pyrrolidin-3-amine

The title compound was prepared similarly to the procedure described in Example 327 substituting Example 836B for Example 322B and substituting 1-bromo-3-chloro-4-fluorobenzene for 1-bromo-3-(trifluoromethyl)benzene, but only stirring for 1 hour. MS (ESI) *m*/*z* 454 (M+H)⁺.

### Example 843

### Trans-N-[4-fluoro-3-(trifluoromethyl)phenyl]-1-[(1-methyl-1H-imidazol-4-yl)sulfonyl]-4-(tetrahydrofuran-2-yl)pyrrolidin-3-amine

The title compound was prepared similarly to the procedure described in Example 603 substituting 4-bromo-1-fluoro-2-(trifluoromethyl)benzene for 1-bromo-3-trifluoromethylbenzene. MS (ESI) *m*/*z* 463.1 (M+H)⁺

### Example 844

### 5-Chloro-N-[trans-1-[(1-methyl-1H-imidazol-4-yl)sulfonyl]-4-(tetrahydrofuran-2-yl)pyrrolidin-3-yl]-4-(trifluoromethyl)pyridin-2-amine

The title compound was prepared similarly to the procedure in Example 848 substituting Example 601B for Example 679H. MS (ESI) *m*/*z* 480.1 (M+H)⁺

### Example 845

### Trans-N-(4-fluoro-3-methylphenyl)-1-[(1-methyl-1H-imidazol-4-yl)sulfonyl]-4-(tetrahydrofuran-2-yl)pyrrolidin-3-amine

The title compound was prepared similarly to the procedure described in Example 603 substituting 4-bromo-1-fluoro-2-methylbenzene for 1-bromo-3-trifluoromethylbenzene. MS (ESI) *m*/*z* 409.1 (M+H)⁺

### Example 846

### Trans-N-[4-fluoro-3-(trifluoromethyl)phenyl]-1-[(1-methyl-1H-imidazol-4-yl)sulfonyl]-4-(tetrahydro-2H-pyran-2-yl)pyrrolidin-3 -amine

The title compound was prepared similarly to the procedure described in Example 337G substituting Example 679H for Example 337F and substituting 4-bromo-1-fluoro-2-(trifluoromethyl)benzene for 1-bromo-3-trifluoromethylbenzene. MS (ESI) *m*/*z* 477.1 (M+H)⁺

### Example 847

### Trans-N-(4-fluoro-3-methylphenyl)-1-[(1-methyl-1H-imidazol-4-yl)sulfonyl]-4-(tetrahydro-2H-pyran-2-yl)pyrrolidin-3-amine

The title compound was prepared similarly to the procedure described in Example 337G substituting Example 679H for Example 337F and substituting 4-bromo-1-fluoro-2-methylbenzene for 1-bromo-3-trifluoromethylbenzene. MS (ESI) *m*/*z* 423.1 (M+H)⁺

### Example 848

### 5-Chloro-N-[trans-1-[(1-methyl-1H-imidazol-4-yl)sulfonyl]-4-(tetrahydro-2H-pyran-2-yl)pyrrolidin-3-yl]-4-(trifluoromethyl)pyridin-2-amine

To a microwave vial charged with Example 679H (80 mg, 0.254 mmol) and N-methyl-2-pyrrolidone (0.5 mL) was added cesium carbonate (141 mg, 0.43 mmol) and 2,5-dichloro-4-(trifluoromethyl)pyridine (71.4 mg, 0.33 mmol) under Argon. This mixture was stirred at 120 °C for 5 hours. The mixture was then partitioned between water and ethyl acetate. The organic fraction was collected, concentrated, and purified by flash chromatography to provide the title compound. MS (ESI) *m*/*z* 494.1 (M+H)⁺

### Example 849

### Trans-N-(3-chlorophenyl)-1-[(1-methyl-1H-imidazol-4-yl)sulfonyl]-4-(tetrahydro-2H-pyran-2-yl)pyrrolidin-3-amine

The title compound was prepared similarly to the procedure described in Example 337G substituting Example 679H for Example 337F and substituting 1-bromo-3-chlorobenzene for 1-bromo-3-trifluoromethylbenzene. MS (ESI) *m*/*z* 425.1 (M+H)⁺

### Example 850

### Trans-N-(3 -chlorophenyl)-1 - [(1 -methyl-1H-imidazol-4-yl)sulfonyl] -4-(tetrahydrofuran-2-yl)pyrrolidin-3-amine

The title compound was prepared similarly to the procedure described in Example 603 substituting 1-bromo-3-chlorobenzene for 1-bromo-3-trifluoromethylbenzene. MS (ESI) *m*/*z* 411.1 (M+H)⁺

### Example 851

### 5-Fluoro-4-methyl-N-[trans-1-[(1-methyl-1H-imidazol-4-yl)sulfonyl]-4-(tetrahydrofuran-2-yl)pyrrolidin-3-yl]pyridin-2-amine

The title compound was prepared similarly to the procedure described in Example 603 substituting 2-bromo-5-fluoro-4-methylpyridine for 1-bromo-3-trifluoromethylbenzene. MS (ESI) *m*/*z* 410.2 (M+H)⁺

### Example 852

### 5-Fluoro-4-methyl-N-[trans-1-[(1-methyl-1H-imidazol-4-yl)sulfonyl]-4-(tetrahydro-2H-pyran-2-yl)pyrrolidin-3-yl]pyridin-2-amine

The title compound was prepared similarly to the procedure described in Example 337G substituting Example 679H for Example 337F and substituting 2-bromo-5-fluoro-4-methylpyridine for 1-bromo-3-trifluoromethylbenzene. MS (ESI) *m*/*z* 424.2 (M+H)⁺

### Example 853

### N-[Trans-1-[(1-methyl-1H-imidazol-4-yl)sulfonyl]-4-(tetrahydrofuran-2-yl)pyrrolidin-3-yl]-6-(trifluoromethyl)pyrimidin-4-amine

The title compound was prepared similarly to the procedure described in Example 603 substituting 4-bromo-6-(trifluoromethyl)pyrimidine for 1-bromo-3-trifluoromethylbenzene. MS (ESI) *m*/*z* 447.1 (M+H)⁺

### Example 854

### (3R,4S)-N-cyclohexyl-4-(4-fluorophenyl)-1-(1-methyl-1H-imidazol-4-ylsulfonyl)pyrrolidin-3-amine

The title compound was prepared similarly to the procedure described in Example 148 substituting Example 322B for Example 3A and substituting cyclohexanone for benzaldehyde. MS (ESI) *m*/*z* 407 (M+H)⁺.

### Example 855

### (3R,4S)-N-(3,3-dimethylcyclohexyl)-4-(4-fluorophenyl)-1-(1-methyl-1H-imidazol-4-ylsulfonyl)pyrrolidin-3-amine

The title compound was prepared similarly to the procedure described in Example 148 substituting Example 322B for Example 3A and substituting 3,3-dimethylcyclohexanone for benzaldehyde. MS (ESI) *m*/*z* 435 (M+H)⁺.

### Example 856

### (3R,4S)-N-(4,4-dimethylcyclohexyl)-4-(4-fluorophenyl)-1-(1-methyl-1H-imidazol-4-ylsulfonyl)pyrrolidin-3-amine

The title compound was prepared similarly to the procedure described in Example 148 substituting Example 322B for Example 3A and substituting 4,4-dimethylcyclohexanone for benzaldehyde. MS (ESI) *m*/*z* 435 (M+H)⁺.

### Example 857

### (3R,4S)-N-cyclopentyl-4-(4-fluorophenyl)-1-(1-methyl-1H-imidazol-4-ylsulfonyl)pyrrolidin-3-amine

The title compound was prepared similarly to the procedure described in Example 148 substituting Example 322B for Example 3A and substituting cyclopentanone for benzaldehyde. MS (ESI) *m*/*z* 393 (M+H)⁺.

### Example 858

### (3R,4S)-N-(3,3-dimethylcyclopentyl)-4-(4-fluorophenyl)-1-(1-methyl-1H-imidazol-4-ylsulfonyl)pyrrolidin-3-amine

The title compound was prepared similarly to the procedure described in Example 148 substituting Example 322B for Example 3A and substituting 3,3-dimethylcyclopentanone for benzaldehyde. MS (ESI) *m*/*z* 421 (M+H)⁺.

### Example 859

### (3R,4S)-N-(1-(2,4-dichlorophenyl)ethyl)-4-(4-fluorophenyl)-1-(1-methyl-1H-imidazol-4-ylsulfonyl)pyrrolidin-3-amine

The title compound was prepared similarly to the procedure described in Example 148 substituting Example 322B for Example 3A and substituting 1-(2,4-dichlorophenyl)ethanone for benzaldehyde. MS (ESI) *m*/*z* 497 (M+H)⁺.

### Example 860

### (3R,4S)-N-(2,3-dihydro-1H-inden-1-yl)-4-(4-fluorophenyl)-1-(1-methyl-1H-imidazol-4-ylsulfonyl)pyrrolidin-3-amine

The title compound was prepared similarly to the procedure described in Example 148 substituting Example 322B for Example 3A and substituting 2,3-dihydro-1H-inden-1-one for benzaldehyde. MS (ESI) *m*/*z* 441 (M+H)⁺.

### Example 861

### (3R,4S)-N-(5-chloro-2,3-dihydro-1H-inden-1-yl)-4-(4-fluorophenyl)-1-(1-methyl-1H-imidazol-4-ylsulfonyl)pyrrolidin-3-amine

The title compound was prepared similarly to the procedure described in Example 148 substituting Example 322B for Example 3A and substituting 5-chloro-2,3-dihydro-1H-inden-1-one for benzaldehyde. MS (ESI) *m*/*z* 475 (M+H)⁺.

### Example 862

### N-(4-chlorobenzyl)-N-((3R,4S)-4-(4-fluorophenyl)-1-(1-methyl-1H-imidazol-4-ylsulfonyl)pyrrolidin-3-yl)acetamide

### Example 862A

### (3R,4S)-N-(4-chlorobenzyl)-4-(4-fluorophenyl)-1-(1-methyl-1H-imidazol-4-ylsulfonyl)pyrrolidin-3-amine

The title compound was prepared similarly to the procedure described in Example 148 substituting Example 322B for Example 3A and substituting 4-chlorobenzaldehyde for benzaldehyde.

### Example 862B

### N-(4-chlorobenzyl)-N-((3R,4S)-4-(4-fluorophenyl)-1-(1-methyl-1H-imidazol-4-ylsulfonyl)pyrrolidin-3-yl)acetamide

The title compound was prepared similarly to the procedure described in Example 4C substituting Example 862A for Example 4B and substituting acetic acid for 2-chloro-3-(trifluoromethyl)benzoic acid. MS (ESI) *m*/*z* 491 (M+H)⁺.

### Example 863

### 5-butyl-3-{(3R,4S)-4-(4-fluorophenyl)-1-[(1-methyl-1H-imidazol-4-yl)sulfonyl]pyrrolidin-3-yl}-1,3-oxazolidin-2-one

### Example 863A

### N-((3R,4S)-4-(4-fluorophenyl)-1-((1-methyl-1H-imidazol-4-yl)sulfonyl)pyrrolidin-3-yl)-2-hydroxyhexanamide

The title compound was prepared according to the procedure outlined in Example 242 E by substituting Example 380A for aniline and 2-hydroxyhexanoic acid for Example 242D. MS (ESI) *m*/*z* 439.4 (M+H)⁺.

### Example 863B

### 1-(((3R,4S)-4-(4-fluorophenyl)-1-((1-methyl-1H-imidazol-4-yl)sulfonyl)pyrrolidin-3-yl)amino)hexan-2-ol

The title compound was prepared according to the procedure outlined in Example 290 by substituting Example 863A for Example 242E. MS (ESI) *m*/*z* 425.4 (M+H)⁺.

### Example 863C

### tert-butyl ((3R,4S)-4-(4-fluorophenyl)-1-((1-methyl-1H-imidazol-4-yl)sulfonyl)pyrrolidin-3-yl)(2-hydroxyhexyl)carbamate

To Example 863B (140 mg, 0.33 mmol) in dry tetrahydrofuran (3 mL) was added diisopropylethylamine (0.1 mL, 0.56 mmol), followed by di-tert-butyl dicarbonate (94 mg, 0.43 mmol) at 0 °C. This was stirred at 0 °C for 3 hours. Then ice bath was removed and stirred was continued at room temperature for 30 minutes. The reaction mixture was concentrated, and the residue obtained was dissolved in ethyl acetate (30 mL), and then washed with H₂O, and separated. The organic layer was dried over Na₂SO₄ and concentrated down to give the title product. MS (ESI) *m*/*z* 525.4 (M+H)⁺.

### Example 863D

### 5-butyl-3-{(3R,4S)-4-(4-fluorophenyl)-1-[(1-methyl-1H-imidazol-4-yl)sulfonyl]pyrrolidin-3-yl}-1,3-oxazolidin-2-one

To Example 863C (174 mg, 0.33 mmol) was added 1.5 mL of dry tetrahydrofuran under N₂, then were added NaH (26.5 mg, 0.66 mmol, 60% dispersion in mineral oil) and MeI (70.6 mg, 0.49 mmol). The reaction mixture was heated up to 60°C for 1 hour, then quenched with H₂O, and extracted with ethyl acetate. The organic layer was concentrated down and purified by reverse phase HPLC column to provide the title product. MS (ESI) *m*/*z* 451.2 (M+H)⁺.

### Example 864

### 4-{(3R,4S)-4-(4-fluorophenyl)-1-[(1-methyl-1H-imidazol-4-yl)sulfonyl]pyrrolidin-3-yl}thiomorpholine 1,1-dioxide

In a 5 mL microwave vial was added (3R,4S)-4-(4-fluorophenyl)-1-((1-methyl-1H-imidazol-4-yl)sulfonyl)pyrrolidin-3-amine (100 mg, 0.308 mmol) and (vinylsulfonyl)ethene (36.4 mg, 0.308 mmol) in 3 mL THF. The vial was stirred with the cap off for 30 seconds and then capped and reacted in the microwave at 100°C for 10 minutes. The reaction mixture was dried under nitrogen, was taken up in 2 mL 50%MeOH/DMSO, and was then purified by reverse phase HPLC to provide the desired compound. MS (ESI) m/z 443 (M+H)⁺

### Example 865

### 5-fluoro-N-{(3R,4S)-4-(4-fluorophenyl)-1-[(1-methyl-1H-imidazol-4-yl)sulfonyl]pyrrolidin-3-yl}-4-(trifluoromethyl)pyridin-2-amine

The title compound was prepared according to the procedure outlined in Example 327 substituting 2-bromo-5-fluoro-4-(trifluoromethyl)pyridine for 1-bromo-3-(trifluoromethyl)benzene. MS (ESI) *m*/*z* 488 (M+H)⁺.

### Example 866

### (3R,4S)-1-[(1-methyl-1H-imidazol-4-yl)sulfonyl]-4-[(2R)-tetrahydro-2H-pyran-2-yl]-N-[3-(trifluoromethoxy)phenyl]pyrrolidin-3-amine

### Example 866 A

### Tetrahydro-2H-pyran-2-carbaldehyde

To a solution of oxalyl dichloride (131 g, 1.03 mol) in dichloromethane (700 mL) was added dimethyl sulfoxide (87 g, 1.12 mol) slowly at -78°C, the reaction was kept at -78°C for 10 min, then (tetrahydro-2H-pyran-2-yl)methanol (100 g, 0.86 mol) was added dropwise and the reaction kept at - 78°C for another 10 min when the addition was completed. Then at -78 °C was added triethylamine (226 g, 2.24 mol) dropwise and the reaction was allowed to come to room temperature for 30 min. Three additional vials were set up as described above. All three reaction mixtures were combined and the mixture was diluted with dichloromethane and filtered through a pad of celite. The filtrate was concentrated with no heat to afford the title product which was used in next step without further purification.

### Example 866 B

### (E)-2-(2-nitrovinyl)tetrahydro-2H-pyran

Example 866A (350 g, 3.07 mol) was dissolved in tetrahydrofuran (1000 mL) under N₂ atmosphere, and nitromethane (374 g, 6.13 mol) was added in one portion. To the stirred solution was added 1,1,3,3-tetramethylguanidine (17.66 g, 0.15 mol), and a mild exothermo reaction ensued. The reaction was stirred for 2.5 hrs at 20°C, then concentrated and purified by silica gel to provide the intermediate. The intermediate was dissolved in tetrahydrofuran (500 mL) under N₂ atmosphere at chilled ice bath, and 2,2,2-trifluoroacetic anhydride (644 g, 3.07 mol) was added rapidly. The reaction was kept for 15 min, then triethylamine (621 g, 6.13 mmol) was added dropwise and the reaction was kept for another 15 min. The reaction mixture was then extracted by ethyl acetate and water, the organic layers were combined and concentrated, and the residue obtained was purified by silica gel to provide the title product.

### Example 866 C

### 1 -benzyl-3 -nitro-4-(tetrahydro-2H-pyran-2-yl)pyrrolidine

The title compound was prepared according to the procedure outlined in Example 2A substituting Example 866B for 4-methoxy-beta-nitrostyrene.

### Example 866 D

### 1-benzyl-4-(tetrahydro-2H-pyran-2-yl)pyrrolidin-3-amine

The title compound was prepared according to the procedure outlined in Example 2B substituting Example 866C for Example 2A.

### Example 866 E

### tert-butyl (1-benzyl-4-(tetrahydro-2H-pyran-2-yl)pyrrolidin-3-yl)carbamate

The title compound was prepared according to the procedure outlined in Example 337C substituting Example 866D for Example 337B.

### Example 866 F

### tert-butyl (4-(tetrahydro-2H-pyran-2-yl)pyrrolidin-3 -yl)carbamate

The title compound was prepared according to the procedure outlined in Example 2D substituting Example 866E for 2C.

### Example 866 G

### tert-butyl (1-((1-methyl-1H-imidazol-4-yl)sulfonyl)-4-(tetrahydro-2H-pyran-2-yl)pyrrolidin-3-yl)carbamate

The title compound was prepared according to the procedure outlined in Example 373C substituting Example 866F for Example 373B.

### Example 866 H

### tert-butyl ((3R,4S)-1-((1-methyl-1H-imidazol-4-yl)sulfonyl)-4-((R)-tetrahydro-2H-pyran-2-yl)pyrrolidin-3 -yl)carbamate

Example 866 G (22 g, 53.1 mmol) was dissolved in methanol (500 ml) keeping the concentration at 44 mg/ml and then chirally separated using the following conditions:
{Column: Chiralpak AD-H, 3 cm ID x 25 cm, Mobile Phase SFC CO2, Back pressure: 100 Bar,
Pressure drop: 81 Bar, Modifier: methanol 12%. Flow Rate: 100 gm/min, Detector: UV 224 nm
Sample concentration: 40 mg/mL in Methanol, Sample load: 2 mL (80 mg)}

The title product was detected by LCMS at t_{R} = 2.801 min, 100% purity, m/z = 415.2(M+H)⁺ Method: LC/MS (The gradient was 1-90% B in 3.4 min, 90-100% B in 0.45 min, 100-1% B in 0.01 min, and then held at 1% B for 0.65 min (0.8 mL/min flow rate). Mobile phase A was 0.0375% trifluoroacetic acid in water, mobile phase B was 0.018% trifluoroacetic acid in acetonitrile. The column used for the chromatography was a 2.1 x 50 mm Venusil XBP-C18 column (5 µm particles). Detection methods are diode array (DAD) and evaporative light scattering (ELSD) detection as well as positive/negative electrospray ionization.

### Example 866 I

### (3R,4S)-1-((1-methyl-1H-imidazol-4-yl)sulfonyl)-4-((R)-tetrahydro-2H-pyran-2-yl)pyrrolidin-3-amine

The title compound was prepared as the hydrochloride salt according to the procedure outlined in Example 373B substituting Example 866 H for Example 373A. The compound thus obtained was free based according to the procedure outlined in Example 380A substituting the hydrochloride salt of Example 8661 for Example 322B.

### Example 866 J

### (3R,4S)-1-[(1-methyl-1H-imidazol-4-yl)sulfonyl]-4-[(2R)-tetrahydro-2H-pyran-2-yl]-N-[3-(trifluoromethoxy)phenyl]pyrrolidin-3-amine

The titled compound was prepared according to the procedure outlined in Example 337G substituting Example 8661 for Example 337F and 1-bromo-3-(trifluoromethoxy)benzene for 1-bromo-3-(trifluoromethyl)benzene. MS (ESI) m/z 475.1 (M+H)⁺

### Example 867

### (3R,4S)-4-(4-fluorophenyl)-1-[(1-methyl-1H-imidazol-4-yl)sulfonyl]-N-[2-methyl-5-(prop-1-en-2-yl)cyclohexyl]pyrrolidin-3-amine

In a 4 mL vial was added (3R,4S)-4-(4-fluorophenyl)-1-((1-methyl-1H-imidazol-4-yl)sulfonyl)pyrrolidin-3-amine (40 mg, 0.123 mmol) and 2-methyl-5-(prop-1-en-2-yl)cyclohexanone (21 mg, 0.135 mmol). Then a buffer pH=4 solution (2 mL, made from 48 g AcOH and 30.5 g NaOAc in 1 L methanol) was added followed by the addition of Si-cyanoborohydride (350 mg, 0.311 mmol, load capacity: 0.89 mmol/g, Silicycle Catalog number: R66730B). The vial was capped and reacted for 16 hours. The reaction mixture was then filtered and purified by reverse phase HPLC to afford the title compound. MS (ESI) m/z 461 (M+H)⁺

### Example 868

### (3R,4S)-4-(4-fluorophenyl)-1-[(1-methyl-1H-imidazol-4-yl)sulfonyl]-N-(2,2,2-trifluoro-1-phenylethyl)pyrrolidin-3-amine

To a solution of Example 322B (100 mg, 0.3 mmol) in dichloromethane (5 mL) was added 2,2,2-trifluoro-1-phenylethanone (53.7 mg, 0.3 mmol) followed by trimethyl aluminum (617 uL, 1 M in heptane). The mixture was stirred for 3 hours, and Borane-dimehtylsulfide complex (308 uL, 2 M in tetrahydrofuran) was added. The mixture was stirred 2 hours. Then the reaction was partitioned between sodium hydroxide (1 M, aq) and dichloromethane. The organic fraction was collected, and purification via HPLC afforded the title compound. MS (DCI) *m*/*z* 483 (M+H)⁺.

### Example 869

### N-[2-chloro-4-(trifluoromethyl)benzyl]-2-[(1-methyl-1H-imidazol-4-yl)sulfonyl]octahydro-3aH-isoindol-3a-amine

### Example 869A

### tert-butyl trans-2-benzyloctahydro-1H-isoindol-3a-ylcarbamate

The title compound was prepared according to the procedure outlined in Example 337C substituting trans-2-benzyloctahydro-1H-isoindol-3a-amine for Example 337B.

### Example 869B

### tert-butyl trans-octahydro-1H-isoindol-3a-ylcarbamate

The title compound was prepared according to the procedure outlined in Example 2D substituting Example 869A for 2C.

### Example 869C

### tert-butyl trans-2-(1-methyl-1H-imidazol-4-ylsulfonyl)octahydro-1H-isoindol-3a-ylcarbamate

The title compound was prepared according to the procedure outlined in Example 373C substituting Example 869B for Example 373B.

### Example 869D

### Trans-2-(1-methyl-1H-imidazol-4-ylsulfonyl)octahydro-1H-isoindol-3a-amine

The title compound was prepared as the hydrochloride salt according to the procedure outlined in Example 373B substituting Example 869C for Example 373A.

### Example 869E

### Trans-N-[2-chloro-4-(trifluoromethyl)benzyl]-2-[(1-methyl-1H-imidazol-4-yl)sulfonyl]octahydro-3aH-isoindol-3a-amine

The title compound was prepared according to the procedure outlined in Example 354E substituting 2-chloro-4-(trifluoromethyl)benzaldehyde for 4-chlorobenzaldehyde and substituting Example 869D for Example 354D. MS (ESI) *m*/*z* 477/479 (3:1) (M+H)⁺.

### Example 870

### 2-chloro-N-{2-[(1-methyl-1H-imidazol-4-yl)sulfonyl]octahydro-3aH-isoindol-3a-yl}-3-(trifluoromethyl)benzamide

The title compound was prepared similarly to the procedure described in Example 404A substituting Example 869D for Example 14A and substituting 2-chloro-3-(trifluoromethyl)benzoic acid for 2-nitro-5-(trifluoromethyl)benzoic acid. MS (ESI) *m*/*z* 491/493 (3:1) (M+H)⁺.

### Example 871

### 2,4-dichloro-N-{2-[(1-methyl-1H-imidazol-4-yl)sulfonyl]octahydro-3aH-isoindol-3a-yl}benzamide

The title compound was prepared similarly to the procedure described in Example 404A substituting Example 869D for Example 14A and substituting 2,4-dichloro-3-benzoic acid for 2-nitro-5-(trifluoromethyl)benzoic acid. MS (ESI) *m*/*z* 457/459/461 (9:6:1) (M+H)⁺.

### Example 872

### 1-hexyl-6-[(1-methyl-1H-imidazol-4-yl)sulfonyl]octahydro-1H-pyrrolo[3,4-b]pyridine

### Example 872A

### tert-butyl 6-(1-methyl-1H-imidazol-4-ylsulfonyl)octahydro-1H-pyrrolo[3,4-b]pyridine-1-carboxylate

The title compound was prepared according to the procedure outlined in Example 373C substituting tert-butyl octahydro-1H-pyrrolo[3,4-b]pyridine-1-carboxylate for Example 373B.

### Example 872B

### 6-(1-methyl-1H-imidazol-4-ylsulfonyl)octahydro-1H-pyrrolo[3,4-b]pyridine

The title compound was prepared as the hydrochloride salt according to the procedure outlined in Example 373B substituting Example 872A for Example 373A.

### Example 872C

### 1-hexyl-6-[(1-methyl-1H-imidazol-4-yl)sulfonyl]octahydro-1H-pyrrolo[3,4-b]pyridine

The title compound was prepared according to the procedure outlined in Example 354E substituting hexanal for 4-chlorobenzaldehyde and substituting Example 872B for Example 354D. MS (ESI) *m*/*z* 355 (M+H)⁺.

### Example 873

### (2,4-dichlorophenyl){6-[(1-methyl-1H-imidazol-4-yl)sulfonyl]octahydro-1H-pyrrolo[3,4-b]pyridin-1-yl}methanone

The title compound was prepared similarly to the procedure described in Example 404A substituting Example 872B for Example 14A and substituting 2,4-dichlorobenzoic acid for 2-nitro-5-(trifluoromethyl)benzoic acid. MS (ESI) *m*/*z* 443/445/447 (9:6:1) (M+H)⁺.

### Example 874

### [2-chloro-3-(trifluoromethyl)phenyl]{6-[(1-methyl-1H-imidazol-4-yl)sulfonyl]octahydro-1H-pyrrolo[3,4-b]pyridin-1-yl}methanone

The title compound was prepared similarly to the procedure described in Example 404A substituting Example 872B for Example 14A and substituting 2-chloro-3-(trifluoromethyl)benzoic acid for 2-nitro-5-(trifluoromethyl)benzoic acid. MS (ESI) *m*/*z* 477/479 (3:1) (M+H)⁺.

### Example 875

### (3R,4S)-4-(4-fluorophenyl)-1-[(1-methyl-1H-imidazol-4-yl)sulfonyl]-N-[2,2,2-trifluoro-1-(3-methylphenyl)ethyl]pyrrolidin-3-amine

The title compound was prepared similarly to the procedure described in Example 868 substituting 2,2,2-trifluoro-1-(m-tolyl)ethanone for 2,2,2-trifluoro-1-phenylethanone. MS (ESI) *m*/*z* 497 (M+H)⁺.

### Example 876

### (3R,4S)-4-(4-fluorophenyl)-1-[(1-methyl-1H-imidazol-4-yl)sulfonyl]-N-[2,2,2-trifluoro-1-(4-methylphenyl)ethyl]pyrrolidin-3-amine

The title compound was prepared similarly to the procedure described in Example 868 substituting 2,2,2-trifluoro-1-(p-tolyl)ethanone for 2,2,2-trifluoro-1-phenylethanone. MS (ESI) *m*/*z* 497 (M+H)⁺.

### Example 877

### (1R,2R,3R,4S)-3-(4-fluorophenyl)-N-[4-fluoro-3-(trifluoromethyl)phenyl]-7-[(1-methyl-1H-imidazol-4-yl)sulfonyl]-7-azabicyclo[2.2.1]heptan-2-amine

### Example 877A

### tert-butyl 2-(trimethylsilyl)pyrrolidine-1-carboxylate

A solution of tert-butyl pyrrolidine-1-carboxylate (6.84 g, 39.9 mmol) in dry Ether (40 mL) charged into a 250 mL flask equipped with a magnetic stirring bar and argon gas balloon, was cooled to -78 °C. Tetramethylethylenediamine (7.23 mL, 47.9 mmol) followed by sec-butyl lithium (36.9 mL, 47.9 mmol) (1.3 M solution in hexane) was introduced. The mixture was allowed to stir for 2 hours at -78 °C. Trimethylsilyl chloride (6.13 mL, 47.9 mmol) was added dropwise. After addition, the reaction mixture was allowed to warm to room temperature gradually and then diluted with 15 mL of saturated aqueous ammonium chloride solution. The organic layer was separated, and the aqueous layer was extracted with ethyl acetate (2 × 30 mL). The combined extracts were dried over sodium sulfate, filtered and concentrated. The crude oily residue, obtained after the concentration, was purified by fractional distillation (bp 55 °C/0.5 mm) to give Example 877A as a colorless oil. MS (ESI) m/z 188 [M-C₄H₈+1]⁺.

### Example 877B

### tert-butyl 2,5-bis(trimethylsilyl)pyrrolidine-1-carboxylate

A 250 mL three-neck flask, equipped with a magnetic stirring bar and argon gas balloon, was charged with a solution of Example 877A (4.86 g, 20.0 mmol) in dry Ether (30 mL) and was cooled to -45 °C. Tetramethylethylenediamine (6.03 mL, 39.9 mmol) followed by sec-butyl lithium (30.7 mL, 39.9 mmol) (1.3 M in hexane) was added to the flask dropwise while stirring. After 15 minutes of stirring at -45 °C, the temperature was raised to -30 °C. After 30 minutes, it was re-cooled to -45 °C and afterward trimethylsilyl chloride (5.10 mL, 39.9 mmol) was added dropwise. The reaction mixture was allowed to warm to room temperature (about 25 °C), and diluted with 10 mL of saturated aqueous ammonium chloride solution. The solution was extracted with ethyl acetate (30 mL × 3). The combined organic layers were dried over sodium sulfate, filtered and concentrated. The resulting mixture was deposited onto silica gel, loaded onto a silica gel column and eluted with petroleum ether/ethyl acetate (100:1) to give Example 877B as a pale yellow oil. MS (ESI) m/z 260 [M-C₄H₈+1]⁺.

### Example 877C

### benzyl-2,5-bis(trimethylsilyl)pyrrolidine

A stirring solution of Example 877B (16.1 g, 51.0 mmol) in DCM (160 mL) was cooled to 0 °C and was treated with trifluoroacetic acid (32 mL) drop-wise. The mixture was allowed to warm to room temperature (about 25 °C), and stirring was continued for an additional 2 hours. The reaction mixture was re-cooled to 0 °C and basified with 20% aqueous sodium hydroxide solution to pH = 10. The organic layer was separated, and the aqueous layer was extracted with CH₂Cl₂ (2 × 60 ml). The combined extracts were washed with brine, dried over Na₂SO₄, and concentrated to give 13 g of crude amine (contain some salt) which was utilized for the next step directly without further purification. To a 260 mL solution of the crude amine (13 g, 51 mmol) in acetonitrile, were added potassium carbonate (8.47 g, 61.3 mmol) and benzyl bromide (7.86 g, 45.9 mmol). The resultant suspension was stirred at room temperature for 16 hours. The mixture was filtered, and the solvent was evaporated under vacuum. The crude yellow oil was purified by silica gel column chromatography, eluting with ethyl acetate/petroleum ether (0:1 to 1:50), to obtain Example 877C as a pale yellow oil. MS (ESI) m/z 306 [M+1]⁺.

### Example 877D

### 7-benzyl-2-(4-fluorophenyl)-3-nitro-7-azabicyclo[2.2.1]heptane

(E)-1-fluoro-4-(2-nitrovinyl)benzene (3.95 g, 23.65 mmol) and Example 877C (6.49 ml, 19.71 mmol) were dissolved in 75 mL acetonitrile under an argon atomosphere. Silver(I) fluoride (5 g, 39.4 mmol) was added in a single portion and the reactoin was allowed to proceed overnight. At this time, LC/MS indicated an excess of nitrostyrene present. An additional 0.5 eq of 1-benzyl-2,5-bis(trimethylsilyl)pyrrolidine (3.25 mL, 9.85 mmol) and NaF (2.5 g, 19.7 mmol) were added. The reaction proceeded further to 85% conversion. The contents were concentrated, taken up in ethyl acetate, washed with sodium bicarbonate, water, and brine. The crude material was purified via FLASH 0-50% ethyl acetate/hexane (120g column) to yield Example 877D as a yellow oil. MS (APCI) m/z 327 [M+1]⁺.

### Example 877E

### 7-benzyl-2-(4-fluorophenyl)-3-nitro-7-azabicyclo[2.2.1]heptane

Example 877D (850 mg, 2.60 mmol) and tetrahydrofuran (10 ml) were added to a Raney Nickel 2800 water slurry (900 mg, 6.90 mmol) in a 50 ml pressure bottle. The contents were stirred for 16 hr under 30 psi hydrogen gas. The mixture was filtered through a nylon membrane and concentrated to yield Example 877E. MS (DCI) *m*/*z* 297 (M+H)⁺.

### Example 877F

### tert-butyl 7-benzyl-3-(4-fluorophenyl)-7-azabicyclo[2.2.1]heptan-2-ylcarbamate

In a 100 mL round bottom flask were added 15 mL dichloromethane and Example 877E. BOC-Anhydride (1116 µl, 4.81 mmol) was dissolved in 5 mL DCM and added dropwise via an addition funnel. The reaction was allowed to proceed for 2 hr. The contents were dried by rotovap and purified by FLASH chromatography (0-50% ethyl acetate/heptane) to yield Example 877F. MS (APCI) *m*/*z* 397 (M+H)⁺.

### Example 877G

### tert-butyl 3-(4-fluorophenyl)-7-azabicyclo[2.2.1]heptan-2-ylcarbamate

Example 877F (1.07 g, 2.70 mmol) and tetrahydrofuran (25 ml) were added to wet 20% Palladium Hydroxide on carbon (0.25 g, 0.182 mmol) in a 50 ml pressure bottle. The contents were stirred for 32 hr under 30 psi hydrogen gas at room temperature. The mixture was filtered through a nylon membrane and concentrated to yield Example 877G. MS (DCI) *m*/*z* 307 (M+H)⁺.

### Example 877H

### 3-(4-fluorophenyl)-7-((1-methyl-1H-imidazol-4-yl)sulfonyl)-7-azabicyclo[2.2.1]heptan-2-amine hydrochloride

In a 20 mL scintillation vial were added 5 mL dichloromethane and Example 877G. To this solution was added 1-methyl-1H-imidazole-4-sulfonyl chloride (749 mg, 4.15 mmol) followed by DIEA (966 µl, 5.53 mmol). The reaction was allowed to proceed for 3 hours and the contents were then concentrated and purified via FLASH chromatography (30-100% ethyl acetate/heptane). The collected fractions were combined and dried. The solid was dissolved in 5 mL dichloromethane and then 5 mL of a 4 N hydrochloric acid solution in dioxane was added. The reaction was allowed to proceed for 72 hour and then the white precipitate was collected by filtration to yield Example 877H as a white powder. MS (APCI) *m*/*z* 351 (M+H)⁺.

### Example 8771

### N-(4-fluoro-3-(trifluoromethyl)phenyl)-3-(4-fluorophenyl)-7-((1-methyl-1H-imidazol-4-yl)sulfonyl)-7-azabicyclo[2.2.1]heptan-2-amine

In a 10 mL microwave vial was added Example 877H (30 mg, 0.086 mmol), 4-bromo-1-fluoro-2-(trifluoromethyl)benzene (20.80 mg, 0.086 mmol), sodium ter-butoxide (16.46 mg, 0.171 mmol), BINAP (2.67 mg, 4.28 µmol) and bis (dibenzylideneacetone)palladium (1.231 mg, 2.140 µmol) followed by the addition of 2 mL dimethoxyethane. The reaction vessel was capped and the reaction mixture was heated under microwave conditions for 15 min at 130°C. Upon completion, the mixture was filtered through celite, concentrated, dissolved in 2 ml 50% MeOH/DMSO, and purified by reverse phase HPLC to obtain the title compound as one of the two products. MS (ESI) m/z 512 (M+H)⁺

### Example 878

### 3-(4-fluorophenyl)-N-[4-fluoro-3-(trifluoromethyl)phenyl]-7-[(1-methyl-1H-imidazol-4-yl)sulfonyl]-7-azabicyclo[2.2.1]heptan-2-amine

The desired compound was obtained as described in Example 877 as the second product from the reverse phase HPLC purification. MS (ESI) m/z 512 (M+H)⁺

### Example 879

### 1-[2-chloro-4-(trifluoromethyl)benzyl]-6-[(1-methyl-1H-imidazol-4-yl)sulfonyl]octahydro-1H-pyrrolo[3,4-b]pyridine

The title compound was prepared according to the procedure outlined in Example 354E substituting 2-chloro-4-(trifluoromethyl)benzaldehyde for 4-chlorobenzaldehyde and substituting Example 872B for Example 354D. MS (ESI) *m*/*z* 463/465 (3:1) (M+H)⁺.

### Example 880

### [2-chloro-4-(trifluoromethyl)phenyl]{6-[(1-methyl-1H-imidazol-4-yl)sulfonyl]octahydro-1H-pyrrolo[3,4-b]pyridin-1-yl}methanone

The title compound was prepared similarly to the procedure described in Example 404A substituting Example 872B for Example 14A and substituting 2-chloro-4-(trifluoromethyl)benzoic acid for 2-nitro-5-(trifluoromethyl)benzoic acid. MS (ESI) *m*/*z* 477/479 (3:1) (M+H)⁺.

### Example 881

### N-(3-chloro-4-fluorobenzyl)-2-[(1-methyl-1H-imidazol-4-yl)sulfonyl]-2-azaspiro[4.5]decane-4-carboxamide

### Example 881A

### Ethyl 2-benzyl-2-azaspiro[4.5]decane-4-carboxylate

The title compound was prepared according to the procedure outlined in Example 2A substituting ethyl 2-cyclohexylideneacetate for 4-methoxy-beta-nitrostyrene.

### Example 881B

### Ethyl 2-azaspiro[4.5]decane-4-carboxylate

The title compound was prepared according to the procedure outlined in Example 2D substituting Example 881A for 2C.

### Example 881C

### Ethyl 2-(1-methyl-1H-imidazol-4-ylsulfonyl)-2-azaspiro[4.5]decane-4-carboxylate

The title compound was prepared according to the procedure outlined in Example 373C substituting Example 881B for Example 373B.

### Example 881D

### 2-(1-methyl-1H-imidazol-4-ylsulfonyl)-2-azaspiro[4.5]decane-4-carboxylic acid

The title compound was prepared according to the procedure outlined in Example 242D substituting Example 881C for 242C.

### Example 881E

### N-(3-chloro-4-fluorobenzyl)-2-[(1-methyl-1H-imidazol-4-yl)sulfonyl]-2-azaspiro[4.5]decane-4-carboxamide

The title compound was prepared similarly to the procedure described in Example 404A substituting 3-chloro-4-fluoro-benzyl amine for Example 14A and substituting Example 881D for 2-nitro-5-(trifluoromethyl)benzoic acid. MS (ESI) *m*/*z* 469/471 (3:1) (M+H)⁺.

### Example 882

### N-(3,4-difluorobenzyl)-2-[(1-methyl-1H-imidazol-4-yl)sulfonyl]-2-azaspiro[4.5]decane-4-carboxamide

The title compound was prepared similarly to the procedure described in Example 404A substituting 3,4-difluoro-benzyl amine for Example 14A and substituting Example 881D for 2-nitro-5-(trifluoromethyl)benzoic acid. MS (ESI) *m*/*z* 453 (M+H)⁺.

### Example 883

### 1-{(3R,4S)-4-(4-fluorophenyl)-1-[(1-methyl-1H-imidazol-4-yl)sulfonyl]pyrrolidin-3-yl}-1,3-dihydro-2H-indol-2-one

### Example 883A

### 2-(2-bromophenyl)-N-((3R,4S)-4-(4-fluorophenyl)-1-(1-methyl-1H-imidazol-4-ylsulfonyl)pyrrolidin-3-yl)acetamide

To a solution of Example 322B (200 mg, 0.62 mmol) in a 1:1 dichloromethane:dimethylformamide solution (6 mL) was added 2-(2-bromophenyl)acetic acid (159 mg, 0.74 mmol) and N1-((ethylimino)methylene)-N3,N3-dimethylpropane-1,3-diamine hydrochloride (177 mg, 0.93 mmol). The solution was stirred for 16 hours. Brine and EtOAc were added and the organic fraction was collected. The organic fraction was washed with brine and 1 M HCl (aq). Purification via flash chromatography (0-100% EtOAc/heptanes) afforded the title compound.

### Example 883B

### 1-{(3R,4S)-4-(4-fluorophenyl)-1-[(1-methyl-1H-imidazol-4-yl)sulfonyl]pyrrolidin-3-yl}-1,3-dihydro-2H-indol-2-one

Example 883A (254 mg, 0.49 mmol) was dissolved in hot tert-butanol (15 mL) and added to a vial containing dicyclohexyl(2',4',6'-triisopropyl-[1,1'-biphenyl]-2-yl)phosphine (17.4 mg, 0.04 mmol), phenylboronic acid (4.5 mg, 0.04 mmol), diacetoxypalladium (3.3 mg, 0.02 mmol), and potassium carbonate (168 mg, 1.2 mmol). The vial was sealed and stirred at 90 °C for 14 hours. Concentration followed by purification via HPLC afforded the title compound. MS (ESI) *m*/*z* 441 (M+H)⁺.

### Example 884

### 2-[(1-methyl-1H-imidazol-4-yl)sulfonyl]-N-[4-(trifluoromethyl)benzyl]octahydro-3aH-isoindol-3a-amine

### Example 884A

### tert-butyl (2-benzyloctahydro-1H-isoindol-3a-yl)carbamate

To 2-benzyloctahydro-1H-isoindol-3a-amine (3.08 g, 13.4 mmol) in 30 mL of ethyl acetate was added di-tert-butyl dicarbonate (3.27 g, 15.0 mmol). This was stirred at room temperature overnight. Then water (30 mL) was added, separated, and the organic layer was washed with water one more time. The organic layer was dried over Na₂SO₄, concentrated down and purified by flash-chromatography on silica gel with 0-7% methanol in dichloromethane to provide the title product MS (ESI) *m*/*z* 331.2 (M+H)⁺.

### Example 884B

### tert-butyl (octahydro-1H-isoindol-3a-yl)carbamate

To Example 884A (4.13 g, 12.50 mmol) in 80 ml of tetrahydrofuran was added 20% Pd(OH)₂/C, wet (0.85 g, 0.617 mmol) in a 250 mL SS pressure bottle. This was stirred for 15 hours at 30 psi and 50 °C. The mixture was filtered through a nylon membrane. The obtained solution was concentrated down to afford the title product. MS (ESI) *m*/*z* 241.0 (M+H)⁺.

### Example 884C

### tert-butyl (2-((1-methyl-1H-imidazol-4-yl)sulfonyl)octahydro-1H-isoindol-3a-yl)carbamate

The title compound was prepared using the same sequence of steps as described in Example 182C by substituting Example 884B for Example 182 B. MS (ESI) *m*/*z* 385.4 (M+H)⁺.

### Example 884D

### 2-((1-methyl-1H-imidazol-4-yl)sulfonyl)octahydro-1H-isoindol-3a-amine

The title compound was prepared as the hydrochloride salt according to the procedure outlined in Example 373B substituting Example 884C for Example 373A. Then it was free based according to the procedure outlined in Example 380A substituting Example 884D HCl for Example 322B.

### Example 884E

### 2-[(1-methyl-1H-imidazol-4-yl)sulfonyl]-N-[4-(trifluoromethyl)benzyl]octahydro-3aH-isoindol-3a-amine

The title compound was prepared according to the procedure outlined in Example 354E substituting 4-(trifluoromethyl)benzaldehyde for 4-chlorobenzaldehyde and substituting Example 884D for Example 354D. MS (ESI) *m*/*z* 443.1 (M+H)⁺.

### Example 885

### trans-N-[2-chloro-4-(trifluoromethyl)benzyl]-2-[(1-methyl-1H-imidazol-4-yl)sulfonyl]-2,3,3a,8-tetrahydroindeno[1,2-c]pyrrol-8a(1H)-amine

### Example 885A

### 2-nitro-1H-indene

To indene (5 g, 43 mmol) in THF (100 mL) was added NaNO₂ (5.44g, 118 mmol) then I₂ (18.7 g, 74 mmol) in portions at r.t. The mixture was stirred for 2h and Na₂S₂O₅(aq) was added until the color mostly faded. THF was removed, the residue extracted with ethyl acetate, and the crude material was purified by silica gel column chromatography eluting with hexanes and ethyl acetate (20:1) to afford the title compound.

### Example 885B

### 2-benzyl-8a-nitro-1,2,3,3a,8,8a-hexahydroindeno[2,1-c]pyrrole

The title compound was prepared according to the procedure outlined in Example 2A substituting Example 885A for 4-methoxy-beta-nitrostyrene.

### Example 885C

### 2-benzyl-1,2,3,3a,8,8a-hexahydroindeno[2,1-c]pyrrol-8a-amine

The title compound was prepared according to the procedure outlined in Example 2B substituting Example 885B for Example 2A.

### Example 885D

### tert-butyl 2-benzyl-1,2,3,3a,8,8a-hexahydroindeno[2,1-c]pyrrol-8a-ylcarbamate

The title compound was prepared according to the procedure outlined in Example 337C substituting Example 885C for Example 337B.

### Example 885E

### tert-butyl 1,2,3,3a,8,8a-hexahydroindeno[2,1-c]pyrrol-8a-ylcarbamate

The title compound was prepared according to the procedure outlined in Example 2D substituting Example 885D for 2C.

### Example 885F

### tert-butyl2-(1-methyl-1H-imidazol-4-ylsulfonyl)-1,2,3,3a,8,8a-hexahydroindeno[2,1-c]pyrrol-8a-ylcarbamate

The title compound was prepared according to the procedure outlined in Example 373C substituting Example 885E for Example 373B.

### Example 885G

### 2-(1-methyl-1H-imidazol-4-ylsulfonyl)-1,2,3,3a,8,8a-hexahydroindeno[2,1-c]pyrrol-8a-amine

The title compound was prepared as the hydrochloride salt according to the procedure outlined in Example 373B substituting Example 885F for Example 373A.

### Example 885H

### trans-N-[2-chloro-4-(trifluoromethyl)benzyl]-2-[(1-methyl-1H-imidazol-4-yl)sulfonyl]-2,3,3a,8-tetrahydroindeno[1,2-c]pyrrol-8a(1H)-amine

The title compound was prepared according to the procedure outlined in Example 354E substituting 3-chloro-4-(trifluoromethyl)benzaldehyde for 4-chlorobenzaldehyde and substituting Example 885G for Example 354D. MS (ESI) *m*/*z* 511/513 (3:1) (M+H)⁺.

### Example 886

### 2-{4-[(4-fluorophenoxy)methyl]-1-[(1-methyl-1H-imidazol-4-yl)sulfonyl]pyrrolidin-3pyridine

### Methyl 1-((1-methyl-1H-imidazol-4-yl)sulfonyl)-4-(pyridin-2-yl)pyrrolidine-3-carboxylate

### Example 886 A

The title compound was prepared according to the procedures outlined in Example 242A to 242C substituting (E)-methyl 3-(pyridin-2-yl)acrylate for (E)-methyl 3-(4-fluorophenyl)acrylate.

### Example 886 B

### 1-((1 -methyl-1H-imidazol-4-yl)sulfonyl)-4-(pyridin-2-yl)pyrrolidin-3-yl)methanol

The title compound was prepared according to the procedure outlined in Example 206 substituting Example 886A for Example 242 C. MS (ESI) m/z 323.1 (M+H)⁺.

### Example 886 C

### 2-{4-[(4-fluorophenoxy)methyl]-1-[(1-methyl-1H-imidazol-4-yl)sulfonyl]pyrrolidin-3-yl}pyridine

The title compound was prepared according to the procedure outlined in Example 245 substituting Example 886B for Example 206 C and substituting 4-fluorophenol for phenol MS (ESI) m/z 417.1 (M+H)⁺.

### Reference Example 887

### 1-[(1-methyl-1H-imidazol-4-yl)sulfonyl]-N-[3-(trifluoromethoxy)phenyl]-2,3-dihydro-1H-indol-3-amine

### Example 887A

### tert-butyl (1-((1-methyl-1H-imidazol-4-yl)sulfonyl)indolin-3-yl)carbamate

The title compound was prepared according to the procedures outlined in Example 1C substituting *tert*-butyl indolin-3-ylcarbamate for Example 1B.

### Example 887B

### 1-((1-methyl-1H-imidazol-4-yl)sulfonyl)indolin-3-amine

The title compound was prepared according to the procedures outlined in Example 1A substituting Example 887A for Example 1A.

### Example 887C

### 1-((1-methyl-1H-imidazol-4-yl)sulfonyl)-N-(3-(trifluoromethoxy)phenyl)indolin-3-amine

The title compound was prepared according to the procedures outlined in Example 29 substituting Example 887B for Example 14A and substituting 1-bromo-3-(trifluoromethoxy)benzene for bromobenzene.

### Example 888

### 2-[(1-methyl-1H-imidazol-4-yl)sulfonyl]-3a-{[6-(trifluoromethyl)pyrimidin-4-yl]oxy}-2,3,3a,4,5,9b-hexahydro-1H-benzo[e]isoindole

### Example 888A

### 3-nitro-1,2-dihydronaphthalene

The title compound was prepared according to the procedure outlined in Example 885A substituting 1,2-dihydronaphthalene for indene.

### Example 888B

### 2-benzyl-3a-nitro-2,3,3a,4,5,9b-hexahydro-1H-benzo[e]isoindole

The title compound was prepared according to the procedure outlined in Example 2A substituting Example 888A for 4-methoxy-beta-nitrostyrene.

### Example 888C

### 2-benzyl-2,3,3a,4,5,9b-hexahydro-1H-benzo[e]isoindol-3a-amine

The title compound was prepared according to the procedure outlined in Example 2B substituting Example 888B for Example 2A.

### Example 888D

### tert-butyl 2-benzyl-2,3,3a,4,5,9b-hexahydro-1H-benzo[e]isoindol-3a-ylcarbamate

The title compound was prepared according to the procedure outlined in Example 337C substituting Example 888C for Example 337B.

### Example 888E

### tert-butyl 2,3,3a,4,5,9b-hexahydro-1H-benzo[e]isoindol-3a-ylcarbamate

The title compound was prepared according to the procedure outlined in Example 2D substituting Example 888D for 2C.

### Example 888F

### tert-butyl 2-(1-methyl-1H-imidazol-4-ylsulfonyl)-2,3,3a,4,5,9b-hexahydro-1H-benzo[e]isoindol-3a-ylcarbamate

The title compound was prepared according to the procedure outlined in Example 373C substituting Example 888E for Example 373B.

### Example 888G

### 2-(1-methyl-1H-imidazol-4-ylsulfonyl)-2,3,3a,4,5,9b-hexahydro-1H-benzo[e]isoindol-3a-amine

The title compound was prepared as the hydrochloride salt according to the procedure outlined in Example 373B substituting Example 888F for Example 373A.

### Example 888H

### 2-[(1-methyl-1H-imidazol-4-yl)sulfonyl]-3a-{[6-(trifluoromethyl)pyrimidin-4-yl]oxy}-2,3,3a,4,5,9b-hexahydro-1H-benzo[e]isoindole

The title compound was prepared according to the procedure outlined in Example 337G substituting Example 888G for Example 337F and substituting 4-bromo-6-(trifluoromethyl)pyrimidine for 1-bromo-3-(trifluoromethyl)benzene. MS (ESI) *m*/*z* 480 (M+H)⁺.

### Example 889

### N-(4-fluorobenzyl)-2-[(1-methyl-1H-imidazol-4-yl)sulfonyl]-1,2,3,4,5,9b-hexahydro-3aH-benzo[e]isoindol-3a-amine

The title compound was prepared according to the procedure outlined in Example 354E substituting 4-fluorobenzaldehyde for 4-chlorobenzaldehyde and substituting Example 888G for Example 354D. MS (ESI) *m*/*z* 441 (M+H)⁺.

### Example 890

### N-[2-chloro-4-(trifluoromethyl)benzyl]-2-[(1-methyl-1H-imidazol-4-yl)sulfonyl]-1,2,3,4,5,9b-hexahydro-3aH-benzo[e]isoindol-3a-amine

The title compound was prepared according to the procedure outlined in Example 354E substituting 3-chloro-4-trifluoromethylbenzaldehyde for 4-chlorobenzaldehyde and substituting Example 888G for Example 354D. MS (ESI) *m*/*z* 525/527 (3:1) (M+H)⁺.

### Example 891

### 2-[(1-methyl-1H-1,2,3-triazol-4-yl)sulfonyl]-N-[6-(trifluoromethyl)pyrimidin-4-yl]-2,3,3a,8-tetrahydroindeno[1,2-c]pyrrol-8a(1H)-amine

The title compound was prepared according to the procedure outlined in Example 337G substituting Example 885G for Example 337F and substituting 4-bromo-6-(trifluoromethyl)pyrimidine for 1-bromo-3-(trifluoromethyl)benzene. MS (ESI) *m*/*z* 466 (M+H)⁺.

### Example 892

### 2-[(1-methyl-1H-imidazol-4-yl)sulfonyl]-N-[4-(trifluoromethyl)pyridin-2-yl]-2,3,3a,8-tetrahydroindeno[1,2-c]pyrrol-8a(1H)-amine

The title compound was prepared according to the procedure outlined in Example 337G substituting Example 885G for Example 337F and substituting 4-bromo-6-(trifluoromethyl)pyrimidine for 1-bromo-3-(trifluoromethyl)benzene. MS (ESI) *m*/*z* 466 (M+H)⁺.

### Example 893

### N-(2,3-dihydro-1H-inden-1-yl)-1-[(1-methyl-1H-imidazol-4-yl)sulfonyl]-4-(1-methylpiperidin-2-yl)pyrrolidin-3-amine

### Example 893A

### tert-butyl (trans)-1-(1-methyl-1H-imidazol-4-ylsulfonyl)-4-(pyridin-2-yl)pyrrolidin-3-ylcarbamate

The title compound was prepared similarly to the procedures described in Example 2A-2E substituting Example 307A for (E)-1-methoxy-4-(2-nitrovinyl)benzene.

### Example 893B

### tert-butyl (trans)-1-(1-methyl-1H-imidazol-4-ylsulfonyl)-4-((R)-1-methylpiperidin-2-yl)pyrrolidin-3-ylcarbamate

To a solution of Example 893A (2 g, 4.91 mmol) in acetic acid (20 ml) in a 50 ml pressure bottle was added 5% Pt/C (wet, 0.4 g, 0.843 mmol) and the solution was shaken for 3 days under hydrogen (30 psi) at 50 °C and 1 day at rom temperature. During this time 50 wt% platinum dioxide was added. Concentration afforded crude tert-butyl (trans)-1-(1-methyl-1H-imidazol-4-ylsulfonyl)-4-((R)-piperidin-2-yl)pyrrolidin-3-ylcarbamate. The title compound was obtained by following the procedure described in Example 3A substituting this crude for Example 3A and substituting formaldehyde for benzaldehyde.

### Example 893C

### (trans)-1-(1-methyl-1H-imidazol-4-ylsulfonyl)-4-((R)-1-methylpiperidin-2-yl)pyrrolidin-3-amine

The title compound was prepared as an HCl salt similarly to the procedure described in Example 2F substituting Example 893B for Example 2E.

### Example 893D

### (trans)-N-(2,3-dihydro-1H-inden-1-yl)-1-[(1-methyl-1H-imidazol-4-yl)sulfonyl]-4-(1-methylpiperidin-2-yl)pyrrolidin-3-amine

The title compound was prepared similarly to the procedure described in Example 148 substituting Example 893C for Example 3A and substituting 2,3-dihydro-1H-inden-1-one for benzaldehyde. MS (ESI) *m*/*z* 444 (M+H)⁺.

### Example 894

### 1-[(1-methyl-1H-imidazol-4-yl)sulfonyl]-4-(1-methylpiperidin-2-yl)-N-[3-(2,2,2-trifluoroethoxy)phenyl]pyrrolidin-3-amine

The title compound was prepared similarly to the procedure described in Example 327 substituting Example 893C for Example 322B and substituting 1-bromo-3-(2,2,2-trifluoroethoxy)benzene for 1-bromo-3-(trifluoromethyl)benzene. MS (ESI) *m*/*z* 502 (M+H)⁺.

### Example 895

### 4-(5-fluoropyridin-2-yl)-1-[(1-methyl-1H-imidazol-4-yl)sulfonyl]-N-[3-(trifluoromethoxy)phenyl]pyrrolidin-3-amine

### Example 895A

### (trans)-4-(5-fluoropyridin-2-yl)-1-(1-methyl-1H-imidazol-4-ylsulfonyl)pyrrolidin-3-amine

The title compound was prepared similarly to the procedures described in Examples 307A-307B substituting 5-fluoropicolinaldehyde for 2-pyridinecarboxaldeyde.

### Example 895B

### (trans)-4-(5-fluoropyridin-2-yl)-1-[(1-methyl-1H-imidazol-4-yl)sulfonyl]-N-[3-(trifluoromethoxy)phenyl]pyrrolidin-3-amine

The title compound was prepared similarly to the procedure described in Example 327 substituting Example 895A for Example 322B and substituting 1-bromo-3-(trifluoromethoxy)benzene for 1-bromo-3-(trifluoromethyl)benzene. MS (ESI) *m*/*z* 486 (M+H)⁺.

### Example 896

### (3R,4S)-4-(1,3-dioxan-2-yl)-1-[(1-methyl-1H-1,2,3-triazol-4-yl)sulfonyl]-N-[3-(trifluoromethoxy)phenyl]pyrrolidin-3-amine

### Example 896A

### (E)-3,3-dimethoxy-1-nitroprop-1-ene

To 2,2-dimethoxyacetaldehyde (4 mL 60%) in nitromethane (10 mL) was added potassium carbonate (338 mg). The mixture was allowed to stir for 2h, ethyl acetate was added, and water was drained. The crude intermediate was dissolved in dichloromethane (30 mL), triethyl amine (6.1 g, 60 mmol) and trifluoroacetic anhydride (6.3 g, 30 mmol) were added at -20 °C. The mixture was allowed to stir at - 20 °C for 10 min and at r.t for 1h. Dichloromethane was removed, ethyl acetate was added and the organic extract was washed with water to afford the title compound.

### Example 896B

### Trans-1-benzyl-3-(dimethoxymethyl)-4-nitropyrrolidine

The title compound was prepared according to the procedure outlined in Example 2A substituting Example 896A for 4-methoxy-beta-nitrostyrene.

### Example 896C

### (3R,4R)-1-benzyl-3-(dimethoxymethyl)-4-nitropyrrolidine

A solution of D-tartaric acid (26.8 g, 178 mmol) in 250 mL ethanol was added in a thin stream to a stirring solution of Example 896B (50 g, 178 mmol) in 250 mL ethanol. Solids began to crash out before the addition was complete. The suspension was stirred overnight, filtered, and washed with ethanol. The crude material was recrystallized from ethanol to afford the title compound.

### Example 896D

### (3R,4R)-1-benzyl-4-(dimethoxymethyl)pyrrolidin-3-amine

The title compound was prepared according to the procedure outlined in Example 2B substituting Example 896C for Example 2A.

### Example 896E

### tert-butyl (3R,4R)-1-benzyl-4-(dimethoxymethyl)pyrrolidin-3-ylcarbamate

The title compound was prepared according to the procedure outlined in Example 337C substituting Example 896D for Example 337B.

### Example 896F

### tert-butyl (3R,4R)-4-(dimethoxymethyl)pyrrolidin-3-ylcarbamate

The title compound was prepared according to the procedure outlined in Example 2D substituting Example 896E for 2C.

### Example 896G

### tert-butyl (3R,4R)-4-(dimethoxymethyl)-1-(1-methyl-1H-1,2,3-triazol-4-ylsulfonyl)pyrrolidin-3-ylcarbamate

The title compound was prepared according to the procedure outlined in Example 373C substituting Example 896F for Example 373B and substituting 1-methyl-1H-1,2,3-triazole-4-sulfonyl chloride for 1-methyl-1H-imidazole-4-sulfonyl chloride.

### Example 896H

### tert-butyl (3R,4R)-4-(1,3-dioxan-2-yl)-1-(1-methyl-1H-1,2,3-triazol-4-ylsulfonyl)pyrrolidin-3-ylcarbamate

To Example 896G (2 g, 4.9 mmol) in toluene (10 mL) was added propane-1,3-diol (1.5 g, 20 mmol) and toluenesulfonic acid (20 mg, 0.1 mmol). The mixture was heated at 80 °C for 3 h. Volatiles were removed to afford the title compound.

### Example 896I

### (3R,4R)-4-(1,3-dioxan-2-yl)-1-(1-methyl-1H-1,2,3-triazol-4-ylsulfonyl)pyrrolidin-3-amine

The title compound was prepared as the hydrochloride salt according to the procedure outlined in Example 373B substituting Example 896H for Example 373A.

### Example 896J

### (3R,4S)-4-(1,3-dioxan-2-yl)-1-[(1-methyl-1H-1,2,3-triazol-4-yl)sulfonyl]-N-[3-(trifluoromethoxy)phenyl]pyrrolidin-3-amine

The title compound was prepared according to the procedure outlined in Example 337G substituting Example 896I for Example 337F and 1-bromo-3-(trifluoromethoxy)benzene for 1-bromo-3-(trifluoromethyl)benzene. MS (ESI) *m*/*z* 478 (M+H)⁺.

### Example 897

### (3R,4S)-4-(1,3-dioxan-2-yl)-1-[(1-methyl-1H-imidazol-4-yl)sulfonyl]-N-[3-(trifluoromethoxy)phenyl]pyrrolidin-3-amine

### Example 897A

### tert-butyl (3R,4R)-4-(dimethoxymethyl)-1-(1-methyl-1H-imidazol-4-ylsulfonyl)pyrrolidin-3-ylcarbamate

The title compound was prepared according to the procedure outlined in Example 373C substituting Example 896F for Example 373B.

### Example 897B

### tert-butyl (3R,4R)-4-(1,3-dioxan-2-yl)-1-(1-methyl-1H-imidazol-4-ylsulfonyl)pyrrolidin-3-ylcarbamate

The title compound was prepared according to the procedure outlined in Example 896H substituting Example 897A for Example 896G.

### Example 897C

### (3R,4R)-4-(1,3-dioxan-2-yl)-1-(1-methyl-1H-imidazol-4-ylsulfonyl)pyrrolidin-3-amine

The title compound was prepared as the hydrochloride salt according to the procedure outlined in Example 373B substituting Example 897B for Example 373A.

### Example 897D

### (3R,4S)-4-(1,3-dioxan-2-yl)-1-[(1-methyl-1H-imidazol-4-yl)sulfonyl]-N-[3-(trifluoromethoxy)phenyl]pyrrolidin-3-amine

The title compound was prepared according to the procedure outlined in Example 337G substituting Example 897C for Example 337F and 1-bromo-3-(trifluoromethoxy)benzene for 1-bromo-3-(trifluoromethyl)benzene. MS (ESI) *m*/*z* 477 (M+H)⁺.

### Example 898

### 1-[(1-methyl-1H-imidazol-4-yl)sulfonyl]-4-(1-methylpiperidin-2-yl)-N-[3-(trifluoromethoxy)phenyl]pyrrolidin-3-amine

The title compound was prepared similarly to the procedure described in Example 327 substituting Example 893C for Example 322B and substituting 1-bromo-3-(trifluoromethoxy)benzene for 1-bromo-3-(trifluoromethyl)benzene. MS (ESI) *m*/*z* 488 (M+H)⁺.

### Example 899

### (3R,4S)-4-(1,3-dioxan-2-yl)-1-[(1-methyl-1H-imidazol-4-yl)sulfonyl]-N-[3-(2,2,2-trifluoroethoxy)phenyl]pyrrolidin-3-amine

The title compound was prepared according to the procedure outlined in Example 337G substituting Example 897C for Example 337F and 1-bromo-3-(trifluoroethoxy)benzene for 1-bromo-3-(trifluoromethyl)benzene. MS (ESI) *m*/*z* 491 (M+H)⁺.

### Example 900

### (3R,4S)-4-(5,5-dimethyl-1,3-dioxan-2-yl)-1-[(1-methyl-1H-imidazol-4-yl)sulfonyl]-N-[3-(trifluoromethoxy)phenyl]pyrrolidin-3-amine

### Example 900A

### (3R,4R)-4-(dimethoxymethyl)-1-(1-methyl-1H-imidazol-4-ylsulfonyl)pyrrolidin-3-amine

The title compound was prepared as the hydrochloride salt according to the procedure outlined in Example 373B substituting Example 897A for Example 373A.

### Example 900B

### (3R,4R)-4-(dimethoxymethyl)-1-(1-methyl-1H-imidazol-4-ylsulfonyl)-N-(3-(trifluoromethoxy)phenyl)pyrrolidin-3-amine

The title compound was prepared according to the procedure outlined in Example 337G substituting Example 900A for Example 337F and substituting 1-bromo-3-(trifluoromethoxy)benzene for 1-bromo-3 -(trifluoromethyl)benzene.

### Example 900C

### (3R,4S)-4-(5,5-dimethyl-1,3-dioxan-2-yl)-1-[(1-methyl-1H-imidazol-4-yl)sulfonyl]-N-[3-(trifluoromethoxy)phenyl]pyrrolidin-3-amine

The title compound was prepared according to the procedure outlined in Example 896H substituting Example 900B for Example 896G and substituting 2,2-dimethylpropane-1,3-diol for propane-1,3-diol. MS (ESI) *m*/*z* 505 (M+H)⁺.

### Example 901

### (3R,4S)-4-(1,3-dioxepan-2-yl)-1-[(1-methyl-1H-imidazol-4-yl)sulfonyl]-N-[3-(trifluoromethoxy)phenyl]pyrrolidin-3-amine

The title compound was prepared according to the procedure outlined in Example 896H substituting Example 900B for Example 896G and substituting butane-1,4-diol for propane-1,3-diol. MS (ESI) *m*/*z* 491(M+H)⁺.

### Example 902

### (3R,4S)-4-(1,3-dioxolan-2-yl)-1-[(1-methyl-1H-imidazol-4-yl)sulfonyl]-N-[3-(trifluoromethoxy)phenyl]pyrrolidin-3-amine

The title compound was prepared according to the procedure outlined in Example 896H substituting Example 900B for Example 896G and substituting ethane-1,2-diol for propane-1,3-diol. MS (ESI) *m*/*z* 491(M+H)⁺. MS (ESI) *m*/*z* 463 (M+H)⁺.

### Example 903

### (3R,4S)-N-(2,3-dihydro-1H-inden-1-yl)-4-(1,3-dioxan-2-yl)-1-[(1-methyl-1H-imidazol-4-yl)sulfonyl]pyrrolidin-3-amine

The title compound was prepared according to the procedure outlined in Example 354E substituting Example 897C for Example 354D and 2,3-dihydro-1H-inden-1-one for 4-chlorobenzaldehyde. MS (ESI) *m*/*z* 433 (M+H)⁺.

### Example 904

### (3R,4S)-N-[2-chloro-4-(trifluoromethyl)benzyl]-4-(1,3-dioxan-2-yl)-1-[(1-methyl-1H-imidazol-4-yl)sulfonyl]pyrrolidin-3 -amine

The title compound was prepared according to the procedure outlined in Example 354E substituting Example 897C for Example 354D and 2-chloro-4-(trifluoromethyl)benzaldehyde for 4-chlorobenzaldehyde. MS (ESI) *m*/*z* 509/511 (3:1) (M+H)⁺.

### Example 905

### (3R,4S)-4-(1,3-dioxan-2-yl)-1-[(1-methyl-1H-imidazol-4-yl)sulfonyl]-N-[3-(trifluoromethoxy)benzyl]pyrrolidin-3-amine

The title compound was prepared according to the procedure outlined in Example 354E substituting Example 897C for Example 354D and 3-(trifluoromethoxy)benzaldehyde for 4-chlorobenzaldehyde. MS (ESI) *m*/*z* 491 (M+H)⁺.

### Example 906

### (3R,4S)-4-(1,3-dioxan-2-yl)-N-(4-fluorobenzyl)-1-[(1-methyl-1H-imidazol-4-yl)sulfonyl]pyrrolidin-3-amine

The title compound was prepared according to the procedure outlined in Example 354E substituting Example 897C for Example 354D and 4-trifluorobenzaldehyde for 4-chlorobenzaldehyde. MS (ESI) *m*/*z* 425 (M+H)⁺.

### Example 907

### (3R,4S)-4-(1,3-dioxan-2-yl)-N-methyl-1-[(1-methyl-1H-imidazol-4-yl)sulfonyl]-N-pentylpyrrolidin-3-amine

### Example 907A

### (3R,4R)-4-(1,3-dioxan-2-yl)-1-(1-methyl-1H-imidazol-4-ylsulfonyl)-N-pentylpyrrolidin-3-amine

The title compound was prepared according to the procedure outlined in Example 354E substituting Example 897C for Example 354D and pentanal for 4-chlorobenzaldehyde.

### Example 907B

### (3R,4S)-4-(1,3-dioxan-2-yl)-N-methyl-1-[(1-methyl-1H-imidazol-4-yl)sulfonyl]-N-pentylpyrrolidin-3-amine

The title compound was prepared according to the procedure outlined in Example 354E substituting Example 907A for Example 354D and formaldehyde for 4-chlorobenzaldehyde. MS (ESI) *m*/*z* 401 (M+H)⁺.

### Example 908

### (3R,4S)-4-(1,3-dioxan-2-yl)-1-[(1-methyl-1H-imidazol-4-yl)sulfonyl]-N,N-dipentylpyrrolidin-3-amine

The title compound was prepared according to the procedure outlined in Example 354E substituting Example 897C for Example 354D and pentanal for 4-chlorobenzaldehyde. MS (ESI) *m*/*z* 457 (M+H)⁺.

### Example 909

### (3R,4S)-4-(1,3-dioxan-2-yl)-N,N-dihexyl-1-[(1-methyl-1H-imidazol-4-yl)sulfonyl]pyrrolidin-3-amine

The title compound was prepared according to the procedure outlined in Example 354E substituting Example 897C for Example 354D and hexanal for 4-chlorobenzaldehyde. MS (ESI) *m*/*z* 599 (M+H)⁺.

### Example 910

### (3R,4S)-4-(1,3-dioxan-2-yl)-N-hexyl-1-[(1-methyl-1H-imidazol-4-yl)sulfonyl]pyrrolidin-3-amine

The title compound was prepared according to the procedure outlined in Example 354E substituting Example 897C for Example 354D and hexanal for 4-chlorobenzaldehyde. MS (ESI) *m*/*z* 401 (M+H)⁺.

### Example 911

### 5-chloro-N-{(3R,4S)-4-(1,3-dioxan-2-yl)-1-[(1-methyl-1H-imidazol-4-yl)sulfonyl]pyrrolidin-3-yl}-4-(trifluoromethyl)pyridin-2-amine

The title compound was prepared similarly to the procedure described in Example 605A substituting Example 897C for Example 396A and substituting 2,5-dichloro-4-(trifluoromethyl)pyridine for 4-chloro-2-(methylthio)-6-(trifluoromethyl)pyrimidine. MS (ESI) *m*/*z* 496/498 (3:1) (M+H)⁺.

### Example 912

### (3R,4S)-4-(1,3-dioxan-2-yl)-N-[4-fluoro-3-(trifluoromethyl)phenyl]-1-[(1-methyl-1H-imidazol-4-yl)sulfonyl]pyrrolidin-3-amine

The title compound was prepared according to the procedure outlined in Example 337G substituting Example 897C for Example 337F and 4-bromo-1-fluoro-2-(trifluoromethyl)benzene for 1-bromo-3-(trifluoromethyl)benzene. MS (ESI) *m*/*z* 479.0 (M+H)⁺.

### Example 913

### 1-[(1-methyl-1H-imidazol-4-yl)sulfonyl]-4-(piperidin-1-yl)-N-[3-(trifluoromethoxy)phenyl]pyrrolidin-3-amine

### Example 913A

### trans-tert-butyl 3-hydroxy-4-(3-(trifluoromethoxy)phenylamino)pyrrolidine-1-carboxylate

The title compound was prepared according to the procedure outlined in Example 373A substituting 3-trifluromethoxyaniline for 3-chloroaniline.

### Example 913B

### tert-butyl 6-(3-(trifluoromethoxy)phenyl)-3,6-diazabicyclo[3.1.0]hexane-3-carboxylate

To a solution of Example 913A (2.9 g, 8 mmol) and 4-methylbenezenesulfonyl chloride (1.5 g, 8 mmol) in toluene (10 mL) was added tetrabutylammonium hydogensulfate (0.54 g, 1.6 mmol) followed by 50% NaOH(aq) (1 mL). The mixture was stirred over night. Ethyl acetate was added, and the organic phase washed with water and then brine. The crude material was purified by silica gel column chromatography eluting with hexanes and ethyl acetate (2:1) to afford the title compound.

### Example 913C

### trans-tert-butyl 3-(piperidin-1-yl)-4-(3-(trifluoromethoxy)phenylamino)pyrrolidine-1-carboxylate

A mixture of Example 913B (2 g, 5.8 mmol) and LiBr (20 mg, 0.22 mmol) in piperidine (5 mL) was heated at 120 °C for 3 days, then concentrated and purified by silica gel column chromatography eluting with hexanes and ethyl acetate (1:1) to afford the title compound.

### Example 913D

### trans-4-(piperidin-1-yl)-N-(3-(trifluoromethoxy)phenyl)pyrrolidin-3-amine

The title compound was prepared as the hydrochloride salt according to the procedure outlined in Example 373B substituting Example 913C for Example 373A.

### Example 913E

### trans-1-[(1-methyl-1H-imidazol-4-yl)sulfonyl]-4-(piperidin-1-yl)-N-[3-(trifluoromethoxy)phenyl]pyrrolidin-3-amine

The title compound was prepared according to the procedure outlined in Example 373C substituting Example 913D for Example 373B. MS (ESI) *m*/*z* 474 (M+H)⁺.

### Example 914

### (3R,4S)-N-(3-chlorophenyl)-4-(1,3-dioxan-2-yl)-1-[(1-methyl-1H-imidazol-4-yl)sulfonyl]pyrrolidin-3 -amine

The title compound was prepared according to the procedure outlined in Example 337G substituting Example 897C for Example 337F and 1-bromo-3-chlorobenzene for 1-bromo-3-(trifluoromethyl)benzene. MS (ESI) *m*/*z* 427.0 (M+H)⁺.

### Example 915

### (3R,4S)-4-(1,3-dioxan-2-yl)-1-[(1-methyl-1H-imidazol-4-yl)sulfonyl]-N-[3-(trifluoromethyl)phenyl]pyrrolidin-3-amine

The title compound was prepared according to the procedure outlined in Example 337G substituting Example 897C for Example 337F. MS (ESI) *m*/*z* 461.0 (M+H)⁺.

### Example 916

### (3R,4S)-N-(3-chloro-4-fluorophenyl)-4-(1,3-dioxan-2-yl)-1-[(1-methyl-1H-imidazol-4-yl)sulfonyl]pyrrolidin-3-amine

The title compound was prepared according to the procedure outlined in Example 337G substituting Example 897C for Example 337F and 4-bromo-2-chloro-1-fluorobenzene for 1-bromo-3-(trifluoromethyl)benzene. MS (ESI) *m*/*z* 445.0 (M+H)⁺.

### Example 917

### (3R,4S)-4-(1,3-dioxan-2-yl)-1-[(1-methyl-1H-imidazol-4-yl)sulfonyl]-N-(3-methylphenyl)pyrrolidin-3-amine

The title compound was prepared according to the procedure outlined in Example 337G substituting Example 897C for Example 337F and 1-bromo-3-methylbenzene for 1-bromo-3-(trifluoromethyl)benzene. MS (ESI) *m*/*z* 407.0 (M+H)⁺.

### Example 918

### 4-({3-(1,3-dioxan-2-yl)-4-[3-(trifluoromethyl)benzyl]pyrrolidin-1-yl}sulfonyl)-1-methyl-1H-imidazole

### Example 918A

### 4,4-dimethoxy-3-(nitromethyl)-2-(3-(trifluoromethyl)benzyl)butanal

The title compound was prepared according to the procedure outlined in Example 182A substituting Example 896A for (E)-1-fluoro-4-(2-nitrovinyl)benzene and substituting 3-(3-(trifluoromethyl)phenyl)propanal for 3-(3-chlorophenyl)propanal.

### Example 918B

### 3-(dimethoxymethyl)-4-(3-(trifluoromethyl)benzyl)pyrrolidine

The title compound was prepared according to the procedure outlined in Example 182B substituting Example 918A for Example 182A.

### Example 918C

### 4-(3-(dimethoxymethyl)-4-(3-(trifluoromethyl)benzyl)pyrrolidin-1-ylsulfonyl)-1-methyl-1H-imidazole

The title compound was prepared according to the procedure outlined in Example 373C substituting Example 918B for Example 373B.

### Example 918D

### 4-({trans-3-(1,3-dioxan-2-yl)-4-[3-(trifluoromethyl)benzyl]pyrrolidin-1-yl}sulfonyl)-1-methyl-1H-imidazole

The title compound was prepared according to the procedure outlined in Example 896H substituting Example 918C for Example 896G. MS (ESI) *m*/*z* 460 (M+H)⁺.

### Example 919

### 4-({3-(1,3-dioxan-2-yl)-4-[3-(trifluoromehyl)benzyl]pyrrolidin-1-yl}sulfonyl)-1-methyl-1H-12,3-triazole

### Example 919A

### 4-(3-(dimethoxymethyl)-4-(3-(trifluoromethyl)benzyl)pyrrolidin-1-ylsulfonyl)-1-methyl-1H-1,2,3-triazole

The title compound was prepared according to the procedure outlined in Example 373C substituting Example 918B for Example 373B and 1-methyl-1H-1,2,3-triazole-4-sulfonyl chloride for 1-methyl-1H-imidazole-4-sulfonyl chloride.

### Example 919B

### 4-({-3-(1,3-dioxan-2-yl)-4-[3-(trifluoromethyl)benzyl]pyrrolidin-1-yl}sulfonyl)-1-methyl-1H-1,2,3-triazole

The title compound was prepared according to the procedure outlined in Example 896H substituting Example 919A for Example 896G. MS (ESI) *m*/*z* 461 (M+H)⁺.

### Example 920

### (3R,4S)-4-(5,7-dioxaspiro[2.5]oct-6-yl)-1-[(1-methyl-1H-imidazol-4-yl)sulfonyl]-N-[3-(trifluoromethoxy)phenyl]pyrrolidin-3-amine

The title compound was prepared according to the procedure outlined in Example 896H substituting Example 900B for Example 896G and substituting cyclopropane-1,1-diyldimethanol for propane-1,3-diol. MS (ESI) *m*/*z* 503 (M+H)⁺.

### Example 921

### (3R,4S)-4-[(4R,6R)-4,6-dimethyl-1,3-dioxan-2-yl]-1-[(1-methyl-1H-imidazol-4-yl)sulfonyl]-N-[3-(trifluoromethoxy)phenyl]pyrrolidin-3-amine

The title compound was prepared according to the procedure outlined in Example 896H substituting Example 900B for Example 896G and substituting (2R,4R)-pentane-2,4-diol for propane-1,3-diol. MS (ESI) *m*/*z* 505 (M+H)⁺.

### Example 922

### (3R,4S)-4-[(4S,6S)-4,6-dimethyl-1,3-dioxan-2-yl]-1-[(1-methyl-1H-imidazol-4-yl)sulfonyl]-N-[3-(trifluoromethoxy)phenyl]pyrrolidin-3-amine

The title compound was prepared according to the procedure outlined in Example 896H substituting Example 900B for Example 896G and substituting (2S,4S)-pentane-2,4-diol for propane-1,3-diol. MS (ESI) *m*/*z* 505 (M+H)⁺.

### Example 923

### (3R,4S)-4-(1,3-dioxan-2-yl)-N-(4-fluoro-3-methylphenyl)-1-[(1-methyl-1H-imidazol-4-yl)sulfonyl]pyrrolidin-3-amine

The title compound was prepared according to the procedure outlined in Example 337G substituting Example 897C for Example 337F and 4-bromo-1-fluoro-2-methylbenzene for 1-bromo-3-(trifluoromethyl)benzene. MS (ESI) *m*/*z* 425.0 (M+H)⁺.

### Example 924

### (3R,4S)-4-(1,3-dioxan-2-yl)-N-[4-fluoro-3-(trifluoromethoxy)phenyl]-1-[(1-methyl-1H-imidazol-4-yl)sulfonyl]pyrrolidin-3-amine

The title compound was prepared according to the procedure outlined in Example 337G substituting Example 897C for Example 337F and 4-bromo-1-fluoro-2-(trifluoromethoxy)benzene for 1-bromo-3-(trifluoromethyl)benzene. MS (ESI) *m*/*z* 495.0 (M+H)⁺.

### Example 925

### (3R,4S)-N-[3-(difluoromethoxy)phenyl]-4-(1,3-dioxan-2-yl)-1-[(1-methyl-1H-imidazol-4-yl)sulfonyl]pyrrolidin-3-amine

The title compound was prepared according to the procedure outlined in Example 337G substituting Example 897C for Example 337F and 1-bromo-3-(difluoromethoxy)benzene for 1-bromo-3-(trifluoromethyl)benzene. MS (ESI) *m*/*z* 459.0 (M+H)⁺.

### Example 926

### (3R,4S)-4-(1,3-dioxan-2-yl)-1-[(1-methyl-1H-imidazol-4-yl)sulfonyl]-N-[3-(propan-2-yl)phenyl]pyrrolidin-3-amine

The title compound was prepared according to the procedure outlined in Example 337G substituting Example 897C for Example 337F and 1-bromo-3-isopropylbenzene for 1-bromo-3-(trifluoromethyl)benzene. MS (ESI) *m*/*z* 435.1 (M+H)⁺.

### Example 927

### 4-(5-fluoropyridin-2-yl)-N-[4-fluoro-3-(trifluoromethyl)phenyl]-1-[(1-methyl-1H-imidazol-4-yl)sulfonyl]pyrrolidin-3-amine

The title compound was prepared similarly to the procedure described in Example 327 substituting Example 895A for Example 322B and substituting 4-bromo-1-fluoro-2-(trifluoromethyl)benzene for 1-bromo-3-(trifluoromethyl)benzene. MS (ESI) *m*/*z* 488 (M+H)⁺.

### Example 928

### (3R,4S)-N-[3-(2,2-difluoroethoxy)phenyl]-4-(1,3-dioxan-2-yl)-1-[(1-methyl-1H-imidazol-4-yl)sulfonyl]pyrrolidin-3 -amine

The title compound was prepared according to the procedure outlined in Example 337G substituting Example 897C for Example 337F and 1-bromo-3-(2,2-difluoroethoxy)benzene for 1-bromo-3-(trifluoromethyl)benzene. MS (ESI) *m*/*z* 473.0 (M+H)⁺.

Further examplary compounds include:
1-(Trans-1-((1-methyl-1H-imidazol-4-yl)sulfonyl)-4-((6-(trifluoromethyl)pyrimidin-4-yl)oxy)pyrrolidin-3-yl)azepane;
4-(Trans-1-((1-methyl-1H-imidazol-4-yl)sulfonyl)-4-((6-(trifluoromethyl)pyrimidin-4-yl)oxy)pyrrolidin-3-yl)morpholine;
4-((Trans-1-((1-methyl-1H-imidazol-4-yl)sulfonyl)-4-(2-methylpiperidin-1-yl)pyrrolidin-3-yl)oxy)-6-(trifluoromethyl)pyrimidine;
4-((Trans-1-((1-methyl-1H-imidazol-4-yl)sulfonyl)-4-(3-methylpiperidin-1-yl)pyrrolidin-3-yl)oxy)-6-(trifluoromethyl)pyrimidine;
4-((Trans-1-((1-methyl-1H-imidazol-4-yl)sulfonyl)-4-(4-methylpiperidin-1-yl)pyrrolidin-3-yl)oxy)-6-(trifluoromethyl)pyrimidine;
4-((Trans-4-(4-fluoropiperidin-1-yl)-1-((1-methyl-1H-imidazol-4-yl)sulfonyl)pyrrolidin-3-yl)oxy)-6-(trifluoromethyl)pyrimidine;
4-((Trans-4-(4,4-difluoropiperidin-1-yl)-1-((1-methyl-1H-imidazol-4-yl)sulfonyl)pyrrolidin-3-yl)oxy)-6-(trifluoromethyl)pyrimidine;
4-((Trans-4-(3-fluoroazetidin-1-yl)-1-((1-methyl-1H-imidazol-4-yl)sulfonyl)pyrrolidin-3-yl)oxy)-6-(trifluoromethyl)pyrimidine;
4-((Trans-1-((1-methyl-1H-imidazol-4-yl)sulfonyl)-4-(piperidin-1-yl)pyrrolidin-3-yl)oxy)-6-(trifluoromethyl)pyrimidine;
Trans-1'-((1-methyl-1H-imidazol-4-yl)sulfonyl)-4'-((6-(trifluoromethyl)pyrimidin-4-yl)oxy)-1,3'-bipyrrolidine;
Trans-3-((3-chlorophenyl)amino)-4-(4-fluorophenyl)-1-((1-methyl-1H-imidazol-4-yl)sulfonyl)pyrrolidin-2-one;
Trans-3-((3-chloro-4-fluorophenyl)amino)-4-(4-fluorophenyl)-1-((1-methyl-1H-imidazol-4-yl)sulfonyl)pyrrolidin-2-one;
Trans-4-(4-fluorophenyl)-1-((1-methyl-1H-imidazol-4-yl)sulfonyl)-3-((3-(trifluoromethyl)phenyl)amino)pyrrolidin-2-one;
Trans-3-((4-fluoro-3-(trifluoromethoxy)phenyl)amino)-4-(4-fluorophenyl)-1-((1-methyl-1H-imidazol-4-yl)sulfonyl)pyrrolidin-2-one;
3-(Trans-4-(4-fluorophenyl)-1-((1-methyl-1H-imidazol-4-yl)sulfonyl)pyrrolidin-3-yl)-5-(4-(trifluoromethyl)phenyl)-4,5-dihydroisoxazole;
2-(Trans-4-(4-fluorophenyl)-1-((1-methyl-1H-imidazol-4-yl)sulfonyl)pyrrolidin-3-yl)-5-(4-(trifluoromethyl)phenyl)oxazole;
5-(3-Chlorophenyl)-2-(trans-4-(4-fluorophenyl)-1-((1-methyl-1H-imidazol-4-yl)sulfonyl)pyrrolidin-3-yl)-1H-imidazole;
4-((Trans-3-(4-fluoro-3-(trifluoromethyl)phenoxy)-4-(4-fluorophenyl)pyrrolidin-1-yl)sulfonyl)-1-methyl-1H-imidazole;
Trans-4-(4-fluorophenyl)-1-((1-methyl-1H-imidazol-4-yl)sulfonyl)-N-(3-(trifluoromethyl)cyclohexyl)pyrrolidin-3-amine;
N-(Trans-4-(4-fluorophenyl)-1-((1-methyl-1H-imidazol-4-yl)sulfonyl)pyrrolidin-3-yl)-1-isopropylpiperidin-4-amine;
Trans-4-(4-fluorophenyl)-1-((1-methyl-1H-imidazol-4-yl)sulfonyl)-N-(2-methyltetrahydrofuran-3-yl)pyrrolidin-3-amine;
1-Ethyl-N-(Trans-4-(4-fluorophenyl)-1-((1-methyl-1H-imidazol-4-yl)sulfonyl)pyrrolidin-3-yl)piperidin-3-amine.

### Biological testing

### 1. [³H]-Glycine uptake into recombinant CHO cells expressing human GlyT1:

Human GlyT1c expressing recombinant hGlyT1c_5_CHO cells were plated at 20,000 cells per well in 96 well Cytostar-T scintillation microplates (Amersham Biosciences) and cultured to sub-confluency for 24 h. For glycine uptake assays the culture medium was aspirated and the cells were washed once with 100 µl HBSS (Gibco BRL, #14025-050) with 5 mM L-Alanine (Merck #1007). 80 µl HBSS buffer were added, followed by 10 µl inhibitor or vehicle (10% DMSO) and 10 µl [³H]-glycine (TRK71, Amersham Biosciences) to a final concentration of 200 nM for initiation of glycine uptake. The plates were placed in a Wallac Microbeta (PerkinElmer) and continuously counted by solid phase scintillation spectrometry during up to 3 hours. Nonspecific uptake was determined in the presence of 10 µM Org24598. IC₅₀ calculations were made by four-parametric logistic nonlinear regression analysis (GraphPad Prism) using determinations within the range of linear increase of [3H]-glycine incorporation between 60 and 120 min.

### 2. Radioligand binding assays using recombinant CHO cell membranes expressing human GlyT1:

Radioligand binding to human GlyT1c transporter-expressing membranes was determined as described in Mezler et al., Molecular Pharmacology 74:1705-1715, 2008.

The following results were obtained with the compounds disclosed in the examples:

| Ex. | Ki [µM] | Ex. | Ki [µM] | Ex. | Ki [µM] | Ex. | Ki [µM] |
|---|---|---|---|---|---|---|---|
| 1 | - | 49 | ≤ 10 | 88 | ≤ 0.01 | 155 | ≤ 0.1 |
| 2 | ≤ 1 | 50 | ≤ 10 | 89 | ≤ 0.1 | 156 | ≤ 0.1 |
| 3 | ≤ 0.1 | 51 | ≤ 0,1 | 90 | ≤ 1 | 157 | ≤ 10 |
| 4 | ≥ 10 | 52 | ≤ 10 | 91 | ≤ 0.1 | 158 | ≤ 0.1 |
| 13 | ≤ 1 | 53 | ≤ 10 | 92 | ≤ 0.1 | 159 | ≤ 0.01 |
| 14 | ≤ 1 | 54 | ≤ 1 | 93 | ≤ 1 | 160 | ≤ 0.01 |
| 15 | ≤ 10 | 55 | ≤ 1 | 94 | ≤ 0.01 | 161 | ≤ 1 |
| 16 | ≤ 1 | 56 | ≤ 1 | 95 | ≤ 1 | 162 | ≤ 1 |
| 17 | ≤ 10 | 57 | ≤ 10 | 100 | ≤ 10 | 163 | ≤ 1 |
| 18 | ≤ 10 | 58 | ≤ 0,1 | 101 | ≤ 1 | 164 | ≤ 10 |
| 19 | ≤ 10 | 59 | ≤ 10 | 102 | ≤ 1 | 165 | ≤ 1 |
| 20 | ≤ 10 | 60 | ≤ 0,1 | 115 | ≤ 0.1 | 166 | ≤ 1 |
| 21 | ≤ 10 | 61 | ≤ 1 | 122 | ≤ 10 | 167 | ≤ 0.1 |
| 22 | ≤ 10 | 62 | ≤ 0.1 | 123 | ≤ 10 | 168 | ≤ 0.1 |
| 23 | ≤ 10 | 63 | ≤ 0.1 | 124 | ≤ 10 | 169 | ≤ 10 |
| 24 | ≤ 10 | 64 | ≤ 0.1 | 125 | > 10 | 170 | ≤ 0.1 |
| 25 | ≤ 10 | 65 | ≤ 1 | 126 | ≤ 10 | 171 | ≤ 0.1 |
| 26 | ≤ 1 | 66 | ≤ 10 | 127 | ≤ 10 | 172 | ≤ 0.1 |
| 27 | ≤ 1 | 67 | ≤ 0.01 | 128 | ≤ 10 | 173 | ≤ 1 |
| 29 | ≤ 1 | 68 | ≤ 1 | 129 | > 10 | 174 | ≤ 1 |
| 30 | ≤ 10 | 69 | ≤ 10 | 130 | > 10 | 175 | ≤ 1 |
| 31 | ≤ 1 | 70 | ≤ 1 | 131 | > 10 | 176 | ≤ 0.01 |
| 32 | ≤ 10 | 71 | ≤ 1 | 132 | ≤ 10 | 177 | ≤ 10 |
| 33 | ≤ 10 | 72 | - | 133 | ≤ 10 | 178 | ≤ 1 |
| 34 | ≤ 10 | 73 | ≤ 10 | 134 | ≤ 10 | 179 | ≤ 10 |
| 35 | ≤ 10 | 74 | ≤ 0.1 | 135 | > 10 | 180 | ≤ 1 |
| 36 | ≤ 1 | 75 | ≤ 1 | 136 | ≤ 10 | 181 | ≤ 10 |
| 37 | ≤ 1 | 76 | ≤ 10 | 137 | ≤ 10 | 182 | ≤ 0.1 |
| 38 | ≤ 10 | 77 | ≤ 1 | 142 | ≤ 10 | 183 | ≤ 10 |
| 39 | ≤ 10 | 78 | ≤ 1 | 143 | ≤ 10 | 184 | ≤ 10 |
| 40 | ≥ 10 | 79 | ≤ 1 | 144 | > 10 | 185 | ≤ 10 |
| 41 | ≤ 10 | 80 | ≤ 1 | 145 | ≤ 0.1 | 186 | ≤ 1 |
| 42 | ≤ 10 | 81 | ≤ 10 | 148 | ≤ 10 | 187 | ≤ 10 |
| 43 | ≤ 10 | 82 | ≤ 10 | 149 | ≤ 0.1 | 188 | ≤ 0.1 |
| 44 | ≤ 10 | 83 | ≤ 1 | 150 | ≤ 0.001 | 189 | ≤ 0.1 |
| 45 | ≥ 10 | 84 | ≤ 1 | 151 | ≤ 0.1 | 190 | ≤ 1 |
| 46 | ≥ 10 | 85 | ≤ 0.01 | 152 | ≤ 1 | 191 | ≤ 10 |
| 47 | ≥ 10 | 86 | ≤ 1 | 153 | ≤ 1 | 192 | ≤ 1 |
| 48 | ≤ 10 | 87 | ≤ 10 | 154 | ≤ 0.1 | 193 | ≤ 10 |

| Ex. | Ki [µM] | Ex. | Ki [µM] | Ex. | Ki [µM] | Ex. | Ki [µM] |
|---|---|---|---|---|---|---|---|
| 194 | ≤ 0.1 | 233 | ≤ 10 | 272 | ≤ 1 | 311 | ≤ 1 |
| 195 | ≤ 10 | 234 | ≤ 1 | 273 | ≤ 1 | 312 | ≤ 0.1 |
| 196 | ≤ 10 | 235 | ≤ 10 | 274 | ≤ 0.1 | 313 | ≤ 1 |
| 197 | ≤ 1 | 236 | ≤ 10 | 275 | ≤ 1 | 314 | ≤ 1 |
| 198 | ≤ 1 | 237 | ≤ 10 | 276 | ≤ 1 | 315 | ≤ 1 |
| 199 | ≤ 1 | 238 | ≤ 10 | 277 | ≤ 10 | 316 | ≤ 1 |
| 200 | ≤ 1 | 239 | ≤ 10 | 278 | ≤ 0.1 | 317 | ≤ 1 |
| 201 | ≤ 0.1 | 240 | ≤ 0.1 | 279 | ≤ 10 | 318 | ≤ 1 |
| 202 | ≤ 1 | 241 | ≤ 10 | 280 | ≤ 0.01 | 319 | ≤ 0.1 |
| 203 | ≤ 10 | 242 | ≤ 10 | 281 | ≤ 0.01 | 320 | ≤ 0.01 |
| 204 | ≤ 0.1 | 243 | ≤ 1 | 282 | ≤ 0.1 | 321 | ≤ 1 |
| 205 | ≤ 1 | 244 | ≤ 1 | 283 | ≤ 0.1 | 322 | ≤ 0.01 |
| 206 | ≤ 10 | 245 | ≤ 0.1 | 284 | ≤ 0.1 | 323 | ≤ 1 |
| 207 | ≤ 10 | 246 | ≤ 0.1 | 285 | ≤ 0.1 | 324 | ≤ 10 |
| 208 | ≤ 10 | 247 | ≤ 0.01 | 286 | ≤ 10 | 325 | ≤ 1 |
| 209 | ≤ 10 | 248 | ≤ 1 | 287 | ≤ 0.01 | 326 | ≤ 1 |
| 210 | ≤ 10 | 249 | ≤ 1 | 288 | ≤ 1 | 327 | ≤ 0.01 |
| 211 | ≤ 1 | 250 | ≤ 10 | 289 | ≤ 1 | 328 | ≤ 0.1 |
| 212 | ≤ 0.10 | 251 | ≤ 0.1 | 290 | ≤ 10 | 329 | ≤ 0.01 |
| 213 | ≤ 1 | 252 | ≤ 1 | 291 | ≤ 0.1 | 329 | ≤ 0.01 |
| 214 | ≤ 0.1 | 253 | ≤ 1 | 292 | ≤ 1 | 330 | ≤ 0.1 |
| 215 | ≤ 0.1 | 254 | ≤ 10 | 293 | ≤ 0.1 | 330 | ≤ 0.1 |
| 216 | ≤ 1 | 255 | ≤ 0.1 | 294 | ≤ 0.01 | 331 | ≤ 1 |
| 217 | ≤ 1 | 256 | ≤ 1 | 295 | ≤ 0.1 | 332 | ≤ 0.1 |
| 218 | ≤ 1 | 257 | ≤ 0.1 | 296 | ≤ 0.1 | 333 | ≤ 1 |
| 219 | ≤ 10 | 258 | ≤ 1 | 297 | ≤ 0.01 | 334 | ≤ 1 |
| 220 | ≤ 0.01 | 259 | ≤ 1 | 298 | ≤ 0.01 | 335 | ≤ 1 |
| 221 | ≤ 1 | 260 | ≤ 0.1 | 299 | ≤ 1 | 336 | ≤ 1 |
| 222 | ≤ 1 | 261 | ≤ 1 | 300 | ≤ 1 | 337 | ≤ 1 |
| 223 | ≤ 0.1 | 262 | ≤ 0.01 | 301 | ≤ 1 | 338 | ≤ 1 |
| 224 | ≤ 1 | 263 | ≤ 0.1 | 302 | ≤ 0.1 | 339 | ≤ 10 |
| 225 | ≤ 10 | 264 | ≤ 1 | 303 | ≤ 10 | 340 | ≤ 1 |
| 226 | ≤ 0.01 | 265 | ≤ 1 | 304 | ≤ 1 | 341 | ≤ 10 |
| 227 | ≤ 10 | 266 | ≤ 1 | 305 | ≤ 0.01 | 342 | ≤ 10 |
| 228 | ≤ 10 | 267 | ≤ 0.01 | 306 | ≤ 0.1 | 343 | ≤ 10 |
| 229 | ≤ 10 | 268 | ≤ 10 | 307 | ≤ 0.1 | 344 | ≤ 0.1 |
| 230 | ≤ 10 | 269 | ≤ 10 | 308 | ≤ 1 | 345 | ≤ 0.1 |
| 231 | ≤ 1 | 270 | ≤ 10 | 309 | ≤ 1 | 346 | ≤ 1 |
| 232 | ≤ 10 | 271 | ≤ 1 | 310 | ≤ 0.1 | 347 | ≤ 10 |

| Ex. | Ki [µM] | Ex. | Ki [µM] | Ex. | Ki [µM] | Ex. | Ki [µM] |
|---|---|---|---|---|---|---|---|
| 348 | ≤ 0.1 | 387 | ≤ 10 | 425 | ≤ 0.01 | 464 | ≤ 1 |
| 349 | ≤ 0.1 | 388 | ≤ 0.1 | 426 | ≤ 1 | 465 | ≤ 1 |
| 350 | > 10 | 389 | ≤ 10 | 427 | ≤ 0.1 | 466 | - |
| 351 | ≤ 1 | 390 | ≤ 0.01 | 428 | ≤ 0.1 | 467 | ≤ 1 |
| 352 | ≤ 0.01 | 390 | ≤ 0.01 | 429 | > 10 | 468 | ≤ 0.1 |
| 353 | ≤ 0.1 | 391 | ≤ 0.1 | 430 | > 10 | 469 | ≤ 1 |
| 354 | ≤ 1 | 392 | ≤ 0.1 | 431 | ≤ 10 | 470 | ≤ 10 |
| 355 | ≤ 1 | 393 | ≤ 0.01 | 432 | ≤ 10 | 471 | ≤ 0.1 |
| 356 | ≤ 0.1 | 394 | ≤ 0.01 | 433 | ≤ 0.1 | 472 | ≤ 1 |
| 357 | ≤ 1 | 395 | ≤ 1 | 434 | ≤ 10 | 473 | ≤ 0.1 |
| 358 | ≤ 0.1 | 396 | ≤ 0.01 | 435 | ≤ 0.1 | 474 | ≤ 10 |
| 359 | ≤ 1 | 397 | ≤ 0.01 | 436 | ≤ 1 | 475 | ≤ 10 |
| 360 | ≤ 0.1 | 398 | ≤ 0.01 | 437 | ≤ 0.1 | 476 | ≤ 10 |
| 361 | ≤ 1 | 399 | ≤ 0.1 | 438 | ≤ 1 | 477 | ≤ 10 |
| 362 | ≤ 0.1 | 400 | ≤ 10 | 439 | ≤ 1 | 478 | ≤ 10 |
| 363 | ≤ 0.1 | 401 | ≤ 10 | 440 | ≤ 0.1 | 479 | > 10 |
| 364 | ≤ 10 | 402 | ≤ 10 | 441 | ≤ 10 | 480 | > 10 |
| 365 | ≤ 10 | 403 | ≤ 0.1 | 442 | ≤ 1 | 481 | ≤ 10 |
| 366 | ≤ 0.1 | 404 | ≤ 10 | 443 | ≤ 1 | 482 | ≤ 10 |
| 367 | ≤ 0.1 | 405 | ≤ 1 | 444 | - | 483 | ≤ 10 |
| 368 | ≤ 0.01 | 406 | ≤ 10 | 445 | ≤ 1 | 484 | ≤ 10 |
| 369 | ≤ 1 | 407 | ≤ 10 | 446 | ≤ 0.1 | 485 | ≤ 10 |
| 370 | ≤ 1 | 408 | ≤ 10 | 447 | ≤ 10 | 486 | ≤ 10 |
| 371 | ≤ 1 | 409 | ≤ 0.1 | 448 | ≤ 0.1 | 487 | ≤ 10 |
| 372 | ≤ 10 | 410 | ≤ 10 | 449 | ≤ 1 | 488 | ≤ 10 |
| 373 | ≤ 10 | 411 | ≤ 10 | 450 | ≤ 1 | 489 | ≤ 10 |
| 374 | ≥ 10 | 412 | ≤ 1 | 451 | - | 490 | ≤ 10 |
| 375 | ≥ 10 | 413 | ≤ 10 | 452 | - | 491 | ≤ 10 |
| 376 | ≤ 1 | 414 | ≤ 10 | 453 | ≤ 1 | 492 | ≥ 10 |
| 377 | ≤ 1 | 415 | ≤ 10 | 454 | ≤ 10 | 493 | ≤ 10 |
| 378 | ≤ 10 | 416 | - | 455 | ≤ 0.1 | 494 | ≤ 10 |
| 379 | ≤ 0.1 | 417 | ≤ 0.01 | 456 | ≤ 1 | 495 | > 10 |
| 380 | ≤ 0.1 | 418 | ≤ 0.1 | 457 | ≤ 10 | 496 | ≥ 10 |
| 381 | ≤ 10 | 419 | ≥ 10 | 458 | ≤ 10 | 497 | ≤ 10 |
| 382 | ≤ 0.1 | 420 | ≤ 10 | 459 | ≤ 10 | 498 | ≤ 10 |
| 383 | ≤ 1 | 421 | ≤ 10 | 460 | ≤ 10 | 499 | ≥ 10 |
| 384 | ≤ 10 | 422 | ≤ 1 | 461 | ≤ 1 | 500 | ≤ 10 |
| 385 | ≤ 10 | 423 | ≤ 10 | 462 | ≤ 0.1 | 501 | ≤ 10 |
| 386 | ≤ 1 | 424 | ≤ 1 | 463 | ≤ 1 | 502 | ≤ 10 |

| Ex. | Ki [µM] | Ex. | Ki [µM] | Ex. | Ki [µM] | Ex. | Ki [µM] |
|---|---|---|---|---|---|---|---|
| 503 | ≥ 10 | 541 | ≤ 10 | 580 | ≤ 1 | 619 | ≤ 1 |
| 504 | ≤ 10 | 542 | ≤ 10 | 581 | ≤ 0.1 | 620 | ≤ 1 |
| 505 | - | 543 | ≤ 10 | 582 | ≤ 10 | 621 | ≤ 10 |
| 506 | ≤ 10 | 544 | ≤ 10 | 583 | ≤ 10 | 622 | ≤ 0.1 |
| 507 | ≤ 10 | 545 | ≤ 1 | 584 | ≤ 10 | 623 | ≤ 0.1 |
| 508 | ≤ 10 | 546 | ≥ 10 | 585 | ≤ 0.1 | 624 | ≤ 1 |
| 509 | ≤ 10 | 547 | ≤ 0.1 | 586 | ≤ 1 | 625 | ≤ 0.01 |
| 510 | ≤ 10 | 548 | ≤ 0.01 | 587 | ≤ 10 | 626 | ≤ 1 |
| 511 | ≤ 10 | 549 | ≤ 10 | 588 | ≤ 10 | 627 | ≤ 1 |
| 512 | ≤ 10 | 550 | ≤ 10 | 589 | ≤ 1 | 628 | ≤ 0.01 |
| 513 | > 10 | 551 | ≤ 10 | 590 | ≤ 10 | 629 | ≤ 1 |
| 514 | ≤ 10 | 552 | ≤ 10 | 591 | ≤ 10 | 630 | ≤ 0.1 |
| 515 | ≤ 10 | 553 | ≤ 1 | 592 | ≤ 10 | 631 | ≤ 0.01 |
| 516 | ≤ 10 | 554 | ≤ 1 | 593 | ≤ 10 | 632 | ≤ 1 |
| 517 | ≤ 10 | 555 | ≤ 10 | 594 | ≤ 10 | 633 | ≤ 1 |
| 518 | > 10 | 556 | ≤ 10 | 595 | ≤ 10 | 634 | ≤ 10 |
| 519 | > 10 | 557 | ≤ 10 | 596 | ≤ 10 | 635 | ≤ 1 |
| 520 | ≤ 10 | 558 | ≤ 10 | 597 | ≤ 10 | 636 | ≤ 1 |
| 521 | ≤ 10 | 559 | ≤ 1 | 598 | ≤ 10 | 637 | ≤ 10 |
| 522 | - | 560 | ≥ 10 | 599 | ≤ 10 | 638 | ≤ 1 |
| 523 | ≤ 10 | 561 | ≤ 1 | 600 | ≤ 10 | 639 | ≤ 10 |
| 524 | ≤ 10 | 562 | ≤ 10 | 601 | ≤ 0.1 | 640 | ≤ 1 |
| 525 | ≤ 10 | 563 | ≤ 10 | 602 | ≤ 1 | 641 | ≤ 0.1 |
| 526 | ≤ 10 | 564 | ≤ 1 | 603 | ≤ 0.1 | 642 | ≤ 0.01 |
| 527 | ≤ 10 | 565 | ≤ 10 | 604 | ≤ 0.1 | 643 | ≤ 10 |
| 528 | > 10 | 566 | ≤ 10 | 605 | ≤ 1 | 644 | ≤ 10 |
| 529 | ≤ 10 | 567 | ≤ 1 | 606 | ≤ 0.1 | 645 | ≤ 0.01 |
| 530 | ≤ 1 | 568 | ≤ 0.1 | 607 | ≤ 10 | 646 | ≤ 0.01 |
| 531 | ≤ 0.1 | 569 | ≤ 1 | 608 | ≤ 10 | 647 | ≤ 10 |
| 532 | ≤ 10 | 570 | ≤ 0.01 | 609 | ≤ 1 | 648 | ≤ 10 |
| 533 | ≤ 10 | 571 | ≤ 10 | 610 | ≤ 10 | 649 | ≤ 0.1 |
| 534 | ≤ 1 | 572 | ≤ 1 | 611 | ≤ 10 | 650 | ≤ 1 |
| 535 | ≤ 10 | 573 | ≤ 0.1 | 612 | ≤ 1 | 651 | ≤ 0.01 |
| 536 | ≤ 1 | 574 | ≤ 0.1 | 613 | ≤ 1 | 651 | ≤ 0.01 |
| 537 | ≤ 10 | 575 | ≤ 10 | 614 | ≤ 1 | 652 | ≤ 0.01 |
| 538 | ≤ 10 | 576 | - | 615 | ≤ 1 | 653 | ≤ 10 |
| 539 | ≤ 10 | 577 | > 10 | 616 | ≤ 1 | 654 | ≤ 1 |
| 540 | ≤ 0.1 | 578 | ≤ 10 | 617 | ≤ 0.01 | 655 | ≤ 1 |
| 540 | ≤ 0.1 | 579 | ≤ 10 | 618 | ≤ 1 | 656 | ≤ 1 |

| Ex. | Ki [µM] | Ex. | Ki [µM] | Ex. | Ki [µM] | Ex. | Ki [µM] |
|---|---|---|---|---|---|---|---|
| 657 | ≤ 1 | 696 | ≤ 10 | 735 | ≤ 10 | 774 | ≤ 0.1 |
| 658 | ≤ 1 | 697 | ≤ 1 | 736 | ≤ 1 | 775 | ≤ 1 |
| 659 | ≤ 10 | 698 | ≤ 1 | 737 | ≤ 0.1 | 776 | ≤ 0.01 |
| 660 | ≤ 1 | 699 | ≤ 1 | 738 | > 10 | 777 | ≥ 10 |
| 661 | ≤ 1 | 700 | ≤ 10 | 739 | ≤ 0.1 | 778 | ≥ 10 |
| 662 | ≤ 10 | 701 | ≤ 1 | 740 | ≤ 10 | 779 | ≤ 10 |
| 663 | ≤ 0.1 | 702 | ≤ 10 | 741 | ≤ 0.1 | 780 | ≤ 10 |
| 664 | ≤ 0.1 | 703 | ≤ 1 | 742 | ≤ 1 | 781 | ≤ 10 |
| 665 | ≤ 0.01 | 704 | ≤ 1 | 743 | ≤ 0.1 | 782 | ≤ 1 |
| 666 | ≤ 10 | 705 | ≤ 1 | 744 | ≤ 0.01 | 783 | ≤ 1 |
| 667 | ≤ 0.1 | 706 | ≤ 10 | 745 | ≤ 0.01 | 784 | ≤ 0.1 |
| 668 | ≤ 10 | 707 | ≤ 1 | 746 | ≤ 0.01 | 785 | ≤ 0.1 |
| 669 | ≤ 0.1 | 708 | ≤ 10 | 747 | ≤ 0.01 | 786 | ≤ 1 |
| 670 | ≤ 0.01 | 709 | ≤ 10 | 748 | ≤ 0.01 | 787 | ≤ 1 |
| 671 | ≤ 1 | 710 | ≤ 1 | 749 | ≤ 0.01 | 788 | ≤ 1 |
| 672 | ≤ 1 | 711 | - | 750 | ≤ 0.1 | 789 | ≤ 1 |
| 673 | ≤ 1 | 712 | ≤ 1 | 751 | ≤ 0.1 | 790 | ≤ 1 |
| 674 | ≤ 1 | 713 | > 10 | 752 | ≤ 0.1 | 791 | ≤ 1 |
| 675 | ≤ 1 | 714 | ≤ 10 | 753 | ≤ 1 | 792 | ≤ 10 |
| 676 | ≤ 1 | 715 | ≤ 1 | 754 | ≤ 10 | 793 | ≤ 10 |
| 677 | ≤ 1 | 716 | ≤ 0.1 | 755 | ≥ 10 | 794 | ≤ 1 |
| 678 | ≤ 1 | 717 | ≤ 0.1 | 756 | ≤ 10 | 795 | ≤ 0.01 |
| 679 | ≤ 1 | 718 | ≤ 0.1 | 757 | ≤ 10 | 796 | ≤ 0.1 |
| 680 | ≤ 0.1 | 719 | ≤ 0.1 | 758 | ≤ 10 | 797 | ≤ 10 |
| 681 | ≤ 0.01 | 720 | ≤ 0.1 | 759 | ≤ 0.01 | 798 | ≤ 0.1 |
| 682 | ≤ 0.1 | 721 | ≤ 0.1 | 760 | > 10 | 799 | ≤ 0.1 |
| 683 | ≤ 0.1 | 722 | ≤ 0.01 | 761 | ≤ 0.1 | 800 | ≤ 0.01 |
| 684 | ≤ 1 | 723 | ≤ 0.1 | 762 | ≤ 0.1 | 801 | ≤ 0.01 |
| 685 | ≤ 0.01 | 724 | ≤ 0.1 | 763 | ≤ 1 | 802 | ≤ 0.1 |
| 686 | ≤ 0.01 | 725 | ≤ 0.01 | 764 | ≤ 1 | 803 | ≤ 0.01 |
| 687 | ≤ 0.1 | 726 | ≤ 0.1 | 765 | ≤ 1 | 804 | - |
| 688 | ≤ 1 | 727 | ≤ 0.01 | 766 | ≤ 1 | 805 | ≤ 1 |
| 689 | ≤ 1 | 728 | ≤ 0.01 | 767 | ≤ 0.1 | 806 | ≤ 0.01 |
| 690 | ≤ 1 | 729 | ≤ 0.01 | 768 | ≤ 0.1 | 807 | ≤ 0.01 |
| 691 | ≤ 0.1 | 730 | ≤ 0.01 | 769 | ≤ 1 | 808 | ≤ 0.01 |
| 692 | ≤ 1 | 731 | ≤ 0.1 | 770 | ≤ 1 | 809 | ≤ 0.01 |
| 693 | ≤ 10 | 732 | ≤ 0.1 | 771 | ≤ 0.1 | 810 | ≤ 0.01 |
| 694 | ≤ 1 | 733 | ≤ 0.01 | 772 | ≤ 10 | 811 | ≤ 0.1 |
| 695 | ≤ 10 | 734 | ≤ 0.1 | 773 | ≤ 0.1 | 812 | - |

| Ex. | Ki [µM] | Ex. | Ki [µM] | Ex. | Ki [µM] | Ex. | Ki [µM] |
|---|---|---|---|---|---|---|---|
| 813 | ≤ 0.1 | 852 | ≤ 1 | 891 | ≤ 0.1 | | |
| 814 | ≤ 1 | 853 | ≤ 1 | 892 | ≤ 0.1 | | |
| 815 | ≤ 0.1 | 854 | - | 893 | ≤ 0.1 | | |
| 816 | ≤ 0.01 | 855 | - | 894 | ≤ 0.01 | | |
| 817 | ≤ 1 | 856 | - | 895 | ≤ 0.01 | | |
| 818 | ≤ 0.01 | 857 | - | 896 | ≤ 0.01 | | |
| 819 | ≤ 0.1 | 858 | - | 897 | ≤ 0.01 | | |
| 820 | ≤ 10 | 859 | - | 898 | ≤ 0.01 | | |
| 821 | ≤ 10 | 860 | - | 899 | ≤ 0.1 | | |
| 822 | ≤ 1 | 861 | - | 900 | ≤ 1 | | |
| 823 | ≤ 1 | 862 | - | 901 | ≤ 0.01 | | |
| 824 | ≤ 1 | 863 | ≤ 1 | 902 | ≤ 1 | | |
| 825 | ≤ 1 | 864 | ≤ 10 | 903 | ≤ 0.01 | | |
| 826 | ≤ 10 | 865 | ≤ 0.01 | 904 | ≤ 0.01 | | |
| 827 | ≤ 1 | 866 | ≤ 0.01 | 905 | ≤ 1 | | |
| 828 | ≤ 10 | 867 | ≤ 0.1 | 906 | ≤ 1 | | |
| 829 | ≤ 10 | 868 | ≤ 10 | 907 | ≤ 1 | | |
| 830 | ≤ 10 | 869 | ≤ 0.01 | 908 | ≤ 10 | | |
| 831 | ≤ 1 | 870 | ≤ 0.1 | 909 | ≤ 1 | | |
| 832 | ≤ 1 | 871 | ≤ 0.1 | 910 | ≤ 0.1 | | |
| 833 | ≤ 10 | 872 | > 10 | 911 | ≤ 0.01 | | |
| 834 | ≤ 1 | 873 | ≤ 10 | 912 | ≤ 0.01 | | |
| 835 | ≤ 1 | 874 | ≤ 10 | 913 | ≤ 0.01 | | |
| 836 | ≤ 0.01 | 875 | ≤ 10 | 914 | ≤ 0.01 | | |
| 837 | ≤ 0.01 | 876 | ≤ 1 | 915 | ≤ 0.01 | | |
| 838 | ≤ 0.01 | 877 | ≤ 10 | 916 | ≤ 0.1 | | |
| 839 | ≤ 0.01 | 878 | ≤ 10 | 917 | ≤ 0.1 | | |
| 840 | ≤ 0.01 | 879 | ≤ 1 | 918 | ≤ 1 | | |
| 841 | ≤ 0.01 | 880 | ≤ 1 | 919 | ≤ 10 | | |
| 842 | ≤ 0.01 | 881 | ≤ 1 | 920 | ≤ 0.01 | | |
| 843 | ≤ 0.1 | 882 | ≤ 1 | 921 | ≤ 0.1 | | |
| 844 | ≤ 1 | 883 | ≤ 10 | 922 | ≤ 0.01 | | |
| 845 | ≤ 1 | 884 | ≤ 0.1 | 923 | ≤ 0.1 | | |
| 846 | ≤ 0.1 | 885 | ≤ 0.1 | 924 | ≤ 0.01 | | |
| 847 | ≤ 0.1 | 886 | ≤ 1 | 925 | ≤ 0.01 | | |
| 848 | ≤ 1 | 887 | ≤ 0.1 | 926 | ≤ 0.01 | | |
| 849 | ≤ 0.1 | 888 | ≤ 1 | 927 | ≤ 0.01 | | |
| 850 | ≤ 1 | 889 | ≤ 0.1 | 928 | ≤ 0.01 | | |
| 851 | ≤ 10 | 890 | ≤ 0.1 | | | | |

### 3. Metabolic stability

Metabolic stability was determined as follows:
0.5 µM test substance was preincubated together with human liver microsomes (0.25 mg of microsomal protein/ml) in 0.05 M potassium phosphate buffer of pH 7.4 in microtiter plates at 37°C for 5 min. The reaction was started by adding NADPH (1.0 mM). After 0, 5, 10, 15, 20 and 30 min the reaction was stopped and cooled with twice the amount of quench solution consisting of acetonitrile/methanol 1:1, and containing 0.2 µM carbutamide. The samples were frozen until analyzed. The remaining concentration of undegraded test substance was determined by LC MSMS. The half-life (T1/2) was determined from the gradient of the signal of test substance/unit time plot, allowing to calculate the half-life of the test substance, assuming first order kinetics, from the decrease in the concentration of the compound with time. The microsomal clearance (mClint) was calculated as follows: mClint = ((ln(2)/t 1/2)/Microsomal Protein Concentration (mg/ml))*1000, leading to the unit of uL/min/mg. The scaled clearance (mClin_scaled) was calculated as mCLint_scaled = m CLint * (Microsomal Yield (mg/kg BW))/1000000*60, leading to the units L/hr/kg. The Microsomal Yield is defined by the specifics of the used microsomes. Calculations were modified from references: Di, The Society for Biomolecular Screening, 2003, 453-462; Obach, DMD, 1999 vol 27. N 11, 1350-1359.

### 4. Determination of efflux ratio using Madin-Darby Canine Kidney Type II cells

Bidirectional transport experiments were performed on Madin-Darby Canine Kidney Type II cells over-expressing multidrug resistance protein 1 (MDR1-MDCK) to evaluate the compounds as potential P-gp substrates.

Compounds were added at 1 µM in HBSS-pH 7.4 (hanks balanced salt solution) to either the apical or basolateral side of MDR1-MDCK cell monolayers grown on Millicell 96-Cell polycarbonate filters. Samples were collected from both apical and basolateral sides at time 0 and after 1h incubation at 37C, compounds concentrations were measured by HPLC/MS/MS and permeability coefficients were then determined in both transport directions. The efflux ratio was subsequently calculated from the permeability coefficient.

## Claims

1. Pyrrolidine derivative of the formula (I) 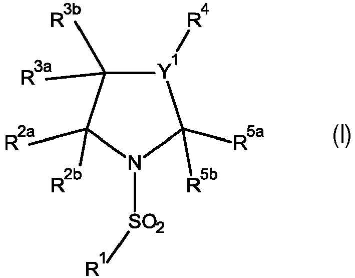 wherein
R¹ is a 5-membered heterocyclic ring selected from pyrazolyl, imidazolyl and triazolyl, which is optionally substituted with 1, 2 or 3 substituents independently selected from the group consisting of halogen, C₁-C₆-alkyl, halogenated C₁-C₆-alkyl, C₃-C₆-cycloalkyl, C₁-C₄-alkoxycarbonyl and C₁-C₆-alkylcarbonylamino;
R^{2a}, R^{2b} are independently hydrogen, halogen, or C₁-C₃-alkyl, or
R^{2a}, R^{2b} together with the carbon atom to which they are bound may form a C=O;
R^{3a} is C₃-C₁₂-cycloalkyl, optionally substituted C₆-C₁₂-aryl, hydroxy, C₁-C₆-alkoxy, halogenated C₁-C₆-alkoxy, C₃-C₁₂-cycloalkyl-C₁-C₄-alkoxy, C₂-C₆-alkenyloxy, C₆-C₁₂-aryl-C₁-C₄-alkoxy, C₃-C₁₂-heterocyclyl-C₁-C₄-alkoxy, optionally substituted C₆-C₁₂-aryloxy, C₃-C₁₂-heterocyclyloxy, or optionally substituted C₃-C₁₂-heterocyclyl; or
R^{3b} is hydrogen, C₁-C₆-alkyl, or hydroxyl; or
R^{3a} and R^{3b} together are optionally substituted C₂-C₅-alkylene;
Y¹ is >CR⁶- or >N-;
R⁶ is hydrogen, C₁-C₆-alkyl, halogenated C₁-C₆-alkyl, (optionally substituted C₆-C₁₂-aryl)-C₁-C₄-alkyl, hydroxy-C₁-C₆-alkyl, C₁-C₄-alkoxy-C₁-C₆-alkyl, or hydroxyl; or
R⁶ and R^{3a} or R^{3b} together are optionally substituted C₁-C₅-alkylene; or
R⁶ is C₁-C₄-alkylene that is bound to a carbon atom in R^{3a}, and R^{3a} is an optionally substituted C₆-C₁₂-aryl or an optionally substituted C₃-C₁₂-heterocyclyl;
R⁴ is -NR^{8a}(CR^{9a}R^{9b})ₙ₄R¹³, -(CR^{7a}R^{7b})ₙ₁OR¹⁰, -(CR^{7c}R^{7d})ₙ₂NR^{11a}R^{11b}, -(CR^{7e}R^{7f})ₙ₃R¹², optionally substituted C₆-C₁₂-aryl, -NR^{8b}COR¹⁴, -NR^{8c}COOR¹⁵, -NR^{8d}CONR^{16a}R^{16b}, -NR^{8e}SO₂R¹⁷,-O(CR^{9c}R^{9d})ₙ₅R¹⁸, -COR¹⁹, -CONR^{20a}R^{20b}, -SO₂R²¹, or optionally substituted C₃-C₁₂-heterocyclyl;
R^{7a}, R^{7b} are independently hydrogen or C₁-C₆-alkyl,
n1 is 1, 2, 3, or 4;
R¹⁰ is hydrogen, optionally substituted C₆-C₁₂-aryl, or optionally substituted C₃-C₁₂-heterocyclyl;
R^{7c}, R^{7d} are independently hydrogen or C₁-C₆-alkyl,
n2 is 1, 2, 3, or 4;
R^{11a} is C₁-C₈-alkyl, (optionally substituted C₃-C₁₂-cycloalkyl)-C₁-C₄-alkyl, C₁-C₆-alkoxy-C₁-C₆-alkyl, (optionally substituted C₆-C₁₂-aryl)-C₁-C₄-alkyl, (optionally substituted C₃-C₁₂-heterocyclyl)-C₁-C₄-alkyl, optionally substituted C₆-C₁₂-aryl, or optionally substituted C₃-C₁₂-heterocyclyl;
R^{11b} is hydrogen or C₁-C₆-alkyl;
R^{7e}, R^{7f} are independently hydrogen or C₁-C₆-alkyl,
n3 is 1, 2, 3, or 4;
R¹² is optionally substituted C₆-C₁₂-aryl or optionally substituted C₃-C₁₂-heterocyclyl;
R^{8a}, R^{8b}, R^{8c}, R^{8d}, R^{8e} are independently hydrogen, C₁-C₆-alkyl, or C₁-C₆-alkylcarbonyl, or
R⁶ and one of R^{8a}, R^{8b}, R^{8c}, R^{8d}, or R^{8e} together are optionally substituted C₁-C₅-alkylene, wherein one or more -CH₂- of C₁-C₅-alkylene may be independently replaced by a an oxygen atom or C=O; or
R^{3a} and one of R^{8a} or R^{8b} together are optionally substituted C₁-C₅-alkylene;
R^{9a}, R^{9b} are independently hydrogen, halogen, C₁-C₆-alkyl, halogenated C₁-C₆-alkyl, hydroxyl, or C₁-C₆-alkoxy;
n4 is 0,1, 2, 3, or 4;
R¹³ is hydrogen, C₁-C₈-alkyl, halogenated C₁-C₆-alkyl, (optionally substituted C₃-C₁₂-cycloalkyl)-C₁-C₄-alkyl, C₁-C₆-alkoxy-C₁-C₆-alkyl, optionally substituted C₃-C₁₂-cycloalkyl, C₂-C₆-alkenyl, optionally substituted C₃-C₆-cycloalkenyl, optionally substituted C₆-C₁₂-aryl, hydroxy, C₁-C₆-alkoxy, C₁-C₆-alkoxy-C₁-C₄-alkoxy, optionally substituted C₆-C₁₂-aryloxy, optionally substituted C₃-C₁₂-heterocyclyloxy, optionally substituted C₃-C₁₂-heterocyclyl, or tri-(C₁-C₄-alkyl)-silyloxy;
R¹⁴ is C₁-C₈-alkyl, halogenated C₁-C₆-alkyl, (optionally substituted C₃-C₁₂-cycloalkyl)-C₁-C₄-alkyl, (optionally substituted C₆-C₁₂-aryl)-C₁-C₄-alkyl, hydroxy-C₁-C₆-alkyl, C₁-C₆-alkoxy-C₁-C₆-alkyl, (optionally substituted C₆-C₁₂-aryloxy)-C₁-C₄-alkyl, C₁-C₆-alkylcarbonyl-C₁-C₄-alkyl, C₁-C₆-alkoxycarbonyl-C₁-C₄-alkyl, C₁-C₆-alkylaminocarbonyl-C₁-C₄-alkyl, optionally substituted (C₃-C₁₂-heterocyclyl)-C₁-C₄-alkyl, optionally substituted C₃-C₁₂-cycloalkyl, optionally substituted C₆-C₁₂-aryl, or optionally substituted C₃-C₁₂-heterocyclyl;
R¹⁵ is C₁-C₈-alkyl, optionally substituted C₆-C₁₂-aryl, or optionally substituted C₃-C₁₂-heterocyclyl;
R^{16a} is (optionally substituted C₆-C₁₂-aryl)-C₁-C₄-alkyl, optionally substituted (C₃-C₁₂-heterocyclyl)-C₁-C₄-alkyl, optionally substituted C₆-C₁₂-aryl, or optionally substituted C₃-C₁₂-heterocyclyl;
R^{16b} is hydrogen or C₁-C₆-alkyl;
R¹⁷ is (optionally substituted C₆-C₁₂-aryl)-C₁-C₄-alkyl, (optionally substituted C₃-C₁₂-heterocyclyl)-C₁-C₄-alkyl, optionally substituted C₆-C₁₂-aryl, or optionally substituted C₃-C₁₂-heterocyclyl;
R^{9c}, R^{9d} are independently hydrogen, halogen, or C₁-C₆-alkyl;
n5 is 0, 1, 2, 3, or 4;
R¹⁸ is hydrogen, C₁-C₈-alkyl, optionally substituted C₃-C₁₂-cycloalkyl, C₁-C₆-alkylcarbonyl, C₁-C₆-alkoxycarbonyl, halogenated C₁-C₆-alkoxycarbonyl, C₆-C₁₂₋aryloxycarbonyl, C₁-C₆-alkylaminocarbonyl, (halogenated C₁-C₄-alkyl)aminocarbonyl, C₆-C₁₂-arylaminocarbonyl, optionally substituted C₆-C₁₂-aryl, C₁-C₆-alkylamine, (C₃-C₁₂-cycloalkyl-C₁-C₄-alkyl)amino, (halogenated C₁-C₆-alkyl)amino, (C₁-C₆-alkoxy- C₁-C₆-alkyl)amino, (C₆-C₁₂-aryl-C₁-C₄-alkyl)amino, C₁-C₆-dialkylamine, optionally substituted C₆-C₁₂-arylamine, or optionally substituted C₃-C₁₂-heterocyclyl;
R¹⁹ is optionally substituted C₆-C₁₂-aryl or optionally substituted C₃-C₁₂-heterocyclyl;
R^{20a} is C₁-C₈-alkyl, (optionally substituted C₃-C₁₂-cycloalkyl)-C₁-C₄-alkyl, C₁-C₆-alkoxy-C₁-C₆-alkyl, (optionally substituted C₆-C₁₂-aryl)-C₁-C₄-alkyl, (optionally substituted C₃-C₁₂-heterocyclyl)-C₁-C₄-alkyl, optionally substituted C₆-C₁₂-aryl, or optionally substituted C₃-C₁₂-heterocyclyl;
R^{20b} is hydrogen or C₁-C₈-alkyl;
R²¹ is optionally substituted C₆-C₁₂-aryl, or optionally substituted C₃-C₁₂-heterocyclyl; and
R^{5a}, R^{5b} are independently hydrogen, halogen, or C₁-C₃-alkyl, or
R^{5a}, R^{5b} together with the carbon atom to which they are bound may form a C=O; or
one of R^{2a} or R^{2b} and one of R^{5a} or R^{5b} together are optionally substituted C₁-C₅-alkylene,
or a physiologically tolerated salt thereof;
provided that the following compounds are excluded:
compounds of formula (II) and the physiologically tolerated salts thereof; 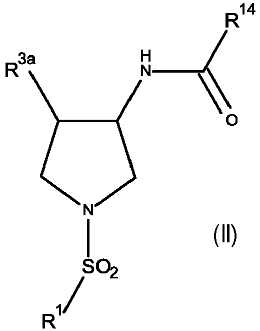 wherein R¹, R^{3a} and R¹⁴ are as defined in the following table
| R¹ | R^{3a} | R¹⁴ |
|---|---|---|
| 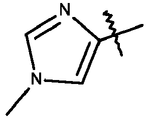 | 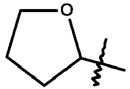 | 4-F-Ph |
| 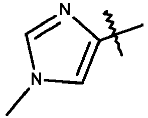 | 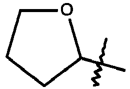 | 4-CF₃-Ph |
| 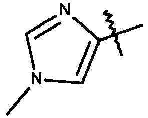 | 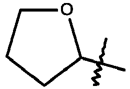 | 2-OMe-Ph |
| 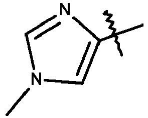 | 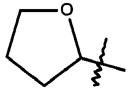 | 2-OMe-4-OCF₃-Ph |
| 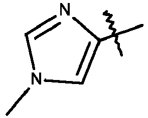 | 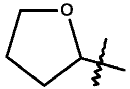 | 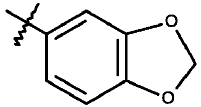 |
| 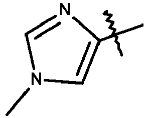 | 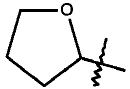 | 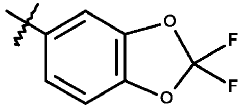 |
| 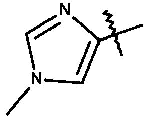 | 4-F-phenyl | 4-CF₃-phenyl |
| 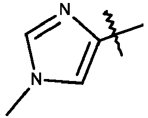 | 4-F-phenyl | 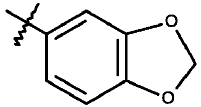 |
compounds of formula (II') 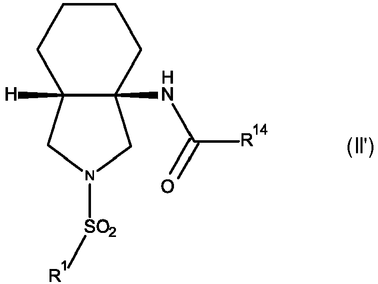 wherein R¹ and R¹⁴ are as defined in the following table
| R¹ | R¹⁴ |
|---|---|
| 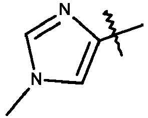 | 4-F-Ph |
| 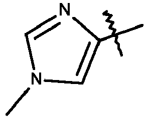 | 2-OMe-Ph |
| 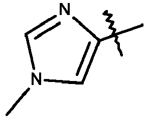 | 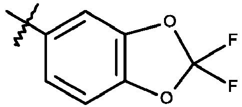 |
and the compounds of the formulae:

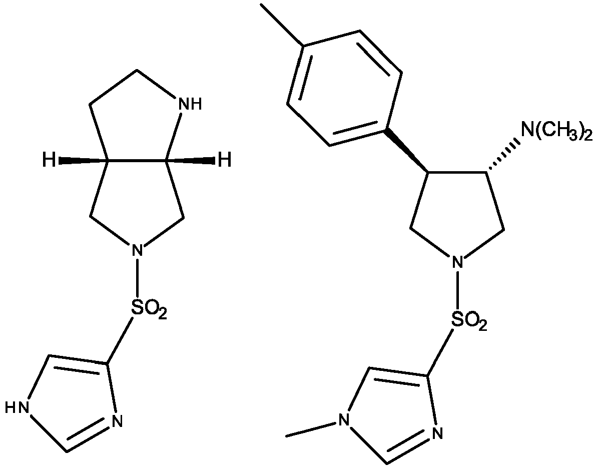

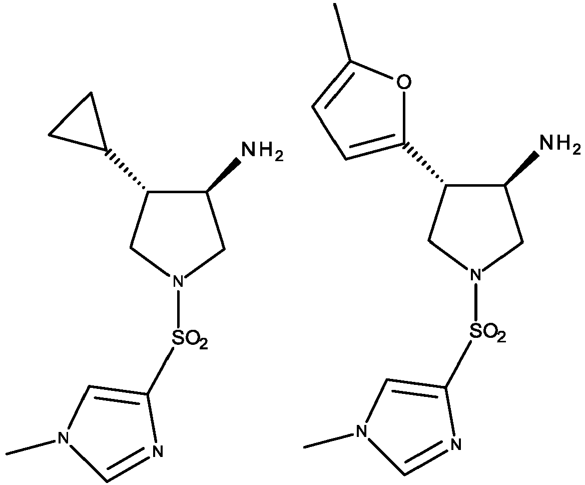

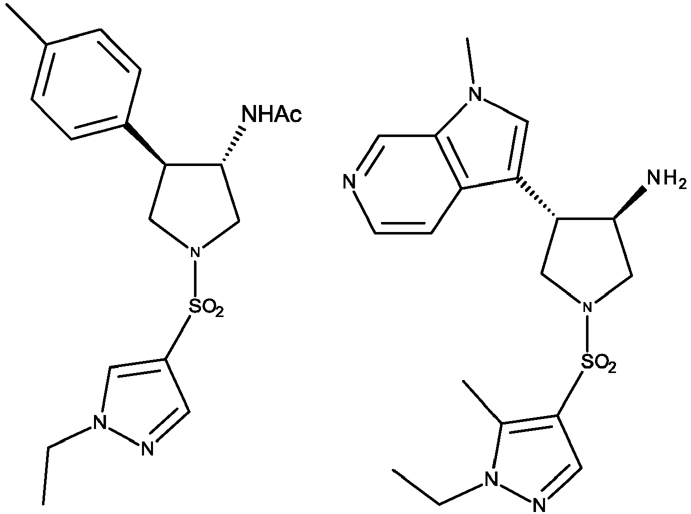

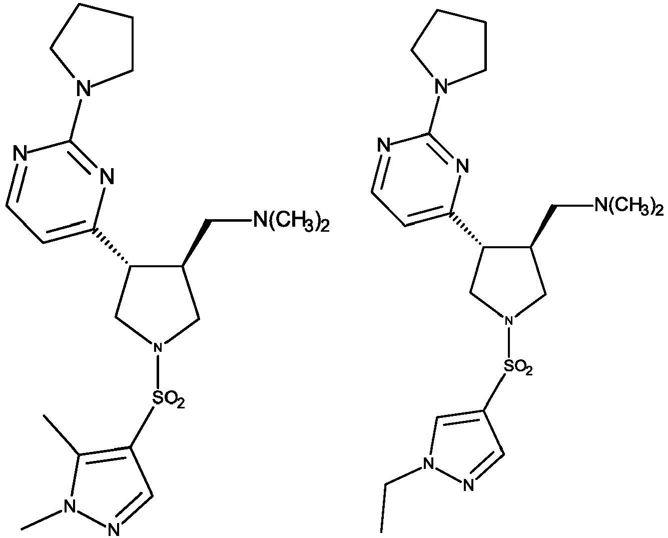

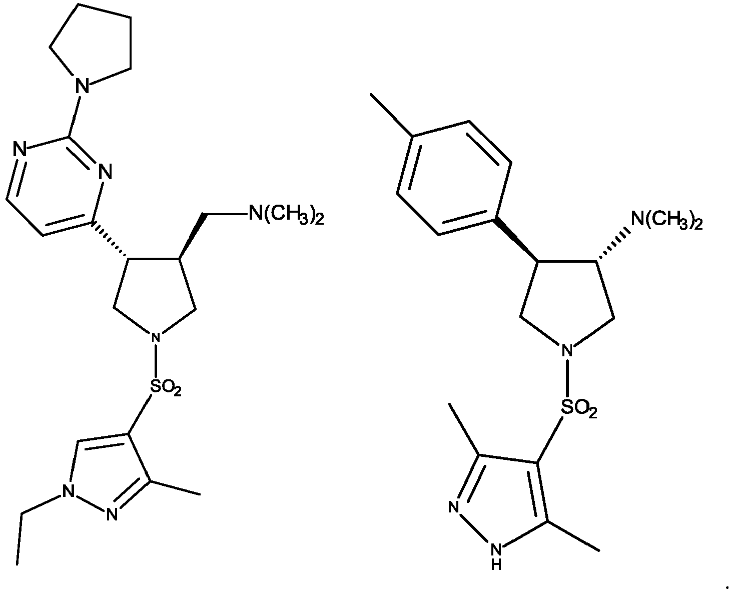

2. The compound as claimed in claim 1, wherein R¹ is optionally substituted 1,3-diazolyl or optionally substituted 1,2,3-triazolyl.

3. The compound as claimed in claim 1, wherein R¹ is 1,2-diazol-4-yl, 1-methyl-1,2-diazol-4-yl, 1-methyl-1,2-diazol-3-yl, 1-methyl-1,3-diazol-4-yl, 1-methyl-1,2-diazol-5-yl, 1,5-dimethyl-1,2-diazol-4-yl, 1,3-dimethyl-1,2-diazol-4-yl, 1,5-dimethyl-1,2-diazol-4-yl, 1,3-dimethyl-1,2-diazol-5-yl, 1,3-dimethyl-5-Cl-1,2-diazol-4-yl, 1-iso-propyl-3-methyl-1,2-diazol-4-yl, 1-Me-3-CF₃-1,2-diazol-4-yl, 1-cyclopentyl-3-Me-1,2-diazol-4-yl, 1,3,5-trimethyl-1,2-diazol-4-yl, 1-CHF₂-3,5-dimethyl-1,2-diazol-4-yl, 1,2-dimethyl-1,3-diazol-4-yl, 1,2-dimethyl-1,3-diazol-5-yl, 1-methyl-5-Cl-1,3-diazol-4-yl, 1-Me-1,2,3-triazol-4-yl, 1-ethyl-1,3-diazol-4-yl or 1-methyl-1,2,4-triazol-3-yl.

4. The compound as claimed in any one of claims 1-3, wherein R^{2a} is hydrogen, halogen or C₁-C₃-alkyl, and R^{2b} is hydrogen.

5. The compound as claimed in any one of claims 1-4, wherein R^{3a} is C₆-C₁₂-aryl optionally substituted with 1, 2, or 3 substituents independently selected from the group consisting of halogen, C₁-C₄-alkyl and C₁-C₄-alkoxy.

6. The compound as claimed in any one of claims 1-4, wherein R^{3a} is C₃-C₁₂-heterocyclyl optionally substituted with 1, 2, or 3 substituents independently selected from the group consisting of halogen, C₁-C₄-alkyl and C₃-C₆-cycloalkyl.

7. The compound as claimed in any one of claims 1-6, wherein R^{3b} is hydrogen.

8. The compound as claimed in any one of claims 1-7, wherein Y¹ is >CR⁶- and R⁶ is hydrogen, methyl, benzyl, hydroxy-methyl, or hydroxy.

9. The compound as claimed in any one of claims 1-8, wherein R^{5a} is hydrogen, halogen or C₁-C₃-alkyl, and R^{5b} is hydrogen.

10. The compound as claimed in any one of claims 1-9, having formula 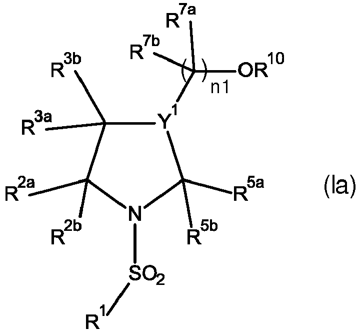
wherein R¹, R^{2a}, R^{2b}, R^{3a}, R^{3b}, Y¹, R^{5a} and R^{5b} are as defined in any one of claims 1-9, and
R^{7a}, R^{7b}
are independently hydrogen or C₁-C₆-alkyl;
n1 is 1, 2, 3, or 4; and
R¹⁰ is hydrogen, optionally substituted C₆-C₁₂-aryl, or optionally substituted C₃-C₁₂-heterocyclyl.

11. The compound as claimed in any one of claims 1-9, having formula 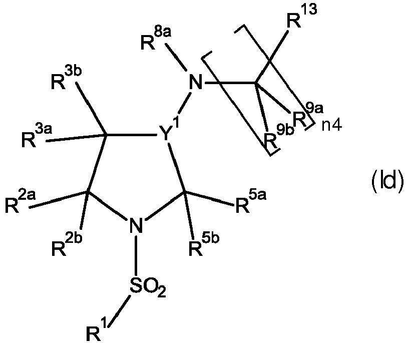
wherein R¹, R^{2a}, R^{2b}, R^{3a}, R^{3b}, Y¹, R^{5a} and R^{5b} are as defined in any one of claims 1-9, and
R^{8a} is hydrogen, C₁-C₆-alkyl, or C₁-C₆-alkylcarbonyl, or
R⁶, R^{8a}
together are optionally substituted C₁-C₅-alkylene, wherein one or more -CH₂- of C₁-C₅-alkylene may be independently replaced by a an oxygen atom or C=O;
R^{9a}, R^{9b}
are independently hydrogen, halogen, C₁-C₆-alkyl, hydroxy, or C₁-C₆-alkoxy;
n4 is 0, 1, 2, 3, or 4;
R¹³ is hydrogen, C₁-C₈-alkyl, halogenated C₁-C₆-alkyl, (optionally substituted C₃-C₁₂-cycloalkyl)-C₁-C₄-alkyl, C₁-C₆-alkoxy-C₁-C₆-alkyl, optionally substituted C₃-C₁₂-cycloalkyl, C₂-C₆-alkenyl, optionally substituted C₃-C₆-cycloalkenyl, optionally substituted C₆-C₁₂-aryl, hydroxy, C₁-C₆-alkoxy, C₁-C₆-alkoxy-C₁-C₄-alkoxy, optionally substituted C₆-C₁₂-aryloxy, optionally substituted C₃-C₁₂-heterocyclyloxy, optionally substituted C₃-C₁₂-heterocyclyl, or tri-(C₁-C₄-alkyl)-silyloxy.

12. The compound as claimed in claim 11, wherein R¹³ is a group of the formula (Id1): 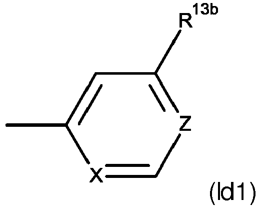 wherein
X is >CH- or >N-;
Z is >C-R^{13c} or >N-;
R^{13b} is halogen, C₁-C₄-alkyl, halogenated C₁-C₄-alkyl, hydroxy-(halogenated C₁-C₄-alkyl), C₆-C₁₂-aryl-C₁-C₄-alkyl, C₃-C₆-cycloalkyl, CN, C₆-C₁₂-aryl optionally substituted with halogen or C₁-C₄-alkyl, C₁-C₄-alkoxy, halogenated C₁-C₄-alkoxy, C₃-C₆-cycloalkoxy, C₆-C₁₂ aryl-C₁-C₄-alkoxy, C₃-C₁₂-heterocyclyl-C₁-C₄-alkoxy, C₆-C₁₂-aryloxy, C₁-C₄-alkyl-sulfonyl, C₁-C₄-alkyl-carbonylamino or C₃-C₁₂-heterocyclyl; and
R^{13c} is hydrogen or halogen.

13. The compound as claimed in any one of claims 1-9, having formula 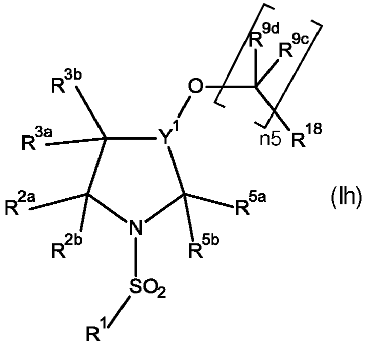
wherein R¹, R^{2a}, R^{2b}, R^{3a}, R^{3b}, Y¹, R^{5a} and R^{5b} are as defined in any one of claims 1-9, and
R^{9c}, R^{9d}
are independently hydrogen, halogen, or C₁-C₆-alkyl;
n5 is 0, 1, 2, 3, or 4; and
R¹⁸ is hydrogen, optionally substituted C₁-C₈-alkyl, optionally substituted C₃-C₁₂-cycloalkyl, C₁-C₆₋alkylcarbonyl, C₁-C₆-alkoxycarbonyl, halogenated C₁-C₆-alkoxycarbonyl, C₆-C₁₂-aryloxycarbonyl, C₁-C₆-alkylaminocarbonyl, (halogenated C₁-C₄-alkyl)aminocarbonyl, C₆-C₁₂₋arylaminocarbonyl, optionally substituted C₆-C₁₂-aryl, C₁-C₆-alkylamine, (C₃-C₁₂-cycloalkyl-C₁-C₄-alkyl)amino, (halogenated C₁-C₆-alkyl)amino, (C₁-C₆-alkoxy- C₁-C₆-alkyl)amino, (C₆-C₁₂-aryl-C₁-C₄-alkyl)amino, C₁-C₆-dialkylamine, optionally substituted C₆-C₁₂-arylamine, or optionally substituted C₃-C₁₂-heterocyclyl.

14. The compound as claimed in claim 1, wherein
R¹ is a 5-membered heterocyclic ring selected from pyrazolyl, imidazolyl and triazolyl, which is optionally substituted with 1, 2 or 3 substituents independently selected from the group consisting of halogen, C₁-C₆-alkyl, halogenated C₁-C₆-alkyl, C₃-C₆-cycloalkyl, C₁-C₄-alkoxycarbonyl and C₁-C₆-alkylcarbonylamino;
R^{2a}, R^{2b} are hydrogen;
R^{3a} is C₃-C₁₂-cycloalkyl, optionally substituted C₆-C₁₂-aryl, hydroxy, C₁-C₆-alkoxy, C₃-C₁₂-cycloalkyl-C₁-C₄-alkoxy, C₂-C₆-alkenyloxy, C₆-C₁₂-aryl-C₁-C₄-alkoxy, optionally substituted C₆-C₁₂-aryloxy, or optionally substituted C₃-C₁₂-heterocyclyl;
R^{3b} is hydrogen or hydroxy;
Y¹ is >CR⁶- or >N-;
R⁶ is hydrogen, C₁-C₆-alkyl, (C₆-C₁₂-aryl)-C₁-C₄-alkyl, hydroxy-C₁-C₆-alkyl, or hydroxy;
R⁴ is -(CR^{7a}R^{7b})ₙ₁OR¹⁰, -(CR^{7c}R^{7d})ₙ₂NR^{11a}R^{11b}, -(CR^{7e}R^{7f})ₙ₃R¹², optionally substituted C₆-C₁₂-aryl, -NR^{8a}(CR^{9a}R^{9b})ₙ₄R¹³, -NR^{8b}COR¹⁴, -NR^{8c}COOR¹⁵, -NR^{8d}CONR^{16a}R^{16b},-O(CR^{9c}R^{9d})ₙ₅R¹⁸, -COR¹⁹, -CONR^{20a}R^{20b}, -SO₂R²¹, or optionally substituted C₃-C₁₂-heterocyclyl;
R^{7a}, R^{7b} are hydrogen, or
n1 is 1;
R¹⁰ is hydrogen, optionally substituted C₆-C₁₂-aryl, or optionally substituted C₃-C₁₂-heterocyclyl;
R^{7c}, R^{7d} are hydrogen;
n2 is 1;
R^{11a} is C₁-C₈-alkyl, C₃-C₁₂-cycloalkyl-C₁-C₄-alkyl, C₁-C₆-alkoxy-C₁-C₆-alkyl, C₆-C₁₂-aryl-C₁-C₄-alkyl, optionally substituted C₆-C₁₂-aryl;
R^{11b} is hydrogen;
R^{7e}, R^{7f} are hydrogen,
n3 is 1;
R¹² is optionally substituted C₆-C₁₂-aryl or optionally substituted C₃-C₁₂-heterocyclyl;
R^{8a}, R^{8b}, R^{8c}, R^{8d}, R^{8e} are independently hydrogen or C₁-C₆-alkyl, or
R⁶ and one of R^{8a}, R^{8b}, R^{8c}, R^{8d}, or R^{8e} together are optionally substituted C₁-C₅-alkylene, wherein one or more -CH₂- of C₁-C₅-alkylene may be independently replaced by a an oxygen atom or C=O;
R^{9a}, R^{9b} are independently hydrogen, halogen, C₁-C₆-alkyl, or C₁-C₆-alkoxy;
n4 is 0, 1, 2, 3, or 4;
R¹³ is hydrogen, C₁-C₈-alkyl, halogenated C₁-C₆-alkyl, C₁-C₆-alkoxy-C₁-C₆-alkyl, optionally substituted C₃-C₁₂-cycloalkyl, C₂-C₆-alkenyl, optionally substituted C₃-C₆-cycloalkenyl, optionally substituted C₆-C₁₂-aryl, C₁-C₆-alkoxy, C₁-C₆-alkoxy-C₁-C₄-alkoxy, optionally substituted C₆-C₁₂-aryloxy, optionally substituted C₃-C₁₂-heterocyclyl, or tri-(C₁-C₄-alkyl)-silyloxy;
R¹⁴ is C₁-C₈-alkyl, halogenated C₁-C₆-alkyl, C₃-C₁₂-cycloalkyl-C₁-C₄-alkyl, hydroxy-C₁-C₆-alkyl, (optionally substituted C₆-C₁₂-aryloxy)-C₁-C₄-alkyl, C₁-C₆-alkylcarbonyl-C₁-C₄-alkyl, C₁-C₆-alkoxycarbonyl-C₁-C₄-alkyl, optionally substituted C₃-C₁₂-cycloalkyl, optionally substituted C₆-C₁₂-aryl, or optionally substituted C₃-C₁₂-heterocyclyl;
R¹⁵ is C₁-C₈-alkyl, C₆-C₁₂-aryl, or optionally substituted C₃-C₁₂-heterocyclyl;
R^{16a} is (optionally substituted C₆-C₁₂-aryl)-C₁-C₄-alkyl or optionally substituted C₆-C₁₂-aryl;
R^{16b} is hydrogen;
R^{9c}, R^{9d} are hydrogen;
n5 is 0, 1, or 2;
R¹⁸ is hydrogen, C₁-C₈-alkyl, C₁-C₆-alkoxycarbonyl, C₁-C₆-alkylaminocarbonyl, (halogenated C₁-C₄-alkyl)aminocarbonyl, optionally substituted C₆-C₁₂-aryl, C₁-C₆-alkylamine, (halogenated C₁-C₆-alkyl)amino, optionally substituted C₆-C₁₂-arylamine, or optionally substituted C₃-C₂-heterocyclyl;
R¹⁹ is optionally substituted C₆-C₁₂-aryl;
R^{20a} is C₁-C₈-alkyl, C₃-C₁₂-cycloalkyl-C₁-C₄-alkyl, C₁-C₆-alkoxy-C₁-C₆-alkyl, (optionally substituted C₆-C₁₂-aryl)-C₁-C₄-alkyl, (optionally substituted C₃-C₁₂-heterocyclyl)-C₁-C₄-alkyl, optionally substituted C₆-C₁₂-aryl, or optionally substituted C₃-C₁₂-heterocyclyl;
R^{20b} is hydrogen or C₁-C₈-alkyl;
R²¹ is C₆-C₁₂-aryl; and
R^{5a}, R^{5b} are hydrogen, or
R^{5a}, R^{5b} together with the carbon atom to which they are bound may form a C=O.

15. The compound as claimed in claim 1 which is:
2-chloro-N-{trans-1-[(1-methyl-1H-imidazol-4-yl)sulfonyl]-4-phenylpyrrolidin-3-yl}-3-(trifluoromethyl)benzamide;
2-chloro-N-{trans-4-(4-methoxyphenyl)-1-[(1-methyl-1H-imidazol-4-yl)sulfonyl]pyrrolidin-3-yl}-3-(trifluoromethyl)benzamide;
2-chloro-N-{trans-4-(4-fluorophenyl)-1-[(1-methyl-1H-imidazol-4-yl)sulfonyl]pyrrolidin-3-yl}-3-(trifluoromethyl)benzamide;
2-chloro-N-{trans-1-[(1-methyl-1H-imidazol-4-yl)sulfonyl]-4-phenylpyrrolidin-3-yl}-3-(trifluoromethyl)benzamide;
2-chloro-N-{trans-4-(4-chlorophenyl)-1-[(1-methyl-1H-imidazol-4-yl)sulfonyl]pyrrolidin-3-yl}-3-(trifluoromethyl)benzamide;
2-chloro-N-{trans-1-[(1-methyl-1H-imidazol-4-yl)sulfonyl]-4-phenylpyrrolidin-3-yl}benzamide;
3-chloro-N-{trans-1-[(1-methyl-1H-imidazol-4-yl)sulfonyl]-4-phenylpyrrolidin-3-yl}benzamide;
4-chloro-N-{trans-1-[(1-methyl-1H-imidazol-4-yl)sulfonyl]-4-phenylpyrrolidin-3-yl}benzamide;
2-fluoro-N-{trans-1-[(1-methyl-1H-imidazol-4-yl)sulfonyl]-4-phenylpyrrolidin-3-yl}benzamide;
3-fluoro-N-{trans-1-[(1-methyl-1H-imidazol-4-yl)sulfonyl]-4-phenylpyrrolidin-3-yl}benzamide;
4-fluoro-N-{trans-1-[(1-methyl-1H-imidazol-4-yl)sulfonyl]-4-phenylpyrrolidin-3-yl}benzamide;
2-methoxy-N-{trans-1-[(1-methyl-1H-imidazol-4-yl)sulfonyl]-4-phenylpyrrolidin-3-yl}benzamide;
3-methoxy-N-{trans-1-[(1-methyl-1H-imidazol-4-yl)sulfonyl]-4-phenylpyrrolidin-3-yl}benzamide;
2-methyl-N-{trans-1-[(1-methyl-1H-imidazol-4-yl)sulfonyl]-4-phenylpyrrolidin-3-yl}benzamide;
3-methyl-N-{trans-1-[(1-methyl-1H-imidazol-4-yl)sulfonyl]-4-phenylpyrrolidin-3-yl}benzamide;
4-methyl-N-{trans-1-[(1-methyl-1H-imidazol-4-yl)sulfonyl]-4-phenylpyrrolidin-3-yl}benzamide;
N-{trans-1-[(1-methyl-1H-imidazol-4-yl)sulfonyl]-4-phenylpyrrolidin-3-yl}benzamide;
2,4-dichloro-N-{trans-1-[(1-methyl-1H-imidazol-4-yl)sulfonyl] -4-phenylpyrrolidin-3-yl}benzamide;
2-chloro-4-fluoro-N-{trans-1-[(1-methyl-1H-imidazol-4-yl)sulfonyl]-4-phenylpyrrolidin-3-yl}benzamide;
trans-1-[(1-methyl-1H-imidazol-4-yl)sulfonyl]-N,4-diphenylpyrrolidin-3-amine;
3,5-dichloro-N-{trans-1-[(1-methyl-1H-imidazol-4-yl)sulfonyl]-4-phenylpyrrolidin-3-yl}benzamide;
2,3-dichloro-N-{trans-1-[(1-methyl-1H-imidazol-4-yl)sulfonyl]-4-phenylpyrrolidin-3-yl}benzamide;
2-cyano-N-{trans-1-[(1-methyl-1H-imidazol-4-yl)sulfonyl]-4-phenylpyrrolidin-3-yl}benzamide;
3-cyano-N-{trans-1-[(1-methyl-1H-imidazol-4-yl)sulfonyl]-4-phenylpyrrolidin-3-yl}benzamide;
4-cyano-N-{trans-1-[(1-methyl-1H-imidazol-4-yl)sulfonyl]-4-phenylpyrrolidin-3-yl}benzamide;
N-{trans-1-[(1-methyl-1H-imidazol-4-yl)sulfonyl]-4-phenylpyrrolidin-3-yl}-2-(trifluoromethyl)benzamide;
N-{trans-1-[(1-methyl-1H-imidazol-4-yl)sulfonyl]-4-phenylpyrrolidin-3-yl}-3-(trifluoromethyl)benzamide;
N-{trans-1-[(1-methyl-1H-imidazol-4-yl)sulfonyl]-4-phenylpyrrolidin-3-yl}-4-(trifluoromethyl)benzamide;
2-chloro-N-[trans-1-{[1-methyl-3-(trifluoromethyl)-1H-pyrazol-4-yl]sulfonyl}-4-phenylpyrrolidin-3-yl]-3-(trifluoromethyl)benzamide;
2-chloro-N-[trans-1-{[1-(difluoromethyl)-3,5-dimethyl-1H-pyrazol-4-yl]sulfonyl}-4-phenylpyrrolidin-3-yl]-3-(trifluoromethyl)benzamide;
2-chloro-N-{trans-1-[(5-chloro-1,3-dimethyl-1H-pyrazol-4-yl)sulfonyl]-4-phenylpyrrolidin-3-yl}-3-(trifluoromethyl)benzamide;
2-chloro-N-{trans-1-[(1,5-dimethyl-1H-pyrazol-4-yl)sulfonyl]-4-phenylpyrrolidin-3-yl}-3-(trifluoromethyl)benzamide;
2-chloro-N-{trans-1-[(1,3-dimethyl-1H-pyrazol-4-yl)sulfonyl]-4-phenylpyrrolidin-3-yl}-3-(trifluoromethyl)benzamide;
2-chloro-N-{trans-1-[(1-methyl-1H-pyrazol-4-yl)sulfonyl]-4-phenylpyrrolidin-3-yl}-3-(trifluoromethyl)benzamide;
2-chloro-N-[trans-4-phenyl-1-(1H-pyrazol-4-ylsulfonyl)pyrrolidin-3-yl]-3-(trifluoromethyl)benzamide;
2-chloro-N-{trans-1-[(1-methyl-1H-pyrazol-5-yl)sulfonyl]-4-phenylpyrrolidin-3-yl}-3-(trifluoromethyl)benzamide;
2-chloro-N-{trans-1-[(5-chloro-1-methyl-1H-imidazol-4-yl)sulfonyl]-4-phenylpyrrolidin-3-yl}-3-(trifluoromethyl)benzamide;
2-chloro-N-{trans-1-[(1-methyl-1H-pyrazol-3-yl)sulfonyl]-4-phenylpyrrolidin-3-yl}-3-(trifluoromethyl)benzamide;
trans-N-(4-methoxyphenyl)-1-[(1-methyl-1H-imidazol-4-yl)sulfonyl]-4-phenylpyrrolidin-3-amine; 3-({trans-1-[(1-methyl-1H-imidazol-4-yl)sulfonyl]-4-phenylpyrrolidin-3-yl}amino)benzonitrile;
4-({trans-1-[(1-methyl-1H-imidazol-4-yl)sulfonyl]-4-phenylpyrrolidin-3-yl}amino)benzonitrile;
3-fluoro-4-({trans-1-[(1-methyl-1H-imidazol-4-yl)sulfonyl]-4-phenylpyrrolidin-3-yl}amino)benzonitrile;
2-chloro-N-{trans-4-(2-fluorophenyl)-1-[(1-methyl-1H-imidazol-4-yl)sulfonyl]pyrrolidin-3-yl}-3-(trifluoromethyl)benzamide;
2-chloro-N-{trans-4-(3-fluorophenyl)-1-[(1-methyl-1H-imidazol-4-yl)sulfonyl]pyrrolidin-3-yl}-3-(trifluoromethyl)benzamide;
2-chloro-N-{trans-4-(3-chlorophenyl)-1-[(1-methyl-1H-imidazol-4-yl)sulfonyl]pyrrolidin-3-yl}-3-(trifluoromethyl)benzamide;
trans-1-[(1-methyl-1H-imidazol-4-yl)sulfonyl]-N-(2-methylphenyl)-4-phenylpyrrolidin-3-amine;
trans-1-[(1-methyl-1H-imidazol-4-yl)sulfonyl]-N-(3-methylphenyl)-4-phenylpyrrolidin-3-amine;
trans-1-[(1-methyl-1H -imidazol-4-yl)sulfonyl]-N-(4-methylphenyl)-4-phenylpyrrolidin-3-amine;
2-chloro-N-{trans-1-[(1-methyl-1H-imidazol-4-yl)sulfonyl]-4-phenylpyrrolidin-3-yl}-4-(trifluoromethyl)benzamide;
3-chloro-N-{trans-1-[(1-methyl-1H-imidazol-4-yl)sulfonyl]-4-phenylpyrrolidin-3-yl}-4-(trifluoromethyl)benzamide;
2-chloro-N-{trans-4-(4-fluorophenyl)-1-[(1-methyl-1H-imidazol-4-yl)sulfonyl]pyrrolidin-3-yl}-4-(trifluoromethyl)benzamide;
3-chloro-N-{trans-4-(4-fluorophenyl)-1-[(1-methyl-1H-imidazol-4-yl)sulfonyl]pyrrolidin-3-yl}-4-(trifluoromethyl)benzamide;
N-{trans-4-(4-fluorophenyl)-1-[(1-methyl-1H-imidazol-4-yl)sulfonyl]pyrrolidin-3-yl}-4-(trifluoromethyl)benzamide;
3,5-dichloro-N-{trans-4-(4-fluorophenyl)-1-[(1-methyl-1H-imidazol-4-yl)sulfonyl]pyrrolidin-3-yl}benzamide;
trans-1-[(1-methyl-1H-imidazol-4-yl)sulfonyl]-4-phenyl-N-[2-(trifluoromethyl)phenyl]pyrrolidin-3-amine;
trans-1-[(1-methyl-1H-imidazol-4-yl)sulfonyl]-4-phenyl-N-[3-(trifluoromethyl)phenyl]pyrrolidin-3-amine;
trans-1-[(1-methyl-1H-imidazol-4-yl)sulfonyl]-4-phenyl-N-[4-(trifluoromethyl)phenyl]pyrrolidin-3-amine;
trans-N-(2-fluorophenyl)-1-[(1-methyl-1H-imidazol-4-yl)sulfonyl]-4-phenylpyrrolidin-3-amine;
trans-N-(3-fluorophenyl)-1-[(1-methyl-1H-imidazol-4-yl)sulfonyl]-4-phenylpyrrolidin-3-amine;
trans-N-(4-fluorophenyl)-1-[(1-methyl-1H-imidazol-4-yl)sulfonyl]-4-phenylpyrrolidin-3-amine;
3-({trans-4-(4-fluorophenyl)-1-[(1-methyl-1H-imidazol-4-yl)sulfonyl]pyrrolidin-3-yl}amino)benzonitrile;
trans-N-(2-chlorophenyl)-1-[(1-methyl-1H-imidazol-4-yl)sulfonyl]-4-phenylpyrrolidin-3-amine;
trans-N-(3-chlorophenyl)-1-[(1-methyl-1H-imidazol-4-yl)sulfonyl]-4-phenylpyrrolidin-3-amine;
trans-N-(4-chlorophenyl)-1-[(1-methyl-1H-imidazol-4-yl)sulfonyl]-4-phenylpyrrolidin-3-amine;
4-fluoro-3-({trans-1-[(1-methyl-1H-imidazol-4-yl)sulfonyl]-4-phenylpyrrolidin-3-yl}amino)benzonitrile;
2-fluoro-3-({trans-1-[(1-methyl-1H-imidazol-4-yl)sulfonyl]-4-phenylpyrrolidin-3-yl}amino)benzonitrile;
3-fluoro-5-({trans-1-[(1-methyl-1H-imidazol-4-yl)sulfonyl]-4-phenylpyrrolidin-3-yl}amino)benzonitrile;
2-chloro-5-({trans-1-[(1-methyl-1H-imidazol-4-yl)sulfonyl]-4-phenylpyrrolidin-3-yl}amino)benzonitrile;
3-chloro-5-({trans-1-[(1-methyl-1H-imidazol-4-yl)sulfonyl]-4-phenylpyrrolidin-3-yl}amino)benzonitrile;
4-chloro-3-({trans-1-[(1-methyl-1H-imidazol-4-yl)sulfonyl]-4-phenylpyrrolidin-3-yl}amino)benzonitrile;
4-methyl-3-({trans-1-[(1-methyl-1H-imidazol-4-yl)sulfonyl]-4-phenylpyrrolidin-3-yl}amino)benzonitrile;
2-methyl-3-({trans-1-[(1-methyl-1H-imidazol-4-yl)sulfonyl]-4-phenylpyrrolidin-3-yl}amino)benzonitrile;
trans-4-(4-fluorophenyl)-1-[(1-methyl-1H-imidazol-4-yl)sulfonyl]-N-(2-methylphenyl)pyrrolidin-3-amine;
trans-4-(4-fluorophenyl)-1-[(1-methyl-1H-imidazol-4-yl)sulfonyl]-N-(3-methylphenyl)pyrrolidin-3-amine;
trans-4-(4-fluorophenyl)-1-[(1-methyl-1H-imidazol-4-yl)sulfonyl]-N-(4-methylphenyl)pyrrolidin-3-amine;
trans-4-(4-fluorophenyl)-1-[(1-methyl-1H-imidazol-4-yl)sulfonyl]-N-[2-(trifluoromethyl)phenyl]pyrrolidin-3-amine;
trans-4-(4-fluorophenyl)-1-[(1-methyl-1H-imidazol-4-yl)sulfonyl]-N-[3-(trifluoromethyl)phenyl]pyrrolidin-3-amine;
trans-4-(4-fluorophenyl)-1-[(1-methyl-1H-imidazol-4-yl)sulfonyl]-N-[4-(trifluoromethyl)phenyl]pyrrolidin-3-amine;
trans-N-(2-fluorophenyl)-4-(4-fluorophenyl)-1-[(1-methyl-1H-imidazol-4-yl)sulfonyl]pyrrolidin-3-amine;
trans-N-(3-fluorophenyl)-4-(4-fluorophenyl)-1-[(1-methyl-1H-imidazol-4-yl)sulfonyl]pyrrolidin-3-amine;
trans-N,4-bis(4-fluorophenyl)-1-[(1-methyl-1H-imidazol-4-yl)sulfonyl]pyrrolidin-3-amine;
trans-N-(2-chlorophenyl)-4-(4-fluorophenyl)-1-[(1-methyl-1H-imidazol-4-yl)sulfonyl]pyrrolidin-3-amine;
trans-N-(3-chlorophenyl)-4-(4-fluorophenyl)-1-[(1-methyl-1H-imidazol-4-yl)sulfonyl]pyrrolidin-3-amine;
trans-N-(4-chlorophenyl)-4-(4-fluorophenyl)-1-[(1-methyl-1H-imidazol-4-yl)sulfonyl]pyrrolidin-3-amine;
2-methoxy-5-({trans-1-[(1-methyl-1H-imidazol-4-yl)sulfonyl]-4-phenylpyrrolidin-3-yl}amino)benzonitrile;
3-methyl-5-({trans-1-[(1-methyl-1H-imidazol-4-yl)sulfonyl]-4-phenylpyrrolidin-3-yl}amino)benzonitrile;
5-({trans-1-[(1-methyl-1H-imidazol-4-yl)sulfonyl]-4-phenylpyrrolidin-3-yl}amino)-2-(trifluoromethoxy)benzonitrile;
2-chloro-N-{trans-4-(2-chlorophenyl)-1-[(1-methyl-1H-imidazol-4-yl)sulfonyl]pyrrolidin-3-yl}-3-(trifluoromethyl)benzamide;
4-{[trans-3-benzyl-4-phenylpyrrolidin-1-yl]sulfonyl}-1-methyl-1H-imidazole;
trans-N-benzyl-4-(4-fluorophenyl)-1-[(1-methyl-1H-imidazol-4-yl)sulfonyl]pyrrolidin-3-amine;
trans-N-[2-chloro-3-(trifluoromethyl)benzyl]-4-(4-fluorophenyl)-1-[(1-methyl-1H-imidazol-4-yl)sulfonyl]pyrrolidin-3-amin;
trans-N-[2-chloro-4-(trifluoromethyl)benzyl]-4-(4-fluorophenyl)-1-[(1-methyl-1H-imidazol-4-yl)sulfonyl]pyrrolidin-3-amine;
trans-N-[3-chloro-4-(trifluoromethyl)benzyl]-4-(4-fluorophenyl)-1-[(1-methyl-1H-imidazol-4-yl)sulfonyl]pyrrolidin-3-amine;
3-[({trans-4-(4-fluorophenyl)-1-[(1-methyl-1H-imidazol-4-yl)sulfonyl]pyrrolidin-3-yl}amino)methyl]benzonitrile;
trans-4-(4-fluorophenyl)-N-(2-methylbenzyl)-1-[(1-methyl-1H-imidazol-4-yl)sulfonyl]pyrrolidin-3-amine;
trans-4-(4-fluorophenyl)-N-(3-methylbenzyl)-1-[(1-methyl-1H-imidazol-4-yl)sulfonyl]pyrrolidin-3-amine;
trans-4-(4-fluorophenyl)-N-(4-methylbenzyl)-1-[(1-methyl-1H-imidazol-4-yl)sulfonyl]pyrrolidin-3-amine;
trans-N-(4-fluorobenzyl)-4-(4-fluorophenyl)-1-[(1-methyl-1H-imidazol-4-yl)sulfonyl]pyrrolidin-3-amine;
(cis)-N-benzyl-1-[(1-methyl-1H-imidazol-4-yl)sulfonyl]-4-phenylpyrrolidin-3-amine;
trans-N-(3,4-difluorophenyl)-4-(4-fluorophenyl)-1-[(1-methyl-1H-imidazol-4-yl)sulfonyl]pyrrolidin-3-amine;
trans-4-(4-fluorophenyl)-N-[4-fluoro-3-(trifluoromethyl)phenyl]-1-[(1-methyl-1H-imidazol-4-yl)sulfonyl]pyrrolidin-3-amine;
trans-N-(3-chloro-4-fluorophenyl)-4-(4-fluorophenyl)-1-[(1-methyl-1H-imidazol-4-yl)sulfonyl]pyrrolidin-3-amine;
trans-N-(3,4-dichlorophenyl)-4-(4-fluorophenyl)-1-[(1-methyl-1H-imidazol-4-yl)sulfonyl]pyrrolidin-3-amine;
trans-N-(4-chloro-3-fluorophenyl)-4-(4-fluorophenyl)-1-[(1-methyl-1H-imidazol-4-yl)sulfonyl]pyrrolidin-3-amine;
trans-N-(2,3-difluorophenyl)-4-(4-fluorophenyl)-1-[(1-methyl-1H-imidazol-4-yl)sulfonyl]pyrrolidin-3-amine;
trans-N-(2,5-difluorophenyl)-4-(4-fluorophenyl)-1-[(1-methyl-1H-imidazol-4-yl)sulfonyl]pyrrolidin-3-amine;
trans-N-(2,4-difluorophenyl)-4-(4-fluorophenyl)-1-[(1-methyl-1H-imidazol-4-yl)sulfonyl]pyrrolidin-3-amine;
trans-4-(4-fluorophenyl)-1-[(1-methyl-1H-imidazol-4-yl)sulfonyl]-N-(2,3,4-trifluorophenyl)pyrrolidin-3-amine;
trans-4-(4-fluorophenyl)-N-[2-fluoro-3-(trifluoromethyl)phenyl]-1-[(1-methyl-1H-imidazol-4-yl)sulfonyl]pyrrolidin-3-amine;
trans-4-(4-fluorophenyl)-N-[3-fluoro-5-(trifluoromethyl)phenyl]-1-[(1-methyl-1H-imidazol-4-yl)sulfonyl]pyrrolidin-3-amine;
trans-4-(4-fluorophenyl)-N-[2-fluoro-5-(trifluoromethyl)phenyl]-1-[(1-methyl-1H-imidazol-4-yl)sulfonyl]pyrrolidin-3-amine;
trans-4-(4-fluorophenyl)-1-[(1-methyl-1H-imidazol-4-yl)sulfonyl]-N-[3-(propan-2-yl)phenyl]pyrrolidin-3-amine;
trans-N-(3-tert-butylphenyl)-4-(4-fluorophenyl)-1-[(1-methyl-1H-imidazol-4-yl)sulfonyl]pyrrolidin-3-amine;
trans-N-[4-chloro-3-(trifluoromethyl)phenyl]-4-(4-fluorophenyl)-1-[(1-methyl-1H-imidazol-4-yl)sulfonyl]pyrrolidin-3-amine;
trans-N-[3-chloro-4-(trifluoromethyl)phenyl]-4-(4-fluorophenyl)-1-[(1-methyl-1H-imidazol-4-yl)sulfonyl]pyrrolidin-3-amine;
trans-N-(3-chloro-5-fluorophenyl)-4-(4-fluorophenyl)-1-[(1-methyl-1H-imidazol-4-yl)sulfonyl]pyrrolidin-3-amine;
trans-N-(3-chloro-4,5-difluorophenyl)-4-(4-fluorophenyl)-1-[(1-methyl-1H-imidazol-4-yl)sulfonyl]pyrrolidin-3-amine;
trans-4-(4-fluorophenyl)-1-[(1-methyl-1H-imidazol-4-yl)sulfonyl]-N-[3-(trifluoromethoxy)phenyl]pyrrolidin-3-amine;
N-{trans-4-(4-fluorophenyl)-1-[(1-methyl-1H-imidazol-4-yl)sulfonyl]pyrrolidin-3-yl}pyridin-4-amine;
N-{trans-4-(4-fluorophenyl)-1-[(1-methyl-1H-imidazol-4-yl)sulfonyl]pyrrolidin-3-yl}pyridin-2-amine;
N-{trans-4-(4-fluorophenyl)-1-[(1-methyl-1H-imidazol-4-yl)sulfonyl]pyrrolidin-3-yl}pyridin-3-amine;
N-{trans-4-(4-fluorophenyl)-1-[(1-methyl-1H-imidazol-4-yl)sulfonyl]pyrrolidin-3-yl}-6-(trifluoromethyl)pyridin-2-amine;
N-{trans-4-(4-fluorophenyl)-1-[(1-methyl-1H-imidazol-4-yl)sulfonyl]pyrrolidin-3-yl}-5-(trifluoromethyl)pyridin-3-amine;
4-{[3-(3-chlorobenzyl)-4-(4-fluorophenyl)pyrrolidin-1-yl]sulfonyl}-1-methyl-1H-imidazole;
2-chloro-N-{(cis)-1-[(1-methyl-1H-imidazol-4-yl)sulfonyl]-4-phenylpyrrolidin-3-yl}-3-(trifluoromethyl)benzamide;
2-chloro-N-{(cis)-1-[(1-methyl-1H-imidazol-4-yl)sulfonyl]-4-phenylpyrrolidin-3-yl}-3-(trifluoromethyl)benzamide;
trans-4-(3-fluorophenyl)-1-[(1-methyl-1H-imidazol-4-yl)sulfonyl]-N-phenylpyrrolidin-3-amine;
trans-N,4-bis(3-fluorophenyl)-1-[(1-methyl-1H-imidazol-4-yl)sulfonyl]pyrrolidin-3-amine;
trans-4-(3-fluorophenyl)-N-(4-fluorophenyl)-1-[(1-methyl-1H-imidazol-4-yl)sulfonyl]pyrrolidin-3-amine;
trans-N-(3-chlorophenyl)-4-(3-fluorophenyl)-1-[(1-methyl-1H-imidazol-4-yl)sulfonyl]pyrrolidin-3-amine;
trans-4-(3-fluorophenyl)-1-[(1-methyl-1H-imidazol-4-yl)sulfonyl]-N-[3-(trifluoromethyl)phenyl]pyrrolidin-3-amine;
trans-4-(3-fluorophenyl)-1-[(1-methyl-1H-imidazol-4-yl)sulfonyl]-N-[4-(trifluoromethyl)phenyl]pyrrolidin-3-amine;
N-{trans-4-(3-fluorophenyl)-1-[(1-methyl-1H-imidazol-4-yl)sulfonyl]pyrrolidin-3-yl}pyridin-4-amine;
trans-N-(3,4-dichlorophenyl)-4-(3-fluorophenyl)-1-[(1-methyl-1H-imidazol-4-yl)sulfonyl]pyrrolidin-3-amine;
trans-N-(4-chloro-3-fluorophenyl)-4-(3-fluorophenyl)-1-[(1-methyl-1H-imidazol-4-yl)sulfonyl]pyrrolidin-3-amine;
trans-N-(3-chloro-4-fluorophenyl)-4-(3-fluorophenyl)-1-[(1-methyl-1H-imidazol-4-yl)sulfonyl]pyrrolidin-3-amine;
N-{trans-4-(3-fluorophenyl)-1-[(1-methyl-1H-imidazol-4-yl)sulfonyl]pyrrolidin-3-yl}-6-(trifluoromethyl)pyridin-2-amine;
N-{trans-4-(3-fluorophenyl)-1-[(1-methyl-1H-imidazol-4-yl)sulfonyl]pyrrolidin-3-yl}-5-(trifluoromethyl)pyridin-3-amine;
trans-N-(3,4-difluorophenyl)-4-(3-fluorophenyl)-1-[(1-methyl-1H-imidazol-4-yl)sulfonyl]pyrrolidin-3-amine;
trans-4-(2-fluorophenyl)-1-[(1-methyl-1H-imidazol-4-yl)sulfonyl]-N-phenylpyrrolidin-3-amine;
trans-4-(2-fluorophenyl)-N-(3-fluorophenyl)-1-[(1-methyl-1H-imidazol-4-yl)sulfonyl]pyrrolidin-3-amine;
trans-4-(2-fluorophenyl)-N-(4-fluorophenyl)-1-[(1-methyl-1H-imidazol-4-yl)sulfonyl]pyrrolidin-3-amine;
trans-N-(3-chlorophenyl)-4-(2-fluorophenyl)-1-[(1-methyl-1H-imidazol-4-yl)sulfonyl]pyrrolidin-3-amine;
trans-N-(4-chlorophenyl)-4-(2-fluorophenyl)-1-[(1-methyl-1H-imidazol-4-yl)sulfonyl]pyrrolidin-3-amine;
N-{trans-4-(2-fluorophenyl)-1-[(1-methyl-1H-imidazol-4-yl)sulfonyl]pyrrolidin-3-yl}-6-(trifluoromethyl)pyridin-2-amine;
N-{trans-4-(2-fluorophenyl)-1-[(1-methyl-1H-imidazol-4-yl)sulfonyl]pyrrolidin-3-yl}-2-(trifluoromethyl)pyridin-4-amine;
4-{[3-(2-bromobenzyl)-4-(2-bromophenyl)pyrrolidin-1-yl]sulfonyl}-1-methyl-1H-imidazole;
{trans-4-(4-fluorophenyl)-1-[(1-methyl-1H-imidazol-4-yl)sulfonyl]pyrrolidin-3-yl}methanol;
N-{trans-1-[(1-methyl-1H-imidazol-4-yl)sulfonyl]-4-phenylpyrrolidin-3-yl}pyridin-2-amine;
N-{trans-1-[(1-methyl-1H-imidazol-4-yl)sulfonyl]-4-phenylpyrrolidin-3-yl}pyridin-3-amine;
N-{trans-1-[(1-methyl-1H-imidazol-4-yl)sulfonyl]-4-phenylpyrrolidin-3-yl}pyridin-4-amine;
N-{trans-1-[(1-methyl-1H-imidazol-4-yl)sulfonyl]-4-phenylpyrrolidin-3-yl}-6-(trifluoromethyl)pyridin-2-amine;
N-{trans-1-[(1-methyl-1H-imidazol-4-yl)sulfonyl]-4-phenylpyrrolidin-3-yl}-2-(trifluoromethyl)pyridin-4-amine;
N-{trans-1-[(1-methyl-1H-imidazol-4-yl)sulfonyl]-4-phenylpyrrolidin-3-yl}-4-(trifluoromethyl)pyridin-2-amine;
N-{trans-1-[(1-methyl-1H-imidazol-4-yl)sulfonyl]-4-phenylpyrrolidin-3-yl}-5-(trifluoromethyl)pyridin-3-amine;
trans-N-[4-fluoro-3-(trifluoromethyl)phenyl]-1-[(1-methyl-1H-imidazol-4-yl)sulfonyl]-4-phenylpyrrolidin-3-amine;
trans-N-(3-chloro-4-fluorophenyl)-1-[(1-methyl-1H-imidazol-4-yl)sulfonyl]-4-phenylpyrrolidin-3-amine;
trans-N-(3,4-difluorophenyl)-1-[(1-methyl-1H-imidazol-4-yl)sulfonyl]-4-phenylpyrrolidin-3-amine;
trans-N-[4-chloro-3-(trifluoromethyl)phenyl]-1-[(1-methyl-1H-imidazol-4-yl)sulfonyl]-4-phenylpyrrolidin-3-amine;
trans-N-(3,4-dichlorophenyl)-1-[(1-methyl-1H-imidazol-4-yl)sulfonyl]-4-phenylpyrrolidin-3-amine;
trans-N-(3-chloro-4,5-difluorophenyl)-1-[(1-methyl-1H-imidazol-4-yl)sulfonyl]-4-phenylpyrrolidin-3-amine;
trans-1-[(1-methyl-1H-imidazol-4-yl)sulfonyl]-4-phenyl-N-[3-(propan-2-yl)phenyl]pyrrolidin-3-amine;
trans-N-(3-tert-butylphenyl)-1-[(1-methyl-1H-imidazol-4-yl)sulfonyl]-4-phenylpyrrolidin-3-amine;
trans-N-[2-fluoro-3-(trifluoromethyl)phenyl]-1-[(1-methyl-1H-imidazol-4-yl)sulfonyl]-4-phenylpyrrolidin-3-amine;
trans-N-[3-fluoro-5-(trifluoromethyl)phenyl]-1-[(1-methyl-1H-imidazol-4-yl)sulfonyl]-4-phenylpyrrolidin-3-amine;
trans-N-[2-fluoro-5-(trifluoromethyl)phenyl]-1-[(1-methyl-1H-imidazol-4-yl)sulfonyl]-4-phenylpyrrolidin-3-amine;
trans-N-(3-chloro-5-fluorophenyl)-1-[(1-methyl-1H-imidazol-4-yl)sulfonyl]-4-phenylpyrrolidin-3-amine;
trans-1-[(1-methyl-1H-imidazol-4-yl)sulfonyl]-4-phenyl-N-[3-(trifluoromethoxy)phenyl]pyrrolidin-3-amine;
N-{trans-1-[(1-methyl-1H-imidazol-4-yl)sulfonyl]-4-phenylpyrrolidin-3-yl}-4-(trifluoromethyl)pyrimidin-2-amine;
N-{trans-1-[(1-methyl-1H-imidazol-4-yl)sulfonyl]-4-phenylpyrrolidin-3-yl}quinolin-7-amine;
N-{trans-1-[(1-methyl-1H-imidazol-4-yl)sulfonyl]-4-phenylpyrrolidin-3-yl}quinolin-6-amine;
N-{trans-1-[(1-methyl-1H-imidazol-4-yl)sulfonyl]-4-phenylpyrrolidin-3-yl}isoquinolin-6-amine;
trans-N-(2,3-difluorophenyl)-1-[(1-methyl-1H-imidazol-4-yl)sulfonyl]-4-phenylpyrrolidin-3-amine;
trans-N-(2,5-difluorophenyl)-1-[(1-methyl-1H-imidazol-4-yl)sulfonyl]-4-phenylpyrrolidin-3-amine;
trans-N-(2,4-difluorophenyl)-1-[(1-methyl-1H-imidazol-4-yl)sulfonyl]-4-phenylpyrrolidin-3-amine;
trans-1-[(1-methyl-1H-imidazol-4-yl)sulfonyl]-4-phenyl-N-(2,3,4-trifluorophenyl)pyrrolidin-3-amine;
6-fluoro-N-{trans-1-[(1-methyl-1H-imidazol-4-yl)sulfonyl]-4-phenylpyrrolidin-3-yl}pyridin-2-amine;
2-fluoro-N-{trans-1-[(1-methyl-1H-imidazol-4-yl)sulfonyl]-4-phenylpyrrolidin-3-yl}pyridin-4-amine;
5-fluoro-N-{trans-1-[(1-methyl-1H-imidazol-4-yl)sulfonyl]-4-phenylpyrrolidin-3-yl}pyridin-3-amine;
6-fluoro-N-{trans-4-(4-fluorophenyl)-1-[(1-methyl-1H-imidazol-4-yl)sulfonyl]pyrrolidin-3-yl}pyridin-2-amine;
2-fluoro-N-{trans-4-(4-fluorophenyl)-1-[(1-methyl-1H-imidazol-4-yl)sulfonyl]pyrrolidin-3-yl}pyridin-4-amine;
N-{trans-1-[(1-methyl-1H-imidazol-4-yl)sulfonyl]-4-phenylpyrrolidin-3-yl}-6-(trifluoromethyl)pyrimidin-4-amine;
N-{trans-1-[(1-methyl-1H-imidazol-4-yl)sulfonyl]-4-phenylpyrrolidin-3-yl}isoquinolin-7-amine;
trans-4-(4-fluorophenyl)-1-[(1-methyl-1H-imidazol-4-yl)sulfonyl]-N-phenylpyrrolidine-3-carboxamide;
trans-4-(4-fluorophenyl)-1-[(1-methyl-1H-imidazol-4-yl)sulfonyl]-N-[4-(trifluoromethyl)phenyl]pyrrolidine-3-carboxamide;
trans-N-(3,5-dichlorophenyl)-4-(4-fluorophenyl)-1-[(1-methyl-1H-imidazol-4-yl)sulfonyl]pyrrolidine-3-carboxamide;
4-{[trans-3-(4-fluorophenyl)-4-(phenoxymethyl)pyrrolidin-1-yl]sulfonyl}-1-methyl-1H-imidazole;
4-{[trans-3-[(2,4-dichlorophenoxy)methyl]-4-(4-fluorophenyl)pyrrolidin-1-yl]sulfonyl}-1-methyl-1H-imidazole;
4-{[trans-3-(4-fluorophenyl)-4-{[3-(trifluoromethoxy)phenoxy]methyl}pyrrolidin-1-yl]sulfonyl}-1-methyl-1H-imidazole;
4-{[trans-3-[(3-chlorophenoxy)methyl]-4-(4-fluorophenyl)pyrrolidin-1-yl]sulfonyl}-1-methyl-1H-imidazole;
4-{[trans-3-{[4-chloro-3-(trifluoromethyl)phenoxy]methyl}-4-(4-fluorophenyl)pyrrolidin-1-yl]sulfonyl}-1-methyl-1H-imidazole;
4-{[trans-3-{[2-chloro-3-(trifluoromethyl)phenoxy]methyl}-4-(4-fluorophenyl)pyrrolidin-1-yl]sulfonyl}-1-methyl-1H-imidazole;
4-{[trans-3-[(3-fluorophenoxy)methyl]-4-(4-fluorophenyl)pyrrolidin-1-yl]sulfonyl}-1-methyl-1H-imidazole;
trans-4-(2-chlorophenyl)-N-(3-fluorophenyl)-1-[(1-methyl-1H-imidazol-4-yl)sulfonyl]pyrrolidin-3-amine;
trans-4-(2-chlorophenyl)-N-(4-fluorophenyl)-1-[(1-methyl-1H-imidazol-4-yl)sulfonyl]pyrrolidin-3-amine;
trans-4-(2-chlorophenyl)-N-(4-chlorophenyl)-1-[(1-methyl-1H-imidazol-4-yl)sulfonyl]pyrrolidin-3-amine;
trans-4-(2-chlorophenyl)-1-[(1-methyl-1H-imidazol-4-yl)sulfonyl]-N-[3-(trifluoromethyl)phenyl]pyrrolidin-3-amine;
trans-4-(2-chlorophenyl)-1-[(1-methyl-1H-imidazol-4-yl)sulfonyl]-N-[4-(trifluoromethyl)phenyl]pyrrolidin-3-amine;
trans-N-(3-chloro-4-fluorophenyl)-4-(2-chlorophenyl)-1-[(1-methyl-1H-imidazol-4-yl)sulfonyl]pyrrolidin-3-amine;
trans-4-(2-chlorophenyl)-N-[4-fluoro-3-(trifluoromethyl)phenyl]-1-[(1-methyl-1H-imidazol-4-yl)sulfonyl]pyrrolidin-3-amine;
N-{trans-4-(2-chlorophenyl)-1-[(1-methyl-1H-imidazol-4-yl)sulfonyl]pyrrolidin-3-yl}-2-(trifluoromethyl)pyridin-4-amine;
trans-N-(2-chlorobenzyl)-4-(4-fluorophenyl)-1-[(1-methyl-1H-imidazol-4-yl)sulfonyl]pyrrolidin-3-amine;
trans-N-(3-chlorobenzyl)-4-(4-fluorophenyl)-1-[(1-methyl-1H-imidazol-4-yl)sulfonyl]pyrrolidin-3-amine;
trans-N-(4-chlorobenzyl)-4-(4-fluorophenyl)-1-[(1-methyl-1H-imidazol-4-yl)sulfonyl]pyrrolidin-3-amine;
trans-N-(2-fluorobenzyl)-4-(4-fluorophenyl)-1-[(1-methyl-1H-imidazol-4-yl)sulfonyl]pyrrolidin-3-amine;
trans-N-(3-fluorobenzyl)-4-(4-fluorophenyl)-1-[(1-methyl-1H-imidazol-4-yl)sulfonyl]pyrrolidin-3-amine;
trans-4-(4-fluorophenyl)-1-[(1-methyl-1H-imidazol-4-yl)sulfonyl]-N-[2-(trifluoromethyl)benzyl]pyrrolidin-3-amine;
trans-4-(4-fluorophenyl)-1-[(1-methyl-1H-imidazol-4-yl)sulfonyl]-N-[3-(trifluoromethyl)benzyl]pyrrolidin-3-amine;
trans-4-(4-fluorophenyl)-1-[(1-methyl-1H-imidazol-4-yl)sulfonyl]-N-[4-(trifluoromethyl)benzyl]pyrrolidin-3-amine;
trans-4-(4-fluorophenyl)-1-[(1-methyl-1H-imidazol-4-yl)sulfonyl]-N-(pyridin-2-ylmethyl)pyrrolidin-3-amine;
trans-4-(4-fluorophenyl)-1-[(1-methyl-1H-imidazol-4-yl)sulfonyl]-N-(pyridin-3-ylmethyl)pyrrolidin-3-amine;
trans-4-(4-fluorophenyl)-1-[(1-methyl-1H-imidazol-4-yl)sulfonyl]-N-(pyridin-4-ylmethyl)pyrrolidin-3-amine;
{trans-4-(4-fluorophenyl)-1-[(1-methyl-1H-imidazol-4-yl)sulfonyl]pyrrolidin-3-yl} (phenyl)methanone;
trans-N-[2-chloro-3-(trifluoromethyl)phenyl]-4-(4-fluorophenyl)-1-[(1-methyl-1H-imidazol-4-yl)sulfonyl]pyrrolidine-3-carboxamide;
trans-4-(4-fluorophenyl)-1-[(1-methyl-1H-imidazol-4-yl)sulfonyl]-N-[3-(trifluoromethoxy)phenyl]pyrrolidine-3-carboxamide;
trans-4-(4-fluorophenyl)-N-[4-fluoro-3-(trifluoromethyl)phenyl]-1-[(1-methyl-1H-imidazol-4-yl)sulfonyl]pyrrolidine-3-carboxamide;
trans-N-(3-chloro-4-fluorophenyl)-4-(4-fluorophenyl)-1-[(1-methyl-1H-imidazol-4-yl)sulfonyl]pyrrolidine-3-carboxamide;
trans-N-[3-chloro-4-(trifluoromethyl)phenyl]-4-(4-fluorophenyl)-1-[(1-methyl-1H-imidazol-4-yl)sulfonyl]pyrrolidine-3-carboxamide;
1-methyl-4-[(3-phenoxy-4-phenylpyrrolidin-1-yl)sulfonyl]-1H-imidazole;
trans-4-(2-chlorophenyl)-N-(3-chlorophenyl)-1-[(1-methyl-1H-imidazol-4-yl)sulfonyl]pyrrolidin-3-amine;
trans-N-(2,4-dichlorobenzyl)-4-(4-fluorophenyl)-1-[(1-methyl-1H-imidazol-4-yl)sulfonyl]pyrrolidin-3-amine;
trans-N-(2,4-dichlorobenzyl)-1-[(1-methyl-1H-imidazol-4-yl)sulfonyl]-4-phenylpyrrolidin-3-amine;
trans-N-[2-chloro-4-(trifluoromethyl)benzyl]-1-[(1-methyl-1H-imidazol-4-yl)sulfonyl]-4-phenylpyrrolidin-3-amine;
trans-N-(2,4-dichlorobenzyl)-4-(2-fluorophenyl)-1-[(1-methyl-1H-imidazol-4-yl)sulfonyl]pyrrolidin-3-amine;
trans-N-[2-chloro-4-(trifluoromethyl)benzyl]-4-(2-fluorophenyl)-1-[(1-methyl-1H-imidazol-4-yl)sulfonyl]pyrrolidin-3-amine;
trans-4-(2-chlorophenyl)-N-(2,4-dichlorobenzyl)-1-[(1-methyl-1H-imidazol-4-yl)sulfonyl]pyrrolidin-3-amine;
trans-4-(2-chlorophenyl)-N-[2-chloro-4-(trifluoromethyl)benzyl]-1-[(1-methyl-1H-imidazol-4-yl)sulfonyl]pyrrolidin-3-amine;
trans-N-(2,4-dichlorobenzyl)-1-[(1-methyl-1H-imidazol-4-yl)sulfonyl]-4-(pyridin-3-yl)pyrrolidin-3-amine;
N-{trans-4-(4-fluorophenyl)-1-[(1-methyl-1H-imidazol-4-yl)sulfonyl]pyrrolidin-3-yl}-4-(trifluoromethyl)pyridin-2-amine;
N-{trans-4-(4-fluorophenyl)-1-[(1-methyl-1H-imidazol-4-yl)sulfonyl]pyrrolidin-3-yl}-2-(trifluoromethyl)pyridin-4-amine;
trans-N-benzyl-4-(4-fluorophenyl)-1-[(1-methyl-1H-imidazol-4-yl)sulfonyl]pyrrolidine-3-carboxamide;
N-({trans-4-(4-fluorophenyl)-1-[(1-methyl-1H-imidazol-4-yl)sulfonyl]pyrrolidin-3-yl}methyl) aniline;
N-({trans-4-(4-fluorophenyl)-1-[(1-methyl-1H-imidazol-4-yl)sulfonyl]pyrrolidin-3-yl}methyl)-4-(trifluoromethyl)aniline;
3,5-dichloro-N-({trans-4-(4-fluorophenyl)-1-[(1-methyl-1H-imidazol-4-yl)sulfonyl]pyrrolidin-3-yl}methyl)aniline;
4-{[trans-3-(4-fluorophenyl)-4-{[3-(trifluoromethyl)phenoxy]methyl}pyrrolidin-1-yl]sulfonyl}-1-methyl-1H-imidazole;
4-{[trans-3-{[2-chloro-4-(trifluoromethyl)phenoxy]methyl}-4-(4-fluorophenyl)pyrrolidin-1-yl]sulfonyl}-1-methyl-1H-imidazole;
4-{[trans-3-(4-fluorophenyl)-4-{[4-fluoro-3-(trifluoromethyl)phenoxy]methyl}pyrrolidin-1-yl]sulfonyl}-1-methyl-1H-imidazole;
4-({trans-3-(4-fluorophenyl)-4-[(3-methylphenoxy)methyl]pyrrolidin-1-yl}sulfonyl)-1-methyl-1H-imidazole;
3-chlorophenyl){trans-4-(4-fluorophenyl)-1-[(1-methyl-1H-imidazol-4-yl)sulfonyl]pyrrolidin-3-yl}methanone;
trans-N-[4-fluoro-3-(trifluoromethoxy)phenyl]-1-[(1-methyl-1H-imidazol-4-yl)sulfonyl]-4-phenylpyrrolidin-3-amine;
trans-N-[4-chloro-3-(trifluoromethoxy)phenyl]-1-[(1-methyl-1H-imidazol-4-yl)sulfonyl]-4-phenylpyrrolidin-3-amine;
{trans-4-(4-fluorophenyl)-1-[(1-methyl-1H-imidazol-4-yl)sulfonyl]pyrrolidin-3-yl}(3-methylphenyl)methanone;
(3-fluorophenyl){(trans-4-(4-fluorophenyl)-1-[(1-methyl-1H-imidazol-4-yl)sulfonyl]pyrrolidin-3-yl}methanone;
{trans-4-(4-fluorophenyl)-1-[(1-methyl-1H-imidazol-4-yl)sulfonyl]pyrrolidin-3-yl}[3-(trifluoromethyl)phenyl]methanone;
(4-fluorophenyl){trans-4-(4-fluorophenyl)-1-[(1-methyl-1H-imidazol-4-yl)sulfonyl]pyrrolidin-3-yl}methanone;
trans-N-(3-chlorophenyl)-4-(4-fluorophenyl)-1-[(1-methyl-1H-imidazol-4-yl)sulfonyl]pyrrolidine-3-carboxamide;
trans-4-(4-fluorophenyl)-N-[4-fluoro-3-(trifluoromethoxy)phenyl]-1-[(1-methyl-1H-imidazol-4-yl)sulfonyl]pyrrolidin-3-amine;
trans-N-[4-chloro-3-(trifluoromethoxy)phenyl]-4-(4-fluorophenyl)-1-[(1-methyl-1H-imidazol-4-yl)sulfonyl]pyrrolidin-3-amine;
trans-N-(2,4-dichlorobenzyl)-1-[(1-methyl-1H-imidazol-4-yl)sulfonyl]-4-(pyridin-2-yl)pyrrolidin-3-amine;
trans-1-[(1-methyl-1H-imidazol-4-yl)sulfonyl]-4-(pyridin-3-yl)-N-[3-(trifluoromethyl)phenyl]pyrrolidin-3-amine;
4-{[3-(3-chlorobenzyl)-4-cyclopropylpyrrolidin-1-yl]sulfonyl}-1-methyl-1H-imidazole;
N-[3-(difluoromethyl)-4-fluorophenyl]-4-(4-fluorophenyl)-1-[(1-methyl-1H-imidazol-4-yl)sulfonyl]pyrrolidin-3-amine;
3-chloro-N-({trans-4-(4-fluorophenyl)-1-[(1-methyl-1H-imidazol-4-yl)sulfonyl]pyrrolidin-3-yl}methyl)-4-(trifluoromethyl)aniline;
N-({trans-4-(4-fluorophenyl)-1-[(1-methyl-1H-imidazol-4-yl)sulfonyl]pyrrolidin-3-yl}methyl)-3-(trifluoromethoxy)aniline;
4-fluoro-N-({trans-4-(4-fluorophenyl)-1-[(1-methyl-1H-imidazol-4-yl)sulfonyl]pyrrolidin-3-yl}methyl)-3-(trifluoromethyl)aniline;
3-chloro-4-fluoro-N-({trans-4-(4-fluorophenyl)-1-[(1-methyl-1H-imidazol-4-yl)sulfonyl]pyrrolidin-3-yl}methyl)aniline;
N-benzyl-1-{trans-4-(4-fluorophenyl)-1-[(1-methyl-1H-imidazol-4-yl)sulfonyl]pyrrolidin-3-yl}methanamine;
trans-4-(4-fluorophenyl)-1-[(1-methyl-1H-imidazol-4-yl)sulfonyl]-N-(3-methylphenyl)pyrrolidine-3-carboxamide;
trans-4-(4-fluorophenyl)-1-[(1-methyl-1H-imidazol-4-yl)sulfonyl]-N-[3-(trifluoromethyl)phenyl]pyrrolidine-3-carboxamide;
4-{[trans-3-[(3-chloro-4-fluorophenoxy)methyl]-4-(4-fluorophenyl)pyrrolidin-1-yl]sulfonyl}-1-methyl-1H-imidazole;
(4-Chloro-3-fluorophenyl){trans-4-(4-fluorophenyl)-1-[(1-methyl-1H-imidazol-4-yl)sulfonyl]pyrrolidin-3-yl}methanone;
trans-N-[2-chloro-4-(trifluoromethyl)benzyl]-1-[(1-methyl-1H-imidazol-4-yl)sulfonyl]-4-(pyridin-2-yl)pyrrolidin-3-amine;
(3S,4R)-N-[2-chloro-4-(trifluoromethyl)benzyl]-4-(4-fluorophenyl)-1-[(1-methyl-1H-imidazol-4-yl)sulfonyl]pyrrolidin-3-amine;
(3R,4S)-N-[2-chloro-4-(trifluoromethyl)benzyl]-4-(4-fluorophenyl)-1-[(1-methyl-1H-imidazol-4-yl)sulfonyl]pyrrolidin-3-amine;
(3S,4R)-4-(4-fluorophenyl)-1-[(1-methyl-1H-imidazol-4-yl)sulfonyl]-N-[3-(trifluoromethoxy)phenyl]pyrrolidin-3-amine;
(3S,4R)-N,4-bis(4-fluorophenyl)-1-[(1-methyl-1H-imidazol-4-yl)sulfonyl]pyrrolidin-3-amine;
(3S,4R)-N-(3-chlorophenyl)-4-(4-fluorophenyl)-1-[(1-methyl-1H-imidazol-4-yl)sulfonyl]pyrrolidin-3-amine;
(3S,4R)-4-(4-fluorophenyl)-1-[(1-methyl-1H-imidazol-4-yl)sulfonyl]-N-[3-(trifluoromethyl)phenyl]pyrrolidin-3-amine;
(3R,4S)-4-(4-fluorophenyl)-1-[(1-methyl-1H-imidazol-4-yl)sulfonyl]-N-[3-(trifluoromethyl)phenyl]pyrrolidin-3-amine;
(3R,4S)-N-(3-chlorophenyl)-4-(4-fluorophenyl)-1-[(1-methyl-1H-imidazol-4-yl)sulfonyl]pyrrolidin-3-amine;
(3R,4S)-4-(4-fluorophenyl)-1-[(1-methyl-1H-imidazol-4-yl)sulfonyl]-N-[3-(trifluoromethoxy)phenyl]pyrrolidin-3-amine;
(3R,4S)-N,4-bis(4-fluorophenyl)-1-[(1-methyl-1H-imidazol-4-yl)sulfonyl]pyrrolidin-3-amine;
3-(3-chlorobenzyl)-1-[(1-methyl-1H-imidazol-4-yl)sulfonyl]-4-phenylpyrrolidin-2-one;
2-Chloro-N-({trans-4-(4-fluorophenyl)-1-[(1-methyl-1H-imidazol-4-yl)sulfonyl]pyrrolidin-3-yl}methyl)-4-(trifluoromethyl)aniline;
3-Chloro-N-({trans-4-(4-fluorophenyl)-1-[(1-methyl-1H-imidazol-4-yl)sulfonyl]pyrrolidin-3-yl}methyl)aniline;
N-({trans-4-(4-fluorophenyl)-1-[(1-methyl-1H-imidazol-4-yl)sulfonyl]pyrrolidin-3-yl}methyl)-3-methylaniline;
N-({trans-4-(4-fluorophenyl)-1-[(1-methyl-1H-imidazol-4-yl)sulfonyl]pyrrolidin-3-yl}methyl)-3-(trifluoromethyl)aniline;
4-({trans-4-(4-fluorophenyl)-1-[(1-methyl-1H-imidazol-4-yl)sulfonyl]pyrrolidin-3-yl}methoxy)-6-(trifluoromethyl)pyrimidine;
trans-4-cyclopropyl-1-[(1-methyl-1H-imidazol-4-yl)sulfonyl]-N-[3-(trifluoromethyl)phenyl]pyrrolidin-3-amine;
trans-N-(3-chlorophenyl)-4-cyclopropyl-1-[(1-methyl-1H-imidazol-4-yl)sulfonyl]pyrrolidin-3-amine;
4-{[3-(2-chlorophenyl)-4-(4-fluorophenyl)pyrrolidin-1-yl]sulfonyl}-1-methyl-1H-imidazole;
4-{[3-(4-chlorophenyl)-4-(4-fluorophenyl)pyrrolidin-1-yl]sulfonyl}-1-methyl-1H-imidazole;
2-chloro-4-({trans-4-(4-fluorophenyl)-1-[(1-methyl-1H-imidazol-4-yl)sulfonyl]pyrrolidin-3-yl}amino)benzonitrile;
2-fluoro-4-({trans-4-(4-fluorophenyl)-1-[(1-methyl-1H-imidazol-4-yl)sulfonyl]pyrrolidin-3-yl}amino)benzonitrile;
3-(3-chlorobenzyl)-1-[(1-methyl-1H-imidazol-4-yl)sulfonyl]-4-phenylimidazolidin-2-one;
trans-1-[(1-methyl-1H-imidazol-4-yl)sulfonyl]-4-(pyridin-2-yl)-N-[3-(trifluoromethyl)phenyl]pyrrolidin-3-amine;
trans-N-(3-chlorophenyl)-1-[(1-methyl-1H-imidazol-4-yl)sulfonyl]-4-(pyridin-2-yl)pyrrolidin-3-amine;
2-({trans-4-(4-fluorophenyl)-1-[(1-methyl-1H-imidazol-4-yl)sulfonyl]pyrrolidin-3-yl}methoxy)-4-(trifluoromethyl)pyridine;
trans-N-(3-cyanophenyl)-4-(4-fluorophenyl)-1-[(1-methyl-1H-imidazol-4-yl)sulfonyl]pyrrolidine-3-carboxamide;
4-{[trans-3-[(4-fluorophenoxy)methyl]-4-(4-fluorophenyl)pyrrolidin-1-yl]sulfonyl}-1-methyl-1H-imidazole;
4-(4-fluorophenyl)-1-[(1-methyl-1H-imidazol-4-yl)sulfonyl]-N-[3-(propan-2-yloxy)phenyl]pyrrolidin-3-amine;
N-{4-(4-fluorophenyl)-1-[(1-methyl-1H-imidazol-4-yl)sulfonyl]pyrrolidin-3-yl}pyridazin-3-amine;
N-{(3R,4S)-4-(4-fluorophenyl)-1-[(1-methyl-1H-imidazol-4-yl)sulfonyl]pyrrolidin-3-yl}-6-(trifluoromethyl)pyridin-2-amine;
(3R,4S)-N-(3-chloro-4-fluorophenyl)-4-(4-fluorophenyl)-1-[(1-methyl-1H-imidazol-4-yl)sulfonyl]pyrrolidin-3-amine;
(3R,4S)-N-(3-fluorophenyl)-4-(4-fluorophenyl)-1-[(1-methyl-1H-imidazol-4-yl)sulfonyl]pyrrolidin-3-amine;
trans-N-(4-chlorobenzyl)-3-methyl-1-[(1-methyl-1H-imidazol-4-yl)sulfonyl]-4-phenylpyrrolidin-3-amine;
trans-3-methyl-1-[(1-methyl-1H-imidazol-4-yl)sulfonyl]-4-phenyl-N-[4-(trifluoromethyl)benzyl]pyrrolidin-3-amine;
trans-N-[2-chloro-4-(trifluoromethyl)benzyl]-3-methyl-1-[(1-methyl-1H-imidazol-4-yl)sulfonyl]-4-phenylpyrrolidin-3-amine;
(3,4-Difluorophenyl){trans-4-(4-fluorophenyl)-1-[(1-methyl-1H-imidazol-4-yl)sulfonyl]pyrrolidin-3-yl}methanone;
trans-N,4-bis(4-fluorophenyl)-1-[(1-methyl-1H-imidazol-4-yl)sulfonyl]pyrrolidine-3-carboxamide;
(3,4-Dichlorophenyl){trans-4-(4-fluorophenyl)-1-[(1-methyl-1H-imidazol-4-yl)sulfonyl]pyrrolidin-3-yl} methanone;
(3,5-Dichlorophenyl){trans-4-(4-fluorophenyl)-1-[(1-methyl-1H-imidazol-4-yl)sulfonyl]pyrrolidin-3-yl} methanone;
(3-Chloro-5-fluorophenyl){trans-4-(4-fluorophenyl)-1-[(1-methyl-1H-imidazol-4-yl)sulfonyl]pyrrolidin-3-yl}methanone;
{trans-4-(4-fluorophenyl)-1-[(1-methyl-1H-imidazol-4-yl)sulfonyl]pyrrolidin-3-yl}[3-(trifluoromethoxy)phenyl] methanone;
{trans-4-(4-fluorophenyl)-1-[(1-methyl-1H-imidazol-4-yl)sulfonyl]pyrrolidin-3-yl}[4-(trifluoromethoxy)phenyl] methanone;
3-[({trans-4-(4-fluorophenyl)-1-[(1-methyl-1H-imidazol-4-yl)sulfonyl]pyrrolidin-3-yl}methyl)amino]benzamide;
3-(Aminomethyl)-N-({trans-4-(4-fluorophenyl)-1-[(1-methyl-1H-imidazol-4-yl)sulfonyl]pyrrolidin-3-yl}methyl)aniline;
4-({trans-4-(4-fluorophenyl)-1-[(1-methyl-1H-imidazol-4-yl)sulfonyl]pyrrolidin-3-yl}methoxy)-2-(trifluoromethyl)pyridine;
4-fluoro-N-({trans-4-(4-fluorophenyl)-1-[(1-methyl-1H-imidazol-4-yl)sulfonyl]pyrrolidin-3-yl}methyl)aniline;
N-{(3R,4S)-4-(4-fluorophenyl)-1-[(1-methyl-1H-imidazol-4-yl)sulfonyl]pyrrolidin-3-yl}-4-(trifluoromethyl)pyridin-2-amine;
trans-4-(4-fluorophenyl)-1-[(1-methyl-1H-imidazol-4-yl)sulfonyl]-N-[4-(trifluoromethyl)pyridin-2-yl]pyrrolidine-3-carboxamide;
trans-4-(4-fluorophenyl)-N-[4-fluoro-3-(trifluoromethoxy)phenyl]-1-[(1-methyl-1H-imidazol-4-yl)sulfonyl]pyrrolidine-3-carboxamide;
trans-N-[4-chloro-3-(trifluoromethoxy)phenyl]-4-(4-fluorophenyl)-1-[(1-methyl-1H-imidazol-4-yl)sulfonyl]pyrrolidine-3-carboxamide;
N-[4-chloro-2-({trans-4-(4-fluorophenyl)-1-[(1-methyl-1H-imidazol-4-yl)sulfonyl]pyrrolidin-3-yl}amino)phenyl]acetamide;
trans-4-[(3-chlorophenyl)amino]-1-[(1-methyl-1H-imidazol-4-yl)sulfonyl]pyrrolidin-3-ol;
N-[trans-1-[(1-methyl-1H-imidazol-4-yl)sulfonyl]-4-(pyridin-2-yl)pyrrolidin-3-yl]-1-(trifluoromethyl)cyclopropanecarboxamide;
N-{(3R,4S)-4-(4-fluorophenyl)-1-[(1-methyl-1H-imidazol-4-yl)sulfonyl]pyrrolidin-3-yl}-1-(trifluoromethyl)cyclopropanecarboxamide;
4-fluoro-N-({trans-4-(4-fluorophenyl)-1-[(1-methyl-1H-imidazol-4-yl)sulfonyl]pyrrolidin-3-yl}methyl)-3-(trifluoromethoxy)aniline;
4-chloro-N-({trans-4-(4-fluorophenyl)-1-[(1-methyl-1H-imidazol-4-yl)sulfonyl]pyrrolidin-3-yl}methyl)-3-(trifluoromethoxy)aniline;
trans-N-(3-chlorophenyl)-4-(4-fluorophenoxy)-1-[(1-methyl-1H-imidazol-4-yl)sulfonyl]pyrrolidin-3-amine;
2-({trans-4-(4-fluorophenyl)-1-[(1-methyl-1H-imidazol-4-yl)sulfonyl]pyrrolidin-3-yl}oxy)-4-(trifluoromethyl)pyridine;
3-({(3R,4S)-4-(4-fluorophenyl)-1-[(1-methyl-1H-imidazol-4-yl)sulfonyl]pyrrolidin-3-yl}amino)benzonitrile;
6-chloro-2-[trans-1-[(1-methyl-1H-imidazol-4-yl)sulfonyl]-4-(pyridin-2-yl)pyrrolidin-3-yl]-2,3-dihydro-1H-isoindol-1-one;
3-benzyl-4-(4-fluorophenyl)-1-[(1-methyl-1H-imidazol-4-yl)sulfonyl]pyrrolidin-3-ol;
N-{trans-4-(4-fluorophenyl)-1-[(1-methyl-1H-imidazol-4-yl)sulfonyl]pyrrolidin-3-yl}-5-(trifluoromethyl)pyridazin-3-amine;
N-{4-(4-fluorophenyl)-1-[(1-methyl-1H-imidazol-4-yl)sulfonyl]pyrrolidin-3-yl}-4-(trifluoromethyl)pyridazin-3-amine;
1-methyl-4-{[trans-3-phenyl-4-(phenylsulfonyl)pyrrolidin-1-yl]sulfonyl}-1H-imidazole;
(3R,4S)-4-(4-fluorophenyl)-1-[(1-methyl-1H-imidazol-4-yl)sulfonyl]-N-{[1-(trifluoromethyl)cyclopropyl]methyl}pyrrolidin-3-amine;
(3S,4R)-N-(3-chloro-4-fluorophenyl)-4-(4-fluorophenyl)-1-[(1-methyl-1H-imidazol-4-yl)sulfonyl]pyrrolidin-3-amine;
N-{trans-3-methyl-1-[(1-methyl-1H-imidazol-4-yl)sulfonyl]-4-phenylpyrrolidin-3-yl}-4-(trifluoromethyl)pyridin-2-amine;
3-({(3S,4R)-4-(4-fluorophenyl)-1-[(1-methyl-1H-imidazol-4-yl)sulfonyl]pyrrolidin-3-yl}amino)benzonitrile;
N-{(3R,4S)-4-(4-fluorophenyl)-1-[(1-methyl-1H-imidazol-4-yl)sulfonyl]pyrrolidin-3-yl}-6-(trifluoromethyl)pyrimidin-4-amine;
(3R,4S)-4-(4-fluorophenyl)-1-[(1-methyl-1H-imidazol-4-yl)sulfonyl]-N-{[5-(trifluoromethyl)furan-2-yl]methyl}pyrrolidin-3-amine;
1,1,1-trifluoro-2-[3-({(3R,4S)-4-(4-fluorophenyl)-1-[(1-methyl-1H-imidazol-4-yl)sulfonyl]pyrrolidin-3-yl}amino)phenyl]propan-2-ol;
(3R,4S)-N-[(5-chlorothiophen-2-yl)methyl]-4-(4-fluorophenyl)-1-[(1-methyl-1H-imidazol-4-yl)sulfonyl]pyrrolidin-3-amine;
(3R,4S)-N-[2-chloro-4-(trifluoromethyl)benzyl]-1-[(1-methyl-1H-imidazol-4-yl)sulfonyl]-4-phenylpyrrolidin-3-amine;
(3S,4R)-N-[2-chloro-4-(trifluoromethyl)benzyl]-1-[(1-methyl-1H-imidazol-4-yl)sulfonyl]-4-phenylpyrrolidin-3-amine;
N-{(3R,4S)-1-[(1-methyl-1H-imidazol-4-yl)sulfonyl]-4-phenylpyrrolidin-3-yl}-4-(trifluoromethyl)pyridin-2-amine;
(3R,4S)-1-[(1-methyl-1H-imidazol-4-yl)sulfonyl]-4-phenyl-N-[3-(trifluoromethyl)phenyl]pyrrolidin-3-amine;
(3R,4S)-1-[(1-methyl-1H-imidazol-4-yl)sulfonyl]-4-phenyl-N-[4-(trifluoromethyl)benzyl]pyrrolidin-3-amine;
1,1,1-trifluoro-2-[3-({(3R,4S)-1-[(1-methyl-1H-imidazol-4-yl)sulfonyl]-4-phenylpyrrolidin-3-yl}amino)phenyl]propan-2-ol;
trans-4-(benzyloxy)-N-(3-chlorophenyl)-1-[(1-methyl-1H-imidazol-4-yl)sulfonyl]pyrrolidin-3-amine;
trans-1-[(1-methyl-1H-imidazol-4-yl)sulfonyl]-4-phenyl-3- {[4-(trifluoromethyl)benzyl]amino}pyrrolidin-3-yl]methanol;
trans-N-(3-chlorophenyl)-1-[(1-methyl-1H-imidazol-4-yl)sulfonyl]-4-(prop-2-en-1-yloxy)pyrrolidin-3-amine;
trans-N-(3-chlorophenyl)-1-[(1-methyl-1H-imidazol-4-yl)sulfonyl]-4-[(2-methylprop-2-en-1-yl)oxy]pyrrolidin-3-amine;
3-{trans-1-[(1-methyl-1H-imidazol-4-yl)sulfonyl]-4-phenylpyrrolidin-3-yl}-6-(trifluoromethyl)-3,4-dihydroquinazolin-2(1H)-one;
4-fluoro-N-{(3R,4S)-1-[(1-methyl-1H-imidazol-4-yl)sulfonyl]-4-phenylpyrrolidin-3-yl}pyridin-2-amine;
N-{(3R,4S)-1-[(1-methyl-1H-imidazol-4-yl)sulfonyl]-4-phenylpyrrolidin-3-yl}-7H-pyrrolo[2,3-d]pyrimidin-4-amine;
trans-7-[(1-methyl-1H-imidazol-4-yl)sulfonyl]-9-phenyl-1-[4-(trifluoromethyl)benzyl]-3-oxa-1,7-diazaspiro[4.4]nonan-2-one;
3,5-difluoro-N-{(3R,4S)-1-[(1-methyl-1H-imidazol-4-yl)sulfonyl]-4-phenylpyrrolidin-3-yl}-4-(trifluoromethyl)pyridin-2-amine;
(3R,4S)-1-[(1-methyl-1H-imidazol-4-yl)sulfonyl]-4-phenyl-N-{[5-(trifluoromethyl)pyridin-2-yl]methyl}pyrrolidin-3-amine;
5-cyclopropyl-N-{(3R,4S)-1-[(1-methyl-1H-imidazol-4-yl)sulfonyl]-4-phenylpyrrolidin-3-yl}pyrazin-2-amine;
5-cyclobutyl-N-1(3R,4S)-1-[(1-methyl-1H-imidazol-4-yl)sulfonyl]-4-phenylpyrrolidin-3-yl}pyrazin-2-amine;
trans-N-(3-chlorophenyl)-1-[(1-methyl-1H-imidazol-4-yl)sulfonyl]-4-(2-methylpropoxy)pyrrolidin-3-amine;
N-{(3R,4S)-1-[(1-methyl-1H-imidazol-4-yl)sulfonyl]-4-phenylpyrrolidin-3-yl}-5-(pyrrolidin-1-yl)pyrazin-2-amine;
N-{(3R,4S)-1-[(1-methyl-1H-imidazol-4-yl)sulfonyl]-4-phenylpyrrolidin-3-yl}-5-(trifluoromethyl)pyrazine-2-carboxamide;
5-chloro-N-{(3R,4S)-1-[(1-methyl-1H-imidazol-4-yl)sulfonyl]-4-phenylpyrrolidin-3-yl}pyrazine-2-carboxamide;
(3R,4S)-N-(4-chlorobenzyl)-1-[(1-methyl-1H-imidazol-4-yl)sulfonyl]-4-phenylpyrrolidin-3-amine;
(3R,4S)-N-(3-chloro-4-fluorophenyl)-1-[(1-methyl-1H-imidazol-4-yl)sulfonyl]-4-phenylpyrrolidin-3-amine;
(3R,4S)-N-(3,4-difluorophenyl)-4-(4-fluorophenyl)-1-[(1-methyl-1H-imidazol-4-yl)sulfonyl]pyrrolidin-3-amine;
6-chloro-N-{(3R,4S)-1-[(1-methyl-1H-imidazol-4-yl)sulfonyl]-4-phenylpyrrolidin-3-yl}pyridazine-3-carboxamide;
N-{(3R,4S)-1-[(1-methyl-1H-imidazol-4-yl)sulfonyl]-4-phenylpyrrolidin-3-yl}-2-(morpholin-4-yl)pyrimidin-4-amine;
4-({(3R,4S)-1-[(1-methyl-1H-imidazol-4-yl)sulfonyl]-4-phenylpyrrolidin-3-yl}amino)-2,3-dihydro-1H-isoindol-1-one;
(3R,4S)-1-[(1-methyl-1H-imidazol-4-yl)sulfonyl]-N-[3-(methylsulfonyl)phenyl]-4-phenylpyrrolidin-3-amine;
(3R,4S)-1-[(1-methyl-1H-imidazol-4-yl)sulfonyl]-4-phenyl-N-{[5-(trifluoromethyl)piperazin-2-yl]methyl}pyrrolidin-3-amine;
5-fluoro-N-{(3R,4S)-1-[(1-methyl-1H-imidazol-4-yl)sulfonyl]-4-phenylpyrrolidin-3-yl}pyrimidin-2-amine;
trans-4-cyclohexyl-1-[(1-methyl-1H-imidazol-4-yl)sulfonyl]-N-[3-(trifluoromethoxy)phenyl]pyrrolidin-3-amine;
(3R,4S)-1-[(1-methyl-1H-imidazol-4-yl)sulfonyl]-4-phenyl-N-(4,4,4-trifluorobutyl)pyrrolidin-3-amine;
(3R,4S)-N-(2-cyclopentylethyl)-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-4-phenyl-N-[(5-propylfuran-2-yl)methyl]pyrrolidin-3-amine;
(3R,4S)-1- [(1-methyl-1H-imidazol-4-yl)sulfonyl]-N-[(4-methyltetrahydro-2H-pyran-4-yl)methyl]-4-phenylpyrrolidin-3-amine;
(3R,4S)-1-[(1-methyl-1H-imidazol-4-yl)sulfonyl]-4-phenyl-N-[(4-phenyl-1,3-thiazol-5-yl)methyl]pyrrolidin-3-amine;
(3R,4S)-1-[(1-methyl-1H-imidazol-4-yl)sulfonyl]-N-[(5-methyl-1,3-thiazol-2-yl)methyl]-4-phenylpyrrolidin-3-amine;
(3R,4S)-N-[(3,5-dichloropyridin-4-yl)methyl]-1-[(1-methyl-1H-imidazol-4-yl)sulfonyl]-4-phenylpyrrolidin-3-amine;
(3R,4S)-N-(2-ethylhexyl)-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-N-[(4-methyl-1,3-thiazol-5-yl)methyl]-4-phenylpyrrolidin-3-amine;
(3R,4S)-N-[(2,4-dichloro-1,3-thiazol-5-yl)methyl]-1-[(1-methyl-1H-imidazol-4-yl)sulfonyl]-4-phenylpyrrolidin-3-amine;
(3R,4S)-N-(2,2-dimethylpropyl)-1-[(1-methyl-1H-imidazol-4-yl)sulfonyl]-4-phenylpyrrolidin-3-amine;
(3R,4S)-N-[(5-methyl-1-benzothiophen-2-yl)methyl]-1-[(1-methyl-1H-imidazol-4-yl)sulfonyl]-4-phenylpyrrolidin-3-amine;
(3R,4S)-N-[(2-bromo-1,3-thiazol-5-yl)methyl]-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-N-(2-methylpropyl)-4-phenylpyrrolidin-3-amine;
(3R,4S)-N-[(3-chloro-1-benzothiophen-2-yl)methyl]-1-[(1-methyl-1H-imidazol-4-yl)sulfonyl]-4-phenylpyrrolidin-3-amine;
(3R,4S)-N-[(2,4-dimethyl-1,3-thiazol-5-yl)methyl]-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-4-phenyl-N-(thieno[2,3-b]pyridin-2-ylmethyl)pyrrolidin-3-amine;
(3R,4S)-N-[(2-methyl-1-benzofuran-3-yl)methyl]-1-[(1-methyl-1H-imidazol-4-yl)sulfonyl]-4-phenylpyrrolidin-3-amine;
(3R,4S)-N-[(6-chloro-2-methylimidazo[1,2-a]pyridin-3-yl)methyl]-1-[(1-methyl-1H-imidazol-4-yl)sulfonyl] -4-phenylpyrrolidin-3-amine;
(3R,4S)-N-{[5-(4-fluorophenyl)pyridin-3-yl]methyl}-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-N-(2-methylpentyl)-4-phenylpyrrolidin-3-amine;
(3R,4S)-1-[(1-methyl-1H-imidazol-4-yl)sulfonyl]-4-phenyl-N-(tetrahydrofuran-3-ylmethyl)pyrrolidin-3-amine;
(3R,4S)-1-[(1-methyl-1H-imidazol-4-yl)sulfonyl]-N-pentyl-4-phenylpyrrolidin-3-amine;
(3R,4S)-N-(2-ethylbutyl)-1-[(1-methyl-1H-imidazol-4-yl)sulfonyl]-4-phenylpyrrolidin-3-amine;
(3R,4S)-N-(2,2-dimethylbutyl)-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-N-[(2-methyl-1,3-thiazol-5-yl)methyl]-4-phenylpyrrolidin-3-amine;
(3R,4S)-1-[(1-methyl-1H-imidazol-4-yl)sulfonyl]-4-phenyl-N-[2-(tetrahydro-2H-pyran-4-yl)ethyl]pyrrolidin-3-amine;
(3R,4S)-N-(1,3-benzothiazol-2-ylmethyl)-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-N-[(4-methyl-1,3-thiazol-2-yl)methyl]-4-phenylpyrrolidin-3-amine;
(3R,4S)-N-[(4,5-dimethylthiophen-2-yl)methyl]-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-4-phenyl-N-(4,5,6,7-tetrahydro-1,3-benzothiazol-2-ylmethyl)pyrrolidin-3-amine;
(3R,4S)-1-[(1-methyl-1H-imidazol-4-yl)sulfonyl]-4-phenyl-N-(1,3-thiazol-5-ylmethyl)pyrrolidin-3-amine;
(3R,4S)-1-[(1-methyl-1H-imidazol-4-yl)sulfonyl]-4-phenyl-N-[2-(2,6,6-trimethylcyclohex-1-en-1-yl)ethyl]pyrrolidin-3-amine;
(3R,4S)-1-[(1-methyl-1H-imidazol-4-yl)sulfonyl]-4-phenyl-N-{[1-(propan-2-yl)piperidin-4-yl]methyl}pyrrolidin-3-amine;
(3R,4S)-N-{[4-(4-fluorophenyl)-1,3-thiazol-2-yl]methyl}-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-N-[2-(4-methylphenyl)ethyl]-4-phenylpyrrolidin-3-amine;
(3R,4S)-1-[(1-methyl-1H-imidazol-4-yl)sulfonyl]-N-[(5-methylthiophen-2-yl)methyl]-4-phenylpyrrolidin-3-amine;
(3R,4S)-N-(3-methylbutyl)-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-4-phenyl-N-(3,5,5-trimethylhexyl)pyrrolidin-3-amine;
(3R,4S)-1-[(1-methyl-1H-imidazol-4-yl)sulfonyl]-4-phenyl-N-(2-phenylpropyl)pyrrolidin-3-amine;
(3R,4S)-N-(3,3-dimethylbutyl)-1-[(1-methyl-1H-imidazol-4-yl)sulfonyl]-4-phenylpyrrolidin-3-amine;
(3R,4S)-N-[(1-methylcyclohexyl)methyl]-1-[(1-methyl-1H-imidazol-4-yl)sulfonyl]-4-phenylpyrrolidin-3-amine;
(3R,4S)-N-[2-(3-chlorophenyl)ethyl]-1-[(1-methyl-1H-imidazol-4-yl)sulfonyl]-4-phenylpyrrolidin-3-amine;
(3R,4S)-N-[2-(4-tert-butylphenoxy)ethyl]-1-[(1-methyl-1H-imidazol-4-yl)sulfonyl]-4-phenylpyrrolidin-3-amine;
(3R,4S)-N-(2-ethyl-3-methylbutyl)-1-[(1-methyl-1H-imidazol-4-yl)sulfonyl]-4-phenylpyrrolidin-3-amine;
(3R,4S)-N-[3-(3-chlorophenyl)propyl]-1-[(1-methyl-1H-imidazol-4-yl)sulfonyl]-4-phenylpyrrolidin-3-amine;
(3R,4S)-N-[(4-bromo-1,3-thiazol-2-yl)methyl]-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-N-[(4-methyl-2-phenyl-1,3-thiazol-5-yl)methyl]-4-phenylpyrrolidin-3-amine;
N-(3,4-difluorophenyl)-1-{[3-methyl-1-(propan-2-yl)-1H-pyrazol-4-yl] sulfonyl}-4-phenylpyrrolidin-3-amine;
N-(3,4-difluorophenyl)-1-{[1-methyl-3-(trifluoromethyl)-1H-pyrazol-4-yl]sulfonyl}-4-phenylpyrrolidin-3-amine;
1-[(1-cyclopentyl-3-methyl-1H-pyrazol-4-yl)sulfonyl]-N-(3,4-difluorophenyl)-4-phenylpyrrolidin-3-amine;
N-(3,4-difluorophenyl)-1-[(1,3-dimethyl-1H-pyrazol-5-yl)sulfonyl]-4-phenylpyrrolidin-3-amine;
N-(3,4-difluorophenyl)-1-[(1,5-dimethyl-1H-pyrazol-4-yl)sulfonyl]-4-phenylpyrrolidin-3-amine;
1-[(5-chloro-1,3-dimethyl-1H-pyrazol-4-yl)sulfonyl]-N-(3,4-difluorophenyl)-4-phenylpyrrolidin-3-amine;
N-(3,4-difluorophenyl)-1-[(1,2-dimethyl-1H-imidazol-5-yl)sulfonyl]-4-phenylpyrrolidin-3-amine;
N-(3,4-difluorophenyl)-1-[(1-methyl-1H-pyrazol-3-yl)sulfonyl]-4-phenylpyrrolidin-3-amine;
N-(3,4-difluorophenyl)-4-phenyl-1-[(1,3,5-trimethyl-1H-pyrazol-4-yl)sulfonyl]pyrrolidin-3-amine;
N-(3,4-difluorophenyl)-1-[(1-methyl-1H-pyrazol-5-yl)sulfonyl]-4-phenylpyrrolidin-3-amine;
trans-N-(3-chlorophenyl)-4-(cyclopropylmethoxy)-1-[(1-methyl-1H-imidazol-4-yl)sulfonyl]pyrrolidin-3-amine;
N-{trans-4-cyclohexyl-1-[(1-methyl-1H-imidazol-4-yl)sulfonyl]pyrrolidin-3-yl}-6-(trifluoromethyl)pyrimidin-4-amine;
2-methoxy-N-{(3R,4S)-1-[(1-methyl-1H-imidazol-4-yl)sulfonyl]-4-phenylpyrrolidin-3-yl}pyrimidin-4-amine;
(3R,4S)-1-[(1-methyl-1H-imidazol-4-yl)sulfonyl]-N-[(2-methyl-1,3-thiazol-4-yl)methyl]-4-phenylpyrrolidin-3-amine;
(3R,4S)-N-[(2-ethyl-1-benzofuran-3-yl)methyl]-1-[(1-methyl-1H-imidazol-4-yl)sulfonyl]-4-phenylpyrrolidin-3-amine;
(3R,4S)-N-(imidazo[1,2-a]pyridin-8-ylmethyl)-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-N-[(5-methylpyridin-2-yl)methyl]-4-phenylpyrrolidin-3-amine;
(3R,4S)-1-[(1-methyl-1H-imidazol-4-yl)sulfonyl]-4-phenyl-N-(pyrazolo[1,5-a]pyridin-3-ylmethyl)pyrrolidin-3-amine;
N-{trans-4-hydroxy-1-[(1-methyl-1H-imidazol-4-yl)sulfonyl]-4-phenylpyrrolidin-3-yl}-4-(trifluoromethyl)benzamide;
1-[(1-methyl-1H-imidazol-4-yl)sulfonyl]-3-phenyl-4-{[4-(trifluoromethyl)benzyl]amino}pyrrolidin-3-ol;
(3R,4S)-N-[4-chloro-3-(trifluoromethyl)phenyl]-4-(4-fluorophenyl)-1-[(1-methyl-1H-imidazol-4-yl)sulfonyl]pyrrolidin-3-amine;
ethyl {(3R,4S)-1-[(1-methyl-1H-imidazol-4-yl)sulfonyl]-4-phenylpyrrolidin-3-yl}carbamate;
propyl {(3R,4S)-1-[(1-methyl-1H-imidazol-4-yl)sulfonyl]-4-phenylpyrrolidin-3-yl}carbamate;
2-methylpropyl {(3R,4S)-1-[(1-methyl-1H-imidazol-4-yl)sulfonyl]-4-phenylpyrrolidin-3-yl}carbamate;
butyl {(3R,4S)-1-[(1-methyl-1H-imidazol-4-yl)sulfonyl]-4-phenylpyrrolidin-3-yl}carbamate;
(3R,4S)-N-(2-cyclopropylethyl)-1-[(1-methyl-1H-imidazol-4-yl)sulfonyl]-4-phenylpyrrolidin-3-amine;
2-cyclopropyl-N-{(3R,4S)-1-[(1-methyl-1H-imidazol-4-yl)sulfonyl]-4-phenylpyrrolidin-3-yl}acetamide;
(3R,4S)-N-[(2E)-hex-2-en-1-yl]-1-[(1-methyl-1H-imidazol-4-yl)sulfonyl]-4-phenylpyrrolidin-3-amine;
(3R,4S)-N-hexyl-1-[(1-methyl-1H-imidazol-4-yl)sulfonyl]-4-phenylpyrrolidin-3-amine;
N-{(3R,4S)-1-[(1-methyl-1H-imidazol-4-yl)sulfonyl]-4-phenylpyrrolidin-3-yl}pentanamide;
N-{(3R,4S)-1-[(1-methyl-1H-imidazol-4-yl)sulfonyl]-4-phenylpyrrolidin-3-yl}hexanamide;
(3R,4S)-N-(2-{[tert-butyl(dimethyl)silyl]oxy}ethyl)-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-4-phenyl-N-{[6-(trifluoromethyl)pyridin-2-yl]methyl}pyrrolidin-3-amine;
(3R,4S)-N-[(3-chlorothiophen-2-yl)methyl]-1-[(1-methyl-1H-imidazol-4-yl)sulfonyl]-4-phenylpyrrolidin-3-amine;
(3R,4S)-N-[(4,4-difluorocyclohexyl)methyl]-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-4-phenyl-N-(pyrazolo[1,5-a]pyridin-7-ylmethyl)pyrrolidin-3-amine;
(3R,4S)-1-[(1-methyl-1H-imidazol-4-yl)sulfonyl]-4-phenyl-N-{[4-(propan-2-yl)-1,3-thiazol-2-yl]methyl}pyrrolidin-3-amine;
(3R,4S)-N-[(2,2-dimethyltetrahydro-2H-pyran-4-yl)methyl]-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-4-phenyl-N-{[5-(tetrahydro-2H-pyran-2-yl)thiophen-2-yl]methyl}pyrrolidin-3-amine;
(3R,4S)-N-[(5-bromo-4-methyl-1,3-thiazol-2-yl)methyl]-1-[(1-methyl-1H-imidazol-4-yl)sulfonyl]-4-phenylpyrrolidin-3-amine;
(3R,4S)-N-(imidazo[1,5-a]pyridin-5-ylmethyl)-1-[(1-methyl-1H-imidazol-4-yl)sulfonyl]-4-phenylpyrrolidin-3-amine;
(3R,4S)-N-(2,3-dimethylpentyl)-1-[(1-methyl-1H-imidazol-4-yl)sulfonyl]-4-phenylpyrrolidin-3-amine;
(3R,4S)-N-[(2-chloro-1,3-thiazol-4-yl)methyl]-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-N-[(5-methylimidazo[1,2-a]pyridin-3-yl)methyl] -4-phenylpyrrolidin-3-amine;
(3R,4S)-1-[(1-methyl-1H-imidazol-4-yl)sulfonyl]-4-phenyl-N-[(2-phenylcyclopropyl)methyl]pyrrolidin-3-amine;
(3R,4S)-N-[(4-chloro-1,3-thiazol-5-yl)methyl]-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-4-phenyl-N-(3,3,3-trifluoro-2-methylpropyl)pyrrolidin-3-amine;
(3R,4S)-N-[(2-chloro-3-fluoropyridin-4-yl)methyl]-1-[(1-methyl-1H-imidazol-4-yl)sulfonyl]-4-phenylpyrrolidin-3-amine;
(3R,4S)-N-[(5-fluoro-1-benzothiophen-2-yl)methyl]-1-[(1-methyl-1H-imidazol-4-yl)sulfonyl]-4-phenylpyrrolidin-3-amine;
(3R,4S)-N-[5-chloro-2-(difluoromethoxy)benzyl]-1-[(1-methyl-1H-imidazol-4-yl)sulfonyl]-4-phenylpyrrolidin-3-amine;
(3R,4S)-N-[(2,5-dichlorothiophen-3-yl)methyl]-1-[(1-methyl-1H-imidazol-4-yl)sulfonyl]-4-phenylpyrrolidin-3-amine;
(3R,4S)-N-{[2-(4-fluorophenyl)pyridin-3-yl]methyl}-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-N-{[2-(4-methylphenyl)-1,3-thiazol-5-yl]methyl}-4-phenylpyrrolidin-3-amine;
(3R,4S)-N-(3-cyclopropylpropyl)-1-[(1-methyl-1H-imidazol-4-yl)sulfonyl]-4-phenylpyrrolidin-3-amine;
5-fluoro-4-methyl-N-{(3R,4S)-1-[(1-methyl-1H-imidazol-4-yl)sulfonyl]-4-phenylpyrrolidin-3-yl}pyridin-2-amine;
1,5-dimethyl-N-{(3R,4S)-1-[(1-methyl-1H-imidazol-4-yl)sulfonyl]-4-phenylpyrrolidin-3-yl}-1H-1,2,4-triazol-3-amine;
1,5-dimethyl-4-({(3R,4S)-1-[(1-methyl-1H-imidazol-4-yl)sulfonyl]-4-phenylpyrrolidin-3-yl}amino)-1H-pyrrole-2-carbonitrile;
(3S,4R)-4-(4-fluorophenyl)-1-[(1-methyl-1H-imidazol-4-yl)sulfonyl]-N-[3-(propan-2-yloxy)propyl]pyrrolidine-3-carboxamide;
(3S,4R)-N-(3-ethoxypropyl)-4-(4-fluorophenyl)-1-[(1-methyl-1H-imidazol-4-yl)sulfonyl]pyrrolidine-3-carboxamide;
(3S,4R)-4-(4-fluorophenyl)-N-(2-methoxyethyl)-1-[(1-methyl-1H-imidazol-4-yl)sulfonyl]pyrrolidine-3-carboxamide;
(3S,4R)-4-(4-fluorophenyl)-1-[(1-methyl-1H-imidazol-4-yl)sulfonyl]-N-propylpyrrolidine-3-carboxamide;
(3S,4R)-N-butyl-4-(4-fluorophenyl)-1-[(1-methyl-1H-imidazol-4-yl)sulfonyl]pyrrolidine-3-carboxamide;
(3S,4R)-4-(4-fluorophenyl)-N-[1-methoxypropan-2-yl]-1-[(1-methyl-1H-imidazol-4-yl)sulfonyl]pyrrolidine-3-carboxamide;
(3R,4S)-4-(4-fluorophenyl)-N-(3-methoxypropyl)-1-[(1-methyl-1H-imidazol-4-yl)sulfonyl]pyrrolidine-3-carboxamide;
(3S,4R)-N-(2-ethoxyethyl)-4-(4-fluorophenyl)-1-[(1-methyl-1H-imidazol-4-yl)sulfonyl]pyrrolidine-3-carboxamide;
(3R,4S)-N-[4-chloro-3-(trifluoromethoxy)phenyl]-4-(4-fluorophenyl)-1-[(1-methyl-1H-imidazol-4-yl)sulfonyl]pyrrolidin-3-amine;
(3S,4R)-N-(cyclopropylmethyl)-4-(4-fluorophenyl)-1-[(1-methyl-1H-imidazol-4-yl)sulfonyl]pyrrolidine-3-carboxamide;
(3S,4R)-4-(4-fluorophenyl)-1-[(1-methyl-1H-imidazol-4-yl)sulfonyl]-N-[2-(propan-2-yloxy)ethyl]pyrrolidine-3-carboxamide;
(3R,4S)-N-(3-methoxypropyl)-1-[(1-methyl-1H-imidazol-4-yl)sulfonyl]-4-phenylpyrrolidin-3-amine;
(3R,4S)-N-(3-ethoxypropyl)-1-[(1-methyl-1H-imidazol-4-yl)sulfonyl]-4-phenylpyrrolidin-3-amine;
(3R,4S)-N-{[1-(methoxymethyl)cyclopropyl]methyl}-1-[(1-methyl-1H-imidazol-4-yl)sulfonyl]-4-phenylpyrrolidin-3-amine;
N-({trans-4-(4-fluorophenyl)-1-[(1-methyl-1H-imidazol-4-yl)sulfonyl]pyrrolidin-3-yl}methyl)-3-(propan-2-yloxy)propan-1-amine;
3-ethoxy-N-({trans-4-(4-fluorophenyl)-1-[(1-methyl-1H-imidazol-4-yl)sulfonyl]pyrrolidin-3-yl}methyl)propan-1-amine;
N-({trans-4-(4-fluorophenyl)-1-[(1-methyl-1H-imidazol-4-yl)sulfonyl]pyrrolidin-3-yl}methyl)-2-methoxyethanamine;
N-({trans-4-(4-fluorophenyl)-1-[(1-methyl-1H-imidazol-4-yl)sulfonyl]pyrrolidin-3-yl}methyl)propan-1-amine;
N-({trans-4-(4-fluorophenyl)-1-[(1-methyl-1H-imidazol-4-yl)sulfonyl]pyrrolidin-3-yl}methyl)butan-1-amine;
(2S)-N-({trans-4-(4-fluorophenyl)-1-[(1-methyl-1H-imidazol-4-yl)sulfonyl]pyrrolidin-3-yl}methyl)-1-methoxypropan-2-amine;
N-({trans-4-(4-fluorophenyl)-1-[(1-methyl-1H-imidazol-4-yl)sulfonyl]pyrrolidin-3-yl}methyl)-3-methoxypropan-1-amine;
2-ethoxy-N-({trans-4-(4-fluorophenyl)-1-[(1-methyl-1H-imidazol-4-yl)sulfonyl]pyrrolidin-3-yl}methyl)ethanamine;
N-({trans-4-(4-fluorophenyl)-1-[(1-methyl-1H-imidazol-4-yl)sulfonyl]pyrrolidin-3-yl}methyl)-2-(propan-2-yloxy)ethanamine;
(3R,4S)-N-(2-methoxyethyl)-1-[(1-methyl-1H-imidazol-4-yl)sulfonyl]-4-phenylpyrrolidin-3-amine;
1-[(1-methyl-1H-imidazol-4-yl)sulfonyl]-4-(tetrahydrofuran-2-yl)-N-[3-(trifluoromethoxy)phenyl]pyrrolidin-3-amine;
1-[(1-methyl-1H-imidazol-4-yl)sulfonyl]-4-(tetrahydrofuran-2-yl)-N-[3-(trifluoromethoxy)phenyl]pyrrolidin-3-amine;
1-[(1-methyl-1H-imidazol-4-yl)sulfonyl]-4-(tetrahydrofuran-2-yl)-N-[3-(trifluoromethyl)phenyl]pyrrolidin-3-amine;
(3R,4S)-1-[(1-methyl-1H-iniidazol-4-yl)sulfonyl]-4-(tetrahydrofuran-2-yl)-N-[3-(trifluoromethyl)phenyl]pyrrolidin-3-amine;
2-methoxy-N-{(3R,4S)-1-[(1-methyl-1H-imidazol-4-yl)sulfonyl]-4-phenylpyrrolidin-3-yl}-6-(trifluoromethyl)pyrimidin-4-amine;
6-chloro-N-{(3R,4S)-1-[(1-methyl-1H-imidazol-4-yl)sulfonyl]-4-phenylpyrrolidin-3-yl}pyrimidin-4-amine;
1-cyclopropyl-N-({(3R,4R)-4-(4-fluorophenyl)-1-[(1-methyl-1H-imidazol-4-yl)sulfonyl]pyrrolidin-3-yl}methyl)methanamine;
(3R,4S)-N-(2-ethoxyethyl)-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-4-phenyl-N-(2-propoxyethyl)pyrrolidin-3-amine;
N-(3,4-difluorophenyl)-1-[(1-methyl-1H-imidazol-4-yl)sulfonyl]-4-[-tetrahydrofuran-2-yl]pyrrolidin-3-amine;
N-(3,4-difluorophenyl)-1-[(1-methyl-1H-imidazol-4-yl)sulfonyl]-4-(tetrahydrofuran-2-yl)pyrrolidin-3-amine;
6-cyclopropyl-N-{(3R,4S)-1-[(1-methyl-1H-imidazol-4-yl)sulfonyl]-4-phenylpyrrolidin-3-yl}pyrimidin-4-amine;
6-ethyl-N-{(3R,4S)-4-(4-fluorophenyl)-1-[(1-methyl-1H-imidazol-4-yl)sulfonyl]pyrrolidin-3-yl}pyrimidin-4-amine;
N-{(3R,4S)-4-(4-fluorophenyl)-1-[(1-methyl-1H-imidazol-4-yl)sulfonyl]pyrrolidin-3-yl}-6-(propan-2-yl)pyrimidin-4-amine;
N-{(3R,4S)-4-(4-fluorophenyl)-1-[(1-methyl-1H-imidazol-4-yl)sulfonyl]pyrrolidin-3-yl}-2-methyl-6-(trifluoromethyl)pyrimidin-4-amine;
N-{(3R,4S)-4-(4-fluorophenyl)-1-[(1-methyl-1H-imidazol-4-yl)sulfonyl]pyrrolidin-3-yl}-6-propylpyrimidin-4-amine;
5-chloro-N-{(3R,4S)-4-(4-fluorophenyl)-1-[(1-methyl-1H-imidazol-4-yl)sulfonyl]pyrrolidin-3-yl}-4-(trifluoromethyl)pyridin-2-amine;
(3S,4R)-N-[4-chloro-3-(trifluoromethyl)phenyl]-1-[(1-methyl-1H-imidazol-4-yl)sulfonyl]-4-(tetrahydrofuran-2-yl)pyrrolidin-3-amine;
(3R,4S)-N-[4-chloro-3-(trifluoromethyl)phenyl]-1-[(1-methyl-1H-imidazol-4-yl)sulfonyl]-4-(tetrahydrofuran-2-yl)pyrrolidin-3-amine;
6-ethoxy-N-{(3R,4S)-1-[(1-methyl-1H-imidazol-4-yl)sulfonyl]-4-phenylpyrrolidin-3-yl}pyrimidin-4-amine;
6-methoxy-N-{(3R,4S)-1-[(1-methyl-1H-imidazol-4-yl)sulfonyl]-4-phenylpyrrolidin-3-yl}pyrimidin-4-amine;
N-{(3R,4S)-1-[(1-methyl-1H-imidazol-4-yl)sulfonyl]-4-phenylpyrrolidin-3-yl}-6-propoxypyrimidin-4-amine;
6-butoxy-N-{(3R,4S)-1-[(1-methyl-1H-imidazol-4-yl)sulfonyl]-4-phenylpyrrolidin-3-yl}pyrimidin-4-amine;
N-{(3R,4S)-1-[(1-methyl-1H-imidazol-4-yl)sulfonyl]-4-phenylpyrrolidin-3-yl}-6-(2-methylpropoxy)pyrimidin-4-amine;
4-{[(3S,4R)-3-(4-fluorophenyl)-4-(hexyloxy)pyrrolidin-1-yl]sulfonyl}-1-methyl-1H-imidazole;
trans-4-(4-fluorophenyl)-1-[(1-methyl-1H-imidazol-4-yl)sulfonyl]-N-pentylpyrrolidine-3-carboxamide;
trans-4-(4-fluorophenyl)-N-hexyl-1-[(1-methyl-1H-imidazol-4-yl)sulfonyl]pyrrolidine-3-carboxamide;
trans-N-(3-chlorobenzyl)-4-(4-fluorophenyl)-1-[(1-methyl-1H-imidazol-4-yl)sulfonyl]pyrrolidine-3-carboxamide;
trans-N-(4-chlorobenzyl)-4-(4-fluorophenyl)-1-[(1-methyl-1H-imidazol-4-yl)sulfonyl]pyrrolidine-3-carboxamide;
trans-N-(4-fluorobenzyl)-4-(4-fluorophenyl)-1-[(1-methyl-14H-imidazol-4-yl)sulfonyl]pyrrolidine-3-carboxamide;
trans-N-(3,4-difluorobenzyl)-4-(4-fluorophenyl)-1-[(1-methyl-1H-imidazol-4-yl)sulfonyl]pyrrolidine-3-carboxamide;
trans-4-(4-fluorophenyl)-1-[(1-methyl-1H-imidazol-4-yl)sulfonyl]-N-[3-(trifluoromethyl)benzyl]pyrrolidine-3-carboxamide;
trans-N-[2-chloro-5-(trifluoromethyl)benzyl]-4-(4-fluorophenyl)-1-[(1-methyl-1H-imidazol-4-yl)sulfonyl]pyrrolidine-3-carboxamide;
trans-4-(4-fluorophenyl)-1-[(1-methyl-1H-imidazol-4-yl)sulfonyl]-N-(pyridin-2-ylmethyl)pyrrolidine-3-carboxamide;
trans-4-(4-fluorophenyl)-1-[(1-methyl-1H-imidazol-4-yl)sulfonyl]-N-{[4-(trifluoromethyl)pyridin-2-yl]methyl}pyrrolidine-3-carboxamide;
trans-N,N-dibutyl-4-(4-fluorophenyl)-1-[(1-methyl-1H-imidazol-4-yl)sulfonyl]pyrrolidine-3-carboxamide;
6-methyl-N-{(3R,4S)-1-[(1-methyl-1H-imidazol-4-yl)sulfonyl]-4-phenylpyrrolidin-3-yl}pyrimidin-4-amine;
(3R,4S)-4-(4-fluorophenyl)-1-[(1-methyl-1H-imidazol-4-yl)sulfonyl]-N-[4-methyl-3-(trifluoromethyl)phenyl]pyrrolidin-3-amine;
4-({(3R,4S)-4-(4-fluorophenyl)-1-[(1-methyl-1H-imidazol-4-yl)sulfonyl]pyrrolidin-3-yl}amino)-2-(trifluoromethyl)benzonitrile;
4-{trans-4-(4-fluorophenyl)-1-[(1-methyl-1H-imidazol-4-yl)sulfonyl]pyrrolidin-3-yl}-1-propyl-1H-1,2,3-triazole;
4-[-1-cyclopropylpiperidin-3-yl]-1-[(1-methyl-1H-imidazol-4-yl)sulfonyl]-N-[3-(trifluoromethoxy)phenyl]pyrrolidin-3-amine;
trans-N-(3-chloro-4-fluorobenzyl)-4-(4-fluorophenyl)-1-[(1-methyl-1H-imidazol-4-yl)sulfonyl]pyrrolidine-3-carboxamide;
trans-4-(4-fluorophenyl)-N-(6-methylheptyl)-1-[(1-methyl-1H-imidazol-4-yl)sulfonyl]pyrrolidine-3-carboxamide;
2-(4-fluorophenoxy)-N-{(3R,4S)-4-(4-fluorophenyl)-1-[(1-methyl-1H-imidazol-4-yl)sulfonyl]pyrrolidin-3-yl}acetamide;
(3R,4S)-N-[4-fluoro-3-(trifluoromethyl)phenyl]-1-[(1-methyl-1H-imidazol-4-yl)sulfonyl]-4-phenylpyrrolidin-3-amine;
(3R,4S)-N-[4-fluoro-3-(trifluoromethoxy)phenyl]-1-[(1-methyl-1H-imidazol-4-yl)sulfonyl]-4-phenylpyrrolidin-3-amine;
(3R,4S)-N-(4-methoxybutyl)-1-[(1-methyl-1H-imidazol-4-yl)sulfonyl]-4-phenylpyrrolidin-3-amine;
(3R,4S)-N-(4-methoxyhexyl)-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-4-phenyl-N-[(2-propylcyclopropyl)methyl]pyrrolidin-3-amine;
(3R,4S)-1-[(1-methyl-1H-imidazol-4-yl)sulfonyl]-4-phenyl-N-[(2-propylcyclopropyl)methyl]pyrrolidin-3-amine;
(3R,4S)-4-(4-fluorophenyl)-N-[4-fluoro-3-(trifluoromethoxy)phenyl]-1-[(1-methyl-1H-imidazol-4-yl)sulfonyl]pyrrolidin-3-amine;
(3R,4S)-4-(4-fluorophenyl)-N-[4-fluoro-3-(trifluoromethyl)phenyl]-1-[(1-methyl-1H-imidazol-4-yl)sulfonyl]pyrrolidin-3-amine;
(3R,4S)-N-[4-methoxy-3-(trifluoromethyl)phenyl]-1-[(1-methyl-1H-imidazol-4-yl)sulfonyl]-4-phenylpyrrolidin-3-amine;
(3R,4S)-N-(1-benzothiophen-5-yl)-1-[(1-methyl-1H-imidazol-4-yl)sulfonyl]-4-phenylpyrrolidin-3-amine;
(3R,4S)-N-(2,3-dihydro-1H-inden-5-yl)-1-[(1-methyl-1H-imidazol-4-yl)sulfonyl]-4-phenylpyrrolidin-3-amine;
(3R,4S)-N-[3,4-bis(trifluoromethyl)phenyl]-1-[(1-methyl-1H-imidazol-4-yl)sulfonyl]-4-phenylpyrrolidin-3-amine;
(3R,4S)-1-[(1-methyl-1H-imidazol-4-yl)sulfonyl]-4-phenyl-N-(5,6,7,8-tetrahydronaphthalen-2-yl)pyrrolidin-3-amine;
(3R,4S)-N-(3,4-dihydro-2H-chromen-6-yl)-1-[(1-methyl-1H-imidazol-4-yl)sulfonyl]-4-phenylpyrrolidin-3-amine;
4-({trans-1-[(1-methyl-1H-imidazol-4-yl)sulfonyl]-4-phenylpyrrolidin-3-yl}methyl)-1-propyl-1H-1,2,3-triazole;
(3R,4S)-N-(1-benzofuran-5-yl)-1-[(1-methyl-1H-imidazol-4-y1)sulfonyl]-4-phenylpyrrolidin-3-amine;
trans-4-(4-fluorophenyl)-1-[(1-methyl-1H-imidazol-4-yl)sulfonyl]-N-[3-(trifluoromethoxy)benzyl]pyrrolidine-3-carboxamide;
methyl 5-({(3R,4S)-4-(4-fluorophenyl)-1-[(1-methyl-1H-imidazol-4-yl)sulfonyl]pyrrolidin-3-yl}amino)-5-oxopentanoate;
(3R,4S)-N-[2-chloro-4-(trifluoromethyl)benzyl]-4-(4-fluorophenyl)-1-[(1-methyl-1H-imidazol-4-yl)sulfonyl]pyrrolidine-3-carboxamide;
(3R,4S)-4-(4-fluorophenyl)-1-[(1-methyl-1H-imidazol-4-yl)sulfonyl]-N-pentylpyrrolidin-3-amine;
(3R,4S)-4-(4-fluorophenyl)-N-hexyl-1-[(1-methyl-1H-imidazol-4-yl)sulfonyl]pyrrolidin-3-amine;
(3R,4S)-N-[2-(2-methoxyethoxy)ethyl]-1-[(1-methyl-1H-imidazol-4-yl)sulfonyl]-4-phenylpyrrolidin-3-amine;
(3R,4S)-N-[2-(4-fluorophenoxy)ethyl]-4-(4-fluorophenyl)-1-[(1-methyl-1H-imidazol-4-yl)sulfonyl]pyrrolidin-3-amine;
4-butyl-1-{trans-1-[(1-methyl-1H-imidazol-4-yl)sulfonyl]-4-phenylpyrrolidin-3-yl}-1H-1,2,3-triazole;
(3R,4S)-N-(4-chloro-3-methylphenyl)-1-[(1-methyl-1H-imidazol-4-yl)sulfonyl]-4-phenylpyrrolidin-3-amine;
(3R,4S)-1-[(1-methyl-1H-iniidazol-4-yl)sulfonyl]-4-phenyl-N-[3-(2,2,2-trifluoroethoxy)phenyl]pyrrolidin-3-amine;
(3R,4S)-N-(2,3-dihydro-1-benzofuran-5-yl)-1-[(1-methyl-1H-imidazol-4-yl)sulfonyl]-4-phenylpyrrolidin-3-amine;
(3R,4S)-N-(4,4-dimethyl-3,4-dihydro-2H-chromen-6-yl)-4-(4-fluorophenyl)-1-[(1-methyl-1H-imidazol-4-yl)sulfonyl]pyrrolidin-3-amine;
N-{(3R,4S)-4-(4-fluorophenyl)-1-[(1-methyl-1H-imidazol-4-yl)sulfonyl]pyrrolidin-3-yl}-2-hydroxyhexanamide;
(3R,4S)-N-(3-methoxyhexyl)-1-[(1-methyl-1H-imidazol-4-yl)sulfonyl]-4-phenylpyrrolidin-3-amine;
(3R,4S)-N-[4-chloro-3-(propan-2-yl)phenyl]-4-(4-fluorophenyl)-1-[(1-methyl-1H-imidazol-4-yl)sulfonyl]pyrrolidin-3-amine;
(3R,4S)-4-(4-fluorophenyl)-1-[(1-methyl-1H-imidazol-4-yl)sulfonyl]-N-[3-methyl-4-(trifluoromethyl)phenyl]pyrrolidin-3-amine;
(3R,4S)-N-(1-benzothiophen-6-yl)-4-(4-fluorophenyl)-1-[(1-methyl-1H-imidazol-4-yl)sulfonyl]pyrrolidin-3-amine;
-4-(1-cyclopropylpiperidin-4-yl)-1-[(1-methyl-1H-imidazol-4-yl)sulfonyl]-N-[3-(trifluoromethoxy)phenyl]pyrrolidin-3-amine;
trans-N-hexyl-1-[(1-methyl-1H-imidazol-4-yl)sulfonyl]-4-(tetrahydro-2H-pyran-2-yl)pyrrolidin-3-amine;
trans-N-[2-chloro-4-(trifluoromethyl)benzyl]-1-[(1-methyl-1H-imidazol-4-yl)sulfonyl]-4-(tetrahydro-2H-pyran-2-yl)pyrrolidin-3-amine;
trans-1-[(1-methyl-1H-imidazol-4-yl)sulfonyl]-4-(tetrahydro-2H-pyran-2-yl)-N-[3-(trifluoromethyl)phenyl]pyrrolidin-3-amine;
N-{trans-1-[(1-methyl-1H-imidazol-4-yl)sulfonyl]-4-[tetrahydro-2H-pyran-2-yl]pyrrolidin-3-yl}-4-(trifluoromethyl)pyridin-2-amine;
N-{trans-1-[(1-methyl-1H-imidazol-4-yl)sulfonyl]-4-[tetrahydro-2H-pyran-2-yl]pyrrolidin-3-yl}-4-(trifluoromethyl)pyridin-2-amine;
(3R,4S)-N-[4-(4,4-dimethyl-1,3-dioxan-2-yl)butyl]-4-(4-fluorophenyl)-1-[(1-methyl-1H-imidazol-4-yl)sulfonyl]pyrrolidin-3-amine;
trans-1-[(1-methyl-1H-imidazol-4-yl)sulfonyl]-4-(tetrahydro-2H-pyran-2-yl)-N-[3-(trifluoromethoxy)phenyl]pyrrolidin-3-amine;
trans-1-[(1-methyl-1H-imidazol-4-yl)sulfonyl]-4-[tetrahydro-2H-pyran-2-yl]-N-[3-(trifluoromethoxy)phenyl]pyrrolidin-3-amine;
N-[trans-1-[(1-methyl-1H-imidazol-4-yl)sulfonyl]-4-(tetrahydro-2H-pyran-2-yl)pyrrolidin-3-yl]-6-(trifluoromethyl)pyrimidin-4-amine;
N-[2-({trans-4-(4-fluorophenyl)-1-[(1-methyl-1H-imidazol-4-yl)sulfonyl]pyrrolidin-3-yl}oxy)ethyl]propan-1-amine;
2-({trans-4-(4-fluorophenyl)-1-[(1-methyl-1H-imidazol-4-yl)sulfonyl]pyrrolidin-3-yl}oxy)-N-propylacetamide;
6-chloro-N-{(3R,4S)-4-(4-fluorophenyl)-1-[(1-methyl-1H-imidazol-4-yl)sulfonyl]pyrrolidin-3-yl}-2-methylpyrimidin-4-amine;
6-chloro-N-{(3R,4S)-4-(4-fluorophenyl)-1-[(1-methyl-1H-imidazol-4-yl)sulfonyl]pyrrolidin-3-yl}pyrimidin-4-amine;
1-{(3R,4S)-4-(4-fluorophenyl)-1-[(1-methyl-1H-imidazol-4-yl)sulfonyl]pyrrolidin-3-yl}-3-phenylurea;
phenyl {(3R,4S)-4-(4-fluorophenyl)-1-[(1-methyl-1H-imidazol-4-yl)sulfonyl]pyrrolidin-3-yl}carbamate;
1-(2-chlorophenyl)-3-{ (3R,4S)-4-( 4-fluorophenyl)-1-[(1-methyl-1H-imidazol-4-yl)sulfonyl]pyrrolidin-3-yl}urea;
N-{(3R,4S)-4-(4-fluorophenyl)-1-[(1-methyl-1H-imidazol-4-yl)sulfonyl]pyrrolidin-3-yl}-5-oxohexanamide;
N-{(3R,4S)-4-(4-fluorophenyl)-1-[(1-methyl-1H-imidazol-4-yl)sulfonyl]pyrrolidin-3-yl}-4-(trifluoromethyl)cyclohexanecarboxamide;
butyl-trans-4-(4-fluorophenyl)-1-[(1-methyl-1H-imidazol-4-yl)sulfonyl]pyrrolidin-3-yl carbonate;
5,5,5-trifluoro-N-{(3R,4S)-4-(4-fluorophenyl)-1-[(1-methyl-1H-imidazol-4-yl)sulfonyl]pyrrolidin-3-yl}pentanamide;
(3S,4R)-4-(4-fluorophenyl)-1-[(1-methyl-1H-imidazol-4-yl)sulfonyl]pyrrolidin-3-yl butylcarbamate;
2,2-difluoro-N-{(3R,4S)-4-(4-fluorophenyl)-1-[(1-methyl-1H-imidazol-4-yl)sulfonyl]pyrrolidin-3-yl}pentanamide;
1-(4-chlorophenyl)-3-{(3R,4S)-4-(4-fluorophenyl)-1-[(1-methyl-1H-imidazol-4-yl)sulfonyl]pyrrolidin-3-yl}urea;
1-(2-fluorophenyl)-3-{(3R,4S)-4-(4-fluorophenyl)-1-[(1-methyl-1H-imidazol-4-yl)sulfonyl]pyrrolidin-3-yl}urea;
1-(3-fluorophenyl)-3-{(3R,4S)-4-(4-fluorophenyl)-1-[(1-methyl-1H-imidazol-4-yl)sulfonyl]pyrrolidin-3-yl}urea;
1-(4-fluorophenyl)-3-{(3R,4S)-4-(4-fluorophenyl)-1-[(1-methyl-1H-imidazol-4-yl)sulfonyl]pyrrolidin-3-yl}urea;
1-(2-chlorobenzyl)-3-{(3R,4S)-4-(4-fluorophenyl)-1-[(1-methyl-1H-imidazol-4-yl)sulfonyl]pyrrolidin-3-yl}urea;
4,5-dichloro-N-{ (3R,4S)-4-(4-fluorophenyl)-1-[(1-methyl-1H-imidazol-4-yl)sulfonyl]pyrrolidin-3-yl}pyridin-2-amine;
(3R,4S)-4-(4-fluorophenyl)-1-[(1-methyl-1H-imidazol-4-yl)sulfonyl]-N-{[4-(trifluoromethyl)cyclohexyl]methyl}pyrrolidin-3-amine;
2,2-difluoro-N-{(3R,4S)-4-(4-fluorophenyl)-1-[(1-methyl-1H-imidazol-4-yl)sulfonyl]pyrrolidin-3-yl}hexanamide;
trans-N-[3,4-difluorophenyl]-1-[(1-methyl-1H-imidazol-4-yl)sulfonyl]-4-(1,3-oxazol-2-yl)pyrrolidin-3-amine;
N-[4-chloro-3-(trifluoromethyl)phenyl]-1-[(1-methyl-1H-imidazol-4-yl)sulfonyl]-4-(1,3-oxazol-2-yl)pyrrolidin-3-amine;
4-{[trans-3-[(4-fluorobenzyl)oxy]-4-(4-fluorophenyl)pyrrolidin-1-yl]sulfonyl}-1-methyl-1H-imidazole;
4-{[trans-3-[(3-fluorobenzyl)oxy]-4-(4-fluorophenyl)pyrrolidin-1-yl]sulfonyl}-1-methyl-1H-imidazole;
4-{[trans-3-(4-fluorophenyl)-4-{[4-(methylsulfonyl)benzyl]oxy}pyrrolidin-1-yl]sulfonyl}-1-methyl-1H-imidazole;
4-{[trans-3-(4-fluorophenyl)-4-{[3-(methylsulfonyl)benzyl]oxy}pyrrolidin-1-yl]sulfonyl}-1-methyl-1H-imidazole;
6-ethoxy-N-{(3R,4S)-4-(4-fluorophenyl)-1-[(1-methyl-1H-imidazol-4-yl)sulfonyl]pyrrolidin-3-yl}pyrimidin-4-amine;
N-{(3R,4S)-4-(4-fluorophenyl)-1-[(1-methyl-1H-imidazol-4-yl)sulfonyl]pyrrolidin-3-yl}-6-(propan-2-yloxy)pyrimidin-4-amine;
6-butoxy-N-{(3R,4S)-4-(4-fluorophenyl)-1-[(1-methyl-1H-imidazol-4-yl)sulfonyl]pyrrolidin-3-yl}pyrimidin-4-amine;
N-{(3R,4S)-4-(4-fluorophenyl)-1-[(1-methyl-1H-imidazol-4-yl)sulfonyl]pyrrolidin-3-yl}-6-(2-methylpropoxy)pyrimidin-4-amine;
6-(cyclobutyloxy)-N-{(3R,4S)-4-(4-fluorophenyl)-1-[(1-methyl-1H-imidazol-4-yl)sulfonyl]pyrrolidin-3-yl}pyrimidin-4-amine;
6-(cyclopentyloxy)-N-{(3R,4S)-4-(4-fluorophenyl)-1-[(1-methyl-1H-imidazol-4-yl)sulfonyl]pyrrolidin-3-yl}pyrimidin-4-amine;
6-(cyclohexyloxy)-N-{(3R,4S)-4-(4-fluorophenyl)-1-[(1-methyl-1H-imidazol-4-yl)sulfonyl]pyrrolidin-3-yl}pyrimidin-4-amine;
N-{(3R,4S)-4-(4-fluorophenyl)-1-[(1-methyl-1H-imidazol-4-yl)sulfonyl]pyrrolidin-3-yl}-6-phenylpyrimidin-4-amine;
N-{(3R,4S)-4-(4-fluorophenyl)-1-[(1-methyl-1H-imidazol-4-yl)sulfonyl]pyrrolidin-3-yl}-6-(2-methylphenyl)pyrimidin-4-amine;
N-{(3R,4S)-4-(4-fluorophenyl)-1-[(1-methyl-1H-imidazol-4-yl)sulfonyl]pyrrolidin-3-yl}-6-(3-methylphenyl)pyrimidin-4-amine;
N-{(3R,4S)-4-(4-fluorophenyl)-1-[(1-methyl-1H-imidazol-4-yl)sulfonyl]pyrrolidin-3-yl}-6-(4-methylphenyl)pyrimidin-4-amine;
6-(2-fluorophenyl)-N-{(3R,4S)-4-(4-fluorophenyl)-1-[(1-methyl-1H-imidazol-4-yl)sulfonyl]pyrrolidin-3-yl}pyrimidin-4-amine;
6-(3-fluorophenyl)-N-{(3R,4S)-4-(4-fluorophenyl)-1-[(1-methyl-1H-imidazol-4-yl)sulfonyl]pyrrolidin-3-yl}pyrimidin-4-amine;
6-(4-fluorophenyl)-N-{(3R,4S)-4-(4-fluorophenyl)-1-[(1-methyl-1H-imidazol-4-yl)sulfonyl]pyrrolidin-3-yl}pyrimidin-4-amine;
6-(3-chlorophenyl)-N-{(3R,4S)-4-(4-fluorophenyl)-1-[(1-methyl-1H-imidazol-4-yl)sulfonyl]pyrrolidin-3-yl}pyrimidin-4-amine;
6-(4-chlorophenyl)-N-{(3R,4S)-4-(4-fluorophenyl)-1-[(1-methyl-1H-imidazol-4-yl)sulfonyl]pyrrolidin-3-yl}pyrimidin-4-amine;
4-({(3S,4R)-3-(4-fluorophenyl)-4-[(4-methylbenzyl)oxy]pyrrolidin-1-yl}sulfonyl)-1-methyl-1H-imidazole;
(3R,4S)-4-(4-fluorophenyl)-1-[(1-methyl-1H-imidazol-4-yl)sulfonyl]-N-(5,5,5-trifluoropentyl)pyrrolidin-3-amine;
(3R,4S)-N-(2,2-difluorohexyl)-4-(4-fluorophenyl)-1-[(1-methyl-1H-imidazol-4-yl)sulfonyl]pyrrolidin-3-amine;
(3R,4S)-N-(2,2-difluoropentyl)-4-(4-fluorophenyl)-1-[(1-methyl-1H-imidazol-4-yl)sulfonyl]pyrrolidin-3-amine;
5,5-difluoro-N-{(3R,4S)-4-(4-fluorophenyl)-1-[(1-methyl-1H-imidazol-4-yl)sulfonyl]pyrrolidin-3-yl}hexanamide;
4-fluoro-N-{(3R,4S)-4-(4-fluorophenyl)-1-[(1-methyl-1H-imidazol-4-yl)sulfonyl]pyrrolidin-3-yl}pyridin-2-amine;
5-fluoro-N-{(3R,4S)-4-(4-fluorophenyl)-1-[(1-methyl-1H-imidazol-4-yl)sulfonyl]pyrrolidin-3-yl}pyridin-2-amine;
N-{(3R,4S)-4-(4-fluorophenyl)-1-[(1-methyl-1H-imidazol-4-yl)sulfonyl]pyrrolidin-3-yl}-4-oxopentanamide;
(3R,4S)-N-(5,5-difluorohexyl)-4-(4-fluorophenyl)-1-[(1-methyl-1H-imidazol-4-yl)sulfonyl]pyrrolidin-3-amine;
4-chloro-N-[2-({trans-4-(4-fluorophenyl)-1-[(1-methyl-1H-imidazol-4-yl)sulfonyl]pyrrolidin-3-yl}oxy)ethyl]aniline;
(3S,4R)-N-[4-fluoro-3-(trifluoromethoxy)phenyl]-1-[(1-methyl-1H-imidazol-4-yl)sulfonyl]-4-(tetrahydro-2H-pyran-2-yl)pyrrolidin-3-amine;
1-benzyl-3-{(3R,4S)-4-(4-fluorophenyl)-1-[(1-methyl-1H-imidazol-4-yl)sulfonyl]pyrrolidin-3-yl}urea;
(3R,4S)-N-(4-chloro-3-methylphenyl)-4-(4-fluorophenyl)-1-[(1-methyl-1H-imidazol-4-yl)sulfonyl]pyrrolidin-3-amine;
(3R,4S)-4-(4-fluorophenyl)-1-[(1-methyl-1H-imidazol-4-yl)sulfonyl]-N-[3-(2,2,2-trifluoroethoxy)phenyl]pyrrolidin-3-amine;
(3R,4S)-4-(4-fluorophenyl)-N-[4-fluoro-3-(pyridin-3-yl)phenyl]-1-[(1-methyl-1H-imidazol-4-yl)sulfonyl]pyrrolidin-3-amine;
(3R,4S)-4-(4-fluorophenyl)-N-[4-fluoro-3-(pyridin-4-yl)phenyl]-1-[(1-methyl-1H-imidazol-4-yl)sulfonyl]pyrrolidin-3-amine;
(3R,4S)-N-(4-fluoro-3-methylphenyl)-4-(4-fluorophenyl)-1-[(1-methyl-1H-imidazol-4-yl)sulfonyl]pyrrolidin-3-amine;
5-fluoro-N-{(3R,4S)-4-(4-fluorophenyl)-1-[(1-methyl-1H-imidazol-4-yl)sulfonyl]pyrrolidin-3-yl}-4-methylpyridin-2-amine;
(3R,4S)-4-(4-fluorophenyl)-N-[4-fluoro-3-(pyrimidin-5-yl)phenyl]-1-[(1-methyl-1H-imidazol-4-yl)sulfonyl]pyrrolidin-3-amine;
N-{(3R,4S)-4-(4-fluorophenyl)-1-[(1-methyl-1H-imidazol-4-yl)sulfonyl]pyrrolidin-3-yl}-3,4'-bipyridin-2'-amine;
N-{(3R,4S)-4-(4-fluorophenyl)-1-[(1-methyl-1H-imidazol-4-yl)sulfonyl]pyrrolidin-3-yl}-4,4'-bipyridin-2-amine;
4-({(3S,4R)-4-(4-fluorophenyl)-1-[(1-methyl-1H-imidazol-4-yl)sulfonyl]pyrrolidin-3-yl}oxy)-6-(trifluoromethyl)pyrimidine;
chloro-3-(trifluoromethyl)phenyl]-1-[(1-methyl-1H-imidazol-4-yl)sulfonyl]-4-(1,3-oxazol-4-yl)pyrrolidin-3-amine;
4,4-difluoro-N-{(3R,4S)-4-(4-fluorophenyl)-1-[(1-methyl-1H-imidazol-4-yl)sulfonyl]pyrrolidin-3-yl}pentanamide;
(3R,4S)-N-(3,4-difluorophenyl)-1-[(1,2-dimethyl-1H-imidazol-4-yl)sulfonyl] -4-(4-fluorophenyl)pyrrolidin-3-amine;
(3R,4S)-1-[(1,2-dimethyl-1H-imidazol-4-yl)sulfonyl]-4-(4-fluorophenyl)-N-[4-fluoro-3-(trifluoromethyl)phenyl]pyrrolidin-3-amine;
(3R,4S)-N-(3,4-difluorophenyl)-1-[(1,2-dimethyl-1H-imidazol-5-yl)sulfonyl] -4-(4-fluorophenyl)pyrrolidin-3-amine;
(3R,4S)-1-[(1,2-dimethyl-1H-imidazol-5-yl)sulfonyl]-4-(4-fluorophenyl)-N-[4-fluoro-3-(trifluoromethyl)phenyl]pyrrolidin-3-amine;
4-chloro-N-{(3R,4S)-4-(4-fluorophenyl)-1-[(1-methyl-1H-imidazol-4-yl)sulfonyl]pyrrolidin-3-yl}pyridin-2-amine;
5-chloro-N-[(3R,4S)-1-[(1,2-dimethyl-1H-imidazol-4-yl)sulfonyl]-4-(4-fluorophenyl)pyrrolidin-3-yl] -4-(trifluoromethyl)pyridin-2-amine;
trans-chloro-4-(trifluoromethyl)benzyl]-4-(3-fluoropyridin-2-yl)-1-[(1-methyl-1H-imidazol-4-yl)sulfonyl]pyrrolidin-3-amine;
trans-4-(3-fluoropyridin-2-yl)-N-hexyl-1-[(1-methyl-1H-imidazol-4-yl)sulfonyl]pyrrolidin-3-amine;
trans-4-(3-fluoropyridin-2-yl)-N,N-dihexyl-1-[(1-methyl-1H-imidazol-4-yl)sulfonyl]pyrrolidin-3-amine;
trans-4-(3-fluoropyridin-2-yl)-1-[(1-methyl-1H-imidazol-4-yl)sulfonyl]-N,N-dipentylpyrrolidin-3-amine;
trans-4-(3-fluoropyridin-2-yl)-1-[(1-methyl-1H-imidazol-4-yl)sulfonyl]-N-pentylpyrrolidin-3-amine;
trans-N-(3,4-difluorophenyl)-4-(3-fluoropyridin-2-yl)-1-[(1-methyl-1H-imidazol-4-yl)sulfonyl]pyrrolidin-3-amine;
trans-4-(3-fluoropyridin-2-yl)-N-[4-fluoro-3-(trifluoromethyl)phenyl]-1-[(1-methyl-1H-imidazol-4-yl)sulfonyl]pyrrolidin-3-amine;
trans-4-(3-fluoropyridin-2-yl)-N-[4-fluoro-3-(trifluoromethoxy)phenyl]-1-[(1-methyl-1H-imidazol-4-yl)sulfonyl]pyrrolidin-3-amine;
5-fluoro-N-{trans-4-(3-fluoropyridin-2-yl)-1-[(1-methyl-1H-imidazol-4-yl)sulfonyl]pyrrolidin-3-yl}-4-methylpyridin-2-amine;
N-{trans-4-(3-fluoropyridin-2-yl)-1-[(1-methyl-1H-imidazol-4-yl)sulfonyl]pyrrolidin-3-yl}-6-(trifluoromethyl)pyrimidin-4-amine;
trans-N-(3-chloro-4-fluorophenyl)-4-(3-fluoropyridin-2-yl)-1-[(1-methyl-1H-imidazol-4-yl)sulfonyl]pyrrolidin-3-amine;
6-chloro-N-{trans-4-(3-fluoropyridin-2-yl)-1-[(1-methyl-1H-imidazol-4-yl)sulfonyl]pyrrolidin-3-yl}pyrimidin-4-amine;
(3R,4S)-N-[3-(benzyloxy)phenyl]-1-[(1-methyl-1H-imidazol-4-yl)sulfonyl]-4-phenylpyrrolidin-3-amine;
6-(benzyloxy)-N-{(3R,4S)-4-(4-fluorophenyl)-1-[(1-methyl-1H-imidazol-4-yl)sulfonyl]pyrrolidin-3-yl}pyrimidin-4-amine;
trans-N-(4-fluoro-3-methylphenyl)-4-(3-fluoropyridin-2-yl)-1-[(1-methyl-1H-imidazol-4-yl)sulfonyl]pyrrolidin-3-amine;
4-(benzyloxy)-5-fluoro-N-{(3R,4S)-1-[(1-methyl-1H-imidazol-4-yl)sulfonyl]-4-phenylpyrrolidin-3-yl}pyridin-2-amine;
(3R,4S)-1-[(1-methyl-1H-imidazol-4-yl)sulfonyl]-4-phenyl-N-[3-(piperidin-4-yl)phenyl]pyrrolidin-3-amine;
6-(azetidin-3-ylmethoxy)-N-{(3R,4S)-4-(4-fluorophenyl)-1-[(1-methyl-1H-imidazol-4-yl)sulfonyl]pyrrolidin-3-yl}pyrimidin-4-amine;
N-{(3R,4S)-4-(4-fluorophenyl)-1-[(1-methyl-1H-imidazol-4-yl)sulfonyl]pyrrolidin-3-yl}-6-(pyrrolidin-3-ylmethoxy)pyrimidin-4-amine;
N-{(3R,4S)-4-(4-fluorophenyl)-1-[(1-methyl-1H-imidazol-4-yl)sulfonyl]pyrrolidin-3-yl}-6-(pyrrolidin-2-ylmethoxy)pyrimidin-4-amine;
trans-1-[(1-methyl-1H-imidazol-4-yl)sulfonyl]-N-(3-methylphenyl)-4-(tetrahydro-2H-pyran-3-yl)pyrrolidin-3-amine;
trans-1-[(1-methyl-1H-imidazol-4-yl)sulfonyl]-4-(tetrahydro-2H-pyran-3-yl)-N-[3-(trifluoromethyl)phenyl]pyrrolidin-3-amine;
trans-N-(3-chlorophenyl)-1-[(1-methyl-1H-imidazol-4-yl)sulfonyl]-4-(tetrahydro-2H-pyran-3-yl)pyrrolidin-3-amine;
trans-1-[(1-methyl-1H-imidazol-4-yl)sulfonyl]-4-[(3S)-tetrahydro-2H-pyran-3-yl]-N-[3-(trifluoromethoxy)phenyl]pyrrolidin-3-amine;
trans-1-[(1-methyl-1H-imidazol-4-yl)sulfonyl]-4-[(3S)-tetrahydro-2H-pyran-3-yl]-N-[3-(2,2,2-trifluoroethoxy)phenyl]pyrrolidin-3-amine;
trans-1-[(1-methyl-1H-imidazol-4-yl)sulfonyl]-N-(3-phenoxyphenyl)-4-[(3S)-tetrahydro-2H-pyran-3-yl]pyrrolidin-3-amine;
trans-N-(biphenyl-3-yl)-1-[(1-methyl-1H-imidazol-4-yl)sulfonyl]-4-[(3S)-tetrahydro-2H-pyran-3-yl]pyrrolidin-3-amine;
trans-N-[4-fluoro-3-(trifluoromethoxy)phenyl]-1-[(1-methyl-1H-imidazol-4-yl)sulfonyl]-4-[(3S)-tetrahydro-2H-pyran-3-yl]pyrrolidin-3-amine;
trans-N-(3-chloro-4-fluorophenyl)-1-[(1-methyl-1H-imidazol-4-yl)sulfonyl]-4-[(3S)-tetrahydro-2H-pyran-3-yl]pyrrolidin-3-amine;
trans-N-(4-fluoro-3-methylphenyl)-1-[(1-methyl-1H-imidazol-4-yl)sulfonyl]-4-[(3S)-tetrahydro-2H-pyran-3-yl]pyrrolidin-3-amine;
trans-N-[4-fluoro-3-(trifluoromethyl)phenyl]-1-[(1-methyl-1H-imidazol-4-yl)sulfonyl]-4-[(3S)-tetrahydro-2H-pyran-3-yl]pyrrolidin-3-amine;
trans-N-[4-chloro-3-(trifluoromethyl)phenyl]-1-[(1-methyl-1H-imidazol-4-yl)sulfonyl]-4-[(3S)-tetrahydro-2H-pyran-3-yl]pyrrolidin-3-amine;
trans-N-[4-chloro-3-(trifluoromethoxy)phenyl]-1-[(1-methyl-1H-imidazol-4-yl)sulfonyl]-4-[(3S)-tetrahydro-2H-pyran-3-yl]pyrrolidin-3-amine;
trans-N-[3-(benzyloxy)phenyl]-1-[(1-methyl-1H-imidazol-4-yl)sulfonyl]-4-[(3S)-tetrahydro-2H-pyran-3-yl]pyrrolidin-3-amine;
5-fluoro-N-{(3R,4S)-4-(4-fluorophenyl)-1-[(1-methyl-1H-imidazol-4-yl)sulfonyl]pyrrolidin-3-yl}-4-(trifluoromethyl)pyridin-2-amine;
3-fluoro-N-{(3R,4S)-4-(4-fluorophenyl)-1-[(1-methyl-1H-imidazol-4-yl)sulfonyl]pyrrolidin-3-yl}-4-(trifluoromethyl)pyridin-2-amine;
N-(2,2-difluoroethyl)-2-({(3R,4S)-4-(4-fluorophenyl)-1-[(1-methyl-1H-imidazol-4-yl)sulfonyl]pyrrolidin-3-yl}oxy)acetamide;
trans-4-(3-fluoropyridin-2-yl)-1-[(1-methyl-1H-imidazol-4-yl)sulfonyl]-N-[3-(trifluoromethyl)phenyl]pyrrolidin-3-amine;
trans-4-(3-fluoropyridin-2-yl)-1-[(1-methyl-1H-imidazol-4-yl)sulfonyl]-N-[3-(trifluoromethoxy)phenyl]pyrrolidin-3-amine;
trans-4-(3-fluoropyridin-2-yl)-1-[(1-methyl-1H-imidazol-4-yl)sulfonyl]-N-[3-(2,2,2-trifluoroethoxy)phenyl]pyrrolidin-3-amine;
(3R,4S)-4-(4-fluorophenyl)-1-[(1-methyl-1H-imidazol-4-yl)sulfonyl]-N-[3-(pyridin-2-yl)phenyl]pyrrolidin-3-amine;
(3R,4S)-1-[(1-methyl-1H-imidazol-4-yl)sulfonyl]-4-phenyl-N-[3-(pyridin-2-yl)phenyl]pyrrolidin-3-amine;
5-fluoro-N-{(3R,4S)-1-[(1-methyl-1H-imidazol-4-yl)sulfonyl]-4-phenylpyrrolidin-3-yl}-4-(2,2,2-trifluoroethoxy)pyridin-2-amine;
5-chloro-N-{trans-4-(3-fluoropyridin-2-yl)-1-[(1-methyl-1H-imidazol-4-yl)sulfonyl]pyrrolidin-3-yl} -4-(trifluoromethyl)pyridin-2-amine;
trans-N-(3-fluorophenyl)-4-(3-fluoropyridin-2-yl)-1-[(1-methyl-1H-imidazol-4-yl)sulfonyl]pyrrolidin-3-amine;
5-chloro-N-{(3R,4S)-4-(4-fluorophenyl)-1-[(1-methyl-1H-imidazol-4-yl)sulfonyl]pyrrolidin-3-yl}-4-(2,2,2-trifluoroethoxy)pyridin-2-amine;
N-{(3R,4S)-4-(4-fluorophenyl)-1-[(1-methyl-1H-imidazol-4-yl)sulfonyl]pyrrolidin-3-yl}-4-(2,2,2-trifluoroethoxy)pyridin-2-amine;
5-chloro-N-{(3R,4S)-1-[(1-methyl-1H-imidazol-4-yl)sulfonyl]-4-phenylpyrrolidin-3-yl}-4-(2,2,2-trifluoroethoxy)pyridin-2-amine;
N-{(3R,4S)-1-[(1-methyl-1H-imidazol-4-yl)sulfonyl]-4-phenylpyrrolidin-3-yl}-4-(2,2,2-trifluoroethoxy)pyridin-2-amine;
5-fluoro-N-{(3R,4S)-4-(4-fluorophenyl)-1-[(1-methyl-1H-imidazol-4-yl)sulfonyl]pyrrolidin-3-yl}-4-(2,2,2-trifluoroethoxy)pyridin-2-amine;
trans-4-(3-fluoropyridin-2-yl)-N-hexyl-1-[(1-methyl-1H-1,2,3-triazol-4-yl)sulfonyl]pyrrolidin-3-amine;
N-{(3R,4S)-4-(4-fluorophenyl)-1-[(1-methyl-1H-imidazol-4-yl)sulfonyl]pyrrolidin-3-yl}-4-(propan-2-yl)pyridin-2-amine;
N-{(3R,4S)-1-[(1-methyl-1H-imidazol-4-yl)sulfonyl]-4-phenylpyrrolidin-3-yl}-4-(propan-2-yl)pyridin-2-amine;
2,2,2-trifluoro-N-[2-({trans-4-(4-fluorophenyl)-1-[(1-methyl-1H-imidazol-4-yl)sulfonyl]pyrrolidin-3-yl}oxy)ethyl]ethanamine;
trans-N-(3-chloro-4-fluorophenyl)-1-[(1-methyl-1H-imidazol-4-yl)sulfonyl]-4-(tetrahydro-2H-pyran-2-yl)pyrrolidin-3-amine;
trans-1-[(1-methyl-1H-imidazol-4-yl)sulfonyl]-4-(tetrahydro-2H-pyran-2-yl)-N-[3-(2,2,2-trifluoroethoxy)phenyl]pyrrolidin-3-amine;
2-({1-[(1-methyl-1H-imidazol-4-yl)sulfonyl]-4-phenylpyrrolidin-3-yl}methyl)-4-(trifluoromethyl)pyridine;
trans-N-[2-chloro-4-(trifluoromethyl)benzyl]-4-(3-fluoropyridin-2-yl)-1-[(1-methyl-1H-1,2,3-triazol-4-yl)sulfonyl]pyrrolidin-3-amine;
trans-1-[(1-methyl-1H-imidazol-4-yl)sulfonyl]-4-(tetrahydrofuran-2-yl)-N-[3-(2,2,2-trifluoroethoxy)phenyl]pyrrolidin-3-amine;
N-hexyl-1-[(1-methyl-1H-imidazol-4-yl)sulfonyl]-4-[(2R)-tetrahydrofuran-2-yl]pyrrolidin-3-amine;
N-hexyl-1-[(1-methyl-1H-imidazol-4-yl)sulfonyl]-4-[(2S)-tetrahydrofuran-2-yl]pyrrolidin-3-amine;
trans-N-[2-chloro-4-(trifluoromethyl)benzyl]-1-[(1-methyl-1H-imidazol-4-yl)sulfonyl]-4-(tetrahydrofuran-2-yl)pyrrolidin-3-amine;
N-[2-chloro-4-(trifluoromethyl)benzyl]-1-[(1-methyl-1H-imidazol-4-yl)sulfonyl]-4-[(2S)-tetrahydrofuran-2-yl]pyrrolidin-3-amine;
trans-N-(3-chloro-4-fluorophenyl)-1-[(1-methyl-1H-imidazol-4-yl)sulfonyl]-4-(tetrahydrofuran-2-yl)pyrrolidin-3-amine;
N-(3-chloro-4-fluorophenyl)-1-[(1-methyl-1H-imidazol-4-yl)sulfonyl]-4-[(2S)-tetrahydrofuran-2-yl]pyrrolidin-3-amine;
1-benzyl-N-{(3R,4S)-1-[(1-methyl-1H-imidazol-4-yl)sulfonyl]-4-phenylpyrrolidin-3-yl}-2,3-dihydro-1H-indol-6-amine;
trans-1-[(1-methyl-1H-imidazol-4-yl)sulfonyl]-4-(tetrahydrofuran-3-yl)-N-[3-(trifluoromethyl)phenyl]pyrrolidin-3-amine;
trans-1-[(1-methyl-1H-imidazol-4-yl)sulfonyl]-N-(3-methylphenyl)-4-(tetrahydrofuran-3-yl)pyrrolidin-3-amine;
trans-N-(3-chlorophenyl)-1-[(1-methyl-1H-imidazol-4-yl)sulfonyl]-4-(tetrahydrofuran-3-yl)pyrrolidin-3-amine;
trans -N-(3-chloro-4-fluorophenyl)-1-[(1-methyl-1H-imidazol-4-yl)sulfonyl]-4-(tetrahydrofuran-3-yl)pyrrolidin-3-amine;
trans -N-[4-fluoro-3-(trifluoromethoxy)phenyl]-1-[(1-methyl-1H-imidazol-4-yl)sulfonyl]-4-(tetrahydrofuran-3-yl)pyrrolidin-3-amine;
trans -N-[4-fluoro-3-(trifluoromethyl)phenyl]-1-[(1-methyl-1H-imidazol-4-yl)sulfonyl]-4-(tetrahydrofuran-3-yl)pyrrolidin-3-amine;
trans -N-[4-chloro-3-(trifluoromethyl)phenyl]-1-[(1-methyl-1H-imidazol-4-yl)sulfonyl]-4-(tetrahydrofuran-3-yl)pyrrolidin-3-amine;
trans -1-[(1-methyl-1H-imidazol-4-yl)sulfonyl]-4-(tetrahydrofuran-3-yl)-N-[3-(2,2,2-trifluoroethoxy)phenyl]pyrrolidin-3-amine;
trans -1-[(1-methyl-1H-imidazol-4-yl)sulfonyl]-4-(tetrahydrofuran-3-yl)-N-[3-(trifluoromethoxy)phenyl]pyrrolidin-3-amine;
trans-4-(4-fluorophenyl)-1-[(1-methyl-1H-1,2,3-triazol-4-yl)sulfonyl]-N-[3-(2,2,2-trifluoroethoxy)phenyl]pyrrolidin-3-amine;
(3R, 4S)4-(4-fluorophenyl)-1-[(1-methyl-1H-1,2,3-triazol-4-yl)sulfonyl]-N-[3-(trifluoromethoxy)phenyl]pyrrolidin-3-amine;
(3R, 4S)-4-(4-fluorophenyl)-N-[4-fluoro-3-(trifluoromethoxy)phenyl]-1-[(1-methyl-1H-1,2,3-triazol-4-yl)sulfonyl]pyrrolidin-3-amine;
(3R, 4S)-4-(4-fluorophenyl)-1-[(1-methyl-1H-1,2,3-triazol-4-yl)sulfonyl]-N-[3-(trifluoromethyl)phenyl]pyrrolidin-3-amine;
(3R, 4S)-4-(4-fluorophenyl)-N-[4-fluoro-3-(trifluoromethyl)phenyl]-1-[(1-methyl-1H-1,2,3-triazol-4-yl)sulfonyl]pyrrolidin-3-amine;
(3R, 4S)-N-(3-chlorophenyl)-4-(4-fluorophenyl)-1-[(1-methyl-1H-1,2,3-triazol-4-yl)sulfonyl]pyrrolidin-3-amine;
(3R, 4S)-N-(3-chloro-4-fluorophenyl)-4-(4-fluorophenyl)-1-[(1-methyl-1H-1,2,3-triazol-4-yl)sulfonyl]pyrrolidin-3-amine;
trans-N-[4-fluoro-3-(trifluoromethyl)phenyl]-1-[(1-methyl-1H-imidazol-4-yl)sulfonyl]-4-(tetrahydrofuran-2-yl)pyrrolidin-3-amine;
5-Chloro-N-[trans-1-[(1-methyl-1H-imidazol-4-yl)sulfonyl]-4-(tetrahydrofuran-2-yl)pyrrolidin-3-yl]-4-(trifluoromethyl)pyridin-2-amine;
trans-N-(4-fluoro-3-methylphenyl)-1-[(1-methyl-1H-imidazol-4-yl)sulfonyl]-4-(tetrahydrofuran-2-yl)pyrrolidin-3-amine;
trans-N-[4-fluoro-3-(trifluoromethyl)phenyl]-1-[(1-methyl-1H-imidazol-4-yl)sulfonyl]-4-(tetrahydro-2H-pyran-2-yl)pyrrolidin-3-amine;
trans-N-(4-fluoro-3-methylphenyl)-1-[(1-methyl-1H-imidazol-4-yl)sulfonyl]-4-(tetrahydro-2H-pyran-2-yl)pyrrolidin-3-amine;
5-chloro-N-[trans-1-[(1-methyl-1H-imidazol-4-yl)sulfonyl]-4-(tetrahydro-2H-pyran-2-yl)pyrrolidin-3-yl]-4-(trifluoromethyl)pyridin-2-amine;
trans-N-(3-chlorophenyl)-1-[(1-methyl-1H-imidazol-4-yl)sulfonyl]-4-(tetrahydro-2H-pyran-2-yl)pyrrolidin-3-amine;
trans-N-(3-chlorophenyl)-1-[(1-methyl-1H-imidazol-4-yl)sulfonyl]-4-(tetrahydrofuran-2-yl)pyrrolidin-3-amine;
5-fluoro-4-methyl-N-[trans-1-[(1-methyl-1H-imidazol-4-yl)sulfonyl]-4-(tetrahydrofuran-2-yl)pyrrolidin-3-yl]pyridin-2-amine;
5-fluoro-4-methyl-N-[trans-1-[(1-methyl-1H-imidazol-4-yl)sulfonyl]-4-(tetrahydro-2H-pyran-2-yl)pyrrolidin-3-yl]pyridin-2-amine;
N-[trans-1-[(1-methyl-1H-imidazol-4-yl)sulfonyl]-4-(tetrahydrofuran-2-yl)pyrrolidin-3-yl]-6-(trifluoromethyl)pyrimidin-4-amine;
(3R,4S)-N-cyclohexyl-4-(4-fluorophenyl)-1-(1-methyl-1H-imidazol-4-ylsulfonyl)pyrrolidin-3-amine;
(3R,4S)-N-(3,3-dimethylcyclohexyl)-4-(4-fluorophenyl)-1-(1-methyl-1H-imidazol-4-ylsulfonyl)pyrrolidin-3-amine;
(3R,4S)-N-(4,4-dimethylcyclohexyl)-4-(4-fluorophenyl)-1-(1-methyl-1H-imidazol-4-ylsulfonyl)pyrrolidin-3-amine;
(3R,4S)-N-cyclopentyl-4-(4-fluorophenyl)-1-(1-methyl-1H-imidazol-4-ylsulfonyl)pyrrolidin-3-amine;
(3R,4S)-N-(3,3-dimethylcyclopentyl)-4-(4-fluorophenyl)-1-(1-methyl-1H-imidazol-4-ylsulfonyl)pyrrolidin-3-amine;
(3R,4S)-N-(1-(2,4-dichlorophenyl)ethyl)-4-(4-fluorophenyl)-1-(1-methyl-1H-imidazol-4-ylsulfonyl)pyrrolidin-3-amine;
(3R,4S)-N-(2,3-dihydro-1H-inden-1-yl)-4-(4-fluorophenyl)-1-(1-methyl-1H-imidazol-4-ylsulfonyl)pyrrolidin-3-amine;
(3R,4S)-N-(5-chloro-2,3-dihydro-1H-inden-1-yl)-4-(4-fluorophenyl)-1-(1-methyl-1H-imidazol-4-ylsulfonyl)pyrrolidin-3-amine;
N-(4-chlorobenzyl)-N-((3R,4S)-4-(4-fluorophenyl)-1-(1-methyl-1H-imidazol-4-ylsulfonyl)pyrrolidin-3-yl)acetamide;
5-butyl-3-{(3R,4S)-4-(4-fluorophenyl)-1-[(1-methyl-1H-imidazol-4-yl)sulfonyl]pyrrolidin-3-yl}-1,3-oxazolidin-2-one;
4-{(3R,4S)-4-(4-fluorophenyl)-1-[(1-methyl-1H-imidazol-4-yl)sulfonyl]pyrrolidin-3-yl}thiomorpholine 1,1-dioxide;
5-fluoro-N-{(3R,4S)-4-(4-fluorophenyl)-1-[(1-methyl-1H-imidazol-4-yl)sulfonyl]pyrrolidin-3-yl}-4-(trifluoromethyl)pyridin-2-amine;
(3R,4S)-1-[(1-methyl-1H-imidazol-4-yl)sulfonyl]-4-[(2R)-tetrahydro-2H-pyran-2-yl]-N-[3-(trifluoromethoxy)phenyl]pyrrolidin-3-amine;
(3R,4S)-4-(4-fluorophenyl)-1-[(1-methyl-1H-imidazol-4-yl)sulfonyl]-N-[2-methyl-5-(prop-1-en-2-yl)cyclohexyl]pyrrolidin-3-amine;
(3R,4S)-4-(4-fluorophenyl)-1-[(1-methyl-1H-imidazol-4-yl)sulfonyl]-N-(2,2,2-trifluoro-1-phenylethyl)pyrrolidin-3-amine;
N-[2-chloro-4-(trifluoromethyl)benzyl]-2-[(1-methyl-1H-imidazol-4-yl)sulfonyl]octahydro-3aH-isoindol-3a-amine;
2-chloro-N-{2-[(1-methyl-1H-imidazol-4-yl)sulfonyl]octahydro-3aH-isoindol-3a-yl}-3-(trifluoromethyl)benzamide;
2.4-dichloro-N-{2-[(1-methyl-1H-imidazol-4-yl)sulfonyl]octahydro-3aH-isoindol-3a-yl}benzamide;
1-hexyl-6-[(1-methyl-1H-imidazol-4-yl)sulfonyl]octahydro-1H-pyrrolo[3,4-b]pyridine;
(2,4-dichlorophenyl){6-[(1-methyl-1H-imidazol-4-yl)sulfonyl]octahydro-1H-pyrrolo[3,4-b]pyridin-1-yl}methanone;
[2-chloro-3-(trifluoromethyl)phenyl]{6-[(1-methyl-1H-imidazol-4-yl)sulfonyl]octahydro-1H-pyrrolo[3,4-b]pyridin-1-yl}methanone;
(3R,4S)-4-(4-fluorophenyl)-1-[(1-methyl-1H-imidazol-4-yl)sulfonyl]-N-[2,2,2-trifluoro-1-(3-methylphenyl)ethyl]pyrrolidin-3-amine;
(3R,4S)-4-(4-fluorophenyl)-1-[(1-methyl-1H-imidazol-4-yl)sulfonyl]-N-[2,2,2-trifluoro-1-(4-methylphenyl)ethyl]pyrrolidin-3-amine;
(1R,2R,3R,4S)-3-(4-fluorophenyl)-N-[4-fluoro-3-(trifluoromethyl)phenyl]-7-[(1-methyl-1H-imidazol-4-yl)sulfonyl]-7-azabicyclo[2.2.1]heptan-2-amine;
3-(4-fluorophenyl)-N-[4-fluoro-3-(trifluoromethyl)phenyl]-7-[(1-methyl-1H-imidazol-4-yl)sulfonyl]-7-azabicyclo[2.2.1]heptan-2-amine;
1-[2-chloro-4-(trifluoromethyl)benzyl]-6-[(1-methyl-1H-imidazol-4-yl)sulfonyl]octahydro-1H-pyrrolo[3,4-b]pyridine;
[2-chloro-4-(trifluoromethyl)phenyl]{6-[(1-methyl-1H-imidazol-4-yl)sulfonyl]octahydro-1H-pyrrolo[3,4-b]pyridin-1-yl}methanone;
N-(3-chloro-4-fluorobenzyl)-2-[(1-methyl-1H-imidazol-4-yl)sulfonyl]-2-azaspiro[4.5]decane-4-carboxamide;
N-(3,4-difluorobenzyl)-2-[(1-methyl-1H-imidazol-4-yl)sulfonyl]-2-azaspiro[4.5]decane-4-carboxamide;
1-{(3R,4S)-4-(4-fluorophenyl)-1-[(1-methyl-1H-imidazol-4-yl)sulfonyl]pyrrolidin-3-yl}-1,3-dihydro-2H-indol-2-one;
2-[(1-methyl-1H-imidazol-4-yl)sulfonyl]-N-[4-(trifluoromethyl)benzyl]octahydro-3aH-isoindol-3a-amine;
trans-N-[2-chloro-4-(trifluoromethyl)benzyl]-2-[(1-methyl-1H-imidazol-4-yl)sulfonyl]-2,3,3a,8-tetrahydroindeno[1,2-c]pyrrol-8a(1H)-amine;
2-{4-[(4-fluorophenoxy)methyl]-1-[(1-methyl-1H-imidazol-4-yl)sulfonyl]pyrrolidin-3-yl}pyridine;
2-[(1-methyl-1H-imidazol-4-yl)sulfonyl]-3a-{[6-(trifluoromethyl)pyrimidin-4-yl]oxy}-2,3,3a,4,5,9b-hexahydro-1H-benzo[e]isoindole;
N-(4-fluorobenzyl)-2-[(1-methyl-1H-imidazol-4-yl)sulfonyl]-1,2,3,4,5,9b-hexahydro-3aH-benzo[e]isoindol-3a-amine;
N-[2-chloro-4-(trifluoromethyl)benzyl]-2-[(1-methyl-1H-imidazol-4-yl)sulfonyl]-1,2,3,4,5,9b-hexahydro-3aH-benzo[e]isoindol-3a-amine;
2-[(1-methyl-1H-1,2,3-triazol-4-yl)sulfonyl]-N-[6-(trifluoromethyl)pyrimidin-4-yl]-2,3,3a,8-tetrahydroindeno[1,2-c]pyrrol-8a(1H)-amine;
2-[(1-methyl-1H-imidazol-4-yl)sulfonyl]-N-[4-(trifluoromethyl)pyridin-2-yl]-2,3,3a,8-tetrahydroindeno[1,2-c]pyrrol-8a(1H)-amine;
N-(2,3-dihydro-1H-inden-1-yl)-1-[(1-methyl-1H-imidazol-4-yl)sulfonyl]-4-(1-methylpiperidin-2-yl)pyrrolidin-3-amine;
1-[(1-methyl-1H-imidazol-4-yl)sulfonyl]-4-(1-methylpiperidin-2-yl)-N-[3-(2,2,2-trifluoroethoxy)phenyl]pyrrolidin-3-amine;
4-(5-fluoropyridin-2-yl)-1-[(1-methyl-1H-imidazol-4-yl)sulfonyl]-N-[3-(trifluoromethoxy)phenyl]pyrrolidin-3-amine;
(3R,4S)-4-(1,3-dioxan-2-yl)-1-[(1-methyl-1H-1,2,3-triazol-4-yl)sulfonyl]-N-[3-(trifluoromethoxy)phenyl]pyrrolidin-3-amine;
(3R,4S)-4-(1,3-dioxan-2-yl)-1-[(1-methyl-1H-imidazol-4-yl)sulfonyl]-N-[3-(trifluoromethoxy)phenyl]pyrrolidin-3-amine;
1-[(1-methyl-1H-imidazol-4-yl)sulfonyl]-4-(1-methylpiperidin-2-yl)-N-[3-(trifluoromethoxy)phenyl]pyrrolidin-3-amine;
1-[(1-methyl-1H-imidazol-4-yl)sulfonyl]-4-(1-methylpiperidin-2-yl)-N-[3-(trifluoromethoxy)phenyl]pyrrolidin-3-amine;
(3R,4S)-4-(5,5-dimethyl-1,3-dioxan-2-yl)-1-[(1-methyl-1H-imidazol-4-yl)sulfonyl]-N-[3-(trifluoromethoxy)phenyl]pyrrolidin-3-amine;
(3R,4S)-4-(1,3-dioxepan-2-yl)-1-[(1-methyl-1H-imidazol-4-yl)sulfonyl]-N-[3-(trifluoromethoxy)phenyl]pyrrolidin-3-amine;
(3R,4S)-4-(1,3-dioxolan-2-yl)-1-[(1-methyl-1H-imidazol-4-yl)sulfonyl]-N-[3-(trifluoromethoxy)phenyl]pyrrolidin-3-amine;
(3R,4S)-N-(2,3-dihydro-1H-inden-1-yl)-4-(1,3-dioxan-2-yl)-1-[(1-methyl-1H-imidazol-4-yl)sulfonyl]pyrrolidin-3-amine;
(3R,4S)-N-[2-chloro-4-(trifluoromethyl)benzyl]-4-(1,3-dioxan-2-yl)-1-[(1-methyl-1H-imidazol-4-yl)sulfonyl]pyrrolidin-3-amine;
(3R,4S)-4-(1,3-dioxan-2-yl)-1-[(1-methyl-1H-imidazol-4-yl)sulfonyl]-N-[3-(trifluoromethoxy)benzyl]pyrrolidin-3-amine;
(3R,4S)-4-(1,3-dioxan-2-yl)-N-(4-fluorobenzyl)-1-[(1-methyl-1H-imidazol-4-yl)sulfonyl]pyrrolidin-3-amine;
(3R,4S)-4-(1,3-dioxan-2-yl)-N-methyl-1-[(1-methyl-1H-imidazol-4-yl)sulfonyl]-N-pentylpyrrolidin-3-amine;
(3R,4S)-4-(1,3-dioxan-2-yl)-1-[(1-methyl-1H-imidazol-4-yl)sulfonyl]-N,N-dipentylpyrrolidin-3-amine;
(3R,4S)-4-(1,3-dioxan-2-yl)-N,N-dihexyl-1-[(1-methyl-1H-imidazol-4-yl)sulfonyl]pyrrolidin-3-amine;
(3R,4S)-4-(1,3-dioxan-2-yl)-N-hexyl-1-[(1-methyl-1H-imidazol-4-yl)sulfonyl]pyrrolidin-3-amine;
5-chloro-N-{(3R,4S)-4-(1,3-dioxan-2-yl)-1-[(1-methyl-1H-imidazol-4-yl)sulfonyl]pyrrolidin-3-yl}-4-(trifluoromethyl)pyridin-2-amine;
(3R,4S)-4-(1,3-dioxan-2-yl)-N-[4-fluoro-3-(trifluoromethyl)phenyl]-1-[(1-methyl-1H-imidazol-4-yl)sulfonyl]pyrrolidin-3-amine;
1-[(1-methyl-1H-imidazol-4-yl)sulfonyl]-4-(piperidin-1-yl)-N-[3-(trifluoromethoxy)phenyl]pyrrolidin-3-amine;
(3R,4S)-N-(3-chlorophenyl)-4-(1,3-dioxan-2-yl)-1-[(1-methyl-1H-imidazol-4-yl)sulfonyl]pyrrolidin-3-amine;
(3R,4S)-4-(1,3-dioxan-2-yl)-1-[(1-methyl-1H-imidazol-4-yl)sulfonyl]-N-[3-(trifluoromethyl)phenyl]pyrrolidin-3-amine;
(3R,4S)-N-(3-chloro-4-fluorophenyl)-4-(1,3-dioxan-2-yl)-1-[(1-methyl-1H-imidazol-4-yl)sulfonyl]pyrrolidin-3-amine;
(3R,4S)-4-(1,3-dioxan-2-yl)-1-[(1-methyl-1H-imidazol-4-yl)sulfonyl]-N-(3-methylphenyl)pyrrolidin-3-amine;
4-({3-(1,3-dioxan-2-yl)-4-[3-(trifluoromethyl)benzyl]pyrrolidin-1-yl}sulfonyl)-1-methyl-1H-imidazole;
4-({3-(1,3-dioxan-2-yl)-4-[3-(trifluoromethyl)benzyl]pyrrolidin-1-yl}sulfonyl)-1-methyl-1H-1,2,3-triazole;
(3R,4S)-4-(5,7-dioxaspiro[2.5]oct-6-yl)-1-[(1-methyl-1H-imidazol-4-yl)sulfonyl]-N-[3-(trifluoromethoxy)phenyl]pyrrolidin-3-amine;
(3R,4S)-4-[(4R,6R)-4,6-dimethyl-1,3-dioxan-2-yl]-1-[(1-methyl-1H-imidazol-4-yl)sulfonyl]-N-[3-(trifluoromethoxy)phenyl]pyrrolidin-3-amine;
(3R,4S)-4-[(4S,6S)-4,6-dimethyl-1,3-dioxan-2-yl]-1-[(1-methyl-1H-imidazol-4-yl)sulfonyl]-N-[3-(trifluoromethoxy)phenyl]pyrrolidin-3-amine;
(3R,4S)-4-(1,3-dioxan-2-yl)-N-(4-fluoro-3-methylphenyl)-1-[(1-methyl-1H-imidazol-4-yl)sulfonyl]pyrrolidin-3-amine;
(3R,4S)-4-(1,3-dioxan-2-yl)-N-[4-fluoro-3-(trifluoromethoxy)phenyl]-1-[(1-methyl-1H-imidazol-4-yl)sulfonyl]pyrrolidin-3-amine;
(3R,4S)-N-[3-(difluoromethoxy)phenyl]-4-(1,3-dioxan-2-yl)-1-[(1-methyl-1H-imidazol-4-yl)sulfonyl]pyrrolidin-3-amine;
(3R,4S)-4-(1,3-dioxan-2-yl)-1-[(1-methyl-1H-imidazol-4-yl)sulfonyl]-N-[3-(propan-2-yl)phenyl]pyrrolidin-3-amine;
4-(5-fluoropyridin-2-yl)-N-[4-fluoro-3-(trifluoromethyl)phenyl]-1-[(1-methyl-1H-imidazol-4-yl)sulfonyl]pyrrolidin-3-amine;
(3R,4S)-N-[3-(2,2-difluoroethoxy)phenyl]-4-(1,3-dioxan-2-yl)-1-[(1-methyl-1H-imidazol-4-yl)sulfonyl]pyrrolidin-3-amine,
or a physiologically tolerated salt thereof.

16. The compound as claimed in claim 1, which is (3R,4S)-1-[(1-methyl-1H-imidazol-4-yl)sulfonyl]-4-[(2R)-tetrahydro-2H-pyran-2-yl]-N-[3-(trifluoromethoxy)phenyl]pyrrolidin-3-amine or a physiologically tolerated salt thereof.

17. The compound as claimed in claim 1, which is (3R,4S)-4-(1,3-dioxan-2-yl)-1-[(1-methyl-1H-imidazol-4-yl)sulfonyl]-N-[3-(trifluoromethoxy)phenyl]pyrrolidin-3-amine or a physiologically tolerated salt thereof.

18. The compound as claimed in claim 1, which is (3R,4S)-N-(2,3-dihydro-1H-inden-1-yl)-4-(4-fluorophenyl)-1-(1-methyl-1H-imidazol-4-ylsulfonyl)pyrrolidin-3-amine or a physiologically tolerated salt thereof.

19. The compound as claimed in claim 1, which is 4-({(3S,4R)-4-(4-fluorophenyl)-1-[(1-methyl-1H-imidazol-4-yl)sulfonyl]pyrrolidin-3-yl}oxy)-6-(trifluoromethyl)pyrimidine or a physiologically tolerated salt thereof.

## Patentansprüche

1. Pyrrolidinderivat der Formel (I) 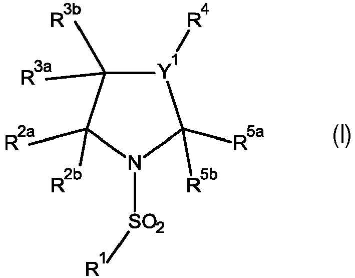 worin
R¹ für einen 5-gliedrigen heterocyclischen Ring steht, ausgewählt unter Pyrazolyl, Imidazolyl und Triazolyl, welcher gegebenenfalls mit 1, 2 oder 3 Substituenten substituiert ist, die unabhängig unter Halogen, C₁-C₆-Alkyl, halogeniertem C₁-C₆-Alkyl, C₃-C₆-Cycloalkyl, C₁-C₄-Alkoxycarbonyl und C₁-C₆-Alkylcarbonylamino ausgewählt sind;
R^{2a}, R^{2b} unabhängig für Wasserstoff, Halogen oder C₁-C₃-Alkyl stehen, oder
R^{2a}, R^{2b} gemeinsam mit dem Kohlenstoffatom, an das sie gebunden sind, C=O bilden;
R^{3a} für C₃-C₁₂-Cycloalkyl, gegebenenfalls substituiertes C₆-C₁₂-Aryl, Hydroxy, C₁-C₆-Alkoxy, halogeniertes C₁-C₆-Alkoxy, C₃-C₁₂-Cycloalkyl-C₁-C₄-alkoxy, C₂-C₆-Alkenyloxy, C₆-C₁₂-Aryl-C₁-C₄-alkoxy, C₃-C₁₂-Heterocyclyl-C₁-C₄-alkoxy, gegebenenfalls substituiertes C₆-C₁₂-Aryloxy, C₃-C₁₂-Heterocyclyloxy oder gegebenenfalls substituiertes C₃-C₁₂-Heterocyclyl steht; oder
R^{3b} für Wasserstoff, C₁-C₆-Alkyl oder Hydroxyl steht; oder
R^{3a} und R^{3b} gemeinsam für gegebenenfalls substituiertes C₂-C₅-Alkylen stehen;
Y¹ für >CR⁶- oder >N-steht;
R⁶ für Wasserstoff, C₁-C₆-Alkyl, halogeniertes C₁-C₆-Alkyl, (gegebenenfalls substituiertes C₆-C₁₂-Aryl)-C₁-C₄-alkyl, Hydroxy-C₁-C₆-alkyl, C₁-C₄-Alkoxy-C₁-C₆-alkyl oder Hydroxyl steht; oder
R⁶ und R^{3a} oder R^{3b} gemeinsam für gegebenenfalls substituiertes C₁-C₅-Alkylen stehen; oder
R⁶ für C₁-C₄-Alkylen steht, welches an ein Kohlenstoffatom in R^{3a} gebunden ist, und R^{3a} für gegebenenfalls substituiertes C₆-C₁₂-Aryl oder gegebenenfalls substituiertes C₃-C₁₂-Heterocyclyl steht;
R⁴ für -NR^{8a}(CR^{9a}R^{9b})ₙ₄R¹³, -(CR^{7a}R^{7b})ₙ₁OR¹⁰, -(CR^{7c}R^{7d})ₙ₂NR^{11a}R^{11b}, -(CR^{7e}R^{7f})ₙ₃R¹², gegebenenfalls substituiertes C₆-C₁₂-Aryl, -NR^{8b}COR¹⁴, -NR^{8c}COOR¹⁵, -NR^{8d}CONR^{16a}R^{16b},-NR^{8e}SO₂R¹⁷, -O(CR^{9c}R^{9d})ₙ₅R¹⁸, -COR¹⁹, -CONR^{20a}R^{20b}, -SO₂R²¹ oder gegebenenfalls substituiertes C₃-C₁₂-Heterocyclyl steht;
R^{7a}, R^{7b} unabhängig für Wasserstoff oder C₁-C₆-Alkyl stehen,
n1 für 1, 2, 3 oder 4 steht;
R¹⁰ für Wasserstoff, gegebenenfalls substituiertes C₆-C₁₂-Aryl oder gegebenenfalls substituiertes C₃-C₁₂-Heterocyclyl steht;
R^{7c}, R^{7d} unabhängig für Wasserstoff oder C₁-C₆-Alkyl steht,
n2 für 1, 2, 3 oder 4 steht;
R^{11a} für C₁-C₈-Alkyl, (gegebenenfalls substituiertes C₃-C₁₂-Cycloalkyl)-C₁-C₄-alkyl, C₁-C₆-Alkoxy-C₁-C₆-alkyl, (gegebenenfalls substituiertes C₆-C₁₂-Aryl)-C₁-C₄-alkyl, (gegebenenfalls substituiertes C₃-C₁₂-Heterocyclyl)-C₁-C₄-alkyl, gegebenenfalls substituiertes C₆-C₁₂-Aryl oder gegebenenfalls substituiertes C₃-C₁₂-Heterocyclyl steht;
R^{11b} für Wasserstoff oder C₁-C₆-Alkyl steht;
R^{7e}, R^{7f} unabhängig für Wasserstoff oder C₁-C₆-Alkyl,
n3 für 1, 2, 3 oder 4 steht;
R¹² für gegebenenfalls substituiertes C₆-C₁₂-Aryl oder gegebenenfalls substituiertes C₃-C₁₂-Heterocyclyl steht;
R^{8a}, R^{8b}, R^{8c}, R^{8d}, R^{8e} unabhängig für Wasserstoff, C₁-C₆-Alkyl oder C₁-C₆-Alkylcarbonyl, oder
R⁶ und eines von R^{8a}, R^{8b}, R^{8c}, R^{8d} oder R^{8e} gemeinsam für gegebenenfalls substituiertes C₁-C₅-Alkylen stehen, worin ein oder mehrere -CH₂- von C₁-C₅-Alkylen unabhängig durch ein Sauerstoffatom oder C=O ersetzt sein können; oder
R^{3a} und eines von R^{8a} oder R^{8b} gemeinsam für gegebenenfalls substituiertes C₁-C₅-Alkylen stehen;
R^{9a}, R^{9b} unabhängig für Wasserstoff, Halogen, C₁-C₆-Alkyl, halogeniertes C₁-C₆-Alkyl, hydroxyl oder C₁-C₆-Alkoxy steht;
n4 für 0, 1, 2, 3 oder 4 steht;
R¹³ für Wasserstoff, C₁-C₈-Alkyl, halogeniertes C₁-C₆-Alkyl, (gegebenenfalls substituiertes C₃-C₁₂-Cycloalkyl)-C₁-C₄-alkyl, C₁-C₆-Alkoxy-C₁-C₆-alkyl, gegebenenfalls substituiertes C₃-C₁₂-Cycloalkyl, C₂-C₆-Alkenyl, gegebenenfalls substituiertes C₃-C₆-Cycloalkenyl, gegebenenfalls substituiertes C₆-C₁₂-Aryl, Hydroxy, C₁-C₆-Alkoxy, C₁-C₆-Alkoxy-C₁-C₄-alkoxy, gegebenenfalls substituiertes C₆-C₁₂-Aryloxy, gegebenenfalls substituiertes C₃-C₁₂-Heterocyclyloxy, gegebenenfalls substituiertes C₃-C₁₂-Heterocyclyl oder Tri-(C₁-C₄-alkyl)-silyloxy steht;
R¹⁴ für C₁-C₈-Alkyl, halogeniertes C₁-C₆-Alkyl, (gegebenenfalls substituiertes C₃-C₁₂-Cycloalkyl)-C₁-C₄-alkyl, (gegebenenfalls substituiertes C₆-C₁₂-Aryl)-C₁-C₄-alkyl, Hydroxy-C₁-C₆-alkyl, C₁-C₆-Alkoxy-C₁-C₆-alkyl, (gegebenenfalls substituiertes C₆-C₁₂-Aryloxy)-C₁-C₄-alkyl, C₁-C₆-Alkylcarbonyl-C₁-C₄-alkyl, C₁-C₆-Alkoxycarbonyl-C₁-C₄-alkyl, C₁-C₆-Alkylaminocarbonyl-C₁-C₄-alkyl, gegebenenfalls substituiertes (C₃-C₁₂-Heterocyclyl)-C₁-C₄-alkyl, gegebenenfalls substituiertes C₃-C₁₂-Cycloalkyl, gegebenenfalls substituiertes C₆-C₁₂-Aryl oder gegebenenfalls substituiertes C₃-C₁₂-Heterocyclyl steht;
R¹⁵ für C₁-C₈-Alkyl, gegebenenfalls substituiertes C₆-C₁₂-Aryl oder gegebenenfalls substituiertes C₃-C₁₂-Heterocyclyl steht;
R^{16a} für (gegebenenfalls substituiertes C₆-C₁₂-Aryl)-C₁-C₄-alkyl, gegebenenfalls substituiertes (C₃-C₁₂-Heterocyclyl)-C₁-C₄-alkyl, gegebenenfalls substituiertes C₆-C₁₂-Aryl oder gegebenenfalls substituiertes C₃-C₁₂-Heterocyclyl steht;
R^{16b} für Wasserstoff oder C₁-C₆-Alkyl steht;
R¹⁷ für (gegebenenfalls substituiertes C₆-C₁₂-Aryl)-C₁-C₄-alkyl, (gegebenenfalls substituiertes C₃-C₁₂-Heterocyclyl)-C₁-C₄-alkyl, gegebenenfalls substituiertes C₆-C₁₂-Aryl oder gegebenenfalls substituiertes C₃-C₁₂-Heterocyclyl steht;
R^{9c}, R^{9d} unabhängig für Wasserstoff, Halogen oder C₁-C₆-Alkyl stehen;
n5 für 0, 1, 2, 3 oder 4 steht;
R¹⁸ für Wasserstoff, C₁-C₈-Alkyl, gegebenenfalls substituiertes C₃-C₁₂-Cycloalkyl, C₁-C₆-Alkylcarbonyl, C₁-C₆-Alkoxycarbonyl, halogeniertes C₁-C₆-Alkoxycarbonyl, C₆-C₁₂-Aryloxycarbonyl, C₁-C₆-Alkylaminocarbonyl, (halogeniertes C₁-C₄-Alkyl)aminocarbonyl, C₆-C₁₂-Arylaminocarbonyl, gegebenenfalls substituiertes C₆-C₁₂-Aryl, C₁-C₆-Alkylamin, (C₃-C₁₂-Cycloalkyl-C₁-C₄-alkyl)amino, (halogeniertes C₁-C₆-Alkyl)amino, (C₁-C₆-Alkoxy-C₁-C₆-alkyl)amino, (C₆-C₁₂-Aryl-C₁-C₄-alkyl)amino, C₁-C₆-Dialkylamin, gegebenenfalls substituiertes C₆-C₁₂-Arylamin oder gegebenenfalls substituiertes C₃-C₁₂-Heterocyclyl steht;
R¹⁹ für gegebenenfalls substituiertes C₆-C₁₂-Aryl oder gegebenenfalls substituiertes C₃-C₁₂-Heterocyclyl steht;
R^{20a} für C₁-C₈-Alkyl, (gegebenenfalls substituiertes C₃-C₁₂-Cycloalkyl)-C₁-C₄-alkyl, C₁-C₆-Alkoxy-C₁-C₆-alkyl, (gegebenenfalls substituiertes C₆-C₁₂-Aryl)-C₁-C₄-alkyl, (gegebenenfalls substituiertes C₃-C₁₂-Heterocyclyl)-C₁-C₄-alkyl, gegebenenfalls substituiertes C₆-C₁₂-Aryl oder gegebenenfalls substituiertes C₃-C₁₂-Heterocyclyl steht;
R^{20b} für Wasserstoff oder C₁-C₈-Alkyl steht;
R²¹ für gegebenenfalls substituiertes C₆-C₁₂-Aryl oder gegebenenfalls substituiertes C₃-C₁₂-Heterocyclyl steht; und
R^{5a}, R^{5b} unabhängig für Wasserstoff, Halogen oder C₁-C₃-Alkyl stehen, oder
R^{5a}, R^{5b} gemeinsam mit dem Kohlenstoffatom, an das sie gebunden sind, C=O bilden; oder
eines von R^{2a} oder R^{2b} und eines von R^{5a} oder R^{5b} gemeinsam für gegebenenfalls substituiertes C₁-C₅-Alkylen stehen,
oder ein physiologisch toleriertes Salz davon;
mit der Maßgabe, dass die folgenden Verbindungen ausgeschlossen sind:
Verbindungen der Formel (II) und die physiologisch tolerierten Salze davon; 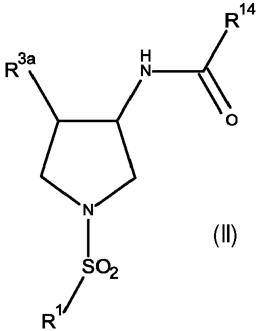 worin R¹, R^{3a} und R¹⁴ so sind, wie in der folgenden Tabelle definiert:
| R¹ | R^{3a} | R¹⁴ |
|---|---|---|
| 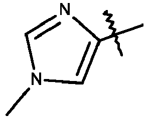 | 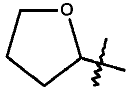 | 4-F-Ph |
| 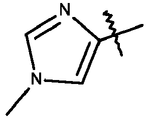 | 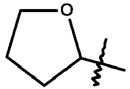 | 4-CF₃-Ph |
| 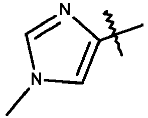 | 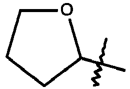 | 2-OMe-Ph |
| 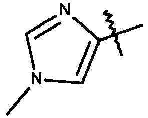 | 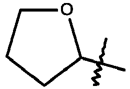 | 2-OMe-4-OCF₃-Ph |
| 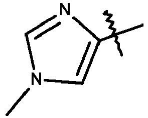 | 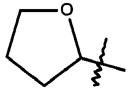 | 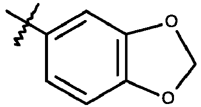 |
| 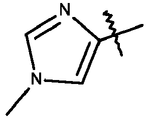 | 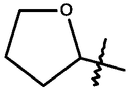 | 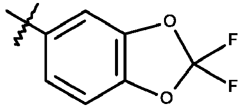 |
| 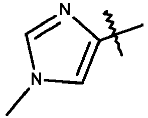 | 4-F-Phenyl | 4-CF₃-Phenyl |
| 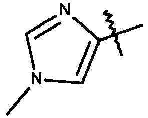 | 4-F-Phenyl | 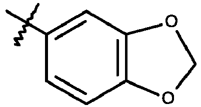 |
Verbindungen der Formel (II') 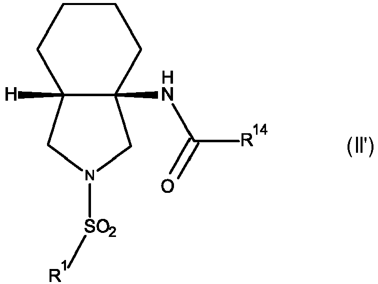 worin R¹ und R¹⁴ so sind, wie in der folgenden Tabelle definiert:
| R¹ | R¹⁴ |
|---|---|
| 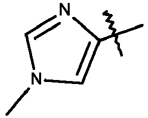 | 4-F-Ph |
| 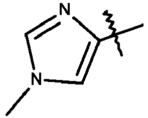 | 2-OMe-Ph |
| 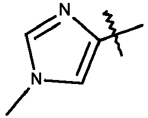 | 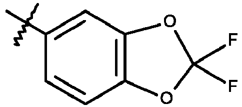 |
und die Verbindungen der Formeln:

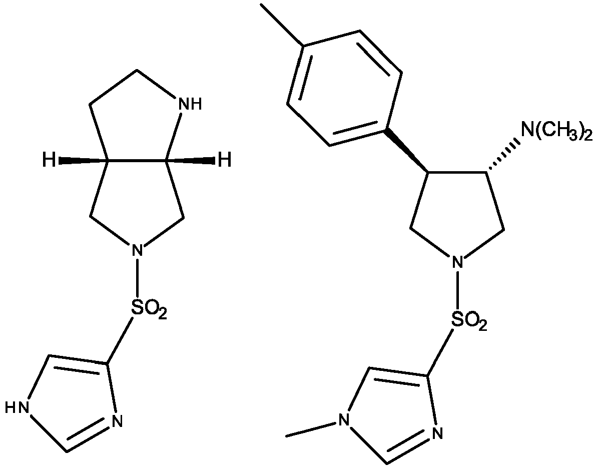

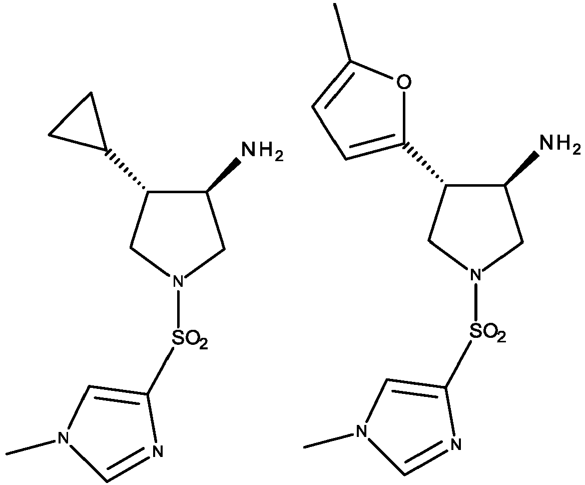

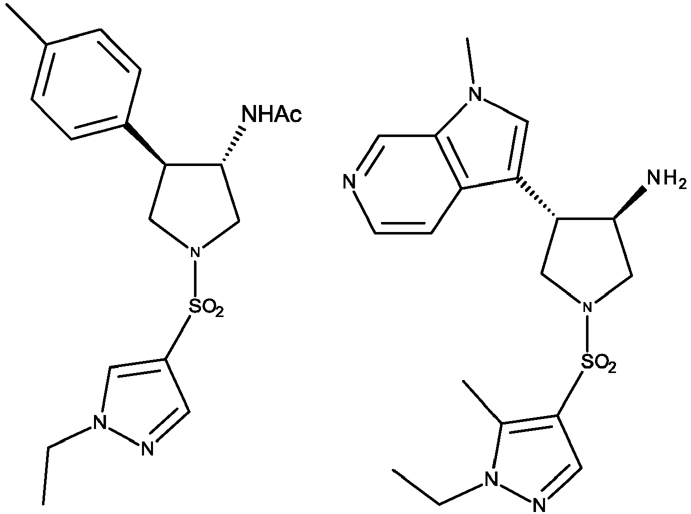

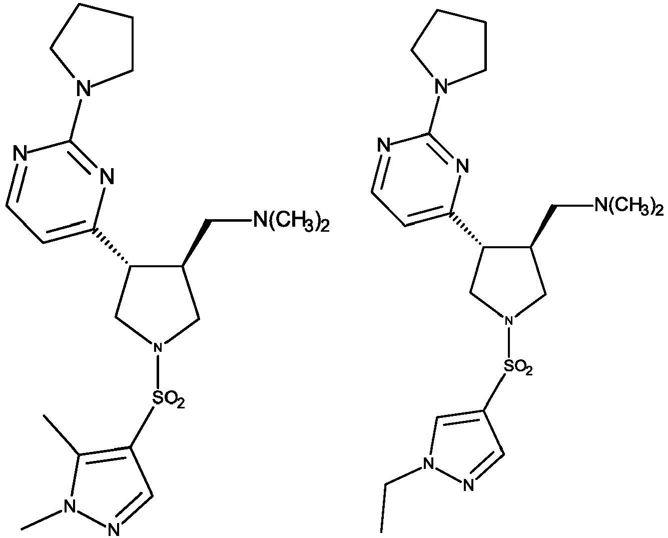

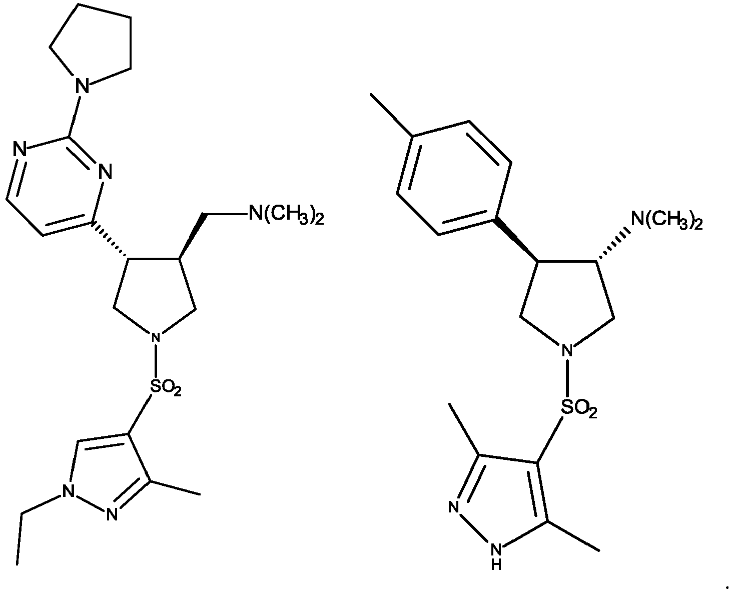

2. Verbindung gemäß Anspruch 1, worin R¹ für gegebenenfalls substituiertes 1,3-Diazolyl oder gegebenenfalls substituiertes 1,2,3-triazolyl.

3. Verbindung gemäß Anspruch 1, worin R¹ für 1,2-Diazol-4-yl, 1-Methyl-1,2-diazol-4-yl, 1-Methyl-1,2-diazol-3-yl, 1-Methyl-1,3-diazol-4-yl, 1-Methyl-1,2-diazol-5-yl, 1,5-Dimethyl-1,2-diazol-4-yl, 1,3-Dimethyl-1,2-diazol-4-yl, 1,5-Dimethyl-1,2-diazol-4-yl, 1,3-Dimethyl-1,2-diazol-5-yl, 1,3-Dimethyl-5-Cl-1,2-diazol-4-yl, 1-Isopropyl-3-methyl-1,2-diazol-4-yl, 1-Me-3-CF₃-1,2-Diazol-4-yl, 1-Cyclopentyl-3-Me-1,2-diazol-4-yl, 1,3,5-Trimethyl-1,2-diazol-4-yl, 1-CHF₂-3,5-Dimethyl-1,2-diazol-4-yl, 1,2-Dimethyl-1,3-diazol-4-yl, 1,2-Dimethyl-1,3-diazol-5-yl, 1-Methyl-5-Cl-1,3-diazol-4-yl, 1-Me-1,2,3-Triazol-4-yl, 1-Ethyl-1,3-diazol-4-yl oder 1-Methyl-1,2,4-triazol-3-yl steht.

4. Verbindung gemäß einem der Ansprüche 1-3, worin R^{2a} für Wasserstoff, Halogen oder C₁-C₃-Alkyl steht und R^{2b} für Wasserstoff steht.

5. Verbindung gemäß einem der Ansprüche 1-4, worin R^{3a} für C₆-C₁₂-Aryl steht, welches gegebenenfalls mit 1, 2 oder 3 Substituenten substituiert ist, die unabhängig unter Halogen, C₁-C₄-Alkyl und C₁-C₄-Alkoxy ausgewählt sind.

6. Verbindung gemäß einem der Ansprüche 1-4, worin R^{3a} für C₃-C₁₂-Heterocyclyl sthet, welches gegebenenfalls mit 1, 2 oder 3 Substituenten substituiert ist, welche unabhängig unter Halogen, C₁-C₄-Alkyl und C₃-C₆-Cycloalkyl ausgewählt sind.

7. Verbindung gemäß einem der Ansprüche 1-6, worin R^{3b} für Wasserstoff steht.

8. Verbindung gemäß einem der Ansprüche 1-7, worin Y¹ für >CR⁶- steht und R⁶ für Wasserstoff, Methyl, Benzyl, Hydroxy-methyl oder Hydroxy steht.

9. Verbindung gemäß einem der Ansprüche 1-8, worin R^{5a} für Wasserstoff, Halogen oder C₁-C₃-Alkyl steht und R^{5b} für Wasserstoff steht.

10. Verbindung gemäß einem der Ansprüche 1-9 der Formel 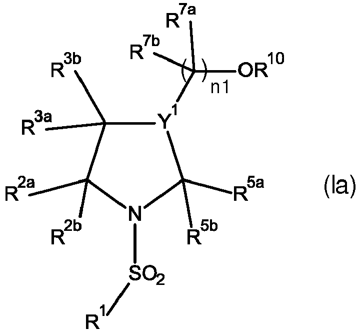
worin R¹, R^{2a}, R^{2b}, R^{3a}, R^{3b}, Y¹, R^{5a} und R^{5b} so sind, wie in einem der Ansprüchel-9 definiert, und
R^{7a}, R^{7b} unabhängig für Wasserstoff oder C₁-C₆-Alkyl stehen;
n1 für 1, 2, 3 oder 4 steht; und
R¹⁰ für Wasserstoff, gegebenenfalls substituiertes C₆-C₁₂-Aryl oder gegebenenfalls substituiertes C₃-C₁₂-Heterocyclyl steht.

11. Verbindung gemäß einem der Ansprüche 1-9 der Formel 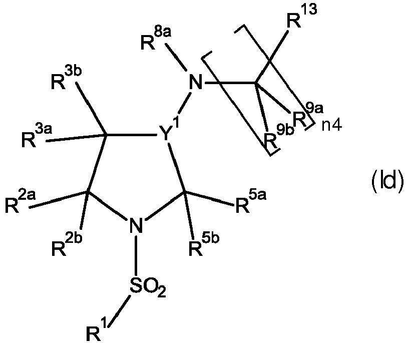
worin R¹, R^{2a}, R^{2b}, R^{3a}, R^{3b}, Y¹, R^{5a} und R^{5b} so sind, wie in einem der Ansprüche1-9 definiert, und
R^{8a} für Wasserstoff, C₁-C₆-Alkyl oder C₁-C₆-Alkylcarbonyl steht, oder
R⁶, R^{8a}
gemeinsam für gegebenenfalls substituiertes C₁-C₅-Alkylen stehen, worin eines oder mehrere - CH₂- von C₁-C₅-Alkylen unabhängig durch ein Sauerstoffatom oder C=O ersetzt sein können;
R^{9a}, R^{9b}
unabhängig für Wasserstoff, Halogen, C₁-C₆-Alkyl, Hydroxy oder C₁-C₆-Alkoxy stehen;
n4 für 0, 1, 2, 3 oder 4 steht;
R¹³ für Wasserstoff, C₁-C₈-Alkyl, halogeniertes C₁-C₆-Alkyl, (gegebenenfalls substituiertes C₃-C₁₂-Cycloalkyl)-C₁-C₄-alkyl, C₁-C₆-Alkoxy-C₁-C₆-alkyl, gegebenenfalls substituiertes C₃-C₁₂-Cycloalkyl, C₂-C₆-Alkenyl, gegebenenfalls substituiertes C₃-C₆-Cycloalkenyl, gegebenenfalls substituiertes C₆-C₁₂-Aryl, Hydroxy, C₁-C₆-Alkoxy, C₁-C₆-Alkoxy-C₁-C₄-alkoxy, gegebenenfalls substituiertes C₆-C₁₂-Aryloxy, gegebenenfalls substituiertes C₃-C₁₂-Heterocyclyloxy, gegebenenfalls substituiertes C₃-C₁₂-Heterocyclyl oder Tri-(C₁-C₄-alkyl)-silyloxy steht.

12. Verbindung gemäß Anspruch 11, worin R¹³ für eine Gruppe der Formel (Id1) steht: 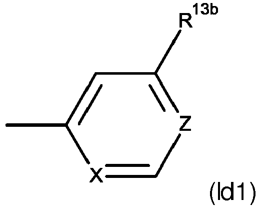 worin
X für >CH- oder >N- steht;
Z für >C-R^{13c} oder >N- steht;
R^{13b} für Halogen, C₁-C₄-Alkyl, halogeniertes C₁-C₄-Alkyl, hydroxy-(halogeniertes C₁-C₄-Alkyl), C₆-C₁₂-Aryl-C₁-C₄-alkyl, C₃-C₆-Cycloalkyl, CN, C₆-C₁₂-Aryl, gegebenenfalls substituiert mit Halogen oder C₁-C₄-Alkyl, C₁-C₄-Alkoxy, halogeniertes C₁-C₄-Alkoxy, C₃-C₆-Cycloalkoxy, C₆-C₁₂-Aryl-C₁-C₄-alkoxy, C₃-C₁₂-Heterocyclyl-C₁-C₄-alkoxy, C₆-C₁₂-Aryloxy, C₁-C₄-Alkyl-sulfonyl, C₁-C₄-Alkyl-carbonylamino oder C₃-C₁₂-Heterocyclyl steht; und
R^{13c} für Wasserstoff oder Halogen steht.

13. Verbindung gemäß einem der Ansprüche 1-9 der Formel 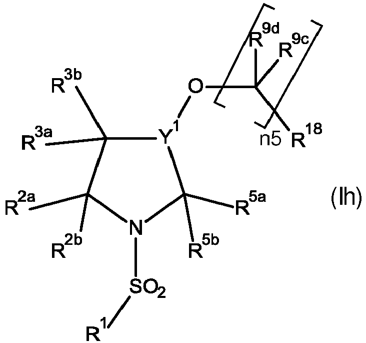
worin R¹, R^{2a}, R^{2b}, R^{3a}, R^{3b}, Y¹, R^{5a} und R^{5b} so sind, wie in einem der Ansprüche 1-9 definiert, und
R^{9c}, R^{9d}
unabhängig für Wasserstoff, Halogen oder C₁-C₆-Alkyl stehen;
n5 für 0, 1, 2, 3 oder 4 steht; und
R¹⁸ für Wasserstoff, gegebenenfalls substituiertes C₁-C₈-Alkyl, gegebenenfalls substituiertes
C₃-C₁₂-Cycloalkyl, C₁-C₆-Alkylcarbonyl, C₁-C₆-Alkoxycarbonyl, halogeniertes C₁-C₆-Alkoxycarbonyl, C₆-C₁₂-Aryloxycarbonyl, C₁-C₆-Alkylaminocarbonyl, (halogeniertes C₁-C₄-Alkyl)aminocarbonyl, C₆-C₁₂-Arylaminocarbonyl, gegebenenfalls substituiertes C₆-C₁₂-Aryl, C₁-C₆-Alkylamin, (C₃-C₁₂-Cycloalkyl-C₁-C₄-alkyl)amino, (halogeniertes C₁-C₆-Alkyl)amino, (C₁-C₆-Alkoxy- C₁-C₆-alkyl)amino, (C₆-C₁₂-Aryl-C₁-C₄-alkyl)amino, C₁-C₆-Dialkylamin, gegebenenfalls substituiertes C₆-C₁₂-Arylamin oder gegebenenfalls substituiertes C₃-C₁₂-Heterocyclyl steht.

14. Verbindung gemäß Anspruch 1, worin
R¹ für einen 5-gliedrigen heterocyclischen Ring steht, ausgewählt unter Pyrazolyl, Imidazolyl und Triazolyl, welcher gegebenenfalls mit 1, 2 oder 3 Substituenten substituiert ist, die unabhängig unter Halogen, C₁-C₆-Alkyl, halogeniertem C₁-C₆-Alkyl, C₃-C₆-Cycloalkyl, C₁-C₄-Alkoxycarbonyl und C₁-C₆-Alkylcarbonylamino ausgewählt sind;
R^{2a}, R^{2b} für Wasserstoff stehen;
R^{3a} für C₃-C₁₂-Cycloalkyl, gegebenenfalls substituiertes C₆-C₁₂-Aryl, Hydroxy, C₁-C₆-Alkoxy, C₃-C₁₂-Cycloalkyl-C₁-C₄-alkoxy, C₂-C₆-Alkenyloxy, C₆-C₁₂-Aryl-C₁-C₄-Alkoxy, gegebenenfalls substituiertes C₆-C₁₂-Aryloxy oder gegebenenfalls substituiertes C₃-C₁₂-Heterocyclyl steht;
R^{3b} für Wasserstoff oder Hydroxy steht;
Y¹ für >CR⁶- oder >N- steht;
R⁶ für Wasserstoff, C₁-C₆-Alkyl, (C₆-C₁₂-Aryl)-C₁-C₄-Alkyl, Hydroxy-C₁-C₆-alkyl oder Hydroxy steht;
R⁴ für -(CR^{7a}R^{7b})ₙ₁OR¹⁰, -(CR^{7c}R^{7d})ₙ₂NR^{11a}R^{11b}, -(CR^{7e}R^{7f})ₙ₃R¹², gegebenenfalls substituiertes C₆-C₁₂-Aryl, -NR^{8a}(CR^{9a}R^{9b})ₙ₄R¹³, -NR^{8b}COR¹⁴, -NR^{8c}COOR¹⁵, -NR^{8d}CONR^{16a}R^{16b},-O(CR^{9c}R^{9d})ₙ₅R¹⁸, -COR¹⁹, -CONR^{20a}R^{20b}, -SO₂R²¹ oder gegebenenfalls substituiertes C₃-C₁₂-Heterocyclyl steht;
R^{7a}, R^{7b} für Wasserstoff stehen, oder
n1 für 1 steht;
R¹⁰ für Wasserstoff, gegebenenfalls substituiertes C₆-C₁₂-Aryl oder gegebenenfalls substituiertes C₃-C₁₂-Heterocyclyl steht;
R^{7c}, R^{7d} für Wasserstoff stehen;
n2 für 1 steht;
R^{11a} für C₁-C₈-Alkyl, C₃-C₁₂-Cycloalkyl-C₁-C₄-alkyl, C₁-C₆-Alkoxy-C₁-C₆-alkyl, C₆-C₁₂-Aryl-C₁-C₄-alkyl, gegebenenfalls substituiertes C₆-C₁₂-Aryl steht;
R^{11b} für Wasserstoff steht;
R^{7e}, R^{7f} für Wasserstoff stehen,
n3 für 1 steht;
R¹² für gegebenenfalls substituiertes C₆-C₁₂-Aryl oder gegebenenfalls substituiertes C₃-C₁₂-Heterocyclyl steht;
R^{8a}, R^{8b}, R^{8c}, R^{8d}, R^{8e} unabhängig für Wasserstoff oder C₁-C₆-Alkyl stehen, oder
R⁶ und eines von R^{8a}, R^{8b}, R^{8c}, R^{8d} oder R^{8e} gemeinsam für gegebenenfalls substituiertes C₁-C₅-Alkylen stehen, worin eines oder mehrere -CH₂- von C₁-C₅-Alkylen unabhängig durch ein Sauerstoffatom oder C=O ersetzt sein können;
R^{9a}, R^{9b} unabhängig für Wasserstoff, Halogen, C₁-C₆-Alkyl oder C₁-C₆-Alkoxy stehen;
n4 für 0, 1, 2, 3 oder 4 steht;
R¹³ für Wasserstoff, C₁-C₈-Alkyl, halogeniertes C₁-C₆-Alkyl, C₁-C₆-Alkoxy-C₁-C₆-alkyl, gegebenenfalls substituiertes C₃-C₁₂-Cycloalkyl, C₂-C₆-Alkenyl, gegebenenfalls substituiertes C₃-C₆-Cycloalkenyl, gegebenenfalls substituiertes C₆-C₁₂-Aryl, C₁-C₆-Alkoxy, C₁-C₆-Alkoxy-C₁-C₄-alkoxy, gegebenenfalls substituiertes C₆-C₁₂-Aryloxy, gegebenenfalls substituiertes C₃-C₁₂-Heterocyclyl oder Tri-(C₁-C₄-alkyl)-silyloxy steht;
R¹⁴ für C₁-C₈-Alkyl, halogeniertes C₁-C₆-Alkyl, C₃-C₁₂-Cycloalkyl-C₁-C₄-alkyl, Hydroxy-C₁-C₆-alkyl, (gegebenenfalls substituiertes C₆-C₁₂-Aryloxy)-C₁-C₄-alkyl, C₁-C₆-Alkylcarbonyl-C₁-C₄-alkyl, C₁-C₆-Alkoxycarbonyl-C₁-C₄-alkyl, gegebenenfalls substituiertes C₃-C₁₂-Cycloalkyl, gegebenenfalls substituiertes C₆-C₁₂-Aryl oder gegebenenfalls substituiertes C₃-C₁₂-Heterocyclyl steht;
R¹⁵ für C₁-C₈-Alkyl, C₆-C₁₂-Aryl oder gegebenenfalls substituiertes C₃-C₁₂-Heterocyclyl steht;
R^{16a} für (gegebenenfalls substituiertes C₆-C₁₂-Aryl)-C₁-C₄-alkyl oder gegebenenfalls substituiertes C₆-C₁₂-Aryl steht;
R^{16b} für Wasserstoff steht;
R^{9c}, R^{9d} für Wasserstoff stehen;
n5 für 0, 1 oder 2 steht;
R¹⁸ für Wasserstoff, C₁-C₈-Alkyl, C₁-C₆-Alkoxycarbonyl, C₁-C₆-Alkylaminocarbonyl, (halogeniertes C₁-C₄-Alkyl)aminocarbonyl, gegebenenfalls substituiertes C₆-C₁₂-Aryl, C₁-C₆-Alkylamin, (halogeniertes C₁-C₆-Alkyl)amino, gegebenenfalls substituiertes C₆-C₁₂-Arylamin oder gegebenenfalls substituiertes C₃-C₁₂-Heterocyclyl steht;
R¹⁹ für gegebenenfalls substituiertes C₆-C₁₂-Aryl steht;
R^{20a} für C₁-C₈-Alkyl, C₃-C₁₂-Cycloalkyl-C₁-C₄-alkyl, C₁-C₆-Alkoxy-C₁-C₆-alkyl, (gegebenenfalls substituiertes C₆-C₁₂-Aryl)-C₁-C₄-alkyl, (gegebenenfalls substituiertes C₃-C₁₂-Heterocyclyl)-C₁-C₄-alkyl, gegebenenfalls substituiertes C₆-C₁₂-Aryl oder gegebenenfalls substituiertes C₃-C₁₂-Heterocyclyl steht;
R^{20b} für Wasserstoff oder C₁-C₈-Alkyl steht;
R²¹ für C₆-C₁₂-Aryl steht; und
R^{5a}, R^{5b} für Wasserstoff stehen, oder
R^{5a}, R^{5b} gemeinsam mit dem Kohlenstoffatom, an das sie gebunden sind, C=O bilden.

15. Verbindung gemäß Anspruch 1, welche:
2-Chlor-N-{trans-1-[(1-methyl-1H-imidazol-4-yl)sulfonyl]-4-phenylpyrrolidin-3-yl}-3-(trifluormethyl)benzamid;
2-Chlor-N-{trans-4-(4-methoxyphenyl)-1-[(1-methyl-1H-imidazol-4-yl)sulfonyl]pyrrolidin-3-yl}-3-(trifluormethyl)benzamid;
2-Chlor-N-{trans-4-(4-fluorphenyl)-1-[(1-methyl-1H-imidazol-4-yl)sulfonyl]pyrrolidin-3-yl}-3-(trifluormethyl)benzamid;
2-Chlor-N-{trans-1-[(1-methyl-1H-imidazol-4-yl)sulfonyl]-4-phenylpyrrolidin-3-yl}-3-(trifluormethyl)benzamid;
2-Chlor-N-{trans-4-(4-chlorphenyl)-1-[(1-methyl-1H-imidazol-4-yl)sulfonyl]pyrrolidin-3-yl}-3-(trifluormethyl)benzamid;
2-Chlor-N-{trans-1-[(1-methyl-1H-imidazol-4-yl)sulfonyl]-4-phenylpyrrolidin-3-yl}benzamid;
3-Chlor-N-{trans-1-[(1-methyl-1H-imidazol-4-yl)sulfonyl]-4-phenylpyrrolidin-3-yl}benzamid;
4-Chlor-N-{trans-1-[(1-methyl-1H-imidazol-4-yl)sulfonyl]-4-phenylpyrrolidin-3-yl}benzamid;
2-Fluor-N-{trans-1-[(1-methyl-1H-imidazol-4-yl)sulfonyl]-4-phenylpyrrolidin-3-yl}benzamid;
3-Fluor-N-{trans-1-[(1-methyl-1H-imidazol-4-yl)sulfonyl]-4-phenylpyrrolidin-3-yl}benzamid;
4-Fluor-N-{trans-1-[(1-methyl-1H-imidazol-4-yl)sulfonyl]-4-phenylpyrrolidin-3-yl}benzamid;
2-Methoxy-N-{trans-1-[(1-methyl-1H-imidazol-4-yl)sulfonyl]-4-phenylpyrrolidin-3-yl}benzamid;
3-Methoxy-N-{trans-1-[(1-methyl-1H-imidazol-4-yl)sulfonyl]-4-phenylpyrrolidin-3-yl}benzamid;
2-Methyl-N-{trans-1-[(1-methyl-1H-imidazol-4-yl)sulfonyl]-4-phenylpyrrolidin-3-yl}benzamid;
3-Methyl-N-{trans-1-[(1-methyl-1H-imidazol-4-yl)sulfonyl]-4-phenylpyrrolidin-3-yl}benzamid;
4-Methyl-N-{trans-1-[(1-methyl-1H-imidazol-4-yl)sulfonyl]-4-phenylpyrrolidin-3-yl}benzamid;
N-{trans-1-[(1-Methyl-1H-imidazol-4-yl)sulfonyl]-4-phenylpyrrolidin-3-yl}benzamid;
2,4-Dichlor-N-{trans-1-[(1-methyl-1H-imidazol-4-yl)sulfonyl]-4-phenylpyrrolidin-3-yl}benzamid;
2-Chlor-4-fluor-N-{trans-1-[(1-methyl-1H-imidazol-4-yl)sulfonyl]-4-phenylpyrrolidin-3-yl}benzamid;
trans-1-[(1-Methyl-1H-imidazol-4-yl)sulfonyl]-N,4-diphenylpyrrolidin-3-amin;
3,5-Dichlor-N-{trans-1-[(1-methyl-1H-imidazol-4-yl)sulfonyl]-4-phenylpyrrolidin-3-yl}benzamid;
2,3-Dichlor-N-{trans-1-[(1-methyl-1H-imidazol-4-yl)sulfonyl]-4-phenylpyrrolidin-3-yl}benzamid;
2-Cyano-N-{trans-1-[(1-methyl-1H-imidazol-4-yl)sulfonyl]-4-phenylpyrrolidin-3-yl}benzamid;
3-Cyano-N-{trans-1-[(1-methyl-1H-imidazol-4-yl)sulfonyl]-4-phenylpyrrolidin-3-yl}benzamid;
4-Cyano-N-{trans-1-[(1-methyl-1H-imidazol-4-yl)sulfonyl]-4-phenylpyrrolidin-3-yl}benzamid;
N-{trans-1-[(1-Methyl-1H-imidazol-4-yl)sulfonyl]-4-phenylpyrrolidin-3-yl}-2-(trifluormethyl)benzamid;
N-{trans-1-[(1-Methyl-1H-imidazol-4-yl)sulfonyl]-4-phenylpyrrolidin-3-yl}-3-(trifluormethyl)benzamid;
N-{trans-1-[(1-Methyl-1H-imidazol-4-yl)sulfonyl]-4-phenylpyrrolidin-3-yl}-4-(trifluormethyl)benzamid;
2-Chlor-N-[trans-1-{[1-methyl-3-(trifluormethyl)-1H-pyrazol-4-yl]sulfonyl}-4-phenylpyrrolidin-3-yl]-3-(trifluormethyl)benzamid;
2-Chlor-N-[trans-1-{[1-(difluormethyl)-3,5-dimethyl-1H-pyrazol-4-yl]sulfonyl}-4-phenylpyrrolidin-3-yl]-3-(trifluormethyl)benzamid;
2-Chlor-N-{trans-1-[(5-chlor-1,3-dimethyl-1H-pyrazol-4-yl)sulfonyl]-4-phenylpyrrolidin-3-yl}-3-(trifluormethyl)benzamid;
2-Chlor-N-{trans-1-[(1,5-dimethyl-1H-pyrazol-4-yl)sulfonyl]-4-phenylpyrrolidin-3-yl}-3-(trifluormethyl)benzamid;
2-Chlor-N-{trans-1-[(1,3-dimethyl-1H-pyrazol-4-yl)sulfonyl]-4-phenylpyrrolidin-3-yl}-3-(trifluormethyl)benzamid;
2-Chlor-N-{trans-1-[(1-methyl-1H-pyrazol-4-yl)sulfonyl]-4-phenylpyrrolidin-3-yl}-3-(trifluormethyl)benzamid;
2-Chlor-N-[trans-4-phenyl-1-(1H-pyrazol-4-ylsulfonyl)pyrrolidin-3-yl]-3-(trifluormethyl)benzamid;
2-Chlor-N-{trans-1-[(1-methyl-1H-pyrazol-5-yl)sulfonyl]-4-phenylpyrrolidin-3-yl}-3-(trifluormethyl)benzamid;
2-Chlor-N-{trans-1-[(5-chlor-1-methyl-1H-imidazol-4-yl)sulfonyl]-4-phenylpyrrolidin-3-yl}-3-(trifluormethyl)benzamid;
2-Chlor-N-{trans-1-[(1-methyl-1H-pyrazol-3-yl)sulfonyl]-4-phenylpyrrolidin-3-yl}-3-(trifluormethyl)benzamid;
trans-N-(4-Methoxyphenyl)-1-[(1-methyl-1H-imidazol-4-yl)sulfonyl]-4-phenylpyrrolidin-3-amin;
3-({trans-1-[(1-Methyl-1H-imidazol-4-yl)sulfonyl]-4-phenylpyrrolidin-3-yl}amino)benzonitril;
4-({trans-1-[(1-Methyl-1H-imidazol-4-yl)sulfonyl]-4-phenylpyrrolidin-3-yl}amino)benzonitril;
3-Fluor-4-({trans-1-[(1-methyl-1H-imidazol-4-yl)sulfonyl]-4-phenylpyrrolidin-3-yl}amino)benzonitril;
2-Chlor-N-{trans-4-(2-fluorphenyl)-1-[(1-methyl-1H-imidazol-4-yl)sulfonyl]pyrrolidin-3-yl}-3-(trifluormethyl)benzamid;
2-Chlor-N-{trans-4-(3-fluorphenyl)-1-[(1-methyl-1H-imidazol-4-yl)sulfonyl]pyrrolidin-3-yl}-3-(trifluormethyl)benzamid;
2-Chlor-N-{trans-4-(3-chlorphenyl)-1-[(1-methyl-1H-imidazol-4-yl)sulfonyl]pyrrolidin-3-yl}-3-(trifluormethyl)benzamid;
trans-1-[(1-Methyl-1H-imidazol-4-yl)sulfonyl]-N-(2-methylphenyl)-4-phenylpyrrolidin-3-amin;
trans-1-[(1-Methyl-1H-imidazol-4-yl)sulfonyl]-N-(3-methylphenyl)-4-phenylpyrrolidin-3-amin;
trans-1-[(1-Methyl-1H-imidazol-4-yl)sulfonyl]-N-(4-methylphenyl)-4-phenylpyrrolidin-3-amin;
2-Chlor-N-{trans-1-[(1-methyl-1H-imidazol-4-yl)sulfonyl]-4-phenylpyrrolidin-3-yl}-4-(trifluormethyl)benzamid;
3-Chlor-N-{trans-1-[(1-methyl-1H-imidazol-4-yl)sulfonyl]-4-phenylpyrrolidin-3-yl}-4-(trifluormethyl)benzamid;
2-Chlor-N-{trans-4-(4-fluorphenyl)-1-[(1-methyl-1H-imidazol-4-yl)sulfonyl]pyrrolidin-3-yl}-4-(trifluormethyl)benzamid;
3-Chlor-N-{trans-4-(4-fluorphenyl)-1-[(1-methyl-1H-imidazol-4-yl)sulfonyl]pyrrolidin-3-yl}-4-(trifluormethyl)benzamid;
N-{trans-4-(4-Fluorphenyl)-1-[(1-methyl-1H-imidazol-4-yl)sulfonyl]pyrrolidin-3-yl}-4-(trifluormethyl)benzamid;
3,5-Dichlor-N-{trans-4-(4-fluorphenyl)-1-[(1-methyl-1H-imidazol-4-yl)sulfonyl]pyrrolidin-3-yl}benzamid;
trans-1-[(1-Methyl-1H-imidazol-4-yl)sulfonyl]-4-phenyl-N-[2-(trifluormethyl)phenyl]pyrrolidin-3-amin;
trans-1-[(1-Methyl-1H-imidazol-4-yl)sulfonyl]-4-phenyl-N-[3-(trifluormethyl)phenyl]pyrrolidin-3-amin;
trans-1-[(1-Methyl-1H-imidazol-4-yl)sulfonyl]-4-phenyl-N-[4-(trifluormethyl)phenyl]pyrrolidin-3-amin;
trans-N-(2-Fluorphenyl)-1-[(1-methyl-1H-imidazol-4-yl)sulfonyl]-4-phenylpyrrolidin-3-amin;
trans-N-(3-Fluorphenyl)-1-[(1-methyl-1H-imidazol-4-yl)sulfonyl]-4-phenylpyrrolidin-3-amin;
trans-N-(4-Fluorphenyl)-1-[(1-methyl-1H-imidazol-4-yl)sulfonyl]-4-phenylpyrrolidin-3-amin;
3-({trans-4-(4-Fluorphenyl)-1-[(1-methyl-1H-imidazol-4-yl)sulfonyl]pyrrolidin-3-yl}amino)benzonitril;
trans-N-(2-Chlorphenyl)-1-[(1-methyl-1H-imidazol-4-yl)sulfonyl]-4-phenylpyrrolidin-3-amin;
trans-N-(3-Chlorphenyl)-1-[(1-methyl-1H-imidazol-4-yl)sulfonyl]-4-phenylpyrrolidin-3-amin;
trans-N-(4-Chlorphenyl)-1-[(1-methyl-1H-imidazol-4-yl)sulfonyl]-4-phenylpyrrolidin-3-amin;
4-Fluor-3-({trans-1-[(1-methyl-1H-imidazol-4-yl)sulfonyl]-4-phenylpyrrolidin-3-yl}amino)benzonitril;
2-Fluor-3-({trans-1-[(1-methyl-1H-imidazol-4-yl)sulfonyl]-4-phenylpyrrolidin-3-yl}amino)benzonitril;
3-Fluor-5-({trans-1-[(1-methyl-1H-imidazol-4-yl)sulfonyl]-4-phenylpyrrolidin-3-yl}amino)benzonitril;
2-Chlor-5-({trans-1-[(1-methyl-1H-imidazol-4-yl)sulfonyl]-4-phenylpyrrolidin-3-yl}amino)benzonitril;
3-Chlor-5-({trans-1-[(1-methyl-1H-imidazol-4-yl)sulfonyl]-4-phenylpyrrolidin-3-yl}amino)benzonitril;
4-Chlor-3-({trans-1-[(1-methyl-1H-imidazol-4-yl)sulfonyl]-4-phenylpyrrolidin-3-yl}amino)benzonitril;
4-Methyl-3-({trans-1-[(1-methyl-1H-imidazol-4-yl)sulfonyl]-4-phenylpyrrolidin-3-yl}amino)benzonitril;
2-Methyl-3-({trans-1-[(1-methyl-1H-imidazol-4-yl)sulfonyl]-4-phenylpyrrolidin-3-yl}amino)benzonitril;
trans-4-(4-Fluorphenyl)-1-[(1-methyl-1H-imidazol-4-yl)sulfonyl]-N-(2-methylphenyl)pyrrolidin-3-amin;
trans-4-(4-Fluorphenyl)-1-[(1-methyl-1H-imidazol-4-yl)sulfonyl]-N-(3-methylphenyl)pyrrolidin-3-amin;
trans-4-(4-Fluorphenyl)-1-[(1-methyl-1H-imidazol-4-yl)sulfonyl]-N-(4-methylphenyl)pyrrolidin-3-amin;
trans-4-(4-Fluorphenyl)-1-[(1-methyl-1H-imidazol-4-yl)sulfonyl]-N-[2-(trifluormethyl)phenyl]pyrrolidin-3-amin;
trans-4-(4-Fluorphenyl)-1-[(1-methyl-1H-imidazol-4-yl)sulfonyl]-N-[3-(trifluormethyl)phenyl]pyrrolidin-3-amin;
trans-4-(4-Fluorphenyl)-1-[(1-methyl-1H-imidazol-4-yl)sulfonyl]-N-[4-(trifluormethyl)phenyl]pyrrolidin-3-amin;
trans-N-(2-Fluorphenyl)-4-(4-fluorphenyl)-1-[(1-methyl-1H-imidazol-4-yl)sulfonyl]pyrrolidin-3-amin;
trans-N-(3-Fluorphenyl)-4-(4-fluorphenyl)-1-[(1-methyl-1H-imidazol-4-yl)sulfonyl]pyrrolidin-3-amin;
trans-N,4-bis(4-Fluorphenyl)-1-[(1-methyl-1H-imidazol-4-yl)sulfonyl]pyrrolidin-3-amin;
trans-N-(2-Chlorphenyl)-4-(4-fluorphenyl)-1-[(1-methyl-1H-imidazol-4-yl)sulfonyl]pyrrolidin-3-amin;
trans-N-(3-Chlorphenyl)-4-(4-fluorphenyl)-1-[(1-methyl-1H-imidazol-4-yl)sulfonyl]pyrrolidin-3-amin;
trans-N-(4-Chlorphenyl)-4-(4-fluorphenyl)-1-[(1-methyl-1H-imidazol-4-yl)sulfonyl]pyrrolidin-3-amin;
2-Methoxy-5-({trans-1-[(1-methyl-1H-imidazol-4-yl)sulfonyl]-4-phenylpyrrolidin-3-yl}amino)benzonitril;
3-Methyl-5-({trans-1-[(1-methyl-1H-imidazol-4-yl)sulfonyl]-4-phenylpyrrolidin-3-yl}amino)benzonitril;
5-({trans-1-[(1-Methyl-1H-imidazol-4-yl)sulfonyl]-4-phenylpyrrolidin-3-yl}amino)-2-(trifluormethoxy)benzonitril;
2-Chlor-N-{trans-4-(2-chlorphenyl)-1-[(1-methyl-1H-imidazol-4-yl)sulfonyl]pyrrolidin-3-yl}-3-(trifluormethyl)benzamid;
4-{[trans-3-Benzyl-4-phenylpyrrolidin-1-yl]sulfonyl}-1-methyl-1H-imidazol;
trans-N-Benzyl-4-(4-fluorphenyl)-1-[(1-methyl-1H-imidazol-4-yl)sulfonyl]pyrrolidin-3-amin;
trans-N-[2-Chlor-3-(trifluormethyl)benzyl]-4-(4-fluorphenyl)-1-[(1-methyl-1H-imidazol-4-yl)sulfonyl]pyrrolidin-3-amin;
trans-N-[2-Chlor-4-(trifluormethyl)benzyl]-4-(4-fluorphenyl)-1-[(1-methyl-1H-imidazol-4-yl)sulfonyl]pyrrolidin-3-amin;
trans-N-[3-Chlor-4-(trifluormethyl)benzyl]-4-(4-fluorphenyl)-1-[(1-methyl-1H-imidazol-4-yl)sulfonyl]pyrrolidin-3-amin;
3-[({trans-4-(4-Fluorphenyl)-1-[(1-methyl-1H-imidazol-4-yl)sulfonyl]pyrrolidin-3-yl}amino)methyl]benzonitril;
trans-4-(4-Fluorphenyl)-N-(2-methylbenzyl)-1-[(1-methyl-1H-imidazol-4-yl)sulfonyl]pyrrolidin-3-amin;
trans-4-(4-Fluorphenyl)-N-(3-methylbenzyl)-1-[(1-methyl-1H-imidazol-4-yl)sulfonyl]pyrrolidin-3-amin;
trans-4-(4-Fluorphenyl)-N-(4-methylbenzyl)-1-[(1-methyl-1H-imidazol-4-yl)sulfonyl]pyrrolidin-3-amin;
trans-N-(4-Fluorbenzyl)-4-(4-fluorphenyl)-1-[(1-methyl-1H-imidazol-4-yl)sulfonyl]pyrrolidin-3-amin;
(cis)-N-Benzyl-1-[(1-methyl-1H-imidazol-4-yl)sulfonyl]-4-phenylpyrrolidin-3-amin;
trans-N-(3,4-Difluorphenyl)-4-(4-fluorphenyl)-1-[(1-methyl-1H-imidazol-4-yl)sulfonyl]pyrrolidin-3-amin;
trans-4-(4-Fluorphenyl)-N-[4-fluor-3-(trifluormethyl)phenyl]-1-[(1-methyl-1H-imidazol-4-yl)sulfonyl]pyrrolidin-3-amin;
trans-N-(3-Chlor-4-fluorphenyl)-4-(4-fluorphenyl)-1-[(1-methyl-1H-imidazol-4-yl)sulfonyl]pyrrolidin-3-amin;
trans-N-(3,4-Dichlorphenyl)-4-(4-fluorphenyl)-1-[(1-methyl-1H-imidazol-4-yl)sulfonyl]pyrrolidin-3-amin;
trans-N-(4-Chlor-3-fluorphenyl)-4-(4-fluorphenyl)-1-[(1-methyl-1H-imidazol-4-yl)sulfonyl]pyrrolidin-3-amin;
trans-N-(2,3-Difluorphenyl)-4-(4-fluorphenyl)-1-[(1-methyl-1H-imidazol-4-yl)sulfonyl]pyrrolidin-3-amin;
trans-N-(2,5-Difluorphenyl)-4-(4-fluorphenyl)-1-[(1-methyl-1H-imidazol-4-yl)sulfonyl]pyrrolidin-3-amin;
trans-N-(2,4-Difluorphenyl)-4-(4-fluorphenyl)-1-[(1-methyl-1H-imidazol-4-yl)sulfonyl]pyrrolidin-3-amin;
trans-4-(4-Fluorphenyl)-1-[(1-methyl-1H-imidazol-4-yl)sulfonyl]-N-(2,3,4-trifluorphenyl)pyrrolidin-3-amin;
trans-4-(4-Fluorphenyl)-N-[2-fluor-3-(trifluormethyl)phenyl]-1-[(1-methyl-1H-imidazol-4-yl)sulfonyl]pyrrolidin-3-amin;
trans-4-(4-Fluorphenyl)-N-[3-fluor-5-(trifluormethyl)phenyl]-1-[(1-methyl-1H-imidazol-4-yl)sulfonyl]pyrrolidin-3-amin;
trans-4-(4-Fluorphenyl)-N-[2-fluor-5-(trifluormethyl)phenyl]-1-[(1-methyl-1H-imidazol-4-yl)sulfonyl]pyrrolidin-3-amin;
trans-4-(4-Fluorphenyl)-1-[(1-methyl-1H-imidazol-4-yl)sulfonyl]-N-[3-(propan-2-yl)phenyl]pyrrolidin-3-amin;
trans-N-(3-tert-Butylphenyl)-4-(4-fluorphenyl)-1-[(1-methyl-1H-imidazol-4-yl)sulfonyl]pyrrolidin-3-amin;
trans-N-[4-Chlor-3-(trifluormethyl)phenyl]-4-(4-fluorphenyl)-1-[(1-methyl-1H-imidazol-4-yl)sulfonyl]pyrrolidin-3-amin;
trans-N-[3-Chlor-4-(trifluormethyl)phenyl]-4-(4-fluorphenyl)-1-[(1-methyl-1H-imidazol-4-yl)sulfonyl]pyrrolidin-3-amin;
trans-N-(3-Chlor-5-fluorphenyl)-4-(4-fluorphenyl)-1-[(1-methyl-1H-imidazol-4-yl)sulfonyl]pyrrolidin-3-amin;
trans-N-(3-Chlor-4,5-difluorphenyl)-4-(4-fluorphenyl)-1-[(1-methyl-1H-imidazol-4-yl)sulfonyl]pyrrolidin-3-amin;
trans-4-(4-Fluorphenyl)-1-[(1-methyl-1H-imidazol-4-yl)sulfonyl]-N-[3-(trifluormethoxy)phenyl]pyrrolidin-3-amin;
N-{trans-4-(4-Fluorphenyl)-1-[(1-methyl-1H-imidazol-4-yl)sulfonyl]pyrrolidin-3-yl}pyridin-4-amin;
N-{trans-4-(4-Fluorphenyl)-1-[(1-methyl-1H-imidazol-4-yl)sulfonyl]pyrrolidin-3-yl}pyridin-2-amin;
N-{trans-4-(4-Fluorphenyl)-1-[(1-methyl-1H-imidazol-4-yl)sulfonyl]pyrrolidin-3-yl}pyridin-3-amin;
N-{trans-4-(4-Fluorphenyl)-1-[(1-methyl-1H-imidazol-4-yl)sulfonyl]pyrrolidin-3-yl}-6-(trifluormethyl)pyridin-2-amin;
N-{trans-4-(4-Fluorphenyl)-1-[(1-methyl-1H-imidazol-4-yl)sulfonyl]pyrrolidin-3-yl}-5-(trifluormethyl)pyridin-3-amin;
4-{[3-(3-Chlorbenzyl)-4-(4-fluorphenyl)pyrrolidin-1-yl]sulfonyl}-1-methyl-1H-imidazol;
2-Chlor-N-{(cis)-1-[(1-methyl-1H-imidazol-4-yl)sulfonyl]-4-phenylpyrrolidin-3-yl}-3-(trifluormethyl)benzamid;
2-Chlor-N-{(cis)-1-[(1-methyl-1H-imidazol-4-yl)sulfonyl]-4-phenylpyrrolidin-3-yl}-3-(trifluormethyl)benzamid;
trans-4-(3-Fluorphenyl)-1-[(1-methyl-1H-imidazol-4-yl)sulfonyl]-N-phenylpyrrolidin-3-amin;
trans-N,4-bis(3-Fluorphenyl)-1-[(1-methyl-1H-imidazol-4-yl)sulfonyl]pyrrolidin-3-amin;
trans-4-(3-Fluorphenyl)-N-(4-fluorphenyl)-1-[(1-methyl-1H-imidazol-4-yl)sulfonyl]pyrrolidin-3-amin;
trans-N-(3-Chlorphenyl)-4-(3-fluorphenyl)-1-[(1-methyl-1H-imidazol-4-yl)sulfonyl]pyrrolidin-3-amin;
trans-4-(3-Fluorphenyl)-1-[(1-methyl-1H-imidazol-4-yl)sulfonyl]-N-[3-(trifluormethyl)phenyl]pyrrolidin-3-amin;
trans-4-(3-Fluorphenyl)-1-[(1-methyl-1H-imidazol-4-yl)sulfonyl]-N-[4-(trifluormethyl)phenyl]pyrrolidin-3-amin;
N-{trans-4-(3-Fluorphenyl)-1-[(1-methyl-1H-imidazol-4-yl)sulfonyl]pyrrolidin-3-yl}pyridin-4-amin;
trans-N-(3,4-Dichlorphenyl)-4-(3-fluorphenyl)-1-[(1-methyl-1H-imidazol-4-yl)sulfonyl]pyrrolidin-3-amin;
trans-N-(4-Chlor-3-fluorphenyl)-4-(3-fluorphenyl)-1-[(1-methyl-1H-imidazol-4-yl)sulfonyl]pyrrolidin-3-amin;
trans-N-(3-Chlor-4-fluorphenyl)-4-(3-fluorphenyl)-1-[(1-methyl-1H-imidazol-4-yl)sulfonyl]pyrrolidin-3-amin;
N-{trans-4-(3-Fluorphenyl)-1-[(1-methyl-1H-imidazol-4-yl)sulfonyl]pyrrolidin-3-yl}-6-(trifluormethyl)pyridin-2-amin;
N-{trans-4-(3-Fluorphenyl)-1-[(1-methyl-1H-imidazol-4-yl)sulfonyl]pyrrolidin-3-yl}-5-(trifluormethyl)pyridin-3-amin;
trans-N-(3,4-Difluorphenyl)-4-(3-fluorphenyl)-1-[(1-methyl-1H-imidazol-4-yl)sulfonyl]pyrrolidin-3-amin;
trans-4-(2-Fluorphenyl)-1-[(1-methyl-1H-imidazol-4-yl)sulfonyl]-N-phenylpyrrolidin-3-amin;
trans-4-(2-Fluorphenyl)-N-(3-fluorphenyl)-1-[(1-methyl-1H-imidazol-4-yl)sulfonyl]pyrrolidin-3-amin;
trans-4-(2-Fluorphenyl)-N-(4-fluorphenyl)-1-[(1-methyl-1H-imidazol-4-yl)sulfonyl]pyrrolidin-3-amin;
trans-N-(3-Chlorphenyl)-4-(2-fluorphenyl)-1-[(1-methyl-1H-imidazol-4-yl)sulfonyl]pyrrolidin-3-amin;
trans-N-(4-Chlorphenyl)-4-(2-fluorphenyl)-1-[(1-methyl-1H-imidazol-4-yl)sulfonyl]pyrrolidin-3-amin;
N-{trans-4-(2-Fluorphenyl)-1-[(1-methyl-1H-imidazol-4-yl)sulfonyl]pyrrolidin-3-yl}-6-(trifluormethyl)pyridin-2-amin;
N-{trans-4-(2-Fluorphenyl)-1-[(1-methyl-1H-imidazol-4-yl)sulfonyl]pyrrolidin-3-yl}-2-(trifluormethyl)pyridin-4-amin;
4-{[3-(2-Brombenzyl)-4-(2-bromphenyl)pyrrolidin-1-yl]sulfonyl}-1-methyl-1H-imidazol;
{trans-4-(4-Fluorphenyl)-1-[(1-methyl-1H-imidazol-4-yl)sulfonyl]pyrrolidin-3-yl}methanol;
N-{trans-1-[(1-Methyl-1H-imidazol-4-yl)sulfonyl]-4-phenylpyrrolidin-3-yl}pyridin-2-amin;
N-{trans-1-[(1-Methyl-1H-imidazol-4-yl)sulfonyl]-4-phenylpyrrolidin-3-yl}pyridin-3-amin;
N-{trans-1-[(1-Methyl-1H-imidazol-4-yl)sulfonyl]-4-phenylpyrrolidin-3-yl}pyridin-4-amin;
N-{trans-1-[(1-Methyl-1H-imidazol-4-yl)sulfonyl]-4-phenylpyrrolidin-3-yl}-6-(trifluormethyl)pyridin-2-amin;
N-{trans-1-[(1-Methyl-1H-imidazol-4-yl)sulfonyl]-4-phenylpyrrolidin-3-yl}-2-(trifluormethyl)pyridin-4-amin;
N-{trans-1-[(1-Methyl-1H-imidazol-4-yl)sulfonyl]-4-phenylpyrrolidin-3-yl}-4-(trifluormethyl)pyridin-2-amin;
N-{trans-1-[(1-Methyl-1H-imidazol-4-yl)sulfonyl]-4-phenylpyrrolidin-3-yl}-5-(trifluormethyl)pyridin-3-amin;
trans-N-[4-Fluor-3-(trifluormethyl)phenyl]-1-[(1-methyl-1H-imidazol-4-yl)sulfonyl]-4-phenylpyrrolidin-3-amin;
trans-N-(3-Chlor-4-fluorphenyl)-1-[(1-methyl-1H-imidazol-4-yl)sulfonyl]-4-phenylpyrrolidin-3-amin;
trans-N-(3,4-Difluorphenyl)-1-[(1-methyl-1H-imidazol-4-yl)sulfonyl]-4-phenylpyrrolidin-3-amin;
trans-N-[4-Chlor-3-(trifluormethyl)phenyl]-1-[(1-methyl-1H-imidazol-4-yl)sulfonyl]-4-phenylpyrrolidin-3-amin;
trans-N-(3,4-Dichlorphenyl)-1-[(1-methyl-1H-imidazol-4-yl)sulfonyl]-4-phenylpyrrolidin-3-amin;
trans-N-(3-Chlor-4,5-difluorphenyl)-1-[(1-methyl-1H-imidazol-4-yl)sulfonyl]-4-phenylpyrrolidin-3-amin;
trans-1-[(1-Methyl-1H-imidazol-4-yl)sulfonyl]-4-phenyl-N-[3-(propan-2-yl)phenyl]pyrrolidin-3-amin;
trans-N-(3-tert-Butylphenyl)-1-[(1-methyl-1H-imidazol-4-yl)sulfonyl]-4-phenylpyrrolidin-3-amin;
trans-N-[2-Fluor-3-(trifluormethyl)phenyl]-1-[(1-methyl-1H-imidazol-4-yl)sulfonyl]-4-phenylpyrrolidin-3-amin;
trans-N-[3-Fluor-5-(trifluormethyl)phenyl]-1-[(1-methyl-1H-imidazol-4-yl)sulfonyl]-4-phenylpyrrolidin-3-amin;
trans-N-[2-Fluor-5-(trifluormethyl)phenyl]-1-[(1-methyl-1H-imidazol-4-yl)sulfonyl]-4-phenylpyrrolidin-3-amin;
trans-N-(3-Chlor-5-fluorphenyl)-1-[(1-methyl-1H-imidazol-4-yl)sulfonyl]-4-phenylpyrrolidin-3-amin;
trans-1-[(1-Methyl-1H-imidazol-4-yl)sulfonyl]-4-phenyl-N-[3-(trifluormethoxy)phenyl]pyrrolidin-3-amin;
N-{trans-1-[(1-Methyl-1H-imidazol-4-yl)sulfonyl]-4-phenylpyrrolidin-3-yl}-4-(trifluormethyl)pyrimidin-2-amin;
N-{trans-1-[(1-Methyl-1H-imidazol-4-yl)sulfonyl]-4-phenylpyrrolidin-3-yl}chinolin-7-amin;
N-{trans-1-[(1-Methyl-1H-imidazol-4-yl)sulfonyl]-4-phenylpyrrolidin-3-yl}chinolin-6-amin;
N-{trans-1-[(1-Methyl-1H-imidazol-4-yl)sulfonyl]-4-phenylpyrrolidin-3-yl}isochinolin-6-amin;
trans-N-(2,3-Difluorphenyl)-1-[(1-methyl-1H-imidazol-4-yl)sulfonyl]-4-phenylpyrrolidin-3-amin;
trans-N-(2,5-Difluorphenyl)-1-[(1-methyl-1H-imidazol-4-yl)sulfonyl]-4-phenylpyrrolidin-3-amin;
trans-N-(2,4-Difluorphenyl)-1-[(1-methyl-1H-imidazol-4-yl)sulfonyl]-4-phenylpyrrolidin-3-amin;
trans-1-[(1-Methyl-1H-imidazol-4-yl)sulfonyl]-4-phenyl-N-(2,3,4-trifluorphenyl)pyrrolidin-3-amin;
6-Fluor-N-{trans-1-[(1-methyl-1H-imidazol-4-yl)sulfonyl]-4-phenylpyrrolidin-3-yl}pyridin-2-amin;
2-Fluor-N-{trans-1-[(1-methyl-1H-imidazol-4-yl)sulfonyl]-4-phenylpyrrolidin-3-yl}pyridin-4-amin;
5-Fluor-N-{trans-1-[(1-methyl-1H-imidazol-4-yl)sulfonyl]-4-phenylpyrrolidin-3-yl}pyridin-3-amin;
6-Fluor-N-{trans-4-(4-fluorphenyl)-1-[(1-methyl-1H-imidazol-4-yl)sulfonyl]pyrrolidin-3-yl}pyridin-2-amin;
2-Fluor-N-{trans-4-(4-fluorphenyl)-1-[(1-methyl-1H-imidazol-4-yl)sulfonyl]pyrrolidin-3-yl}pyridin-4-amin;
N-{trans-1-[(1-Methyl-1H-imidazol-4-yl)sulfonyl]-4-phenylpyrrolidin-3-yl}-6-(trifluormethyl)pyrimidin-4-amin;
N-{trans-1-[(1-Methyl-1H-imidazol-4-yl)sulfonyl]-4-phenylpyrrolidin-3-yl}isochinolin-7-amin;
trans-4-(4-Fluorphenyl)-1-[(1-methyl-1H-imidazol-4-yl)sulfonyl]-N-phenylpyrrolidin-3-carboxamid;
trans-4-(4-Fluorphenyl)-1-[(1-methyl-1H-imidazol-4-yl)sulfonyl]-N-[4-(trifluormethyl)phenyl]pyrrolidin-3-carboxamid;
trans-N-(3,5-Dichlorphenyl)-4-(4-fluorphenyl)-1-[(1-methyl-1H-imidazol-4-yl)sulfonyl]pyrrolidin-3-carboxamid;
4-{[trans-3-(4-Fluorphenyl)-4-(phenoxymethyl)pyrrolidin-1-yl]sulfonyl}-1-methyl-1H-imidazol;
4-{[trans-3-[(2,4-Dichlorphenoxy)methyl]-4-(4-fluorphenyl)pyrrolidin-1-yl]sulfonyl}-1-methyl-1H-imidazol;
4-{[trans-3-(4-Fluorphenyl)-4-{[3-(trifluormethoxy)phenoxy]methyl}pyrrolidin-1-yl]sulfonyl}-1-methyl-1H-imidazol;
4-{[trans-3-[(3-Chlorphenoxy)methyl]-4-(4-fluorphenyl)pyrrolidin-1-yl]sulfonyl}-1-methyl-1H-imidazol;
4-{[trans-3-{[4-Chlor-3-(trifluormethyl)phenoxy]methyl}-4-(4-fluorphenyl)pyrrolidin-1-yl]sulfonyl}-1-methyl-1H-imidazol;
4-{[trans-3-{[2-Chlor-3-(trifluormethyl)phenoxy]methyl}-4-(4-fluorphenyl)pyrrolidin-1-yl]sulfonyl}-1-methyl-1H-imidazol;
4-{[trans-3-[(3-Fluorphenoxy)methyl]-4-(4-fluorphenyl)pyrrolidin-1-yl]sulfonyl}-1-methyl-1H-imidazol;
trans-4-(2-Chlorphenyl)-N-(3-fluorphenyl)-1-[(1-methyl-1H-imidazol-4-yl)sulfonyl]pyrrolidin-3-amin;
trans-4-(2-Chlorphenyl)-N-(4-fluorphenyl)-1-[(1-methyl-1H-imidazol-4-yl)sulfonyl]pyrrolidin-3-amin;
trans-4-(2-Chlorphenyl)-N-(4-chlorphenyl)-1-[(1-methyl-1H-imidazol-4-yl)sulfonyl]pyrrolidin-3-amin;
trans-4-(2-Chlorphenyl)-1-[(1-methyl-1H-imidazol-4-yl)sulfonyl]-N-[3-(trifluormethyl)phenyl]pyrrolidin-3-amin;
trans-4-(2-Chlorphenyl)-1-[(1-methyl-1H-imidazol-4-yl)sulfonyl]-N-[4-(trifluormethyl)phenyl]pyrrolidin-3-amin;
trans-N-(3-Chlor-4-fluorphenyl)-4-(2-chlorphenyl)-1-[(1-methyl-1H-imidazol-4-yl)sulfonyl]pyrrolidin-3-amin;
trans-4-(2-Chlorphenyl)-N-[4-fluor-3-(trifluormethyl)phenyl]-1-[(1-methyl-1H-imidazol-4-yl)sulfonyl]pyrrolidin-3-amin;
N-{trans-4-(2-Chlorphenyl)-1-[(1-methyl-1H-imidazol-4-yl)sulfonyl]pyrrolidin-3-yl}-2-(trifluormethyl)pyridin-4-amin;
trans-N-(2-Chlorbenzyl)-4-(4-fluorphenyl)-1-[(1-methyl-1H-imidazol-4-yl)sulfonyl]pyrrolidin-3-amin;
trans-N-(3-Chlorbenzyl)-4-(4-fluorphenyl)-1-[(1-methyl-1H-imidazol-4-yl)sulfonyl]pyrrolidin-3-amin;
trans-N-(4-Chlorbenzyl)-4-(4-fluorphenyl)-1-[(1-methyl-1H-imidazol-4-yl)sulfonyl]pyrrolidin-3-amin;
trans-N-(2-Fluorbenzyl)-4-(4-fluorphenyl)-1-[(1-methyl-1H-imidazol-4-yl)sulfonyl]pyrrolidin-3-amin;
trans-N-(3-Fluorbenzyl)-4-(4-fluorphenyl)-1-[(1-methyl-1H-imidazol-4-yl)sulfonyl]pyrrolidin-3-amin;
trans-4-(4-Fluorphenyl)-1-[(1-methyl-1H-imidazol-4-yl)sulfonyl]-N-[2-(trifluormethyl)benzyl]pyrrolidin-3-amin;
trans-4-(4-Fluorphenyl)-1-[(1-methyl-1H-imidazol-4-yl)sulfonyl]-N-[3-(trifluormethyl)benzyl]pyrrolidin-3-amin;
trans-4-(4-Fluorphenyl)-1-[(1-methyl-1H-imidazol-4-yl)sulfonyl]-N-[4-(trifluormethyl)benzyl]pyrrolidin-3-amin;
trans-4-(4-Fluorphenyl)-1-[(1-methyl-1H-imidazol-4-yl)sulfonyl]-N-(pyridin-2-ylmethyl)pyrrolidin-3-amin;
trans-4-(4-Fluorphenyl)-1-[(1-methyl-1H-imidazol-4-yl)sulfonyl]-N-(pyridin-3-ylmethyl)pyrrolidin-3-amin;
trans-4-(4-Fluorphenyl)-1-[(1-methyl-1H-imidazol-4-yl)sulfonyl]-N-(pyridin-4-ylmethyl)pyrrolidin-3-amin;
{trans-4-(4-Fluorphenyl)-1-[(1-methyl-1H-imidazol-4-yl)sulfonyl]pyrrolidin-3-yl} (phenyl)methanon;
trans-N-[2-Chlor-3-(trifluormethyl)phenyl]-4-(4-fluorphenyl)-1-[(1-methyl-1H-imidazol-4-yl)sulfonyl]pyrrolidin-3-carboxamid;
trans-4-(4-Fluorphenyl)-1-[(1-methyl-1H-imidazol-4-yl)sulfonyl]-N-[3-(trifluormethoxy)phenyl]pyrrolidin-3-carboxamid;
trans-4-(4-Fluorphenyl)-N-[4-fluor-3-(trifluormethyl)phenyl]-1-[(1-methyl-1H-imidazol-4-yl)sulfonyl]pyrrolidin-3-carboxamid;
trans-N-(3-Chlor-4-fluorphenyl)-4-(4-fluorphenyl)-1-[(1-methyl-1H-imidazol-4-yl)sulfonyl]pyrrolidin-3-carboxamid;
trans-N-[3-Chlor-4-(trifluormethyl)phenyl]-4-(4-fluorphenyl)-1-[(1-methyl-1H-imidazol-4-yl)sulfonyl]pyrrolidin-3-carboxamid;
1-Methyl-4-[(3-phenoxy-4-phenylpyrrolidin-1-yl)sulfonyl]-1H-imidazol;
trans-4-(2-Chlorphenyl)-N-(3-chlorphenyl)-1-[(1-methyl-1H-imidazol-4-yl)sulfonyl]pyrrolidin-3-amin;
trans-N-(2,4-Dichlorbenzyl)-4-(4-fluorphenyl)-1-[(1-methyl-1H-imidazol-4-yl)sulfonyl]pyrrolidin-3-amin;
trans-N-(2,4-Dichlorbenzyl)-1-[(1-methyl-1H-imidazol-4-yl)sulfonyl]-4-phenylpyrrolidin-3-amin;
trans-N-[2-Chlor-4-(trifluormethyl)benzyl]-1-[(1-methyl-1H-imidazol-4-yl)sulfonyl]-4-phenylpyrrolidin-3-amin;
trans-N-(2,4-Dichlorbenzyl)-4-(2-fluorphenyl)-1-[(1-methyl-1H-imidazol-4-yl)sulfonyl]pyrrolidin-3-amin;
trans-N-[2-Chlor-4-(trifluormethyl)benzyl]-4-(2-fluorphenyl)-1-[(1-methyl-1H-imidazol-4-yl)sulfonyl]pyrrolidin-3-amin;
trans-4-(2-Chlorphenyl)-N-(2,4-dichlorbenzyl)-1-[(1-methyl-1H-imidazol-4-yl)sulfonyl]pyrrolidin-3-amin;
trans-4-(2-Chlorphenyl)-N-[2-chlor-4-(trifluormethyl)benzyl]-1-[(1-methyl-1H-imidazol-4-yl)sulfonyl]pyrrolidin-3-amin;
trans-N-(2,4-Dichlorbenzyl)-1-[(1-methyl-1H-imidazol-4-yl)sulfonyl]-4-(pyridin-3-yl)pyrrolidin-3-amin;
N-{trans-4-(4-Fluorphenyl)-1-[(1-methyl-1H-imidazol-4-yl)sulfonyl]pyrrolidin-3-yl}-4-(trifluormethyl)pyridin-2-amin;
N-{trans-4-(4-Fluorphenyl)-1-[(1-methyl-1H-imidazol-4-yl)sulfonyl]pyrrolidin-3-yl}-2-(trifluormethyl)pyridin-4-amin;
trans-N-Benzyl-4-(4-fluorphenyl)-1-[(1-methyl-1H-imidazol-4-yl)sulfonyl]pyrrolidin-3-carboxamid;
N-({trans-4-(4-Fluorphenyl)-1-[(1-methyl-1H-imidazol-4-yl)sulfonyl]pyrrolidin-3-yl}methyl)anilin;
N-({trans-4-(4-Fluorphenyl)-1-[(1-methyl-1H-imidazol-4-yl)sulfonyl]pyrrolidin-3-yl}methyl)-4-(trifluormethyl)anilin;
3,5-Dichlor-N-({trans-4-(4-fluorphenyl)-1-[(1-methyl-1H-imidazol-4-yl)sulfonyl]pyrrolidin-3-yl}methyl) anilin;
4-{[trans-3-(4-Fluorphenyl)-4-{[3-(trifluormethyl)phenoxy]methyl}pyrrolidin-1-yl]sulfonyl}-1-methyl-1H-imidazol;
4-{[trans-3-{[2-Chlor-4-(trifluormethyl)phenoxy]methyl}-4-(4-fluorphenyl)pyrrolidin-1-yl]sulfonyl}-1-methyl-1H-imidazol;
4-{[trans-3-(4-Fluorphenyl)-4-{[4-fluor-3-(trifluormethyl)phenoxy]methyl}pyrrolidin-1-yl]sulfonyl}-1-methyl-1H-imidazol;
4-({trans-3-(4-Fluorphenyl)-4-[(3-methylphenoxy)methyl]pyrrolidin-1-yl}sulfonyl)-1-methyl-1H-imidazol;
3-Chlorphenyl){trans-4-(4-fluorphenyl)-1-[(1-methyl-1H-imidazol-4-yl)sulfonyl]pyrrolidin-3-yl}methanon;
trans-N-[4-Fluor-3-(trifluormethoxy)phenyl]-1-[(1-methyl-1H-imidazol-4-yl)sulfonyl]-4-phenylpyrrolidin-3-amin;
trans-N-[4-Chlor-3-(trifluormethoxy)phenyl]-1-[(1-methyl-1H-imidazol-4-yl)sulfonyl]-4-phenylpyrrolidin-3-amin;
{trans-4-(4-Fluorphenyl)-1-[(1-methyl-1H-imidazol-4-yl)sulfonyl]pyrrolidin-3-yl}(3-methylphenyl)methanon;
(3-Fluorphenyl){(trans-4-(4-fluorphenyl)-1-[(1-methyl-1H-imidazol-4-yl)sulfonyl]pyrrolidin-3-yl}methanon;
{trans-4-(4-Fluorphenyl)-1-[(1-methyl-1H-imidazol-4-yl)sulfonyl]pyrrolidin-3-yl}[3-(trifluormethyl)phenyl] methanon;
(4-Fluorphenyl){trans-4-(4-fluorphenyl)-1-[(1-methyl-1H-imidazol-4-yl)sulfonyl]pyrrolidin-3-yl}methanon;
trans-N-(3-Chlorphenyl)-4-(4-fluorphenyl)-1-[(1-methyl-1H-imidazol-4-yl)sulfonyl]pyrrolidin-3-carboxamid;
trans-4-(4-Fluorphenyl)-N-[4-fluor-3-(trifluormethoxy)phenyl]-1-[(1-methyl-1H-imidazol-4-yl)sulfonyl]pyrrolidin-3-amin;
trans-N-[4-Chlor-3-(trifluormethoxy)phenyl]-4-(4-fluorphenyl)-1-[(1-methyl-1H-imidazol-4-yl)sulfonyl]pyrrolidin-3-amin;
trans-N-(2,4-Dichlorbenzyl)-1-[(1-methyl-1H-imidazol-4-yl)sulfonyl]-4-(pyridin-2-yl)pyrrolidin-3-amin;
trans-1-[(1-Methyl-1H-imidazol-4-yl)sulfonyl]-4-(pyridin-3-yl)-N-[3-(trifluormethyl)phenyl]pyrrolidin-3-amin;
4-{[3-(3-Chlorbenzyl)-4-cyclopropylpyrrolidin-1-yl]sulfonyl}-1-methyl-1H-imidazol;
N-[3-(Difluormethyl)-4-fluorphenyl]-4-(4-fluorphenyl)-1-[(1-methyl-1H-imidazol-4-yl)sulfonyl]pyrrolidin-3-amin;
3-Chlor-N-({trans-4-(4-fluorphenyl)-1-[(1-methyl-1H-imidazol-4-yl)sulfonyl]pyrrolidin-3-yl}methyl)-4-(trifluormethyl)anilin;
N-({trans-4-(4-Fluorphenyl)-1-[(1-methyl-1H-imidazol-4-yl)sulfonyl]pyrrolidin-3-yl}methyl)-3-(trifluormethoxy)anilin;
4-Fluor-N-({trans-4-(4-fluorphenyl)-1-[(1-methyl-1H-imidazol-4-yl)sulfonyl]pyrrolidin-3-yl}methyl)-3-(trifluormethyl)anilin;
3-Chlor-4-fluor-N-({trans-4-(4-fluorphenyl)-1-[(1-methyl-1H-imidazol-4-yl)sulfonyl]pyrrolidin-3-yl}methyl) anilin;
N-Benzyl-1-{trans-4-(4-fluorphenyl)-1-[(1-methyl-1H-imidazol-4-yl)sulfonyl]pyrrolidin-3-yl}methanamin;
trans-4-(4-Fluorphenyl)-1-[(1-methyl-1H-imidazol-4-yl)sulfonyl]-N-(3-methylphenyl)pyrrolidin-3-carboxamid;
trans-4-(4-Fluorphenyl)-1-[(1-methyl-1H-imidazol-4-yl)sulfonyl]-N-[3-(trifluormethyl)phenyl]pyrrolidin-3-carboxamid;
4-{[trans-3-[(3-Chlor-4-fluorphenoxy)methyl]-4-(4-fluorphenyl)pyrrolidin-1-yl]sulfonyl}-1-methyl-1H-imidazol;
(4-Chlor-3-fluorphenyl){trans-4-(4-fluorphenyl)-1-[(1-methyl-1H-imidazol-4-yl)sulfonyl]pyrrolidin-3-yl}methanon;
trans-N-[2-Chlor-4-(trifluormethyl)benzyl]-1-[(1-methyl-1H-imidazol-4-yl)sulfonyl]-4-(pyridin-2-yl)pyrrolidin-3-amin;
(3S,4R)-N-[2-Chlor-4-(trifluormethyl)benzyl]-4-(4-fluorphenyl)-1-[(1-methyl-1H-imidazol-4-yl)sulfonyl]pyrrolidin-3-amin;
(3R,4S)-N-[2-Chlor-4-(trifluormethyl)benzyl]-4-(4-fluorphenyl)-1-[(1-methyl-1H-imidazol-4-yl)sulfonyl]pyrrolidin-3-amin;
(3S,4R)-4-(4-Fluorphenyl)-1-[(1-methyl-1H-imidazol-4-yl)sulfonyl]-N-[3-(trifluormethoxy)phenyl]pyrrolidin-3-amin;
(3S,4R)-N,4-bis(4-Fluorphenyl)-1-[(1-methyl-1H-imidazol-4-yl)sulfonyl]pyrrolidin-3-amin;
(3S,4R)-N-(3-Chlorphenyl)-4-(4-fluorphenyl)-1-[(1-methyl-1H-imidazol-4-yl)sulfonyl]pyrrolidin-3-amin;
(3S,4R)-4-(4-Fluorphenyl)-1-[(1-methyl-1H-imidazol-4-yl)sulfonyl]-N-[3-(trifluormethyl)phenyl]pyrrolidin-3-amin;
(3R,4S)-4-(4-Fluorphenyl)-1-[(1-methyl-1H-imidazol-4-yl)sulfonyl]-N-[3-(trifluormethyl)phenyl]pyrrolidin-3-amin;
(3R,4S)-N-(3-Chlorphenyl)-4-(4-fluorphenyl)-1-[(1-methyl-1H-imidazol-4-yl)sulfonyl]pyrrolidin-3-amin;
(3R,4S)-4-(4-Fluorphenyl)-1-[(1-methyl-1H-imidazol-4-yl)sulfonyl]-N-[3-(trifluormethoxy)phenyl]pyrrolidin-3-amin;
(3R,4S)-N,4-bis(4-Fluorphenyl)-1-[(1-methyl-1H-imidazol-4-yl)sulfonyl]pyrrolidin-3-amin;
3-(3-Chlorbenzyl)-1-[(1-methyl-1H-imidazol-4-yl)sulfonyl]-4-phenylpyrrolidin-2-on;
2-Chlor-N-({trans-4-(4-fluorphenyl)-1-[(1-methyl-1H-imidazol-4-yl)sulfonyl]pyrrolidin-3-yl}methyl)-4-(trifluormethyl)anilin;
3-Chlor-N-({trans-4-(4-fluorphenyl)-1-[(1-methyl-1H-imidazol-4-yl)sulfonyl]pyrrolidin-3-yl}methyl)anilin;
N-({trans-4-(4-Fluorphenyl)-1-[(1-methyl-1H-imidazol-4-yl)sulfonyl]pyrrolidin-3-yl}methyl)-3-methylanilin;
N-({trans-4-(4-Fluorphenyl)-1-[(1-methyl-1H-imidazol-4-yl)sulfonyl]pyrrolidin-3-yl}methyl)-3-(trifluormethyl)anilin;
4-({trans-4-(4-Fluorphenyl)-1-[(1-methyl-1H-imidazol-4-yl)sulfonyl]pyrrolidin-3-yl}methoxy)-6-(trifluormethyl)pyrimidin;
trans-4-Cyclopropyl-1-[(1-methyl-1H-imidazol-4-yl)sulfonyl]-N-[3-(trifluormethyl)phenyl]pyrrolidin-3-amin;
trans-N-(3-Chlorphenyl)-4-cyclopropyl-1-[(1-methyl-1H-imidazol-4-yl)sulfonyl]pyrrolidin-3-amin;
4-{[3-(2-Chlorphenyl)-4-(4-fluorphenyl)pyrrolidin-1-yl]sulfonyl}-1-methyl-1H-imidazol;
4-{[3-(4-Chlorphenyl)-4-(4-fluorphenyl)pyrrolidin-1-yl]sulfonyl}-1-methyl-1H-imidazol;
2-Chlor-4-({trans-4-(4-fluorphenyl)-1-[(1-methyl-1H-imidazol-4-yl)sulfonyl]pyrrolidin-3-yl}amino)benzonitril;
2-Fluor-4-({trans-4-(4-fluorphenyl)-1-[(1-methyl-1H-imidazol-4-yl)sulfonyl]pyrrolidin-3-yl}amino)benzonitril;
3-(3-Chlorbenzyl)-1-[(1-methyl-1H-imidazol-4-yl)sulfonyl]-4-phenylimidazolidin-2-on;
trans-1-[(1-Methyl-1H-imidazol-4-yl)sulfonyl]-4-(pyridin-2-yl)-N-[3-(trifluormethyl)phenyl]pyrrolidin-3-amin;
trans-N-(3-Chlorphenyl)-1-[(1-methyl-1H-imidazol-4-yl)sulfonyl]-4-(pyridin-2-yl)pyrrolidin-3-amin;
2-({trans-4-(4-Fluorphenyl)-1-[(1-methyl-1H-imidazol-4-yl)sulfonyl]pyrrolidin-3-yl}methoxy)-4-(trifluormethyl)pyridin;
trans-N-(3-Cyanophenyl)-4-(4-fluorphenyl)-1-[(1-methyl-1H-imidazol-4-yl)sulfonyl]pyrrolidin-3-carboxamid;
4-{[trans-3-[(4-Fluorphenoxy)methyl]-4-(4-fluorphenyl)pyrrolidin-1-yl]sulfonyl}-1-methyl-1H-imidazol;
4-(4-Fluorphenyl)-1-[(1-methyl-1H-imidazol-4-yl)sulfonyl]-N-[3-(propan-2-yloxy)phenyl]pyrrolidin-3-amin;
N-{4-(4-Fluorphenyl)-1-[(1-methyl-1H-imidazol-4-yl)sulfonyl]pyrrolidin-3-yl}pyridazin-3-amin;
N-{(3R,4S)-4-(4-Fluorphenyl)-1-[(1-methyl-1H-imidazol-4-yl)sulfonyl]pyrrolidin-3-yl}-6-(trifluormethyl)pyridin-2-amin;
(3R,4S)-N-(3-Chlor-4-fluorphenyl)-4-(4-fluorphenyl)-1-[(1-methyl-1H-imidazol-4-yl)sulfonyl]pyrrolidin-3-amin;
(3R,4S)-N-(3-Fluorphenyl)-4-(4-fluorphenyl)-1-[(1-methyl-1H-imidazol-4-yl)sulfonyl]pyrrolidin-3-amin;
trans-N-(-3-methyl-1-[(1-methyl-1H-imidazol-4-yl)sulfonyl]-4-phenylpyrrolidin-3-amin;
trans-3-Methyl-1-[(1-methyl-1H-imidazol-4-yl)sulfonyl]-4-phenyl-N-[4-(trifluormethyl)benzyl]pyrrolidin-3-amin;
trans-N-[2-Chlor-4-(trifluormethyl)benzyl]-3-methyl-1-[(1-methyl-1H-imidazol-4-yl)sulfonyl]-4-phenylpyrrolidin-3-amin;
(3,4-Difluorphenyl){trans-4-(4-fluorphenyl)-1-[(1-methyl-1H-imidazol-4-yl)sulfonyl]pyrrolidin-3-yl}methanon;
trans-N,4-bis(4-Fluorphenyl)-1-[(1-methyl-1H-imidazol-4-yl)sulfonyl]pyrrolidin-3-carboxamid;
(3,4-Dichlorphenyl){trans-4-(4-fluorphenyl)-1-[(1-methyl-1H-imidazol-4-yl)sulfonyl]pyrrolidin-3-yl}methanon;
(3,5-Dichlorphenyl){trans-4-(4-fluorphenyl)-1-[(1-methyl-1H-imidazol-4-yl)sulfonyl]pyrrolidin-3-yl}methanon;
(3-Chlor-5-fluorphenyl){trans-4-(4-fluorphenyl)-1-[(1-methyl-1H-imidazol-4-yl)sulfonyl]pyrrolidin-3-yl}methanon;
{trans-4-(4-Fluorphenyl)-1-[(1-methyl-1H-imidazol-4-yl)sulfonyl]pyrrolidin-3-yl}[3-(trifluormethoxy)phenyl] methanon;
{trans-4-(4-Fluorphenyl)-1-[(1-methyl-1H-imidazol-4-yl)sulfonyl]pyrrolidin-3-yl}[4-(trifluormethoxy)phenyl] methanon;
3-[({trans-4-(4-Fluorphenyl)-1-[(1-methyl-1H-imidazol-4-yl)sulfonyl]pyrrolidin-3-yl}methyl)amino]benzamid;
3-(Aminomethyl)-N-({trans-4-(4-fluorphenyl)-1-[(1-methyl-1H-imidazol-4-yl)sulfonyl]pyrrolidin-3-yl}methyl)anilin;
4-({trans-4-(4-Fluorphenyl)-1-[(1-methyl-1H-imidazol-4-yl)sulfonyl]pyrrolidin-3-yl}methoxy)-2-(trifluormethyl)pyridin;
4-Fluor-N-({trans-4-(4-fluorphenyl)-1-[(1-methyl-1H-imidazol-4-yl)sulfonyl]pyrrolidin-3-yl}methyl)anilin;
N-{(3R,4S)-4-(4-Fluorphenyl)-1-[(1-methyl-1H-imidazol-4-yl)sulfonyl]pyrrolidin-3-yl}-4-(trifluormethyl)pyridin-2-amin;
trans-4-(4-Fluorphenyl)-1-[(1-methyl-1H-imidazol-4-yl)sulfonyl]-N-[4-(trifluormethyl)pyridin-2-yl]pyrrolidin-3-carboxamid;
trans-4-(4-Fluorphenyl)-N-[4-fluor-3-(trifluormethoxy)phenyl]-1-[(1-methyl-1H-imidazol-4-yl)sulfonyl]pyrrolidin-3-carboxamid;
trans-N-[4-Chlor-3-(trifluormethoxy)phenyl]-4-(4-fluorphenyl)-1-[(1-methyl-1H-imidazol-4-yl)sulfonyl]pyrrolidin-3-carboxamid;
N-[4-Chlor-2-({trans-4-(4-fluorphenyl)-1-[(1-methyl-1H-imidazol-4-yl)sulfonyl]pyrrolidin-3-yl}amino)phenyl]acetamid;
trans-4-[(3-Chlorphenyl)amino]-1-[(1-methyl-1H-imidazol-4-yl)sulfonyl]pyrrolidin-3-ol;
N-[trans-1-[(1-methyl-1H-imidazol-4-yl)sulfonyl]-4-(pyridin-2-yl)pyrrolidin-3-yl]-1-(trifluormethyl)cyclopropanecarboxamid;
N-{(3R,4S)-4-(4-Fluorphenyl)-1-[(1-methyl-1H-imidazol-4-yl)sulfonyl]pyrrolidin-3-yl}-1-(trifluormethyl)cyclopropanecarboxamid;
4-Fluor-N-({trans-4-(4-fluorphenyl)-1-[(1-methyl-1H-imidazol-4-yl)sulfonyl]pyrrolidin-3-yl}methyl)-3-(trifluormethoxy)anilin;
4-Chlor-N-({trans-4-(4-fluorphenyl)-1-[(1-methyl-1H-imidazol-4-yl)sulfonyl]pyrrolidin-3-yl}methyl)-3-(trifluormethoxy)anilin;
trans-N-(3-Chlorphenyl)-4-(4-fluorphenoxy)-1-[(1-methyl-1H-imidazol-4-yl)sulfonyl]pyrrolidin-3-amin;
2-({trans-4-(4-Fluorphenyl)-1-[(1-methyl-1H-imidazol-4-yl)sulfonyl]pyrrolidin-3-yl}oxy)-4-(trifluormethyl)pyridin;
3-({(3R,4S)-4-(4-Fluorphenyl)-1-[(1-methyl-1H-imidazol-4-yl)sulfonyl]pyrrolidin-3-yl}amino)benzonitril;
6-Chlor-2-[trans-1-[(1-methyl-1H-imidazol-4-yl)sulfonyl]-4-(pyridin-2-yl)pyrrolidin-3-yl]-2,3-dihydro-1H-isoindol-1-on;
3-Benzyl-4-(4-fluorphenyl)-1-[(1-methyl-1H-imidazol-4-yl)sulfonyl]pyrrolidin-3-ol;
N-{trans-4-(4-Fluorphenyl)-1-[(1-methyl-1H-imidazol-4-yl)sulfonyl]pyrrolidin-3-yl}-5-(trifluormethyl)pyridazin-3-amin;
N-{4-(4-Fluorphenyl)-1-[(1-methyl-1H-imidazol-4-yl)sulfonyl]pyrrolidin-3-yl}-4-(trifluormethyl)pyridazin-3-amin;
1-Methyl-4-{[trans-3-phenyl-4-(phenylsulfonyl)pyrrolidin-1-yl]sulfonyl}-1H-imidazol;
(3R,4S)-4-(4-Fluorphenyl)-1-[(1-methyl-1H-imidazol-4-yl)sulfonyl]-N-{[1-(trifluormethyl)cyclopropyl]methyl}pyrrolidin-3-amin;
(3S,4R)-N-(3-Chlor-4-fluorphenyl)-4-(4-fluorphenyl)-1-[(1-methyl-1H-imidazol-4-yl)sulfonyl]pyrrolidin-3-amin;
N-{trans-3-Methyl-1-[(1-methyl-1H-imidazol-4-yl)sulfonyl]-4-phenylpyrrolidin-3-yl}-4-(trifluormethyl)pyridin-2-amin;
3-({(3S,4R)-4-(4-Fluorphenyl)-1-[(1-methyl-1H-imidazol-4-yl)sulfonyl]pyrrolidin-3-yl}amino)benzonitril;
N-{(3R,4S)-4-(4-Fluorphenyl)-1-[(1-methyl-1H-imidazol-4-yl)sulfonyl]pyrrolidin-3-yl}-6-(trifluormethyl)pyrimidin-4-amin;
(3R,4S)-4-(4-Fluorphenyl)-1-[(1-methyl-1H-imidazol-4-yl)sulfonyl]-N-{[5-(trifluormethyl)furan-2-yl]methyl}pyrrolidin-3-amin;
1,1,1-Trifluor-2-[3-({(3R,4S)-4-(4-fluorphenyl)-1-[(1-methyl-1H-imidazol-4-yl)sulfonyl}amino)phenyl]propan-2-ol;
(3R,4S)-N-[(5-Chlorthiophen-2-yl)methyl]-4-(4-fluorphenyl)-1-[(1-methyl-1H-imidazol-4-yl)sulfonyl]pyrrolidin-3-amin;
(3R,4S)-N-[2-Chlor-4-(trifluormethyl)benzyl]-1-[(1-methyl-1H-imidazol-4-yl)sulfonyl]-4-phenylpyrrolidin-3-amin;
(3S,4R)-N-[2-Chlor-4-(trifluormethyl)benzyl]-1-[(1-methyl-1H-imidazol-4-yl)sulfonyl]-4-phenylpyrrolidin-3-amin;
N-{(3R,4S)-1-[(1-Methyl-1H-imidazol-4-yl)sulfonyl]-4-phenylpyrrolidin-3-yl}-4-(trifluormethyl)pyridin-2-amin;
(3R,4S)-1-[(1-Methyl-1H-imidazol-4-yl)sulfonyl]-4-phenyl-N-[3-(trifluormethyl)phenyl]pyrrolidin-3-amin;
(3R,4S)-1-[(1-methyl-1H-imidazol-4-yl)sulfonyl] -4-phenyl-N-[4-(trifluormethyl)benzyl]pyrrolidin-3-amin;
1,1,1-Trifluor-2-[3-({(3R,4S)-1-[(1-methyl-1H-imidazol-4-yl)sulfonyl]-4-phenylpyrrolidin-3-yl}amino)phenyl]propan-2-ol;
trans-4-(Benzyloxy)-N-(3-chlorphenyl)-1-[(1-methyl-1H-imidazol-4-yl)sulfonyl]pyrrolidin-3-amin;
trans-1-[(1-Methyl-1H-imidazol-4-yl)sulfonyl]-4-phenyl-3-{[4-(trifluormethyl)benzyl]amino}pyrrolidin-3-yl]methanol;
trans-N-(3-Chlorphenyl)-1-[(1-methyl-1H-imidazol-4-yl)sulfonyl]-4-(prop-2-en-1-yloxy)pyrrolidin-3-amin;
trans-N-(3-Chlorphenyl)-1-[(1-methyl-1H-imidazol-4-yl)sulfonyl]-4-[(2-methylprop-2-en-1-yl)oxy]pyrrolidin-3-amin;
3-{trans-1-[(1-methyl-1H-imidazol-4-yl)sulfonyl]-4-phenylpyrrolidin-3-yl}-6-(trifluormethyl)-3,4-dihydrochinazolin-2(1H)-on;
4-Fluor-N-{(3R,4S)-1-[(1-methyl-1H-imidazol-4-yl)sulfonyl]-4-phenylpyrrolidin-3-yl}pyridin-2-amin;
N-{(3R,4S)-1-[(1-Methyl-1H-imidazol-4-yl)sulfonyl]-4-phenylpyrrolidin-3-yl}-7H-pyrrolo[2,3-d]pyrimidin-4-amin;
trans-7-[(1-Methyl-1H-imidazol-4-yl)sulfonyl]-9-phenyl-1-[4-(trifluormethyl)benzyl]-3-oxa-1,7-diazaspiro[4.4]nonan-2-on;
3,5-Difluor-N-{(3R,4S)-1-[(1-methyl-1H-imidazol-4-yl)sulfonyl]-4-phenylpyrrolidin-3-yl}-4-(trifluormethyl)pyridin-2-amin;
(3R,4S)-1-[(1-Methyl-1H-imidazol-4-yl)sulfonyl]-4-phenyl-N-{[5-(trifluormethyl)pyridin-2-yl]methyl}pyrrolidin-3-amin;
5-Cyclopropyl-N-{(3R,4S)-1-[(1-methyl-1H-imidazol-4-yl)sulfonyl]-4-phenylpyrrolidin-3-yl}pyrazin-2-amin;
5-Cyclobutyl-N-{(3R,4S)-1-[(1-methyl-1H-imidazol-4-yl)sulfonyl]-4-phenylpyrrolidin-3-yl}pyrazin-2-amin;
trans-N-(3-Chlorphenyl)-1-[(1-methyl-1H-imidazol-4-yl)sulfonyl]-4-(2-methylpropoxy)pyrrolidin-3-amin;
N-{(3R,4S)-1-[(1-Methyl-1H-imidazol-4-yl)sulfonyl]-4-phenylpyrrolidin-3-yl}-5-(pyrrolidin-1-yl)pyrazin-2-amin;
N-{(3R,4S)-1-[(1-Methyl-1H-imidazol-4-yl)sulfonyl]-4-phenylpyrrolidin-3-yl}-5-(trifluormethyl)pyrazin-2-carboxamid;
5-Chlor-N-{(3R,4S)-1-[(1-methyl-1H-imidazol-4-yl)sulfonyl]-4-phenylpyrrolidin-3-yl}pyrazine-2-carboxamid;
(3R,4S)-N-(4-Chlorbenzyl)-1-[(1-methyl-1H-imidazol-4-yl)sulfonyl]-4-phenylpyrrolidin-3-amin;
(3R,4S)-N-(3-Chlor-4-fluorphenyl)-1-[(1-methyl-1H-imidazol-4-yl)sulfonyl]-4-phenylpyrrolidin-3-amin;
(3R,4S)-N-(3,4-Difluorphenyl)-4-(4-fluorphenyl)-1-[(1-methyl-1H-imidazol-4-yl)sulfonyl]pyrrolidin-3-amin;
6-Chlor-N-{(3R,4S)-1-[(1-methyl-1H-imidazol-4-yl)sulfonyl]-4-phenylpyrrolidin-3-yl}pyridazin-3-carboxamid;
N-{(3R,4S)-1-[(1-Methyl-1H-imidazol-4-yl)sulfonyl]-4-phenylpyrrolidin-3-yl}-2-(morpholin-4-yl)pyrimidin-4-amin;
4-({(3R,4S)-1-[(1-Methyl-1H-imidazol-4-yl)sulfonyl]-4-phenylpyrrolidin-3-yl}amino)-2,3-dihydro-1H-isoindol-1-on;
(3R,4S)-1-[(1-Methyl-1H-imidazol-4-yl)sulfonyl]-N-[3-(methylsulfonyl)phenyl]-4-phenylpyrrolidin-3-amin;
(3R,4S)-1-[(1-Methyl-1H-imidazol-4-yl)sulfonyl]-4-phenyl-N-{[5-(trifluormethyl)piperazin-2-yl]methyl}pyrrolidin-3-amin;
5-Fluor-N-{(3R,4S)-1-[(1-methyl-1H-imidazol-4-yl)sulfonyl]-4-phenylpyrrolidin-3-yl}pyrimidin-2-amin;
trans-4-Cyclohexyl-1-[(1-methyl-1H-imidazol-4-yl)sulfonyl]-N-[3-(trifluormethoxy)phenyl]pyrrolidin-3-amin;
(3R,4S)-1-[(1-Methyl-1H-imidazol-4-yl)sulfonyl]-4-phenyl-N-(4,4,4-trifluorbutyl)pyrrolidin-3-amin;
(3R,4S)-N-(2-Cyclopentylethyl)-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-4-phenyl-N-[(5-propylfuran-2-yl)methyl]pyrrolidin-3-amin;
(3R,4S)-1-[(1-Methyl-1H-imidazol-4-yl)sulfonyl]-N-[(4-methyltetrahydro-2H-pyran-4-yl)methyl]-4-phenylpyrrolidin-3-amin;
(3R,4S)-1-[(1-Methyl-1H-imidazol-4-yl)sulfonyl]-4-phenyl-N-[(4-phenyl-1,3-thiazol-5-yl)methyl]pyrrolidin-3-amin;
(3R,4S)-1-[(1-Methyl-1H-imidazol-4-yl)sulfonyl]-N-[(5-methyl-1,3-thiazol-2-yl)methyl]-4-phenylpyrrolidin-3-amin;
(3R,4S)-N-[(3,5-Dichlorpyridin-4-yl)methyl]-1-[(1-methyl-1H-imidazol-4-yl)sulfonyl]-4-phenylpyrrolidin-3-amin;
(3R,4S)-N-(2-Ethylhexyl)-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-N-[(4-methyl-1,3-thiazol-5-yl)methyl]-4-phenylpyrrolidin-3-amin;
(3R,4S)-N-[(2,4-Dichlor-1,3-thiazol-5-yl)methyl]-1-[(1-methyl-1H-imidazol-4-yl)sulfonyl]-4-phenylpyrrolidin-3-amin;
(3R,4S)-N-(2,2-Dimethylpropyl)-1-[(1-methyl-1H-imidazol-4-yl)sulfonyl]-4-phenylpyrrolidin-3-amin;
(3R,4S)-N-[(5-Methyl-1-benzothiophen-2-yl)methyl]-1-[(1-methyl-1H-imidazol-4-yl)sulfonyl] -4-phenylpyrrolidin-3-amin;
(3R,4S)-N-[(2-Brom-1,3-thiazol-5-yl)methyl]-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-N-(2-methylpropyl)-4-phenylpyrrolidin-3-amin;
(3R,4S)-N-[(3-Chlor-1-benzothiophen-2-yl)methyl]-1-[(1-methyl-1H-imidazol-4-yl)sulfonyl]-4-phenylpyrrolidin-3-amin;
(3R,4S)-N-[(2,4-Dimethyl-1,3-thiazol-5-yl)methyl]-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-4-phenyl-N-(thieno[2,3-b]pyridin-2-ylmethyl)pyrrolidin-3-amin;
(3R,4S)-N-[(2-Methyl-1-benzofuran-3-yl)methyl]-1-[(1-methyl-1H-imidazol-4-yl)sulfonyl]-4-phenylpyrrolidin-3-amin;
(3R,4S)-N-[(6-Chlor-2-methylimidazo[1,2-a]pyridin-3-yl)methyl]-1-[(1-methyl-1H-imidazol-4-yl)sulfonyl] -4-phenylpyrrolidin-3-amin;
(3R,4S)-N-{[5-(4-Fluorphenyl)pyridin-3-yl]methyl}-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-N-(2-methylpentyl)-4-phenylpyrrolidin-3-amin;
(3R,4S)-1-[(1-Methyl-1H-imidazol-4-yl)sulfonyl]-4-phenyl-N-(tetrahydrofuran-3-ylmethyl)pyrrolidin-3-amin;
(3R,4S)-1-[(1-Methyl-1H-imidazol-4-yl)sulfonyl]-N-pentyl-4-phenylpyrrolidin-3-amin;
(3R,4S)-N-(2-Ethylbutyl)-1-[(1-methyl-1H-imidazol-4-yl)sulfonyl]-4-phenylpyrrolidin-3-amin;
(3R,4S)-N-(2,2-Dimethylbutyl)-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-N-[(2-methyl-1,3-thiazol-5-yl)methyl]-4-phenylpyrrolidin-3-amin;
(3R,4S)-1-[(1-Methyl-1H-imidazol-4-yl)sulfonyl]-4-phenyl-N-[2-(tetrahydro-2H-pyran-4-yl)ethyl]pyrrolidin-3-amin;
(3R,4S)-N-(1,3-Benzthiazol-2-ylmethyl)-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-N-[(4-methyl-1,3-thiazol-2-yl)methyl]-4-phenylpyrrolidin-3-amin;
(3R,4S)-N-[(4,5-Dimethylthiophen-2-yl)methyl]-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-4-phenyl-N-(4,5,6,7-tetrahydro-1,3-benzothiazol-2-ylmethyl)pyrrolidin-3-amin;
(3R,4S)-1-[(1-Methyl-1H-imidazol-4-yl)sulfonyl]-4-phenyl-N-(1,3-thiazol-5-ylmethyl)pyrrolidin-3-amin;
(3R,4S)-1-[(1-Methyl-1H-imidazol-4-yl)sulfonyl]-4-phenyl-N-[2-(2,6,6-trimethylcyclohex-1-en-1-yl)ethyl]pyrrolidin-3-amin;
(3R,4S)-1-[(1-Methyl-1H-imidazol-4-yl)sulfonyl]-4-phenyl-N-{[1-(propan-2-yl)piperidin-4-yl]methyl}pyrrolidin-3-amin;
(3R,4S)-N-{[4-(4-Fluorphenyl)-1,3-thiazol-2-yl]methyl}-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-N-[2-(4-methylphenyl)ethyl]-4-phenylpyrrolidin-3-amin;
(3R,4S)-1-[(1-Methyl-1H-imidazol-4-yl)sulfonyl]-N-[(5-methylthiophen-2-yl)methyl]-4-phenylpyrrolidin-3-amin;
(3R,4S)-N-(3-Methylbutyl)-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-4-phenyl-N-(3,5,5-trimethylhexyl)pyrrolidin-3-amin;
(3R,4S)-1-[(1-Methyl-1H-imidazol-4-yl)sulfonyl]-4-phenyl-N-(2-phenylpropyl)pyrrolidin-3-amin;
(3R,4S)-N-(3,3-Dimethylbutyl)-1-[(1-methyl-1H-imidazol-4-yl)sulfonyl]-4-phenylpyrrolidin-3-amin;
(3R,4S)-N-[(1-Methylcyclohexyl)methyl]-1-[(1-methyl-1H-imidazol-4-yl)sulfonyl]-4-phenylpyrrolidin-3-amin;
(3R,4S)-N-[2-(3-Chlorphenyl)ethyl]-1-[(1-methyl-1H-imidazol-4-yl)sulfonyl]-4-phenylpyrrolidin-3-amin;
(3R,4S)-N-[2-(4-tert-Butylphenoxy)ethyl]-1-[(1-methyl-1H-imidazol-4-yl)sulfonyl]-4-phenylpyrrolidin-3-amin;
(3R,4S)-N-(2-Ethyl-3-methylbutyl)-1-[(1-methyl-1H-imidazol-4-yl)sulfonyl]-4-phenylpyrrolidin-3-amin;
(3R,4S)-N-[3-(3-Chlorphenyl)propyl]-1-[(1-methyl-1H-imidazol-4-yl)sulfonyl]-4-phenylpyrrolidin-3-amin;
(3R,4S)-N-[(4-Brom-1,3-thiazol-2-yl)methyl]-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-N-[(4-methyl-2-phenyl-1,3-thiazol-5-yl)methyl]-4-phenylpyrrolidin-3-amin;
N-(3,4-Difluorphenyl)-1-{[3-methyl-1-(propan-2-yl)-1H-pyrazol-4-yl]sulfonyl}-4-phenylpyrrolidin-3-amin;
N-(3,4-Difluorphenyl)-1-{[1-methyl-3-(trifluormethyl)-1H-pyrazol-4-yl]sulfonyl}-4-phenylpyrrolidin-3-amin;
1-[(1-Cyclopentyl-3-methyl-1H-pyrazol-4-yl)sulfonyl]-N-(3,4-difluorphenyl)-4-phenylpyrrolidin-3-amin;
N-(3,4-Difluorphenyl)-1-[(1,3-dimethyl-1H-pyrazol-5-yl)sulfonyl]-4-phenylpyrrolidin-3-amin;
N-(3,4-Difluorphenyl)-1-[(1,5-dimethyl-1H-pyrazol-4-yl)sulfonyl]-4-phenylpyrrolidin-3-amin;
1-[(5-Chlor-1,3-dimethyl-1H-pyrazol-4-yl)sulfonyl]-N-(3,4-difluorphenyl)-4-phenylpyrrolidin-3-amin;
N-(3,4-Difluorphenyl)-1-[(1,2-dimethyl-1H-imidazol-5-yl)sulfonyl] -4-phenylpyrrolidin-3-amin;
N-(3,4-Difluorphenyl)-1-[(1-methyl-1H-pyrazol-3-yl)sulfonyl]-4-phenylpyrrolidin-3-amin;
N-(3,4-Difluorphenyl)-4-phenyl-1-[(1,3,5-trimethyl-1H-pyrazol-4-yl)sulfonyl]pyrrolidin-3-amin;
N-(3,4-Difluorphenyl)-1-[(1-methyl-1H-pyrazol-5-yl)sulfonyl]-4-phenylpyrrolidin-3-amin;
trans-N-(3-Chlorphenyl)-4-(cyclopropylmethoxy)-1-[(1-methyl-1H-imidazol-4-yl)sulfonyl]pyrrolidin-3-amin;
N-{trans-4-Cyclohexyl-1-[(1-methyl-1H-imidazol-4-yl)sulfonyl]pyrrolidin-3-yl}-6-(trifluormethyl)pyrimidin-4-amin;
2-Methoxy-N-{(3R,4S)-1-[(1-methyl-1H-imidazol-4-yl)sulfonyl]-4-phenylpyrrolidin-3-yl}pyrimidin-4-amin;
(3R,4S)-1-[(1-Methyl-1H-imidazol-4-yl)sulfonyl]-N-[(2-methyl-1,3-thiazol-4-yl)methyl]-4-phenylpyrrolidin-3-amin;
(3R,4S)-N-[(2-Ethyl-1-benzofuran-3-yl)methyl]-1-[(1-methyl-1H-imidazol-4-yl)sulfonyl]-4-phenylpyrrolidin-3-amin;
(3R,4S)-N-(Imidazo[1,2-a]pyridin-8-ylmethyl)-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-N-[(5-methylpyridin-2-yl)methyl]-4-phenylpyrrolidin-3-amin;
(3R,4S)-1-[(1-Methyl-1H-imidazol-4-yl)sulfonyl]-4-phenyl-N-(pyrazolo[1,5-a]pyridin-3-ylmethyl)pyrrolidin-3-amin;
N-{trans-4-Hydroxy-1-[(1-methyl-1H-imidazol-4-yl)sulfonyl]-4-phenylpyrrolidin-3-yl}-4-(trifluormethyl)benzamid;
1-[(1-Methyl-1H-imidazol-4-yl)sulfonyl]-3-phenyl-4-{[4-(trifluormethyl)benzyl]amino}pyrrolidin-3-ol;
(3R,4S)-N-[4-Chlor-3-(trifluormethyl)phenyl]-4-(4-fluorphenyl)-1-[(1-methyl-1H-imidazol-4-yl)sulfonyl]pyrrolidin-3-amin;
Ethyl-{(3R,4S)-1-[(1-methyl-1H-imidazol-4-yl)sulfonyl]-4-phenylpyrrolidin-3-yl}carbamat;
Propyl-{(3R,4S)-1-[(1-methyl-1H-imidazol-4-yl)sulfonyl]-4-phenylpyrrolidin-3-yl}carbamat;
2-Methylpropyl-{(3R,4S)-1-[(1-methyl-1H-imidazol-4-yl)sulfonyl]-4-phenylpyrrolidin-3-yl}carbamat;
Butyl-{(3R,4S)-1-[(1-methyl-1H-imidazol-4-yl)sulfonyl]-4-phenylpyrrolidin-3-yl}carbamat;
(3R,4S)-N-(2-Cyclopropylethyl)-1-[(1-methyl-1H-imidazol-4-yl)sulfonyl]-4-phenylpyrrolidin-3-amin;
2-Cyclopropyl-N-{(3R,4S)-1-[(1-methyl-1H-imidazol-4-yl)sulfonyl]-4-phenylpyrrolidin-3-yl}acetamid;
(3R,4S)-N-[(2E)-Hex-2-en-1-yl]-1-[(1-methyl-1H-imidazol-4-yl)sulfonyl]-4-phenylpyrrolidin-3-amin;
(3R,4S)-N-Hexyl-1-[(1-methyl-1H-imidazol-4-yl)sulfonyl]-4-phenylpyrrolidin-3-amin;
N-{(3R,4S)-1-[(1-Methyl-1H-imidazol-4-yl)sulfonyl]-4-phenylpyrrolidin-3-yl}pentanamid;
N-{(3R,4S)-1-[(1-Methyl-1H-imidazol-4-yl)sulfonyl]-4-phenylpyrrolidin-3-yl }hexanamid;
(3R,4S)-N-(2-{[tert-Butyl(dimethyl)silyl]oxy}ethyl)-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-4-phenyl-N-{[6-(trifluormethyl)pyridin-2-yl]methyl}pyrrolidin-3-amin;
(3R,4S)-N-[(3-Chlorthiophen-2-yl)methyl]-1-[(1-methyl-1H-imidazol-4-yl)sulfonyl]-4-phenylpyrrolidin-3-amin;
(3R,4S)-N-[(4,4-Difluorcyclohexyl)methyl]-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-4-phenyl-N-(pyrazolo[1,5-a]pyridin-7-ylmethyl)pyrrolidin-3-amin;
(3R,4S)-1-[(1-Methyl-1H-iniidazol-4-yl)sulfonyl]-4-phenyl-N-{[4-(propan-2-yl)-1,3-thiazol-2-yl]methyl}pyrrolidin-3-amin;
(3R,4S)-N-[(2,2-Dimethyltetrahydro-2H-pyran-4-yl)methyl]-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-4-phenyl-N-{[5-(tetrahydro-2H-pyran-2-yl)thiophen-2-yl]methyl}pyrrolidin-3-amin;
(3R,4S)-N-[(5-Brom-4-methyl-1,3-thiazol-2-yl)methyl]-1-[(1-methyl-1H-imidazol-4-yl)sulfonyl]-4-phenylpyrrolidin-3-amin;
(3R,4S)-N-(Imidazo[1,5-a]pyridin-5-ylmethyl)-1-[(1-methyl-1H-imidazol-4-yl)sulfonyl]-4-phenylpyrrolidin-3-amin;
(3R,4S)-N-(2,3-Dimethylpentyl)-1-[(1-methyl-1H-imidazol-4-yl)sulfonyl]-4-phenylpyrrolidin-3-amin;
(3R,4S)-N-[(2-Chlor-1,3-thiazol-4-yl)methyl]-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-N-[(5-methylimidazo[1,2-a]pyridin-3-yl)methyl]-4-phenylpyrrolidin-3-amin;
(3R,4S)-1-[(1-Methyl-1H-imidazol-4-yl)sulfonyl]-4-phenyl-N-[(2-phenylcyclopropyl)methyl]pyrrolidin-3-amin;
(3R,4S)-N-[(4-Chlor-1,3-thiazol-5-yl)methyl]-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-4-phenyl-N-(3,3,3-trifluor-2-methylpropyl)pyrrolidin-3-amin;
(3R,4S)-N-[(2-Chlor-3-fluorpyridin-4-yl)methyl]-1-[(1-methyl-1H-imidazol-4-yl)sulfonyl]-4-phenylpyrrolidin-3-amin;
(3R,4S)-N-[(5-Fluor-1-benzothiophen-2-yl)methyl]-1-[(1-methyl-1H-imidazol-4-yl)sulfonyl]-4-phenylpyrrolidin-3-amin;
(3R,4S)-N-[5-Chlor-2-(difluormethoxy)benzyl]-1-[(1-methyl-1H-imidazol-4-yl)sulfonyl]-4-phenylpyrrolidin-3-amin;
(3R,4S)-N-[(2,5-Dichlorthiophen-3-yl)methyl]-1-[(1-methyl-1H-imidazol-4-yl)sulfonyl]-4-phenylpyrrolidin-3-amin;
(3R,4S)-N-{[2-(4-Fluorphenyl)pyridin-3-yl]methyl}-1-[(1-methyl-1H-imidazol-4-yl)sulfonyl]-4-phenylpyrrolidin-3-amin;
(3R,4S)-1-[(1-methyl-1H-imidazol-4-yl)sulfonyl]-N-{[2-(4-methylphenyl)-1,3-thiazol-5-yl]methyl}-4-phenylpyrrolidin-3-amin;
(3R,4S)-N-(3-Cyclopropylpropyl)-1-[(1-methyl-1H-imidazol-4-yl)sulfonyl]-4-phenylpyrrolidin-3-amin;
5-Fluor-4-methyl-N-1(3R,4S)-1-[(1-methyl-1H-imidazol-4-yl)sulfonyl]-4-phenylpyrrolidin-3-yl}pyridin-2-amin;
1,5-Dimethyl-N-{(3R,4S)-1-[(1-methyl-1H-imidazol-4-yl)sulfonyl]-4-phenylpyrrolidin-3-yl}-1H-1,2,4-triazol-3-amin;
1,5-Dimethyl-4-({(3R,4S)-1-[(1-methyl-1H-imidazol-4-yl)sulfonyl]-4-phenylpyrrolidin-3-yl}amino)-1H-pyrrol-2-carbonitril;
(3S,4R)-4-(4-Fluorphenyl)-1-[(1-methyl-1H-imidazol-4-yl)sulfonyl]-N-[3-(propan-2-yloxy)propyl]pyrrolidin-3-carboxamid;
(3S,4R)-N-(3-Ethoxypropyl)-4-(4-fluorphenyl)-1-[(1-methyl-1H-imidazol-4-yl)sulfonyl]pyrrolidin-3-carboxamid;
(3S,4R)-4-(4-Fluorphenyl)-N-(2-methoxyethyl)-1-[(1-methyl-1H-imidazol-4-yl)sulfonyl]pyrrolidin-3-carboxamid;
(3S,4R)-4-(4-Fluorphenyl)-1-[(1-methyl-1H-imidazol-4-yl)sulfonyl]-N-propylpyrrolidin-3-carboxamid;
(3S,4R)-N-Butyl-4-(4-fluorphenyl)-1-[(1-methyl-1H-imidazol-4-yl)sulfonyl]pyrrolidin-3-carboxamid;
(3S,4R)-4-(4-Fluorphenyl)-N-[1-methoxypropan-2-yl]-1-[(1-methyl-1H-imidazol-4-yl)sulfonyl]pyrrolidin-3-carboxamid;
(3R,4S)-4-(4-Fluorphenyl)-N-(3-methoxypropyl)-1-[(1-methyl-1H-imidazol-4-yl)sulfonyl]pyrrolidin-3-carboxamid;
(3S,4R)-N-(2-Ethoxyethyl)-4-(4-fluorphenyl)-1-[(1-methyl-1H-imidazol-4-yl)sulfonyl]pyrrolidin-3-carboxamid;
(3R,4S)-N-[4-Chlor-3-(trifluormethoxy)phenyl]-4-(4-fluorphenyl)-1-[(1-methyl-1H-imidazol-4-yl)sulfonyl]pyrrolidin-3-amin;
(3S,4R)-N-(Cyclopropylmethyl)-4-(4-fluorphenyl)-1-[(1-methyl-1H-imidazol-4-yl)sulfonyl]pyrrolidin-3-carboxamid;
(3S,4R)-4-(4-Fluorphenyl)-1-[(1-methyl-1H-imidazol-4-yl)sulfonyl]-N-[2-(propan-2-yloxy)ethyl]pyrrolidin-3-carboxamid;
(3R,4S)-N-(3-Methoxypropyl)-1-[(1-methyl-1H-imidazol-4-yl)sulfonyl]-4-phenylpyrrolidin-3-amin;
(3R,4S)-N-(3-Ethoxypropyl)-1-[(1-methyl-1H-imidazol-4-yl)sulfonyl]-4-phenylpyrrolidin-3-amin;
(3R,4S)-N-{[1-(Methoxymethyl)cyclopropyl]methyl}-1-[(1-methyl-1H-imidazol-4-yl)sulfonyl]-4-phenylpyrrolidin-3-amin;
N-({trans-4-(4-Fluorphenyl)-1-[(1-methyl-1H-imidazol-4-yl)sulfonyl]pyrrolidin-3-yl}methyl)-3-(propan-2-yloxy)propan-1-amin;
3-Ethoxy-N-({trans-4-(4-fluorphenyl)-1-[(1-methyl-1H-imidazol-4-yl)sulfonyl]pyrrolidin-3-yl}methyl)propan-1-amin;
N-({trans-4-(4-Fluorphenyl)-1-[(1-methyl-1H-imidazol-4-yl)sulfonyl]pyrrolidin-3-yl}methyl)-2-methoxyethanamin;
N-({trans-4-(4-Fluorphenyl)-1-[(1-methyl-1H-imidazol-4-yl)sulfonyl]pyrrolidin-3-yl}methyl)propan-1-amin;
N-({trans-4-(4-Fluorphenyl)-1-[(1-methyl-1H-imidazol-4-yl)sulfonyl]pyrrolidin-3-yl}methyl)butan-1-amin;
(2S)-N-({trans-4-(4-Fluorphenyl)-1-[(1-methyl-1H-imidazol-4-yl)sulfonyl]pyrrolidin-3-yl}methyl)-1-methoxypropan-2-amin;
N-({trans-4-(4-Fluorphenyl)-1-[(1-methyl-1H-imidazol-4-yl)sulfonyl]pyrrolidin-3-yl}methyl)-3-methoxypropan-1-amin;
2-Ethoxy-N-({trans-4-(4-fluorphenyl)-1-[(1-methyl-1H-imidazol-4-yl)sulfonyl]pyrrolidin-3-yl}methyl)ethanamin;
N-({trans-4-(4-Fluorphenyl)-1-[(1-methyl-1H-imidazol-4-yl)sulfonyl]pyrrolidin-3-yl}methyl)-2-(propan-2-yloxy)ethanamin;
(3R,4S)-N-(2-Methoxyethyl)-1-[(1-methyl-1H-imidazol-4-yl)sulfonyl]-4-phenylpyrrolidin-3-amin;
1-[(1-Methyl-1H-imidazol-4-yl)sulfonyl]-4-(tetrahydrofuran-2-yl)-N-[3-(trifluormethoxy)phenyl]pyrrolidin-3-amin;
1-[(1-Methyl-1H-imidazol-4-yl)sulfonyl]-4-(tetrahydrofuran-2-yl)-N-[3-(trifluormethoxy)phenyl]pyrrolidin-3-amin;
1-[(1-Methyl-1H-imidazol-4-yl)sulfonyl]-4-(tetrahydrofuran-2-yl)-N-[3-(trifluormethyl)phenyl]pyrrolidin-3-amin;
(3R,4S)-1-[(1-Methyl-1H-imidazol-4-yl)sulfonyl]-4-(tetrahydrofuran-2-yl)-N-[3-(trifluormethyl)phenyl]pyrrolidin-3-amin;
2-Methoxy-N-{(3R,4S)-1-[(1-methyl-1H-imidazol-4-yl)sulfonyl]-4-phenylpyrrolidin-3-yl}-6-(trifluormethyl)pyrimidin-4-amin;
6-Chlor-N-{(3R,4S)-1-[(1-methyl-1H-imidazol-4-yl)sulfonyl]-4-phenylpyrrolidin-3-yl}pyrimidin-4-amin;
1-Cyclopropyl-N-({(3R,4R)-4-(4-fluorphenyl)-1-[(1-methyl-1H-imidazol-4-yl)sulfonyl]pyrrolidin-3-yl}methyl)methanamin;
(3R,4S)-N-(2-Ethoxyethyl)-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-4-phenyl-N-(2-propoxyethyl)pyrrolidin-3-amin;
N-(3,4-Difluorphenyl)-1-[(1-methyl-1H-imidazol-4-yl)sulfonyl]-4-[-tetrahydrofuran-2-yl]pyrrolidin-3-amin;
N-(3,4-Difluorphenyl)-1-[(1-methyl-1H-imidazol-4-yl)sulfonyl]-4-(tetrahydrofuran-2-yl)pyrrolidin-3-amin;
6-Cyclopropyl-N-{(3R,4S)-1-[(1-methyl-1H-imidazol-4-yl)sulfonyl]-4-phenylpyrrolidin-3-yl}pyrimidin-4-amin;
6-Ethyl-N-{(3R,4S)-4-(4-fluorphenyl)-1-[(1-methyl-1H-imidazol-4-yl)sulfonyl]pyrrolidin-3-yl}pyrimidin-4-amin;
N-{(3R,4S)-4-(4-Fluorphenyl)-1-[(1-methyl-1H-imidazol-4-yl)sulfonyl]pyrrolidin-3-yl}-6-(propan-2-yl)pyrimidin-4-amin;
N-{(3R,4S)-4-(4-Fluorphenyl)-1-[(1-methyl-1H-imidazol-4-yl)sulfonyl]pyrrolidin-3-yl}-2-methyl-6-(trifluormethyl)pyrimidin-4-amin;
N-{(3R,4S)-4-(4-Fluorphenyl)-1-[(1-methyl-1H-imidazol-4-yl)sulfonyl]pyrrolidin-3-yl}-6-propylpyrimidin-4-amin;
5-Chlor-N-{(3R,4S)-4-(4-fluorphenyl)-1-[(1-methyl-1H-imidazol-4-yl)sulfonyl]pyrrolidin-3-yl}-4-(trifluormethyl)pyridin-2-amin;
(3S,4R)-N-[4-Chlor-3-(trifluormethyl)phenyl]-1-[(1-methyl-1H-imidazo1-4-yl)sulfonyl]-4-(tetrahydrofuran-2-yl)pyrrolidin-3-amin;
(3R,4S)-N-[4-Chlor-3-(trifluormethyl)phenyl]-1-[(1-methyl-1H-imidazol-4-yl)sulfonyl]-4-(tetrahydrofuran-2-yl)pyrrolidin-3-amin;
6-Ethoxy-N-{(3R,4S)-1-[(1-methyl-1H-imidazol-4-yl)sulfonyl]-4-phenylpyrrolidin-3-yl}pyrimidin-4-amin;
6-Methoxy-N-{(3R,4S)-1-[(1-methyl-1H-imidazol-4-yl)sulfonyl]-4-phenylpyrrolidin-3-yl}pyrimidin-4-amin;
N-{(3R,4S)-1-[(1-Methyl-1H-imidazol-4-yl)sulfonyl] -4-phenylpyrrolidin-3-yl}-6-propoxypyrimidin-4-amin;
6-Butoxy-N-{(3R,4S)-1-[(1-methyl-1H-imidazol-4-yl)sulfonyl] -4-phenylpyrrolidin-3-yl}pyrimidin-4-amin;
N-{(3R,4S)-1-[(1-Methyl-1H-imidazol-4-yl)sulfonyl] -4-phenylpyrrolidin-3-yl} -6-(2-methylpropoxy)pyrimidin-4-amin;
4-{[(3S,4R)-3-(4-Fluorphenyl)-4-(hexyloxy)pyrrolidin-1-yl]sulfonyl}-1-methyl-1H-imidazol;
trans-4-(4-Fluorphenyl)-1-[(1-methyl-1H-imidazol-4-yl)sulfonyl]-N-pentylpyrrolidin-3-carboxamid;
trans-4-(4-Fluorphenyl)-N-hexyl-1-[(1-methyl-1H-imidazol-4-yl)sulfonyl]pyrrolidin-3-carboxamid;
trans-N-(3-Chlorbenzyl)-4-(4-fluorphenyl)-1-[(1-methyl-1H-imidazol-4-yl)sulfonyl]pyrrolidin-3-carboxamid;
trans-N-(4-Chlorbenzyl)-4-(4-fluorphenyl)-1-[(1-methyl-1H-imidazol-4-yl)sulfonyl]pyrrolidin-3-carboxamid;
trans-N-(4-Fluorbenzyl)-4-(4-fluorphenyl)-1-[(1-methyl-1H-imidazol-4-yl)sulfonyl]pyrrolidin-3-carboxamid;
trans-N-(3,4-Difluorbenzyl)-4-(4-fluorphenyl)-1-[(1-methyl-1H-imidazol-4-yl)sulfonyl]pyrrolidin-3-carboxamid;
trans-4-(4-Fluorphenyl)-1-[(1-methyl-1H-imidazol-4-yl)sulfonyl]-N-[3-(trifluormethyl)benzyl]pyrrolidin-3-carboxamid;
trans-N-[2-Chlor-5-(trifluormethyl)benzyl]-4-(4-fluorphenyl)-1-[(1-methyl-1H-imidazol-4-yl)sulfonyl]pyrrolidin-3-carboxamid;
trans-4-(4-Fluorphenyl)-1-[(1-methyl-1H-imidazol-4-yl)sulfonyl]-N-(pyridin-2-ylmethyl)pyrrolidin-3-carboxamid;
trans-4-(4-Fluorphenyl)-1-[(1-methyl-1H-imidazol-4-yl)sulfonyl]-N-{[4-(trifluormethyl)pyridin-2-yl]methyl}pyrrolidin-3-carboxamid;
trans-N,N-Dibutyl-4-(4-fluorphenyl)-1-[(1-methyl-1H-imidazol-4-yl)sulfonyl]pyrrolidin-3-carboxamid;
6-Methyl-N-{(3R,4S)-1-[(1-methyl-1H-imidazol-4-yl)sulfonyl]-4-phenylpyrrolidin-3-yl}pyrimidin-4-amin;
(3R,4S)-4-(4-Fluorphenyl)-1-[(1-methyl-1H-imidazol-4-yl)sulfonyl]-N-[4-methyl-3-(trifluormethyl)phenyl]pyrrolidin-3-amin;
4-({(3R,4S)-4-(4-Fluorphenyl)-1-[(1-methyl-1H-imidazol-4-yl)sulfonyl]pyrrolidin-3-yl}amino)-2-(trifluormethyl)benzonitril;
4-{trans-4-(4-Fluorphenyl)-1-[(1-methyl-1H-imidazol-4-yl)sulfonyl]pyrrolidin-3-yl}-1-propyl-1H-1,2,3-triazol;
4-[-1-Cyclopropylpiperidin-3-yl]-1-[(1-methyl-1H-imidazol-4-yl)sulfonyl]-N-[3-(trifluormethoxy)phenyl]pyrrolidin-3-amin;
trans-N-(3-Chlor-4-fluorbenzyl)-4-(4-fluorphenyl)-1-[(1-methyl-1H-imidazol-4-yl)sulfonyl]pyrrolidin-3-carboxamid;
trans-4-(4-Fluorphenyl)-N-(6-methylheptyl)-1-[(1-methyl-1H-imidazol-4-yl)sulfonyl]pyrrolidin-3-carboxamid;
2-(4-Fluorphenoxy)-N-{(3R,4S)-4-(4-fluorphenyl)-1-[(1-methyl-1H-imidazol-4-yl)sulfonyl]pyrrolidin-3-yl}acetamid;
(3R,4S)-N-[4-Fluor-3-(trifluormethyl)phenyl]-1-[(1-methyl-1H-imidazol-4-yl)sulfonyl]-4-phenylpyrrolidin-3-amin;
(3R,4S)-N-[4-Fluor-3-(trifluormethoxy)phenyl]-1-[(1-methyl-1H-imidazol-4-yl)sulfonyl]-4-phenylpyrrolidin-3-amin;
(3R,4S)-N-(4-Methoxybutyl)-1-[(1-methyl-1H-imidazol-4-yl)sulfonyl]-4-phenylpyrrolidin-3-amin;
(3R,4S)-N-(4-Methoxyhexyl)-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-4-phenyl-N-[(2-propylcyclopropyl)methyl]pyrrolidin-3-amin;
(3R,4S)-1-[(1-Methyl-1H-imidazol-4-yl)sulfonyl]-4-phenyl-N-[(2-propylcyclopropyl)methyl]pyrrolidin-3-amin;
(3R,4S)-4-(4-Fluorphenyl)-N-[4-fluor-3-(trifluormethoxy)phenyl]-1-[(1-methyl-1H-imidazol-4-yl)sulfonyl]pyrrolidin-3-amin;
(3R,4S)-4-(4-Fluorphenyl)-N-[4-fluor-3-(trifluormethyl)phenyl]-1-[(1-methyl-1H-imidazol-4-yl)sulfonyl]pyrrolidin-3-amin;
(3R,4S)-N-[4-Methoxy-3-(trifluormethyl)phenyl]-1-[(1-methyl-1H-imidazol-4-yl)sulfonyl]-4-phenylpyrrolidin-3-amin;
(3R,4S)-N-(1-Benzothiophen-5-yl)-1-[(1-methyl-1H-imidazol-4-yl)sulfonyl]-4-phenylpyrrolidin-3-amin;
(3R,4S)-N-(2,3-Dihydro-1H-inden-5-yl)-1-[(1-methyl-1H-imidazol-4-yl)sulfonyl]-4-phenylpyrrolidin-3-amin;
(3R,4S)-N-[3,4-bis(Trifluormethyl)phenyl]-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-4-phenyl-N-(5,6,7,8-tetrahydronaphthalen-2-yl)pyrrolidin-3-amin;
(3R,4S)-N-(3,4-Dihydro-2H-chromen-6-yl)-1-[(1-methyl-1H-imidazol-4-yl)sulfonyl]-4-phenylpyrrolidin-3-amin;
4-({trans-1-[(1-Methyl-1H-imidazol-4-yl)sulfonyl]-4-phenylpyrrolidin-3-yl}methyl)-1-propyl-1H-1,2,3-triazol;
(3R,4S)-N-(1-Benzofuran-5-yl)-1-[(1-methyl-1H-imidazol-4-yl)sulfonyl]-4-phenylpyrrolidin-3-amin;
trans-4-(4-Fluorphenyl)-1-[(1-methyl-1H-imidazol-4-yl)sulfonyl]-N-[3-(trifluormethoxy)benzyl]pyrrolidin-3-carboxamid;
Methyl-5-({(3R,4S)-4-(4-fluorphenyl)-1-[(1-methyl-1H-imidazol-4-yl)sulfonyl]pyrrolidin-3-yl}amino)-5-oxopentanoat;
(3R,4S)-N-[2-Chlor-4-(trifluormethyl)benzyl]-4-(4-fluorphenyl)-1-[(1-methyl-1H-imidazol-4-yl)sulfonyl]pyrrolidin-3-carboxamid;
(3R,4S)-4-(4-Fluorphenyl)-1-[(1-methyl-1H-imidazol-4-yl)sulfonyl]-N-pentylpyrrolidin-3-amin;
(3R,4S)-4-(4-Fluorphenyl)-N-hexyl-1-[(1-methyl-1H-imidazol-4-yl)sulfonyl]pyrrolidin-3-amin;
(3R,4S)-N-[2-(2-Methoxyethoxy)ethyl]-1-[(1-methyl-1H-imidazol-4-yl)sulfonyl]-4-phenylpyrrolidin-3-amin;
(3R,4S)-N-[2-(4-Fluorphenoxy)ethyl]-4-(4-fluorphenyl)-1-[(1-methyl-1H-imidazol-4-yl)sulfonyl]pyrrolidin-3-amin;
4-Butyl-1-{trans-1-[(1-methyl-1H-imidazol-4-yl)sulfonyl]-4-phenylpyrrolidin-3-yl}-1H-1,2,3-triazol;
(3R,4S)-N-(4-Chlor-3-methylphenyl)-1-[(1-methyl-1H-imidazol-4-yl)sulfonyl]-4-phenylpyrrolidin-3-amin;
(3R,4S)-1-[(1-Methyl-1H-imidazol-4-yl)sulfonyl]-4-phenyl-N-[3-(2,2,2-trifluorethoxy)phenyl]pyrrolidin-3-amin;
(3R,4S)-N-(2,3-Dihydro-1-benzofuran-5-yl)-1-[(1-methyl-1H-imidazol-4-yl)sulfonyl]-4-phenylpyrrolidin-3-amin;
(3R,4S)-N-(4,4-Dimethyl-3,4-dihydro-2H-chromen-6-yl)-4-(4-fluorphenyl)-1-[(1-methyl-1H-imidazol-4-yl)sulfonyl]pyrrolidin-3-amin;
N-{(3R,4S)-4-(4-Fluorphenyl)-1-[(1-methyl-1H-imidazol-4-yl)sulfonyl]pyrrolidin-3-yl}-2-hydroxyhexanamid;
(3R,4S)-N-(3-Methoxyhexyl)-1-[(1-methyl-1H-imidazol-4-yl)sulfonyl]-4-phenylpyrrolidin-3-amin;
(3R,4S)-N-[4-Chlor-3-(propan-2-yl)phenyl]-4-(4-fluorphenyl)-1-[(1-methyl-1H-imidazol-4-yl)sulfonyl]pyrrolidin-3-amin;
(3R,4S)-4-(4-Fluorphenyl)-1-[(1-methyl-1H-imidazol-4-yl)sulfonyl]-N-[3-methyl-4-(trifluormethyl)phenyl]pyrrolidin-3-amin;
(3R,4S)-N-(1-Benzothiophen-6-yl)-4-(4-fluorphenyl)-1-[(1-methyl-1H-imidazol-4-yl)sulfonyl]pyrrolidin-3-amin;
-4-(1-Cyclopropylpiperidin-4-yl)-1-[(1-methyl-1H-imidazol-4-yl)sulfonyl]-N-[3-(trifluormethoxy)phenyl]pyrrolidin-3-amin;
trans-N-Hexyl-1-[(1-methyl-1H-imidazol-4-yl)sulfonyl]-4-(tetrahydro-2H-pyran-2-yl)pyrrolidin-3-amin;
trans-N-[2-Chlor-4-(trifluormethyl)benzyl]-1-[(1-methyl-1H-imidazol-4-yl)sulfonyl]-4-(tetrahydro-2H-pyran-2-yl)pyrrolidin-3-amin;
trans-1-[(1-Methyl-1H-imidazol-4-yl)sulfonyl]-4-(tetrahydro-2H-pyran-2-yl)-N-[3-(trifluormethyl)phenyl]pyrrolidin-3-amin;
N-{trans-1-[(1-Methyl-1H-imidazol-4-yl)sulfonyl]-4-[tetrahydro-2H-pyran-2-yl]pyrrolidin-3-yl}-4-(trifluormethyl)pyridin-2-amin;
N-{trans-1-[(1-Methyl-1H-imidazol-4-yl)sulfonyl]-4-[tetrahydro-2H-pyran-2-yl]pyrrolidin-3-yl}-4-(trifluormethyl)pyridin-2-amin;
(3R,4S)-N-[4-(4,4-Dimethyl-1,3-dioxan-2-yl)butyl]-4-(4-fluorphenyl)-1-[(1-methyl-1H-imidazol-4-yl)sulfonyl]pyrrolidin-3-amin;
trans-1-[(1-Methyl-1H-imidazol-4-yl)sulfonyl]-4-(tetrahydro-2H-pyran-2-yl)-N-[3-(trifluormethoxy)phenyl]pyrrolidin-3-amin;
trans-1-[(1-Methyl-1H-imidazol-4-yl)sulfonyl]-4-[tetrahydro-2H-pyran-2-yl]-N-[3-(trifluormethoxy)phenyl]pyrrolidin-3-amin;
N-[trans-1-[(1-Methyl-1H-imidazol-4-yl)sulfonyl]-4-(tetrahydro-2H-pyran-2-yl)pyrrolidin-3-yl] -6-(trifluormethyl)pyrimidin-4-amin;
N-[2-({trans-4-(4-Fluorphenyl)-1-[(1-methyl-1H-imidazol-4-yl)sulfonyl]pyrrolidin-3-yl}oxy)ethyl]propan-1-amin;
2-({trans-4-(4-Fluorphenyl)-1-[(1-methyl-1H-imidazol-4-yl)sulfonyl]pyrrolidin-3-yl}oxy)-N-propylacetamid;
6-Chlor-N-{(3R,4S)-4-(4-fluorphenyl)-1-[(1-methyl-1H-imidazol-4-yl)sulfonyl]pyrrolidin-3-yl}-2-methylpyrimidin-4-amin;
6-Chlor-N-{(3R,4S)-4-(4-fluorphenyl)-1-[(1-methyl-1H-imidazol-4-yl)sulfonyl]pyrrolidin-3-yl}pyrimidin-4-amin;
1-{(3R,4S)-4-(4-Fluorphenyl)-1-[(1-methyl-1H-imidazol-4-yl)sulfonyl]pyrrolidin-3-yl}-3-phenylharnstoff;
Phenyl-{(3R,4S)-4-(4-fluorphenyl)-1-[(1-methyl-1H-imidazol-4-yl)sulfonyl]pyrrolidin-3-yl}carbamat;
1-(2-Chlorphenyl)-3-1(3R,4S)-4-(4-fluorphenyl)-1-[(1-methyl-1H-imidazol-4-yl)sulfonyl]pyrrolidin-3-yl}harnstoff;
N-{(3R,4S)-4-(4-Fluorphenyl)-1-[(1-methyl-1H-imidazol-4-yl)sulfonyl]pyrrolidin-3-yl}-5-oxohexanamid;
N-{(3R,4S)-4-(4-Fluorphenyl)-1-[(1-methyl-1H-imidazol-4-yl)sulfonyl]pyrrolidin-3-yl}-4-(trifluormethyl)cyclohexanecarboxamid;
Butyl-trans-4-(4-fluorphenyl)-1-[(1-methyl-1H-imidazol-4-yl)sulfonyl]pyrrolidin-3-yl-carbonat;
5,5,5-Trifluor-N-{(3R,4S)-4-(4-fluorphenyl)-1-[(1-methyl-1H-imidazol-4-yl)sulfonyl]pyrrolidin-3-yl}pentanamid;
(3S,4R)-4-(4-Fluorphenyl)-1-[(1-methyl-1H-imidazol-4-yl)sulfonyl]pyrrolidin-3-yl butylcarbamat;
2,2-Difluor-N-{(3R,4S)-4-(4-fluorphenyl)-1-[(1-methyl-1H-imidazol-4-yl)sulfonyl]pyrrolidin-3-yl}pentanamid;
1-(4-Chlorphenyl)-3-{(3R,4S)-4-(4-fluorphenyl)-1-[(1-methyl-1H-imidazol-4-yl)sulfonyl]pyrrolidin-3-yl}harnstoff;
1-(2-Fluorphenyl)-3-{(3R,4S)-4-(4-fluorphenyl)-1-[(1-methyl-1H-imidazol-4-yl)sulfonyl]pyrrolidin-3-yl}harnstoff;
1-(3-Fluorphenyl)-3-{(3R,4S)-4-(4-fluorphenyl)-1-[(1-methyl-1H-imidazol-4-yl)sulfonyl]pyrrolidin-3-yl}harnstoff;
1-(4-Fluorphenyl)-3-{(3R,4S)-4-(4-fluorphenyl)-1-[(1-methyl-1H-imidazol-4-yl)sulfonyl]pyrrolidin-3-yl}harnstoff;
1-(2-Chlorbenzyl)-3-{(3R,4S)-4-(4-fluorphenyl)-1-[(1-methyl-1H-imidazol-4-yl)sulfonyl]pyrrolidin-3-yl}harnstoff;
4,5-Dichlor-N-{(3R,4S)-4-(4-fluorphenyl)-1-[(1-methyl-1H-imidazol-4-yl)sulfonyl]pyrrolidin-3-yl}pyridin-2-amin;
(3R,4S)-4-(4-Fluorphenyl)-1-[(1-methyl-1H-imidazol-4-yl)sulfonyl]-N-{[4-(trifluormethyl)cyclohexyl]methyl}pyrrolidin-3-amin;
2,2-Difluor-N-{(3R,4S)-4-(4-fluorphenyl)-1-[(1-methyl-1H-imidazol-4-yl)sulfonyl]pyrrolidin-3-yl}hexanamid;
trans-N-[3,4-Difluorphenyl]-1-[(1-methyl-1H-imidazol-4-yl)sulfonyl]-4-(1,3-oxazol-2-yl)pyrrolidin-3-amin;
N-[4-Chlor-3-(trifluormethyl)phenyl]-1-[(1-methyl-1H-imidazol-4-yl)sulfonyl]-4-(1,3-oxazol-2-yl)pyrrolidin-3-amin;
4-{[trans-3-[(4-Fluorbenzyl)oxy]-4-(4-fluorphenyl)pyrrolidin-1-yl]sulfonyl}-1-methyl-1H-imidazol;
4-{[trans-3-[(3-Fluorbenzyl)oxy]-4-(4-fluorphenyl)pyrrolidin-1-yl]sulfonyl}-1-methyl-1H-imidazol;
4-{[trans-3-(4-Fluorphenyl)-4-{[4-(methylsulfonyl)benzyl]oxy}pyrrolidin-1-yl]sulfonyl}-1-methyl-1H-imidazol;
4-{[trans-3-(4-Fluorphenyl)-4-{[3-(methylsulfonyl)benzyl]oxy}pyrrolidin-1-yl]sulfonyl}-1-methyl-1H-imidazol;
6-Ethoxy-N-{(3R,4S)-4-(4-fluorphenyl)-1-[(1-methyl-1H-imidazol-4-yl)sulfonyl]pyrrolidin-3-yl}pyrimidin-4-amin;
N-{(3R,4S)-4-(4-Fluorphenyl)-1-[(1-methyl-1H-imidazol-4-yl)sulfonyl]pyrrolidin-3-yl}-6-(propan-2-yloxy)pyrimidin-4-amin;
6-Butoxy-N-{(3R,4S)-4-(4-fluorphenyl)-1-[(1-methyl-1H-imidazol-4-yl)sulfonyl]pyrrolidin-3-yl}pyrimidin-4-amin;
N-{(3R,4S)-4-(4-Fluorphenyl)-1-[(1-methyl-1H-imidazol-4-yl)sulfonyl]pyrrolidin-3-yl} -6-(2-methylpropoxy)pyrimidin-4-amin;
6-(Cyclobutyloxy)-N-{(3R,4S)-4-(4-fluorphenyl)-1-[(1-methyl-1H-imidazol-4-yl)sulfonyl]pyrrolidin-3-yl}pyrimidin-4-amin;
6-(Cyclopentyloxy)-N-{(3R,4S)-4-(4-fluorphenyl)-1-[(1-methyl-1H-imidazol-4-yl)sulfonyl]pyrrolidin-3-yl}pyrimidin-4-amin;
6-(Cyclohexyloxy)-N-{(3R,4S)-4-(4-fluorphenyl)-1-[(1-methyl-1H-imidazol-4-yl)sulfonyl]pyrrolidin-3-yl}pyrimidin-4-amin;
N-{(3R,4S)-4-(4-Fluorphenyl)-1-[(1-methyl-1H-imidazol-4-yl)sulfonyl]pyrrolidin-3-yl}-6-phenylpyrimidin-4-amin;
N-{(3R,4S)-4-(4-Fluorphenyl)-1-[(1-methyl-1H-imidazol-4-yl)sulfonyl]pyrrolidin-3-yl}-6-(2-methylphenyl)pyrimidin-4-amin;
N-{(3R,4S)-4-(4-Fluorphenyl)-1-[(1-methyl-1H-imidazol-4-yl)sulfonyl]pyrrolidin-3-yl}-6-(3-methylphenyl)pyrimidin-4-amin;
N-{(3R,4S)-4-(4-Fluorphenyl)-1-[(1-methyl-1H-imidazol-4-yl)sulfonyl]pyrrolidin-3-yl}-6-(4-methylphenyl)pyrimidin-4-amin;
6-(2-Fluorphenyl)-N-{(3R,4S)-4-(4-fluorphenyl)-1-[(1-methyl-1H-imidazol-4-yl)sulfonyl]pyrrolidin-3-yl}pyrimidin-4-amin;
6-(3-Fluorphenyl)-N-{(3R,4S)-4-(4-fluorphenyl)-1-[(1-methyl-1H-imidazol-4-yl)sulfonyl]pyrrolidin-3-yl}pyrimidin-4-amin;
6-(4-Fluorphenyl)-N-{(3R,4S)-4-(4-fluorphenyl)-1-[(1-methyl-1H-imidazol-4-yl)sulfonyl]pyrrolidin-3-yl}pyrimidin-4-amin;
6-(3-Chlorphenyl)-N-{(3R,4S)-4-(4-fluorphenyl)-1-[(1-methyl-1H-imidazol-4-yl)sulfonyl]pyrrolidin-3-yl}pyrimidin-4-amin;
6-(4-Chlorphenyl)-N-{(3R,4S)-4-(4-fluorphenyl)-1-[(1-methyl-1H-imidazol-4-yl)sulfonyl]pyrrolidin-3-yl}pyrimidin-4-amin;
4-({(3S,4R)-3-(4-Fluorphenyl)-4-[(4-methylbenzyl)oxy]pyrrolidin-1-yl}sulfonyl)-1-methyl-1H-imidazol;
(3R,4S)-4-(4-Fluorphenyl)-1-[(1-methyl-1H-imidazol-4-yl)sulfonyl]-N-(5,5,5-trifluorpentyl)pyrrolidin-3-amin;
(3R,4S)-N-(2,2-Difluorhexyl)-4-(4-fluorphenyl)-1-[(1-methyl-1H-imidazol-4-yl)sulfonyl]pyrrolidin-3-amin;
(3R,4S)-N-(2,2-Difluorpentyl)-4-(4-fluorphenyl)-1-[(1-methyl-1H-imidazol-4-yl)sulfonyl]pyrrolidin-3-amin;
5,5-Difluor-N-{(3R,4S)-4-(4-fluorphenyl)-1-[(1-methyl-1H-imidazol-4-yl)sulfonyl]pyrrolidin-3-yl}hexanamid;
4-Fluor-N-{(3R,4S)-4-(4-fluorphenyl)-1-[(1-methyl-1H-imidazol-4-yl)sulfonyl]pyrrolidin-3-yl}pyridin-2-amin;
5-Fluor-N-{(3R,4S)-4-(4-fluorphenyl)-1-[(1-methyl-1H-imidazol-4-yl)sulfonyl]pyrrolidin-3-yl}pyridin-2-amin;
N-{(3R,4S)-4-(4-Fluorphenyl)-1-[(1-methyl-1H-imidazol-4-yl)sulfonyl]pyrrolidin-3-yl}-4-oxopentanamid;
(3R,4S)-N-(5,5-Difluorhexyl)-4-(4-fluorphenyl)-1-[(1-methyl-1H-imidazol-4-yl)sulfonyl]pyrrolidin-3-amin;
4-Chlor-N-[2-({trans-4-(4-fluorphenyl)-1-[(1-methyl-1H-imidazol-4-yl)sulfonyl]pyrrolidin-3-yl}oxy)ethyl] anilin;
(3S,4R)-N-[4-Fluor-3-(trifluormethoxy)phenyl]-1-[(1-methyl-1H-imidazol-4-yl)sulfonyl]-4-(tetrahydro-2H-pyran-2-yl)pyrrolidin-3-amin;
1-Benzyl-3-{(3R,4S)-4-(4-fluorphenyl)-1-[(1-methyl-1H-imidazol-4-yl)sulfonyl]pyrrolidin-3-yl}harnstoff;
(3R,4S)-N-(4-Chlor-3-methylphenyl)-4-(4-fluorphenyl)-1-[(1-methyl-1H-imidazol-4-yl)sulfonyl]pyrrolidin-3-amin;
(3R,4S)-4-(4-Fluorphenyl)-1-[(1-methyl-1H-imidazol-4-yl)sulfonyl]-N-[3-(2,2,2-trifluorethoxy)phenyl]pyrrolidin-3-amin;
(3R,4S)-4-(4-Fluorphenyl)-N-[4-fluor-3-(pyridin-3-yl)phenyl]-1-[(1-methyl-1H-imidazol-4-yl)sulfonyl]pyrrolidin-3-amin;
(3R,4S)-4-(4-Fluorphenyl)-N-[4-fluor-3-(pyridin-4-yl)phenyl]-1-[(1-methyl-1H-imidazol-4-yl)sulfonyl]pyrrolidin-3-amin;
(3R,4S)-N-(4-Fluor-3-methylphenyl)-4-(4-fluorphenyl)-1-[(1-methyl-1H-imidazol-4-yl)sulfonyl]pyrrolidin-3-amin;
5-Fluor-N-{(3R,4S)-4-(4-fluorphenyl)-1-[(1-methyl-1H-imidazol-4-yl)sulfonyl]pyrrolidin-3-yl}-4-methylpyridin-2-amin;
(3R,4S)-4-(4-Fluorphenyl)-N-[4-fluor-3-(pyrimidin-5-yl)phenyl]-1-[(1-methyl-1H-imidazol-4-yl)sulfonyl]pyrrolidin-3-amin;
N-{(3R,4S)-4-(4-Fluorphenyl)-1-[(1-methyl-1H-imidazol-4-yl)sulfonyl]pyrrolidin-3-yl} -3,4'-bipyridin-2'-amin;
N-{(3R,4S)-4-(4-Fluorphenyl)-1-[(1-methyl-1H-imidazol-4-yl)sulfonyl]pyrrolidin-3-yl} -4,4'-bipyridin-2-amin;
4-({(3S,4R)-4-(4-Fluorphenyl)-1-[(1-methyl-1H-imidazol-4-yl)sulfonyl]pyrrolidin-3-yl}oxy)-6-(trifluormethyl)pyrimidin;
chlor-3-(Trifluormethyl)phenyl]-1-[(1-methyl-1H-imidazol-4-yl)sulfonyl]-4-(1,3-oxazol-4-yl)pyrrolidin-3-amin;
4,4-Difluor-N-{(3R,4S)-4-(4-fluorphenyl)-1-[(1-methyl-1H-imidazol-4-yl)sulfonyl]pyrrolidin-3-yl}pentanamid;
(3R,4S)-N-(3,4-Difluorphenyl)-1-[(1,2-dimethyl-1H-imidazol-4-yl)sulfonyl] -4-(4-fluorphenyl)pyrrolidin-3-amin;
(3R,4S)-1-[(1,2-Dimethyl-1H-imidazol-4-yl)sulfonyl]-4-(4-fluorphenyl)-N-[4-fluor-3-(trifluormethyl)phenyl]pyrrolidin-3-amin;
(3R,4S)-N-(3,4-Difluorphenyl)-1-[(1,2-dimethyl-1H-imidazol-5-yl)sulfonyl] -4-(4-fluorphenyl)pyrrolidin-3-amin;
(3R,4S)-1-[(1,2-Dimethyl-1H-imidazol-5-yl)sulfonyl]-4-(4-fluorphenyl)-N-[4-fluor-3-(trifluormethyl)phenyl]pyrrolidin-3-amin;
4-Chlor-N-{(3R,4S)-4-(4-fluorphenyl)-1-[(1-methyl-1H-imidazol-4-yl)sulfonyl]pyrrolidin-3-yl}pyridin-2-amin;
5-Chlor-N-[(3R,4S)-1-[(1,2-dimethyl-1H-imidazol-4-yl)sulfonyl] -4-(4-fluorphenyl)pyrrolidin-3-yl]-4-(trifluormethyl)pyridin-2-amin;
trans-Chlor-4-(trifluormethyl)benzyl]-4-(3-fluorpyridin-2-yl)-1-[(1-methyl-1H-imidazol-4-yl)sulfonyl]pyrrolidin-3-amin;
trans-4-(3-Fluorpyridin-2-yl)-N-hexyl-1-[(1-methyl-1H-imidazol-4-yl)sulfonyl]pyrrolidin-3-amin;
trans-4-(3-Fluorpyridin-2-yl)-N,N-dihexyl-1-[(1-methyl-1H-imidazol-4-yl)sulfonyl]pyrrolidin-3-amin;
trans-4-(3-Fluorpyridin-2-yl)-1-[(1-methyl-1H-imidazol-4-yl)sulfonyl]-N,N-dipentylpyrrolidin-3-amin;
trans-4-(3-Fluorpyridin-2-yl)-1-[(1-methyl-1H-imidazol-4-yl)sulfonyl]-N-pentylpyrrolidin-3-amin;
trans-N-(3,4-Difluorphenyl)-4-(3-fluorpyridin-2-yl)-1-[(1-methyl-1H-imidazol-4-yl)sulfonyl]pyrrolidin-3-amin;
trans-4-(3-Fluorpyridin-2-yl)-N-[4-fluor-3-(trifluormethyl)phenyl]-1-[(1-methyl-1H-imidazol-4-yl)sulfonyl]pyrrolidin-3-amin;
trans-4-(3-Fluorpyridin-2-yl)-N-[4-fluor-3-(trifluormethoxy)phenyl]-1-[(1-methyl-1H-imidazol-4-yl)sulfonyl]pyrrolidin-3-amin;
5-Fluor-N-{trans-4-(3-fluorpyridin-2-yl)-1-[(1-methyl-1H-imidazol-4-yl)sulfonyl]pyrrolidin-3-yl}-4-methylpyridin-2-amin;
N-{trans-4-(3-Fluorpyridin-2-yl)-1-[(1-methyl-1H-imidazol-4-yl)sulfonyl]pyrrolidin-3-yl}-6-(trifluormethyl)pyrimidin-4-amin;
trans-N-(3-Chlor-4-fluorphenyl)-4-(3-fluorpyridin-2-yl)-1-[(1-methyl-1H-imidazol-4-yl)sulfonyl]pyrrolidin-3-amin;
6-Chlor-N-{trans-4-(3-fluorpyridin-2-yl)-1-[(1-methyl-1H-imidazol-4-yl)sulfonyl]pyrrolidin-3-yl}pyrimidin-4-amin;
(3R,4S)-N-[3-(Benzyloxy)phenyl]-1-[(1-methyl-1H-imidazol-4-yl)sulfonyl]-4-phenylpyrrolidin-3-amin;
6-(Benzyloxy)-N-{(3R,4S)-4-(4-fluorphenyl)-1-[(1-methyl-1H-imidazol-4-yl)sulfonyl]pyrrolidin-3-yl}pyrimidin-4-amin;
trans-N-(4-Fluor-3-methylphenyl)-4-(3-fluorpyridin-2-yl)-1-[(1-methyl-1H-imidazol-4-yl)sulfonyl]pyrrolidin-3-amin;
4-(Benzyloxy)-5-fluor-N-{(3R,4S)-1-[(1-methyl-1H-imidazol-4-yl)sulfonyl]-4-phenylpyrrolidin-3-yl}pyridin-2-amin;
(3R,4S)-1-[(1-Methyl-1H-imidazol-4-yl)sulfonyl]-4-phenyl-N-[3-(piperidin-4-yl)phenyl]pyrrolidin-3-amin;
6-(Azetidin-3-ylmethoxy)-N-{(3R,4S)-4-(4-fluorphenyl)-1-[(1-methyl-1H-imidazol-4-yl)sulfonyl]pyrrolidin-3-yl}pyrimidin-4-amin;
N-{(3R,4S)-4-(4-Fluorphenyl)-1-[(1-methyl-1H-imidazol-4-yl)sulfonyl]pyrrolidin-3-yl}-6-(pyrrolidin-3-ylmethoxy)pyrimidin-4-amin;
N-{(3R,4S)-4-(4-Fluorphenyl)-1-[(1-methyl-1H-imidazol-4-yl)sulfonyl]pyrrolidin-3-yl}-6-(pyrrolidin-2-ylmethoxy)pyrimidin-4-amin;
trans-1-[(1-Methyl-1H-imidazol-4-yl)sulfonyl]-N-(3-methylphenyl)-4-(tetrahydro-2H-pyran-3-yl)pyrrolidin-3-amin;
trans-1-[(1-Methyl-1H-imidazol-4-yl)sulfonyl]-4-(tetrahydro-2H-pyran-3-yl)-N-[3-(trifluormethyl)phenyl]pyrrolidin-3-amin;
trans-N-(3-Chlorphenyl)-1-[(1-methyl-1H-imidazol-4-yl)sulfonyl]-4-(tetrahydro-2H-pyran-3-yl)pyrrolidin-3-amin;
trans-1-[(1-Methyl-1H-imidazol-4-yl)sulfonyl]-4-[(3S)-tetrahydro-2H-pyran-3-yl]-N-[3-(trifluormethoxy)phenyl]pyrrolidin-3-amin;
trans-1-[(1-Methyl-1H-imidazol-4-yl)sulfonyl]-4-[(3S)-tetrahydro-2H-pyran-3-yl]-N-[3-(2,2,2-trifluorethoxy)phenyl]pyrrolidin-3-amin;
trans-1-[(1-Methyl-1H-imidazol-4-yl)sulfonyl]-N-(3-phenoxyphenyl)-4-[(3S)-tetrahydro-2H-pyran-3-yl]pyrrolidin-3-amin;
trans-N-(Biphenyl-3-yl)-1-[(1-methyl-1H-imidazol-4-yl)sulfonyl]-4-[(3S)-tetrahydro-2H-pyran-3-yl]pyrrolidin-3-amin;
trans-N-[4-Fluor-3-(trifluormethoxy)phenyl]-1-[(1-methyl-1H-imidazol-4-yl)sulfonyl]-4-[(3S)-tetrahydro-2H-pyran-3-yl]pyrrolidin-3-amin;
trans-N-(3-Chlor-4-fluorphenyl)-1-[(1-methyl-1H-imidazol-4-yl)sulfonyl]-4-[(3S)-tetrahydro-2H-pyran-3-yl]pyrrolidin-3-amin;
trans-N-(4-Fluor-3-methylphenyl)-1-[(1-methyl-1H-imidazol-4-yl)sulfonyl]-4-[(3S)-tetrahydro-2H-pyran-3-yl]pyrrolidin-3-amin;
trans-N-[4-Fluor-3-(trifluormethyl)phenyl]-1-[(1-methyl-1H-imidazol-4-yl)sulfonyl]-4-[(3S)-tetrahydro-2H-pyran-3-yl]pyrrolidin-3-amin;
trans-N-[4-Chlor-3-(trifluormethyl)phenyl]-1-[(1-methyl-1H-imidazol-4-yl)sulfonyl]-4-[(3S)-tetrahydro-2H-pyran-3-yl]pyrrolidin-3-amin;
trans-N-[4-Chlor-3-(trifluormethoxy)phenyl]-1-[(1-methyl-1H-imidazol-4-yl)sulfonyl]-4-[(3S)-tetrahydro-2H-pyran-3-yl]pyrrolidin-3-amin;
trans-N-[3-(Benzyloxy)phenyl]-1-[(1-methyl-1H-imidazol-4-yl)sulfonyl]-4-[(3S)-tetrahydro-2H-pyran-3-yl]pyrrolidin-3-amin;
5-Fluor-N-{(3R,4S)-4-(4-fluorphenyl)-1-[(1-methyl-1H-imidazol-4-yl)sulfonyl]pyrrolidin-3-yl}-4-(trifluormethyl)pyridin-2-amin;
3-Fluor-N-{(3R,4S)-4-(4-fluorphenyl)-1-[(1-methyl-1H-imidazol-4-yl)sulfonyl]pyrrolidin-3-yl}-4-(trifluormethyl)pyridin-2-amin;
N-(2,2-Difluorethyl)-2-({(3R,4S)-4-(4-fluorphenyl)-1-[(1-methyl-1H-imidazol-4-yl)sulfonyl]pyrrolidin-3-yl}oxy)acetamid;
trans-4-(3-Fluorpyridin-2-yl)-1-[(1-methyl-1H-imidazol-4-yl)sulfonyl]-N-[3-(trifluormethyl)phenyl]pyrrolidin-3-amin;
trans-4-(3-Fluorpyridin-2-yl)-1-[(1-methyl-1H-imidazol-4-yl)sulfonyl]-N-[3-(trifluormethoxy)phenyl]pyrrolidin-3-amin;
trans-4-(3-Fluorpyridin-2-yl)-1-[(1-methyl-1H-imidazol-4-yl)sulfonyl]-N-[3-(2,2,2-trifluorethoxy)phenyl]pyrrolidin-3-amin;
(3R,4S)-4-(4-Fluorphenyl)-1-[(1-methyl-1H-imidazol-4-yl)sulfonyl]-N-[3-(pyridin-2-yl)phenyl]pyrrolidin-3-amin;
(3R,4S)-1-[(1-Methyl-1H-imidazol-4-yl)sulfonyl]-4-phenyl-N-[3-(pyridin-2-yl)phenyl]pyrrolidin-3-amin;
5-Fluor-N-{(3R,4S)-1-[(1-methyl-1H-imidazol-4-yl)sulfonyl]-4-phenylpyrrolidin-3-yl}-4-(2,2,2-trifluorethoxy)pyridin-2-amin;
5-Chlor-N-{trans-4-(3-fluorpyridin-2-yl)-1-[(1-methyl-1H-imidazol-4-yl)sulfonyl]pyrrolidin-3-yl}-4-(trifluormethyl)pyridin-2-amin;
trans-N-(3-Fluorphenyl)-4-(3-fluorpyridin-2-yl)-1-[(1-methyl-1H-imidazol-4-yl)sulfonyl]pyrrolidin-3-amin;
5-Chlor-N-{(3R,4S)-4-(4-fluorphenyl)-1-[(1-methyl-1H-imidazol-4-yl)sulfonyl]pyrrolidin-3-yl}-4-(2,2,2-trifluorethoxy)pyridin-2-amin;
N-{(3R,4S)-4-(4-Fluorphenyl)-1-[(1-methyl-1H-imidazol-4-yl)sulfonyl]pyrrolidin-3-yl}-4-(2,2,2-trifluorethoxy)pyridin-2-amin;
5-Chlor-N-{(3R,4S)-1-[(1-methyl-1H-imidazol-4-yl)sulfonyl]-4-phenylpyrrolidin-3-yl}-4-(2,2,2-trifluorethoxy)pyridin-2-amin;
N-{(3R,4S)-1-[(1-Methyl-1H-imidazol-4-yl)sulfonyl]-4-phenylpyrrolidin-3-yl}-4-(2,2,2-trifluorethoxy)pyridin-2-amin;
5-Fluor-N-{(3R,4S)-4-(4-fluorphenyl)-1-[(1-methyl-1H-imidazol-4-yl)sulfonyl]pyrrolidin-3-yl}-4-(2,2,2-trifluorethoxy)pyridin-2-amin;
trans-4-(3-Fluorpyridin-2-yl)-N-hexyl-1-[(1-methyl-1H-1,2,3-triazol-4-yl)sulfonyl]pyrrolidin-3-amin;
N-{(3R,4S)-4-(4-Fluorphenyl)-1-[(1-methyl-1H-imidazol-4-yl)sulfonyl]pyrrolidin-3-yl}-4-(propan-2-yl)pyridin-2-amin;
N-{(3R,4S)-1-[(1-Methyl-1H-imidazol-4-yl)sulfonyl]-4-phenylpyrrolidin-3-yl}-4-(propan-2-yl)pyridin-2-amin;
2,2,2-Trifluor-N-[2-({trans-4-(4-fluorphenyl)-1-[(1-methyl-1H-imidazol-4-yl)sulfonyl]pyrrolidin-3-yl}oxy)ethyl]ethanamin;
trans-N-(3-Chlor-4-fluorphenyl)-1-[(1-methyl-1H-imidazol-4-yl)sulfonyl]-4-(tetrahydro-2H-pyran-2-yl)pyrrolidin-3-amin;
trans-1-[(1-Methyl-1H-imidazol-4-yl)sulfonyl]-4-(tetrahydro-2H-pyran-2-yl)-N-[3-(2,2,2-trifluorethoxy)phenyl]pyrrolidin-3-amin;
2-({1-[(1-Methyl-1H-imidazol-4-yl)sulfonyl]-4-phenylpyrrolidin-3-yl}methyl)-4-(trifluormethyl)pyridin;
trans-N-[2-Chlor-4-(trifluormethyl)benzyl]-4-(3-fluorpyridin-2-yl)-1-[(1-methyl-1H-1,2,3-triazol-4-yl)sulfonyl]pyrrolidin-3-amin;
trans-1-[(1-Methyl-1H-imidazol-4-yl)sulfonyl]-4-(tetrahydrofuran-2-yl)-N-[3-(2,2,2-trifluorethoxy)phenyl]pyrrolidin-3-amin;
N-Hexyl-1-[(1-methyl-1H-imidazol-4-yl)sulfonyl]-4-[(2R)-tetrahydrofuran-2-yl]pyrrolidin-3-amin;
N-Hexyl-1-[(1-methyl-1H-imidazol-4-yl)sulfonyl]-4-[(2S)-tetrahydrofuran-2-yl]pyrrolidin-3-amin;
trans-N-[2-Chlor-4-(trifluormethyl)benzyl]-1-[(1-methyl-1H-imidazol-4-yl)sulfonyl]-4-(tetrahydrofuran-2-yl)pyrrolidin-3-amin;
N-[2-Chlor-4-(trifluormethyl)benzyl]-1-[(1-methyl-1H-imidazol-4-yl)sulfonyl]-4-[(2S)-tetrahydrofuran-2-yl]pyrrolidin-3-amin;
trans-N-(3-Chlor-4-fluorphenyl)-1-[(1-methyl-1H-imidazol-4-yl)sulfonyl]-4-(tetrahydrofuran-2-yl)pyrrolidin-3-amin;
N-(3-Chlor-4-fluorphenyl)-1-[(1-methyl-1H-imidazol-4-yl)sulfonyl]-4-[(2S)-tetrahydrofuran-2-yl]pyrrolidin-3-amin;
1-Benzyl-N-{(3R,4S)-1-[(1-methyl-1H-imidazol-4-yl)sulfonyl]-4-phenylpyrrolidin-3-yl}-2,3-dihydro-1H-indol-6-amin;
trans-1-[(1-Methyl-1H-imidazol-4-yl)sulfonyl]-4-(tetrahydrofuran-3-yl)-N-[3-(trifluormethyl)phenyl]pyrrolidin-3-amin;
trans-1-[(1-Methyl-1H-imidazol-4-yl)sulfonyl]-N-(3-methylphenyl)-4-(tetrahydrofuran-3-yl)pyrrolidin-3-amin;
trans-N-(3-Chlorphenyl)-1-[(1-methyl-1H-imidazol-4-yl)sulfonyl]-4-(tetrahydrofuran-3-yl)pyrrolidin-3-amin;
trans-N-(3-Chlor-4-fluorphenyl)-1-[(1-methyl-1H-imidazol-4-yl)sulfonyl]-4-(tetrahydrofuran-3-yl)pyrrolidin-3-amin;
trans-N-[4-Fluor-3-(trifluormethoxy)phenyl]-1-[(1-methyl-1H-imidazol-4-yl)sulfonyl]-4-(tetrahydrofuran-3-yl)pyrrolidin-3-amin;
trans-N-[4-Fluor-3-(trifluormethyl)phenyl]-1-[(1-methyl-1H-imidazol-4-yl)sulfonyl]-4-(tetrahydrofuran-3-yl)pyrrolidin-3-amin;
trans-N-[4-Chlor-3-(trifluormethyl)phenyl]-1-[(1-methyl-1H-imidazol-4-yl)sulfonyl]-4-(tetrahydrofuran-3-yl)pyrrolidin-3-amin;
trans-1-[(1-Methyl-1H-imidazol-4-yl)sulfonyl]-4-(tetrahydrofuran-3-yl)-N-[3-(2,2,2-trifluorethoxy)phenyl]pyrrolidin-3-amin;
trans-1-[(1-Methyl-1H-imidazol-4-yl)sulfonyl]-4-(tetrahydrofuran-3-yl)-N-[3-(trifluormethoxy)phenyl]pyrrolidin-3-amin;
trans-4-(4-Fluorphenyl)-1-[(1-methyl-1H-1,2,3-triazol-4-yl)sulfonyl]-N-[3-(2,2,2-trifluorethoxy)phenyl]pyrrolidin-3-amin;
(3R,4S)4-(4-Fluorphenyl)-1-[(1-methyl-1H-1,2,3-triazol-4-yl)sulfonyl]-N-[3-(trifluormethoxy)phenyl]pyrrolidin-3-amin;
(3R,4S)-4-(4-Fluorphenyl)-N-[4-fluor-3-(trifluormethoxy)phenyl]-1-[(1-methyl-1H-1,2,3-triazol-4-yl)sulfonyl]pyrrolidin-3-amin;
(3R,4S)-4-(4-Fluorphenyl)-1-[(1-methyl-1H-1,2,3-triazol-4-yl)sulfonyl]-N-[3-(trifluormethyl)phenyl]pyrrolidin-3-amin;
(3R,4S)-4-(4-Fluorphenyl)-N-[4-fluor-3-(trifluormethyl)phenyl]-1-[(1-methyl-1H-1,2,3-triazol-4-yl)sulfonyl]pyrrolidin-3-amin;
(3R,4S)-N-(3-Chlorphenyl)-4-(4-fluorphenyl)-1-[(1-methyl-1H-1,2,3-triazol-4-yl)sulfonyl]pyrrolidin-3-amin;
(3R,4S)-N-(3-Chlor-4-fluorphenyl)-4-(4-fluorphenyl)-1-[(1-methyl-1H-1,2,3-triazol-4-yl)sulfonyl]pyrrolidin-3-amin;
trans-N-[4-Fluor-3-(trifluormethyl)phenyl]-1-[(1-methyl-1H-imidazol-4-yl)sulfonyl]-4-(tetrahydrofuran-2-yl)pyrrolidin-3-amin;
5-Chlor-N-[trans-1-[(1-methyl-1H-imidazol-4-yl)sulfonyl]-4-(tetrahydrofuran-2-yl)pyrrolidin-3-yl]-4-(trifluormethyl)pyridin-2-amin;
trans-N-(4-Fluor-3-methylphenyl)-1-[(1-methyl-1H-imidazol-4-yl)sulfonyl]-4-(tetrahydrofuran-2-yl)pyrrolidin-3-amin;
trans-N-[4-Fluor-3-(trifluormethyl)phenyl]-1-[(1-methyl-1H-imidazol-4-yl)sulfonyl]-4-(tetrahydro-2H-pyran-2-yl)pyrrolidin-3-amin;
trans-N-(4-Fluor-3-methylphenyl)-1-[(1-methyl-1H-imidazol-4-yl)sulfonyl]-4-(tetrahydro-2H-pyran-2-yl)pyrrolidin-3-amin;
5-Chlor-N-[trans-1-[(1-methyl-1H-imidazol-4-yl)sulfonyl]-4-(tetrahydro-2H-pyran-2-yl)pyrrolidin-3-yl]-4-(trifluormethyl)pyridin-2-amin;
trans-N-(3-Chlorphenyl)-1-[(1-methyl-1H-imidazol-4-yl)sulfonyl]-4-(tetrahydro-2H-pyran-2-yl)pyrrolidin-3-amin;
trans-N-(3-Chlorphenyl)-1-[(1-methyl-1H-imidazol-4-yl)sulfonyl]-4-(tetrahydrofuran-2-yl)pyrrolidin-3-amin;
5-Fluor-4-methyl-N-[trans-1-[(1-methyl-1H-imidazol-4-yl)sulfonyl]-4-(tetrahydrofuran-2-yl)pyrrolidin-3-yl]pyridin-2-amin;
5-Fluor-4-methyl-N-[trans-1-[(1-methyl-1H-imidazol-4-yl)sulfonyl]-4-(tetrahydro-2H-pyran-2-yl)pyrrolidin-3-yl]pyridin-2-amin;
N-[trans-1-[(1-Methyl-1H-imidazol-4-yl)sulfonyl]-4-(tetrahydrofuran-2-yl)pyrrolidin-3-yl]-6-(trifluormethyl)pyrimidin-4-amin;
(3R,4S)-N-Cyclohexyl-4-(4-fluorphenyl)-1-(1-methyl-1H-imidazol-4-ylsulfonyl)pyrrolidin-3-amin;
(3R,4S)-N-(3,3-Dimethylcyclohexyl)-4-(4-fluorphenyl)-1-(1-methyl-1H-imidazol-4-ylsulfonyl)pyrrolidin-3-amin;
(3R,4S)-N-(4,4-Dimethylcyclohexyl)-4-(4-fluorphenyl)-1-(1-methyl-1H-imidazol-4-ylsulfonyl)pyrrolidin-3-amin;
(3R,4S)-N-Cyclopentyl-4-(4-fluorphenyl)-1-(1-methyl-1H-imidazol-4-ylsulfonyl)pyrrolidin-3-amin;
(3R,4S)-N-(3,3-Dimethylcyclopentyl)-4-(4-fluorphenyl)-1-(1-methyl-1H-imidazol-4-ylsulfonyl)pyrrolidin-3-amin;
(3R,4S)-N-(1-(2,4-Dichlorphenyl)ethyl)-4-(4-fluorphenyl)-1-(1-methyl-1H-imidazol-4-ylsulfonyl)pyrrolidin-3-amin;
(3R,4S)-N-(2,3-Dihydro-1H-inden-1-yl)-4-(4-fluorphenyl)-1-(1-methyl-1H-imidazol-4-ylsulfonyl)pyrrolidin-3-amin;
(3R,4S)-N-(5-Chlor-2,3-dihydro-1H-inden-1-yl)-4-(4-fluorphenyl)-1-(1-methyl-1H-imidazol-4-ylsulfonyl)pyrrolidin-3-amin;
N-(4-Chlorbenzyl)-N-((3R,4S)-4-(4-fluorphenyl)-1-(1-methyl-1H-imidazol-4-ylsulfonyl)pyrrolidin-3-yl)acetamid;
5-Butyl-3-{(3R,4S)-4-(4-fluorphenyl)-1-[(1-methyl-1H-imidazol-4-yl)sulfonyl]pyrrolidin-3-yl}-1,3-oxazolidin-2-on;
4-{(3R,4S)-4-(4-Fluorphenyl)-1-[(1-methyl-1H-imidazol-4-yl)sulfonyl]pyrrolidin-3-yl}thiomorpholine 1,1-dioxid;
5-Fluor-N-{(3R,4S)-4-(4-fluorphenyl)-1-[(1-methyl-1H-imidazol-4-yl)sulfonyl]pyrrolidin-3-yl}-4-(trifluormethyl)pyridin-2-amin;
(3R,4S)-1-[(1-Methyl-1H-imidazol-4-yl)sulfonyl]-4-[(2R)-tetrahydro-2H-pyran-2-yl]-N-[3-(trifluormethoxy)phenyl]pyrrolidin-3-amin;
(3R,4S)-4-(4-Fluorphenyl)-1-[(1-methyl-1H-imidazol-4-yl)sulfonyl]-N-[2-methyl-5-(prop-1-en-2-yl)cyclohexyl]pyrrolidin-3-amin;
(3R,4S)-4-(4-Fluorphenyl)-1-[(1-methyl-1H-imidazol-4-yl)sulfonyl]-N-(2,2,2-trifluor-1-phenylethyl)pyrrolidin-3-amin;
N-[2-Chlor-4-(trifluormethyl)benzyl]-2-[(1-methyl-1H-imidazol-4-yl)sulfonyl]octahydro-3aH-isoindol-3a-amin;
2-Chlor-N-12-[(1-methyl-1H-imidazol-4-yl)sulfonyl]octahydro-3aH-isoindol-3a-yl}-3-(trifluormethyl)benzamid;
2,4-Dichlor-N-{2-[(1-methyl-1H-imidazol-4-yl)sulfonyl]octahydro-3aH-isoindol-3a-yl}benzamid;
1-hexyl-6-[(1-methyl-1H-imidazol-4-yl)sulfonyl]octahydro-1H-pyrrolo[3,4-b]pyridin;
(2,4-Dichlorphenyl){[(1-methyl-1H-imidazol-4-yl)sulfonyl]octahydro-1H-pyrrolo[3,4-b]pyridin-1-yl}methanon;
[2-Chlor-3-(trifluormethyl)phenyl]{6-[(1-methyl-1H-imidazol-4-yl)sulfonyl]octahydro-1H-pyrrolo[3,4-b]pyridin-1-yl}methanon;
(3R,4S)-4-(4-Fluorphenyl)-1-[(1-methyl-1H-imidazol-4-yl)sulfonyl]-N-[2,2,2-trifluor-1-(3-methylphenyl)ethyl]pyrrolidin-3-amin;
(3R,4S)-4-(4-Fluorphenyl)-1-[(1-methyl-1H-imidazol-4-yl)sulfonyl]-N-[2,2,2-trifluor-1-(4-methylphenyl)ethyl]pyrrolidin-3-amin;
(1R,2R,3R,4S)-3-(4-Fluorphenyl)-N-[4-fluor-3-(trifluormethyl)phenyl]-7-[(1-methyl-1H-imidazol-4-yl)sulfonyl]-7-azabicyclo[2.2.1]heptan-2-amin;
3-(4-Fluorphenyl)-N-[4-fluor-3-(trifluormethyl)phenyl]-7-[(1-methyl-1H-imidazol-4-yl)sulfonyl]-7-azabicyclo[2.2.1]heptan-2-amin;
1-[2-Chlor-4-(trifluormethyl)benzyl]-6-[(1-methyl-1H-imidazol-4-yl)sulfonyl]octahydro-1H-pyrrolo[3,4-b]pyridin;
[2-Chlor-4-(trifluormethyl)phenyl]{6-[(1-methyl-1H-imidazol-4-yl)sulfonyl]octahydro-1H-pyrrolo[3,4-b]pyridin-1-yl}methanon;
N-(3-Chlor-4-fluorbenzyl)-2-[(1-methyl-1H-imidazol-4-yl)sulfonyl]-2-azaspiro[4.5]decane-4-carboxamid;
N-(3,4-Difluorbenzyl)-2-[(1-methyl-1H-imidazol-4-yl)sulfonyl]-2-azaspiro[4.5]decane-4-carboxamid;
1-{(3R,4S)-4-(4-Fluorphenyl)-1-[(1-methyl-1H-imidazol-4-yl)sulfonyl]pyrrolidin-3-yl}-1,3-dihydro-2H-indol-2-on;
2-[(1-Methyl-1H-imidazol-4-yl)sulfonyl]-N-[4-(trifluormethyl)benzyl]octahydro-3aH-isoindol-3a-amin;
trans-N-[2-Chlor-4-(trifluormethyl)benzyl]-2-[(1-methyl-1H-imidazol-4-yl)sulfonyl]-2,3,3a,8-tetrahydroindeno[1,2-c]pyrrol-8a(1H)-amin;
2-{4-[(4-Fluorphenoxy)methyl]-1-[(1-methyl-1H-imidazol-4-yl)sulfonyl]pyrrolidin-3-yl}pyridin;
2-[(1-Methyl-1H-imidazol-4-yl)sulfonyl]-3a-{[6-(trifluormethyl)pyrimidin-4-yl]oxy}-2,3,3a,4,5,9b-hexahydro-1H-benzo[e]isoindol;
N-(4-Fluorbenzyl)-2-[(1-methyl-1H-imidazol-4-yl)sulfonyl]-1,2,3,4,5,9b-hexahydro-3aH-benzo[e]isoindol-3a-amin;
N-[2-Chlor-4-(trifluormethyl)benzyl]-2-[(1-methyl-1H-imidazol-4-yl)sulfonyl]-1,2,3,4,5,9b-hexahydro-3aH-benzo[e]isoindol-3a-amin;
2-[(1-Methyl-1H-1,2,3-triazol-4-yl)sulfonyl]-N-[6-(trifluormethyl)pyrimidin-4-yl]-2,3,3a,8-tetrahydroindeno[1,2-c]pyrrol-8a(1H)-amin;
2-[(1-Methyl-1H-imidazol-4-yl)sulfonyl]-N-[4-(trifluormethyl)pyridin-2-yl]-2,3,3a,8-tetrahydroindeno[1,2-c]pyrrol-8a(1H)-amin;
N-(2,3-Dihydro-1H-inden-1-yl)-1-[(1-methyl-1H-imidazol-4-yl)sulfonyl]-4-(1-methylpiperidin-2-yl)pyrrolidin-3-amin;
1-[(1-Methyl-1H-imidazol-4-yl)sulfonyl]-4-(1-methylpiperidin-2-yl)-N-[3-(2,2,2-trifluorethoxy)phenyl]pyrrolidin-3-amin;
4-(5-Fluorpyridin-2-yl)-1-[(1-methyl-1H-imidazol-4-yl)sulfonyl]-N-[3-(trifluormethoxy)phenyl]pyrrolidin-3-amin;
(3R,4S)-4-(1,3-Dioxan-2-yl)-1-[(1-methyl-1H-1,2,3-triazol-4-yl)sulfonyl]-N-[3-(trifluormethoxy)phenyl]pyrrolidin-3-amin;
(3R,4S)-4-(1,3-Dioxan-2-yl)-1-[(1-methyl-1H-imidazol-4-yl)sulfonyl]-N-[3-(trifluormethoxy)phenyl]pyrrolidin-3-amin;
1-[(1-methyl-1H-Imidazol-4-yl)sulfonyl]-4-(1-methylpiperidin-2-yl)-N-[3-(trifluormethoxy)phenyl]pyrrolidin-3-amin;
1-[(1-methyl-1H-Imidazol-4-yl)sulfonyl]-4-(1-methylpiperidin-2-yl)-N-[3-(trifluormethoxy)phenyl]pyrrolidin-3-amin;
(3R,4S)-4-(5,5-Dimethyl-1,3-dioxan-2-yl)-1-[(1-methyl-1H-imidazol-4-yl)sulfonyl]-N-[3-(trifluormethoxy)phenyl]pyrrolidin-3-amin;
(3R,4S)-4-(1,3-Dioxepan-2-yl)-1-[(1-methyl-1H-imidazol-4-yl)sulfonyl]-N-[3-(trifluormethoxy)phenyl]pyrrolidin-3-amin;
(3R,4S)-4-(1,3-Dioxolan-2-yl)-1-[(1-methyl-1H-imidazol-4-yl)sulfonyl]-N-[3-(trifluormethoxy)phenyl]pyrrolidin-3-amin;
(3R,4S)-N-(2,3-Dihydro-1H-inden-1-yl)-4-(1,3-dioxan-2-yl)-1-[(1-methyl-1H-imidazol-4-yl)sulfonyl]pyrrolidin-3-amin;
(3R,4S)-N-[2-Chlor-4-(trifluormethyl)benzyl]-4-(1,3-dioxan-2-yl)-1-[(1-methyl-1H-imidazol-4-yl)sulfonyl]pyrrolidin-3-amin;
(3R,4S)-4-(1,3-Dioxan-2-yl)-1-[(1-methyl-1H-imidazol-4-yl)sulfonyl]-N-[3-(trifluormethoxy)benzyl]pyrrolidin-3-amin;
(3R,4S)-4-(1,3-Dioxan-2-yl)-N-(4-fluorbenzyl)-1-[(1-methyl-1H-imidazol-4-yl)sulfonyl]pyrrolidin-3-amin;
(3R,4S)-4-(1,3-Dioxan-2-yl)-N-methyl-1-[(1-methyl-1H-imidazol-4-yl)sulfonyl]-N-pentylpyrrolidin-3-amin;
(3R,4S)-4-(1,3-Dioxan-2-yl)-1-[(1-methyl-1H-imidazol-4-yl)sulfonyl]-N,N-dipentylpyrrolidin-3-amin;
(3R,4S)-4-(1,3-Dioxan-2-yl)-N,N-dihexyl-1-[(1-methyl-1H-imidazol-4-yl)sulfonyl]pyrrolidin-3-amin;
(3R,4S)-4-(1,3-Dioxan-2-yl)-N-hexyl-1-[(1-methyl-1H-imidazol-4-yl)sulfonyl]pyrrolidin-3-amin;
5-Chlor-N-{(3R,4S)-4-(1,3-dioxan-2-yl)-1-[(1-methyl-1H-imidazol-4-yl)sulfonyl]pyrrolidin-3-yl}-4-(trifluormethyl)pyridin-2-amin;
(3R,4S)-4-(1,3-Dioxan-2-yl)-N-[4-fluor-3-(trifluormethyl)phenyl]-1-[(1-methyl-1H-imidazol-4-yl)sulfonyl]pyrrolidin-3-amin;
1-[(1-Methyl-1H-imidazol-4-yl)sulfonyl]-4-(piperidin-1-yl)-N-[3-(trifluormethoxy)phenyl]pyrrolidin-3-amin;
(3R,4S)-N-(3-Chlorphenyl)-4-(1,3-dioxan-2-yl)-1-[(1-methyl-1H-imidazol-4-yl)sulfonyl]pyrrolidin-3-amin;
(3R,4S)-4-(1,3-Dioxan-2-yl)-1-[(1-methyl-1H-imidazol-4-yl)sulfonyl]-N-[3-(trifluormethyl)phenyl]pyrrolidin-3-amin;
(3R,4S)-N-(3-Chlor-4-fluorphenyl)-4-(1,3-dioxan-2-yl)-1-[(1-methyl-1H-imidazol-4-yl)sulfonyl]pyrrolidin-3-amin;
(3R,4S)-4-(1,3-Dioxan-2-yl)-1-[(1-methyl-1H-imidazol-4-yl)sulfonyl]-N-(3-methylphenyl)pyrrolidin-3-amin;
4-({3-(1,3-Dioxan-2-yl)-4-[3-(trifluormethyl)benzyl]pyrrolidin-1-yl}sulfonyl)-1-methyl-1H-imidazol;
4-({3-(1,3-Dioxan-2-yl)-4-[3-(trifluormethyl)benzyl]pyrrolidin-1-yl}sulfonyl)-1-methyl-1H-1,2,3-triazol;
(3R,4S)-4-(5,7-Dioxaspiro[2.5]oct-6-yl)-1-[(1-methyl-1H-imidazol-4-yl)sulfonyl]-N-[3-(trifluormethoxy)phenyl]pyrrolidin-3-amin;
(3R,4S)-4-[(4R,6R)-4,6-Dimethyl-1,3-dioxan-2-yl]-1-[(1-methyl-1H-imidazol-4-yl)sulfonyl]-N-[3-(trifluormethoxy)phenyl]pyrrolidin-3-amin;
(3R,4S)-4-[(4S,6S)-4,6-Dimethyl-1,3-dioxan-2-yl]-1-[(1-methyl-1H-imidazol-4-yl)sulfonyl]-N-[3-(trifluormethoxy)phenyl]pyrrolidin-3-amin;
(3R,4S)-4-(1,3-Dioxan-2-yl)-N-(4-fluor-3-methylphenyl)-1-[(1-methyl-1H-imidazol-4-yl)sulfonyl]pyrrolidin-3-amin;
(3R,4S)-4-(1,3-Dioxan-2-yl)-N-[4-fluor-3-(trifluormethoxy)phenyl]-1-[(1-methyl-1H-imidazol-4-yl)sulfonyl]pyrrolidin-3-amin;
(3R,4S)-N-[3-(Difluormethoxy)phenyl]-4-(1,3-dioxan-2-yl)-1-[(1-methyl-1H-imidazol-4-yl)sulfonyl]pyrrolidin-3-amin;
(3R,4S)-4-(1,3-Dioxan-2-yl)-1-[(1-methyl-1H-imidazol-4-yl)sulfonyl]-N-[3-(propan-2-yl)phenyl]pyrrolidin-3-amin;
4-(5-Fluorpyridin-2-yl)-N-[4-fluor-3-(trifluormethyl)phenyl]-1-[(1-methyl-1H-imidazol-4-yl)sulfonyl]pyrrolidin-3-amin;
(3R,4S)-N-[3-(2,2-Difluoroethoxy)phenyl]-4-(1,3-dioxan-2-yl)-1-[(1-methyl-1H-imidazol-4-yl)sulfonyl]pyrrolidin-3-amin ist,
oder ein physiologisch toleriertes Salz davon ist.

16. Verbindung gemäß Anspruch 1, welche (3R,4S)-1-[(1-Methyl-1H-imidazol-4-yl)sulfonyl]-4-[(2R)-tetrahydro-2H-pyran-2-yl]-N-[3-(trifluormethoxy)phenyl]pyrrolidin-3-amin oder ein physiologisch toleriertes Salz davon ist.

17. Verbindung gemäß Anspruch 1, welche (3R,4S)-4-(1,3-Dioxan-2-yl)-1-[(1-methyl-1H-imidazol-4-yl)sulfonyl]-N-[3-(trifluormethoxy)phenyl]pyrrolidin-3-amin oder ein physiologisch toleriertes Salz davon ist.

18. Verbindung gemäß Anspruch 1, welche (3R,4S)-N-(2,3-Dihydro-1H-inden-1-yl)-4-(4-fluorphenyl)-1-(1-methyl-1H-imidazol-4-ylsulfonyl)pyrrolidin-3-amin oder ein physiologisch toleriertes Salz davon ist.

19. Verbindung gemäß Anspruch 1, welche 4-({(3S,4R)-4-(4-Fluorphenyl)-1-[(1-methyl-1H-imidazol-4-yl)sulfonyl]pyrrolidin-3-yl}oxy)-6-(trifluormethyl)pyrimidin oder ein physiologisch toleriertes Salz davon ist.

## Revendications

1. Dérivé de pyrrolidine de formule (I) 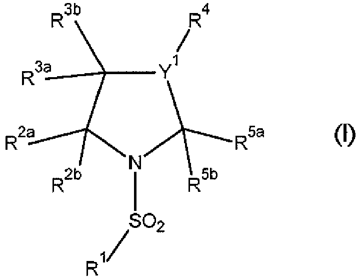 où
R¹ représente un cycle hétérocyclique à 5 chaînons choisi parmi les groupements pyrazolyle, imidazolyle et triazolyle, qui est éventuellement substitué par 1, 2 ou 3 substituants indépendamment choisis dans le groupe constitué par les atomes d'halogène et les groupements alkyle en C₁-C₆, halogénoalkyle en C₁-C₆, cycloalkyle en C₃-C₆, (alkoxy en C₁-C₄) -carbonyle et (alkyle en C₁-C₆)-carbonylamino ;
chacun des radicaux R^{2a}, R^{2b}
représente indépendamment un atome d'hydrogène ou d'halogène ou un groupement alkyle en C₁-C₃, ou
R^{2a}, R^{2b}
peuvent former ensemble et avec l'atome de carbone auquel ils sont liés un groupement C=O ;
R^{3a}
représente un groupement cycloalkyle en C₃-C₁₂, aryle en C₆-C₁₂ éventuellement substitué, hydroxy, alkoxy en C₁-C₆, halogénoalkoxy en C₁-C₆, (cycloalkyle en C₃-C₁₂)-(alkoxy en C₁-C₄), alcényloxy en C₂-C₆, (aryle en C₆-C₁₂)-(alkoxy en C₁-C₄), (hétérocyclyle en C₃-C₁₂) - (alkoxy en C₁-C₄) , aryloxy en C₆-C₁₂ éventuellement substitué, hétérocyclyloxy en C₃-C₁₂ ou hétérocyclyle en C₃-C₁₂ éventuellement substitué ; ou
R^{3b} représente un atome d'hydrogène ou un groupement alkyle en C₁-C₆ ou hydroxyle ; ou
R^{3a} et R^{3b}
forment ensemble un groupement alkylène en C₂-C₅ éventuellement substitué ;
Y¹ représente >CR⁶- ou >N- ;
R⁶ représente un atome d'hydrogène ou un groupement alkyle en C₁-C₆, halogénoalkyle en C₁-C₆, (aryle en C₆-C₁₂ éventuellement substitué)-(alkyle en C₁-C₄), hydroxyalkyle en C₁-C₆, (alkoxy en C₁-C₄)-(alkyle en C₁-C₆) ou hydroxyle ; ou
R⁶ et R^{3a} ou R^{3b}
forment ensemble un groupement alkylène en C₁-C₅ éventuellement substitué ; ou
R⁶
représente un groupement alkylène en C₁-C₄ qui est lié à un atome de carbone de R^{3a}, et R^{3a} représente un groupement aryle en C₆-C₁₂ éventuellement substitué ou hétérocyclyle en C₃-C₁₂ éventuellement substitué ;
R⁴ représente un groupement -NR^{8a}(CR^{9a}R^{9b})ₙ₄R¹³,-(CR^{7a}R^{7b})ₙ₁OR¹⁰, -(CR^{7c}R^{7d}NR^{11a}R^{11b}, -(CR^{7e}R^{7f})ₙ₃R¹², aryle en C₆-C₁₂ éventuellement substitué, -NR^{8b}COR¹⁴, -NR^{8c}COOR¹⁵,-NR^{8d}CONR^{16a}R^{16b}, -NR^{8e}SO₂R¹⁷, -O(CR^{9c}R^{9d})ₙ₅R¹⁸, -COR¹⁹,-CONR^{20a}R^{20b}, -SO₂R²¹ ou ou hétérocyclyle en C₃-C₁₂ éventuellement substitué ;
chacun des radicaux R^{7a}, R^{7b}
représente indépendamment un atome d'hydrogène ou un groupement alkyle en C₁-C₆,
n1 est égal à 1, 2, 3 ou 4 ;
R¹⁰ représente un atome d'hydrogène ou un groupement aryle en C₆-C₁₂ éventuellement substitué, ou hétérocyclyle en C₃-C₁₂ éventuellement substitué ;
chacun des radicaux R^{7c}, R^{7d}
représente indépendamment un atome d'hydrogène ou un groupement alkyle en C₁-C₆,
n2 est égal à 1, 2, 3 ou 4 ;
R^{11a} représente un groupement alkyle en C₁-C₈, (cycloalkyle en C₃-C₁₂ éventuellement substitué)-(alkyle en C₁-C₄), (alkoxy en C₁-C₆-(alkyle en C₁-C₆, (aryle en C₆-C₁₂ éventuellement substitué) - (alkyle en C₁-C₄), (hétérocyclyle en C₃-C₁₂ éventuellement substitué)-(alkyle en C₁-C₄), aryle en C₆-C₁₂ éventuellement substitué, ou hétérocyclyle en C₃-C₁₂ éventuellement substitué ;
R^{11b}
représente un atome d'hydrogène ou un groupement alkyle en C₁-C₆ ;
chacun des radicaux R^{7e}, R^{7f}
représente indépendamment un atome d'hydrogène ou un groupement alkyle en C₁-C₆,
n3 est égal à 1, 2, 3 ou 4 ;
R¹²
représente un groupement aryle en C₆-C₁₂ éventuellement substitué, ou hétérocyclyle en C₃-C₁₂ éventuellement substitué ;
chacun des radicaux R^{8a}, R^{8b}, R^{8c}, R^{8d}, R^{8e} représente indépendamment un atome d'hydrogène ou un groupement alkyle en C₁-C₆ ou (alkyle en C₁-C₆)-carbonyle, ou
R⁶ et l'un des radicaux R^{8a}, R^{8b}, R^{8c}, R^{8d} ou R^{8e} forment ensemble un groupement alkylène en C₁-C₅ éventuellement substitué, où un ou plusieurs des radicaux -CH₂- du groupement alkylène en C₁-C₅ peuvent être indépendamment remplacés par un atome d'oxygène ou C=O ; ou
R^{3a} et l'un des radicaux R^{8a} ou R^{8b}
forment ensemble un groupement alkylène en C₁-C₅ éventuellement substitué ;
chacun des radicaux R^{9a}, R^{9b}
représente indépendamment un atome d'hydrogène ou d'halogène, ou un groupement alkyle en C₁-C₆, halogénoalkyle en C₁-C₆, hydroxyle ou alkoxy en C₁-C₆ ;
n4 est égal à 0, 1, 2, 3 ou 4 ;
R¹³ représente un atome d'hydrogène ou un groupement alkyle en C₁-C₈, halogénoalkyle en C₁-C₆, (cycloalkyle en C₃-C₁₂ éventuellement substitué) - (alkyle en C₁-C₄), (alkoxy en C₁-C₆)-(alkyle en C₁-C₆), cycloalkyle en C₆-C₁₂ éventuellement substitué, alcényle en C₁-C₆, cycloalcényle en C₃-C₆ éventuellement substitué, aryle en C₆-C₁₂ éventuellement substitué, hydroxy, alkoxy en C₁-C₆, (alkoxy en C₁-C₆-(alkoxy en C₁-C₄), aryloxy en C₆-C₁₂ éventuellement substitué, hétérocyclyloxy en C₃-C₁₂ éventuellement substitué, hétérocyclyle en C₃-C₁₂ éventuellement substitué ou tri-(alkyle en C₁-C₄)-silyloxy ;
R¹⁴ représente un groupement alkyle en C₁-C₆, halogénoalkyle en C₁-C₆, (cycloalkyle en C₃-C₁₂ éventuellement substitué)-(alkyle en C₁-C₄), (aryle en C₆-C₁₂ éventuellement substitué)-(alkyle en C₁-C₄), hydroxyalkyle en C₁-C₆, (alkoxy en C₁-C₆-(alkyle en C₁-C₆), (aryloxy en C₆-C₁₂ éventuellement substitué)-(alkyle en C₁-C₄), (alkyle en C₁-C₆-carbonyl-(alkyle en C₁-C₄), (alkoxy en C₁-C₆)-carbonyl-(alkyle en C₁-C₄), (alkyle en C₁-C₆)-aminocarbonyl-(alkyle en C₁-C₄), (hétérocyclyle en C₃-C₁₂)-(alkyle en C₁-C₄) éventuellement substitué, cycloalkyle en C₃-C₁₂ éventuellement substitué, aryle en C₆-C₁₂ éventuellement substitué ou hétérocyclyle en C₃-C₁₂ éventuellement substitué ;
R¹⁵ représente un groupement alkyle en C₁-C₆, aryle en C₆-C₁₂ éventuellement substitué, ou hétérocyclyle en C₃-C₁₂ éventuellement substitué ;
R^{16a} représente un groupement (aryle en C₆-C₁₂ éventuellement substitué)-(alkyle en C₁-C₄), (hétérocyclyle en C₃-C₁₂)-(alkyle en C₁-C₄) éventuellement substitué, aryle en C₆-C₁₂ éventuellement substitué ou hétérocyclyle en C₃-C₁₂ éventuellement substitué ;
R^{16b} représente un atome d'hydrogène ou un groupement alkyle en C₁-C₆ ;
R¹⁷ représente un groupement (aryle en C₆-C₁₂ éventuellement substitué)-(alkyle en C₁-C₄), (hétérocyclyle en C₃-C₁₂ éventuellement substitué)-(alkyle en C₁-C₄), aryle en C₆-C₁₂ éventuellement substitué ou hétérocyclyle en C₃-C₁₂ éventuellement substitué ;
chacun des radicaux R^{9c}, R^{9d}
représente indépendamment un atome d'hydrogène ou d'halogène ou un groupement alkyle en C₁-C₆ ;
n5 est égal à 0, 1, 2, 3 ou 4 ;
R¹⁸ représente un atome d'hydrogène ou un groupement alkyle en C₁-C₆, cycloalkyle en C₃-C₁₂ éventuellement substitué, (alkyle en C₁-C₆)-carbonyle, (alkoxy en C₁-C₆)-carbonyle, (halogénoalkoxy en C₁-C₆)-carbonyle, (aryloxy en C₆-C₁₂) -carbonyle, (alkyle en C₁-C₆-aminocarbonyle, (halogénoalkyle en C₁-C₄)-aminocarbonyle, (aryle en C₆-C₁₂)-aminocarbonyle, aryle en C₆-C₁₂ éventuellement substitué, (alkyle en C₁-C₆-amine, (cycloalkyle en C₃-C₁₂) - (alkyle en C₁-C₄) -amino, (halogénoalkyle en C₁-C₆ -amino, (alkoxy en C₁-C₆-(alkyle en C₁-C₆ -amino, (aryle en C₆-C₁₂) - (alkyle en C₁-C₄) -amino, di-(alkyle en C₁-C₆-amine, (aryle en C₆-C₁₂ éventuellement substitué)-amine, ou hétérocyclyle en C₃-C₁₂ éventuellement substitué ;
R¹⁹ représente un groupement aryle en C₆-C₁₂ éventuellement substitué, ou hétérocyclyle en C₃-C₁₂ éventuellement substitué ;
R^{20a} représente un groupement alkyle en C₁-C₆, (cycloalkyle en C₃-C₁₂ éventuellement substitué)-(alkyle en C₁-C₄), (alkoxy en C₁-C₆-(alkyle en C₁-C₆, (aryle en C₆-C₁₂ éventuellement substitué)-(alkyle en C₁-C₄), (hétérocyclyle en C₃-C₁₂ éventuellement substitué)-(alkyle en C₁-C₄), aryle en C₆-C₁₂ éventuellement substitué ou hétérocyclyle en C₃-C₁₂ éventuellement substitué ;
R^{20b} représente un atome d'hydrogène ou un groupement alkyle en C₁-C₆ ;
R²¹ représente un groupement aryle en C₆-C₁₂ éventuellement substitué, ou hétérocyclyle en C₃-C₁₂ éventuellement substitué ; et
chacun des radicaux R^{5a}, R^{5b}
représente indépendamment un atome d'hydrogène ou d'halogène ou un groupement alkyle en C₁-C₃, ou
R^{5a}, R^{5b}
peuvent former ensemble et avec l'atome de carbone auquel ils sont liés un groupement C=O ; ou
l'un des radicaux R^{2a} ou R^{2b} et l'un des radicaux R^{5a} ou R^{5b}
forment ensemble un groupement alkylène en C₁-C₅ éventuellement substitué,
ou sel physiologiquement toléré de celui-ci ;
à la condition que les composés suivants soient exclus :
les composés de formule (II) et les sels physiologiquement tolérés de ceux-ci ; 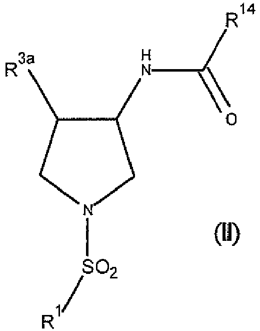 où R¹, R^{3a} et R¹⁴ sont tels que définis dans le tableau suivant
| R¹ | R^{3a} | R¹⁴ |
|---|---|---|
| 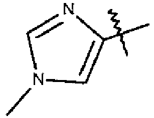 | 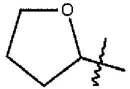 | 4-F-Ph |
| 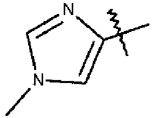 | 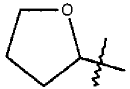 | 4-CF₃-Ph |
| 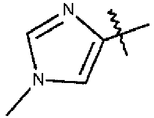 | 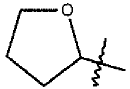 | 2-OMe-Ph |
| 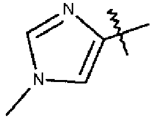 | 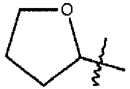 | 2-OMe-4-OCF₃-Ph |
| 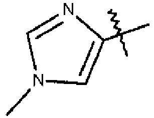 | 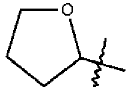 | 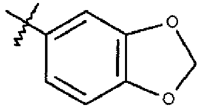 |
| 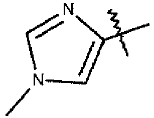 | 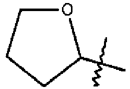 | 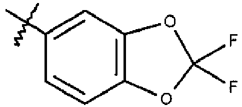 |
| 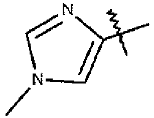 | 4-F-phényle | 4-CF₃-phényle |
| 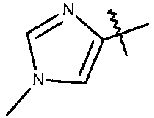 | 4-F-phényle | 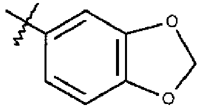 |
les composés de formule (II') 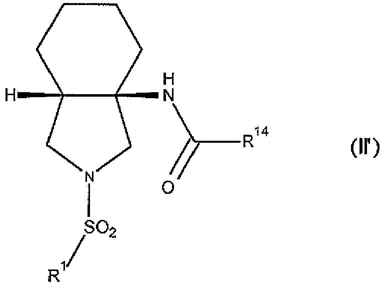 où R¹ et R¹⁴ sont tels que définis dans le tableau suivant
| R¹ | R¹⁴ |
|---|---|
| 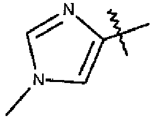 | 4-F-Ph |
| 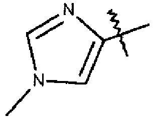 | 2-OMe-Ph |
| 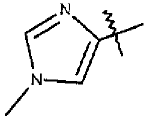 | 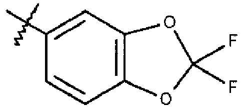 |
et les composés de formule :

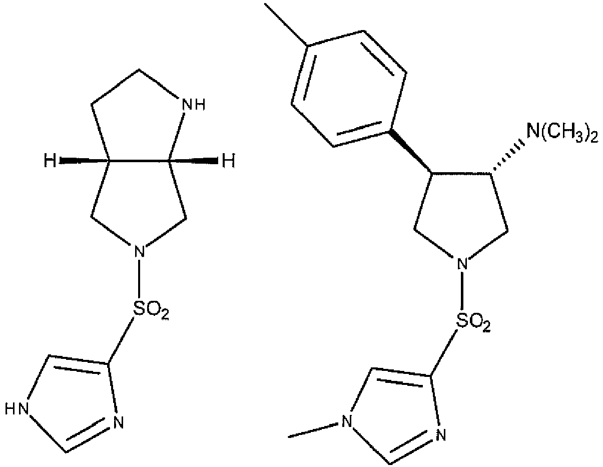

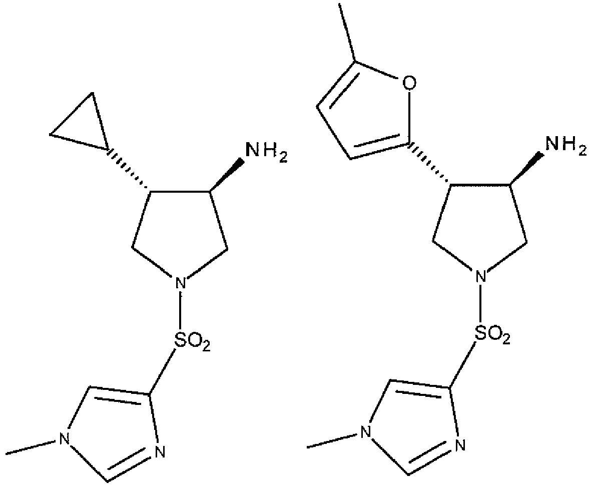

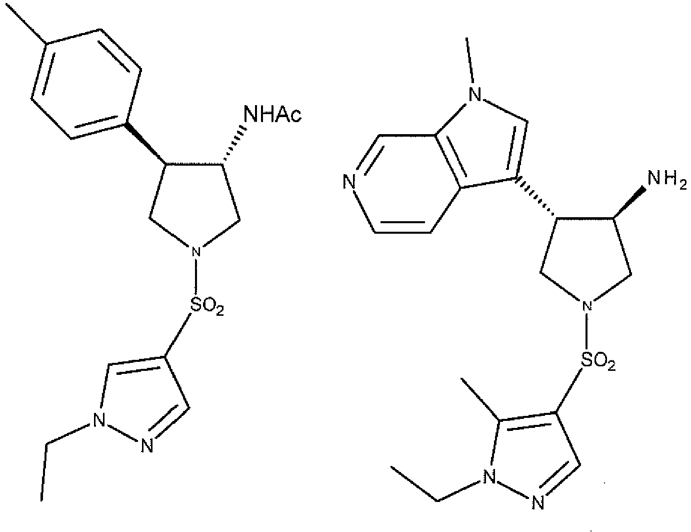

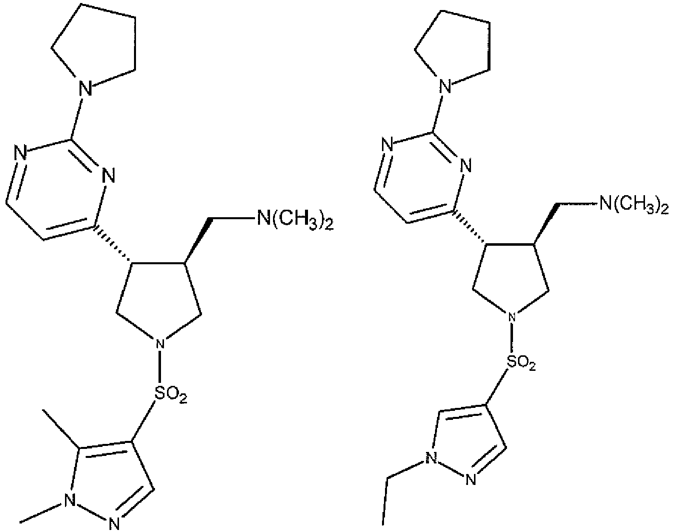

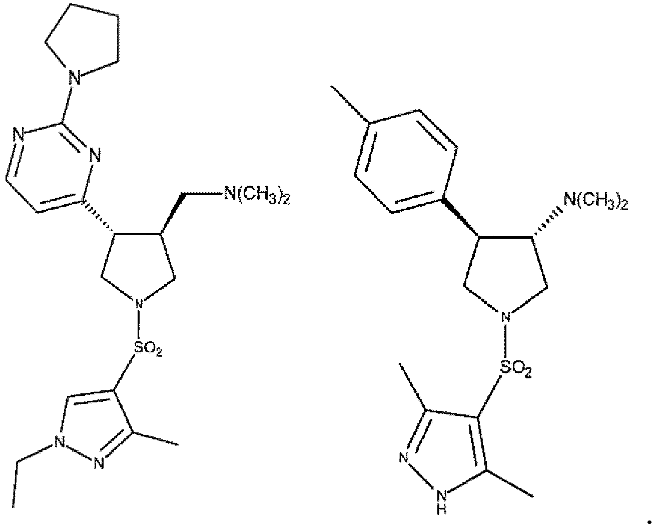

2. Composé selon la revendication 1, où R¹ représente un groupement 1,3-diazolyle éventuellement substitué ou 1,2,3-triazolyle éventuellement substitué.

3. Composé selon la revendication 1, où R¹ représente un groupement 1,2-diazol-4-yle, 1-méthyl-1,2-diazol-4-yle, 1-méthyl-1,2-diazol-3-yle, 1-méthyl-1,3-diazol-4-yle, 1-méthyl-1,2-diazol-5-yle, 1,5-diméthyl-1,2-diazol-4-yle, 1,3-diméthyl-1,2-diazol-4-yle, 1,5-diméthyl-1,2-diazol-4-yle, 1,3-diméthyl-1,2-diazol-5-yle, 1,3-diméthyl-5-Cl-1,2-diazol-4-yle, 1-iso-propyl-3-méthyl-1,2-diazol-4-yle, 1-Me-3-CF₃-1,2-diazol-4-yle, 1-cyclopentyl-3-Me-1,2-diazol-4-yle, 1,3,5-triméthyl-1,2-diazol-4-yle, 1-CHF₂-3,5-diméthyl-1,2-diazol-4-yle, 1,2-diméthyl-1,3-diazol-4-yle, 1,2-diméthyl-1,3-diazol-5-yle, 1-méthyl-5-Cl-1,3-diazol-4-yle, 1-Me-1,2,3-triazol-4-yle, 1-éthyl-1,3-diazol-4-yle ou 1-méthyl-1,2,4-triazol-3-yle.

4. Composé selon l'une quelconque des revendications 1 à 3, où R^{2a} représente un atome d'hydrogène ou d'halogène ou un groupement alkyle en C₁-C₃, et R^{2b} représente un atome d'hydrogène.

5. Composé selon l'une quelconque des revendications 1 à 4, où R^{3a} représente un groupement aryle en C₆-C₁₂ éventuellement substitué par 1, 2 ou 3 substituants indépendamment choisis dans le groupe constitué par les atomes d'halogène et les groupements alkyle en C₁-C₄ et alkoxy en C₁-C₄.

6. Composé selon l'une quelconque des revendications 1 à 4, où R^{3a} représente un groupement hétérocyclyle en C₃-C₁₂ éventuellement substitué par 1, 2 ou 3 substituants indépendamment choisis dans le groupe constitué par les atomes d'halogène et les groupements alkyle en C₁-C₄ et cycloalkyle en C₃-C₆.

7. Composé selon l'une quelconque des revendications 1 à 6, où R^{3b} représente un atome d'hydrogène.

8. Composé selon l'une quelconque des revendications 1 à 7, où Y¹ représente un groupement >CR⁶- et R⁶ représente un atome d'hydrogène ou un groupement méthyle, benzyle, hydroxyméthyle ou hydroxy.

9. Composé selon l'une quelconque des revendications 1 à 8, où R^{5a} représente un atome d'hydrogène ou d'halogène ou un groupement alkyle en C₁-C₃, et R^{5b} représente un atome d'hydrogène.

10. Composé selon l'une quelconque des revendications 1 à 9, répondant à la formule 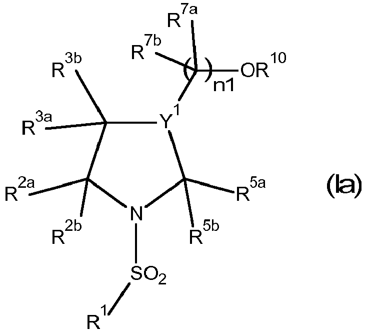
où R¹, R^{2a}, R^{2b}, R^{3a}, R^{3b}, Y¹, R^{5a} et R^{5b} sont tels que définis dans l'une quelconque des revendications 1 à 9, et
chacun des radicaux R^{7a}, R^{7b}
représente indépendamment un atome d'hydrogène ou un groupement alkyle en C₁-C₆ ;
n1 est égal à 1, 2, 3 ou 4 ; et
R¹⁰ représente un atome d'hydrogène ou un groupement aryle en C₆-C₁₂ éventuellement substitué, ou hétérocyclyle en C₃-C₁₂ éventuellement substitué.

11. Composé selon l'une quelconque des revendications 1 à 9, répondant à la formule 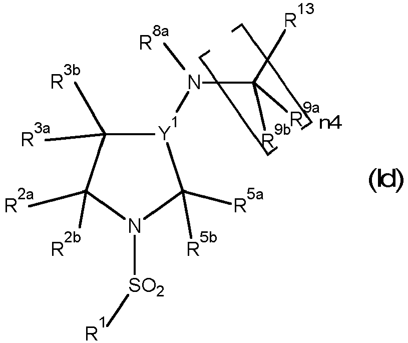
où R¹, R^{2a}, R^{2b}, R^{3a}, R^{3b}, Y¹, R^{5a} et R^{5b} sont tels que définis dans l'une quelconque des revendications 1 à 9, et
R^{8a} représente un atome d'hydrogène ou un groupement alkyle en C₁-C₆ ou (alkyle en C₁-C₆) -carbonyle, ou
R⁶, R^{8a}
forment ensemble un groupement alkylène en C₁-C₅ éventuellement substitué, où un ou plusieurs des radicaux -CH₂- du groupement alkylène en C₁-C₅ peuvent être indépendamment remplacés par un atome d'oxygène ou C=O ;
chacun des radicaux R^{9a}, R^{9b}
représente indépendamment un atome d'hydrogène ou d'halogène ou un groupement alkyle en C₁-C₆, hydroxyle ou alkoxy en C₁-C₆ ;
n4 est égal à 0, 1, 2, 3 ou 4 ;
R¹³ représente un atome d'hydrogène ou un groupement alkyle en C₁-C₆, halogénoalkyle en C₁-C₆, (cycloalkyle en C₃-C₁₂ éventuellement substitué)-(alkyle en C₁-C₄), (alkoxy en C₁-C₆)-(alkyle en C₁-C₆), cycloalkyle en C₆-C₁₂ éventuellement substitué, alcényle en C₁-C₆, cycloalcényle en C₃-C₆ éventuellement substitué, aryle en C₆-C₁₂ éventuellement substitué, hydroxy, alkoxy en C₁-C₆, (alkoxy en C₁-C₆-(alkoxy en C₂-C₄), aryloxy en C₆-C₁₂ éventuellement substitué, hétérocyclyloxy en C₃-C₁₂ éventuellement substitué, hétérocyclyle en C₁-C₁₂ éventuellement substitué ou tri-(alkyle en C₃-C₄)-silyloxy.

12. Composé selon la revendication 11, où R¹³ représente un groupement de formule (Id1) : 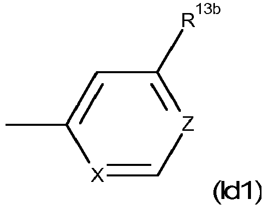 où
X représente >CH- ou >N- ;
Z représente >C-R^{13c} ou >N- ;
R^{13b} représente un atome d'halogène ou un groupement alkyle en C₁-C₄, halogénoalkyle en C₁-C₄, hydroxy-(halogénoalkyle en C₁-C₄), (aryle en C₆-C₁₂) - (alkyle en C₁-C₄), cycloalkyle en C₃-C₆, CN, aryle en C₆-C₁₂ éventuellement substitué par halogène ou alkyle en C₁-C₄, alkoxy en C₁-C₄, halogénoalkoxy en C₁-C₄, cycloalkoxy en C₃-C₆, (aryle en C₆-C₁₂) -(alkoxy en C₁-C₄), (hétérocyclyle en C₃-C₁₂) -(alkoxy en C₁-C₄), aryloxy en C₆-C₁₂, (alkyle en C₁-C₄) -sulfonyle, (alkyle en C₁-C₄)-carbonylamino ou hétérocyclyle en C₃-C₁₂ ; et
R^{13c} représente un atome d'hydrogène ou d'halogène.

13. Composé selon l'une quelconque des revendications 1 à 9, répondant à la formule 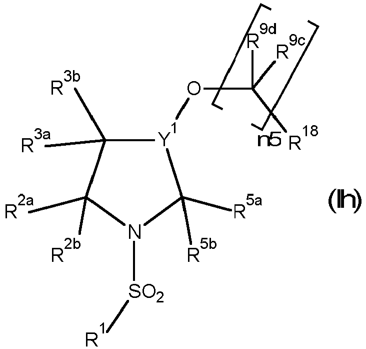
où R¹, R^{2a}, R^{2b}, R^{3a}, R^{3b}, Y¹, R^{5a} et R^{5b} sont tels que définis dans l'une quelconque des revendications 1 à 9, et
chacun des radicaux R^{9c}, R^{9d}
représente indépendamment un atome d'hydrogène ou d'halogène ou un groupement alkyle en C₁-C₆ ;
n5 est égal à 0, 1, 2, 3 ou 4 ; et
R¹⁸ représente un atome d'hydrogène ou un groupement alkyle en C₁-C₆ éventuellement substitué, cycloalkyle en C₃-C₁₂ éventuellement substitué, (alkyle en C₁-C₆-carbonyle, (alkoxy en C₁-C₆)-carbonyle, (halogénoalkoxy en C₁-C₆)-carbonyle, (aryloxy en C₆-C₁₂) -carbonyle, (alkyle en C₁-C₆-aminocarbonyle, (halogénoalkyle en C₁-C₄) -aminocarbonyle, (aryle en C₆-C₁₂) -aminocarbonyle, aryle en C₆-C₁₂ éventuellement substitué, (alkyle en C₁-C₆)-amine, (cycloalkyle en C₃-C₁₂)-(alkyle en C₁-C₄)-amino, (halogénoalkyle en C₁-C₆ -amino, (alkoxy en C₁-C₆)-(alkyle en C₁-C₆-amino, (aryle en C₆-C₁₂)-(alkyle en C₁-C₄)-amino, di-(alkyle en C₁-C₆-amine, (aryle en C₆-C₁₂)-amine éventuellement substitué ou hétérocyclyle en C₃-C₁₂ éventuellement substitué.

14. Composé selon la revendication 1, où
R¹ représente un cycle hétérocyclique à 5 chaînons choisi parmi les groupements pyrazolyle, imidazolyle et triazolyle, qui est éventuellement substitué par 1, 2 ou 3 substituants indépendamment choisis dans le groupe constitué par les atomes d'halogène et les groupements alkyle en C₁-C₆, halogénoalkyle en C₁-C₆, cycloalkyle en C₃-C₆, (alkoxy en C₁-C₄) -carbonyle et (alkyle en C₁-C₆)-carbonylamino ;
chacun des radicaux R^{2a}, R^{2b}
représente un atome d'hydrogène ;
R^{3a} représente un groupement cycloalkyle en C₃-C₁₂, aryle en C₆-C₁₂ éventuellement substitué, hydroxy, alkoxy en C₁-C₆, (cycloalkyle en C₃-C₁₂)-(alkoxy en C₁-C₄), alcényloxy en C₂-C₆, (aryle en C₆-C₁₂)-(alkoxy en C₁-C₄), aryloxy en C₆-C₁₂ éventuellement substitué ou hétérocyclyle en C₃-C₁₂ éventuellement substitué ;
R^{3b} représente un atome d'hydrogène ou un groupement hydroxy ;
Y¹ représente >CR⁶- ou >N- ;
R⁶ représente un atome d'hydrogène ou un groupement alkyle en C₁-C₆, (aryle en C₆-C₁₂) - (alkyle en C₁-C₄), hydroxyalkyle en C₁-C₆ ou hydroxy ;
R⁴ représente un groupement - (CR^{7a}R^{7b})ₙ₁OR¹⁰,-(CR^{7c}R^{7d})ₙ₂NR^{11a}R^{11b}, -(CR^{7e}R^{7f})ₙ₃R¹², aryle en C₆-C₁₂ éventuellement substitué, -NR^{8a}(CR^{9a}R^{9b})ₙ₄R¹³, -NR^{8b}COR¹⁴,-NR^{8c}COOR¹⁵, -NR^{8d}CONR^{16a}R^{16b}, -O(CR^{9c}R^{9d})ₙ₅R¹⁸, -COR¹⁹,-CONR^{20a}R^{20b}, -SO₂R²¹ ou hétérocyclyle en C₃-C₁₂ éventuellement substitué ;
chacun des radicaux R^{7a}, R^{7b}
représente un atome d'hydrogène, ou
n1 est égal à 1 ;
R¹⁰ représente un atome d'hydrogène ou un groupement aryle en C₆-C₁₂ éventuellement substitué, ou hétérocyclyle en C₃-C₁₂ éventuellement substitué ;
chacun des radicaux R^{7c}, R^{7d}
représente un atome d'hydrogène ;
n2 est égal à 1 ;
R^{11a} représente un groupement alkyle en C₁-C₆, (cycloalkyle en C₃-C₁₂)-(alkyle en C₁-C₄), (alkoxy en C₁-C₆)-(alkyle en C₁-C₆), (aryle en C₆-C₁₂)-(alkyle en C₁-C₄), aryle en C₆-C₁₂ éventuellement substitué ;
R^{11b}
représente un atome d'hydrogène ;
chacun des radicaux R^{7e}, R^{7f}
représente un atome d'hydrogène,
n3 est égal à 1 ;
R¹²
représente un groupement aryle en C₆-C₁₂ éventuellement substitué, ou hétérocyclyle en C₃-C₁₂ éventuellement substitué ;
chacun des radicaux R^{8a}, R^{8b}, R^{8c}, R^{8d}, R^{8e} représente indépendamment un atome d'hydrogène ou un groupement alkyle en C₁-C₆, ou
R⁶ et l'un des radicaux R^{8a}, R^{8b}, R^{8c}, R^{8d} ou R^{8e} forment ensemble un groupement alkylène en C₁-C₅ éventuellement substitué, où un ou plusieurs des radicaux -CH₂- du groupement alkylène en C₁-C₅ peuvent être indépendamment remplacés par un atome d'oxygène ou C=O ;
chacun des radicaux R^{9a}, R^{9b}
représente indépendamment un atome d'hydrogène ou d'halogène ou un groupement alkyle en C₁-C₆ ou alkoxy en C₁-C₆ ;
n4 est égal à 0, 1, 2, 3 ou 4 ;
R¹³ représente un atome d'hydrogène ou un groupement alkyle en C₁-C₈, halogénoalkyle en C₁-C₆, (alkoxy en C₁-C₆)-(alkyle en C₁-C₆), cycloalkyle en C₃-C₁₂ éventuellement substitué, alcényle en C₂-C₆, cycloalcényle en C₃-C₆ éventuellement substitué, aryle en C₆-C₁₂ éventuellement substitué, alkoxy en C₁-C₆, (alkoxy en C₁-C₆)-(alkoxy en C₁-C₄), aryloxy en C₆-C₁₂ éventuellement substitué, hétérocyclyle en C₃-C₁₂ éventuellement substitué ou tri-(alkyle en C₁-C₄)-silyloxy ;
R¹⁴ représente un groupement alkyle en C₁-C₆, halogénoalkyle en C₁-C₆, (cycloalkyle en C₃-C₁₂)-(alkyle en C₁-C₄), hydroxyalkyle en C₁-C₆, (aryloxy en C₆-C₁₂ éventuellement substitué)-(alkyle en C₁-C₄), (alkyle en C₁-C₆)-carbonyl-(alkyle en C₁-C₄), (alkoxy en C₁-C₆)-carbonyl-(alkyle en C₁-C₄), cycloalkyle en C₃-C₁₂ éventuellement substitué, aryle en C₆-C₁₂ éventuellement substitué ou hétérocyclyle en C₃-C₁₂ éventuellement substitué ;
R¹⁵ représente un groupement alkyle en C₁-C₆, aryle en C₆-C₁₂ ou hétérocyclyle en C₃-C₁₂ éventuellement substitué ;
R^{16a} représente un groupement (aryle en C₆-C₁₂ éventuellement substitué)-(alkyle en C₁-C₄) ou aryle en C₆-C₁₂ éventuellement substitué ;
R^{16b} représente un atome d'hydrogène ;
chacun des radicaux R^{9c}, R^{9d}
représente un atome d'hydrogène ;
n5 est égal à 0, 1 ou 2 ;
R¹⁸ représente un atome d'hydrogène ou un groupement alkyle en C₁-C₈, (alkoxy en C₁-C₆)-carbonyle, (alkyle en C₁-C₆)-aminocarbonyle, (halogénoalkyle en C₁-C₄)-aminocarbonyle, aryle en C₆-C₁₂ éventuellement substitué, (alkyle en C₁-C₆)-aminé, (halogénoalkyle en C₁-C₆)-amino, (aryle en C₆-C₁₂ éventuellement substitué)-amine ou hétérocyclyle en C₃-C₁₂ éventuellement substitué ;
R¹⁹ représente un groupement aryle en C₆-C₁₂ éventuellement substitué ;
R^{20a} représente un groupement alkyle en C₁-C₆, (cycloalkyle en C₃-C₁₂)-(alkyle en C₁-C₄), (alkoxy en C₁-C₆)-(alkyle en C₁-C₆), (aryle en C₆-C₁₂ éventuellement substitué)-(alkyle en C₁-C₄), (hétérocyclyle en C₃-C₁₂ éventuellement substitué)-(alkyle en C₁-C₄), aryle en C₆-C₁₂ éventuellement substitué ou hétérocyclyle en C₃-C₁₂ éventuellement substitué ;
R^{20b} représente un atome d'hydrogène ou un groupement alkyle en C₁-C₈ ;
R²¹ représente un groupement aryle en C₆-C₁₂ ; et
chacun des radicaux R^{5a}, R^{5b}
représente un atome d'hydrogène, ou
R^{5a} R^{5b}
peuvent former ensemble et avec l'atome de carbone auquel ils sont liés un groupement C=O.

15. Composé selon la revendication 1, qui est l'un des suivants :
2-chloro-N-{trans-1-[(1-méthyl-1H-imidazol-4-yl)sulfonyl]-4-phénylpyrrolidin-3-yl}-3-(trifluorométhyl)benzamide ;
2-chloro-N-{trans-4-(4-méthoxyphényl)-1-[(1-méthyl-1H-imidazol-4-yl)sulfonyl]pyrrolidin-3-yl}-3-(trifluorométhyl)benzamide ;
2-chloro-N-{trans-4-(4-fluorophényl)-1-[(1-méthyl-1H-imidazol-4-yl)sulfonyl]pyrrolidin-3-yl}-3-(trifluorométhyl)benzamide ;
2-chloro-N-{trans-1-[(1-méthyl-1H-imidazol-4-yl)sulfonyl]-4-phénylpyrrolidin-3-yl}-3-(trifluorométhyl)benzamide ;
2-chloro-N-{trans-4-(4-chlorophényl)-1-[(1-méthyl-1H-imidazol-4-yl)sulfonyl]pyrrolidin-3-yl}-3-(trifluorométhyl)benzamide ;
2-chloro-N-{trans-1-[(1-méthyl-1H-imidazol-4-yl)sulfonyl]-4-phénylpyrrolidin-3-yl}benzamide ;
3-chloro-N-{trans-1-[(1-méthyl-1H-imidazol-4-yl)sulfonyl]-4-phénylpyrrolidin-3-yl}benzamide ;
4-chloro-N-{trans-1-[(1-méthyl-1H-imidazol-4-yl)sulfonyl]-4-phénylpyrrolidin-3-yl}benzamide ;
2-fluoro-N-{trans-1-[(1-méthyl-1H-imidazol-4-yl)sulfonyl]-4-phénylpyrrolidin-3-yl}benzamide ;
3-fluoro-N-{trans-1-[(1-méthyl-1H-imidazol-4-yl)sulfonyl]-4-phénylpyrrolidin-3-yl}benzamide ;
4-fluoro-N-{trans-1-[(1-méthyl-1H-imidazol-4-yl)sulfonyl]-4-phénylpyrrolidin-3-yl}benzamide ;
2-méthoxy-N-{trans-1-[(1-méthyl-1H-imidazol-4-yl)sulfonyl]-4-phénylpyrrolidin-3-yl}benzamide ;
3-méthoxy-N-{trans-1-[(1-méthyl-1H-imidazol-4-yl)sulfonyl]-4-phénylpyrrolidin-3-yl}benzamide ;
2-méthyl-N-{trans-1-[(1-méthyl-1H-imidazol-4-yl)sulfonyl]-4-phénylpyrrolidin-3-yl}benzamide ;
3-méthyl-N-{trans-1-[(1-méthyl-1H-imidazol-4-yl)sulfonyl]-4-phénylpyrrolidin-3-yl}benzamide ;
4-méthyl-N-{trans-1-[(1-méthyl-1H-imidazol-4-yl)sulfonyl]-4-phénylpyrrolidin-3-yl}benzamide ;
N-{trans-1-[(1-méthyl-1H-imidazol-4-yl)sulfonyl]-4-phénylpyrrolidin-3-yl}benzamide ;
2,4-dichloro-N-{trans-1-[(1-méthyl-1H-imidazol-4-yl)sulfonyl]-4-phénylpyrrolidin-3-yl}benzamide ;
2-chloro-4-fluoro-N-{trans-1-[(1-méthyl-1H-imidazol-4-yl)sulfonyl]-4-phénylpyrrolidin-3-yl}benzamide ;
trans-1-[(1-méthyl-1H-imidazol-4-yl)sulfonyl]-N,4-diphénylpyrrolidin-3-amine ;
3,5-dichloro-N-{trans-1-[(1-méthyl-1H-imidazol-4-yl)sulfonyl]-4-phénylpyrrolidin-3-yl}benzamide ;
2,3-dichloro-N-{trans-1-[(1-méthyl-1H-imidazol-4-yl)sulfonyl]-4-phénylpyrrolidin-3-yl}benzamide ;
2-cyano-N-{trans-1-[(1-méthyl-1H-imidazol-4-yl)sulfonyl]-4-phénylpyrrolidin-3-yl}benzamide ;
3-cyano-N-{trans-1-[(1-méthyl-1H-imidazol-4-yl)sulfonyl]-4-phénylpyrrolidin-3-yl}benzamide ;
4-cyano-N-{trans-1-[(1-méthyl-1H-imidazol-4-yl)sulfonyl]-4-phénylpyrrolidin-3-yl}benzamide ;
N-{trans-1-[(1-méthyl-1H-imidazol-4-yl)sulfonyl]-4-phénylpyrrolidin-3-yl}-2-(trifluorométhyl)benzamide ;
N-{trans-1-[(1-méthyl-1H-imidazol-4-yl)sulfonyl]-4-phénylpyrrolidin-3-yl}-3-(trifluorométhyl)benzamide ;
N-{trans-1-[(1-méthyl-1H-imidazol-4-yl)sulfonyl]-4-phénylpyrrolidin-3-yl}-4-(trifluorométhyl)benzamide ;
2-chloro-N-[trans-1-{[1-méthyl-3-(trifluorométhyl)-1H-pyrazol-4-yl]sulfonyl}-4-phénylpyrrolidin-3-yl]-3-(trifluorométhyl)benzamide ;
2-chloro-N-[trans-1-{[1-(difluorométhyl)-3,5-diméthyl-1H-pyrazol-4-yl]sulfonyl}-4-phénylpyrrolidin-3-yl]-3-(trifluorométhyl)benzamide ;
2-chloro-N-{trans-1-[(5-chloro-1,3-diméthyl-1H-pyrazol-4-yl)sulfonyl]-4-phénylpyrrolidin-3-yl}-3-(trifluorométhyl)benzamide ;
2-chloro-N-{trans-1-[(1,5-diméthyl-1H-pyrazol-4-yl)sulfonyl]-4-phénylpyrrolidin-3-yl}-3-(trifluorométhyl)benzamide ;
2-chloro-N-{trans-1-[(1,3-diméthyl-1H-pyrazol-4-yl)sulfonyl]-4-phénylpyrrolidin-3-yl}-3-(trifluorométhyl)benzamide ;
2-chloro-N-{trans-1-[(1-méthyl-1H-pyrazol-4-yl)sulfonyl]-4-phénylpyrrolidin-3-yl}-3-(trifluorométhyl)benzamide ;
2-chloro-N-[trans-4-phényl-1-(1H-pyrazol-4-ylsulfonyl)pyrrolidin-3-yl]-3-(trifluorométhyl)benzamide ;
2-chloro-N-{trans-1-[(1-méthyl-1H-pyrazol-5-yl)sulfonyl]-4-phénylpyrrolidin-3-yl}-3-(trifluorométhyl)benzamide ;
2-chloro-N-{trans-1-[(5-chloro-1-méthyl-1H-imidazol-4-yl)sulfonyl]-4-phénylpyrrolidin-3-yl}-3-(trifluorométhyl)benzamide ;
2-chloro-N-{trans-1-[(1-méthyl-1H-pyrazol-3-yl)sulfonyl]-4-phénylpyrrolidin-3-yl}-3-(trifluorométhyl)benzamide ;
trans-N-(4-méthoxyphényl)-1-[(1-méthyl-1H-imidazol-4-yl)sulfonyl]-4-phénylpyrrolidin-3-amine ;
3-({trans-1-[(1-méthyl-1H-imidazol-4-yl)sulfonyl]-4-phénylpyrrolidin-3-yl}amino)benzonitrile ;
4-({trans-1-[(1-méthyl-1H-imidazol-4-yl)sulfonyl]-4-phénylpyrrolidin-3-yl}amino)benzonitrile ;
3-fluoro-4-({trans-1-[(1-méthyl-1H-imidazol-4-yl)sulfonyl]-4-phénylpyrrolidin-3-yl}amino)benzonitrile ;
2-chloro-N-{trans-4-(2-fluorophényl)-1-[(1-méthyl-1H-imidazol-4-yl)sulfonyl]pyrrolidin-3-yl}-3-(trifluorométhyl)benzamide ;
2-chloro-N-{trans-4-(3-fluorophényl)-1-[(1-méthyl-1H-imidazol-4-yl)sulfonyl]pyrrolidin-3-yl}-3-(trifluorométhyl)benzamide ;
2-chloro-N-{trans-4-(3-chlorophényl)-1-[(1-méthyl-1H-imidazol-4-yl)sulfonyl]pyrrolidin-3-yl}-3-(trifluorométhyl)benzamide ;
trans-1-[(1-méthyl-1H-imidazol-4-yl)sulfonyl]-N-(2-méthylphényl)-4-phénylpyrrolidin-3-amine ;
trans-1-[(1-méthyl-1H-imidazol-4-yl)sulfonyl]-N-(3-méthylphényl)-4-phénylpyrrolidin-3-amine ;
trans-1-[(1-méthyl-1H-imidazol-4-yl)sulfonyl]-N-(4-méthylphényl)-4-phénylpyrrolidin-3-amine ;
2-chloro-N-{trans-1-[(1-méthyl-1H-imidazol-4-yl)sulfonyl]-4-phénylpyrrolidin-3-yl}-4-(trifluorométhyl)benzamide ;
3-chloro-N-{trans-1-[(1-méthyl-1H-imidazol-4-yl)sulfonyl]-4-phénylpyrrolidin-3-yl}-4-(trifluorométhyl)benzamide ;
2-chloro-N-{trans-4-(4-fluorophényl)-1-[(1-méthyl-1H-imidazol-4-yl)sulfonyl]pyrrolidin-3-yl}-4-(trifluorométhyl)benzamide ;
3-chloro-N-{trans-4-(4-fluorophényl)-1-[(1-méthyl-1H-imidazol-4-yl)sulfonyl]pyrrolidin-3-yl}-4-(trifluorométhyl)benzamide ;
N-{trans-4-(4-fluorophényl)-1-[(1-méthyl-1H-imidazol-4-yl)sulfonyl]pyrrolidin-3-yl}-4-(trifluorométhyl)benzamide ;
3,5-dichloro-N-{trans-4-(4-fluorophényl)-1-[(1-méthyl-1H-imidazol-4-yl)sulfonyl]pyrrolidin-3-yl}benzamide ;
trans-1-[(1-méthyl-1H-imidazol-4-yl)sulfonyl]-4-phényl-N-[2-(trifluorométhyl)phényl]pyrrolidin-3-amine ;
trans-1-[(1-méthyl-1H-imidazol-4-yl)sulfonyl]-4-phényl-N-[3-(trifluorométhyl)phényl]pyrrolidin-3-amine ;
trans-1-[(1-méthyl-1H-imidazol-4-yl)sulfonyl]-4-phényl-N-[4-(trifluorométhyl)phényl]pyrrolidin-3-amine ;
trans-N-(2-fluorophényl)-1-[(1-méthyl-1H-imidazol-4-yl)sulfonyl]-4-phénylpyrrolidin-3-amine ;
trans-N-(3-fluorophényl)-1-[(1-méthyl-1H-imidazol-4-yl)sulfonyl]-4-phénylpyrrolidin-3-amine ;
trans-N-(4-fluorophényl)-1-[(1-méthyl-1H-imidazol-4-yl)sulfonyl]-4-phénylpyrrolidin-3-amine ;
3-({trans-4-(4-fluorophényl)-1-[(1-méthyl-1H-imidazol-4-yl)sulfonyl]pyrrolidin-3-yl}amino)benzonitrile ;
trans-N-(2-chlorophényl)-1-[(1-méthyl-1H-imidazol-4-yl)sulfonyl]-4-phénylpyrrolidin-3-amine ;
trans-N-(3-chlorophényl)-1-[(1-méthyl-1H-imidazol-4-yl)sulfonyl]-4-phénylpyrrolidin-3-amine ;
trans-N-(4-chlorophényl)-1-[(1-méthyl-1H-imidazol-4-yl)sulfonyl]-4-phénylpyrrolidin-3-amine ;
4-fluoro-3-({trans-1-[(1-méthyl-1H-imidazol-4-yl)sulfonyl]-4-phénylpyrrolidin-3-yl}amino)benzonitrile ;
2-fluoro-3-({trans-1-[(1-méthyl-1H-imidazol-4-yl)sulfonyl]-4-phénylpyrrolidin-3-yl}amino)benzonitrile ;
3-fluoro-5-({trans-1-[(1-méthyl-1H-imidazol-4-yl)sulfonyl]-4-phénylpyrrolidin-3-yl}amino)benzonitrile ;
2-chloro-5-({trans-1-[(1-méthyl-1H-imidazol-4-yl)sulfonyl]-4-phénylpyrrolidin-3-yl}amino)benzonitrile ;
3-chloro-5-({trans-1-[(1-méthyl-1H-imidazol-4-yl)sulfonyl]-4-phénylpyrrolidin-3-yl}amino)benzonitrile ;
4-chloro-3-({trans-1-[(1-méthyl-1H-imidazol-4-yl)sulfonyl]-4-phénylpyrrolidin-3-yl}amino)benzonitrile ;
4-méthyl-3-({trans-1-[(1-méthyl-1H-imidazol-4-yl)sulfonyl]-4-phénylpyrrolidin-3-yl}amino)benzonitrile ;
2-méthyl-3-({trans-1-[(1-méthyl-1H-imidazol-4-yl)sulfonyl]-4-phénylpyrrolidin-3-yl}amino)benzonitrile ;
trans-4-(4-fluorophényl)-1-[(1-méthyl-1H-imidazol-4-yl)sulfonyl]-N-(2-méthylphényl)pyrrolidin-3-amine ;
trans-4-(4-fluorophényl)-1-[(1-méthyl-1H-imidazol-4-yl)sulfonyl]-N-(3-méthylphényl)pyrrolidin-3-amine ;
trans-4-(4-fluorophényl)-1-[(1-méthyl-1H-imidazol-4-yl)sulfonyl]-N-(4-méthylphényl)pyrrolidin-3-amine ;
trans-4-(4-fluorophényl)-1-[(1-méthyl-1H-imidazol-4-yl)sulfonyl]-N-[2-(trifluorométhyl)phényl]pyrrolidin-3-amine ;
trans-4-(4-fluorophényl)-1-[(1-méthyl-1H-imidazol-4-yl)sulfonyl]-N-[3-(trifluorométhyl)phényl]pyrrolidin-3-amine ;
trans-4-(4-fluorophényl)-1-[(1-méthyl-1H-imidazol-4-yl)sulfonyl]-N-[4-(trifluorométhyl)phényl]pyrrolidin-3-amine ;
trans-N-(2-fluorophényl)-4-(4-fluorophényl)-1-[(1-méthyl-1H-imidazol-4-yl)sulfonyl]pyrrolidin-3-amine ;
trans-N-(3-fluorophényl)-4-(4-fluorophényl)-1-[(1-méthyl-1H-imidazol-4-yl)sulfonyl]pyrrolidin-3-amine ;
trans-N,4-bis(4-fluorophényl)-1-[(1-méthyl-1H-imidazol-4-yl)sulfonyl]pyrrolidin-3-amine ;
trans-N-(2-chlorophényl)-4-(4-fluorophényl)-1-[(1-méthyl-1H-imidazol-4-yl)sulfonyl]pyrrolidin-3-amine ;
trans-N-(3-chlorophényl)-4-(4-fluorophényl)-1-[(1-méthyl-1H-imidazol-4-yl)sulfonyl]pyrrolidin-3-amine ;
trans-N-(4-chlorophényl)-4-(4-fluorophényl)-1-[(1-méthyl-1H-imidazol-4-yl)sulfonyl]pyrrolidin-3-amine ;
2-méthoxy-5-({trans-1-[(1-méthyl-1H-imidazol-4-yl)sulfonyl]-4-phénylpyrrolidin-3-yl}amino)benzonitrile ;
3-méthyl-5-({trans-1-[(1-méthyl-1H-imidazol-4-yl)sulfonyl]-4-phénylpyrrolidin-3-yl}amino)benzonitrile ;
5-({trans-1-[(1-méthyl-1H-imidazol-4-yl)sulfonyl]-4-phénylpyrrolidin-3-yl}amino)-2-(trifluorométhoxy)benzonitrile ;
2-chloro-N-{trans-4-(2-chlorophényl)-1-[(1-méthyl-1H-imidazol-4-yl)sulfonyl]pyrrolidin-3-yl}-3-(trifluorométhyl)benzamide ;
4-{[trans-3-benzyl-4-phénylpyrrolidin-1-yl]sulfonyl}-1-méthyl-1H-imidazole ;
trans-N-benzyl-4-(4-fluorophényl)-1-[(1-méthyl-1H-imidazol-4-yl)sulfonyl]pyrrolidin-3-amine ;
trans-N-[2-chloro-3-(trifluorométhyl)benzyl]-4-(4-fluorophényl)-1-[(1-méthyl-1H-imidazol-4-yl)sulfonyl]pyrrolidin-3-amine ;
trans-N-[2-chloro-4-(trifluorométhyl)benzyl]-4-(4-fluorophényl)-1-[(1-méthyl-1H-imidazol-4-yl)sulfonyl]pyrrolidin-3-amine ;
trans-N-[3-chloro-4-(trifluorométhyl)benzyl]-4-(4-fluorophényl)-1-[(1-méthyl-1H-imidazol-4-yl)sulfonyl]pyrrolidin-3-amine ;
3-[({trans-4-(4-fluorophényl)-1-[(1-méthyl-1H-imidazol-4-yl)sulfonyl]pyrrolidin-3-yl}amino)méthyl]benzonitrile ;
trans-4-(4-fluorophényl)-N-(2-méthylbenzyl)-1-[(1-méthyl-1H-imidazol-4-yl)sulfonyl]pyrrolidin-3-amine ;
trans-4-(4-fluorophényl)-N-(3-méthylbenzyl)-1-[(1-méthyl-1H-imidazol-4-yl)sulfonyl]pyrrolidin-3-amine ;
trans-4-(4-fluorophényl)-N-(4-méthylbenzyl)-1-[(1-méthyl-1H-imidazol-4-yl)sulfonyl]pyrrolidin-3-amine ;
trans-N-(4-fluorobenzyl)-4-(4-fluorophényl)-1-[(1-méthyl-1H-imidazol-4-yl)sulfonyl]pyrrolidin-3-amine ; (cis)-N-benzyl-1-[(1-méthyl-1H-imidazol-4-yl)sulfonyl]-4-phénylpyrrolidin-3-amine ;
trans-N-(3,4-difluorophényl)-4-(4-fluorophényl)-1-[(1-méthyl-1H-imidazol-4-yl)sulfonyl]pyrrolidin-3-amine ;
trans-4-(4-fluorophényl)-N-[4-fluoro-3-(trifluorométhyl)phényl]-1-[(1-méthyl-1H-imidazol-4-yl)sulfonyl]pyrrolidin-3-amine ;
trans-N-(3-chloro-4-fluorophényl)-4-(4-fluorophényl)-1-[(1-méthyl-1H-imidazol-4-yl)sulfonyl]pyrrolidin-3-amine ;
trans-N-(3,4-dichlorophényl)-4-(4-fluorophényl)-1-[(1-méthyl-1H-imidazol-4-yl)sulfonyl]pyrrolidin-3-amine ;
trans-N-(4-chloro-3-fluorophényl)-4-(4-fluorophényl)-1-[(1-méthyl-1H-imidazol-4-yl)sulfonyl]pyrrolidin-3-amine ;
trans-N-(2,3-difluorophényl)-4-(4-fluorophényl)-1-[(1-méthyl-1H-imidazol-4-yl)sulfonyl]pyrrolidin-3-amine ;
trans-N-(2,5-difluorophényl)-4-(4-fluorophényl)-1-[(1-méthyl-1H-imidazol-4-yl)sulfonyl]pyrrolidin-3-amine ;
trans-N-(2,4-difluorophényl)-4-(4-fluorophényl)-1-[(1-méthyl-1H-imidazol-4-yl)sulfonyl]pyrrolidin-3-amine ;
trans-4-(4-fluorophényl)-1-[(1-méthyl-1H-imidazol-4-yl)sulfonyl]-N-(2,3,4-trifluorophényl)pyrrolidin-3-amine ;
trans-4-(4-fluorophényl)-N-[2-fluoro-3-(trifluorométhyl)phényl]-1-[(1-méthyl-1H-imidazol-4-yl)sulfonyl]pyrrolidin-3-amine ;
trans-4-(4-fluorophényl)-N-[3-fluoro-5-(trifluorométhyl)phényl]-1-[(1-méthyl-1H-imidazol-4-yl)sulfonyl]pyrrolidin-3-amine ;
trans-4-(4-fluorophényl)-N-[2-fluoro-5-(trifluorométhyl)phényl]-1-[(1-méthyl-1H-imidazol-4-yl)sulfonyl]pyrrolidin-3-amine ;
trans-4-(4-fluorophényl)-1-[(1-méthyl-1H-imidazol-4-yl)sulfonyl]-N-[3-(propan-2-yl)phényl]pyrrolidin-3-amine ;
trans-N-(3-tert-butylphényl)-4-(4-fluorophényl)-1-[(1-méthyl-1H-imidazol-4-yl)sulfonyl]pyrrolidin-3-amine ;
trans-N-[4-chloro-3-(trifluorométhyl)phényl]-4-(4-fluorophényl)-1-[(1-méthyl-1H-imidazol-4-yl)sulfonyl]pyrrolidin-3-amine ;
trans-N-[3-chloro-4-(trifluorométhyl)phényl]-4-(4-fluorophényl)-1-[(1-méthyl-1H-imidazol-4-yl)sulfonyl]pyrrolidin-3-amine ;
trans-N-(3-chloro-5-fluorophényl)-4-(4-fluorophényl)-1-[(1-méthyl-1H-imidazol-4-yl)sulfonyl]pyrrolidin-3-amine ;
trans-N-(3-chloro-4,5-difluorophényl)-4-(4-fluorophényl)-1-[(1-méthyl-1H-imidazol-4-yl)sulfonyl]pyrrolidin-3-amine ;
trans-4-(4-fluorophényl)-1-[(1-méthyl-1H-imidazol-4-yl)sulfonyl]-N-[3-(trifluorométhoxy)phényl]pyrrolidin-3-amine ;
N-{trans-4-(4-fluorophényl)-1-[(1-méthyl-1H-imidazol-4-yl)sulfonyl]pyrrolidin-3-yl}pyridin-4-amine ;
N-{trans-4-(4-fluorophényl)-1-[(1-méthyl-1H-imidazol-4-yl)sulfonyl]pyrrolidin-3-yl}pyridin-2-amine ;
N-{trans-4-(4-fluorophényl)-1-[(1-méthyl-1H-imidazol-4-yl)sulfonyl]pyrrolidin-3-yl}pyridin-3-amine ;
N-{trans-4-(4-fluorophényl)-1-[(1-méthyl-1H-imidazol-4-yl)sulfonyl]pyrrolidin-3-yl}-6-(trifluorométhyl)pyridin-2-amine ;
N-{trans-4-(4-fluorophényl)-1-[(1-méthyl-1H-imidazol-4-yl)sulfonyl]pyrrolidin-3-yl}-5-(trifluorométhyl)pyridin-3-amine ;
4-{[3-(3-chlorobenzyl)-4-(4-fluorophényl)pyrrolidin-1-yl]sulfonyl}-1-méthyl-1H-imidazole ;
2-chloro-N-{(cis)-1-[(1-méthyl-1H-imidazol-4-yl)sulfonyl]-4-phénylpyrrolidin-3-yl}-3-(trifluorométhyl)benzamide ;
2-chloro-N-{(cis)-1-[(1-méthyl-1H-imidazol-4-yl)sulfonyl]-4-phénylpyrrolidin-3-yl}-3-(trifluorométhyl)benzamide ;
trans-4-(3-fluorophényl)-1-[(1-méthyl-1H-imidazol-4-yl)sulfonyl]-N-phénylpyrrolidin-3-amine ;
trans-N,4-bis(3-fluorophényl)-1-[(1-méthyl-1H-imidazol-4-yl)sulfonyl]pyrrolidin-3-amine ;
trans-4-(3-fluorophényl)-N-(4-fluorophényl)-1-[(1-méthyl-1H-imidazol-4-yl)sulfonyl]pyrrolidin-3-amine ;
trans-N-(3-chlorophényl)-4-(3-fluorophényl)-1-[(1-méthyl-1H-imidazol-4-yl)sulfonyl]pyrrolidin-3-amine ;
trans-4-(3-fluorophényl)-1-[(1-méthyl-1H-imidazol-4-yl)sulfonyl]-N-[3-(trifluorométhyl)phényl]pyrrolidin-3-amine ;
trans-4-(3-fluorophényl)-1-[(1-méthyl-1H-imidazol-4-yl)sulfonyl]-N-[4-(trifluorométhyl)phényl]pyrrolidin-3-amine ;
N-{trans-4-(3-fluorophényl)-1-[(1-méthyl-1H-imidazol-4-yl)sulfonyl]pyrrolidin-3-yl}pyridin-4-amine ;
trans-N-(3,4-dichlorophényl)-4-(3-fluorophényl)-1-[(1-méthyl-1H-imidazol-4-yl)sulfonyl]pyrrolidin-3-amine ;
trans-N-(4-chloro-3-fluorophényl)-4-(3-fluorophényl)-1-[(1-méthyl-1H-imidazol-4-yl)sulfonyl]pyrrolidin-3-amine ;
trans-N-(3-chloro-4-fluorophényl)-4-(3-fluorophényl)-1-[(1-méthyl-1H-imidazol-4-yl)sulfonyl]pyrrolidin-3-amine ;
N-{trans-4-(3-fluorophényl)-1-[(1-méthyl-1H-imidazol-4-yl)sulfonyl]pyrrolidin-3-yl}-6-(trifluorométhyl)pyridin-2-amine ;
N-{trans-4-(3-fluorophényl)-1-[(1-méthyl-1H-imidazol-4-yl)sulfonyl]pyrrolidin-3-yl}-5-(trifluorométhyl)pyridin-3-amine ;
trans-N-(3,4-difluorophényl)-4-(3-fluorophényl)-1-[(1-méthyl-1H-imidazol-4-yl)sulfonyl]pyrrolidin-3-amine ;
trans-4-(2-fluorophényl)-1-[(1-méthyl-1H-imidazol-4-yl)sulfonyl]-N-phénylpyrrolidin-3-amine ;
trans-4-(2-fluorophényl)-N-(3-fluorophényl)-1-[(1-méthyl-1H-imidazol-4-yl)sulfonyl]pyrrolidin-3-amine ;
trans-4-(2-fluorophényl)-N-(4-fluorophényl)-1-[(1-méthyl-1H-imidazol-4-yl)sulfonyl]pyrrolidin-3-amine ;
trans-N-(3-chlorophényl)-4-(2-fluorophényl)-1-[(1-méthyl-1H-imidazol-4-yl)sulfonyl]pyrrolidin-3-amine ;
trans-N-(4-chlorophényl)-4-(2-fluorophényl)-1-[(1-méthyl-1H-imidazol-4-yl)sulfonyl]pyrrolidin-3-amine ;
N-{trans-4-(2-fluorophényl)-1-[(1-méthyl-1H-imidazol-4-yl)sulfonyl]pyrrolidin-3-yl}-6-(trifluorométhyl)pyridin-2-amine ;
N-{trans-4-(2-fluorophényl)-1-[(1-méthyl-1H-imidazol-4-yl)sulfonyl]pyrrolidin-3-yl}-2-(trifluorométhyl)pyridin-4-amine ;
4-{[3-(2-bromobenzyl)-4-(2-bromophényl)pyrrolidin-1-yl]sulfonyl}-1-méthyl-1H-imidazole ;
{trans-4-(4-fluorophényl)-1-[(1-méthyl-1H-imidazol-4-yl)sulfonyl]pyrrolidin-3-yl}méthanol ;
N-{trans-1-[(1-méthyl-1H-imidazol-4-yl)sulfonyl]-4-phénylpyrrolidin-3-yl}pyridin-2-amine ;
N-{trans-1-[(1-méthyl-1H-imidazol-4-yl)sulfonyl]-4-phénylpyrrolidin-3-yl}pyridin-3-amine ;
N-{trans-1-[(1-méthyl-1H-imidazol-4-yl)sulfonyl]-4-phénylpyrrolidin-3-yl}pyridin-4-amine ;
N-{trans-1-[(1-méthyl-1H-imidazol-4-yl)sulfonyl]-4-phénylpyrrolidin-3-yl}-6-(trifluorométhyl)pyridin-2-amine ;
N-{trans-1-[(1-méthyl-1H-imidazol-4-yl)sulfonyl]-4-phénylpyrrolidin-3-yl}-2-(trifluorométhyl)pyridin-4-amine ;
N-{trans-1-[(1-méthyl-1H-imidazol-4-yl)sulfonyl]-4-phénylpyrrolidin-3-yl}-4-(trifluorométhyl)pyridin-2-amine ;
N-{trans-1-[(1-méthyl-1H-imidazol-4-yl)sulfonyl]-4-phénylpyrrolidin-3-yl}-5-(trifluorométhyl)pyridin-3-amine ;
trans-N-[4-fluoro-3-(trifluorométhyl)phényl]-1-[(1-méthyl-1H-imidazol-4-yl)sulfonyl]-4-phénylpyrrolidin-3-amine ;
trans-N-(3-chloro-4-fluorophényl)-1-[(1-méthyl-1H-imidazol-4-yl)sulfonyl]-4-phénylpyrrolidin-3-amine ;
trans-N-(3,4-difluorophényl)-1-[(1-méthyl-1H-imidazol-4-yl)sulfonyl]-4-phénylpyrrolidin-3-amine ;
trans-N-[4-chloro-3-(trifluorométhyl)phényl]-1-[(1-méthyl-1H-imidazol-4-yl)sulfonyl]-4-phénylpyrrolidin-3-amine ;
trans-N-(3,4-dichlorophényl)-1-[(1-méthyl-1H-imidazol-4-yl)sulfonyl]-4-phénylpyrrolidin-3-amine ;
trans-N-(3-chloro-4,5-difluorophényl)-1-[(1-méthyl-1H-imidazol-4-yl)sulfonyl]-4-phénylpyrrolidin-3-amine ;
trans-1-[(1-méthyl-1H-imidazol-4-yl)sulfonyl]-4-phényl-N-[3-(propan-2-yl)phényl]pyrrolidin-3-amine ;
trans-N-(3-tert-butylphényl)-1-[(1-méthyl-1H-imidazol-4-yl)sulfonyl]-4-phénylpyrrolidin-3-amine ;
trans-N-[2-fluoro-3-(trifluorométhyl)phényl]-1-[(1-méthyl-1H-imidazol-4-yl)sulfonyl]-4-phénylpyrrolidin-3-amine ;
trans-N-[3-fluoro-5-(trifluorométhyl)phényl]-1-[(1-méthyl-1H-imidazol-4-yl)sulfonyl]-4-phénylpyrrolidin-3-amine ;
trans-N-[2-fluoro-5-(trifluorométhyl)phényl]-1-[(1-méthyl-1H-imidazol-4-yl)sulfonyl]-4-phénylpyrrolidin-3-amine ;
trans-N-(3-chloro-5-fluorophényl)-1-[(1-méthyl-1H-imidazol-4-yl)sulfonyl]-4-phénylpyrrolidin-3-amine ;
trans-1-[(1-méthyl-1H-imidazol-4-yl)sulfonyl]-4-phényl-N-[3-(trifluorométhoxy)phényl]pyrrolidin-3-amine ;
N-{trans-1-[(1-méthyl-1H-imidazol-4-yl)sulfonyl]-4-phénylpyrrolidin-3-yl}-4-(trifluorométhyl)pyrimidin-2-amine ;
N-{trans-1-[(1-méthyl-1H-imidazol-4-yl)sulfonyl]-4-phénylpyrrolidin-3-yl}quinoléin-7-amine ;
N-{trans-1-[(1-méthyl-1H-imidazol-4-yl)sulfonyl]-4-phénylpyrrolidin-3-yl}quinoléin-6-amine ;
N-{trans-1-[(1-méthyl-1H-imidazol-4-yl)sulfonyl]-4-phénylpyrrolidin-3-yl}isoquinoléin-6-amine ;
trans-N-(2,3-difluorophényl)-1-[(1-méthyl-1H-imidazol-4-yl)sulfonyl]-4-phénylpyrrolidin-3-amine ;
trans-N-(2,5-difluorophényl)-1-[(1-méthyl-1H-imidazol-4-yl)sulfonyl]-4-phénylpyrrolidin-3-amine ;
rans-N-(2,4-difluorophényl)-1-[(1-méthyl-1H-imidazol-4-yl)sulfonyl]-4-phénylpyrrolidin-3-amine ;
trans-1-[(1-méthyl-1H-imidazol-4-yl)sulfonyl]-4-phényl-N-(2,3,4-trifluorophényl)pyrrolidin-3-amine ;
6-fluoro-N-{trans-1-[(1-méthyl-1H-imidazol-4-yl)sulfonyl]-4-phénylpyrrolidin-3-yl}pyridin-2-amine ;
2-fluoro-N-{trans-1-[(1-méthyl-1H-imidazol-4-yl)sulfonyl]-4-phénylpyrrolidin-3-yl}pyridin-4-amine ;
5-fluoro-N-{trans-1-[(1-méthyl-1H-imidazol-4-yl)sulfonyl]-4-phénylpyrrolidin-3-yl}pyridin-3-amine ;
6-fluoro-N-{trans-4-(4-fluorophényl)-1-[(1-méthyl-1H-imidazol-4-yl)sulfonyl]pyrrolidin-3-yl}pyridin-2-amine ;
2-fluoro-N-{trans-4-(4-fluorophényl)-1-[(1-méthyl-1H-imidazol-4-yl)sulfonyl]pyrrolidin-3-yl}pyridin-4-amine ;
N-{trans-1-[(1-méthyl-1H-imidazol-4-yl)sulfonyl]-4-phénylpyrrolidin-3-yl}-6-(trifluorométhyl)pyrimidin-4-amine ;
N-{trans-1-[(1-méthyl-1H-imidazol-4-yl)sulfonyl]-4-phénylpyrrolidin-3-yl}isoquinoléin-7-amine ;
trans-4-(4-fluorophényl)-1-[(1-méthyl-1H-imidazol-4-yl)sulfonyl]-N-phénylpyrrolidine-3-carboxamide ;
trans-4-(4-fluorophényl)-1-[(1-méthyl-1H-imidazol-4-yl)sulfonyl]-N-[4-(trifluorométhyl)phényl]pyrrolidine-3-carboxamide ;
trans-N-(3,5-dichlorophényl)-4-(4-fluorophényl)-1-[(1-méthyl-1H-imidazol-4-yl)sulfonyl]pyrrolidine-3-carboxamide ;
4-{[trans-3-(4-fluorophényl)-4-(phénoxyméthyl)pyrrolidin-1-yl]sulfonyl}-1-méthyl-1H-imidazole ;
4-{[trans-3-[(2,4-dichlorophénoxy)méthyl]-4-(4-fluorophényl)pyrrolidin-1-yl]sulfonyl}-1-méthyl-1H-imidazole ;
4-{[trans-3-(4-fluorophényl)-4-{[3-(trifluorométhoxy)phénoxy]méthyl}pyrrolidin-1-yl]sulfonyl}-1-méthyl-1H-imidazole ;
4-{[trans-3-[(3-chlorophénoxy)méthyl]-4-(4-fluorophényl)pyrrolidin-1-yl]sulfonyl}-1-méthyl-1H-imidazole ;
4-{[trans-3-{[4-chloro-3-(trifluorométhyl)phénoxy]méthyl}-4-(4-fluorophényl)pyrrolidin-1-yl]sulfonyl}-1-méthyl-1H-imidazole ;
4-{[trans-3-{[2-chloro-3-(trifluorométhyl)phénoxy]méthyl}-4-(4-fluorophényl)pyrrolidin-1-yl]sulfonyl}-1-méthyl-1H-imidazole ;
4-{[trans-3-[(3-fluorophénoxy)méthyl]-4-(4-fluorophényl)pyrrolidin-1-yl]sulfonyl}-1-méthyl-1H-imidazole ;
trans-4-(2-chlorophényl)-N-(3-fluorophényl)-1-[(1-méthyl-1H-imidazol-4-yl)sulfonyl]pyrrolidin-3-amine ;
trans-4-(2-chlorophényl)-N-(4-fluorophényl)-1-[(1-méthyl-1H-imidazol-4-yl)sulfonyl]pyrrolidin-3-amine ;
trans-4-(2-chlorophényl)-N-(4-chlorophényl)-1-[(1-méthyl-1H-imidazol-4-yl)sulfonyl]pyrrolidin-3-amine ;
trans-4-(2-chlorophényl)-1-[(1-méthyl-1H-imidazol-4-yl)sulfonyl]-N-[3-(trifluorométhyl)phényl]pyrrolidin-3-amine ;
trans-4-(2-chlorophényl)-1-[(1-méthyl-1H-imidazol-4-yl)sulfonyl]-N-[4-(trifluorométhyl)phényl]pyrrolidin-3-amine ;
trans-N-(3-chloro-4-fluorophényl)-4-(2-chlorophényl)-1-[(1-méthyl-1H-imidazol-4-yl)sulfonyl]pyrrolidin-3-amine ;
trans-4-(2-chlorophényl)-N-[4-fluoro-3-(trifluorométhyl)phényl]-1-[(1-méthyl-1H-imidazol-4-yl)sulfonyl]pyrrolidin-3-amine ;
N-{trans-4-(2-chlorophényl)-1-[(1-méthyl-1H-imidazol-4-yl)sulfonyl]pyrrolidin-3-yl}-2-(trifluorométhyl)pyridin-4-amine ;
trans-N-(2-chlorobenzyl)-4-(4-fluorophényl)-1-[(1-méthyl-1H-imidazol-4-yl)sulfonyl]pyrrolidin-3-amine ;
trans-N-(3-chlorobenzyl)-4-(4-fluorophényl)-1-[(1-méthyl-1H-imidazol-4-yl)sulfonyl]pyrrolidin-3-amine ;
trans-N-(4-chlorobenzyl)-4-(4-fluorophényl)-1-[(1-méthyl-1H-imidazol-4-yl)sulfonyl]pyrrolidin-3-amine ;
trans-N-(2-fluorobenzyl)-4-(4-fluorophényl)-1-[(1-méthyl-1H-imidazol-4-yl)sulfonyl]pyrrolidin-3-amine ;
trans-N-(3-fluorobenzyl)-4-(4-fluorophényl)-1-[(1-méthyl-1H-imidazol-4-yl)sulfonyl]pyrrolidin-3-amine ;
trans-4-(4-fluorophényl)-1-[(1-méthyl-1H-imidazol-4-yl)sulfonyl]-N-[2-(trifluorométhyl)benzyl]pyrrolidin-3-amine ;
trans-4-(4-fluorophényl)-1-[(1-méthyl-1H-imidazol-4-yl)sulfonyl]-N-[3-(trifluorométhyl)benzyl]pyrrolidin-3-amine ;
trans-4-(4-fluorophényl)-1-[(1-méthyl-1H-imidazol-4-yl)sulfonyl]-N-[4-(trifluorométhyl)benzyl]pyrrolidin-3-amine ;
trans-4-(4-fluorophényl)-1-[(1-méthyl-1H-imidazol-4-yl)sulfonyl]-N-(pyridin-2-ylméthyl)pyrrolidin-3-amine ;
trans-4-(4-fluorophényl)-1-[(1-méthyl-1H-imidazol-4-yl)sulfonyl]-N-(pyridin-3-ylméthyl)pyrrolidin-3-amine ;
trans-4-(4-fluorophényl)-1-[(1-méthyl-1H-imidazol-4-yl)sulfonyl]-N-(pyridin-4-ylméthyl)pyrrolidin-3-amine ;
{trans-4-(4-fluorophényl)-1-[(1-méthyl-1H-imidazol-4-yl)sulfonyl]pyrrolidin-3-yl}(phényl)méthanone ;
trans-N-[2-chloro-3-(trifluorométhyl)phényl]-4-(4-fluorophényl)-1-[(1-méthyl-1H-imidazol-4-yl)sulfonyl]pyrrolidine-3-carboxamide ;
trans-4-(4-fluorophényl)-1-[(1-méthyl-1H-imidazol-4-yl)sulfonyl]-N-[3-(trifluorométhoxy)phényl]pyrrolidine-3-carboxamide ;
trans-4-(4-fluorophényl)-N-[4-fluoro-3-(trifluorométhyl)phényl]-1-[(1-méthyl-1H-imidazol-4-yl)sulfonyl]pyrrolidine-3-carboxamide ;
trans-N-(3-chloro-4-fluorophényl)-4-(4-fluorophényl)-1-[(1-méthyl-1H-imidazol-4-yl)sulfonyl]pyrrolidine-3-carboxamide ;
trans-N-[3-chloro-4-(trifluorométhyl)phényl]-4-(4-fluorophényl)-1-[(1-méthyl-1H-imidazol-4-yl)sulfonyl]pyrrolidine-3-carboxamide ;
1-méthyl-4-[(3-phénoxy-4-phénylpyrrolidin-1-yl)sulfonyl]-1H-imidazole ;
trans-4-(2-chlorophényl)-N-(3-chlorophényl)-1-[(1-méthyl-1H-imidazol-4-yl)sulfonyl]pyrrolidin-3-amine ;
trans-N-(2,4-dichlorobenzyl)-4-(4-fluorophényl)-1-[(1-méthyl-1H-imidazol-4-yl)sulfonyl]pyrrolidin-3-amine ;
trans-N-(2,4-dichlorobenzyl)-1-[(1-méthyl-1H-imidazol-4-yl)sulfonyl]-4-phénylpyrrolidin-3-amine ;
trans-N-[2-chloro-4-(trifluorométhyl)benzyl]-1-[(1-méthyl-1H-imidazol-4-yl)sulfonyl]-4-phénylpyrrolidin-3-amine ;
trans-N-(2,4-dichlorobenzyl)-4-(2-fluorophényl)-1-[(1-méthyl-1H-imidazol-4-yl)sulfonyl]pyrrolidin-3-amine ;
trans-N-[2-chloro-4-(trifluorométhyl)benzyl]-4-(2-fluorophényl)-1-[(1-méthyl-1H-imidazol-4-yl)sulfonyl]pyrrolidin-3-amine ;
trans-4-(2-chlorophényl)-N-(2,4-dichlorobenzyl)-1-[(1-méthyl-1H-imidazol-4-yl)sulfonyl]pyrrolidin-3-amine ;
trans-4-(2-chlorophényl)-N-[2-chloro-4-(trifluorométhyl)benzyl]-1-[(1-méthyl-1H-imidazol-4-yl)sulfonyl]pyrrolidin-3-amine ;
trans-N-(2,4-dichlorobenzyl)-1-[(1-méthyl-1H-imidazol-4-yl)sulfonyl]-4-(pyridin-3-yl)pyrrolidin-3-amine ;
N-{trans-4-(4-fluorophényl)-1-[(1-méthyl-1H-imidazol-4-yl)sulfonyl]pyrrolidin-3-yl}-4-(trifluorométhyl)pyridin-2-amine ;
N-{trans-4-(4-fluorophényl)-1-[(1-méthyl-1H-imidazol-4-yl)sulfonyl]pyrrolidin-3-yl}-2-(trifluorométhyl)pyridin-4-amine ;
trans-N-benzyl-4-(4-fluorophényl)-1-[(1-méthyl-1H-imidazol-4-yl)sulfonyl]pyrrolidine-3-carboxamide ;
N-({trans-4-(4-fluorophényl)-1-[(1-méthyl-1H-imidazol-4-yl)sulfonyl]pyrrolidin-3-yl}méthyl)aniline ;
N-({trans-4-(4-fluorophényl)-1-[(1-méthyl-1H-imidazol-4-yl)sulfonyl]pyrrolidin-3-yl}méthyl)-4-(trifluorométhyl)aniline ;
3,5-dichloro-N-({trans-4-(4-fluorophényl)-1-[(1-méthyl-1H-imidazol-4-yl)sulfonyl]pyrrolidin-3-yl}méthyl)aniline ;
4-{[trans-3-(4-fluorophényl)-4-{[3-(trifluorométhyl)phénoxy]méthyl}pyrrolidin-1-yl]sulfonyl}-1-méthyl-1H-imidazole ;
4-{[trans-3-{[2-chloro-4-(trifluorométhyl)phénoxy]méthyl}-4-(4-fluorophényl)pyrrolidin-1-yl]sulfonyl}-1-méthyl-1H-imidazole ;
4-{[trans-3-(4-fluorophényl)-4-{[4-fluoro-3-(trifluorométhyl)phénoxy]méthyl}pyrrolidin-1-yl]sulfonyl}-1-méthyl-1H-imidazole ;
4-({trans-3-(4-fluorophényl)-4-[(3-méthylphénoxy)méthyl]pyrrolidin-1-yl}sulfonyl)-1-méthyl-1H-imidazole ;
3-chlorophényl){trans-4-(4-fluorophényl)-1-[(1-méthyl-1H-imidazol-4-yl)sulfonyl]pyrrolidin-3-yl}méthanone ;
trans-N-[4-fluoro-3-(trifluorométhoxy)phényl]-1-[(1-méthyl-1H-imidazol-4-yl)sulfonyl]-4-phénylpyrrolidin-3-amine ;
trans-N-[4-chloro-3-(trifluorométhoxy)phényl]-1-[(1-méthyl-1H-imidazol-4-yl)sulfonyl]-4-phénylpyrrolidin-3-amine ;
{trans-4-(4-fluorophényl)-1-[(1-méthyl-1H-imidazol-4-yl)sulfonyl]pyrrolidin-3-yl}(3-méthylphényl)méthanone ;
(3-fluorophényl){(trans-4-(4-fluorophényl)-1-[(1-méthyl-1H-imidazol-4-yl)sulfonyl]pyrrolidin-3-yl}méthanone ;
{trans-4-(4-fluorophényl)-1-[(1-méthyl-1H-imidazol-4-yl)sulfonyl]pyrrolidin-3-yl}[3-(trifluorométhyl)phényl]méthanone ;
(4-fluorophényl){trans-4-(4-fluorophényl)-1-[(1-méthyl-1H-imidazol-4-yl)sulfonyl]pyrrolidin-3-yl}méthanone ;
trans-N-(3-chlorophényl)-4-(4-fluorophényl)-1-[(1-méthyl-1H-imidazol-4-yl)sulfonyl]pyrrolidine-3-carboxamide ;
trans-4-(4-fluorophényl)-N-[4-fluoro-3-(trifluorométhoxy)phényl]-1-[(1-méthyl-1H-imidazol-4-yl)sulfonyl]pyrrolidin-3-amine ;
trans-N-[4-chloro-3-(trifluorométhoxy)phényl]-4-(4-fluorophényl)-1-[(1-méthyl-1H-imidazol-4-yl)sulfonyl]pyrrolidin-3-amine ;
trans-N-(2,4-dichlorobenzyl)-1-[(1-méthyl-1H-imidazol-4-yl)sulfonyl]-4-(pyridin-2-yl)pyrrolidin-3-amine ;
trans-1-[(1-méthyl-1H-imidazol-4-yl)sulfonyl]-4-(pyridin-3-yl)-N-[3-(trifluorométhyl)phényl]pyrrolidin-3-amine ;
4-{[3-(3-chlorobenzyl)-4-cyclopropylpyrrolidin-1-yl]sulfonyl}-1-méthyl-1H-imidazole ;
N-[3-(difluorométhyl)-4-fluorophényl]-4-(4-fluorophényl)-1-[(1-méthyl-1H-imidazol-4-yl)sulfonyl]pyrrolidin-3-amine ;
3-chloro-N-({trans-4-(4-fluorophényl)-1-[(1-méthyl-1H-imidazol-4-yl)sulfonyl]pyrrolidin-3-yl}méthyl)-4-(trifluorométhyl)aniline ;
N-({trans-4-(4-fluorophényl)-1-[(1-méthyl-1H-imidazol-4-yl)sulfonyl]pyrrolidin-3-yl}méthyl)-3-(trifluorométhoxy)aniline ;
4-fluoro-N-({trans-4-(4-fluorophényl)-1-[(1-méthyl-1H-imidazol-4-yl)sulfonyl]pyrrolidin-3-yl}méthyl)-3-(trifluorométhyl)aniline ;
3-chloro-4-fluoro-N-({trans-4-(4-fluorophényl)-1-[(1-méthyl-1H-imidazol-4-yl)sulfonyl]pyrrolidin-3-yl}méthyl)aniline ;
N-benzyl-1-{trans-4-(4-fluorophényl)-1-[(1-méthyl-1H-imidazol-4-yl)sulfonyl]pyrrolidin-3-yl}méthanamine ;
trans-4-(4-fluorophényl)-1-[(1-méthyl-1H-imidazol-4-yl)sulfonyl]-N-(3-méthylphényl)pyrrolidine-3-carboxamide ;
trans-4-(4-fluorophényl)-1-[(1-méthyl-1H-imidazol-4-yl)sulfonyl]-N-[3-(trifluorométhyl)phényl]pyrrolidine-3-carboxamide ;
4-{[trans-3-[(3-chloro-4-fluorophénoxy)méthyl]-4-(4-fluorophényl)pyrrolidin-1-yl]sulfonyl}-1-méthyl-1H-imidazole ;
(4-chloro-3-fluorophényl){trans-4-(4-fluorophényl)-1-[(1-méthyl-1H-imidazol-4-yl)sulfonyl]pyrrolidin-3-yl}méthanone ;
trans-N-[2-chloro-4-(trifluorométhyl)benzyl]-1-[(1-méthyl-1H-imidazol-4-yl)sulfonyl]-4-(pyridin-2-yl)pyrrolidin-3-amine ;
(3S,4R)-N-[2-chloro-4-(trifluorométhyl)benzyl]-4-(4-fluorophényl)-1-[(1-méthyl-1H-imidazol-4-yl)sulfonyl]pyrrolidin-3-amine ;
(3R,4S)-N-[2-chloro-4-(trifluorométhyl)benzyl]-4-(4-fluorophényl)-1-[(1-méthyl-1H-imidazol-4-yl)sulfonyl]pyrrolidin-3-amine ;
(3S,4R)-4-(4-fluorophényl)-1-[(1-méthyl-1H-imidazol-4-yl)sulfonyl]-N-[3-(trifluorométhoxy)phényl]pyrrolidin-3-amine ;
(3S,4R)-N,4-bis(4-fluorophényl)-1-[(1-méthyl-1H-imidazol-4-yl)sulfonyl]pyrrolidin-3-amine ;
(3S,4R)-N-(3-chlorophényl)-4-(4-fluorophényl)-1-[(1-méthyl-1H-imidazol-4-yl)sulfonyl]pyrrolidin-3-amine ;
(3S,4R)-4-(4-fluorophényl)-1-[(1-méthyl-1H-imidazol-4-yl)sulfonyl]-N-[3-(trifluorométhyl)phényl]pyrrolidin-3-amine ;
(3R,4S)-4-(4-fluorophényl)-1-[(1-méthyl-1H-imidazol-4-yl)sulfonyl]-N-[3-(trifluorométhyl)phényl]pyrrolidin-3-amine ;
(3R,4S)-N-(3-chlorophényl)-4-(4-fluorophényl)-1-[(1-méthyl-1H-imidazol-4-yl)sulfonyl]pyrrolidin-3-amine ;
(3R,4S)-4-(4-fluorophényl)-1-[(1-méthyl-1H-imidazol-4-yl)sulfonyl]-N-[3-(trifluorométhoxy)phényl]pyrrolidin-3-amine ;
(3R,4S)-N,4-bis(4-fluorophényl)-1-[(1-méthyl-1H-imidazol-4-yl)sulfonyl]pyrrolidin-3-amine ;
3-(3-chlorobenzyl)-1-[(1-méthyl-1H-imidazol-4-yl)sulfonyl]-4-phénylpyrrolidin-2-one ;
2-chloro-N-({trans-4-(4-fluorophényl)-1-[(1-méthyl-1H-imidazol-4-yl)sulfonyl]pyrrolidin-3-yl}méthyl)-4-(trifluorométhyl)aniline ;
3-chloro-N-({trans-4-(4-fluorophényl)-1-[(1-méthyl-1H-imidazol-4-yl)sulfonyl]pyrrolidin-3-yl}méthyl)aniline ;
N-({trans-4-(4-fluorophényl)-1-[(1-méthyl-1H-imidazol-4-yl)sulfonyl]pyrrolidin-3-yl}méthyl)-3-méthylaniline ;
N-({trans-4-(4-fluorophényl)-1-[(1-méthyl-1H-imidazol-4-yl)sulfonyl]pyrrolidin-3-yl}méthyl)-3-(trifluorométhyl)aniline ;
4-({trans-4-(4-fluorophényl)-1-[(1-méthyl-1H-imidazol-4-yl)sulfonyl]pyrrolidin-3-yl}méthoxy)-6-(trifluorométhyl)pyrimidine ;
trans-4-cyclopropyl-1-[(1-méthyl-1H-imidazol-4-yl)sulfonyl]-N-[3-(trifluorométhyl)phényl]pyrrolidin-3-amine ;
trans-N-(3-chlorophényl)-4-cyclopropyl-1-[(1-méthyl-1H-imidazol-4-yl)sulfonyl]pyrrolidin-3-amine ;
4-{[3-(2-chlorophényl)-4-(4-fluorophényl)pyrrolidin-1-yl]sulfonyl}-1-méthyl-1H-imidazole ;
4-{[3-(4-chlorophényl)-4-(4-fluorophényl)pyrrolidin-1-yl]sulfonyl}-1-méthyl-1H-imidazole ;
2-chloro-4-({trans-4-(4-fluorophényl)-1-[(1-méthyl-1H-imidazol-4-yl)sulfonyl]pyrrolidin-3-yl}amino)benzonitrile ;
2-fluoro-4-({trans-4-(4-fluorophényl)-1-[(1-méthyl-1H-imidazol-4-yl)sulfonyl]pyrrolidin-3-yl}amino)benzonitrile ;
3-(3-chlorobenzyl)-1-[(1-méthyl-1H-imidazol-4-yl)sulfonyl]-4-phénylimidazolidin-2-one ;
trans-1-[(1-méthyl-1H-imidazol-4-yl)sulfonyl]-4-(pyridin-2-yl)-N-[3-(trifluorométhyl)phényl]pyrrolidin-3-amine ;
trans-N-(3-chlorophényl)-1-[(1-méthyl-1H-imidazol-4-yl)sulfonyl]-4-(pyridin-2-yl)pyrrolidin-3-amine ;
2-({trans-4-(4-fluorophényl)-1-[(1-méthyl-1H-imidazol-4-yl)sulfonyl]pyrrolidin-3-yl}méthoxy)-4-(trifluorométhyl)pyridine ;
trans-N-(3-cyanophényl)-4-(4-fluorophényl)-1-[(1-méthyl-1H-imidazol-4-yl)sulfonyl]pyrrolidine-3-carboxamide ;
4-{[trans-3-[(4-fluorophénoxy)méthyl]-4-(4-fluorophényl)pyrrolidin-1-yl]sulfonyl}-1-méthyl-1H-imidazole ;
4-(4-fluorophényl)-1-[(1-méthyl-1H-imidazol-4-yl)sulfonyl]-N-[3-(propan-2-yloxy)phényl]pyrrolidin-3-amine ;
N-{4-(4-fluorophényl)-1-[(1-méthyl-1H-imidazol-4-yl)sulfonyl]pyrrolidin-3-yl}pyridazin-3-amine ;
N-{(3R,4S)-4-(4-fluorophényl)-1-[(1-méthyl-1H-imidazol-4-yl)sulfonyl]pyrrolidin-3-yl}-6-(trifluorométhyl)pyridin-2-amine ;
(3R,4S)-N-(3-chloro-4-fluorophényl)-4-(4-fluorophényl)-1-[(1-méthyl-1H-imidazol-4-yl)sulfonyl]pyrrolidin-3-amine ;
(3R,4S)-N-(3-fluorophényl)-4-(4-fluorophényl)-1-[(1-méthyl-1H-imidazol-4-yl)sulfonyl]pyrrolidin-3-amine ;
trans-N-(4-chlorobenzyl)-3-méthyl-1-[(1-méthyl-1H-imidazol-4-yl)sulfonyl]-4-phénylpyrrolidin-3-amine ;
trans-3-méthyl-1-[(1-méthyl-1H-imidazol-4-yl)sulfonyl]-4-phényl-N-[4-(trifluorométhyl)benzyl]pyrrolidin-3-amine ;
trans-N-[2-chloro-4-(trifluorométhyl)benzyl]-3-méthyl-1-[(1-méthyl-1H-imidazol-4-yl)sulfonyl]-4-phénylpyrrolidin-3-amine ;
(3,4-Difluorophényl){trans-4-(4-fluorophényl)-1-[(1-méthyl-1H-imidazol-4-yl)sulfonyl]pyrrolidin-3-yl}méthanone ;
trans-N,4-bis(4-fluorophényl)-1-[(1-méthyl-1H-imidazol-4-yl)sulfonyl]pyrrolidine-3-carboxamide ;
(3,4-Dichlorophényl){trans-4-(4-fluorophényl)-1-[(1-méthyl-1H-imidazol-4-yl)sulfonyl]pyrrolidin-3-yl}méthanone ;
(3,5-Dichlorophényl){trans-4-(4-fluorophényl)-1-[(1-méthyl-1H-imidazol-4-yl)sulfonyl]pyrrolidin-3-yl}méthanone ;
(3-chloro-5-fluorophényl){trans-4-(4-fluorophényl)-1-[(1-méthyl-1H-imidazol-4-yl)sulfonyl]pyrrolidin-3-yl}méthanone ;
{trans-4-(4-fluorophényl)-1-[(1-méthyl-1H-imidazol-4-yl)sulfonyl]pyrrolidin-3-yl}[3-(trifluorométhoxy)phényl]méthanone ;
{trans-4-(4-fluorophényl)-1-[(1-méthyl-1H-imidazol-4-yl)sulfonyl]pyrrolidin-3-yl}[4-(trifluorométhoxy)phényl]méthanone ;
3-[({trans-4-(4-fluorophényl)-1-[(1-méthyl-1H-imidazol-4-yl)sulfonyl]pyrrolidin-3-yl}méthyl)amino]benzamide ;
3-(Aminométhyl)-N-({trans-4-(4-fluorophényl)-1-[(1-méthyl-1H-imidazol-4-yl)sulfonyl]pyrrolidin-3-yl}méthyl)aniline ;
4-({trans-4-(4-fluorophényl)-1-[(1-méthyl-1H-imidazol-4-yl)sulfonyl]pyrrolidin-3-yl}méthoxy)-2-(trifluorométhyl)pyridine ;
4-fluoro-N-({trans-4-(4-fluorophényl)-1-[(1-méthyl-1H-imidazol-4-yl)sulfonyl]pyrrolidin-3-yl}méthyl)aniline ;
N-{(3R,4S)-4-(4-fluorophényl)-1-[(1-méthyl-1H-imidazol-4-yl)sulfonyl]pyrrolidin-3-yl}-4-(trifluorométhyl)pyridin-2-amine ;
trans-4-(4-fluorophényl)-1-[(1-méthyl-1H-imidazol-4-yl)sulfonyl]-N-[4-(trifluorométhyl)pyridin-2-yl]pyrrolidine-3-carboxamide ;
trans-4-(4-fluorophényl)-N-[4-fluoro-3-(trifluorométhoxy)phényl]-1-[(1-méthyl-1H-imidazol-4-yl)sulfonyl]pyrrolidine-3-carboxamide ;
trans-N-[4-chloro-3-(trifluorométhoxy)phényl]-4-(4-fluorophényl)-1-[(1-méthyl-1H-imidazol-4-yl)sulfonyl]pyrrolidine-3-carboxamide ;
N-[4-chloro-2-({trans-4-(4-fluorophényl)-1-[(1-méthyl-1H-imidazol-4-yl)sulfonyl]pyrrolidin-3-yl}amino)phényl]acétamide ;
trans-4-[(3-chlorophényl)amino]-1-[(1-méthyl-1H-imidazol-4-yl)sulfonyl]pyrrolidin-3-ol ;
N-[trans-1-[(1-méthyl-1H-imidazol-4-yl)sulfonyl]-4-(pyridin-2-yl)pyrrolidin-3-yl]-1-(trifluorométhyl)cyclopropanecarboxamide ;
N-{(3R,4S)-4-(4-fluorophényl)-1-[(1-méthyl-1H-imidazol-4-yl)sulfonyl]pyrrolidin-3-yl}-1-(trifluorométhyl)cyclopropanecarboxamide ;
4-fluoro-N-({trans-4-(4-fluorophényl)-1-[(1-méthyl-1H-imidazol-4-yl)sulfonyl]pyrrolidin-3-yl}méthyl)-3-(trifluorométhoxy)aniline ;
4-chloro-N-({trans-4-(4-fluorophényl)-1-[(1-méthyl-1H-imidazol-4-yl)sulfonyl]pyrrolidin-3-yl}méthyl)-3-(trifluorométhoxy)aniline ;
trans-N-(3-chlorophényl)-4-(4-fluorophénoxy)-1-[(1-méthyl-1H-imidazol-4-yl)sulfonyl]pyrrolidin-3-amine ;
2-({trans-4-(4-fluorophényl)-1-[(1-méthyl-1H-imidazol-4-yl)sulfonyl]pyrrolidin-3-yl}oxy)-4-(trifluorométhyl)pyridine ;
3-({(3R,4S)-4-(4-fluorophényl)-1-[(1-méthyl-1H-imidazol-4-yl)sulfonyl]pyrrolidin-3-yl}amino)benzonitrile ;
6-chloro-2-[trans-1-[(1-méthyl-1H-imidazol-4-yl)sulfonyl]-4-(pyridin-2-yl)pyrrolidin-3-yl]-2,3-dihydro-1H-isoindol-1-one ;
3-benzyl-4-(4-fluorophényl)-1-[(1-méthyl-1H-imidazol-4-yl)sulfonyl]pyrrolidin-3-ol ;
N-{trans-4-(4-fluorophényl)-1-[(1-méthyl-1H-imidazol-4-yl)sulfonyl]pyrrolidin-3-yl}-5-(trifluorométhyl)pyridazin-3-amine ;
N-{4-(4-fluorophényl)-1-[(1-méthyl-1H-imidazol-4-yl)sulfonyl]pyrrolidin-3-yl}-4-(trifluorométhyl)pyridazin-3-amine ;
1-méthyl-4-{[trans-3-phényl-4-(phénylsulfonyl)pyrrolidin-1-yl]sulfonyl}-1H-imidazole ;
(3R,4S)-4-(4-fluorophényl)-1-[(1-méthyl-1H-imidazol-4-yl)sulfonyl]-N-{[1-(trifluorométhyl)cyclopropyl]méthyl}pyrrolidin-3-amine ;
(3S,4R)-N-(3-chloro-4-fluorophényl)-4-(4-fluorophényl)-1-[(1-méthyl-1H-imidazol-4-yl)sulfonyl]pyrrolidin-3-amine ;
N-{trans-3-méthyl-1-[(1-méthyl-1H-imidazol-4-yl)sulfonyl]-4-phénylpyrrolidin-3-yl}-4-(trifluorométhyl)pyridin-2-amine ;
3-({(3S,4R)-4-(4-fluorophényl)-1-[(1-méthyl-1H-imidazol-4-yl)sulfonyl]pyrrolidin-3-yl}amino)benzonitrile ;
N-{(3R,4S)-4-(4-fluorophényl)-1-[(1-méthyl-1H-imidazol-4-yl)sulfonyl]pyrrolidin-3-yl}-6-(trifluorométhyl)pyrimidin-4-amine ;
(3R,4S)-4-(4-fluorophényl)-1-[(1-méthyl-1H-imidazol-4-yl)sulfonyl]-N-{[5-(trifluorométhyl)furan-2-yl]méthyl}pyrrolidin-3-amine ;
1,1,1-trifluoro-2-[3-({(3R,4S)-4-(4-fluorophényl)-1-[(1-méthyl-1H-imidazol-4-yl)sulfonyl]pyrrolidin-3-yl}amino)phényl]propan-2-ol ;
(3R,4S)-N-[(5-chlorothiophén-2-yl)méthyl]-4-(4-fluorophényl)-1-[(1-méthyl-1H-imidazol-4-yl)sulfonyl]pyrrolidin-3-amine ;
(3R,4S)-N-[2-chloro-4-(trifluorométhyl)benzyl]-1-[(1-méthyl-1H-imidazol-4-yl)sulfonyl]-4-phénylpyrrolidin-3-amine ;
(3S,4R)-N-[2-chloro-4-(trifluorométhyl)benzyl]-1-[(1-méthyl-1H-imidazol-4-yl)sulfonyl]-4-phénylpyrrolidin-3-amine ;
N-{(3R,4S)-1-[(1-méthyl-1H-imidazol-4-yl)sulfonyl]-4-phénylpyrrolidin-3-yl}-4-(trifluorométhyl)pyridin-2-amine ;
(3R,4S)-1-[(1-méthyl-1H-imidazol-4-yl)sulfonyl]-4-phényl-N-[3-(trifluorométhyl)phényl]pyrrolidin-3-amine ;
(3R,4S)-1-[(1-méthyl-1H-imidazol-4-yl)sulfonyl]-4-phényl-N-[4-(trifluorométhyl)benzyl]pyrrolidin-3-amine ;
1,1,1-trifluoro-2-[3-({(3R,4S)-1-[(1-méthyl-1H-imidazol-4-yl)sulfonyl]-4-phénylpyrrolidin-3-yl}amino)phényl]propan-2-ol ;
trans-4-(benzyloxy)-N-(3-chlorophényl)-1-[(1-méthyl-1H-imidazol-4-yl)sulfonyl]pyrrolidin-3-amine ;
trans-1-[(1-méthyl-1H-imidazol-4-yl)sulfonyl]-4-phényl-3-{[4-(trifluorométhyl)benzyl]amino}pyrrolidin-3-yl]méthanol ;
trans-N-(3-chlorophényl)-1-[(1-méthyl-1H-imidazol-4-yl)sulfonyl]-4-(prop-2-én-1-yloxy)pyrrolidin-3-amine ;
trans-N-(3-chlorophényl)-1-[(1-méthyl-1H-imidazol-4-yl)sulfonyl]-4-[(2-méthylprop-2-én-1-yl)oxy]pyrrolidin-3-amine ;
3-{trans-1-[(1-méthyl-1H-imidazol-4-yl)sulfonyl]-4-phénylpyrrolidin-3-yl}-6-(trifluorométhyl)-3,4-dihydroquinazolin-2(1H)-one ;
4-fluoro-N-{(3R,4S)-1-[(1-méthyl-1H-imidazol-4-yl)sulfonyl]-4-phénylpyrrolidin-3-yl}pyridin-2-amine ;
N-{(3R,4S)-1-[(1-méthyl-1H-imidazol-4-yl)sulfonyl]-4-phénylpyrrolidin-3-yl}-7H-pyrrolo[2,3-d]pyrimidin-4-amine ;
trans-7-[(1-méthyl-1H-imidazol-4-yl)sulfonyl]-9-phényl-1-[4-(trifluorométhyl)benzyl]-3-oxa-1,7-diazaspiro[4.4]nonan-2-one ;
3,5-difluoro-N-{(3R,4S)-1-[(1-méthyl-1H-imidazol-4-yl)sulfonyl]-4-phénylpyrrolidin-3-yl}-4-(trifluorométhyl)pyridin-2-amine ;
(3R,4S)-1-[(1-méthyl-1H-imidazol-4-yl)sulfonyl]-4-phényl-N-{[5-(trifluorométhyl)pyridin-2-yl]méthyl}pyrrolidin-3-amine ;
5-cyclopropyl-N-{(3R,4S)-1-[(1-méthyl-1H-imidazol-4-yl)sulfonyl]-4-phénylpyrrolidin-3-yl}pyrazin-2-amine ;
5-cyclobutyl-N-{(3R,4S)-1-[(1-méthyl-1H-imidazol-4-yl)sulfonyl]-4-phénylpyrrolidin-3-yl}pyrazin-2-amine ;
trans-N-(3-chlorophényl)-1-[(1-méthyl-1H-imidazol-4-yl)sulfonyl]-4-(2-méthylpropoxy)pyrrolidin-3-amine ;
N-{(3R,4S)-1-[(1-méthyl-1H-imidazol-4-yl)sulfonyl]-4-phénylpyrrolidin-3-yl}-5-(pyrrolidin-1-yl)pyrazin-2-amine ;
N-{(3R,4S)-1-[(1-méthyl-1H-imidazol-4-yl)sulfonyl]-4-phénylpyrrolidin-3-yl}-5-(trifluorométhyl)pyrazine-2-carboxamide ;
5-chloro-N-{(3R,4S)-1-[(1-méthyl-1H-imidazol-4-yl)sulfonyl]-4-phénylpyrrolidin-3-yl}pyrazine-2-carboxamide ;
(3R,4S)-N-(4-chlorobenzyl)-1-[(1-méthyl-1H-imidazol-4-yl)sulfonyl]-4-phénylpyrrolidin-3-amine ;
(3R,4S)-N-(3-chloro-4-fluorophényl)-1-[(1-méthyl-1H-imidazol-4-yl)sulfonyl]-4-phénylpyrrolidin-3-amine ;
(3R,4S)-N-(3,4-difluorophényl)-4-(4-fluorophényl)-1-[(1-méthyl-1H-imidazol-4-yl)sulfonyl]pyrrolidin-3-amine ;
6-chloro-N-{(3R,4S)-1-[(1-méthyl-1H-imidazol-4-yl)sulfonyl]-4-phénylpyrrolidin-3-yl}pyridazine-3-carboxamide ;
N-{(3R,4S)-1-[(1-méthyl-1H-imidazol-4-yl)sulfonyl]-4-phénylpyrrolidin-3-yl}-2-(morpholin-4-yl)pyrimidin-4-amine ;
4-({(3R,4S)-1-[(1-méthyl-1H-imidazol-4-yl)sulfonyl]-4-phénylpyrrolidin-3-yl}amino)-2,3-dihydro-1H-isoindol-1-one ;
(3R,4S)-1-[(1-méthyl-1H-imidazol-4-yl)sulfonyl]-N-[3-(méthylsulfonyl)phényl]-4-phénylpyrrolidin-3-amine ;
(3R,4S)-1-[(1-méthyl-1H-imidazol-4-yl)sulfonyl]-4-phényl-N-{[5-(trifluorométhyl)pipérazin-2-yl]méthyl}pyrrolidin-3-amine ;
5-fluoro-N-{(3R,4S)-1-[(1-méthyl-1H-imidazol-4-yl)sulfonyl]-4-phénylpyrrolidin-3-yl}pyrimidin-2-amine ;
trans-4-cyclohexyl-1-[(1-méthyl-1H-imidazol-4-yl)sulfonyl]-N-[3-(trifluorométhoxy)phényl]pyrrolidin-3-amine ;
(3R,4S)-1-[(1-méthyl-1H-imidazol-4-yl)sulfonyl]-4-phényl-N-(4,4,4-trifluorobutyl)pyrrolidin-3-amine ;
(3R,4S)-N-(2-cyclopentyléthyl)-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-4-phényl-N-[(5-propylfuran-2-yl)méthyl]pyrrolidin-3-amine ;
(3R,4S)-1-[(1-méthyl-1H-imidazol-4-yl)sulfonyl]-N-[(4-méthyltétrahydro-2H-pyran-4-yl)méthyl]-4-phénylpyrrolidin-3-amine ;
(3R,4S)-1-[(1-méthyl-1H-imidazol-4-yl)sulfonyl]-4-phényl-N-[(4-phényl-1,3-thiazol-5-yl)méthyl]pyrrolidin-3-amine ;
(3R,4S)-1-[(1-méthyl-1H-imidazol-4-yl)sulfonyl]-N-[(5-méthyl-1,3-thiazol-2-yl)méthyl]-4-phénylpyrrolidin-3-amine ;
(3R,4S)-N-[(3,5-dichloropyridin-4-yl)méthyl]-1-[(1-méthyl-1H-imidazol-4-yl)sulfonyl]-4-phénylpyrrolidin-3-amine ;
(3R,4S)-N-(2-éthylhexyl)-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-N-[(4-méthyl-1,3-thiazol-5-yl)méthyl]-4-phénylpyrrolidin-3-amine ;
(3R,4S)-N-[(2,4-dichloro-1,3-thiazol-5-yl)méthyl]-1-[(1-méthyl-1H-imidazol-4-yl)sulfonyl]-4-phénylpyrrolidin-3-amine ;
(3R,4S)-N-(2,2-diméthylpropyl)-1-[(1-méthyl-1H-imidazol-4-yl)sulfonyl]-4-phénylpyrrolidin-3-amine ;
(3R,4S)-N-[(5-méthyl-1-benzothiophén-2-yl)méthyl]-1-[(1-méthyl-1H-imidazol-4-yl)sulfonyl]-4-phénylpyrrolidin-3-amine ;
(3R,4S)-N-[(2-bromo-1,3-thiazol-5-yl)méthyl]-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-N-(2-méthylpropyl)-4-phénylpyrrolidin-3-amine ;
(3R,4S)-N-[(3-chloro-1-benzothiophén-2-yl)méthyl]-1-[(1-méthyl-1H-imidazol-4-yl)sulfonyl]-4-phénylpyrrolidin-3-amine ;
(3R,4S)-N-[(2,4-diméthyl-1,3-thiazol-5-yl)méthyl]-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-4-phényl-N-(thiéno[2,3-b]pyridin-2-ylméthyl)pyrrolidin-3-amine ;
(3R,4S)-N-[(2-méthyl-1-benzofuran-3-yl)méthyl]-1-[(1-méthyl-1H-imidazol-4-yl)sulfonyl]-4-phénylpyrrolidin-3-amine ;
(3R,4S)-N-[(6-chloro-2-méthylimidazo[1,2-a]pyridin-3-yl)méthyl]-1-[(1-méthyl-1H-imidazol-4-yl)sulfonyl]-4-phénylpyrrolidin-3-amine ;
(3R,4S)-N-{[5-(4-fluorophényl)pyridin-3-yl]méthyl}-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-N-(2-méthylpentyl)-4-phénylpyrrolidin-3-amine ;
(3R,4S)-1-[(1-méthyl-1H-imidazol-4-yl)sulfonyl]-4-phényl-N-(tétrahydrofuran-3-ylméthyl)pyrrolidin-3-amine ;
(3R,4S)-1-[(1-méthyl-1H-imidazol-4-yl)sulfonyl]-N-pentyl-4-phénylpyrrolidin-3-amine ;
(3R,4S)-N-(2-éthylbutyl)-1-[(1-méthyl-1H-imidazol-4-yl)sulfonyl]-4-phénylpyrrolidin-3-amine ;
(3R,4S)-N-(2,2-diméthylbutyl)-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-N-[(2-méthyl-1,3-thiazol-5-yl)méthyl]-4-phénylpyrrolidin-3-amine ;
(3R,4S)-1-[(1-méthyl-1H-imidazol-4-yl)sulfonyl]-4-phényl-N-[2-(tétrahydro-2H-pyran-4-yl)éthyl]pyrrolidin-3-amine ;
(3R,4S)-N-(1,3-benzothiazol-2-ylméthyl)-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-N-[(4-méthyl-1,3-thiazol-2-yl)méthyl]-4-phénylpyrrolidin-3-amine ;
(3R,4S)-N-[(4,5-diméthylthiophén-2-yl)méthyl]-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-4-phényl-N-(4,5,6,7-tétrahydro-1,3-benzothiazol-2-ylméthyl)pyrrolidin-3-amine ;
(3R,4S)-1-[(1-méthyl-1H-imidazol-4-yl)sulfonyl]-4-phényl-N-(1,3-thiazol-5-ylméthyl)pyrrolidin-3-amine ;
(3R,4S)-1-[(1-méthyl-1H-imidazol-4-yl)sulfonyl]-4-phényl-N-[2-(2,6,6-triméthylcyclohex-1-én-1-yl)éthyl]pyrrolidin-3-amine ;
(3R,4S)-1-[(1-méthyl-1H-imidazol-4-yl)sulfonyl]-4-phényl-N-{[1-(propan-2-yl)pipéridin-4-yl]méthyl}pyrrolidin-3-amine ;
(3R,4S)-N-{[4-(4-fluorophényl)-1,3-thiazol-2-yl]méthyl}-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-N-[2-(4-méthylphényl)éthyl]-4-phénylpyrrolidin-3-amine ;
(3R,4S)-1-[(1-méthyl-1H-imidazol-4-yl)sulfonyl]-N-[(5-méthylthiophén-2-yl)méthyl]-4-phénylpyrrolidin-3-amine ;
(3R,4S)-N-(3-méthylbutyl)-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-4-phényl-N-(3,5,5-triméthylhexyl)pyrrolidin-3-amine ;
(3R,4S)-1-[(1-méthyl-1H-imidazol-4-yl)sulfonyl]-4-phényl-N-(2-phénylpropyl)pyrrolidin-3-amine ;
(3R,4S)-N-(3,3-diméthylbutyl)-1-[(1-méthyl-1H-imidazol-4-yl)sulfonyl]-4-phénylpyrrolidin-3-amine ;
(3R,4S)-N-[(1-méthylcyclohexyl)méthyl]-1-[(1-méthyl-1H-imidazol-4-yl)sulfonyl]-4-phénylpyrrolidin-3-amine ;
(3R,4S)-N-[2-(3-chlorophényl)éthyl]-1-[(1-méthyl-1H-imidazol-4-yl)sulfonyl]-4-phénylpyrrolidin-3-amine ;
(3R,4S)-N-[2-(4-tert-butylphénoxy)éthyl]-1-[(1-méthyl-1H-imidazol-4-yl)sulfonyl]-4-phénylpyrrolidin-3-amine ;
(3R,4S)-N-(2-éthyl-3-méthylbutyl)-1-[(1-méthyl-1H-imidazol-4-yl)sulfonyl]-4-phénylpyrrolidin-3-amine ;
(3R,4S)-N-[3-(3-chlorophényl)propyl]-1-[(1-méthyl-1H-imidazol-4-yl)sulfonyl]-4-phénylpyrrolidin-3-amine ;
(3R,4S)-N-[(4-bromo-1,3-thiazol-2-yl)méthyl]-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-N-[(4-méthyl-2-phényl-1,3-thiazol-5-yl)méthyl]-4-phénylpyrrolidin-3-amine ;
N-(3,4-difluorophényl)-1-{[3-méthyl-1-(propan-2-yl)-1H-pyrazol-4-yl]sulfonyl}-4-phénylpyrrolidin-3-amine ;
N-(3,4-difluorophényl)-1-{[1-méthyl-3-(trifluorométhyl)-1H-pyrazol-4-yl]sulfonyl}-4-phénylpyrrolidin-3-amine ;
1-[(1-cyclopentyl-3-méthyl-1H-pyrazol-4-yl)sulfonyl]-N-(3,4-difluorophényl)-4-phénylpyrrolidin-3-amine ;
N-(3,4-difluorophényl)-1-[(1,3-diméthyl-1H-pyrazol-5-yl)sulfonyl]-4-phénylpyrrolidin-3-amine ;
N-(3,4-difluorophényl)-1-[(1,5-diméthyl-1H-pyrazol-4-yl)sulfonyl]-4-phénylpyrrolidin-3-amine ;
1-[(5-chloro-1,3-diméthyl-1H-pyrazol-4-yl)sulfonyl]-N-(3,4-difluorophényl)-4-phénylpyrrolidin-3-amine ;
N-(3,4-difluorophényl)-1-[(1,2-diméthyl-1H-imidazol-5-yl)sulfonyl]-4-phénylpyrrolidin-3-amine ;
N-(3,4-difluorophényl)-1-[(1-méthyl-1H-pyrazol-3-yl)sulfonyl]-4-phénylpyrrolidin-3-amine ;
N-(3,4-difluorophényl)-4-phényl-1-[(1,3,5-triméthyl-1H-pyrazol-4-yl)sulfonyl]pyrrolidin-3-amine ;
N-(3,4-difluorophényl)-1-[(1-méthyl-1H-pyrazol-5-yl)sulfonyl]-4-phénylpyrrolidin-3-amine ;
trans-N-(3-chlorophényl)-4-(cyclopropylméthoxy)-1-[(1-méthyl-1H-imidazol-4-yl)sulfonyl]pyrrolidin-3-amine ;
N-{trans-4-cyclohexyl-1-[(1-méthyl-1H-imidazol-4-yl)sulfonyl]pyrrolidin-3-yl}-6-(trifluorométhyl)pyrimidin-4-amine ;
2-méthoxy-N-{(3R,4S)-1-[(1-méthyl-1H-imidazol-4-yl)sulfonyl]-4-phénylpyrrolidin-3-yl}pyrimidin-4-amine ;
(3R,4S)-1-[(1-méthyl-1H-imidazol-4-yl)sulfonyl]-N-[(2-méthyl-1,3-thiazol-4-yl)méthyl]-4-phénylpyrrolidin-3-amine ;
(3R,4S)-N-[(2-éthyl-1-benzofuran-3-yl)méthyl]-1-[(1-méthyl-1H-imidazol-4-yl)sulfonyl]-4-phénylpyrrolidin-3-amine ;
(3R,4S)-N-(imidazo[1,2-a]pyridin-8-ylméthyl)-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-N-[(5-méthylpyridin-2-yl)méthyl]-4-phénylpyrrolidin-3-amine ;
(3R,4S)-1-[(1-méthyl-1H-imidazol-4-yl)sulfonyl]-4-phényl-N-(pyrazolo[1,5-a]pyridin-3-ylméthyl)pyrrolidin-3-amine ;
N-{trans-4-hydroxy-1-[(1-méthyl-1H-imidazol-4-yl)sulfonyl]-4-phénylpyrrolidin-3-yl}-4-(trifluorométhyl)benzamide ;
1-[(1-méthyl-1H-imidazol-4-yl)sulfonyl]-3-phényl-4-{[4-(trifluorométhyl)benzyl]amino}pyrrolidin-3-ol ;
(3R,4S)-N-[4-chloro-3-(trifluorométhyl)phényl]-4-(4-fluorophényl)-1-[(1-méthyl-1H-imidazol-4-yl)sulfonyl]pyrrolidin-3-amine ;
{(3R,4S)-1-[(1-méthyl-1H-imidazol-4-yl)sulfonyl]-4-phénylpyrrolidin-3-yl}carbamate d'éthyle ;
{(3R,4S)-1-[(1-méthyl-1H-imidazol-4-yl)sulfonyl]-4-phénylpyrrolidin-3-yl}carbamate de propyle ;
{(3R,4S)-1-[(1-méthyl-1H-imidazol-4-yl)sulfonyl]-4-phénylpyrrolidin-3-yl}carbamate de 2-méthylpropyle ;
{(3R,4S)-1-[(1-méthyl-1H-imidazol-4-yl)sulfonyl]-4-phénylpyrrolidin-3-yl}carbamate de butyle ;
(3R,4S)-N-(2-cyclopropyléthyl)-1-[(1-méthyl-1H-imidazol-4-yl)sulfonyl]-4-phénylpyrrolidin-3-amine ;
2-cyclopropyl-N-{(3R,4S)-1-[(1-méthyl-1H-imidazol-4-yl)sulfonyl]-4-phénylpyrrolidin-3-yl}acétamide ;
(3R,4S)-N-[(2E)-hex-2-én-1-yl]-1-[(1-méthyl-1H-imidazol-4-yl)sulfonyl]-4-phénylpyrrolidin-3-amine ;
(3R,4S)-N-hexyl-1-[(1-méthyl-1H-imidazol-4-yl)sulfonyl]-4-phénylpyrrolidin-3-amine ;
N-{(3R,4S)-1-[(1-méthyl-1H-imidazol-4-yl)sulfonyl]-4-phénylpyrrolidin-3-yl}pentanamide ;
N-{(3R,4S)-1-[(1-méthyl-1H-imidazol-4-yl)sulfonyl]-4-phénylpyrrolidin-3-yl}hexanamide ;
(3R,4S)-N-(2-{[tert-butyl(diméthyl)silyl]oxy}éthyl)-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-4-phényl-N-{[6-(trifluorométhyl)pyridin-2-yl]méthyl}pyrrolidin-3-amine ;
(3R,4S)-N-[(3-chlorothiophén-2-yl)méthyl]-1-[(1-méthyl-1H-imidazol-4-yl)sulfonyl]-4-phénylpyrrolidin-3-amine ;
(3R,4S)-N-[(4,4-difluorocyclohexyl)méthyl]-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-4-phényl-N-(pyrazolo[1,5-a]pyridin-7-ylméthyl)pyrrolidin-3-amine ;
(3R,4S)-1-[(1-méthyl-1H-imidazol-4-yl)sulfonyl]-4-phényl-N-{[4-(propan-2-yl)-1,3-thiazol-2-yl]méthyl}pyrrolidin-3-amine ;
(3R,4S)-N-[(2,2-diméthyltétrahydro-2H-pyran-4-yl)méthyl]-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-4-phényl-N-{[5-(tétrahydro-2H-pyran-2-yl)thiophén-2-yl]méthyl}pyrrolidin-3-amine ;
(3R,4S)-N-[(5-bromo-4-méthyl-1,3-thiazol-2-yl)méthyl]-1-[(1-méthyl-1H-imidazol-4-yl)sulfonyl]-4-phénylpyrrolidin-3-amine ;
(3R,4S)-N-(imidazo[1,5-a]pyridin-5-ylméthyl)-1-[(1-méthyl-1H-imidazol-4-yl)sulfonyl]-4-phénylpyrrolidin-3-amine ;
(3R,4S)-N-(2,3-diméthylpentyl)-1-[(1-méthyl-1H-imidazol-4-yl)sulfonyl]-4-phénylpyrrolidin-3-amine ;
(3R,4S)-N-[(2-chloro-1,3-thiazol-4-yl)méthyl]-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-N-[(5-méthylimidazo[1,2-a]pyridin-3-yl)méthyl]-4-phénylpyrrolidin-3-amine ;
(3R,4S)-1-[(1-méthyl-1H-imidazol-4-yl)sulfonyl]-4-phényl-N-[(2-phénylcyclopropyl)méthyl]pyrrolidin-3-amine ;
(3R,4S)-N-[(4-chloro-1,3-thiazol-5-yl)méthyl]-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-4-phényl-N-(3,3,3-trifluoro-2-méthylpropyl)pyrrolidin-3-amine ;
(3R,4S)-N-[(2-chloro-3-fluoropyridin-4-yl)méthyl]-1-[(1-méthyl-1H-imidazol-4-yl)sulfonyl]-4-phénylpyrrolidin-3-amine ;
(3R,4S)-N-[(5-fluoro-1-benzothiophén-2-yl)méthyl]-1-[(1-méthyl-1H-imidazol-4-yl)sulfonyl]-4-phénylpyrrolidin-3-amine ;
(3R,4S)-N-[5-chloro-2-(difluorométhoxy)benzyl]-1-[(1-méthyl-1H-imidazol-4-yl)sulfonyl]-4-phénylpyrrolidin-3-amine ;
(3R,4S)-N-[(2,5-dichlorothiophén-3-yl)méthyl]-1-[(1-méthyl-1H-imidazol-4-yl)sulfonyl]-4-phénylpyrrolidin-3-amine ;
(3R,4S)-N-{[2-(4-fluorophényl)pyridin-3-yl]méthyl}-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-N-{[2-(4-méthylphényl)-1,3-thiazol-5-yl]méthyl}-4-phénylpyrrolidin-3-amine ;
(3R,4S)-N-(3-cyclopropylpropyl)-1-[(1-méthyl-1H-imidazol-4-yl)sulfonyl]-4-phénylpyrrolidin-3-amine ;
5-fluoro-4-méthyl-N-{(3R,4S)-1-[(1-méthyl-1H-imidazol-4-yl)sulfonyl]-4-phénylpyrrolidin-3-yl}pyridin-2-amine ;
1,5-diméthyl-N-{(3R,4S)-1-[(1-méthyl-1H-imidazol-4-yl)sulfonyl]-4-phénylpyrrolidin-3-yl}-1H-1,2,4-triazol-3-amine ;
1,5-diméthyl-4-({(3R,4S)-1-[(1-méthyl-1H-imidazol-4-yl)sulfonyl]-4-phénylpyrrolidin-3-yl}amino)-1H-pyrrole-2-carbonitrile ;
(3S,4R)-4-(4-fluorophényl)-1-[(1-méthyl-1H-imidazol-4-yl)sulfonyl]-N-[3-(propan-2-yloxy)propyl]pyrrolidine-3-carboxamide ;
(3S,4R)-N-(3-éthoxypropyl)-4-(4-fluorophényl)-1-[(1-méthyl-1H-imidazol-4-yl)sulfonyl]pyrrolidine-3-carboxamide ;
(3S,4R)-4-(4-fluorophényl)-N-(2-méthoxyéthyl)-1-[(1-méthyl-1H-imidazol-4-yl)sulfonyl]pyrrolidine-3-carboxamide ;
(3S,4R)-4-(4-fluorophényl)-1-[(1-méthyl-1H-imidazol-4-yl)sulfonyl]-N-propylpyrrolidine-3-carboxamide ;
(3S,4R)-N-butyl-4-(4-fluorophényl)-1-[(1-méthyl-1H-imidazol-4-yl)sulfonyl]pyrrolidine-3-carboxamide ;
(3S,4R)-4-(4-fluorophényl)-N-[1-méthoxypropan-2-yl]-1-[(1-méthyl-1H-imidazol-4-yl)sulfonyl]pyrrolidine-3-carboxamide ;
(3R,4S)-4-(4-fluorophényl)-N-(3-méthoxypropyl)-1-[(1-méthyl-1H-imidazol-4-yl)sulfonyl]pyrrolidine-3-carboxamide ;
(3S,4R)-N-(2-éthoxyéthyl)-4-(4-fluorophényl)-1-[(1-méthyl-1H-imidazol-4-yl)sulfonyl]pyrrolidine-3-carboxamide ;
(3R,4S)-N-[4-chloro-3-(trifluorométhoxy)phényl]-4-(4-fluorophényl)-1-[(1-méthyl-1H-imidazol-4-yl)sulfonyl]pyrrolidin-3-amine ;
(3S,4R)-N-(cyclopropylméthyl)-4-(4-fluorophényl)-1-[(1-méthyl-1H-imidazol-4-yl)sulfonyl]pyrrolidine-3-carboxamide ;
(3S,4R)-4-(4-fluorophényl)-1-[(1-méthyl-1H-imidazol-4-yl)sulfonyl]-N-[2-(propan-2-yloxy)éthyl]pyrrolidine-3-carboxamide ;
(3R,4S)-N-(3-méthoxypropyl)-1-[(1-méthyl-1H-imidazol-4-yl)sulfonyl]-4-phénylpyrrolidin-3-amine ;
(3R,4S)-N-(3-éthoxypropyl)-1-[(1-méthyl-1H-imidazol-4-yl)sulfonyl]-4-phénylpyrrolidin-3-amine ;
(3R,4S)-N-{[1-(méthoxyméthyl)cyclopropyl]méthyl}-1-[(1-méthyl-1H-imidazol-4-yl)sulfonyl]-4-phénylpyrrolidin-3-amine ;
N-({trans-4-(4-fluorophényl)-1-[(1-méthyl-1H-imidazol-4-yl)sulfonyl]pyrrolidin-3-yl}méthyl)-3-(propan-2-yloxy)propan-1-amine ;
3-éthoxy-N-({trans-4-(4-fluorophényl)-1-[(1-méthyl-1H-imidazol-4-yl)sulfonyl]pyrrolidin-3-yl}méthyl)propan-1-amine ;
N-({trans-4-(4-fluorophényl)-1-[(1-méthyl-1H-imidazol-4-yl)sulfonyl]pyrrolidin-3-yl}méthyl)-2-méthoxyéthanamine ;
N-({trans-4-(4-fluorophényl)-1-[(1-méthyl-1H-imidazol-4-yl)sulfonyl]pyrrolidin-3-yl}méthyl)propan-1-amine ;
N-({trans-4-(4-fluorophényl)-1-[(1-méthyl-1H-imidazol-4-yl)sulfonyl]pyrrolidin-3-yl}méthyl)butan-1-amine ;
(2S)-N-({trans-4-(4-fluorophényl)-1-[(1-méthyl-1H-imidazol-4-yl)sulfonyl]pyrrolidin-3-yl}méthyl)-1-méthoxypropan-2-amine ;
N-({trans-4-(4-fluorophényl)-1-[(1-méthyl-1H-imidazol-4-yl)sulfonyl]pyrrolidin-3-yl}méthyl)-3-méthoxypropan-1-amine ;
2-éthoxy-N-({trans-4-(4-fluorophényl)-1-[(1-méthyl-1H-imidazol-4-yl)sulfonyl]pyrrolidin-3-yl}méthyl)éthanamine ;
N-({trans-4-(4-fluorophényl)-1-[(1-méthyl-1H-imidazol-4-yl)sulfonyl]pyrrolidin-3-yl}méthyl)-2-(propan-2-yloxy)éthanamine ;
(3R,4S)-N-(2-méthoxyéthyl)-1-[(1-méthyl-1H-imidazol-4-yl)sulfonyl]-4-phénylpyrrolidin-3-amine ;
1-[(1-méthyl-1H-imidazol-4-yl)sulfonyl]-4-(tétrahydrofuran-2-yl)-N-[3-(trifluorométhoxy)phényl]pyrrolidin-3-amine ;
1-[(1-méthyl-1H-imidazol-4-yl)sulfonyl]-4-(tétrahydrofuran-2-yl)-N-[3-(trifluorométhoxy)phényl]pyrrolidin-3-amine ;
1-[(1-méthyl-1H-imidazol-4-yl)sulfonyl]-4-(tétrahydrofuran-2-yl)-N-[3-(trifluorométhyl)phényl]pyrrolidin-3-amine ;
(3R,4S)-1-[(1-méthyl-1H-imidazol-4-yl)sulfonyl]-4-(tétrahydrofuran-2-yl)-N-[3-(trifluorométhyl)phényl]pyrrolidin-3-amine ;
2-méthoxy-N-{(3R,4S)-1-[(1-méthyl-1H-imidazol-4-yl)sulfonyl]-4-phénylpyrrolidin-3-yl}-6-(trifluorométhyl)pyrimidin-4-amine ;
6-chloro-N-{(3R,4S)-1-[(1-méthyl-1H-imidazol-4-yl)sulfonyl]-4-phénylpyrrolidin-3-yl}pyrimidin-4-amine ;
1-cyclopropyl-N-({(3R,4R)-4-(4-fluorophényl)-1-[(1-méthyl-1H-imidazol-4-yl)sulfonyl]pyrrolidin-3-yl}méthyl)méthanamine ;
(3R,4S)-N-(2-éthoxyéthyl)-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-4-phényl-N-(2-propoxyéthyl)pyrrolidin-3-amine ;
N-(3,4-difluorophényl)-1-[(1-méthyl-1H-imidazol-4-yl)sulfonyl]-4-[-tétrahydrofuran-2-yl]pyrrolidin-3-amine ;
N-(3,4-difluorophényl)-1-[(1-méthyl-1H-imidazol-4-yl)sulfonyl]-4-(tétrahydrofuran-2-yl)pyrrolidin-3-amine ;
6-cyclopropyl-N-{(3R,4S)-1-[(1-méthyl-1H-imidazol-4-yl)sulfonyl]-4-phénylpyrrolidin-3-yl}pyrimidin-4-amine ;
6-éthyl-N-{(3R,4S)-4-(4-fluorophényl)-1-[(1-méthyl-1H-imidazol-4-yl)sulfonyl]pyrrolidin-3-yl}pyrimidin-4-amine ;
N-{(3R,4S)-4-(4-fluorophényl)-1-[(1-méthyl-1H-imidazol-4-yl)sulfonyl]pyrrolidin-3-yl}-6-(propan-2-yl)pyrimidin-4-amine ;
N-{(3R,4S)-4-(4-fluorophényl)-1-[(1-méthyl-1H-imidazol-4-yl)sulfonyl]pyrrolidin-3-yl}-2-méthyl-6-(trifluorométhyl)pyrimidin-4-amine ;
N-{(3R,4S)-4-(4-fluorophényl)-1-[(1-méthyl-1H-imidazol-4-yl)sulfonyl]pyrrolidin-3-yl}-6-propylpyrimidin-4-amine ;
5-chloro-N-{(3R,4S)-4-(4-fluorophényl)-1-[(1-méthyl-1H-imidazol-4-yl)sulfonyl]pyrrolidin-3-yl}-4-(trifluorométhyl)pyridin-2-amine ;
(3S,4R)-N-[4-chloro-3-(trifluorométhyl)phényl]-1-[(1-méthyl-1H-imidazol-4-yl)sulfonyl]-4-(tétrahydrofuran-2-yl)pyrrolidin-3-amine ;
(3R,4S)-N-[4-chloro-3-(trifluorométhyl)phényl]-1-[(1-méthyl-1H-imidazol-4-yl)sulfonyl]-4-(tétrahydrofuran-2-yl)pyrrolidin-3-amine ;
6-éthoxy-N-{(3R,4S)-1-[(1-méthyl-1H-imidazol-4-yl)sulfonyl]-4-phénylpyrrolidin-3-yl}pyrimidin-4-amine ;
6-méthoxy-N-{(3R,4S)-1-[(1-méthyl-1H-imidazol-4-yl)sulfonyl]-4-phénylpyrrolidin-3-yl}pyrimidin-4-amine ;
N-{(3R,4S)-1-[(1-méthyl-1H-imidazol-4-yl)sulfonyl]-4-phénylpyrrolidin-3-yl}-6-propoxypyrimidin-4-amine ;
6-butoxy-N-{(3R,4S)-1-[(1-méthyl-1H-imidazol-4-yl)sulfonyl]-4-phénylpyrrolidin-3-yl}pyrimidin-4-amine ;
N-{(3R,4S)-1-[(1-méthyl-1H-imidazol-4-yl)sulfonyl]-4-phénylpyrrolidin-3-yl}-6-(2-méthylpropoxy)pyrimidin-4-amine ;
4-{[(3S,4R)-3-(4-fluorophényl)-4-(hexyloxy)pyrrolidin-1-yl]sulfonyl}-1-méthyl-1H-imidazole ;
Trans-4-(4-fluorophényl)-1-[(1-méthyl-1H-imidazol-4-yl)sulfonyl]-N-pentylpyrrolidine-3-carboxamide ;
Trans-4-(4-fluorophényl)-N-hexyl-1-[(1-méthyl-1H-imidazol-4-yl)sulfonyl]pyrrolidine-3-carboxamide ;
Trans-N-(3-chlorobenzyl)-4-(4-fluorophényl)-1-[(1-méthyl-1H-imidazol-4-yl)sulfonyl]pyrrolidine-3-carboxamide ;
Trans-N-(4-chlorobenzyl)-4-(4-fluorophényl)-1-[(1-méthyl-1H-imidazol-4-yl)sulfonyl]pyrrolidine-3-carboxamide ;
Trans-N-(4-fluorobenzyl)-4-(4-fluorophényl)-1-[(1-méthyl-1H-imidazol-4-yl)sulfonyl]pyrrolidine-3-carboxamide ;
Trans-N-(3,4-difluorobenzyl)-4-(4-fluorophényl)-1-[(1-méthyl-1H-imidazol-4-yl)sulfonyl]pyrrolidine-3-carboxamide ;
Trans-4-(4-fluorophényl)-1-[(1-méthyl-1H-imidazol-4-yl)sulfonyl]-N-[3-(trifluorométhyl)benzyl]pyrrolidine-3-carboxamide ;
Trans-N-[2-chloro-5-(trifluorométhyl)benzyl]-4-(4-fluorophényl)-1-[(1-méthyl-1H-imidazol-4-yl)sulfonyl]pyrrolidine-3-carboxamide ;
Trans-4-(4-fluorophényl)-1-[(1-méthyl-1H-imidazol-4-yl)sulfonyl]-N-(pyridin-2-ylméthyl)pyrrolidine-3-carboxamide ;
Trans-4-(4-fluorophényl)-1-[(1-méthyl-1H-imidazol-4-yl)sulfonyl]-N-{[4-(trifluorométhyl)pyridin-2-yl]méthyl}pyrrolidine-3-carboxamide ;
Trans-N,N-dibutyl-4-(4-fluorophényl)-1-[(1-méthyl-1H-imidazol-4-yl)sulfonyl]pyrrolidine-3-carboxamide ;
6-méthyl-N-{(3R,4S)-1-[(1-méthyl-1H-imidazol-4-yl)sulfonyl]-4-phénylpyrrolidin-3-yl}pyrimidin-4-amine ;
(3R,4S)-4-(4-fluorophényl)-1-[(1-méthyl-1H-imidazol-4-yl)sulfonyl]-N-[4-méthyl-3-(trifluorométhyl)phényl]pyrrolidin-3-amine ;
4-({(3R,4S)-4-(4-fluorophényl)-1-[(1-méthyl-1H-imidazol-4-yl)sulfonyl]pyrrolidin-3-yl}amino)-2-(trifluorométhyl)benzonitrile ;
4-{Trans-4-(4-fluorophényl)-1-[(1-méthyl-1H-imidazol-4-yl)sulfonyl]pyrrolidin-3-yl}-1-propyl-1H-1,2,3-triazole ;
4-[-1-cyclopropylpipéridin-3-yl]-1-[(1-méthyl-1H-imidazol-4-yl)sulfonyl]-N-[3-(trifluorométhoxy)phényl]pyrrolidin-3-amine ;
Trans-N-(3-chloro-4-fluorobenzyl)-4-(4-fluorophényl)-1-[(1-méthyl-1H-imidazol-4-yl)sulfonyl]pyrrolidine-3-carboxamide ;
Trans-4-(4-fluorophényl)-N-(6-méthylheptyl)-1-[(1-méthyl-1H-imidazol-4-yl)sulfonyl]pyrrolidine-3-carboxamide ;
2-(4-fluorophénoxy)-N-{(3R,4S)-4-(4-fluorophényl)-1-[(1-méthyl-1H-imidazol-4-yl)sulfonyl]pyrrolidin-3-yl}acétamide ;
(3R,4S)-N-[4-fluoro-3-(trifluorométhyl)phényl]-1-[(1-méthyl-1H-imidazol-4-yl)sulfonyl]-4-phénylpyrrolidin-3-amine ;
(3R,4S)-N-[4-fluoro-3-(trifluorométhoxy)phényl]-1-[(1-méthyl-1H-imidazol-4-yl)sulfonyl]-4-phénylpyrrolidin-3-amine ;
(3R,4S)-N-(4-méthoxybutyl)-1-[(1-méthyl-1H-imidazol-4-yl)sulfonyl]-4-phénylpyrrolidin-3-amine ;
(3R,4S)-N-(4-méthoxyhexyl)-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-4-phényl-N-[(2-propylcyclopropyl)méthyl]pyrrolidin-3-amine ;
(3R,4S)-1-[(1-méthyl-1H-imidazol-4-yl)sulfonyl]-4-phényl-N-[(2-propylcyclopropyl)méthyl]pyrrolidin-3-amine ;
(3R,4S)-4-(4-fluorophényl)-N-[4-fluoro-3-(trifluorométhoxy)phényl]-1-[(1-méthyl-1H-imidazol-4-yl)sulfonyl]pyrrolidin-3-amine ;
(3R,4S)-4-(4-fluorophényl)-N-[4-fluoro-3-(trifluorométhyl)phényl]-1-[(1-méthyl-1H-imidazol-4-yl)sulfonyl]pyrrolidin-3-amine ;
(3R,4S)-N-[4-méthoxy-3-(trifluorométhyl)phényl]-1-[(1-méthyl-1H-imidazol-4-yl)sulfonyl]-4-phénylpyrrolidin-3-amine ;
(3R,4S)-N-(1-benzothiophén-5-yl)-1-[(1-méthyl-1H-imidazol-4-yl)sulfonyl]-4-phénylpyrrolidin-3-amine ;
(3R,4S)-N-(2,3-dihydro-1H-indén-5-yl)-1-[(1-méthyl-1H-imidazol-4-yl)sulfonyl]-4-phénylpyrrolidin-3-amine ;
(3R,4S)-N-[3,4-bis(trifluorométhyl)phényl]-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-4-phényl-N-(5,6,7,8-tétrahydronaphtalén-2-yl)pyrrolidin-3-amine ;
(3R,4S)-N-(3,4-dihydro-2H-chromén-6-yl)-1-[(1-méthyl-1H-imidazol-4-yl)sulfonyl]-4-phénylpyrrolidin-3-amine ;
4-({Trans-1-[(1-méthyl-1H-imidazol-4-yl)sulfonyl]-4-phénylpyrrolidin-3-yl}méthyl)-1-propyl-1H-1,2,3-triazole ;
(3R,4S)-N-(1-benzofuran-5-yl)-1-[(1-méthyl-1H-imidazol-4-yl)sulfonyl]-4-phénylpyrrolidin-3-amine ;
Trans-4-(4-fluorophényl)-1-[(1-méthyl-1H-imidazol-4-yl)sulfonyl]-N-[3-(trifluorométhoxy)benzyl]pyrrolidine-3-carboxamide ;
5-({(3R,4S)-4-(4-fluorophényl)-1-[(1-méthyl-1H-imidazol-4-yl)sulfonyl]pyrrolidin-3-yl}amino)-5-oxopentanoate de méthyle ;
(3R,4S)-N-[2-chloro-4-(trifluorométhyl)benzyl]-4-(4-fluorophényl)-1-[(1-méthyl-1H-imidazol-4-yl)sulfonyl]pyrrolidine-3-carboxamide ;
(3R,4S)-4-(4-fluorophényl)-1-[(1-méthyl-1H-imidazol-4-yl)sulfonyl]-N-pentylpyrrolidin-3-amine ;
(3R,4S)-4-(4-fluorophényl)-N-hexyl-1-[(1-méthyl-1H-imidazol-4-yl)sulfonyl]pyrrolidin-3-amine;
(3R,4S)-N-[2-(2-méthoxyéthoxy)éthyl]-1-[(1-méthyl-1H-imidazol-4-yl)sulfonyl]-4-phénylpyrrolidin-3-amine ;
(3R,4S)-N-[2-(4-fluorophénoxy)éthyl]-4-(4-fluorophényl)-1-[(1-méthyl-1H-imidazol-4-yl)sulfonyl]pyrrolidin-3-amine ;
4-butyl-1-{Trans-1-[(1-méthyl-1H-imidazol-4-yl)sulfonyl]-4-phénylpyrrolidin-3-yl}-1H-1,2,3-triazole ;
(3R,4S)-N-(4-chloro-3-méthylphényl)-1-[(1-méthyl-1H-imidazol-4-yl)sulfonyl]-4-phénylpyrrolidin-3-amine ;
(3R,4S)-1-[(1-méthyl-1H-imidazol-4-yl)sulfonyl]-4-phényl-N-[3-(2,2,2-trifluoroéthoxy)phényl]pyrrolidin-3-amine ;
(3R,4S)-N-(2,3-dihydro-1-benzofuran-5-yl)-1-[(1-méthyl-1H-imidazol-4-yl)sulfonyl]-4-phénylpyrrolidin-3-amine ;
(3R,4S)-N-(4,4-diméthyl-3,4-dihydro-2H-chromén-6-yl)-4-(4-fluorophényl)-1-[(1-méthyl-1H-imidazol-4-yl)sulfonyl]pyrrolidin-3-amine ;
N-{(3R,4S)-4-(4-fluorophényl)-1-[(1-méthyl-1H-imidazol-4-yl)sulfonyl]pyrrolidin-3-yl}-2-hydroxyhexanamide ;
(3R,4S)-N-(3-méthoxyhexyl)-1-[(1-méthyl-1H-imidazol-4-yl)sulfonyl]-4-phénylpyrrolidin-3-amine ;
(3R,4S)-N-[4-chloro-3-(propan-2-yl)phényl]-4-(4-fluorophényl)-1-[(1-méthyl-1H-imidazol-4-yl)sulfonyl]pyrrolidin-3-amine ;
(3R,4S)-4-(4-fluorophényl)-1-[(1-méthyl-1H-imidazol-4-yl)sulfonyl]-N-[3-méthyl-4-(trifluorométhyl)phényl]pyrrolidin-3-amine ;
(3R,4S)-N-(1-benzothiophén-6-yl)-4-(4-fluorophényl)-1-[(1-méthyl-1H-imidazol-4-yl)sulfonyl]pyrrolidin-3-amine ;
-4-(1-cyclopropylpipéridin-4-yl)-1-[(1-méthyl-1H-imidazol-4-yl)sulfonyl]-N-[3-(trifluorométhoxy)phényl]pyrrolidin-3-amine ;
Trans-N-hexyl-1-[(1-méthyl-1H-imidazol-4-yl)sulfonyl]-4-(tétrahydro-2H-pyran-2-yl)pyrrolidin-3-amine ;
Trans-N-[2-chloro-4-(trifluorométhyl)benzyl]-1-[(1-méthyl-1H-imidazol-4-yl)sulfonyl]-4-(tétrahydro-2H-pyran-2-yl)pyrrolidin-3-amine ;
Trans-1-[(1-méthyl-1H-imidazol-4-yl)sulfonyl]-4-(tétrahydro-2H-pyran-2-yl)-N-[3-(trifluorométhyl)phényl]pyrrolidin-3-amine ;
N-{Trans-1-[(1-méthyl-1H-imidazol-4-yl)sulfonyl]-4-[tétrahydro-2H-pyran-2-yl]pyrrolidin-3-yl}-4-(trifluorométhyl)pyridin-2-amine ;
N-{Trans-1-[(1-méthyl-1H-imidazol-4-yl)sulfonyl]-4-[tétrahydro-2H-pyran-2-yl]pyrrolidin-3-yl}-4-(trifluorométhyl)pyridin-2-amine ;
(3R,4S)-N-[4-(4,4-diméthyl-1,3-dioxan-2-yl)butyl]-4-(4-fluorophényl)-1-[(1-méthyl-1H-imidazol-4-yl)sulfonyl]pyrrolidin-3-amine ;
Trans-1-[(1-méthyl-1H-imidazol-4-yl)sulfonyl]-4-(tétrahydro-2H-pyran-2-yl)-N-[3-(trifluorométhoxy)phényl]pyrrolidin-3-amine ;
Trans-1-[(1-méthyl-1H-imidazol-4-yl)sulfonyl]-4-[tétrahydro-2H-pyran-2-yl]-N-[3-(trifluorométhoxy)phényl]pyrrolidin-3-amine ;
N-[Trans-1-[(1-méthyl-1H-imidazol-4-yl)sulfonyl]-4-(tétrahydro-2H-pyran-2-yl)pyrrolidin-3-yl]-6-(trifluorométhyl)pyrimidin-4-amine ;
N-[2-({Trans-4-(4-fluorophényl)-1-[(1-méthyl-1H-imidazol-4-yl)sulfonyl]pyrrolidin-3-yl}oxy)éthyl]propan-1-amine ;
2-({Trans-4-(4-fluorophényl)-1-[(1-méthyl-1H-imidazol-4-yl)sulfonyl]pyrrolidin-3-yl}oxy)-N-propylacétamide ;
6-chloro-N-{(3R,4S)-4-(4-fluorophényl)-1-[(1-méthyl-1H-imidazol-4-yl)sulfonyl]pyrrolidin-3-yl}-2-méthylpyrimidin-4-amine ;
6-chloro-N-{(3R,4S)-4-(4-fluorophényl)-1-[(1-méthyl-1H-imidazol-4-yl)sulfonyl]pyrrolidin-3-yl}pyrimidin-4-amine ;
1-{(3R,4S)-4-(4-fluorophényl)-1-[(1-méthyl-1H-imidazol-4-yl)sulfonyl]pyrrolidin-3-yl}-3-phénylurée ;
{(3R,4S)-4-(4-fluorophényl)-1-[(1-méthyl-1H-imidazol-4-yl)sulfonyl]pyrrolidin-3-yl}carbamate de phényle ;
1-(2-chlorophényl)-3-{(3R,4S)-4-(4-fluorophényl)-1-[(1-méthyl-1H-imidazol-4-yl)sulfonyl]pyrrolidin-3-yl}urée ;
N-{(3R,4S)-4-(4-fluorophényl)-1-[(1-méthyl-1H-imidazol-4-yl)sulfonyl]pyrrolidin-3-yl}-5-oxohexanamide ;
N-{(3R,4S)-4-(4-fluorophényl)-1-[(1-méthyl-1H-imidazol-4-yl)sulfonyl]pyrrolidin-3-yl}-4-(trifluorométhyl)cyclohexanecarboxamide ;
Carbonate de butyle et de trans-4-(4-fluorophényl)-1-[(1-méthyl-1H-imidazol-4-yl)sulfonyl]pyrrolidin-3-yle ;
5,5,5-trifluoro-N-{(3R,4S)-4-(4-fluorophényl)-1-[(1-méthyl-1H-imidazol-4-yl)sulfonyl]pyrrolidin-3-yl}pentanamide ;
butylcarbamate de (3S,4R)-4-(4-fluorophényl)-1-[(1-méthyl-1H-imidazol-4-yl)sulfonyl]pyrrolidin-3-yle ;
2,2-difluoro-N-{(3R,4S)-4-(4-fluorophényl)-1-[(1-méthyl-1H-imidazol-4-yl)sulfonyl]pyrrolidin-3-yl}pentanamide ;
1-(4-chlorophényl)-3-{(3R,4S)-4-(4-fluorophényl)-1-[(1-méthyl-1H-imidazol-4-yl)sulfonyl]pyrrolidin-3-yl}urée ;
1-(2-fluorophényl)-3-{(3R,4S)-4-(4-fluorophényl)-1-[(1-méthyl-1H-imidazol-4-yl)sulfonyl]pyrrolidin-3-yl}urée ;
1-(3-fluorophényl)-3-{(3R,4S)-4-(4-fluorophényl)-1-[(1-méthyl-1H-imidazol-4-yl)sulfonyl]pyrrolidin-3-yl}urée ;
1-(4-fluorophényl)-3-{(3R,4S)-4-(4-fluorophényl)-1-[(1-méthyl-1H-imidazol-4-yl)sulfonyl]pyrrolidin-3-yl}urée ;
1-(2-chlorobenzyl)-3-{(3R,4S)-4-(4-fluorophényl)-1-[(1-méthyl-1H-imidazol-4-yl)sulfonyl]pyrrolidin-3-yl}urée ;
4,5-dichloro-N-{(3R,4S)-4-(4-fluorophényl)-1-[(1-méthyl-1H-imidazol-4-yl)sulfonyl]pyrrolidin-3-yl}pyridin-2-amine ;
(3R,4S)-4-(4-fluorophényl)-1-[(1-méthyl-1H-imidazol-4-yl)sulfonyl]-N-{[4-(trifluorométhyl)cyclohexyl]méthyl}pyrrolidin-3-amine ;
2,2-difluoro-N-{(3R,4S)-4-(4-fluorophényl)-1-[(1-méthyl-1H-imidazol-4-yl)sulfonyl]pyrrolidin-3-yl}hexanamide ;
trans-N-[3,4-difluorophényl]-1-[(1-méthyl-1H-imidazol-4-yl)sulfonyl]-4-(1,3-oxazol-2-yl)pyrrolidin-3-amine ;
N-[4-chloro-3-(trifluorométhyl)phényl]-1-[(1-méthyl-1H-imidazol-4-yl)sulfonyl]-4-(1,3-oxazol-2-yl)pyrrolidin-3-amine ;
4-{[trans-3-[(4-fluorobenzyl)oxy]-4-(4-fluorophényl)pyrrolidin-1-yl]sulfonyl}-1-méthyl-1H-imidazole ;
4-{[trans-3-[(3-fluorobenzyl)oxy]-4-(4-fluorophényl)pyrrolidin-1-yl]sulfonyl}-1-méthyl-1H-imidazole ;
4-{[trans-3-(4-fluorophényl)-4-{[4-(méthylsulfonyl)benzyl]oxy}pyrrolidin-1-yl]sulfonyl}-1-méthyl-1H-imidazole ;
4-{[trans-3-(4-fluorophényl)-4-{[3-(méthylsulfonyl)benzyl]oxy}pyrrolidin-1-yl]sulfonyl}-1-méthyl-1H-imidazole ;
6-éthoxy-N-{(3R,4S)-4-(4-fluorophényl)-1-[(1-méthyl-1H-imidazol-4-yl)sulfonyl]pyrrolidin-3-yl}pyrimidin-4-amine ;
N-{(3R,4S)-4-(4-fluorophényl)-1-[(1-méthyl-1H-imidazol-4-yl)sulfonyl]pyrrolidin-3-yl}-6-(propan-2-yloxy)pyrimidin-4-amine ;
6-butoxy-N-{(3R,4S)-4-(4-fluorophényl)-1-[(1-méthyl-1H-imidazol-4-yl)sulfonyl]pyrrolidin-3-yl}pyrimidin-4-amine ;
N-{(3R,4S)-4-(4-fluorophényl)-1-[(1-méthyl-1H-imidazol-4-yl)sulfonyl]pyrrolidin-3-yl}-6-(2-méthylpropoxy)pyrimidin-4-amine ;
6-(cyclobutyloxy)-N-{(3R,4S)-4-(4-fluorophényl)-1-[(1-méthyl-1H-imidazol-4-yl)sulfonyl]pyrrolidin-3-yl}pyrimidin-4-amine ;
6-(cyclopentyloxy)-N-{(3R,4S)-4-(4-fluorophényl)-1-[(1-méthyl-1H-imidazol-4-yl)sulfonyl]pyrrolidin-3-yl}pyrimidin-4-amine ;
6-(cyclohexyloxy)-N-{(3R,4S)-4-(4-fluorophényl)-1-[(1-méthyl-1H-imidazol-4-yl)sulfonyl]pyrrolidin-3-yl}pyrimidin-4-amine ;
N-{(3R,4S)-4-(4-fluorophényl)-1-[(1-méthyl-1H-imidazol-4-yl)sulfonyl]pyrrolidin-3-yl}-6-phénylpyrimidin-4-amine ;
N-{(3R,4S)-4-(4-fluorophényl)-1-[(1-méthyl-1H-imidazol-4-yl)sulfonyl]pyrrolidin-3-yl}-6-(2-méthylphényl)pyrimidin-4-amine ;
N-{(3R,4S)-4-(4-fluorophényl)-1-[(1-méthyl-1H-imidazol-4-yl)sulfonyl]pyrrolidin-3-yl}-6-(3-méthylphényl)pyrimidin-4-amine ;
N-{(3R,4S)-4-(4-fluorophényl)-1-[(1-méthyl-1H-imidazol-4-yl)sulfonyl]pyrrolidin-3-yl}-6-(4-méthylphényl)pyrimidin-4-amine ;
6-(2-fluorophényl)-N-{(3R,4S)-4-(4-fluorophényl)-1-[(1-méthyl-1H-imidazol-4-yl)sulfonyl]pyrrolidin-3-yl}pyrimidin-4-amine ;
6-(3-fluorophényl)-N-{(3R,4S)-4-(4-fluorophényl)-1-[(1-méthyl-1H-imidazol-4-yl)sulfonyl]pyrrolidin-3-yl}pyrimidin-4-amine ;
6-(4-fluorophényl)-N-{(3R,4S)-4-(4-fluorophényl)-1-[(1-méthyl-1H-imidazol-4-yl)sulfonyl]pyrrolidin-3-yl}pyrimidin-4-amine ;
6-(3-chlorophényl)-N-{(3R,4S)-4-(4-fluorophényl)-1-[(1-méthyl-1H-imidazol-4-yl)sulfonyl]pyrrolidin-3-yl}pyrimidin-4-amine ;
6-(4-chlorophényl)-N-{(3R,4S)-4-(4-fluorophényl)-1-[(1-méthyl-1H-imidazol-4-yl)sulfonyl]pyrrolidin-3-yl}pyrimidin-4-amine ;
4-({(3S,4R)-3-(4-fluorophényl)-4-[(4-méthylbenzyl)oxy]pyrrolidin-1-yl}sulfonyl)-1-méthyl-1H-imidazole ;
(3R,4S)-4-(4-fluorophényl)-1-[(1-méthyl-1H-imidazol-4-yl)sulfonyl]-N-(5,5,5-trifluoropentyl)pyrrolidin-3-amine ;
(3R,4S)-N-(2,2-difluorohexyl)-4-(4-fluorophényl)-1-[(1-méthyl-1H-imidazol-4-yl)sulfonyl]pyrrolidin-3-amine;
(3R,4S)-N-(2,2-difluoropentyl)-4-(4-fluorophényl)-1-[(1-méthyl-1H-imidazol-4-yl)sulfonyl]pyrrolidin-3-amine ;
5,5-difluoro-N-{(3R,4S)-4-(4-fluorophényl)-1-[(1-méthyl-1H-imidazol-4-yl)sulfonyl]pyrrolidin-3-yl}hexanamide ;
4-fluoro-N-{(3R,4S)-4-(4-fluorophényl)-1-[(1-méthyl-1H-imidazol-4-yl)sulfonyl]pyrrolidin-3-yl}pyridin-2-amine ;
5-fluoro-N-{(3R,4S)-4-(4-fluorophényl)-1-[(1-méthyl-1H-imidazol-4-yl)sulfonyl]pyrrolidin-3-yl}pyridin-2-amine ;
N-{(3R,4S)-4-(4-fluorophényl)-1-[(1-méthyl-1H-imidazol-4-yl)sulfonyl]pyrrolidin-3-yl}-4-oxopentanamide ;
(3R,4S)-N-(5,5-difluorohexyl)-4-(4-fluorophényl)-1-[(1-méthyl-1H-imidazol-4-yl)sulfonyl]pyrrolidin-3-amine ;
4-chloro-N-[2-({trans-4-(4-fluorophényl)-1-[(1-méthyl-1H-imidazol-4-yl)sulfonyl]pyrrolidin-3-yl}oxy)éthyl]aniline ;
(3S,4R)-N-[4-fluoro-3-(trifluorométhoxy)phényl]-1-[(1-méthyl-1H-imidazol-4-yl)sulfonyl]-4-(tétrahydro-2H-pyran-2-yl)pyrrolidin-3-amine ;
1-benzyl-3-{(3R,4S)-4-(4-fluorophényl)-1-[(1-méthyl-1H-imidazol-4-yl)sulfonyl]pyrrolidin-3-yl}urée ;
(3R,4S)-N-(4-chloro-3-méthylphényl)-4-(4-fluorophényl)-1-[(1-méthyl-1H-imidazol-4-yl)sulfonyl]pyrrolidin-3-amine ;
(3R,4S)-4-(4-fluorophényl)-1-[(1-méthyl-1H-imidazol-4-yl)sulfonyl]-N-[3-(2,2,2-trifluoroéthoxy)phényl]pyrrolidin-3-amine ;
(3R,4S)-4-(4-fluorophényl)-N-[4-fluoro-3-(pyridin-3-yl)phényl]-1-[(1-méthyl-1H-imidazol-4-yl)sulfonyl]pyrrolidin-3-amine ;
(3R,4S)-4-(4-fluorophényl)-N-[4-fluoro-3-(pyridin-4-yl)phényl]-1-[(1-méthyl-1H-imidazol-4-yl)sulfonyl]pyrrolidin-3-amine ;
(3R,4S)-N-(4-fluoro-3-méthylphényl)-4-(4-fluorophényl)-1-[(1-méthyl-1H-imidazol-4-yl)sulfonyl]pyrrolidin-3-amine ;
5-fluoro-N-{(3R,4S)-4-(4-fluorophényl)-1-[(1-méthyl-1H-imidazol-4-yl)sulfonyl]pyrrolidin-3-yl}-4-méthylpyridin-2-amine ;
(3R,4S)-4-(4-fluorophényl)-N-[4-fluoro-3-(pyrimidin-5-yl)phényl]-1-[(1-méthyl-1H-imidazol-4-yl)sulfonyl]pyrrolidin-3-amine ;
N-{(3R,4S)-4-(4-fluorophényl)-1-[(1-méthyl-1H-imidazol-4-yl)sulfonyl]pyrrolidin-3-yl}-3,4'-bipyridin-2'-amine ;
N-{(3R,4S)-4-(4-fluorophényl)-1-[(1-méthyl-1H-imidazol-4-yl)sulfonyl]pyrrolidin-3-yl}-4,4'-bipyridin-2-amine ;
4-({(3S,4R)-4-(4-fluorophényl)-1-[(1-méthyl-1H-imidazol-4-yl)sulfonyl]pyrrolidin-3-yl}oxy)-6-(trifluorométhyl)pyrimidine ;
chloro-3-(trifluorométhyl)phényl]-1-[(1-méthyl-1H-imidazol-4-yl)sulfonyl]-4-(1,3-oxazol-4-yl)pyrrolidin-3-amine ;
4,4-difluoro-N-{(3R,4S)-4-(4-fluorophényl)-1-[(1-méthyl-1H-imidazol-4-yl)sulfonyl]pyrrolidin-3-yl}pentanamide ;
(3R,4S)-N-(3,4-difluorophényl)-1-[(1,2-diméthyl-1H-imidazol-4-yl)sulfonyl]-4-(4-fluorophényl)pyrrolidin-3-amine ;
(3R,4S)-1-[(1,2-diméthyl-1H-imidazol-4-yl)sulfonyl]-4-(4-fluorophényl)-N-[4-fluoro-3-(trifluorométhyl)phényl]pyrrolidin-3-amine ;
(3R,4S)-N-(3,4-difluorophényl)-1-[(1,2-diméthyl-1H-imidazol-5-yl)sulfonyl]-4-(4-fluorophényl)pyrrolidin-3-amine ;
(3R,4S)-1-[(1,2-diméthyl-1H-imidazol-5-yl)sulfonyl]-4-(4-fluorophényl)-N-[4-fluoro-3-(trifluorométhyl)phényl]pyrrolidin-3-amine ;
4-chloro-N-{(3R,4S)-4-(4-fluorophényl)-1-[(1-méthyl-1H-imidazol-4-yl)sulfonyl]pyrrolidin-3-yl}pyridin-2-amine ;
5-chloro-N-[(3R,4S)-1-[(1,2-diméthyl-1H-imidazol-4-yl)sulfonyl]-4-(4-fluorophényl)pyrrolidin-3-yl]-4-(trifluorométhyl)pyridin-2-amine ;
trans-chloro-4-(trifluorométhyl)benzyl]-4-(3-fluoropyridin-2-yl)-1-[(1-méthyl-1H-imidazol-4-yl)sulfonyl]pyrrolidin-3-amine ;
trans-4-(3-fluoropyridin-2-yl)-N-hexyl-1-[(1-méthyl-1H-imidazol-4-yl)sulfonyl]pyrrolidin-3-amine ;
trans-4-(3-fluoropyridin-2-yl)-N,N-dihexyl-1-[(1-méthyl-1H-imidazol-4-yl)sulfonyl]pyrrolidin-3-amine ;
trans-4-(3-fluoropyridin-2-yl)-1-[(1-méthyl-1H-imidazol-4-yl)sulfonyl]-N,N-dipentylpyrrolidin-3-amine ;
trans-4-(3-fluoropyridin-2-yl)-1-[(1-méthyl-1H-imidazol-4-yl)sulfonyl]-N-pentylpyrrolidin-3-amine ;
trans-N-(3,4-difluorophényl)-4-(3-fluoropyridin-2-yl)-1-[(1-méthyl-1H-imidazol-4-yl)sulfonyl]pyrrolidin-3-amine ;
trans-4-(3-fluoropyridin-2-yl)-N-[4-fluoro-3-(trifluorométhyl)phényl]-1-[(1-méthyl-1H-imidazol-4-yl)sulfonyl]pyrrolidin-3-amine ;
trans-4-(3-fluoropyridin-2-yl)-N-[4-fluoro-3-(trifluorométhoxy)phényl]-1-[(1-méthyl-1H-imidazol-4-yl)sulfonyl]pyrrolidin-3-amine ;
5-fluoro-N-{trans-4-(3-fluoropyridin-2-yl)-1-[(1-méthyl-1H-imidazol-4-yl)sulfonyl]pyrrolidin-3-yl}-4-méthylpyridin-2-amine ;
N-{trans-4-(3-fluoropyridin-2-yl)-1-[(1-méthyl-1H-imidazol-4-yl)sulfonyl]pyrrolidin-3-yl}-6-(trifluorométhyl)pyrimidin-4-amine ;
trans-N-(3-chloro-4-fluorophényl)-4-(3-fluoropyridin-2-yl)-1-[(1-méthyl-1H-imidazol-4-yl)sulfonyl]pyrrolidin-3-amine ;
6-chloro-N-{trans-4-(3-fluoropyridin-2-yl)-1-[(1-méthyl-1H-imidazol-4-yl)sulfonyl]pyrrolidin-3-yl}pyrimidin-4-amine ;
(3R,4S)-N-[3-(benzyloxy)phényl]-1-[(1-méthyl-1H-imidazol-4-yl)sulfonyl]-4-phénylpyrrolidin-3-amine ;
6-(benzyloxy)-N-{(3R,4S)-4-(4-fluorophényl)-1-[(1-méthyl-1H-imidazol-4-yl)sulfonyl]pyrrolidin-3-yl}pyrimidin-4-amine ;
trans-N-(4-fluoro-3-méthylphényl)-4-(3-fluoropyridin-2-yl)-1-[(1-méthyl-1H-imidazol-4-yl)sulfonyl]pyrrolidin-3-amine ;
4-(benzyloxy)-5-fluoro-N-{(3R,4S)-1-[(1-méthyl-1H-imidazol-4-yl)sulfonyl]-4-phénylpyrrolidin-3-yl}pyridin-2-amine ;
(3R,4S)-1-[(1-méthyl-1H-imidazol-4-yl)sulfonyl]-4-phényl-N-[3-(pipéridin-4-yl)phényl]pyrrolidin-3-amine ;
6-(azétidin-3-ylméthoxy)-N-{(3R,4S)-4-(4-fluorophényl)-1-[(1-méthyl-1H-imidazol-4-yl)sulfonyl]pyrrolidin-3-yl}pyrimidin-4-amine ;
N-{(3R,4S)-4-(4-fluorophényl)-1-[(1-méthyl-1H-imidazol-4-yl)sulfonyl]pyrrolidin-3-yl}-6-(pyrrolidin-3-ylméthoxy)pyrimidin-4-amine ;
N-{(3R,4S)-4-(4-fluorophényl)-1-[(1-méthyl-1H-imidazol-4-yl)sulfonyl]pyrrolidin-3-yl}-6-(pyrrolidin-2-ylméthoxy)pyrimidin-4-amine ;
trans-1-[(1-méthyl-1H-imidazol-4-yl)sulfonyl]-N-(3-méthylphényl)-4-(tétrahydro-2H-pyran-3-yl)pyrrolidin-3-amine ;
trans-1-[(1-méthyl-1H-imidazol-4-yl)sulfonyl]-4-(tétrahydro-2H-pyran-3-yl)-N-[3-(trifluorométhyl)phényl]pyrrolidin-3-amine ;
trans-N-(3-chlorophényl)-1-[(1-méthyl-1H-imidazol-4-yl)sulfonyl]-4-(tétrahydro-2H-pyran-3-yl)pyrrolidin-3-amine ;
trans-1-[(1-méthyl-1H-imidazol-4-yl)sulfonyl]-4-[(3S)-tétrahydro-2H-pyran-3-yl]-N-[3-(trifluorométhoxy)phényl]pyrrolidin-3-amine ;
trans-1-[(1-méthyl-1H-imidazol-4-yl)sulfonyl]-4-[(3S)-tétrahydro-2H-pyran-3-yl]-N-[3-(2,2,2-trifluoroéthoxy)phényl]pyrrolidin-3-amine ;
trans-1-[(1-méthyl-1H-imidazol-4-yl)sulfonyl]-N-(3-phénoxyphényl)-4-[(3S)-tétrahydro-2H-pyran-3-yl]pyrrolidin-3-amine ;
trans-N-(biphényl-3-yl)-1-[(1-méthyl-1H-imidazol-4-yl)sulfonyl]-4-[(3S)-tétrahydro-2H-pyran-3-yl]pyrrolidin-3-amine ;
trans-N-[4-fluoro-3-(trifluorométhoxy)phényl]-1-[(1-méthyl-1H-imidazol-4-yl)sulfonyl]-4-[(3S)-tétrahydro-2H-pyran-3-yl]pyrrolidin-3-amine ;
trans-N-(3-chloro-4-fluorophényl)-1-[(1-méthyl-1H-imidazol-4-yl)sulfonyl]-4-[(3S)-tétrahydro-2H-pyran-3-yl]pyrrolidin-3-amine ;
trans-N-(4-fluoro-3-méthylphényl)-1-[(1-méthyl-1H-imidazol-4-yl)sulfonyl]-4-[(3S)-tétrahydro-2H-pyran-3-yl]pyrrolidin-3-amine ;
trans-N-[4-fluoro-3-(trifluorométhyl)phényl]-1-[(1-méthyl-1H-imidazol-4-yl)sulfonyl]-4-[(3S)-tétrahydro-2H-pyran-3-yl]pyrrolidin-3-amine ;
trans-N-[4-chloro-3-(trifluorométhyl)phényl]-1-[(1-méthyl-1H-imidazol-4-yl)sulfonyl]-4-[(3S)-tétrahydro-2H-pyran-3-yl]pyrrolidin-3-amine ;
trans-N-[4-chloro-3-(trifluorométhoxy)phényl]-1-[(1-méthyl-1H-imidazol-4-yl)sulfonyl]-4-[(3S)-tétrahydro-2H-pyran-3-yl]pyrrolidin-3-amine ;
trans-N-[3-(benzyloxy)phényl]-1-[(1-méthyl-1H-imidazol-4-yl)sulfonyl]-4-[(3S)-tétrahydro-2H-pyran-3-yl]pyrrolidin-3-amine ;
5-fluoro-N-{(3R,4S)-4-(4-fluorophényl)-1-[(1-méthyl-1H-imidazol-4-yl)sulfonyl]pyrrolidin-3-yl}-4-(trifluorométhyl)pyridin-2-amine ;
3-fluoro-N-{(3R,4S)-4-(4-fluorophényl)-1-[(1-méthyl-1H-imidazol-4-yl)sulfonyl]pyrrolidin-3-yl}-4-(trifluorométhyl)pyridin-2-amine ;
N-(2,2-difluoroéthyl)-2-({(3R,4S)-4-(4-fluorophényl)-1-[(1-méthyl-1H-imidazol-4-yl)sulfonyl]pyrrolidin-3-yl}oxy)acétamide ;
trans-4-(3-fluoropyridin-2-yl)-1-[(1-méthyl-1H-imidazol-4-yl)sulfonyl]-N-[3-(trifluorométhyl)phényl]pyrrolidin-3-amine ;
trans-4-(3-fluoropyridin-2-yl)-1-[(1-méthyl-1H-imidazol-4-yl)sulfonyl]-N-[3-(trifluorométhoxy)phényl]pyrrolidin-3-amine ;
trans-4-(3-fluoropyridin-2-yl)-1-[(1-méthyl-1H-imidazol-4-yl)sulfonyl]-N-[3-(2,2,2-trifluoroéthoxy)phényl]pyrrolidin-3-amine ;
(3R,4S)-4-(4-fluorophényl)-1-[(1-méthyl-1H-imidazol-4-yl)sulfonyl]-N-[3-(pyridin-2-yl)phényl]pyrrolidin-3-amine ;
(3R,4S)-1-[(1-méthyl-1H-imidazol-4-yl)sulfonyl]-4-phényl-N-[3-(pyridin-2-yl)phényl]pyrrolidin-3-amine ;
5-fluoro-N-{(3R,4S)-1-[(1-méthyl-1H-imidazol-4-yl)sulfonyl]-4-phénylpyrrolidin-3-yl}-4-(2,2,2-trifluoroéthoxy)pyridin-2-amine ;
5-chloro-N-{trans-4-(3-fluoropyridin-2-yl)-1-[(1-méthyl-1H-imidazol-4-yl)sulfonyl]pyrrolidin-3-yl}-4-(trifluorométhyl)pyridin-2-amine ;
trans-N-(3-fluorophényl)-4-(3-fluoropyridin-2-yl)-1-[(1-méthyl-1H-imidazol-4-yl)sulfonyl]pyrrolidin-3-amine ;
5-chloro-N-{(3R,4S)-4-(4-fluorophényl)-1-[(1-méthyl-1H-imidazol-4-yl)sulfonyl]pyrrolidin-3-yl}-4-(2,2,2-trifluoroéthoxy)pyridin-2-amine ;
N-{(3R,4S)-4-(4-fluorophényl)-1-[(1-méthyl-1H-imidazol-4-yl)sulfonyl]pyrrolidin-3-yl}-4-(2,2,2-trifluoroéthoxy)pyridin-2-amine ;
5-chloro-N-{(3R,4S)-1-[(1-méthyl-1H-imidazol-4-yl)sulfonyl]-4-phénylpyrrolidin-3-yl}-4-(2,2,2-trifluoroéthoxy)pyridin-2-amine ;
N-{(3R,4S)-1-[(1-méthyl-1H-imidazol-4-yl)sulfonyl]-4-phénylpyrrolidin-3-yl}-4-(2,2,2-trifluoroéthoxy)pyridin-2-amine ;
5-fluoro-N-{(3R,4S)-4-(4-fluorophényl)-1-[(1-méthyl-1H-imidazol-4-yl)sulfonyl]pyrrolidin-3-yl}-4-(2,2,2-trifluoroéthoxy)pyridin-2-amine ;
trans-4-(3-fluoropyridin-2-yl)-N-hexyl-1-[(1-méthyl-1H-1,2,3-triazol-4-yl)sulfonyl]pyrrolidin-3-amine ;
N-{(3R,4S)-4-(4-fluorophényl)-1-[(1-méthyl-1H-imidazol-4-yl)sulfonyl]pyrrolidin-3-yl}-4-(propan-2-yl)pyridin-2-amine ;
N-{(3R,4S)-1-[(1-méthyl-1H-imidazol-4-yl)sulfonyl]-4-phénylpyrrolidin-3-yl}-4-(propan-2-yl)pyridin-2-amine ;
2,2,2-trifluoro-N-[2-({trans-4-(4-fluorophényl)-1-[(1-méthyl-1H-imidazol-4-yl)sulfonyl]pyrrolidin-3-yl}oxy)éthyl]éthanamine ;
trans-N-(3-chloro-4-fluorophényl)-1-[(1-méthyl-1H-imidazol-4-yl)sulfonyl]-4-(tétrahydro-2H-pyran-2-yl)pyrrolidin-3-amine ;
trans-1-[(1-méthyl-1H-imidazol-4-yl)sulfonyl]-4-(tétrahydro-2H-pyran-2-yl)-N-[3-(2,2,2-trifluoroéthoxy)phényl]pyrrolidin-3-amine ;
2-({1-[(1-méthyl-1H-imidazol-4-yl)sulfonyl]-4-phénylpyrrolidin-3-yl}méthyl)-4-(trifluorométhyl)pyridine ;
trans-N-[2-chloro-4-(trifluorométhyl)benzyl]-4-(3-fluoropyridin-2-yl)-1-[(1-méthyl-1H-1,2,3-triazol-4-yl)sulfonyl]pyrrolidin-3-amine ;
trans-1-[(1-méthyl-1H-imidazol-4-yl)sulfonyl]-4-(tétrahydrofuran-2-yl)-N-[3-(2,2,2-trifluoroéthoxy)phényl]pyrrolidin-3-amine ;
N-hexyl-1-[(1-méthyl-1H-imidazol-4-yl)sulfonyl]-4-[(2R)-tétrahydrofuran-2-yl]pyrrolidin-3-amine ;
N-hexyl-1-[(1-méthyl-1H-imidazol-4-yl)sulfonyl]-4-[(2S)-tétrahydrofuran-2-yl]pyrrolidin-3-amine ;
trans-N-[2-chloro-4-(trifluorométhyl)benzyl]-1-[(1-méthyl-1H-imidazol-4-yl)sulfonyl]-4-(tétrahydrofuran-2-yl)pyrrolidin-3-amine ;
N-[2-chloro-4-(trifluorométhyl)benzyl]-1-[(1-méthyl-1H-imidazol-4-yl)sulfonyl]-4-[(2S)-tétrahydrofuran-2-yl]pyrrolidin-3-amine ;
trans-N-(3-chloro-4-fluorophényl)-1-[(1-méthyl-1H-imidazol-4-yl)sulfonyl]-4-(tétrahydrofuran-2-yl)pyrrolidin-3-amine ;
N-(3-chloro-4-fluorophényl)-1-[(1-méthyl-1H-imidazol-4-yl)sulfonyl]-4-[(2S)-tétrahydrofuran-2-yl]pyrrolidin-3-amine ;
1-benzyl-N-{(3R,4S)-1-[(1-méthyl-1H-imidazol-4-yl)sulfonyl]-4-phénylpyrrolidin-3-yl}-2,3-dihydro-1H-indol-6-amine ;
trans-1-[(1-méthyl-1H-imidazol-4-yl)sulfonyl]-4-(tétrahydrofuran-3-yl)-N-[3-(trifluorométhyl)phényl]pyrrolidin-3-amine ;
trans-1-[(1-méthyl-1H-imidazol-4-yl)sulfonyl]-N-(3-méthylphényl)-4-(tétrahydrofuran-3-yl)pyrrolidin-3-amine ;
trans-N-(3-chlorophényl)-1-[(1-méthyl-1H-imidazol-4-yl)sulfonyl]-4-(tétrahydrofuran-3-yl)pyrrolidin-3-amine ;
trans-N-(3-chloro-4-fluorophényl)-1-[(1-méthyl-1H-imidazol-4-yl)sulfonyl]-4-(tétrahydrofuran-3-yl)pyrrolidin-3-amine ;
trans-N-[4-fluoro-3-(trifluorométhoxy)phényl]-1-[(1-méthyl-1H-imidazol-4-yl)sulfonyl]-4-(tétrahydrofuran-3-yl)pyrrolidin-3-amine ;
trans-N-[4-fluoro-3-(trifluorométhyl)phényl]-1-[(1-méthyl-1H-imidazol-4-yl)sulfonyl]-4-(tétrahydrofuran-3-yl)pyrrolidin-3-amine ;
trans-N-[4-chloro-3-(trifluorométhyl)phényl]-1-[(1-méthyl-1H-imidazol-4-yl)sulfonyl]-4-(tétrahydrofuran-3-yl)pyrrolidin-3-amine ;
trans-1-[(1-méthyl-1H-imidazol-4-yl)sulfonyl]-4-(tétrahydrofuran-3-yl)-N-[3-(2,2,2-trifluoroéthoxy)phényl]pyrrolidin-3-amine ;
trans-1-[(1-méthyl-1H-imidazol-4-yl)sulfonyl]-4-(tétrahydrofuran-3-yl)-N-[3-(trifluorométhoxy)phényl]pyrrolidin-3-amine ;
trans-4-(4-fluorophényl)-1-[(1-méthyl-1H-1,2,3-triazol-4-yl)sulfonyl]-N-[3-(2,2,2-trifluoroéthoxy)phényl]pyrrolidin-3-amine ;
(3R, 4S)4-(4-fluorophényl)-1-[(1-méthyl-1H-1,2,3-triazol-4-yl)sulfonyl]-N-[3-(trifluorométhoxy)phényl]pyrrolidin-3-amine ;
(3R, 4S)-4-(4-fluorophényl)-N-[4-fluoro-3-(trifluorométhoxy)phényl]-1-[(1-méthyl-1H-1,2,3-triazol-4-yl)sulfonyl]pyrrolidin-3-amine ;
(3R, 4S)-4-(4-fluorophényl)-1-[(1-méthyl-1H-1,2,3-triazol-4-yl)sulfonyl]-N-[3-(trifluorométhyl)phényl]pyrrolidin-3-amine ;
(3R, 4S)-4-(4-fluorophényl)-N-[4-fluoro-3-(trifluorométhyl)phényl]-1-[(1-méthyl-1H-1,2,3-triazol-4-yl)sulfonyl]pyrrolidin-3-amine ;
(3R, 4S)-N-(3-chlorophényl)-4-(4-fluorophényl)-1-[(1-méthyl-1H-1,2,3-triazol-4-yl)sulfonyl]pyrrolidin-3-amine ;
(3R, 4S)-N-(3-chloro-4-fluorophényl)-4-(4-fluorophényl)-1-[(1-méthyl-1H-1,2,3-triazol-4-yl)sulfonyl]pyrrolidin-3-amine ;
trans-N-[4-fluoro-3-(trifluorométhyl)phényl]-1-[(1-méthyl-1H-imidazol-4-yl)sulfonyl]-4-(tétrahydrofuran-2-yl)pyrrolidin-3-amine ;
5-chloro-N-[trans-1-[(1-méthyl-1H-imidazol-4-yl)sulfonyl]-4-(tétrahydrofuran-2-yl)pyrrolidin-3-yl]-4-(trifluorométhyl)pyridin-2-amine ;
trans-N-(4-fluoro-3-méthylphényl)-1-[(1-méthyl-1H-imidazol-4-yl)sulfonyl]-4-(tétrahydrofuran-2-yl)pyrrolidin-3-amine ;
trans-N-[4-fluoro-3-(trifluorométhyl)phényl]-1-[(1-méthyl-1H-imidazol-4-yl)sulfonyl]-4-(tétrahydro-2H-pyran-2-yl)pyrrolidin-3-amine ;
trans-N-(4-fluoro-3-méthylphényl)-1-[(1-méthyl-1H-imidazol-4-yl)sulfonyl]-4-(tétrahydro-2H-pyran-2-yl)pyrrolidin-3-amine ;
5-chloro-N-[trans-1-[(1-méthyl-1H-imidazol-4-yl)sulfonyl]-4-(tétrahydro-2H-pyran-2-yl)pyrrolidin-3-yl]-4-(trifluorométhyl)pyridin-2-amine ;
trans-N-(3-chlorophényl)-1-[(1-méthyl-1H-imidazol-4-yl)sulfonyl]-4-(tétrahydro-2H-pyran-2-yl)pyrrolidin-3-amine ;
trans-N-(3-chlorophényl)-1-[(1-méthyl-1H-imidazol-4-yl)sulfonyl]-4-(tétrahydrofuran-2-yl)pyrrolidin-3-amine ;
5-fluoro-4-méthyl-N-[trans-1-[(1-méthyl-1H-imidazol-4-yl)sulfonyl]-4-(tétrahydrofuran-2-yl)pyrrolidin-3-yl]pyridin-2-amine ;
5-fluoro-4-méthyl-N-[trans-1-[(1-méthyl-1H-imidazol-4-yl)sulfonyl]-4-(tétrahydro-2H-pyran-2-yl)pyrrolidin-3-yl]pyridin-2-amine ;
N-[trans-1-[(1-méthyl-1H-imidazol-4-yl)sulfonyl]-4-(tétrahydrofuran-2-yl)pyrrolidin-3-yl]-6-(trifluorométhyl)pyrimidin-4-amine ;
(3R,4S)-N-cyclohexyl-4-(4-fluorophényl)-1-(1-méthyl-1H-imidazol-4-ylsulfonyl)pyrrolidin-3-amine ;
(3R,4S)-N-(3,3-diméthylcyclohexyl)-4-(4-fluorophényl)-1-(1-méthyl-1H-imidazol-4-ylsulfonyl)pyrrolidin-3-amine ;
(3R,4S)-N-(4,4-diméthylcyclohexyl)-4-(4-fluorophényl)-1-(1-méthyl-1H-imidazol-4-ylsulfonyl)pyrrolidin-3-amine ;
(3R,4S)-N-cyclopentyl-4-(4-fluorophényl)-1-(1-méthyl-1H-imidazol-4-ylsulfonyl)pyrrolidin-3-amine ;
(3R,4S)-N-(3,3-diméthylcyclopentyl)-4-(4-fluorophényl)-1-(1-méthyl-1H-imidazol-4-ylsulfonyl)pyrrolidin-3-amine ;
(3R,4S)-N-(1-(2,4-dichlorophényl)éthyl)-4-(4-fluorophényl)-1-(1-méthyl-1H-imidazol-4-ylsulfonyl)pyrrolidin-3-amine ;
(3R,4S)-N-(2,3-dihydro-1H-indén-1-yl)-4-(4-fluorophényl)-1-(1-méthyl-1H-imidazol-4-ylsulfonyl)pyrrolidin-3-amine ;
(3R,4S)-N-(5-chloro-2,3-dihydro-1H-indén-1-yl)-4-(4-fluorophényl)-1-(1-méthyl-1H-imidazol-4-ylsulfonyl)pyrrolidin-3-amine ;
N-(4-chlorobenzyl)-N-((3R,4S)-4-(4-fluorophényl)-1-(1-méthyl-1H-imidazol-4-ylsulfonyl)pyrrolidin-3-yl)acétamide ;
5-butyl-3-{(3R,4S)-4-(4-fluorophényl)-1-[(1-méthyl-1H-imidazol-4-yl)sulfonyl]pyrrolidin-3-yl}-1,3-oxazolidin-2-one;
4-{(3R,4S)-4-(4-fluorophényl)-1-[(1-méthyl-1H-imidazol-4-yl)sulfonyl]pyrrolidin-3-yl}thiomorpholine1,1-dioxyde ;
5-fluoro-N-{(3R,4S)-4-(4-fluorophényl)-1-[(1-méthyl-1H-imidazol-4-yl)sulfonyl]pyrrolidin-3-yl}-4-(trifluorométhyl)pyridin-2-amine ;
(3R,4S)-1-[(1-méthyl-1H-imidazol-4-yl)sulfonyl]-4-[(2R)-tétrahydro-2H-pyran-2-yl]-N-[3-(trifluorométhoxy)phényl]pyrrolidin-3-amine ;
(3R,4S)-4-(4-fluorophényl)-1-[(1-méthyl-1H-imidazol-4-yl)sulfonyl]-N-[2-méthyl-5-(prop-1-én-2-yl)cyclohexyl]pyrrolidin-3-amine ;
(3R,4S)-4-(4-fluorophényl)-1-[(1-méthyl-1H-imidazol-4-yl)sulfonyl]-N-(2,2,2-trifluoro-1-phényléthyl)pyrrolidin-3-amine ;
N-[2-chloro-4-(trifluorométhyl)benzyl]-2-[(1-méthyl-1H-imidazol-4-yl)sulfonyl]octahydro-3aH-isoindol-3a-amine ;
2-chloro-N-{2-[(1-méthyl-1H-imidazol-4-yl)sulfonyl]octahydro-3aH-isoindol-3a-yl}-3-(trifluorométhyl)benzamide ;
2,4-dichloro-N-{2-[(1-méthyl-1H-imidazol-4-yl)sulfonyl]octahydro-3aH-isoindol-3a-yl}benzamide ;
1-hexyl-6-[(1-méthyl-1H-imidazol-4-yl)sulfonyl]octahydro-1H-pyrrolo[3,4-b]pyridine ;
(2,4-dichlorophényl){6-[(1-méthyl-1H-imidazol-4-yl)sulfonyl]octahydro-1H-pyrrolo[3,4-b]pyridin-1-yl}méthanone ;
[2-chloro-3-(trifluorométhyl)phényl]{6-[(1-méthyl-1H-imidazol-4-yl)sulfonyl]octahydro-1H-pyrrolo[3,4-b]pyridin-1-yl}méthanone ;
(3R,4S)-4-(4-fluorophényl)-1-[(1-méthyl-1H-imidazol-4-yl)sulfonyl]-N-[2,2,2-trifluoro-1-(3-méthylphényl)éthyl]pyrrolidin-3-amine ;
(3R,4S)-4-(4-fluorophényl)-1-[(1-méthyl-1H-imidazol-4-yl)sulfonyl]-N-[2,2,2-trifluoro-1-(4-méthylphényl)éthyl]pyrrolidin-3-amine ;
(1R,2R,3R,4S)-3-(4-fluorophényl)-N-[4-fluoro-3-(trifluorométhyl)phényl]-7-[(1-méthyl-1H-imidazol-4-yl)sulfonyl]-7-azabicyclo[2.2.1]heptan-2-amine ;
3-(4-fluorophényl)-N-[4-fluoro-3-(trifluorométhyl)phényl]-7-[(1-méthyl-1H-imidazol-4-yl)sulfonyl]-7-azabicyclo[2.2.1]heptan-2-amine ;
1-[2-chloro-4-(trifluorométhyl)benzyl]-6-[(1-méthyl-1H-imidazol-4-yl)sulfonyl]octahydro-1H-pyrrolo[3,4-b]pyridine ;
[2-chloro-4-(trifluorométhyl)phényl]{6-[(1-méthyl-1H-imidazol-4-yl)sulfonyl]octahydro-1H-pyrrolo[3,4-b]pyridin-1-yl}méthanone ;
N-(3-chloro-4-fluorobenzyl)-2-[(1-méthyl-1H-imidazol-4-yl)sulfonyl]-2-azaspiro[4.5]décane-4-carboxamide ;
N-(3,4-difluorobenzyl)-2-[(1-méthyl-1H-imidazol-4-yl)sulfonyl]-2-azaspiro[4.5]décane-4-carboxamide ;
1-{(3R,4S)-4-(4-fluorophényl)-1-[(1-méthyl-1H-imidazol-4-yl)sulfonyl]pyrrolidin-3-yl}-1,3-dihydro-2H-indol-2-one ;
2-[(1-méthyl-1H-imidazol-4-yl)sulfonyl]-N-[4-(trifluorométhyl)benzyl]octahydro-3aH-isoindol-3a-amine ;
trans-N-[2-chloro-4-(trifluorométhyl)benzyl]-2-[(1-méthyl-1H-imidazol-4-yl)sulfonyl]-2,3,3a,8-tétrahydroindéno[1,2-c]pyrrol-8a(1H)-amine ;
2-{4-[(4-fluorophénoxy)méthyl]-1-[(1-méthyl-1H-imidazol-4-yl)sulfonyl]pyrrolidin-3-yl}pyridine ;
2-[(1-méthyl-1H-imidazol-4-yl)sulfonyl]-3a-{[6-(trifluorométhyl)pyrimidin-4-yl]oxy}-2,3,3a,4,5,9b-hexahydro-1H-benzo[e]isoindole ;
N-(4-fluorobenzyl)-2-[(1-méthyl-1H-imidazol-4-yl)sulfonyl]-1,2,3,4,5,9b-hexahydro-3aH-benzo[e]isoindol-3a-amine ;
N-[2-chloro-4-(trifluorométhyl)benzyl]-2-[(1-méthyl-1H-imidazol-4-yl)sulfonyl]-1,2,3,4,5,9b-hexahydro-3aH-benzo[e]isoindol-3a-amine ;
2-[(1-méthyl-1H-1,2,3-triazol-4-yl)sulfonyl]-N-[6-(trifluorométhyl)pyrimidin-4-yl]-2,3,3a,8-tétrahydroindéno[1,2-c]pyrrol-8a(1H)-amine ;
2-[(1-méthyl-1H-imidazol-4-yl)sulfonyl]-N-[4-(trifluorométhyl)pyridin-2-yl]-2,3,3a,8-tétrahydroindéno[1,2-c]pyrrol-8a(1H)-amine ;
N-(2,3-dihydro-1H-indén-1-yl)-1-[(1-méthyl-1H-imidazol-4-yl)sulfonyl]-4-(1-méthylpipéridin-2-yl)pyrrolidin-3-amine ;
1-[(1-méthyl-1H-imidazol-4-yl)sulfonyl]-4-(1-méthylpipéridin-2-yl)-N-[3-(2,2,2-trifluoroéthoxy)phényl]pyrrolidin-3-amine ;
4-(5-fluoropyridin-2-yl)-1-[(1-méthyl-1H-imidazol-4-yl)sulfonyl]-N-[3-(trifluorométhoxy)phényl]pyrrolidin-3-amine ;
(3R,4S)-4-(1,3-dioxan-2-yl)-1-[(1-méthyl-1H-1,2,3-triazol-4-yl)sulfonyl]-N-[3-(trifluorométhoxy)phényl]pyrrolidin-3-amine ;
(3R,4S)-4-(1,3-dioxan-2-yl)-1-[(1-méthyl-1H-imidazol-4-yl)sulfonyl]-N-[3-(trifluorométhoxy)phényl]pyrrolidin-3-amine ;
1-[(1-méthyl-1H-imidazol-4-yl)sulfonyl]-4-(1-méthylpipéridin-2-yl)-N-[3-(trifluorométhoxy)phényl]pyrrolidin-3-amine ;
1-[(1-méthyl-1H-imidazol-4-yl)sulfonyl]-4-(1-méthylpipéridin-2-yl)-N-[3-(trifluorométhoxy)phényl]pyrrolidin-3-amine ;
(3R,4S)-4-(5,5-diméthyl-1,3-dioxan-2-yl)-1-[(1-méthyl-1H-imidazol-4-yl)sulfonyl]-N-[3-(trifluorométhoxy)phényl]pyrrolidin-3-amine ;
(3R,4S)-4-(1,3-dioxépan-2-yl)-1-[(1-méthyl-1H-imidazol-4-yl)sulfonyl]-N-[3-(trifluorométhoxy)phényl]pyrrolidin-3-amine ;
(3R,4S)-4-(1,3-dioxolan-2-yl)-1-[(1-méthyl-1H-imidazol-4-yl)sulfonyl]-N-[3-(trifluorométhoxy)phényl]pyrrolidin-3-amine ;
(3R,4S)-N-(2,3-dihydro-1H-indén-1-yl)-4-(1,3-dioxan-2-yl)-1-[(1-méthyl-1H-imidazol-4-yl)sulfonyl]pyrrolidin-3-amine ;
(3R,4S)-N-[2-chloro-4-(trifluorométhyl)benzyl]-4-(1,3-dioxan-2-yl)-1-[(1-méthyl-1H-imidazol-4-yl)sulfonyl]pyrrolidin-3-amine ;
(3R,4S)-4-(1,3-dioxan-2-yl)-1-[(1-méthyl-1H-imidazol-4-yl)sulfonyl]-N-[3-(trifluorométhoxy)benzyl]pyrrolidin-3-amine ;
(3R,4S)-4-(1,3-dioxan-2-yl)-N-(4-fluorobenzyl)-1-[(1-méthyl-1H-imidazol-4-yl)sulfonyl]pyrrolidin-3-amine ;
(3R,4S)-4-(1,3-dioxan-2-yl)-N-méthyl-1-[(1-méthyl-1H-imidazol-4-yl)sulfonyl]-N-pentylpyrrolidin-3-amine ;
(3R,4S)-4-(1,3-dioxan-2-yl)-1-[(1-méthyl-1H-imidazol-4-yl)sulfonyl]-N,N-dipentylpyrrolidin-3-amine ;
(3R,4S)-4-(1,3-dioxan-2-yl)-N,N-dihexyl-1-[(1-méthyl-1H-imidazol-4-yl)sulfonyl]pyrrolidin-3-amine ;
(3R,4S)-4-(1,3-dioxan-2-yl)-N-hexyl-1-[(1-méthyl-1H-imidazol-4-yl)sulfonyl]pyrrolidin-3-amine ;
5-chloro-N-{(3R,4S)-4-(1,3-dioxan-2-yl)-1-[(1-méthyl-1H-imidazol-4-yl)sulfonyl]pyrrolidin-3-yl}-4-(trifluorométhyl)pyridin-2-amine ;
(3R,4S)-4-(1,3-dioxan-2-yl)-N-[4-fluoro-3-(trifluorométhyl)phényl]-1-[(1-méthyl-1H-imidazol-4-yl)sulfonyl]pyrrolidin-3-amine ;
1-[(1-méthyl-1H-imidazol-4-yl)sulfonyl]-4-(pipéridin-1-yl)-N-[3-(trifluorométhoxy)phényl]pyrrolidin-3-amine ;
(3R,4S)-N-(3-chlorophényl)-4-(1,3-dioxan-2-yl)-1-[(1-méthyl-1H-imidazol-4-yl)sulfonyl]pyrrolidin-3-amine ;
(3R,4S)-4-(1,3-dioxan-2-yl)-1-[(1-méthyl-1H-imidazol-4-yl)sulfonyl]-N-[3-(trifluorométhyl)phényl]pyrrolidin-3-amine ;
(3R,4S)-N-(3-chloro-4-fluorophényl)-4-(1,3-dioxan-2-yl)-1-[(1-méthyl-1H-imidazol-4-yl)sulfonyl]pyrrolidin-3-amine ;
(3R,4S)-4-(1,3-dioxan-2-yl)-1-[(1-méthyl-1H-imidazol-4-yl)sulfonyl]-N-(3-méthylphényl)pyrrolidin-3-amine ;
4-({3-(1,3-dioxan-2-yl)-4-[3-(trifluorométhyl)benzyl]pyrrolidin-1-yl}sulfonyl)-1-méthyl-1H-imidazole ;
4-({3-(1,3-dioxan-2-yl)-4-[3-(trifluorométhyl)benzyl]pyrrolidin-1-yl}sulfonyl)-1-méthyl-1H-1,2,3-triazole ;
(3R,4S)-4-(5,7-dioxaspiro[2.5]oct-6-yl)-1-[(1-méthyl-1H-imidazol-4-yl)sulfonyl]-N-[3-(trifluorométhoxy)phényl]pyrrolidin-3-amine ;
(3R,4S)-4-[(4R,6R)-4,6-diméthyl-1,3-dioxan-2-yl]-1-[(1-méthyl-1H-imidazol-4-yl)sulfonyl]-N-[3-(trifluorométhoxy)phényl]pyrrolidin-3-amine ;
(3R,4S)-4-[(4S,6S)-4,6-diméthyl-1,3-dioxan-2-yl]-1-[(1-méthyl-1H-imidazol-4-yl)sulfonyl]-N-[3-(trifluorométhoxy)phényl]pyrrolidin-3-amine ;
(3R,4S)-4-(1,3-dioxan-2-yl)-N-(4-fluoro-3-méthylphényl)-1-[(1-méthyl-1H-imidazol-4-yl)sulfonyl]pyrrolidin-3-amine ;
(3R,4S)-4-(1,3-dioxan-2-yl)-N-[4-fluoro-3-(trifluorométhoxy)phényl]-1-[(1-méthyl-1H-imidazol-4-yl)sulfonyl]pyrrolidin-3-amine ;
(3R,4S)-N-[3-(difluorométhoxy)phényl]-4-(1,3-dioxan-2-yl)-1-[(1-méthyl-1H-imidazol-4-yl)sulfonyl]pyrrolidin-3-amine ;
(3R,4S)-4-(1,3-dioxan-2-yl)-1-[(1-méthyl-1H-imidazol-4-yl)sulfonyl]-N-[3-(propan-2-yl)phényl]pyrrolidin-3-amine ;
4-(5-fluoropyridin-2-yl)-N-[4-fluoro-3-(trifluorométhyl)phényl]-1-[(1-méthyl-1H-imidazol-4-yl)sulfonyl]pyrrolidin-3-amine ;
(3R,4S)-N-[3-(2,2-difluoroéthoxy)phényl]-4-(1,3-dioxan-2-yl)-1-[(1-méthyl-1H-imidazol-4-yl)sulfonyl]pyrrolidin-3-amine,
ou leurs sels physiologiquement tolérés.

16. Composé selon la revendication 1, qui est (3R,4S)-1-[(1-méthyl-1H-imidazol-4-yl)sulfonyl]-4-[(2R)-tétrahydro-2H-pyran-2-yl]-N-[3-(trifluorométhoxy)phényl]pyrrolidin-3-amine, ou sel physiologiquement toléré de celui-ci.

17. Composé selon la revendication 1, qui est (3R,4S)-4-(1,3-dioxan-2-yl)-1-[(1-méthyl-1H-imidazol-4-yl)sulfonyl]-N-[3-(trifluorométhoxy)phényl]pyrrolidin-3-amine, ou sel physiologiquement toléré de celui-ci.

18. Composé selon la revendication 1, qui est (3R,4S)-N-(2,3-dihydro-1H-indén-1-yl)-4-(4-fluorophényl)-1-(1-méthyl-1H-imidazol-4-ylsulfonyl)pyrrolidin-3-amine, ou sel physiologiquement toléré de celui-ci.

19. Composé selon la revendication 1, qui est 4-({(3S,4R)-4-(4-fluorophényl)-1-[(1-méthyl-1H-imidazol-4-yl)sulfonyl]pyrrolidin-3-yl}oxy)-6-(trifluorométhyl)pyrimidine, ou sel physiologiquement toléré de celui-ci.
